(19) Europäisches Patentamt / European Patent Office / Office européen des brevets

(11) **EP 4 623 936 A1**

(12) **EUROPEAN PATENT APPLICATION**
published in accordance with Art. 153(4) EPC


(43) Date of publication:
**01.10.2025 Bulletin 2025/40**

(21) Application number: **23893928.4**

(22) Date of filing: **22.11.2023**

(51) International Patent Classification (IPC):
***A61K 47/68*** (2017.01) ***A61K 47/65*** (2017.01)
***A61K 39/395*** (2006.01) ***C07K 16/28*** (2006.01)
***C07K 16/30*** (2006.01) ***C07D 491/22*** (2006.01)
***C07D 491/147*** (2006.01) ***A61K 31/47*** (2006.01)
***A61K 31/4745*** (2006.01) ***A61K 31/437*** (2006.01)
***A61K 31/357*** (2006.01) ***A61K 31/40*** (2006.01)
***A61K 31/165*** (2006.01) ***A61K 31/4375*** (2006.01)
***A61P 35/00*** (2006.01) ***A61P 35/02*** (2006.01)

(52) Cooperative Patent Classification (CPC):
**A61K 31/165; A61K 31/357; A61K 31/40; A61K 31/437; A61K 31/4375; A61K 31/47; A61K 31/4745; A61K 39/395; A61K 47/65; A61K 47/68; A61P 35/00; A61P 35/02; C07D 491/147; C07D 491/22; C07K 16/28;** (Cont.)

(86) International application number:
**PCT/CN2023/133434**

(87) International publication number:
**WO 2024/109840 (30.05.2024 Gazette 2024/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **22.11.2022 CN 202211545125**
**09.06.2023 CN 202310683602**



(71) Applicant: **Keymed Biosciences (Chengdu) Co., Ltd.**
**Chengdu, Sichuan 610219 (CN)**

(72) Inventor: **CHEN, Bo**
**Chengdu, Sichuan 610219 (CN)**

(74) Representative: **PGA S.p.A., Milano, Succursale di Lugano**
**Via Castagnola, 21c**
**6900 Lugano (CH)**


Remarks:
The complete document including Reference Table(s) and the Sequence Listing(s) can be downloaded from the EPO website

(54) **FUSED RING COMPOUND, CONJUGATE THEREOF AND USE THEREOF**


(57) The present disclosure relates to a fused ring compound, a conjugate thereof and the use thereof. The compound and the conjugate of the present disclosure have good activity of inhibiting tumor cells, stability, and *in vivo* drug efficacy in animals.


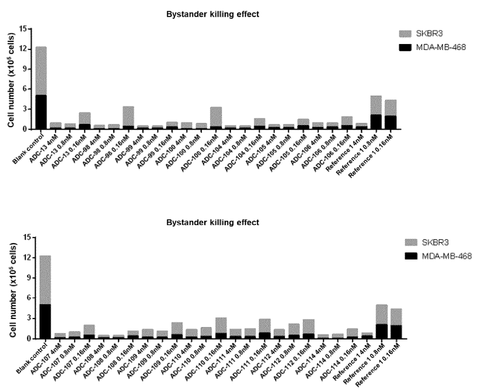

Figure 1

EP 4 623 936 A1

(52) Cooperative Patent Classification (CPC): (Cont.)
**C07K 16/30**

## Description

The present application claims the priority rights of the following prior applications: the Chinese invention patent application with application number 202211545125.3 and title "Fused ring compound, conjugate thereof and use thereof" filed with China National Intellectual Property Administration on November 22, 2022, and the Chinese invention patent application with application number 202310683602.0 and title "Fused ring compound, conjugate thereof and use thereof" filed with China National Intellectual Property Administration on June 9, 2023. The full text of the above-mentioned prior applications is incorporated herein by reference.

## TECHNICAL FIELD

The present disclosure relates to a fused ring compound, conjugate thereof and use thereof, belonging to the field of medicine.

## BACKGROUND

The concept of antibody-drug conjugate (ADC) or ADC drug has a long history. As early as 1913, Nobel Prize winner Professor Paul Ehrlich first proposed the concept of "magic bullet", which is to install a cytotoxic drug on a specific monoclonal antibody to achieve targeted killing of tumor cells. The ADC drugs currently on the market are based on this theory. By conjugating a cytotoxic drug to a monoclonal antibody, the monoclonal antibody is used as a carrier to efficiently deliver a small molecule cytotoxic drug to a target tumor cell in a targeted manner.

ADC drug uses a specific linker to connect an antibody and a small molecule cytotoxic drug, and its main components include antibody, linker and small molecule cytotoxic drug. After an ADC drug enters the blood, its antibody component can recognize a target and binds to a tumor cell that highly expresses a cell surface antigen. When the ADC-antigen complex enters the tumor cell through endocytosis, the complex is degraded by lysosome, and the cytotoxic load (drug) will be released, destroying DNA or preventing tumor cell division, thereby killing the tumor cell.

The first-generation ADC drugs emerged from an attempt in 1958 to use anti-mouse leukocyte immunoglobulin coupled with methotrexate to treat leukemia. Mylotarg is the first launched ADC drug. It was approved by the FDA in 2000 for the treatment of acute myeloid leukemia, but it was withdrawn from the market because Mylotarg caused severe and fatal liver damage and had no obvious survival benefit. The disadvantages of the first-generation ADC drugs include: their antibodies were murine, their cytotoxicity was not potent enough, and their targets were low-expressed.

The starting point for the development of the second-generation ADC drugs is Trastuzumab Emtansine, which is the first approved ADC drug targeting breast cancer. The most successful ADC drug in the field of hematological tumors is Brentuximab Vedotin, which mainly targets classical Hodgkin's lymphoma and anaplastic large cell lymphoma. The advantages of the second-generation ADC drugs include: diversity in the development of target antigens and humanized antibodies, and the disadvantages include: too low or too high drug loading rate, narrow therapeutic window and low effectiveness.

The typical representative of the third-generation ADC drugs is Enfortumab Vedotin, which is the second ADC drug targeting solid tumors. It is suitable for patients to whom PD-1/PD-L1 antibody treatment is ineffective, but is not sensitive to tubulin inhibitors.

At present, it is still necessary to develop a drug molecule and conjugate thereof with reasonable molecular structure design and improved pharmacodynamic activity.

## SUMMARY OF THE INVENTION

To solve the above technical problems, the present disclosure provides a conjugate represented by the following Formula (C), or a stereoisomer, racemate, tautomer, isotopologue, isotope-labeled compound, nitroxide or pharmaceutically acceptable salt thereof:

Tp-L-G (C)

wherein, Tp represents a targeting moiety;

L is selected from a chemical bond or a linker;

G represents a group represented by the following Formula (G):

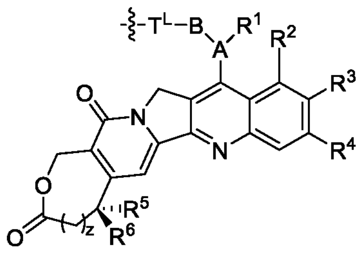

(G)

wherein, A is selected from N or C-$R^A$;

when A is N, $R^1$ is selected from the group consisting of hydrogen, hydroxyl, and the following groups which are unsubstituted or substituted with one, two or more $R^B$: alkyl, cycloalkyl, cycloalkylalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

when A is C-$R^A$, $R^A$ and $R^1$ are the same or different and are independently selected from the group consisting of hydrogen, halogen, hydroxyl, sulfhydryl, cyano, nitro, and the following groups which are unsubstituted or substituted with one, two or more $R^C$: alkyl, cycloalkyl, cycloalkylalkyl, alkyloxy, cycloalkyloxy, alkyloxyalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

or, $R^A$ and $R^1$, together with the atom to which they are attached, form a 3- to 10-membered ring structure which is unsubstituted or substituted with one, two or more $R^D$;

z is selected from 0 or 1;

$R^2$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

$R^3$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

or, $R^1$ and $R^2$, together with the atoms to which they are attached, form a ring structure which is unsubstituted or substituted with one, two or more $R^E$, for example, the ring structure is a 5- to 10-membered ring structure, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a ring structure which is unsubstituted or substituted with one, two or more $R^F$, for example, the ring structure is a 4- to 10-membered ring structure, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of alkyl, alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: cycloalkyl, cycloalkylalkyl, cycloalkyloxy, heterocyclyl, heterocyclylalkyl, heterocyclyloxy, alkyl, alkyloxy, aryl, arylalkyl, aryloxy, heteroaryl, heteroarylalkyl, heteroaryloxy, amino;

B is absent or selected from the group consisting of triazolyl, -$NR^7$- or -$N(NR^7R^8)$-;

$R^7$ and $R^8$ are the same or different and are independently selected from the group consisting of hydrogen and the following groups which are unsubstituted or substituted with one, two or more $R^H$: alkyl, cycloalkyl, cycloalkylalkyl, aryl, arylalkyl, heteroaryl, heteroarylalkyl, heterocyclyl, heterocyclylalkyl, HC(=O)-, alkyl-C(=O)-, cycloalkyl-C(=O)NH-, heterocyclyl-C(=O)NH-, aryl-C(=O)NH-, heteroaryl-C(=O)NH-;

$T^L$ is selected from the group consisting of chemical bond, #O-$(CR^9R^{10})_m$-$(CR^{11}R^{12})_n$-C(=O)*, #O-$(CR^9R^{10})_m$-$(CR^{11}R^{12})_n$*, #N($R^{13}$)-$(CR^9R^{10})^m$-$(CR^{11}R^{12})_n$-C(O)*, #S-$(CR^9R^{10})_m$-$(CR^{11}R^{12})_n$-C(O)*, #O-$(CR^9R^{10})_m$-$(CR^{11}R^{12})_n$-C($NR^{13}$)*, #O-$(CR^9R^{10})_m$-$(CR^{11}R^{12})_n$-P(=O)($OR^{14}$)*, *O-$(CR^9R^{10})_m$-$(CR^{11}R^{12})_n$-C(=O)#, *O-$(CR^9R^{10})_m$-$(CR^{11}R^{12})_n$#, *N($R^{13}$)-$(CR^9R^{10})_m$-$(CR^{11}R^{12})_n$-C(O)#, *S-$(CR^9R^{10})_m$-$(CR^{11}R^{12})_n$-C(O)#, *O-$(CR^9R^{10})_m$-$(CR^{11}R^{12})_n$-C($NR^{13}$)#, *O-$(CR^9R^{10})_m$-$(CR^{11}R^{12})_n$-P(=O)($OR^{14}$)#, wherein # represents the site attached to L, and * represents the site attached to B;

each of $R^9$, $R^{10}$, $R^{11}$ and $R^{12}$ is the same or different, and is independently selected from the group consisting of hydrogen, halogen, cyano, and the following groups which are unsubstituted or substituted with one, two or more $R^I$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy;

or, $R^9$ and $R^{10}$, together with the atoms to which they are attached, form a ring structure which is unsubstituted or substituted with one, two or more $R^I$, for example, the ring structure is a 3- to 10-membered ring structure; or, $R^{11}$ and $R^{12}$, together with the atoms to which they are attached, form a ring structure which is unsubstituted or substituted with one, two or more $R^I$, for example, the ring structure is a 3- to 10-membered ring structure; or, $R^9$ and $R^{11}$, together with the atoms to which they are attached, form a ring structure which is unsubstituted or substituted with one, two or more $R^I$, for example, the ring structure is a 3- to 10-membered ring structure; wherein any of the ring structures can be a monocyclic hydrocarbyl, bicyclic hydrocarbyl, heteromonocyclic hydrocarbyl, or heterobicyclic hydrocarbyl, each of which is unsubstituted or substituted with one, two or more $R^I$; for example, any of the ring structures can be the following group which is unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: alkyl, cycloalkyl, cycloalkylalkyl, aryl, arylalkyl, heteroaryl, heteroarylalkyl, heterocyclyl, heterocyclylalkyl;

each m is the same or different, and is independently selected from an integer of 0 to 10;

each n is the same or different, and is independently selected from an integer of 0 to 10;

each of $R^B$, $R^C$, $R^D$, $R^E$, $R^F$, $R^G$, $R^H$, $R^I$ and $R^J$ is the same or different, and is independently selected from the group consisting of deuterium, halogen, hydroxyl, cyano, nitro, and the following groups which are unsubstituted or substituted with one, two or more $R^K$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^L$ is the same or different and is independently selected from the group consisting of deuterium, halogen, hydroxyl, cyano, nitro, alkyl, aryloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

the wavy line represents the site attached to L;

provided that, when $R^6$ is selected from the group consisting of alkyl, alkyloxy, aryl, arylalkyl, aryloxy, heteroaryl, heteroarylalkyl, heteroaryloxy or amino, each of which is unsubstituted or substituted with one, two or more $R^G$:

A is N; $R^1$ and $R^2$, together with the atoms to which they are attached, do not form a 5- to 10-membered ring structure which is unsubstituted or substituted with one, two or more $R^E$; and $T^L$ is not a chemical bond when $R^7$ is hydrogen;

or, $R^1$ and $R^2$, together with the atoms to which they are attached, form a 5- to 10-membered ring structure which is unsubstituted or substituted with one, two or more $R^E$, and the 5- to 10-membered ring structure can be selected from,

for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbonyl; and $R^A$ is selected from the following groups which are unsubstituted or substituted with one, two or more $R^C$: cycloalkyl, cycloalkylalkyl, cycloalkyloxy;

or, B is $-N(NR^7R^8)-$;

or, $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl.

According to the embodiments of the present invention, definitions of the groups in Formula (G) are independently selected from:

A is selected from N or $C-R^A$;

when A is N, $R^1$ is selected from the group consisting of hydrogen, hydroxyl, and the following groups which are unsubstituted or substituted with one, two or more $R^B$: alkyl, cycloalkyl, cycloalkylalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

when A is $C-R^A$, $R^A$ and $R^1$ are the same or different and are independently selected from the group consisting of hydrogen, halogen, hydroxyl, sulfhydryl, cyano, nitro, and the following groups which are unsubstituted or substituted with one, two or more $R^C$: alkyl, cycloalkyl, cycloalkylalkyl, alkyloxy, cycloalkyloxy, alkyloxyalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

or, $R^A$ and $R^1$, together with the atom to which they are attached, form a 3- to 10-membered ring structure which is unsubstituted or substituted with one, two or more $R^D$;

z is selected from 0 or 1;

$R^2$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

$R^3$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

or, $R^1$ and $R^2$, together with the atoms to which they are attached, form a 5- to 10-membered ring structure which is unsubstituted or substituted with one, two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, or heterobicyclic hydrocarbyl;

or $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, or heterobicyclic hydrocarbyl;

$R^4$ is selected from the group consisting of alkyl, alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: cycloalkyl, cycloalkylalkyl, cycloalkyloxy, heterocyclyl, heterocyclylalkyl, heterocyclyloxy;

B is absent or selected from the group consisting of triazolyl, $-NR^7-$ or $-N(NR^7R^8)-$;

$R^7$ and $R^8$ are the same or different and are independently selected from the group consisting of hydrogen, and the

following groups which are unsubstituted or substituted with one, two or more $R^H$: alkyl, cycloalkyl, cycloalkylalkyl, aryl, arylalkyl, heteroaryl, heteroarylalkyl, heterocyclyl, heterocyclylalkyl, HC(=O)-, alkyl-C(=O)-, cycloalkyl-C(=O)NH-, heterocyclyl-C(=O)NH-, aryl-C(=O)NH-, heteroaryl-C(=O)NH-;

$T^L$ is selected from the group consisting of chemical bond, #O-$(CR^9R^{10})_m$-$(CR^{11}R^{12})_n$-C(=O)*, #O-$(CR^9R^{10})_m$-$(CR^{11}R^{12})_n$*, #N($R^{13}$)-$(CR^9R^{10})^m$-$(CR^{11}R^{12})_n$-C(O)*, #S-$(CR^9R^{10})_m$-$(CR^{11}R^{12})_n$-C(O)*, #O-$(CR^9R^{10})_m$-$(C^{11}R^{12})_n$-C($NR^{13}$)*, #O-$(CR^9R^{10})_m$-$(CR^{11}R^{12})_n$-P(=O)($OR^{14}$)*, *O-$(CR^9R^{10})_m$-$(CR^{11}R^{12})_n$-C(=O)#, *O-$(CR^9R^{10})_m$-$(CR^{11}R^{12})_n$#, *N($R^{13}$)-$(CR^9R^{10})_m$-$(CR^{11}R^{12})_n$-C(O)#, *S-$(CR^9R^{10})_m$-$(CR^{11}R^{12})_n$-C(O)#, *O-$(CR^9R^{10})_m$-$(CR^{11}R^{12})_n$-C($NR^{13}$)#, *O-$(CR^9R^{10})_m$-$(CR^{11}R^{12})_n$-P(=O)($OR^{14}$)#, wherein # represents the site attached with L, and * represents the site attached with B;

each of $R^9$, $R^{10}$, $R^{11}$ and $R^{12}$ is the same or different and is independently selected from the group consisting of hydrogen, halogen, cyano, and the following groups which are unsubstituted or substituted with one, two or more $R^I$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; wherein any of the 3- to 10-membered ring structures can be selected from the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and are independently selected from the following groups which are unsubstituted or substituted with one, two or more $R^J$: alkyl, cycloalkyl, cycloalkylalkyl, aryl, arylalkyl alkyl, heteroaryl, heteroarylalkyl, heterocyclyl, heterocycloalkyl;

m and n are the same or different and are independently selected from an integer of 0 to 10;

each of $R^B$, $R^C$, $R^D$, $R^E$, $R^F$, $R^G$, $R^H$, $R^I$ and $R^J$ is the same or different and are independently selected from the group consisting of deuterium, halogen, hydroxyl, cyano, nitro, and the following groups which are unsubstituted or substituted with one, two or more $R^K$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^K$ is the same or different, and is independently selected from the group consisting of deuterium, halogen, hydroxyl, cyano, nitro, and the following groups which are unsubstituted or substituted with one, two or more $R^L$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^L$ is the same or different and are independently selected from the group consisting of deuterium, halogen, hydroxyl, cyano, nitro, alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-.

According to the embodiments of the present invention, definitions of the groups in Formula (G) may be independently selected from:

A is selected from N or C-$R^A$;

when A is N, $R^1$ is selected from the group consisting of hydrogen, hydroxyl, and the following groups which are unsubstituted or substituted with one, two or more $R^B$: alkyl, cycloalkyl, cycloalkylalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

when A is C-$R^A$, $R^A$ and $R^1$ are the same or different and are independently selected from the group consisting of hydrogen, halogen, hydroxyl, sulfhydryl, cyano, nitro, and the following groups which are unsubstituted or substituted with one, two or more $R^C$: alkyl, cycloalkyl, cycloalkylalkyl, alkyloxy, cycloalkyloxy, alkyloxy alkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

or, $R^A$ and $R^1$, together with the atom to which they are attached, form a 3- to 10-membered ring structure which is unsubstituted or substituted with one, two or more $R^D$;

z is selected from 0 or 1;

$R^2$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

$R^3$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

or, $R^1$ and $R^2$, together with the atoms to which they are attached, form a 5- to 10-membered ring structure which is unsubstituted or substituted with one, two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from alkyl, alkyloxy, halogen, hydroxyl, amino, cyano;

$R^5$ is selected from hydroxyl;

$R^6$ is selected from the following groups which are unsubstituted or substituted with one, two or more $R^G$: cycloalkyl, cycloalkylalkyl, cycloalkyloxy, heterocyclyl, heterocyclylalkyl, heterocyclyloxy;

B is absent or selected from -$NR^7$- or -$N(NR^7R^8)$-;

$R^7$ and $R^8$ are the same or different and are independently selected from the group consisting of hydrogen, and the following groups which are unsubstituted or substituted with one, two or more $R^H$: alkyl, cycloalkyl, cycloalkylalkyl, aryl, arylalkyl, heteroaryl, heteroarylalkyl, heterocyclyl, heterocyclylalkyl, HC(=O)-, alkyl-C(=O)-, cycloalkyl-C(=O)NH-, heterocyclyl-C(=O)NH-, aryl-C(=O)NH-, heteroaryl-C(=O)NH-;

$T^L$ is selected from the group consisting of chemical bond, #O-$(CR^9R^{10})_m$-$(CR^{11}R^{12})_n$-C(=O)*, #O-$(CR^9R^{10})_m$-$(CR^{11}R^{12})_n$*, #N($R^{13}$)-$(CR^9R^{10})^m$-$(CR^{11}R^{12})_n$-C(O)*, #S-$(CR^9R^{10})_m$-$(CR^{11}R^{12})_n$-C(O)*, #O-$(CR^9R^{10})_m$-$(CR^{11}R^{12})_n$-C($NR^{13}$)*, #O-$(CR^9R^{10})_m$-$(CR^{11}R^{12})_n$-P(=O)($OR^{14}$)*, *O-$(CR^9R^{10})_m$-$(CR^{11}R^{12})_n$-C(=O)#, *O-$(CR^9R^{10})_m$-$(CR^{11}R^{12})_n$#, *N($R^{13}$)-$(CR^9R^{10})_m$-$(CR^{11}R^{12})_n$-C(O)#, *S-$(CR^9R^{10})_m$-$(CR^{11}R^{12})_n$-C(O)#, *O-$(CR^9R^{10})_m$-$(CR^{11}R^{12})_n$-C($NR^{13}$)#, *O-$(CR^9R^{10})_m$-$(CR^{11}R^{12})_n$-P(=O)($OR^{14}$)#, wherein # represents the site attached to L, and * represents the site attached to B;

each of $R^9$, $R^{10}$, $R^{11}$ and $R^{12}$ is the same or different and is independently selected from the group consisting of hydrogen, halogen, cyano, and the following groups which are unsubstituted or substituted with one, two or more $R^I$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; wherein any of the 3- to 10-membered ring structures can be

selected from, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: alkyl, cycloalkyl, cycloalkylalkyl, aryl, arylalkyl, heteroaryl, heteroarylalkyl, heterocyclyl, heterocyclylalkyl;

m and n are the same or different, and are independently selected from an integer of 0 to 10;

each of $R^B$, $R^C$, $R^D$, $R^E$, $R^F$, $R^G$, $R^H$, $R^I$ and $R^J$ is the same or different, and is independently selected from the group consisting of deuterium, halogen, hydroxyl, cyano, nitro, and the following groups which are unsubstituted or substituted with one, two or more $R^K$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^K$ is the same or different, and is independently selected from the group consisting of deuterium, halogen, hydroxyl, cyano, nitro, and the following groups which are unsubstituted or substituted with one, two or more $R^L$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^L$ is the same or different and is independently selected from the group consisting of deuterium, halogen, hydroxyl, cyano, nitro, alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-.

According to the embodiments of the present invention, in the Formula (G) described in the context, the hydrogen, carbon or other atoms in the formula are optionally replaced by its isotope, regardless of whether the group is defined as being selected from the above definitions or directly indicated in the formula, and regardless of whether the group is defined as a substituted group or an unsubstituted group. For example, the hydrogen in any group of the formula (G) can be selected from $^1H$, $^2H$ or $^3H$. As an example, any hydrogen in the substituent of the unsubstituted or substituted alkyl, alkylene, alkylidene, phenyl, pyridyl or lactone group or on the ring-forming atoms thereof (if any) in the compound of formula (G) can be independently selected from $^1H$, $^2H$ or$^3H$.

According to the embodiments of the present invention, definitions of the groups in Formula (G) may be independently selected from:

A is selected from N or C-$R^A$;

when A is N, $R^1$ is selected from the group consisting of $^1H$, $^2H$, hydroxyl, deuterated hydroxyl, and the following groups which are unsubstituted or substituted with one, two or more $R^B$: $C_{1-10}$ alkyl, $C_{3-10}$ cycloalkyl, $C_{3-10}$ cycloalkyl-$C_{1-10}$ alkyl, 3- to 10-membered heterocyclyl, 3-to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is N, $R^1$ is selected from the group consisting of $^1H$, $^2H$, hydroxyl, deuterated hydroxyl, and the following groups which are unsubstituted or substituted with one, two or more $R^B$: $C_{1-6}$ alkyl, $C_{3-6}$ cycloalkyl, $C_{3-6}$ cycloalkyl-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

when A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-10}$ alkyl, $C_{3-10}$ cycloalkyl, $C_{3-10}$ cycloalkyl-$C_{1-10}$ alkyl, $C_{1-10}$ alkyloxy, $C_{3-10}$ cycloalkyloxy, $C_{1-10}$ alkyloxy-$C_{1-10}$ alkyl, 3- to 10-membered heterocyclyl, 3- to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when

A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-6}$ alkyl, $C_{3-6}$ cycloalkyl, $C_{3-6}$ cycloalkyl-$C_{1-6}$ alkyl, $C_{1-6}$ alkyloxy, $C_{3-6}$ cycloalkyloxy, $C_{1-6}$ alkyloxy-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-$C(=O)_nH$-;

or, $R^A$ and $R^1$, together with the atom to which they are attached, form a 3- to 8-membered ring structure which is unsubstituted or substituted with one, two or more $R^D$, for example, the 3-to 8-membered ring structure is 3-, 4-, 5-, 6-, 7- or 8-membered ring structure;

z is selected from 0 or 1;

$R^2$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^2$ is selected from the group consisting of $^1H$, $^2H$, hydrogen, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy;

$R^3$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^3$ is selected from the group consisting of $^1H$, $^2H$, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-10}$ alkyl, $C_{3-6}$ cycloalkyloxy;

or, $R^1$ and $R^2$, together with the atoms to which they are attached, form a 5- to 10-membered ring structure which is unsubstituted or substituted with two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a 4- to 10-membered ring structure which is unsubstituted or substituted with one, two or more $R^F$, the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of $C_{1-10}$ alkyl, $C_{1-10}$ alkyloxy, halogen, hydroxyl, sulfhydryl, amino, cyano; preferably, $R^4$ is selected from the group consisting of $C_{1-6}$ alkyl, $C_{1-6}$ alkyloxy, halogen, hydroxyl, sulfhydryl, amino and cyano;

$R^5$ is selected from hydroxyl;

$R^6$ is selected from the following groups which are unsubstituted or substituted with one, two or more $R^G$: $C_{3-10}$ cycloalkyl, $C_{3-10}$ cycloalkyl-$C_{1-10}$ alkyl, $C_{3-10}$ cycloalkyloxy, 3- to 10-membered heterocyclyl, 3- to 10-membered heterocyclyl-$C_{1-10}$ alkyl, 3- to 10-membered heterocyclyloxy; preferably, $R^6$ is selected from the following groups which are unsubstituted or substituted with one, two or more $R^G$: $C_{3-6}$ cycloalkyl, $C_{3-6}$ cycloalkyl-$C_{1-6}$ alkyl, $C_{3-6}$ cycloalkyloxy, 3- to 6-membered heterocyclyl, 3- to 6-membered heterocyclyl-$C_{1-6}$ alkyl, 3- to 6-membered heterocyclyloxy;

B is absent or selected from the group consisting of triazolyl, - $NR^7$- or -$N(NR^7R^8)$-;

$R^7$ and $R^8$ are the same or different and are independently selected from the group consisting of $^1H$, $^2H$, and the following groups which are unsubstituted or substituted with one, two or more $R^H$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl, HC(=O)-, $C_{1-10}$ alkyl-C(=O)-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, $R^7$ and $R^8$ are the same or different and are independently selected from the group consisting of hydrogen, and the following groups which are unsubstituted or substituted with one, two or more $R^H$: $C_{1-6}$

alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl, HC(=O)-, $C_{1-6}$ alkyl-C(=O)-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

$T^L$ is selected from the group consisting of chemical bond, #O-$(CR^9R^{10})_m$-$(CR^{11}R^{12})_n$-C(=O)*, #O-$(CR^9R^{10})_m$-$(CR^{11}R^{12})_n$*, #N($R^{13}$)-$(CR^9R^{10})^m$-$(CR^{11}R^{12})_n$-C(O)*, #S-$(CR^9R^{10})_m$-$(CR^{11}R^{12})_n$-C(O)*, #O-$(CR^9R^{10})_m$-$(CR^{11}R^{12})_n$-C($NR^{13}$)*, #O-$(CR^9R^{10})_m$-$(CR^{11}R^{12})_n$-P(=O)($OR^{14}$)*, *O-$(CR^9R^{10})_m$-$(CR^{11}R^{12})_n$-C(=O)#, *O-$(CR^9R^{10})_m$-$(CR^{11}R^{12})_n$#, *N($R^{13}$)-$(CR^9R^{10})_m$-$(CR^{11}R^{12})_n$-C(O)#, *S-$(CR^9R^{10})_m$-$(CR^{11}R^{12})_n$-C(O)#, *O-$(CR^9R^{10})_m$-$(CR^{11}R^{12})_n$-C($NR^{13}$)#, *O-$(CR^9R^{10})_m$-$(CR^{11}R^{12})_n$-P(=O)($OR^{14}$)#, wherein # represents the site attached to L, and * represents the site attached to B;

each of $R^9$, $R^{10}$, $R^{11}$ and $R^{12}$ is the same or different, and is independently selected from the group consisting of $^1H$, $^2H$, halogen, cyano, and the following groups which are unsubstituted or substituted with one, two or more $R^1$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy; preferably, each of $R^9$, $R^{10}$, $R^{11}$ and $R^{12}$ is the same or different and is independently selected from the group consisting of hydrogen, and the following groups which are unsubstituted or substituted with one, two or more $R^I$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-10}$ alkyl, 3- to 6-membered heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; wherein any of the 3- to 10-membered ring structures can be selected from, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and are independently selected from the groups which are unsubstituted or substituted with one, two or more $R^J$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl; preferably, each $R^{13}$ is the same or different and are independently selected from the following groups which are unsubstituted or substituted with one, two or more $R^J$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3-to 6-membered heterocyclyl-$C_{1-6}$ alkyl;

m and n are the same or different and are independently selected from an integer of 0 to 10, such as 0, 1, 2, 3, 4, 5, 6, 7, 8, 9 or 10;

each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each $R^K$ is the same or different and is independently selected from the group consisting of deuterium, halogen, hydroxyl, cyano, nitro, and the following groups which are unsubstituted or substituted with one, two or more $R^L$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^L$ is the same or different and is independently selected from the group consisting of deuterium, halogen, hydroxyl, cyano, nitro, $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-.

According to the embodiments of the present invention, definitions of the groups in Formula (G) may be independently selected from:

A is selected from N or C-$R^A$;

when A is N, $R^1$ is selected from the group consisting of $^1H$, $^2H$, hydroxyl, deuterated hydroxyl, and the following groups which are unsubstituted or substituted with one, two or more $R^B$: $C_{1-10}$ alkyl, $C_{3-10}$ cycloalkyl, $C_{3-10}$ cycloalkyl-$C_{1-10}$ alkyl, 3- to 10-membered heterocyclyl, 3-to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is N, $R^1$ is selected from the group consisting of $^1H$, $^2H$, hydroxyl, deuterated hydroxyl, and the following groups which are unsubstituted or substituted with one, two or more $R^B$: $C_{1-6}$ alkyl, $C_{3-6}$ cycloalkyl, $C_{3-6}$ cycloalkyl-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

when A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-10}$ alkyl, $C_{3-10}$ cycloalkyl, $C_{3-10}$ cycloalkyl-$C_{1-10}$ alkyl, $C_{1-10}$ alkyloxy, $C_{3-10}$ cycloalkyloxy, $C_{1-10}$ alkyloxy-$C_{1-10}$ alkyl, 3- to 10-membered heterocyclyl, 3- to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-6}$ alkyl, $C_{3-6}$ cycloalkyl, $C_{3-6}$ cycloalkyl-$C_{1-6}$ alkyl, $C_{1-6}$ alkyloxy, $C_{3-6}$ cycloalkyloxy, $C_{1-6}$ alkyloxy-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

or, $R^A$ and $R^1$, together with the atom to which they are attached, form a 3- to 8-membered ring structure which is unsubstituted or substituted with one, two or more $R^D$, for example, the 3-to 8-membered ring structure is a 3-, 4-, 5-, 6-, 7- or 8-membered ring structure;

z is selected from 0 or 1;

$R^2$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^2$ is selected from the group consisting of $^1H$, $^2H$, hydrogen, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy;

$R^3$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^3$ is selected from the group consisting of $^1H$, $^2H$, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-10}$ alkyl, $C_{3-6}$ cycloalkyloxy;

provided that, $R^1$, $R^2$ and the atoms to which they are attached, or $R^2$, $R^3$ and the atoms to which they are attached, at least one of these two sets of groups together form a 4- to 10-membered ring structure which is unsubstituted or substituted with one, two or more $R^E$, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of $C_{1-10}$ alkyl, $C_{1-10}$ alkyloxy, halogen, hydroxyl, sulfhydryl, amino, cyano; preferably, $R^4$ is selected from the group consisting of $C_{1-6}$ alkyl, $C_{1-6}$ alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: $C_{1-10}$ alkyl, $C_{1-10}$ alkyloxy, $C_{6-10}$ aryl, $C_{6-10}$ arylalkyl, $C_{6-10}$ aryloxy, 5- to 6-membered heteroaryl, 5- to 6-membered heteroarylalkyl, 5- to 6-membered heteroaryloxy;

B is absent or selected from the group consisting of triazolyl, $-NR^7-$ or $-N(NR^7R^8)-$;

$R^7$ and $R^8$ are the same or different and are independently selected from the group consisting of $^1H$, $^2H$, and the following groups which are unsubstituted or substituted with one, two or more $R^H$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl, HC(=O)-, $C_{1-10}$ alkyl-C(=O)-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, $R^7$ and $R^8$ are the same or different and are independently selected from the group consisting of hydrogen, and the following groups which are unsubstituted or substituted with one, two or more $R^H$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl, HC(=O)-, $C_{1-6}$ alkyl-C(=O)-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

$T^L$ is selected from the group consisting of chemical bond, $\#O-(CR^9R^{10})_m-(CR^{11}R^{12})_n-C(=O)*$, $\#O-(CR^9R^{10})_m-(CR^{11}R^{12})_n*$, $\#N(R^{13})-(CR^9R^{10})^m-(CR^{11}R^{12})_n-C(O)*$, $\#S-(CR^9R^{10})_m-(CR^{11}R^{12})_n-C(O)*$, $\#O-(CR^9R^{10})_m-(CR^{11}R^{12})_n-C(NR^{13})*$, $\#O-(CR^9R^{10})_m-(CR^{11}R^{12})_n-P(=O)(OR^{14})*$, $*O-(CR^9R^{10})_m-(CR^{11}R^{12})_n-C(=O)\#$, $*O-(CR^9R^{10})_m-(CR^{11}R^{12})_n\#$, $*N(R^{13})-(CR^9R^{10})_m-(CR^{11}R^{12})_n-C(O)\#$, $*S-(CR^9R^{10})_m-(CR^{11}R^{12})_n-C(O)\#$, $*O-(CR^9R^{10})_m-(CR^{11}R^{12})_n-C(NR^{13})\#$, $*O-(CR^9R^{10})_m-(CR^{11}R^{12})_n-P(=O)(OR^{14})\#$, wherein # represents the site attached to L, and * represents the site attached to B;

each of $R^9$, $R^{10}$, $R^{11}$ and $R^{12}$ is the same or different, and is independently selected from the group consisting of $^1H$, $^2H$, halogen, cyano, and the following groups which are unsubstituted or substituted with one, two or more $R^1$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy; preferably, each of $R^9$, $R^{10}$, $R^{11}$ and $R^{12}$ is the same or different and is independently selected from the group consisting of hydrogen, halogen, cyano, and the following groups which are unsubstituted or substituted with one, two

or more $R^I$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-10}$ alkyl, 3- to 6-membered heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; wherein, any of the 3- to 10-membered ring structures can be selected from, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl; preferably, each $R^{13}$ is the same or different and is independently selected from the following groups which are unsubstituted or substituted with one, two or more $R^J$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl;

m and n are the same or different and are independently selected from an integer of 0 to 10, such as 0, 1, 2, 3, 4, 5, 6, 7, 8, 9 or 10;

each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, amino, nitro, the following groups which are unsubstituted or substituted with one, two or more $R^K$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each of $R^B$, $R^C$, $R^D$, $R^E$, $R^F$, $R^G$, $R^H$, $R^I$ and $R^J$ is the same or different, and is independently selected from the group consisting of deuterium, halogen, hydroxyl, cyano, nitro, and the following groups which are unsubstituted or substituted with one, two or more $R^K$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each $R^K$ is the same or different, and is independently selected from the group consisting of deuterium, halogen, hydroxyl, cyano, nitro, and the following groups which are unsubstituted or substituted with one, two or more $R^L$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^L$ is the same or different and is independently selected from the group consisting of deuterium, halogen, hydroxyl, cyano, nitro, $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$

alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-.

According to the embodiments of the present invention, definitions of the groups in Formula (G) may be independently selected from:

A is selected from N;

$R^1$ is selected from the group consisting of $^1H$, $^2H$, hydroxyl, deuterated hydroxyl, and the following groups which are unsubstituted or substituted with one, two or more $R^B$: $C_{1-10}$ alkyl, $C_{3-10}$ cycloalkyl, $C_{3-10}$ cycloalkyl-$C_{1-10}$ alkyl, 3- to 10-membered heterocyclyl, 3- to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is N, $R^1$ is selected from the group consisting of $^1H$, $^2H$, hydroxyl, deuterated hydroxyl, and the following groups which are unsubstituted or substituted with one, two or more $R^B$: $C_{1-6}$ alkyl, $C_{3-6}$ cycloalkyl, $C_{3-6}$ cycloalkyl-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

z is selected from 0 or 1;

$R^2$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^2$ is selected from the group consisting of $^1H$, $^2H$, hydrogen, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy;

$R^3$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^3$ is selected from the group consisting of $^1H$, $^2H$, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-10}$ alkyl, $C_{3-6}$ cycloalkyloxy;

or, $R^1$ and $R^2$, together with the atoms to which they are attached, form a 5- to 10-membered ring structure which is unsubstituted or substituted with one, two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4, 5-, 6-, 7-, 8-, 9- or 10-membered monocyclic hydrocarbyl, bicyclic hydrocarbyl, heteromonocyclic hydrocarbyl, heterobicyclic hydrocarbonyl;

$R^4$ is selected from the group consisting of $C_{1-10}$ alkyl, $C_{1-10}$ alkyloxy, halogen, hydroxyl, sulfhydryl, amino, cyano; preferably, $R^4$ is selected from the group consisting of $C_{1-6}$ alkyl, $C_{1-6}$ alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: $C_{3-10}$ cycloalkyl, $C_{3-10}$ cycloalkyl-$C_{1-10}$ alkyl, $C_{3-10}$ cycloalkyloxy, 3- to 10-membered heterocyclyl, 3- to 10-membered heterocyclyl-$C_{1-10}$ alkyl, 3- to 10-membered heterocyclyloxy; preferably, $R^6$ is selected from the following groups which are unsubstituted or substituted with one, two or more $R^G$: $C_{3-6}$ cycloalkyl, $C_{3-6}$ cycloalkyl-$C_{1-6}$ alkyl, $C_{3-6}$ cycloalkyloxy, 3- to 6-membered heterocyclyl, 3- to 6-membered heterocyclyl-$C_{1-6}$ alkyl, 3- to 6-membered heterocyclyloxy;

B is selected from $-NR^7-$ or $-N(NR^7R^8)-$;

$R^7$ and $R^8$ are the same or different and are independently selected from the group consisting of $^1H$, $^2H$, and the following groups which are unsubstituted or substituted with one, two or more $R^H$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl, HC(=O)-, $C_{1-10}$ alkyl-C(=O)-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, $R^7$ and $R^8$ are the same or different and are independently selected from the group consisting of hydrogen, and the following groups which are unsubstituted or substituted with one, two or more $R^H$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl, HC(=O)-, $C_{1-6}$ alkyl-C(=O)-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

$T^L$ is selected from the group consisting of chemical bond, $\#O-(CR^9R^{10})_m-(CR^{11}R^{12})_n-C(=O)*$, $\#O-(CR^9R^{10})_m-(CR^{11}R^{12})_n-*$, $\#N(R^{13})-(CR^9R^{10})_m-(CR^{11}R^{12})_n-C(O)*$, $\#S-(CR^9R^{10})_m-(CR^{11}R^{12})_n-C(O)*$, $\#O-(CR^9R^{10})_m-(CR^{11}R^{12})_n-C(NR^{13})*$, $\#O-(CR^9R^{10})_m-(CR^{11}R^{12})_n-P(=O)(OR^{14})*$, $*O-(CR^9R^{10})_m-(CR^{11}R^{12})_n-C(=O)\#$, $*O-(CR^9R^{10})_m-(CR^{11}R^{12})_n\#$, $*N(R^{13})-(CR^9R^{10})_m-(CR^{11}R^{12})_n-C(O)\#$, $*S-(CR^9R^{10})_m-(CR^{11}R^{12})_n-C(O)\#$, $*O-(CR^9R^{10})_m-(CR^{11}R^{12})_n-C(NR^{13})\#$, $*O-(CR^9R^{10})_m-(CR^{11}R^{12})_n-P(=O)(OR^{14})\#$, wherein # represents the site attached to L, and * represents the site attached to B;

each of $R^9$, $R^{10}$, $R^{11}$ and $R^{12}$ is the same or different and is independently selected from the group consisting of $^1H$, $^2H$, halogen, cyano, and the following groups which are unsubstituted or substituted with one, two or more $R^1$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy; preferably, each of $R^9$, $R^{10}$, $R^{11}$ and $R^{12}$ is the same or different and is independently selected from the group consisting of hydrogen, halogen, cyano, and the following groups which are unsubstituted or substituted with one, two or more $R^I$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-10}$ alkyl, 3- to 6-membered heterocyclyloxy;

or, $R^9$ and $R^{10}$, together with the atoms to which they are attached, form a 3- to 10-membered ring structure which is unsubstituted or substituted with one, two or more $R^1$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^1$; any of the 3- to 10-membered ring structures can be selected from, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl; preferably, each $R^{13}$ is the same or different and is independently selected from the following groups which are unsubstituted or substituted with one, two or more $R^J$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl;

m and n are the same or different and are independently selected from an integer of 0 to 10, such as 0, 1, 2, 3, 4, 5, 6, 7, 8, 9 or 10;

each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano and nitro, and the following groups which are unsubstituted or substituted with one, two or more $R^K$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered

heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each $R^K$ is the same or different and is independently selected from the group consisting of deuterium, halogen, hydroxyl, cyano, nitro, and the following groups which are unsubstituted or substituted with one, two or more $R^L$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^L$ is the same or different and is independently selected from the group consisting of deuterium, halogen, hydroxyl, cyano, nitro, $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-.

According to the embodiments of the present disclosure, $R^1$ and $R^2$, and the quinolyl to which they are connected form one of the following substructures, wherein the substructures may be unsubstituted or substituted with one, two or more $R^E$:

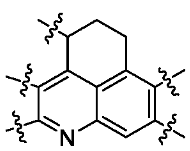 , 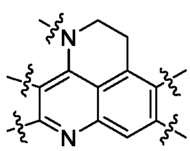 , 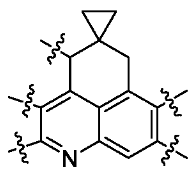 , 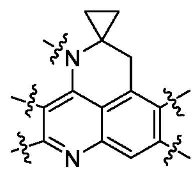 , 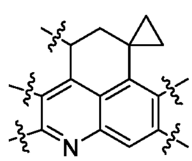 ,

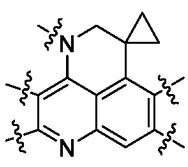 , 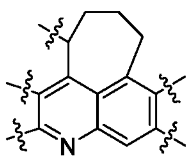 , 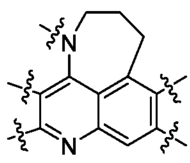 , 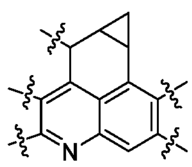 , 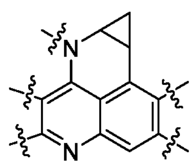 .

or, according to the embodiments of the present disclosure, $R^2$ and $R^3$, together with the quinolyl to which they are connected, form one of the following substructures, wherein the substructures may be unsubstituted or substituted with one, two or more $R^F$:

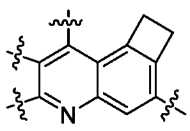, 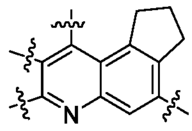, 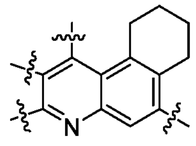.

According to the embodiments of the present disclosure, B is absent or selected from the group consisting of

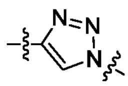, 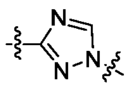,

-NH- or -N($NR^7R^8$)-, wherein $R^7$ and $R^8$ independently have the definitions as described above.

According to the embodiments of the present disclosure, G is selected from the group represented by the following Formula (G-1):

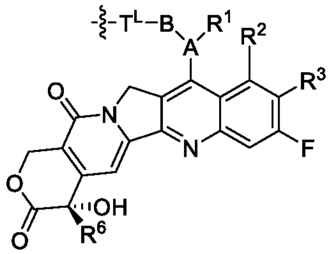

(G-1)

wherein, A, B, $T^L$, $R^1$, $R^2$, $R^3$, and $R^6$ have the definitions as described above.

According to the embodiments of the present disclosures, G is selected from the group represented by Formula (G-2) or (G-3):

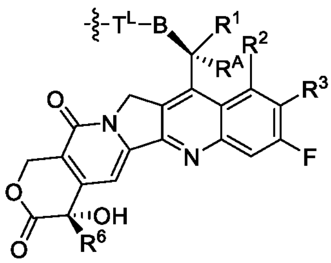

(G-2)

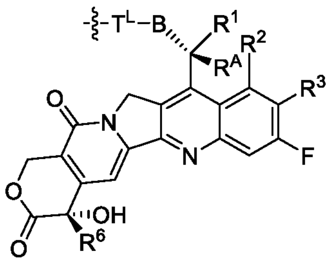

(G-3)

wherein, $R^A$, B, $T^L$, $R^1$, $R^2$, $R^3$, and $R^6$ have the definitions as described above.

According to the embodiments of the present disclosures, G is selected from the following group or the group formed by connecting -$T^L$- with the following group, at the wavy line of the following group:

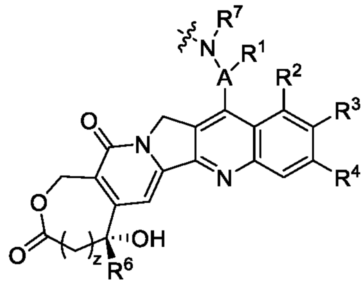

wherein, A, $R^1$, $R^2$, $R^3$, $R^4$, $R^6$, $R^7$, and z have the definitions as described above.

According to the embodiments of the present disclosures, G is selected from the following group or the group formed by connecting -$T^L$- with the following group, at the wavy line of the following group:

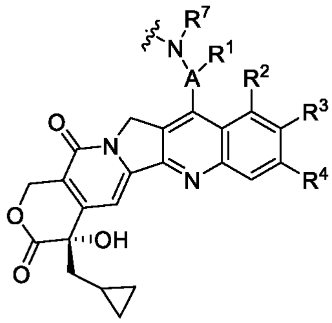

wherein, A, $R^1$, $R^2$, $R^3$, $R^4$, and $R^7$ have the definitions as described above.

According to the embodiments of the present disclosures, G is selected from the following group or the group formed by connecting -$T^L$- with the following group, at the wavy line of the following group:

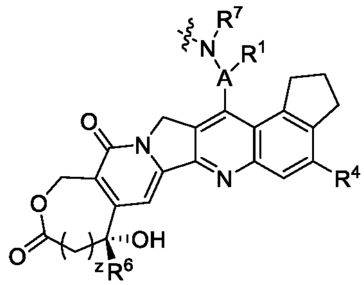

wherein, A, $R^1$, $R^4$, $R^6$, $R^7$, and z have the definitions as described above.

According to the exemplary embodiments of the present disclosures, G is selected from the following groups or the groups formed by connecting -$T^L$- with the following group, at the wavy line of the following groups:

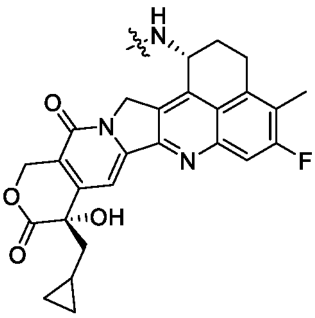

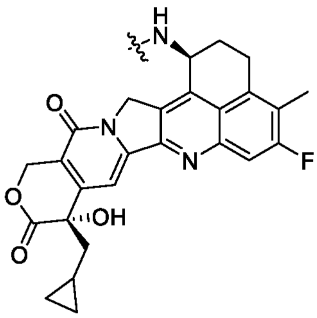

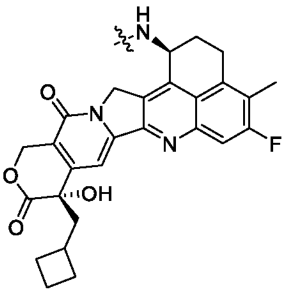

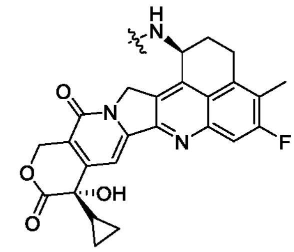

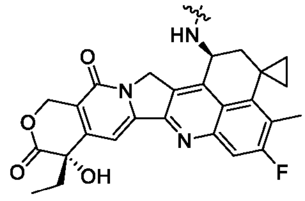

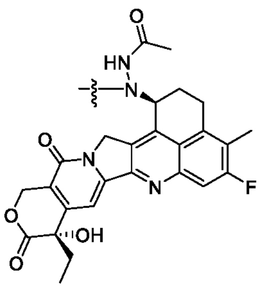

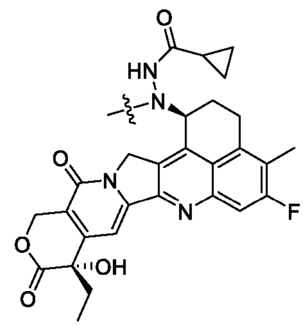

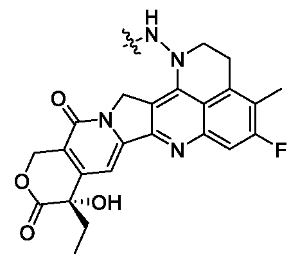

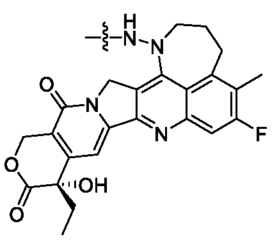

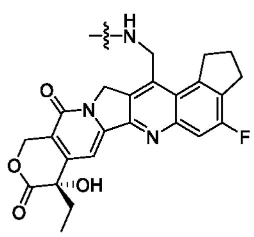

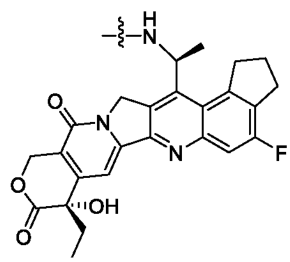

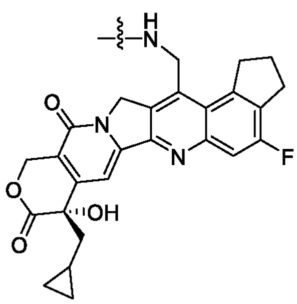

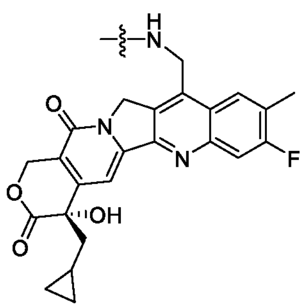

.

In the above aspect of the present invention, L is selected from a chemical bond or a linker represented by the Formula (L) as defined below:

$$\#L^1\text{-}L^2\text{-}L^3\text{-}L^4\text{-}L^5{*} \qquad (L)$$

wherein, $L^1$ is a linking moiety to the targeting moiety Tp, formed from a reactive group $L^{1'}$ through its coupling with the targeting moiety Tp, and # represents the site attached to the targeting moiety Tp;

for example, $L^{1'}$ is a maleimide group, then $L^1$ is the following structure:

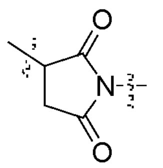

or its ring-opening form:

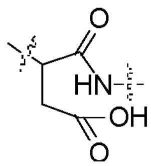 or 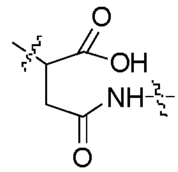 ;

$L^2$ is absent or a spacer between $L^1$ and $L^3$;

$L^3$ is a peptide moiety;

$L^4$ is absent or a spacer between the peptide moiety and $L^5$;

$L^5$ is a linking moiety between $L^4$ and a bioactive molecule G, formed from a reactive group $L^{5'}$ through its reaction with the bioactive molecule G or intermediates thereof, and * represents the site attached to the bioactive molecule G;

optionally:

the following hydrophilic moiety or steric hindrance moiety is inserted between the above moieties of L, preferably between $L^1$ and $L^2$, or between $L^2$ and $L^3$, or is inserted by replacing $L^2$:

hydrophilic moiety:

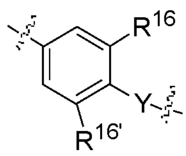 or 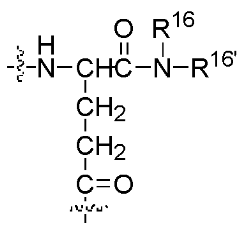 or 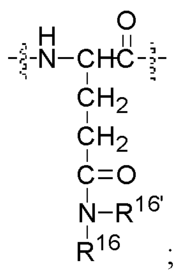 ;

steric hindrance moiety:

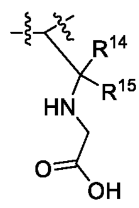 ;

$R^{16}$ and $R^{16'}$ are the same or different, and at least one of them is selected from a hydrophilic group, and the other is selected from the following substituents: hydrogen, halogen, cyano, amino, nitro, and the following groups which are unsubstituted or substituted with one, two or more $R^{zg}$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, aminocarbonyl;

the hydrophilic group is selected from a polyethylene glycol group, a $C_{1-10}$ alkyl substituted with 1 to 10 hydroxyl groups, or a sugar ring-containing group, or $C_{1-6}$ alkyl-NHCO- (e.g., $C_{1-4}$ alkyl-NHCO-), preferably a polyethylene

glycol group, more preferably -C(=O)-NH-$(CH_2CH_2O)_p$-$C_{1-10}$ alkyl or -NH-$(CH_2CH_2O)_p$-$C_{1-10}$ alkyl, or preferably a $C_{1-10}$ alkyl substituted with 1 to 10 hydroxyl groups, more preferably

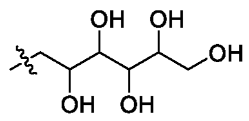

;

each p is the same or different, and is independently selected from an integer of 0 to 10, preferably 1, 2, 3, 4, 5, 6, 7 or 8;

Y is selected from the group consisting of O, S, $C_{1-10}$ alkylene, wherein 1, 2 or 3 of the methylene groups of the alkylene can be optionally replaced by O or S;

$R^{14}$ and $R^{15}$ are the same or different and are independently selected from the group consisting of hydrogen, halogen, cyano, amino, nitro, and the following groups which are unsubstituted or substituted with one, two or more $R^{zh}$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy;

or, $R^{14}$ and $R^{15}$, together with the atom to which they are attached, form a $C_{3-10}$ cycloalkyl which is unsubstituted or substituted with one, two or more $R^{zh}$;

each of $R^{zg}$ and $R^{zh}$ is the same or different and is independently selected from the group consisting of halogen, hydroxyl, amino, cyano, nitro, $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy;

in some embodiments of the present invention, the hydrophilic moiety or steric hindrance moiety in L as defined above is absent.

In some further preferred embodiments of the present invention, $L^1$ is formed from any of $L^{1'}$ through its reaction with the targeting moiety Tp, $L^{1'}$ is a sulfhydryl-reactive group, an amine-reactive group, a carboxyl-reactive group, a proline residue-reactive group, a tyrosine residue-reactive group, a disulfide bridge-containing group, etc.; for an antibody introduced with a non-natural amino acid, it can also be selected from reactive groups in click chemistry such as ketone, azide, alkyne, cyclopropene or diene;

$L^{1'}$ is preferably a sulfhydryl-reactive group;
$L^{1'}$ is further preferably a maleimide group or a substituted maleimide group, and $L^1$-$L^2$ preferably has the following structure:

a fragment that is prepared from (N-maleimidomethyl)-carboxylic acid-N-hydroxysuccinimide ester, and has the structure as follows:

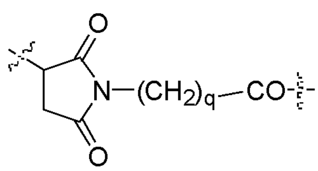

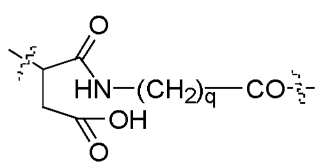

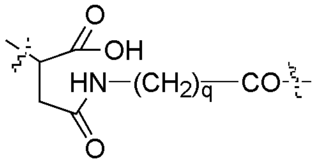

(q is an integer of 0 to 10, preferably 1, 2, 3, 4, 5, 6, 7 or 8);

or a fragment that is prepared from (meta)-maleimidobenzoic acid N-hydroxysuccinimide ester (MBS), and has the structure as follows:

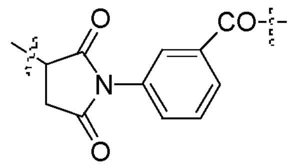

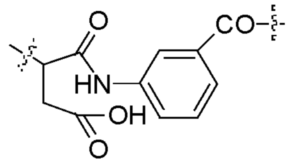

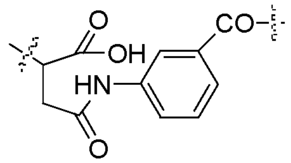

or a fragment that is prepared from 4-(N-maleimidomethyl)-cyclohexane-1-carboxylic acid N-hydoxysuccinimide ester (SMCC), and has the structure as follows:

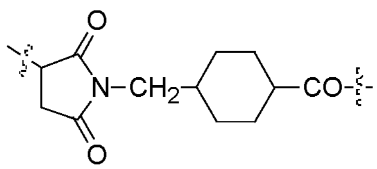

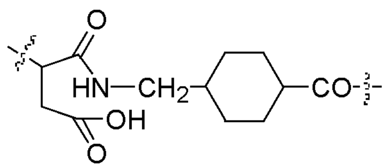

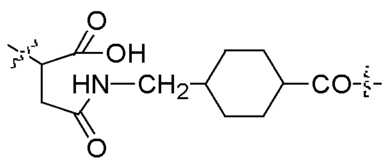

or $L^{1'}$ is preferably a sulfhydryl-reactive group having the following structure:

Hal-Het-

Hal is selected from the group consisting of halogen, OMs, OTs, OTf, nitro, and the following groups which are optionally substituted with one or more $R^{z6}$: alkyl thioether group, aryl thioether group, heteroaryl thioether group, alkyl sulfoxide group, aryl sulfoxide group, heteroaryl sulfoxide group, alkyl sulfonyl group, aryl sulfonyl group, heteroaryl sulfonyl group; wherein $R^{z6}$ is independently selected from the group consisting of H (hydrogen), D (deuterium), halogen, CN, nitro, $C_{1-6}$ alkyl, halogenated $C_{1-6}$ alkyl, $C_{1-6}$ alkoxy, 6- to 10-membered aryl and 5- to 12-membered heteroaryl;

Het is selected from 5- to 10-membered heteroaryl which is optionally substituted with one or more $R^{z7}$; wherein $R^{z7}$ is independently selected from the group consisting of H (hydrogen), D (deuterium), halogen, CN, nitro, $C_{1-4}$ alkyl and halogenated $C_{1-4}$ alkyl;

in a preferred embodiment, Hal is preferably mesyl, and Het is preferably pyrimidinyl;

in a preferred embodiment, Hal-Het- is:

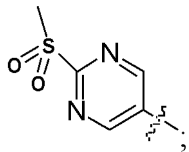
;

the corresponding $L^1$ has the structure as follows:

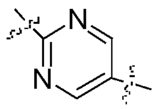
.

and $L^{1'}$-$L^2$ preferably has the structure as follows:

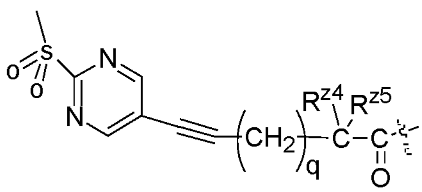

q is an integer selected from 0 to 10, preferably 1, 2, 3, 4, 5, 6, 7 or 8;

$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl.

further preferably has the structure as follows:

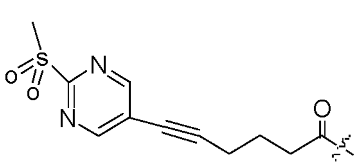
or
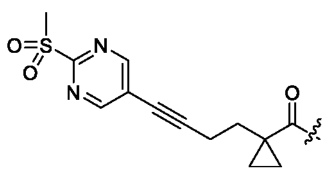
.

In a further preferred embodiment of the present invention,

$L^2$ is absent or selected from the group consisting of $C_{1-10}$ alkylene, $C_{2-10}$ alkenylene, $C_{2-10}$ alkynylene, $C_{3-10}$ cycloalkyl, $C_{6-12}$ aryl or 6- to 12-membered heteroaryl or a combination of the above fragments, and is optionally interrupted by a carbonyl group, O, S, or N, and optionally substituted with $C_{1-6}$ alkyl, $C_{3-6}$ cycloalkyl, halogen, halogenated $C_{1-6}$ alkyl, and optionally, the alkyl or halogenated alkyl, together with the C atom to which it is connected, forms a $C_{3-6}$ cycloalkyl, $L^2$ is connected to $L^1$ or $L^3$ through any functional group or covalent bond;

preferably, $L^2$ is connected to the N-terminal of the peptide moiety of $L^3$ through $-C(R^{z4}R^{z5})$. CO-.

In a further preferred embodiment of the present invention, $R^{z4}$ and $R^{z5}$ are the same or different, and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl.

In a further preferred embodiment of the present invention, $L^2$ is selected from the group consisting of $-(CH_2)_q-$, $-(CH_2)_q-C(=O)-$, or $-(C\equiv C)-(CH_2)_q-C(R^{z4}R^{z5})-C(=O)-$, wherein q is an integer of 0 to 10, preferably 1, 2, 3, 4, 5, 6, 7 or 8; in a further preferred embodiment of the present invention, $R^{z4}$ and $R^{z5}$ are the same or different, and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl.

In a further preferred embodiment of the present invention,

$L^3$ is selected from a divalent peptide group comprising 2 to 8 optionally substituted natural amino acid residues or optionally substituted non-natural amino acid residues, each of the amino acid residues is the same or different and is independently selected from the following amino acid residues: alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), selenocysteine (Sec), pyrrolysine (Pyl), homoserine, homocysteine, demethylpyrrolysine.

Further preferably, $L^3$ is selected from a divalent peptide group composed of 2, 3, 4, 5 or 6 optionally substituted natural amino acid residues or optionally substituted non-natural amino acid residues, each of the amino acid residues is the same or different and is independently selected from the following amino acid residues: alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), selenocysteine (Sec), pyrrolysine (Pyl), homoserine, homocysteine, demethylpyrrolysine; for example, -ValCit-; -CitVal-; -AlaAla-; -AlaCit-; -CitAla-; -AsnCit-; -CitAsn-; -CitCit-; -ValGlu-; -GluVal-; -SerCit-; -CitSer-; -LysCit-; -CitLys-; -AspCit-; -CitAsp-; -AlaVal-; -ValAla-; -PheAla-; -AlaPhe-; -PheLys-; -LysPhe-; -ValLys-; -LysVal-; - AlaLys-; -LysAla-; -PheCit-; -CitPhe-; -LeuCit-; -CitLeu-; -IleCit-; -CitIle-; -PheArg-; -ArgPhe-; -CitTrp-; -TrpCit-; -PhePheLys-; -LysPhePhe-; -DPhePheLys-; -DLysPhePhe-; -GlyPheLys-; - LysPheGly-; -GlyPheLeuGly-; -GlyLeuPheGly-; -AlaLeuAlaLeu-; -GlyGlyGly-; - GlyGlyGlyGly-; -GlyPheValGly-; -GlyValPheGly-; -GlyGlyPheGly-; -AlaAlaAla-; most preferably, $L^3$ is -GlyGlyPheGly-.

Most preferably, $L^3$ is -GlyGlyPheGly-.

In a further preferred embodiment of the present invention,

$L^4$ is preferably a group with self-cleaving properties, and a self-cleaving group or self-immolative group is a group that initiates drug release through an intramolecular reaction such as 1,4-elimination, 1,6-elimination or cyclization elimination independent of the action of an enzyme.

$L^5$ is formed from any reactive group $L^{5'}$ through its reaction with a bioactive molecule or intermediate thereof, wherein $L^{5'}$ is preferably a carboxylic acid group or an active ester group, which reacts with OH, SH or NH or $NH_2$ in the bioactive molecule to form $L^5$ with the structure of: -C(O)O-, -C(O)S-, -C(O)N- or -C(O)NH- (including the atom of O, S or N in the bioactive molecule);

$L^4$-$L^5$ is preferably:

a PABC spacer arm with the structure as follows:

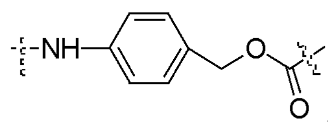

;

a GABA spacer arm with the structure as follows:

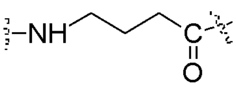

;

an α,α-dimethyl GABA spacer arm with the structure as follows:

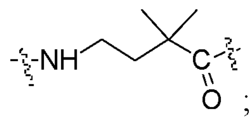

or a β,β-dimethyl GABA spacer arm with the structure as follows:

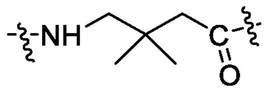

Most preferably, $L^4$-$L^5$ is: -$NR^{z1}$-$C(R^{z2}R^{z3})$-$T^L$-;
wherein:

$R^{z1}$ is H or $C_{1-4}$ alkyl;
$R^{z2}$ and $R^{z3}$ are the same or different and are independently selected from H or $C_{1-4}$ alkyl;
$T^L$ has the definition as described above, and one of $T^L$ in $L^4$-$L^5$ or G is a chemical bond.

In the most preferred aspect of the present invention,

$L^1$ is generated by coupling Tp with a sulfhydryl-reactive group contained in maleimide group, a substituted maleimide group or Hal-Het-;
the following hydrophilic moiety is inserted between $L^1$ and $L^2$, or between $L^2$ and $L^3$, or is inserted by replacing $L^2$:
hydrophilic moiety:

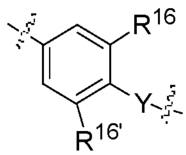 or 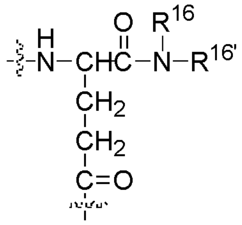 or 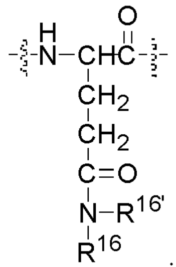

In the most preferred aspect of the present invention:

$L^1$ is generated by coupling Tp with a sulfhydryl-reactive group contained in maleimide group or a substituted maleimide group;
the following steric hindrance moiety is inserted between $L^1$ and $L^2$, or between $L^2$ and $L^3$, or is inserted by replacing $L^2$:
steric hindrance moiety:

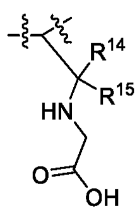

In the most preferred aspect of the present invention:

$L^1$ is generated by coupling Tp with a sulfhydryl-reactive group contained in Hal-Het-;
$L^4$-$L^5$ is: -$NR^{z1}$-$C(R^{z2}R^{z3})$-$T^L$-;
wherein:

$R^{z1}$ is H or $C_{1-4}$ alkyl;

$R^{z2}$ and $R^{z3}$ are the same or different and are independently selected from H or $C_{1-4}$ alkyl;

$T^L$ has the definition as described above, and one of $T^L$ in $L^4$-$L^5$ or G is a chemical bond.

It is further preferred that:

when $T^L$ in G is a chemical bond, $L^4$-$L^5$ is: $-NR^{z1}-C(R^{z2}R^{z3})-O-(CH_2)_m-C(R^{z4}R^{z5})-CO-$;

wherein:

$R^{z1}$ is H or $C_{1-4}$ alkyl;

m is an integer of 0 to 4;

$R^{z2}$ and $R^{z3}$ are the same or different and are independently selected from H or $C_{1-4}$ alkyl;

$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl.

The present invention further provides a conjugate of the following structure:

Tp-L-D (D)

wherein Tp is the targeting moiety as defined above;

D is a bioactive molecular fragment, preferably a molecular fragment with anti-tumor biological activity;

wherein, L is selected from the linker represented by Formula (L):

$\#L^1-L^2-L^3-L^4-L^5*$ (L)

wherein, $L^1$ is a linking moiety to the targeting moiety Tp, and formed from a reactive group $L^{1'}$ through its reaction with the targeting moiety Tp, and # represents the site connected to the Tp moiety;

$L^2$ is absent or a spacer between $L^1$ and $L^3$;

$L^3$ is a peptide moiety;

$L^4$ is absent or a spacer between the peptide moiety and $L^5$;

$L^5$ is a linking moiety connecting $L^4$ with the bioactive molecule D, and formed from a reactive group $L^{5'}$ through its reaction with the bioactive molecule or an intermediate thereof, and * represents the site connected to the bioactive molecule D;

provided that:

Condition I:
the following hydrophilic moiety is inserted between the fragments of L as defined above:
hydrophilic moiety:

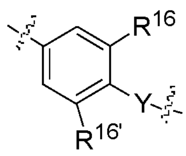 or 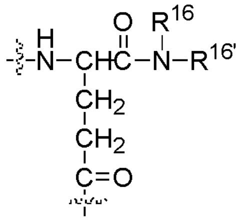 or 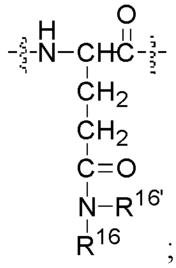 ;

or Condition II:

$L^1$ is generated by coupling Tp with a sulfhydryl-reactive group contained in maleimide group or a substituted maleimide group;
the following steric hindrance moiety is inserted between $L^1$ and $L^2$, or between $L^2$ and $L^3$, or inserted by replacing $L^2$:
steric hindrance moiety:

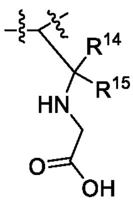 ;

or Condition III:

$L^1$ is generated by coupling Tp with a sulfhydryl-reactive group contained in Hal-Het-;
$L^4$-$L^5$ is: -$NR^{z1}$-$C(R^{z2}R^{z3})$-$T^L$-;
wherein:

$R^{z1}$ is H or $C_{1-4}$ alkyl;
$R^{z2}$ and $R^{z3}$ are the same or different, and are independently selected from H or $C_{1-4}$ alkyl;
$T^L$ has the definition as described above.

In a further embodiment,

$L^1$ is generated by coupling Tp with a sulfhydryl-reactive group contained in maleimide group or Hal-Het-;
the following hydrophilic moiety is inserted between $L^1$ and $L^2$, or between $L^2$ and $L^3$, or is inserted by replacing $L^2$:
hydrophilic moiety:

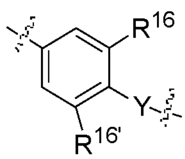 or 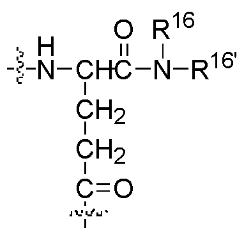 or 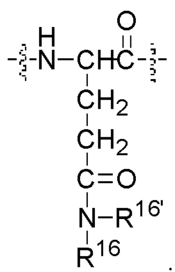 .

In a further embodiment,
in the most preferred aspect of the present invention:

$L^1$ is generated by coupling Tp with a sulfhydryl-reactive group contained in Hal-Het-;
$L^4$-$L^5$ is: -$NR^{z1}$-$C(R^{z2}R^{z3})$-O-$(CH_2)_m$-$C(R^{z4}R^{z5})$-CO-;
wherein:

$R^{z1}$ is H or $C_{1-4}$ alkyl;
m is an integer of 0 to 4;
$R^{z2}$ and $R^{z3}$ are the same or different, and are independently selected from H or $C_{1-4}$ alkyl;
$R^{z4}$ and $R^{z5}$ are the same or different, and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl.
or Condition IV:
$L^1$-$L^2$ is generated by coupling Tp with the following structure:

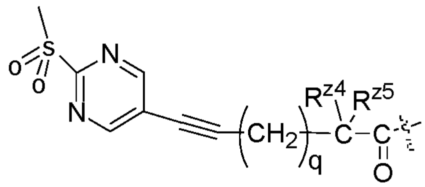

q is an integer selected from 0 to 10, preferably 1, 2, 3, 4, 5, 6, 7 or 8;
$R^{z4}$ and $R^{z5}$ are the same or different, and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl, wherein at least one of $R^{z4}$ and $R^{z5}$ is not H;
it is further preferred that $R^{z4}$ and $R^{z5}$ together form a $C_{3-6}$ cycloalkyl;
$L^1$-$L^2$ is most preferably generated by coupling Tp with

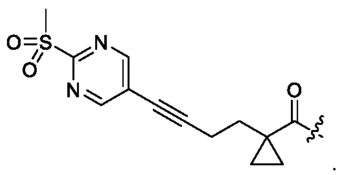

The present invention also provides the following intermediate for the synthesis of the conjugate:

$L^{1'}$-$L^2$-$L^3$-$L^4$-$L^5$-G

wherein $L^{1'}$, $L^2$, $L^3$, $L^4$, $L^5$, G are as defined above.

The present invention also provides the following intermediate for the synthesis of the conjugate:

$U^{1'}$-$L^2$-$L^3$-$L^4$-$L^5$-D

wherein $L^{1'}$, $L^2$, $L^3$, $L^4$, $L^5$, and D are as defined above, provided that:

Condition I:
the following hydrophilic moiety is inserted between the fragments of L as defined above:
hydrophilic moiety:

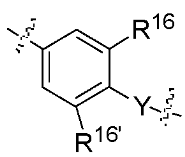 or 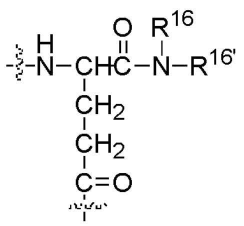 or 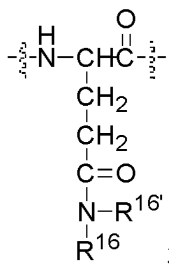 ;

or Condition II:

$L^{1'}$ is a sulfhydryl contained in maleimide group or a substituted maleimide group;
the following steric hindrance moiety is inserted between $L^1$ and $L^2$, or between $L^2$ and $L^3$, or is inserted by replacing $L^2$:

steric hindrance moiety:

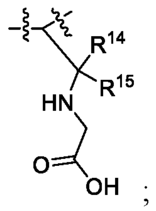

;

or Condition III:

$L^{1'}$ is selected from the sulfhydryl-reactive group of Hal-Het-;
$L^4$-$L^5$ is: -$NR^{z1}$-C($R^{z2}R^{z3}$)-$T^L$-;
wherein:

$R^{z1}$ is H or $C_{1-4}$ alkyl;
$R^{z2}$ and $R^{z3}$ are the same or different and are independently selected from H or $C_{1-4}$ alkyl;
$T^L$ is as defined above.

or Condition IV:

$L^{1'}$-$L^2$ has the following structure:

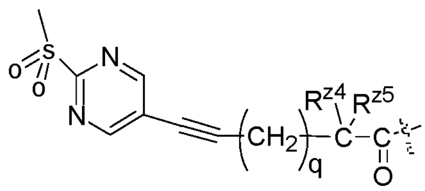

q is an integer of 0 to 10, preferably 1, 2, 3, 4, 5, 6, 7 or 8;
$R^{z4}$ and $R^{z5}$ are the same or different, and are independently selected from H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl, wherein at least one of $R^{z4}$ and $R^{z5}$ is not H; further preferably, $R^{z4}$ and $R^{z5}$ together form a $C_{3-6}$ cycloalkyl;
$L^{1'}$-$L^2$ is most preferably

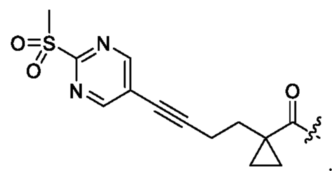

.

In a further embodiment,

$L^{1'}$ is a sulfhydryl contained in maleimide group, a substituted maleimide group or Hal-Het-;
the following hydrophilic moiety is inserted between $L^{1'}$ and $L^2$, or between $L^2$ and $L^3$, or is inserted by replacing $L^2$:
hydrophilic moiety:

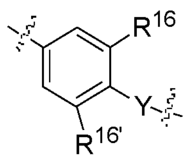

or

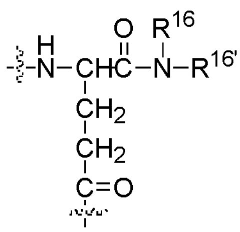

or

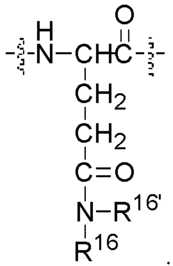

.

In a further embodiment,
in the most preferred aspect of the present invention:

$L^{1'}$ is a sulfhydryl contained in Hal-Het-;
$L^4$-$L^5$ is: $-NR^{z1}-C(R^{z2}R^{z3})-O-(CH_2)_m-C(R^{z4}R^{z5})-CO-$;
wherein:

$R^{z1}$ is H or $C_{1-4}$ alkyl;

m is an integer of 0 to 4;

$R^{z2}$ and $R^{z3}$ are the same or different and are independently selected from H or $C_{1-4}$ alkyl;

$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl.

Those skilled in the art should understand that the bioactive molecule D can be a compound with biological activity or potential biological activity disclosed in the Chinese, American or European Pharmacopoeia or disclosed in other publications. As an example, the drug can be selected from cytotoxic drugs, cytostatic drugs or immunosuppressive drugs, such as anti-tubulin agents, tubulin inhibitors, DNA minor groove binders, DNA replication inhibitors, alkylating agents, antibiotics, antifolates, antimetabolites, chemosensitizers, topoisomerase inhibitors, vinca alkaloids, etc. Examples of such cytotoxic drugs include, for example, auristatin, camptothecin, duocarmycin, etoposide, maytansine and maytansine alkaloids, taxanes, benzodiazepines or benzodiazepines-containing drugs and vinca alkaloids.

Tp is a targeting moiety (e.g., a small molecule ligand, a protein, a polypeptide, a non-protein agent (e.g., sugar, RNA or DNA)).

In some preferred embodiments, the target of Tp is selected from the group consisting of epidermal growth factor, Trop-2, CD37, HER2, CD70, EGFRvIII, Mesothelin, Folate receptor1, Mucin 1, CD138, CD20, CD19, CD30, SLTRK6, Nectin 4, Tissue factor, Mucin16, Endothelin receptor, STEAP1, SLC39A6, Guanylylcyclase C, PSMA, CCD79b, CD22, Sodium phosphate cotransporter 2B, GPNMB, Trophoblast glycoprotein, AGS-16, EGFR, CD33, CD66e, CD74, CD56, PD-L1, TACSTD2, DR5, E16, 0772P, MPF, Napi3b, Sema 5b, PSCA hlg, ETBR, MSG783, STEAP2, TrpM4, CRIPTO, CD21, CD79b, FcRH2, NCA, MDP, IL20Rα, Brevican, E phB2R, ASLG659, PSCA, GEDA, BAFF-R, CD79a, CXCR5, HLA-DOB, P2X5, CD72, LY64, FcRH1, IRTA2, TENB2, integrin α5β6, α4β7, FGF2, FGFR2, HER3, CA6, DLL3, DLL4, P-cadherin, EpCAM, pCAD, CD223, LYPD3, LY6E, EFNA4, ROR1, SLITRK6, 5T4, ENPP3, Claudin18.2, BMPR1B, Tyro7, c-Met, ApoE, CD1 lc, CD40, CD45(PTPRC), CD49D(ITGA4), CD80, CSF1R, CTSD, GZMB, Ly86, MS4A7, PIK3AP1, PIK3CD, CCR5, IFNG, IL10RA1, I L-6, ACTA2, COL7A1, LOX, LRRC15, MCPT8, MMP10, NOG, SERPINEl, STAT1, TGFBR1, CTSS, PGF, VEGFA, C1QA, C1QB, ANGPTL4, EGLN, EGLN3, BNIP3, AIF1, CCL5, CXCL10, CXCL11, IFI6, PLOD2, KISS1R, STC2, DDIT4, PFKFB3, PGK1, PDK1, AKR1C1, AKR1C2, CADM1, CDH11, COL6A3, CTGF, HMOX1, KRT33A, LUM, WNT5A, IGFBP3, MMP14, CDCP1, PDGFRA, TCF4, TGF, TGFB1, TGFB2, CDl lb, ADGRE1, EMR2, TNFRSF21, UPK1B, TNFSF9, MMP16, MFI2, IGF-1R, RNF43, NaPi2b and BCMA.

In some preferred embodiments, Tp is a small molecule ligand, such as a folic acid derivative, a glutamate urea derivative, a somatostatin derivative, an arylsulfonamide derivative (e.g., a carbonic anhydrase IX inhibitor), an ICG dye, a cyanine dye or a derivative thereof.

According to the embodiments of the present disclosure, the preferred ligand is selected from an antibody or antigen-binding fragment thereof, wherein the antibody is selected from a chimeric antibody, a humanized antibody or a fully human antibody; preferably a monoclonal antibody.

According to an exemplary embodiment of the present disclosure, the antibody or antigen-binding fragment thereof is at least one selected from the following antibodies or antigen-binding fragments thereof: anti-CD20 antibody, anti-CD22 antibody, anti-CD30 antibody, anti-CD33 antibody, anti-CD44 antibody, anti-CD56 antibody, anti-CD70 antibody, anti-CD73 antibody, anti-CD105 antibody, anti-CEA antibody, anti-A33 antibody, anti-Cripto antibody, anti-EphA2 antibody, anti-G250 antibody, anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-MUCl antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody or anti-Mesothelin antibody, and the antibody may be a bispecific antibody or a multispecific antibody.

As an example, the antibody or antigen-binding fragment thereof is at least one selected from the following antibodies or antigen-binding fragments thereof: Trastuzumab, Pertuzumab, Nimotuzumab, Enoblituzumab, Emibetuzumab, Inotuzumab, Pinatuzumab, Brentuximab, Gemtuzumab, Bivatuzumab, Lorvotuzumab, cBR96, and Glematumamab or Glembatumumab.

For example, Trastuzumab has sequences selected from the following:

light chain

DIQMTQSPSSLSASVGDRVTITCRASQDVNTAVAWYQQKPGKAPKLLIYSASFLYS GVPSRFSGSRSGTDFTLTISSLQPEDFATYYCQQHYTTPPTFGQGTKVEIKRTVAAPSVFIF PPSDEQLKSGTASVVCLLNNFYPREAKVQWKVDNALQSGNSQESVTEQDSKDSTYSLSS TLTLSKADYEKHKVYACEVTHQGLSSPVTKSFNRGEC

SEQ ID NO:1

SEQ ID NO:1
heavy chain

EVQLVESGGGLVQPGGSLRLSCAASGFNIKDTYIHWVRQAPGKGLEWVARIYPTN GYTRYADSVKGRFTISADTSKNTAYLQMNSLRAEDTAVYYCSRWGGDGFYAMDYWG

QGTLVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPR EPQVYTLPPSREEMTKNQVSLTCLVKGFYPSDIAVEWESNGQPENNYKTTPPVLDSDGS FFLYSKLTVDKSRWQQGNVFSCSVMHEALHNHYTQKSLSLSPGK

SEQ ID NO:2

Pertuzumab has sequences selected from the following:

light chain

DIQMTQSPSSLSASVGDRVTITCKASQDVSIGVAWYQQKPGKAPKLLIYSASYRYT GVPSRFSGSGSGTDFTLTISSLQPEDFATYYCQQYYIYPYTFGQGTKVEIKRTVAAPSVFI FPPSDEQLKSGTASVVCLLNNFYPREAKVQWKVDNALQSGNSQESVTEQDSKDSTYSLS STLTLSKADYEKHKVYACEVTHQGLSSPVTKSFNRGEC

SEQ ID NO.3

heavy chain

EVQLVESGGGLVQPGGSLRLSCAASGFTFTDYTMDWVRQAPGKGLEWVADVNPN SGGSIYNQRFKGRFTLSVDRSKNTLYLQMNSLRAEDTAVYYCARNLGPSFYFDYWGQG TLVTVSSASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKTHTCPPCP APELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQ VYTLPPSREEMTKNQVSLTCLVKGFYPSDIAVEWESNGQPENNYKTTPPVLDSDGSFFL YSKLTVDKSRWQQGNVFSCSVMHEALHNHYTQKSLSLSPGK

SEQ ID NO.4

Nimotuzumab has sequences selected from the following:

light chain

DIQMTQSPSSLSASVGDRVTITCRSSQNIVHSNGNTYLDWYQQTPGKAPKLLIYKVS NRFSGVPSRFSGSGSGTDFTFTISSLQPEDIATYYCFQYSHVPWTFGQGTKLQITREVAAP SVFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC

SEQ ID NO:5

heavy chain

QVQLQQSGAEVKKPGSSVKVSCKASGYTFTNYYIYWVRQAPGQGLEWIGGINPTS GGSNFNEKFKTRVTITVDESTNTAYMELSSLRSEDTAFYFCARQGLWFDSDGRGFDFWG QGSTVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPR EPQVYTLPPSRDELTKNQVSLTCLVKGFYPSDIAVEWESNGQPENNYKTTPPVLDSDGSF FLYSKLTVDKSRWQQGNVFSCSVMHEALHNHYTQKSLSLSPGK

SEQ ID NO:6

Patritumab has sequences as the following:

light chain

DIEMTQSPDSLAVSLGERATINCRSSQSVLYSSSNRNYLAWYQQNPGQPPKLLIYWASTRESGVPDRFSGSGSGTDFTLTISSLQAEDVAVYYCQQYYSTPRTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

SEQ ID NO:7

heavy chain

QVQLQQWGAGLLKPSETLSLTCAVYGGSFSGYYWSWIRQPPGKGLEWIGEINHSGSTNYNPSLKSRVTISVETSKNQFSLKLSSVTAADTAVYYCARDKWTWYFDLWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO:8.

According to the embodiments of the present disclosure, the conjugate, or the linker or linker-drug thereof may be one selected from the followings, wherein u, v, and w are independently selected from an integer of 0 to 10 (e.g., 0, 1, 2, 3, 4, 5, 6, 7, 8, 9 or 10), G has the above-mentioned definition, LG has the above-mentioned definition of Tp; $R^{20}$, $R^{20'}$ are the same or different, and are independently selected from the group consisting of H, $C_{1-4}$ alkyl, $C_{3-6}$ cycloalkyl, $C_{3-6}$ cycloalkyl-$C_{1-4}$ alkyl, or $R^{20}$, $R^{20'}$ and the carbon atom to which they are attached together form a $C_{3-6}$ cycloalkyl;
for example, the $C_{1-4}$ alkyl can be independently selected from the group consisting of methyl, ethyl, propyl, isopropyl, butyl, isobutyl or tert-butyl; the $C_{3-6}$ cycloalkyl can be independently selected from the group consisting of cyclopropyl, cyclobutyl, cyclopentyl or cyclohexyl;
for example, both of $R^{20}$ and $R^{20'}$ are H, or one is not H, or both are not H:

| No. | Conjugate, its linker or linker-drug |
|---|---|
| 1 | 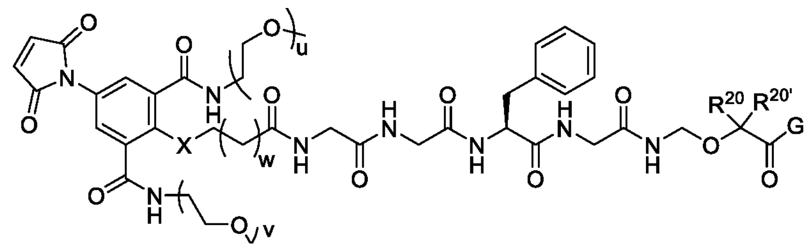 |
| 2 | 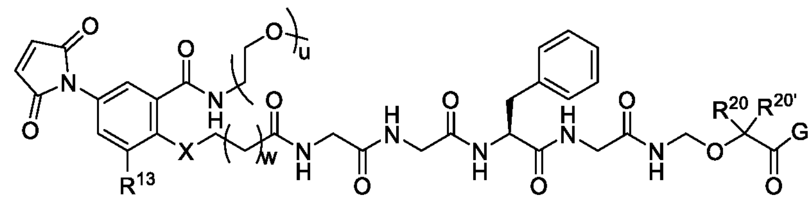 |

(continued)

| No. | Conjugate, its linker or linker-drug |
|---|---|
| 3 | 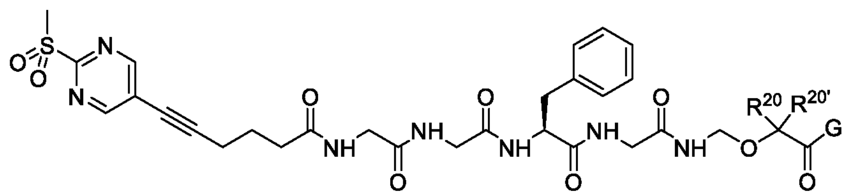 |
| 4 | 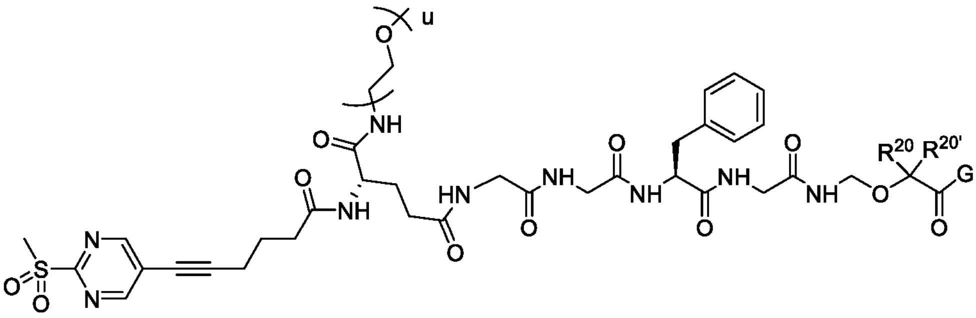 |
| 5 | 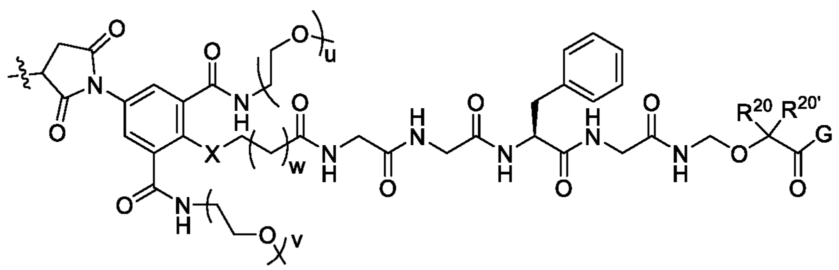 |
| 6 | 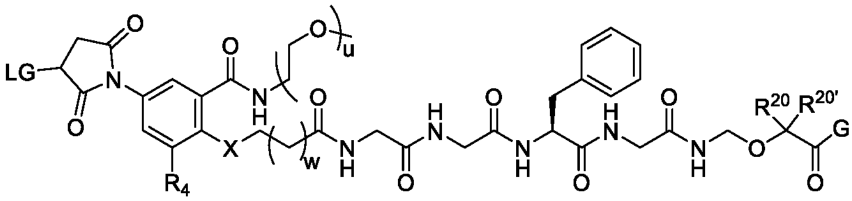 |
| 7 | 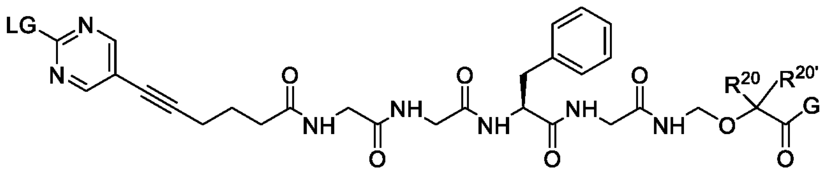 |
| 8 | 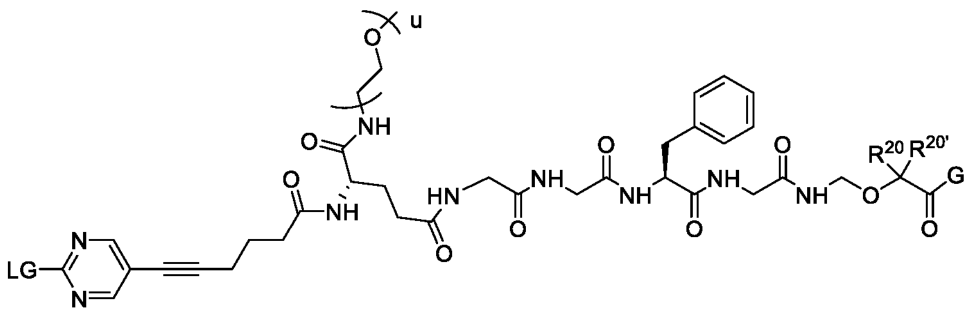 |

(continued)

| No. | Conjugate, its linker or linker-drug |
|---|---|
| 9 | 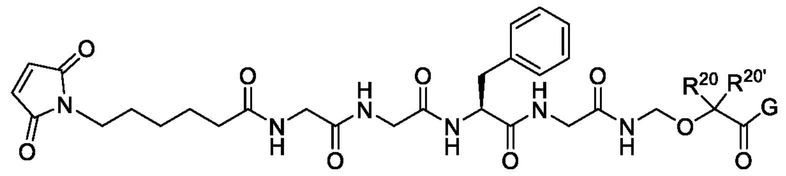 |
| 10 | 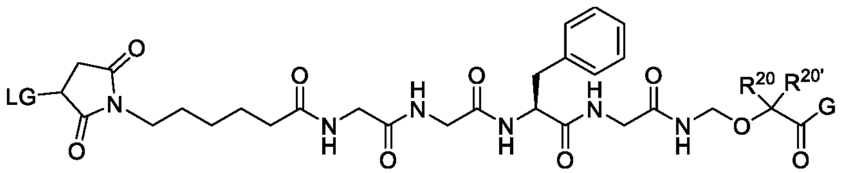 |
| 11 | 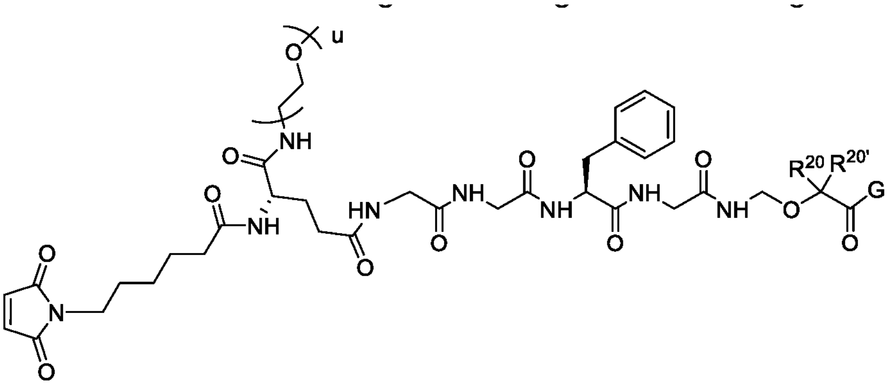 |
| 12 | 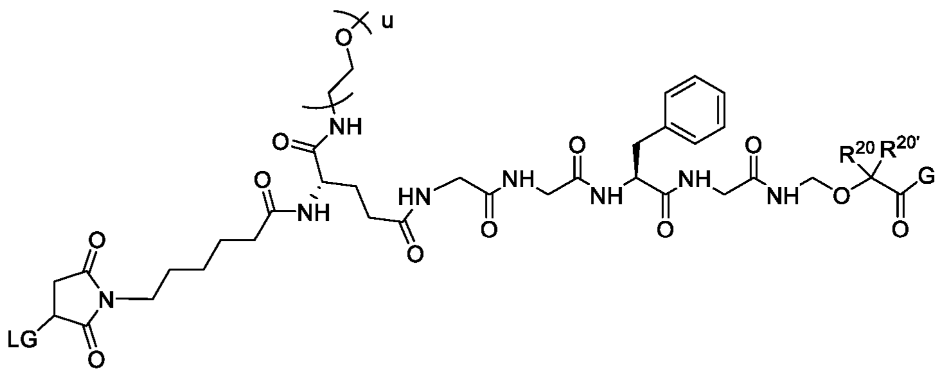 |
| 13 | 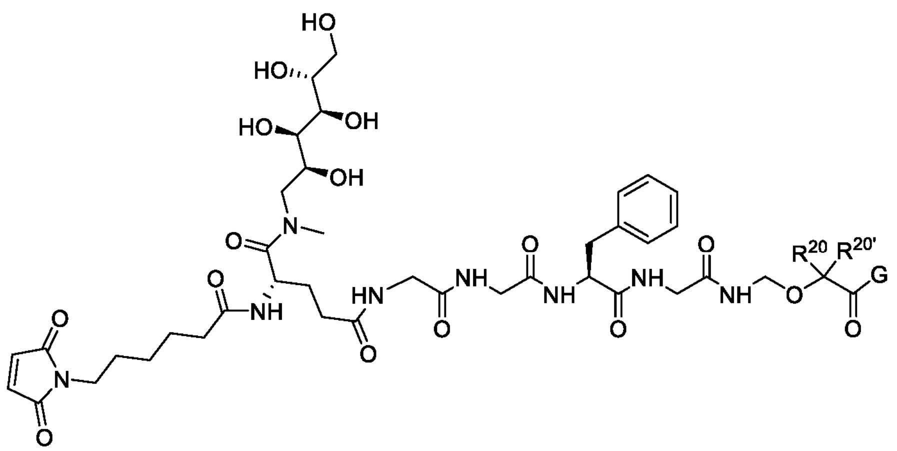 |

(continued)

| No. | Conjugate, its linker or linker-drug |
|---|---|
| 14 | 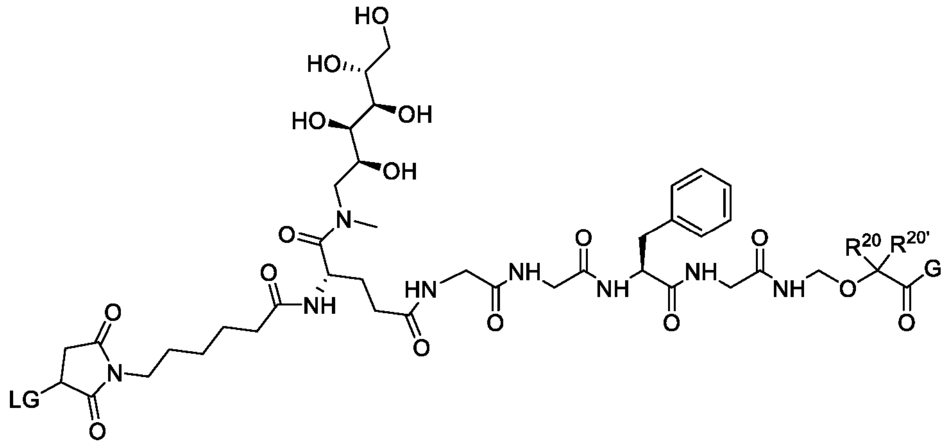 |
| 15 | 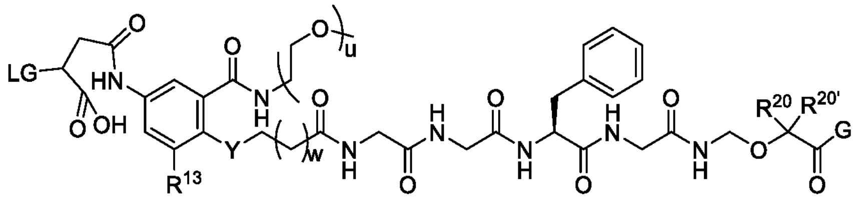 |
| 16 | 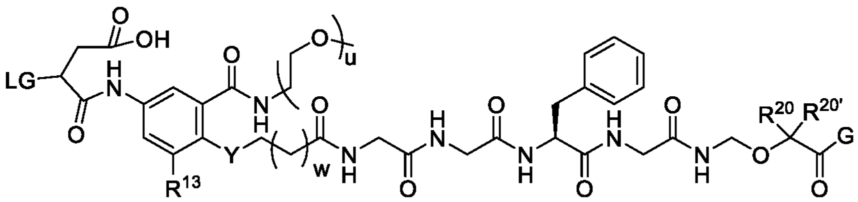 |
| 17 | 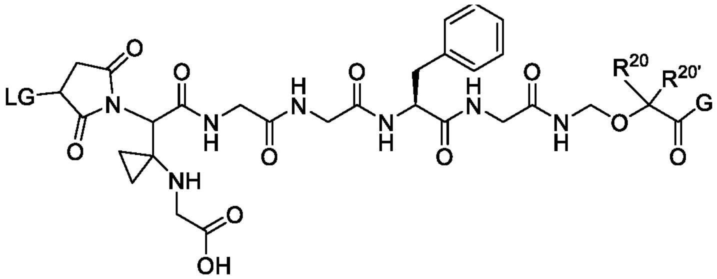 |
| 18 | 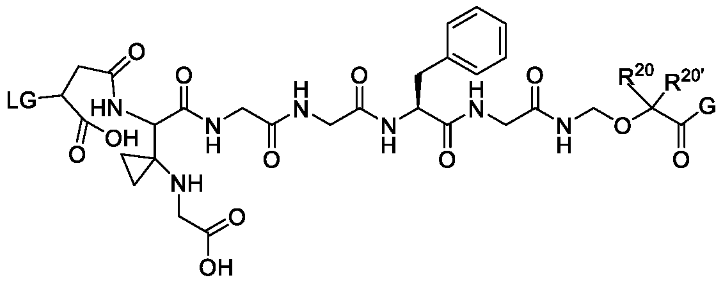 |

(continued)

| No. | Conjugate, its linker or linker-drug |
|---|---|
| 19 | 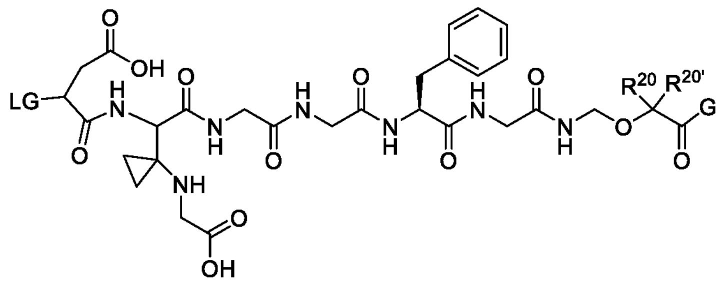 |
| 20 | 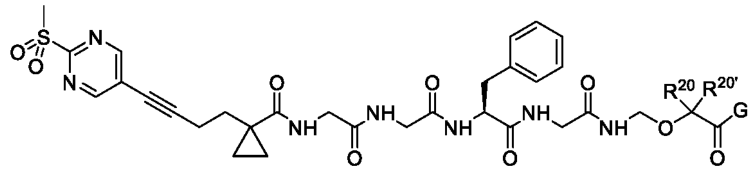 |
| 21 | 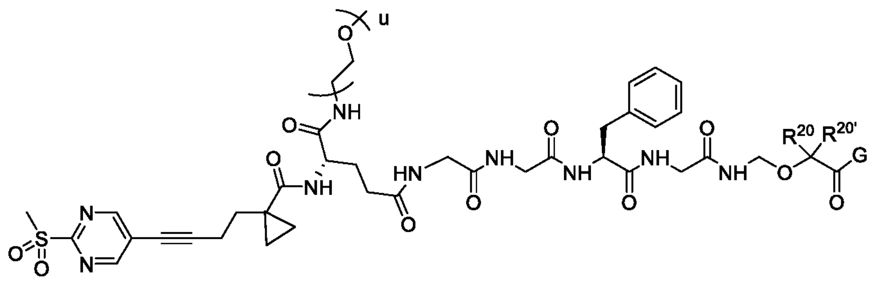 |
| 22 | 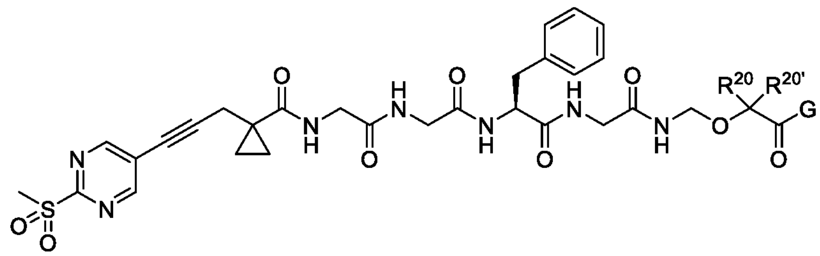 |
| 23 | 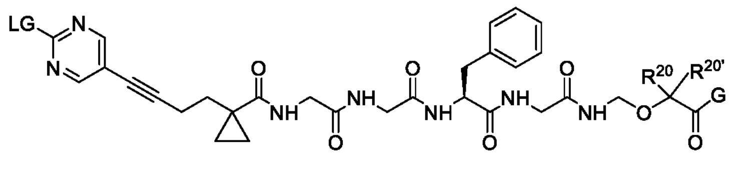 |
| 24 | 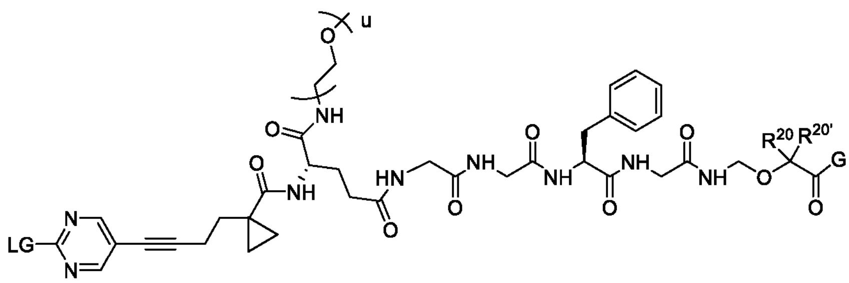 |

(continued)

| No. | Conjugate, its linker or linker-drug |
|---|---|
| 25 | 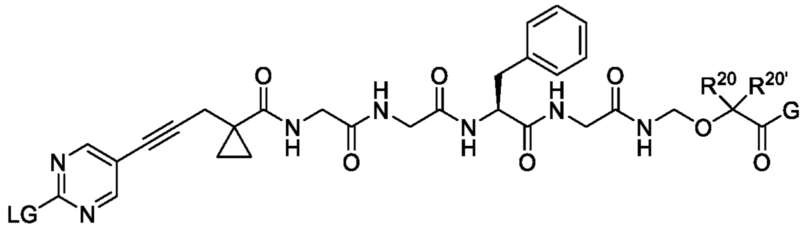 |
| 26 | 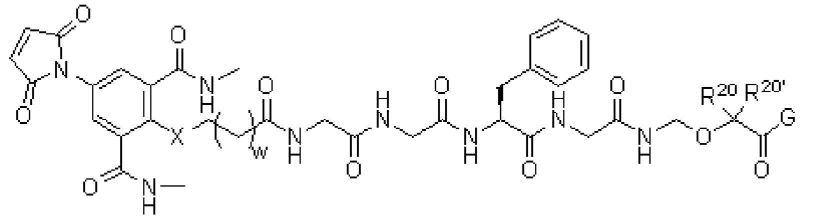 |
| 27 | 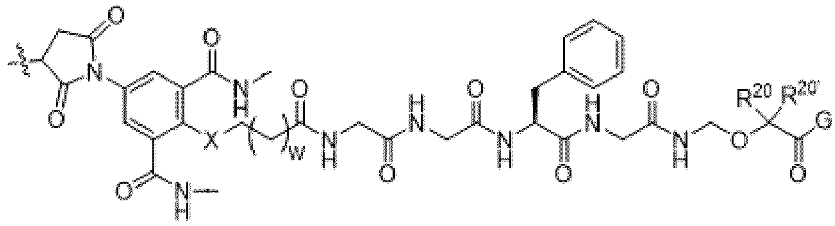 |

According to the embodiments of the present disclosure, the conjugate may have a structure as shown in the following formula:

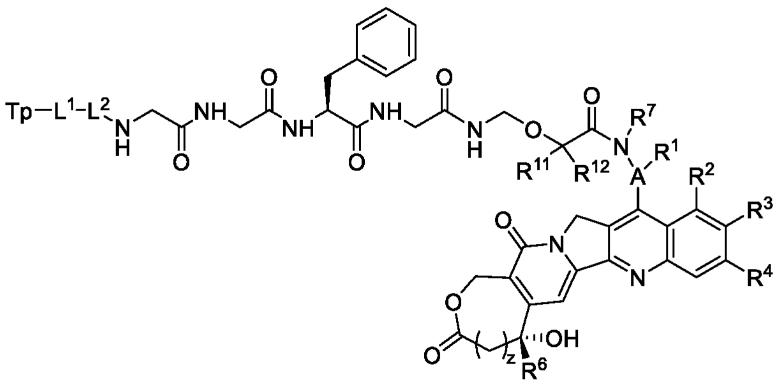

wherein, A, $R^1$, $R^2$, $R^3$, $R^4$, $R^6$, $R^7$ , $R^{11}$, $R^{12}$, $L^1$, $L^2$, and Tp have the definitions described above.

According to the embodiments of the present disclosure, the conjugate may have a structure as shown in the following formula:

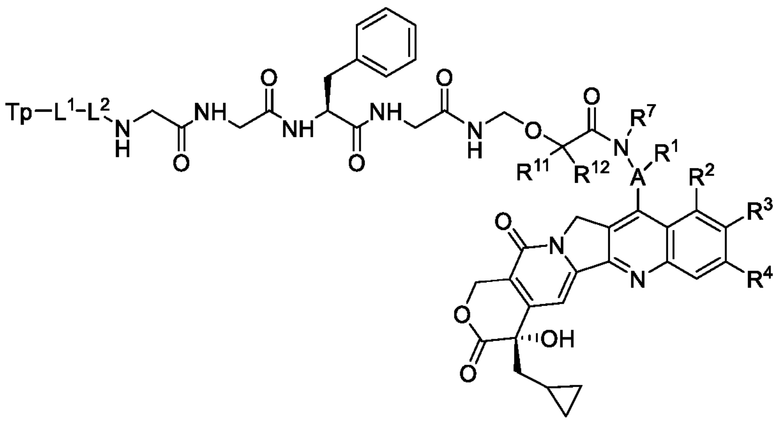

wherein, A, $R^1$, $R^2$, $R^3$, $R^4$, $R^7$, $R^{11}$, $R^{12}$, $L^1$, $L^2$, and Tp have the definitions described above.

According to the embodiments of the present disclosure, the conjugate may have a structure as shown in the following formula:

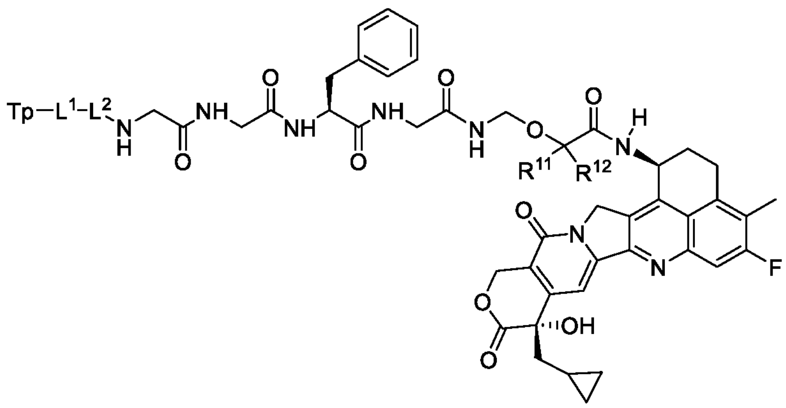

wherein, $R^{11}$, $R^{12}$, $L^1$, $L^2$, and Tp have the definitions described above.

According to the embodiments of the present disclosure, the conjugate may be one selected from the followings:

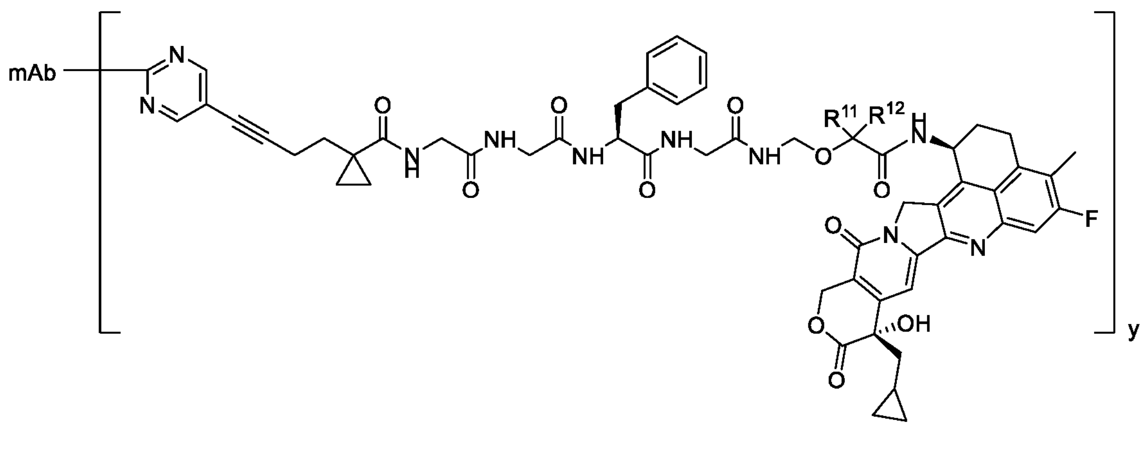

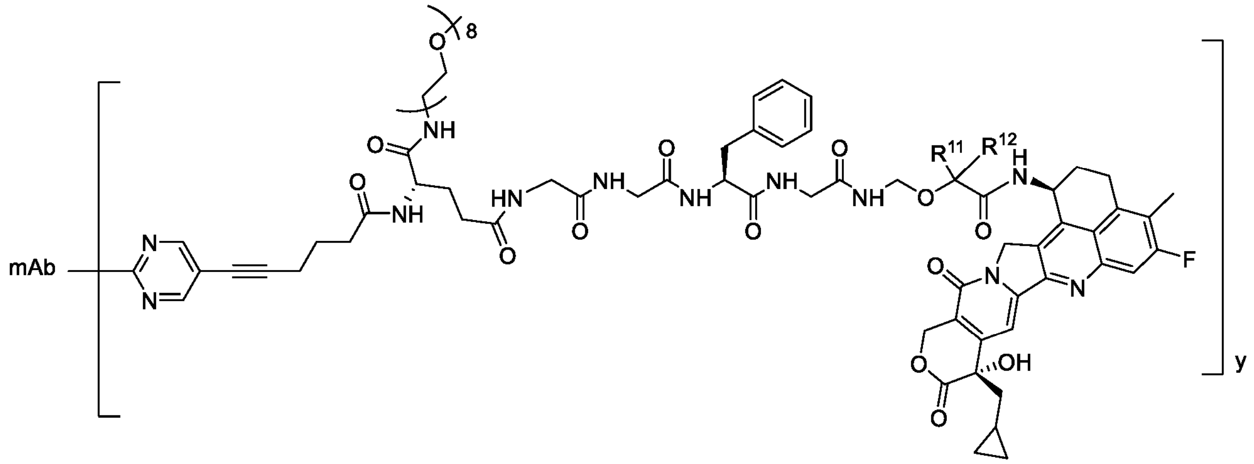

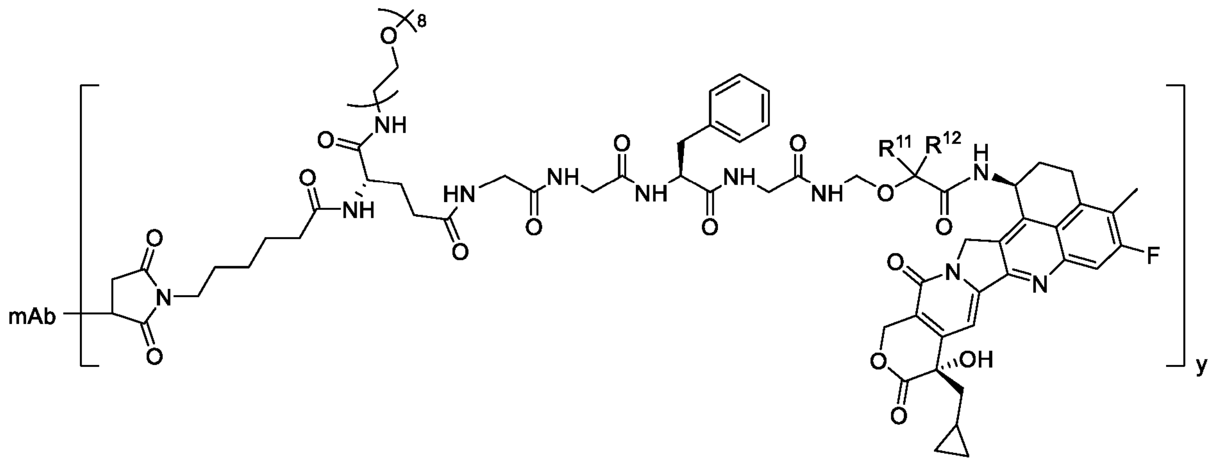

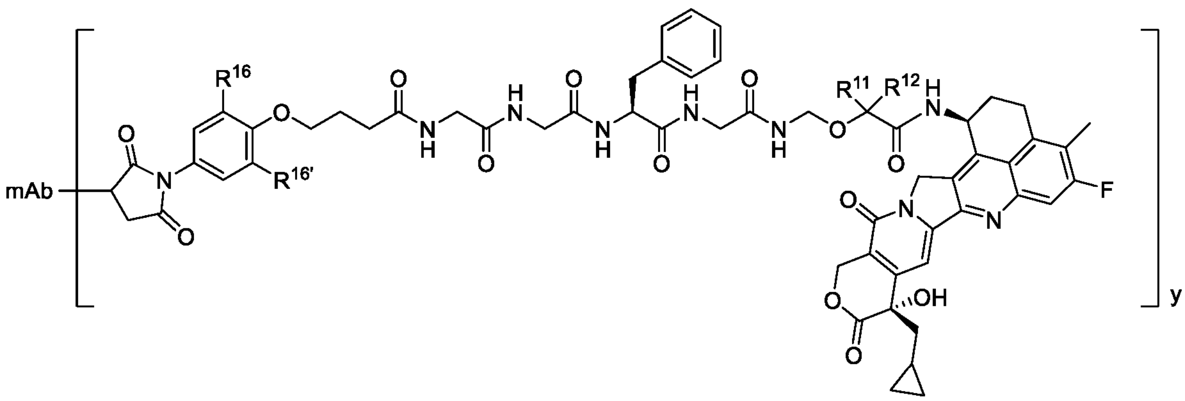

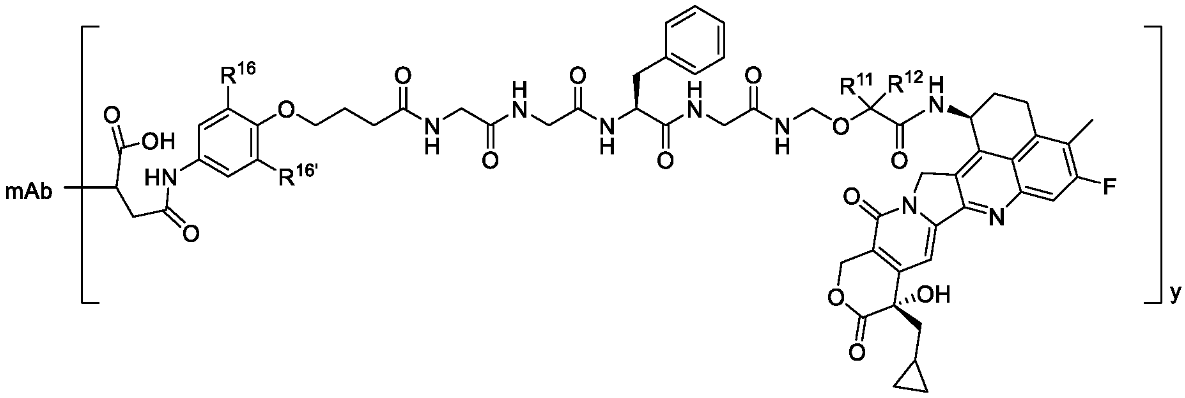

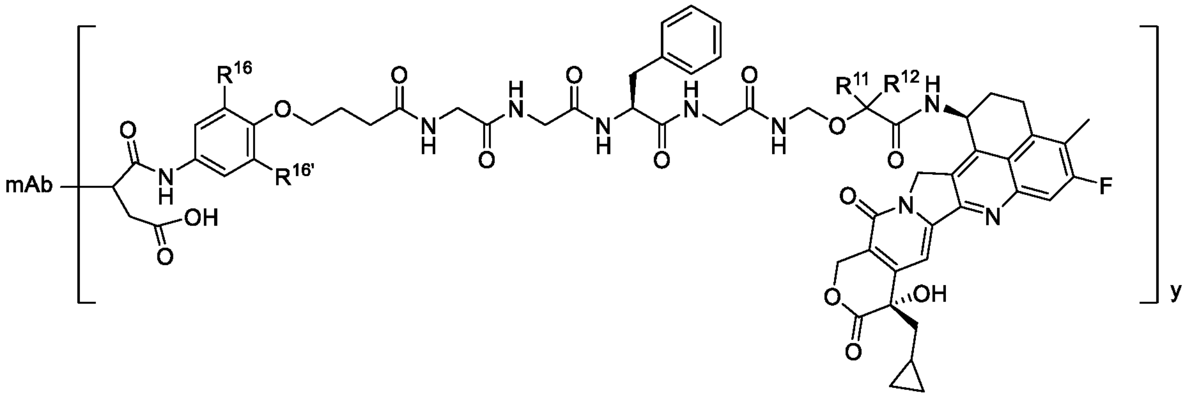

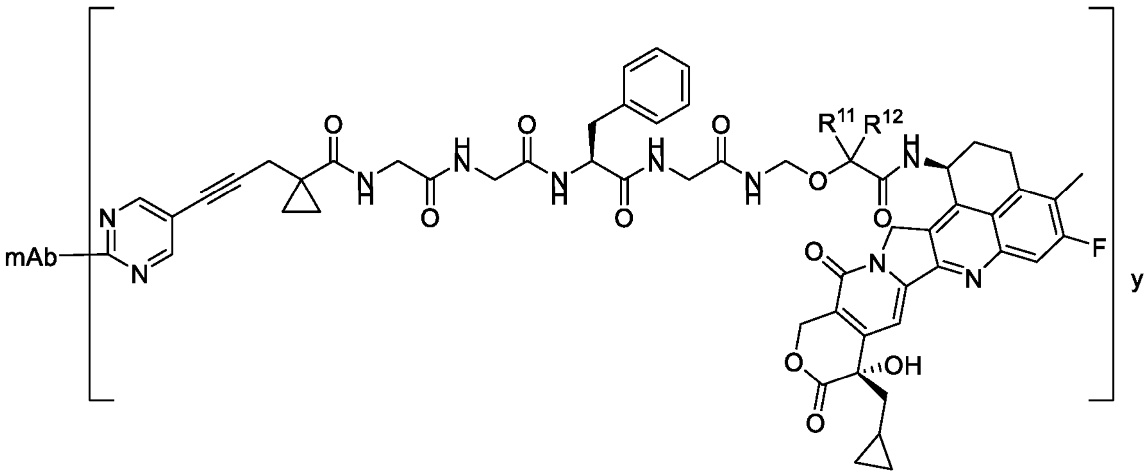

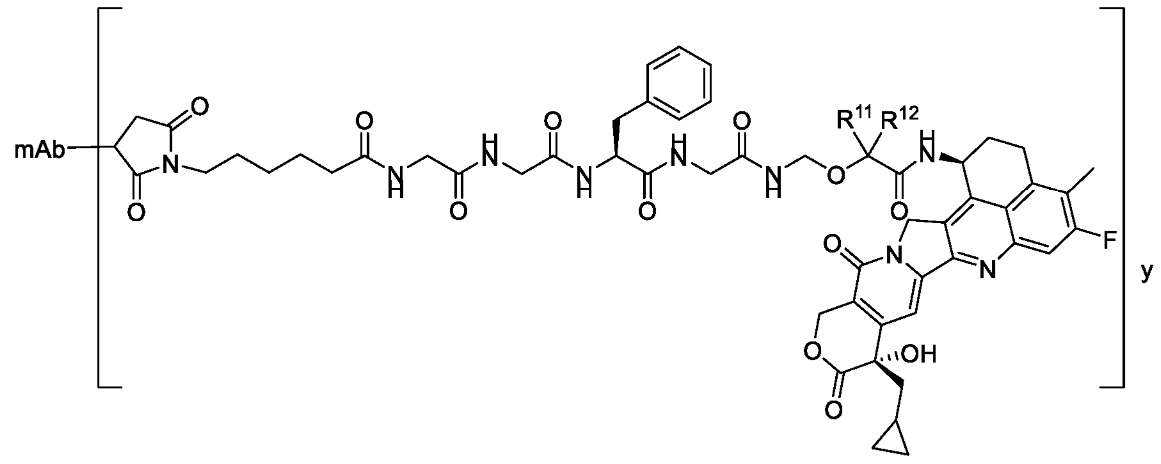

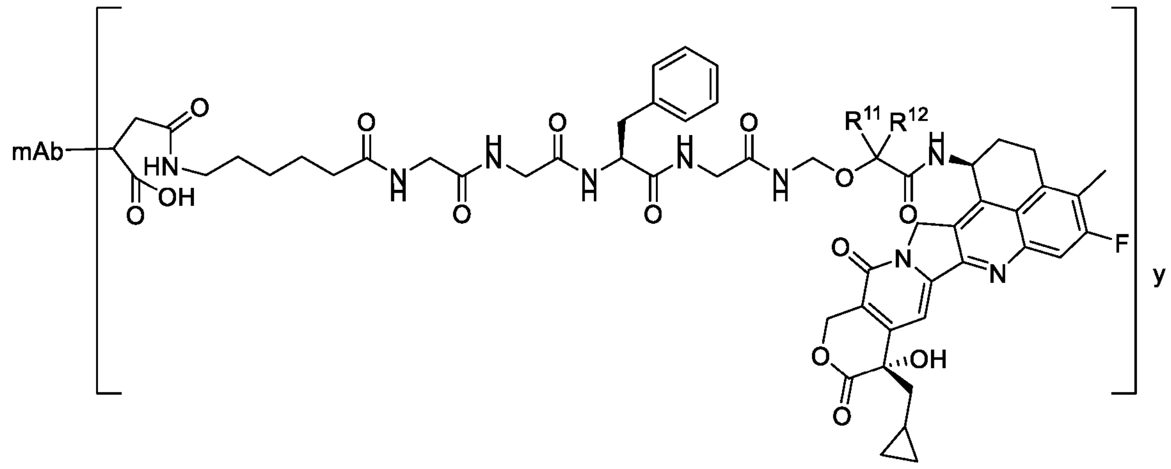

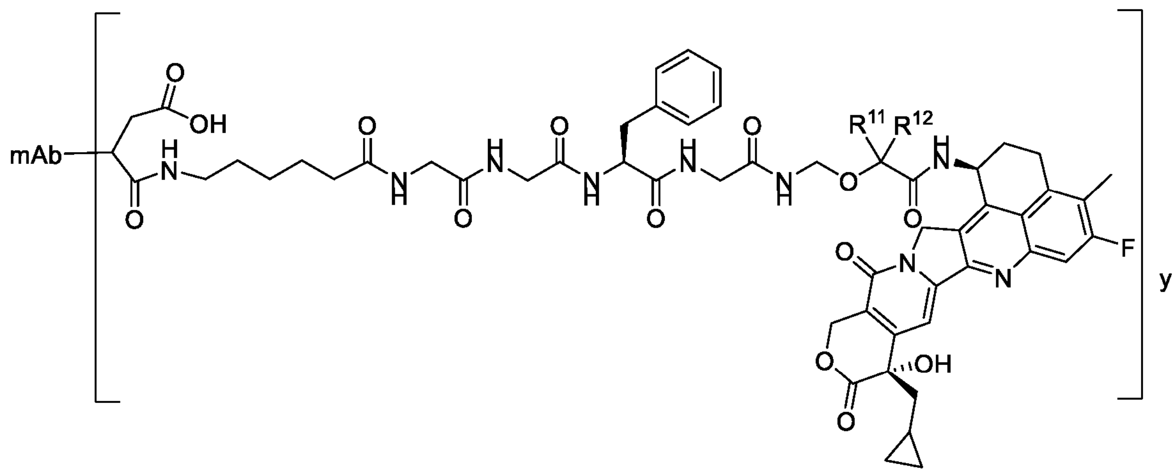

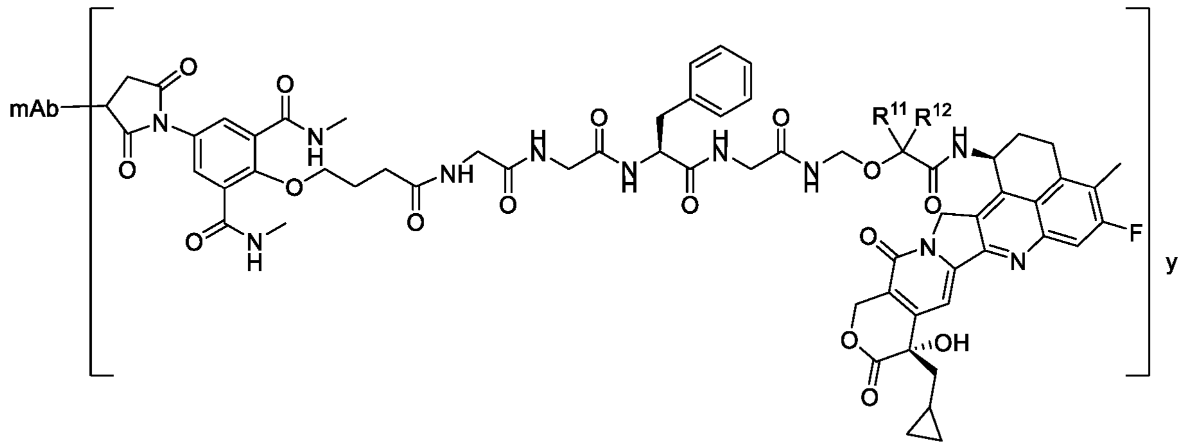

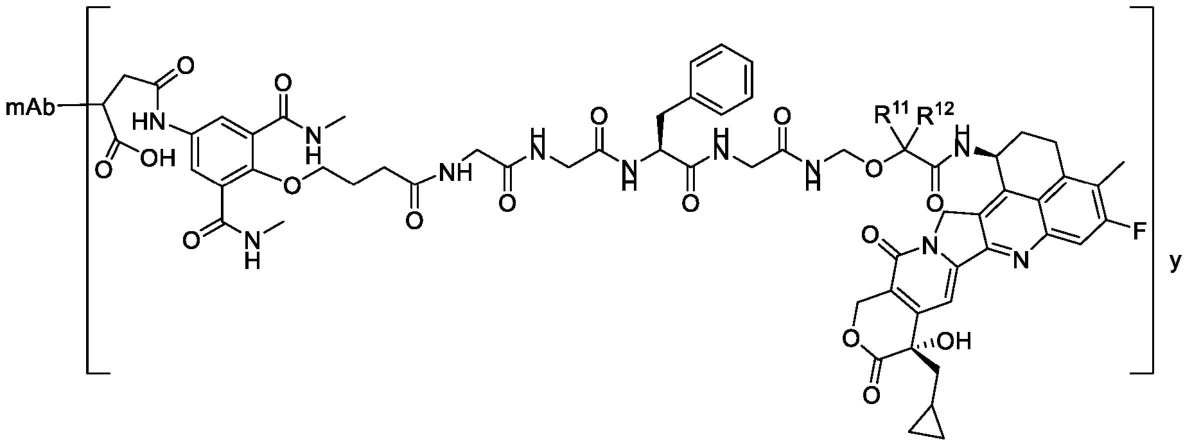

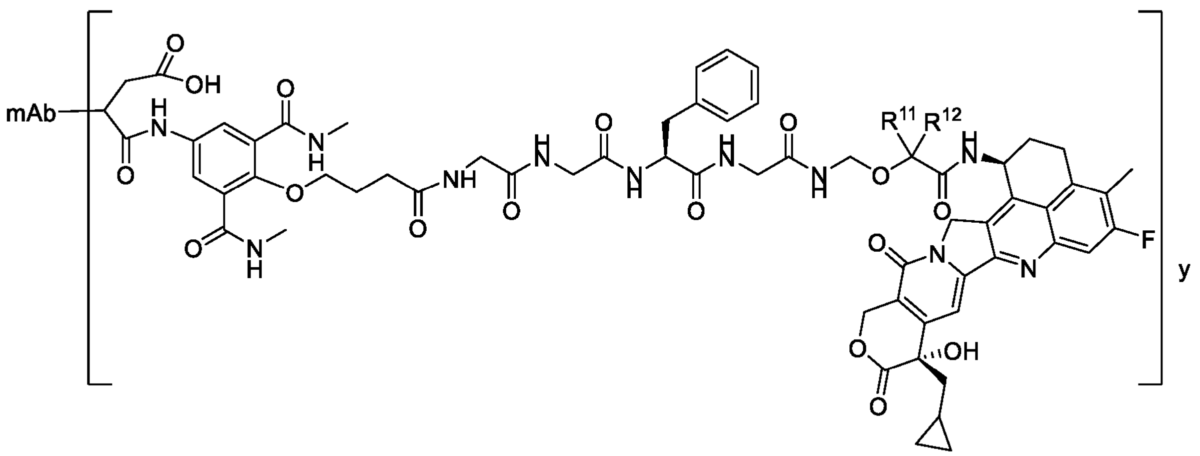

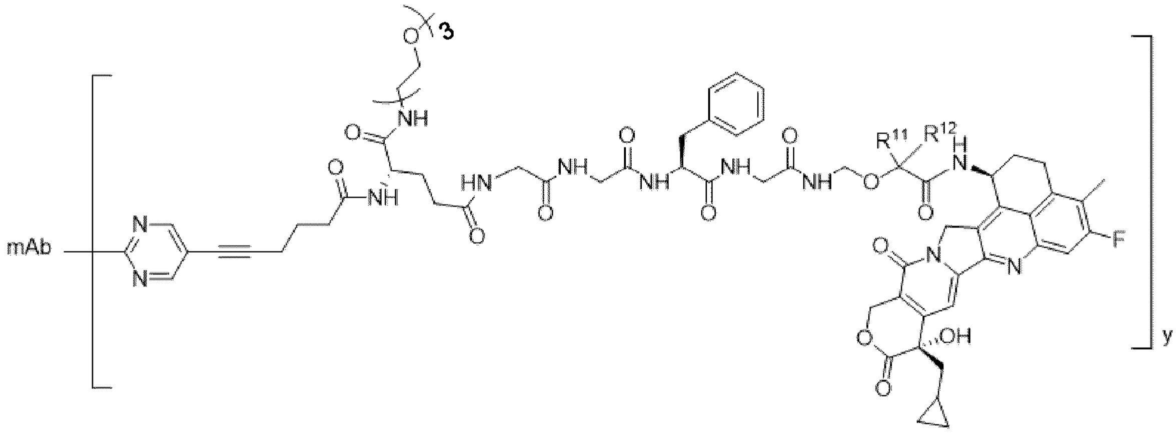

,

wherein, $R^{11}$, $R^{12}$, $R^{16}$, and $R^{16'}$ have the definitions described above;

for example, $R^{11}$ and $R^{12}$ are the same or different and are independently selected from the group consisting of H, $C_{1-4}$ alkane $C_{3-6}$ cycloalkyl, $C_{3-6}$ cycloalkyl-$C_{1-4}$ alkyl, or $R^{11}$ and $R^{12}$, together with the carbon atom to which they are attached, form a $C_{3-6}$ cycloalkyl;

for example, the $C_{1-4}$ alkyl can be independently selected from the group consisting of methyl, ethyl, propyl, isopropyl, butyl, isobutyl or tert-butyl; the $C_{3-6}$ cycloalkyl group can be independently selected from the group consisting of cyclopropyl, cyclobutyl, cyclopentyl or cyclohexyl;

for example, both of $R^{11}$ and $R^{12}$ are H, or one of them is not H, or both of them are not H;

mAb represents a monoclonal antibody;

y represents the average number of small molecule drugs attached to each mAb (DAR), which can be selected from an integer or decimal, such as an integer or decimal selected from 1 to 50, an integer or decimal of 1 to 20, or an integer or decimal of 1 to 10.

According to the embodiment of the present disclosure, the conjugate may have a structure as shown in the following formula:

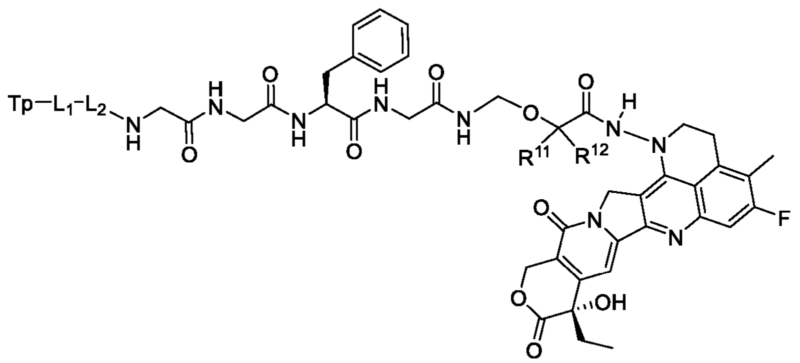

wherein, $R^{11}$, $R^{12}$, $L^1$, $L^2$, and Tp have the definitions as described above.

According to the embodiments of the present disclosure, the conjugate may be one selected from the followings:

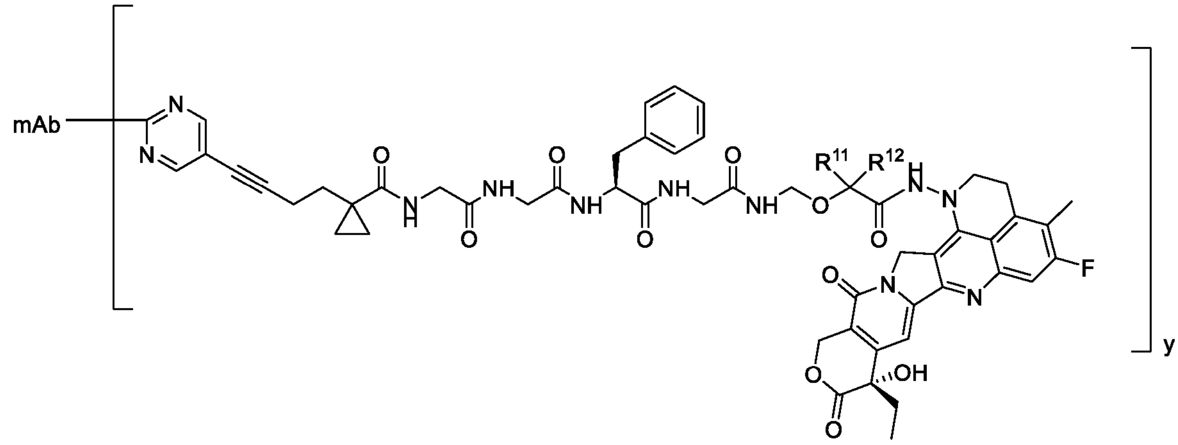

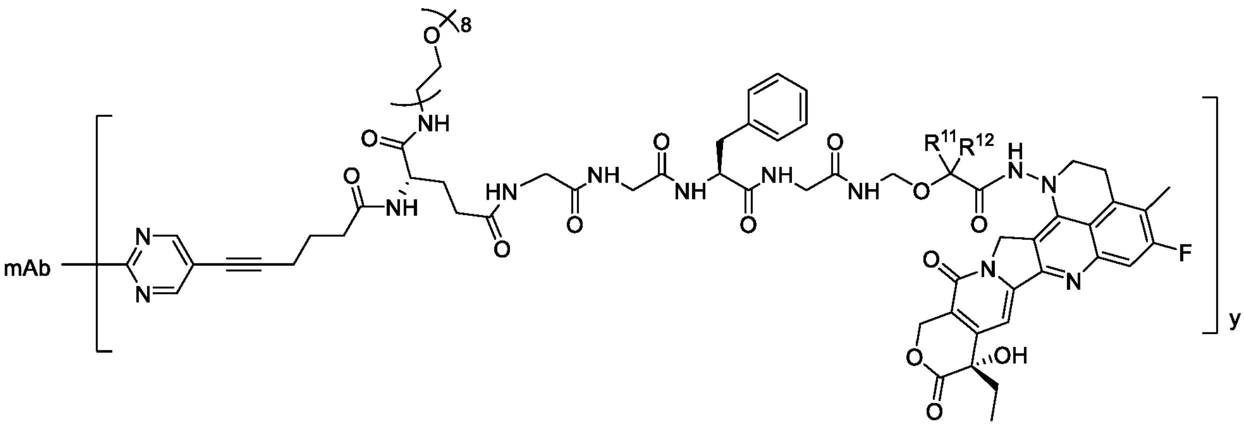

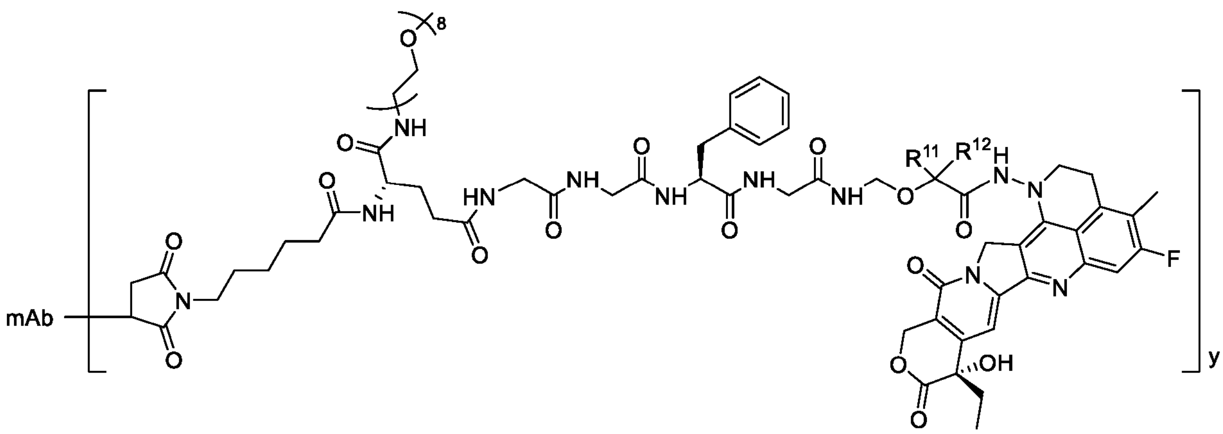

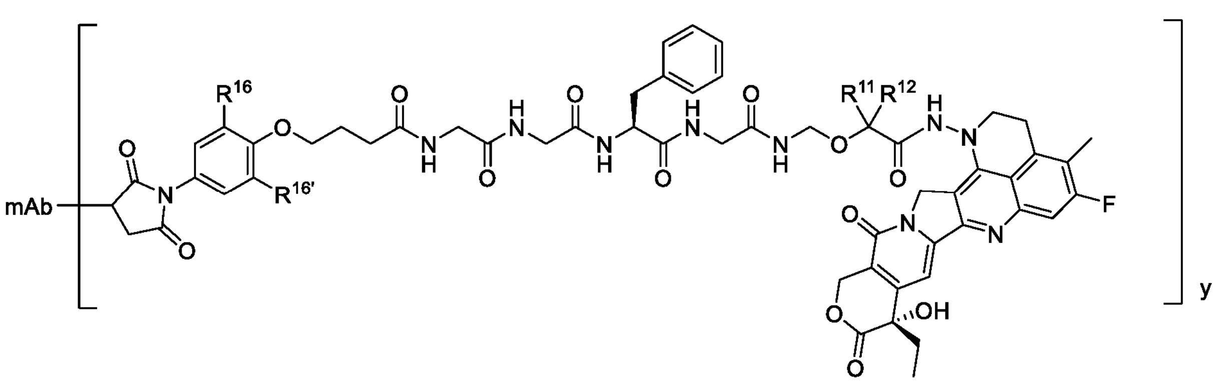

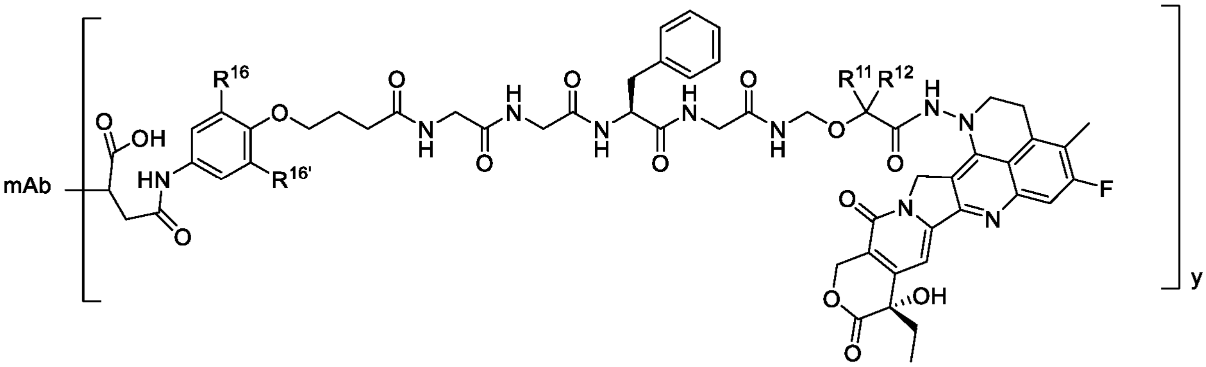

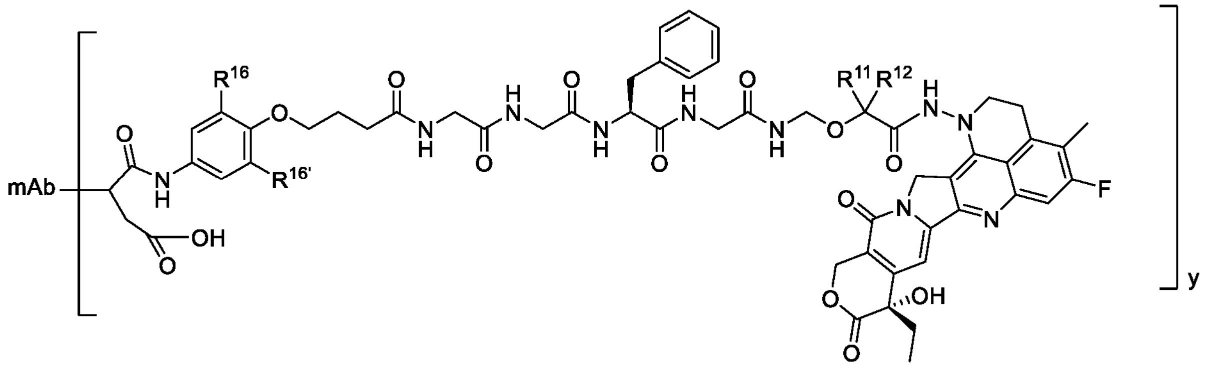

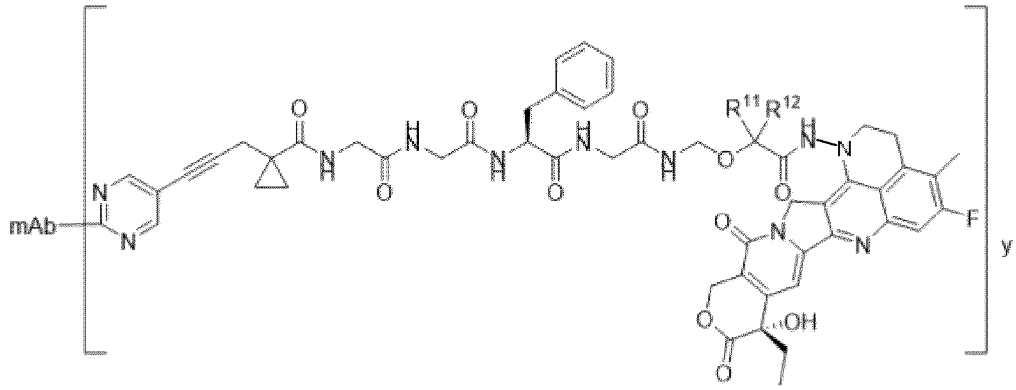

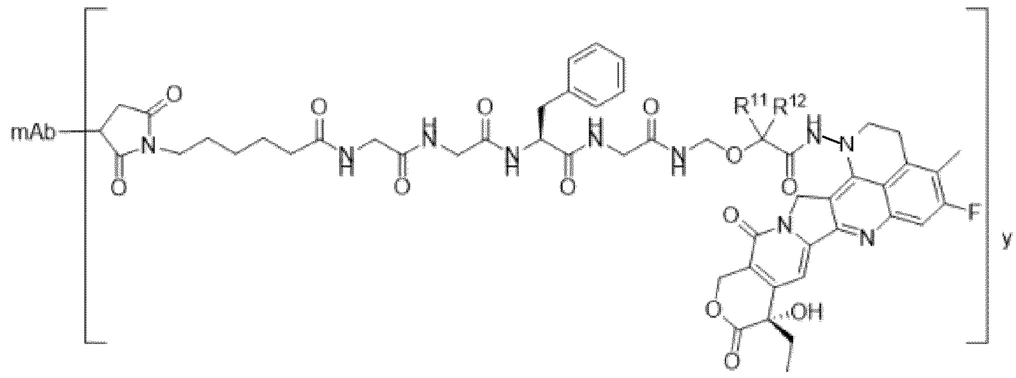

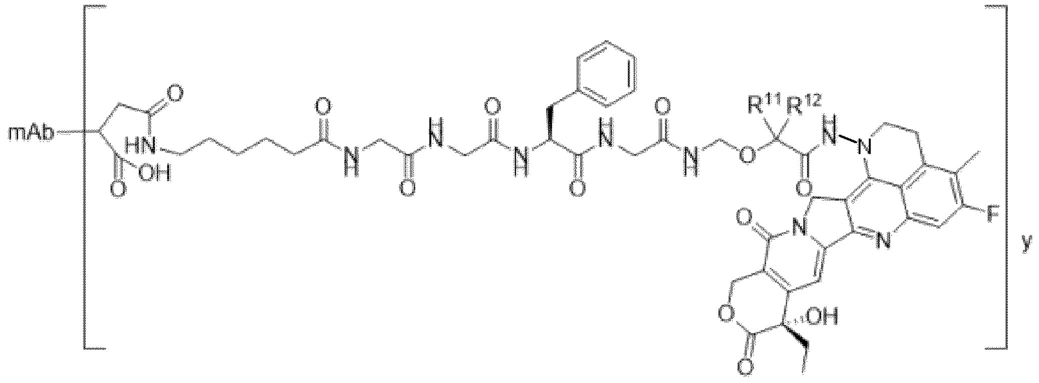

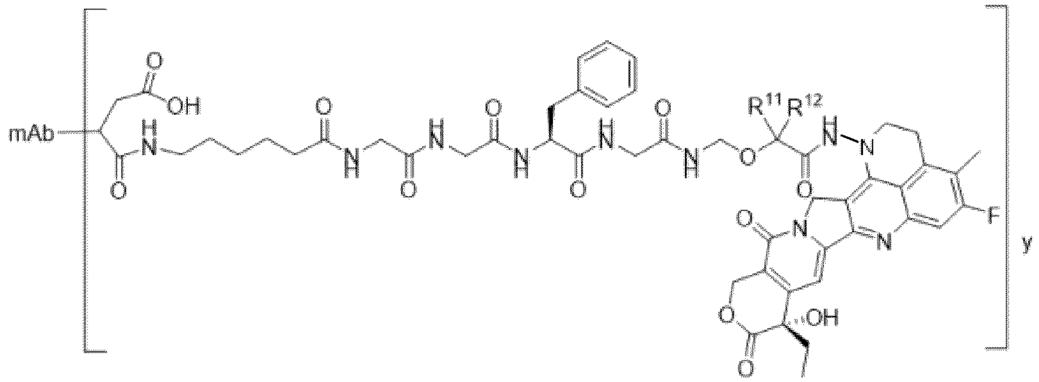

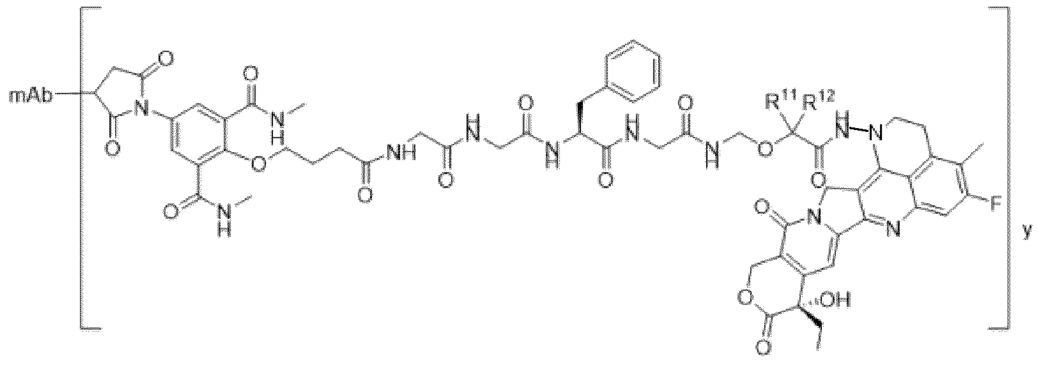

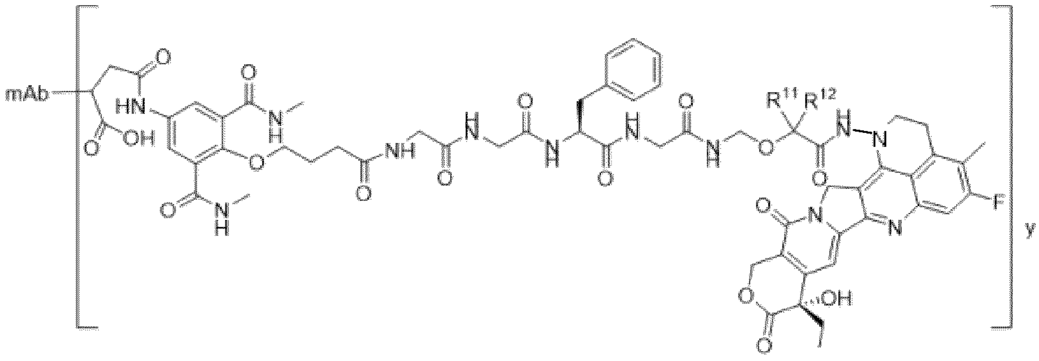

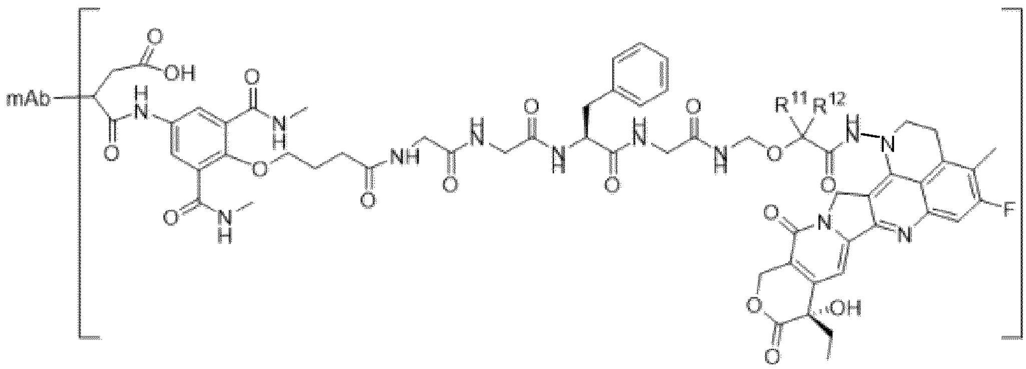

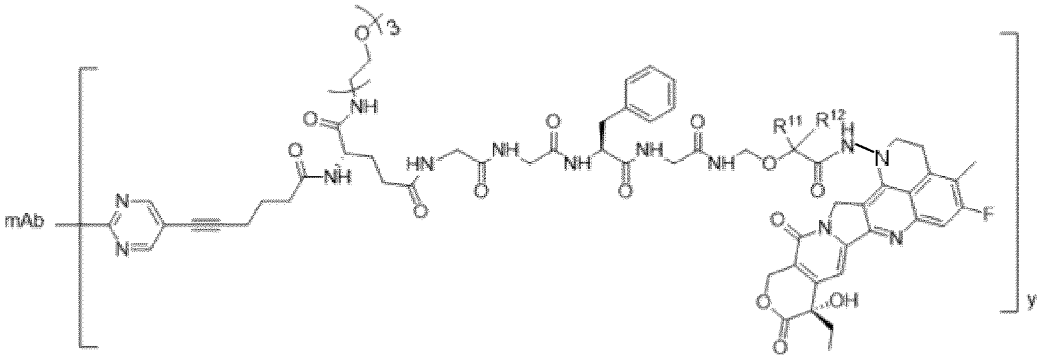

wherein, $R^{11}$, $R^{12}$, $R^{16}$, and $R^{16'}$ independently have the definitions as described above;

mAb represents a monoclonal antibody;

y represents the average number of small molecule drugs attached to each monoclonal antibody (DAR), and can be selected from an integer or decimal, such as an integer or decimal of 1 to 50, an integer or decimal of 1 to 20, or an integer or decimal of 1 to 10.

According to the embodiment of the present disclosure, the conjugate may have a structure as shown in the following formula:

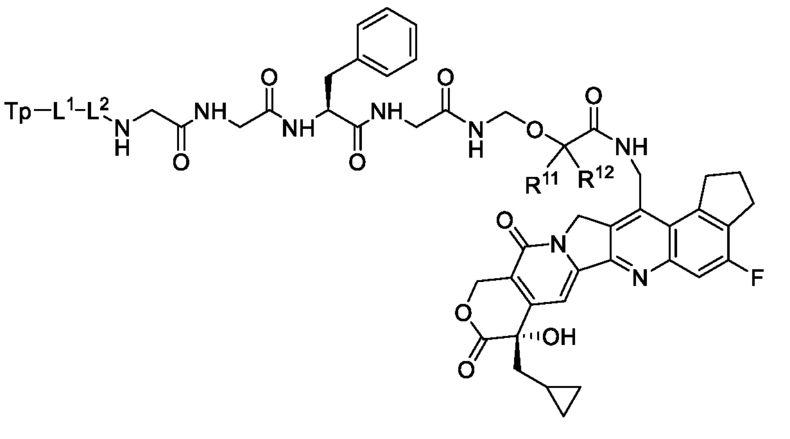

wherein, $R^{11}$, $R^{12}$, $L^1$, $L^2$, and Tp have the definitions as described above.

According to the embodiments of the present disclosure, the conjugate can be one selected from the followings:

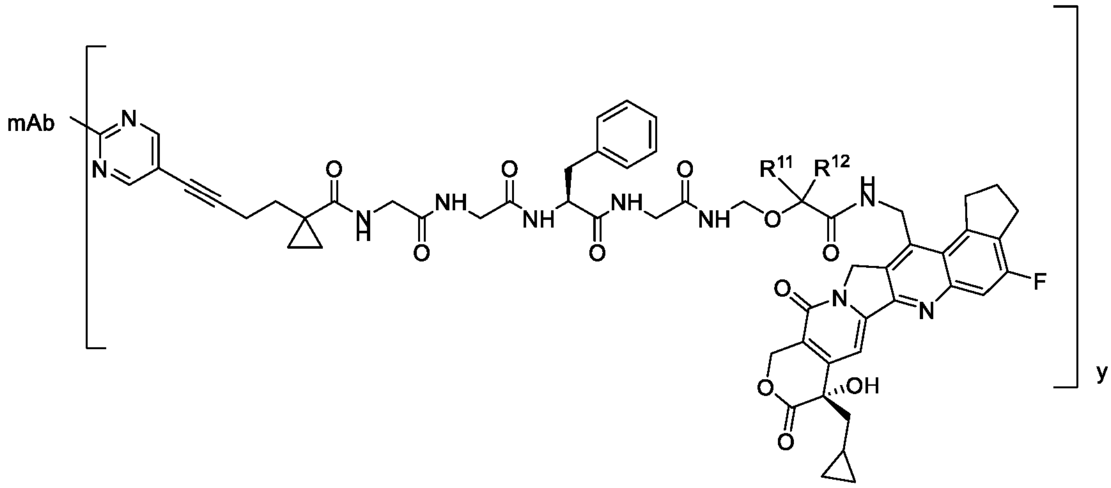

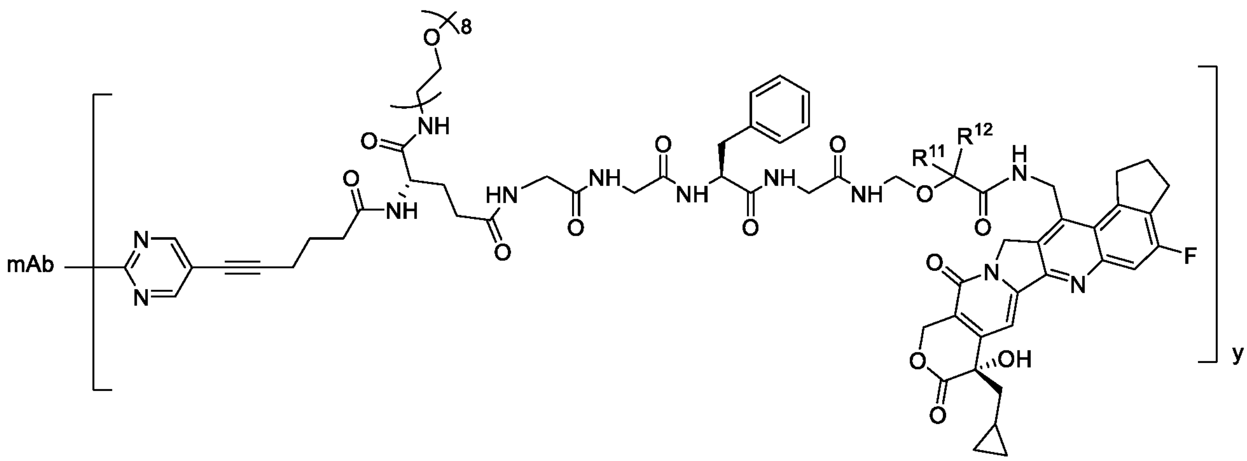

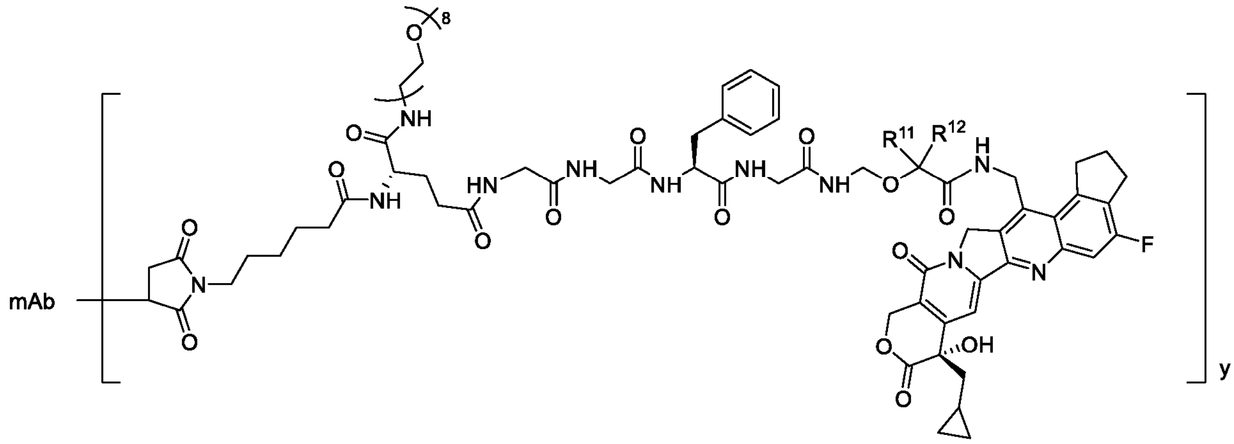

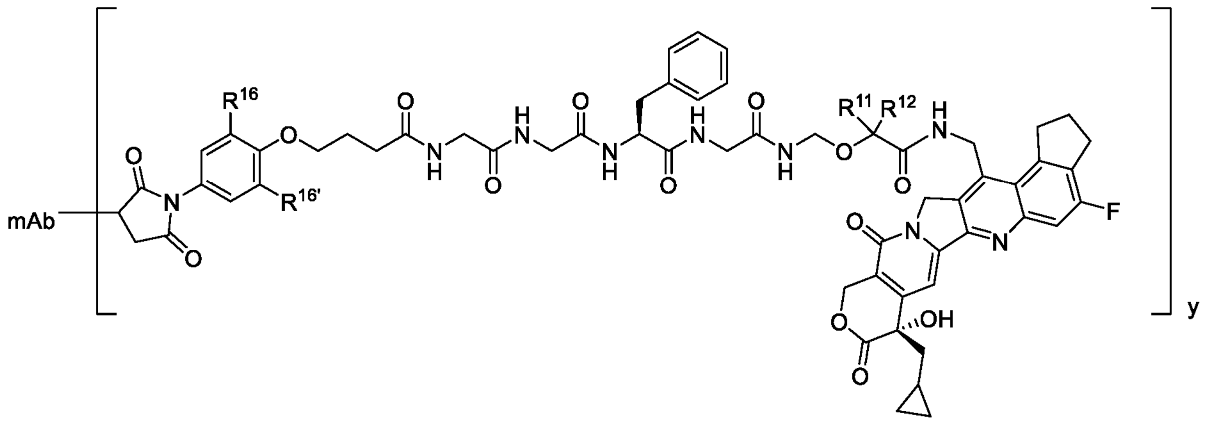

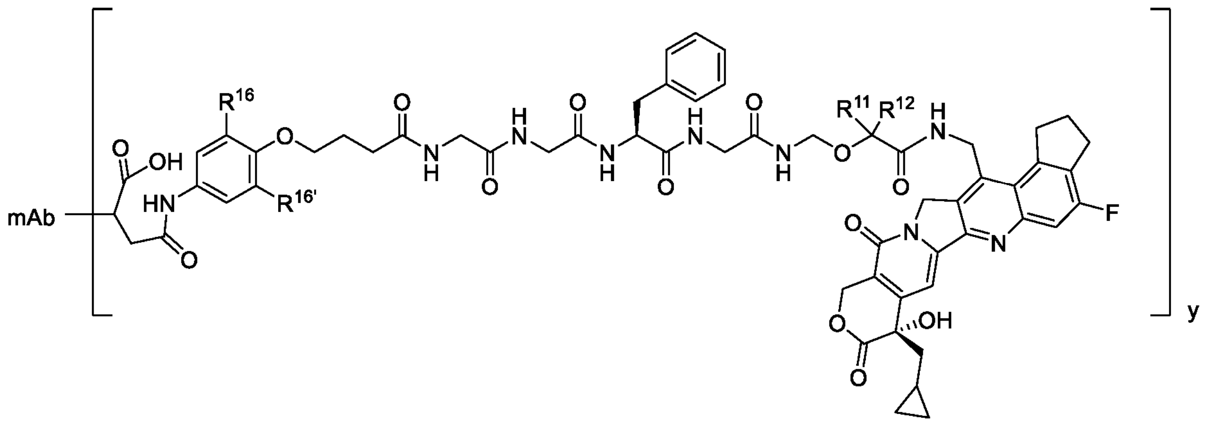

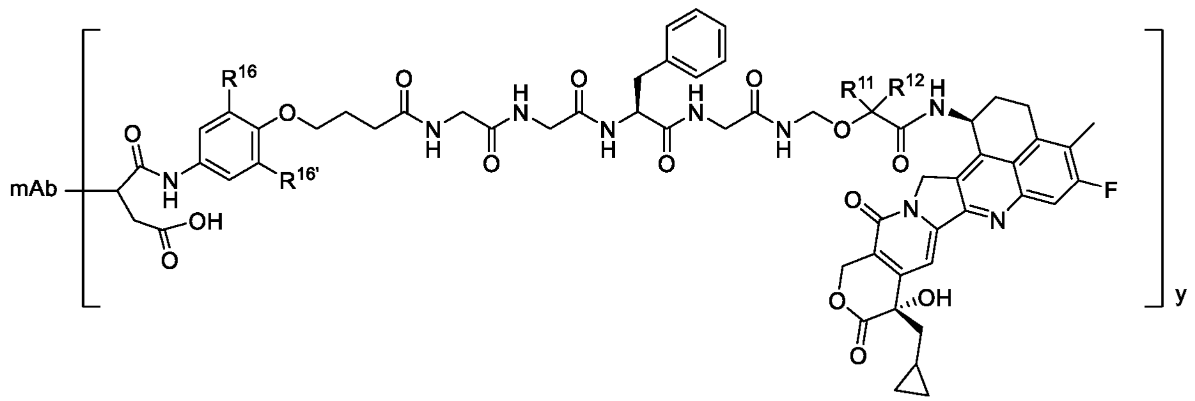

wherein, $R^{11}$, $R^{12}$, $R^{16}$, and $R^{16'}$ have the definitions as described above;

mAb represents a monoclonal antibody;

y represents the average number of small molecule drugs attached to each monoclonal antibody (DAR), which can be selected from an integer or decimal, such as an integer or decimal selected from 1 to 50, an integer or decimal selected from 1 to 20, or an integer or decimal selected from 1 to 10.

According to the embodiment of the present disclosure, examples of the conjugate intermediates, namely linker-drugs, may be selected from one of the followings:

| | |
|---|---|
| 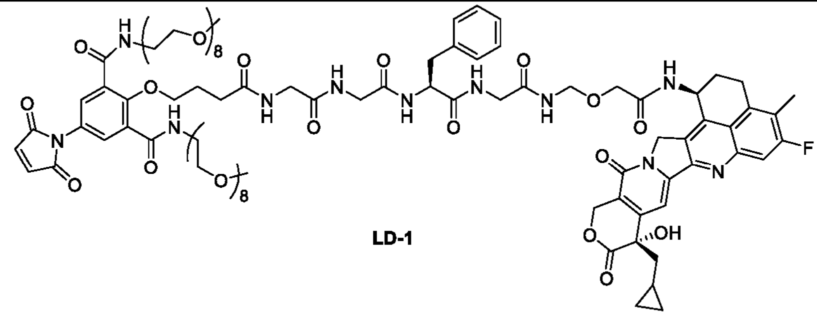 | LD-1 |
| 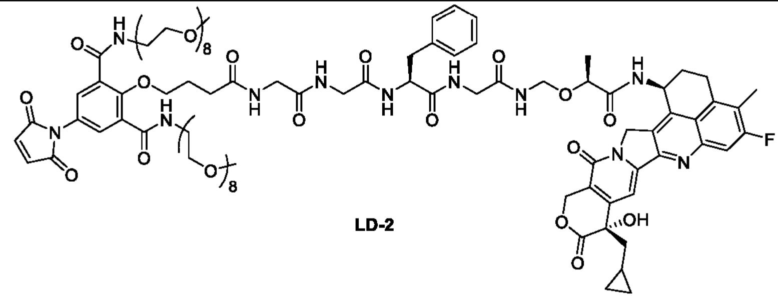 | LD-2 |
| 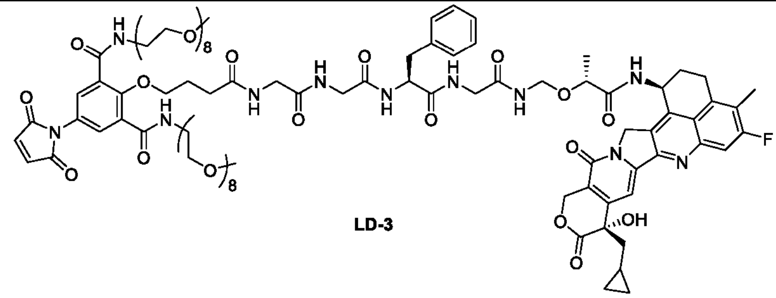 | LD-3 |

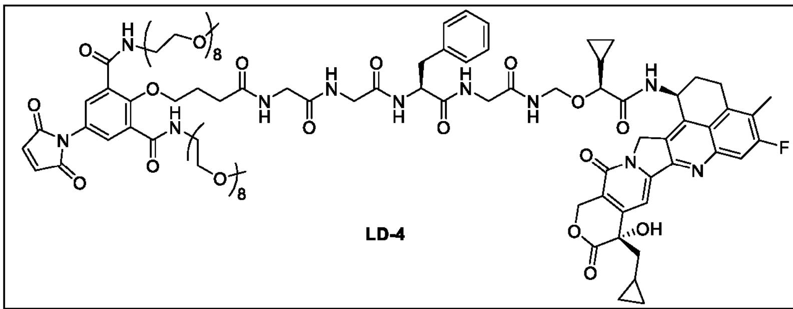

LD-4

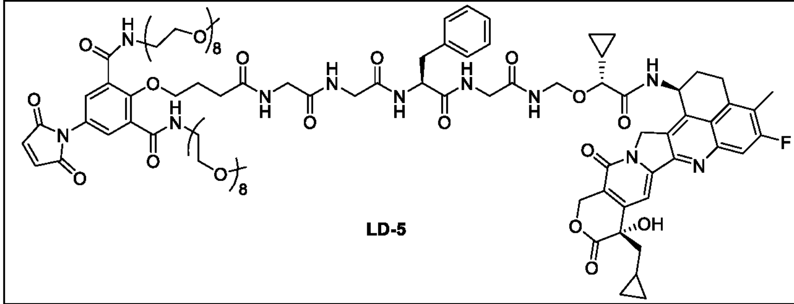

LD-5

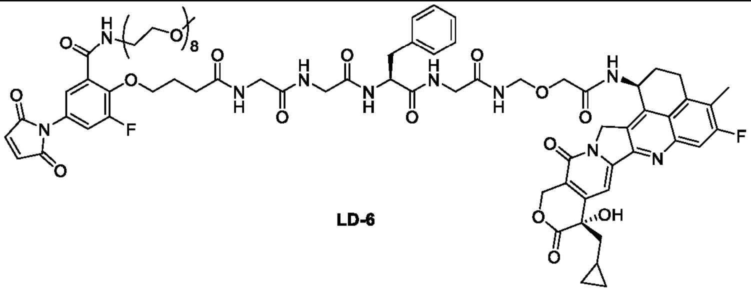

LD-6

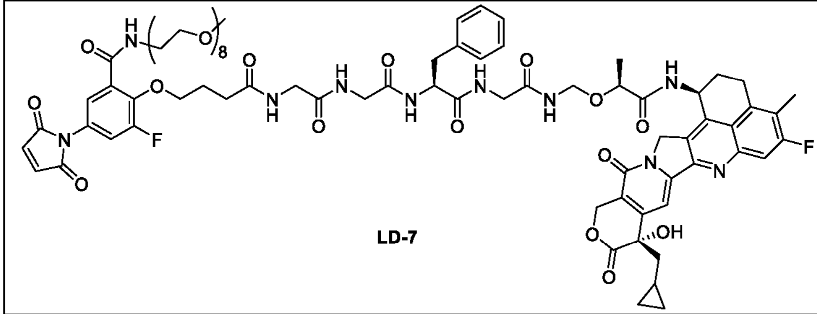

LD-7

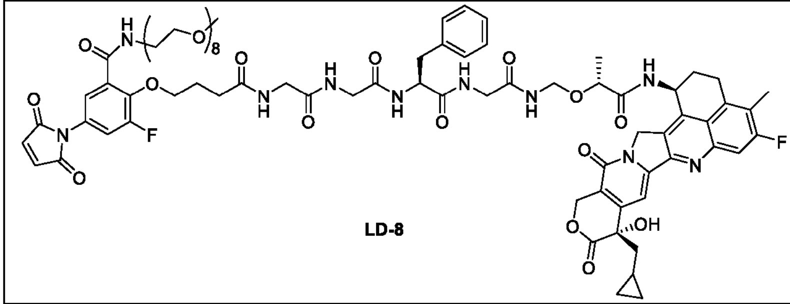

LD-8

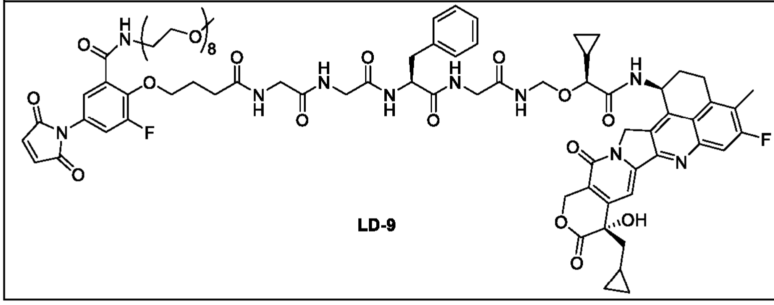

LD-9

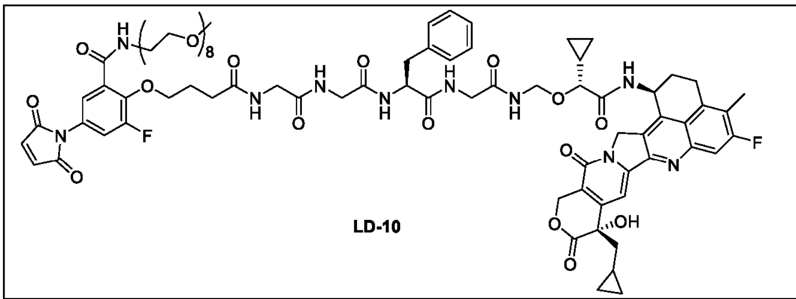

LD-10

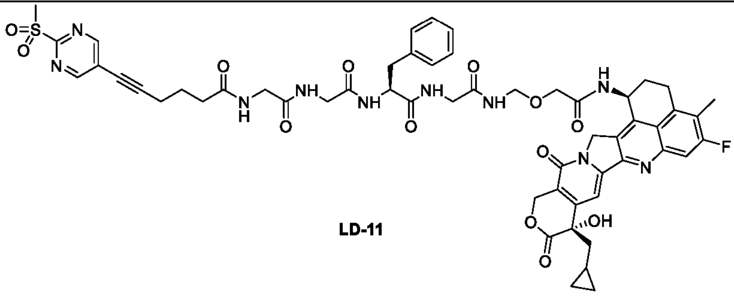

LD-11

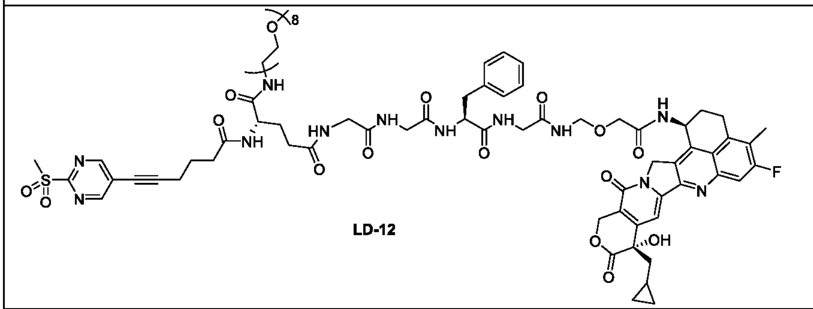

LD-12

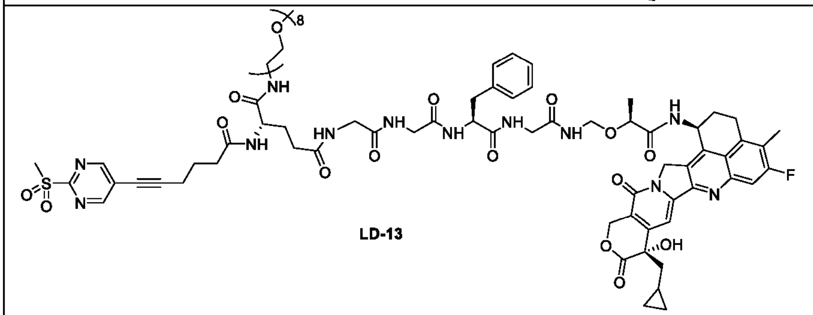

LD-13

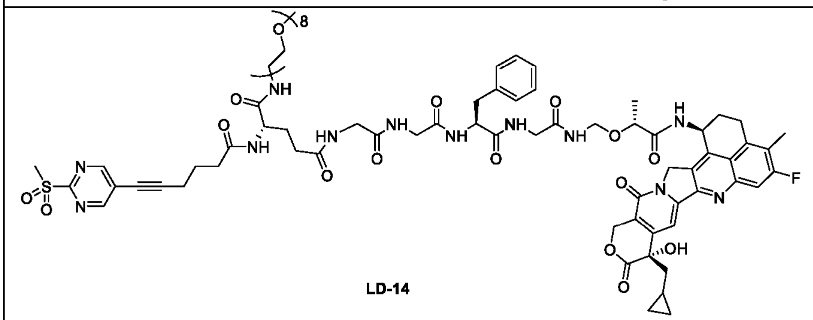

LD-14

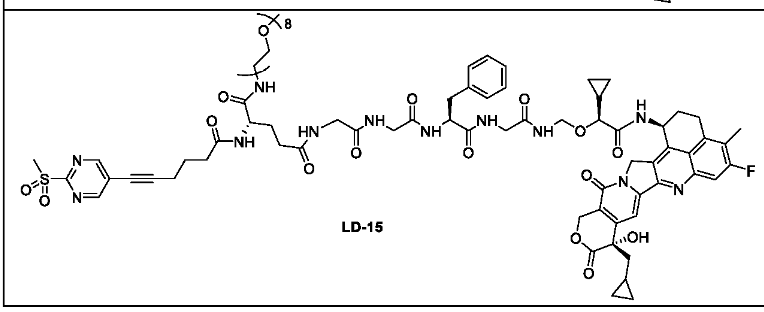

LD-15

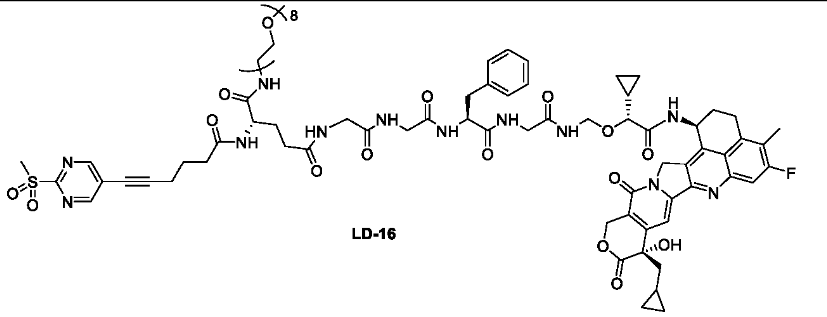

LD-16

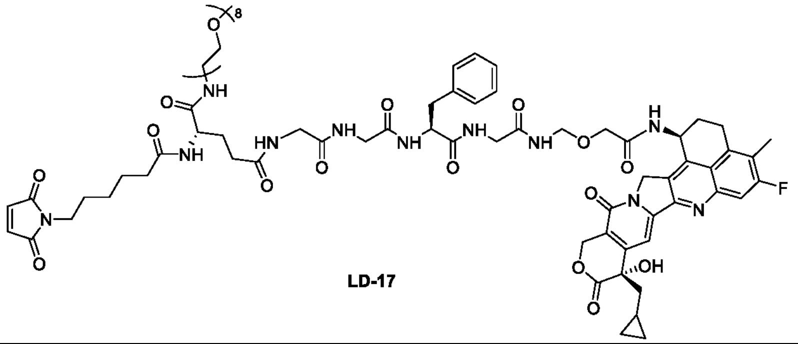

LD-17

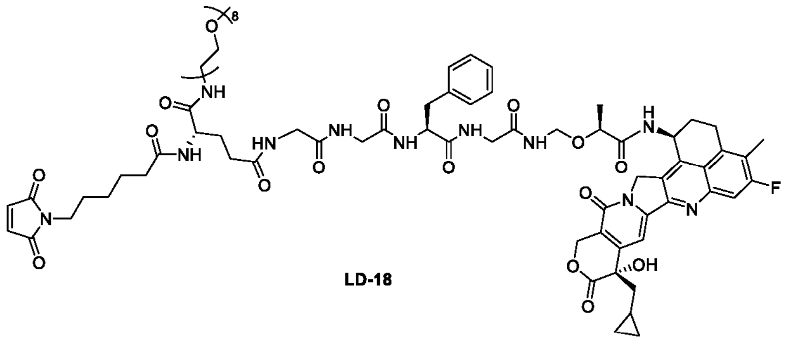

LD-18

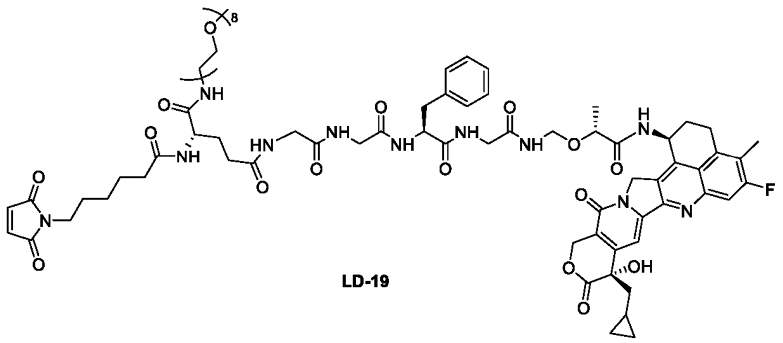

LD-19

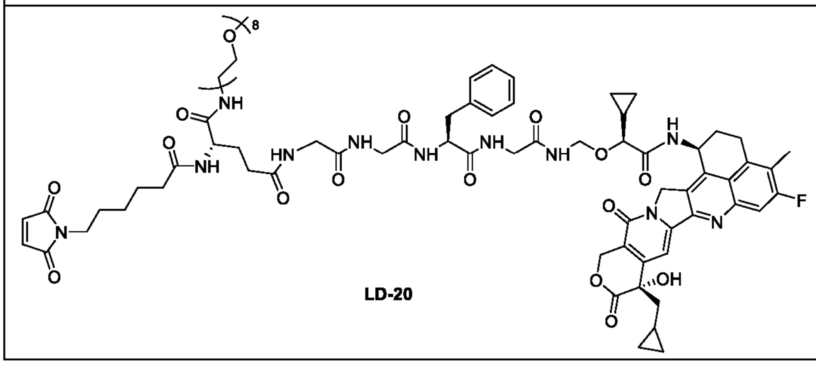

LD-20

| |
|---|
| 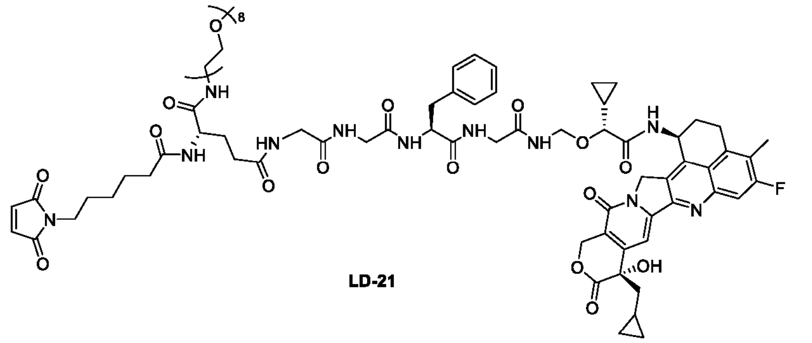 LD-21 |
| 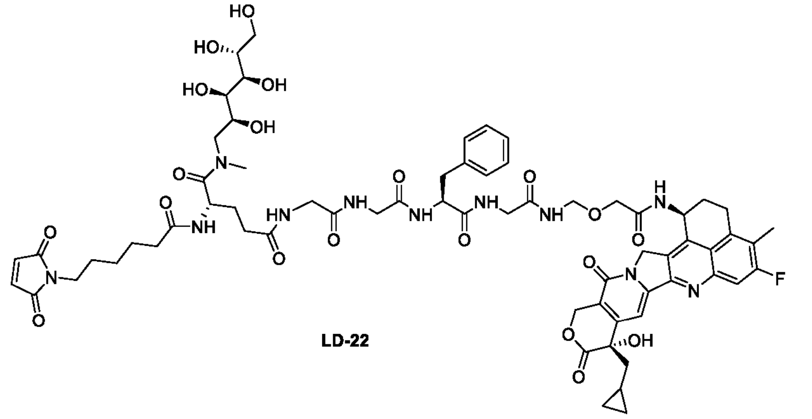 LD-22 |
| 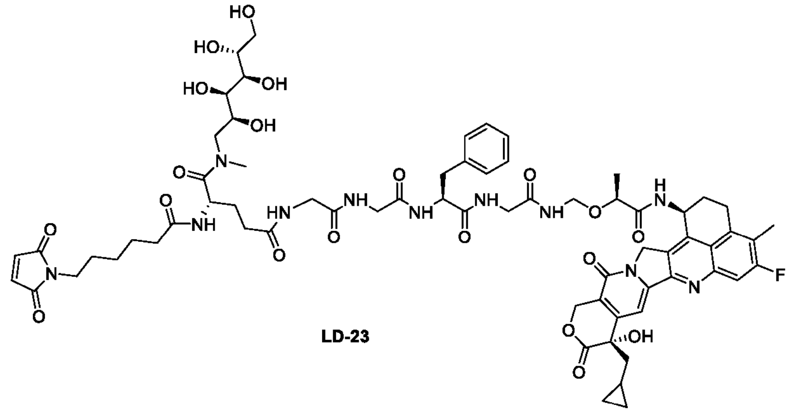 LD-23 |
| 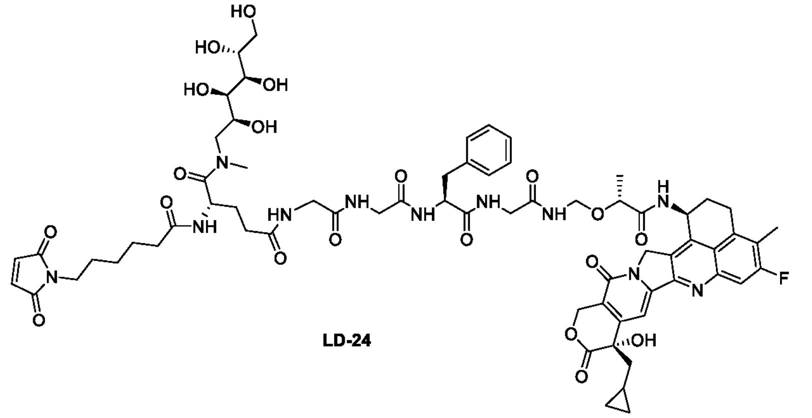 LD-24 |

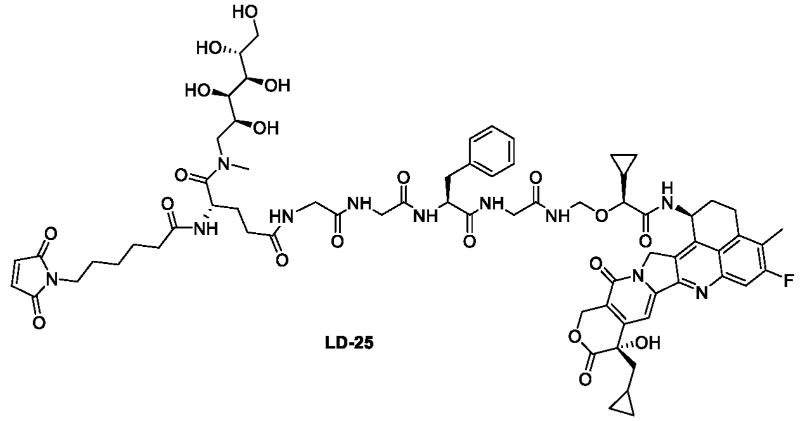

LD-25

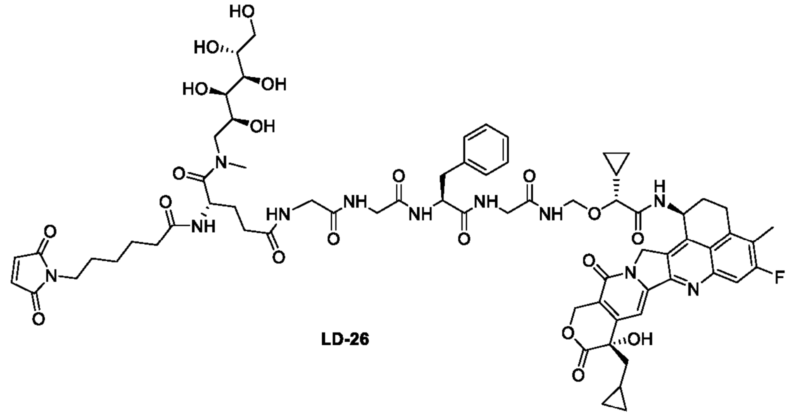

LD-26

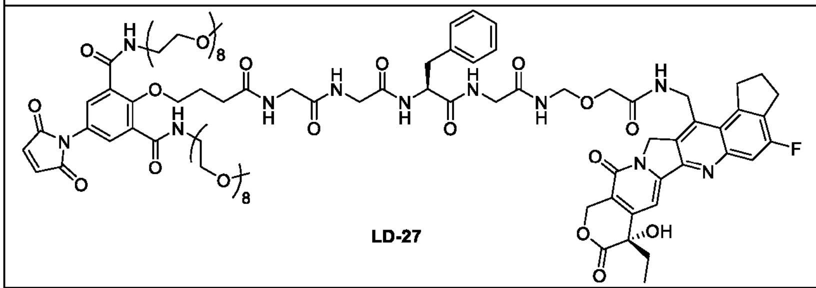

LD-27

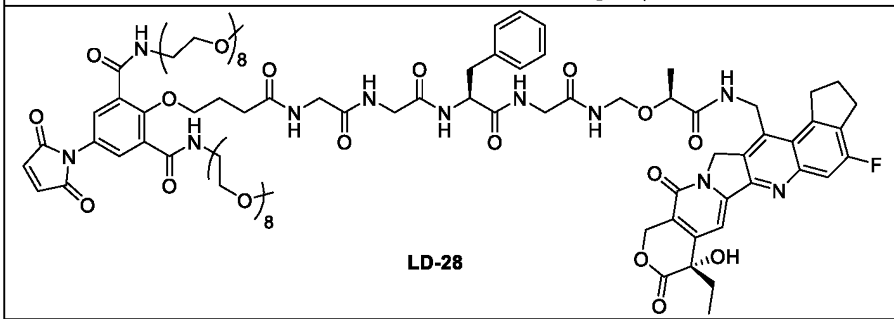

LD-28

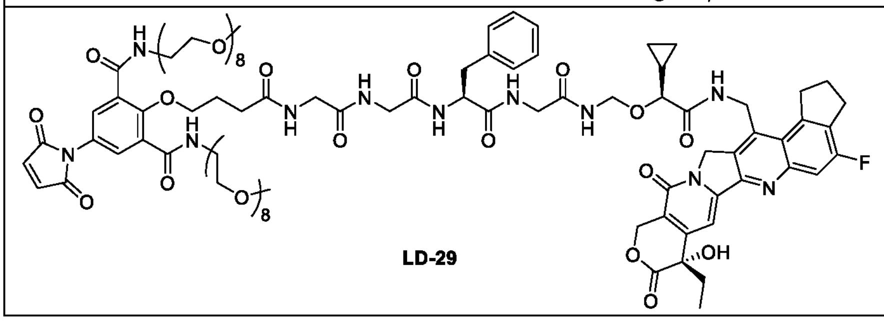

LD-29

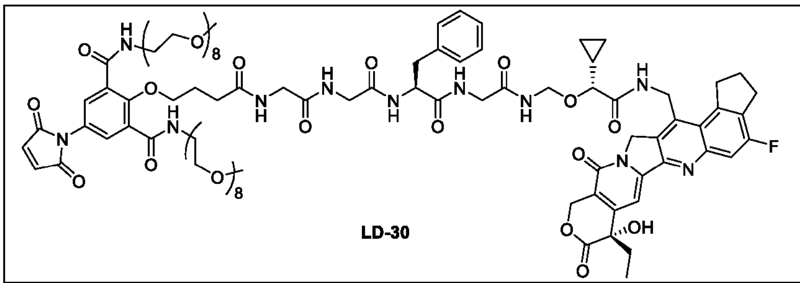

LD-30

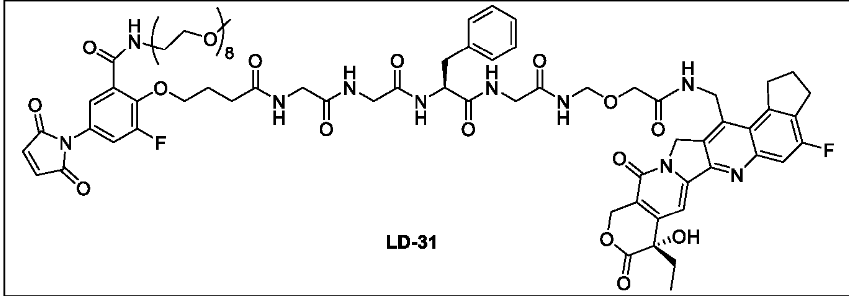

LD-31

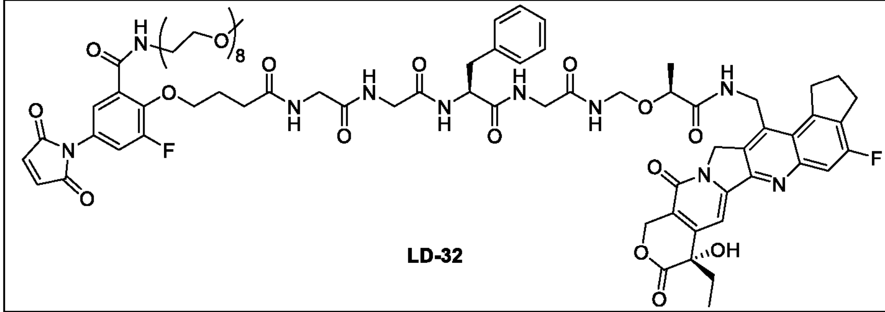

LD-32

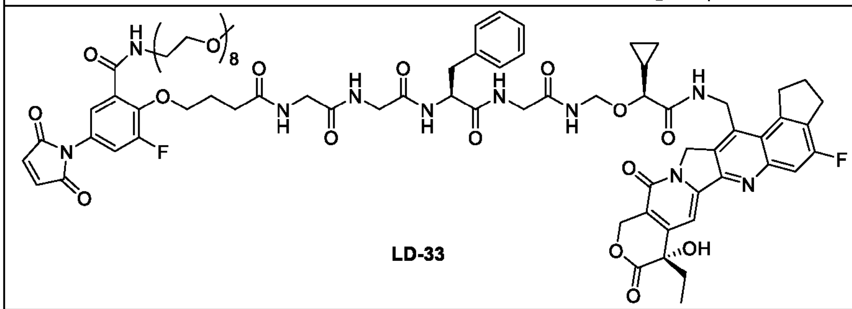

LD-33

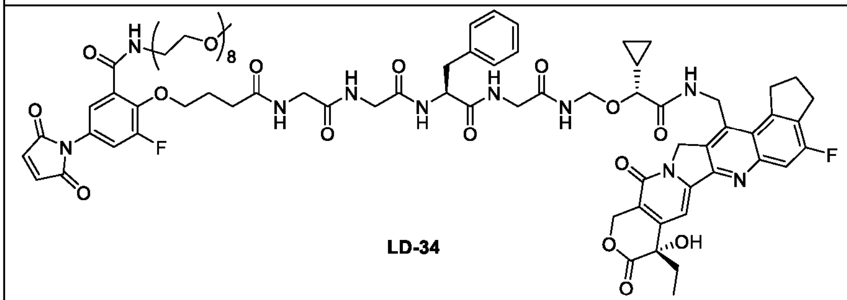

LD-34

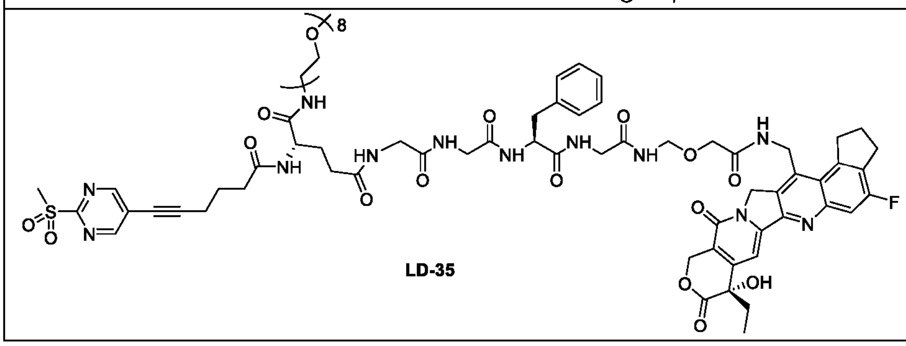

LD-35

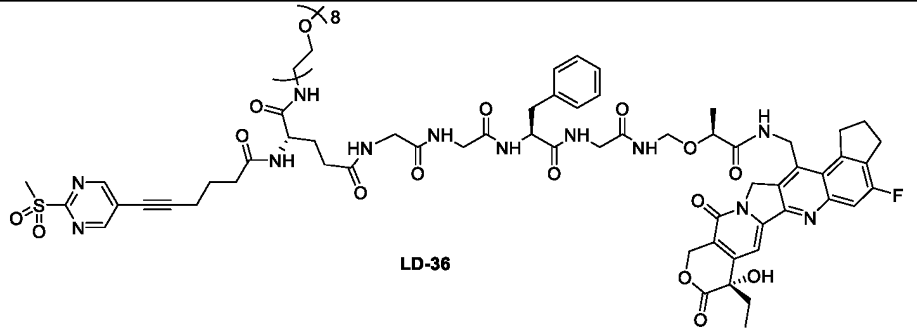

LD-36

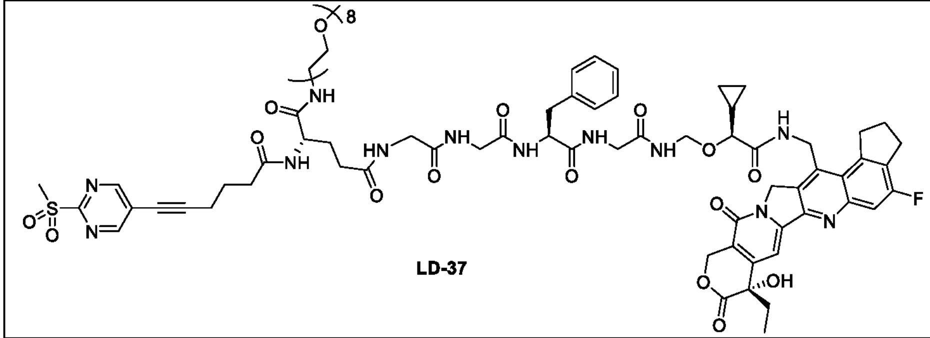

LD-37

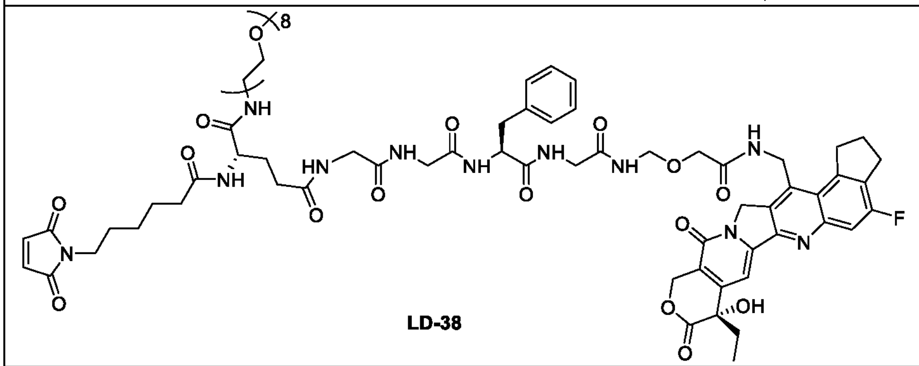

LD-38

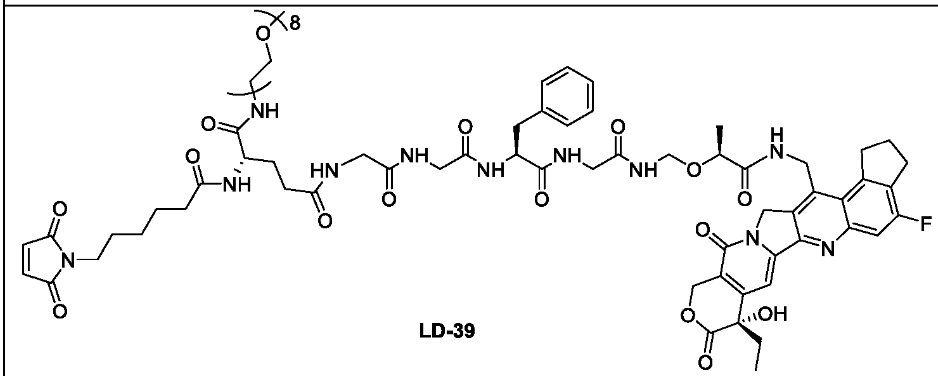

LD-39

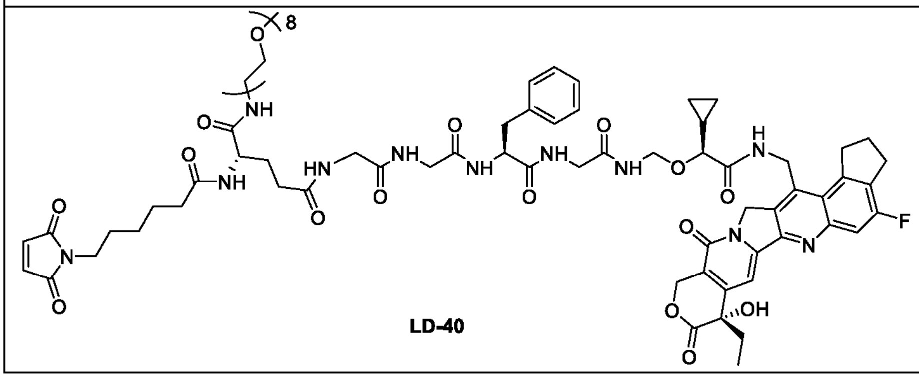

LD-40

| |
|---|
| 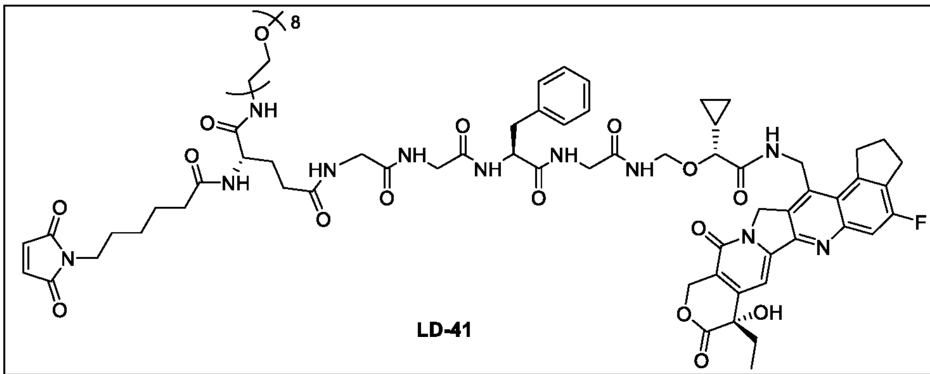 LD-41 |
| 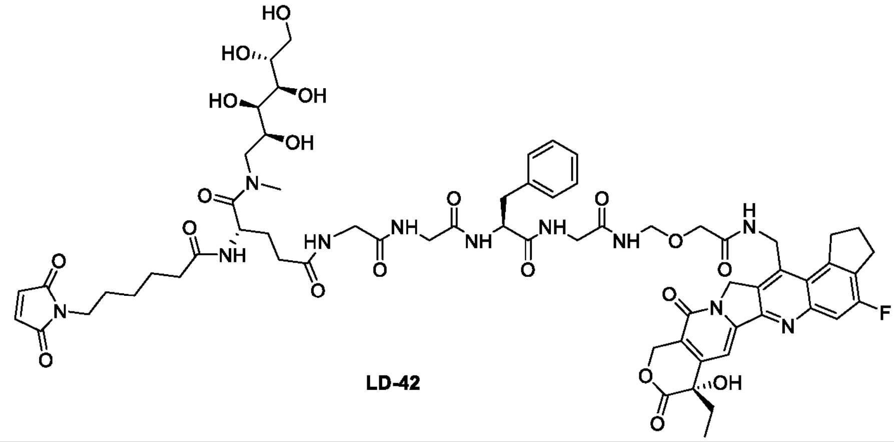 LD-42 |
| 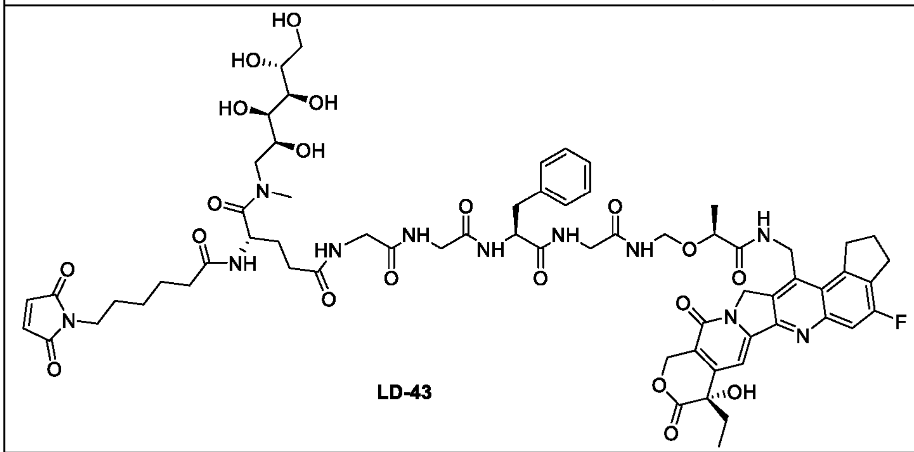 LD-43 |
| 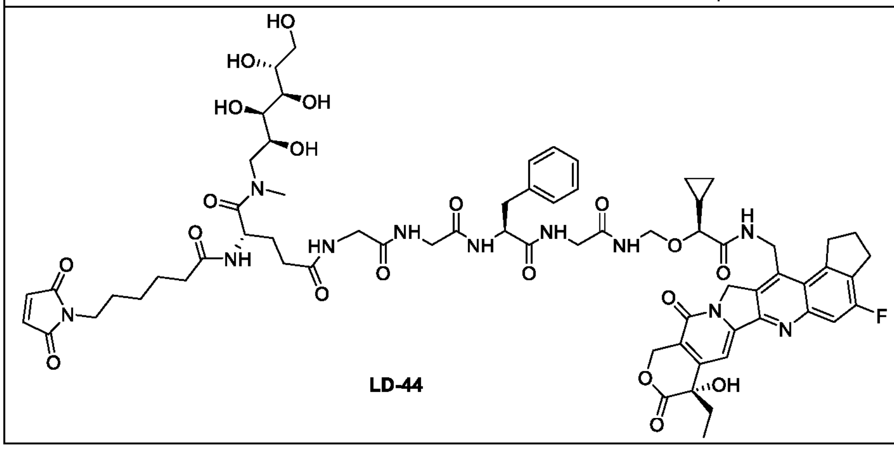 LD-44 |

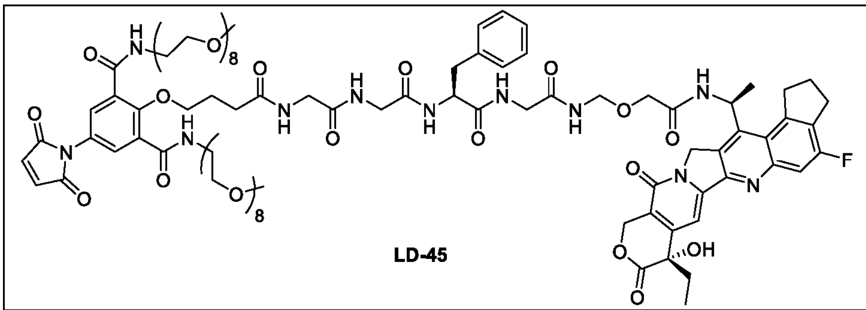

LD-45

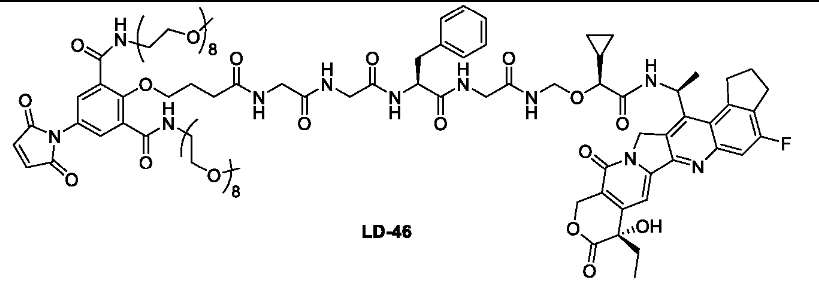

LD-46

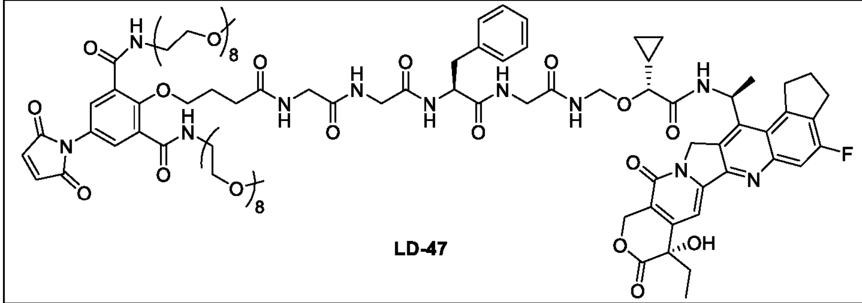

LD-47

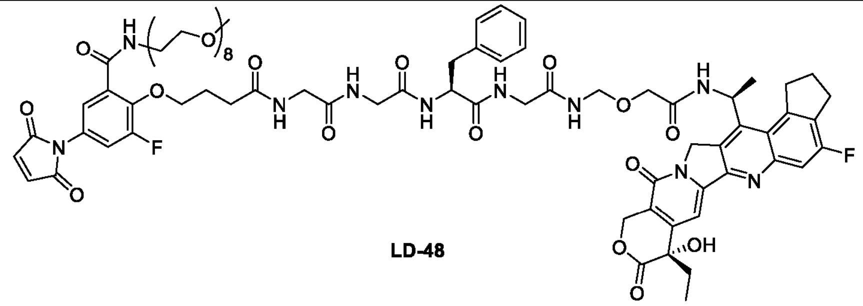

LD-48

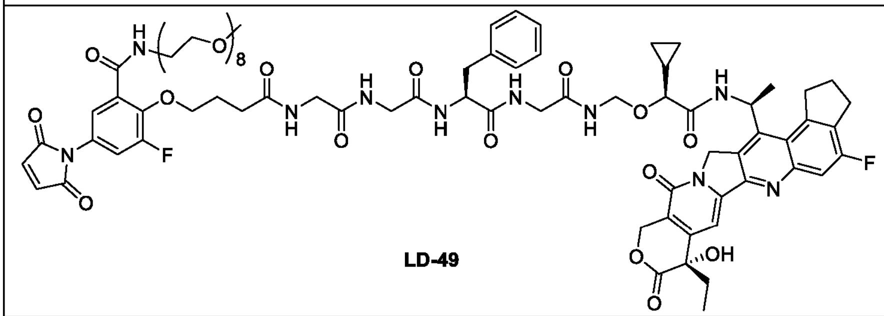

LD-49

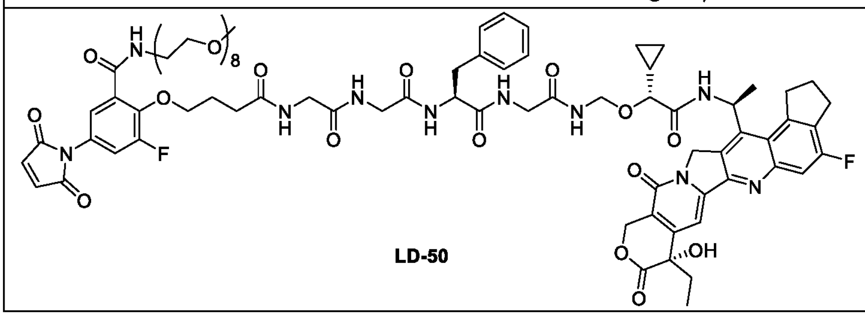

LD-50

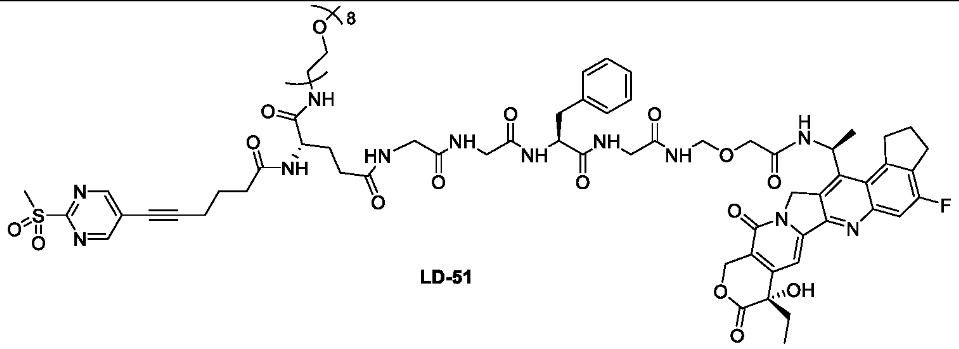

LD-51

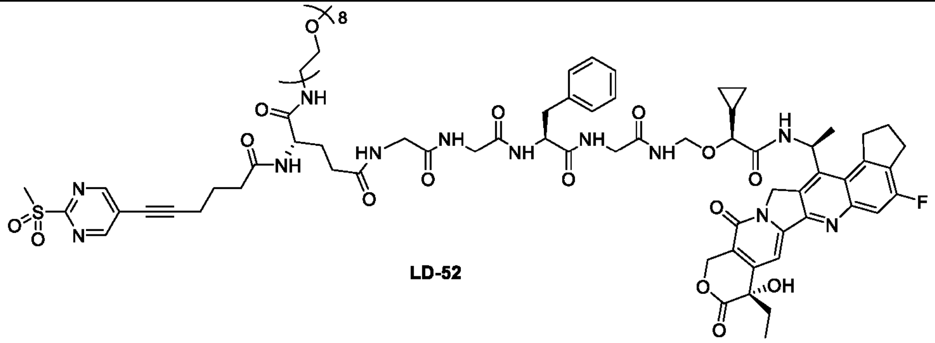

LD-52

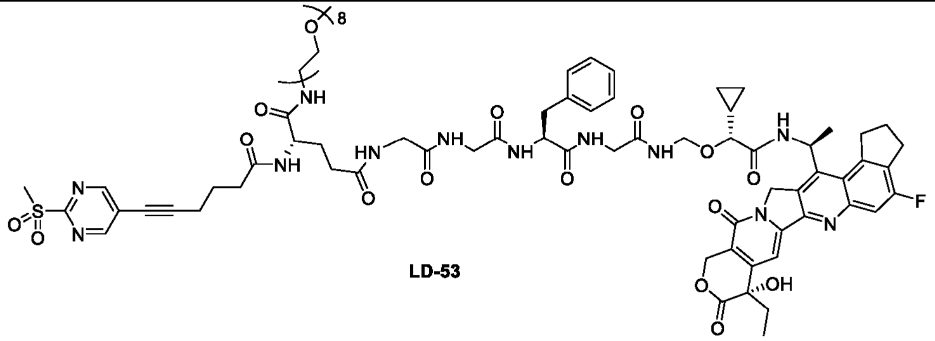

LD-53

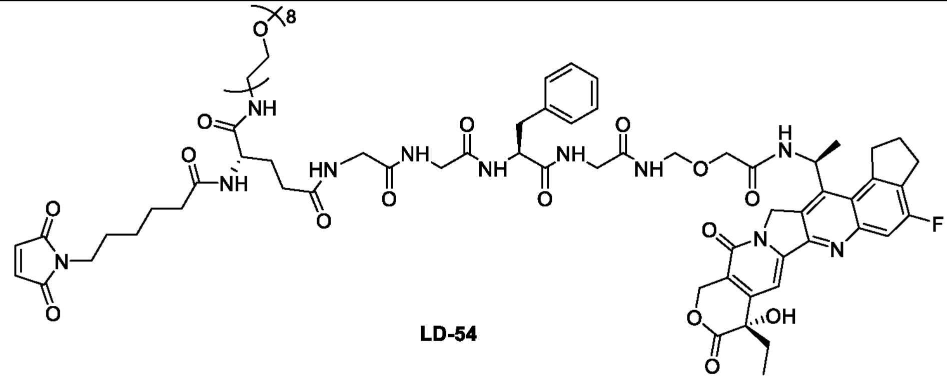

LD-54

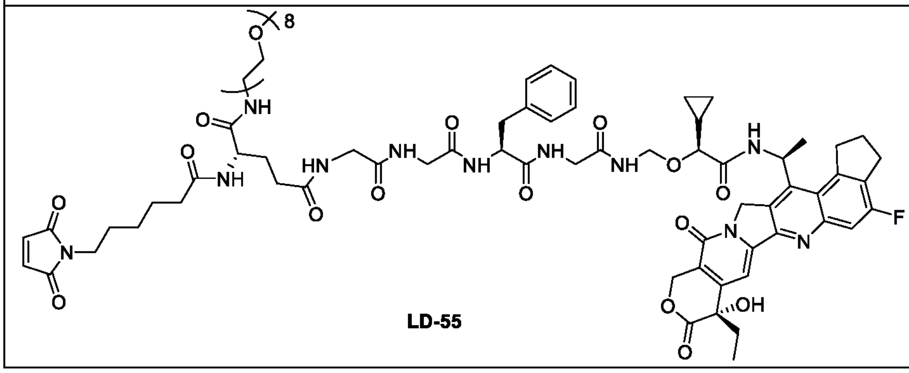

LD-55

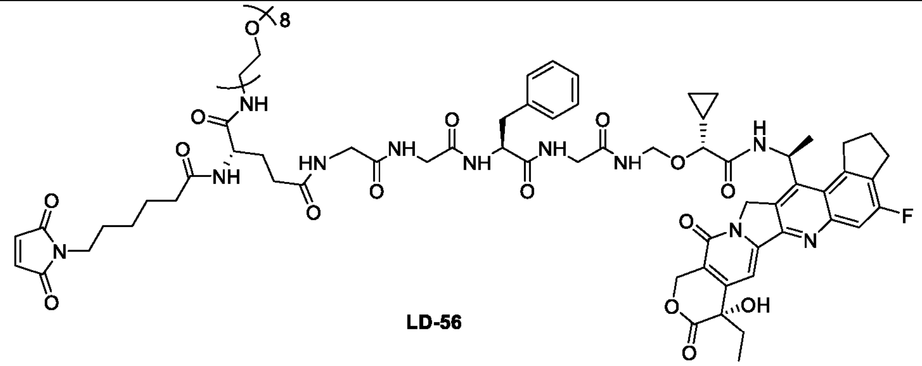

LD-56

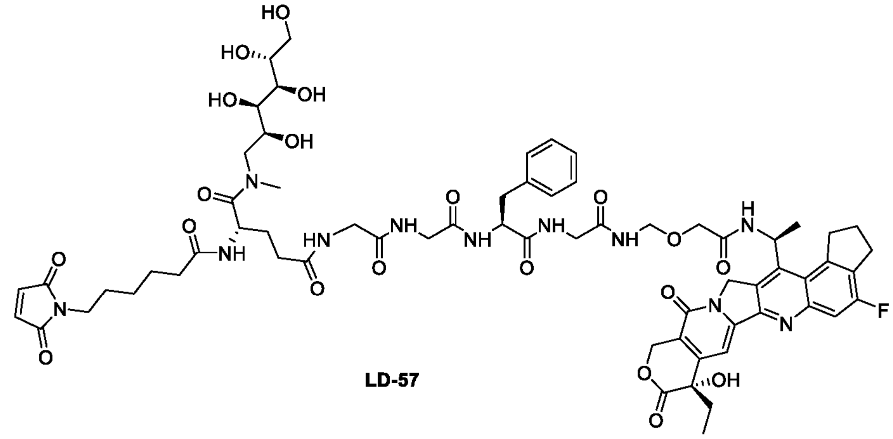

LD-57

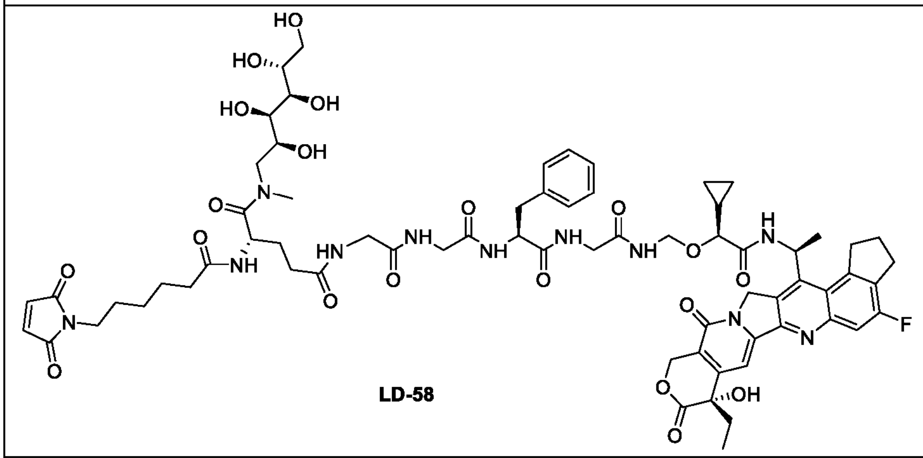

LD-58

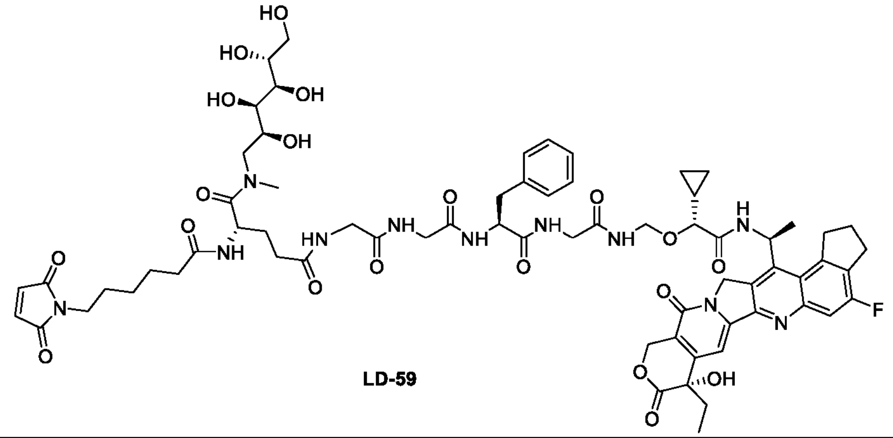

LD-59

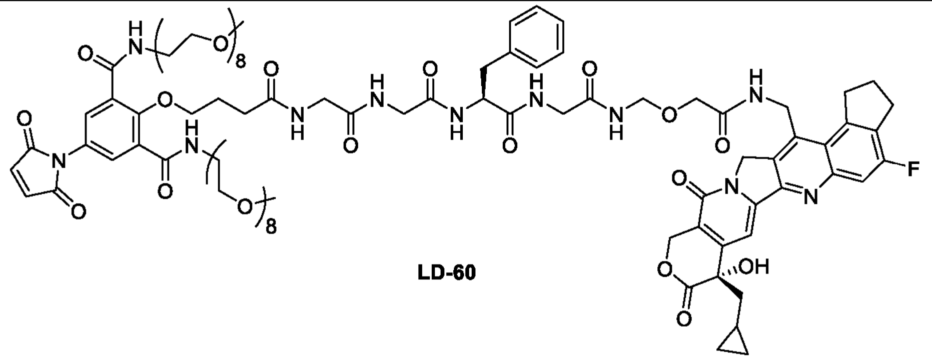

LD-60

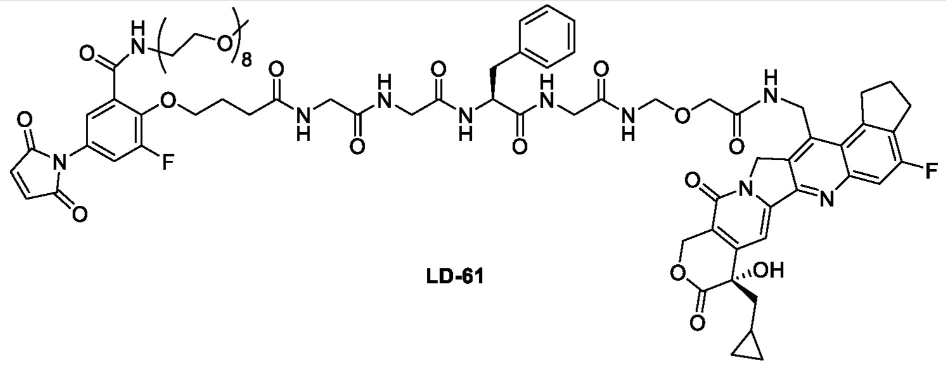

LD-61

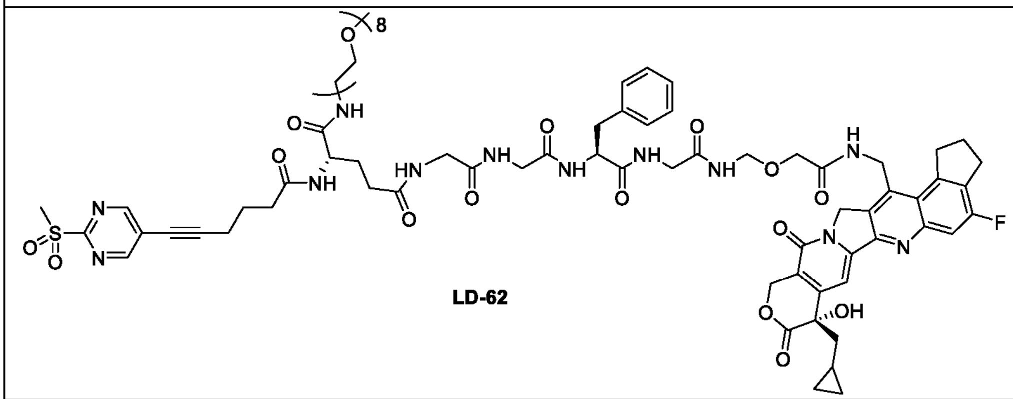

LD-62

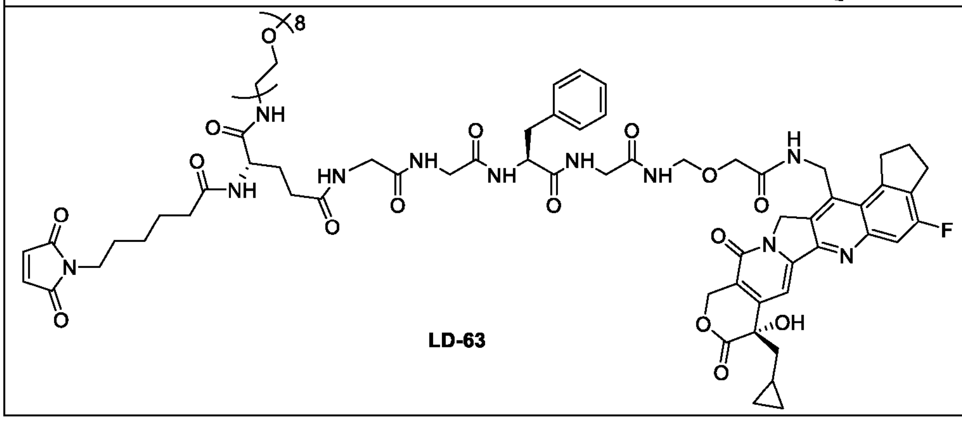

LD-63

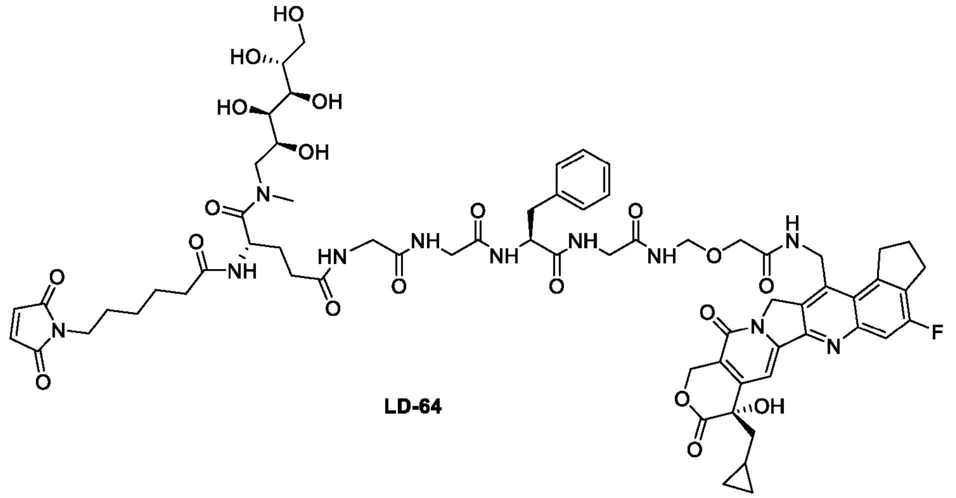

LD-64

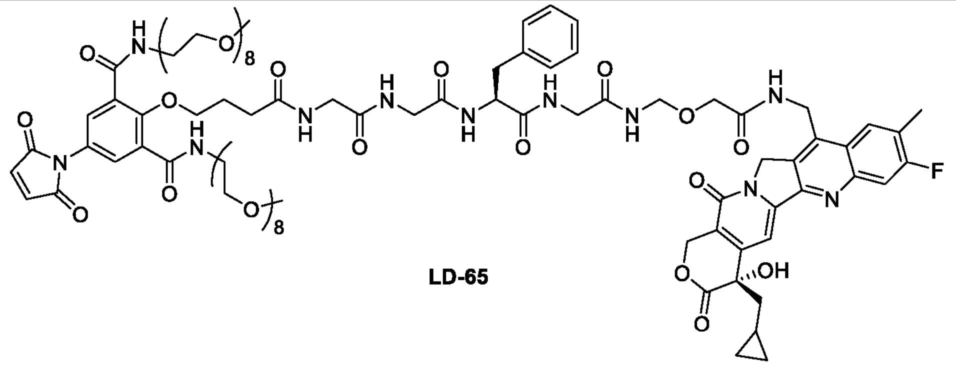

LD-65

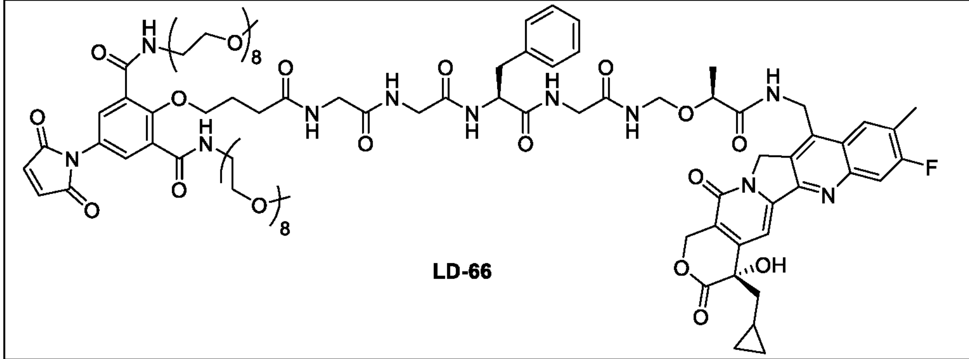

LD-66

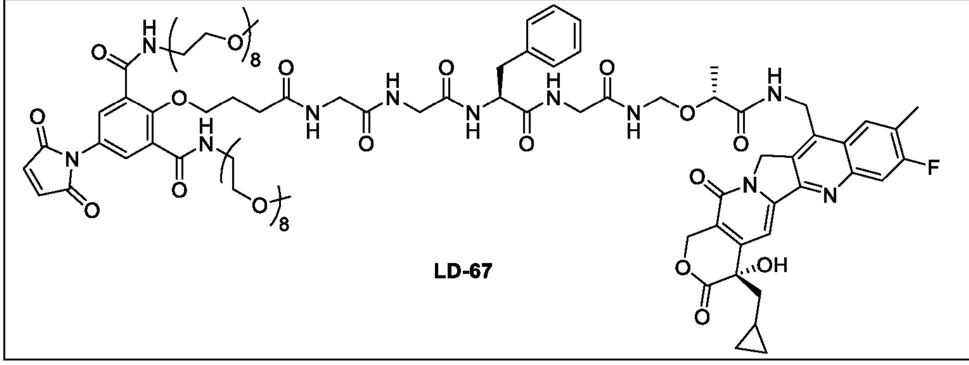

LD-67

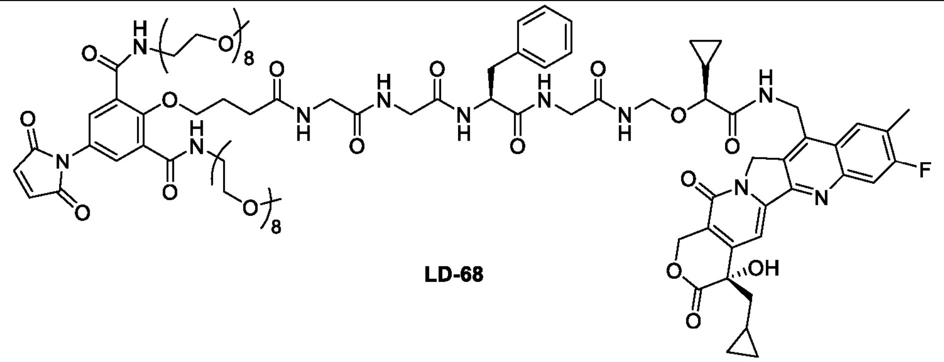

LD-68

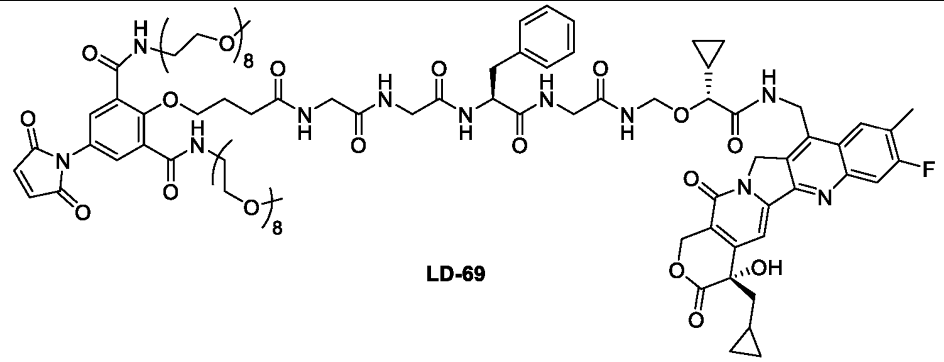

LD-69

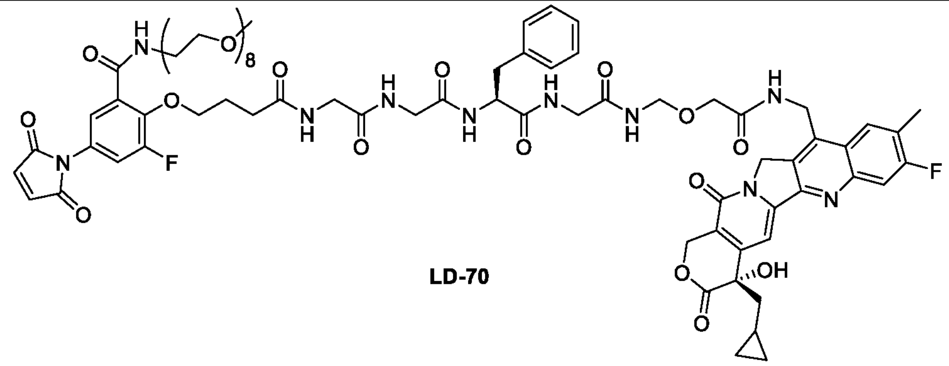

LD-70

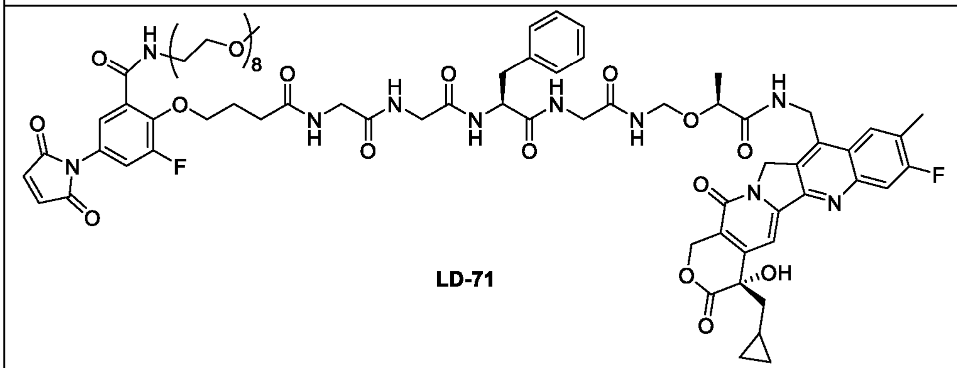

LD-71

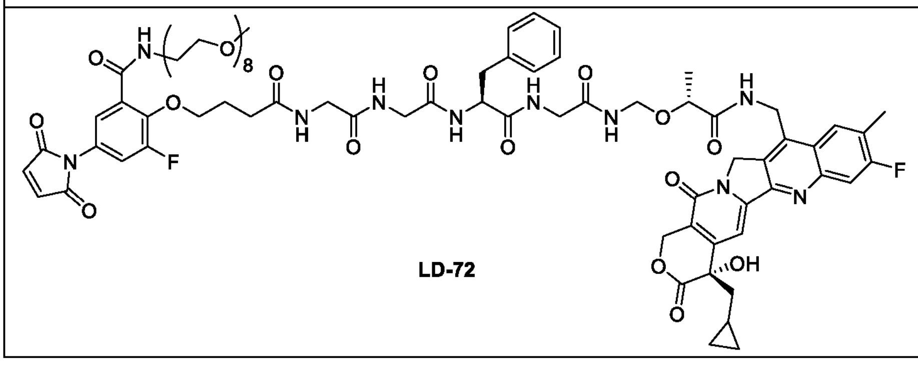

LD-72

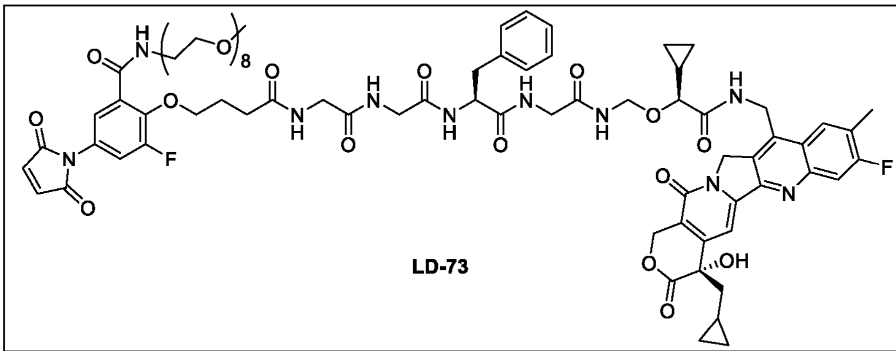

**LD-73**

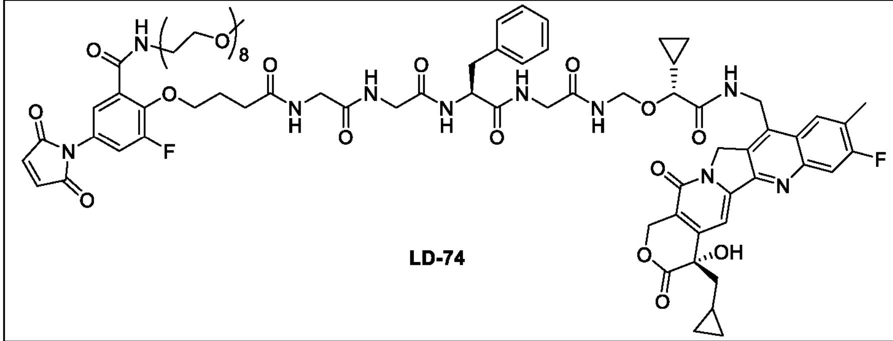

**LD-74**

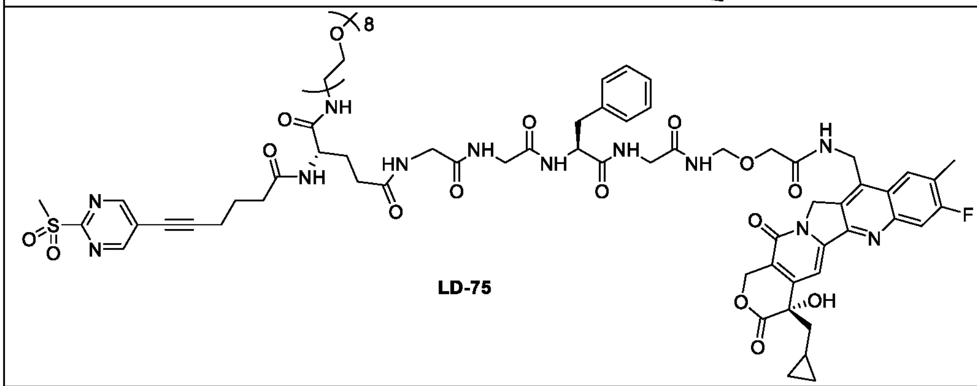

**LD-75**

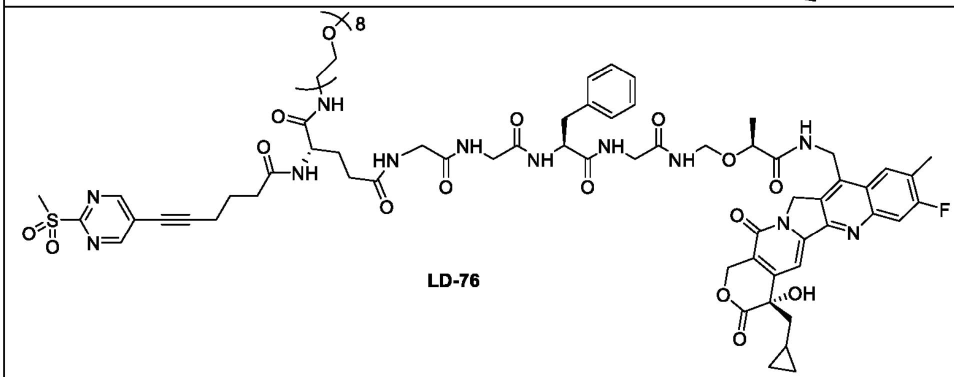

**LD-76**

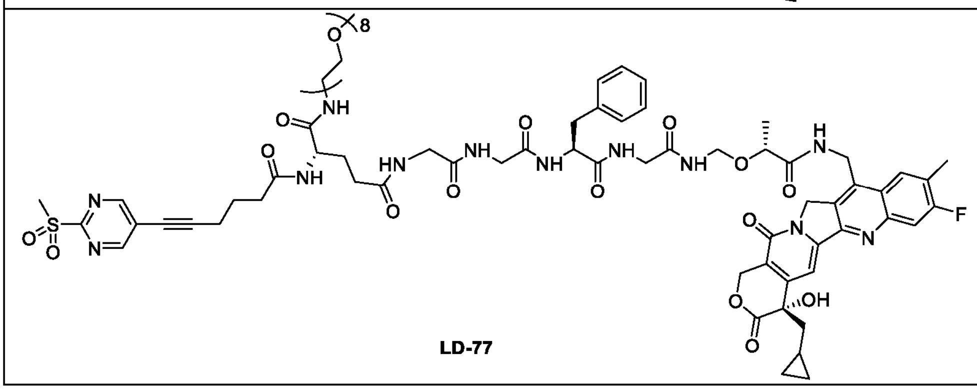

**LD-77**

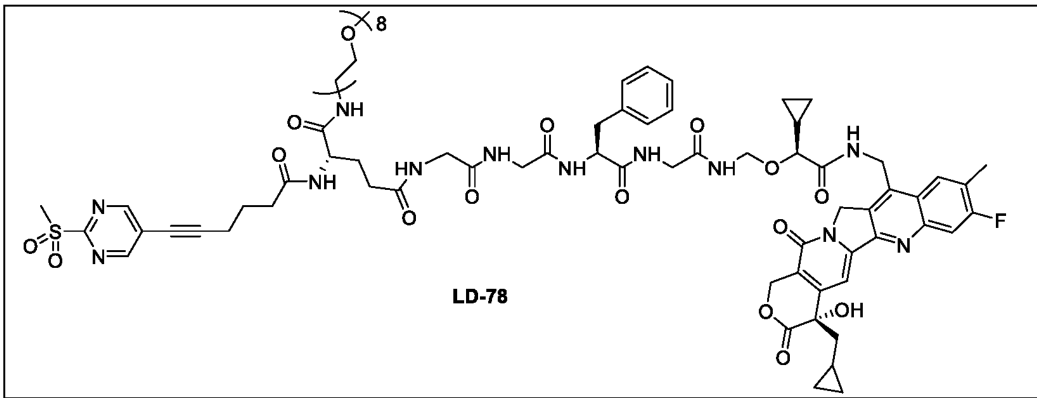

**LD-78**

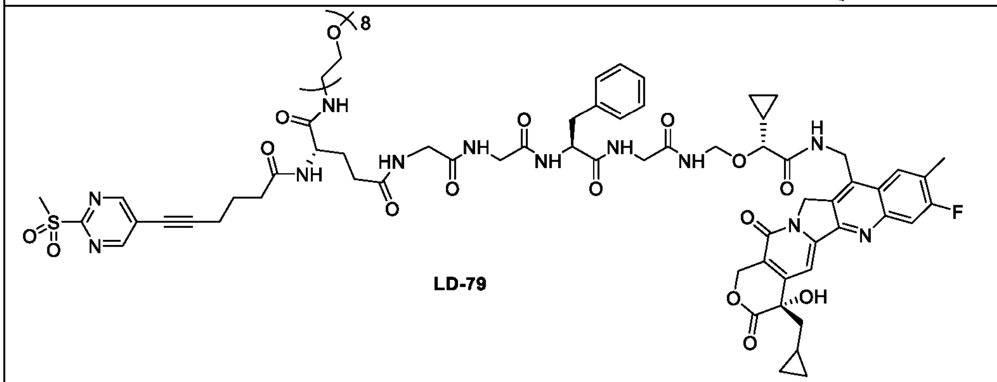

**LD-79**

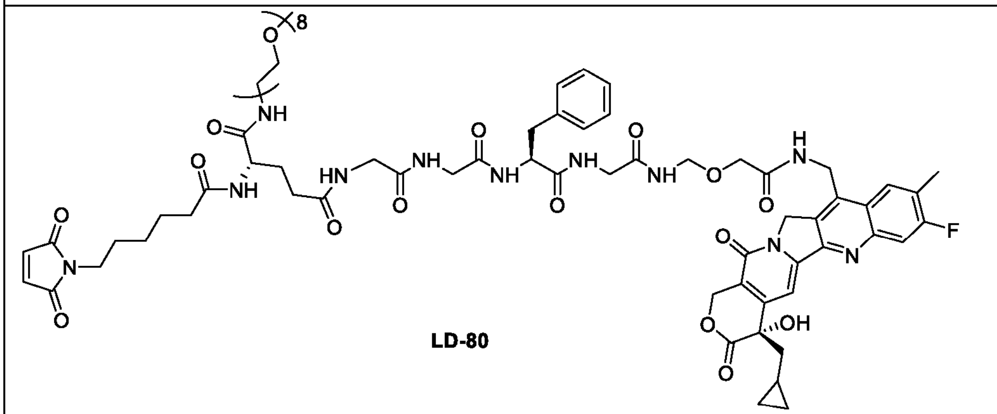

**LD-80**

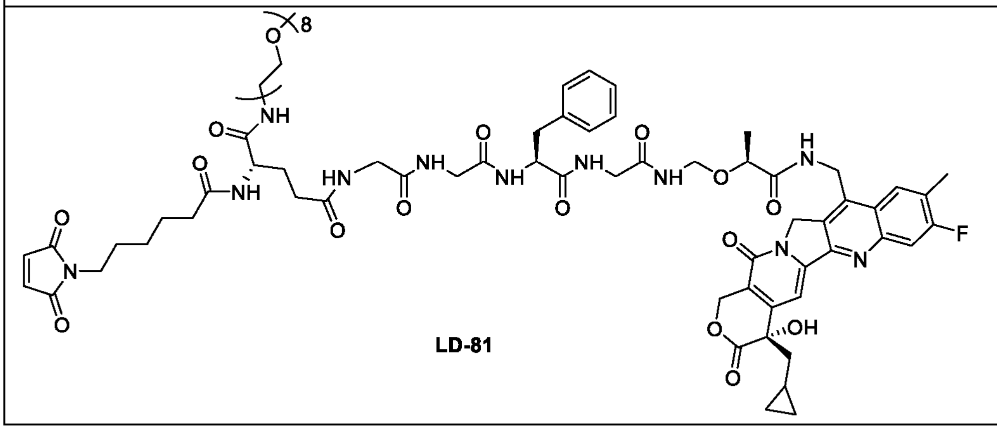

**LD-81**

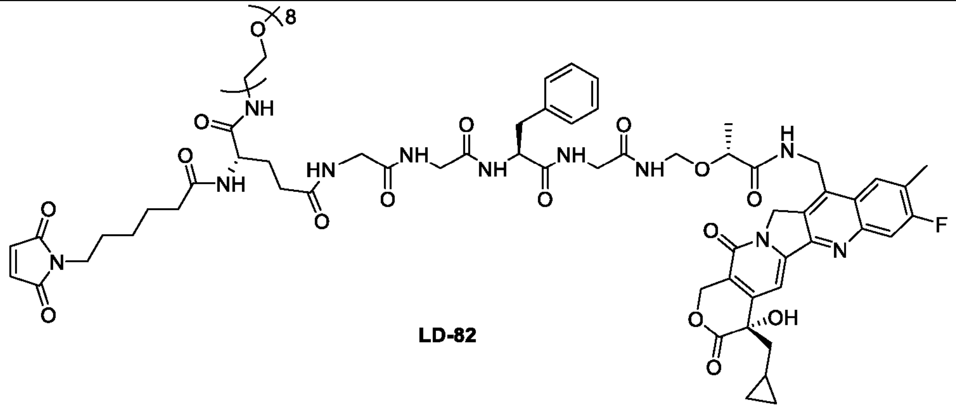

LD-82

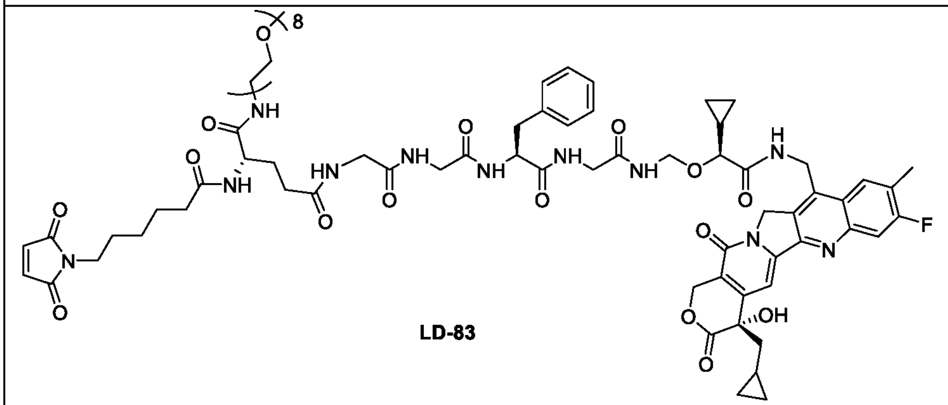

LD-83

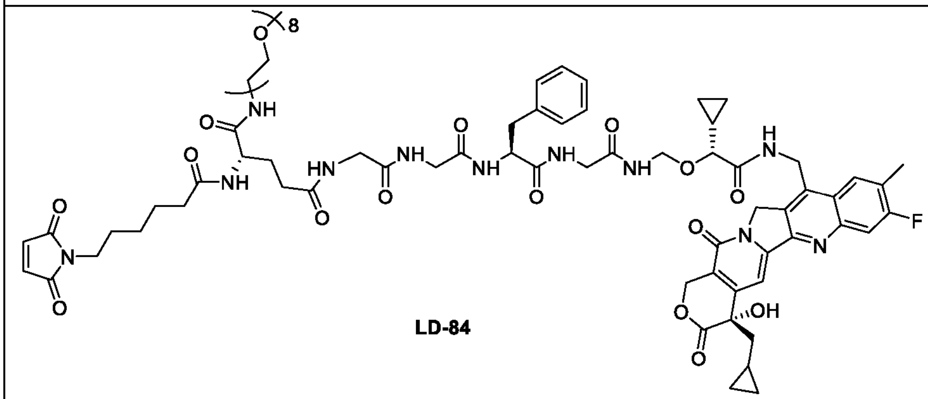

LD-84

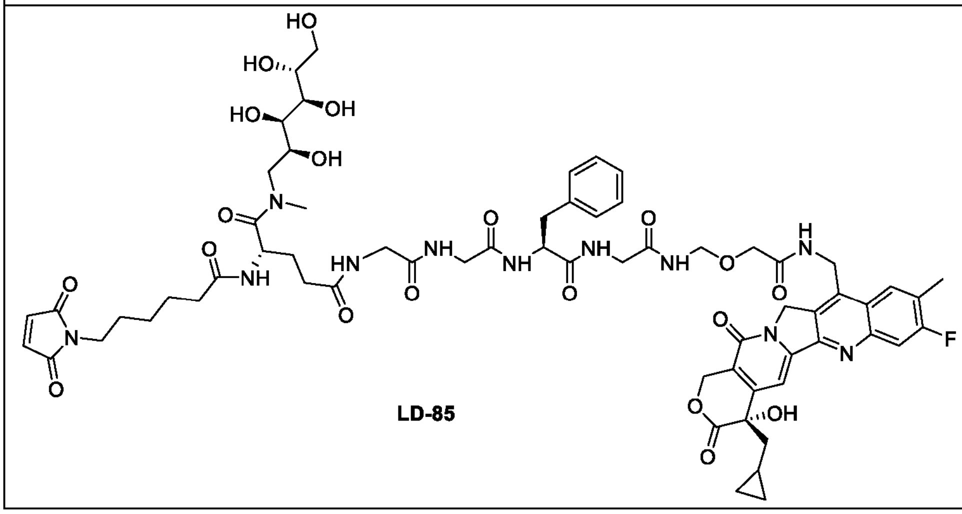

LD-85

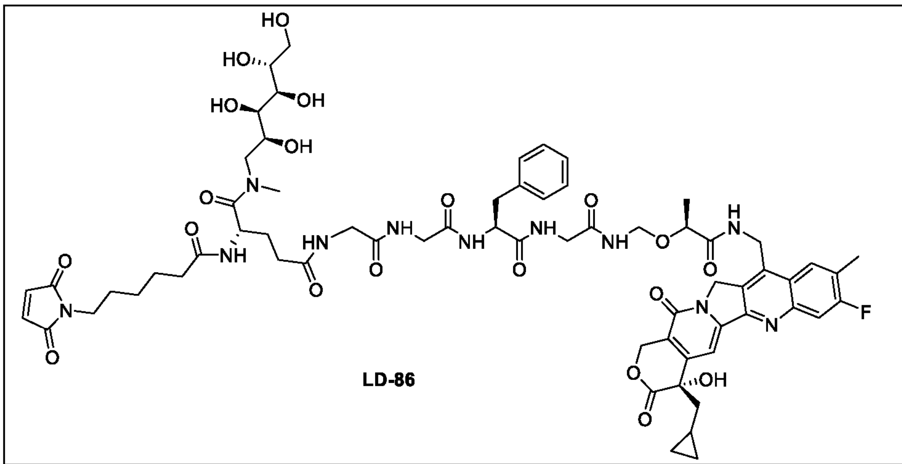

LD-86

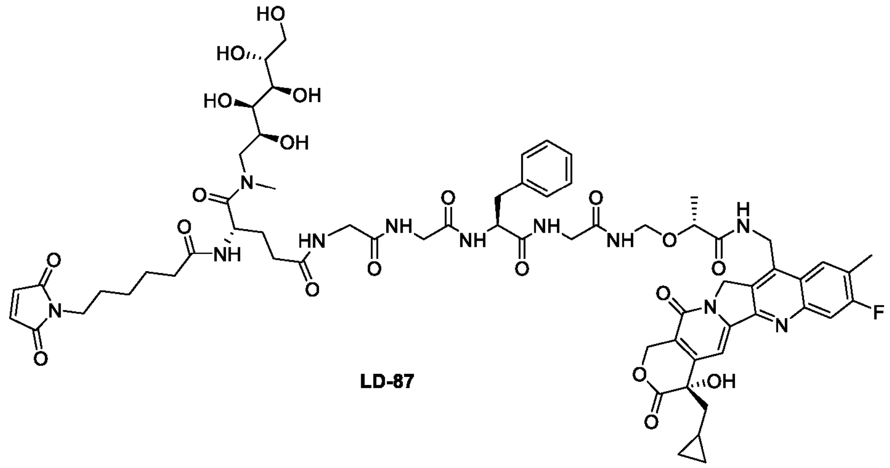

LD-87

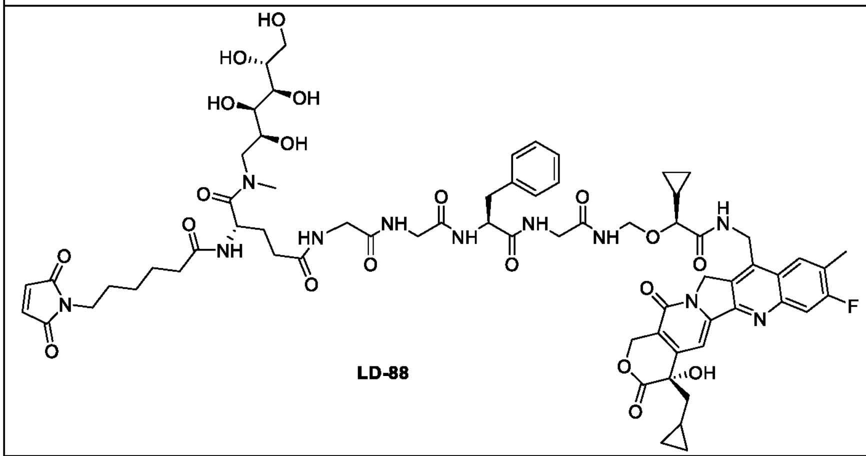

LD-88

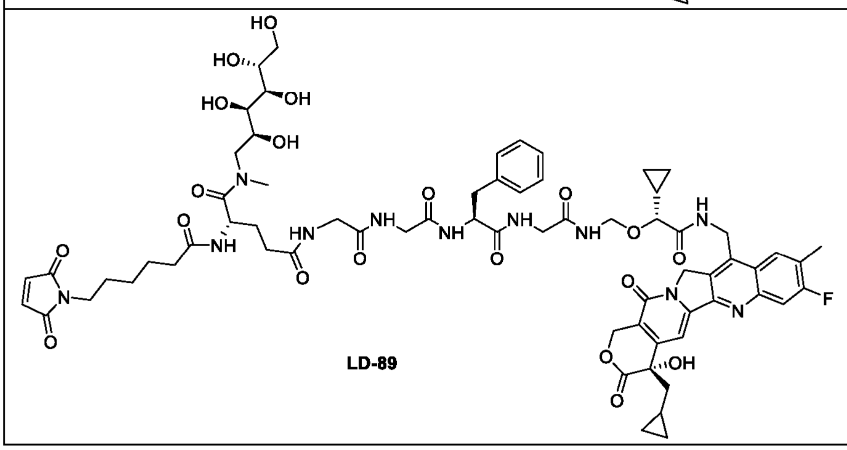

LD-89

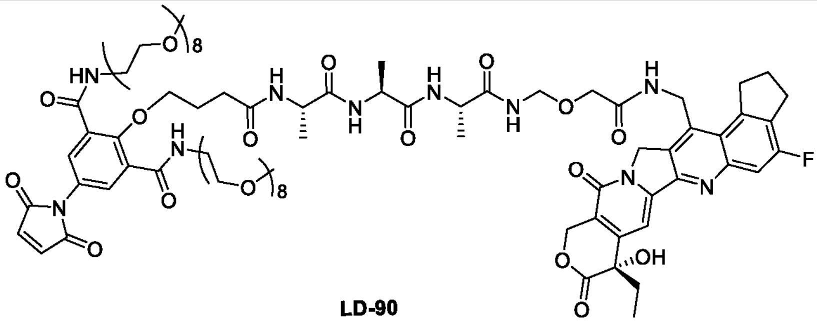

**LD-90**

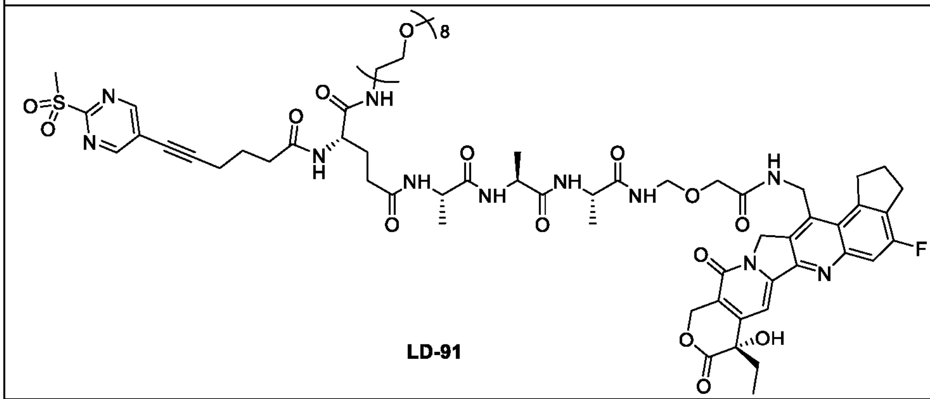

**LD-91**

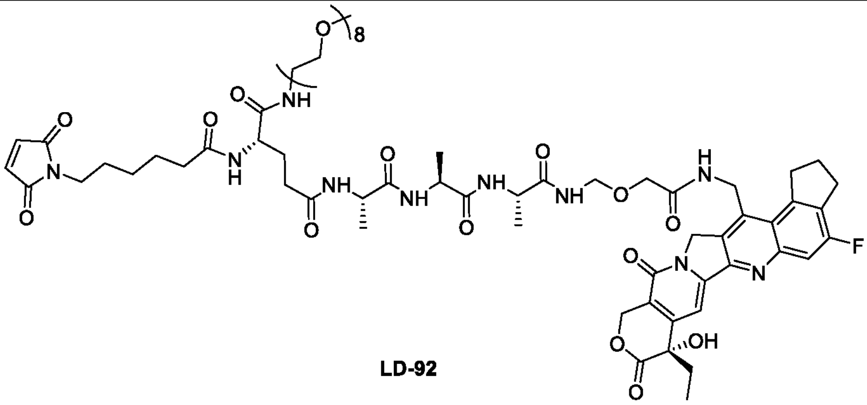

**LD-92**

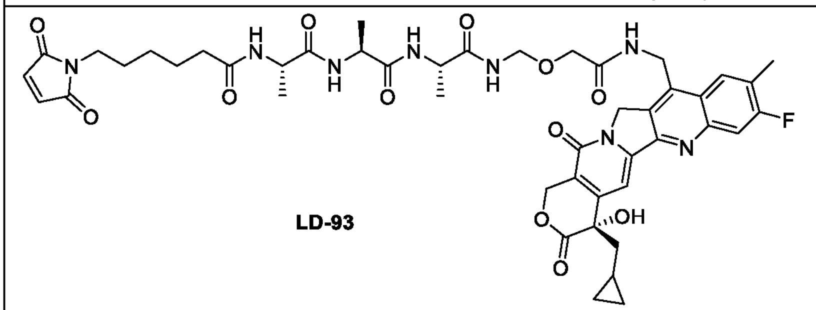

**LD-93**

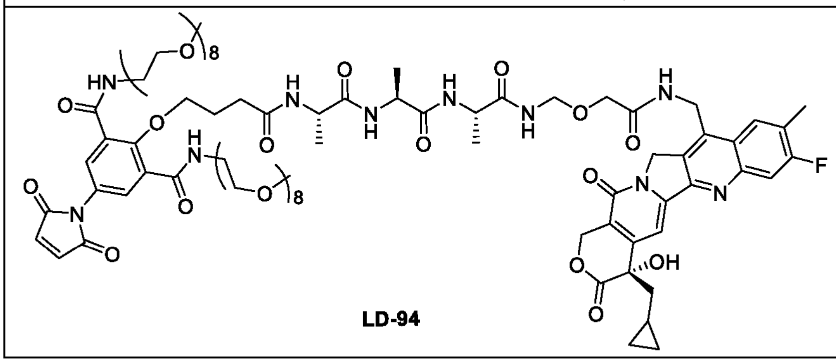

**LD-94**

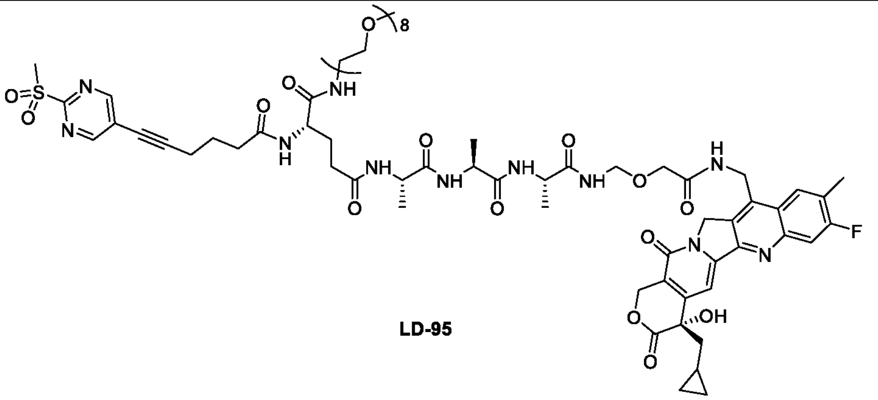

LD-95

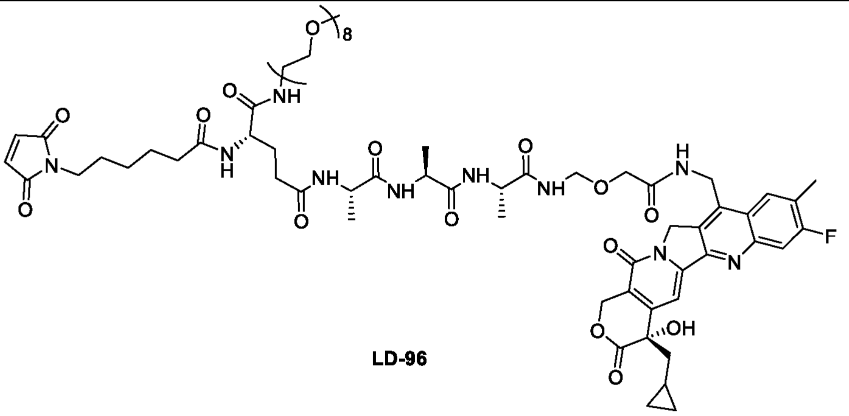

LD-96

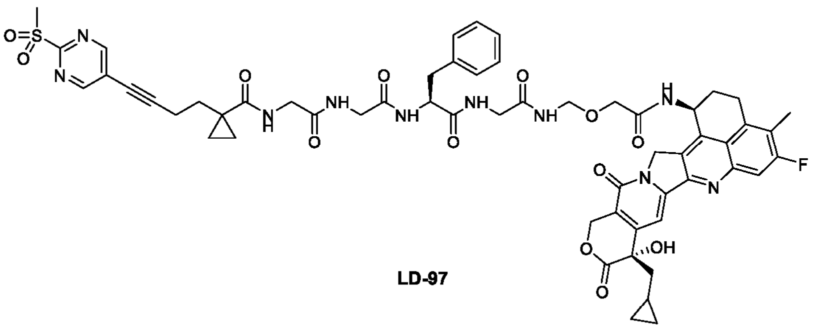

LD-97

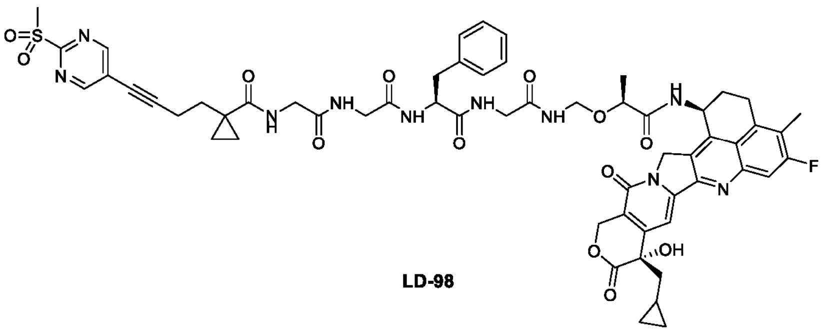

LD-98

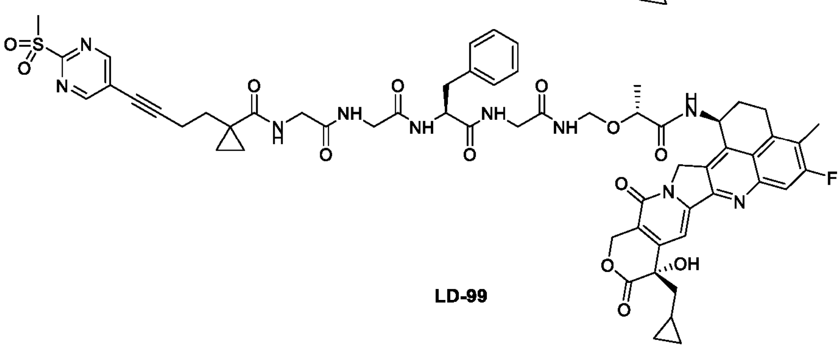

LD-99

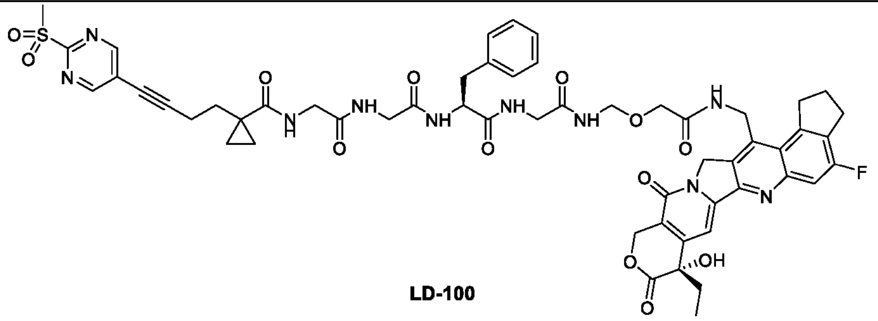

**LD-100**

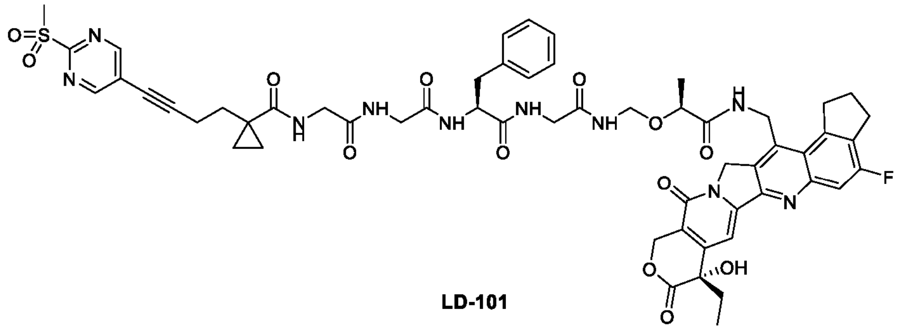

**LD-101**

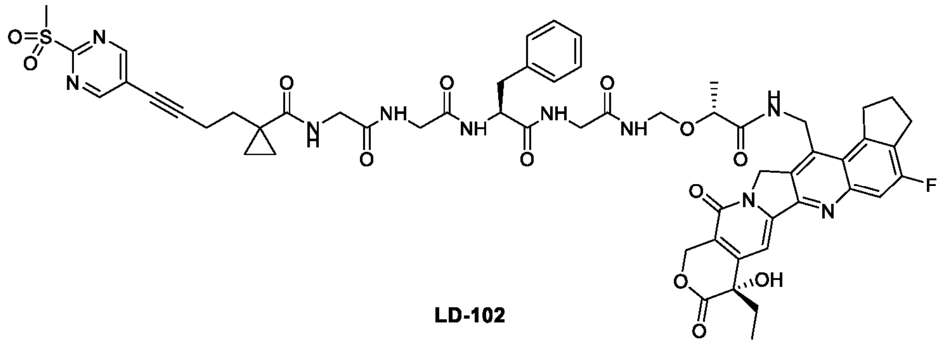

**LD-102**

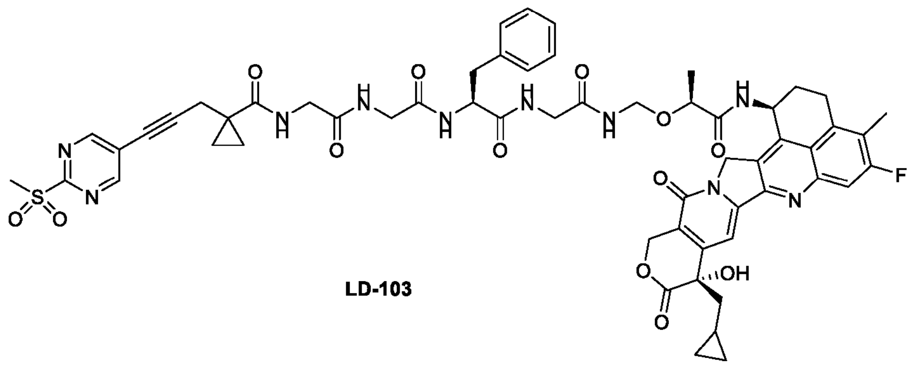

**LD-103**

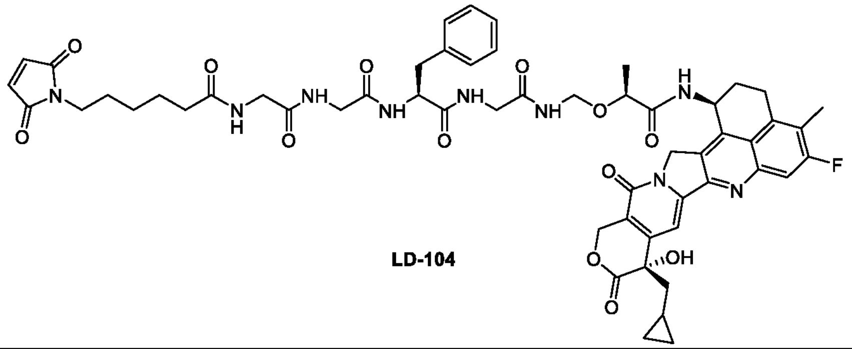

**LD-104**

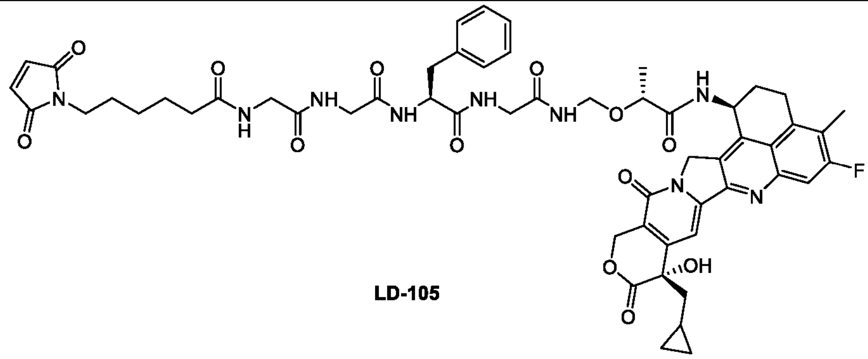

LD-105

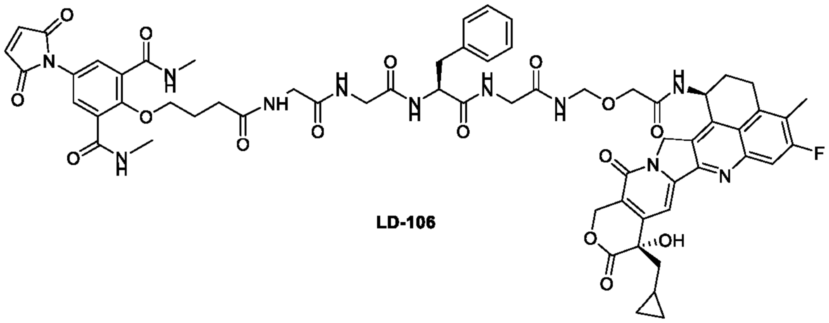

LD-106

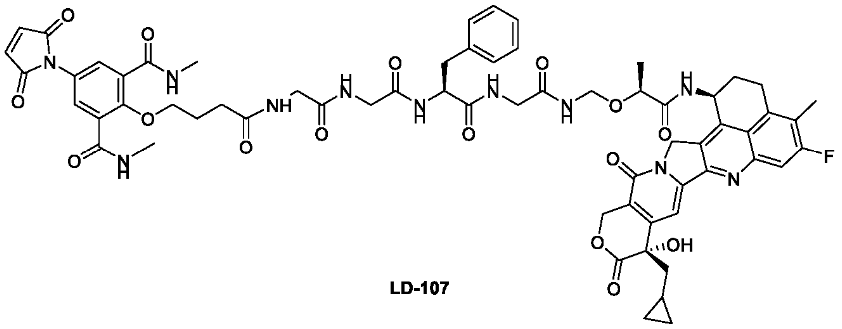

LD-107

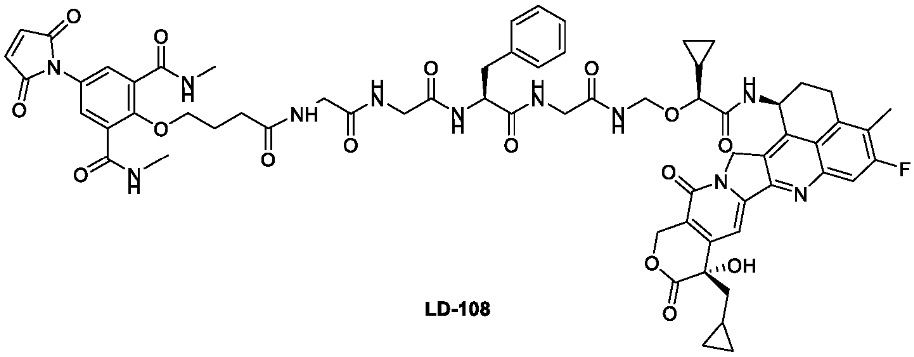

LD-108

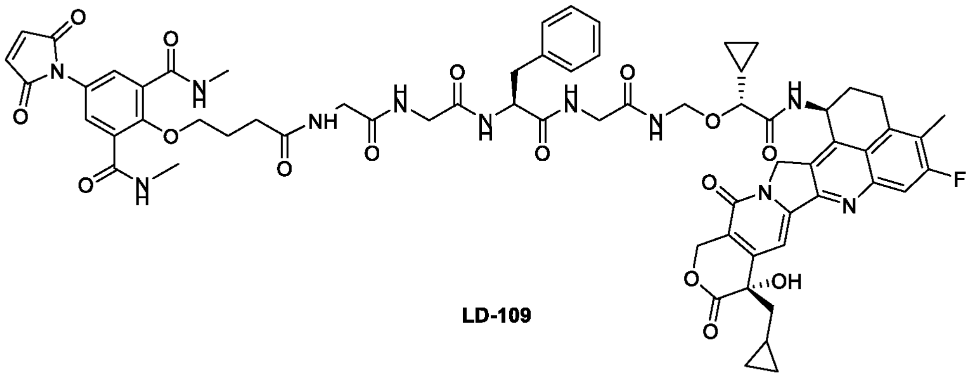

LD-109

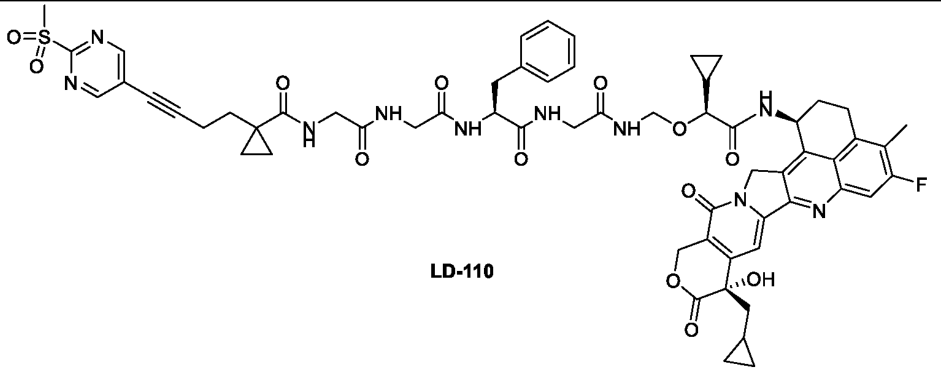

LD-110

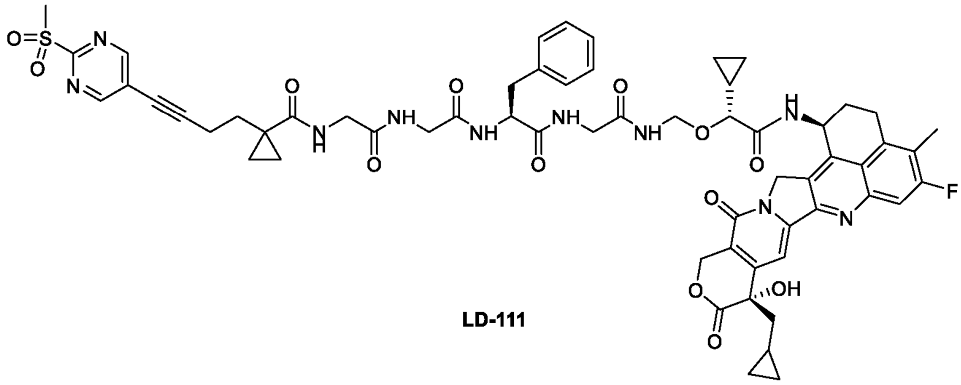

LD-111

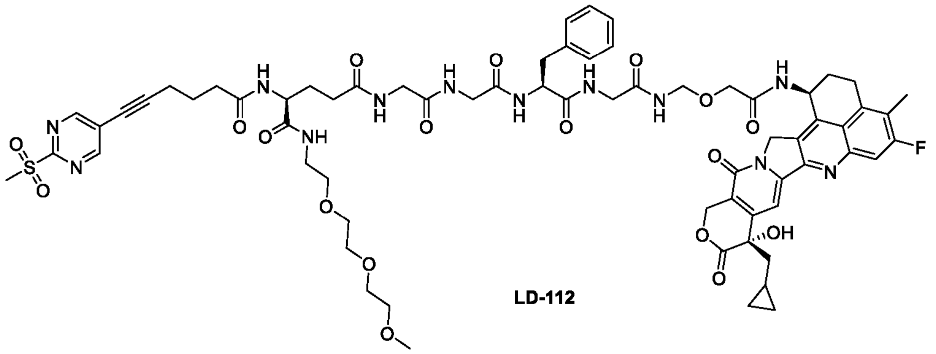

LD-112

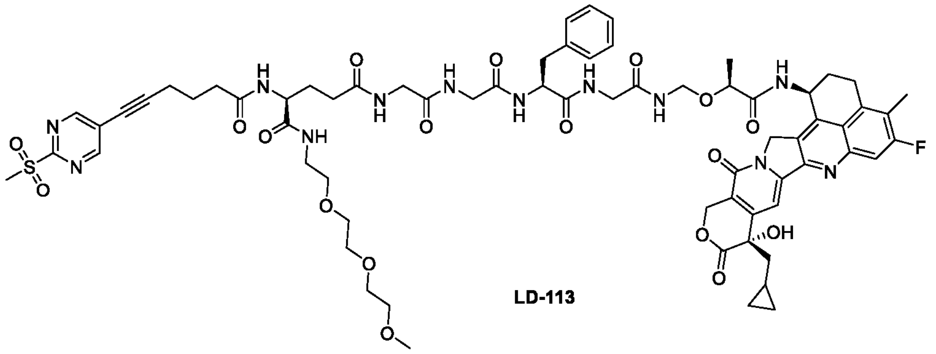

LD-113

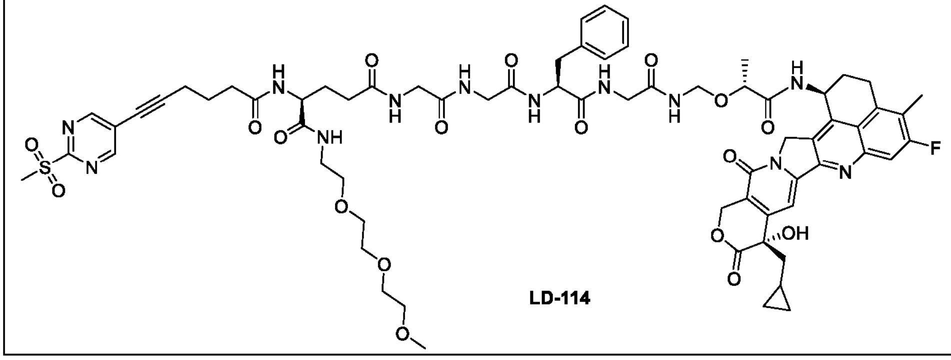

LD-114

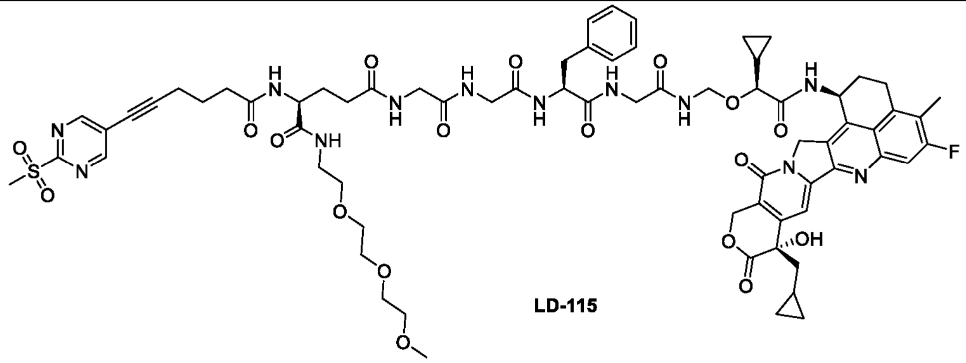

LD-115

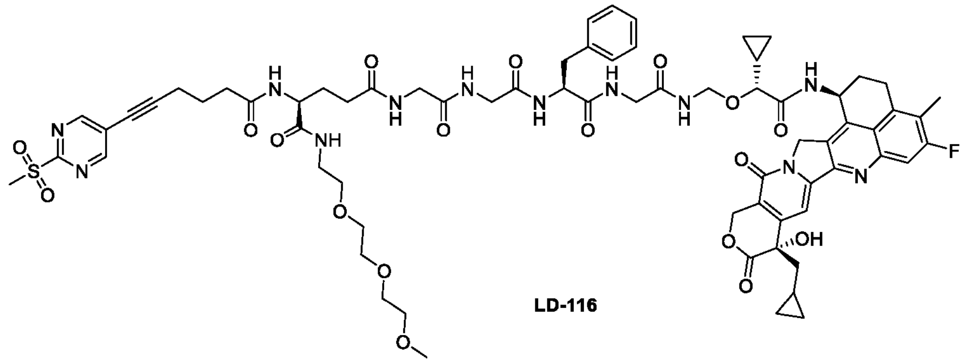

LD-116

According to the embodiments of the present disclosure, the example of the conjugate may be selected from one of the followings, wherein:
y represents the average number of small molecule drugs attached to each monoclonal antibody (DAR), which can be selected from an integer or decimal, such as an integer or decimal selected from 1 to 50, an integer or decimal selected from 1 to 20, or an integer or decimal selected from 1 to 10:

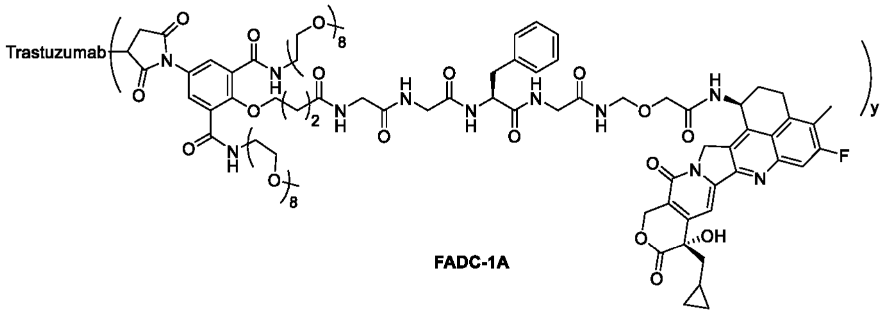

FADC-1A

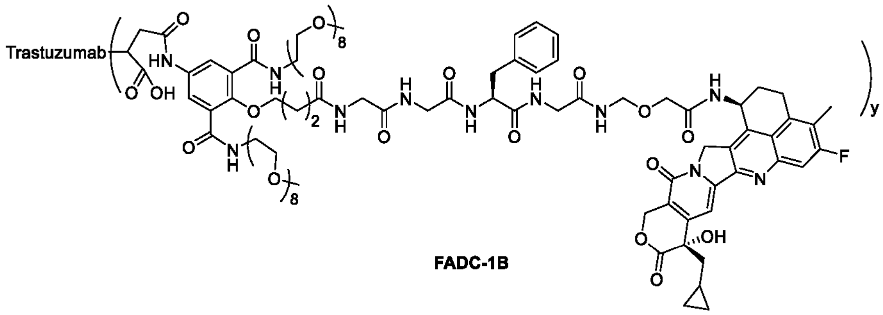

FADC-1B

(continued)

| |
|---|
| 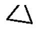 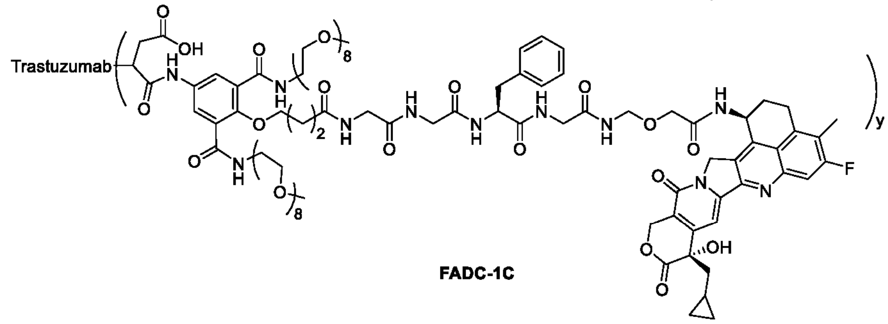 FADC-1C |
| 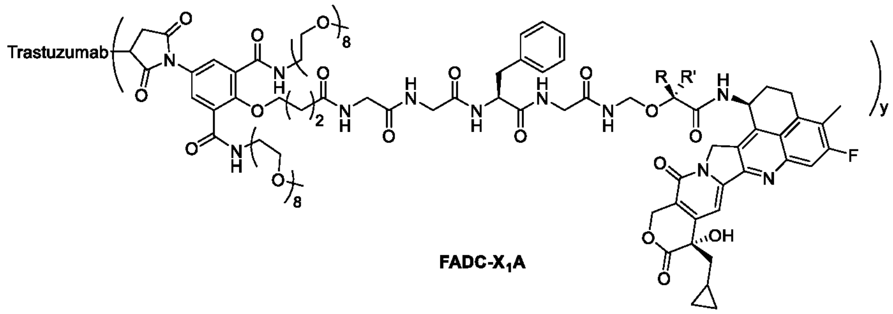 **FADC-$X_1$A** 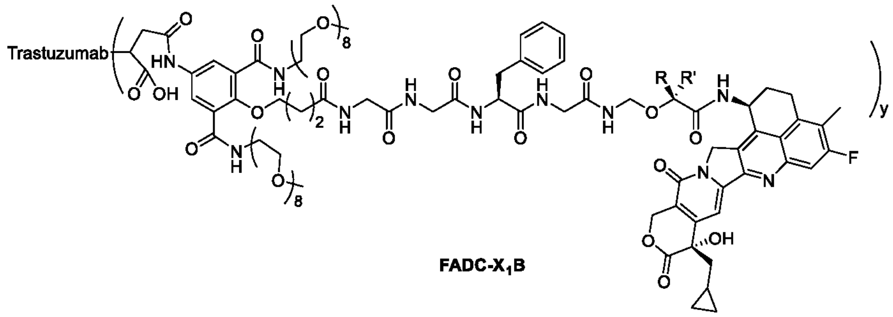 **FADC-$X_1$B** 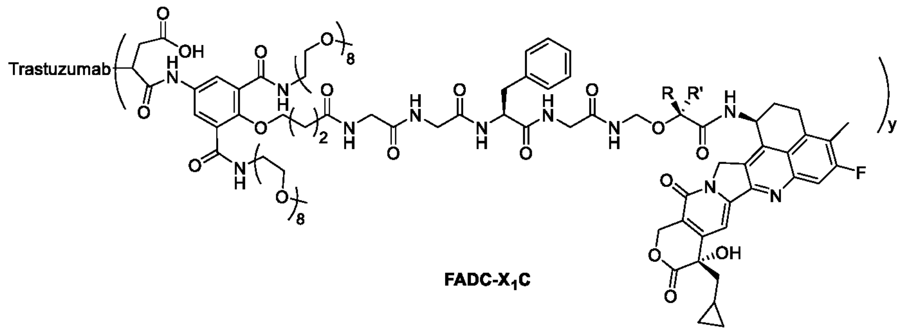 **FADC-$X_1$C**<br>wherein, R is selected from $-CH_3$, R' is selected from -H;<br>R is selected from -H, R' is selected from $-CH_3$;<br>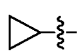 ,<br>R is selected from R' is selected from -H; or<br>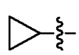 ;<br>R is selected from -H, R' is selected from |

(continued)

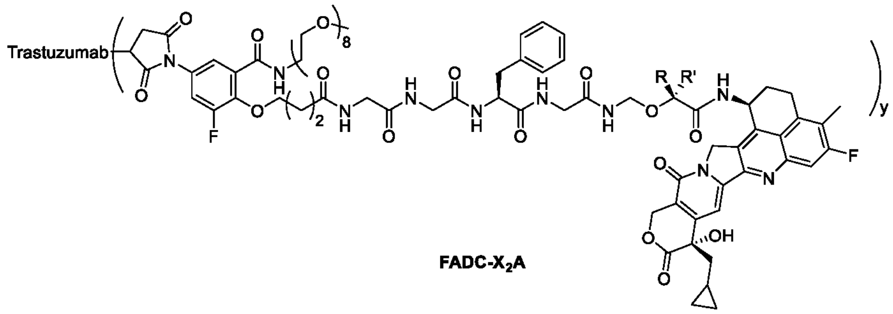

FADC-$X_2$A

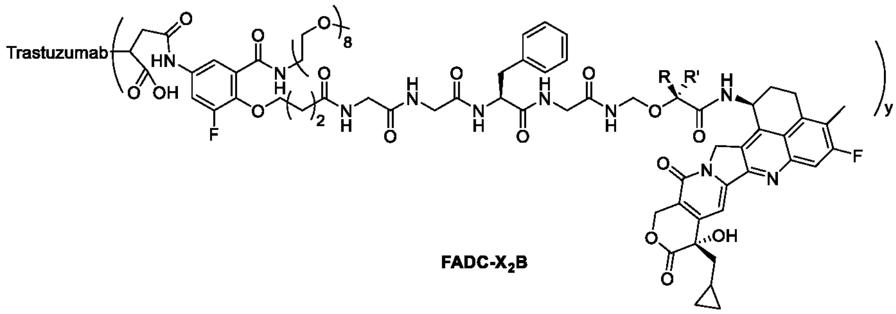

FADC-$X_2$B

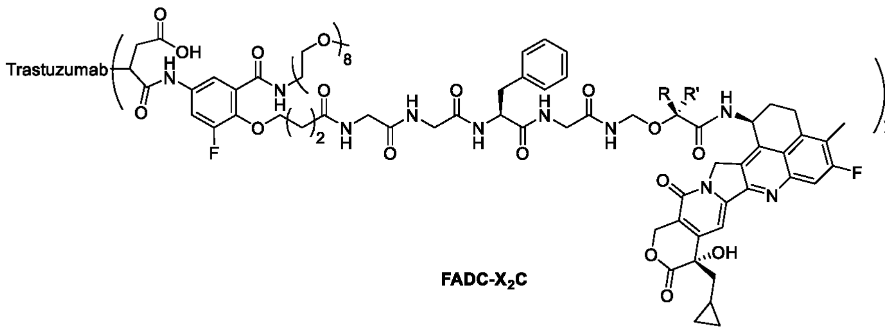

FADC-$X_2$C

wherein, R is selected from -H, R' is selected from -H;
R is selected from $-CH_3$, R' is selected from -H;
R is selected from -H, R' is selected from $-CH_3$;

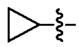
,

R is selected from R' is selected from -H; or

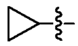
;

R is selected from -H, R' is selected from

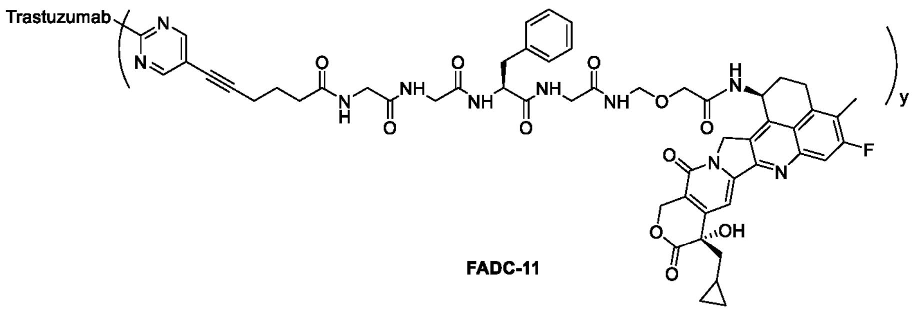

FADC-11

(continued)

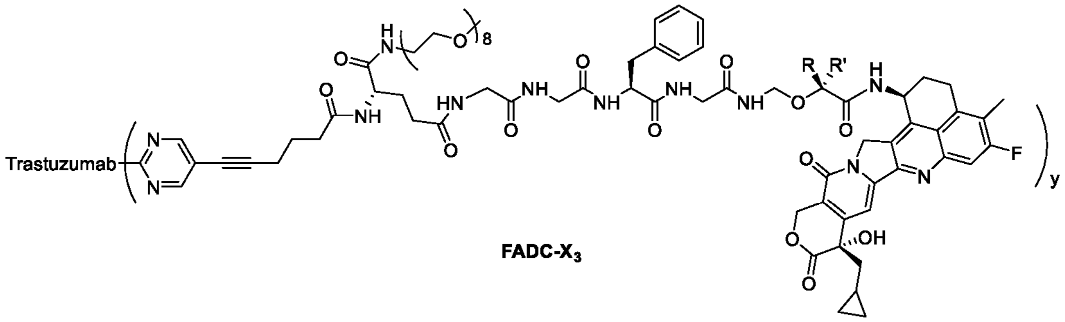

FADC-$X_3$

wherein, R is selected from -H, R' is selected from -H;
R is selected from $-CH_3$, R' is selected from -H;
R is selected from -H, R' is selected from $-CH_3$;

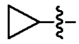,

R is selected from R' is selected from -H; or

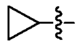;

R is selected from -H, R' is selected from

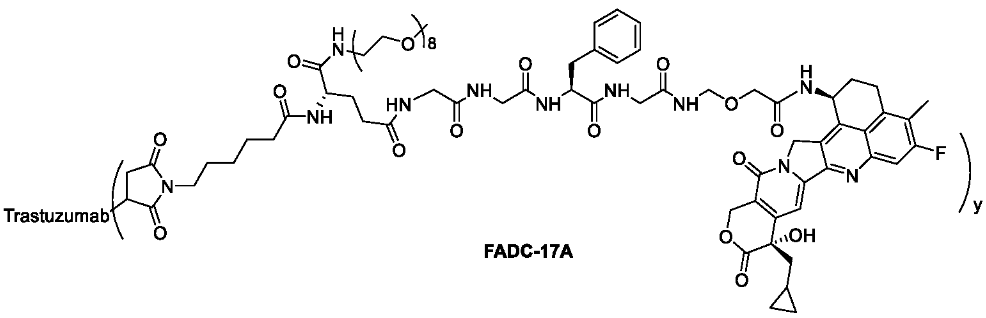

FADC-17A

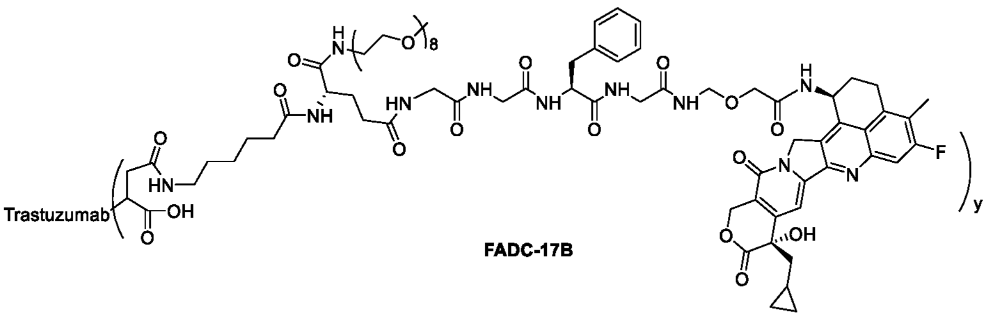

FADC-17B

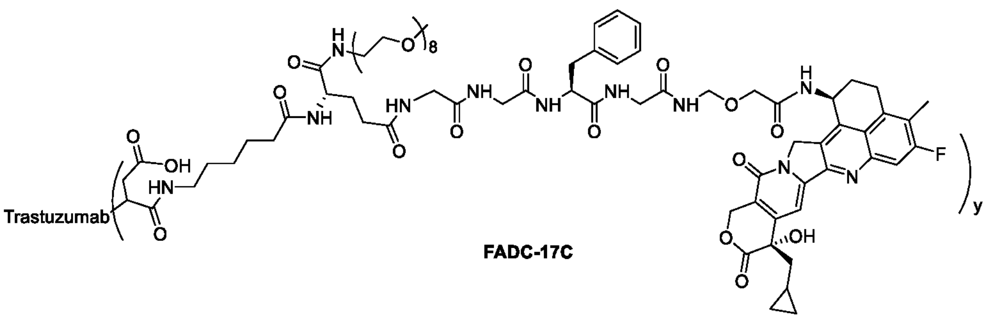

FADC-17C

(continued)

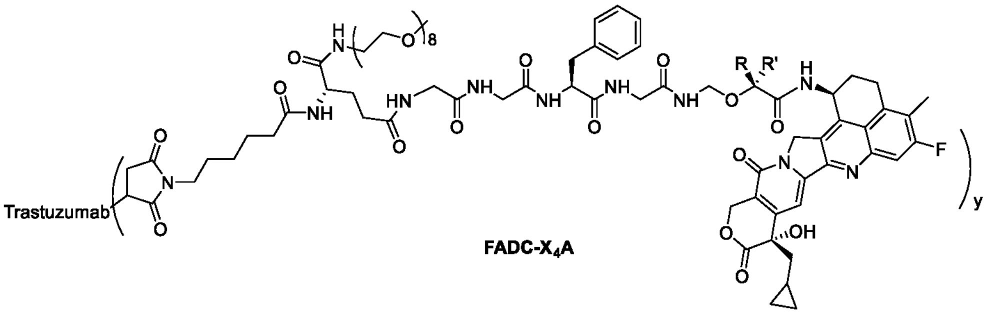

FADC-X$_4$A

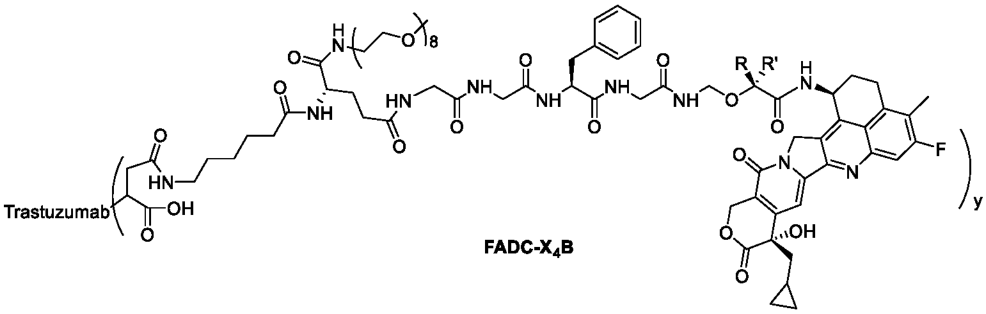

FADC-X$_4$B

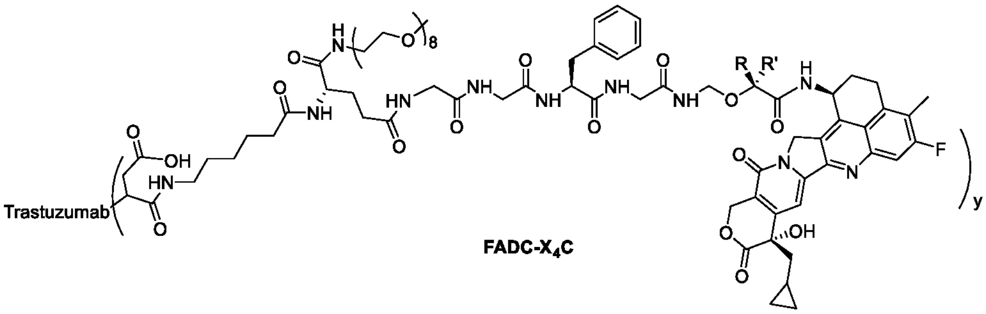

FADC-X$_4$C

wherein, R is selected from $-CH_3$, R' is selected from -H;
R is selected from -H, R' is selected from $-CH_3$;

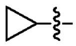,

R is selected from R' is selected from -H; or

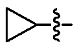;

R is selected from -H, R' is selected from

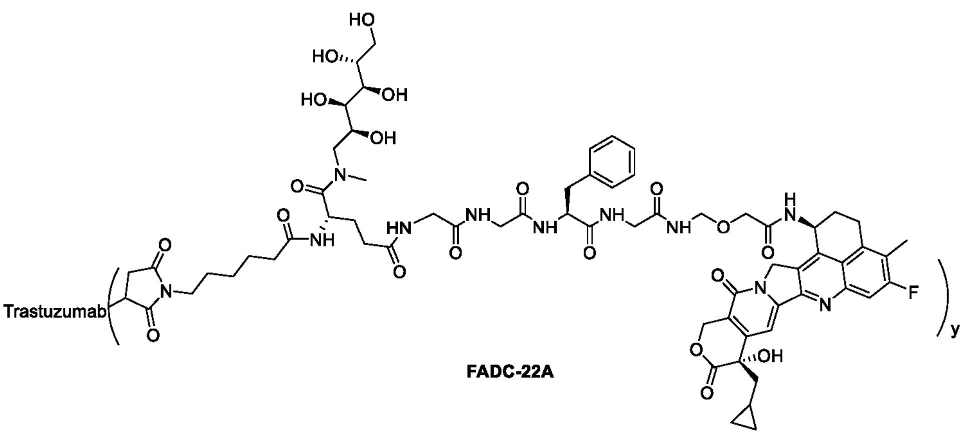

FADC-22A

(continued)

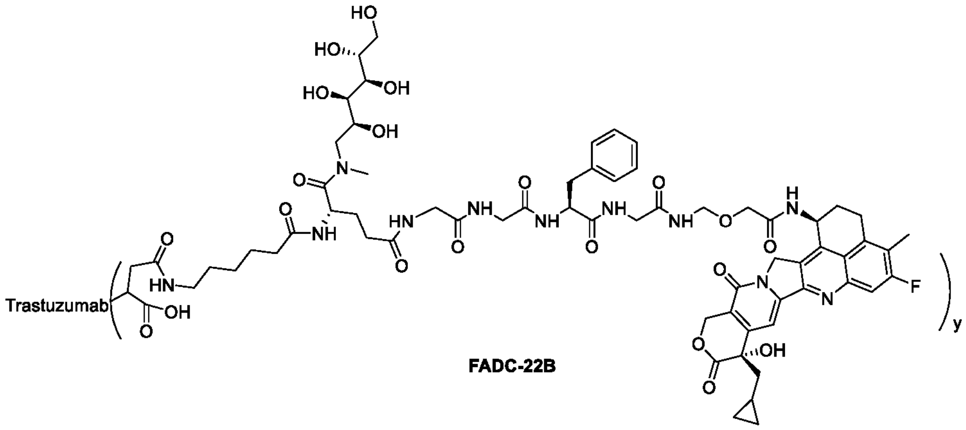

FADC-22B

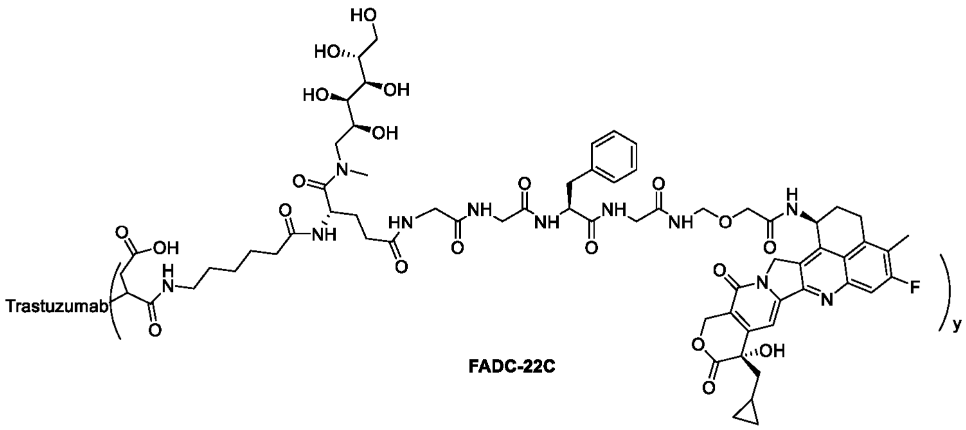

FADC-22C

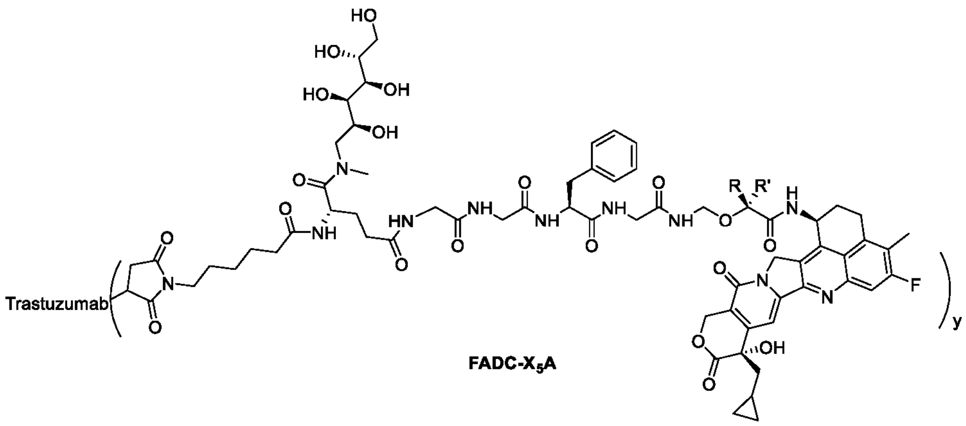

FADC-$X_5$A

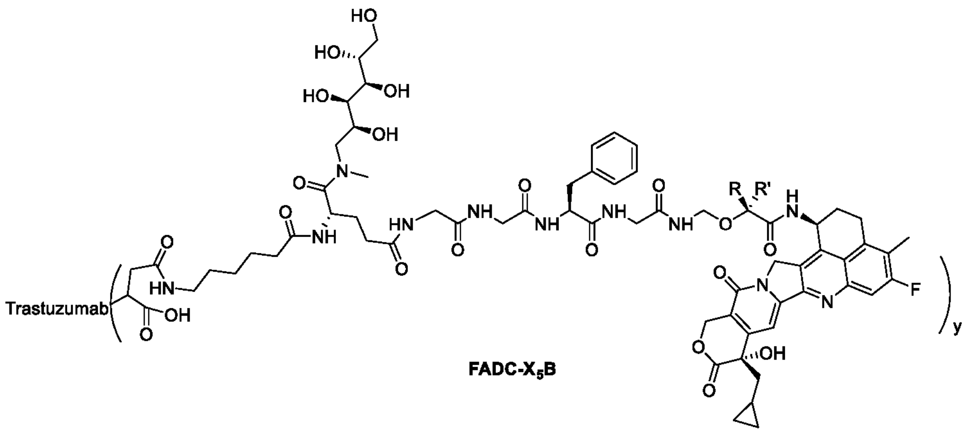

FADC-$X_5$B

(continued)

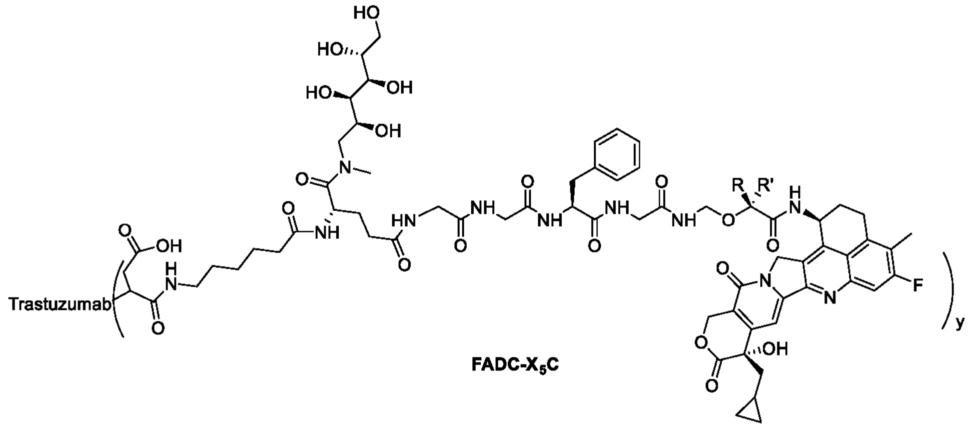

**FADC-X$_5$C**

wherein, R is selected from $-CH_3$, R' is selected from -H;
R is selected from -H, R' is selected from $-CH_3$;

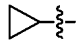,

R is selected from R' is selected from -H; or

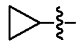;

R is selected from -H, R' is selected from

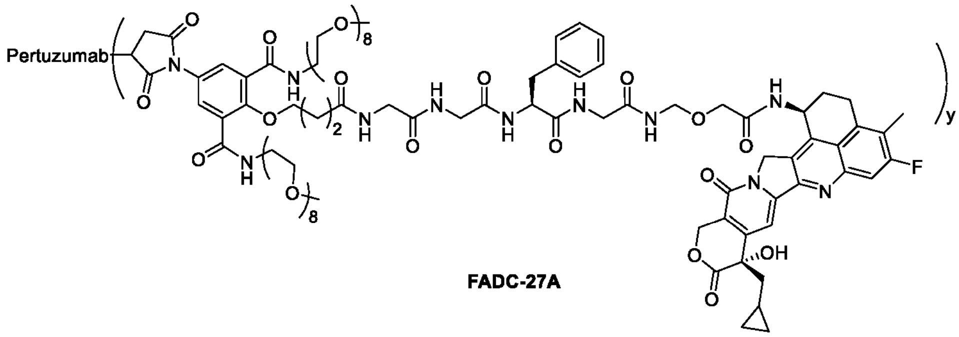

**FADC-27A**

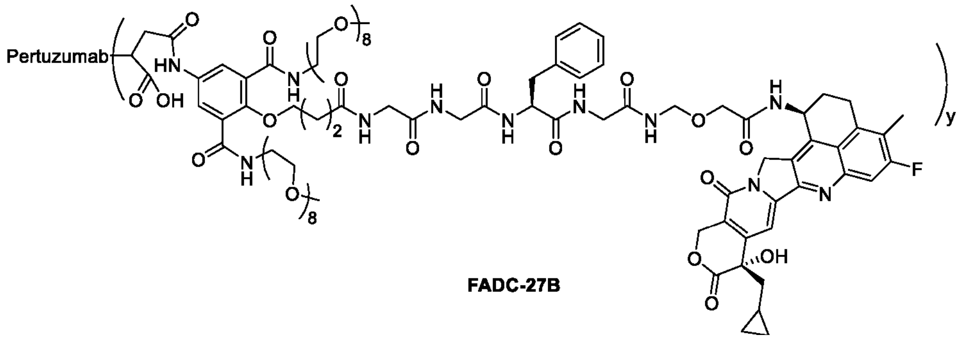

**FADC-27B**

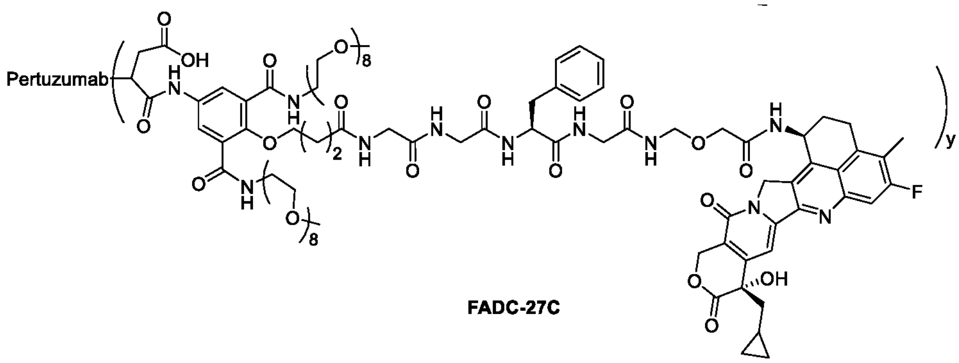

**FADC-27C**

(continued)

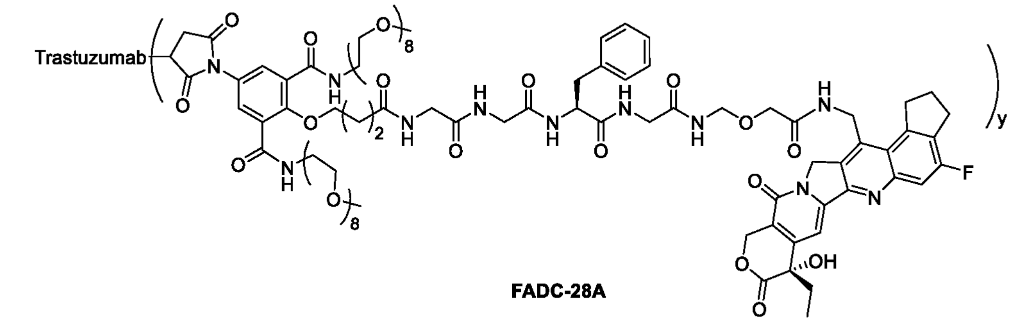

FADC-28A

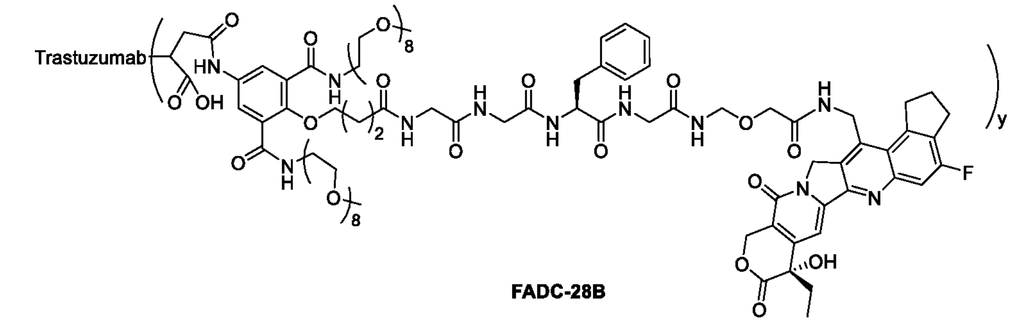

FADC-28B

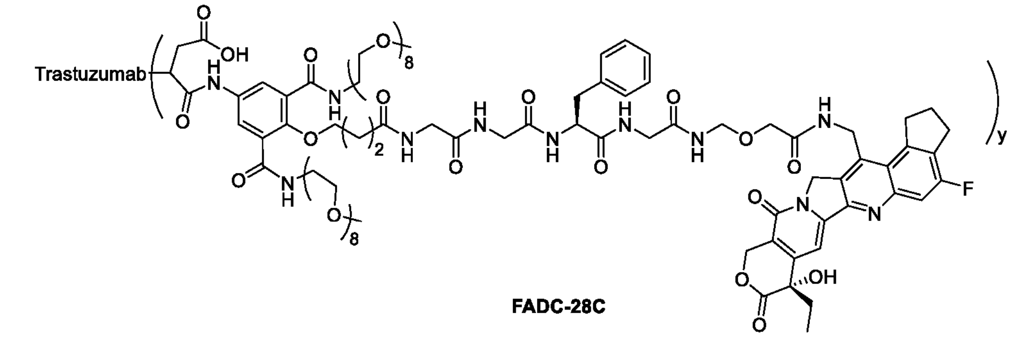

FADC-28C

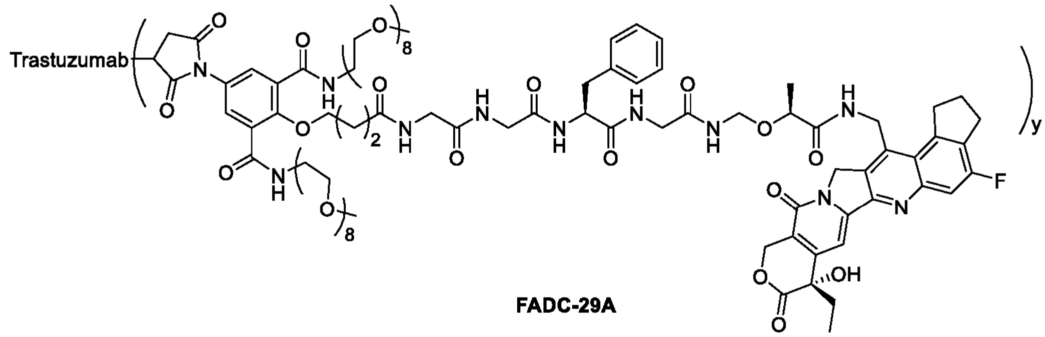

FADC-29A

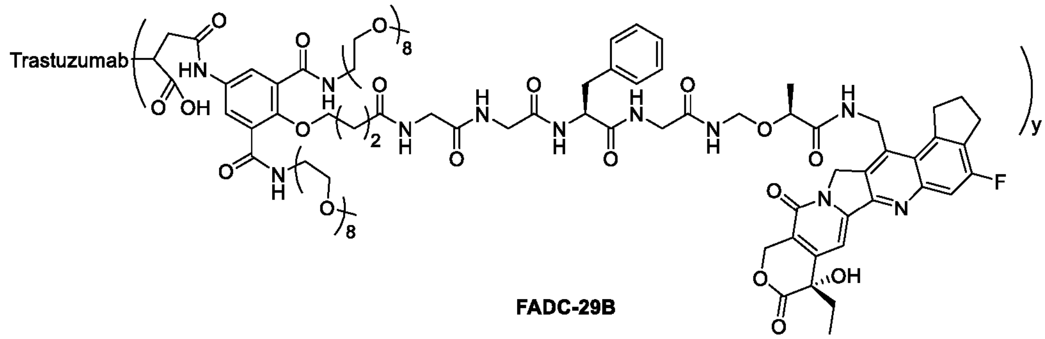

FADC-29B

(continued)

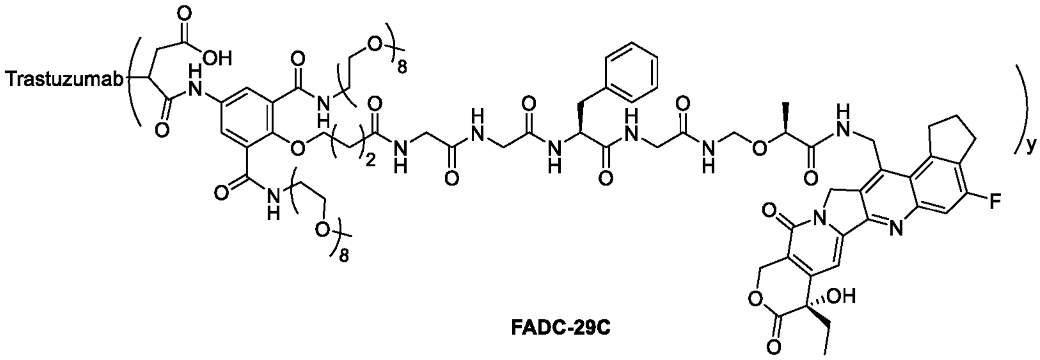

FADC-29C

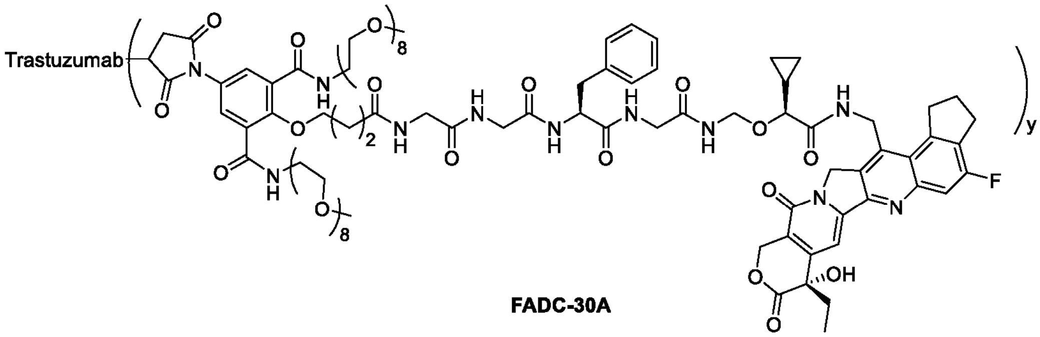

FADC-30A

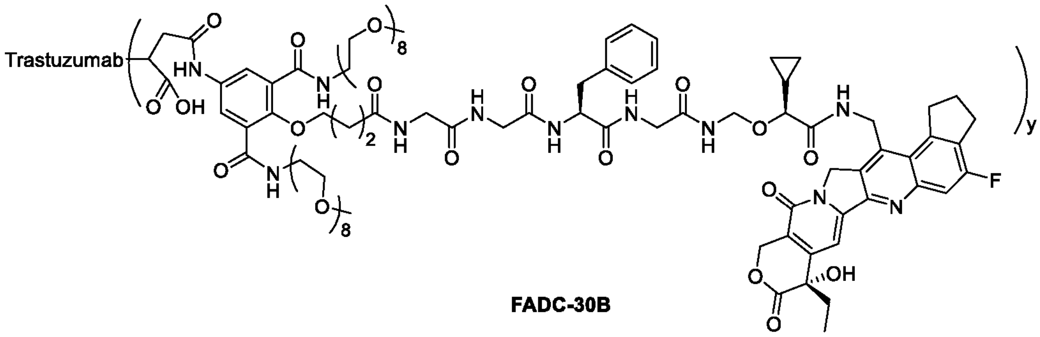

FADC-30B

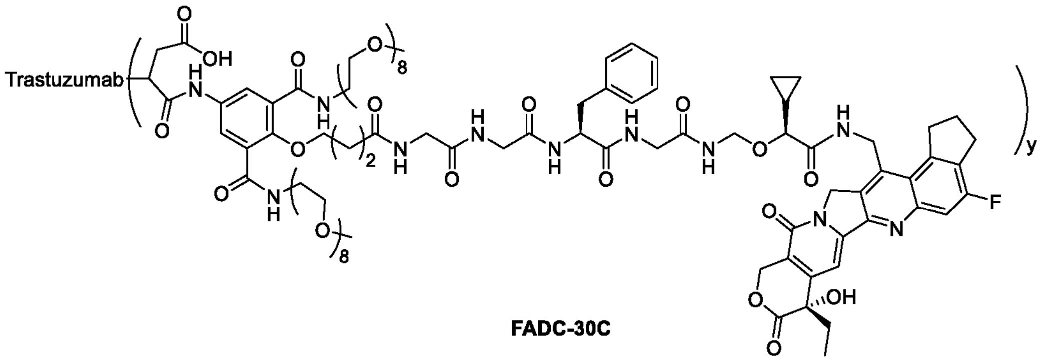

FADC-30C

(continued)

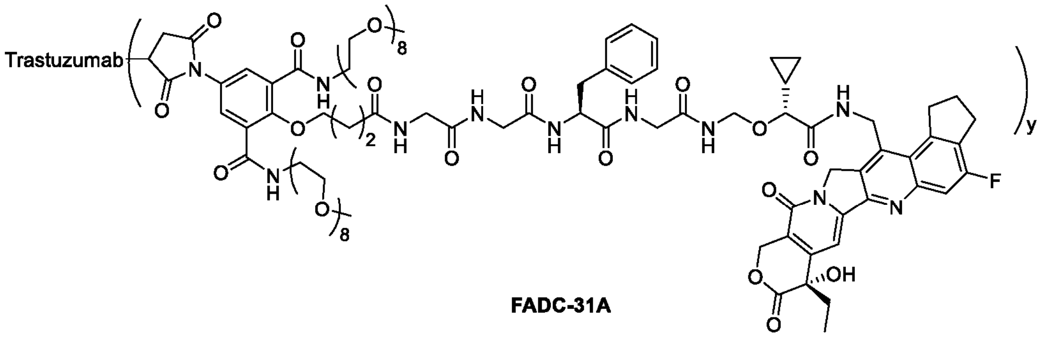

FADC-31A

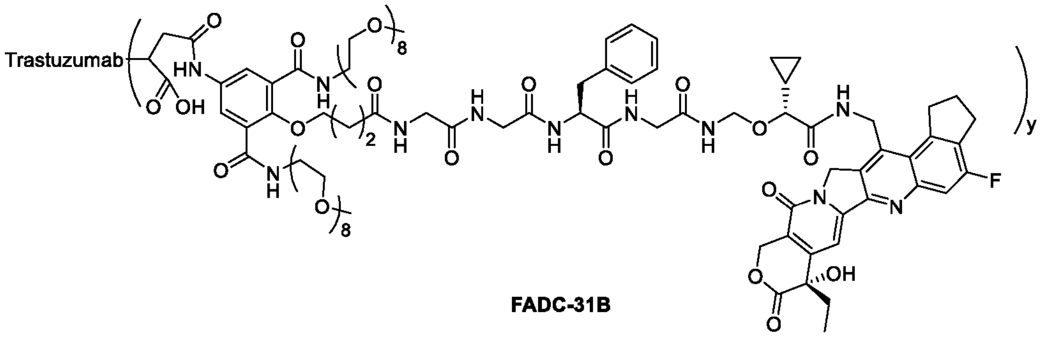

FADC-31B

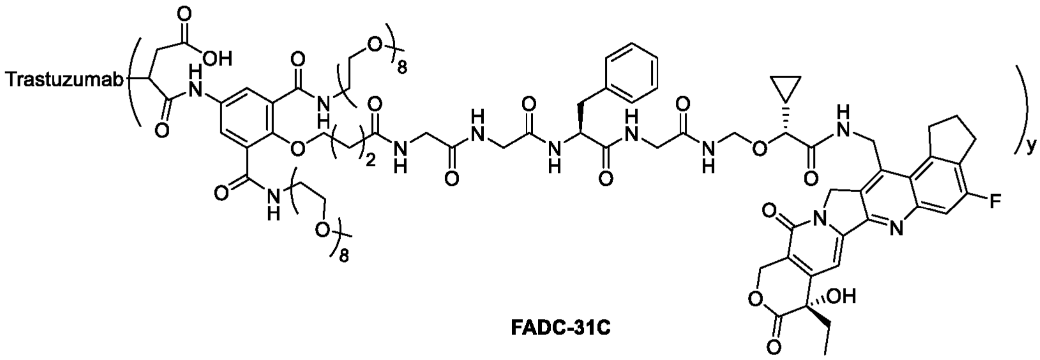

FADC-31C

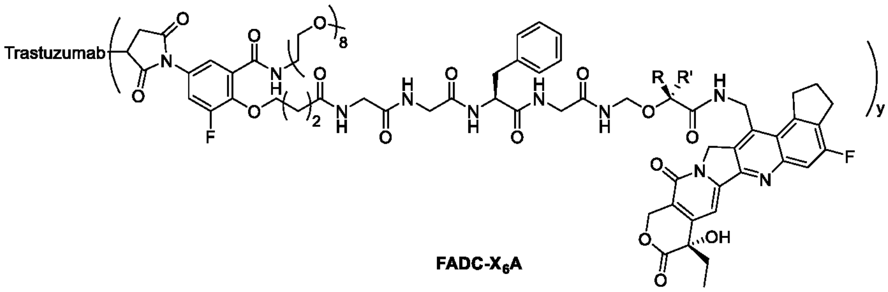

FADC-$X_6$A

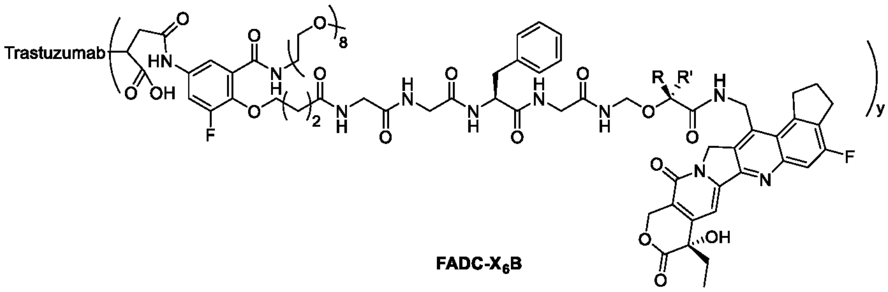

FADC-$X_6$B

(continued)

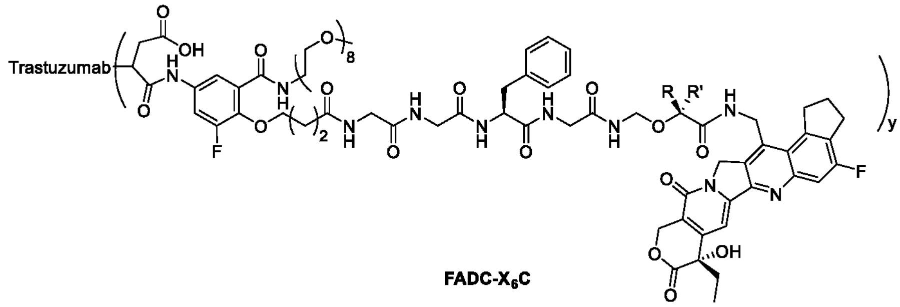

FADC-$X_6$C

wherein, R is selected from -H, R' is selected from -H;

R is selected from -$CH_3$, R' is selected from -H;

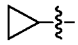

,

R is selected from R' is selected from -H; or

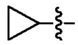

;

R is selected from -H, R' is selected from

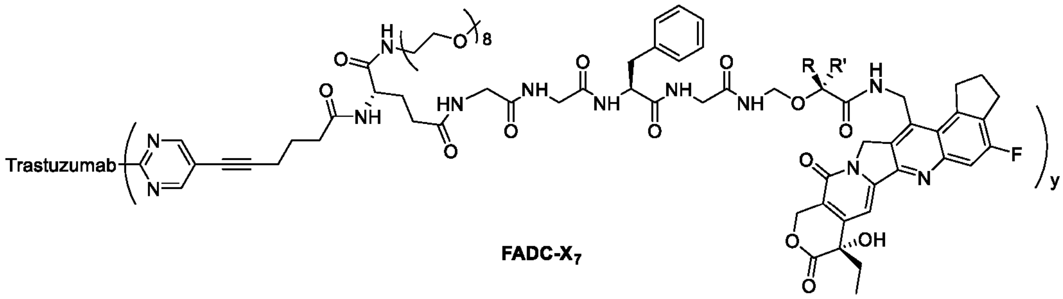

FADC-$X_7$

wherein, R is selected from -H, R' is selected from -H;

R is selected from -$CH_3$, R' is selected from -H; or

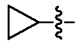

,

R is selected from R' is selected from -H;

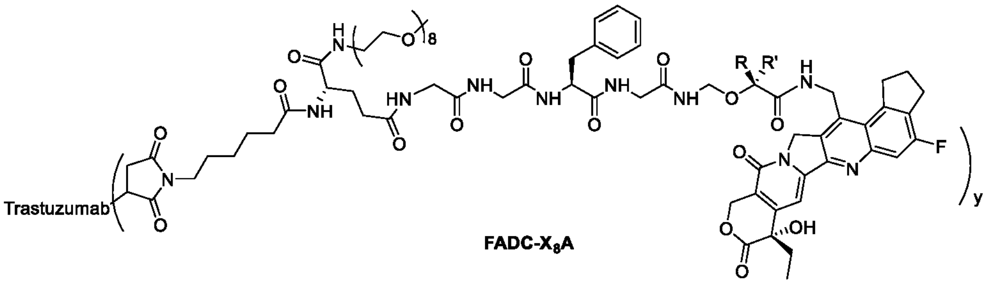

FADC-$X_8$A

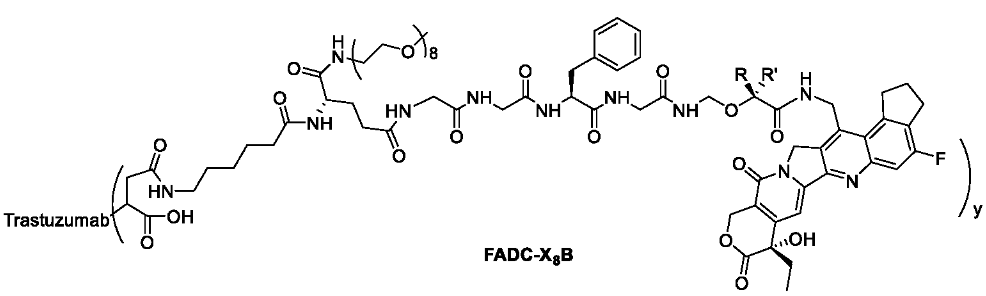

FADC-$X_8$B

(continued)

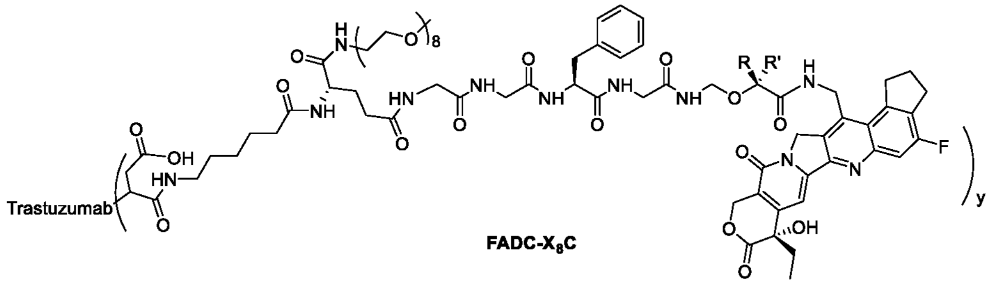

FADC-X$_8$C

wherein, R is selected from -H, R' is selected from -H;
R is selected from -$CH_3$, R' is selected from -H;

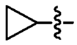,

R is selected from R' is selected from -H; or

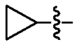;

R is selected from -H, R' is selected from

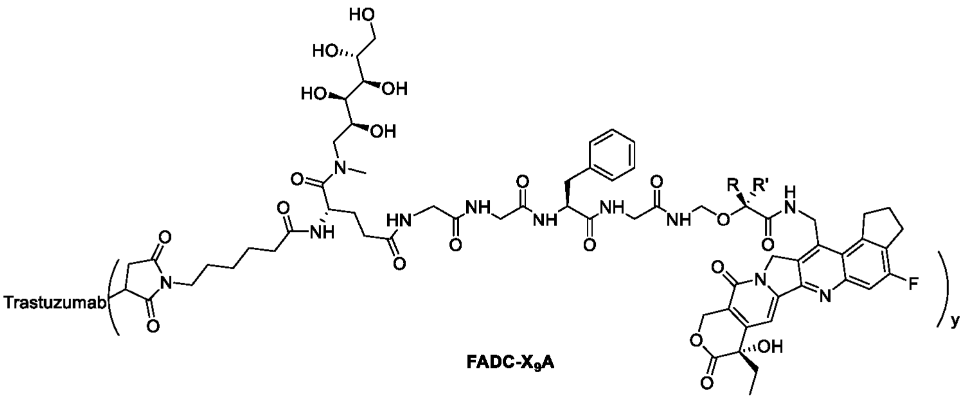

FADC-X$_9$A

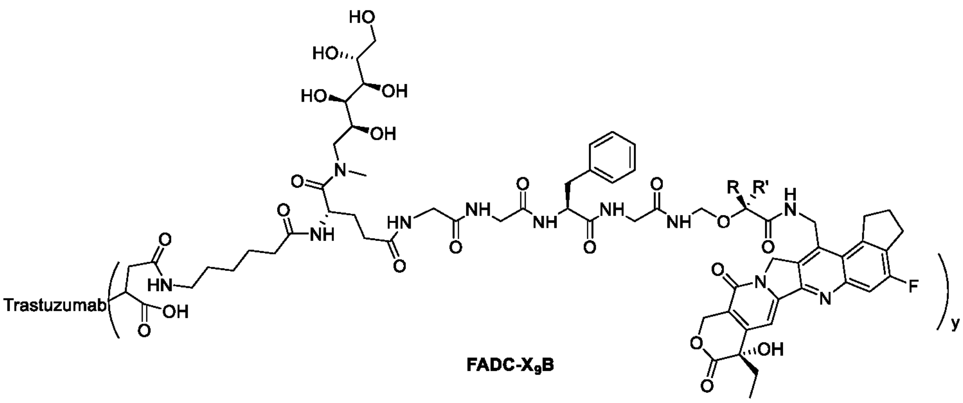

FADC-X$_9$B

(continued)

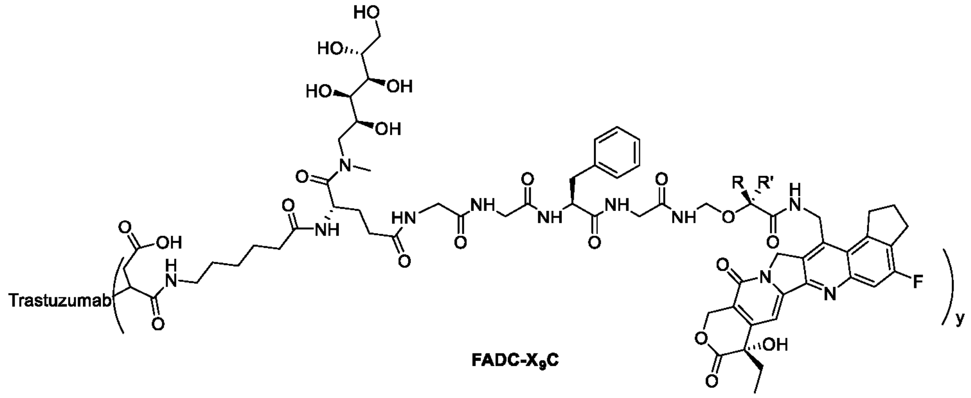

FADC-$X_9$C

wherein, R is selected from -H, R' is selected from -H;
R is selected from $-CH_3$, R' is selected from -H; or

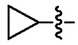

,

R is selected from R' is selected from -H;

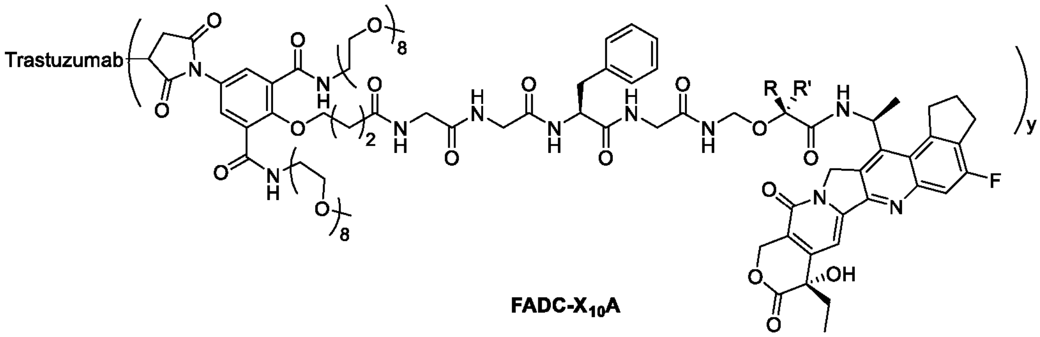

FADC-$X_{10}$A

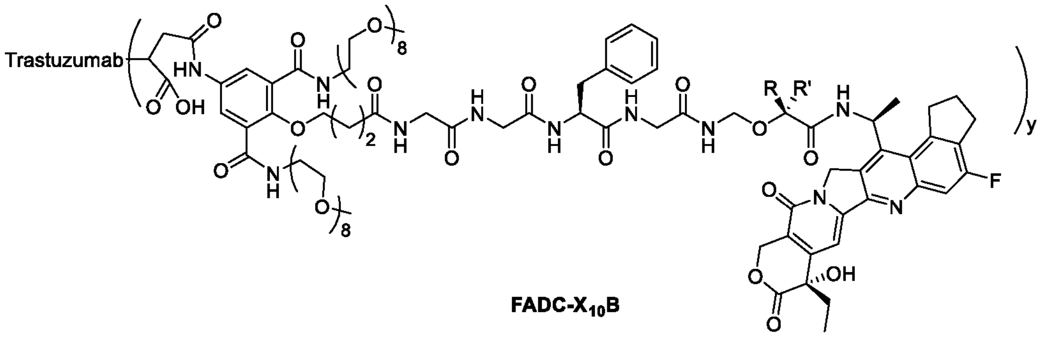

FADC-$X_{10}$B

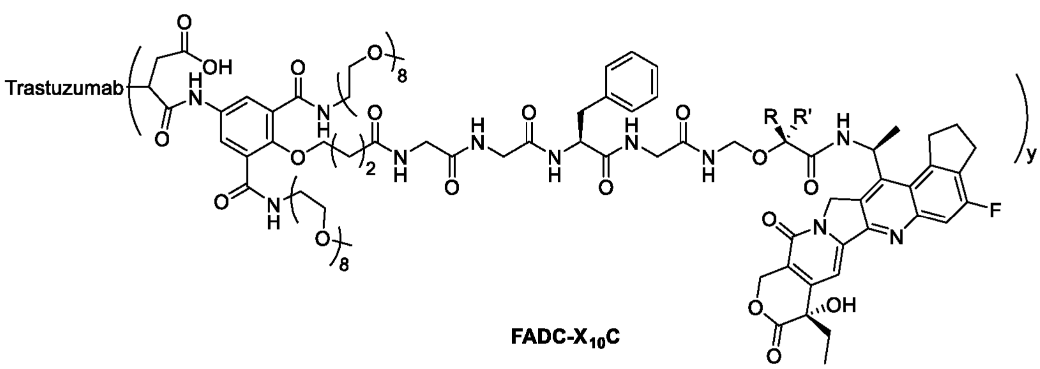

FADC-$X_{10}$C

wherein, R is selected from -H, R' is selected from -H;

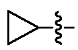

,

R is selected from R' is selected from -H; or

(continued)

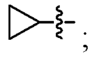

R is selected from -H, R' is selected from

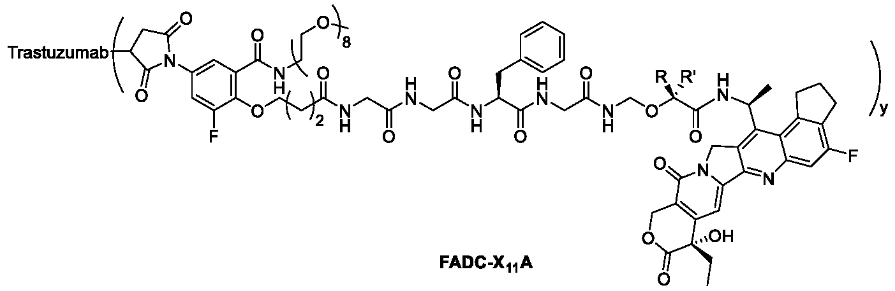

FADC-$X_{11}$A

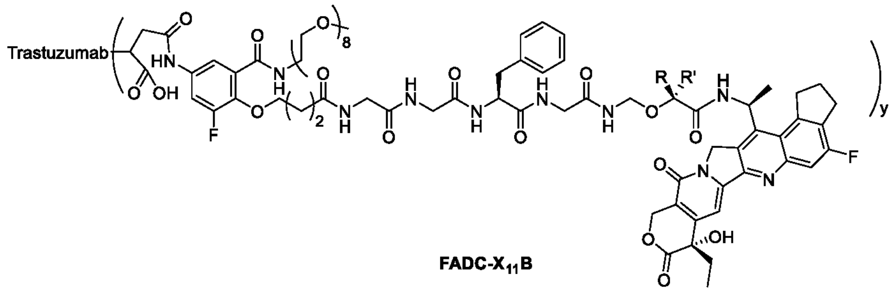

FADC-$X_{11}$B

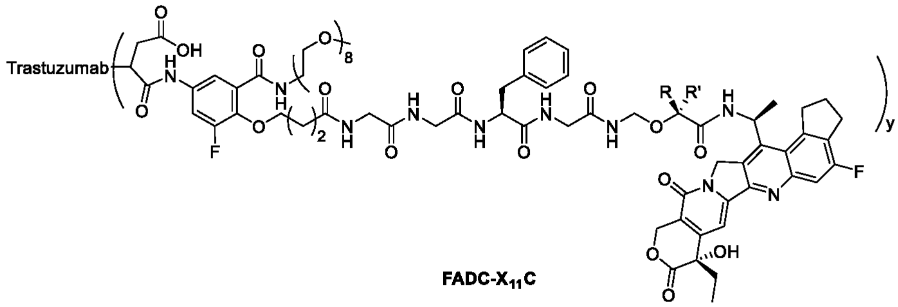

FADC-$X_{11}$C

wherein, R is selected from -H, R' is selected from -H;

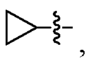

R is selected from R' is selected from -H; or

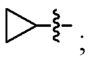

R is selected from -H, R' is selected from

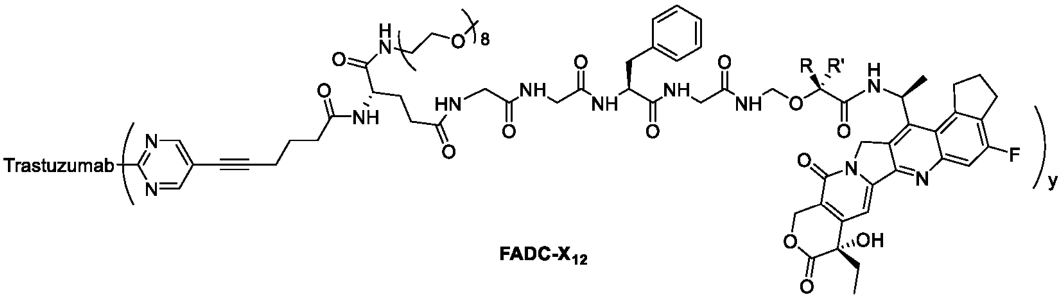

FADC-$X_{12}$

wherein, R is selected from -H, R' is selected from -H;

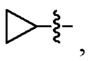

R is selected from R' is selected from -H; or

(continued)

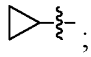;

R is selected from -H, R' is selected from

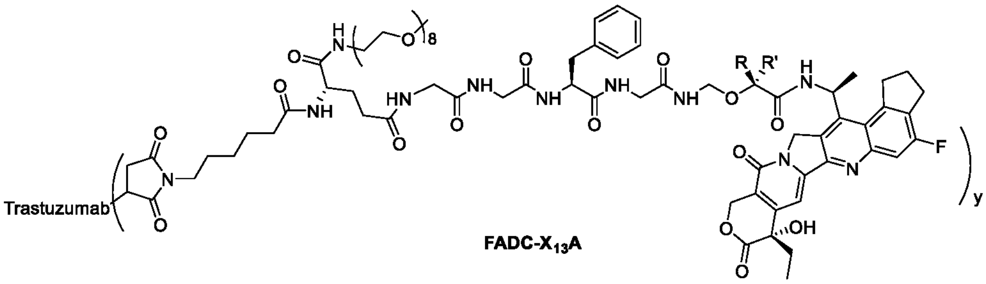

FADC-$X_{13}$A

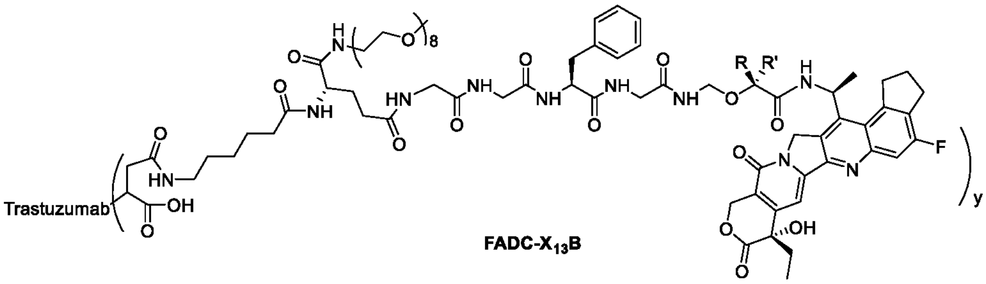

FADC-$X_{13}$B

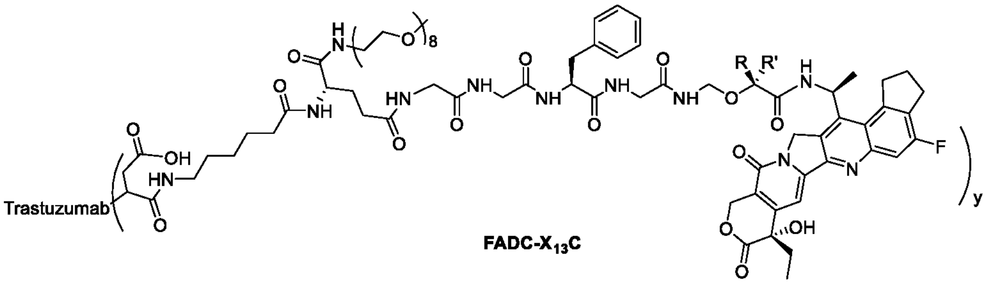

FADC-$X_{13}$C

wherein, R is selected from -H, R' is selected from -H;

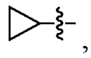,

R is selected from R' is selected from -H; or

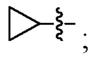;

R is selected from -H, R' is selected from

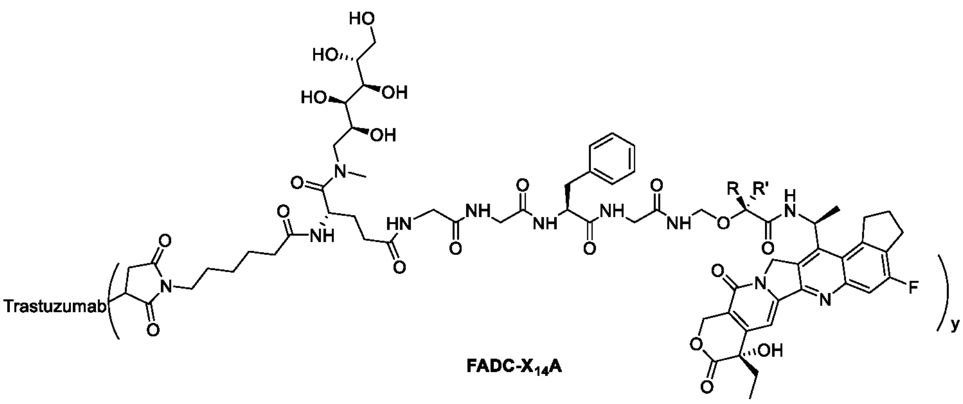

FADC-$X_{14}$A

(continued)

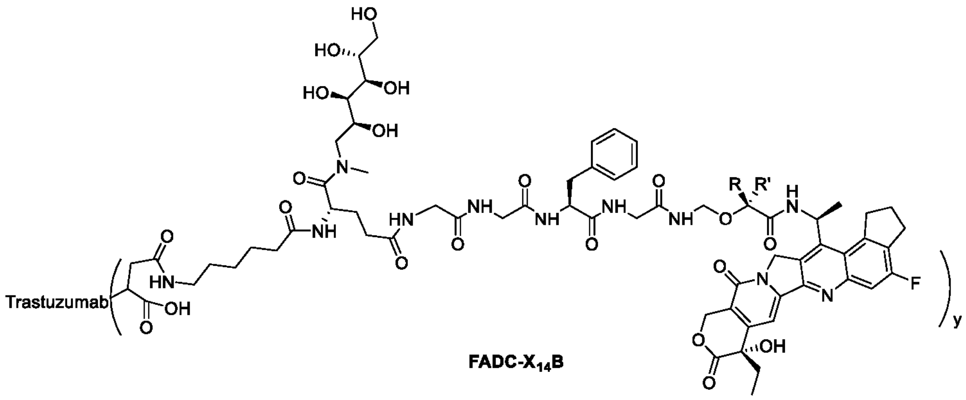

**FADC-X$_{14}$B**

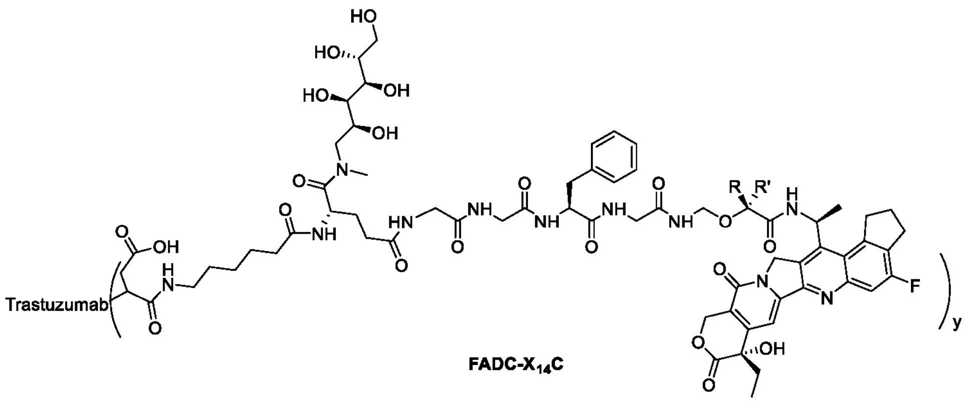

**FADC-X$_{14}$C**

wherein, R is selected from -H, R' is selected from -H;

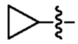,

R is selected from R' is selected from -H; or

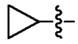;

R is selected from -H, R' is selected from

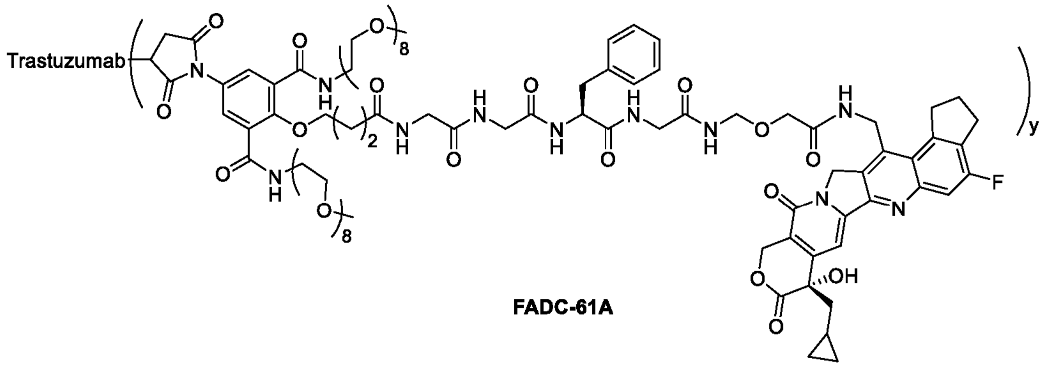

**FADC-61A**

(continued)

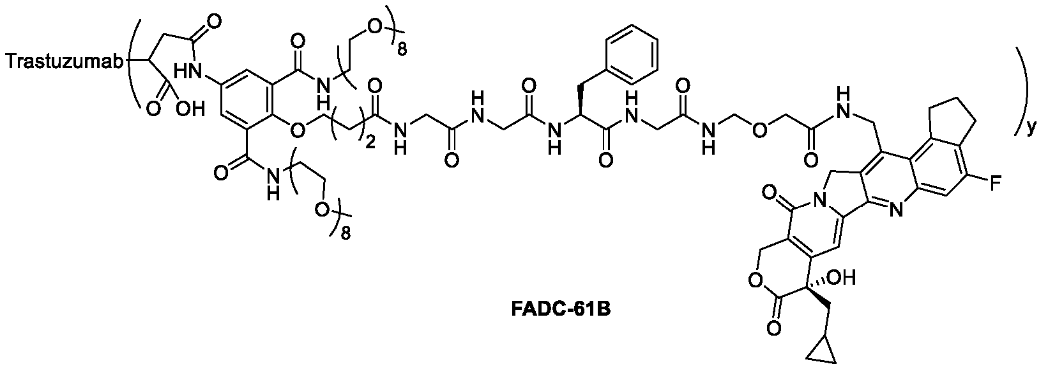

FADC-61B

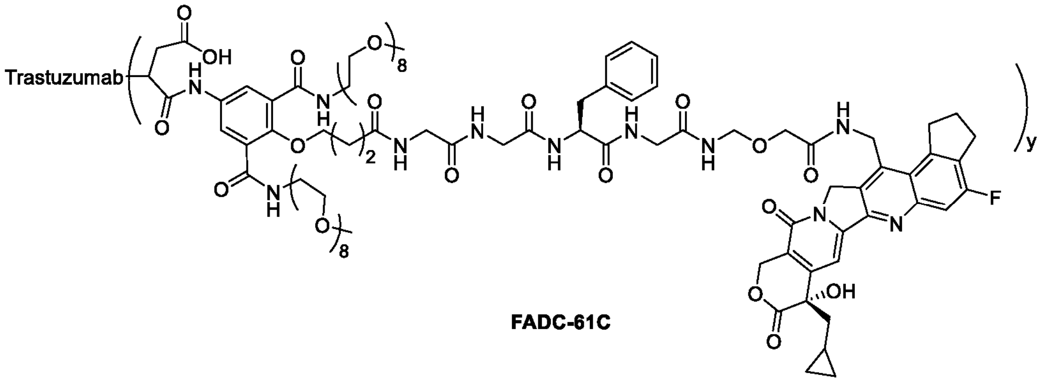

FADC-61C

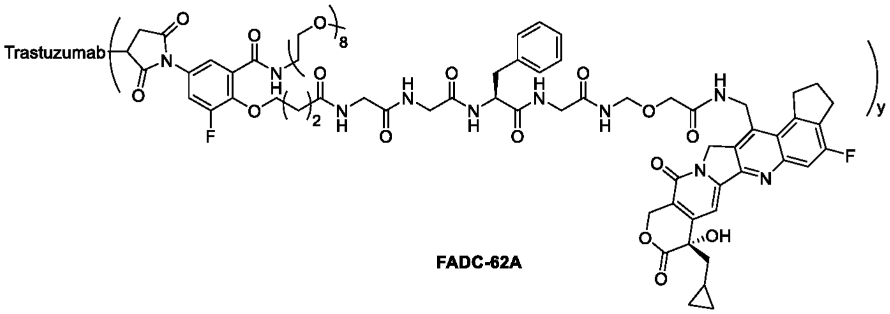

FADC-62A

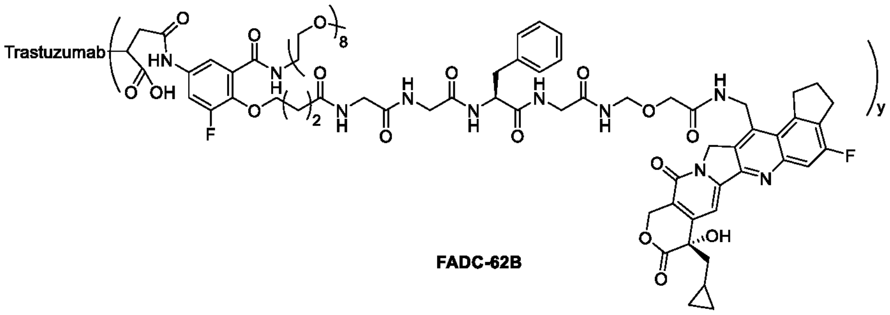

FADC-62B

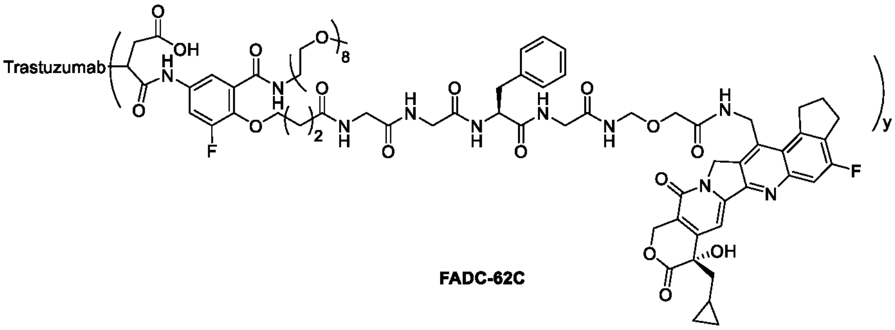

FADC-62C

(continued)

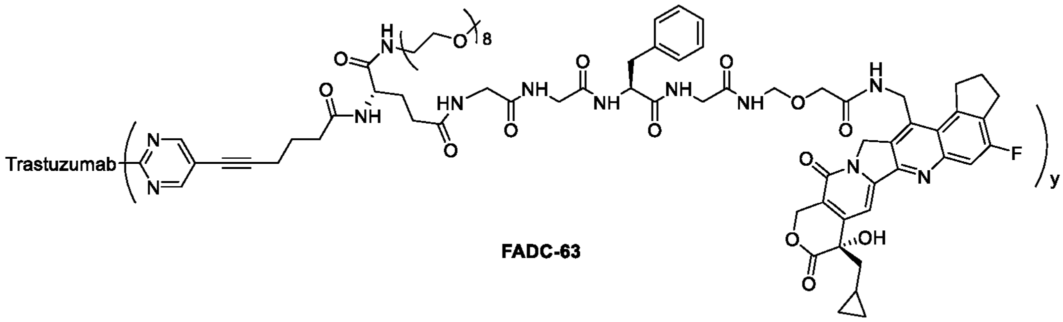

FADC-63

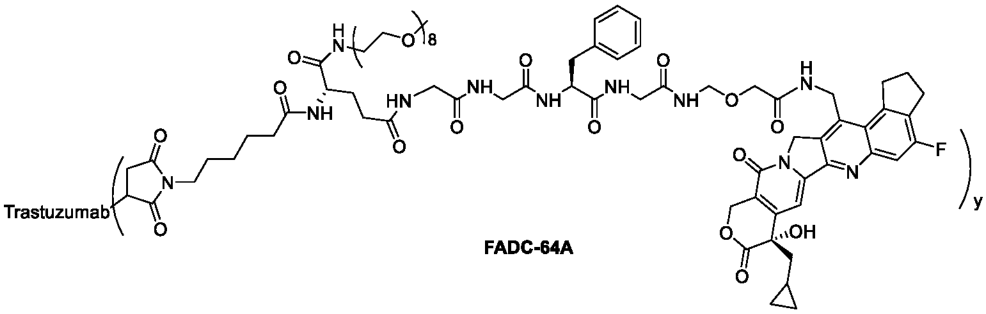

FADC-64A

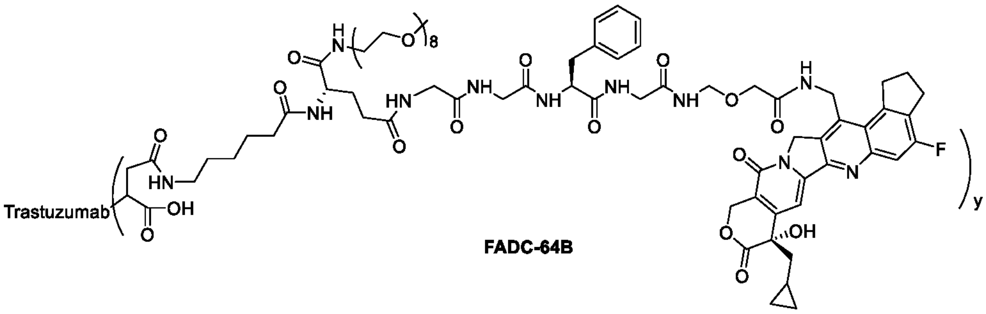

FADC-64B

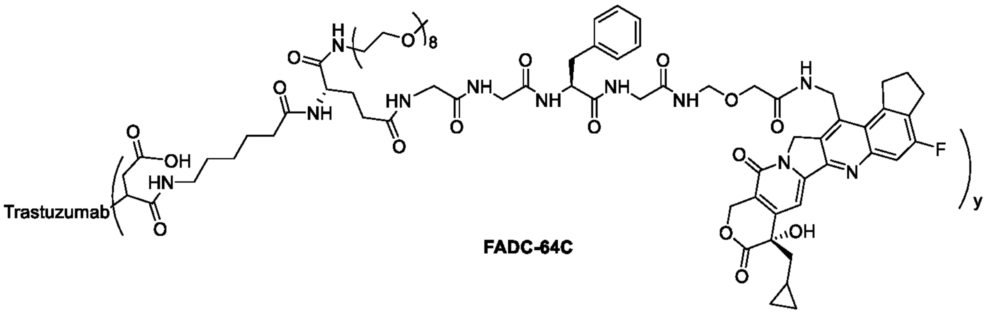

FADC-64C

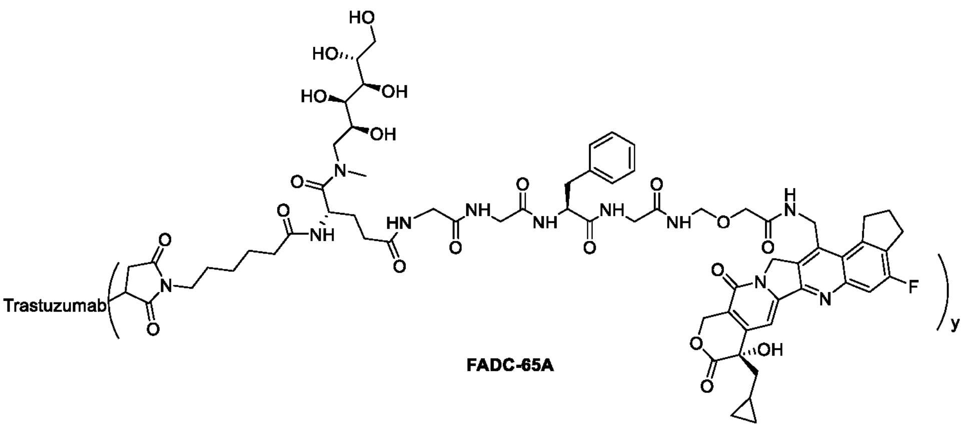

FADC-65A

(continued)

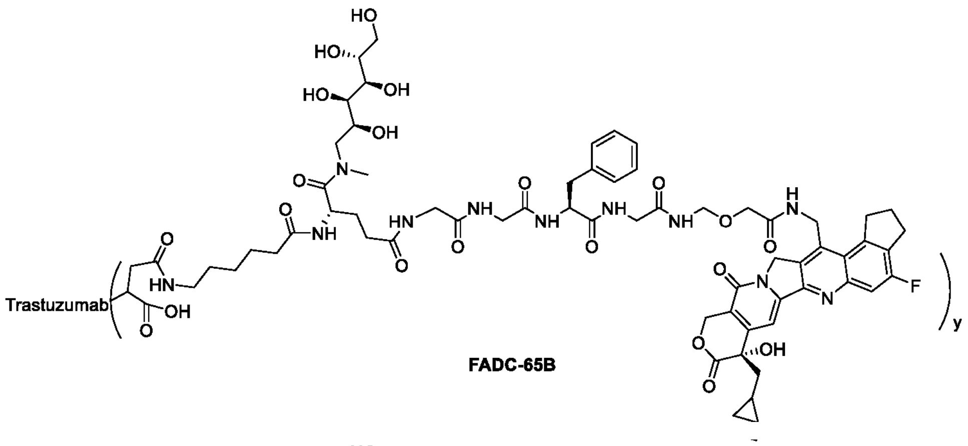

FADC-65B

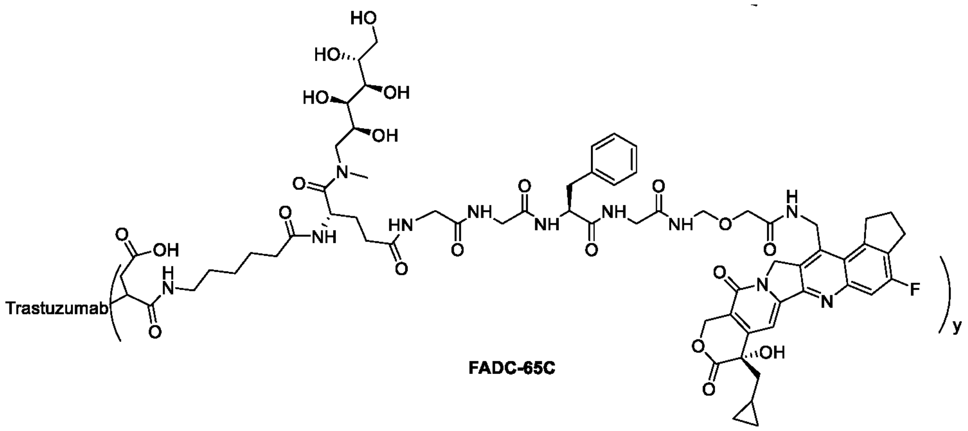

FADC-65C

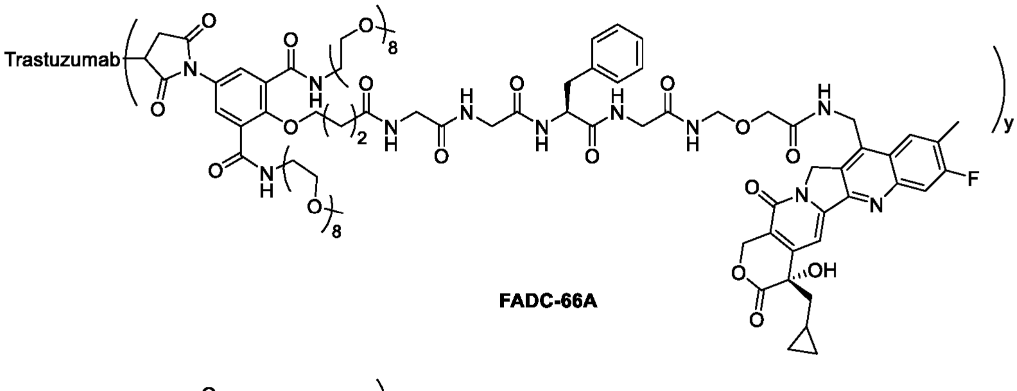

FADC-66A

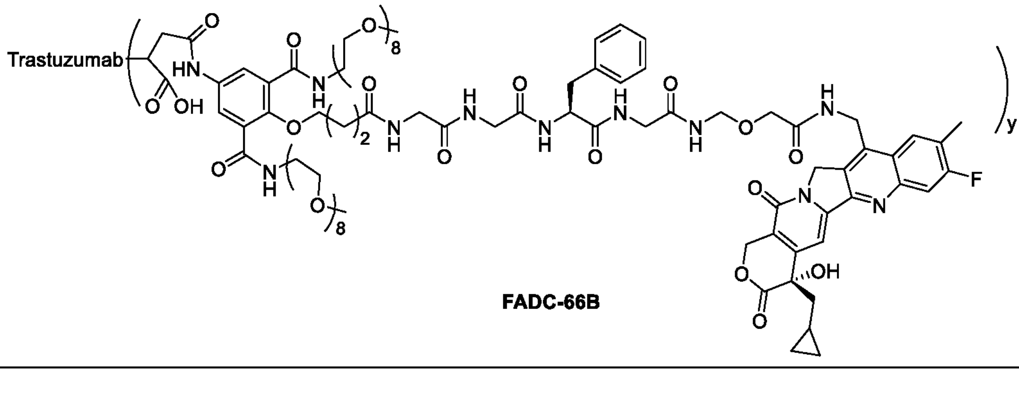

FADC-66B

(continued)

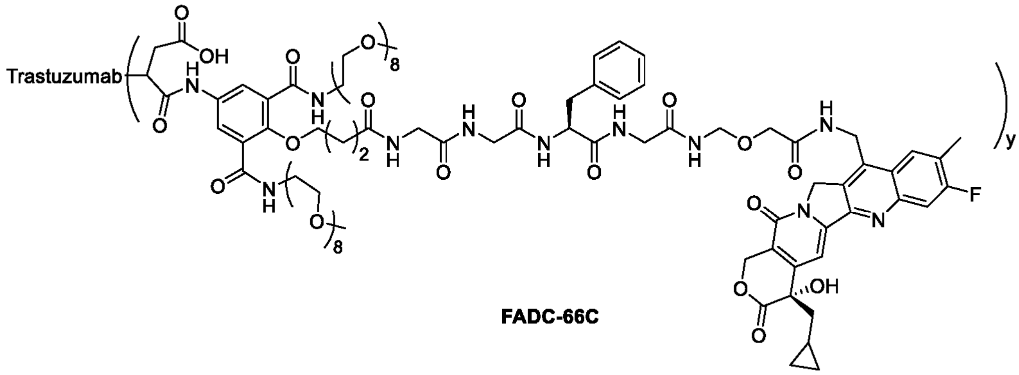

FADC-66C

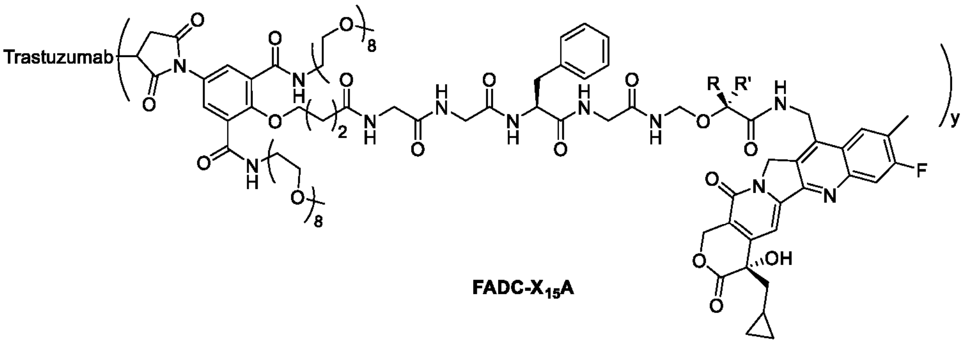

FADC-$X_{15}$A

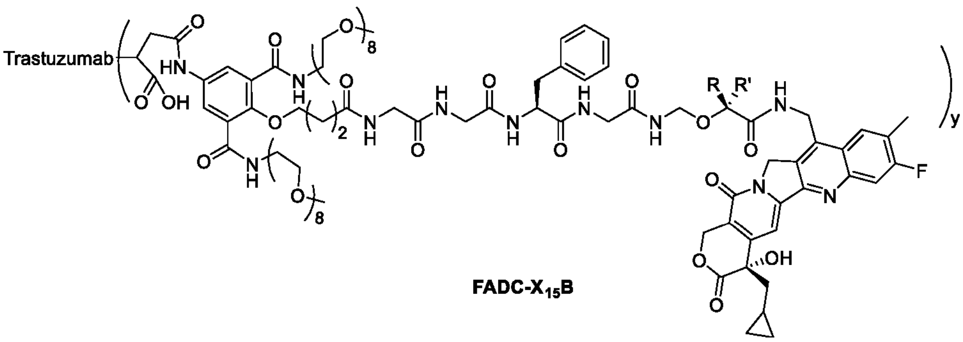

FADC-$X_{15}$B

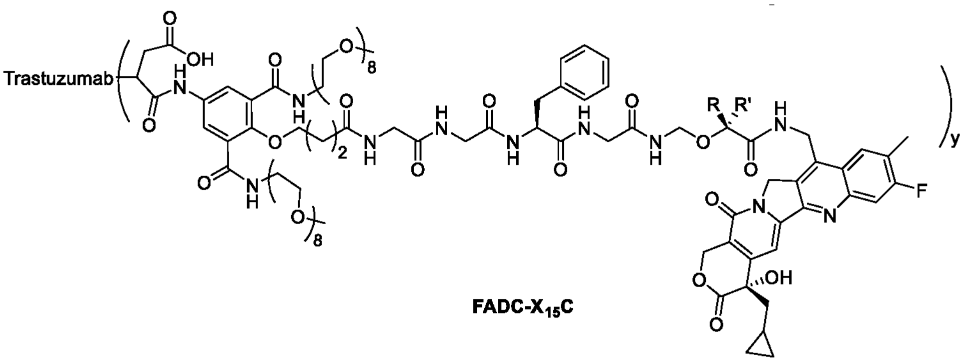

FADC-$X_{15}$C

wherein, R is selected from $-CH_3$, R' is selected from -H;
R is selected from -H, R' is selected from $-CH_3$;

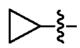,

R is selected from R' is selected from -H; or

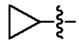;

R is selected from -H, R' is selected from

(continued)

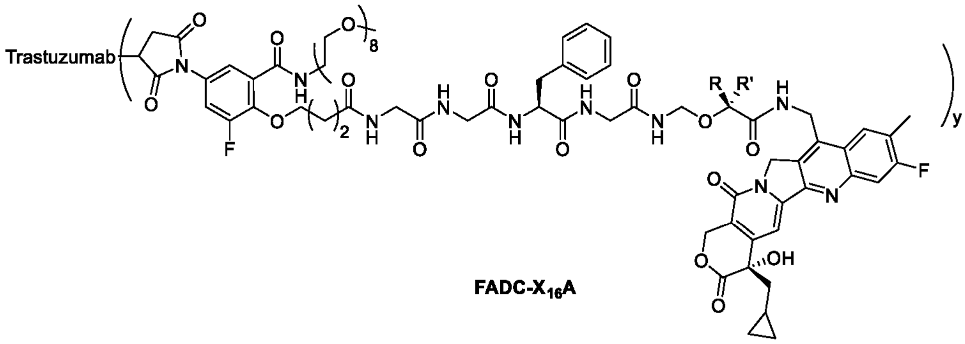

FADC-$X_{16}$A

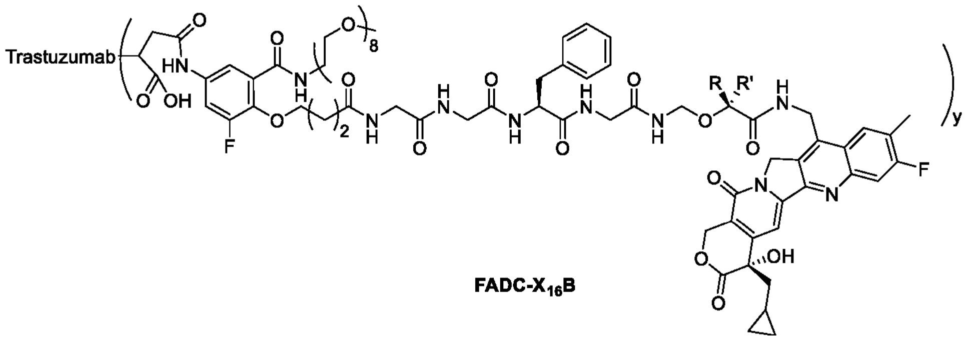

FADC-$X_{16}$B

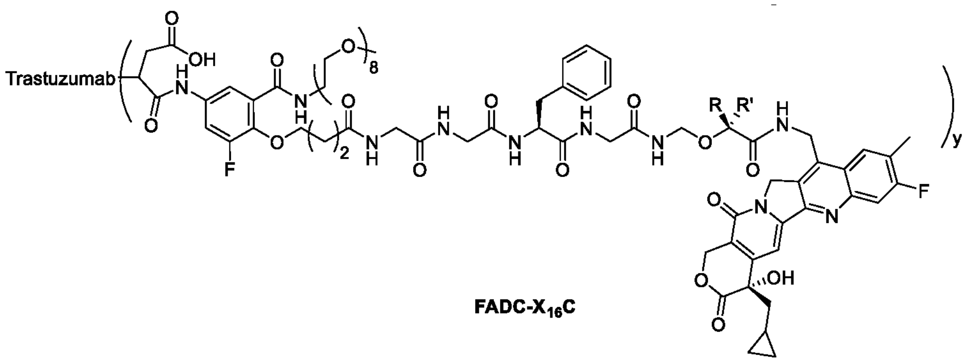

FADC-$X_{16}$C

wherein, R is selected from -H, R' is selected from -H;
R is selected from $-CH_3$, R' is selected from -H;
R is selected from -H, R' is selected from $-CH_3$;

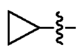,

R is selected from R' is selected from -H; or

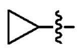;

R is selected from -H, R' is selected from

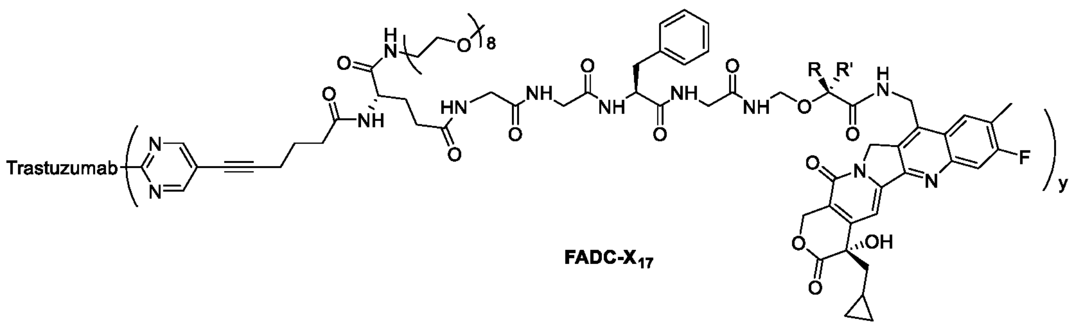

FADC-$X_{17}$

wherein, R is selected from -H, R' is selected from -H;

(continued)

R is selected from $-CH_3$, R' is selected from -H;

R is selected from -H, R' is selected from $-CH_3$;

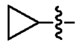,

R is selected from R' is selected from -H; or

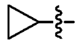;

R is selected from -H, R' is selected from

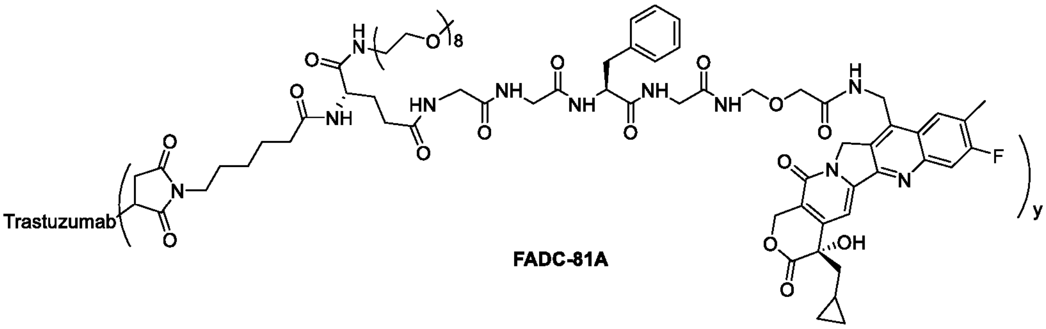

FADC-81A

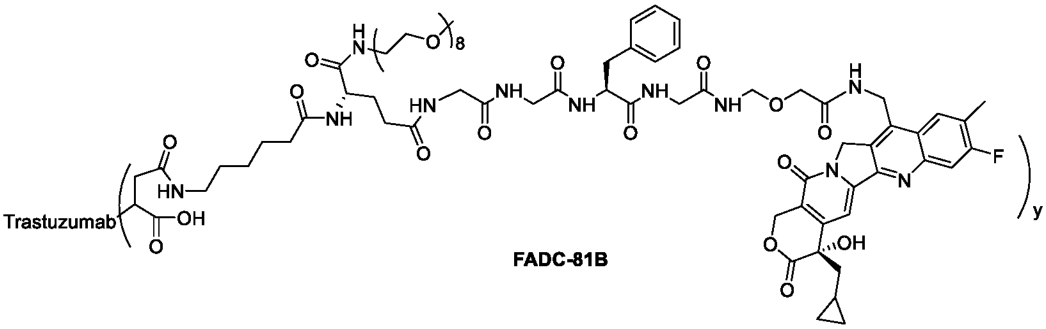

FADC-81B

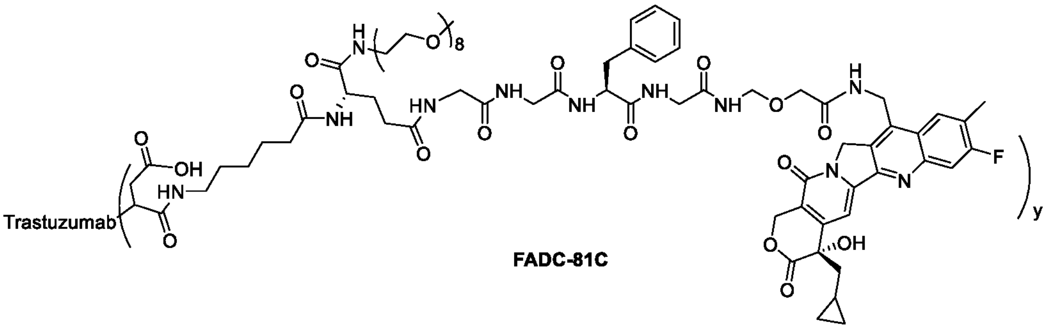

FADC-81C

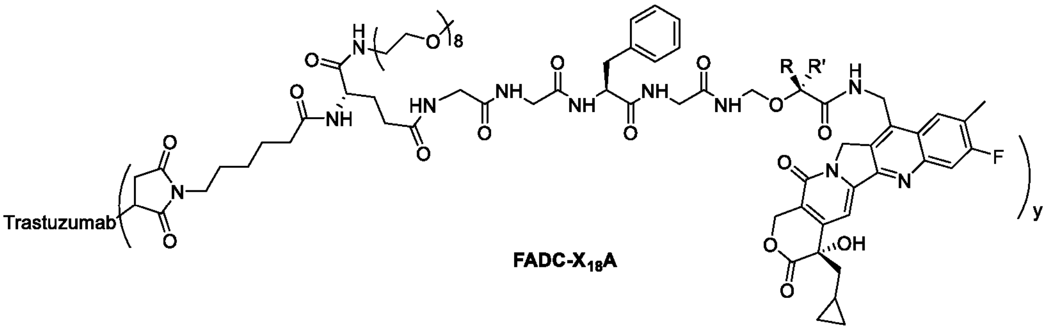

FADC-$X_{18}$A

(continued)

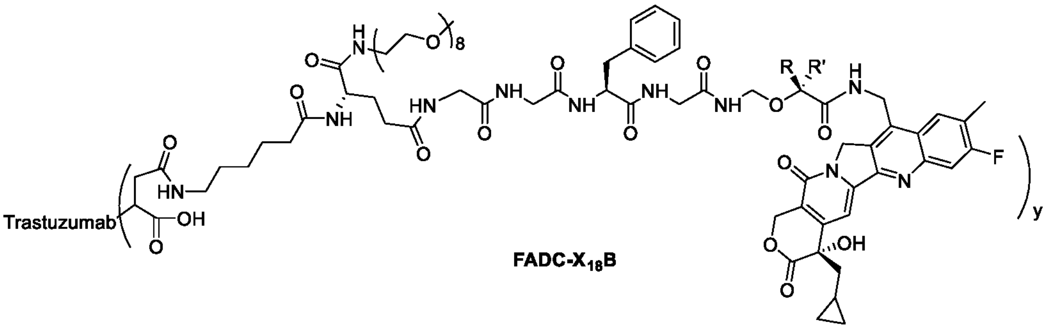

**FADC-$X_{18}$B**

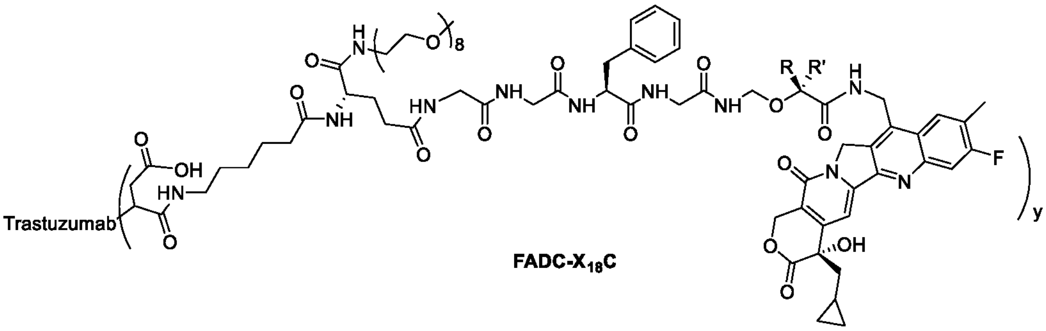

**FADC-$X_{18}$C**

wherein, R is selected from $-CH_3$, R' is selected from -H;
R is selected from -H, R' is selected from $-CH_3$;

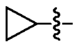,

R is selected from R' is selected from -H; or

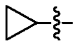;

R is selected from -H, R' is selected from

(continued)

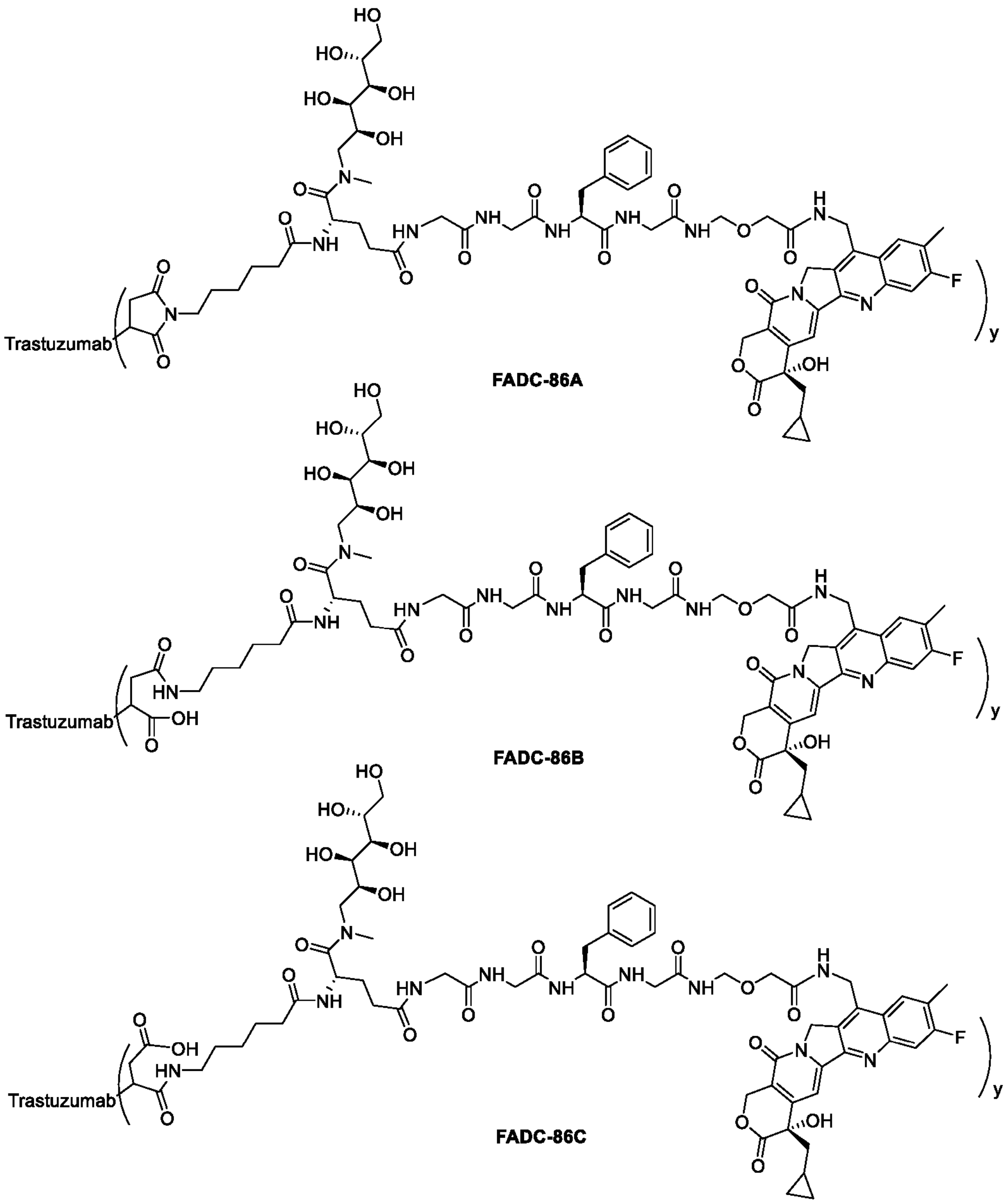

FADC-86A

FADC-86B

FADC-86C

(continued)

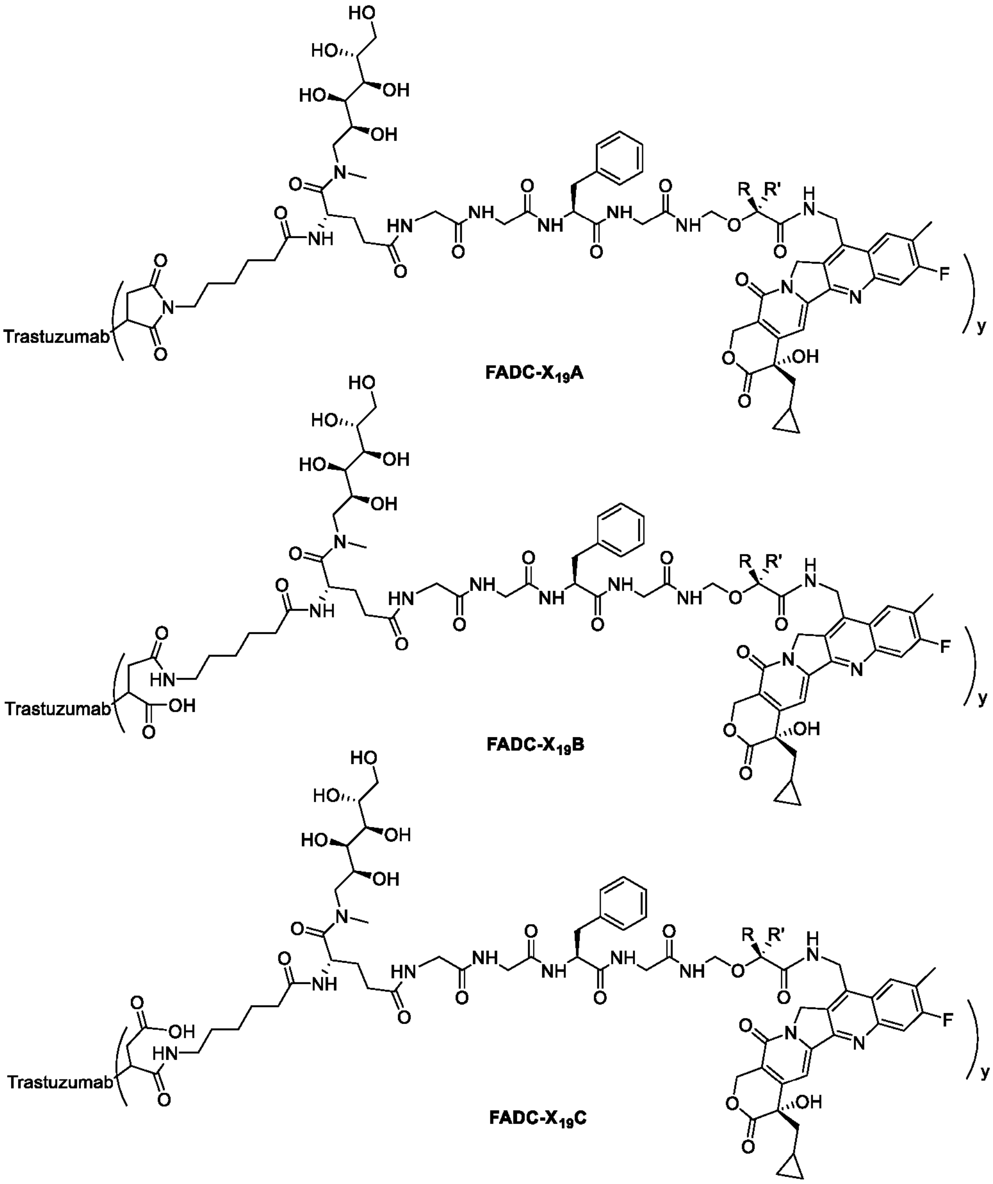

FADC-$X_{19}$A

FADC-$X_{19}$B

FADC-$X_{19}$C

wherein, R is selected from $-CH_3$, R' is selected from -H;
R is selected from -H, R' is selected from $-CH_3$;

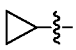,

R is selected from R' is selected from -H; or

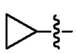;

R is selected from -H, R' is selected from

(continued)

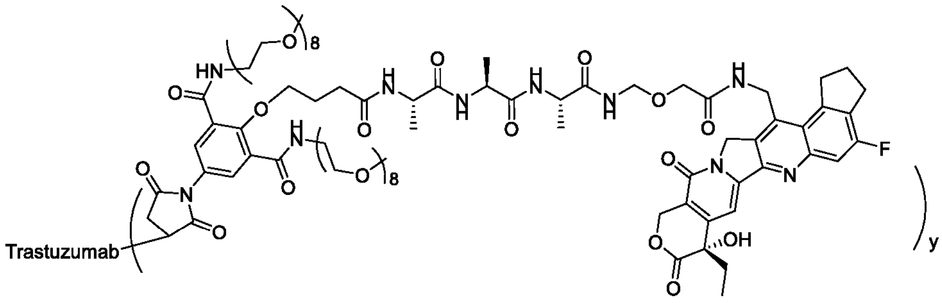

FADC-91A

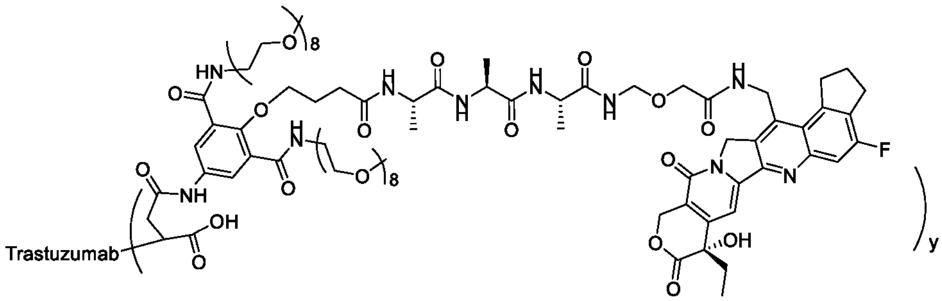

FADC-91B

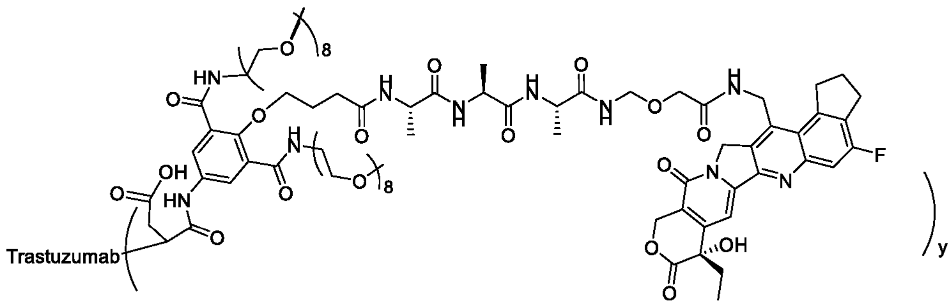

FADC-91C

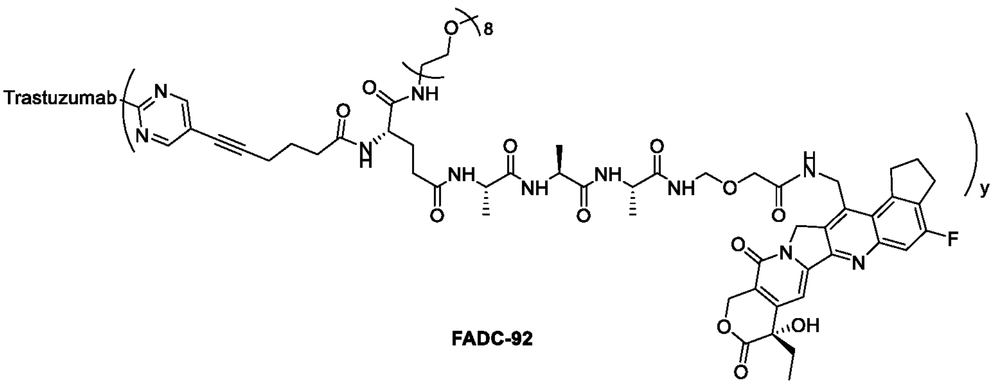

FADC-92

(continued)

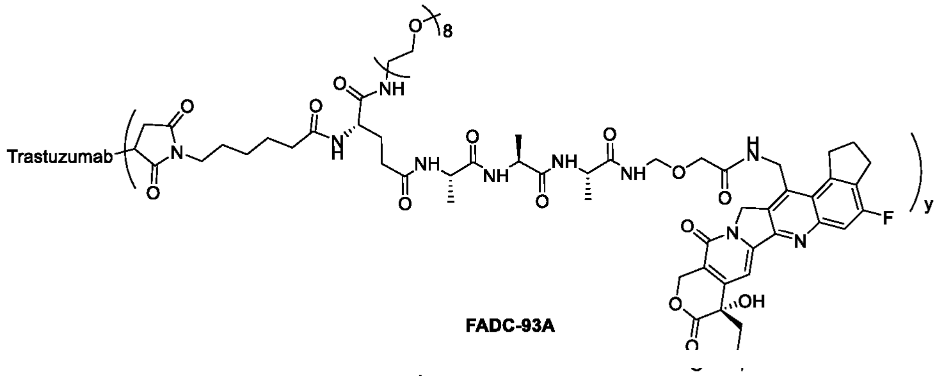

FADC-93A

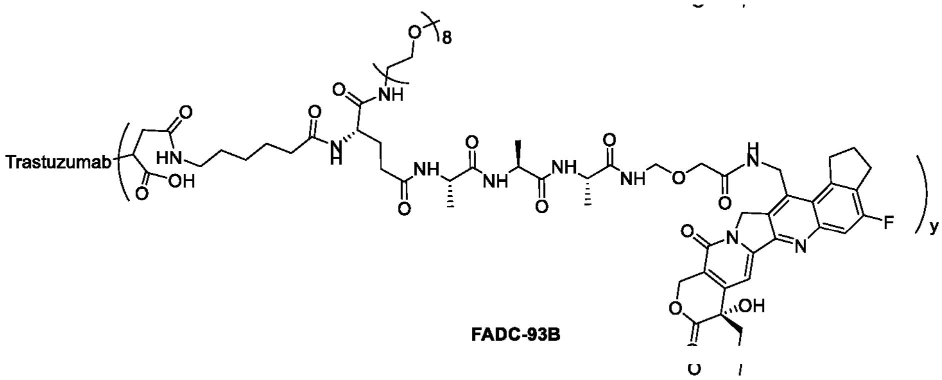

FADC-93B

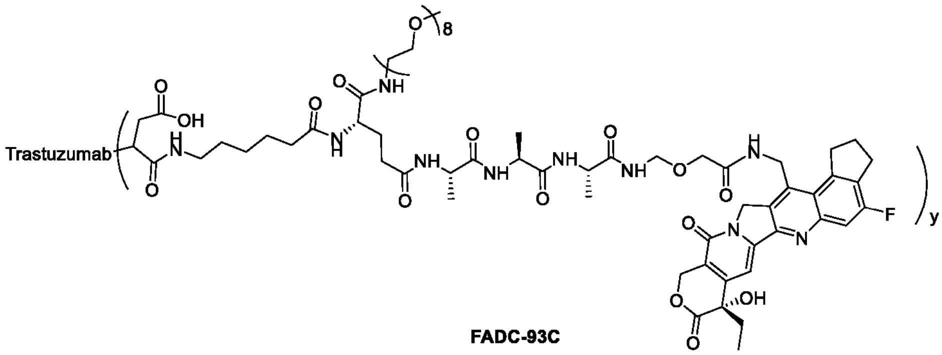

FADC-93C

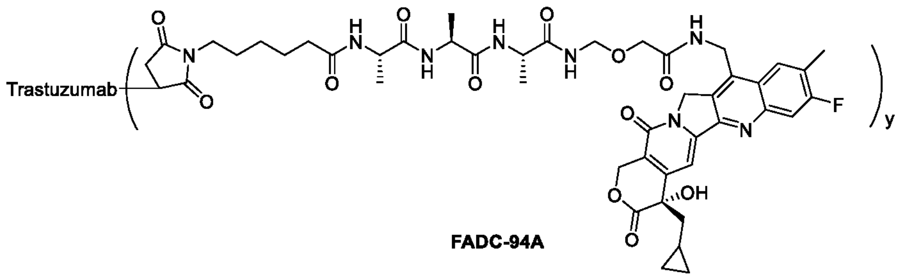

FADC-94A

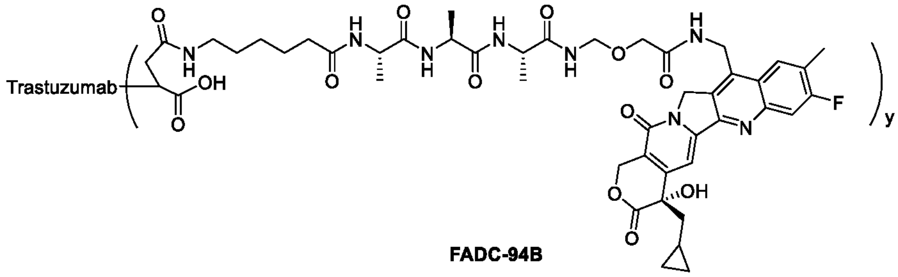

FADC-94B

(continued)

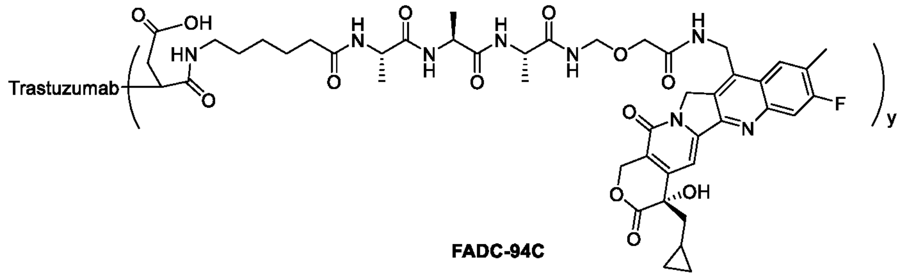

FADC-94C

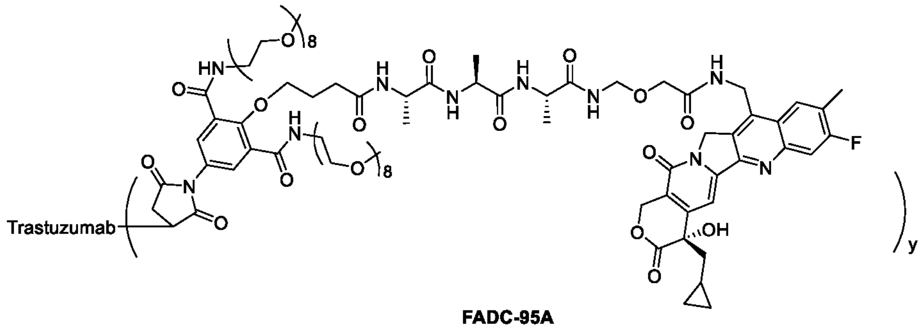

FADC-95A

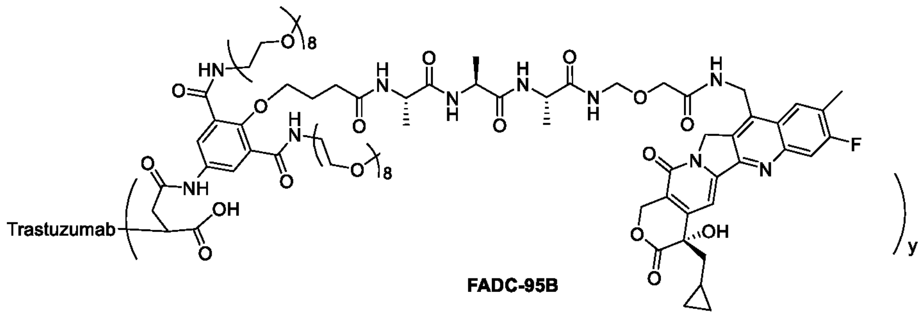

FADC-95B

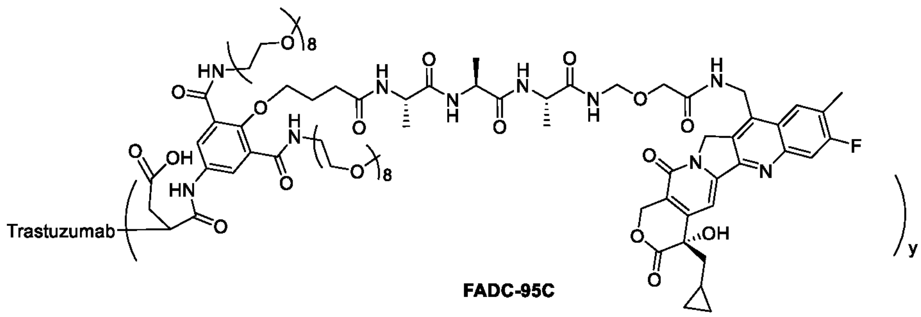

FADC-95C

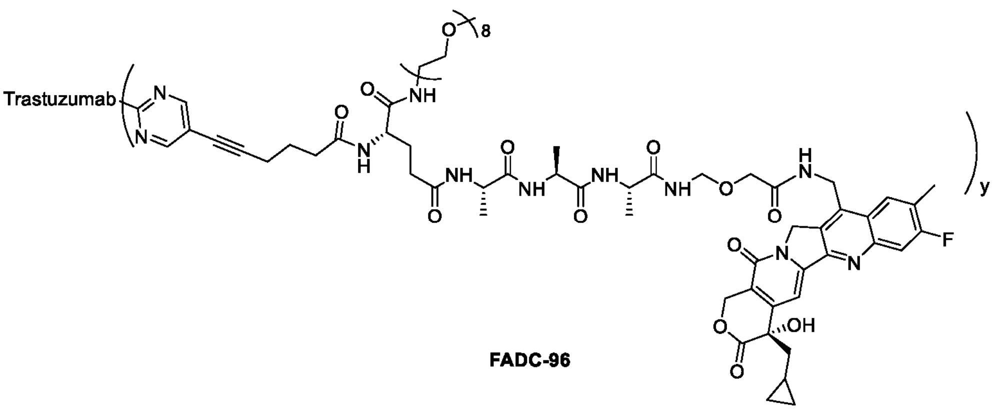

FADC-96

(continued)

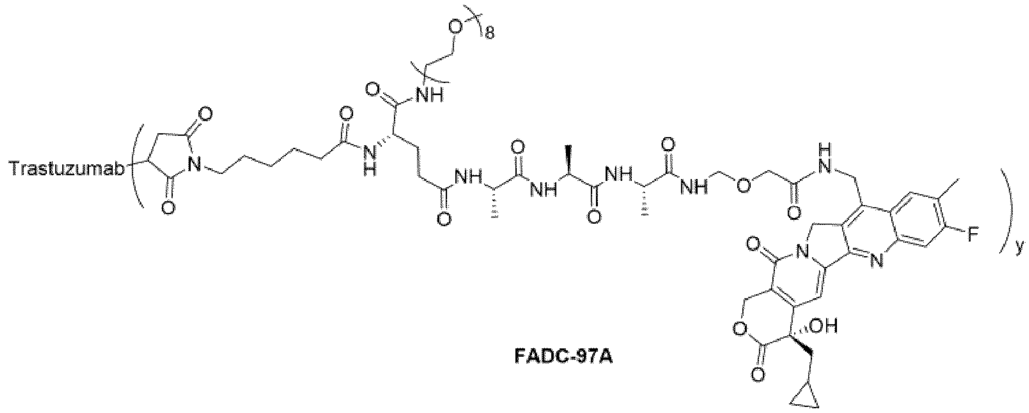

FADC-97A

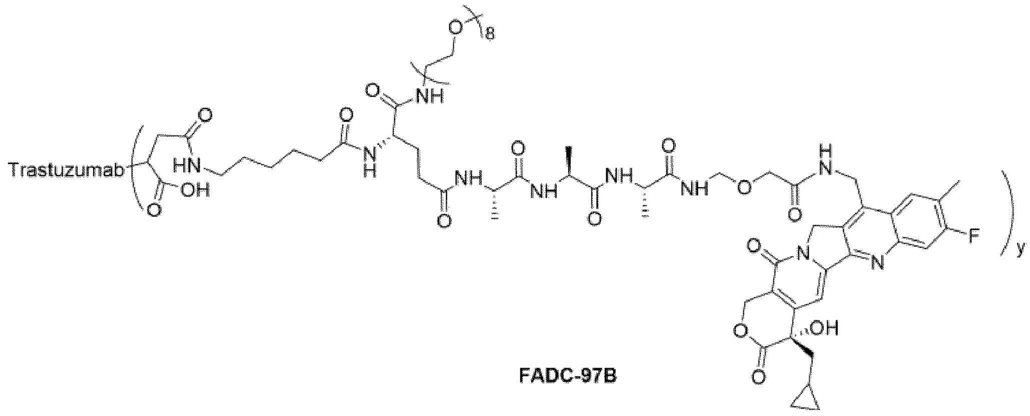

FADC-97B

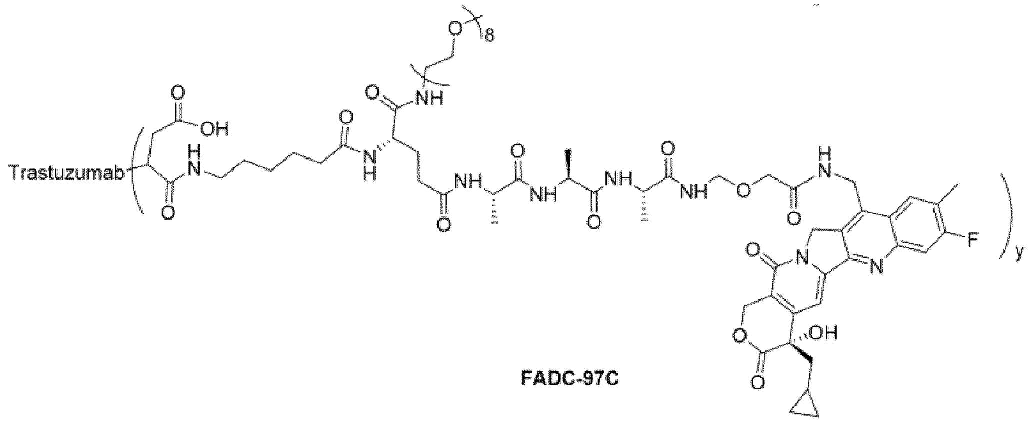

FADC-97C

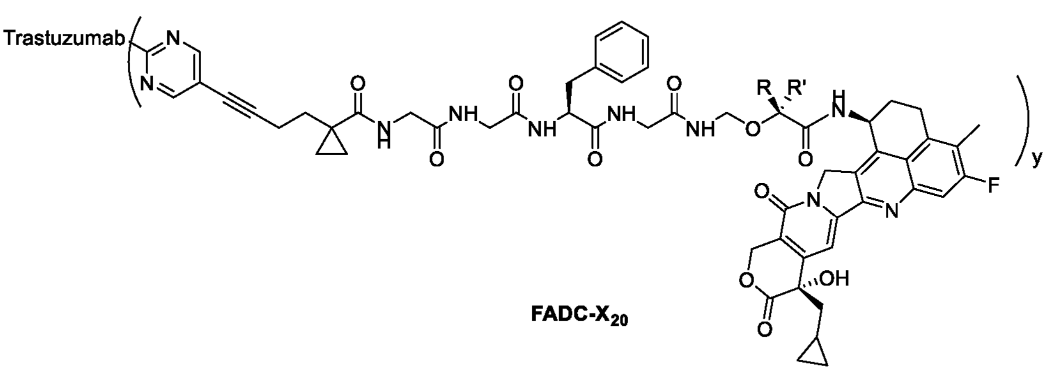

FADC-$X_{20}$

wherein, R = -H, R' = -H;
R = $-CH_3$, R' = -H;
R = -H, R' = $-CH_3$;

(continued)

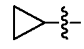

,

R = R' = -H; or

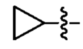

;

R = -H, R' =

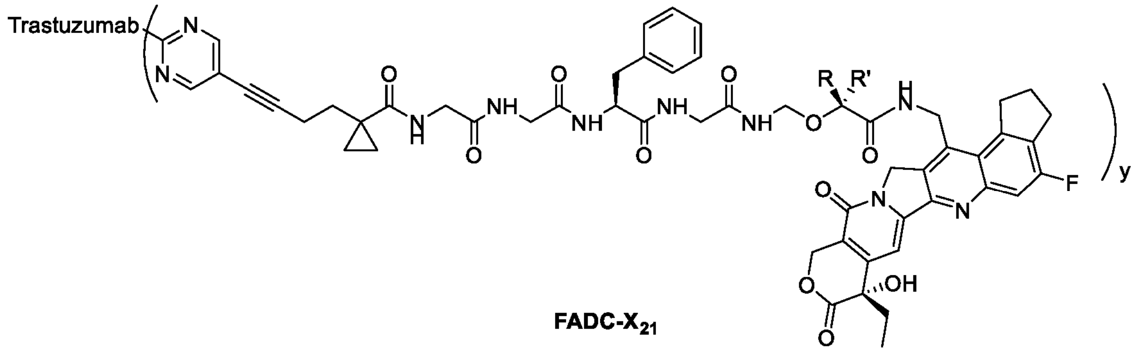

**FADC-X$_{21}$**

wherein, R = -H, R' = -H;
R = -$CH_3$, R' = -H; or
R = -H, R' = -$CH_3$;

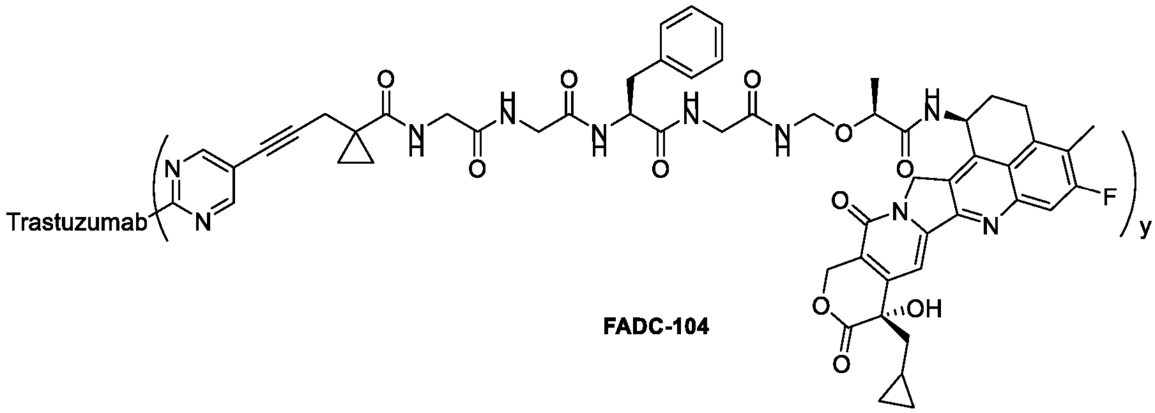

**FADC-104**

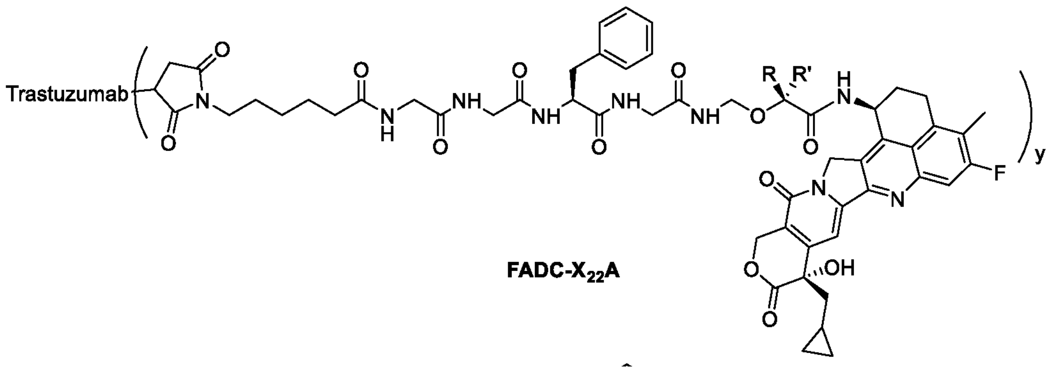

**FADC-X$_{22}$A**

(continued)

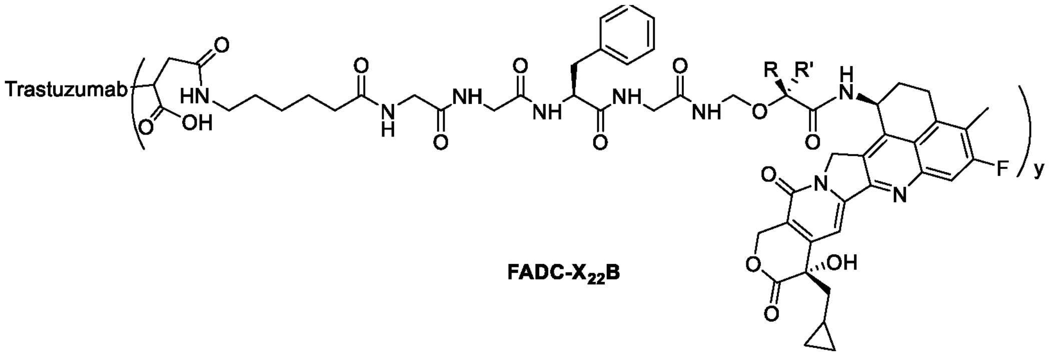

FADC-$X_{22}$B

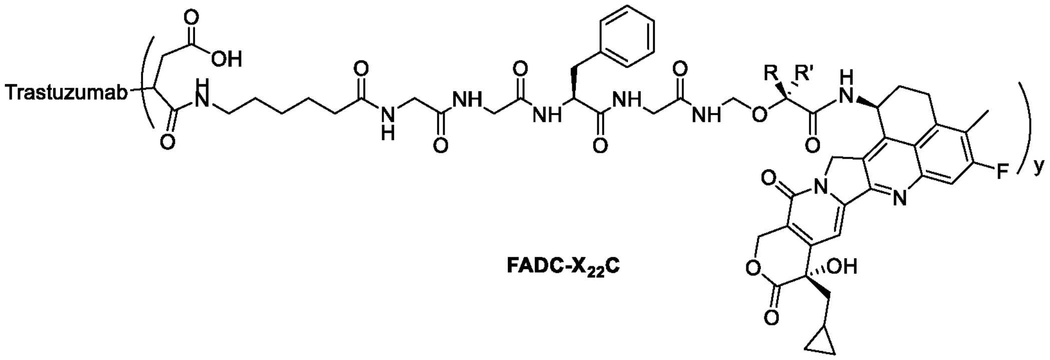

FADC-$X_{22}$C

wherein, R = -H, R' = -$CH_3$; or
R = -$CH_3$, R' = -H;

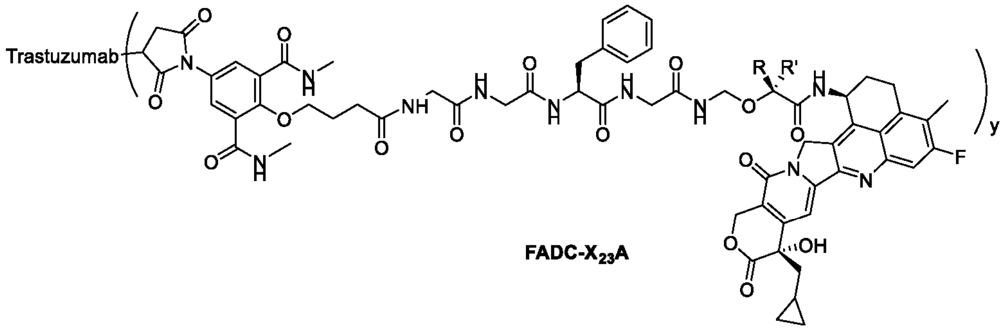

FADC-$X_{23}$A

(continued)

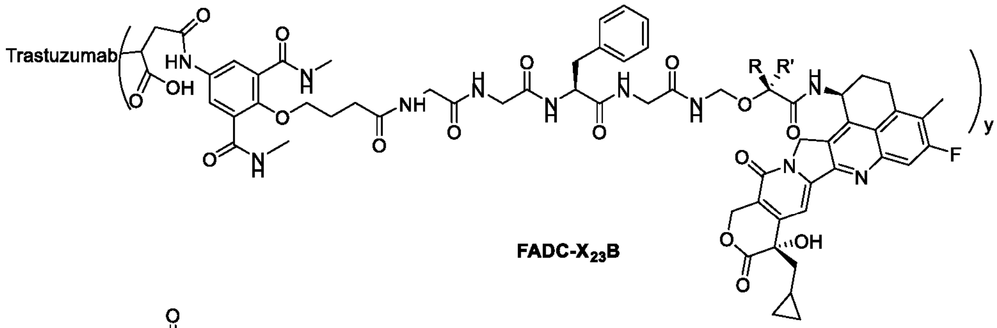

FADC-X$_{23}$B

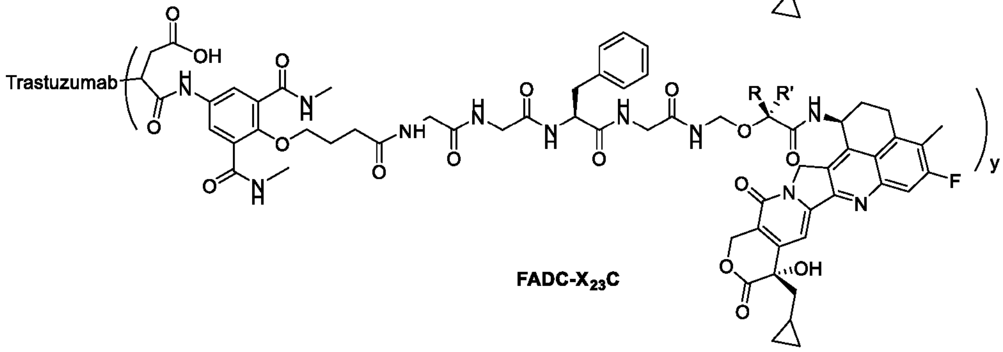

FADC-X$_{23}$C

wherein, R = -H, R' = -H;
R = -$CH_3$, R' = -H;

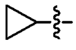,

R = R' = -H; or

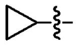;

R = -H, R' =

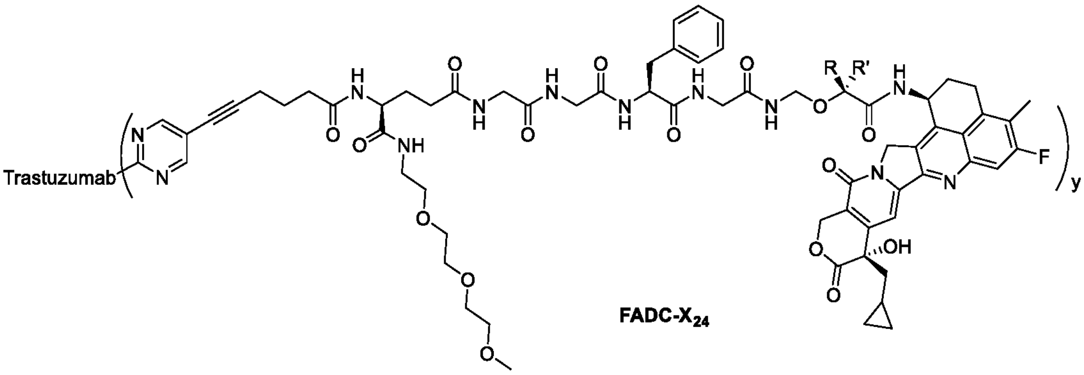

FADC-X$_{24}$

wherein, R = -H, R' = -H;
R = -$CH_3$, R' = -H;
R = -H, R' = -$CH_3$;

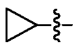,

R = R' = -H; or

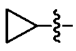;

R = -H, R' =

(continued)

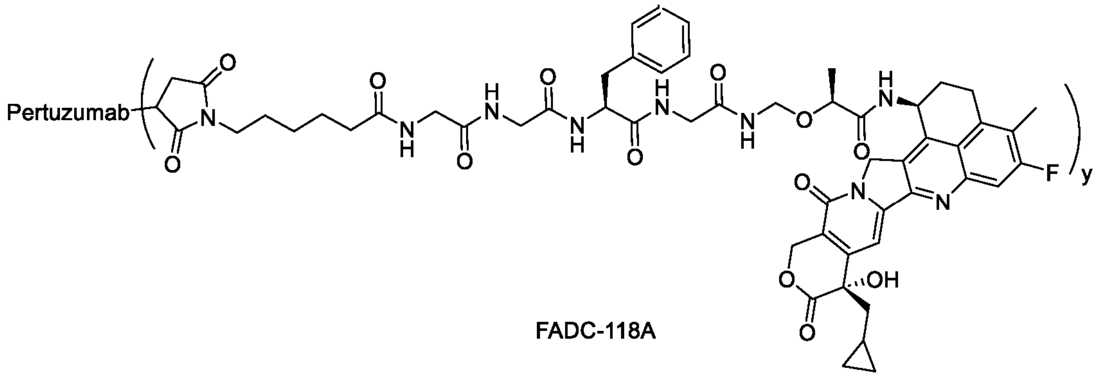

FADC-118A

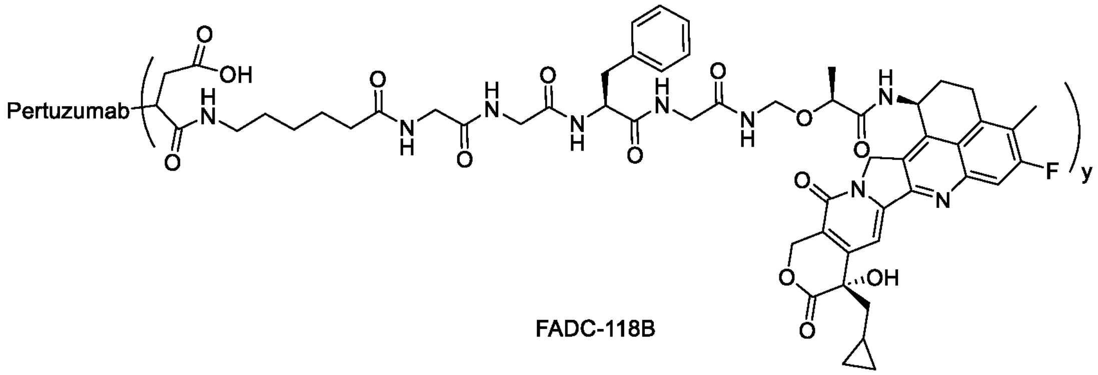

FADC-118B

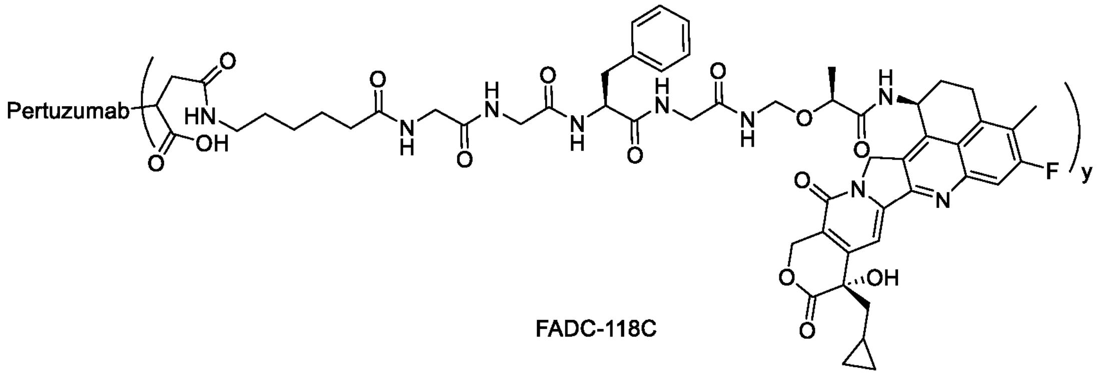

FADC-118C

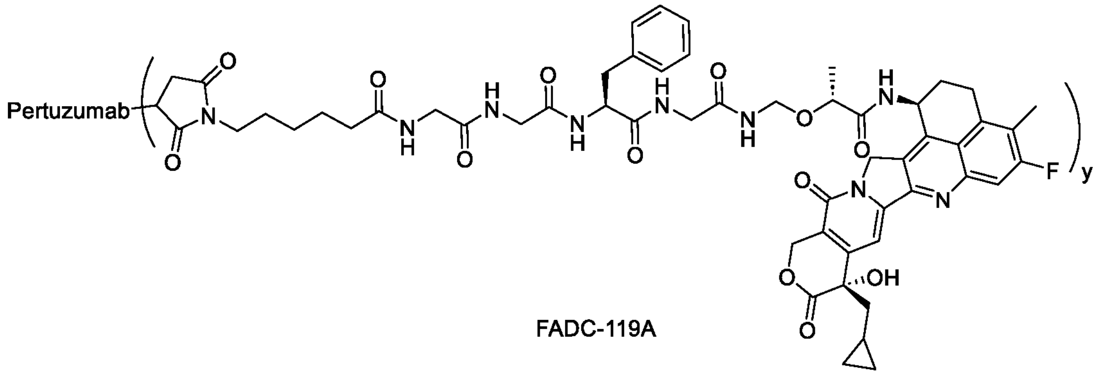

FADC-119A

(continued)

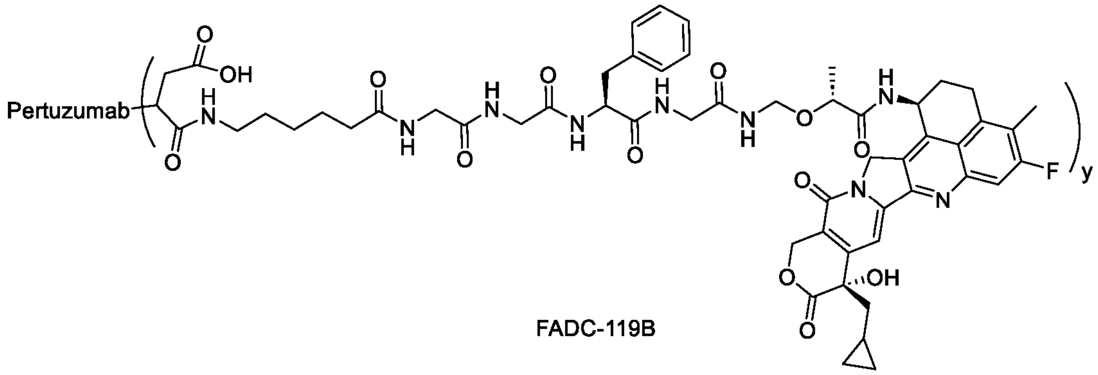

FADC-119B

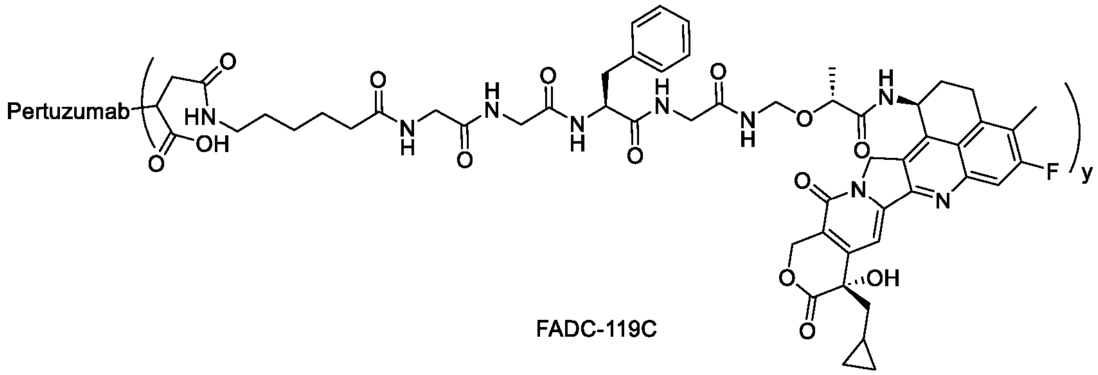

FADC-119C

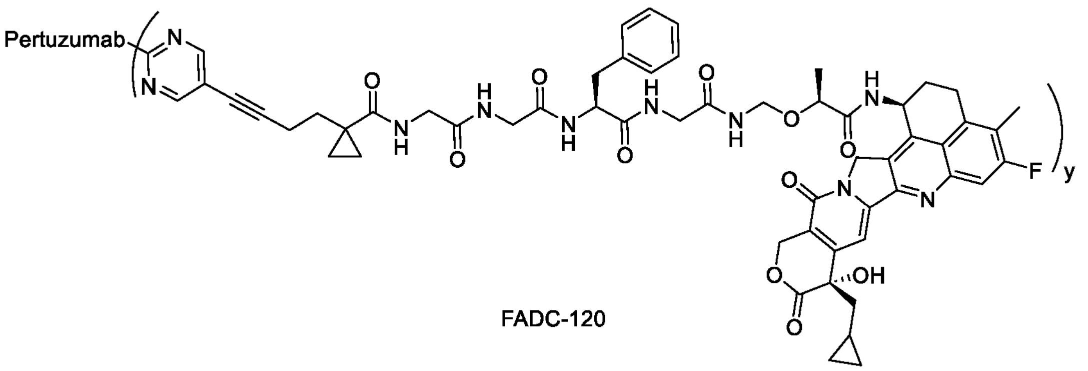

FADC-120

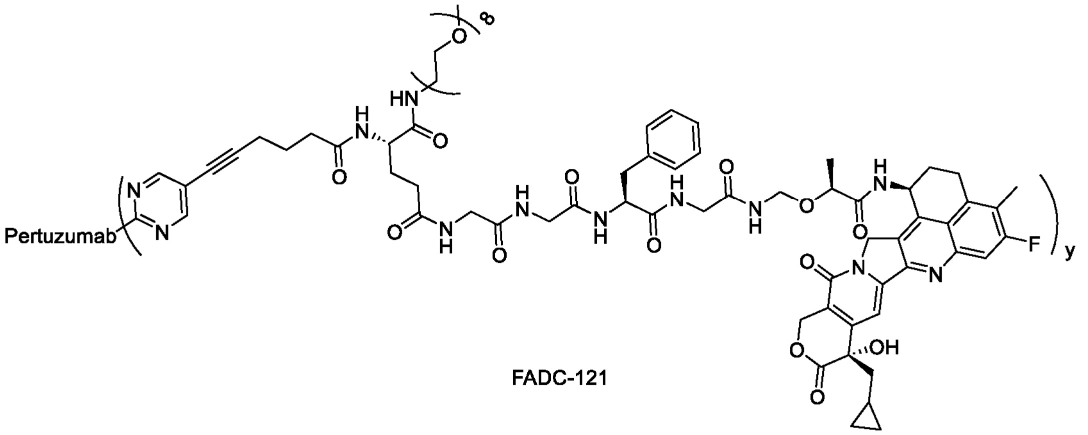

FADC-121

(continued)

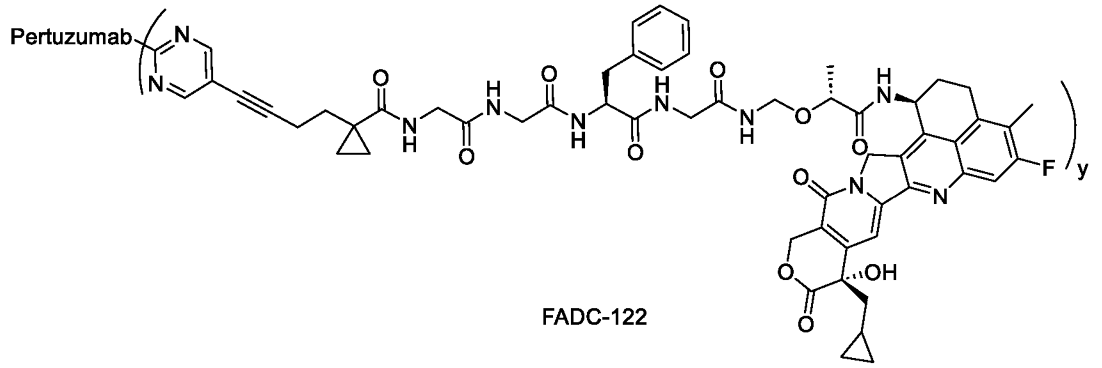

FADC-122

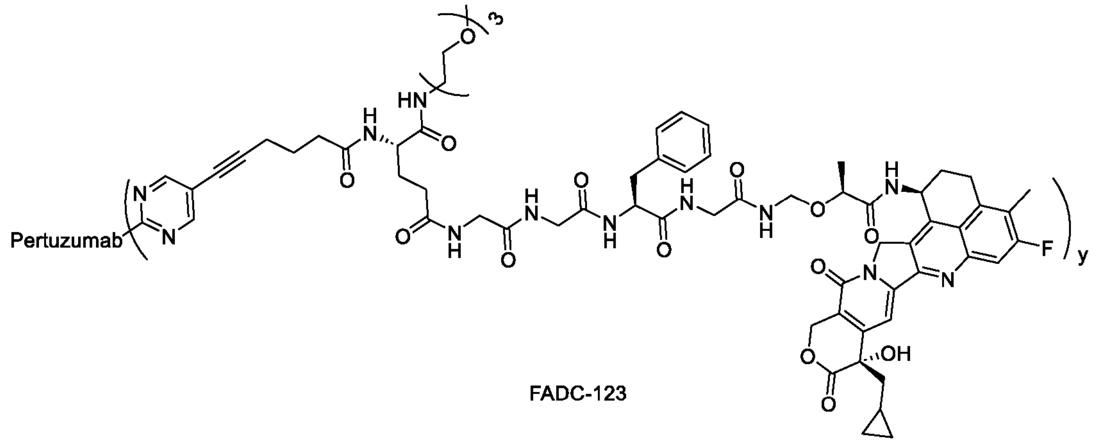

FADC-123

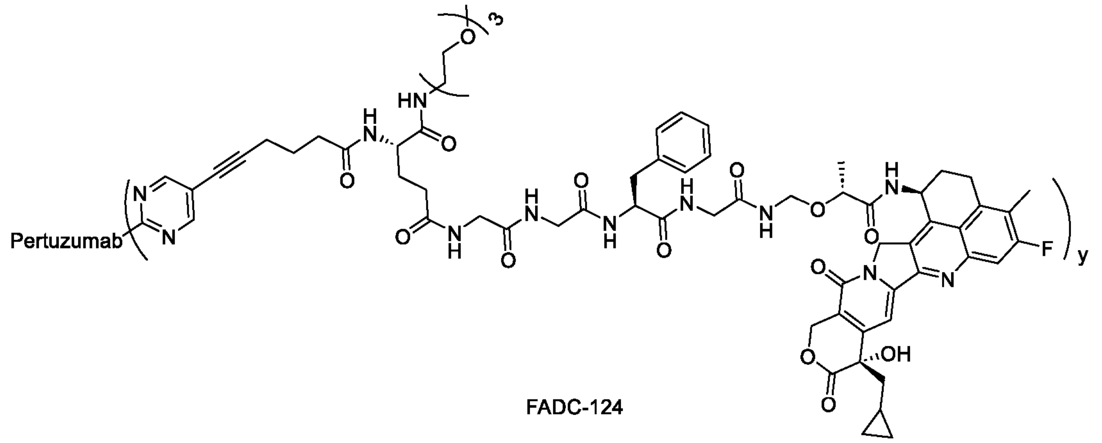

FADC-124

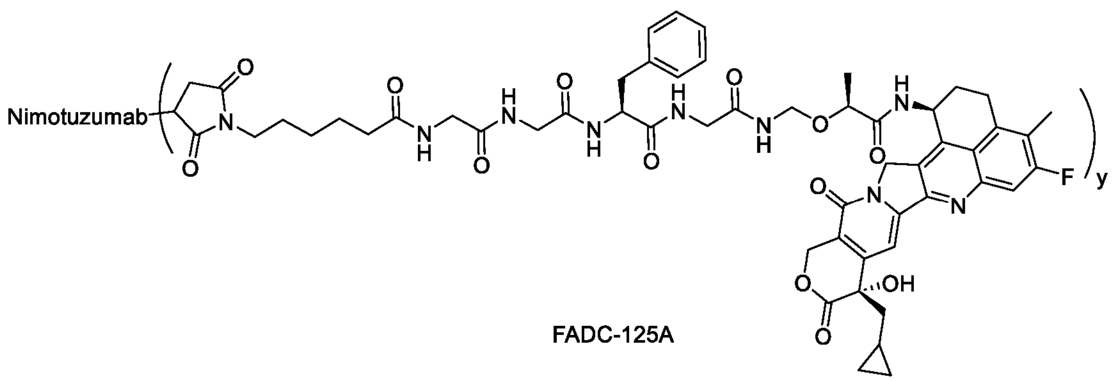

FADC-125A

(continued)

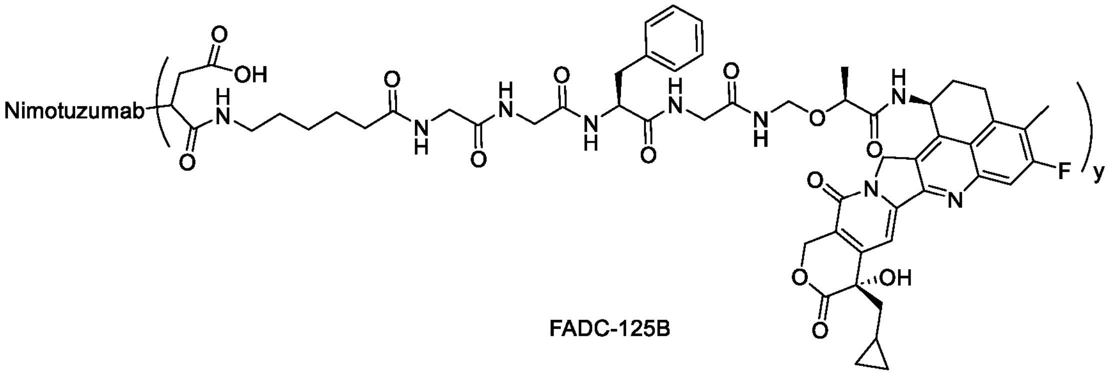

FADC-125B

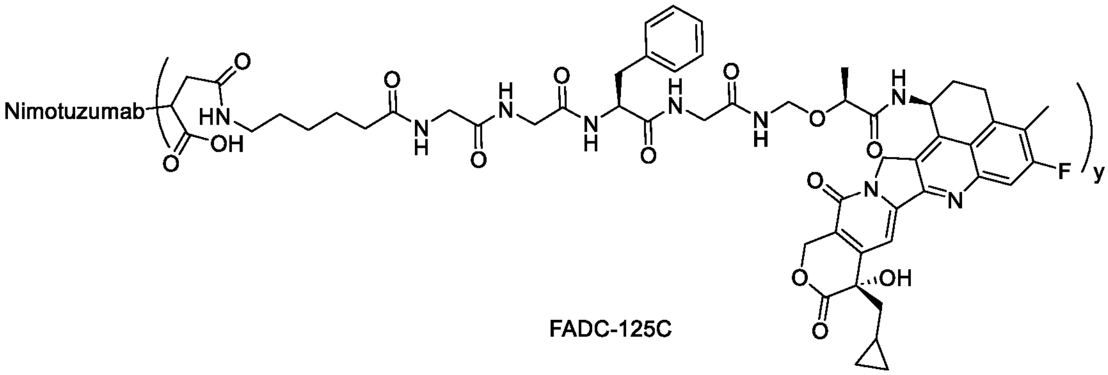

FADC-125C

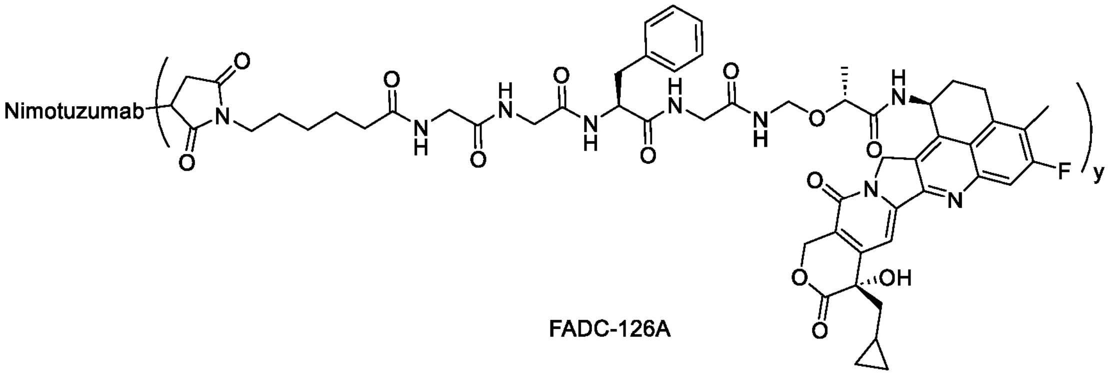

FADC-126A

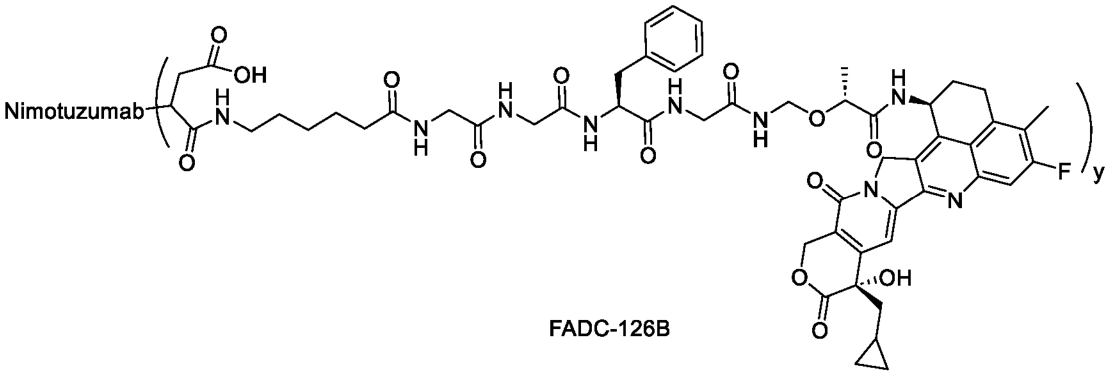

FADC-126B

(continued)

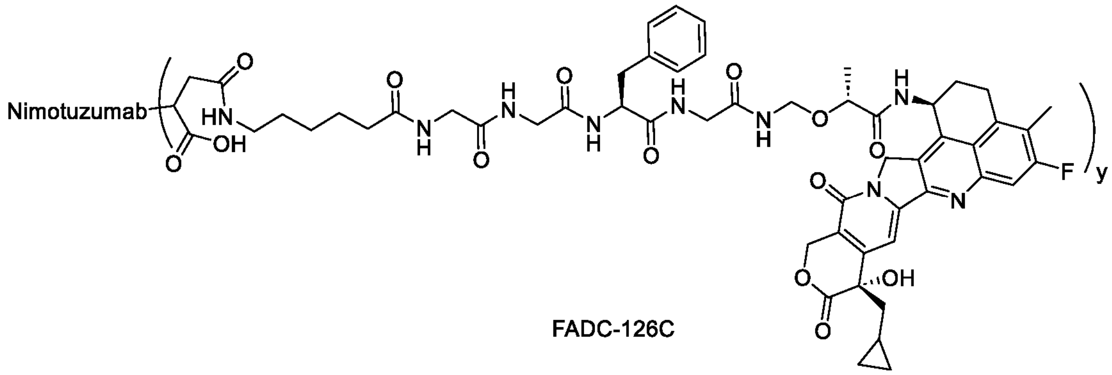

FADC-126C

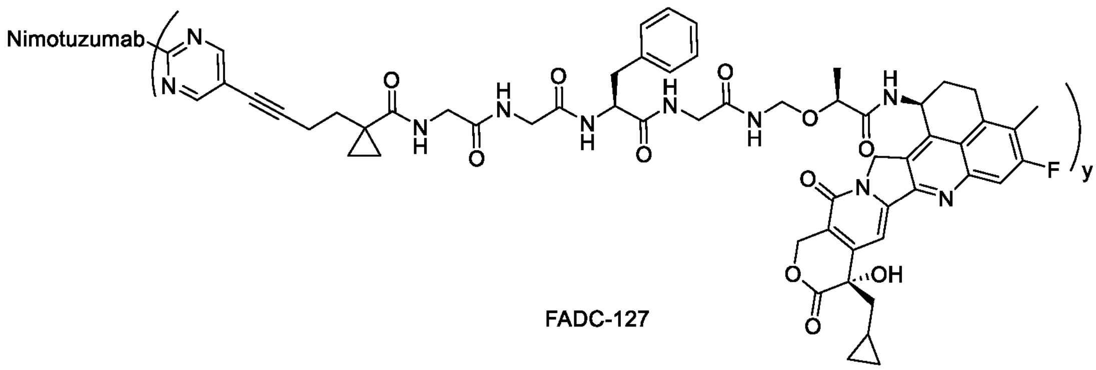

FADC-127

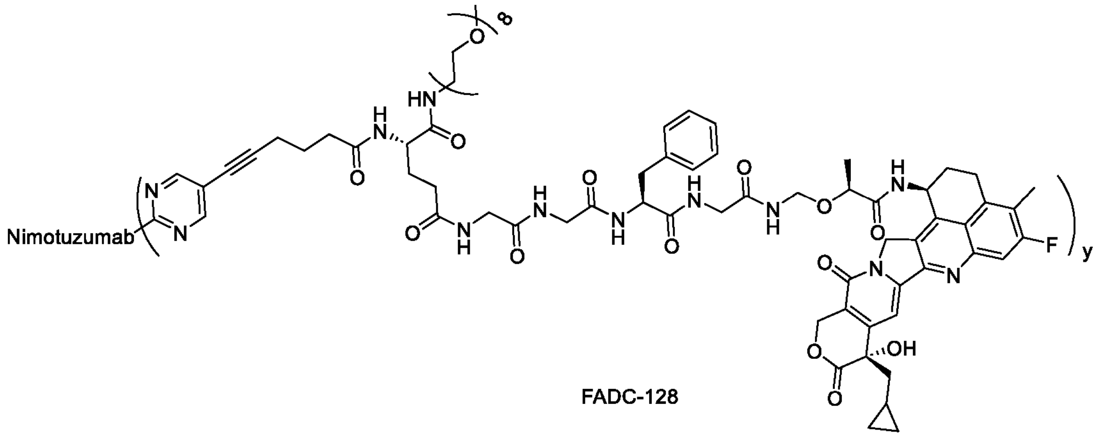

FADC-128

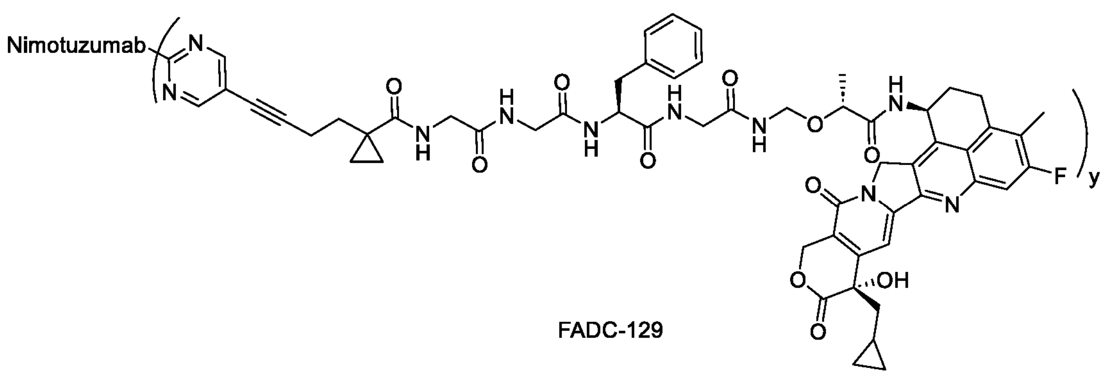

FADC-129

(continued)

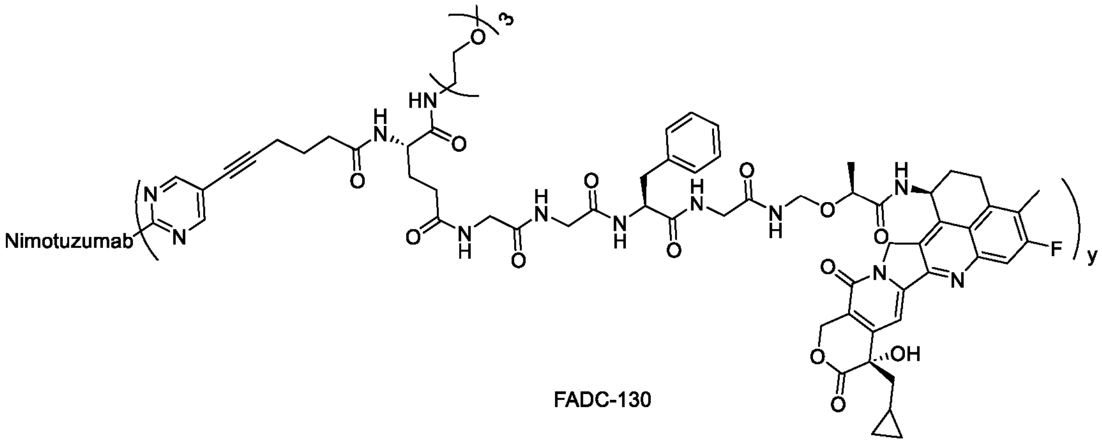

FADC-130

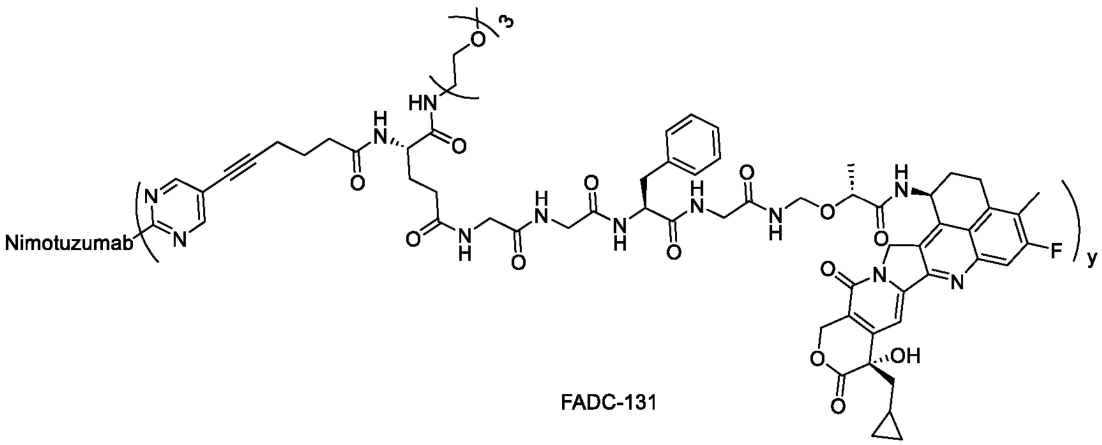

FADC-131

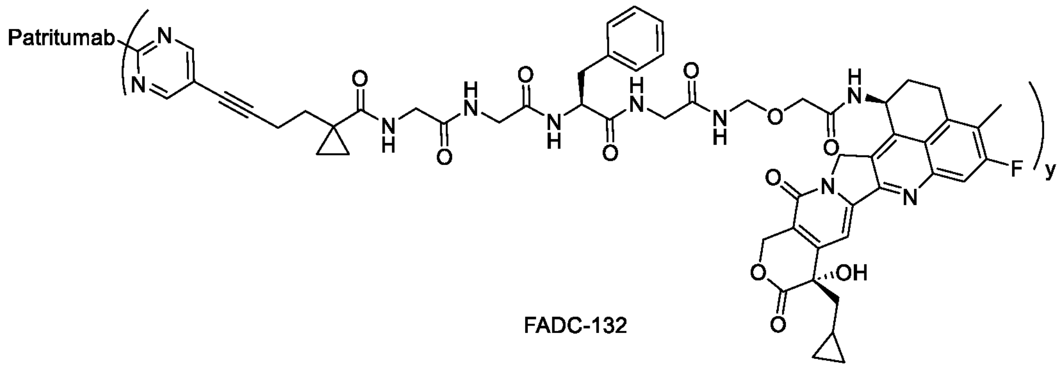

FADC-132

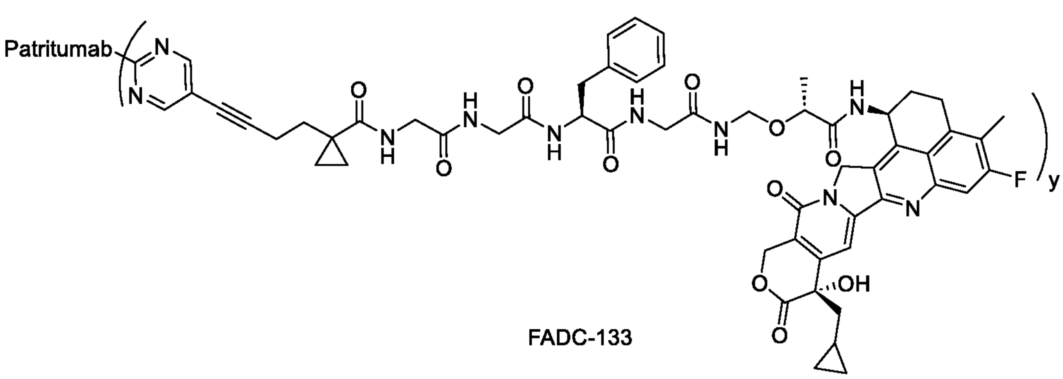

FADC-133

(continued)

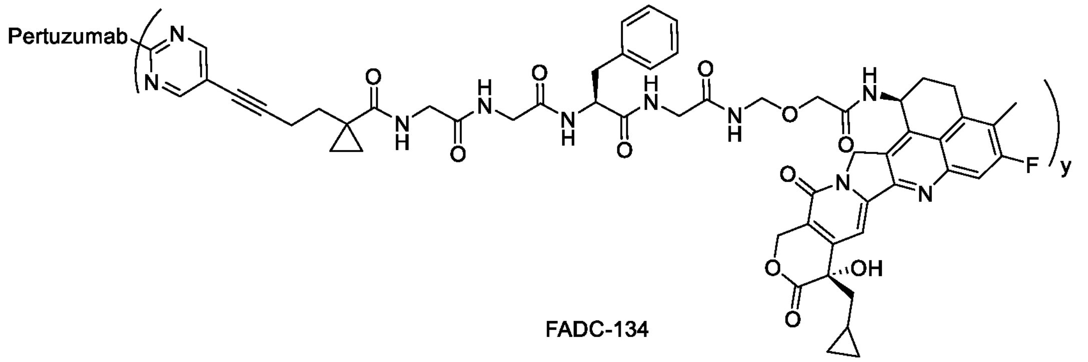

FADC-134

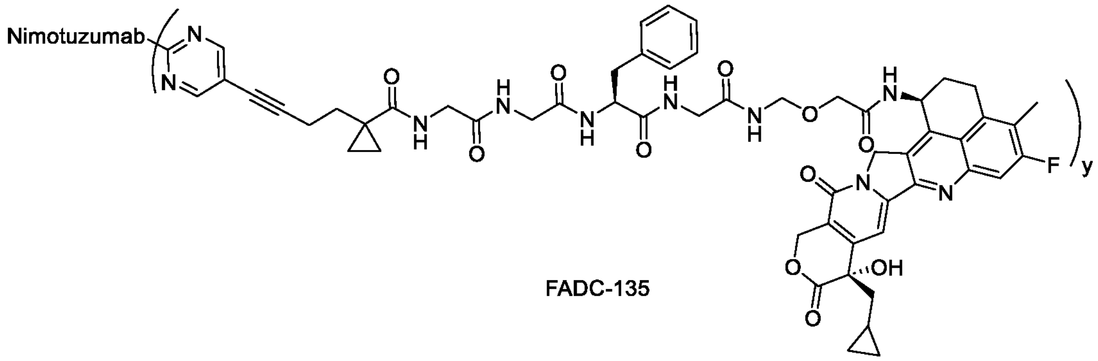

FADC-135

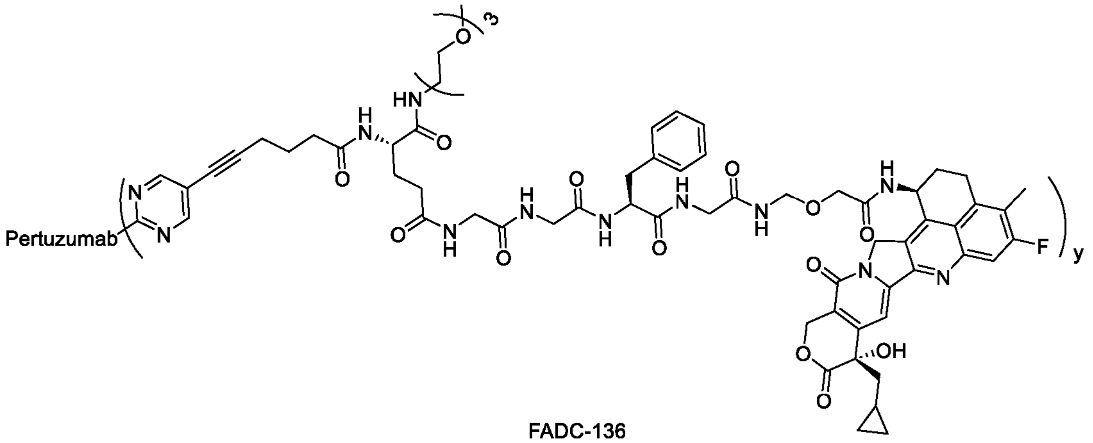

FADC-136

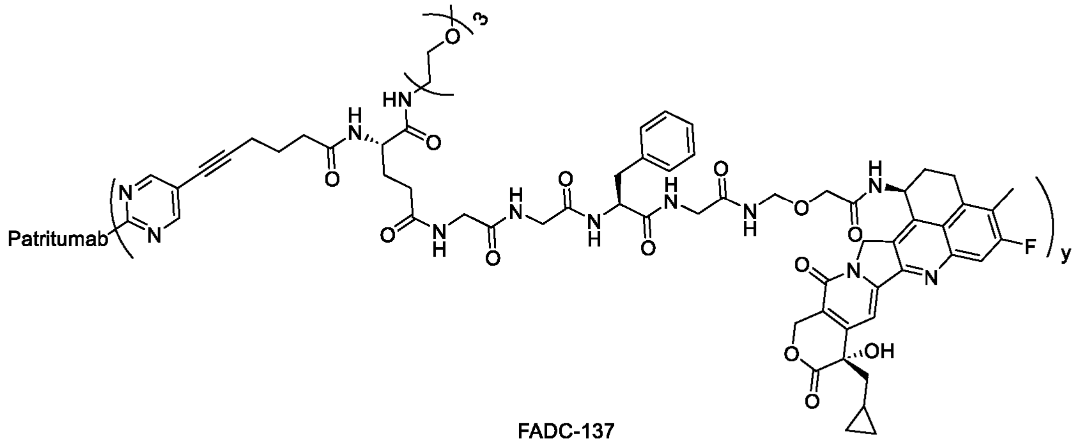

FADC-137

(continued)

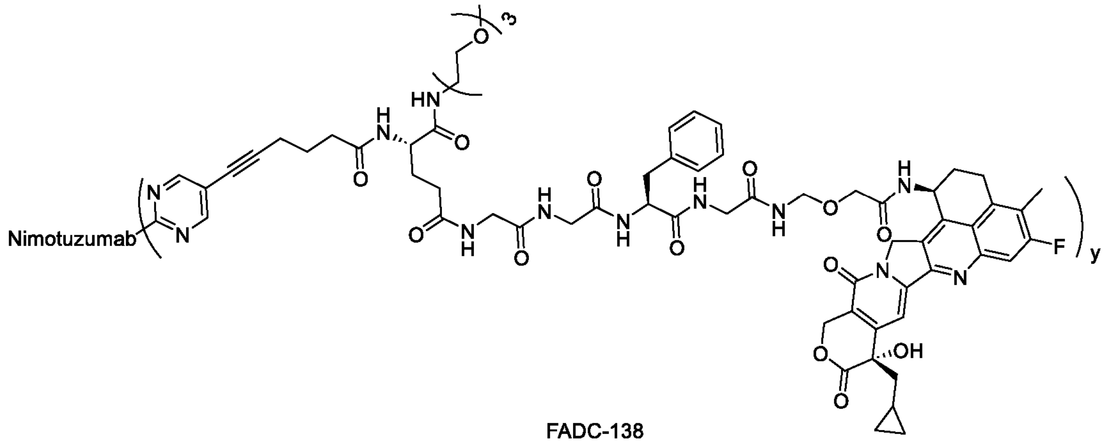

FADC-138

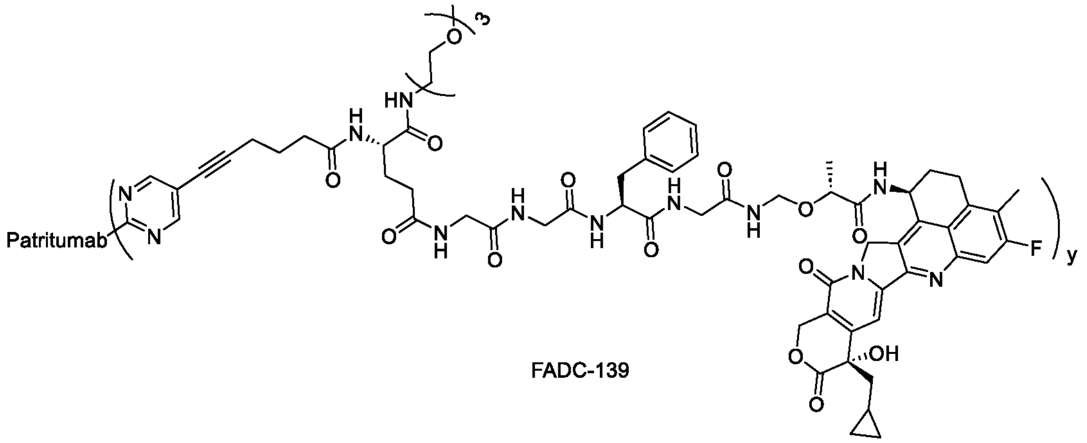

FADC-139

The present invention also provides a method for preparing the conjugate, or a stereoisomer, racemate, tautomer, isotopologue, isotope-labeled compound, nitroxide or pharmaceutically acceptable salt thereof, wherein the preparation method comprises the following steps:

Step 1: providing a linker represented by $L^{1'}$-$L^2$-$L^3$-$L^4$-$L^{5'}$ (L');

preferably, in the linker,

$L^4$-$L^{5'}$ is: -$NR^{z1}$-$C(R^{z2}R^{z3})$-$T^L$- in its carboxylic acid form or active ester form;

wherein:

$R^{z1}$ is H or $C_{1-4}$ alkyl;

$R^{z2}$ and $R^{z3}$ are the same or different and are independently selected from H or $C_{1-4}$ alkyl;

further preferably:

$L^4$-$L^{5'}$ is: -$NR^{z1}$-$C(R^{z2}R^{z3})$-O-$(CH_2)_m$-$C(R^{z4}R^{z5})$-CO- in its carboxylic acid form or active ester form;

wherein:

$R^{z1}$ is H or $C_{1-4}$ alkyl;

m is an integer selected from 0 to 4;

$R^{z2}$ and $R^{z3}$ are the same or different and are independently selected from H or $C_{1-4}$ alkyl;

$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl;

Step 2: coupling the linker with an intermediate compound represented by Formula (G') to obtain a coupled intermediate represented by $L^{1'}$-$L^2$-$L^3$-$L^4$-$L^5$-G (C');

wherein, the structure of the intermediate compound as represented by Formula (G') is:

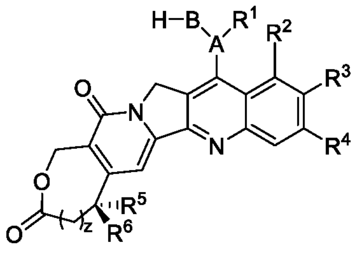

(G')

wherein, A, B, $T^L$, $R^1$, $R^2$, $R^3$, $R^4$, $R^5$, $R^6$, $L^{1'}$, $L^1$, $L^2$, $L^3$, $L^4$, $L^5$, and $L^{5'}$ independently have the definitions as described above;

preferably, the preparation method further comprises Step 3: coupling the coupled intermediate represented by Formula (C') with the targeting moiety Tp;

optionally, if necessary, functional groups of reaction substrates can also be protected with a protecting group known in the art to allow the reaction to proceed, and the protecting group is removed after the reaction.

According to an embodiment of the present invention, the protecting group may be selected from protecting groups known in the art, such as a hydroxy protecting group or an amino protecting group, to allow the reaction to proceed.

The present disclosure also provides a compound obtained by connecting the above-mentioned group G to a hydrogen atom, wherein $T^L$ of the group G is connected to the hydrogen atom.

The present disclosure also provides a compound represented by the following Formula (GH), or a stereoisomer, racemate, tautomer, isotopologue, isotope-labeled compound, nitroxide or pharmaceutically acceptable salt thereof:

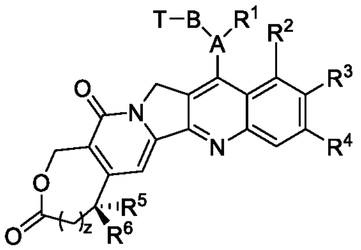

(GH)

wherein, A is selected from N or C-$R^A$;

when A is N, $R^1$ is selected from the group consisting of hydrogen, hydroxyl, and the following groups which are unsubstituted or substituted with one, two or more $R^B$: alkyl, cycloalkyl, cycloalkylalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

when A is C-$R^A$, $R^A$ and $R^1$ are the same or different and are independently selected from the group consisting of hydrogen, halogen, hydroxyl, sulfhydryl, cyano, nitro, and the following groups which are unsubstituted or substituted with one, two or more $R^C$: alkyl, cycloalkyl, cycloalkylalkyl, alkyloxy, cycloalkyloxy, alkyloxyalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

or, $R^A$ and $R^1$, together with the atom to which they are attached, form a 3- to 10-membered ring structure which is unsubstituted or substituted with one, two or more $R^D$;

z is selected from 0 or 1;

$R^2$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

$R^3$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

or, $R^1$ and $R^2$, together with the atoms to which they are attached, form a ring structure which is unsubstituted or substituted with one, two or more $R^E$, for example, the ring structure is a 5- to 10-membered ring structure, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a ring structure which is unsubstituted or substituted with one, two or more $R^F$, for example, the ring structure is a 4- to 10-membered ring structure, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of alkyl, alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: cycloalkyl, cycloalkylalkyl, cycloalkyloxy, heterocyclyl, heterocyclylalkyl, heterocyclyloxy, alkyl, alkyloxy, aryl, arylalkyl, aryloxy, heteroaryl, heteroarylalkyl, heteroaryloxy, amino;

B is absent or selected from the group consisting of triazolyl, $-NR^7-$ or $-N(NR^7R^8)-$;

$R^7$ and $R^8$ are the same or different and are independently selected from the group consisting of hydrogen and the following groups which are unsubstituted or substituted with one, two or more $R^H$: alkyl, cycloalkyl, cycloalkylalkyl, aryl, arylalkyl, heteroaryl, heteroarylalkyl, heterocyclyl, heterocyclylalkyl, HC(=O)-, alkyl-C(=O)-, cycloalkyl-C(=O)NH-, heterocyclyl-C(=O)NH-, aryl-C(=O)NH-, heteroaryl-C(=O)NH-;

$T^L$ is selected from the group consisting of chemical bond, $\#O-(CR^9R^{10})_m-(CR^{11}R^{12})_n-C(=O)*$, $\#O-(CR^9R^{10})_m-(CR^{11}R^{12})_n*$, $\#N(R^{13})-(CR^9R^{10})_m-(CR^{11}R^{12})_n-C(O)*$, $\#S-(CR^9R^{10})_m-(CR^{11}R^{12})_n-C(O)*$, $\#O-(CR^9R^{10})_m-(CR^{11}R^{12})_n-C(NR^{13})*$, $\#O-(CR^9R^{10})_m-(CR^{11}R^{12})_n-P(=O)(OR^{14})*$, $*O-(CR^9R^{10})_m-(CR^{11}R^{12})_n-C(=O)\#$, $*O-(CR^9R^{10})_m-(CR^{11}R^{12})_n\#$, $*N(R^{13})-(CR^9R^{10})_m-(CR^{11}R^{12})_n-C(O)\#$, $*S-(CR^9R^{10})_m-(CR^{11}R^{12})_n-C(O)\#$, $*O-(CR^9R^{10})_m-(CR^{11}R^{12})_n-C(NR^{13})\#$, $*O-(CR^9R^{10})_m-(CR^{11}R^{12})_n-P(=O)(OR^{14})\#$, wherein # represents the site attached to L, and * represents the site attached to B;

each of $R^9$, $R^{10}$, $R^{11}$ and $R^{12}$ is the same or different, and independently selected from the group consisting of hydrogen, halogen, cyano, and the following groups which are unsubstituted or substituted with one, two or more $R^I$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy;

or, $R^9$ and $R^{10}$, together with the atoms to which they are attached, form a ring structure which is unsubstituted or substituted with one, two or more $R^I$, for example, the ring structure is a 3- to 10-membered ring structure; or, $R^{11}$ and $R^{12}$, together with the atoms to which they are attached, form a ring structure which is unsubstituted or substituted with one, two or more $R^I$, for example, the ring structure is a 3- to 10-membered ring structure; or, $R^9$ and $R^{11}$, together with the atoms to which they are attached, form a ring structure which is unsubstituted or substituted with one, two or more $R^I$, for example, the ring structure is a 3- to 10-membered ring structure; wherein any of the ring structures can be a monocyclic hydrocarbyl, bicyclic hydrocarbyl, heteromonocyclic hydrocarbyl, or heterobicyclic hydrocarbyl, each of which is unsubstituted or substituted with one, two or more $R^I$; for example, any of the ring structures can be the following group which is unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: alkyl, cycloalkyl, cycloalkylalkyl, aryl, arylalkyl, heteroaryl, heteroarylalkyl, heterocyclyl, heterocyclylalkyl;

each m is the same or different, and is independently selected from an integer of 0 to 10;

each n is the same or different, and is independently selected from an integer of 0 to 10;

each of $R^B$, $R^C$, $R^D$, $R^E$, $R^F$, $R^G$, $R^H$, $R^I$ and $R^J$ is the same or different, and is independently selected from the group consisting of deuterium, halogen, hydroxyl, cyano, nitro, and the following groups which are unsubstituted or substituted with one, two or more $R^K$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^L$ is the same or different and is independently selected from the group consisting of deuterium, halogen, hydroxyl, cyano, nitro, alkyl, aryloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

provided that, when $R^6$ is selected from the group consisting of alkyl, alkyloxy, aryl, arylalkyl, aryloxy, heteroaryl, heteroarylalkyl, heteroaryloxy or amino, each of which is unsubstituted or substituted with one, two or more $R^G$:

A is N; $R^1$ and $R^2$, together with the atoms to which they are attached, do not form a 5- to 10-membered ring structure which is unsubstituted or substituted with one, two or more $R^E$; and $T^L$ is not a chemical bond when $R^7$ is hydrogen;

or, $R^1$ and $R^2$, together the atoms to which they are attached form a 5- to 10-membered ring structure which is unsubstituted or substituted with one, two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl; and $R^A$ is selected from the following groups which are unsubstituted or substituted with one, two or more $R^C$: cycloalkyl, cycloalkylalkyl, cycloalkyloxy;

or, B is $-N(NR^7R^8)-$;

or, $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl.

According to an embodiment of the present invention, definitions of the groups in Formula (GH) may be independently selected from:

A is selected from N or C-$R^A$;

when A is N, $R^1$ is selected from the group consisting of hydrogen, hydroxyl, and the following groups which are unsubstituted or substituted with one, two or more $R^B$: alkyl, cycloalkyl, cycloalkylalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

when A is C-$R^A$, $R^A$ and $R^1$ are the same or different and are independently selected from the group consisting of hydrogen, halogen, hydroxyl, sulfhydryl, cyano, nitro, and the following groups which are unsubstituted or substituted with one, two or more $R^C$: alkyl, cycloalkyl, cycloalkylalkyl, alkyloxy, cycloalkyloxy, alkyloxyalkyl, heterocyclyl,

heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

or, $R^A$ and $R^1$, together with the atom to which they are attached, form a 3- to 10-membered ring structure which is unsubstituted or substituted with one, two or more $R^D$;

z is selected from 0 or 1;

$R^2$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

$R^3$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

or, $R^1$ and $R^2$, together with the atoms to which they are attached, form a 5- to 10-membered ring structure which is unsubstituted or substituted with one, two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, or heterobicyclic hydrocarbyl;

or $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, or heterobicyclic hydrocarbyl;

$R^4$ is selected from the group consisting of alkyl, alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: cycloalkyl, cycloalkylalkyl, cycloalkyloxy, heterocyclyl, heterocyclylalkyl, heterocyclyloxy;

B is absent or selected from the group consisting of triazolyl, $-NR^7-$ or $-N(NR^7R^8)-$;

$R^7$ and $R^8$ are the same or different and are independently selected from the group consisting of hydrogen, and the following groups which are unsubstituted or substituted with one, two or more $R^H$: alkyl, cycloalkyl, cycloalkylalkyl, aryl, arylalkyl, heteroaryl, heteroarylalkyl, heterocyclyl, heterocyclylalkyl, HC(=O)-, alkyl-C(=O)-, cycloalkyl-C(=O)NH-, heterocyclyl-C(=O)NH-, aryl-C(=O)NH-, heteroaryl-C(=O)NH-;

$T^L$ is selected from the group consisting of chemical bond, $\#O-(CR^9R^{10})_m-(CR^{11}R^{12})_n-C(=O)*$, $\#O-(CR^9R^{10})_m-(CR^{11}R^{12})_n*$, $\#N(R^{13})-(CR^9R^{10})_m-(CR^{11}R^{12})_n-C(O)*$, $\#S-(CR^9R^{10})_m-(CR^{11}R^{12})_n-C(O)*$, $\#O-(CR^9R^{10})_m-(CR^9R^{10})_n-C(NR^{13})*$, $\#O-(CR^9R^{10})_m-(CR^{11}R^{12})_n-P(=O)(OR^{14})*$, $*O-(CR^9R^{10})_m-(CR^{11}R^{12})_n-C(=O)\#$, $*O-(CR^9R^{10})_m-(CR^{11}R^{12})_n\#$, $*N(R^{13})-(CR^9R^{10})_m-(CR^{11}R^{12})_n-C(O)\#$, $*S-(CR^9R^{10})_m-(CR^{11}R^{12})_n-C(O)\#$, $*O-(CR^9R^{10})_m-(CR^{11}R^{12})_n-C(NR^{13})\#$, $*O-(CR^9R^{10})_m-(CR^{11}R^{12})_n-P(=O)(OR^{14})\#$, wherein # represents the site attached with L, and * represents the site attached with B;

each of $R^9$, $R^{10}$, $R^{11}$ and $R^{12}$ is the same or different and is independently selected from the group consisting of hydrogen, halogen, cyano, and the following groups which are unsubstituted or substituted with one, two or more $R^I$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; wherein any of the 3- to 10-membered ring structures can be selected from the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: alkyl, cycloalkyl, cycloalkylalkyl, aryl, arylalkyl alkyl, heteroaryl, heteroarylalkyl, heterocyclyl, heterocycloalkyl;

m and n are the same or different and are independently selected from an integer of 0 to 10;

each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^L$ is the same or different and is independently selected from the group consisting of deuterium, halogen, hydroxyl, cyano, nitro, alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-.

According to an embodiment of the present invention, definitions of the groups in Formula (GH) may be independently selected from:

A is selected from N or C-$R^A$;

when A is N, $R^1$ is selected from the group consisting of hydrogen, hydroxyl, and the following groups which are unsubstituted or substituted with one, two or more $R^B$: alkyl, cycloalkyl, cycloalkylalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

when A is C-$R^A$, $R^A$ and $R^1$ are the same or different and are independently selected from the group consisting of hydrogen, halogen, hydroxyl, sulfhydryl, cyano, nitro, and the following groups which are unsubstituted or substituted with one, two or more $R^C$: alkyl, cycloalkyl, cycloalkylalkyl, alkyloxy, cycloalkyloxy, alkyloxy alkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

or, $R^A$ and $R^1$, together with the atom to which they are attached, form a 3- to 10-membered ring structure which is unsubstituted or substituted with one, two or more $R^D$;

z is selected from 0 or 1;

$R^2$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

$R^3$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

or, $R^1$ and $R^2$, together with the atoms to which they are attached, form a 5- to 10-membered ring structure which is unsubstituted or substituted with one, two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4-, 5-, 6-, 7-, 8-, 9- or 10-membered monocyclic hydrocarbyl, bicyclic hydrocarbyl,

heteromonocyclic hydrocarbyl, heterobicyclic hydrocarbyl;

$R^4$ is selected from alkyl, alkyloxy, halogen, hydroxyl, amino, cyano;

$R^5$ is selected from hydroxyl;

$R^6$ is selected from the following groups which are unsubstituted or substituted with one, two or more $R^G$: cycloalkyl, cycloalkylalkyl, cycloalkyloxy, heterocyclyl, heterocyclylalkyl, heterocyclyloxy;

B is absent or selected from $-NR^7-$ or $-N(NR^7R^8)-$;

$R^7$ and $R^8$ are the same or different and are independently selected from the group consisting of hydrogen, and the following groups which are unsubstituted or substituted with one, two or more $R^H$ : alkyl, cycloalkyl, cycloalkylalkyl, aryl, arylalkyl, heteroaryl, heteroarylalkyl, heterocyclyl, heterocyclylalkyl, HC(=O)-, alkyl-C(=O)-, cycloalkyl-C(=O)NH-, heterocyclyl-C(=O)NH-, aryl-C(=O)NH-, heteroaryl-C(=O)NH-;

$T^L$ is selected from the group consisting of chemical bond, $\#O-(CR^9R^{10})_m-(CR^{11}R^{12})_n-C(=O)*$, $\#O-(CR^9R^{10})_m-(CR^{11}R^{12})_n*$, $\#N(R^{13})-(CR^9R^{10})_m-(CR^{11}R^{12})_n-C(O)*$, $\#S-(CR^9R^{10})_m-(CR^{11}R^{12})_n-C(O)*$, $\#O-(CR^9R^{10})_m-(CR^{11}R^{12})_n-C(NR^{13})*$, $\#O-(CR^9R^{10})_m-(CR^{11}R^{12})_n-P(=O)(OR^{14})*$, $*O-(CR^9R^{10})_m-(CR^{11}R^{12})_n-C(=O)\#$, $*O-(CR^9R^{10})_m-(CR^{11}R^{12})_n\#$, $*N(R^{13})-(CR^9R^{10})_m-(CR^{11}R^{12})_n-C(O)\#$, $*S-(CR^9R^{10})_m-(CR^{11}R^{12})_n-C(O)\#$, $*O-(CR^9R^{10})_m-(CR^{11}R^{12})_n-C(NR^{13})\#$, $*O-(CR^9R^{10})_m-(CR^{11}R^{12})_n-P(=O)(OR^{14})\#$, wherein # represents the site attached to L, and * represents the site attached to B;

each of $R^9$, $R^{10}$, $R^{11}$ and $R^{12}$ is the same or different and is independently selected from the group consisting of hydrogen, halogen, cyano, and the following groups which are unsubstituted or substituted with one, two or more $R^I$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; wherein any of the 3- to 10-membered ring structures can be selected from, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: alkyl, cycloalkyl, cycloalkylalkyl, aryl, arylalkyl, heteroaryl, heteroarylalkyl, heterocyclyl, heterocyclylalkyl;

m and n are the same or different, and are independently selected from an integer of 0 to 10;

each of $R^B$, $R^C$, $R^D$, $R^E$, $R^F$, $R^G$, $R^H$, $R^I$ and $R^J$ is the same or different, and is independently selected from the group consisting of deuterium, halogen, hydroxyl, cyano, nitro, and the following groups which are unsubstituted or substituted with one, two or more $R^K$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^K$ is the same or different, and is independently selected from the group consisting of deuterium, halogen, hydroxyl, cyano, nitro, and the following groups which are unsubstituted or substituted with one, two or more $R^L$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^L$ is the same or different and is independently selected from the group consisting of deuterium, halogen, hydroxyl, cyano, nitro, alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-,

cycloalkyl-C(=O)NH-, heterocyclyl-C(=O)NH-, aryl-C(=O)NH-, heteroaryl-C(=O)NH-.

According to an embodiment of the present invention, in the Formula (GH) described in the context, the hydrogen, carbon or other atoms in the formula are optionally replaced by its isotopes, regardless of whether the group is defined as being selected from the above definitions or directly indicated in the formula, and regardless of whether the group is defined as a substituted group or an unsubstituted group. For example, the hydrogen in any group of the formula (GH) can be selected from $^1H$, $^2H$ or $^3H$. As an example, any hydrogen in the substituent of the unsubstituted or substituted alkyl, alkylene, alkylidene, phenyl, pyridyl or lactone group or on the ring-forming atoms (if any) in the compound of Formula (GH) can be independently selected from $^1H$, $^2H$ or$^3H$.

According to an embodiment of the present invention, definitions of the groups in Formula (GH) may be independently selected from:

A is selected from N or C-$R^A$;

when A is N, $R^1$ is selected from the group consisting of $^1H$, $^2H$, hydroxyl, deuterated hydroxyl, and the following groups which are unsubstituted or substituted with one, two or more $R^B$: $C_{1-10}$ alkyl, $C_{3-10}$ cycloalkyl, $C_{3-10}$ cycloalkyl-$C_{1-10}$ alkyl, 3- to 10-membered heterocyclyl, 3-to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is N, $R^1$ is selected from the group consisting of $^1H$, $^2H$, hydroxyl, deuterated hydroxyl, and the following groups which are unsubstituted or substituted with one, two or more $R^B$: $C_{1-6}$ alkyl, $C_{3-6}$ cycloalkyl, $C_{3-6}$ cycloalkyl-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

when A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-10}$ alkyl, $C_{3-10}$ cycloalkyl, $C_{3-10}$ cycloalkyl-$C_{1-10}$ alkyl, $C_{1-10}$ alkyloxy, $C_{3-10}$ cycloalkyloxy, $C_{1-10}$ alkyloxy-$C_{1-10}$ alkyl, 3- to 10-membered heterocyclyl, 3- to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-6}$ alkyl, $C_{3-6}$ cycloalkyl, $C_{3-6}$ cycloalkyl-$C_{1-6}$ alkyl, $C_{1-6}$ alkyloxy, $C_{3-6}$ cycloalkyloxy, $C_{1-6}$ alkyloxy-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-$C(=O)_nH$-;

or, $R^A$ and $R^1$, together with the atom to which they are attached, form a 3- to 8-membered ring structure which is unsubstituted or substituted with one, two or more $R^D$, for example, the 3-to 8-membered ring structure is 3-, 4-, 5-, 6-, 7- or 8-membered ring structure;

z is selected from 0 or 1;

$R^2$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^2$ is selected from the group consisting of $^1H$, $^2H$, hydrogen, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy;

$R^3$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^3$ is selected from the group consisting of $^1H$, $^2H$, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-10}$ alkyl, $C_{3-6}$ cycloalkyloxy;

or, $R^1$ and $R^2$, together with the atoms to which they are attached, form a 5- to 10-membered ring structure which is unsubstituted or substituted with two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a 4- to 10-membered ring structure which is unsubstituted or substituted with one, two or more $R^F$, the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of $C_{1-10}$ alkyl, $C_{1-10}$ alkyloxy, halogen, hydroxyl, sulfhydryl, amino, cyano; preferably, $R^4$ is selected from the group consisting of $C_{1-6}$ alkyl, $C_{1-6}$ alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: $C_{3-10}$ cycloalkyl, $C_{3-10}$ cycloalkyl-$C_{1-10}$ alkyl, $C_{3-10}$ cycloalkyloxy, 3- to 10-membered heterocyclyl, 3- to 10-membered heterocyclyl-$C_{1-10}$ alkyl, 3- to 10-membered heterocyclyloxy; preferably, $R^6$ is selected from the following groups which are unsubstituted or substituted with one, two or more $R^G$: $C_{3-6}$ cycloalkyl, $C_{3-6}$ cycloalkyl-$C_{1-6}$ alkyl, $C_{3-6}$ cycloalkyloxy, 3- to 6-membered heterocyclyl, 3- to 6-membered heterocyclyl-$C_{1-6}$ alkyl, 3- to 6-membered heterocyclyloxy;

B is absent or selected from the group consisting of triazolyl, - $NR^7$- or -$N(NR^7R^8)$-;

$R^7$ and $R^8$ are the same or different and are independently selected from the group consisting of $^1H$, $^2H$, and the following groups which are unsubstituted or substituted with one, two or more $R^H$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl, HC(=O)-, $C_{1-10}$ alkyl-C(=O)-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, $R^7$ and $R^8$ are the same or different and are independently selected from the group consisting of hydrogen, and the following groups which are unsubstituted or substituted with one, two or more $R^H$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl, HC(=O)-, $C_{1-6}$ alkyl-C(=O)-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

$T^L$ is selected from the group consisting of chemical bond, #O-$(CR^9R^{10})_m$-$(CR^{11}R^{12})_n$-C(=O)*, #O-$(CR^9R^{10})_m$-$(CR^{11}R^{12})_n$*, #N($R^{13}$)-$(CR^9R^{10})_m$-$(CR^{11}R^{12})_n$-C(O)*, #S-$(CR^9R^{10})_m$-$(CR^{11}R^{12})_n$-C(O)*, #O-$(CR^9R^{10})_m$-$(CR^{11}R^{12})_n$-C($NR^{13}$)*, #O-$(CR^9R^{10})_m$-$(CR^{11}R^{12})_n$-P(=O)($OR^{14}$)*, *O-$(CR^9R^{10})_m$-$(CR^{11}R^{12})_n$-C(=O)#, *O-$(CR^9R^{10})_m$-$(CR^{11}R^{12})_n$#, *N($R^{13}$)-$(CR^9R^{10})_m$-$(CR^{11}R^{12})_n$-C(O)#, *S-$(CR^9R^{10})_m$-$(CR^{11}R^{12})_n$-C(O)#, *O-$(CR^9R^{10})_m$-$(CR^{11}R^{12})_n$-C($NR^{13}$)#, *O-$(CR^9R^{10})_m$-$(CR^{11}R^{12})_n$-P(=O)($OR^{14}$)#, wherein # represents the site attached to L, and * represents the site attached to B;

each of $R^9$, $R^{10}$, $R^{11}$ and $R^{12}$ is the same or different, and is independently selected from the group consisting of $^1H$, $^2H$, halogen, cyano, and the following groups which are unsubstituted or substituted with one, two or more $R^I$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy; preferably, each of $R^9$, $R^{10}$, $R^{11}$ and $R^{12}$ is the same or different and is independently selected from the group consisting of hydrogen, and the following groups which are unsubstituted or substituted with one, two or more $R^I$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-10}$ alkyl, 3- to 6-membered heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$, together with the atoms to which they are attached form a 3- to 10-membered ring structure which is unsubstituted or substituted with one, two or more $R^I$; or, $R^9$

and $R^{11}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; wherein any of the 3- to 10-membered ring structures can be selected from, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the groups which are unsubstituted or substituted with one, two or more $R^J$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl; preferably, each $R^{13}$ is the same or different and is independently selected from the following groups which are unsubstituted or substituted with one, two or more $R^J$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl;

m and n are the same or different and are independently selected from an integer of 0 to 10, such as 0, 1, 2, 3, 4, 5, 6, 7, 8, 9 or 10;

each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each $R^K$ is the same or different and is independently selected from the group consisting of deuterium, halogen, hydroxyl, cyano, nitro, and the following groups which are unsubstituted or substituted with one, two or more $R^L$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^L$ is the same or different and is independently selected from the group consisting of deuterium, halogen, hydroxyl, cyano, nitro, $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-.

According to an embodiment of the present invention, definitions of the groups in Formula (GH) may be independently selected from:

A is selected from N or C-$R^A$;

when A is N, $R^1$ is selected from the group consisting of $^1H$, $^2H$, hydroxyl, deuterated hydroxyl, and the following groups which are unsubstituted or substituted with one, two or more $R^B$: $C_{1-10}$ alkyl, $C_{3-10}$ cycloalkyl, $C_{3-10}$ cycloalkyl-$C_{1-10}$ alkyl, 3- to 10-membered heterocyclyl, 3-to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is N, $R^1$ is selected from the group consisting of $^1H$, $^2H$, hydroxyl, deuterated hydroxyl, and the following groups which are unsubstituted or substituted with one, two or more $R^B$: $C_{1-6}$ alkyl, $C_{3-6}$ cycloalkyl, $C_{3-6}$ cycloalkyl-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

when A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-10}$ alkyl, $C_{3-10}$ cycloalkyl, $C_{3-10}$ cycloalkyl-$C_{1-10}$ alkyl, $C_{1-10}$ alkyloxy, $C_{3-10}$ cycloalkyloxy, $C_{1-10}$ alkyloxy-$C_{1-10}$ alkyl, 3- to 10-membered heterocyclyl, 3- to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-6}$ alkyl, $C_{3-6}$ cycloalkyl, $C_{3-6}$ cycloalkyl-$C_{1-6}$ alkyl, $C_{1-6}$ alkyloxy, $C_{3-6}$ cycloalkyloxy, $C_{1-6}$ alkyloxy-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

or, $R^A$ and $R^1$, together with the atom to which they are attached, form a 3- to 8-membered ring structure which is unsubstituted or substituted with one, two or more $R^D$, for example, the 3-to 8-membered ring structure is a 3-, 4-, 5-, 6-, 7- or 8-membered ring structure;

z is selected from 0 or 1;

$R^2$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^2$ is selected from the group consisting of $^1H$, $^2H$, hydrogen, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy;

$R^3$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^3$ is selected from the group consisting of $^1H$, $^2H$, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-10}$ alkyl, $C_{3-6}$ cycloalkyloxy;

provided that, $R^1$, $R^2$ and the atoms to which they are attached, or $R^2$, $R^3$ and the atoms to which they are attached, at least one of these two sets of groups together form a 4- to 10-membered ring structure which is unsubstituted or substituted with one, two or more $R^E$, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of $C_{1-10}$ alkyl, $C_{1-10}$ alkyloxy, halogen, hydroxyl, sulfhydryl, amino, cyano; preferably, $R^4$ is selected from the group consisting of $C_{1-6}$ alkyl, $C_{1-6}$ alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: $C_{1-10}$ alkyl, $C_{1-10}$ alkyloxy, $C_{6-10}$ aryl, $C_{6-10}$ arylalkyl, $C_{6-10}$ aryloxy, 5- to 6-membered heteroaryl, 5- to 6-membered heteroarylalkyl, 5- to 6-membered heteroaryloxy;

B is absent or selected from the group consisting of triazolyl, $-NR^7-$ or $-N(NR^7R^8)-$;

$R^7$ and $R^8$ are the same or different and are independently selected from the group consisting of $^1H$, $^2H$, and the following groups which are unsubstituted or substituted with one, two or more $R^H$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl, HC(=O)-, $C_{1-10}$ alkyl-C(=O)-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, $R^7$ and $R^8$ are the same or different and are independently selected from the group consisting of hydrogen, and the following groups which are unsubstituted or substituted with one, two or more $R^H$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl, HC(=O)-, $C_{1-6}$ alkyl-C(=O)-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

$T^L$ is selected from the group consisting of chemical bond, $\#O-(CR^9R^{10})_m-(CR^{11}R^{12})_n-C(=O)*$, $\#O-(CR^9R^{10})_m-(CR^{11}R^{12})_n*$, $\#N(R^{13})-(CR^9R^{10})_m-(CR^{11}R^{12})_n-C(O)*$, $\#S-(CR^9R^{10})_m-(CR^{11}R^{12})_n-C(O)*$, $\#O-(CR^9R^{10})_m-(CR^{11}R^{12})_n-C(NR^{13})*$, $\#O-(CR^9R^{10})_m-(CR^{11}R^{12})_n-P(=O)(OR^{14})*$, $*O-(CR^9R^{10})_m-(CR^{11}R^{12})_n-C(=O)\#$, $*O-(CR^9R^{10})_m-(CR^{11}R^{12})_n\#$, $*N(R^{13})-(CR^9R^{10})_m-(CR^{11}R^{12})_n-C(O)\#$, $*S-(CR^9R^{10})_m-(CR^{11}R^{12})_n-C(O)\#$, $*O-(CR^9R^{10})_m-(CR^{11}R^{12})_n-C(NR^{13})\#$, $*O-(CR^9R^{10})_m-(CR^{11}R^{12})_n-P(=O)(OR^{14})\#$, wherein # represents the site attached to L, and * represents the site attached to B;

each of $R^9$, $R^{10}$, $R^{11}$ and $R^{12}$ is the same or different, and is independently selected from the group consisting of $^1H$, $^2H$, halogen, cyano, and the following groups which are unsubstituted or substituted with one, two or more $R^I$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy; preferably, each of $R^9$, $R^{10}$, $R^{11}$ and $R^{12}$ is the same or different and is independently selected from the group consisting of hydrogen, halogen, cyano, and the following groups which are unsubstituted or substituted with one, two or more $R^I$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-10}$ alkyl, 3- to 6-membered heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; wherein, any of the 3- to 10-membered ring structures can be selected from, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl; preferably, each $R^{13}$ is the same or different and is independently selected from the following groups which are unsubstituted or substituted with one, two or more $R^J$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl;

m and n are the same or different and are independently selected from an integer of 0 to 10, such as 0, 1, 2, 3, 4, 5, 6, 7, 8, 9 or 10;

each of $R^B$, $R^C$, $R^D$, $R^E$, $R^F$, $R^G$, $R^H$, $R^I$ and $R^J$ is the same or different and is independently selected from the group

consisting of deuterium, halogen, hydroxyl, amino, nitro, the following groups which are unsubstituted or substituted with one, two or more $R^K$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each of $R^B$, $R^C$, $R^D$, $R^E$, $R^F$, $R^G$, $R^H$, $R^I$ and $R^J$ is the same or different, and is independently selected from the group consisting of deuterium, halogen, hydroxyl, cyano, nitro, and the following groups which are unsubstituted or substituted with one, two or more $R^K$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each $R^K$ is the same or different, and is independently selected from the group consisting of deuterium, halogen, hydroxyl, cyano, nitro, and the following groups which are unsubstituted or substituted with one, two or more $R^L$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^L$ is the same or different and is independently selected from the group consisting of deuterium, halogen, hydroxyl, cyano, nitro, $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-.

According to an embodiment of the present invention, definitions of the groups in Formula (GH) may be independently selected from:

A is selected from N;

$R^1$ is selected from the group consisting of $^1H$, $^2H$, hydroxyl, deuterated hydroxyl, and the following groups which are unsubstituted or substituted with one, two or more $R^B$: $C_{1-10}$ alkyl, $C_{3-10}$ cycloalkyl, $C_{3-10}$ cycloalkyl-$C_{1-10}$ alkyl, 3- to 10-membered heterocyclyl, 3- to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is N, $R^1$ is selected from the group consisting of $^1H$, $^2H$, hydroxyl, deuterated hydroxyl, and the following groups which are unsubstituted or substituted with one, two or more $R^B$: $C_{1-6}$ alkyl, $C_{3-6}$ cycloalkyl, $C_{3-6}$ cycloalkyl-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

z is selected from 0 or 1;

$R^2$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^2$ is selected from the group consisting of $^1H$, $^2H$, hydrogen, halogen, and the following

groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy;

$R^3$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^3$ is selected from the group consisting of $^1H$, $^2H$, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-10}$ alkyl, $C_{3-6}$ cycloalkyloxy;

or, $R^1$ and $R^2$, together with the atoms to which they are attached, form a 5- to 10-membered ring structure which is unsubstituted or substituted with one, two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of $C_{1-10}$ alkyl, $C_{1-10}$ alkyloxy, halogen, hydroxyl, sulfhydryl, amino, cyano; preferably, $R^4$ is selected from the group consisting of $C_{1-6}$ alkyl, $C_{1-6}$ alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: $C_{3-10}$ cycloalkyl, $C_{3-10}$ cycloalkyl-$C_{1-10}$ alkyl, $C_{3-10}$ cycloalkyloxy, 3- to 10-membered heterocyclyl, 3- to 10-membered heterocyclyl-$C_{1-10}$ alkyl, 3- to 10-membered heterocyclyloxy; preferably, $R^6$ is selected from the following groups which are unsubstituted or substituted with one, two or more $R^G$: $C_{3-6}$ cycloalkyl, $C_{3-6}$ cycloalkyl-$C_{1-6}$ alkyl, $C_{3-6}$ cycloalkyloxy, 3- to 6-membered heterocyclyl, 3- to 6-membered heterocyclyl-$C_{1-6}$ alkyl, 3- to 6-membered heterocyclyloxy;

B is selected from $-NR^7-$ or $-N(NR^7R^8)-$;

$R^7$ and $R^8$ are the same or different and are independently selected from the group consisting of $^1H$, $^2H$, and the following groups which are unsubstituted or substituted with one, two or more $R^H$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl, HC(=O)-, $C_{1-10}$ alkyl-C(=O)-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, $R^7$ and $R^8$ are the same or different and are independently selected from the group consisting of hydrogen, and the following groups which are unsubstituted or substituted with one, two or more $R^H$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl, HC(=O)-, $C_{1-6}$ alkyl-C(=O)-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

$T^L$ is selected from the group consisting of chemical bond, $\#O-(CR^9R^{10})_m-(CR^{11}R^{12})_n-C(=O)*$, $\#O-(CR^9R^{10})_m-(CR^{11}R^{12})_n*$, $\#N(R^{13})-(CR^9R^{10})_m-(CR^{11}R^{12})_n-C(O)*$, $\#S-(CR^9R^{10})_m-(CR^{11}R^{12})_n-C(O)*$, $\#O-(CR^9R^{10})_m-(CR^{11}R^{12})_n-C(NR^{13})*$, $\#O-(CR^9R^{10})_m-(CR^{11}R^{12})_n-P(=O)(OR^{14})*$, $*O-(CR^9R^{10})_m-(CR^{11}R^{12})_n-C(=O)\#$, $*O-(CR^9R^{10})_m-(CR^{11}R^{12})_n\#$, $*N(R^{13})-(CR^9R^{10})_m-(CR^{11}R^{12})_n-C(O)\#$, $*S-(CR^9R^{10})_m-(CR^{11}R^{12})_n-C(O)\#$, $*O-(CR^9R^{10})_m-(CR^{11}R^{12})_n-C(NR^{13})\#$, $*O-(CR^9R^{10})_m-(CR^{11}R^{12})_n-P(=O)(OR^{14})\#$, wherein # represents the site attached to L, and * represents the site attached to B;

each of $R^9$, $R^{10}$, $R^{11}$ and $R^{12}$ is the same or different and is independently selected from the group consisting of $^1H$, $^2H$, halogen, cyano, and the following groups which are unsubstituted or substituted with one, two or more $R^I$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy;

preferably, each of $R^9$, $R^{10}$, $R^{11}$ and $R^{12}$ is the same or different and is independently selected from the group consisting of hydrogen, halogen, cyano, and the following groups which are unsubstituted or substituted with one, two or more $R^I$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-10}$ alkyl, 3- to 6-membered heterocyclyloxy;

or, $R^9$ and $R^{10}$, together with the atoms to which they are attached, form a 3- to 10-membered ring structure which is unsubstituted or substituted with one, two or more $R^1$; or, $R^{11}$ and $R^{12}$, together with the atoms to which they are attached, form a 3-to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; any of the 3- to 10-membered ring structures can be, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl; preferably, each $R^{13}$ is the same or different and is independently selected from the following groups which are unsubstituted or substituted with one, two or more $R^J$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl;

m and n are the same or different and are independently selected from an integer of 0 to 10, such as 0, 1, 2, 3, 4, 5, 6, 7, 8, 9 or 10;

each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each $R^K$ is the same or different and is independently selected from the group consisting of deuterium, halogen, hydroxyl, cyano, nitro, and the following groups which are unsubstituted or substituted with one, two or more $R^L$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^L$ is the same or different and is independently selected from the group consisting of deuterium, halogen,

hydroxyl, cyano, nitro, $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-.

According to the embodiments of the present disclosure, $R^1$ and $R^2$, together with the quinolyl to which they are connected, form one of the following substructures, wherein the substructures may be unsubstituted or substituted with one, two or more $R^E$:

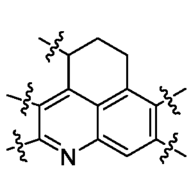,
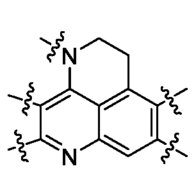,
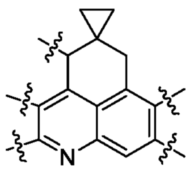,
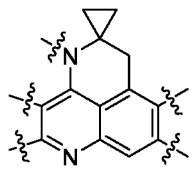,
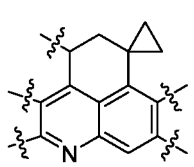,

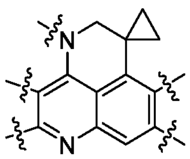,
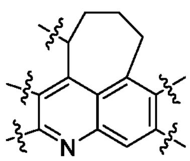,
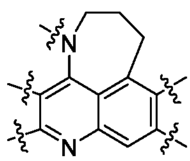,
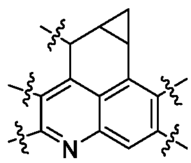,
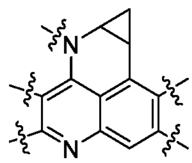.

Alternatively, according to the embodiments of the present disclosure, $R^2$ and $R^3$, together with the quinolyl to which they are connected, form one of the following substructures, wherein the substructures may be unsubstituted or substituted with one, two or more $R^F$:

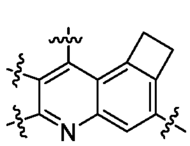,
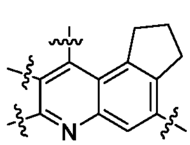,
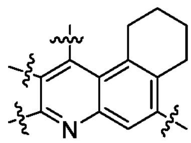.

According to the embodiments of the present disclosure, B is absent or selected from the group consisting of

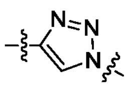,
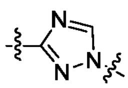,

-NH- or -N($NR^7R^8$)-, wherein $R^7$ and $R^8$ independently have the definitions as described above.

According to an embodiment of the present disclosure, the compound represented by Formula (GH) may be selected from the compound represented by Formula (GH-1):

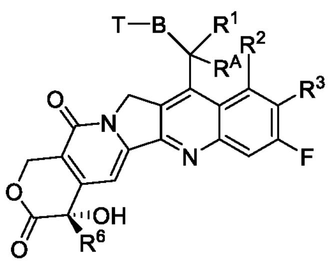

(GH-1)

wherein, $R^A$, B, T, $R^1$, $R^2$, $R^3$, and $R^6$ are independently defined as described above.

According to an embodiment of the present disclosure, the compound represented by Formula (GH) may be selected from the compound represented by Formula (GH-2) or (GH-3):

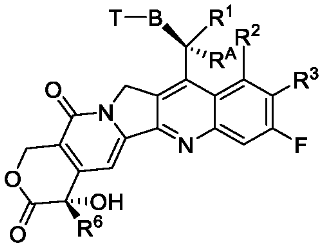

(GH-2)

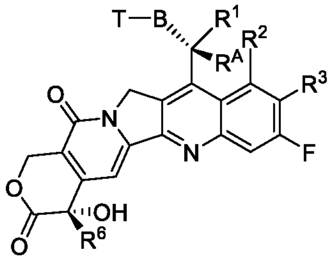

(GH-3)

wherein, $R^A$, B, T, $R^1$, $R^2$, $R^3$, and $R^6$ are independently defined as described above.

According to the embodiment of the present disclosure, the compound represented by Formula (GH) can be selected from the following compounds:

| | | |
|---|---|---|
| 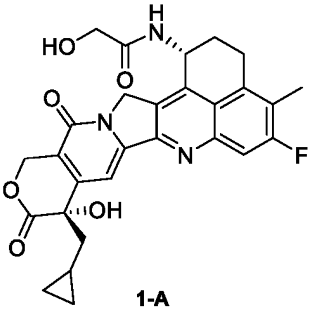 1-A | 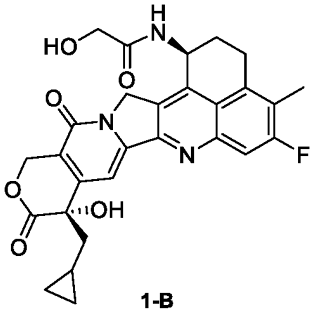 1-B | |
| 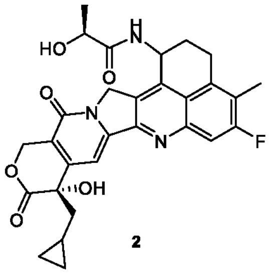 2 | 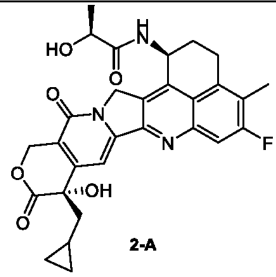 2-A | 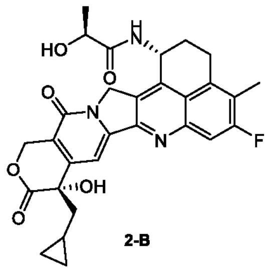 2-B |
| 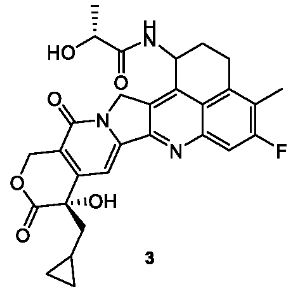 3 | 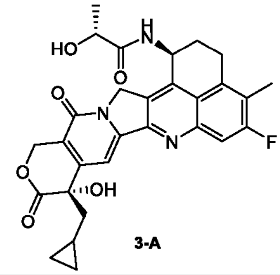 3-A | 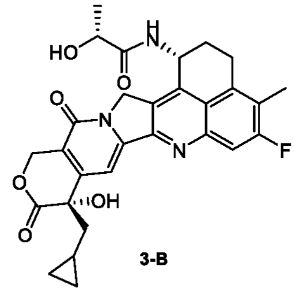 3-B |
| 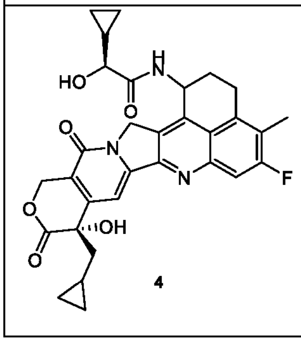 4 | 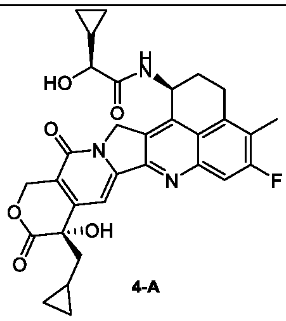 4-A | 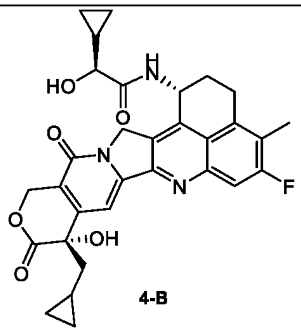 4-B |

| | | |
|---|---|---|
| 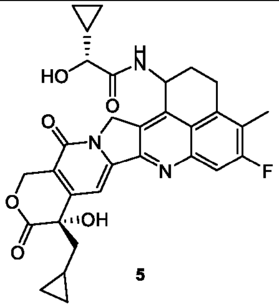 5 | 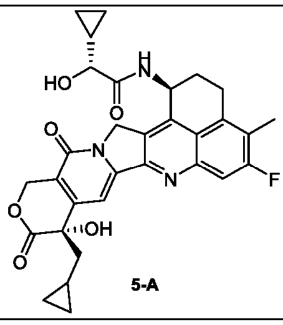 5-A | 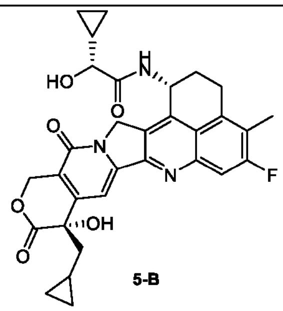 5-B |
| 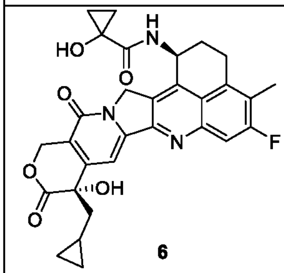 6 | | |
| 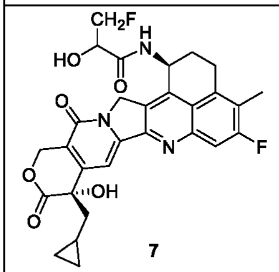 7 | 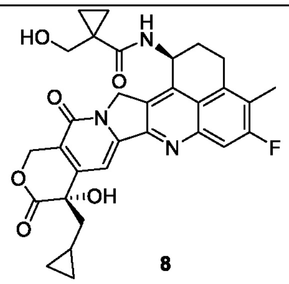 8 | 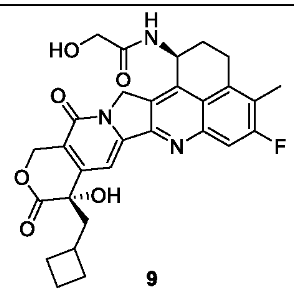 9 |
| 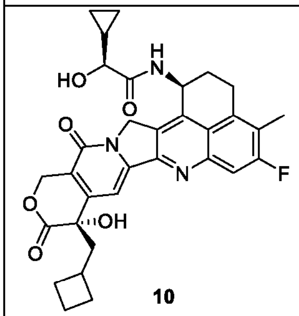 10 | 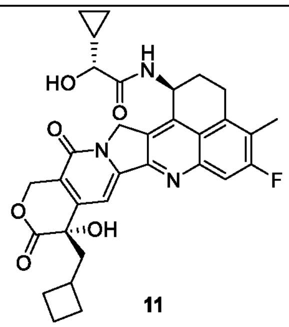 11 | 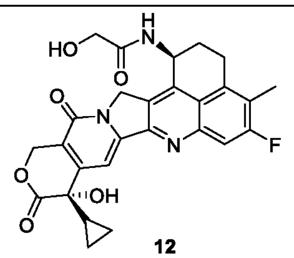 12 |
| 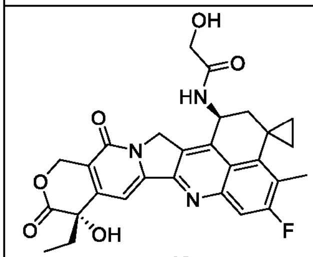 13 | 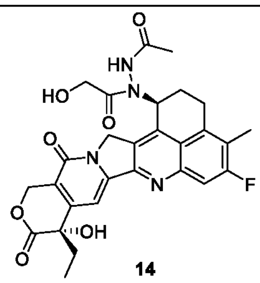 14 | 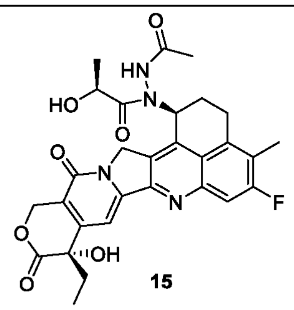 15 |
| 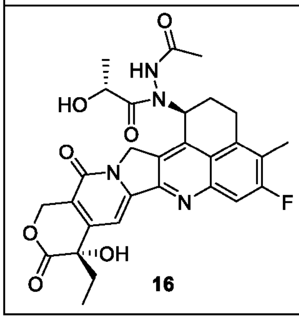 16 | 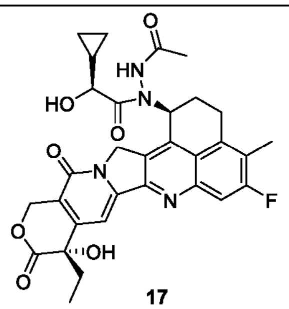 17 | 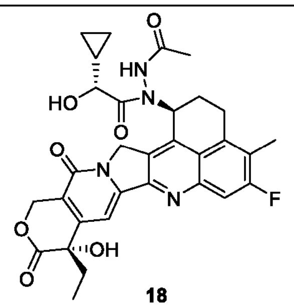 18 |

| | | |
|---|---|---|
| 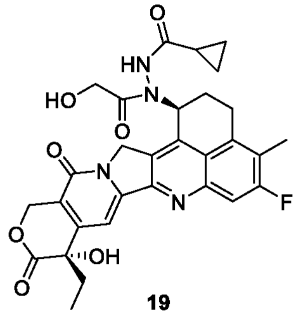 19 | 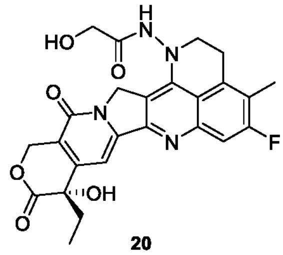 20 | 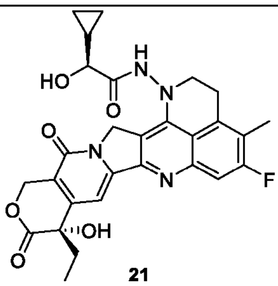 21 |
| 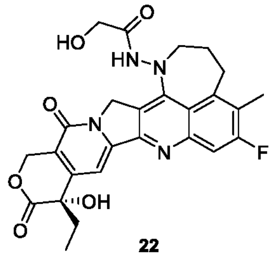 22 | 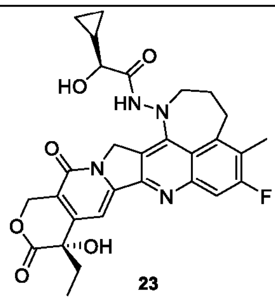 23 | 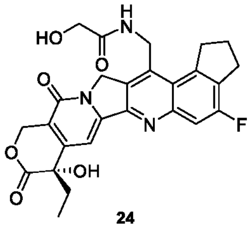 24 |
| 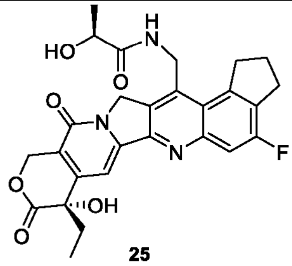 25 | 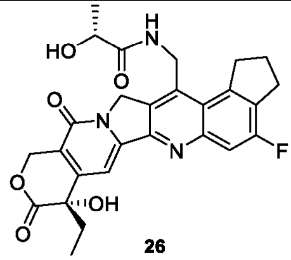 26 | 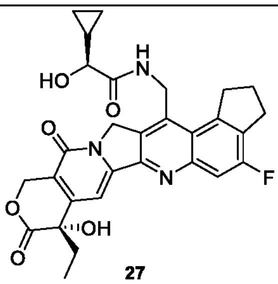 27 |
| 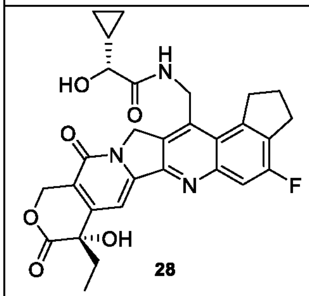 28 | 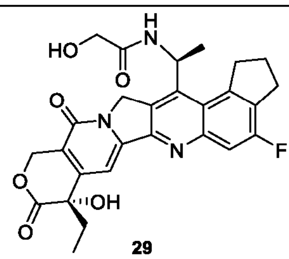 29 | 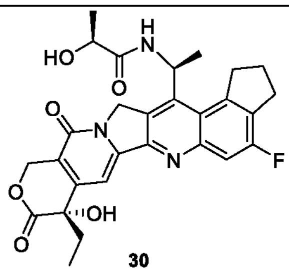 30 |
| 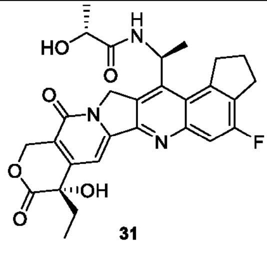 31 | 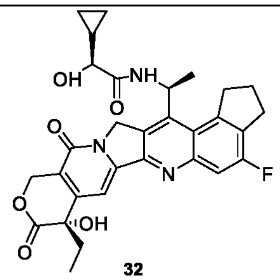 32 | 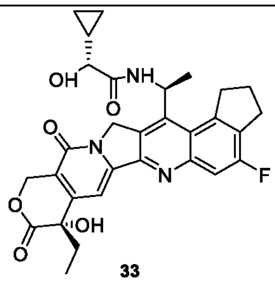 33 |
| 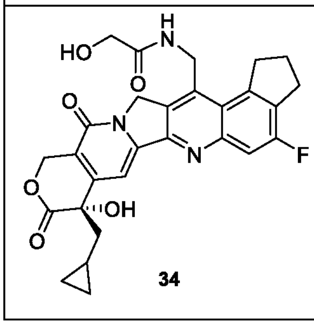 34 | 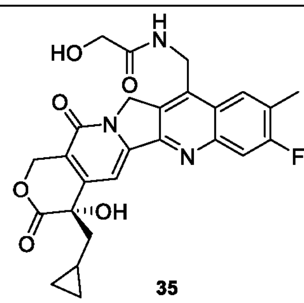 35 | 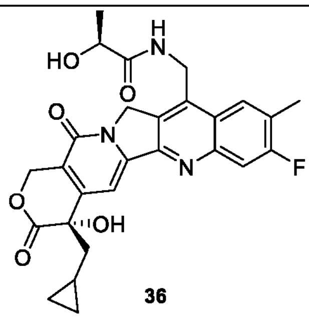 36 |

| | | |
|---|---|---|
| 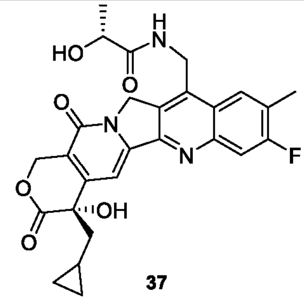 37 | 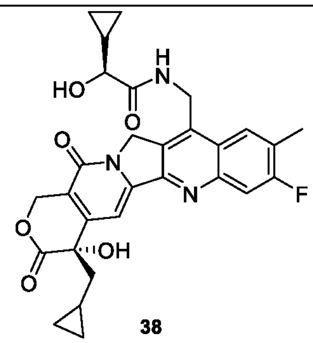 38 | 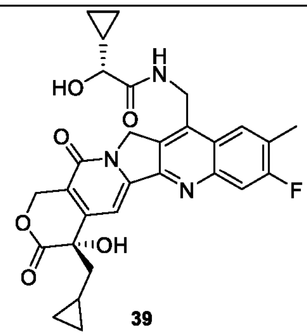 39 |

The present disclosure also provides a method for preparing the compound represented by Formula (GH), or a stereoisomer, racemate, tautomer, isotopologue, isotope-labeled compound, nitroxide or pharmaceutically acceptable salt thereof, the method comprises a step of reacting the compound of Formula (i) with the compound of Formula (ii):

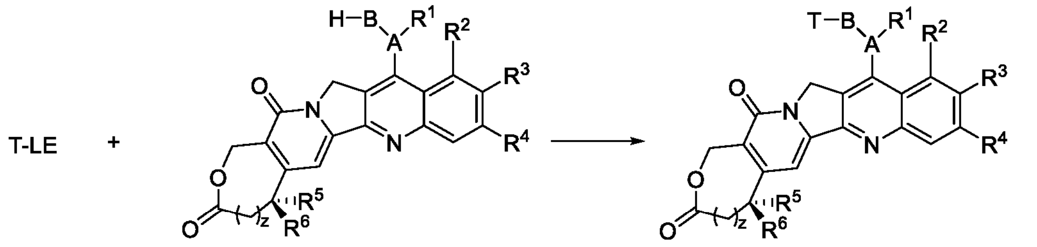

(i) (ii) (GH)

wherein, A, B, T, $R^1$, $R^2$, $R^3$, $R^4$, $R^5$, $R^6$, and z independently have the definitions described above;

LE represents a group that is removed or leaves after the reaction.

The present invention also provides a method for preparing a compound of Formula (ii), comprising a step of reacting a compound of Formula (iii) with a compound of Formula (iv) to obtain a compound of Formula (v):

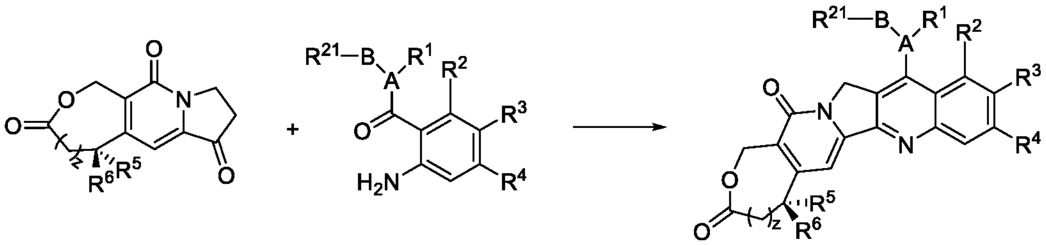

(iii) (iv) (v)

wherein, A, B, $R^1$, $R^2$, $R^3$, $R^4$, $R^5$, $R^6$, and z independently have the definitions described above;

$R^{21}$ is selected from H or a protecting group.

According to an embodiment of the present invention, when $R^{21}$ is selected from H, the compound of Formula (v) is the compound of Formula (ii).

According to an embodiment of the present invention, when $R^{21}$ is selected from a protecting group, the compound of Formula (v) may be reacted under conditions of deprotection after obtaining the compound of Formula (v), to obtain the compound of Formula (ii).

According to an embodiment of the present invention, the protecting group is selected from an amino protecting group.

According to an embodiment of the present invention, the type of the protecting group (e.g., an amino protecting group) and the conditions for deprotection may be selected from types or conditions known to those skilled in the art.

The present disclosure also provides a compound represented by Formula (iii):

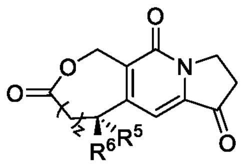

(iii)

wherein, $R^5$, $R^6$, and z independently have the definitions described above.

The present disclosure also provides a compound represented by Formula (v):

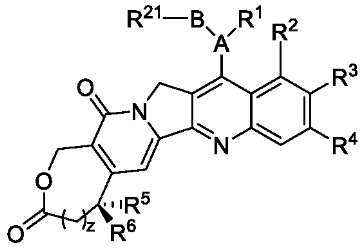

(v)

wherein, A, B, $R^1$, $R^2$, $R^3$, $R^4$, $R^5$, $R^6$, $R^{21}$, and z independently have the definitions described above.

The present disclosure also provides a pharmaceutical composition, which comprises at least one selected from the following: the compound represented by Formula (GH), or a stereoisomer, racemate, tautomer, isotopologue, isotope-labeled compound, nitroxide, or pharmaceutically acceptable salt thereof, the conjugate represented by Formula (C), or a stereoisomer, racemate, tautomer, isotopologue, isotope-labeled compound, nitroxide, or pharmaceutically acceptable salt thereof.

Preferably, if present, the compound represented by Formula (GH), or a stereoisomer, racemate, tautomer, isotopologue, isotope-labeled compound, nitroxide or pharmaceutically acceptable salt thereof, or the conjugate represented by Formula (C), or a stereoisomer, racemate, tautomer, isotopologue, isotope-labeled compound, nitroxide or pharmaceutically acceptable salt thereof in the pharmaceutical composition is present in a therapeutically effective amount.

According to an embodiment of the present disclosure, the pharmaceutical composition comprises a therapeutically effective amount of at least one selected from the following: the compound represented by Formula (GH), or a stereoisomer, racemate, tautomer, isotopologue, isotope-labeled compound, nitroxide or pharmaceutically acceptable salt thereof, the conjugate represented by Formula (C), or a stereoisomer, racemate, tautomer, isotopologue, isotope-labeled compound, nitroxide or pharmaceutically acceptable salt thereof.

The present disclosure also provides use of the compound represented by the Formula (GH), or a stereoisomer, racemate, tautomer, isotopologue, isotope-labeled compound, nitroxide or pharmaceutically acceptable salt thereof, or the conjugate represented by the Formula (C), or a stereoisomer, racemate, tautomer, isotopologue, isotope-labeled compound, nitroxide or pharmaceutically acceptable salt thereof, for preventing and/or treating a disease or disorder, and/or in the manufacture of a medicament.

According to an embodiment of the present disclosure, the medicament is used for preventing and/or treating a disease or disorder.

The present disclosure also provides a method for preventing and/or treating a disease or disorder, the method comprising administering to a patient a therapeutically effective amount of at least one of the following: the compound represented by the Formula (GH), or a stereoisomer, racemate, tautomer, isotopologue, isotope-labeled compound, nitroxide or pharmaceutically acceptable salt thereof, the conjugate represented by the Formula (C), or a stereoisomer, racemate, tautomer, isotopologue, isotope-labeled compound, nitroxide or pharmaceutically acceptable salt thereof.

According to an embodiment of the present disclosure, the disease or disorder can be selected from a tumor, such as a solid tumor or a hematological cancer.

Examples of the solid tumor include malignancies of various organ systems, such as sarcomas, adenocarcinomas, blastomas, and carcinomas, such as those affecting liver, lung, breast, lymph, biliary-intestinal (e.g., colon), genitourinary tract (e.g., kidney, urothelial cells), prostate, and pharynx. Adenocarcinomas include malignancies such as most colon cancers, rectal cancers, renal cell carcinomas, hepatocarcinomas, small cell lung cancers, non-small cell lung cancers, small intestinal cancers, and esophageal cancers. In one embodiment, the cancer is melanoma, such as

advanced melanoma. Examples of other cancers that may be treated include: bone cancer, pancreatic cancer, skin cancer, head and neck cancer, malignant melanoma of skin or in eyes, uterine cancer, ovarian cancer, rectal cancer, colorectal cancer, cancer of anal region, cancer of peritoneum, stomach cancer, esophageal cancer, salivary gland cancer, testicular cancer, fallopian tube cancer, endometrial cancer, cervical cancer, vaginal cancer, vulvar cancer, penile cancer, malignant glioma, neuroblastoma, Hodgkin's disease, non-Hodgkin's lymphoma, esophageal cancer, small intestine cancer, cancer of endocrine system, thyroid cancer, parathyroid cancer, adrenal cancer, soft tissue sarcoma, urethral cancer, chronic or acute leukemia (including acute myeloid leukemia, chronic myeloid leukemia, acute lymphoblastic leukemia, chronic lymphocytic leukemia), solid tumors of children, lymphocytic lymphoma, bladder cancer, kidney cancer or ureter cancer, renal pelvic cancer, central nervous system (CNS) tumor, primary CNS lymphoma, tumor angiogenesis, spinal cord axis tumor, brain stem glioma, pituitary adenoma, Kaposi's sarcoma, neuroendocrine tumor (including carcinoid tumor, gastrinoma, and islet cell carcinoma), mesothelioma, schwannoma (including acoustic neuroma), meningioma, epidermoid carcinoma, squamous cell carcinoma, T-cell lymphoma, environmentally induced cancer (including cancer induced by asbestos), and any combination of the above cancers.

Examples of the above hematological cancers include leukemias, lymphomas, and malignant lymphoproliferative disorders that affect the blood, bone marrow, and lymphatic system. Leukemias can be classified into acute leukemias and chronic leukemias. Acute leukemias can be further classified into acute myeloid leukemia (AML) and acute lymphocytic leukemia (ALL). Chronic leukemias include chronic myeloid leukemia (CML) and chronic lymphocytic leukemia (CLL). Other related conditions include myelodysplastic syndrome (MDS, formerly known as "preleukemia"), which is a diverse collection of hematological conditions associated with ineffective production (or dysplasia) of bone marrow blood cells and the risk of transformation to AML. Lymphomas are a group of blood cell tumors that develop from lymphocytes. Exemplary lymphomas include non-Hodgkin lymphoma and Hodgkin lymphoma.

According to an embodiment of the present disclosure, the pharmaceutical composition may also comprise a pharmaceutically acceptable excipient, such as a carrier or excipient. The pharmaceutically acceptable excipient is preferably chemically non-reactive or inert to the active ingredient. For example, the pharmaceutically acceptable excipient is at least one selected from the following excipients, including but not limited to: fillers, disintegrants, binders, lubricants, surfactants, flavoring agents, wetting agents, matrix, etc.

According to the embodiments of the present disclosure, the administration route of the pharmaceutical composition includes but is not limited to gastrointestinal administration or parenteral administration; wherein the gastrointestinal administration may be oral administration; the parenteral administration may be topical administration, transdermal administration, injection administration, etc.

In one embodiment, the administration route of the pharmaceutical composition may be topical administration combined with oral administration.

According to the embodiments of the present disclosure, the dosage form of the pharmaceutical composition may be selected from capsules, tablets, patches, films, pills, powders, lozenges, sachets, cachets, elixirs, suspensions, emulsions, solutions, syrups, aerosols, ointments, creams, suppositories or injections.

Definitions and explanations of terms

Unless otherwise specified, definitions of the terms described in the description and claims of the present application, including those provided as examples, exemplary or preferred, in tables, and in the specific compounds of the examples, etc., may be arbitrarily combined and integrated with each other. Such combination and integration shall fall within the scope of the description of the present application.

Unless otherwise specified, the numerical ranges recorded in the present description and claims are equivalent to recording at least each specific integer value therein. For example, the numerical range "1 to 10" is equivalent to recording each integer value in the numerical range "1 to 10", namely 1, 2, 3, 4, 5, 6, 7, 8, 9, 10. It should be understood that when "one", "two" or "more" are used herein to describe substituents, "more" should refer to an integer $\geq 3$, such as 3, 4, 5, 6, 7, 8, 9 or 10. In addition, when certain numerical ranges are defined as "numbers", it should be understood that the two endpoints of the range, each integer in the range, and each decimal in the range are recorded. For example, "a number from 0 to 10" should be understood as not only recording each integer of 0, 1, 2, 3, 4, 5, 6, 7, 8, 9 and 10, but also at least recording the sum of each integer therein and 0.1, 0.2, 0.3, 0.4, 0.5, 0.6, 0.7, 0.8, 0.9, respectively. Similarly, "an integer or decimal of 1 to 50", "an integer or decimal of 1 to 20", "an integer or decimal of 1 to 10" should be understood as recording the two endpoints of each of the above numerical ranges, as well as each integer in each range and each decimal in the range.

The term "isotopologue" comprises replacing an atom in a compound or conjugate of the present disclosure with other isotopes having the same atomic number but different atomic mass or mass number. For example, in the compound or conjugate of the present disclosure, "hydrogen" can be selected from protium ($^{1}H$), deuterium ($^{2}H$) and tritium ($^{3}$H); "carbon" can be selected from $^{12}$C, $^{13}$C and $^{14}$C. Therefore, the compound or conjugate of the present disclosure should at least be understood as compounds or conjugates containing various deuterated forms. For example, one, two or more of

available hydrogen atoms connected to a carbon atom (e.g., hydrogen atoms of $C_{1-10}$ alkyl) can be independently replaced by deuterium atoms. Those skilled in the art can synthesize deuterated compounds or conjugates with reference to relevant literatures. When preparing deuterated compounds or conjugates, commercially available deuterated starting materials can be used, or deuterated reagents can be used for synthesis using conventional techniques. Optional deuterated reagents include, but are not limited to, deuterated borane, trideuterated borane in tetrahydrofuran solution, deuterated lithium aluminum hydride, deuterated iodoethane and deuterated iodomethane.

The term "isotope-labeled compound" includes, but is not limited to, the compound or conjugate of the present disclosure that is labeled with at least one of the following elements: $^{111}In$, $^{177}Lu$, $^{212}Bi$, $^{213}Bi$, $^{211}At$, $^{62}Cu$, $^{67}Cu$, $^{90}Y$, $^{125}I$, $^{131}I$, $^{32}P$, $^{33}P$, $^{47}Sc$, $^{111}Ag$, $^{67}Ga$, $^{142}Pr$, $^{153}Sm$, $^{161}Tb$, $^{166}Dy$, $^{166}Ho$, $^{186}Re$, $^{188}Re$, $^{189}Re$, $^{212}Pb$, $^{223}Ra$, $^{225}Ac$, $^{59}Fe$, $^{75}Se$, $^{77}As$, $^{89}Sr$, $^{99}Mo$, $^{105}Rh$, $^{109}Pd$, $^{143}Pr$, $^{149}Pm$, $^{169}Er$, $^{194}Ir$, $^{198}Au$, $^{199}Au$, $^{227}Th$ and $^{211}Pb$.

The term "halogen" refers to fluorine, chlorine, bromine and iodine.

The term "alkyl" should be understood to preferably refer to a straight or branched saturated monovalent hydrocarbyl, preferably "$C_{1-10}$ alkyl". "$C_{1-10}$ alkyl" should be understood to preferably refer to a straight or branched saturated monovalent hydrocarbyl having 1 to 10 carbon atoms. For example, "$C_{1-6}$ alkyl" refers to a straight and branched alkyl having 1, 2, 3, 4, 5 or 6 carbon atoms. The alkyl is, for example, methyl, ethyl, propyl, butyl, pentyl, hexyl, isopropyl, isobutyl, sec-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl or 1,2-dimethylbutyl, etc. or isomers thereof.

The term "cycloalkyl" should be understood to refer to a saturated monovalent monocyclic or bicyclic (fused, bridged or spiro ring) hydrocarbyl, preferably "$C_{3-10}$ cycloalkyl". The term "$C_{3-10}$ cycloalkyl" should be understood to refer to a saturated monovalent monocyclic or bicyclic (fused, bridged or spiro ring) hydrocarbyl having 3 to 10 carbon atoms, such as 3, 4, 5, 6, 7, 8, 9 or 10 carbon atoms. The $C_{3-10}$ cycloalkyl may be a monocyclic hydrocarbyl, such as cyclopropyl, cyclobutyl, cyclopentyl, cyclohexyl, cycloheptyl, cyclooctyl, cyclononyl or cyclodecyl, or a bicyclic hydrocarbyl such as decalin ring.

The term "ring structure" should be understood to refer to a group having a monocyclic, bicyclic (fused, bridged or spiro ring) or multicyclic ring structure, such as a monocyclic hydrocarbyl, a bicyclic hydrocarbyl, a tricyclic hydrocarbyl, a heteromonocyclic hydrocarbyl, a heterobicyclic hydrocarbyl, a heterotricyclic hydrocarbyl, etc.; and the ring structure may be saturated or unsaturated. The heteromonocyclic hydrocarbyl, heterobicyclic hydrocarbyl, and heterotricyclic hydrocarbyl may contain 1 to 10, preferably 1 to 5 heteroatoms, independently selected from N, O and S, such as 1, 2, 3, 4, 5, 6, 7, 8, 9 or 10 heteroatoms independently selected from N, O and S. The bicyclic ring may be the following ring structure containing no heteroatoms or containing 1, 2 or 3 heteroatoms independently selected from N, O and S: one or two of aryl, heteroaryl, cycloalkyl, heterocyclyl, which are independent or fused to each other, spiro[2.5] ring, spiro[3.3] ring, spiro[4.2] ring, spiro[4.3] ring, spiro[5.2] ring, spiro[5.4] ring, bicyclo[2.1.1], bicyclo[2.2.1], bicyclo[2.2.2], bicyclo[3.2.1], bicyclo [4.1.0], etc.. Alternatively, the ring structure may have 1, 2, 3, 4 or 5 double bonds, such as carbon-carbon double bonds or carbon-nitrogen double bonds.

The term "3- to 10-membered ring structure" should be understood to refer to a group having a monocyclic or bicyclic (fused, bridged or spiro ring) structure with 3, 4, 5, 6, 7, 8, 9 or 10 ring atoms, such as a monocyclic hydrocarbyl, a bicyclic hydrocarbyl, a heteromonocyclic hydrocarbyl or a heterobicyclic hydrocarbyl; and the ring structure may be saturated or unsaturated. The heteromonocyclic hydrocarbyl or heterobicyclic hydrocarbyl may contain 1 to 5, preferably 1 to 3 heteroatoms independently selected from N, O and S, such as 1, 2, 3 heteroatoms independently selected from N, O and S. The bicyclic ring may be the following ring structure containing no heteroatoms or containing 1, 2, or 3 heteroatoms independently selected from N, O, and S: one or two of aryl, heteroaryl, cycloalkyl, heterocyclyl, which are independent or fused to each other, spiro[2.5] ring, spiro[3.3] ring, spiro[4.2] ring, spiro[4.3] ring, spiro[5.2] ring, spiro[5.4] ring, bicyclo [2.1.1], bicyclo[2.2.1], bicyclo[2.2.2], bicyclo[3.2.1], bicyclo[4.1.0], etc. Alternatively, the ring structure may have 1, 2, 3, 4, or 5 double bonds, such as carbon-carbon double bonds or carbon-nitrogen double bonds.

The term "4- to 10-membered ring structure" should be understood to refer to a group having a monocyclic or bicyclic (fused, bridged or spiro ring) structure with 4, 5, 6, 7, 8, 9 or 10 ring atoms, such as a monocyclic hydrocarbyl, a bicyclic hydrocarbyl, a heteromonocyclic hydrocarbyl or a heterobicyclic hydrocarbyl; and the ring structure may be saturated or unsaturated. The heteromonocyclic hydrocarbyl or heterobicyclic hydrocarbyl may contain 1 to 5, preferably 1 to 3 heteroatoms independently selected from N, O and S, such as 1, 2, 3 heteroatoms independently selected from N, O and S. The bicyclic ring may be the following ring structure containing no heteroatoms or containing 1, 2, or 3 heteroatoms independently selected from N, O, and S: one or two of aryl, heteroaryl, cycloalkyl, heterocyclyl, which are independent or fused to each other, spiro[2.5] ring, spiro[3.3] ring, spiro[4.2] ring, spiro[4.3] ring, spiro[5.2] ring, spiro[5.4] ring, bicyclo [2.1.1], bicyclo[2.2.1], bicyclo[2.2.2], bicyclo[3.2.1], bicyclo[4.1.0], etc. Alternatively, the ring structure may have 1, 2, 3, 4, or 5 double bonds, such as carbon-carbon double bonds or carbon-nitrogen double bonds.

The term "5- to 10-membered ring structure" should be understood to refer to a group having a monocyclic or bicyclic (fused, bridged or spiro ring) structure with 5, 6, 7, 8, 9 or 10 ring atoms, such as a monocyclic hydrocarbyl, a

bicyclic hydrocarbyl, a heteromonocyclic hydrocarbyl or a heterobicyclic hydrocarbyl; and the ring structure may be saturated or unsaturated. The heteromonocyclic hydrocarbyl or heterobicyclic hydrocarbyl may contain 1 to 5, preferably 1 to 3 heteroatoms independently selected from N, O and S, such as 1, 2, 3 heteroatoms independently selected from N, O and S. The bicyclic ring may be the following ring structure containing on heteroatoms or containing 1, 2, or 3 heteroatoms independently selected from N, O, and S: one or two of aryl, heteroaryl, cycloalkyl, heterocyclyl, which are independent or fused to each other, spiro[2.5] ring, spiro[3.3] ring, spiro[4.2] ring, spiro[4.3] ring, spiro[5.2] ring, spiro[5.4] ring, bicyclic [2.1.1], bicyclic[2.2.1], bicyclic[2.2.2], bicyclic[3.2.1], bicyclic[4.1.0], etc.

Alternatively, the "4- to 10-membered ring structure" and "5- to 10-membered ring structure" may have 1, 2, 3, 4, or 5 double bonds, such as carbon-carbon double bonds or carbon-nitrogen double bonds.

It should be understood that when the group of the compound described in the present invention forms the above-mentioned ring structure, it may form a fused ring, spiro ring or bicyclic ring with the original ring structure of the compound, and when there is an unsaturated bond in the original ring structure of the compound, the ring structure formed by the group preferably retains the unsaturated bond in the original ring structure.

The term "heterocyclyl" refers to a saturated or unsaturated monovalent monocyclic, bicyclic (fused, bridged or spiro ring) hydrocarbyl, which contains 1 to 5 heteroatoms independently selected from N, O and S, but is not aromatic. The "heterocyclyl" is preferably a "3- to 20-membered heterocyclyl". The term "3- to 20-membered heterocyclyl" refers to a saturated monovalent monocyclic, bicyclic (fused, bridged or spiro ring) hydrocarbyl, which contains 1 to 5 heteroatoms independently selected from N, O and S, and the total number of ring atoms is 3 to 20 (e.g., 3, 4, 5, 6, 7, 8, 9, 10, etc.), preferably a "3- to 10-membered heterocyclyl". The term "3-to 10-membered heterocyclyl" refers to a saturated or unsaturated monovalent monocyclic, bicyclic (fused, bridged or spiro) hydrocarbyl, which contains 1 to 5, preferably 1 to 3 heteroatoms independently selected from N, O and S, such as 1, 2, 3 heteroatoms independently selected from N, O and S, but which is not aromatic. The heterocyclyl may be attached to the rest part of the molecule via any of the carbon atoms or nitrogen atoms (if present). In particular, the heterocyclyl may include, but is not limited to: 4-membered ring, such as azetidinyl, oxetanyl (e.g., azetidin-1-yl); 5-membered ring, such as tetrahydrofuranyl, dioxolyl, pyrrolidinyl, imidazolidinyl, pyrazolidinyl, pyrrolinyl; or 6-membered ring, such as tetrahydropyranyl, piperidinyl, morpholinyl, dithianyl, thiomorpholinyl, piperazinyl or trithianyl; or 7-membered ring, such as diazecycloheptyl. Optionally, the heterocyclyl can be benzo-fused. The heterocyclyl can be bicyclic, for example, but not limited to, 5,5-membered ring, such as hexahydrocyclopenta [c]pyrrol-2(1*H*)-yl ring, or 5,6-membered bicyclic ring, such as hexahydropyrrolo[1,2-a]pyrazin-2(1*H*)-yl ring. The ring containing nitrogen atoms can be partially unsaturated, that is, it can contain one or more double bonds, for example, but not limited to, 2,5-dihydro-1*H*-pyrrolyl, 4H-[1,3,4]thiadiazinyl, 4,5-dihydrooxazolyl or 4H-[1,4]thiazinyl, or, it can be benzo-fused, for example, but not limited to, dihydroisoquinolinyl. According to the present disclosure, the heterocyclyl is non-aromatic. When the 3- to 20-membered heterocyclyl is connected with other groups to form the compound disclosed herein, the carbon atoms on the 3- to 20-membered heterocyclyl may be connected with other groups, or the heterocyclic atoms on the 3- to 20-membered heterocyclyl may be connected with other groups. For example, when the 3- to 20-membered heterocyclyl is selected from piperazinyl, the nitrogen atom on the piperazinyl may be connected with other groups. Alternatively, when the 3- to 20-membered heterocyclyl is selected from piperidinyl, the nitrogen atom on the piperidinyl ring and the carbon atom on the para position may be connected with other groups.

The term "aryl" should be understood as a monocyclic, bicyclic or tricyclic hydrocarbyl with monovalent aromaticity or partial aromaticity, preferably a "$C_{6-20}$ aryl". The term "$C_{6-20}$ aryl" should be understood to preferably refer to a monocyclic, bicyclic or tricyclic hydrocarbyl with monovalent aromaticity or partial aromaticity having 6 to 20 carbon atoms, preferably a "$C_{6-14}$ aryl". The term "$C_{6-14}$ aryl" should be understood to preferably refer to a monocyclic, bicyclic or tricyclic hydrocarbyl ("$C_{6-14}$ aryl") with monovalent aromaticity or partial aromaticity having 6, 7, 8, 9, 10, 11, 12, 13 or 14 carbon atoms, in particular, a ring having 6 carbon atoms ("$C_6$ aryl"), such as phenyl; or biphenyl, or a ring having 9 carbon atoms ("$C_9$ aryl"), such as indanyl or indenyl, or a ring having 10 carbon atoms ("$C_{10}$ aryl"), such as tetrahydronaphthyl, dihydronaphthyl or naphthyl, or a ring having 13 carbon atoms ("$C_{13}$ aryl"), such as fluorenyl, or a ring having 14 carbon atoms ("$C_{14}$ aryl"), such as anthracenyl. When the $C_{6-20}$ aryl is substituted, it may be monosubstituted or polysubstituted. Moreover, there is no restriction on the substitution site, for example, it may be ortho-, para- or meta-substituted.

The term "heteroaryl" should be understood as a monovalent monocyclic, bicyclic or tricyclic aromatic ring system group containing 1 to 5 heteroatoms independently selected from N, O and S, preferably a "5- to 20-membered heteroaryl". The term "5- to 20-membered heteroaryl" should be understood to include such a monovalent monocyclic, bicyclic or tricyclic aromatic ring system group which has 5 to 20 ring atoms and contain 1 to 5 heteroatoms independently selected from N, O and S, for example, a "5- to 14-membered heteroaryl". The term "5- to 14-membered heteroaryl" should be understood to include such a monovalent monocyclic, bicyclic or tricyclic aromatic ring system group which has 5, 6, 7, 8, 9, 10, 11, 12, 13 or 14 ring atoms, in particular 5 or 6 or 9 or 10 carbon atoms, and which contains 1 to 5, preferably 1 to 3 heteroatoms each independently selected from N, O and S; and, in each case, it may additionally be benzo-fused. In particular, the heteroaryl is selected from the group consisting of thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4H-pyrazolyl and the like and benzo derivatives thereof, such as benzofuranyl, benzothienyl, benzoxazolyl, benzisoxazolyl, benzimidazolyl, benzotriazolyl, indazolyl,

indolyl, isoindolyl and the like; or pyridyl, pyridazinyl, pyrimidinyl, pyrazinyl, triazinyl and the like and benzo derivatives thereof, such as quinolyl, quinazolinyl, isoquinolyl and the like; or azocinyl, indolizinyl, purinyl and the like and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl and the like. When the 5- to 20-membered heteroaryl is connected to other groups to form the compounds disclosed herein, the carbon atoms on the 5- to 20-membered heteroaryl ring may be connected to other groups, or the heteroatoms on the 5- to 20-membered heteroaryl ring may be connected to other groups. When the 5- to 20-membered heteroaryl is substituted, it may be monosubstituted or polysubstituted. In addition, there is no limitation on the substitution site, for example, the hydrogen attached to the carbon atom on the heteroaryl ring may be substituted, or the hydrogen attached to the heteroatom on the heteroaryl ring may be substituted.

Unless otherwise specified, the heterocyclyl, heteroaryl or heteroarylene includes all possible isomeric forms thereof, such as positional isomers thereof. Therefore, for some illustrative non-limiting examples, it may comprise forms with substitution or binding to other group at one, two or more positions selected from its 1-, 2-, 3-, 4-, 5-, 6-, 7-, 8-, 9-, 10-, 11-, 12-positions, etc. (if present), including pyridin-2-yl, pyridin-2-yl, pyridin-3-yl, pyridin-3-yl, pyridin-4-yl and pyridin-4-yl; thienyl or thienyl include thien-2-yl, thien-2-yl, thien-3-yl and thien-3-yl; pyrazol-1-yl, pyrazol-3-yl, pyrazol-4-yl, pyrazol-5-yl.

The term "oxo" refers to that a carbon atom, nitrogen atom or sulfur atom in a substituent is oxidized to form an oxy substituent (=O). It should be understood that when a carbon atom is substituted with oxy, a carbonyl -C(=O)- is formed.

Unless otherwise specified, the definitions of terms herein also apply to groups containing the terms. For example, the definition of alkyl also applies to alkyl in alkyloxy or cycloalkylalkyl; the definition of cycloalkyl also applies to cycloalkyl in cycloalkyloxy or cycloalkylalkyl; for example, the definition of $C_{1-10}$ alkyl also applies to $C_{1-10}$ alkyl in $C_{1-10}$ alkyloxy or $C_{3-10}$ cycloalkyl-$C_{1-10}$ alkyl; the definition of $C_{3-10}$ cycloalkyl also applies to $C_{3-10}$ cycloalkyl in $C_{3-10}$ cycloalkyloxy or $C_{3-10}$ cycloalkyl-$C_{1-10}$ alkyl.

Those skilled in the art will appreciate that the compounds disclosed herein may exist in the form of various pharmaceutically acceptable salts. If these compounds have a basic center, they may form acid addition salts; if these compounds have an acidic center, they may form base addition salts; if these compounds contain both an acidic center (e.g., a carboxyl group) and a basic center (e.g., an amino group), they may also form inner salts.

The compounds of the present disclosure may exist in the form of solvates (e.g., hydrates), wherein the compounds of the present disclosure contain a polar solvent, in particular water, methanol or ethanol, as a structural element of crystal lattices of the compounds. The amount of polar solvent, in particular water, may be present in a stoichiometric or non-stoichiometric ratio.

According to their molecular structures, the compounds or groups of the present disclosure may be chiral or have chiral carbon atoms, and thus may exist in various enantiomeric forms. Thus, these compounds or groups may exist in racemic forms or optically active forms. For example, when the group A in the general Formula (G), (G') or (GH) is C-$R^A$, and the groups to which the carbon atom is attached are not the same, the carbon atom is a chiral carbon atom, which may have a chiral configuration of R or S. The compounds or intermediates thereof of the present disclosure may be separated into enantiomeric compounds by chemical or physical methods known to those skilled in the art, or used in such form for synthesis. In the case of racemic amines, diastereomers may be prepared from the mixture by reaction with an optically active resolving agent. Examples of suitable resolving agents are optically active acids, such as R and S forms of tartaric acid, diacetyltartaric acid, dibenzoyltartaric acid, mandelic acid, malic acid, lactic acid, appropriate N-protected amino acids (e.g., N-benzoylproline or N-phenylsulfonylproline) or various optically active camphorsulfonic acids. Chromatographic enantiomeric resolution can also be advantageously performed with the aid of optically active resolving agents (e.g., dinitrobenzoylphenylglycine, cellulose triacetate or other carbohydrate derivatives or chirally derivatized methacrylate polymers immobilized on silica gel). Suitable eluents for this purpose are aqueous or alcoholic solvent mixtures, for example, hexane/isopropanol/acetonitrile.

The term "tautomer" refers to functional group isomers resulting from the rapid movement of an atom between two positions in a molecule. The compounds of the present disclosure may exhibit tautomerism. Tautomeric compounds may exist as two or more interconvertible species. Prototropic tautomers result from the migration of a covalently bonded hydrogen atom between two atoms. Tautomers generally exist in equilibrium, and attempts to separate a single tautomer generally result in a mixture whose physicochemical properties are consistent with the mixture of compounds. The position of the equilibrium depends on the intramolecular chemical properties. For example, in many aliphatic aldehydes and ketones such as acetaldehyde, the keto form predominates, while in phenols, the enol form predominates. The present disclosure encompasses all tautomeric forms of the compounds.

The corresponding stable isomers can be separated according to known methods, such as by extraction, filtration or column chromatography.

The term "patient" refers to any animal, including mammals, preferably mice, rats, other rodents, rabbits, dogs, cats, pigs, cattle, sheep, horses or primates, and most preferably humans.

The phrase "therapeutically effective amount" as used herein refers to the amount of active compound or drug that causes a biological or medical response that a researcher, veterinarian, physician or other clinician is seeking in a

tissue, system, animal, individual or human, which comprises one or more of the following: (1) prevention of disease: for example, prevention of disease, disorder or condition in an individual who is susceptible to the disease, disorder or condition but has not yet experienced or developed the pathology or symptoms of the disease; (2) inhibition of disease: for example, inhibition of disease, disorder or condition in an individual who is experiencing or developing the pathology or symptoms of the disease, disorder or condition (i.e., preventing further development of the pathology and/or symptoms); (3) relief of disease: for example, relief of disease, disorder or condition in an individual who is experiencing or developing the pathology or symptoms of the disease, disorder or condition (i.e., reversing the pathology and/or symptoms).

The term "ligand" refers to a macromolecular compound that can recognize and bind to an antigen or receptor associated with a target cell. The role of ligand is to present a drug to a target cell population bound to the ligand, and the examples of ligand include but are not limited to protein hormone, lectin, growth factor, antibody or other molecules that can bind to cells. In the embodiment of the present disclosure, the ligand is represented by LG, and the ligand can form a connection bond with the connecting unit through a heteroatom on the ligand. The preferred ligand is an antibody or antigen-binding fragment thereof, and the antibody is selected from a chimeric antibody, a humanized antibody, a fully human antibody or a murine antibody; preferably a monoclonal antibody.

The term "drug" refers to any compound having a desired biological activity and a reactive functional group, and the reactive function group can be used to incorporate the drug into the conjugate of the present disclosure. The desired biological activity includes diagnosis, cure, relief, treatment, or prevention of diseases in humans or other animals. The reactive functional group forms a bond with the functional group $L^4$ or $L^5$. In some embodiments, the drug has a nitrogen atom or a hydroxyl group that can form a bond with the functional group $L^4$ or $L^5$.

The term "antibody" refers to an immunoglobulin, which comprises a tetrapeptide chain structure formed by two identical heavy chains and two identical light chains connected by interchain disulfide bonds (also known as "monoantibody" or "monoepitope antibody"), or a tetrapeptide chain structure formed by two different heavy chains and two different light chains connected by interchain disulfide bonds (also known as "bi-antibody" or "bi-epitope antibody"). Immunoglobulins are different in amino acid composition and arrangement order of heavy chain constant region, and thus are different in antigenicity. Based on this, immunoglobulins can be divided into five types, or called immunoglobulin isotypes, namely IgM, IgD, IgG, IgA and IgE, and their corresponding heavy chains are $\mu$ chain, $\delta$ chain, $\gamma$ chain, $\alpha$ chain, and $\varepsilon$ chain respectively. According to the difference in the amino acid composition of hinge region and the number and position of heavy chain disulfide bonds, the same type Ig can be divided into different subtypes, for example, IgG can be divided into IgG1, IgG2, IgG3, and IgG4. Light chains are divided into $\kappa$ chain or $\lambda$ chain according to the differences in constant region. Each of the five types of Ig can have $\kappa$ chain or $\lambda$ chain. The antibody described in the present disclosure is preferably a specific antibody against a cell surface antigen on a target cell, and its non-limiting examples are the following antibodies: one or more of anti-HER2 (ErbB2) antibody, anti-EGFR antibody, anti-B7-H3 antibody, anti-c-Met antibody, anti-HER3 (ErbB3) antibody, anti-HER4 (ErbB4) antibody, anti-CD20 antibody, anti-CD22 antibody, anti-CD30 antibody, anti-CD33 antibody, anti-CD44 antibody, anti-CD56 antibody, anti-CD70 antibody, anti-CD73 antibody, anti-CD105 antibody, anti-CEA antibody, anti-A33 antibody, anti-Cripto antibody, anti-EphA2 antibody, anti-G250 antibody, anti-MUCl antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody or anti-Mesothelin antibody; preferably Trastuzumab (trade name Herceptin), Pertuzumab (also known as 2C4, trade name Perjeta), Nimotuzumab (trade name Taixinsheng), Enoblituzumab, Emibetuzumab, Inotuzumab, Pinatuzumab, Brentuximab, Gemtuzumab, Bivatuzumab, Lorvotuzumab, cBR96, and Glematumamab or Glembatumumab.

The sequences of about 110 amino acids near the N-terminals of antibody heavy chain and light chain vary greatly, which are variable regions (Fv regions); the remaining amino acid sequences near the C-terminals are relatively stable, which are constant regions. The variable regions include three hypervariable regions (HVR) and four relatively conservative framework regions (FR). The three hypervariable regions determine the specificity of antibody, also known as complementarity determining region (CDR). Each of light chain variable region (LCVR) and heavy chain variable region (HCVR) consists of three CDR regions and four FR regions, arranged in the following order from the amino terminus to the carboxyl terminus: FR1, CDR1, FR2, CDR2, FR3, CDR3, FR4. The three CDR regions of light chain refer to LCDR1, LCDR2, and LCDR3; the three CDR regions of heavy chain refer to HCDR1, HCDR2, and HCDR3.

The antibodies disclosed herein comprise murine antibody, chimeric antibody, humanized antibody, and fully human antibody, preferably humanized antibody and fully human antibody.

The term "murine antibody" in the present disclosure refers to an antibody prepared from mice according to the knowledge and skills in the art. During preparation, the test subject is injected with a specific antigen, and then a hybridoma expressing an antibody with the desired sequence or functional properties is isolated.

The term "chimeric antibody" refers to an antibody formed by fusing the variable region of a murine antibody with the constant region of a human antibody, which can reduce the immune response induced by the murine antibody. To establish a chimeric antibody, it is necessary to first establish a hybridoma that secretes a mouse-specific monoclonal antibody, then the variable region gene is cloned from the mouse hybridoma cells, and then the constant region gene of human antibody is cloned if necessary, the mouse variable region gene is ligated to the human constant region gene to

form a chimeric gene, which is then inserted into an expression vector, and finally the chimeric antibody molecule is expressed in an eukaryotic system or a prokaryotic system.

The term "humanized antibody", also known as CDR-grafted antibody, refers to an antibody produced by grafting a mouse CDR sequence into a human antibody variable region framework, that is, a human germline antibody framework sequence of different type. It can overcome the heterologous response induced by chimeric antibodies due to carrying a large amount of mouse protein components. Such framework sequences can be obtained from public DNA databases or public references that include germline antibody gene sequences. For example, germline DNA sequences of human heavy and light chain variable region genes can be found in the "VBase" human germline sequence database (available on the Internet at www.mrccpe.com.ac.uk/vbase), and in Kabat, E.A. et al., 1991 Sequences of Proteins of Immunological Interest, 5th edition. In order to avoid a decrease in activity caused by a decrease in immunogenicity, minimal reverse mutations or back mutations may be performed on the human antibody variable region framework sequences to maintain activity. The humanized antibodies disclosed herein also comprise humanized antibodies after CDR affinity maturation has been further performed by phage display. Literature further describing methods for humanizing murine antibodies includes, for example, Queen et al., Proc., Natl. Acad. Sci. USA, 88, 2869, 1991, and the methods of Winter and his colleagues [Jones et al., Nature, 321, 522 (1986), Riechmann, et al., Nature, 332, 323-327 (1988), Verhoeyen, et al., Science, 239, 1534 (1988)].

The term "fully human antibody", "fully humanized antibody" or "completely human antibody" is also called "fully human monoclonal antibody", in which both the variable region and the constant region of antibody are humanized, thereby eliminating immunogenicity and toxic side effects. The development of monoclonal antibodies has gone through four stages, namely: murine monoclonal antibodies, chimeric monoclonal antibodies, humanized monoclonal antibodies and fully human monoclonal antibodies. The present disclosure involves a fully human monoclonal antibody. The main technologies related to the preparation of fully human antibodies include: human hybridoma technology, EBV-transformed B lymphocyte technology, phage display technology, transgenic mouse antibody preparation technology, and single B cell antibody preparation technology.

The term "antigen-binding fragment" refers to one or more fragments of an antibody that retain the ability to specifically bind to an antigen. It has been shown that fragments of a full-length antibody can be used to perform the antigen-binding function of antibody. Examples of binding fragments included in "antigen binding fragments" include: (i) Fab fragment, a monovalent fragment consisting of VL, VH, CL and CH1 domains; (ii) $F(ab')_2$ fragment, a bivalent fragment comprising two Fab fragments connected by a disulfide bridge at the hinge region, (iii) Fd fragment, consisting of VH and CH1 domains; (iv) Fv fragment, consisting of the VH and VL domains of a single arm of an antibody; (v) single domain or dAb fragment (Ward et al., (1989) Nature 341: 544-546), consisting of a VH domain; and (vi) isolated complementarity determining region (CDR); or (vii) a combination of two or more isolated CDRs, optionally connected by a synthetic linker. In addition, although the two domains VL and VH of Fv fragment are encoded by separate genes, they can be connected by a synthetic linker using recombinant methods, so that they can be produced as a single protein chain in which the VL and VH regions are paired to form a monovalent molecule (called single-chain Fv (scFv); see, for example, Bird et al. (1988) Science 242: 423-426; and Huston et al. (1988) Proc. Natl. Acad. Sci USA 85: 5879-5883). Such single-chain antibodies are also intended to be included in the term "antigen-binding fragment" of antibody. Such antibody fragments are obtained using conventional techniques known to those skilled in the art, and the fragments are screened for functionality in the same manner as for intact antibodies. Antigen-binding fragments can be produced by recombinant DNA technology or by enzymatic or chemical cleavage of intact immunoglobulins. The antibody may be an antibody of different isotype, for example, an IgG (e.g., IgG1, IgG2, IgG3 or IgG4 subtype), IgA1, IgA2, IgD, IgE or IgM antibody.

Fab is an antibody fragment having a molecular weight of about 50,000 and having antigen-binding activity among fragments obtained by treating an IgG antibody molecule with protease papain (cleaving the amino acid residue at position 224 of H chain), wherein about half of the N-terminal side of the H chain and the entire L chain are bound together by disulfide bonds.

$F(ab')_2$ is an antibody fragment having a molecular weight of about 100,000 and having antigen-binding activity, which is obtained by digesting the lower part of two disulfide bonds in the hinge region of IgG with the enzyme pepsin and comprises two Fab regions connected at the hinge position.

Fab' is an antibody fragment having a molecular weight of about 50,000 and having antigen-binding activity, which is obtained by cleaving the disulfide bonds of hinge region of the above-mentioned $F(ab')_2$.

In addition, the Fab' can be produced by inserting the DNA encoding the Fab' fragment of the antibody into a prokaryotic expression vector or an eukaryotic expression vector and introducing the vector into a prokaryotic organism or an eukaryotic organism to express the Fab'.

The term "single-chain antibody", "single-chain Fv" or "scFv" refers to a molecule comprising an antibody heavy chain variable domain (or region; VH) and an antibody light chain variable domain (or region; VL) connected by a linker. Such scFv molecules may have a general structure: $NH_2$-VL-linker-VH-COOH or $NH_2$-VH-linker-VL-COOH. Suitable prior art linkers consist of repeated GGGGS amino acid sequences or variants thereof, for example, variants using 1 to 4 repeats (Holliger et al. (1993), Proc. Natl. Acad. Sci. USA 90: 6444-6448). Other linkers that can be used in the present disclosure

are described by Alfthan et al. (1995), Proc. Natl. Acad. Sci. USA 90: 6444-6448.otein Eng.8:725-731, Choi et al. (2001), Eur. J. Immunol.31:94-106, Hu et al. (1996), Cancer Res.56:3055-3061, Kipriyanov et al. (1999), J. Mol. Biol.293:41-56, and Roovers et al. (2001), Cancer Immunol.

The term "CDR" refers to one of the six hypervariable regions within the variable domain of an antibody that primarily contribute to antigen binding. One of the most commonly used definitions of the six CDRs is provided by Kabat E.A. et al. (1991) Sequences of proteins of immunological interest. NIH Publication 91-3242). As used herein, the Kabat definition of CDR applies only to CDR1, CDR2 and CDR3 (CDR L1, CDR L2, CDR L3 or L1, L2, L3) of the light chain variable domain, and CDR2 and CDR3 (CDR H2, CDR H3 or H2, H3) of the heavy chain variable domain.

The term "antibody framework" refers to a portion of variable domain VL or VH that serves as a scaffold for antigen-binding loop (CDR) of that variable domain. Essentially, it is a variable domain without CDR.

The term "epitope" or "antigenic determinant" refers to a site on an antigen to which an immunoglobulin or antibody specifically binds. An epitope typically comprises at least 3, 4, 5, 6, 7, 8, 9, 10, 11, 12, 13, 14 or 15 consecutive or non-consecutive amino acids in a unique spatial conformation. See, for example, Epitope Mapping Protocols in Methods in Molecular Biology, Vol. 66, G.E.Morris, Ed. (1996).

The terms "specific binding", "selective binding", "selectively binding" and "specifically binding" refer to the binding of an antibody to an epitope on a predetermined antigen. Typically, the antibody binds with an affinity (KD) of about less than $10^{-7}$M, for example, about less than $10^{-8}$M, $10^{-9}$M or $10^{-10}$M or less.

The term "nucleic acid molecule" refers to DNA molecule and RNA molecule. Nucleic acid molecules can be single-stranded or double-stranded, but are preferably double-stranded DNA. A nucleic acid is "operably linked" when it is placed in a functional relationship with another nucleic acid sequence. For example, if a promoter or enhancer affects the transcription of a coding sequence, then the promoter or enhancer is operably linked to the coding sequence.

The term "vector" refers to a nucleic acid molecule capable of transporting another nucleic acid to which it has been ligated. In one embodiment, the vector is a "plasmid", which refers to a circular double-stranded DNA loop into which an additional DNA segment can be ligated. In another embodiment, the vector is a viral vector, in which an additional DNA segment can be ligated to the viral genome. The vectors disclosed herein are capable of autonomous replication in a host cell into which they have been introduced (e.g., bacterial vectors with bacterial replication origins, and episomal mammalian vectors) or can be integrated into the genome of a host cell after introduction into the host cell, thereby replicating along with the host genome (e.g., non-episodic mammalian vectors).

Methods for producing and purifying antibodies and antigen-binding fragments are well known in the prior art, such as Guide to Antibody Experimental Technology, Chapters 5-8 and 15, the Cold Spring Harbor. Antigen-binding fragments can also be prepared by conventional methods. In the antibodies or antigen-binding fragments described in the present invention, one or more human FR regions are added into a non-human CDR region by using genetic engineering methods. Human FR germline sequences can be obtained from the ImMunoGeneTics (IMGT) website http://imgt.cines.fr by alignment on the IMGT human antibody variable region germline gene database and MOE software, or from the Journal of Immunoglobulins, 2001 ISBN012441351.

The term "host cell" refers to a cell into which an expression vector has been introduced. Host cells may include bacterial, microbial, plant or animal cells. Easily transformed bacteria include members of the *enterobacteriaceae* family, such as strains of *Escherichia coli* or *Salmonella*; *Bacillaceae,* such as *Bacillus subtilis*; *Pneumococcus*; *Streptococcus* and *Haemophilus influenzae.* Suitable microorganisms include *Saccharomyces cerevisiae* and *Pichia pastoris.* Suitable animal host cell lines include CHO (Chinese hamster ovary cell line) and NS0 cells.

The engineered antibodies or antigen-binding fragments disclosed herein can be prepared and purified by conventional methods. For example, cDNA sequences encoding heavy and light chains can be cloned and recombined into GS expression vectors. The recombinant immunoglobulin expression vector can stably transfect CHO cells. As a more recommended prior art, mammalian expression systems lead to glycosylation of antibodies, especially at the highly conserved N-terminal site in the Fc region. Positive clones are expanded in serum-free medium in a bioreactor to produce antibodies. The culture fluid that secretes antibodies can be purified by conventional techniques. For example, purification is performed using an A or G Sepharose FF column containing an adjusted buffer. Non-specifically bound components are washed away. The bound antibodies are then eluted using a pH gradient method, and the antibody fragments are detected by SDS-PAGE and collected. The antibodies can be filtered and concentrated by conventional methods. Soluble mixtures and polymers can also be removed by conventional methods, such as molecular sieves and ion exchange. The obtained product should be immediately frozen at, for example, -70°C, or freeze-dried.

The term "peptide" refers to a compound fragment between amino acids and proteins, which is composed of two or more amino acid molecules connected to each other by peptide bonds, and is a structural and functional fragment of protein. For example, hormones, enzymes and the like all are essentially peptides.

The term "sugar" refers to a biological macromolecule composed of three elements: C, H, and O, which can be classified into monosaccharide, disaccharide, and polysaccharide.

## Beneficial effects

The compounds and conjugates disclosed in the present invention have excellent tumor cell inhibition activity, stability, and *in vivo* efficacy in animals, and can be used as effective drugs for inhibiting tumor cells and preventing and/or treating cancer.

## Brief Description of the Drawings

Figure 1 shows the test results of the bystander killing effect of the antibody-drug conjugate of the present invention on tumor cells in Test Example 4;

Figure 2 shows the test results of the growth inhibition on nude mouse NCI-N87 transplanted tumor in the experiment of Test 5A in Test Example 5;

Figure 3 shows the summary results of the weight change of experimental mice in the experiment of Test 5A in Test Example 5;

Figure 4 shows the test results of the growth inhibition of nude mouse NCI-N87 transplanted tumor in the experiment of Test 5B in Test Example 5;

Figure 5 shows the summary results of the weight change of experimental mice in the experiment of Test 5B in Test Example 5;

Figure 6 shows the test results of the dissociation degree value of the plasma stability of the antibody-drug conjugate of the present invention in Test Example 6;

Figure 7 shows the test results of the growth inhibition on nude mouse NCI-N87 transplanted tumor in the experiment of Test 5C in Test Example 5;

Figure 8 shows the summary results of the weight change of experimental mice in the experiment of Test 5C in Test Example 5;

Figure 9 shows the results of the bystander killing test of Pertuzumab antibody-drug conjugate in Test Example 7;

Figure 10 shows the results of the bystander killing test of antibody-drug conjugate on EGFR target tumor cells in Test Example 9;

Figure 11 shows the test results of the growth inhibition on JIMT-1 mouse subcutaneous transplanted tumor model in Test Example 10;

Figure 12 shows the summary results of the weight change of experimental mice in Test Example 10;

Figure 13 shows the test results of the growth inhibition on NCI-H322 mouse subcutaneous transplanted tumor model in Test Example 11;

Figure 14 shows the summary results of the weight change of experimental mice in Test Example 11;

Figure 15 shows the test results of the bystander killing effect of ADC-113 on HER2 target tumor cells in Test Example 14;

Figure 16 shows the test results of the bystander killing effect of ADC-115 on HER2 target tumor cells in Test Example 14;

Figure 17 shows the test result of the growth inhibition on nude mouse NCI-N87 transplanted tumors administrated with different doses of ADC-113 in Test Example 16;

Figure 18 shows the summary results of the weight change of experimental mice administrated with different doses of ADC-113 in Test Example 16;

Figure 19 shows the test results of the growth inhibition on nude mouse NCI-N87 transplanted tumor administrated with ADC-115 in Test Example 17;

Figure 20 shows the summary results of the weight change of experimental mice administrated with ADC-115 in Test Example 17.

## DETAILED DESCRIPTION

The technical solution of the present disclosure will be further described in detail below in conjunction with specific examples. It should be understood that the following embodiments are only exemplary illustrations and explanations of the present disclosure, and should not be interpreted as limiting the scope of protection of the present disclosure. All technologies realized based on the above contents of the present disclosure fall within the scope sought to be protected by the present disclosure.

Unless otherwise specified, the starting materials and reagents used in the following examples were commercially available products or could be prepared by known methods.

I. Antibody Examples

The following antibodies were prepared according to conventional antibody methods, for example, after vector construction, eukaryotic cells such as HEK293 cells (Life Technologies Cat. No. 11625019) could be transfected.

The exemplary antibody sequences are as follows:
The following shows the sequences of Trastuzumab

light chain

DIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIKRTVAAPSVFIF
PPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC

SEQ ID NO:1

heavy chain

EVQLVESGGGLVQPGGSLRLSCAASGFNIKDTYIHWVRQAPGKGLEWVARIYPTN
GYTRYADSVKGRFTISADTSKNTAYLQMNSLRAEDTAVYYCSRWGGDGFYAMDYWG
QGTLVTVSSASTKGPSVFPLAPSSKSTSGGTAALGCLVKDYFPEPVTVSWNSGALTSGV
HTFPAVLQSSGLYSLSSVVTVPSSSLGTQTYICNVNHKPSNTKVDKKVEPKSCDKTHTCP
PCPAPELLGGPSVFLFPPKPKDTLMISRTPEVTCVVVDVSHEDPEVKFNWYVDGVEVHN
AKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGS
FFLYSKLTVDKSRWQQGNVFSCSVMHEALHNHYTQKSLSLSPGK

SEQ ID NO:2

The following shows the sequences of Pertuzumab

light chain

DIQMTQSPSSLSASVGDRVTITCKASQDVSIGVAWYQQKPGKAPKLLIYSASYRYT GVPSRFSGSGSGTDFTLTISSLQPEDFATYYCQQYYIYPYTFGQGTKVEIKRTVAAPSVFI FPPSDEQLKSGTASVVCLLNNFYPREAKVQWKVDNALQSGNSQESVTEQDSKDSTYSLS STLTLSKADYEKHKVYACEVTHQGLSSPVTKSFNRGEC

SEQ ID NO.3

heavy chain

EVQLVESGGGLVQPGGSLRLSCAASGFTFTDYTMDWVRQAPGKGLEWVADVNPN SGGSIYNQRFKGRFTLSVDRSKNTLYLQMNSLRAEDTAVYYCARNLGPSFYFDYWGQG TLVTVSSASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKTHTCPPCP APELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQ VYTLPPSREEMTKNQVSLTCLVKGFYPSDIAVEWESNGQPENNYKTTPPVLDSDGSFFL YSKLTVDKSRWQQGNVFSCSVMHEALHNHYTQKSLSLSPGK

SEQ ID NO.4

The following shows the sequences of Nimotuzumab

light chain

DIQMTQSPSSLSASVGDRVTITCRSSQNIVHSNGNTYLDWYQQTPGKAPKLLIYKVS NRFSGVPSRFSGSGSGTDFTFTISSLQPEDIATYYCFQYSHVPWTFGQGTKLQITREVAAP SVFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC

SEQ ID NO:5

heavy chain

QVQLQQSGAEVKKPGSSVKVSCKASGYTFTNYYIYWVRQAPGQGLEWIGGINPTS GGSNFNEKFKTRVTITVDESTNTAYMELSSLRSEDTAFYFCARQGLWFDSDGRGFDFWG QGSTVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPR EPQVYTLPPSRDELTKNQVSLTCLVKGFYPSDIAVEWESNGQPENNYKTTPPVLDSDGSF FLYSKLTVDKSRWQQGNVFSCSVMHEALHNHYTQKSLSLSPGK

SEQ ID NO:6

The following shows the sequences of Patritumab

light chain

DIEMTQSPDSLAVSLGERATINCRSSQSVLYSSSNRNYLAWYQQNPGQPPKLLIYW ASTRESGVPDRFSGSGSGTDFTLTISSLQAEDVAVYYCQQYYSTPRTFGQGTKVEIKRTV AAPSVFIFPPSDEQLKSGTASVVCLLNNFYPREAKVQWKVDNALQSGNSQESVTEQDSK DSTYSLSSTLTLSKADYEKHKVYACEVTHQGLSSPVTKSFNRGEC

SEQ ID NO:7

heavy chain

QVQLQQWGAGLLKPSETLSLTCAVYGGSFSGYYWSWIRQPPGKGLEWIGEINHSG STNYNPSLKSRVTISVETSKNQFSLKLSSVTAADTAVYYCARDKWTWYFDLWGRGTLV TVSSASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKRVEPKSCDKTHTCPPCPAPE LLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYT LPPSREEMTKNQVSLTCLVKGFYPSDIAVEWESNGQPENNYKTTPPVLDSDGSFFLYSKL TVDKSRWQQGNVFSCSVMHEALHNHYTQKSLSLSPGK

SEQ ID NO:8

II. Compound Examples

The structures of the compounds were determined by nuclear magnetic resonance (NMR) or mass spectrometry (MS). Chemical shifts δ are given in units of $10^{-6}$ (ppm). NMR measurements were performed by using a Bruker nuclear magnetic spectrometer. The solvents used were deuterated dimethyl sulfoxide (DMSO-d6), deuterated chloroform ($CDCl_3$), and deuterated methanol ($CD_3OD$). The internal standard was tetramethylsilane (TMS).

LCMS was performed by using: Agilent 1260 Infinity II (ESI) mass spectrometer, Waters UPLC H Class plus (ESI)

or Shimadzu LCMS-2020 (ESI).

High performance liquid chromatography (HPLC) analysis was performed by using: Agilent 1260 or Shimadzu LC-20AD.

Preparative high performance liquid chromatography (pre-HPLC) was performed by using: GILSON GX-281 or Agilent 1260 Infinity II preparative HPLC.

Chiral preparation was performed by supercritical fluid chromatography (SFC) using Shimadzu LC-30Adsf or Shimadzu LC-20AD.

Thin layer chromatography silica gel plate used was GF254 acrylic adhesive silica gel plate produced by Anhui Liangchen Silicon Source Material Co., Ltd. The silica gel plate used in thin layer chromatography (TLC) was 0.2 mm silica gel plate, and 0.5 mm silica gel plate was used for separation and purification of product by thin layer chromatography.

For column chromatography, 200 to 300 mesh silica gel from Anhui Liangchen Silicon Source Materials Co., Ltd. was generally used as carrier.

Kinase average inhibition rate and $IC_{50}$ value were determined using SpectraMax i3X microplate reader (MD Company, USA).

The known starting materials of the present disclosure could be synthesized by methods known in the art, or could be purchased from companies such as Bitec Pharmaceuticals, Leyan, Shaoyuan Chemical Technology, and Annaiji Chemicals.

In the following examples, unless otherwise specified, the reactions were carried out under argon or nitrogen atmosphere.

Argon or nitrogen atmosphere meant that the reaction bottle was connected to an argon or nitrogen balloon with a volume of about 1L.

Hydrogen atmosphere meant that the reaction bottle was connected to a hydrogen balloon with a volume of about 1L.

Hydrogenation reaction was usually carried out by repeating three times of the operations of vacuuming and filling hydrogen.

Oxygen atmosphere meant that the reaction bottle was connected to an oxygen balloon with a volume of about 1L.

In the following examples, unless otherwise specified, the solution referred to an aqueous solution, and the reaction temperature was room temperature, which was 20 °C to 30 °C.

The progress of the reactions in the examples was monitored by thin layer chromatography (TLC) using developing solvents, the eluent system of column chromatography used for purifying compounds and the developing solvent system of thin layer chromatography included: A: dichloromethane/methanol system, B: petroleum ether/ethyl acetate system, in which the solvent volume ratio was adjusted according to the polarity of compounds, and a small amount of alkaline or acidic reagent such as triethylamine and acetic acid could also be added for adjustment.

Example 2-1: Preparation of Compound 1 and its isomers **1-A, 1-B, 1-C and 1-D**

*N*-((1*R*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **1-A**

*N*-((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **1-B**

*N*-((1*S*,9*R*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **1-C**

*N*-((1*R*,9*R*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **1-D**

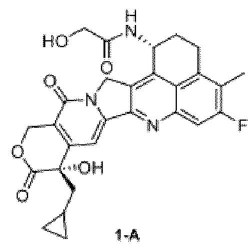
1-A

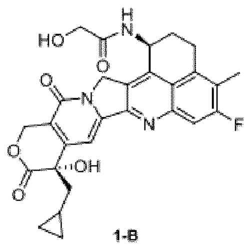
1-B

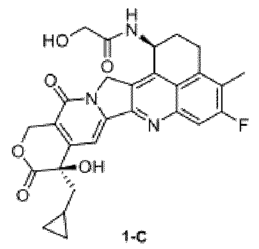
1-C

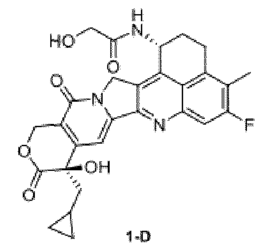
1-D

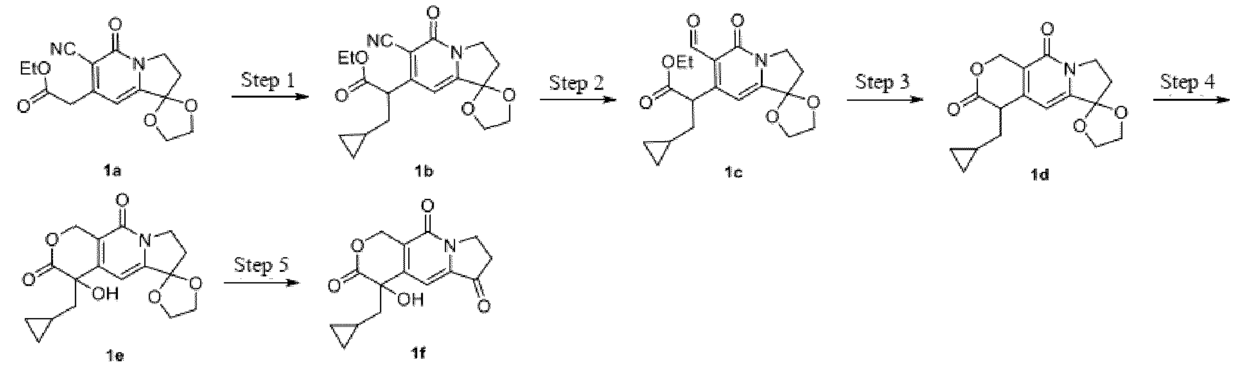

1a 1b 1c 1d 1e 1f

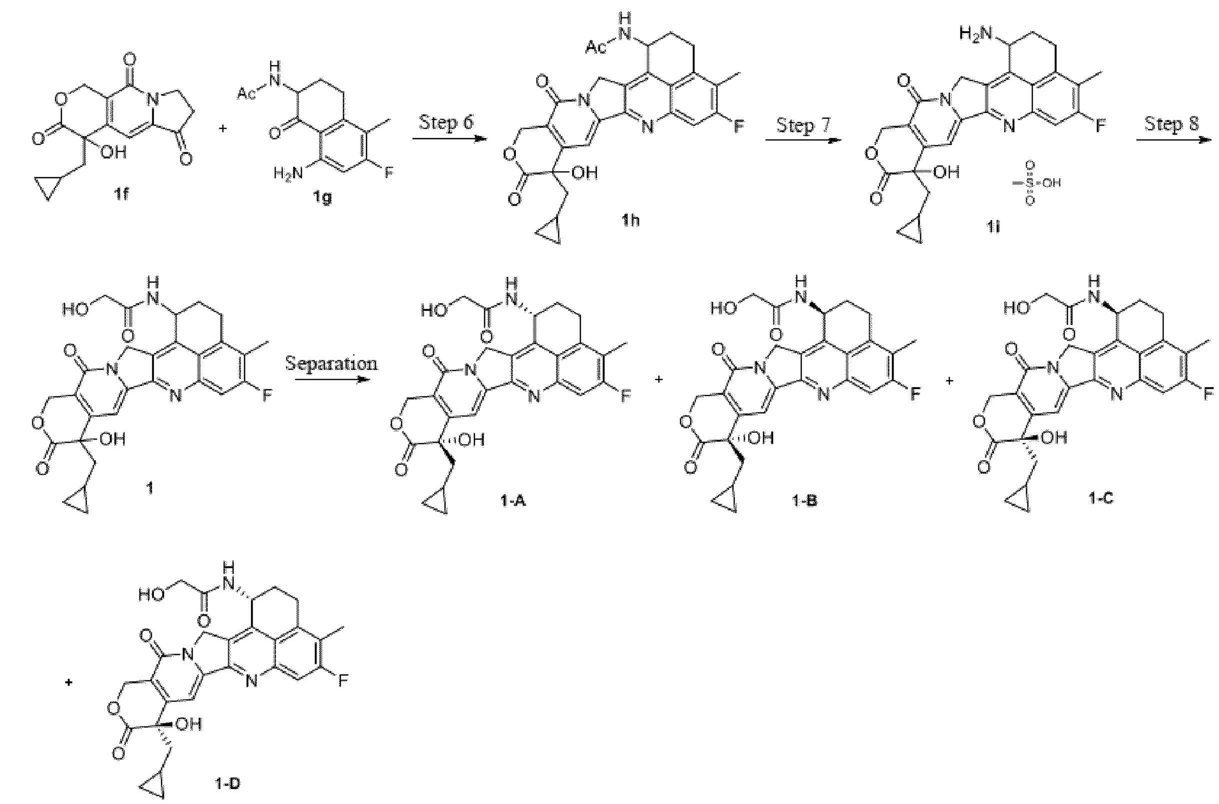

1f 1g 1h 1i 1 1-A 1-B 1-C 1-D

Step 1

Ethyl 2-(6-cyano-5-oxo-2,3-dihydro-5*H*-spiro[indolizine-1,2'-[1,3]dioxolan]-7-yl)- 3-cyclopropyl-propanoate **1b**

**1a** (1.01 g, 3.31 mmol, prepared by applying the method disclosed in Example 30 on page 28 of patent application "WO2019238046") was dissolved in 15 mL of acetonitrile, added with bromomethyl cyclopropane (894.93 mg, 6.63 mmol) and potassium carbonate (916.16 mg, 6.63 mmol), and stirred at 80 °C for 13 hours. 10 mL of water was added. The diluted reaction solution was extracted with ethyl acetate (15 mLx2). The organic phase was washed with saturated sodium chloride solution (10 mLx2), then dried over anhydrous sodium sulfate and filtered, and the filtrate was concentrated by

vacuum distillation. The resulting residue was purified by silica gel column chromatography using the eluent system A to obtain the title product **1b** (1.12 g, yield: 92%) in the form of yellow solid.
MS m/z (ESI): 359.1 [M+1].

Step 2

Ethyl 3-cyclopropyl-2-(6-formyl-5-oxo-2,3-dihydro-5*H*-spiro[indolizine-1,2'-[1,3] dioxolan]-7-yl)propionate **1c**

**1b** (1.12 g, 3.05 mmol) was dissolved in a mixed solvent of 5 mL water, 5 mL acetonitrile and 5 mL formic acid, added with Raney nickel (261.72 mg) and subjected to replacement with hydrogen gas for three times. The reaction mixture was stirred at 60 °C for 4 hours under hydrogen gas (15 Psi). The reaction mixture was filtered through Celite. The filter cake was washed with dichloromethane (50 mL x3). The filtrate was washed with aqueous hydrochloric acid (4M, 20 mL), and then washed with sodium carbonate aqueous solution (12M, 50 mL). The organic phase was dried over anhydrous sodium sulfate and filtered. The filtrate was concentrated by vacuum distillation. The residue was purified by reverse phase liquid chromatography (separation conditions: chromatographic column: 120 g Flash Column Welch Ultimate XB_C18 20-40 μm; mobile phase: A-water: B-acetonitrile, gradient elution, flow rate: 85 mL/min, instrument: ISCO) to obtain the title product **1c** (680 mg, yield: 60%) in the form of yellow solid.
MS m/z (ESI): 362.1 [M+1].

Step 3

4-(Cyclopropylmethyl)-1,4,7,8-tetrahydro-3*H*,10*H*-spiro[pyrano[3,4-*f*] indolizine-6,2'-[1,3]dioxolane]-3,10-dione **1d**

**1c** (680 mg, 1.85 mmol) was dissolved in 10 mL of dichloromethane under protection of nitrogen gas, cooled to 0 °C in ice-water bath, and added with sodium borohydride (108.02 mg, 2.86 mmol) in batches, and the reaction solution was stirred at 0 °C for 30 minutes. At 25 °C, acetic acid (133.15 mg, 2.22 mmol) was added dropwise, gas was generated, and stirring was continued for 2 hours. At 15 °C, 30 mL of water was added dropwise, gas was generated, and stirring was continued at 15 °C for 1.5 hours. The reaction solution was washed with water (50 mL), the washed organic phase was added with p-toluenesulfonic acid monohydrate (35.15 mg, 184.78 μmol) at 15 °C and stirred at 15 °C for 12 hours. 50 mL of water was added and the reaction solution was extracted with dichloromethane (45 mLx3), the organic phase was dried over anhydrous sodium sulfate and filtered, and the filtrate was concentrated by vacuum distillation. The obtained residue was purified by silica gel column chromatography using the developing system B to obtain the title product **1d** (200 mg, yield: 32%) in the form of yellow oil.
MS m/z (ESI): 318.1 [M+1].

Step 4

4-(Cyclopropylmethyl)-4-hydroxy-1,4,7,8-tetrahydro-3*H*,10*H*-spiro[pyrano[3,4-*f*] indolizine-6,2'-[1,3]dioxolane]-3,10-dione **1e**

**1d** (202.13 mg, 598.74 μmol) was dissolved in 0.5 mL of methanol, cooled to 0 °C in ice-water bath, added with potassium carbonate (82.75 mg, 598.74 μmol) in batches, and stirred at 0 °C for 5 hours under oxygen bubbling (15 psi). The reaction solution was poured into 10 mL of saturated ammonium chloride aqueous solution, and concentrated by vacuum distillation to remove methanol. The reaction solution was extracted with dichloromethane (20 mLx3). The organic phase was dried over anhydrous sodium sulfate and filtered, and concentrated by vacuum distillation to obtain the crude title product **1e** (140 mg) in the form of yellow oil, and the product was used directly in the next step without purification.
MS m/z (ESI): 334.1 [M+1].

Step 5

4-(Cyclopropylmethyl)-4-hydroxy-7,8-dihydro-1*H*-pyrano[3,4-*f*] indolizine-3,6,10(4*H*)-trione **1f**

**1e** (64.52 mg, 180.00 μmol) was dissolved in a mixed solution of 0.5 mL of trifluoroacetic acid and 0.125 mL of water, and stirred at 25 °C for 1 hour. The reaction solution was concentrated by vacuum distillation, and the resultant residue without purification was used to obtain the crude title product **1f** (40 mg) in the form of yellow solid, and the product was used directly in the next reaction without purification.
MS m/z (ESI): 290.1 [M +1].

Step 6 *N*-(9-(Cyclopropylmethyl)-5-fluoro-9-hydroxy-4-methyl-10,13-dioxo-2,3,9,10,13,15-hexahydro-1*H*,12*H*-benzo [*de*]pyrano[3',4':6,7]indolizino[1,2-*b*]quinolin-1-yl)acetamide **1h**

**1f** (40 mg, 128.59 μmol) and **1g** (38.62 mg, 154.31 μmol, prepared by applying the method disclosed in Example 5-1 on page 61 of patent application "CN111065621A"), pyridinium 4-toluene sulfonate (6.46 mg, 25.72 μmol) was dissolved in 0.5 mL of toluene, and stirred at 130 °C for 2 hours under nitrogen protection. The reaction solution was filtered through Celite, the filtrate was added with 10 mL of water and extracted with dichloromethane (8 mL x 3). The organic phase was dried over anhydrous sodium sulfate and filtered, and the filtrate was concentrated under reduced pressure. The resulting residue was purified by preparative thin-layer chromatography using the eluent system A to obtain the title product **1h** (15 mg, yield: 23%) in the form of brown solid.
MS m/z (ESI): 504.2 [M+1].

Step 7

1-Amino-9-(cyclopropylmethyl)-5-fluoro-9-hydroxy-4-methyl-1,2,3,9,12,15-hexahydro-10*H*,13*H*-benzo[*de*]pyrano [3',4':6,7]indolizino[1,2-*b*]quinoline-10,13-dione methanesulfonate **1i**

**1h** (50 mg, 96.5 μmol) was dissolved in 1 mL of 1,2-dimethoxyethane, added with 0.5 mL of methanesulfonic acid and 0.5 mL of water, and stirred at 85 °C for 18 hours. The reaction solution was poured into 10 mL o f water, adjusted to pH 7-8 with saturated sodium bicarbonate solution, and extracted with dichloromethane (10 mLx5). The organic phase was dried over anhydrous sodium sulfate and filtered, and the filtrate was concentrated under reduced pressure to obtain the crude title product **1i** (40 mg) in the form of brown solid. The product was used directly in the next reaction without purification.
MS m/z (ESI): 462.3 [M+1].

Step 8

*N*-((1*R*,9*S*)-9-(Cyclopropylmethyl)-5-fluoro-9-hydroxy-4-methyl-10,13-dioxo-2,3,9,10,13, 15-hexahydro-1*H*,12*H*-benzo [*de*]pyrano[3',4':6,7]indolizino[1,2-*b*]quinolin-1-yl)- 2-hydroxyacetamide **1-A**

*N*-((1*S*,9*S*)-9-(Cyclopropylmethyl)-5-fluoro-9-hydroxy-4-methyl-10,13-dioxo-2,3,9,10,13, 15-hexahydro-1*H*,12*H*-benzo [*de*]pyrano[3',4':6,7]indolizino[1,2-*b*]quinolin-1-yl)- 2-hydroxyacetamide **1-B**

*N*-((15,9*R*)-9-(Cyclopropylmethyl)-5-fluoro-9-hydroxy-4-methyl-10,13-dioxo-2,3,9,10,13, 15-hexahydro-1*H*,12*H*-benzo [*de*]pyrano[3',4':6,7]indolizino[1,2-*b*]quinolin-1-yl)- 2-hydroxyacetamide **1-C**

*N*-((1*R*,9*R*)-9-(Cyclopropylmethyl)-5-fluoro-9-hydroxy-4-methyl-10,13-dioxo-2,3,9,10,13, 15-hexahydro-1*H*,12*H*-benzo [*de*]pyrano[3',4':6,7]indolizino[1,2-*b*]quinolin-1-yl)- 2-hydroxyacetamide **1-D**

The mesylate of **1i** (35 mg, 62.2 μmol) and glycolic acid (5.7 mg, 74.6 μmol) were dissolved in 1.5 mL of *N,N*-dimethylformamide, then added with 1-hydroxybenzotriazole (12.6 mg, 93.2 μmol), *N,N*-diisopropylethylamine (16.1 mg, 124 μmol) and 1-(3-dimethylaminopropyl)-3-ethylcarbodiimide hydrochloride (17.9 mg, 93.2 μmol) in sequence, and stirred at 25 °C for 1 hour. The reaction solution was added with 20 mL of water, and the diluted reaction solution was extracted with dichloromethane (10 mLx5). The organic phase was dried over anhydrous sodium sulfate and filtered, and the filtrate was concentrated under reduced pressure. The residue was purified by supercritical fluid chromatography to obtain four single-configuration title products **1-A, 1-B, 1-C** and **1-D:**

Single-configuration Compound **I** of Compound **1** (designated as 1-B, 1.02 mg)

SFC analysis: retention time: 1.472 minutes, purity: 98%. (Chromatographic column: Chiralpak IC-3 50x4.6mm I.D., 3μm, mobile phase: A-carbon dioxide, B-methanol and acetonitrile (0.05% diethylamine), isocratic elution: B: 60%, flow rate: 3 mL/min, instrument: Shimadzu LC-30ADsf).
MS m/z (ESI): 520.5 [M+1].
$^1$HNMR (400 MHz, $CD_3OD$) δ 7.71 - 7.61 (m, 2H), 5.73 - 5.66 (m, 1H), 5.63 - 5.56 (m, 1H), 5.40 - 5.32 (m, 2H), 5.12 - 5.06 (m, 1H), 4.28 - 4.22 (m, 1H), 4.18 - 4.12 (m, 1H), 3.32 - 3.32 (m, 1H), 3.20 - 3.11 (m, 1H), 2.42 (s, 3H), 2.39 - 2.30 (m, 1H), 2.29 - 2.19 (m, 1H), 1.97 - 1.89 (m, 1H), 1.88 - 1.80 (m, 1H), 0.94 - 0.86 (m, 1H), 0.51 - 0.38 (m, 2H), 0.14 - 0.07 (m, 1H), 0.04 - - 0.01 (m, 1H).

Single-configuration Compound **II** of Compound **1** (designated as 1-A, 1.02 mg)

SFC analysis: retention time: 2.075 minutes, purity: 93%. (Chromatographic column: Chiralpak AS-3 50x4.6mm I.D., 3μm, mobile phase: A-carbon dioxide, B-methanol (0.05% diethylamine), gradient elution: B%: 5%-40%, flow rate: 3 mL/min, instrument: Shimadzu LC-30ADsf).
MS m/z (ESI): 520.4 [M+1].
$^{1}$HNMR (400 MHz, $CD_3OD$) δ 7.66 - 7.53 (m, 2H), 5.70 - 5.63 (m, 1H), 5.60 - 5.54 (m, 1H), 5.41 - 5.31 (m, 2H), 5.22 - 5.14 (m, 1H), 4.34 - 4.23 (m, 1H), 4.22 - 4.11 (m, 1H), 3.43 - 3.36 (m, 1H), 3.22 - 3.13 (m, 1H), 2.43 - 2.37 (m, 3H), 2.35 - 2.26 (m, 2H), 1.95 - 1.80 (m, 2H), 0.93 - 0.86 (m, 1H), 0.55 - 0.35 (m, 2H), 0.17 - 0.08 (m, 1H), 0.07 - -0.03 (m, 1H).

Single-configuration Compound **III** of Compound **1** (1.21 mg)

SFC analysis: retention time: 0.468 minutes, purity: 98%. (Chromatographic column: Chiralcel OD-3 50x4.6mm I.D., 3μm, mobile phase: A-carbon dioxide, B-methanol (0.05% diethylamine), gradient elution: B%: 5%-40%, flow rate: 3 mL/min, instrument: Shimadzu LC-30ADsf).
MS m/z (ESI): 520.1 [M+1].
$^{1}$HNMR (400 MHz, $CD_3OD$) δ 7.71 - 7.64 (m, 2H), 5.73 - 5.67 (m, 1H), 5.62 - 5.56 (m, 1H), 5.43 - 5.40 (m, 1H), 5.38 - 5.35 (m, 1H), 5.18 - 5.16 (m, 1H), 4.28 - 4.21 (m, 1H), 4.18 - 4.12 (m, 1H), 3.50 - 3.45 (m, 1H), 3.20 - 3.12 (m, 1H), 2.49 - 2.40 (m, 3H), 2.36 - 2.23 (m, 2H), 1.96 - 1.82 (m, 2H), 0.93 - 0.88 (m, 1H), 0.52 - 0.39 (m, 2H), 0.15 - 0.08 (m, 1H), 0.06 - -0.02 (m, 1H).

Single-configuration Compound **IV** of Compound **1** (0.27 mg)

SFC analysis: retention time: 0.464 minutes, purity: 98%. (Chromatographic column: Chiralcel OD-3 50x4.6mm I.D., 3μm, mobile phase: A-carbon dioxide, B-methanol (0.05% diethylamine), gradient elution: B%: 5%-40%, flow rate: 3 mL/min, instrument: Shimadzu LC-30ADsf).
MS m/z (ESI): 520.1 [M+1].
$^{1}$H NMR (400 MHz, $CD_3OD$) δ 7.71 - 7.62 (m, 2H), 5.73 - 5.67 (m, 1H), 5.62 - 5.56 (m, 1H), 5.41 - 5.35 (m, 2H), 5.23 - 5.20 (m, 1H), 4.33 - 4.23 (m, 1H), 4.21 - 4.12 (m, 1H), 3.38 - 3.36 (m, 1H), 3.22 - 3.13 (m, 1H), 2.44 (s, 3H), 2.37 - 2.28 (m, 2H), 1.96 - 1.83 (m, 2H), 0.91 - 0.86 (m, 1H), 0.50 - 0.38 (m, 2H), 0.14 - 0.09 (m, 1H), 0.06 - -0.01 (m, 1H).

Examples 2-2 to 2-6

By referring to the method of Example 2-1, corresponding substrates were used to prepare the following compounds:

| | | |
|---|---|---|
| 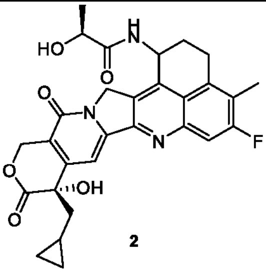 2 | 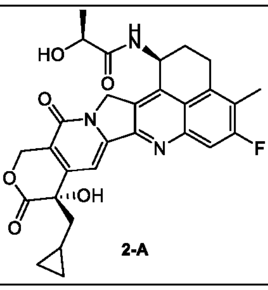 2-A | 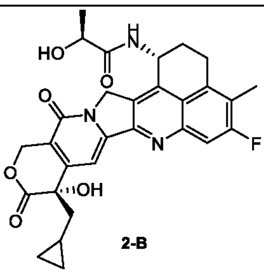 2-B |
| 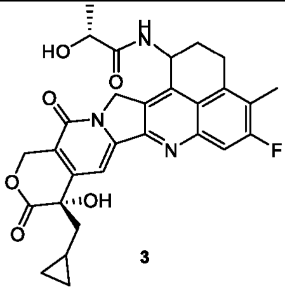 3 | 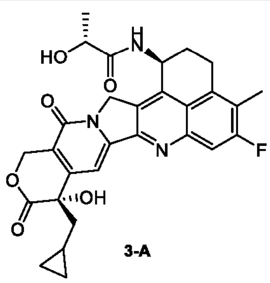 3-A | 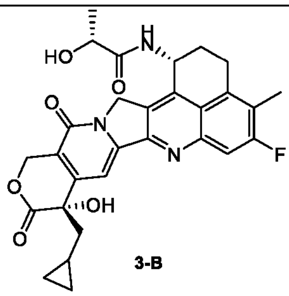 3-B |
| 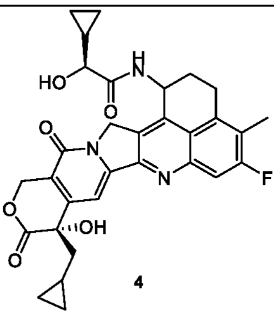 4 | 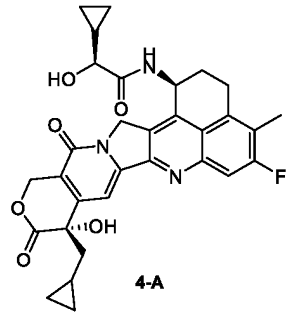 4-A | 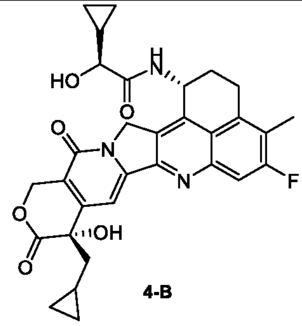 4-B |
| 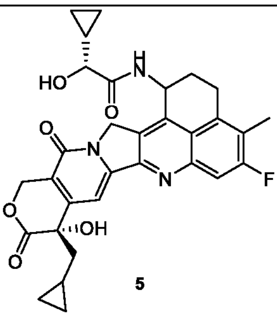 5 | 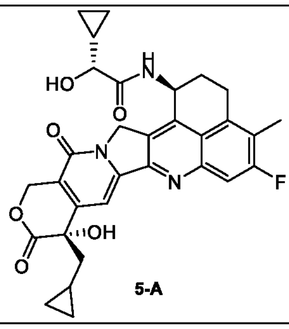 5-A | 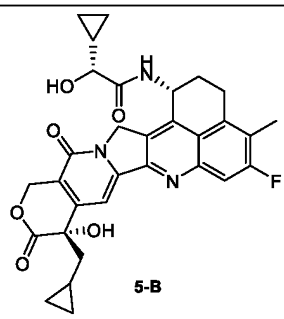 5-B |
| 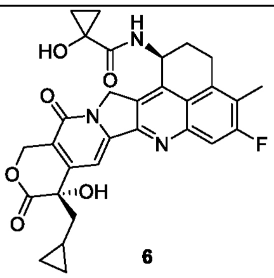 6 | | |

Example 2-2: Preparation of Compound 2 and its isomers 2-A and 2-B

(*S*)-*N*-((1*S*,9*S*)-9-(Cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxypropanamide **2-A**

(*S*)-*N*-((1*R*,9*S*)-9-(Cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxypropanamide **2-B**

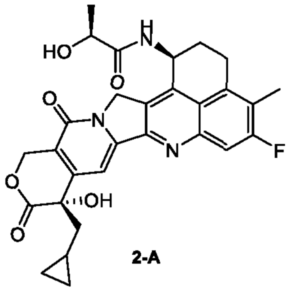
2-A

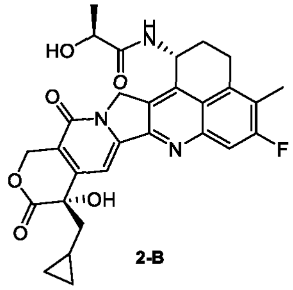
2-B

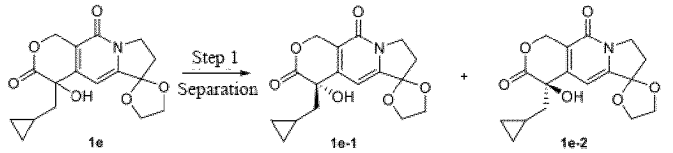

1e 1e-1 + 1e-2

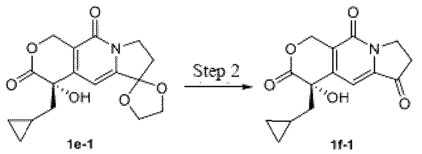

1e-1 1f-1

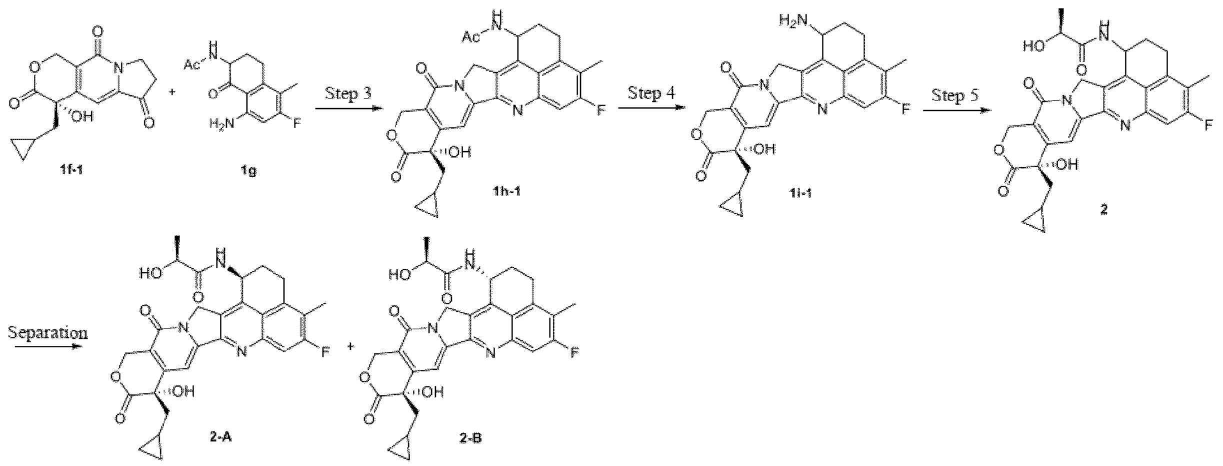

1f-1 + 1g 1h-1 1i-1 2

2-A + 2-B

Step 1

(*S*)-4-(Cyclopropylmethyl)-4-hydroxy-1,4,7,8-tetrahydro-3*H*,10*H*-spiro[3,4-*f*]indolizine-6,2'-[1,3]dioxolane]-3,10-dione **1e-1**

(*R*)-4-(Cyclopropylmethyl)-4-hydroxy-1,4,7,8-tetrahydro-3*H*,10*H*-spiro[3,4-*f*]indolizine-6,2'-[1,3]dioxolane]-3,10-dione **1e-2**

**1e** (1.30 g, 3.88 mmol) was separated by SFC (separation conditions: chromatographic column: DAICEL CHIRALPAK AS 250mmx50mm, 10 μm; mobile phase: A-carbon dioxide: B-methanol (0.1% $NH_3 \cdot H_2O$), isocratic elution: B: 20%, flow rate: 120 mL/min, instrument: Shimadzu LC-30ADsf), to obtain the title product **1e-1** (301 mg, yield: 22.1%) in the form of yellow solid, and the title product **1e-2** (285 mg, yield: 19.4%) in the form of yellow solid.

Single-configuration Compound **1e-1**

SFC analysis: retention time: 1.392 minutes. (Chromatographic column: Chiralpak AS-3 50x4.6mm I.D., 3μm, mobile phase: A-carbon dioxide, B-methanol (0.05% diethylamine), gradient elution: B%: 5%-40%, flow rate: 3 mL/min, instrument: Shimadzu LC-30ADsf).

MS m/z (ESI): 334.0 [M+1].

Single-configuration Compound **1e-2**

SFC analysis: retention time: 1.762 minutes. (Chromatographic column: Chiralpak AS-3 50x4.6mm I.D., 3μm, mobile phase: A-carbon dioxide, B-methanol (0.05% diethylamine), gradient elution: B%: 5%-40%, flow rate: 3 mL/min, instrument: Shimadzu LC-30ADsf).
MS m/z (ESI): 333.9 [M+1].

Step 2

(*S*)-4-(Cyclopropylmethyl)-4-hydroxy-7,8-dihydro-1*H*-pyrano[3,4-*f*]indolizine- 3,6,10(4*H*)-trione **1f-1**

**1e-1** (301 mg, 857 μmol) was dissolved in a mixed solution of 2 mL of trifluoroacetic acid and 0.5 mL of water, and stirred at 25 °C for 4 hours. The reaction solution was concentrated by vacuum distillation, and the resultant residue without purification was used to obtain the crude title product **1f-1** (209 mg) in the form of yellow solid. The product was used directly in the next step without purification.
MS m/z (ESI): 290.1 [M+1].

Step 3

*N*-((9*S*)-9-(Cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)acetamide **1h-1**

**1f-1** (50.0 mg, 159 μmol), **1g** (48.0 mg, 192 μmol) and pyridinium 4-toluenesulfonate (8.0 mg, 31.8 μmol) were dissolved in 2 mL of toluene, and stirred at 120 °C for 16 hours under nitrogen protection. The reaction solution was filtered through Celite, the filtrate was added with 20 mL of water and extracted with dichloromethane (15 mLx3). The organic phase was dried over anhydrous sodium sulfate and filtered, and the filtrate was concentrated under reduced pressure. The residue was purified by preparative thin layer chromatography using the eluent system A to obtain the title product **1h-1** (190 mg, yield: 56%) in the form of yellow solid.
MS m/z (ESI): 504.1 [M+1].

Step 4

(9*S*)-1-Amino-9-(cyclopropylmethyl)-5-fluoro-9-hydroxy-4-methyl-1,2,3,9,12,15-hexahydro-10*H*,13*H*-benzo[*de*]pyrano[3',4':6,7]indolizino[1,2-*b*]quinoline-10,13-dione **1i-1**

**1h-1** (60.0 mg, 111 μmol) was dissolved in 4.5 mL of 1,2-dimethoxyethane, added with 1.5 mL of methanesulfonic acid and 1.5 mL of water, and stirred at 85 °C for 16 hours. The reaction solution was added dropwise to 10 mL of water under stirring and extracted with dichloromethane (10 mLx5), the organic phase was washed with 25 mL of 0.05 M hydrochloric acid aqueous solution, the aqueous phase was filtered, all the extracted aqueous phases were combined, the combined aqueous phase was adjusted to pH 7-8 with saturated potassium bicarbonate solution at 0 °C and extracted with a mixed solvent (dichloromethane/methanol: 20/3, 15 mLx5). The organic phase was dried over anhydrous sodium sulfate and filtered, and the filtrate was concentrated under reduced pressure to obtain the crude title product **1i-1** (22 mg) in the form of brown solid. The product was used directly in the next step without purification.
MS m/z (ESI): 462.1 [M+1].

Step 5

(*S*)-*N*-((1*S*,9*S*)-9-(Cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxypropanamide **2-A**

(*S*)-*N*-((1*R*,9*S*)-9-(Cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxypropanamide **2-B**

The mesylate salt of **1i-1** (22.0 mg, 47.7 μmol) and (2S)-2-hydroxypropionic acid (6.4 mg, 71.5 μmol) were dissolved in 1.5 mL of *N,N*-dimethylformamide, then added with 1-hydroxybenzotriazole (9.7 mg, 71.5 μmol), *N,N*-diisopropylethylamine (18.5 mg, 143 μmol) and 1-(3-dimethylaminopropyl)-3-ethylcarbodiimide hydrochloride (13.7 mg, 71.5 μmol) in sequence, and stirred at 25 °C for 4 hours. The reaction solution was added with toluene (4 mL x2), concentrated under reduced pressure at 25 °C. The residue was diluted to 1.5 mL with *N,N*-dimethylformamide. The

diluted solution was purified by pre-HPLC (chromatographic column: Phenomenex luna C18 150x25mmx10μm; mobile phase: A-water (0.225% formic acid), B-acetonitrile; gradient elution: B%: 26%-56%) to obtain a mixture (10 mg), which was further purified by supercritical fluid chromatography to obtain the two single-configuration title products **2-A** and **2-B:**

Single-configuration Compound **2-A** of Compound **2** (5.55 mg, yield: 20%, off-white solid)

SFC analysis: retention time: 1.381 minutes, purity: 92%. (Chromatographic column: Chiralcel OJ-3 50x4.6mm I.D., 3μm, mobile phase: A-carbon dioxide, B-methanol (0.05% diethylamine), gradient elution: B%: 5%-40%, flow rate: 3 mL/min, instrument: Shimadzu LC-30ADsf).
MS m/z (ESI): 534.1 [M+1].
$^1$H NMR (400 MHz, $CD_3OD$) δ 7.61 (s, 1H), 7.53 - 7.49 (m, 1H), 5.65 - 5.54 (m, 2H), 5.36 - 5.29 (m, 1H), 5.25 - 5.18 (m, 1H), 4.81 - 4.77 (m, 1H), 4.41 - 4.33 (m, 1H), 3.28 - 3.23 (m, 1H), 3.15 - 3.05 (m, 1H), 2.36 - 2.33 (m, 3H), 2.33 - 2.27 (m, 1H), 2.17 - 2.10 (m, 1H), 1.95 - 1.87 (m, 1H), 1.85 - 1.77 (m, 1H), 1.47 - 1.44 (m, 3H), 0.91 - 0.87 (m, 1H), 0.51 - 0.35 (m, 2H), 0.15 - 0.06 (m, 1H), 0.04 - -0.05 (m, 1H).

Single-configuration Compound **2-B** of Compound **2** (4.06 mg, yield: 16%, yellow solid)

SFC analysis: retention time: 1.299 minutes, purity: 97%. (Chromatographic column: Chiralcel OJ-3 50x4.6mm I.D., 3μm, mobile phase: A-carbon dioxide, B-methanol (0.05% diethylamine), gradient elution: B%: 5%-40%, flow rate: 3 mL/min, instrument: Shimadzu LC-30ADsf).
MS m/z (ESI): 534.1 [M+1].
$^1$H NMR (400 MHz, $CD_3OD$) δ 7.61 (s, 1H), 7.57 - 7.51 (m, 1H), 5.65 - 5.52 (m, 2H), 5.42 - 5.30 (m, 2H), 5.12 - 5.02 (m, 1H), 4.38 - 4.30 (m, 1H), 3.29 - 3.24 (m, 1H), 3.20 - 3.07 (m, 1H), 2.43 - 2.37 (m, 3H), 2.35 - 2.26 (m, 2H), 1.97 - 1.80 (m, 2H), 1.64 - 1.58 (m, 3H), 0.92 - 0.83 (m, 1H), 0.51 - 0.36 (m, 2H), 0.16 - 0.07 (m, 1H), 0.06 - -0.02 (m, 1H).

Example 2-3: Preparation of Compound **3** and its isomers **3-A** and **3-B**

(*R*)-*N*-((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxypropanamide **3-A**

(*R*)-*N*-((1*R*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxypropanamide **3-B**

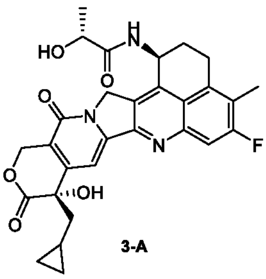
3-A

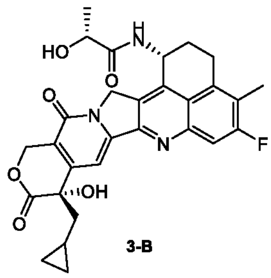
3-B

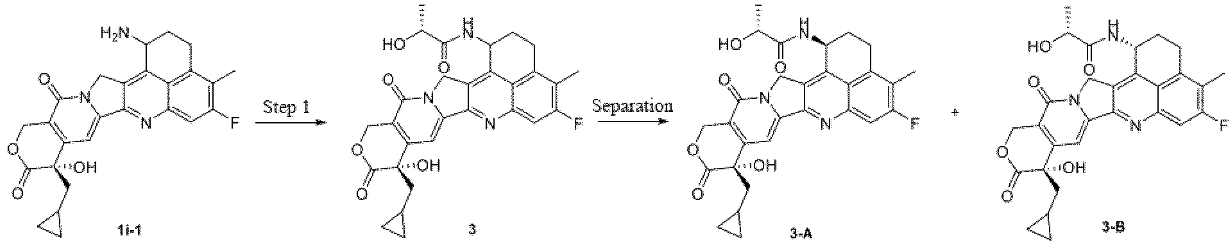

1i-1 3 3-A + 3-B

Step 1

(*R*)-*N*-((1*S*,9*S*)-9-(Cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)- 2-hydroxypropanamide **3-A**

(*R*)-*N*-((1*R*,9*S*)-9-(Cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)- 2-hydroxypropanamide **3-B**

**1i-1** (40.0 mg, 80.8 μmol) and (2*R*)-2-hydroxypropionic acid (10.9 mg, 121 μmol) were dissolved in 1.5 mL of *N*,*N*-dimethylformamide, then added with 1-hydroxybenzotriazole (16.4 mg, 121 μmol), *N*,*N*-diisopropylethylamine (31.3 mg, 242 μmol) and 1-(3-dimethylaminopropyl)-3-ethylcarbodiimide hydrochloride (23.2 mg, 121 μmol) in sequence, and stirred at 25 °C for 4 hours. The reaction solution was added with toluene (4 mLx2), and concentrated under reduced pressure at 25 °C. The residue was diluted to 1.5 mL with *N,N*-dimethylformamide. The diluted solution was purified by pre-HPLC (chromatographic column: Xtimate C18 150x40mmx10μm; mobile phase: A-water (0.225% formic acid), B-acetonitrile; gradient elution: B%: 23%-53%), and the obtained mixture was purified by supercritical fluid chromatography to obtain the two single-configuration title products **3-A** and **3-B:**

Single-configuration Compound **3-A** of Compound **3** (6.11 mg, yield: 14%, off-white solid)

SFC analysis: retention time: 1.413 minutes, purity: 97%. (Chromatographic column: Chiralcel OJ-3 50x4.6mm I.D., 3μm, mobile phase: A-carbon dioxide, B-methanol (0.05% diethylamine), gradient elution: B%: 5%-40%, flow rate: 3 mL/min, instrument: Shimadzu LC-30ADsf).

MS m/z (ESI): 534.3 [M+1].

$^1$H NMR (400 MHz, $CD_3OD$) δ 7.61 (s, 1H), 7.54 - 7.48 (m, 1H), 5.70 - 5.62 (m, 1H), 5.59 - 5.51 (m, 1H), 5.38 - 5.30 (m, 1H), 5.29 - 5.21 (m, 1H), 4.83 - 4.78 (m, 1H), 4.33 - 4.25 (m, 1H), 3.30 - 3.23 (m, 1H), 3.20 - 3.09 (m, 1H), 2.40 - 2.36 (m, 3H), 2.35 - 2.31 (m, 1H), 2.25 - 2.14 (m, 1H), 1.95 - 1.88 (m, 1H), 1.86 - 1.79 (m, 1H), 1.60 - 1.54 (m, 3H), 0.95 - 0.82 (m, 1H), 0.52 - 0.35 (m, 2H), 0.15 - 0.07 (m, 1H), 0.04 - -0.04 (m, 1H).

Single-configuration Compound **3-B** of Compound **3** (2.78 mg, yield: 6%, off-white solid)

SFC analysis: retention time: 2.013 minutes, purity: 97%. (Chromatographic column: Chiralcel OD-3 50x4.6mm I.D., 3μm, mobile phase: A-carbon dioxide, B-methanol (0.05% diethylamine), gradient elution: B%: 5%-40%, flow rate: 3 mL/min, instrument: Shimadzu LC-30ADsf).

MS m/z (ESI): 534.1 [M+1].

$^1$HNMR (400 MHz, DMSO-*d6*) δ 8.42 - 8.36 (m, 1H), 7.81 - 7.75 (m, 1H), 7.37 (s, 1H), 6.57 (s, 1H), 5.58 - 5.49 (m, 2H), 5.41 (s, 2H), 5.26 - 5.12 (m, 2H), 4.17 - 4.09 (m, 1H), 3.19 - 3.14 (m, 1H), 2.42 - 2.36 (m, 3H), 2.23 - 2.10 (m, 2H), 1.90 - 1.81 (m, 1H), 1.79 - 1.71 (m, 1H), 1.32 - 1.27 (m, 3H), 0.85 - 0.77 (m, 1H), 0.39 - 0.27 (m, 2H), 0.10 - 0.02 (m, 1H), -0.03 - -0.11 (m, 1H).

Example 2-4: Preparation of Compound **4** and its isomers **4-A** and **4-B**

(*S*)-2-Cyclopropyl-*N*-((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **4-A**

(*S*)-2-cyclopropyl-*N*-((1*R*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **4-B**

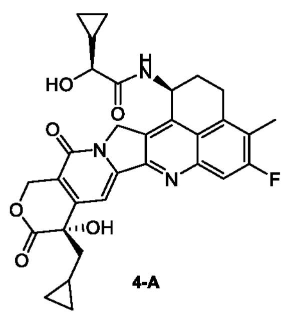

**4-A**

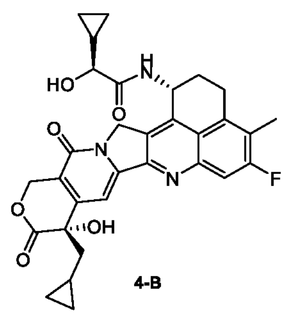

**4-B**

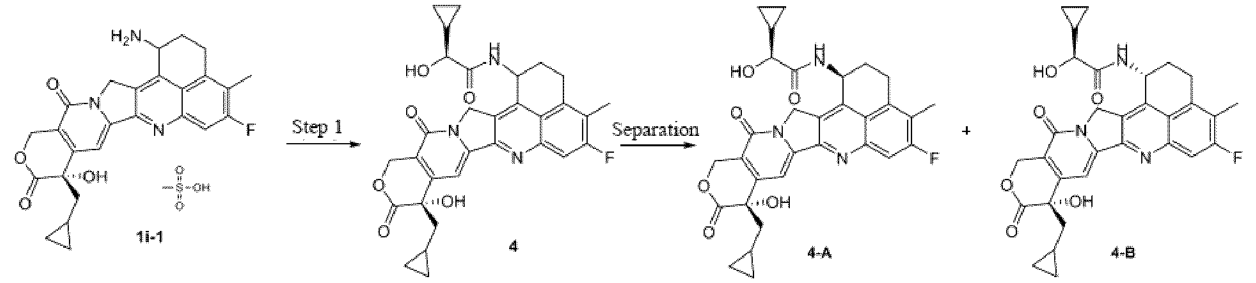


Step 1

The mesylate of **1i-1** (37.0 mg, 78.5 μmol) and (2S)-2-cyclopropyl-2-hydroxyacetic acid (9.59 mg, 78.5 μmol) were dissolved in 1 mL of *N*,*N*-dimethylformamide, then added with 1-hydroxybenzotriazole (15.9 mg, 118 μmol), *N*,*N*-diisopropylethylamine (30.4 mg, 236 μmol) and 1-(3-dimethylaminopropyl)-3-ethylcarbodiimide hydrochloride (22.6 mg, 118 μmol) in sequence, and stirred at 25 °C for 12 hours. The reaction solution was added with ethyl acetate (10 mL) and washed with water (5 mLx2), the organic phase was dried over anhydrous sodium sulfate and filtered, and the filtrate was concentrated under reduced pressure. The residue was purified by pre-HPLC (chromatographic column: Phenomenex luna C18 150x25mmx10μm; mobile phase: A-water (0.225% formic acid), B-acetonitrile; gradient elution: B%: 31%-61%) to obtain a mixture in the form of yellow solid (15 mg, yield 31.2%), which was then separated by preparative SFC (chromatographic column: REGIS (s,s) WHELK-O1250x30mm, 5μm; mobile phase: A-carbon dioxide (isopropanol), B-acetonitrile; isocratic elution: B: 65%), to obtain the two single-configuration title products **4-A** and **4-B:**

Single-configuration Compound **4-A** of Compound **4** (7.0 mg, yield: 50.2%, yellow solid)

SFC analysis: retention time: 0.761 minutes, purity: 98%. (Chromatographic column: (S,S)WHELK-O1 50x4.6mm I.D., 3.5μm, mobile phase: A-carbon dioxide, B-isopropanol and acetonitrile (0.05% diethylamine), isocratic elution: B: 65%, flow rate: 3 mL/min, instrument: Shimadzu LC-30ADsf).
MS m/z (ESI): 560.5 [M+1].
$^1$HNMR (400 MHz, $CD_3OD$) δ 7.67 (s, 1H), 7.64 - 7.60 (m, 1H), 5.68 - 5.62 (m, 1H), 5.61 - 5.55 (m, 1H), 5.41 - 5.37 (m, 1H), 5.36 - 5.33 (m, 1H), 5.06 - 5.00 (m, 1H), 3.87 - 3.84 (m, 1H), 3.21 - 3.11 (m, 2H), 2.43 - 2.40 (m, 3H), 2.37 - 2.31 (m, 1H), 2.25 - 2.18 (m, 1H), 1.96 - 1.89 (m, 1H), 1.87 - 1.80 (m, 1H), 1.32 - 1.28 (m, 1H), 0.92 - 0.87 (m, 1H), 0.61 - 0.55 (m, 2H), 0.53 - 0.45 (m, 3H), 0.44 - 0.38 (m, 1H), 0.14 - 0.08 (m, 1H), 0.04 - -0.03 (m, 1H).

Single-configuration Compound **4-B** of Compound **4** (6.0 mg, yield: 41.9%, yellow solid)

SFC analysis: retention time: 1.486 minutes, purity: 96%. (Chromatographic column: (S,S)WHELK-O1 50x4.6mm I.D., 3.5μm, mobile phase: A-carbon dioxide, B-isopropanol and acetonitrile (0.05% diethylamine), isocratic elution: B: 65%, flow rate: 3 mL/min, instrument: Shimadzu LC-30ADsf).
MS m/z (ESI): 560.3 [M+1].
$^1$HNMR (400 MHz, $CD_3OD$) δ 7.64 (s, 1H), 7.61 - 7.57 (m, 1H), 5.67 - 5.62 (m, 1H), 5.59 - 5.52 (m, 1H), 5.46 - 5.39 (m, 1H), 5.38 - 5.32 (m, 1H), 5.21 - 5.14 (m, 1H), 3.72 - 3.68 (m, 1H), 3.22 - 3.11 (m, 2H), 2.42 - 2.39 (m, 3H), 2.34 - 2.28 (m, 2H), 1.96 - 1.89 (m, 1H), 1.87 - 1.81 (m, 1H), 1.42 - 1.35 (m, 1H), 0.91 - 0.85 (m, 1H), 0.69 - 0.58 (m, 3H), 0.56 - 0.51 (m, 1H), 0.48 - 0.40 (m, 2H), 0.15 - 0.07 (m, 1H), 0.06 - -0.02 (m, 1H).

Example 2-5: Preparation of Compound **5** and its isomers **5-A** and **5-B**

(*R*)-2-Cyclopropyl-*N*-((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **5-A**

(*R*)-2-Cyclopropyl-*N*-((1*R*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **5-B**

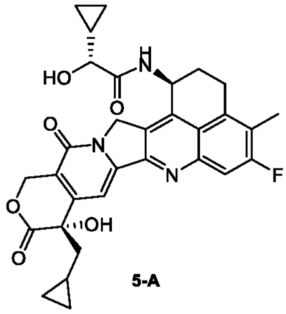

5-A

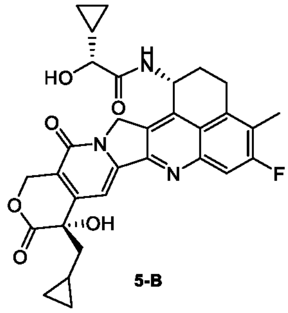

5-B

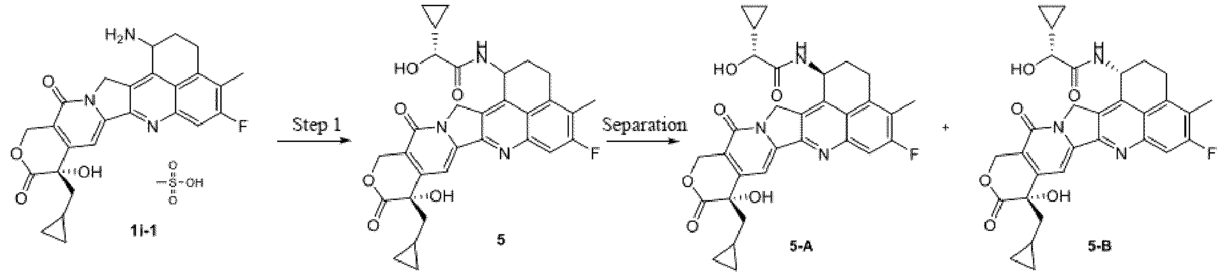


1i-1 5 5-A 5-B

Step 1

The mesylate of **1i-1** (25.0 mg, 42.3 μmol) and (2R)-2-cyclopropyl-2-hydroxyacetic acid (5.89 mg, 50.7 μmol) were dissolved in 1.5 mL of *N,N*-dimethylformamide, then added with 1-hydroxybenzotriazole (8.57 mg, 63.4 μmol), *N,N*-diisopropylethylamine (16.4 mg, 127 μmol) and 1-(3-dimethylaminopropyl)-3-ethylcarbodiimide hydrochloride (12.2 mg, 63.4 μmol) in sequence, and stirred at 20 °C for 4 hours. The reaction solution was added with toluene (4 mL x2), and concentrated under reduced pressure at 25 °C. The residue was diluted to 1.5 mL with *N,N*-dimethylformamide. The diluted solution was purified by pre-HPLC (chromatographic column: Phenomenex luna C18 150x25mmx10μm; mobile phase: A-water (0.225% formic acid), B-acetonitrile; gradient elution: B%: 28%-58%) to obtain two single-configuration title products **5-A** and **5-B:**

Single-configuration Compound **5-A** of Compound **5** (4.95 mg, yield: 20%, off-white solid)

SFC analysis: retention time: 2.115 minutes, purity: 97%. (Chromatographic column: Chiralpak IC-3 50x4.6mm I.D., 3μm, mobile phase: A-carbon dioxide, B-methanol and acetonitrile (0.05% diethylamine), isocratic elution: B: 50%, flow rate: 3 mL/min, instrument: Shimadzu LC-30ADsf).

MS m/z (ESI): 560.1 [M+1].

$^1$H NMR (400 MHz, $CD_3OD$) δ 7.70 - 7.64 (m, 2H), 5.70 - 5.65 (m, 1H), 5.60 - 5.54 (m, 1H), 5.46 - 5.38 (m, 1H), 5.37 - 5.32 (m, 1H), 5.23 - 5.17 (m, 1H), 3.72 - 3.67 (m, 1H), 3.36 - 3.34 (m, 1H), 3.23 - 3.17 (m, 1H), 2.48 - 2.42 (m, 3H), 2.37 - 2.31 (m, 1H), 2.31 - 2.25 (m, 1H), 1.96 - 1.89 (m, 1H), 1.88 - 1.80 (m, 1H), 1.34 - 1.31 (m, 1H), 0.92 - 0.89 (m, 1H), 0.68 - 0.42 (m, 6H), 0.15 - 0.07 (m, 1H), 0.05 - -0.02 (m, 1H).

Single-configuration Compound **5-B** of Compound **5** (1.95 mg, yield: 8%, white solid)

SFC analysis: retention time: 1.179 minutes, purity: 97%. (Chromatographic column: Chiralpak IC-3 50x4.6mm I.D., 3μm, mobile phase: A-carbon dioxide, B-methanol and acetonitrile (0.05% diethylamine), isocratic elution: B: 50%, flow rate: 3 mL/min, instrument: Shimadzu LC-30ADsf).

MS m/z (ESI): 560.1 [M+1].

$^1$H NMR (400 MHz, $CD_3OD$) δ 7.69 (s, 1H), 7.69 - 7.62 (m, 1H), 5.69 - 5.63 (m, 1H), 5.61 - 5.55 (m, 1H), 5.49 - 5.42 (m, 1H), 5.40 - 5.34 (m, 1H), 5.24 - 5.16 (m, 1H), 3.91 - 3.84 (m, 1H), 3.38 - 3.36 (m, 1H), 3.24 - 3.16 (m, 1H), 2.44 (s, 3H), 2.40 - 2.23 (m, 2H), 1.97 - 1.80 (m, 2H), 1.29 - 1.21 (m, 1H), 0.95 - 0.85 (m, 1H), 0.64 - 0.36 (m, 6H), 0.17 - 0.07 (m, 1H), 0.07 - -0.02 (m, 1H).

Example 2-6: Preparation of Compound **6**

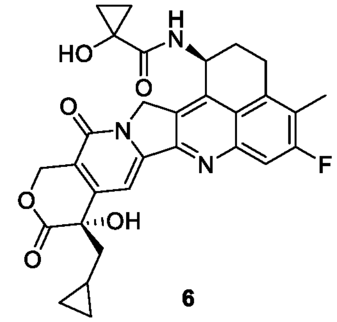

6

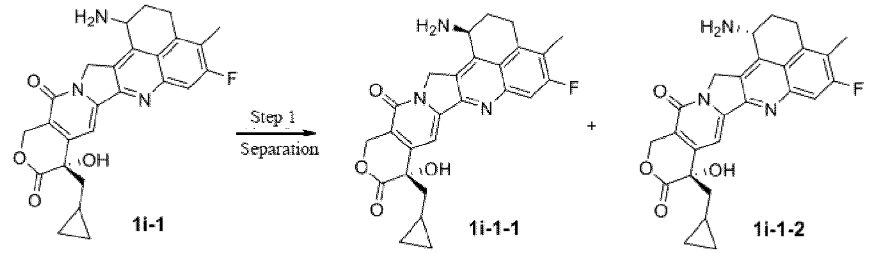


1i-1 1i-1-1 1i-1-2

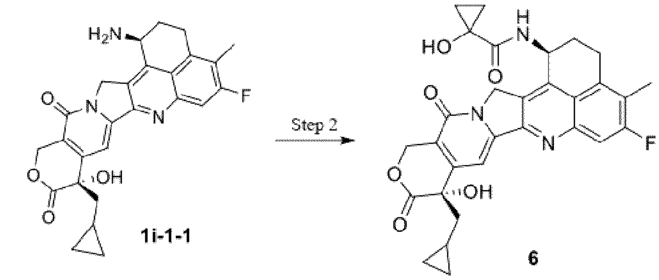


1i-1-1 6

Step 1

(1*S*,9*S*)-1-Amino-9-(cyclopropylmethyl)-5-fluoro-9-hydroxy-4-methyl-1,2,3,9,12,15-hexahydro-10*H*,13*H*-benzo[*de*]pyrano[3',4':6,7]indolizino[1,2-*b*]quinoline-10,13-dione **1i-1-1**

(1*R*,9*S*)-1-amino-9-(cyclopropylmethyl)-5-fluoro-9-hydroxy-4-methyl-1,2,3,9,12,15-hexahydro-10*H*,13*H*-benzo[*de*]pyrano[3',4':6,7]indolizino[1,2-*b*]quinoline-10,13-dione **1i-1-2**

**1i-1** (550 mg, 1.10 mmol) was separated by SFC (separation conditions: chromatographic column: DAICEL CHIRALPAK IC 250mmx50mm, 10 μm; mobile phase: A-n-hexane: B-ethanol, isocratic elution: B: 45%), to obtain the title product **1i-1-1** (260 mg, yield: 41%) in the form of yellow solid, and the title product **1i-1-2** (290 mg, yield: 46%) in the form of yellow solid.

Single-configuration Compound **1i-1-1**

SFC analysis: retention time: 3.039 minutes. (Chromatographic column: Chiralpak IC-3 50x4.6mm I.D., 3μm, mobile phase: A-n-hexane (0.05% isopropylamine), B-ethanol and acetonitrile (0.05% isopropylamine), isocratic elution: B: 45%, flow rate: 1 mL/min, instrument: Shimadzu LC-20AD).
MS m/z (ESI): 462.3 [M+1].

Single-configuration Compound **1i-1-2**

SFC analysis: retention time: 4.951 minutes. (Chromatographic column: Chiralpak IC-3 50x4.6mm I.D., 3μm, mobile phase: A-n-hexane (0.05% isopropylamine), B-ethanol and acetonitrile (0.05% isopropylamine), isocratic elution: B: 45%, flow rate: 1 mL/min, instrument: Shimadzu LC-20AD).
MS m/z (ESI): 462.3 [M+1].

Step 2

**1i-1-1** (13 mg, 25.35 μmol), 1-hydroxycyclopropanecarboxylic acid (4 mg, 38.03 μmol) and 2-(7-azobenzotriazole)-*N*,*N*,*N'*,*N'*-tetramethyluronium hexafluorophosphate (12 mg, 30.42 μmol) were dissolved in 1 mL of *N*,*N*-dimethylformamide, added with *N*,*N*-diisopropylethylamine (10 mg, 76.06 μmol), and stirred at 25 °C for 1 hour. The reaction solution was purified by pre-HPLC (chromatographic column: InfinityLab Poroshell 120 SB-C18 21.2x150 mm, 4 μm; mobile phase: A-water (0.1% formic acid), B-acetonitrile; gradient elution: B%: 20%-80%) to obtain the title product 6 (2.25 mg, yield: 16%) in the form of white solid.

MS m/z (ESI): 546.3 [M+1].

$^1$H NMR (400 MHz, DMSO-*d6*) δ 8.66 - 8.58 (m, 1H), 7.80 - 7.74 (m, 1H), 7.41 - 7.34 (m, 1H), 6.62 - 6.58 (m, 1H), 6.37 - 6.29 (m, 1H), 5.61 - 5.53 (m, 1H), 5.48 - 5.37 (m, 2H), 5.33 - 5.24 (m, 1H), 5.12 - 5.04 (m, 1H), 3.28 - 3.22 (m, 1H), 3.17 - 3.06 (m, 1H), 2.44 - 2.33 (m, 3H), 2.28 - 2.15 (m, 2H), 1.86 - 1.73 (m, 2H), 1.27 - 1.15 (m, 2H), 1.00 - 0.89 (m, 2H), 0.87 - 0.75 (m, 1H), 0.40 - 0.25 (m, 2H), 0.08 - 0.03 (m, 1H), -0.06 - -0.14 (m, 1H).

Examples 2-7 to 2-13

By referring to the method of Example 2-1, corresponding substrates were used to prepare the following compounds:

| | | |
|---|---|---|
| 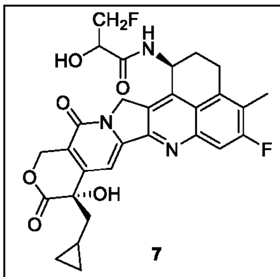 **7** | 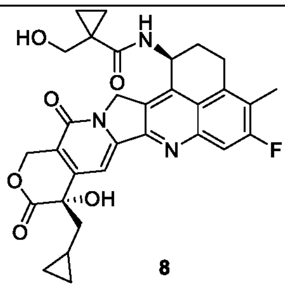 **8** | 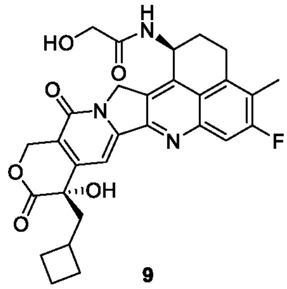 **9** |
| 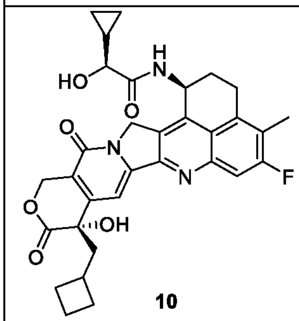 **10** | 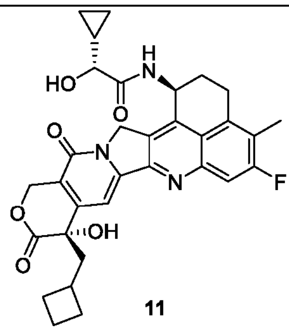 **11** | 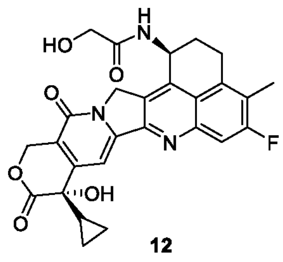 **12** |
| 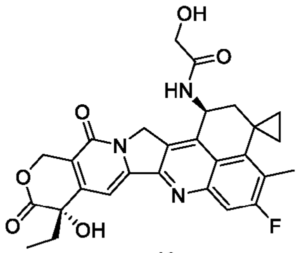 **13** | | |

Example 2-14: Preparation of Compound **14**

*N'*-Acetyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetohydrazide 14

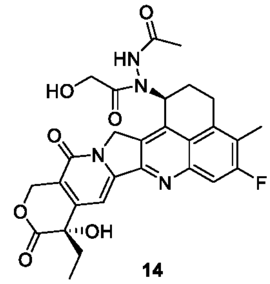

14

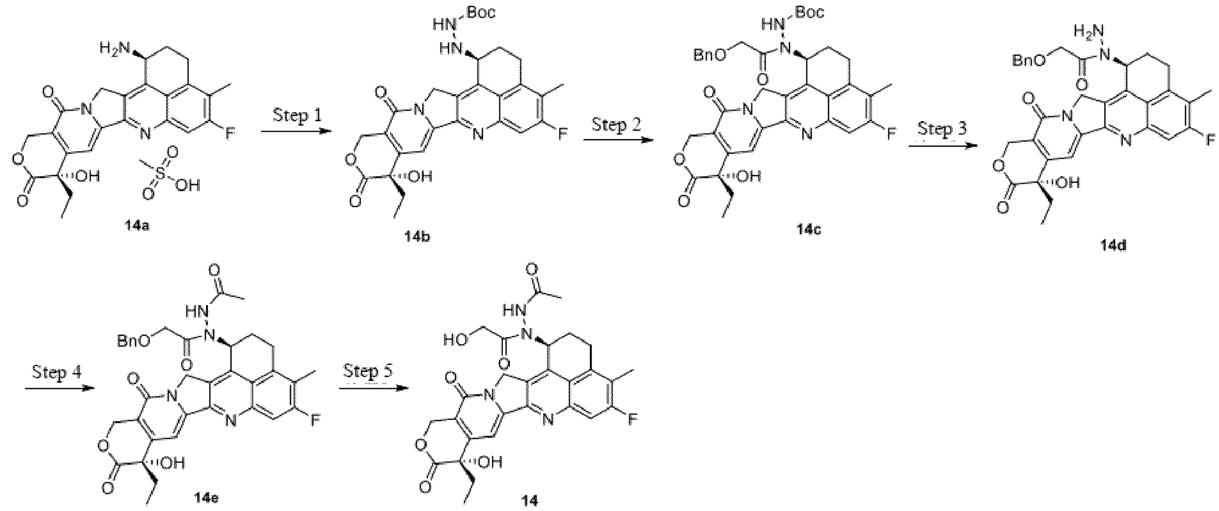


14a 14b 14c 14d 14e 14

Step 1

*tert*-Butyl 2-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)hydrazine-1-carboxylate **14b**

14a (100 mg, 0.19 mmol) and 2-(tert-butyl) 3,3-diethyl 1,2-oxaziridine-2,3,3-tricarboxylate (54.9 mg, 0.19 mmol, obtained by applying the method disclosed in the literature "Organic Letters, 2005, vol. 7, # 4, p. 713-716") were dissolved in 10 mL of dichloromethane, added with triethylamine (38.4 mg, 0.38 mmol), and stirred at 25 °C for 12 hours. 20 mL of water was added, and the diluted reaction solution was separated. The organic phase was washed with saturated sodium chloride solution (10 mL), the organic phase was dried over anhydrous sodium sulfate and filtered, and the filtrate was concentrated by vacuum distillation. The residue was purified by preparative thin layer chromatography using the eluent system A to obtain the title product **14b** (55 mg, yield: 53%) in the form of white solid.
MS m/z (ESI): 551.2 [M+1].

Step 2

*tert*-Butyl 2-(2-(benzyloxy)acetyl)-2-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)hydrazine-1-carboxylate **14c**

**14b** (55 mg, 0.1 mmol) was dissolved in 10 mL of tetrahydrofuran, and added with sodium bicarbonate (840 mg, 10 mmol). To the above solution, a tetrahydrofuran solution of 2-benzyloxyacetyl chloride (184 mg, 1.0 mmol, 5 mL) was added dropwise under nitrogen protection at 25 °C, and stirred at 25 °C for 1 hour. The reaction solution was poured into 10 mL of water, and extracted with ethyl acetate (10 mLx2). The organic phase was washed with saturated sodium chloride solution (10 mLx2), dried over anhydrous sodium sulfate, and filtered. The filtrate was concentrated by vacuum distillation. The residue was purified by preparative thin layer chromatography using the development system A to obtain the title product **14c** (58 mg, yield: 83%) in the form of white solid.
MS m/z (ESI): 699.3 [M+1].

Step 3

2-(Benzyloxy)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)acetohydrazine **14d**

**14c** (58 mg, 0.09 mmol) was dissolved in 10 mL of dichloromethane, added with 1 mL of trifluoroacetic acid, and stirred at 25 °C for 5 hours. The reaction solution was concentrated by vacuum distillation to obtain the crude title product 14d (70 mg) in the form of light brown solid. The product was used directly in the next step without purification.
MS m/z (ESI): 599.2 [M+1].

Step 4

*N'*-Acetyl-2-(benzyloxy)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl) acetohydrazine **14e**

**14d** (70 mg, 0.09 mmol) was dissolved in 10 mL of tetrahydrofuran, and added with sodium bicarbonate (756 mg, 9 mmol). To the above solution, a tetrahydrofuran solution of acetyl chloride (71 mg, 0.9 mmol, 3 mL) was added dropwise under nitrogen protection at 25 °C, and stirred at 25 °C for 1 hour. The reaction solution was poured into 10 mL of water, and extracted with ethyl acetate (10 mLx2). The organic phase was washed with saturated sodium chloride solution (10 mLx2), dried over anhydrous sodium sulfate, and filtered. The filtrate was concentrated by vacuum distillation. The residue was purified by preparative thin layer chromatography using the development system A to obtain the title product **14e** (45 mg, yield: 78%) as an off-white solid.
MS m/z (ESI): 641.7 [M+1].

Step 5

*N'*-Acetyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)- 2-hydroxyacetohydrazide **14**

**14e** (45 mg, 0.07 mmol) was dissolved in 10 mL of tetrahydrofuran, cooled to -10 °C under nitrogen protection in ice-salt bath, added with palladium dichloride (50 mg, 0.282 mmol), and subjected to replacement with hydrogen gas three times. The reaction solution was stirred at 0 °C for 2 hours. The reaction solution was filtered through Celite, and the filtrate was concentrated by vacuum distillation. The residue was purified by preparative HPLC (separation conditions: chromatographic column: Sunfire Prep C18 OBD 10μm, 19x250mm, mobile phase: A-acetonitrile/methanol = 1/1, B-water (0.2% formic acid), gradient elution, A%: 45%-95%, flow rate: 20 mL/min, instrument: GILSON GX-281) to obtain the title product **14** (4.5 mg, yield: 12%) in the form of off-white solid.
MS m/z (ESI): 551.1 [M+1].
$^1$H NMR (400 MHz, DMSO-*d6*) δ 10.22-9.65 (m, 1H), 7.79 (d, J = 10.8 Hz, 1H), 7.36-7.28 (m, 1H), 6.58-6.52 (m, 1H), 6.22-5.99 (m, 1H), 5.43 (s, 2H), 5.33 (s, 1H), 5.15-5.00 (m, 2H), 4.26-4.13 (m, 1H), 3.98-3.71 (m, 1H), 3.21-3.04 (m, 2H), 2.42-2.34 (m, 3H), 2.25-2.08 (m, 1H), 1.92-1.82 (m, 2H), 1.67-1.24 (m, 3H), 1.25-1.20 (m, 1H), 0.91-0.84 (m, 3H).

Examples 2-15 to 2-18, 2-20, 2-21 and 2-23

By referring to the method of Example 2-14, corresponding substrates were used to prepare the following compounds:

| | | |
|---|---|---|
| 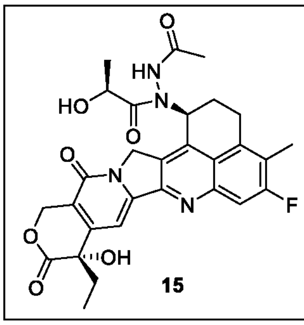 **15** | 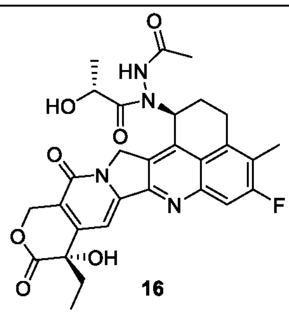 **16** | 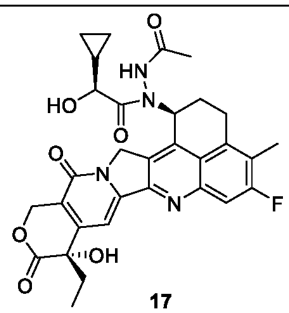 **17** |

| | | |
|---|---|---|
| 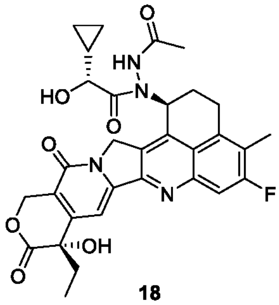 18 | 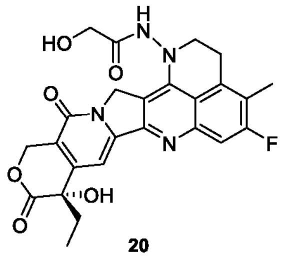 20 | 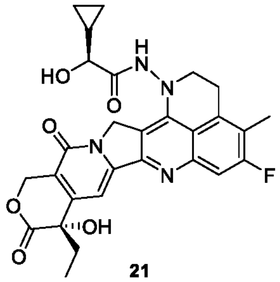 21 |
| 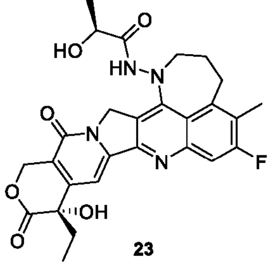 23 | | |

Example 2-19: Preparation of Compound **19**

*N*'-((1*S*,9*S*)-9-Ethyl-5-fluoro-9-hydroxy-4-methyl-10,13-dioxo-2,3,9,10,13,15-hexahydro-1*H*,12*H*-benzo[de]pyrano[3',4':6,7]indolizino[1,2-b]quinolin-1-yl)-N'-(2-hydroxyacetyl)cyclopropanecarbohydrazide **19**

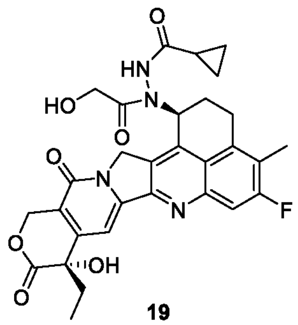

**19**

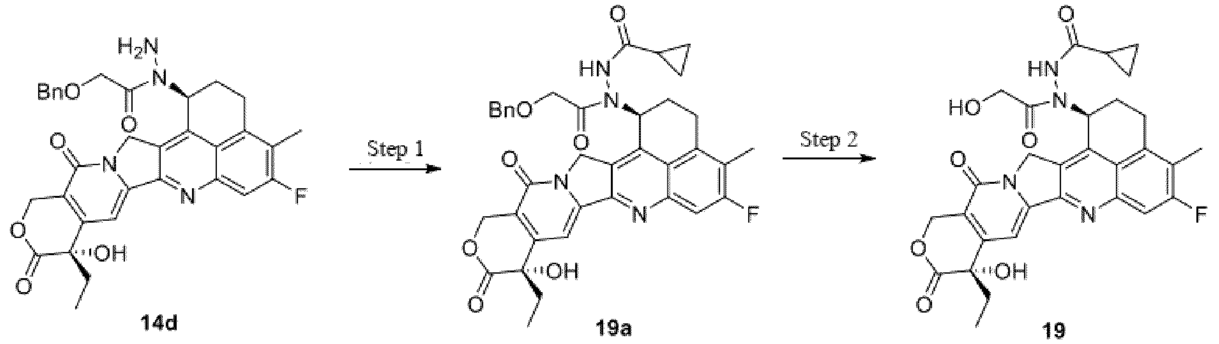


**14d** **19a** **19**

Step 1

*N*'-(2-(Benzyloxy)acetyl)-*N*'-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-b]quinolin-1-yl) cyclopropanecarbohydrazide **19a**

**14d** (50 mg, 0.08 mmol) was dissolved in 10 mL of tetrahydrofuran, added with sodium bicarbonate (672 mg, 8

mmol), and added dropwise with a tetrahydrofuran solution (5 mL) of cyclopropanecarbonyl chloride (83 mg, 0.8 mmol) under nitrogen protection. The reaction solution was stirred at 25 °C for 16 hours. The reaction solution was poured into water (10 mL) and extracted with ethyl acetate (10 mLx2). The organic phase was washed with saturated sodium chloride solution (10 mLx2), dried over anhydrous sodium sulfate, and filtered. The filtrate was concentrated by vacuum distillation. The residue was purified by pre-HPLC (separation conditions: chromatographic column: Sunfire Prep C18 OBD 10μm, 19x250mm, mobile phase: A-acetonitrile/methanol = 1/1, B-water (0.2% formic acid), gradient elution, A%: 45%-95%, flow rate: 20 mL/min, instrument: GILSON GX-281), and lyophilized to obtain the title product **19a** (19 mg, yield: 34%) in the form of off-white solid.

MS m/z (ESI): 667.2 [M+1].

Step 2

*N'*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano [3',4':6,7]indolizino[1,2-*b*]quinolin-1-yl)-*N'*-(2-hydroxyacetyl) cyclopropanecarbohydrazide **19**

**19a** (19 mg, 0.03 mmol) was dissolved in 5 mL of tetrahydrofuran under nitrogen protection, cooled to -10 °C in ice-salt bath, added with palladium dichloride (20 mg, 0.113 mmol), and subjected to replacement with hydrogen gas three times. The reaction solution was stirred at 0 °C for 15 hours. The reaction solution was filtered through Celite, and the filtrate was concentrated by vacuum distillation. The residue was purified by pre-HPLC (separation conditions: chromatographic column: Sunfire Prep C18 OBD 10μm, 19x250mm, mobile phase: A-acetonitrile/methanol = 1/1, B-water (0.2% formic acid), gradient elution, A%: 45%-95%, flow rate: 20 mL/min, instrument: GILSON GX-281) to obtain the title product **19** (4.2 mg, yield: 26%) in the form of white solid.

MS m/z (ESI): 577.2 [M+1].

Example 2-22: Preparation of Compound **22**

(*S*)-*N*-(10-Ethyl-6-fluoro-10-hydroxy-5-methyl-11,14-dioxo-3,4,10,11,14,16-hexahydro-13*H*-azepino[2,3,4-*de*]pyrano [3',4':6,7]indolizino[1,2-*b*]quinolin-1(2*H*)-yl)-2-hydroxyacetamide **22**

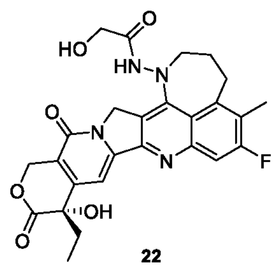

**22**

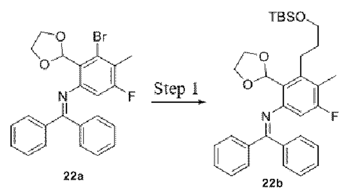


22a 22b

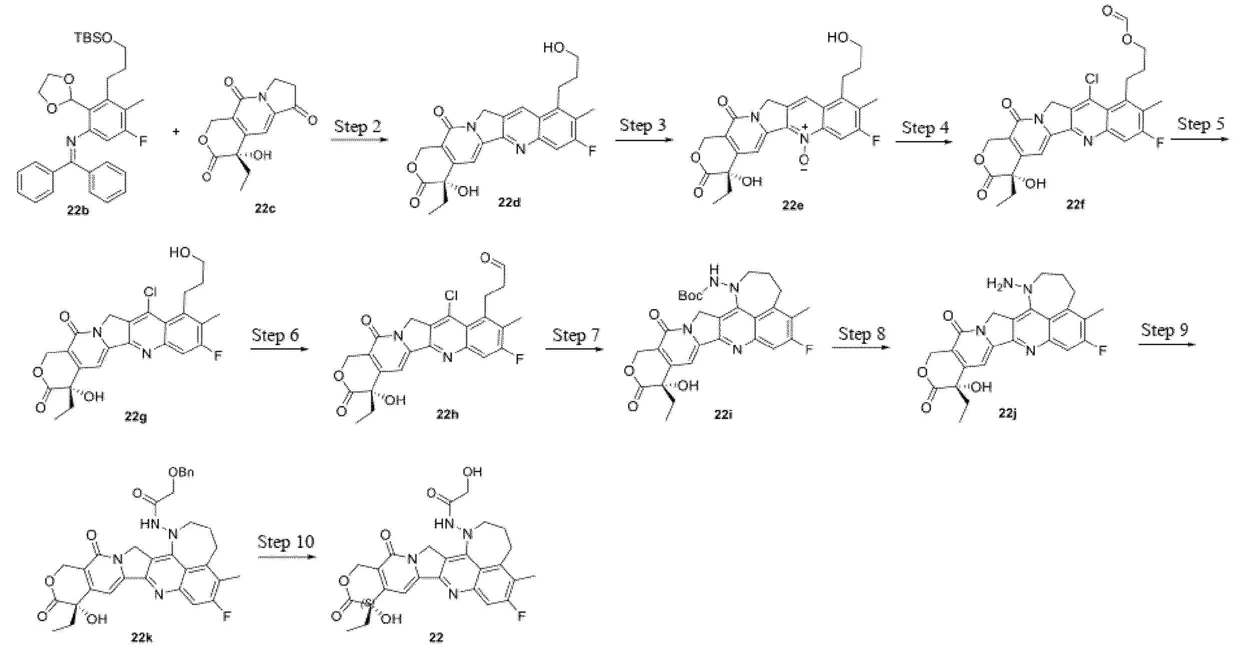


Step 1

*N*-(3-(3-((*tert*-butyldimethylsilyl)oxy)propyl)-2-(1,3-dioxolan-2-yl)-5-fluoro-4-methylpheny l)- 1,1-diphenylmethanimine **22b**

Allyloxy-*tert*-butyl-dimethylsilane (6.89 g, 36.00 mmol, 90% purity, obtained by applying the method disclosed in the literature "Organic Letters, 2017, vol. 19, #11, p. 2869-2872) was dissolved in 70 mL of toluene under protection of nitrogen gas, cooled to 10 °C in ice-water bath, and added with 9-borabicyclo[3.3.1]nonane (0.5 M, 86.41 mL, 43.20 mmol). The reaction solution was stirred at 80 °C for 20 minutes, and the completion of the reaction was monitored by TLC. The reaction solution was cooled to 10 °C in ice-water bath, and sodium hydroxide (2.88 g, 72.01 mmol, 24 mL) aqueous solution, tetrabutylammonium iodide (664.95 mg, 1.80 mmol), **22a** (5.02 g, 10.80 mmol) and 1,1'-bis(diphenylphosphino) ferrocene(II) palladium dichloride (526.89 mg, 720.09 μmol) were added to the reaction solution in sequence under nitrogen protection, and stirred at 80 °C for 15 hours. The completion of the reaction was monitored by LC-MS. 100 mL of water was added to dilute the reaction solution, and the reaction solution was extracted with dichloromethane (100 mLx3). The organic phase was washed with water (100 mLx2), dried over anhydrous sodium sulfate and filtered. The filtrate was concentrated by vacuum distillation. The obtained residue was purified by reverse phase high performance liquid chromatography to obtain the title product **22b** (2.60 g, yield: 12%, purity: 88.75%) in the form of yellow colloid.

MS m/z (ESI): 534.5 [M+1].

Step 2

(*S*)-4-Ethyl-8-fluoro-4-hydroxy-10-(3-hydroxypropyl)-9-methyl-1,12-dihydro-14*H*-pyrano[3',4':6,7]indolizino[1,2-*b*]quinoline-3,14(4*H*)-dione **22d**

**22b** (380 mg, 654.99 μmol) was dissolved in 5 mL of ethanol, added with **22c** (103.45 mg, 393.00 μmol, prepared by applying the method disclosed in patent application "WO2019238046 A1") and concentrated hydrochloric acid (12 M, 0.5 mL), and stirred at 80 °C for 2 hours, and the completion of the reaction was monitored by LC-MS. The reaction solution was concentrated by vacuum distillation to remove ethanol, 5 mL of water was added to dilute the reaction solution, the diluted reaction solution was extracted with dichloromethane (10 mLx3), the organic phase was dried over anhydrous sodium sulfate and filtered, and the filtrate was concentrated by vacuum distillation. The residue was purified by silica gel column chromatography using the eluent system A to obtain the title product **22d** (80 mg, yield: 27%) in the form of yellow solid.

MS m/z (ESI): 439.2 [M+1].

Step 3

(*S*)-4-Ethyl-8-fluoro-4-hydroxy-10-(3-hydroxypropyl)-9-methyl-3,14-dioxo-3,4,12,14-tetrahydro-1*H*-pyrano[3',4':6,7]indolizino[1,2-b]quinoline 6-oxide **22e**

**22d** (334 mg, 684 μmol) was dissolved in 30 mL of acetic acid, added with hydrogen peroxide (12.8 g, 113 mmol, the content is 30%), and stirred at 70 °C for 1.5 hours. The completion of the reaction was monitored by LC-MS. The reaction solution was cooled to 0 °C, added with 15 mL of sodium thiosulfate solution to quench the reaction, and extracted with ethyl acetate (20 mLx2), the organic phase was washed with saturated sodium chloride solution (15 mLx2), the filtrate was dried over anhydrous sodium sulfate and filtered, and the filtrate was concentrated under reduced pressure. The residue was purified by silica gel column chromatography using the eluent system A to obtain the title product **22e** (110 mg, yield: 31%) in the form of yellow solid.
MS m/z (ESI): 455.1 [M+1].

Step 4

(*S*)-3-(11-Chloro-4-ethyl-8-fluoro-4-hydroxy-9-methyl-3,14-dioxo-3,4,12,14-tetrahydro-1*H*-pyrano[3',4':6,7]indolizino[1,2-*b*]quinolin-10-yl)propyl formate **22f**

**22e** (110 mg, 227 μmol) was dissolved in 5 mL of *N,N*-dimethylformamide, added with oxalyl chloride (144 mg, 1.13 mmol), and stirred at 0 °C for 1 hour. The reaction solution was added with 8 mL of water, extracted with ethyl acetate (10 mLx3), the organic phase was washed with saturated sodium chloride solution (8 mLx3), the filtrate was dried over anhydrous sodium sulfate and filtered, and the filtrate was concentrated under reduced pressure to obtain the crude title product **22f** (100 mg) in the form of yellow solid. The product was used directly in the next reaction without purification.
MS m/z (ESI): 501.1 [M+1].

Step 5

(*S*)-11-Chloro-4-ethyl-8-fluoro-4-hydroxy-10-(3-hydroxypropyl)-9-methyl-1,12-dihydro-14*H*- pyrano[3',4':6,7]indolizino[1,2-*b*]quinoline-3,14(4*H*)-dione **22g**

**22f** (100 mg, 199 μmol) was dissolved in 4 mL of methanol, added with aqueous hydrochloric acid solution (2 M, 1.00 mL), and stirred at 25 °C for 1 hour. Methanol was removed by concentration under reduced pressure, 3 mL of water was added to the residue, and ethyl acetate (5 mLx3) was used for extraction. The organic phase was washed with saturated sodium chloride solution (8 mLx3), the filtrate was dried over anhydrous sodium sulfate and filtered, and the filtrate was concentrated under reduced pressure to obtain the crude title product **22g** (90 mg) in the form of yellow solid. The product was used directly in the next reaction without purification.
MS m/z (ESI): 473.1 [M+1].

Step 6

(*S*)-3-(11-Chloro-4-ethyl-8-fluoro-4-hydroxy-9-methyl-3,14-dioxo-3,4,12,14-tetrahydro-1*H*- pyrano[3',4':6,7]indolizino[1,2-*b*]quinolin-10-yl)propanal **22h**

**22g** (90.0 mg, 190 μmol) was dissolved in 4 mL of dichloromethane, added with Dess-Martin reagent (242 mg, 570 μmol), and stirred at 25 °C for 2 hours. 5 mL of water was added to the reaction solution, and dichloromethane (3 mLx2) was used for extraction. The filtrate was dried over anhydrous sodium sulfate and filtered, and the filtrate was concentrated under reduced pressure to obtain the crude title product **22h** (90 mg) in the form of yellow solid. The product was used directly in the next reaction without purification.
MS m/z (ESI): 471.1 [M+1].

Step 7

*tert*-Butyl (*S*)-(10-ethyl-6-fluoro-10-hydroxy-5-methyl-11,14-dioxo-3,4,10,11,14,16-hexahydro- 13*H*-azepino[2,3,4-*de*]pyrano[3',4':6,7]indolizino[1,2-*b*]quinolin-1(2*H*)-yl)carbamate **22i**

**22h** (90.0 mg, 191 μmol) and *tert*-butyl carbazate (27.8 mg, 210 μmol) were dissolved in 1 mL of methanol, added with sodium cyanoborohydride (60.1 mg, 955 μmol), and stirred at 25 °C for 4 hours. The methanol was removed by concentration under reduced pressure, 2 mL of water was added to the residue, and dichloromethane (3 mLx2) was used for extraction. The filtrate was dried over anhydrous sodium sulfate and filtered, and the filtrate was concentrated under

reduced pressure. 15 mg of the residue was taken and purified by pre-HPLC (separation conditions: chromatographic column: Phenomenex luna C18, 150x25mmx10μm, mobile phase: A-water (0.2% formic acid), B-acetonitrile, gradient elution, B%: 33%-63%) to obtain the title product **22i** (1.01 mg, yield: 13%) in the form of off-white solid.

MS m/z (ESI): 551.4 [M+1].

Step 8

(*S*)-1-Amino-10-ethyl-6-fluoro-10-hydroxy-5-methyl-2,3,4,10,13,16-hexahydro-14*H*-azepino[2,3,4-*de*]pyrano[3',4':6,7] indolizino[1,2-*b*]quinoline-11,14(1*H*)-dione **22j**

**22i** (20.0 mg, 22.3 μmol) was dissolved in 1 mL of dichloromethane, added with trifluoroacetic acid (2.05 g, 17.9 mmol), and stirred at 25 °C for 1 hour. The methanol was removed by concentration under reduced pressure, 2 mL of water was added to the residue, and the mixture was extracted with dichloromethane (3 mLx2). The filtrate was dried over anhydrous sodium sulfate and filtered, and the filtrate was concentrated under reduced pressure. 6 mg of the residue was purified by pre-HPLC (separation conditions: chromatographic column: Welch Xtimate C18, 150x25mmx5μm, mobile phase: A-water (0.2% formic acid), B-acetonitrile, gradient elution, B%: 8%-38%) to obtain the title product **22j** (1.06 mg, yield: 64%) in the form of yellow colloid.

MS m/z (ESI): 451.4 [M+1].

Step 9

(*S*)-2-(Benzyloxy)-*N*-(10-ethyl-6-fluoro-10-hydroxy-5-methyl-11,14-dioxo-3,4,10,11,14,16-hexahydro-13*H*-azepino [2,3,4-*de*]pyrano[3',4':6,7]indolizino[1,2-*b*]quinolin-1(2*H*)-yl)acetamide **22k**

**22j** (20 mg, 25.44 μmol) was dissolved in 0.5 mL of tetrahydrofuran and 0.5 mL of saturated sodium bicarbonate solution, added with 2-benzyloxyacetyl chloride (23.48 mg, 127.18 μmol) under nitrogen protection, and stirred at 25 °C for 10 hours under nitrogen protection. The reaction solution was added with 1 mL of water and extracted with ethyl acetate (2 mLx3). The organic phase was washed with saturated sodium chloride solution (2 mLx2), dried over anhydrous sodium sulfate, and filtered. The filtrate was concentrated under reduced pressure. The residue was purified by preparative thin layer chromatography using the development system A to obtain the title product **22k** (4 mg, yield: 22%) in the form of yellow solid.

MS m/z (ESI): 599.1 [M+1].

Step 10

(*S*)-*N*-(10-Ethyl-6-fluoro-10-hydroxy-5-methyl-11,14-dioxo-3,4,10,11,14,16-hexahydro-13*H*-azepino[2,3,4-*de*]pyrano [3',4':6,7]indolizino[1,2-*b*]quinolin-1(2*H*)-yl)-2-hydroxyacetamide **22**

**22k** (3.5 mg, 4.85 μmol) was dissolved in 1 mL of tetrahydrofuran, added with palladium(II) chloride (860.53 μg, 4.85 μmol), and subjected to replacement with hydrogen three times. The reaction solution was stirred at 0 °C under hydrogen gas (15 Psi) for 0.5 h. The reaction solution was filtered with Celite, the filtrate was concentrated by vacuum distillation, and the residue was purified by pre-HPLC (separation conditions: chromatographic column: Phenomenex luna C18, 150x25mmx10μm, mobile phase: A-water (0.2% formic acid), B-acetonitrile, gradient elution, B%: 13%-43%) to obtain the title product **22** (0.3 mg, yield: 9%) in the form of yellow solid.

MS m/z (ESI): 509.3 [M+1].

Example 2-24: Preparation of Compound **24**

(*S*)-*N*-((8-ethyl-4-fluoro-8-hydroxy-9,12-dioxo-2,3,8,9,12,14-hexahydro-1*H*,11*H*-cyclopenta[*f*]pyrano[3',4':6,7]indolizino [1,2-*b*]quinolin-15-yl)methyl)-2-hydroxyacetamide **24**

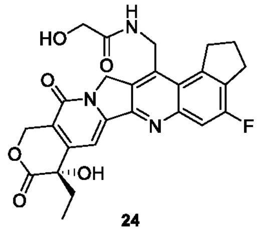

24

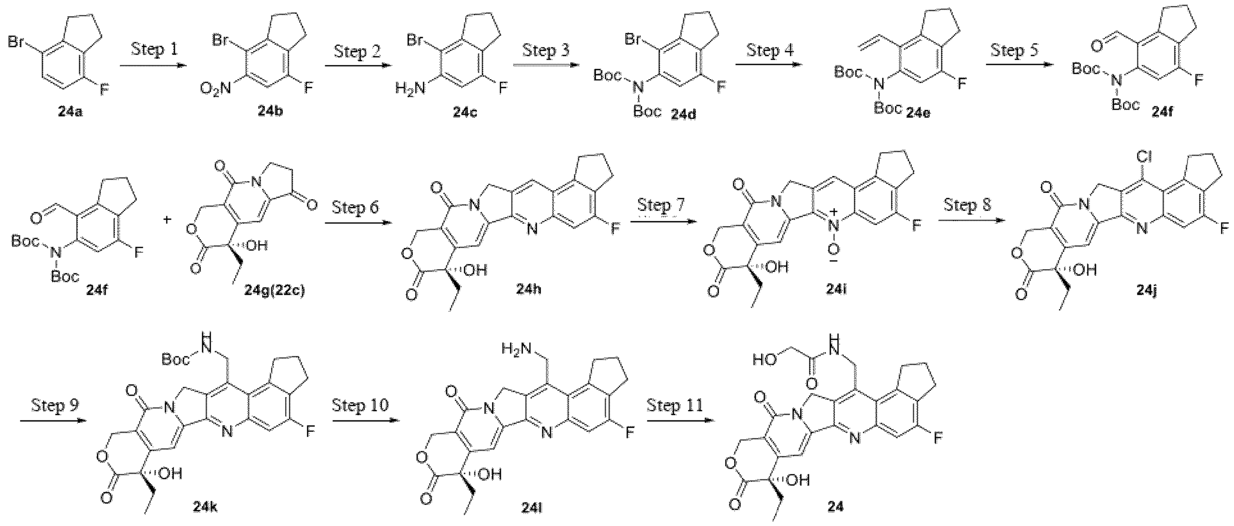


Step 1

4-Bromo-7-fluoro-5-nitro-2,3-dihydro-1*H*-indene **24b**

**24a** (17 g, 79.05 mmol, prepared by applying the method disclosed in Example 9 on page 31 of patent application "WO2021093820 A1") was dissolved in 200 mL of trifluoroacetic acid under nitrogen protection, and cooled to 0 °C in ice-water bath. To the reaction solution, nitric acid (20.45 g, 292.08 mmol, the content is 90%) was added, and stirred at 0 °C for 10 minutes; then the ice-water bath was removed, and the stirring was continued at 25 °C for 30 minutes. 100 mL of water was added, and the diluted reaction solution was extracted with ethyl acetate (500 mLx3). The organic phase was washed with water (500 mLx3), dried over anhydrous sodium sulfate, and filtered. The filtrate was concentrated by vacuum distillation. The residue was purified by silica gel column chromatography with the developing system B to obtain the title product **24b** (8.96 g, yield: 44%) in the form of colorless oil.

$^1H$ NMR (400 MHz, $CDCl_3$) $\delta$ 7.45 (d, J = 8.0 Hz, 1H), 3.05-3.16 (m, 4H), 2.18-2.27 (m, 2H).

Step 2

4-Bromo-7-fluoro-2,3-dihydro-1*H*-inden-5-amine **24c**

**24b** (8.96 g, 34.61 mmol) was dissolved in a mixed solution of 100 mL of ethanol and 20 mL of water, and added with iron powder (5.80 g, 103.82 mmol) and ammonium chloride (5.55 g, 103.82 mmol) in sequence under nitrogen protection, and the reaction solution was stirred at 80 °C for 1 hour. The reaction solution was filtered, and the filtrate was concentrated by vacuum distillation to obtain the crude title product **24c** (7.2 g) in the form of white solid. The product was used directly in the next step without purification.

MS m/z (ESI): 229.9 [M+1].

Step 3

*tert*-Butyl (4-bromo-7-fluoro-2,3-dihydro-1*H*-inden-5-yl)(*tert*-butoxycarbonyl)carbamate **24d**

**24c** (7.2 g, 31.29 mmol) was dissolved in 50 mL of tetrahydrofuran, added with di-*tert*-butyl dicarbonate (27.32 g, 125.18 mmol), 4-dimethylaminopyridine (38.23 mg, 312.94 μmol) and triethylamine (12.67 g, 125.18 mmol), and the

reaction solution was stirred at 25 °C for 16 hours. The reaction solution was concentrated by vacuum distillation, and the obtained residue was purified by silica gel column chromatography with the developing system B to obtain the title product **24d** (9.2 g, yield: 68%) in the form of white solid.

MS m/z (ESI): 317.9 [M+1-56-56].

Step 4

*tert*-Butyl (*tert*-butyloxycarbonyl)(7-fluoro-4-vinyl-2,3-dihydro-1*H*-inden-5-yl)carbamate **24e**

**24d** (1.5 g, 4.38 mmol) and pinacol vinylboronate (810.20 mg, 5.26 mmol) were dissolved in a mixed solution of 6 mL of 1,4-dioxane and 2 mL of water, and added with tripotassium phosphate (2.79 g, 13.15 mmol) and (2-dicyclohexylphosphino-2',4',6'-tri-isopropyl-1,1'-biphenyl)(2'-amino-1,1'-biphenyl-2-yl)palladium (II) methanesulfonate (371.07 mg, 438.38 μmol) in sequence under nitrogen protection, and the reaction solution was stirred at 95 °C for 10 hours. 20 mL of water was added, and the diluted reaction solution was extracted with ethyl acetate (10 mLx3). The organic phase was washed with saturated sodium chloride solution (5 mLx2), dried over anhydrous sodium sulfate, and filtered. The filtrate was concentrated by vacuum distillation. The obtained residue was purified by silica gel column chromatography using the developing system B to obtain the title product **24e** (350 mg, yield: 26%) in the form of colorless oil.

MS m/z (ESI): 222.1 [M+1-100-56].

Step 5

*tert*-Butyl (*tert*-butyloxycarbonyl)(7-fluoro-4-formyl-2,3-dihydro-1*H*-inden-5-yl)carbamate **24f**

**24e** (755 mg, 2.00 mmol) was dissolved in a mixed solution of 1.5 mL of dioxane and 1.5 mL of water, added with sodium periodate (1.29 g, 6.01 mmol) and potassium osmate dihydrate (73.8 mg, 200 μmol), and stirred at 25 °C for 5 hours. The reaction solution was filtered, and the filtrate was concentrated by vacuum distillation. The obtained residue was purified by silica gel column chromatography using the developing system B to obtain the title product **24f** (538 mg, yield: 71%) in the form of yellow solid.

MS m/z (ESI): 180.2 [M+1-100-100].

Step 6

(*S*)-8-Ethyl-4-fluoro-8-hydroxy-1,2,3,8,11,14-hexahydro-9*H*,12*H*-cyclopenta[*f*]pyrano[3',4':6,7]indolizino[1,2-*b*]quinoline-9,12-dione **24h**

**24f** (538 mg, 1.42 mmol) and **24g** (i.e., the above-mentioned Compound **22c**) (375 mg, 1.42 mmol, prepared by applying the method disclosed in Example 30 on page 28 of patent application "WO2019238046 A1") were dissolved in 10 mL of ethanol, added with concentrated hydrochloric acid (12 M, 1.58 mL), and stirred at 80 °C for 2 hours. The reaction solution was concentrated by vacuum distillation to remove ethanol, 10 mL of water was added, and the filter cake was obtained by filtration, and the crude title product **24h** (500 mg) was obtained as a yellow solid. The product was used directly in the next step without purification.

MS m/z (ESI): 407.2 [M+1].

Step 7

(*S*)-8-Ethyl-4-fluoro-8-hydroxy-9,12-dioxo-2,3,8,9,12,14-hexahydro-1*H*,11*H*-cyclopenta[*f*] pyrano[3',4':6,7]indolizino [1,2-*b*]quinoline 6-oxide **24i**

**24h** (500 mg, 1.21 mmol) was dissolved in 70 mL of acetic acid, added with hydrogen peroxide (16.9 g, 149 mmol, the content is 30%) under nitrogen protection, and stirred at 75 °C for 3 hours. LCMS showed that the starting material **24h** was in excess, hydrogen peroxide (16.5 g, 145 mmol, the content is 30%) was added again, the resulting mixture was stirred at 75 °C for 4 hours. The reaction solution was poured into 100 mL of water, and filtered to obtain a filter cake to obtain the crude title product **24i** (400 mg) as a yellow solid. The product was used directly in the next step without purification.

MS m/z (ESI): 423.2 [M+1].

Step 8

(*S*)-15-Chloro-8-ethyl-4-fluoro-8-hydroxy-1,2,3,8,11,14-hexahydro-9*H*,12*H*-cyclopenta[*f*] pyrano[3',4':6,7]indolizino [1,2-*b*]quinoline-9,12-dione **24j**

**24i** (100 mg, 142 μmol) was dissolved in 2 mL of *N*,*N*-dimethylformamide, cooled to 0 °C in ice-water bath under nitrogen protection, added with oxalyl chloride (45.0 mg, 355 μmol), and stirred at 25 °C for 1 hour. The reaction solution was poured into 10 mL of water and filtered to obtain a filter cake, and the crude title product **24j** (70 mg) was obtained as a brown solid. The product was used directly in the next step without purification.
MS m/z (ESI): 441.2 [M+1].

Step 9

*tert*-Butyl (*S*)-((8-ethyl-4-fluoro-8-hydroxy-9,12-dioxo-2,3,8,9,12,14-hexahydro-1H,11*H*-cyclopenta[f]pyrano[3',4':6,7] indolizino[1,2-b]quinolin-15-yl)methyl)carbamate **24k**

**24j** (20.0 mg, 26.3 μmol) and potassium (((*tert*-butoxycarbonyl)amino)methyl) trifluoroborate (14.0 mg, 59.2 μmol) were dissolved in a mixed solution of 1 mL of dioxane and 0.2 mL of water, added with 1,1-bis(diphenylphosphino) ferrocenepalladium dichloride (2.89 mg, 3.95 μmol) and potassium carbonate (25.1 mg, 118 μmol) under nitrogen protection, and stirred at 95 °C for 1 hour under nitrogen protection. The reaction mixture was poured into aqueous hydrochloric acid solution (1 M, 2 mL), and the filtrate was concentrated by vacuum distillation to obtain the crude title product **24k** (20 mg) as a brown oil. The product was used directly in the next step without purification.
MS m/z (ESI): 436.2 [M+1-100].

Step 10

(*S*)-15-(Aminomethyl)-8-ethyl-4-fluoro-8-hydroxy-1,2,3,8,11,14-hexahydro-9*H*,12*H*-cyclopenta[*f*]pyrano[3',4':6,7]indolizino[1,2-*b*]quinoline-9,12-dione **241**

**24k** (1.00 mg, 1.87 μmol) was dissolved in 0.6 mL of dichloromethane, added with a dioxane solution of hydrochloric acid (4 M, 0.2 mL), and stirred at 25 °C for 1 hour. 1 mL of water was added, and the diluted reaction solution was extracted with ethyl acetate (1 mLx3). The aqueous phase was filtered, and the filtrate was concentrated by vacuum distillation to obtain the crude title product **24l** (0.5 mg) as a yellow solid. The product was used directly in the next reaction without purification.
MS m/z (ESI): 436.1 [M+1].

Step 11

(*S*)-*N*-((8-Ethyl-4-fluoro-8-hydroxy-9,12-dioxo-2,3,8,9,12,14-hexahydro-1H,11*H*-cyclopenta[*f*]pyrano[3',4':6,7]indolizino[1,2-*b*]quinolin-15-yl)methyl)-2-hydroxyacetamide **24**

**24l** (13.3 mg, 22.9 μmol) and 2-hydroxyacetic acid (2.10 mg, 27.5 μmol) were dissolved in 1 mL of dichloromethane, added with *N,N*-diisopropylethylamine (8.90 mg, 68.9 μmol), 1-(3-dimethylaminopropyl)-3-ethylcarbodiimide hydrochloride (6.60 mg, 34.4 μmol) and 1-hydroxybenzotriazole (4.65 mg, 34.4 μmol), and stirred at 25 °C for 30 minutes. The reaction solution was concentrated by vacuum distillation, added with 2 mL of water, and extracted with ethyl acetate (1 mLx3), the organic phase was washed with saturated sodium chloride solution (1 mL), the organic phase was dried over anhydrous sodium sulfate and filtered, and the filtrate was concentrated by vacuum distillation. The residue was purified by preparative HPLC (separation conditions: chromatographic column: Welch ultimate C18 150x25mmx7μm, mobile phase: A-water (containing 0.225% formic acid), B-acetonitrile, gradient elution, flow rate: 25 mL/min, instrument: GILSON GX-281) to obtain the title product **24** (0.98 mg, yield: 8%) as a yellow solid.
MS m/z (ESI): 494.1 [M+1].
$^1$H NMR (400 MHz, $CD_3OD$) δ 7.71 - 7.66 (m, 1H), 7.63 (s, 1H), 5.61 - 5.56 (m, 1H), 5.50 - 5.48 (m, 1H), 5.43 - 5.35 (m, 3H), 5.18 - 5.09 (m, 3H), 3.73 - 3.65 (m, 2H), 3.20 - 3.12 (m, 2H), 2.39 - 2.29 (m, 2H), 2.02 - 1.93 (m, 2H), 1.05 - 0.94 (m, 3H).

Examples 2-25 to 2-33: Preparation of Compounds **25** to **33**

By referring to Example 2-24, corresponding substrates were used to prepare the following compounds **25** to **33:**

Example 2-25

(*S*)-*N*-(((*S*)-8-Ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)methyl)-2-hydroxypropanamide **25**

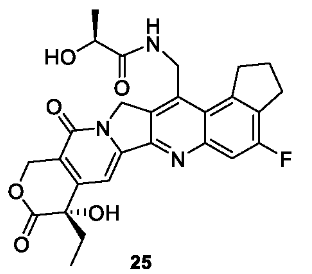

**25**

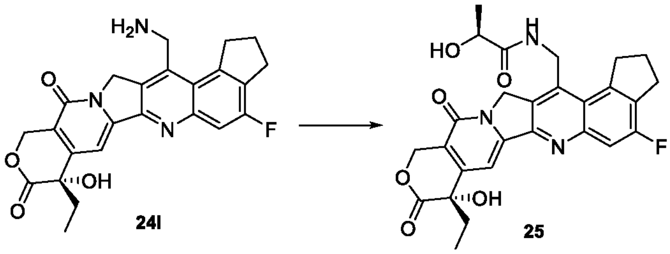

**24l** (22.3 mg, 45.1 μmol) and (2R)-2-hydroxypropionic acid (4.07 mg, 45.2 μmol) were dissolved in 0.5 mL of dichloromethane, added with *N,N*-diisopropylethylamine (17.5 mg, 135 μmol), 1-(3-dimethylaminopropyl)-3-ethylcarbodiimide hydrochloride (10.4 mg, 54.2 μmol) and 1-hydroxybenzotriazole (7.33 mg, 54.2 μmol), and stirred at 25 °C for 3 hours. The reaction solution was concentrated by vacuum distillation, and the residue was purified by pre-HPLC (separation conditions: chromatographic column: Phenomenex luna C18 250x50mmx15μm, mobile phase: A-water (0.225% formic acid), B-acetonitrile, gradient elution, B%: 23%-53%) to obtain the title product **25** (0.49 mg, yield: 2%) as a yellow gum. SFC analysis: retention time: 0.931 minutes, purity: 97%. (Chromatographic column: Chiralcel OD-3 50x4.6mm I.D., 3μm, mobile phase: A-carbon dioxide, B-isopropanol and acetonitrile (0.05% diethylamine), isocratic elution: B: 40%, flow rate: 3 mL/min, instrument: Shimadzu LC-30ADsf).

MS m/z (ESI): 508.1 [M+1].

$^1$H NMR (400 MHz, $CD_3OD$) δ 7.70 (d, J = 9.6 Hz, 1H), 7.63 (s, 1H), 5.63 - 5.56 (m, 1H), 5.43 - 5.36 (m, 3H), 5.15 - 5.10 (m, 2H), 4.25 - 4.18 (m, 1H), 3.71 - 3.66 (m, 2H), 3.19 - 3.13 (m, 2H), 2.39 - 2.31 (m, 2H), 2.01 - 1.93 (m, 2H), 1.43 - 1.38 (m, 3H), 1.04 - 0.98 (m, 3H).

Example 2-26

(*R*)-*N*-(((*S*)-8-Ethyl-4-fluoro-8-hydroxy-9,12-dioxo-2,3,8,9,12,14-hexahydro-1*H*,11*H*-cyclo penta[*f*]pyrano[3',4':6,7]indolizino[1,2-*b*]quinolin-15-yl)methyl)-2-hydroxypropanamide **26**

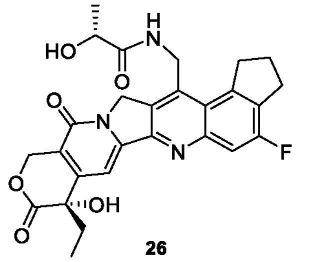

**26**

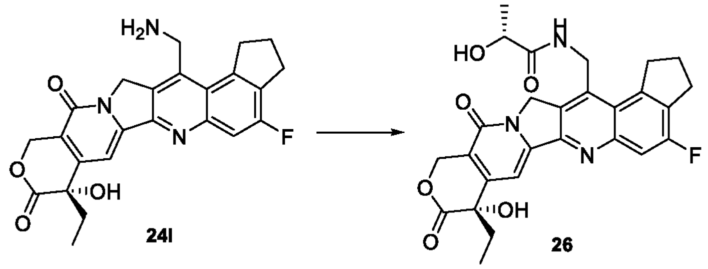

**24l** (22.3 mg, 45.2 μmol) and (2S)-2-hydroxypropionic acid (4.07 mg, 45.1 μmol) were dissolved in 0.5 mL of dichloromethane, added with *N,N*-diisopropylethylamine (17.5 mg, 135 μmol), 1-(3-dimethylaminopropyl)-3-ethylcarbodiimide hydrochloride (10.4 mg, 54.2 μmol) and 1-hydroxybenzotriazole (7.33 mg, 54.2 μmol), and stirred at 25 °C for 3 hours. The reaction solution was concentrated by vacuum distillation, and the residue was purified by pre-HPLC (separation conditions: chromatographic column: Welch ultimate C18 150x25mmx7μm, mobile phase: A-water (0.225% formic acid), B-acetonitrile, gradient elution, B%: 20%-50%) to obtain the title product **26** (1.09 mg, yield: 5%) as a yellow jelly. SFC analysis: retention time: 0.745 minutes, purity: 98%. (Chromatographic column: Chiralcel OD-3 50x4.6mm I.D., 3μm, mobile phase: A-carbon dioxide, B-isopropanol and acetonitrile (0.05% diethylamine), isocratic elution: B: 40%, flow rate: 3 mL/min, instrument: Shimadzu LC-30ADsf).

MS m/z (ESI): 508.1 [M+1].

$^1$H NMR (400 MHz, $CD_3OD$) δ 7.72 (d, J = 9.4 Hz, 1H), 7.66 (s, 1H), 5.63 - 5.59 (m, 1H), 5.45 - 5.39 (m, 3H), 5.17 - 5.14 (m, 2H), 4.26 - 4.22 (m, 1H), 3.74 - 3.68 (m, 2H), 3.21 - 3.16 (m, 2H), 2.40 - 2.35 (m, 2H), 2.01 - 1.95 (m, 2H), 1.44 - 1.39 (m, 3H), 1.06 - 1.00 (m, 3H).

Example 2-27

(*S*)-2-Cyclopropyl-*N*-(((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)methyl)-2-hydroxyacetamide **27**

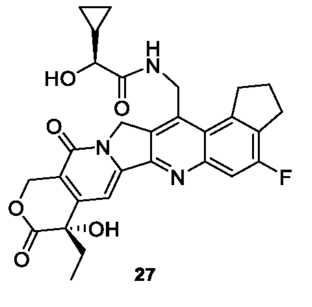

**27**

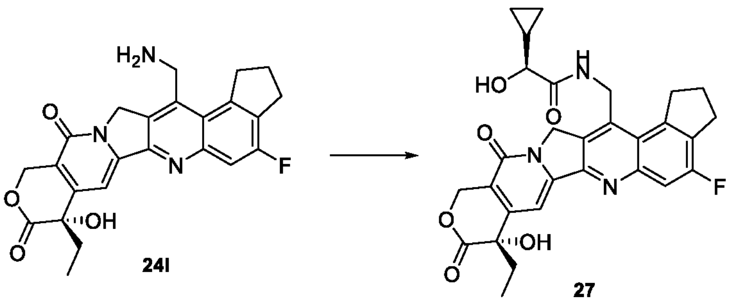

**24l** (25.0 mg, 50.5 μmol) and (2S)-2-cyclopropyl-2-hydroxyacetic acid (8.80 mg, 75.8 μmol) were dissolved in 1 mL of dichloromethane, added with *N,N*-diisopropylethylamine (26.1 mg, 202 μmol), 1-(3-dimethylaminopropyl)-3-ethylcarbodiimide hydrochloride (14.5 mg, 75.8 μmol) and 1-hydroxybenzotriazole (10.2 mg, 75.8 μmol), and stirred at 25 °C for 30 minutes. The reaction solution was concentrated by vacuum distillation, and the residue was purified by pre-HPLC (separation conditions: chromatographic column: Welch ultimate C18 150x25mmx7μm, mobile phase: A-water (0.225% formic acid), B-acetonitrile, gradient elution, flow rate: 25 mL/min) to obtain the title product 27 (2.15 mg, yield:

8%) as a brown oil. SFC analysis: retention time: 1.131 minutes, purity: 97%. (Chromatographic column: Chiralcel OD-3 50x4.6mm I.D., 3 μm; mobile phase: A-carbon dioxide: B-ethanol (0.05% diethylamine), isocratic elution: B: 40%, flow rate: 3 mL/min, instrument: Shimadzu LC-30ADsf).

MS m/z (ESI): 534.3 [M+1]. $^1$H NMR (400 MHz, $CD_3OD$) δ 7.72-7.66 (m, 1H), 7.63 (s, 1H), 5.61-5.55 (m, 1H), 5.45-5.34 (m, 3H), 5.12 (s, 2H), 3.75-3.64 (m, 3H), 3.19-3.10 (m, 2H), 2.40-2.30 (m, 2H), 2.02-1.91 (m, 2H), 1.21-1.14 (m, 1H), 1.06-0.93 (m, 3H), 0.57-0.40 (m, 4H).

Example 2-28

(*R*)-2-Cyclopropyl-*N*-(((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)methyl)-2-hydroxyacetamide **28**

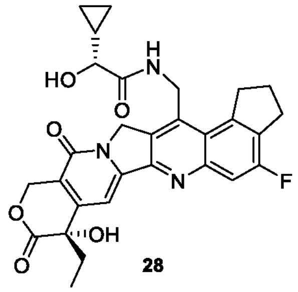

**28**

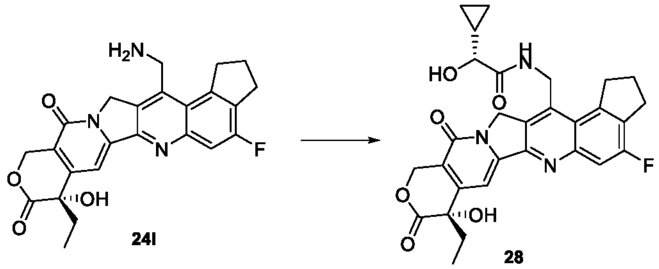

**24l** **28**

**24l** (25.0 mg, 50.5 μmol) and (2R)-2-cyclopropyl-2-hydroxyacetic acid (8.80 mg, 75.8 μmol) were dissolved in 1 mL of dichloromethane, added with *N*,*N*-diisopropylethylamine (26.1 mg, 202 μmol), 1-(3-dimethylaminopropyl)3-ethylcarbodiimide hydrochloride (14.5 mg, 75.8 μmol) and 1-hydroxybenzotriazole (10.2 mg, 75.8 μmol), and stirred at 25 °C for 30 minutes. The reaction mixture was concentrated by vacuum distillation, and the residue was purified by pre-HPLC (separation conditions: column: Phenomenex luna C18 150x25mmx10μm, mobile phase: A-water (0.225% formic acid), B-acetonitrile, gradient elution) to obtain the title product **28** (2.77 mg, yield: 10%) as a brown oil. SFC analysis: retention time: 0.898 minutes, purity: 99%. (Chromatographic column: Chiralcel OD-3 50x4.6mm I.D., 3 μm; mobile phase: A-carbon dioxide: B-ethanol (0.05% diethylamine), isocratic elution: B: 40%, flow rate: 3 mL/min, instrument: Shimadzu LC-30ADsf).

MS m/z (ESI): 534.4 [M+1].

$^1$H NMR (400 MHz, $CD_3OD$) δ 7.75-7.71 (m, 1H), 7.66 (s, 1H), 5.63-5.59 (m, 1H), 5.47-5.43 (m, 2H), 5.40-5.37 (m, 1H), 5.16-5.14 (m, 2H), 3.75-3.66 (m, 3H), 3.21-3.15 (m, 2H), 2.41-2.32 (m, 2H), 2.04-1.95 (m, 2H), 1.21-1.15 (m, 1H), 1.07-1.00 (m, 3H), 0.56-0.42 (m, 4H).

Examples 2-29 to 2-33

By referring to Example 2-28, corresponding substrates were used to prepare the following compounds:

| | | |
|---|---|---|
| 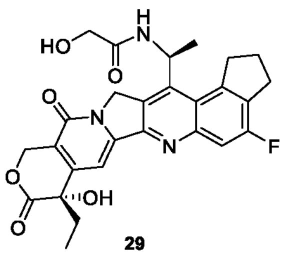 29 | 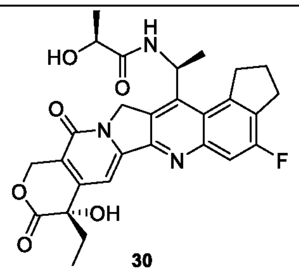 30 | |
| 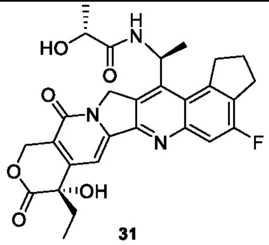 31 | 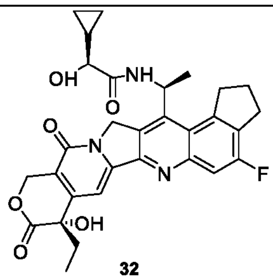 32 | 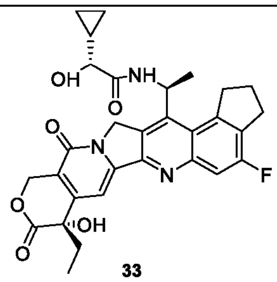 33 |

Example 2-34

(*S*)-*N*-((8-(cyclopropylmethyl)-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)methyl)-2-hydroxyacetamide 34

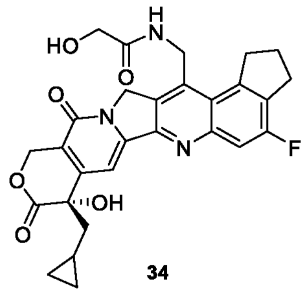

34

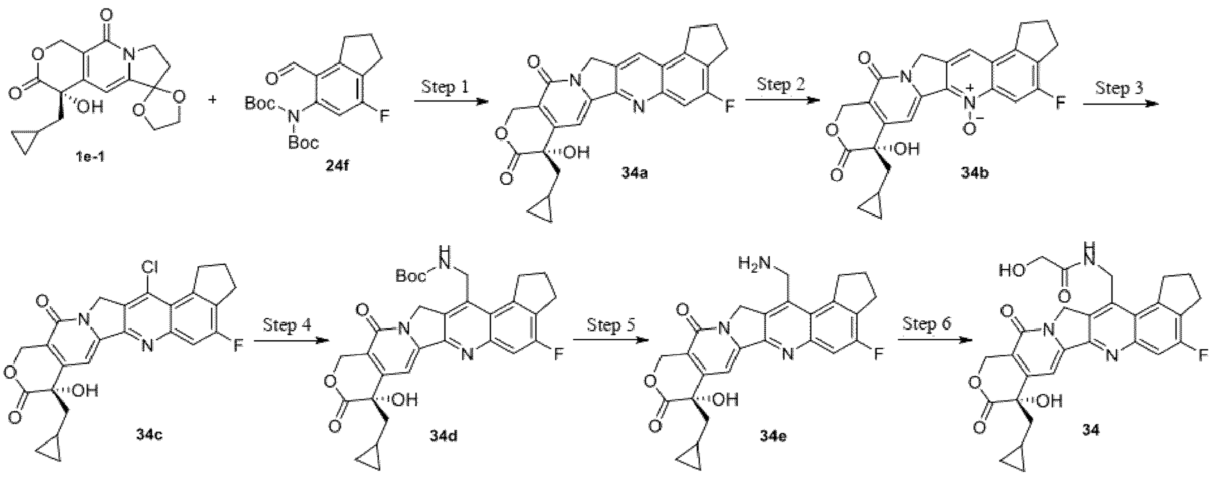


1e-1 24f 34a 34b 34c 34d 34e 34

Step 1

**24f** (500 mg, 1.32 mmol) and **1e-1** (578 mg, 1.73 mmol) were dissolved in 20 mL of ethanol, added with concentrated hydrochloric acid (12 M, 5.02 mL), and stirred at 80 °C for 16 hours. The reaction solution was concentrated by vacuum distillation, 10 mL of water was added to precipitate solid, and the solid was collected by filtration to obtain the

title product **34a** (496 mg) as a yellow solid. The product was used directly in the next step without purification.

MS m/z (ESI): 433.1 [M+1].

Step 2

**34a** (596 mg, 1.38 mmol) was dissolved in 36 mL of acetic acid, added with hydrogen peroxide (3.86 g, 34.0 mmol, the content is 30%), and stirred at 75 °C for 3 hours. 100 mL of water was added to the reaction solution to precipitate solid, and the solid was collected by filtration to obtain the title product **34b** (500 mg) as a yellow solid. The product was used directly in the next step without purification.

MS m/z (ESI): 449.0 [M+1].

Step 3

**34b** (490 mg, 915 μmol) was dissolved in 30 mL of *N*,*N*-dimethylformamide, cooled to 0 °C in ice-water bath under nitrogen protection, added with oxalyl chloride (465 mg, 3.66 mmol), and stirred at 0 °C for 1 hour. The reaction solution was poured into 100 mL of water to precipitate solid, and the solid was collected by filtration to obtain the title product **34c** (500 mg) as a yellow solid. The product was used directly in the next reaction without purification.

MS m/z (ESI): 467.1 [M+1].

Step 4

**34c** (200 mg, 428 μmol) was dissolved in a mixed solvent of 5 mL of dioxane and 0.5 mL of water, added with potassium *N*-*tert*-butylcarbonylmethyl trifluoroborate (203 mg, 856 μmol), 1,1'-bis(diphenylphosphino)ferrocene palladium(II) dichloride (31.3 mg, 42.8 μmol) and tripotassium phosphate (272 mg, 1.29 mmol), and stirred at 95 °C for 5 hours under nitrogen protection. The reaction solution was concentrated by vacuum distillation to obtain the title product **34d** (250 mg) as a brown solid. The product was used directly in the next reaction without purification.

MS m/z (ESI): 562.3 [M+1].

Step 5

**34d** (240 mg, 427 μmol) was dissolved in 5 mL of aqueous hydrochloric acid (6 M) and stirred at 40 °C for 10 hours. The reaction solution was added with 5 mL of water, and extracted with dichloromethane (10 mLx3). The aqueous phase was lyophilized to obtain the hydrochloride of the title product **34e** (200 mg) as a yellow solid. The product was used directly in the next step without purification.

MS m/z (ESI): 462.3 [M+1].

Step 6

**34e** hydrochloride (20.0 mg, 40.1 μmol) and glycolic acid (4.6 mg, 60.2 μmol) were dissolved in 2 mL of *N,N*-dimethylformamide, added with *N,N*-diisopropylethylamine (15.5 mg, 120 μmol), 1-(3-dimethylaminopropyl)-3-ethylcarbodiimide hydrochloride (11.5 mg, 60.2 μmol) and 1-hydroxybenzotriazole (8.1 mg, 60.2 μmol), and stirred at 25 °C for 2 hours. The reaction solution was added with 1 mL of water, and extracted with dichloromethane (3 mLx3). The organic phase was dried over anhydrous sodium sulfate and filtered, and the filtrate was concentrated by vacuum distillation. The residue was purified by pre-HPLC (separation conditions: chromatographic column: Phenomenex Luna C18 150x25mmx10μm, mobile phase: A-water (0.225% formic acid), B-acetonitrile, gradient elution, B%: 26%-56%) to obtain the title product **34** (2.51 mg, yield: 12%) as a yellow solid.

MS m/z (ESI): 520.3 [M+1]. $^1$H NMR (400 MHz, $CD_3OD$) δ 7.78 - 7.62 (m, 2H), 5.64 - 5.55 (m, 1H), 5.47 - 5.32 (m, 3H), 5.20 - 5.10 (m, 2H), 4.15 - 4.04 (m, 2H), 3.74 - 3.63 (m, 2H), 3.21 - 3.10 (m, 2H), 2.40 - 2.29 (m, 2H), 1.96 - 1.82 (m, 2H), 0.94 - 0.86 (m, 1H), 0.51 - 0.38 (m, 2H), 0.15 - -0.02 (m, 2H).

Example 2-35

(*S*)-*N*-((4-(Cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)methyl)-2-hydroxyacetamide **35**

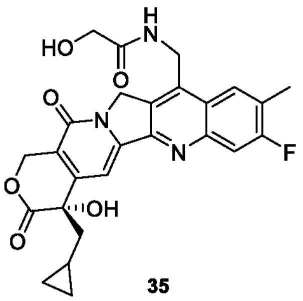

**35**

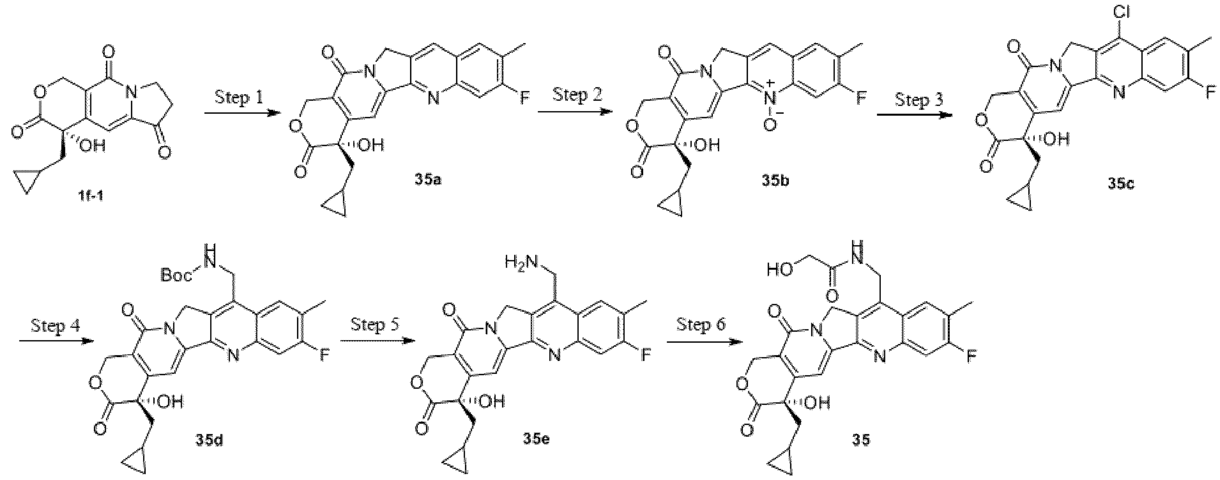


Step 1

2-Amino-4-fluoro-5-methylbenzaldehyde hydrochloride (138 mg, 686 μmol) and 1f-1 (200 mg, 686 μmol) were dissolved in 35 mL of toluene, added with pyridium 4-toluenesulfonate (103 mg, 412 μmol) and o-methylphenol (596 mg, 5.52 mmol), and stirred at 135 °C for 3 hours. The reaction solution was concentrated by vacuum distillation, and the residue was purified by silica gel column chromatography using the eluent system A to obtain the title product **35a** (213 mg, yield: 73%) as a yellow solid.
MS m/z (ESI): 407.2 [M+1].

Step 2

**35a** (250 mg, 479 μmol) was dissolved in 12 mL of acetic acid, added with hydrogen peroxide (4.41 g, 38.9 mmol, the content is 30%), and stirred at 70 °C for 1.5 hours. At 0 °C, the reaction solution was quenched with 20 mL of sodium thiosulfate, extracted with ethyl acetate (25 mLx2), the organic phase was washed with saturated sodium chloride solution (25 mLx2), the organic phase was dried over anhydrous sodium sulfate and filtered, and the filtrate was concentrated by vacuum distillation. The residue was purified by silica gel column chromatography using the eluent system A to obtain the title product **35b** (120 mg, yield: 55%) as a yellow solid.
MS m/z (ESI): 423.2 [M+1].

Step 3

**35b** (120 mg, 263 μmol) was dissolved in 2 mL of *N*,*N*-dimethylformamide, cooled to 0 °C in ice-water bath under nitrogen protection, added with oxalyl chloride (167 mg, 1.32 mmol), and stirred at 0 °C for 1 hour. The reaction solution was poured into 10 mL of water to precipitate solid, and the solid was collected by filtration, and dried to obtain the title product **35c** (115 mg) as a yellow solid. The product was used directly in the next step without purification.
MS m/z (ESI): 441.1 [M+1].

Step 4

**35c** (90.0 mg, 167 μmol) was dissolved in a mixed solvent of 3 mL of dioxane and 0.2 mL of water, added with

potassium *N-tert*-butylcarbonylmethyl trifluoroborate (198 mg, 838 μmol), 1,1-bis(diphenylphosphino)ferrocenepalladium dichloride (24.5 mg, 33.5 μmol) and cesium carbonate (163 mg, 503 μmol), and stirred at 105 °C for 5 hours under nitrogen protection. The reaction solution was added with 5 mL of water, and extracted with ethyl acetate (8 mLx3). The organic phase was dried over anhydrous sodium sulfate and filtered, and the filtrate was concentrated by vacuum distillation to obtain the title product **35d** (110 mg) as a yellow solid. The product was used directly in the next step without purification.

MS m/z (ESI): 536.1 [M+1].

Step 5

**35d** (110 mg, 205 μmol) was dissolved in 3 mL of aqueous hydrochloric acid solution (6 M), and stirred at 40 °C for 10 hours. The reaction solution was added with 5 mL of water and and extracted with dichloromethane (10 mLx3), the aqueous phase was lyophilized to obtain the hydrochloride salt of the title product **35e** (70 mg) as a yellow solid. The product was used directly in the next reaction without purification.

MS m/z (ESI): 436.0 [M+1].

Step 6

The hydrochloride of **35e** (30.0 mg, 68.8 μmol) and glycolic acid (5.76 mg, 75.7 μmol) were dissolved in 2 mL of *N*,*N*-dimethylformamide, added with *N,N*-diisopropylethylamine (26.7 mg, 206 μmol), 1-(3-dimethylaminopropyl)-3-ethylcarbodiimide hydrochloride (19.8 mg, 103 μmol) and 1-hydroxybenzotriazole (13.9 mg, 103 μmol), and stirred at 25 °C for 1.5 hours. The reaction solution was added with 2 mL of water, and extracted with dichloromethane (5 mLx3). The organic phase was dried over anhydrous sodium sulfate and filtered, and the filtrate was concentrated by vacuum distillation. The residue was purified by pre-HPLC (separation conditions: chromatographic column: Phenomenex Luna C18 150x25mmx10μm, mobile phase: A-water (0.225% formic acid), B-acetonitrile, gradient elution, B%: 19%-49%) to obtain the title product **35** (1.63 mg, yield: 5%) as a yellow solid.

MS m/z (ESI): 494.3 [M+1].

$^1$H NMR (400 MHz, $CD_3OD$) δ 8.34 - 8.29 (m, 1H), 7.83 - 7.77 (m, 1H), 7.73 - 7.70 (m, 1H), 5.64 - 5.58 (m, 1H), 5.56 - 5.49 (m, 2H), 5.44 - 5.37 (m, 1H), 5.07 - 5.01 (m, 2H), 4.06 - 3.99 (m, 2H), 2.62 - 2.51 (m, 3H), 1.95 - 1.83 (m, 2H), 0.93 - 0.88 (m, 1H), 0.50 - 0.38 (m, 2H), 0.15 - 0.08 (m, 1H), 0.07 - -0.01 (m, 1H).

Example 2-36

(*S*)-*N*-(((*S*)-4-(Cyclopropylmethyl)-8-fluoro-4-hydroxy-9-methyl-3,14-dioxo-3,4,12,14-tetra hydro-1*H*-pyrano[3',4':6,7]indolizino[1,2-*b*]quinolin-11-yl)methyl)-2-hydroxypropanamide **36**

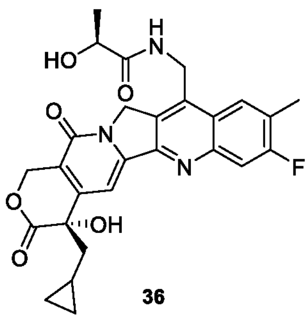

**36**

The title compound **36** was prepared through **35e** and 2-amino-4-fluoro-5-methylbenzaldehyde (prepared by applying the method disclosed in Example 1 on page 73 of patent application "WO2022140504 A1") with reference to the methods of steps 6 to 11 of Example 2-24.

Specifically, (2*S*)-2-hydroxypropionic acid (2.1 mg, 23.3 μmol) was dissolved in 0.5 mL of *N,N*-dimethylformamide, then added 1*H*-benzotriazol-1-yloxytripyrrolidino hexafluorophosphate (13.1 mg, 25.2 μmol), *N,N*-diisopropylethylamine (14.9 mg, 155 μmol) and **35e** (10.2 mg, 23.4 μmol) in sequence, and stirred at 25 °C for 0.5 h. The reaction solution was concentrated by vacuum distillation, and the obtained residue was purified by pre-HPLC (separation conditions: chromatographic column: InfinityLab Poroshell 120 SB-C18 21.2x150 mm, 4 um, mobile phase: A-water (0.1% formic acid), B--acetonitrile, gradient elution, B%: 10%-70%) to obtain the title product **36** (5.3 mg, yield: 44.56%) as a white solid.

MS m/z (ESI): 508.4 [M+1].

$^1$H NMR (400 MHz, DMSO-d6) δ 8.71 (t, J = 6.0 Hz, 1H), 8.42 (d, J = 8.3 Hz, 1H), 7.89 (d, J = 10.8 Hz, 1H), 7.38 (s, 1H), 6.58 (s, 1H), 5.61 (d, J = 4.9 Hz, 1H), 5.49 (d, J = 2.1 Hz, 2H), 5.43 (d, J = 2.6 Hz, 2H), 4.92 - 4.75 (m, 2H), 4.05 - 3.94 (m, 1H), 3.09 - 2.95 (m, 4H), 1.89 - 1.75 (m, 2H), 1.75 - 1.68 (m, 4H), 1.20 (d, J = 6.8 Hz, 3H), 0.86 - 0.76 (m, 1H), 0.41 - 0.25 (m, 2H), 0.11 - 0.02 (m, 1H), -0.04 - -0.13 (m, 1H).

Example 2-37

(*R*)-*N*-(((*S*)-4-(Cyclopropylmethyl)-8-fluoro-4-hydroxy-9-methyl-3,14-dioxo-3,4,12,14-tetr ahydro-1*H*-pyrano[3',4':6,7] indolizino[1,2-*b*]quinolin-11-yl)methyl)-2-hydroxypropanamide **37**

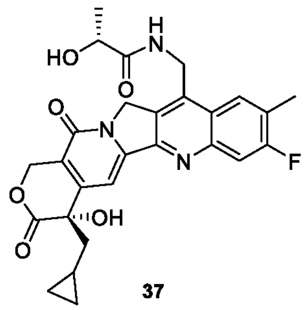

**37**

The title compound **37** was prepared through **35e** and 2-amino-4-fluoro-5-methylbenzaldehyde (prepared by applying the method disclosed in Example 1 on page 73 of patent application "WO2022140504 A1"), with reference to the methods of steps 6 to 11 of Example 2-24.

Specifically, (2*R*)-2-hydroxypropionic acid (2.1 mg, 23.3 μmol) was dissolved in 0.5 mL of *N,N*-dimethylformamide, then added with 1*H*-benzotriazol-1-yloxytripyrrolidino hexafluorophosphate (13.1 mg, 25.2 μmol), *N,N*-diisopropylethylamine (14.9 mg, 155 μmol) and **35e** (10.2 mg, 23.4 μmol) in sequence, and stirred at 25 °C for 0.5 hours. The reaction solution was concentrated by vacuum distillation, and the residue was purified by pre-HPLC (separation conditions: chromatographic column: InfinityLab Poroshell 120 SB-C18 21.2x150 mm, 4 um, mobile phase: A-water (0.1% formic acid), B-acetonitrile, gradient elution, B%: 10%-70%) to obtain the title product **37** (3.6 mg, yield: 30.27%) as a white solid.

MS m/z (ESI): 508.4 [M+1].

$^1$H NMR (400 MHz, DMSO-d6) δ 8.71 (s, 1H), 8.42 (d, J = 8.2 Hz, 1H), 7.89 (d, J = 10.8 Hz, 1H), 7.38 (s, 1H), 6.59 (s, 1H), 5.61 (d, J = 4.9 Hz, 1H), 5.49 (s, 2H), 5.43 (d, J = 3.0 Hz, 2H), 4.91 - 4.75 (m, 2H), 4.04 - 3.96 (m, 1H), 3.03 - 3.00 (m, 4H), 1.89 - 1.77 (m, 2H), 1.76 - 1.73 (m, 4H), 1.19 (d, J = 6.8 Hz, 3H), 0.85 - 0.76 (m, 1H), 0.40 - 0.25 (m, 2H), 0.12 - 0.03 (m, 1H), -0.04 - - 0.13 (m, 1H).

Example 2-38

(*S*)-2-Cyclopropyl-*N*-(((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)methyl)-2-hydroxyacetamide **38**

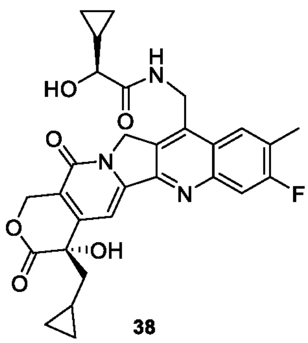

**38**

By using **35e** and 2-amino-4-fluoro-5-methylbenzaldehyde (prepared by applying the method disclosed in Example 1 on page 73 of patent application "WO2022140504 A1"), the title compound **38** was prepared by referring to the methods of steps 6 to 11 of Example 2-24.

Example 2-39

(*R*)-2-Cyclopropyl-*N*-(((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)methyl)-2-hydroxyacetamide **39**

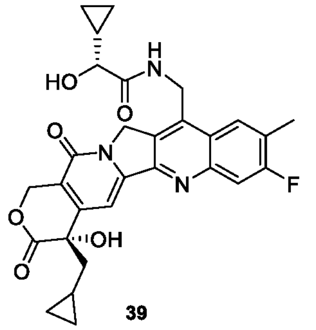

**39**

By using **35e** and 2-amino-4-fluoro-5-methylbenzaldehyde (prepared by applying the method disclosed in Example 1 on page 73 of patent application "WO2022140504 A1"), the title compound **39** was prepared by the same methods of steps 6 to 11 of Example 2-24.

III. Preparation Examples of conjugate intermediate linker-drugs

Example 3-1

2-(((*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15,18-hexaoxo-3-oxa-5,8,11,14,17-pentaazahenicosan-21-yl)oxy)-5-(2,5-dioxo-2,5-dihydro-1*H*-pyrrol-1-yl)-*N^1^*,*N^3^*-di(2,5,8,11,14,17,20,23-octaoxapentacosan-25-yl)isophthalamide **LD-1**

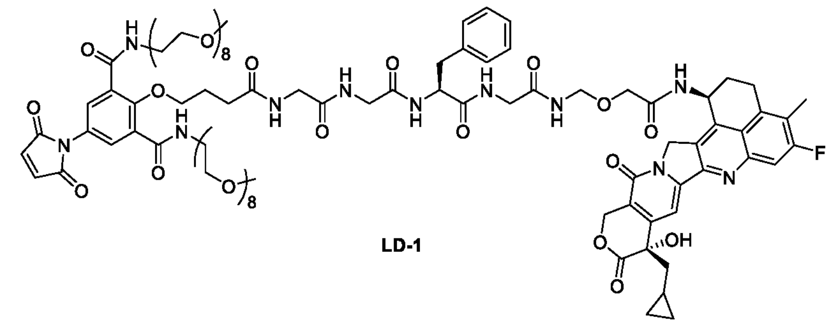

**LD-1**

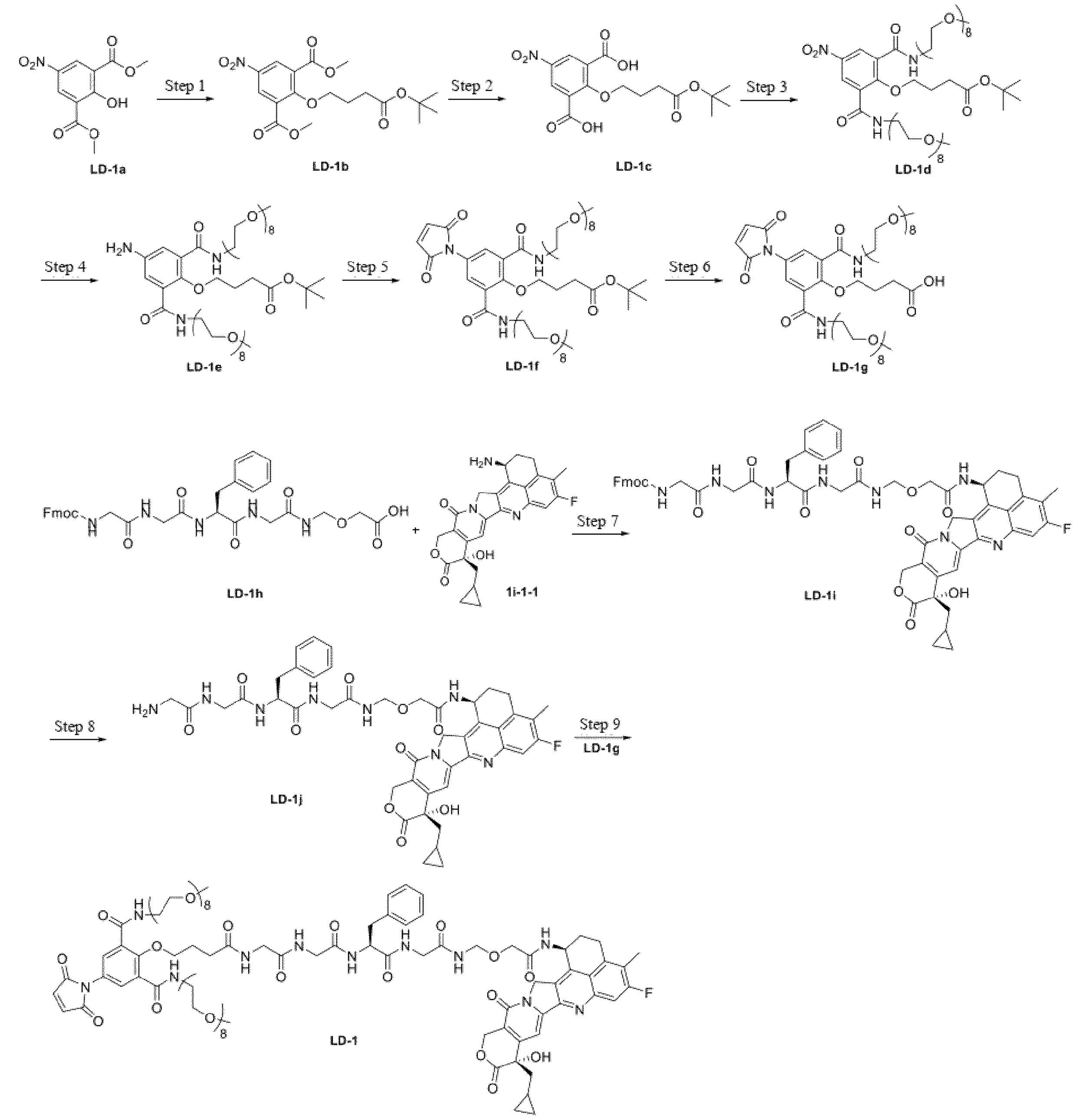


Step 1

Dimethyl 2-(4-(*tert*-Butyloxy)-4-oxobutyloxy)-5-nitroisophthalate **LD-1b**

**LD-1a** (5 g, 19.55 mmol, prepared by applying the method disclosed in "Journal of Physical Organic Chemistry, 2010, vol. 16, #10, p. 682-690"), *tert*-butyl 4-hydroxybutyrate (3.76 g, 23.47 mmol) and triphenylphosphine (7.69 g, 29.33 mmol) were dissolved in a mixed solvent of 40 mL of *N,N*-dimethylformamide and 120 mL of tetrahydrofuran, cooled to 0 °C under nitrogen protection in ice-water bath. The reaction solution was added with diisopropyl azodicarboxylate (5.93 g, 29.33 mmol) slowly and dropwise, and stirred at 25 °C for 20 hours. 150 mL of water was added, and the diluted reaction solution was extracted with ethyl acetate (200 mLx3). The organic phase was dried over anhydrous sodium sulfate and filtered, and the filtrate was concentrated by vacuum distillation. The residue was purified by reverse phase high performance liquid chromatography (separation conditions: chromatographic column: I.D.100mmxH300mm Welch Ultimate XB_C18 20-40μm, mobile phase: A-water, B-acetonitrile, gradient elution, flow rate: 200 mL/min, instrument: Agela Astra) to obtain the title product **LD-1b** (4.8 g, yield: 61%) as a yellow solid.
MS m/z (ESI): 420.2 [M+23].

Step 2

2-(4-(*tert*-Butoxy)-4-oxobutoxy)-5-nitroisophthalic acid **LD-1c**

**LD-1b** (1.5 g, 3.71 mmol) was dissolved in a mixed solvent of 15 mL of water and 15 mL of tetrahydrofuran, added with lithium hydroxide monohydrate (311.10 mg, 7.41 mmol), and stirred at 25 °C for 2 hours. 1N hydrochloric acid solution was added to the reaction solution to adjust the pH value to 2-3, and ethyl acetate was used for extraction (40 mLx3). The organic phase was dried over anhydrous sodium sulfate and filtered, and the filtrate was concentrated by vacuum distillation to obtain the crude title product **LD-1c** (1.49 g) as a yellow solid. The product was used directly in the next step without purification.
MS m/z (ESI): 392.1 [M+23].

Step 3

*tert*-Butyl 4-(2,6-bis((2,5,8,11,14,17,20,23-octaoxapentacosan-25-yl)carbamoyl)-4-nitrophenoxy)butanoate **LD-1d**

**LD-1c** (400 mg, 933.60 μmol) was dissolved in 5 mL of *N,N*-dimethylformamide, added with 1-hydroxybenzotriazole (378.45 mg, 2.80 mmol), 1-(3-dimethylaminopropyl)- 3-ethylcarbodiimide hydrochloride (536.92 mg, 2.80 mmol), *N*,*N*-diisopropylethylamine (723.97 mg, 5.60 mmol) and 3,6,9,12,15,18,21,24-octaoxapentacosane-1-amine (895.03 mg, 2.33 mmol), and stirred at 25 °C for 2 hours. 10 mL of water was added, and dichloromethane (10 mLx3) was used for extraction. The organic phase was dried over anhydrous sodium sulfate and filtered, the filtrate was concentrated by vacuum distillation, and the obtained residue was purified by silica gel column chromatography with the developing system B to obtain the title product **LD-1d** (515 mg, yield: 50%) as a yellow oil.
MS m/z (ESI): 1117.7 [M+18].

Step 4

*tert*-Butyl 4-(2,6-bis((2,5,8,11,14,17,20,23-octaoxapentacosan-25-yl)carbamoyl)- 4-aminophenoxy)butanoate **LD-1e**

**LD-1d** (515 mg, 466.74 μmol) was dissolved in a mixed solvent of 20 mL of ethanol and 10 mL of water, added with iron powder (130.32 mg, 2.33 mmol) and ammonium chloride (249.66 mg, 4.67 mmol), and stirred at 80 °C for 16 hours. The reaction solution was filtered with Celite, 10 mL of water was added to the filtrate, the diluted filtrate was extracted with dichloromethane (10 mLx3), the organic phase was dried over anhydrous sodium sulfate and filtered, and the filtrate was concentrated by vacuum distillation. The obtained residue was purified by silica gel column chromatography with the eluent system A to obtain the title product **LD-1e** (435 mg, yield: 87%) as a yellow oil.
MS m/z (ESI): 1070.8 [M+1].

Step 5

*tert*-Butyl 4-(2,6-bis((2,5,8,11,14,17,20,23-octaoxapentacosan-25-yl)carbamoyl)-4- (2,5-dioxo-2,5-dihydro-1*H*-pyrrol-1-yl)phenoxy)butanoate **LD-1f**

**LD-1e** (380 mg, 352.47 μmol) and 2,5-furandione (38.02 mg, 387.71 μmol) were dissolved in 6 mL of dioxane, and stirred at 25 °C for 1 hour. After the completion of the reaction of the starting material **LD-1e** monitored by TLC, the reaction solution was added with ammonium persulfate (160.87 mg, 704.93 μmol) and dimethyl sulfoxide (55.08 mg, 704.93 μmol), and stirred at 100 °C for 1 hour. The reaction solution was concentrated by vacuum distillation, and the residue was purified by preparative HPLC (separation conditions: chromatographic column: Welch Xtimate C18 150x25mmx5μm; mobile phase: A-water (0.1% trifluoroacetic acid), B-acetonitrile, gradient elution, B%: 25%-55%, flow rate 25 mL/min) to obtain the title product **LD-1f** (91 mg, yield: 21%) as a yellow oil.
MS m/z (ESI): 1167.8 [M+18].

Step 6

4-(2,6-Bis((2,5,8,11,14,17,20,23-octaoxapentacosan-25-yl)carbamoyl)-4-(2,5-dioxo-2,5-dihydro-1*H*-pyrrol-1-yl)phenoxy)butanoic acid **LD-1g**
**LD-1f** (2 mg, 1.74 μmol) was dissolved in 0.5 mL of dichloromethane, added with trifluoroacetic acid (0.1 mL), and stirred at 25 °C for 1 hour. The reaction solution was concentrated by vacuum distillation to obtain the crude title product **LD-1g** (1.5 mg) as a colorless oil. The product was used directly in the next step without purification.

MS m/z (ESI): 1111.8 [M+18].

Step 7

(9*H*-Fluoren-9-yl)methyl ((*S*)-10-benzyl-1-(((1*S*,9*S*)-9-(cyclopropylmethyl)-5-fluoro-9-hydroxy- 4-methyl-10,13-dioxo-2,3,9,10,13,15-hexahydro-1*H*,12*H*-benzo[*de*]pyrano[3',4':6,7]indolizino [1,2-*b*]quinolin-1-yl)amino)-1,6,9,12,15-pentaoxo-3-oxa-5,8,11,14-tetraazahexadecan-16-yl) carbamate **LD-1i**

**LD-1h** (68.7 mg, 94.7 μmol, prepared by applying the method disclosed in Example LND1067-L1 on page 9 of patent application "CN113402584 A") and **1i-1-1** (50.0 mg, 78.9 μmol, trifluoroacetate) were dissolved in 2 mL of *N,N*-dimethylformamide, added with 1-hydroxybenzotriazole (16.0 mg, 118 μmol), 1-(3-dimethylaminopropyl)-3-ethylcarbodiimide hydrochloride (22.7 mg, 118 μmol), *N,N*-diisopropylethylamine (30.6 mg, 236 μmol), and stirred at 25 °C for 3 hours. 1 mL of water was added, dichloromethane (3 mLx3) was used for extraction, the organic phase was dried over anhydrous sodium sulfate and filtered, and the filtrate was concentrated by vacuum distillation. The obtained residue was purified by silica gel column chromatography with the eluent system A to obtain the title product **LD-1i** (60 mg, yield: 57%) as a yellow solid.

MS m/z (ESI): 1089.6 [M+1].

Step 8

(*S*)-2-(2-(2-Aminoacetamido)acetamido)-*N*-(2-(((2-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7] indolizino[1,2-*b*]quinolin-1-yl)amino)-2-oxoethoxy) methyl)amino)-2-oxoethyl)-3-phenylpropanamide **LD-1j**

**LD-1i** (55.0 mg, 41.5 μmol) was dissolved in 1.5 mL of acetonitrile, added with diethylamine (213 mg, 2.91 mmol), and stirred at 25 °C for 1 hour. The reaction solution was concentrated by vacuum distillation to obtain the crude title product **LD-1j** (35 mg) as a yellow solid. The product was used directly in the next step without purification.

MS m/z (ESI): 867.3 [M+1].

Step 9

2-(((*S*)-10-Benzyl-1-(((1S,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl) amino)-1,6,9,12,15,18-hexaoxo-3-oxa-5,8,11,14,17-pentaazahenicosan-21-yl)oxy)-5-(2,5-dioxo -2,5-dihydro-1*H*-pyrrol-1-yl)-$N^1$,$N^3$-bis(2,5,8,11,14,17,20,23-octaoxapentacosan-25-yl)-isophthalamide **LD-1**

The title compound **LD-1** was obtained by using **LD-1j** and **LD-1g** in the presence of 1-hydroxybenzotriazole, 1-(3-dimethylaminopropyl)-3-ethylcarbodiimide hydrochloride and *N,N*-diisopropylethylamine.

MS m/z (ESI): 972.5 [1/2(M+2)].

By referring to the method of Example 3-1, corresponding substrates were used to prepare **LD-2** to **LD-12:**

Example 3-2: Preparation of LD-2

2-(((2*S*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-methyl-1,6,9,12,15,18-hexaoxo-3-oxa-5,8,11,14,17-pentaazahenicosan-21-yl)oxy)-5-(2,5-dioxo-2,5-dihydro-1*H*-pyrrol-1-yl)-$N^1$,$N^3$-di(2,5,8,11,14,17,20,23-octaoxapentacosan-25-yl)isophthalamide **LD-2**

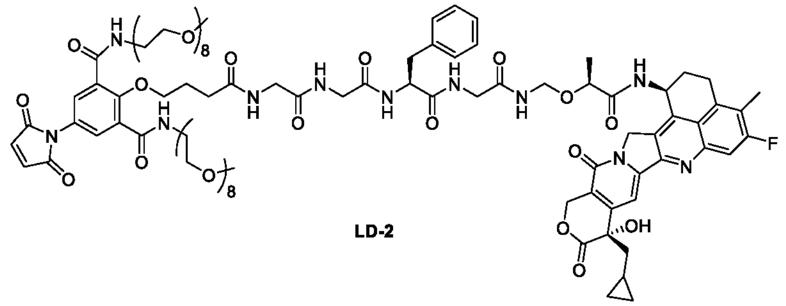

LD-2

MS m/z (ESI): 979.5 [1/2(M+2)].

Example 3-3: Preparation of LD-3

2-(((2*R*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinol in-1-yl)amino)-2-methyl-1,6,9,12,15,18-hexaoxo-3-oxa-5,8,11,14,17-pentaazahenicosan-21-yl)oxy)-5-(2,5-dioxo-2,5-dihydro-1*H*-pyrrol-1-yl)-*N*[1],*N*[3]-di(2,5,8,11,14,17,20,23-octaoxapentacosan-25-yl)isophthalamide **LD-3**

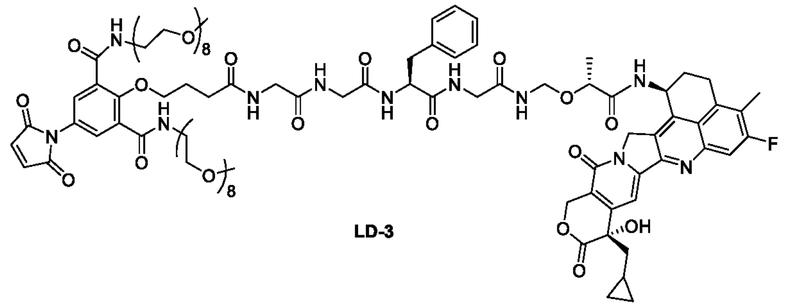

LD-3

MS m/z (ESI): 979.6 [1/2(M+2)].

Example 3-4: Preparation of LD-4

2-(((2*S*,10*S*)-10-Benzyl-2-cyclopropyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizin o[1,2-*b*]quinolin-1-yl)amino)-1,6,9,12,15,18-hexaoxo-3-oxa-5,8,11,14,17-pentaazahenicosan-21-yl)oxy)-5-(2,5-dioxo-2,5-dihydro-1*H*-pyrrol-1-yl)-*N*[1],*N*[3]-di(2,5,8,11,14,17,20,23-octaoxapentacosan-25-yl)isophthalamide **LD-4**

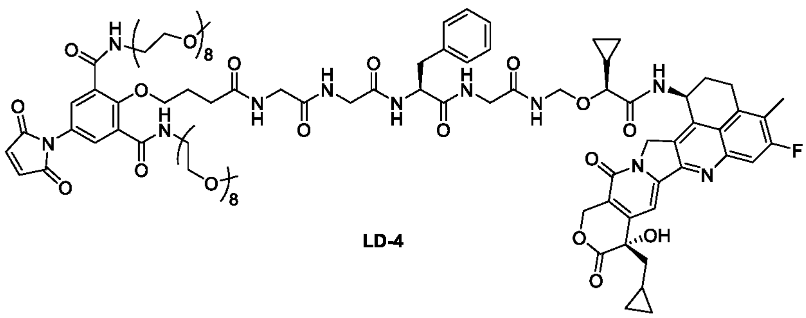

LD-4

MS m/z (ESI): 992.5 [1/2(M+2)].

Example 3-5: Preparation of LD-5

2-(((2*R*,10*S*)-10-Benzyl-2-cyclopropyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizin o[1,2-*b*]quinolin-1-yl)amino)-1,6,9,12,15,18-hexaoxo-3-oxa-5,8,11,14,17-pentaazahenicosan-21-yl)oxy)-5-(2,5-dioxo-2,5-dihydro-1*H*-pyrrol-1-yl)-*N1*,*N3*-di(2,5,8,11,14,17,20,23-octaoxapentacosan-25-yl)isophthalamide **LD-5**

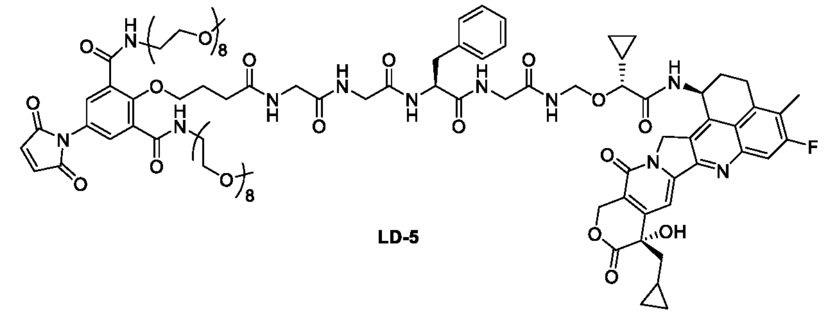

LD-5

MS m/z (ESI): 992.5 [1/2(M+2)].

Example 3-6: Preparation of LD-6

2-(((*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15,18-hexaoxo-3-oxa-5,8,11,14,17-pentaazahenicosan-21-yl)oxy)-5-(2,5-diox o-2,5-dihydro-1*H*-pyrrol-1-yl)-3-fluoro-*N*-(2,5,8,11,14,17,20,23-octaoxapentacosan-25-yl)benzamide **LD-6**

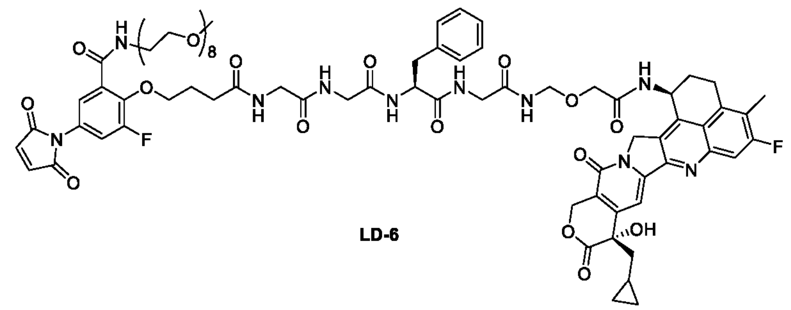

LD-6

MS m/z (ESI): 776.8 [1/2(M+2)].

Example 3-7: Preparation of LD-7

2-(((2*S*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-methyl-1,6,9,12,15,18-hexaoxo-3-oxa-5,8,11,14,17-pentaazahenicosan-21-yl)o xy)-5-(2,5-dioxo-2,5-dihydro-1*H*-pyrrol-1-yl)-3-fluoro-*N*-(2,5,8,11,14,17,20,23-octaoxapentacosan-25-yl)benzamide **LD-7**

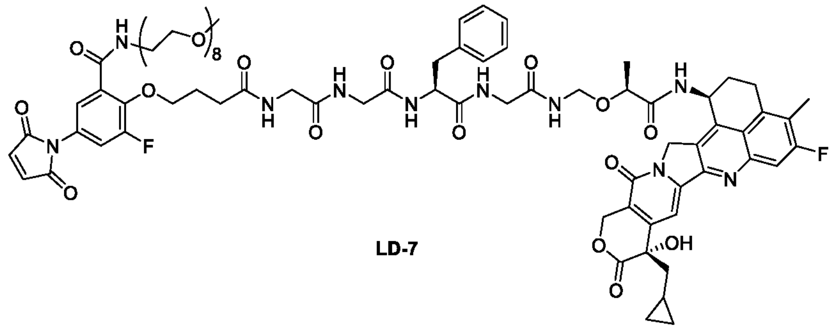

LD-7

Example 3-8: Preparation of LD-8

2-(((2*R*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinol in-1-yl)amino)-2-methyl-1,6,9,12,15,18-hexaoxo-3-oxa-5,8,11,14,17-pentaazahenicosan-21-yl)oxy)-5-(2,5-dioxo-2,5-dihydro-1*H*-pyrrol-1-yl)-3-fluoro-*N*-(2,5,8,11,14,17,20,23-octaoxapentacosan-25-yl)benzamide **LD-8**

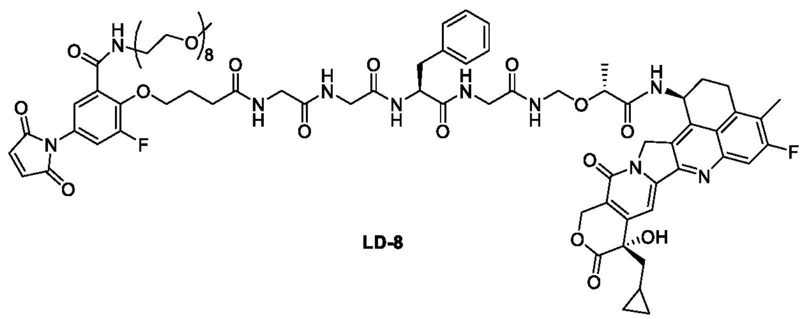

LD-8

Example 3-9: Preparation of LD-9

2-(((2*S*,10*S*)-10-benzyl-2-cyclopropyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15,18-hexaoxo-3-oxa-5,8,11,14,17-pentaazahenicosan-21-yl)oxy)-5-(2,5-dioxo-2,5-dihydro-1*H*-pyrrol-1-yl)-3-fluoro-*N*-(2,5,8,11,14,17,20,23-octaoxapentacosan-25-yl)benzamide **LD-9**

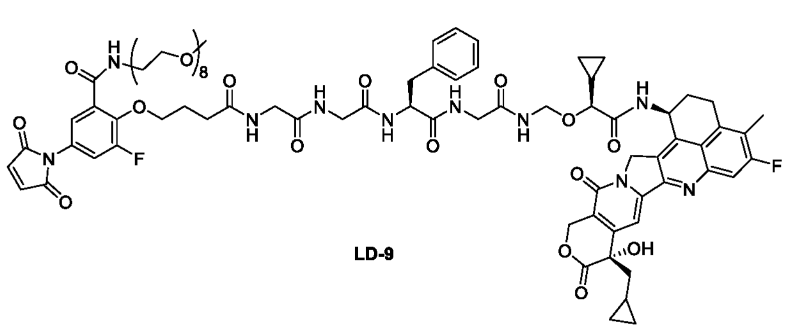

LD-9

Example 3-10: Preparation of LD-10

2-(((2*R*,10*S*)-10-Benzyl-2-cyclopropyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizin o[1,2-*b*]quinolin-1-yl)amino)-1,6,9,12,15,18-hexaoxo-3-oxa-5,8,11,14,17-pentaazahenicosan-21-yl)oxy)-5-(2,5-dioxo-2,5-dihydro-1*H*-pyrrol-1-yl)-3-fluoro-*N*-(2,5,8,11,14,17,20,23-octaoxapentacosan-25-yl)benzamide **LD-10**

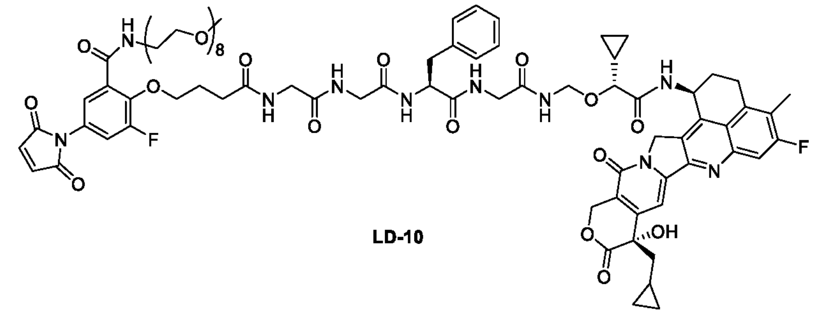

**LD-10**

Example 3-11: Preparation of LD-11

*N*-((*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-6-(2-(methylsulfonyl)pyrimidin-5-yl)hex-5-ynamide **LD-11**

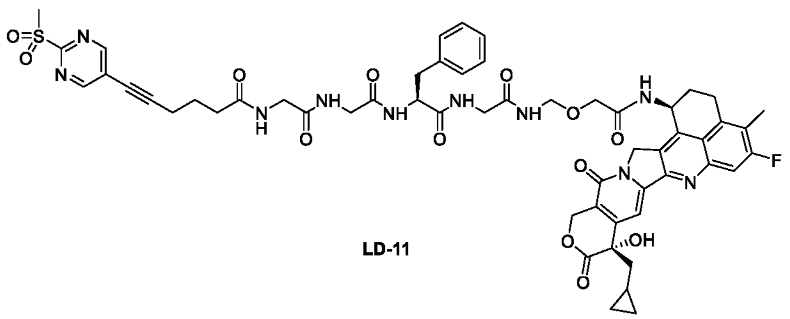

**LD-11**

MS m/z (ESI): 559.8 [1/2(M+2)].

Example 3-12: Preparation of LD-12

(*S*)-$N^5$-((*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-2-(6-(2-(methylsul fonyl)pyrimidin-5-yl)hex-5-ynamido)-$N^1$-(2,5,8,11,14,17,20,23-octaoxapentacosan-25-yl)pentanediamide **LD-12**

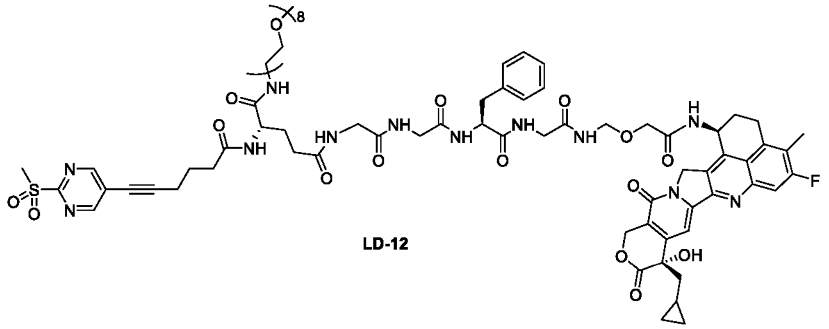

**LD-12**

MS m/z (ESI): 806.9 [1/2(M+2)].

Example 3-13: Preparation of LD-13

(*S*)-*N*[5]-((2*S*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-methyl-1,6,9,12,15-pentaoxo-3-oxa-5,8,11,14-tetraazahexadecan-16-yl)-2-(6-(2-(methylsulfonyl)pyrimidin-5-yl)hex-5-ynamido)-*N*[1]-(2,5,8,11,14,17,20,23-octaoxapentacosan-25-yl)pentanediamide **LD-13**

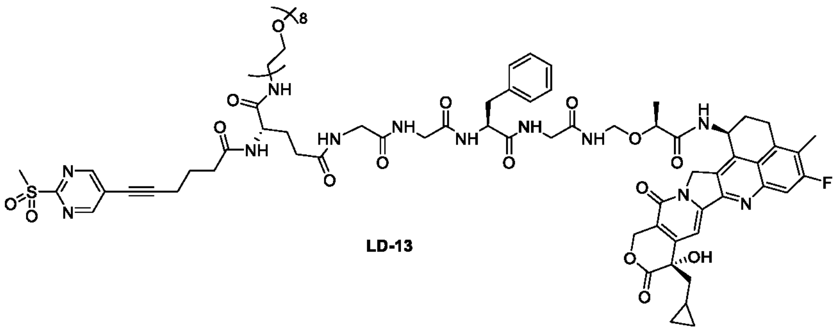

**LD-13**

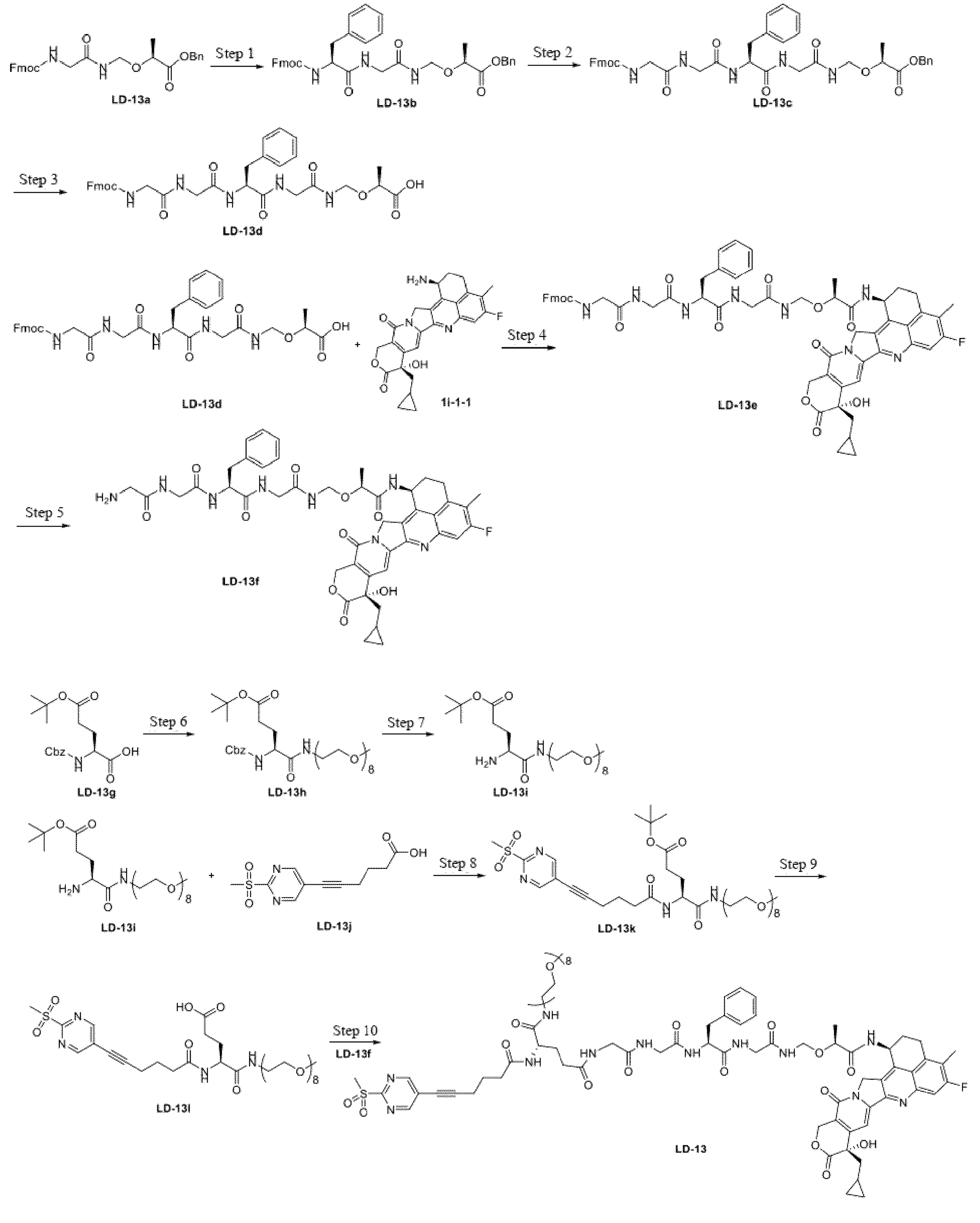


Step 1

Benzyl (*5S,13S*)-5-Benzyl-1-(9*H*-fluoro-9-yl)-13-methyl-3,6,9-trioxo-2,12-dioxa-4,7,10-triazatetradecan-14-oate **LD-13b**

**LD-13a** (1.70 g, 3.17 mmol, prepared by applying the method disclosed in Example 3 on page 8 of patent application "WO2022056696 A1") was dissolved in 20 mL of *N,N*-dimethylformamide, added with 1,8-diazabicyclo[5.4.0] undec-7-ene (241 mg, 1.59 mmol), and stirred at 20 °C for 1 hour. Then, (((9*H*-fluoren-9-yl)methoxy)carbonyl)-L-phenylalanine (1.16 g, 3.00 mmol), 1-hydroxybenzotriazole (608 mg, 4.51 mmol) and 1-(3-dimethylaminopropyl)-3-ethylcarbodiimide hydrochloride (863 mg, 4.51 mmol) were added to the reaction solution in sequence, and stirred at 25 °C for 1 hour. 50 mL of water was added, ethyl acetate (45 mLx3) was used for extraction, the organic phase was dried over anhydrous sodium sulfate and filtered, the filtrate was concentrated by vacuum distillation. The residue was purified by silica gel column chromatography using the eluent system A to obtain the title product **LD-13b** (1.35 g, yield: 65%) as a white solid.

MSm/z (ESI): 658.1 [M+23].

Step 2

Benzyl (11*S*,19*S*)-11-Benzyl-1-(9*H*-fluoren-9-yl)-19-methyl-3,6,9,12,15-pentaoxo-2,18-dioxa-4,7,10,13,16-pentaazaicosan-20-oate **LD-13c**

**LD-13b** (1.25 g, 1.81 mmol) was dissolved in 20 mL of *N,N*-dimethylformamide, added with 1,8-diazabicyclo [5.4.0]undec-7-ene (165 mg, 1.09 mmol), and stirred at 25 °C for 1 hour. Then, (((9*H*-fluoren-9-yl)methoxy)carbonyl) glycylglycine (750 mg, 2.12 mmol) and 2-(7-azobenzotriazole)-*N,N,N',N'*-tetramethyluronium hexafluorophosphate (1.01 g, 2.65 mmol) were added to the reaction solution in sequence, and stirred at 25 °C for 1 hour. 50 mL of water was added, ethyl acetate (45 mLx3) was used for extraction, the organic phase was dried over anhydrous sodium sulfate and filtered, the filtrate was concentrated by vacuum distillation. The residue was purified by silica gel column chromatography with the eluent system A to obtain the title product **LD-13c** (850 mg, yield: 53%) as a white solid.

MS m/z (ESI): 767.4 [M+18].

Step 3

(11*S*,19*S*)-11-Benzyl-1-(9*H*-fluoren-9-yl)-19-methyl-3,6,9,12,15-pentaoxo-2,18-dioxa-4,7,10,13,16-pentaazaicosan-20-oic acid **LD-13d**

Under nitrogen protection, **LD-13c** (750 mg, 817 μmol) and palladium-on-carbon (434 mg, 10%, wetted with ca. 55% water) were dissolved in 4 mL of ethyl acetate and 8 mL of ethanol, and subjected to replacement with hydrogen gas three times. The reaction solution was stirred at 25 °C under hydrogen (15 Psi) for 1 hour. The reaction solution was filtered with Celite, and the filtrate was concentrated by vacuum distillation to obtain the crude title product **LD-13d** (450 mg, yield: 67%) as a white solid. The product was used directly in the next reaction without purification.

MS m/z (ESI): 677.2 [M+18].

Step 4

(9*H*-Fluoren-9-yl)methyl ((2*S*,10*S*)-10-benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[de]pyrano[3',4':6,7] indolizino[1,2-*b*]quinolin-1-yl)amino)-2-methyl-1,6,9,12,15-pentaoxo-3-oxa-5,8,11,14-tetraazahexadecan-16-yl)carbamate **LD-13e**

**LD-13d** (30.0 mg, 36.8 μmol) and **1i-1-1** trifluoroacetate (21.5 mg, 36.8 μmol) were dissolved in 1 mL of *N,N*-dimethylformamide, added with 1-hydroxybenzotriazole (7.47 mg, 55.2 μmol), 1-(3-dimethylaminopropyl)-3-ethylcarbodiimide hydrochloride (10.6 mg, 55.3 μmol) and *N,N*-diisopropylethylamine (14.2 mg, 110 μmol) in sequence, and stirred at 25 °C for 1 hour. 2 mL of water was added, dichloromethane (5 mLx3) was used for extraction, the organic phase was dried over anhydrous sodium sulfate and filtered, and the filtrate was concentrated by vacuum distillation. The obtained residue was purified by silica gel column chromatography with the eluent system A to obtain the title product **LD-13e** (32.8 mg, yield: 65%) as a yellow solid.

MS m/z (ESI): 1103.2 [M+1].

Step 5

(*S*)-2-(2-(2-Aminoacetamido)acetamido)-*N*-(2-(((((*S*)-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano [3',4':6,7]indolizino[1,2-*b*]quinolin-1-yl)amino)-1-oxopropan-2-yl)oxy)methyl)amino)-2-oxoethyl)-3-phenylpropanamide **LD-13f**

**LD-13e** (32.0 mg, 23.5 μmol) was dissolved in 1 mL of acetonitrile, added with diethylamine (142 mg, 1.94 mmol), and stirred at 25 °C for 1 hour. The reaction solution was concentrated by vacuum distillation, and the residue was purified by silica gel column chromatography using the eluent system A to obtain the title product **LD-13f** (18 mg, yield: 87%) as a

white solid.

MS m/z (ESI): 881.2 [M+1].

Step 6

**LD-13g** (1.0 g, 2.97 mmol) was dissolved in 10 mL of *N,N*-dimethylformamide, added with *N,N*-diisopropylethylamine (1.15 g, 8.90 mmol), 1-hydroxybenzotriazole (400 mg, 2.97 mmol) and 1-(3-dimethylaminopropyl)-3-ethylcarbodiimide hydrochloride (570 mg, 2.97 mmol) in sequence, and stirred at 0 °C for 15 minutes. The reaction solution was added with 3,6,9,12,15,18,21,24-octaoxapentacosan-1-amine (1.14 g, 2.97 mmol), gradually heated to 25 °C and stirred for 2 hours. 20 mL of water was added, and ethyl acetate (30 mLx3) was used for extraction. The organic phase was dried over anhydrous sodium sulfate and filtered, the filtrate was concentrated by vacuum distillation. The residue was purified by silica gel column chromatography using the eluent system A to obtain the title product **LD-13h** (1.28 g, yield: 62%) as a colorless oil.

MS m/z (ESI): 703.4 [M+1].

Step 7

**LD-13h** (1.28 g, 1.82 mmol) was dissolved in 13 mL of methanol; under nitrogen protection, palladium-on-carbon (10%, wetted with ca. 55% water, 300 mg) was added and stirred at 20 °C for 1 hour. The reaction mixture was subjected to replacement with hydrogen three times, and stirred at 25 °C for 2 hours under hydrogen gas (15 Psi). The reaction mixture was filtered with Celite, and the filtrate was concentrated by vacuum distillation to obtain the crude title product **LD-13i** (900 mg, yield: 87%) as a colorless oil. The product was used directly in the next reaction without purification.

MS m/z (ESI): 569.3 [M+1].

Step 8

**LD-13i** (300 mg, 0.53 mmol) was dissolved in 5 mL of *N,N*-dimethylformamide, added with 2-(7-azobenzotriazole)-*N,N,N',N'*-tetramethyluronium hexafluorophosphate (200 mg, 0.53 mmol) and N,N-diisopropylethylamine (204 mg, 1.58 mmol), then added with **LD-13j** (142 mg, 0.53 mmol, prepared by applying the method disclosed in Example 2 on page 30 of patent application "WO2021228141 A1"), and stirred at 25 °C for 2 hours. 10 mL of water was added, ethyl acetate (15 mLx3) was used for extraction, the organic phase was dried over anhydrous sodium sulfate and filtered, the filtrate was concentrated by vacuum distillation, the residue was purified by silica gel column chromatography with the eluent system A to obtain the title product **LD-13k** (107.85 mg, yield: 25%) as a colorless oil.

MS m/z (ESI): 819.4 [M+1].

Step 9

**LD-13k** (260 mg, 0.32 mmol) was dissolved in 5 mL of dichloromethane, added with 1 mL of trifluoroacetic acid, and stirred at 25 °C for 2 hours. The reaction solution was concentrated by vacuum distillation, and the residue was purified by pre-HPLC (separation conditions: chromatographic column: Sunfire Prep C18 OBD 10μm, 19x250mm, mobile phase: A-acetonitrile, B-water, gradient elution, A%: 35%-95%) to obtain the title product **LD-131** (82.84 mg, yield: 34%) as a white solid.

MS m/z (ESI): 763.3 [M+1].

Step 10

**LD-131** (17.9 mg, 23.5 μmol) and **LD-13f** (18.0 mg, 20.4 μmol) were dissolved in 1 mL of *N,N*-dimethylformamide, added with 1-hydroxybenzotriazole (4.14 mg, 30.6 μmol), 1-(3-dimethylaminopropyl)-3-ethylcarbodiimide hydrochloride (5.88 mg, 30.6 μmol) and *N,N*-diisopropylethylamine (7.92 mg, 61.3 μmol) in sequence, and stirred at 20 °C for 1 hour. The reaction solution was concentrated by vacuum distillation, and the residue was purified by pre-HPLC (separation conditions: chromatographic column: Welch Xtimate C18 150x25mmx5μm, mobile phase: A-water (0.1% trifluoroacetic acid), B-acetonitrile, gradient elution, B%: 30%-60%) to obtain the title product **LD-13** (2.09 mg, yield: 6%) as a purple solid.

MS m/z (ESI): 813.9 [1/2(M+2)].

MS m/z (ESI): 1625.5 [M+1].

$^1$H NMR (400 MHz, $CD_3OD$) δ 8.99 - 8.88 (m, 2H), 8.64 - 8.57 (m, 1H), 8.54 - 8.49 (m, 1H), 8.22 - 8.14 (m, 1H), 8.08 - 8.01 (m, 1H), 7.70 - 7.59 (m, 2H), 7.27 - 7.20 (m, 2H), 7.20 - 7.07 (m, 3H), 5.72 - 5.64 (m, 1H), 5.61 - 5.54 (m, 1H), 5.49 - 5.41 (m, 1H), 5.40 - 5.25 (m, 2H), 5.20 - 5.13 (m, 1H), 4.78 - 4.71 (m, 2H), 4.39 - 4.25 (m, 3H), 3.94 - 3.86 (m, 3H), 3.80 - 3.75 (m, 1H), 3.73 - 3.55 (m, 26H), 3.55 - 3.49 (m, 4H), 3.38 - 3.33 (m, 6H), 3.20 - 3.12 (m, 1H), 2.97 - 2.84 (m, 2H), 2.60 - 2.53 (m,

2H), 2.50 - 2.38 (m, 5H), 2.38 - 2.27 (m, 3H), 2.26 - 2.14 (m, 2H), 2.11 - 2.02 (m, 2H), 1.98 - 1.83 (m, 4H), 1.78 - 1.71 (m, 1H), 1.65 - 1.56 (m, 1H), 1.49 - 1.41 (m, 3H), 0.94 - 0.80 (m, 2H), 0.46 - 0.33 (m, 2H), 0.09 - 0.03 (m, 1H), 0.01 - -0.07 (m, 1H).

By referring to the method of Example 3-13, corresponding substrates were used to prepare **LD-14** to **LD-16:**

Example 3-14: Preparation of LD-14

(*S*)-*N*[5]-((2*R*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-methyl-1,6,9,12,15-pentaoxo-3-oxa-5,8,11,14-tetraazahexadecan-16-yl)-2-(6-(2-(methylsulfonyl)pyrimidin-5-yl)hex-5-ynamido)-*N*[1]-(2,5,8,11,14,17,20,23-octaoxapentacosan-25-yl)pentanediamide **LD-14**

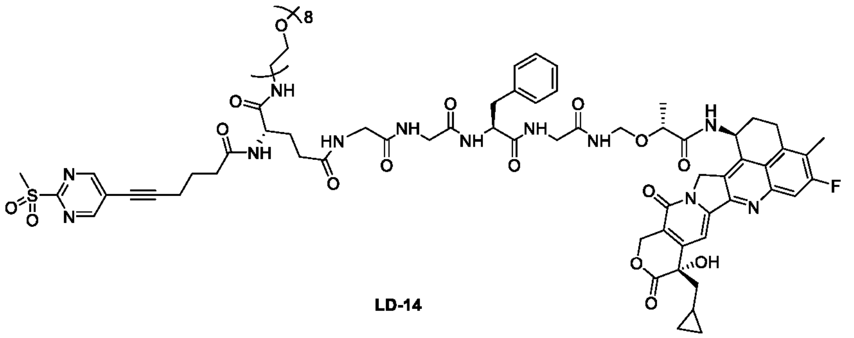

LD-14

MS m/z (ESI): 813.9 [1/2(M+2)].

Example 3-15: Preparation of LD-15

(S)-*N*[5]-((2*S*,10*S*)-10-benzyl-2-cyclopropyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-2-(6-(2-(methylsulfonyl)pyrimidin-5-yl)hex-5-ynamido)-*N*[1]-(2,5,8,11,14,17,20,23-octaoxapentacosan-25-yl)pentanediamide **LD-15**

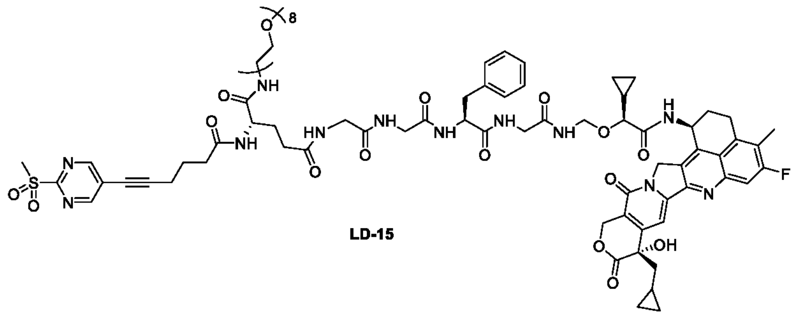

LD-15

Example 3-16: Preparation of LD-16

(*S*)-*N*[5]-((2*R*,10*S*)-10-Benzyl-2-cyclopropyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-y l)-2-(6-(2-(methylsulfonyl)pyrimidin-5-yl)hex-5-ynamido)-*N*[1]-(2,5,8,11,14,17,20,23-octaoxapentacosan-25-yl)pentanediamide **LD-16**

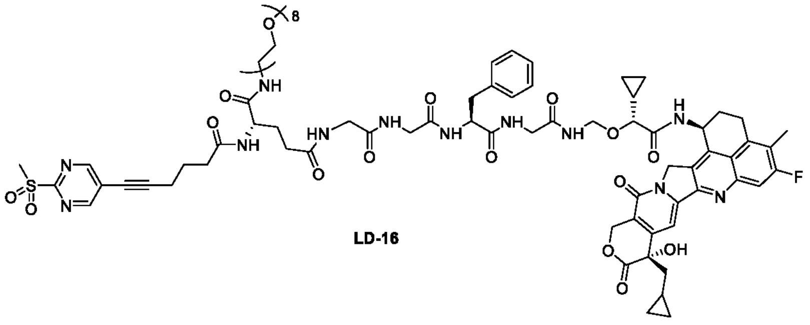

LD-16

Example 3-17: Preparation of LD-17

(*S*)-$N^5$-((*S*)-10-Benzyl-1-(((1*S*,9*S*)-9-(cyclopropylmethyl)-5-fluoro-9-hyd+roxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-2-(6-(2,5-dioxo-2, 5-dihydro-1*H*-pyrrol-1-yl)hexanamido)-$N^1$-(2,5,8,11,14,17,20,23-octaoxapentacosan-25-yl)pentanediamide **LD-17**

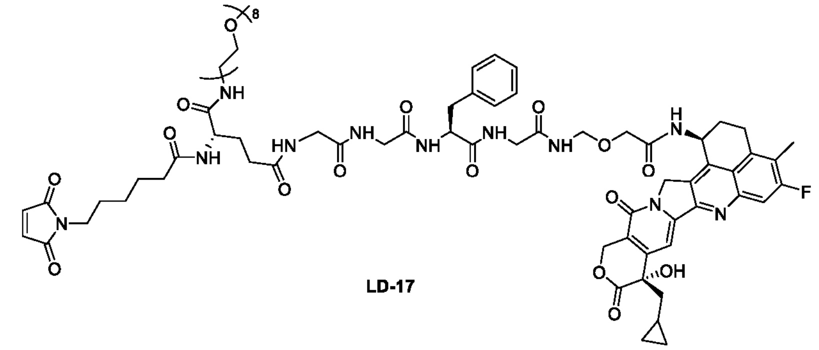

LD-17

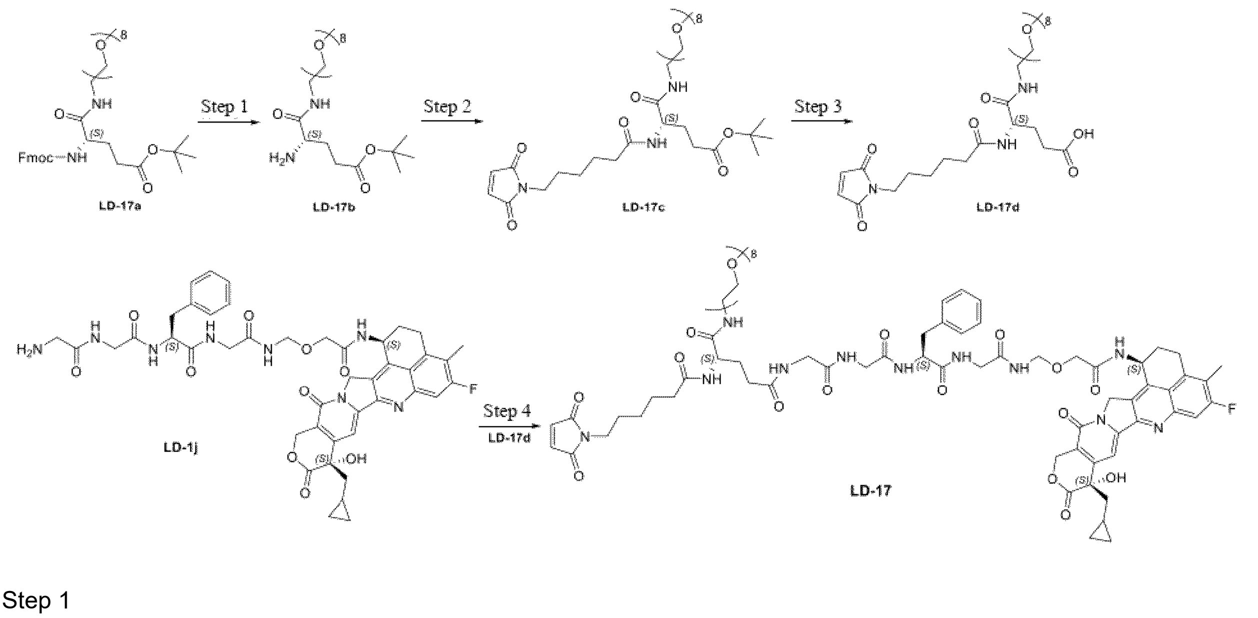


Step 1

*tert*-Butyl (*S*)-28-((((9*H*-fluorene-9-yl)methoxy)carbonyl)amino)-27-oxo-2,5,8,11,14,17,20,23- octaoxa-26-azahentriacontan-31-oate **LD-17b**

**LD-17a** (75.0 mg, 92.0 μmol, prepared by applying the method disclosed in Example 2 on page 210 of patent

application "WO2022262516 A1") was dissolved in 1.5 mL of acetonitrile, added with 1,2-dichloroethane (375 mg, 3.80 mmol), and stirred at 25 °C for 1 hour. The reaction solution was concentrated by vacuum distillation to obtain the crude title product **LD-17b** (50 mg) as a yellow solid. The product was used directly in the next reaction without purification.

MS m/z (ESI): 569.4 [M+1].

Step 2

*tert*-Butyl (*S*)-28-(6-(2,5-dioxo-2,5-dihydro-1*H*-pyrrol-1-yl)hexanamido)-27-oxo-2,5,8,11,14,17,20,23-octaoxa-26-azahentriacontan-31-oate **LD-17c**

**LD-17b** (80.0 mg, 140 μmol) and 6-(2,5-dioxopyrrol-1-yl)hexanoic acid (44.5 mg, 211 μmol) were dissolved in 2 mL of dichloromethane, added with 1-hydroxybenzotriazole (28.5 mg, 211 μmol), 1-(3-dimethylaminopropyl)-3-ethylcarbodiimide hydrochloride (40.4 mg, 211 μmol) and N,N-diisopropylethylamine (54.5 mg, 422 μmol) in sequence, and stirred at 25 °C for 8 hours. 10 mL of water was added to the reaction solution, extracted with dichloromethane (15 mLx3), the organic phase was dried over anhydrous sodium sulfate and filtered, and the filtrate was concentrated by vacuum distillation. The residue was purified by pre-HPLC (separation conditions: chromatographic column: Welch Xtimate C18 150x25mmx5μm, mobile phase: A-water (0.1% trifluoroacetic acid), B-acetonitrile, gradient elution, B%: 23%-53%) to obtain the title product **LD-17c** (60 mg, yield: 90%) as a yellow jelly.

MS m/z (ESI): 762.4 [M+1].

Step 3

(*S*)-28-(6-(2,5-Dioxo-2,5-dihydro-1*H*-pyrrol-1-yl)hexanamido)-27-oxo-2,5,8,11,14,17,20,23-octaoxa-26-azahentriacontan-31-oic acid **LD-17d**

**LD-17c** (20.0 mg, 26.2 μmol) was dissolved in 1 mL of dichloromethane, added with trifluoroacetic acid (307 mg, 2.69 mmol), and stirred at 25 °C for 1 hour. The reaction solution was filtered, and the filtrate was concentrated by vacuum distillation. The residue was purified by pre-HPLC (separation conditions: chromatographic column: Welch Xtimate C18 150x25mmx5μm, mobile phase: A-water (0.1% trifluoroacetic acid), B-acetonitrile, gradient elution, B%: 10%-40%) to obtain the title product **LD-17d** (12 mg, yield: 64%) as a yellow colloid.

MS m/z (ESI): 706.4 [M+1].

Step 4

**LD-17d** and **LD-1j** were used as starting materials to prepare the title product **LD-17** by a method similar to that of **LD-13.**

By referring to the method of Example 3-13, corresponding substrates were used to prepare **LD-18** to **LD-96:**

Example 3-18: Preparation of LD-18

(S)-$N^5$-((2*S*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-methyl-1,6,9,12,15-pentaoxo-3-oxa-5,8,11,14-tetraazahexadecan-16-yl)-2-(6-(2,5-dioxo-2,5-dihydro-1*H*-pyrrol-1-yl)hexanamido)-$N^1$-(2,5,8,11,14,17,20,23-octaoxapent acosan-25-yl)pentanediamide **LD-18**

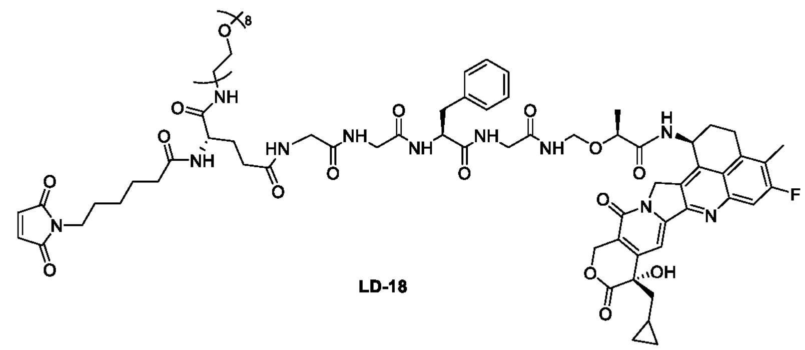

LD-18

Example 3-19: Preparation of LD-19

(*S*)-*N*5-((2*R*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-methyl-1,6,9,12,15-pentaoxo-3-oxa-5,8,11,14-tetraazahexadecan-16-yl)-2-(6-(2,5-dioxo-2,5-dihydro-1*H*-pyrrol-1-yl)hexanamido)-*N*1-(2,5,8,11,14,17,20,23-octaoxapentacosan-25-yl)pentanediamide **LD-19**

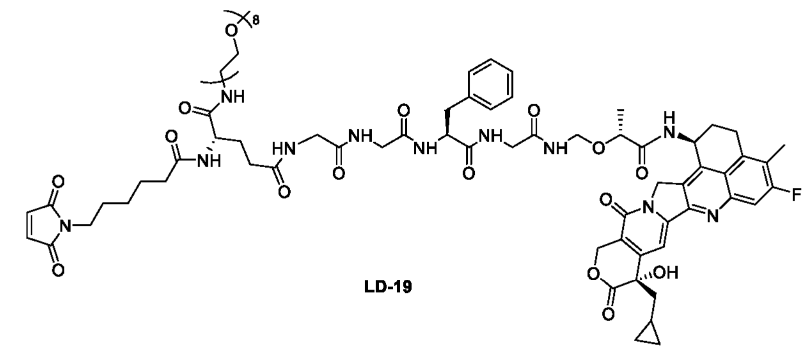

**LD-19**

Example 3-20 LD-20

(S)-*N*5-((2*S*,10*S*)-10-Benzyl-2-cyclopropyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl) -2-(6-(2,5-dioxo-2,5-dihydro-1*H*-pyrrol-1-yl)hexanamido)-*N*1-(2,5,8,11,14,17,20,23-octaoxapentacosan-25-yl)pentanediamide **LD-20**

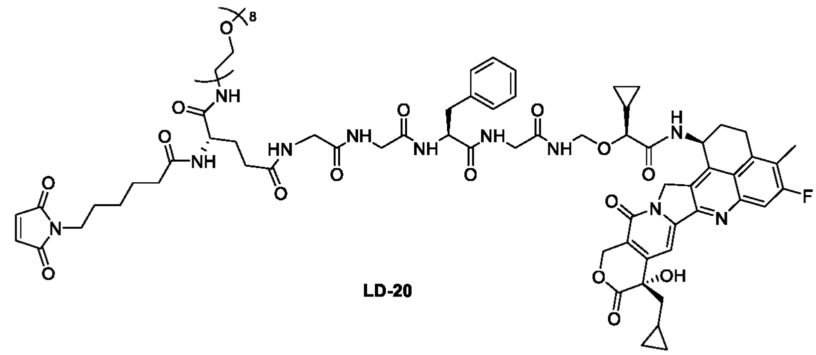

**LD-20**

Example 3-21: Preparation of LD-21

(*S*)-*N*5-((2*R*,10*S*)-10-Benzyl-2-cyclopropyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-y l)-2-(6-(2,5-dioxo-2,5-dihydro-1*H*-pyrrol-1-yl)hexanamido)-*N*1-(2,5,8,11,14,17,20,23-octaoxapentacosan-25-yl)pentanediamide LD-21

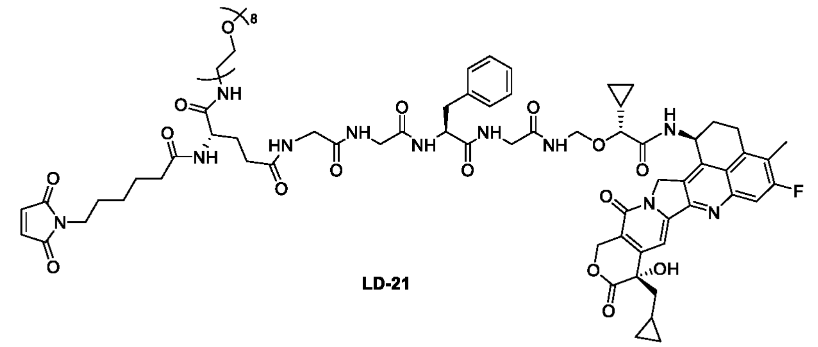

LD-21

Example 3-22: Preparation ofLD-22

(*S*)-*N*5-((*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-2-(6-(2,5-dioxo-2, 5-dihydro-1*H*-pyrrol-1-yl)hexanamido)-*N*1-methyl-*N*1-((2*S*,3*R*,4*R*,5*R*)-2,3,4,5,6-pentahydroxyhexyl)pentanediamide **LD-22**

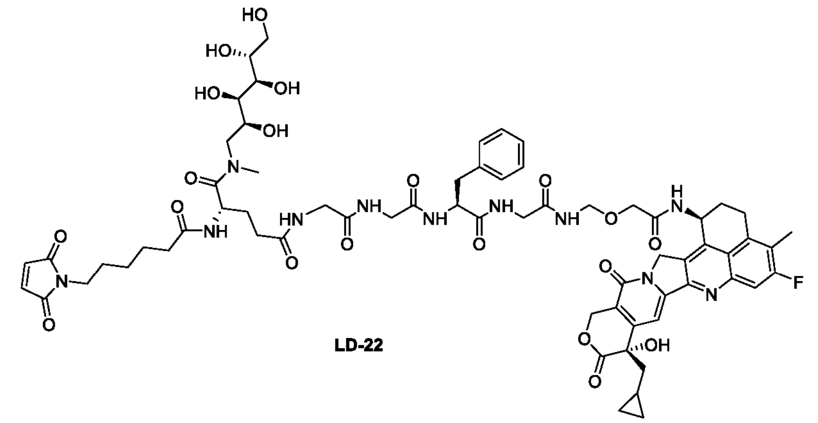

LD-22

Example 3-23: Preparation of LD-23

(*S*)-*N*5-((2*S*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-methyl-1,6,9,12,15-pentaoxo-3-oxa-5,8,11,14-tetraazahexadecan-16-yl)-2-(6-(2,5-dioxo-2,5-dihydro-1*H*-pyrrol-1-yl)hexanamido)-*N*1-methyl-*N*1-((2*S*,3*R*,4*R*,5*R*)-2,3,4,5,6-pentahydroxyhexyl)pentanediamide **LD-23**

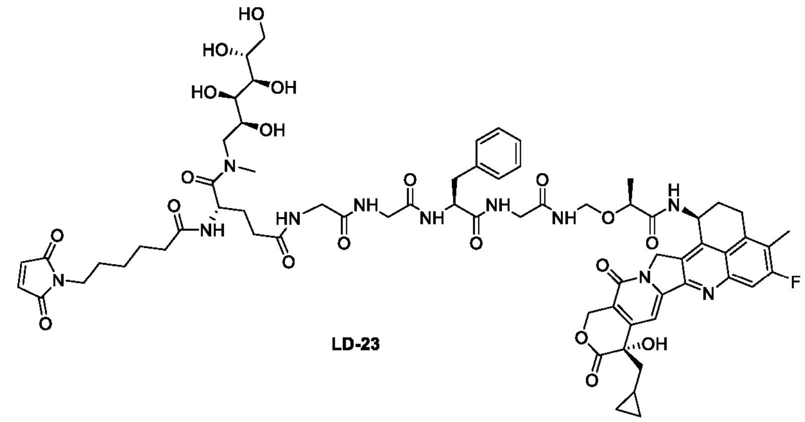

LD-23

Example 3-24: Preparation of LD-24

(*S*)-*N*⁵-((2*R*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-methyl-1,6,9,12,15-pentaoxo-3-oxa-5,8,11,14-tetraazahexadecan-16-yl)-2-(6-(2,5-dioxo-2,5-dihydro-1*H*-pyrrol-1-yl)hexanamido)-*N*¹-methyl-N¹-((2*S*,3*R*,4*R*,5*R*)-2,3,4,5,6-pentahydroxyhexyl)pentanediamide **LD-24**

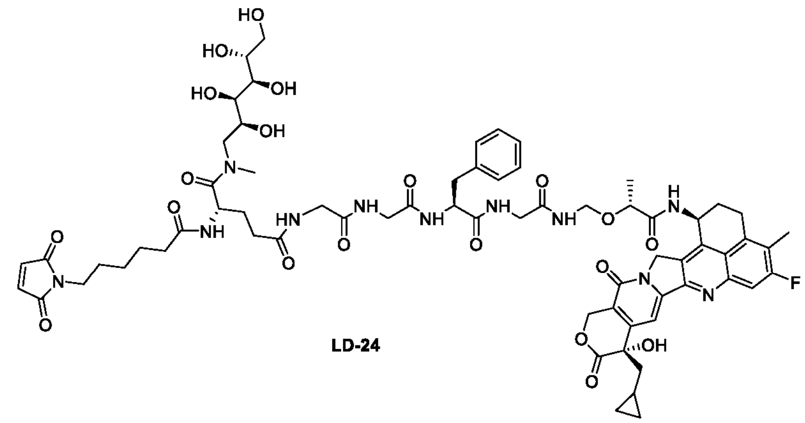

LD-24

Example 3-25: Preparation of LD-25

(*S*)-*N*⁵-((2*S*,10*S*)-10-Benzyl-2-cyclopropyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl) -2-(6-(2,5-dioxo-2,5-dihydro-1*H*-pyrrol-1-yl)hexanamido)-*N*¹-methyl-*N*¹-((2*S*,3*R*,4*R*,5*R*)-2,3,4,5,6-pentahydroxyhexyl)pentanediamide **LD-25**

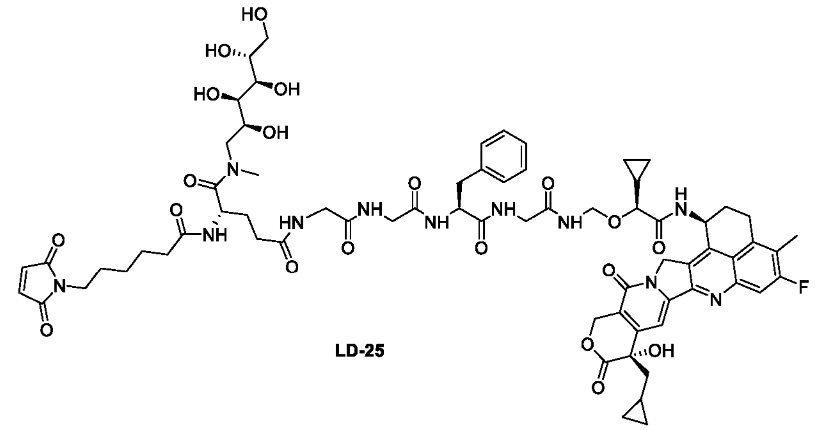

LD-25

Example 3-26: Preparation of LD-26

(*S*)-*N*[5]-((2*R*,10*S*)-10-Benzyl-2-cyclopropyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-y l)-2-(6-(2,5-dioxo-2,5-dihydro-1*H*-pyrrol-1-yl)hexanamido)-*N*[1]-methyl-*N*[1]-((2*S*,3*R*,4*R*,5*R*)-2,3,4,5,6-pentahydroxyhexyl)pentanediamide **LD-26**

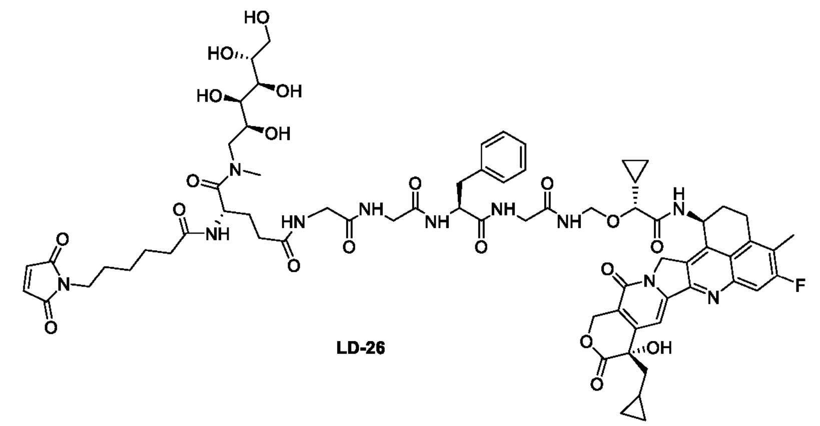

LD-26

Example 3-27: Preparation of LD-27

2-(((*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-*N*[1],*N*[3]-di(2,5,8,1,11,14,17,20,23-octaoxapentacosan-25-yl)isophthalamide **LD-27**

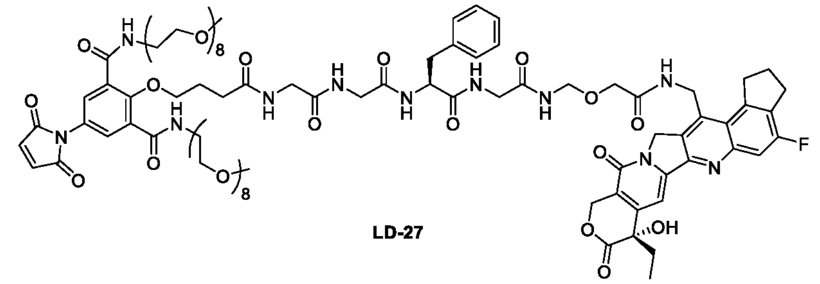

LD-27

MS m/z (ESI): 959.5 [1/2(M+2)].

Example 3-28: Preparation of LD-28

2-(((4*S*,12*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4-methyl-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-$N^1$,$N^3$-di(2,5,8,11,14,17,20,23-octaoxapentacosan-25-yl)isophthalamide **LD-28**

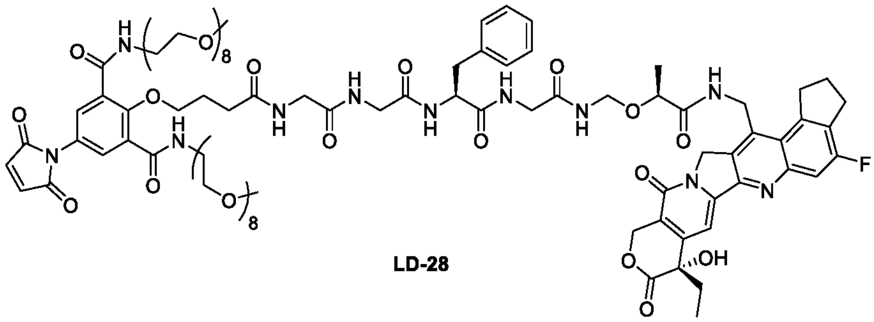

**LD-28**

MS m/z (ESI): 966.4 [1/2(M+2)].

Example 3-29: Preparation of LD-29

2-(((4*S*,12*S*)-12-Benzyl-4-cyclopropyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-$N^1$,$N^3$-di(2,5,8,11,14,17,20,23-octaoxapentacosan-25-yl)isophthalamide **LD-29**

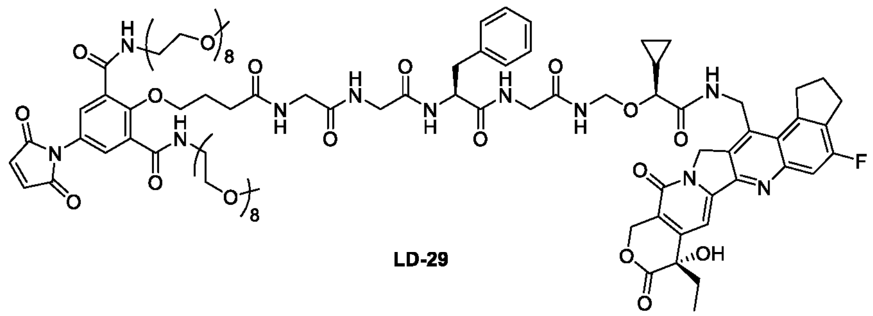

**LD-29**

Example 3-30: Preparation of LD-30

2-(((4*R*,12*S*)-12-Benzyl-4-cyclopropyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-$N^1$,$N^3$-di(2,5,8,11,14,17,20,23-octaoxapentacosan-25-yl)isophthalamide **LD-30**

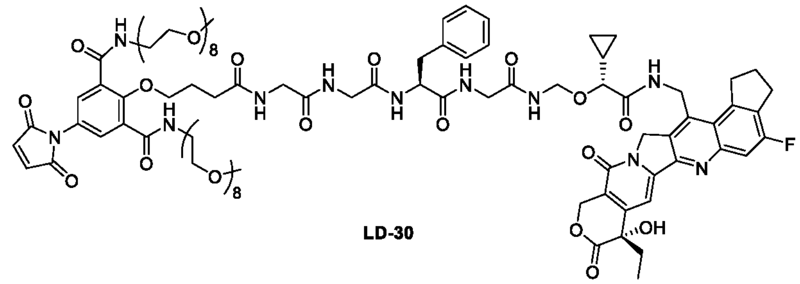

LD-30

Example 3-31: Preparation ofLD-31

2-(((*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-3-fluoro-*N*-(2,5,8,11,14,17,20,23-octaoxapentacosan-25-yl)benzamide **LD-31**

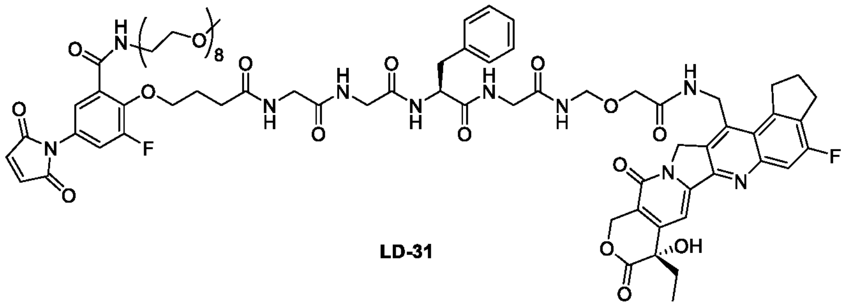

LD-31

Example 3-32: Preparation ofLD-32

2-(((4*S*,12*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4-methyl-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-3-fluoro- *N*-(2,5,8,11,14,17,20,23-octaoxapentacosan-25-yl)benzamide **LD-32**

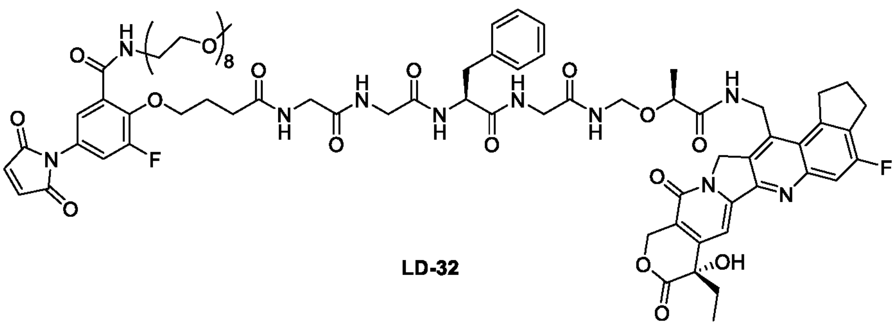

LD-32

Example 3-33: Preparation of LD-33

2-(((4*S*,12*S*)-12-Benzyl-4-cyclopropyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-3-fluoro-*N*-(2,5,8,11,14,17,20,23-octaoxapentacosan-25-yl)benzamide **LD-33**

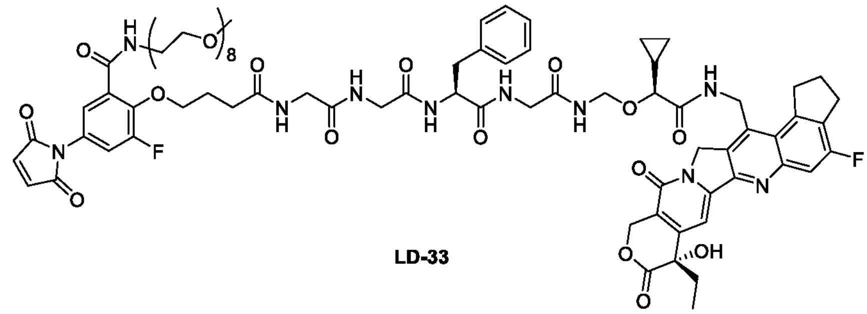

**LD-33**

Example 3-34: Preparation of LD-34

2-(((4*R*,12*S*)-12-Benzyl-4-cyclopropyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-3-fluoro-*N*-(2,5,8,11,14,17,20,23-octaoxapentacosan-25-yl)benzamide **LD-34**

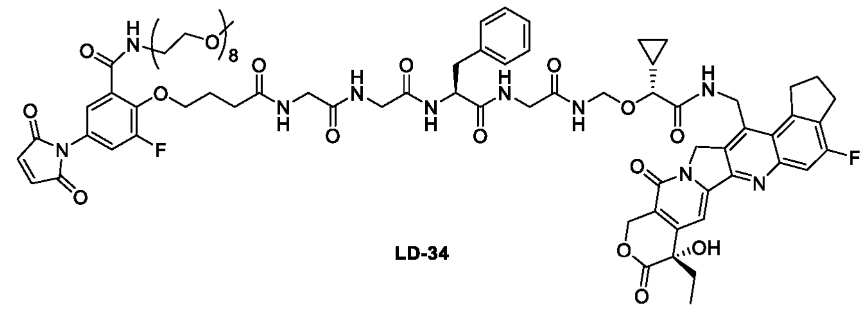

**LD-34**

Example 3-35: Preparation of LD-35

(*S*)-*N*$^5$-((*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17-pentaoxo-5-oxa-2,7,10,13,16-pentaazaoctadecan-18-yl)-2-(6-(2-(methylsulfonyl)pyrimidin-5-yl)hex-5-ynamido)-*N*$^1$-(2,5,8,11,14,17,20,23-octaoxapentacosan-25-yl)pentanediamide **LD-35**

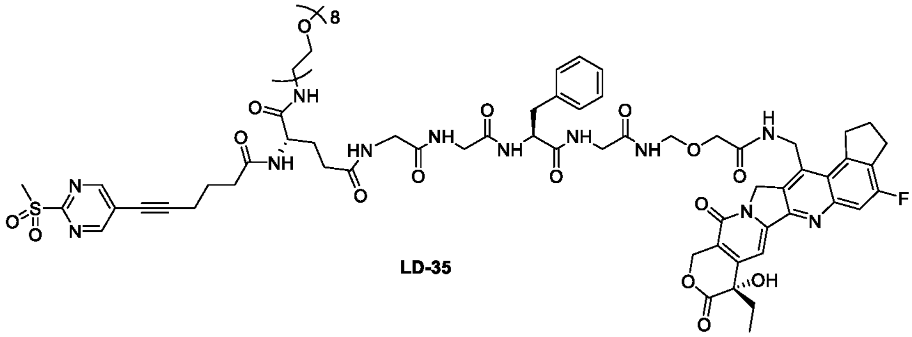

LD-35

MS m/z (ESI): 793.9 [1/2(M+2)].

Example 3-36: Preparation of LD-36

(*S*)-*N*[5]-((4*S*,12*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4-methyl-3,8,11,14,17-pentaoxo-5-oxa-2,10,13,16-pentaazaoctadecan-18-yl)-2-(6-(2-(methylsulfonyl)pyrimidin-5-yl)hex-5-ynamido)-*N*[1]-(2,5,8,11,14,17,20,23-octaoxapentacosan-25-yl)pentanediamide **LD-36**

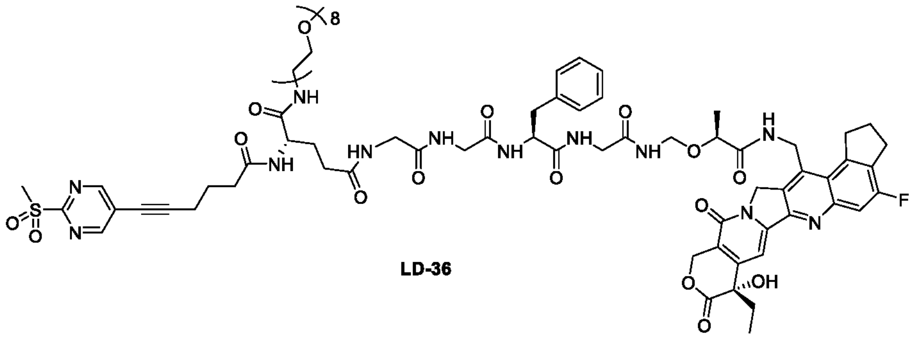

LD-36

MS m/z (ESI): 800.8 [1/2(M+2)].

Example 3-37: Preparation of LD-37

(*S*)-*N*[5]-((4*S*,12*S*)-12-Benzyl-4-cyclopropyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17-pentaoxo-5-oxa-2,7,10,13,16-pentaazaoctadecan-18-yl)-2-(6-(2-(methylsulfonyl)pyri midin-5-yl)hex-5-ynamido)-*N*[1]-(2,5,8,11,14,17,20,23-octaoxapentacosan-25-yl)pentanediamide **LD-37**

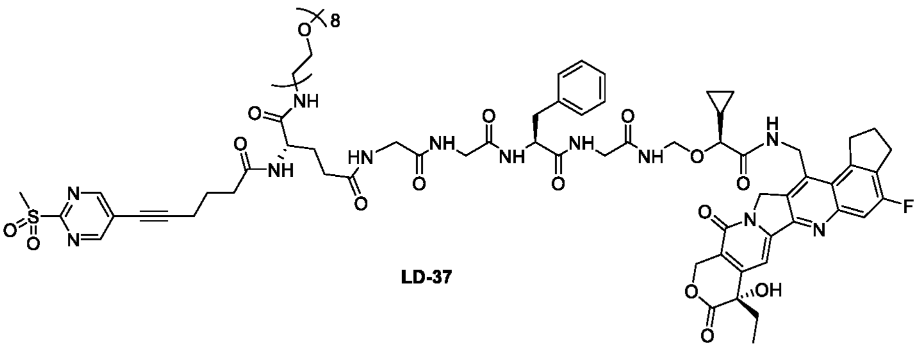

LD-37

Example 3-38: Preparation of LD-38

(*S*)-*N*5-((*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17-pentaoxo-5-oxa-2,7,10,13,16-pentaazaoctadecan-18-yl)-2-(6-(2,5-dioxo-2,5-dihydro-1*H*-pyrrol-1-yl)hexanamido)-*N*1-(2,5,8,11,14,17,20,23-octaoxapentacosan-25-yl)pentanediamide **LD-38**

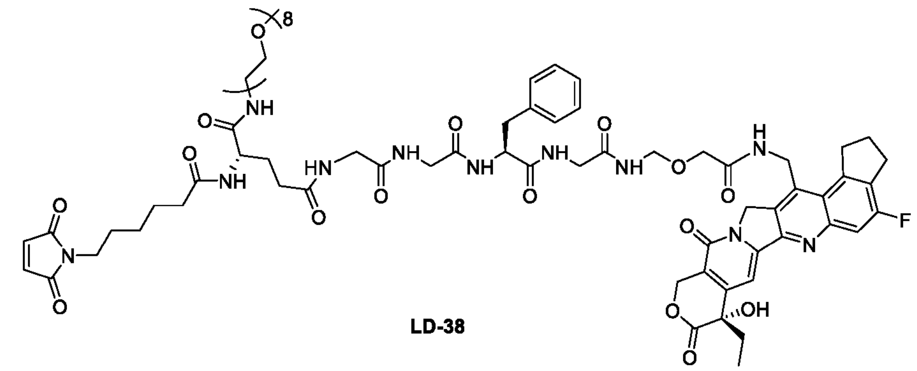

LD-38

Example 3-39: Preparation of LD-39

(*S*)-*N*5-((4*S*,12*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4-methyl-3,8,11,14,17-pentaoxo-5-oxa-2,7,10,13,16-pentaazaoctadecan-18-yl)-2-(6-(2,5-dioxo-2,5-dihydro-1*H*-p yrrol-1-yl)hexanamido)-*N*1-(2,5,8,11,14,17,20,23-octaoxapentacosan-25-yl)pentanediamide **LD-39**

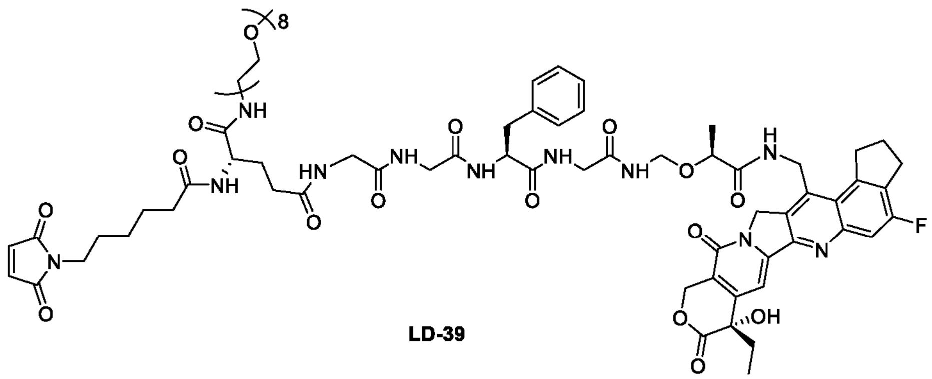

LD-39

Example 3-40: Preparation of LD-40

(*S*)-*N*5-((4S,12*S*)-12-Benzyl-4-cyclopropyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17-pentaoxo-5-oxa-2,7,10,13,16-pentaazaoctadecan-18-yl)-2-(6-(2,5-dioxo-2,5-dihydro-1*H*-pyrrol-1-yl)hexanamido)-*N*1-(2,5,8,11,14,17,20,23-octaoxapentacosan-25-yl)pentanediamide **LD-40**

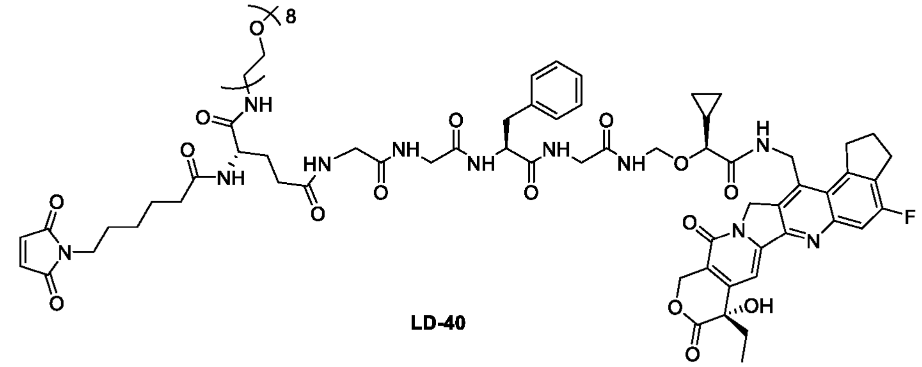

**LD-40**

Example 3-41: Preparation of LD-41

(*S*)-*N*[5]-((4*R*,12*S*)-12-Benzyl-4-cyclopropyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17-pentaoxo-5-oxa-2,7,10,13,16-pentazaoctadecan-18-yl)-2-(6-(2,5-dioxy-2,5-dihydro-1 *H*-pyrrol-1-yl)hexanamido)-*N*[1]-(2,5,8,11,14,17,20,23-octaoxapentacosan-25-yl)pentanediamide **LD-41**

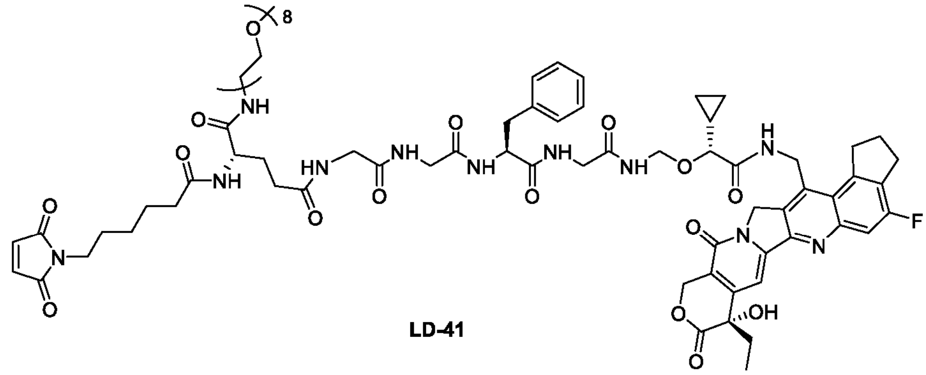

**LD-41**

Example 3-42: Preparation of LD-42

(*S*)-*N*[5]-((*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17-pentaoxo-5-oxa-2,7,10,13,16-pentaazaoctadecan-18-yl)-2-(6-(2,5-dioxo-2,5-dihydro-1*H*-pyrrol-1-yl)hexanamido)-*N*[1]-methyl-*N*[1]-((2*S*,3*R*,4*R*,5*R*)-2,3,4,5,6-pentahydroxyhexyl)pentanediamide **LD-42**

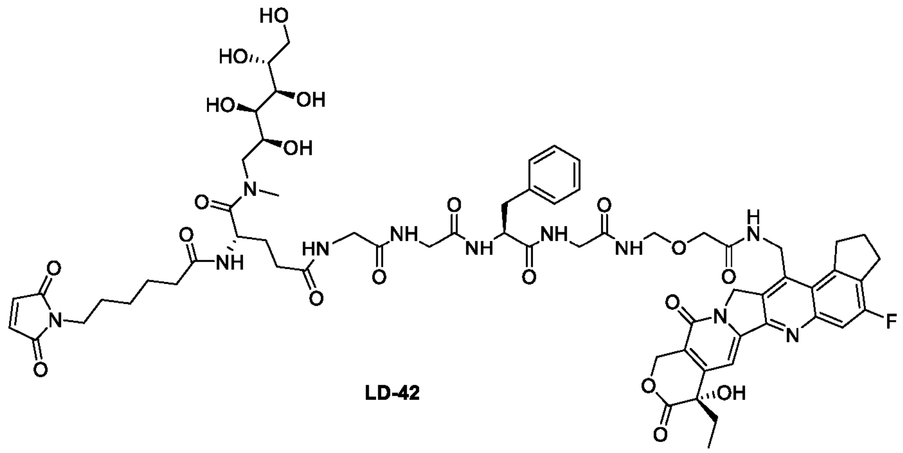

**LD-42**

Example 3-43: Preparation of LD-43

(*S*)-*N*[5]-((4*S*,12*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4-methyl-3,8,11,14,17-pentaoxo-5-oxa-2,7,10,13,16-pentaazaoctadecan-18-yl)-2-(6-(2,5-dioxo-2,5-dihydro-1*H*-p yrrol-1-yl)hexanamido)-*N*[1]-methyl-*N*[1]-((2*S*,3*R*,4*R*,5*R*)-2,3,4,5,6-pentahydroxyhexyl)pentanediamide **LD-43**

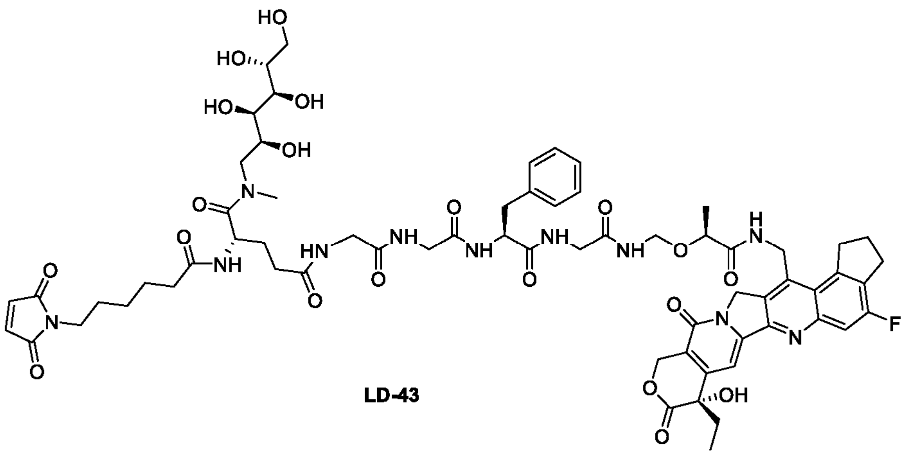

**LD-43**

Examples 3-44: LD-44

(*S*)-*N*[5]-((4*S*,12*S*)-12-Benzyl-4-cyclopropyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17-pentaoxo-5-oxa-2,7,10,13,16-pentaazaoctadecan-18-yl)-2-(6-(2,5-dioxo-2,5-dihydro-1*H*-pyrrol-1-yl)hexanamido)-*N*[1]-methyl-*N*[1]-((2*S*,3*R*,4*R*,5*R*)-2,3,4,5,6-pentahydroxyhexyl)pentanediamide **LD-44**

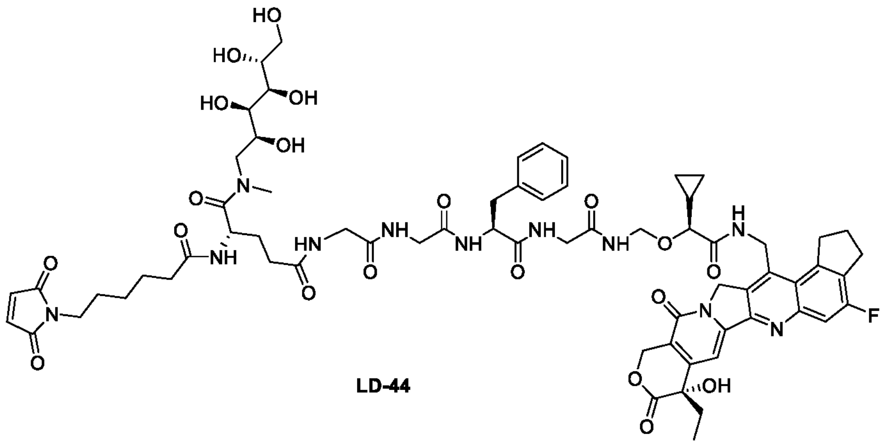

LD-44

Example 3-45: LD-45

2-(((2*S*,13*S*)-13-Benzyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18,21-hexaoxo-6-oxa-3,8,11,14,17,20-hexaazatetracosan-24-yl)oxy)-5-(2,5-dioxo-2,5-dihydro-1*H*-pyrrol-1-yl)-*N1*,*N3*-di(2,5,8,11,14,17,20,23-octaoxapentacosan-25-yl)isophthalamide **LD-45**

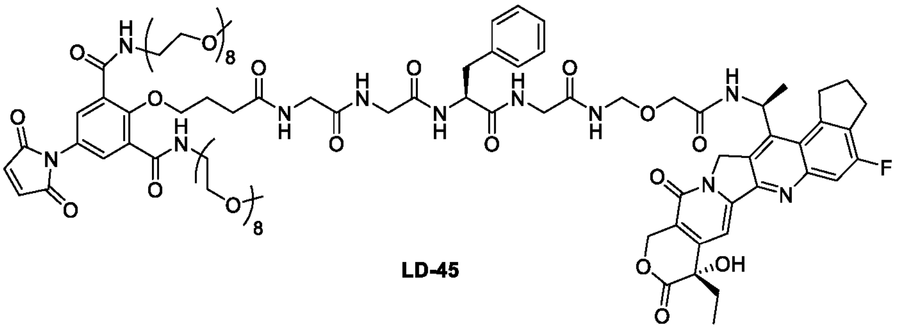

LD-45

Example 3-46: LD-46

2-(((2*S*,5*R*,13*S*)-13-Benzyl-5-cyclopropyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18,21-hexaoxo-6-oxa-3,8,11,14,17,20-hexaazatetracosan-24-yl)oxy)-5-(2,5-dioxo-2,5-dihydro-1*H*-pyrrol-1-yl)-*N1*,*N3*-di(2,5,8,11,14,17,20,23-octaoxapentacosan-25-yl)isophthalamide **LD-46**

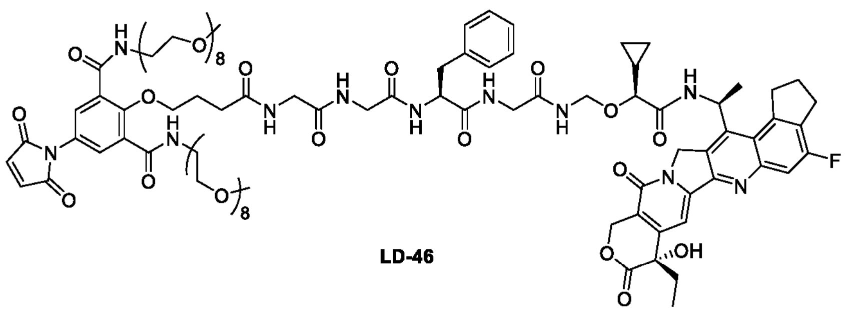

LD-46

Example 3-47: LD-47

2-(((2*S*,5*R*,13*S*)-13-Benzyl-5-cyclopropyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18,21-hexaoxo-6-oxa-3,8,11,14,17,20-hexaazatetracosan-24-yl)oxy)-5-(2,5-dioxo-2,5-dihydro-1*H*-pyrrol-1-yl)-*N¹,N³*-di(2,5,8,11,14,17,20,23-octaoxapentacosan-25-yl)isophthalamide **LD-47**

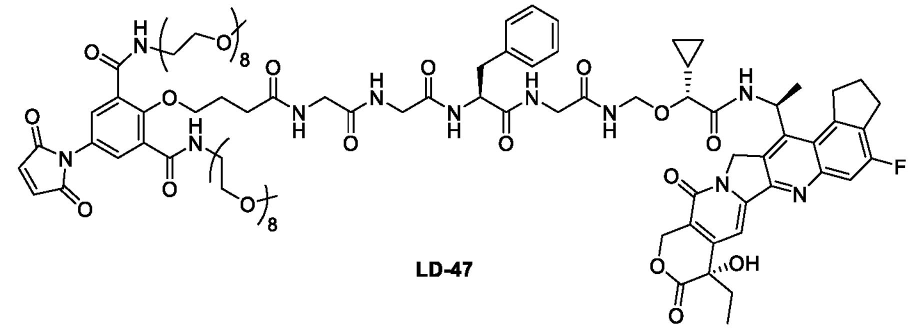

**LD-47**

Example 3-48: LD-48

2-(((2*S*,13*S*)-13-Benzyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18,21-hexaoxo-6-oxa-3,8,11,14,17,20-hexaazatetracosan-24-yl)oxy)-5-(2,5-dioxo-2,5-dihydro-1*H*-pyrrol-1-yl)-3-fluoro-*N*-(2,5,8,11,14,17,20,23-octaoxapentacosan-25-yl) benzamide

**LD-48**

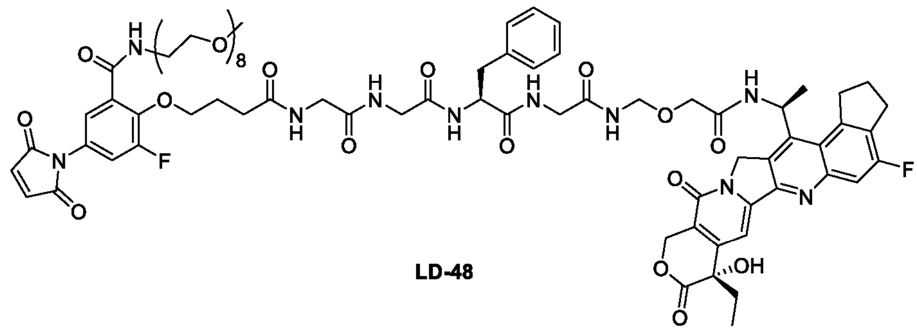

**LD-48**

Example 3-49: LD-49

2-(((2*S*,5*R*,13*S*)-13-Benzyl-5-cyclopropyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18,21-hexaoxo-6-oxa-3,8,11,14,17,20-hexaazatetracosan-24-yl)oxy)-5-(2,5-dioxo-2,5-dihydro-1*H*-pyrrol-1-yl)-3-fluoro-*N*-(2,5,8,11,14,17,20,23-octaoxapentacosan-25-yl)benzamide **LD-49**

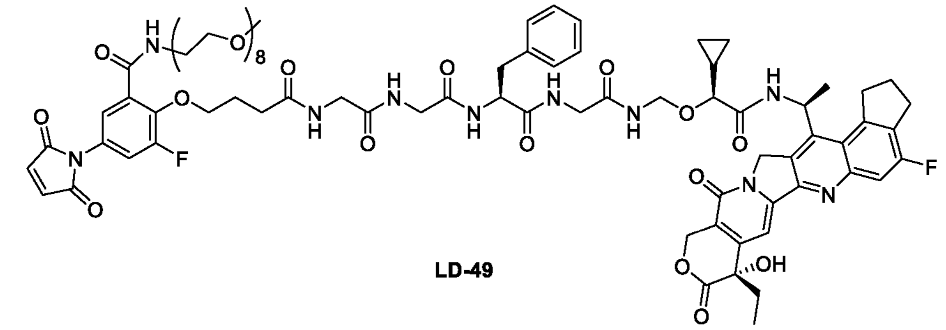

LD-49

Example 3-50: LD-50

2-(((2*S*,5*R*,13*S*)-13-Benzyl-5-cyclopropyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18,21-hexaoxo-6-oxa-3,8,11,14,17,20-hexaazatetracosan-24-yl)oxy)-5-(2,5-dioxo-2,5-dihydro-1*H*-pyrrol-1-yl)-3-fluoro-*N*-(2,5,8,11,14,17,20,23-octaoxapentacosan-25-yl)benzamide **LD-50**

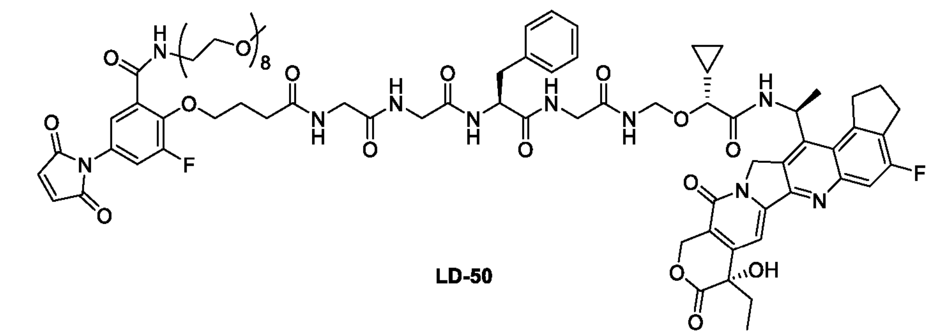

LD-50

Example 3-51: LD-51

(*S*)-*N*[5]-((2*S*,13*S*)-13-Benzyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18-pentaoxo-6-oxa-3,8,11,14,17-pentaazanonadecan-19-yl)-2-(6-(2-(methylsulfonyl)pyrimidin-5-yl)he x-5-ynamido)-*N*[1]-(2,5,8,11,14,17,20,23-octaoxapentacosan-25-yl)pentanediamide **LD-51**

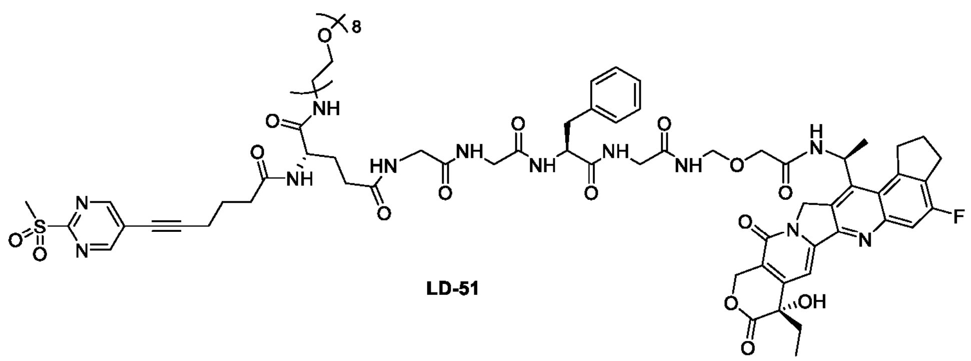

LD-51

Example 3-52: LD-52

(*S*)-*N*⁵-((2*S*,5*R*,13*S*)-13-Benzyl-5-cyclopropyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18-pentaoxo-6-oxa-3,8,11,14,17-pentaazanonadecan-19-yl)-2-(6-(2-(methylsulfonyl) pyrimidin-5-yl)hex-5-ynamido)-*N*¹-(2,5,8,11,14,17,20,23-octaoxapentacosan-25-yl)pentanediamide **LD-52**

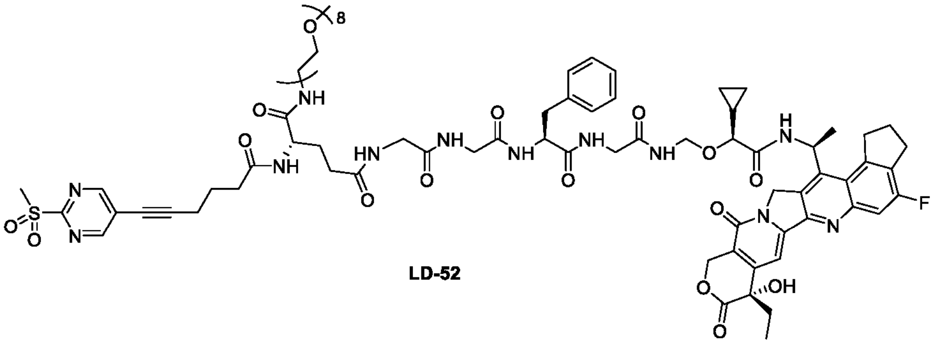

**LD-52**

Example 3-53: LD-53

(*S*)-*N*⁵-((2*S*,5*R*,13*S*)-13-Benzyl-5-cyclopropyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18-pentaoxo-6-oxa-3,8,11,14,17-pentaazanonadecan-19-yl)-2-(6-(2-(methylsulfonyl) pyrimidin-5-yl)hex-5-ynamido)-*N*¹-(2,5,8,11,14,17,20,23-octaoxapentacosan-25-yl)pentanediamide **LD-53**

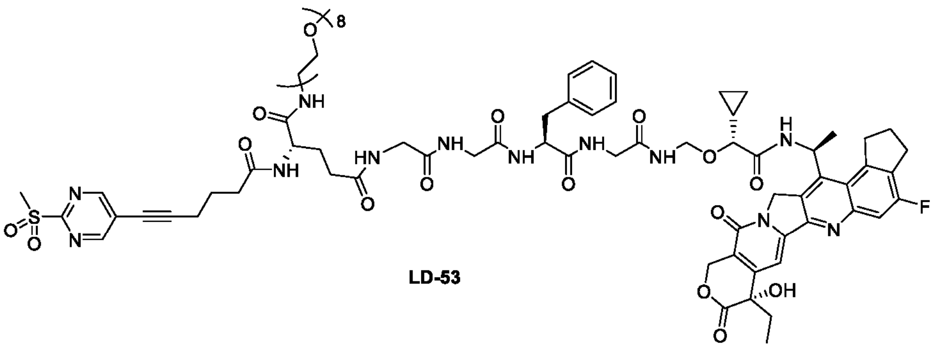

**LD-53**

Example 3-54: LD-54

(*S*)-*N*⁵-((2*S*,13*S*)-13-Benzyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18-pentaoxo-6-oxa-3,8,11,14,17-pentaazanonadecan-19-yl)-2-(6-(2,5-dioxo-2,5-dihydro-1*H*-pyrrol-1- yl)hexanamido)-*N*¹-(2,5,8,11,14,17,20,23-octaoxapentacosan-25-yl)pentanediamide **LD-54**

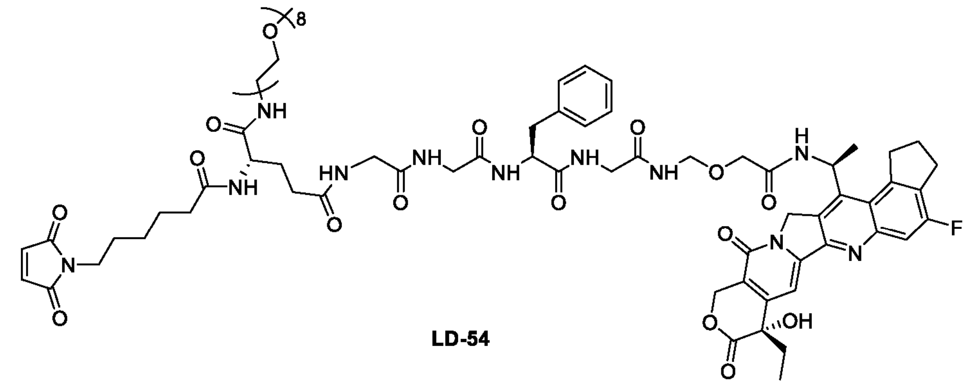

LD-54

Example 3-55: LD-55

(*S*)-*N*[5]-((2*S*,5*R*,13*S*)-13-Benzyl-5-cyclopropyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18-pentaoxo-6-oxa-3,8,11,14,17-pentaazanonadecan-19-yl)-2-(6-(2,5-dioxo-2,5-dihy dro-1*H*-pyrrol-1-yl)hexanamido)-*N*[1]-(2,5,8,11,14,17,20,23-octaoxapentacosan-25-yl)pentanediamide **LD-55**

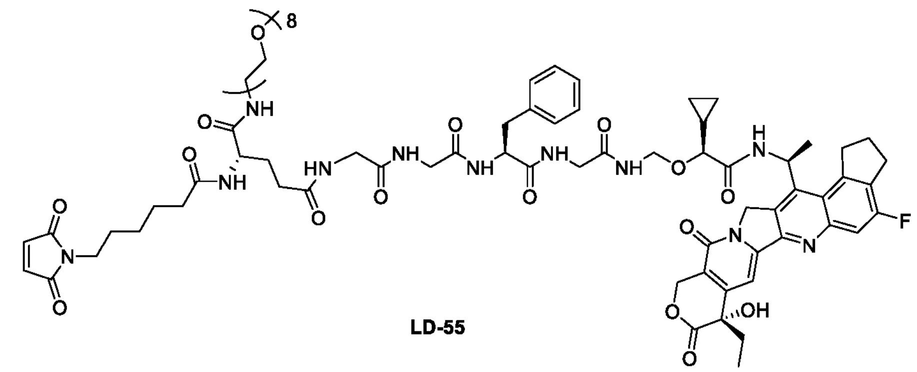

LD-55

Example 3-56: LD-56

(*S*)-*N*[5]-((2*S*,5*R*,13*S*)-13-Benzyl-5-cyclopropyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18-pentaoxo-6-oxa-3,8,11,14,17-pentaazanonadecan-19-yl)-2-(6-(2,5-dioxo-2,5-dihy dro-1*H*-pyrrol-1-yl)hexanamido)-*N*[1]-(2,5,8,11,14,17,20,23-octaoxapentacosan-25-yl)pentanediamide **LD-56**

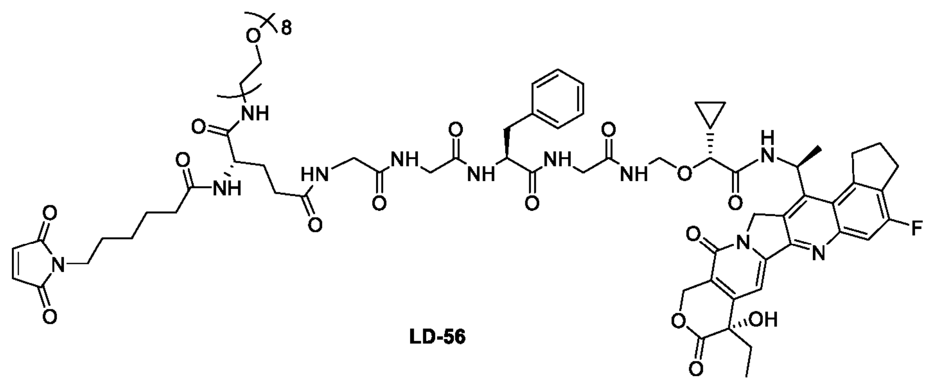

LD-56

Example 3-57: LD-57

(*S*)-*N*[5]-((2*S*,13*S*)-13-Benzyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18-pentaoxo-6-oxa-3,8,11,14,17-pentaazanonadecan-19-yl)-2-(6-(2,5-dioxo-2,5-dihydro-1*H*-pyrrol-1- yl)hexanamido)-*N*[1]-methyl-*N*[1]-((2*S*,3*R*,4*R*,5*R*)-2,3,4,5,6-pentahydroxyhexyl)pentanediamide **LD-57**

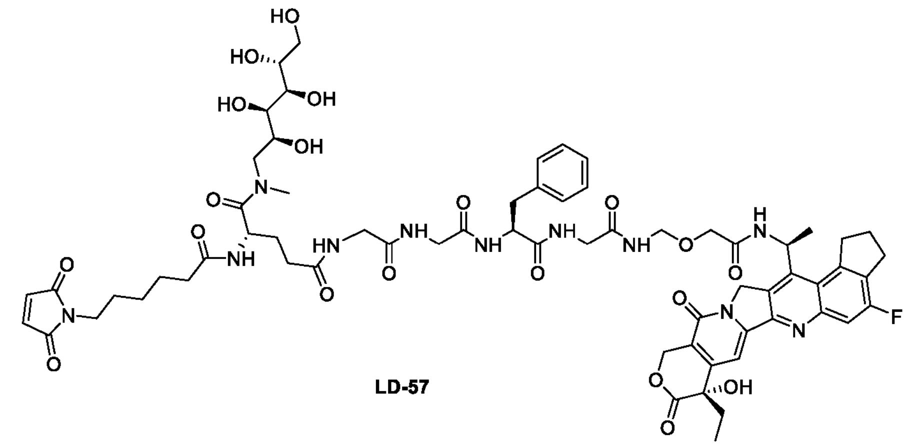

**LD-57**

Example 3-58: LD-58

(*S*)-*N*[5]-((2*S*,5*R*,13*S*)-13-Benzyl-5-cyclopropyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18-pentaoxo-6-oxa-3,8,11,14,17-pentaazanonadecan-19-yl)-2-(6-(2,5-dioxo-2,5-dihy dro-1*H*-pyrrol-1-yl)hexanamido)-*N*[1]-methyl-*N*[1]-((2*S*,3*R*,4*R*,5*R*)-2,3,4,5,6-pentahydroxyhexyl)pentanediamide **LD-58**

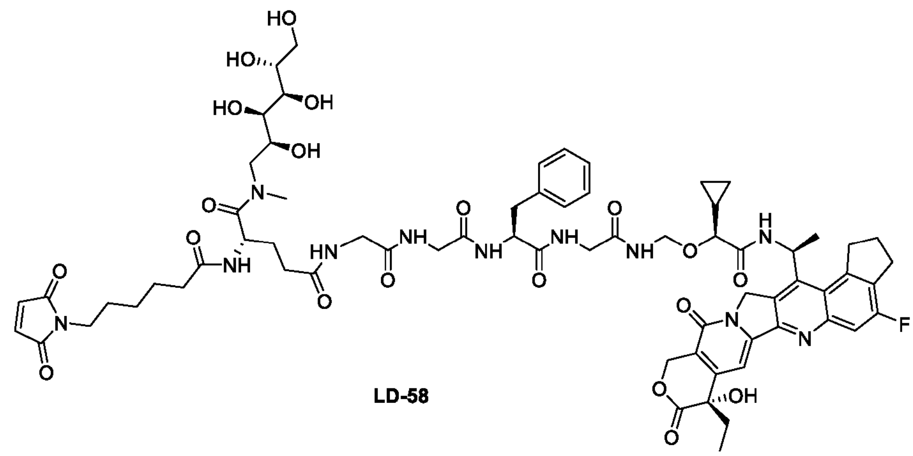

**LD-58**

Example 3-59: LD-59

(*S*)-*N*[5]-((2*S*,5*R*,13*S*)-13-Benzyl-5-cyclopropyl-2-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4,9,12,15,18-pentaoxo-6-oxa-3,8,11,14,17-pentaazanonadecan-19-yl)-2-(6-(2,5-dioxo-2,5-dihy dro-1*H*-pyrrol-1-yl)hexanamido)-*N*[1]-methyl-*N*[1]-((2*S*,3*R*,4*R*,5*R*)-2,3,4,5,6-pentahydroxyhexyl)pentanediamide **LD-59**

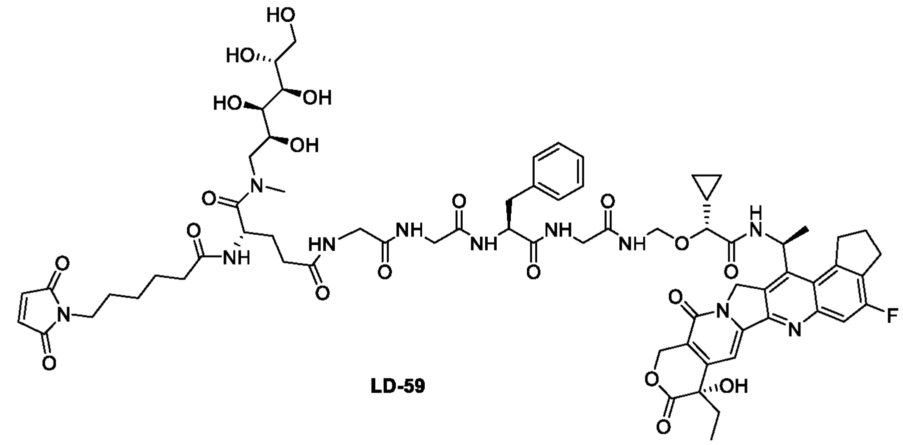

**LD-59**

Example 3-60: LD-60

2-(((S)-12-Benzyl-1-((S)-8-(cyclopropylmethyl)-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-*N*[1],*N*[3]-di(2,5,8,11,14,17,20,23-octaoxapentacosan-25-yl)isophthalamide **LD-60**

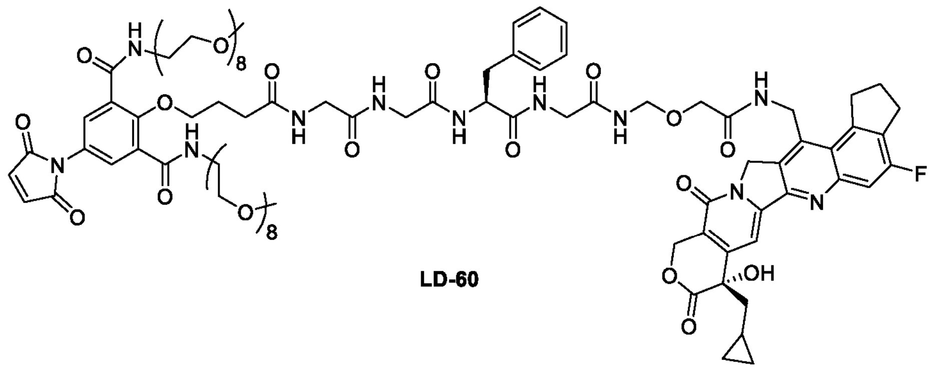

**LD-60**

Example 3-61: LD-61

2-(((*S*)-12-Benzyl-1-((*S*)-8-(cyclopropylmethyl)-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-3-fluoro-*N*-(2,5,8,11,14,17,20,23-octaoxapentacosan-25-yl) benzamide **LD-61**

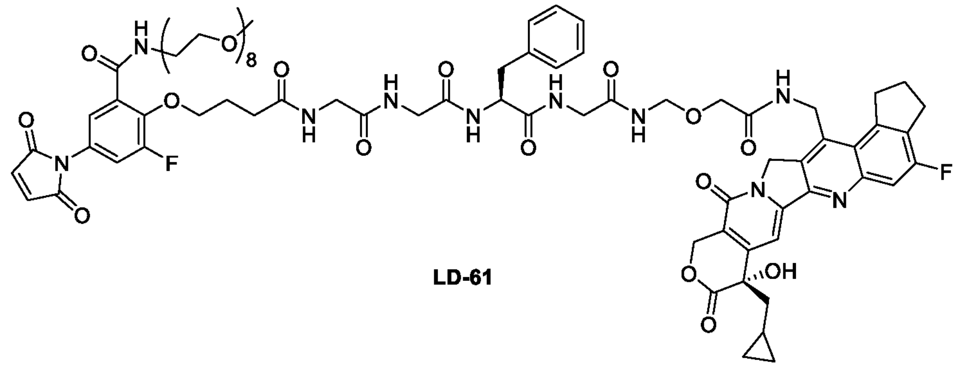

**LD-61**

Example 3-62: LD-62

(*S*)-*N*[5]-((*S*)-12-Benzyl-1-((*S*)-8-(cyclopropylmethyl)-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17-pentaoxo-5-oxa-2,7,10,13,16-pentaazaoctadecan-18-yl)-2-(6-(2-(methylsulfonyl)pyrimidin-5-yl)hex-5-ynamido)-*N*[1]-(2,5,8,11,14,17,20,23-octaoxapentacosan-25-yl)pentanediamide **LD-62**

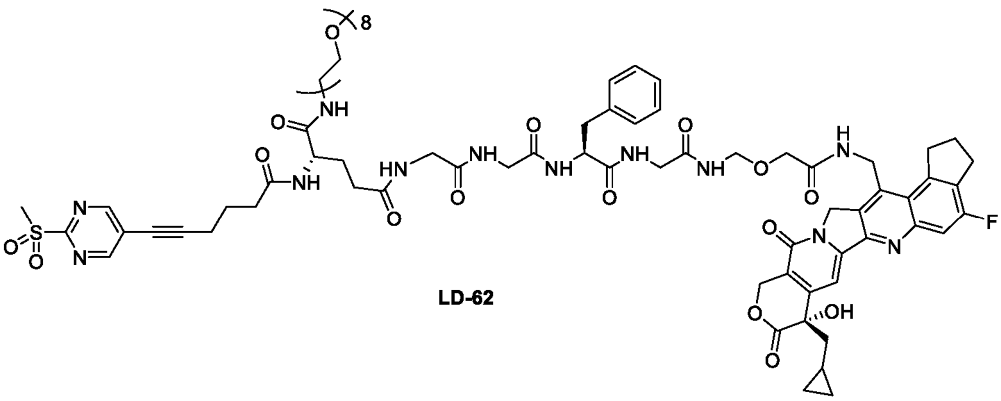

**LD-62**

Example 3-63: LD-63

(*S*)-*N*[5]-((*S*)-12-Benzyl-1-((*S*)-8-(cyclopropylmethyl)-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17-pentaoxo-5-oxa-2,7,10,13,16-pentaazaoctadecan-18-yl)-2-(6-(2,5-dioxo-2,5-dihydro-1*H*-p yrrol-1-yl)hexanamido)-*N*[1]-(2,5,8,11,14,17,20,23-octaoxapentacosan-25-yl)pentanediamide **LD-63**

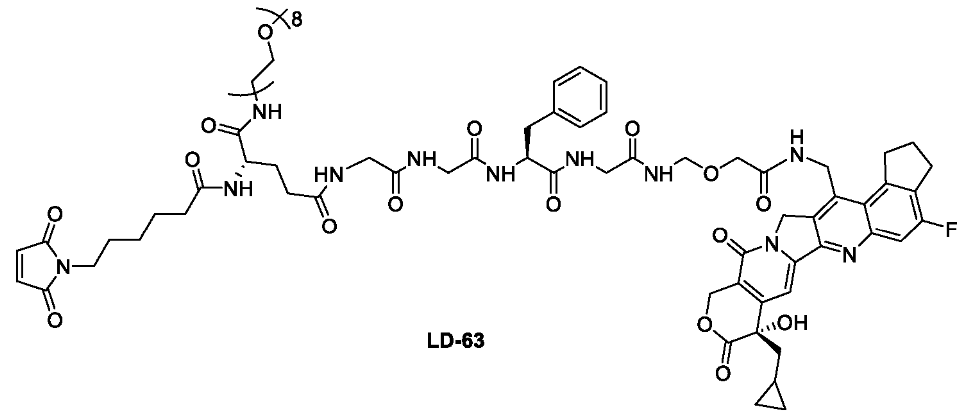

**LD-63**

Example 3-64: LD-64

(*S*)-*N*[5]-((*S*)-12-Benzyl-1-((*S*)-8-(cyclopropylmethyl)-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-3,8,11,14,17-pentaoxo-5-oxa-2,7,10,13,16-pentaazaoctadecan-18-yl)-2-(6-(2,5-dioxo-2,5-dihydro-1*H*-p yrrol-1-yl)hexanamido)-*N*[1]-methyl-*N*[1]-((2*S*,3*R*,4*R*,5*R*)-2,3,4,5,6-pentahydroxyhexyl)pentanediamide **LD-64**

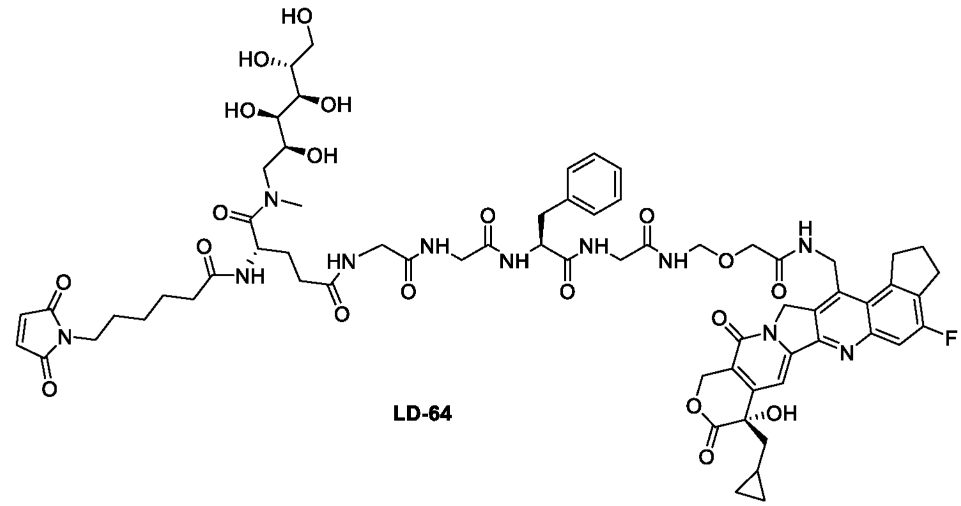

**LD-64**

Example 3-65: LD-65

2-(((*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3, 8,11,14,17,20-hexaoxo-5-oxa-2,7,10,13,16,19-hexaazatricosan-23-yl) oxy)-5-(2,5-dioxo-2,5-dihydro-1*H*-pyrrol-1-yl)-*N*[1],*N*[3]-di(2,5,8,11,14,17,20,23-octaoxapentacosan-25-yl)isophthalamide **LD-65**

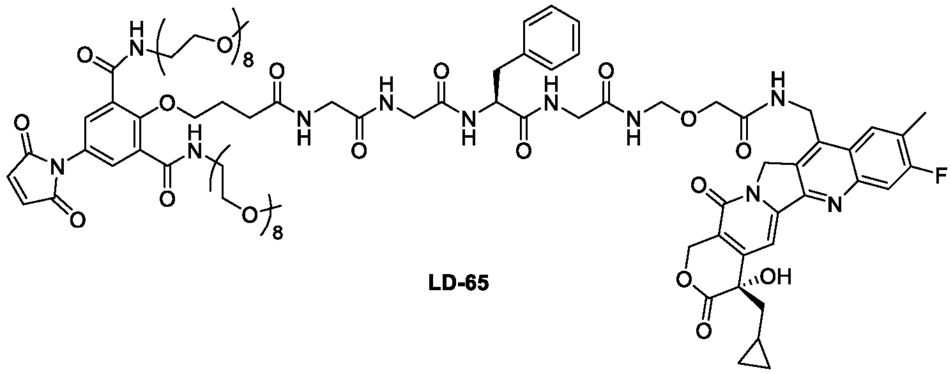

**LD-65**

Example 3-66: LD-66

2-(((4*S*,12*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-4-methyl-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-*N1,N3*-di(2,5,8,11,14,17,20,23-octaoxapentacosan-25-yl) isophthalamide **LD-66**

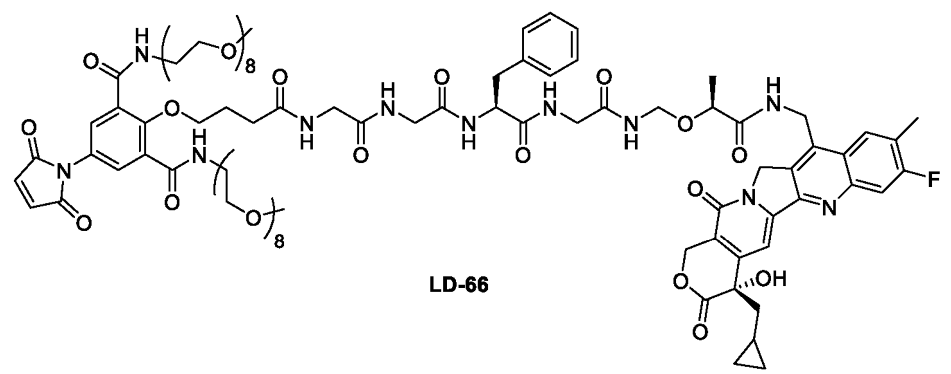

**LD-66**

Example 3-67: LD-67

2-(((4*R*,12*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-4-methyl-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-*N1,N3*-di(2,5,8,11,14,17,20,23-octaoxapentacosan-25-yl) isophthalamide **LD-67**

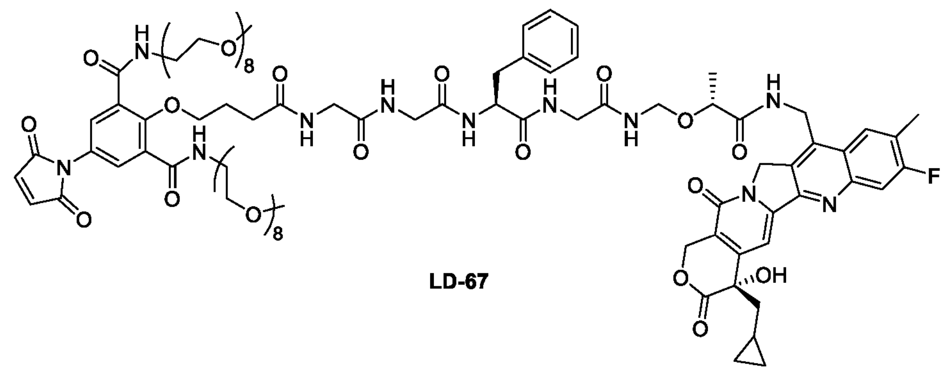

**LD-67**

Example 3-68: LD-68

2-(((4*S*,12*S*)-12-Benzyl-4-cyclopropyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-*N1,N3*-di(2,5,8,11,14,17,20,23-octaoxapentacosan-25-yl)isophthalamide **LD-68**

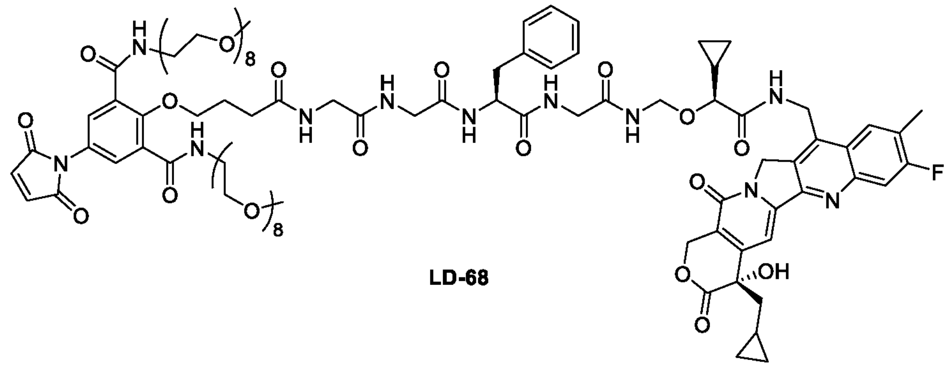

**LD-68**

Example 3-69: LD-69

2-(((4*R*,12*S*)-12-Benzyl-4-cyclopropyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-*N1,N3*-di(2,5,8,11,14,17,20,23-octaoxapentacosan-25-yl)isophthalamide **LD-69**

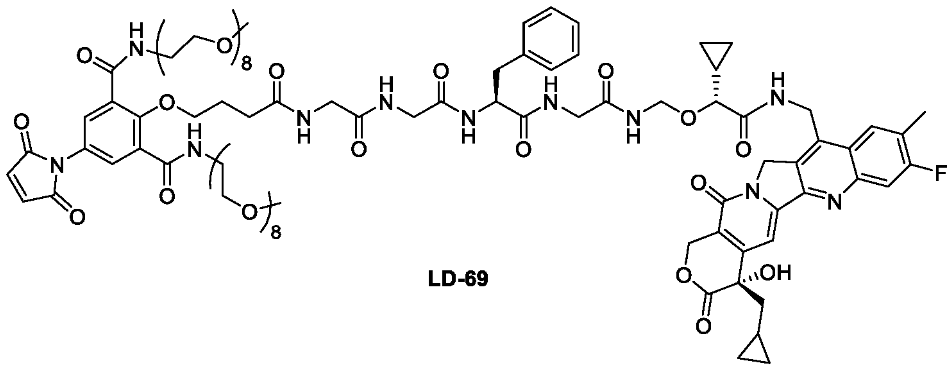

**LD-69**

Example 3-70: LD-70

2-(((*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-3-fluoro-*N*-(2,5,8,11,14,17,20,23-octaoxapentacosan-25-yl) benzamide **LD-70**

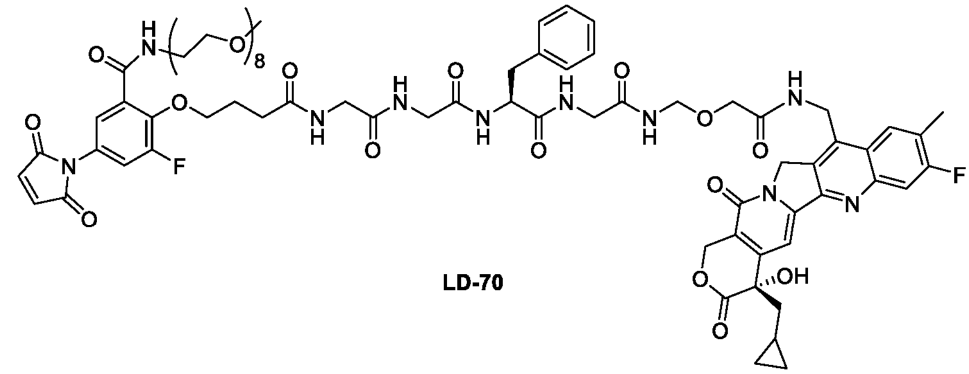

**LD-70**

Example 3-71: LD-71

2-(((4*S*,12*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-4-methyl-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-3-fluoro-*N*-(2,5,8,11,14,17,20,23-octaoxapentacosan-25-yl) benzamide **LD-71**

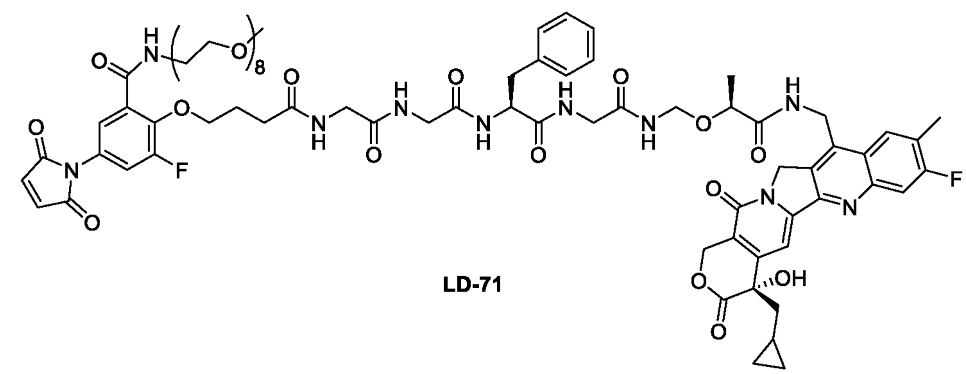

**LD-71**

Example 3-72: LD-72

2-(((4*R*,12*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-4-methyl-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-3-fluoro-*N*-(2,5,8,11,14,17,20,23-octaoxapentacosan-25-yl) benzamide **LD-72**

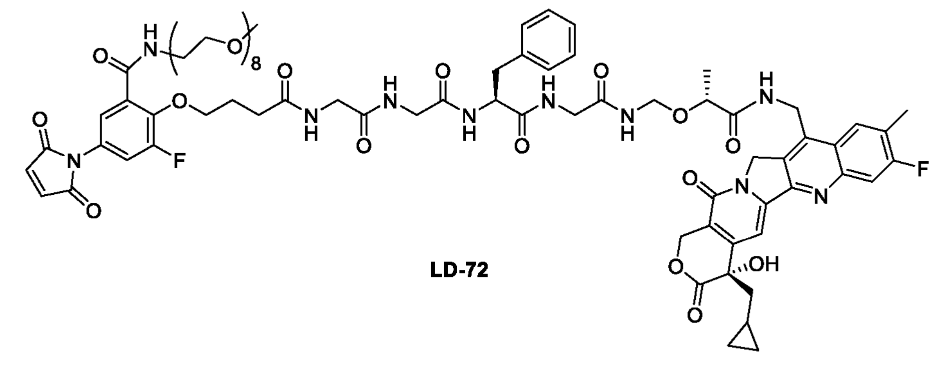

**LD-72**

Example 3-73: LD-73

2-(((4*S*,12*S*)-12-Benzyl-4-cyclopropyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-3-fluoro-*N*-(2,5,8,11,14,17,20,23-octaoxapentacosan-25-yl)benzamide **LD-73**

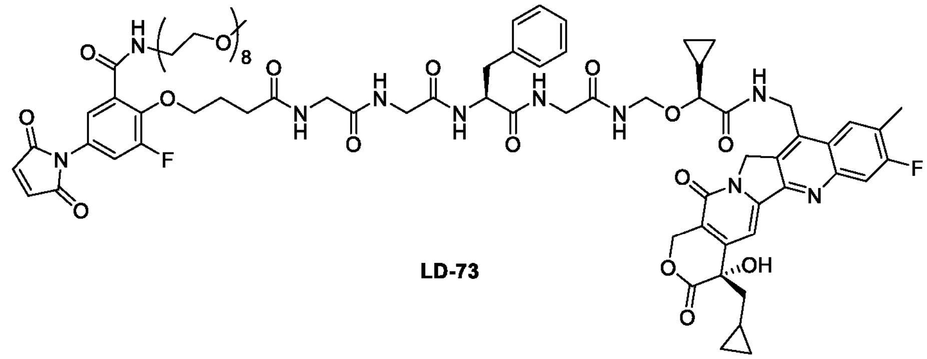

**LD-73**

Example 3-74: LD-74

2-(((4*R*,12*S*)-12-Benzyl-4-cyclopropyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17,20-hexaoxo-5-oxa-2,7,10,13,16,19-hexaazatricosan-23-yl)oxy)-5-(2,5-dioxo-2,5-dihydro-1*H*-pyrrol-1-yl)-3-fluoro-*N*-(2,5,8,11,14,17,20,23-octaoxapentacosan-25-yl)benzamide **LD-74**

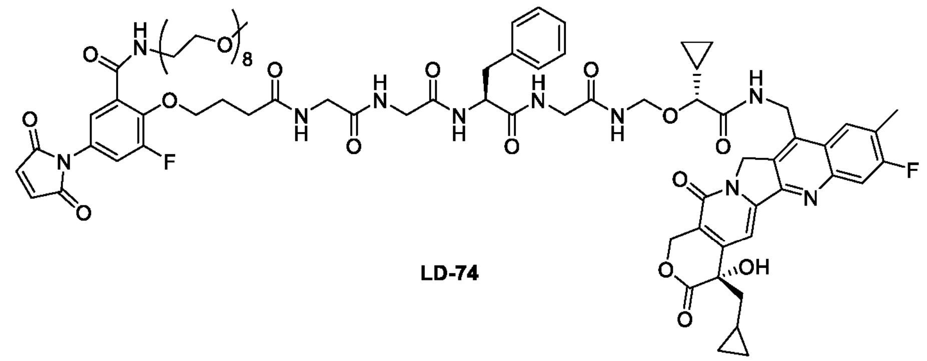

**LD-74**

Example 3-75: LD-75

(*S*)-*N*$^5$-((*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17-pentaoxo-5-oxa-2,7,10,13,16-pentaazaoctadecan-18-yl)-2-(6-(2-(methylsulfonyl)pyrimidin-5-yl)hex-5-ynamido)-*N*$^1$-(2,5,8,11,14,17,20,23-octaoxapentacosan-25-yl)pentanediamide **LD-75**

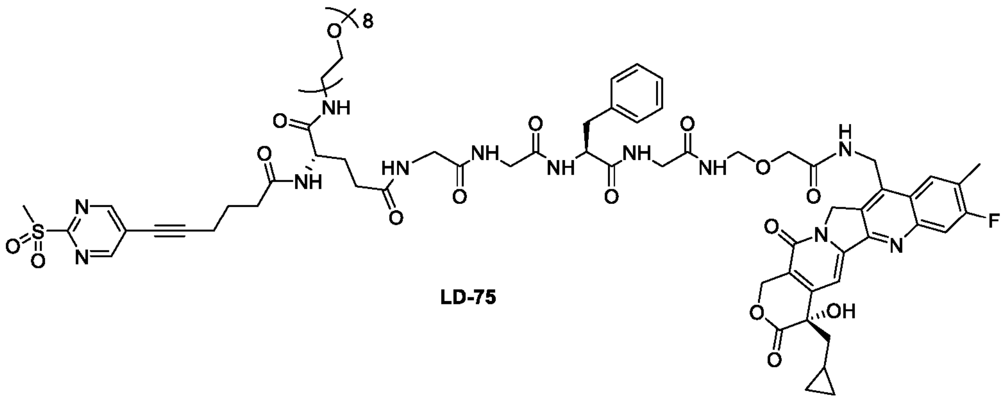

**LD-75**

Example 3-76: LD-76

(*S*)-*N*5-((4*S*,12*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-4-methyl-3,8,11,14,17-pentaoxo-5-oxa-2,7,10,13,16-pentaazaoctadecan-18-yl)-2-(6-(2-(methylsulfonyl)pyrimi din-5-yl)hex-5-ynamido)-*N*1-(2,5,8,11,14,17,20,23-octaoxapentacosan-25-yl)pentanediamide **LD-76**

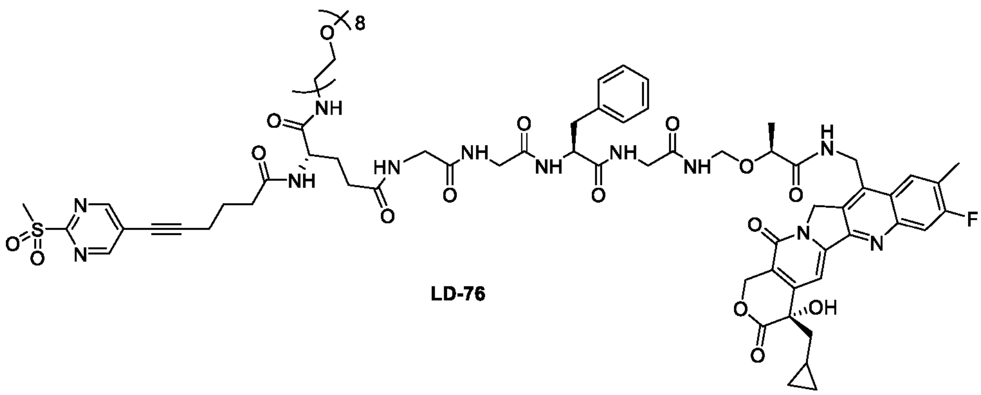

**LD-76**

Example 3-77: LD-77

(*S*)-*N*5-((4*R*,12*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-4-methyl-3,8,11,14,17-pentaoxo-5-oxa-2,7,10,13,16-pentaazaoctadecan-18-yl)-2-(6-(2-(methylsulfonyl)pyrimi din-5-yl)hex-5-ynamido)-*N*1-(2,5,8,11,14,17,20,23-octaoxapentacosan-25-yl)pentanediamide **LD-77**

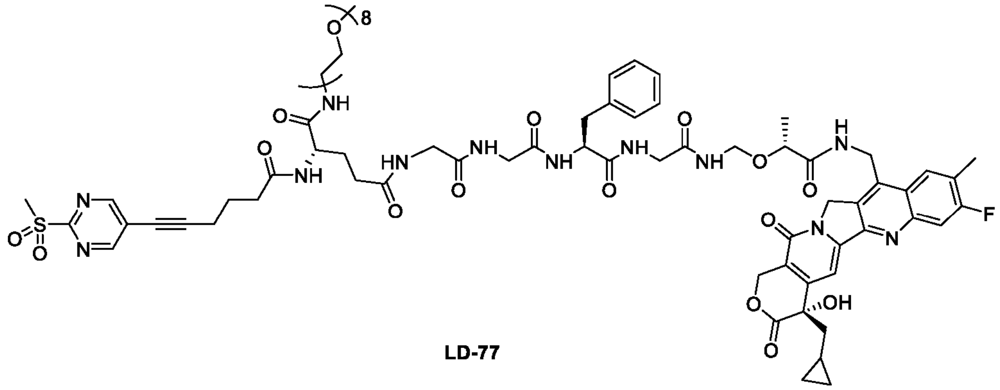

**LD-77**

Example 3-78: LD-78

(*S*)-*N*[5]-((4*S*,12*S*)-12-Benzyl-4-cyclopropyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17-pentaoxo-5-oxa-2,7,10,13,16-pentaazaoctadecan-18-yl)-2-(6-(2-(methylsulfonyl)p yrimidin-5-yl)hex-5-ynamido)-*N*[1]-(2,5,8,11,14,17,20,23-octaoxapentacosan-25-yl)pentanediamide **LD-78**

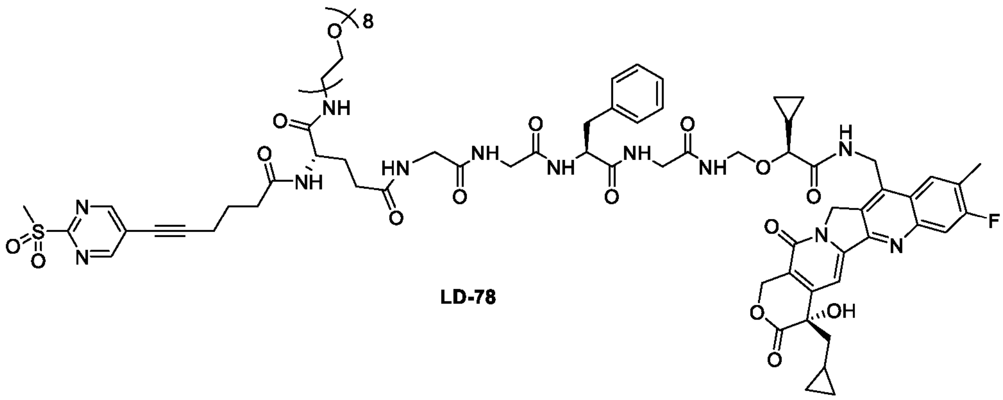

**LD-78**

Example 3-79: LD-79

(*S*)-*N*[5]-((4*S*,12*S*)-12-Benzyl-4-cyclopropyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17-pentaoxo-5-oxa-2,7,10,13,16-pentaazaoctadecan-18-yl)-2-(6-(2-(methylsulfonyl)p yrimidin-5-yl)hex-5-ynamido)-*N*[1]-(2,5,8,11,14,17,20,23-octaoxapentacosan-25-yl)pentanediamide **LD-79**

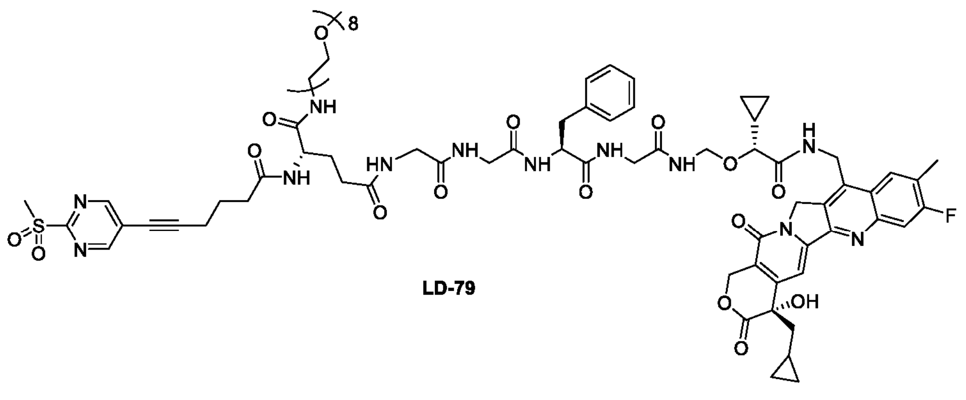

**LD-79**

Example 3-80: LD-80

(*S*)-*N*5-((*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17-penta oxo-5-oxa-2,7,10,13,16-pentaazaoctadecan-18-yl)-2-(6-(2,5-dioxo-2,5-dihydro-1*H*-pyrrol-1-yl)hexanamido)-*N*1-(2,5,8,11,14,17,20,23-octaoxapentacosan-25-yl)pentanediamide **LD-80**

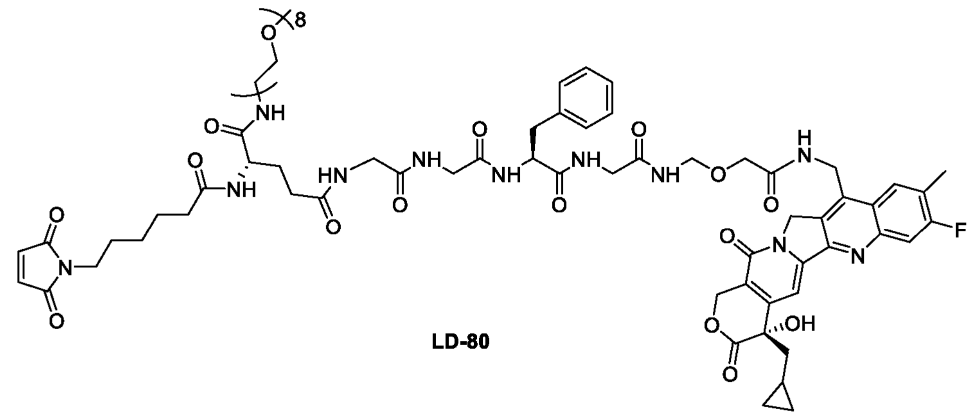

**LD-80**

Example 3-81: LD-81

(*S*)-*N*5-((4*S*,12*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-4-methyl-3,8,11,14,17-pentaoxo-5-oxa-2,7,10,13,16-pentaazaoctadecan-18-yl)-2-(6-(2,5-dioxo-2,5-dihydro-1 *H*-pyrrol-1-yl)hexanamido)-*N*1-(2,5,8,11,14,17,20,23-octaoxapentacosan-25-yl)pentanediamide **LD-81**

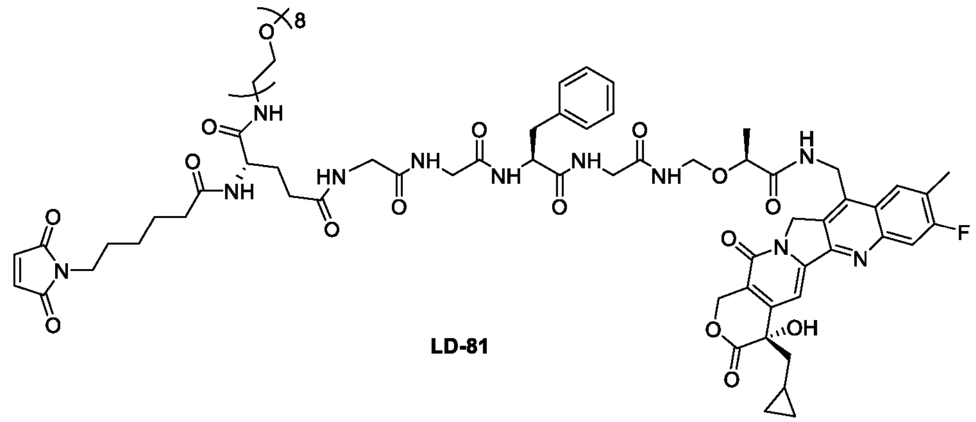

**LD-81**

Example 3-82: LD-82

(*S*)-*N*5-((4*S*,12*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-4-methyl-3,8,11,14,17-pentaoxo-5-oxa-2,7,10,13,16-pentaazaoctadecan-18-yl)-2-(6-(2,5-dioxo-2,5-dihydro-1 *H*-pyrrol-1-yl)hexanamido)-*N*1-(2,5,8,11,14,17,20,23-octaoxapentacosan-25-yl)pentanediamide **LD-82**

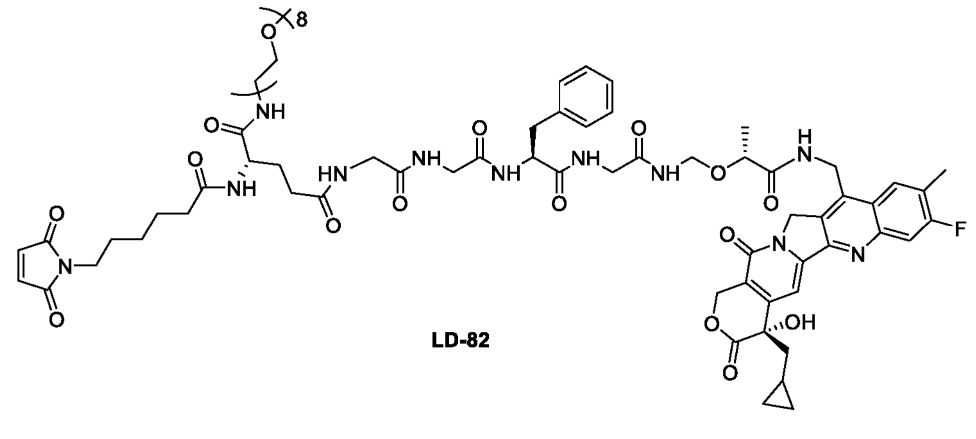

**LD-82**

Example 3-83: LD-83

(*S*)-$N^5$-((4*S*,12*S*)-12-Benzyl-4-cyclopropyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17-pentaoxo-5-oxa-2,7,10,13,16-pentaazaoctadecan-18-yl)-2-(6-(2,5-dioxo-2,5-dihyd ro-1*H*-pyrrol-1-yl)hexanamido)-$N^1$-(2,5,8,11,14,17,20,23-octaoxapentacosan-25-yl)pentanediamide **LD-83**

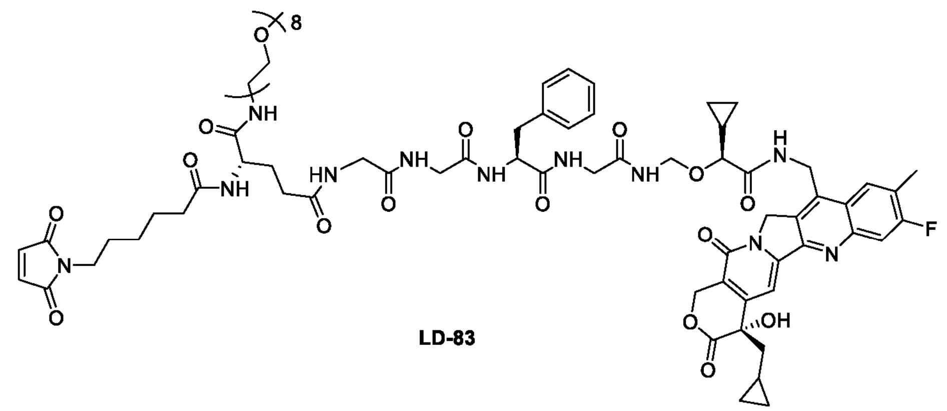

**LD-83**

Example 3-84: LD-84

(*S*)-$N^5$-((4*S*,12*S*)-12-Benzyl-4-cyclopropyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17-pentaoxo-5-oxa-2,7,10,13,16-pentaazaoctadecan-18-yl)-2-(6-(2,5-dioxo-2,5-dihyd ro-1*H*-pyrrol-1-yl)hexanamido)-$N^1$-(2,5,8,11,14,17,20,23-octaoxapentacosan-25-yl)pentanediamide **LD-84**

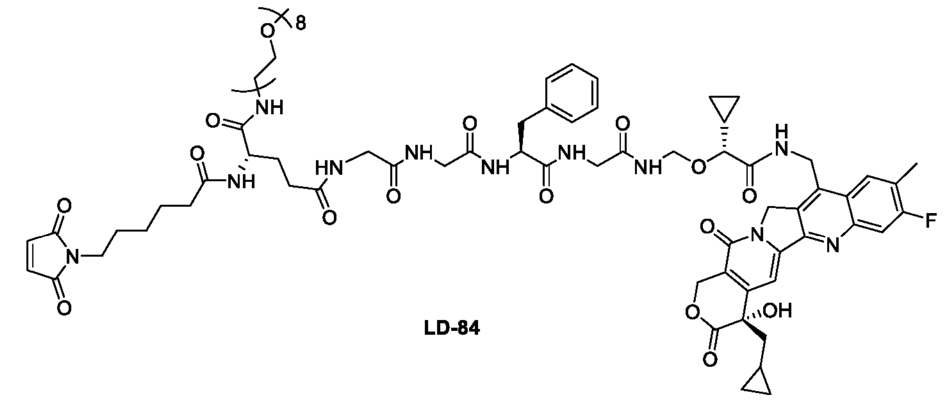

**LD-84**

Example 3-85: LD-85

(*S*)-*N*5-((*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17-pentaoxo-5-oxa-2,7,10,13,16-pentaazaoctadecan-18-yl)-2-(6-(2,5-dioxo-2,5-dihydro-1*H*-pyrrol-1-yl) hexanamido)-*N*1-methyl-*N*1-((2*S*,3*R*,4*R*,5*R*)-2,3,4,5,6-pentahydroxyhexyl)pentanediamide LD-85

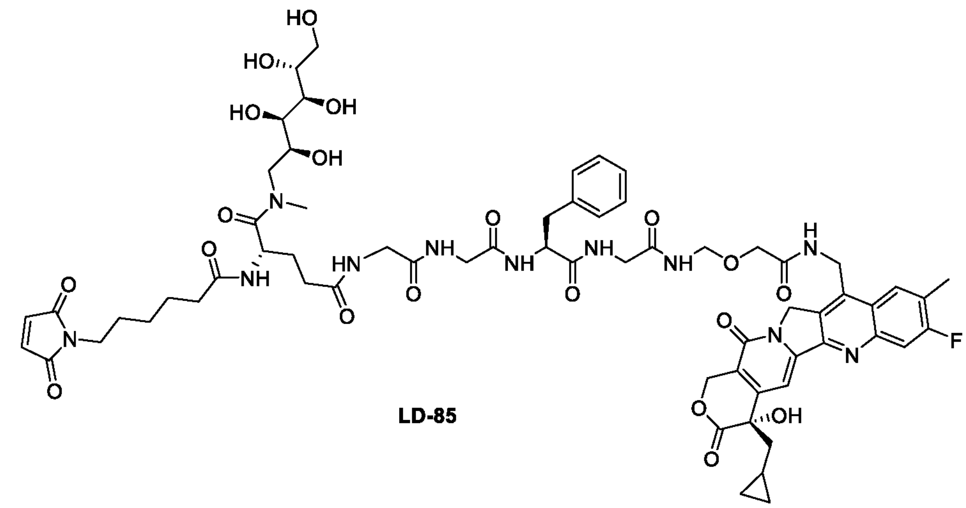

**LD-85**

Example 3-86 LD-86

(*S*)-*N*5-((4*S*,12*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-4-methyl-3,8,11,14,17-pentaoxo-5-oxa-2,7,10,13,16-pentaazaoctadecan-18-yl)-2-(6-(2,5-dioxo-2,5-dihydro-1 *H*-pyrrol-1-yl)hexanamido)-*N*1-methyl-*N*1-((2*S*,3*R*,4*R*,5*R*)-2,3,4,5,6-pentahydroxyhexyl)pentanediamide **LD-86**

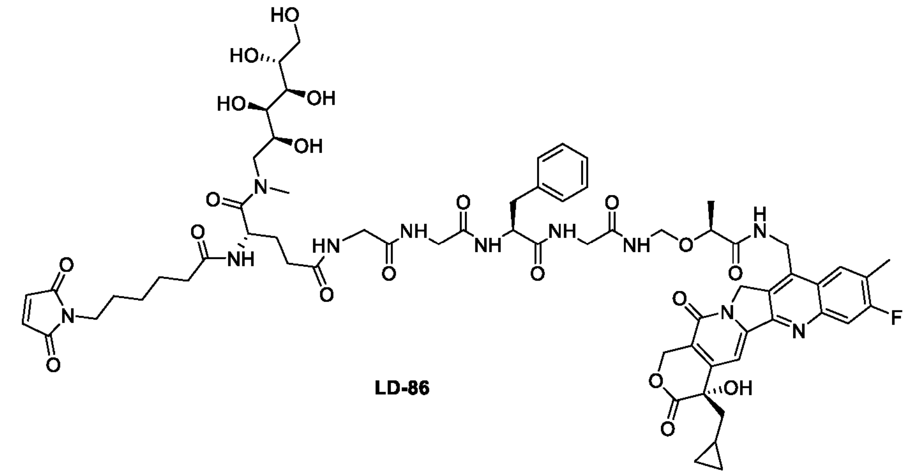

LD-86

Example 3-87: LD-87

(*S*)-*N*[5]-((4*S*,12*S*)-12-Benzyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-4-methyl-3,8,11,14,17-pentaoxo-5-oxa-2,7,10,13,16-pentaazaoctadecan-18-yl)-2-(6-(2,5-dioxo-2,5-dihydro-1 *H*-pyrrol-1-yl)hexanamido)-*N*[1]-methyl-*N*[1]-((2*S*,3*R*,4*R*,5*R*)-2,3,4,5,6-pentahydroxyhexyl)pentanediamide **LD-87**

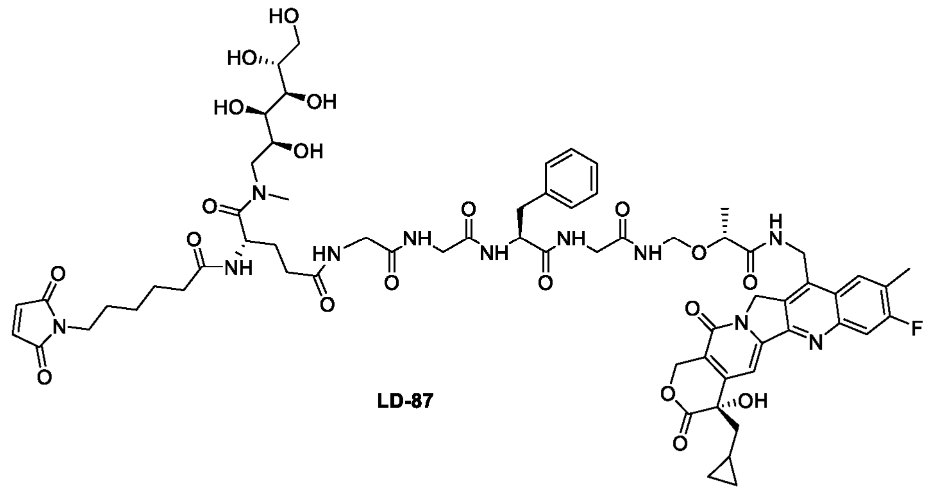

LD-87

Example 3-88: LD-88

(*S*)-*N*[5]-((4*S*,12*S*)-12-Benzyl-4-cyclopropyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17-pentaoxo-5-oxa-2,7,10,13,16-pentaazaoctadecan-18-yl)-2-(6-(2,5-dioxo-2,5-dihyd ro-1*H*-pyrrol-1-yl)hexanamido)-*N*[1]-methyl-*N*[1]-((2*S*,3*R*,4*R*,5*R*)-2,3,4,5,6-pentahydroxyhexyl)pentanediamide **LD-88**

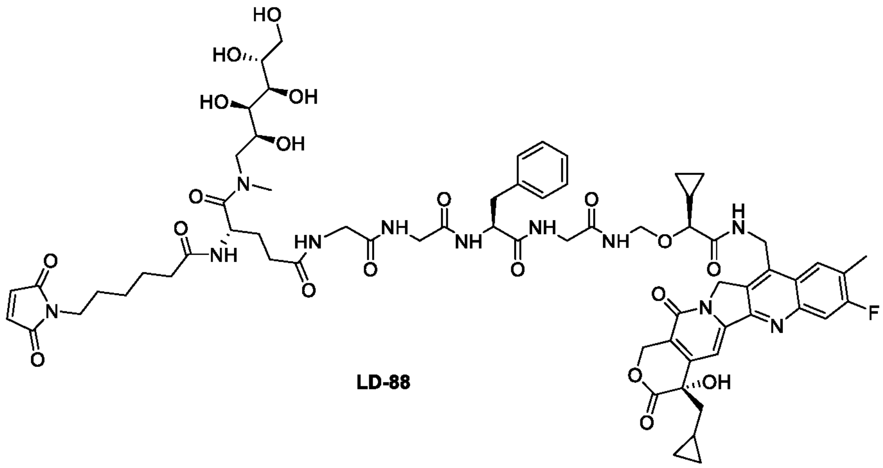

**LD-88**

Example 3-89: LD-89

(*S*)-*N*[5]-((4*R*,12*S*)-12-Benzyl-4-cyclopropyl-1-((*S*)-4-(cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-3,8,11,14,17-pentaoxo-5-oxa-2,7,10,13,16-pentaazaoctadecan-18-yl)-2-(6-(2,5-dioxo-2,5-dihyd ro-1*H*-pyrrol-1-yl)hexanamido)-*N*[1]-methyl-*N*[1]-((2*S*,3*R*,4*R*,5*R*)-2,3,4,5,6-pentahydroxyhexyl)pentanediamide **LD-89**

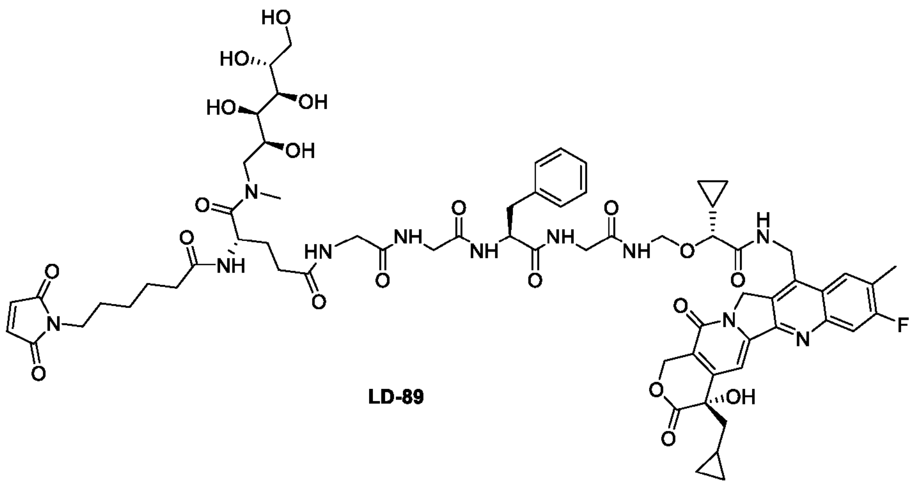

**LD-89**

Example 3-90: LD-90

5-(2,5-Dioxo-2,5-dihydro-1*H*-pyrrol-1-yl)-2-(((9*S*,12*S*,15*S*)-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-9,12,15-trimethyl-3,8,11,14,17-pentaoxo-5-oxa-2,7,10,13,16-pentaazaicosan-20- yl)oxy)-*N*[1],*N*[3]-di(2,5,8,11,14,17,20,23-octaoxapentacosan-25-yl)isophthalamide **LD-90**

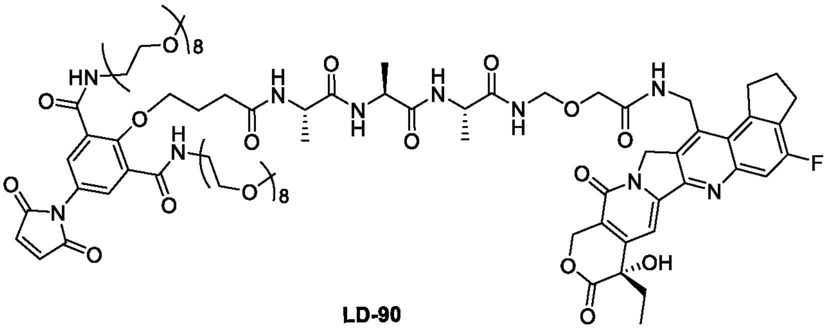

LD-90

MS m/z (ESI): 907.0 [1/2(M+2)].

Example 3-91: LD-91

(*S*)-*N*5-((9*S*,12*S*,15*S*)-1-((*S*)-8-Ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-9,12-dimethyl-3,8,11,14-tetraoxo-5-oxa-2,7,10,13-tetraazahexadecan-15-yl)-2-(6-(2-(methylsulfonyl)pyrimidin-5-yl)he x-5-ynamido)-*N*1-(2,5,8,11,14,17,20,23-octaoxapentacosan-25-yl)pentanediamide **LD-91**

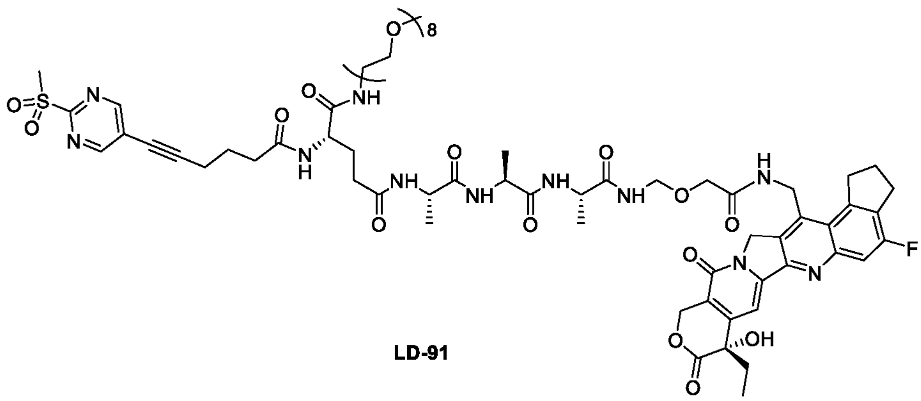

LD-91

Example 3-92: LD-92

(*S*)-2-(6-(2,5-Dioxo-2,5-dihydro-1*H*-pyrrol-1-yl)hexanamido)-*N*5-((9*S*,12*S*,15*S*)-1-((*S*)-8-ethyl-4-fluoro-9,12-dioxo-2,3,8,9,12,14-hexahydro-1*H*,11*H*-cyclopenta[*f*]pyrano[3',4':6,7]indolizino[1,2-*b*]quinolin-15-yl)-9,12-dimethyl-3,8,11,14-tetraoxo-5-oxa-2,7,10,13-tetraazahexadecan-15-yl)-*N*1-(2,5,8,11,14,17,20,23-octaoxapentacosan-25-yl)pentanediamide **LD-92**

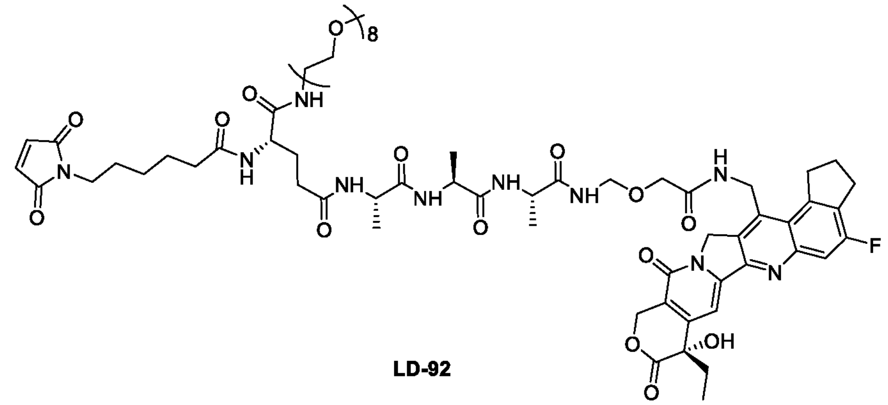

**LD-92**

Example 3-93: LD-93

*N*-((9*S*,12*S*,15*S*)-1-((*S*)-4-(Cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-9,12-dimethyl-3,8,11,14-tetraoxo-5-oxa-2,7,10,13-tetraazahexadecan-15-yl)-6-(2,5-dioxo-2,5-dihydro-1*H*-pyrrol-1-yl)hexanamide **LD-93**

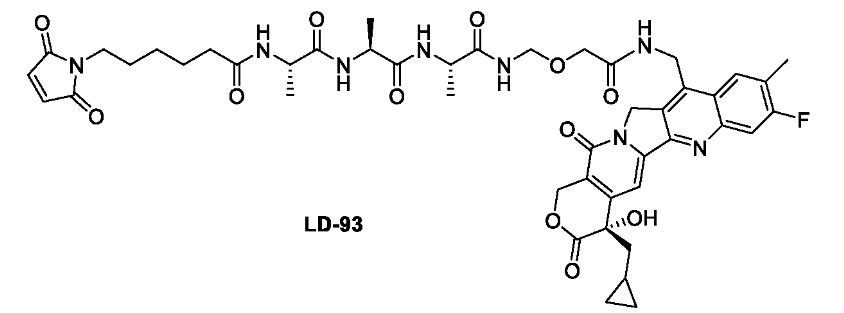

**LD-93**

Example 3-94: LD-94

2-(((9*S*,12*S*,15*S*)-1-((*S*)-4-(Cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-9,12,15-trimethyl-3,8,11,14,17-pentaoxo-5-oxa-2,7,10,13,16-pentaazaicosan-20-yl)oxy)-5-(2,5-dioxo-2,5-dihydro-1*H*-pyrrol-1-yl)-*N*[1],*N*[3]-di(2,5,8,11,14,17,20,23-octaoxapentacosan-25-yl)isophthalamide **LD-94**

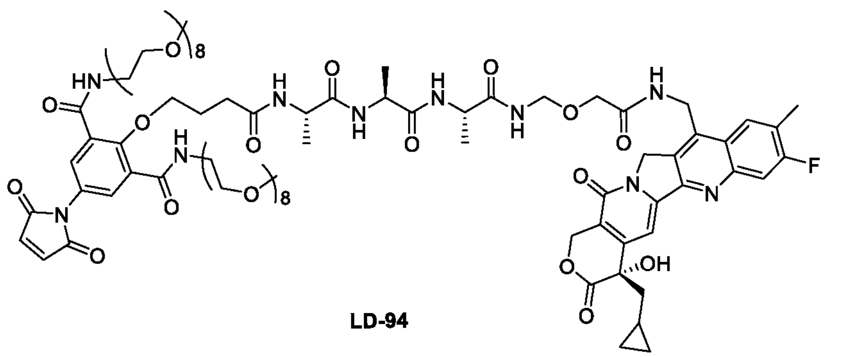

**LD-94**

Example 3-95: LD-95

(*S*)-*N*[5]-((9*S*,12*S*,15*S*)-1-((*S*)-4-(Cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-9,12-dimethyl-3,8,11,14-tetraoxo-5-oxa-2,7,10,13-tetraazahexadecan-15-yl)-2-(6-(2-(methylsulfonyl)pyrimidin-5-yl) hex-5-ynamido)-*N*[1]-(2,5,8,11,14,17,20,23-octaoxapentacosan-25-yl) pentanediamide **LD-95**

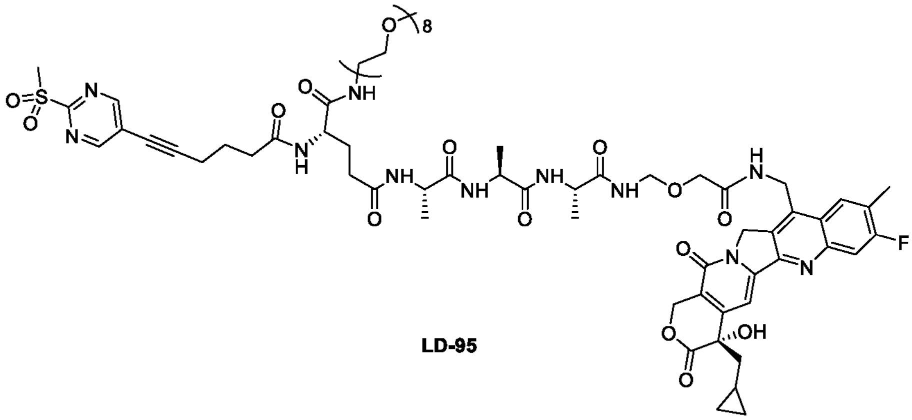

**LD-95**

Example 3-96: LD-96

(*S*)-*N*[5]-((9*S*,12*S*,15*S*)-1-((*S*)-4-(Cyclopropylmethyl)-8-fluoro-4-hydroxy-9-methyl-3,14-dioxo-3,4,12,14-tetrahydro-1*H*-pyrano[3',4':6,7]indolizino[1,2-*b*]quinolin-11-yl)-9,12-dimethyl-3,8, 11,14-tetraoxo-5-oxa-2,7,10,13-tetraazahexadecan-15-yl)-2-(6-(2,5-dioxo-2,5-dihydro-1*H*-pyrrol-1-yl)hexanamido)-*N*[1]-(2,5,8,11,14,17,20,23-octaoxapentacosan-25-yl)pentanediamide **LD-96**

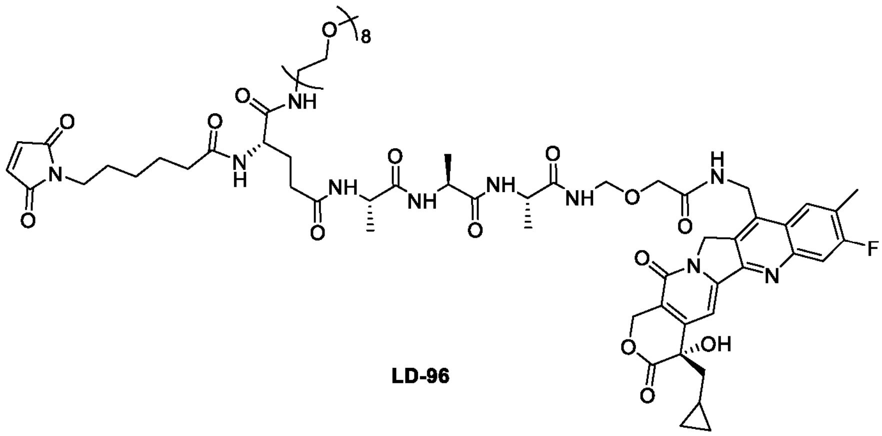

**LD-96**

Example 3-97: LD-97

*N*-((*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadecan-16-yl)-1-(4-(2-(methylsulfonyl)pyrimidin-5-yl)but-3-yn-1-yl)cyclopropane-1-carboxamide **LD-97**

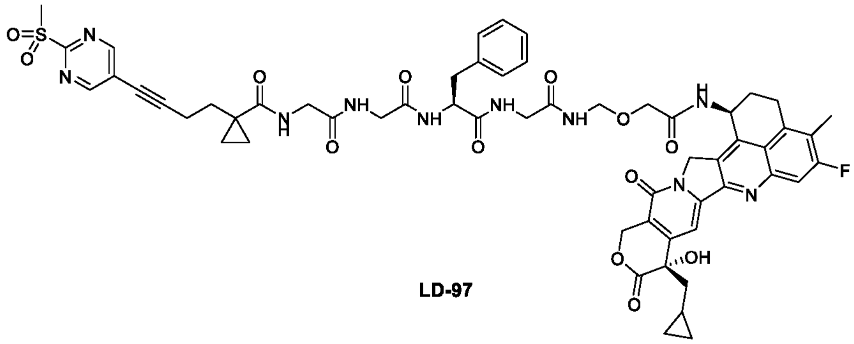

LD-97

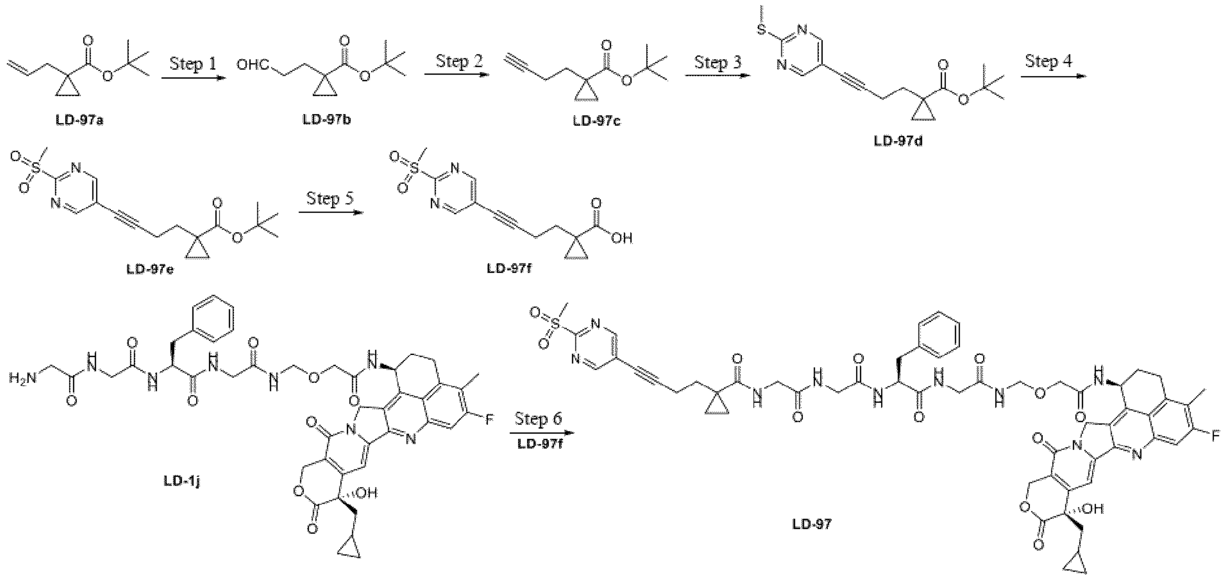


LD-97a LD-97b LD-97c LD-97d LD-97e LD-97f LD-1j LD-97

Step 1

**LD-97a** (2.20 g, 12.1 mmol, prepared by applying the method disclosed in Example 4 on page 62 of patent application "WO2020077038 A1"), silver nitrite (111 mg, 724 μmol), bis(benzonitrile)palladium(II) chloride (555 mg, 1.45 mmol), nitromethane (4.90 g, 80.3 mmol) and copper chloride dihydrate (II) (247 mg, 1.45 mmol) were dissolved in 44 mL of tert-butanol, subjected to replacement with oxygen gas three times, and stirred at 25 °C for 12 hours under oxygen gas. At 25 °C, 150 mL of water was added to the reaction solution, dichloromethane (50 mLx3) was used for extraction, the organic phase was dried with anhydrous sodium sulfate and filtered, and the filtrate was concentrated by vacuum distillation. The residue was purified by silica gel column chromatography with the developing system B to obtain the title product **LD-97b** (800 mg, yield: 33%) as a white solid.

Step 2

**LD-97b** (800 mg, 4.04 mmol) was dissolved in 24 mL of methanol, then added with potassium carbonate (1.12 g, 8.07 mmol) and dimethyl (1-diazo-2-oxopropyl)phosphonate (930 mg, 4.84 mmol), and stirred at 25 °C for 12 hours. 50 mL of water was added to the reaction solution, and ethyl acetate (20 mLx3) was used for extraction. The organic phase was dried over anhydrous sodium sulfate and filtered, and the filtrate was concentrated by vacuum distillation. The residue was purified by silica gel column chromatography with the developing system B to obtain the title product **LD-97c** (650 mg, yield: 83%) as a white solid.

Step 3

**LD-97c** (650 mg, 3.35 mmol), 5-bromo-2-methylthiopyrimidine (686 mg, 3.35 mmol), triethylamine (3.39 g, 33.4 mmol), cuprous iodide (63.7 mg, 334 μmol) and bistriphenylphosphine palladium dichloride (244 mg, 349 μmol) were dissolved in 10 mL of tetrahydrofuran, subjected to replacement with nitrogen gas three times, and stirred at 60 °C for 2 hours under nitrogen protection. The reaction solution was concentrated under reduced pressure, and the obtained

residue was purified by silica gel column chromatography with the developing system B to obtain the title product **LD-97d** (770 mg, yield: 68%) as a white solid.

MS m/z (ESI): 319.6 [M+1].

Step 4

**LD-97d** (1.80 g, 5.65 mmol) was dissolved in 20 mL of dichloromethane, then added with *m*-chloroperbenzoic acid (2.87 g, 14.1 mmol, purity 85.0%), and stirred at 25 °C for 2 hours. The reaction solution was concentrated under reduced pressure, and the obtained residue was purified by silica gel column chromatography with the developing system B to obtain the title product **LD-97e** (1.70 g, yield: 85%) as a white solid.

MS m/z (ESI): 351.1 [M+1].

Step 5

**LD-97e** (500 mg, 1.43 mmol) was dissolved in 6 mL of dichloromethane, then added with trifluoroacetic acid (2.30 g, 20.2 mmol), and stirred at 25 °C for 2 hours. The reaction solution was concentrated under reduced pressure to obtain the crude title product **LD-97f** (418 mg) as a white solid. The product was used directly in the next step without purification.

MS m/z (ESI): 295.2 [M+1].

Step 6

**LD-97f** (14.2 mg, 48.4 μmol) and **LD-1j** (35.0 mg, 40.3 μmol) were dissolved in 2 mL of N,N-dimethylformamide, then added with 1-hydroxybenzotriazole (8.2 mg, 60.5 μmol), *N,N*-diisopropylethylamine (15.6 mg, 121 μmol) and 1-(3-dimethylaminopropyl)-3-ethylcarbodiimide hydrochloride (11.6 mg, 60.6 μmol) in sequence, and stirred at 25 °C for 2 hours. The reaction solution was concentrated under reduced pressure to remove the solvent, 1 mL of water was added to the residue, dichloromethane (3 mLx2) was used for extraction, the organic phase was washed with saturated sodium chloride solution (2 mL), then dried over anhydrous sodium sulfate and filtered, and the filtrate was concentrated under reduced pressure. The residue was purified by pre-HPLC (chromatographic column: Phenomenex Kinetex EVO C18 150x30mmx5μm; mobile phase: A-water (0.225% formic acid), B-acetonitrile; gradient elution: B%: 35%-55%) to obtain the title product **LD-97** (8.00 mg, yield: 17%) as a yellow solid.

MS m/z (ESI): 1143.5 [M+1].

$^1$H NMR (400 MHz, DMSO-d6) δ 9.11 - 9.00 (m, 2H),8.67 - 8.59 (m, 1H), 8.55 - 8.48 (m, 1H), 8.34 - 8.28 (m, 1H), 8.17 - 8.11 (m, 1H), 8.00 - 7.94 (m, 1H), 7.90 - 7.83 (m, 1H), 7.81 - 7.75 (m, 1H), 7.41 - 7.35 (m, 1H), 7.28 - 7.11 (m, 5H), 6.60 - 6.55 (m, 1H), 5.63 - 5.56 (m, 1H), 5.47 - 5.35 (m, 2H), 5.25 - 5.17 (m, 2H), 4.70 - 4.59 (m, 2H), 4.51 - 4.43 (m, 1H), 4.07 - 3.98 (m, 2H), 3.79 - 3.53 (m, 6H), 3.44 - 3.36 (m, 3H), 3.20 - 3.13 (m, 2H), 3.05 - 2.99 (m, 1H), 2.81 - 2.73 (m, 1H), 2.71 - 2.62 (m, 2H), 2.40 - 2.36 (m, 3H), 2.22 - 2.13 (m, 2H), 1.92 - 1.85 (m, 2H), 1.85 - 1.79 (m, 1H), 1.78 - 1.71 (m, 1H), 1.04 - 0.95 (m, 2H), 0.83 - 0.76 (m, 1H), 0.73 - 0.65 (m, 2H), 0.37 - 0.25 (m, 2H), 0.09 - 0.01 (m, 1H), -0.05 - -0.12 (m, 1H).

Example 3-98: LD-98

*N*-((2*S*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinoli n-1-yl)amino)-2-methyl-1,6,9,12,12-pentaoxo-3-oxa-5,8,11,14-tetraazahexadecan-16-yl)-1-(4-(2-(methylsulfonyl)pyrimidin-5-yl)but-3-yn-1-yl)cyclopropane-1-carboxamide **LD-98**

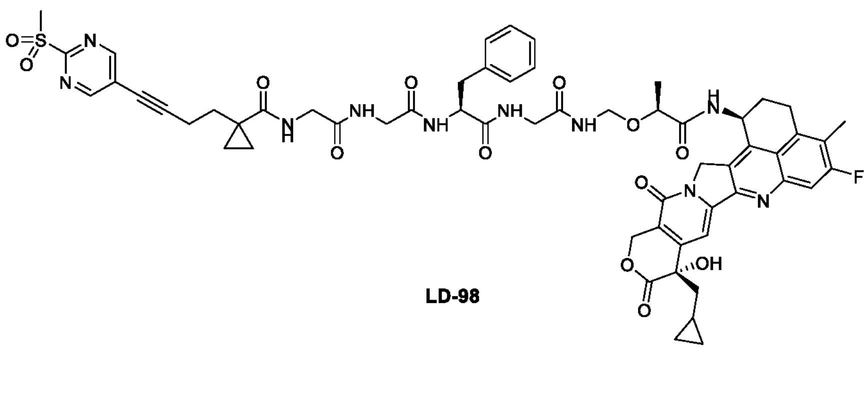

LD-98

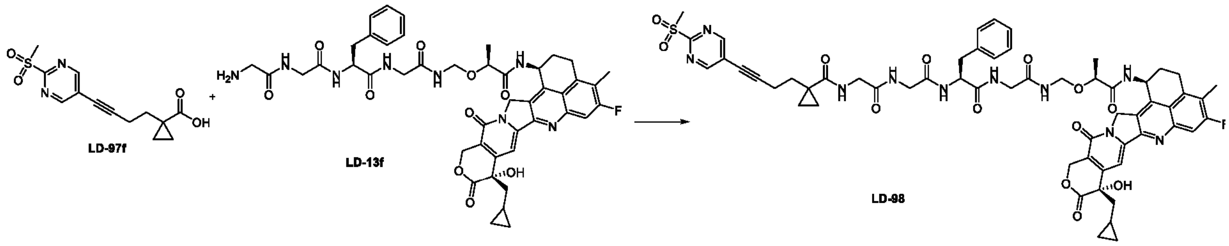

LD-97f

LD-13f

LD-98

**LD-97f** (10.8 mg, 36.8 μmol) and **LD-13f** (27.0 mg, 30.6 μmol) were dissolved in 1 mL of *N,N*-dimethylformamide, added with 1-hydroxybenzotriazole (6.21 mg, 45.9 μmol), *N,N*-diisopropylethylamine (11.9 mg, 91.9 μmol) and 1-(3-dimethylaminopropyl)- 3-ethylcarbodiimide hydrochloride (8.81 mg, 45.9 μmol), and stirred at 25 °C 1 hour. The reaction solution was concentrated under reduced pressure to remove the solvent, 3 mL of water was added to the residue, and dichloromethane (3 mLx3) was used for extraction. The organic phase was washed with saturated sodium chloride solution (2 mL), and then dried over anhydrous sodium sulfate, and filtered. The filtrate was concentrated under reduced pressure. The obtained residue was purified by silica gel column chromatography using the eluent system A to obtain a crude product, and the crude product was then purified by pre-HPLC (chromatographic column: Phenomenex C18 150 mm x 30 mm x 5 μm; mobile phase: A -water (0.1% trifluoroacetic acid), B-acetonitrile; gradient elution: B%: 25%-55%) to obtain the title product **LD-98** (2.11 mg, yield: 6%) as a white solid.

MS m/z (ESI): 1157.8 [M+1].

$^1$H NMR (400 MHz, $CD_3OD$) δ 8.89 - 8.80 (m, 2H), 7.66 - 7.58 (m, 2H), 7.26 - 7.19 (m, 2H), 7.19 - 7.08 (m, 3H), 5.71 - 5.65 (m, 1H), 5.62 - 5.54 (m, 1H), 5.50 - 5.42 (m, 1H), 5.33 - 5.25 (m, 1H), 5.22 - 5.15 (m, 1H), 4.78 - 4.71 (m, 2H), 4.33 - 4.25 (m, 2H), 3.91 - 3.72 (m, 4H), 3.61 - 3.45 (m, 3H), 3.36 - 3.33 (m, 3H), 3.22 - 3.12 (m, 1H), 2.94 - 2.84 (m, 2H), 2.73 - 2.64 (m, 2H), 2.46 - 2.37 (m, 3H), 2.37 - 2.28 (m, 1H), 2.26 - 2.18 (m, 1H), 2.01 - 1.90 (m, 2H), 1.89 - 1.81 (m, 1H), 1.76 - 1.69 (m, 1H), 1.51 - 1.42 (m, 3H), 1.17 - 1.12 (m, 2H), 0.94 - 0.83 (m, 3H), 0.45 - 0.31 (m, 2H), 0.06 - -0.08 (m, 2H).

Example 3-99: LD-99

*N*-((2*R*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-methyl-1,6,9,12,15-pentaoxo-3-oxa-5,8,11,14-tetraazahexadecan-16-yl)-1-(4-(2-(methylsulfonyl)pyrimidin-5-yl)but -3-yn-1-yl)cyclopropane-1-carboxamide **LD-99**

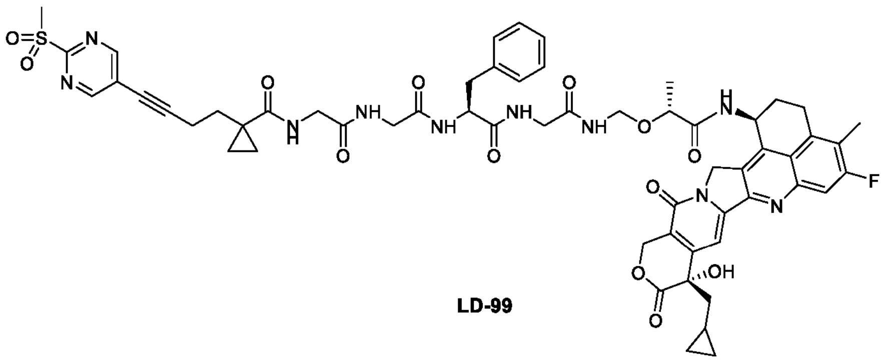

**LD-99**

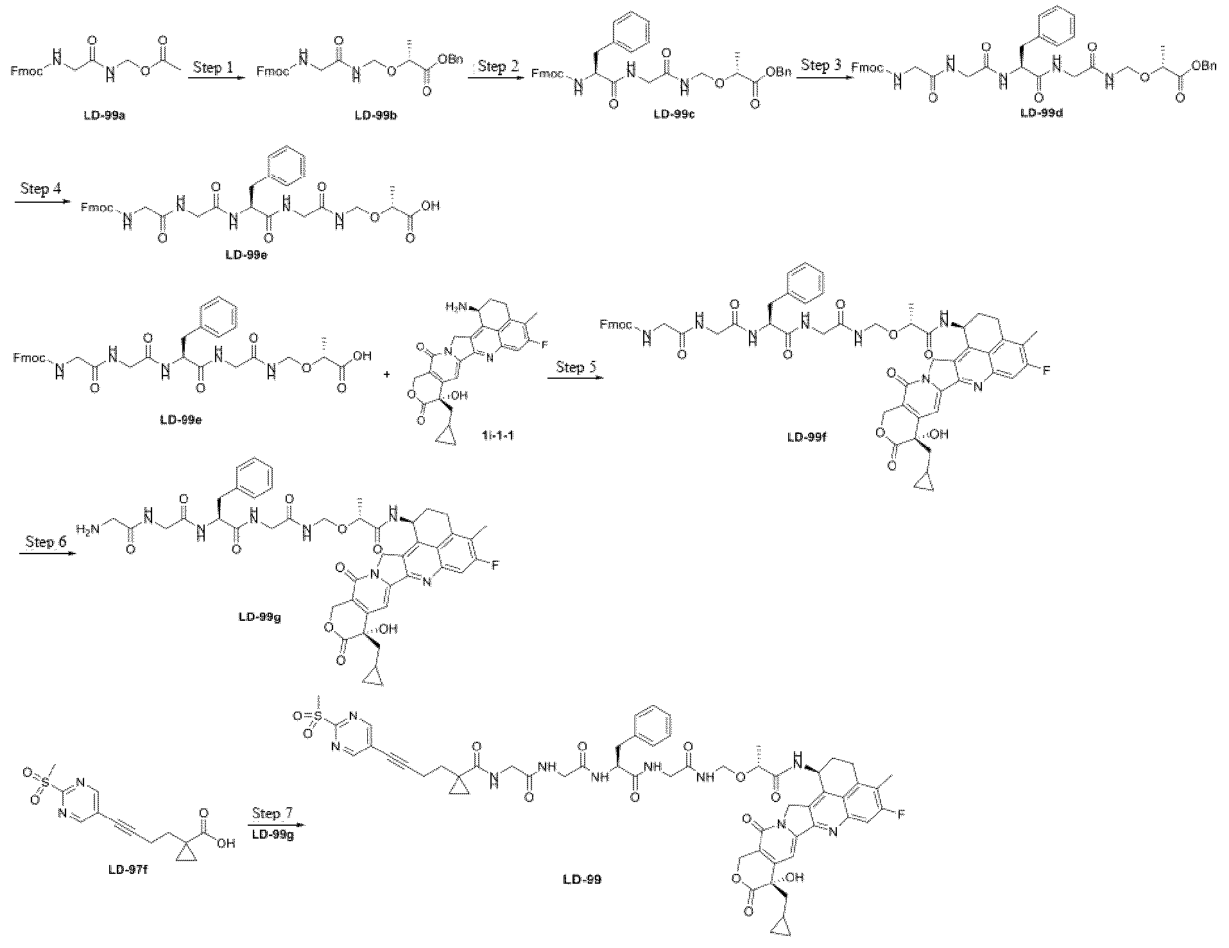


Step 1

Benzyl (*R*)-1-(9*H*-fluorene-9-yl)-10-methyl-3,6-dioxo-2,9-dioxa-4,7-diazaundecan-11-oate **LD-99b**

**LD-99a** (3.00 g, 7.87 mmol, prepared by applying the method disclosed in Example 1.15 on page 226 of patent application "WO2022135332 A1") was dissolved in 50 mL of dichloromethane, added with pyridium 4-toluenesulfonate (1.98 g, 7.87 mmol) and benzyl (2R)-2-hydroxypropanoate (2.84 g, 15.7 mmol), and stirred at 50 °C for 10 hours. 40 mL of water was added to the reaction solution, and dichloromethane (50 mLx3) was used for extraction. The organic phase was dried over anhydrous sodium sulfate and filtered, and the filtrate was concentrated by distillation under reduced pressure. The resulting residue was purified by silica gel column chromatography using the developing system B to obtain the title product **LD-99b** (1.55 g, yield:: 40%).

Step 2

Benzyl (5*S*,13*R*)-5-benzyl-1-(9*H*-fluoren-9-yl)-13-methyl-3,6,9-trioxo-2,12-dioxa-4,7,10-triazatetradecan-14-oate **LD-99c**

**LD-99b** (2.63 g, 5.38 mmol) was dissolved in 30 mL of dichloromethane, added with 1,8-diazabicyclo[5.4.0] undec-7-ene (491 mg, 3.23 mmol), and stirred at 25 °C for 1 hour. Then, (((9H-fluoren-9-yl)methoxy)carbonyl)-L-phenylalanine (2.29 g, 5.91 mmol), 1-hydroxybenzotriazole (1.09 g, 8.06 mmol) and 1-(3-dimethylaminopropyl)- 3-ethylcarbodiimide hydrochloride (1.54 g, 8.06 mmol) added to the reaction solution, and stirred at 25 °C for 2 hours. 10 mL of water was added, and dichloromethane (10 mLx3) was used for extraction, the organic phase was dried over anhydrous sodium sulfate and filtered, and the filtrate was concentrated by vacuum distillation. The obtained residue was purified by silica gel column chromatography using the eluent system A to obtain the title product **LD-99c** (1.60 g, yield: 37%) as a yellow solid.

MS m/z (ESI): 658.4 [M+23].

Step 3

Benzyl (11*S*,19*R*)-11-benzyl-1-(9*H*-fluoren-9-yl)-19-methyl-3,6,9,12,15-pentaoxo-2,18-dioxa- 4,7,10,13,16-pentaazaicosan-20-oate **LD-99d**

**LD-99c** (1.60 g, 1.97 mmol) was dissolved in 15 mL of dichloromethane, added with 1,8-diazabicyclo[5.4.0] undec-7-ene (179 mg, 1.18 mmol), and stirred at 25 °C for 1 hour. Then, (((9*H*-fluoren-9-yl)methoxy)carbonyl)glycylglycine (836 mg, 2.36 mmol) and 2-(7-azobenzotriazole)-*N,N,N',N'*-tetramethyluronium hexafluorophosphate (1.12 g, 2.95 mmol) were added in sequence to the reaction solution, and stirred at 25 °C for 1 hour. 10 mL of water was added, dichloromethane (10 mLx3) was used for extraction, and the organic phase was dried over anhydrous sodium sulfate and filtered, and the filtrate was concentrated by distillation under reduced pressure. The resulting residue was purified by silica gel column chromatography using the eluent system A to obtain the title product **LD-99d** (1.15 g, yield: 70%) as a yellow solid.).

MS m/z (ESI): 772.4 [M+23].

Step 4

(11*S*,19*R*)-11-Benzyl-1-(9*H*-fluoren-9-yl)-19-methyl-3,6,9,12,15-pentaoxo-2,18-dioxa-4,7,10,13,16-pentaazaicosan-20-oic acid **LD-99e**

Under nitrogen protection, **LD-99d** (500 mg, 602 μmol) and palladium-on-carbon (448 mg, 10%, weitted with ca. 55% water) was dissolved in 2 mL of ethyl acetate and 4 mL of ethanol, and subjected to replacement with hydrogen gas three times. The reaction solution was stirred at 25 °C for 2 hours under hydrogen gas (15 Psi). The reaction solution was filtered through Celite, and the filtrate was concentrated by distillation under reduced pressure to obtain the crude title product **LD-99e** (330 mg, yield: 76%) as a white solid. The product was used directly in the next reaction without purification.

MS m/z (ESI): 682.4 [M+23].

Step 5

(9*H*-Fluoren-9-yl)methyl ((2*R*,10*S*)-10-benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7] indolizino[1,2-*b*]quinolin-1-yl)amino)-2-methyl-1,6,9,12,15-pentaoxo-3-oxa-5,8,11,14-tetraazahexadecan-16-yl)carbamate **LD-99f**

**LD-99e** (30.2 mg, 41.7 μmol) and **1i-1-1** (15.0 mg, 32.1 μmol) were dissolved in 2 mL of *N,N*-dimethylformamide, added with 2-(7-azobenzotriazole)-*N,N,N',N'*-tetramethyluronium hexafluorophosphate (18.3 mg, 48.1 μmol) and *N,N*-diisopropylethylamine (12.4 mg, 96.2 μmol) in sequence, and stirred at 25 °C for 1 hour. 1 mL of water was added, dichloromethane (3 mLx2) was used for extraction. The organic phase was dried over anhydrous sodium sulfate and filtered, and the filtrate was concentrated by vacuum distillation. The obtained residue was purified by silica gel column chromatography with the eluent system A to obtain the title product **LD-99f** (25.0 mg, yield: 59%) as a yellow solid.

MS m/z (ESI): 1103.6 [M+1].

Step 6

(*S*)-2-(2-(2-Aminoacetamido)acetamido)-*N*-(2-(((((*R*)-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano [3',4':6,7]indolizino[1,2-*b*]quinolin-1-yl)amino)-1-oxopropan-2-yl)oxy)methyl)amino)- 2-oxyethyl)-3-phenylpropanamide **LD-99g**

**LD-99f** (44.0 mg, 39.9 μmol) was dissolved in 1 mL of acetonitrile, added with diethylamine (142 mg, 1.94 mmol), and stirred at 25 °C for 0.5 h. The reaction solution was concentrated by vacuum distillation to obtain the crude title product **LD-99g** (30.0 mg) as a yellow solid. The product was used directly in the next reaction without purification.

MS m/z (ESI): 881.5 [M+1].

Step 7

*N*-((2*R*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*] quinolin-1-yl)amino)-2-methyl-1,6,9,12,15-pentaoxo-3-oxa-5,8,11,14-tetraazahexadecan-16-yl)-1-(4-(2-(methylsulfonyl)pyrimidin-5-yl)but-3-yn-1-yl)cyclopropane-1-carboxamide **LD-99**

**LD-97f** (16.0 mg, 54.5 μmol) and **LD-99g** (40.0 mg, 45.4 μmol) were dissolved in 1 mL of *N,N*-dimethylformamide, then added with 1-hydroxybenzotriazole (9.20 mg, 68.1 μmol), *N,N*-diisopropylethylamine (23.5 mg, 181 μmol) and 1-(3-dimethylaminopropyl)- 3-ethylcarbodiimide hydrochloride (13.1 mg, 68.1 μmol) in sequence, and stirred at 25 °C for 1 hour. The reaction solution was concentrated under reduced pressure to remove the solvent, and the resulting residue was purified by pre-HPLC (chromatographic column: Welch Xtimate C18 150x25mmx5μm; mobile phase: A-water (0.1% trifluoroacetic acid), B-acetonitrile; gradient elution: B%: 36%-56%) to obtain the title product **LD-99** (10.6 mg, yield: 69%) as a white solid.

MS m/z (ESI): 1157.2 [M+1].

$^1$H NMR (400 MHz, DMSO-*d6*) δ 9.12 - 9.00 (m, 2H), 8.68 - 8.60 (m, 1H), 8.60 - 8.52 (m, 1H), 8.32 - 8.26 (m, 1H), 8.15 - 8.08 (m, 1H), 8.01 - 7.92 (m, 1H), 7.90 - 7.84 (m, 1H), 7.81 - 7.74 (m, 1H), 7.36 (s, 1H), 7.27 - 7.10 (m, 5H), 6.61 - 6.56 (m, 1H), 5.63 - 5.56 (m, 1H), 5.46 - 5.35 (m, 2H), 5.25 - 5.07 (m, 2H), 4.74 - 4.66 (m, 1H), 4.58 - 4.50 (m, 1H), 4.50 - 4.43 (m, 1H), 4.17 - 4.08 (m, 1H), 3.79 - 3.53 (m, 6H), 3.44 - 3.37 (m, 3H), 3.18 - 3.05 (m, 2H), 3.04 - 2.96 (m, 1H), 2.78 - 2.63 (m, 3H), 2.39 - 2.35 (m, 3H), 2.23 - 2.13 (m, 2H), 1.94 - 1.84 (m, 2H), 1.83 - 1.71 (m, 2H), 1.48 - 1.36 (m, 3H), 1.06 - 0.95 (m, 2H), 0.86 - 0.77 (m, 1H), 0.75 - 0.66 (m, 2H), 0.39 - 0.24 (m, 2H), 0.09 - 0.01 (m, 1H), -0.04 - -0.13 (m, 1H).

Example 3-100: LD-100

*N*-((*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano [3',4':6,7]indolizino[1,2-*b*]quinolin-15-yl)-3,8,11,14,17-pentaoxo-5-oxa-2,7,10,13,16-pentaazaoctadecan-18-yl)-1-(4-(2-(methylsulfonyl)pyrimidin-5-yl)but-3-yn-1-yl)cyclopropane-1-carboxamide **LD-100**

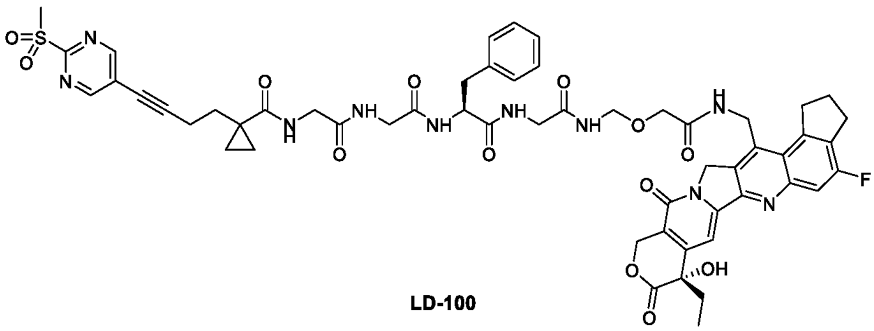

**LD-100**

Example 3-101: LD-101

*N*-((4*S*,12*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*] pyrano[3',4':6,7]indolizino[1,2-*b*]quinolin-15-yl)-4-methyl-3,8,11,14,17-pentaoxo-5-oxa-2,7,10,13,16-pentaazaoctadecan-18-yl)-1-(4-(2-(methylsulfonyl)pyrimidin-5-yl)but-3-yn-1-yl)cyclopropane-1-carboxamide **LD-101**

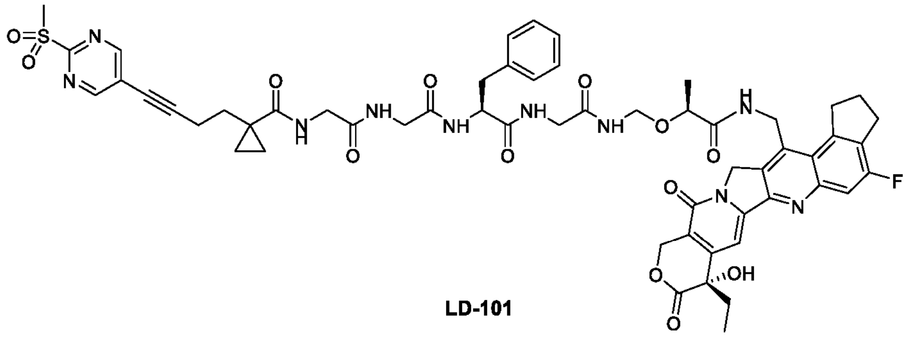

**LD-101**

Example 3-102: LD-102

*N*-((4*R*,12*S*)-12-Benzyl-1-((*S*)-8-ethyl-4-fluoro-8-hydroxy-9,12-dioxo-2,3,8,9,12,14-hexahydro-1*H*,11*H*-cyclopenta[*f*]pyrano[3',4':6,7]indolizino[1,2-*b*]quinolin-15-yl)-4-methyl-3,8,11,14,17-pentaoxo-5-oxa-2,7,10,13,16-pentaazaoctadecan-18-yl)-1-(4-(2-(methylsulfonyl)pyrimidin-5-yl)but-3-yn-1-yl)cyclopropane-1-carboxamide **LD-102**

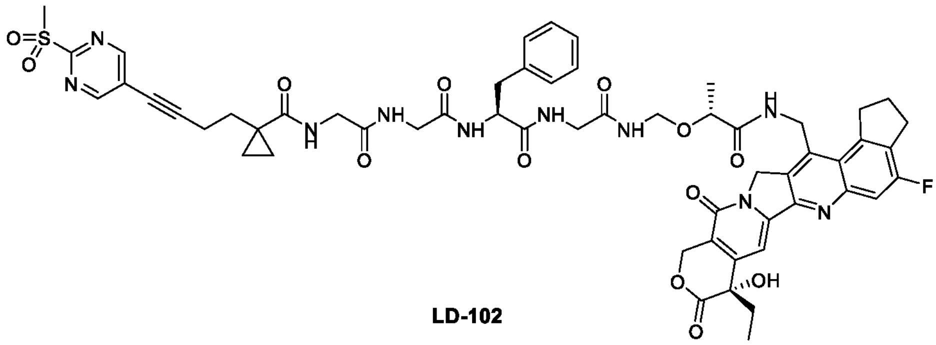

**LD-102**

Example 3-103: LD-103

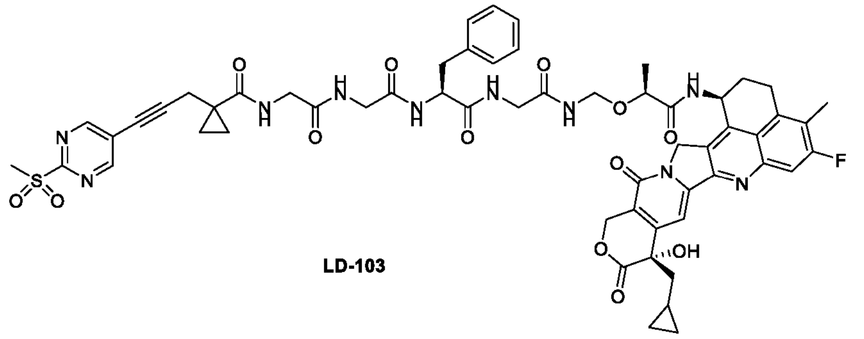

LD-103

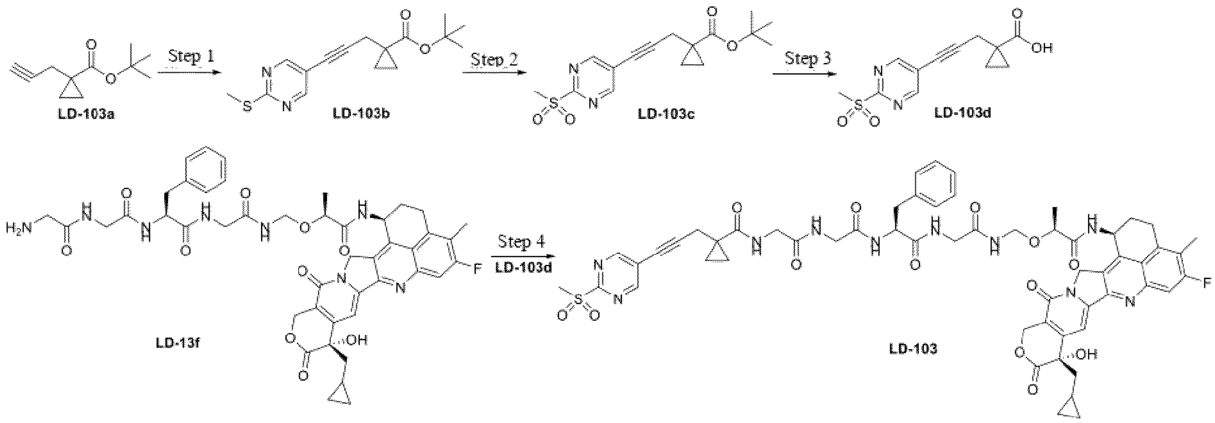


Step 1

**LD-103a** (1.00 g, 5.55 mmol, prepared by applying the method disclosed in Example 34 on page 124 of patent application "WO2022135332 A1"), 5-bromo-2-methylthiopyrimidine (1.14 g, 5.55 mmol), triethylamine (2.42 g, 23.9 mmol), bistriphenylphosphine palladium dichloride (389 mg, 554 μmol) and cuprous iodide (105 mg, 554 μmol) were dissolved in 6 mL of tetrahydrofuran, subjected to replacement with nitrogen gas three times, and stirred at 60 °C for 2 hours under nitrogen protection. The reaction solution was filtered through Celite, 20 mL of water was added to the filtrate, ethyl acetate (15 mLx3) was used for extraction, the organic phase was dried over anhydrous sodium sulfate and filtered, and the filtrate was concentrated by vacuum distillation. The resulting residue was purified by silica gel column chromatography with the developing system B to obtain the title product **LD-103b** (502 mg, yield: 30%) as a yellow solid.
MS m/z (ESI): 305.5 [M+1].

Step 2

**LD-103b** (450 mg, 1.43 mmol) was dissolved in 5 mL of dichloromethane, cooled to 0 °C, slowly added with m-chloroperbenzoic acid (640 mg, 3.15 mmol, purity 85%), and stirred at 25 °C for 2 hours. Sodium thiosulfate aqueous solution (50 mL) was added, dichloromethane (45 mLx3) was used for extraction, the organic phase was dried over anhydrous sodium sulfate and filtered, and the filtrate was concentrated by vacuum distillation. The residue was purified by silica gel column chromatography with the developing system B to obtain the title product **LD-103c** (250 mg, yield: 50%) as a yellow solid.
MS m/z (ESI): 337.1 [M+1].

Step 3

**LD-103c** (100 mg, 273 μmol) was dissolved in 1 mL of dichloromethane, added with trifluoroacetic acid (423 mg, 3.72 mmol), and stirred at 25 °C for 2 hours. The reaction solution was concentrated by vacuum distillation to obtain the crude title product **LD-103d** (80 mg) as a yellow solid. The product was used directly in the next step without purification.
MS m/z (ESI): 280.9 [M+1].

Step 4

**LD-13f** (19.0 mg, 21.6 μmol) and **LD-103d** (7.23 mg, 23.7 μmol) were dissolved in 1 mL of *N,N*-dimethylformamide, then added 1-hydroxybenzotriazole (4.37 mg, 32.4 μmol), *N,N*-diisopropylethylamine (8.36 mg, 64.7 μmol) and 1-(3-dimethylaminopropyl)- 3-ethylcarbodiimide hydrochloride (6.20 mg, 32.4 μmol) in sequence, and stirred at 20 °C for 2 hours. 50 mL of water was added to the reaction solution, and extracted with dichloromethane (45 mLx3). The organic phase was dried over anhydrous sodium sulfate and filtered, and the filtrate was concentrated by vacuum distillation. The resulting residue was purified by pre-HPLC (chromatographic column: Welch Xtimate C18 150x25mmx5μm; mobile phase: A-water (0.1% trifluoroacetic acid), B-acetonitrile; gradient elution: B%: 28%-58%), and freeze-dried to obtain a crude product, and the crude product was further purified by silica gel column chromatography with the eluent system A to obtain the title product **LD-103** (1.50 mg, yield: 6%) as a white solid.

MS m/z (ESI): 1143.2 [M+1].

$^1H$ NMR (400 MHz, $CD_3OD$) δ 8.94 - 8.88 (m, 2H), 7.67 - 7.61 (m, 2H), 7.26 - 7.20 (m, 2H), 7.19 - 7.10 (m, 3H), 5.71 - 5.66 (m, 1H), 5.60 - 5.54 (m, 1H), 5.49 - 5.42 (m, 1H), 5.32 - 5.26 (m, 1H), 5.23 - 5.16 (m, 1H), 4.77 - 4.69 (m, 2H), 4.32 - 4.26 (m, 2H), 3.94 - 3.87 (m, 3H), 3.80 - 3.74 (m, 1H), 3.59 - 3.53 (m, 1H), 3.49 - 3.44 (m, 2H), 3.31 - 3.31 (m, 3H), 3.19 - 3.12 (m, 1H), 3.01 - 2.95 (m, 1H), 2.90 - 2.84 (m, 3H), 2.45 - 2.40 (m, 3H), 2.36 - 2.29 (m, 1H), 2.23 - 2.17 (m, 1H), 1.88 - 1.82 (m, 1H), 1.75 - 1.69 (m, 1H), 1.48 - 1.43 (m, 3H), 1.22 - 1.19 (m, 2H), 0.94 - 0.90 (m, 3H), 0.43 - 0.33 (m, 2H), 0.07 - 0.01 (m, 1H), -0.01 - -0.08 (m, 1H).

Example 3-104: LD-104

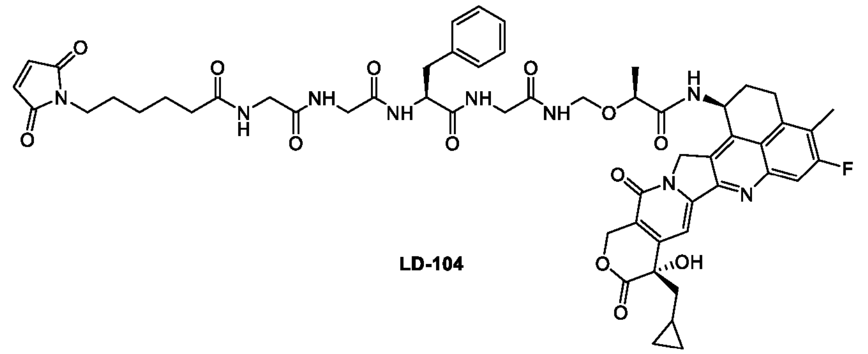

LD-104

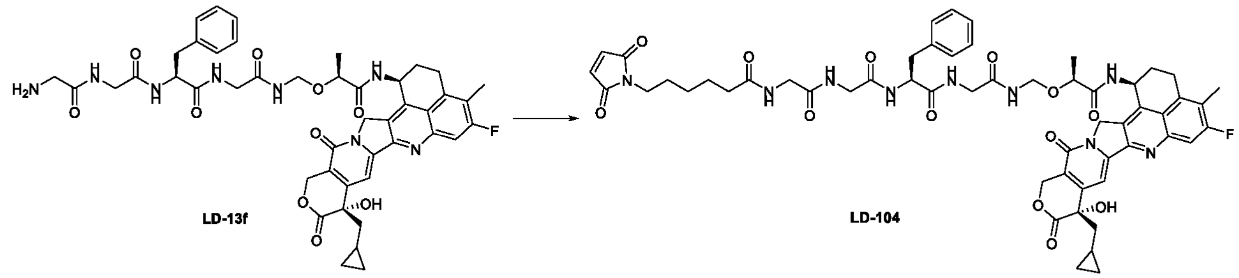

LD-13f

LD-104

**LD-13f** (18.0 mg, 20.4 μmol) and 6-(2,5-dioxopyrrol-1-yl)hexanoic acid (5.2 mg, 24.5 μmol) were dissolved in 1 mL of *N,N*-dimethylformamide, then added with 1-hydroxybenzotriazole (4.1 mg, 30.6 μmol), *N,N*-diisopropylethylamine (7.9 mg, 61.3 μmol) and 1-(3-dimethylaminopropyl)-3-ethylcarbodiimide hydrochloride (5.9 mg, 30.6 μmol) in sequence, and stirred at 25 °C for 1 hour. The reaction solution was concentrated under reduced pressure, and the residue was purified by pre-HPLC (chromatographic column: Welch Xtimate C18 150x25mmx5μm; mobile phase: A-water (0.1% trifluoroacetic acid), B-acetonitrile; gradient elution: B%: 26%-56%) to obtain the title product **LD-104** (3.94 mg, yield: 16%) as a yellow solid.

MS m/z (ESI): 1074.2 [M+1].

$^1H$ NMR (400 MHz, CD3CN) δ 8.00 - 7.91 (m, 1H), 7.79 - 7.71 (m, 1H), 7.55 - 7.49 (m, 1H), 7.42 - 7.31 (m, 3H), 7.28 - 7.17 (m, 4H), 7.17 - 7.05 (m, 3H), 6.72 - 6.63 (m, 2H), 5.62 - 5.45 (m, 2H), 5.25 - 5.14 (m, 2H), 4.88 - 4.79 (m, 1H), 4.72 - 4.62 (m, 2H), 4.26 - 4.12 (m, 2H), 3.74 - 3.62 (m, 4H), 3.56 - 3.50 (m, 1H), 3.46 - 3.40 (m, 1H), 3.39 - 3.32 (m, 2H), 3.27 - 3.04 (m, 6H), 2.38 - 2.26 (m, 4H), 2.17 - 2.08 (m, 3H), 1.84 - 1.74 (m, 1H), 1.72 - 1.62 (m, 1H), 1.52 - 1.44 (m, 3H), 1.43 - 1.36 (m, 3H), 1.25 - 1.14 (m, 2H), 0.84 - 0.74 (m, 1H), 0.39 - 0.30 (m, 2H), 0.08 - -0.10 (m, 2H).

Example 3-105: LD-105

*N*-((2*R*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-methyl-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide **LD-105**

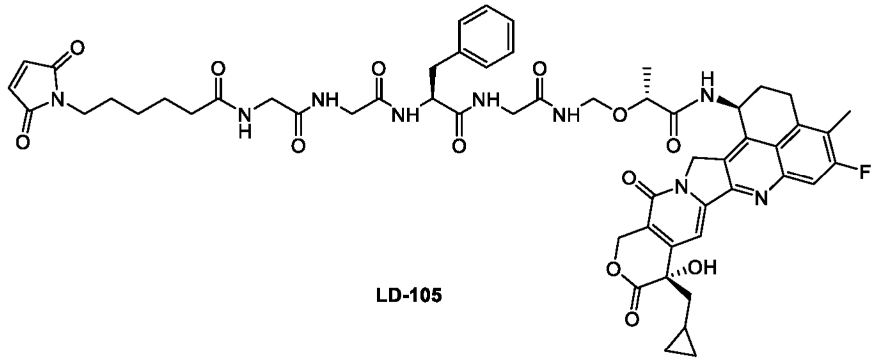

**LD-105**

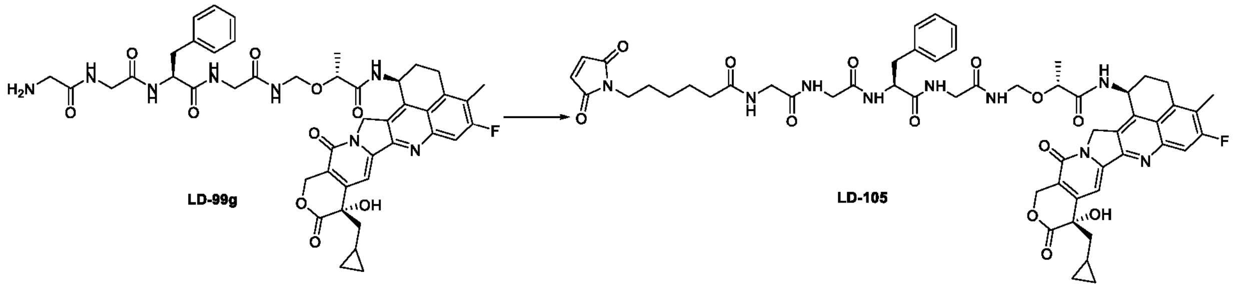

LD-99g

LD-105

*N*-((2*R*,10*S*)-10-Benzyl-1-(((1*S*,9*S*)-9-(cyclopropylmethyl)-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-methyl-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide **LD-105**

**LD-99g** (30.0 mg, 34.1 μmol) and 6-(2,5-dioxopyrrol-1-yl)hexanoic acid (8.6 mg, 40.8 μmol) were dissolved in 0.5 mL of *N,N*-dimethylformamide, then added with 1-hydroxybenzotriazole (6.90 mg, 51.0 μmol), *N,N*-diisopropylethylamine (8.8 mg, 68.1 μmol) and 1-(3-dimethylaminopropyl)-3-ethylcarbodiimide hydrochloride (9.8 mg, 51.0 μmol) in sequence, and stirred at °C for 1 hour. 1 mL of water was added, dichloromethane (3 mLx2) was used for extraction, the organic phase was dried over anhydrous sodium sulfate and filtered, and the filtrate was concentrated by vacuum distillation. The resulting residue was purified by pre-HPLC (chromatographic column: Welch Xtimate C18 150x25mmx5μm; mobile phase: A-water (0.1% trifluoroacetic acid), B-acetonitrile; gradient elution: B%: 32%-52%) to obtain the title product **LD-105** (3.17 mg, yield: 8%) as a white solid.

MS m/z (ESI): 1074.6 [M+1].

$^1$H NMR (400 MHz, DMSO-d6) δ 8.65 - 8.58 (m, 1H), 8.57 - 8.52 (m, 1H), 8.31 - 8.26 (m, 1H), 8.11 - 7.97 (m, 3H), 7.80 - 7.74 (m, 1H), 7.39 - 7.35 (m, 1H), 7.26 - 7.13 (m, 5H), 7.01 - 6.93 (m, 2H), 6.59 - 6.55 (m, 1H), 5.63 - 5.55 (m, 1H), 5.44 - 5.36 (m, 2H), 5.27 - 5.18 (m, 1H), 5.14 - 5.06 (m, 1H), 4.72 - 4.66 (m, 1H), 4.57 - 4.51 (m, 1H), 4.48 - 4.42 (m, 1H), 4.16 - 4.09 (m, 1H), 3.76 - 3.63 (m, 5H), 3.61 - 3.54 (m, 1H), 3.17 - 3.09 (m, 2H), 3.04 - 2.95 (m, 2H), 2.78 - 2.69 (m, 2H), 2.41 - 2.36 (m, 3H), 2.26 - 2.15 (m, 3H), 2.11 - 2.05 (m, 2H), 1.82 - 1.74 (m, 2H), 1.48 - 1.39 (m, 6H), 1.19 - 1.13 (m, 2H), 0.85 - 0.77 (m, 1H), 0.37 - 0.28 (m, 2H), 0.09 - 0.02 (m, 1H), -0.04 - -0.12 (m, 1H).

Example 3-106: LD-106

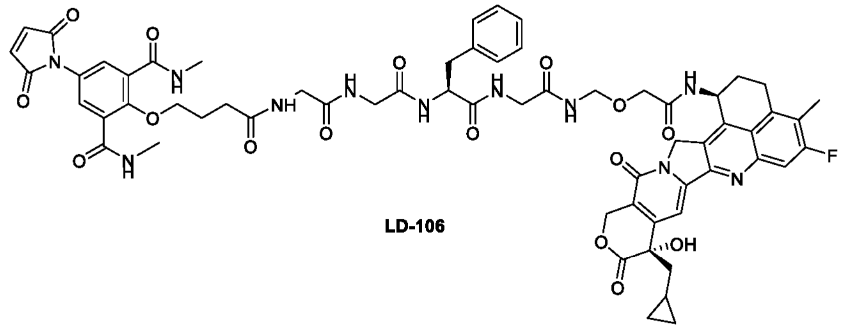

LD-106

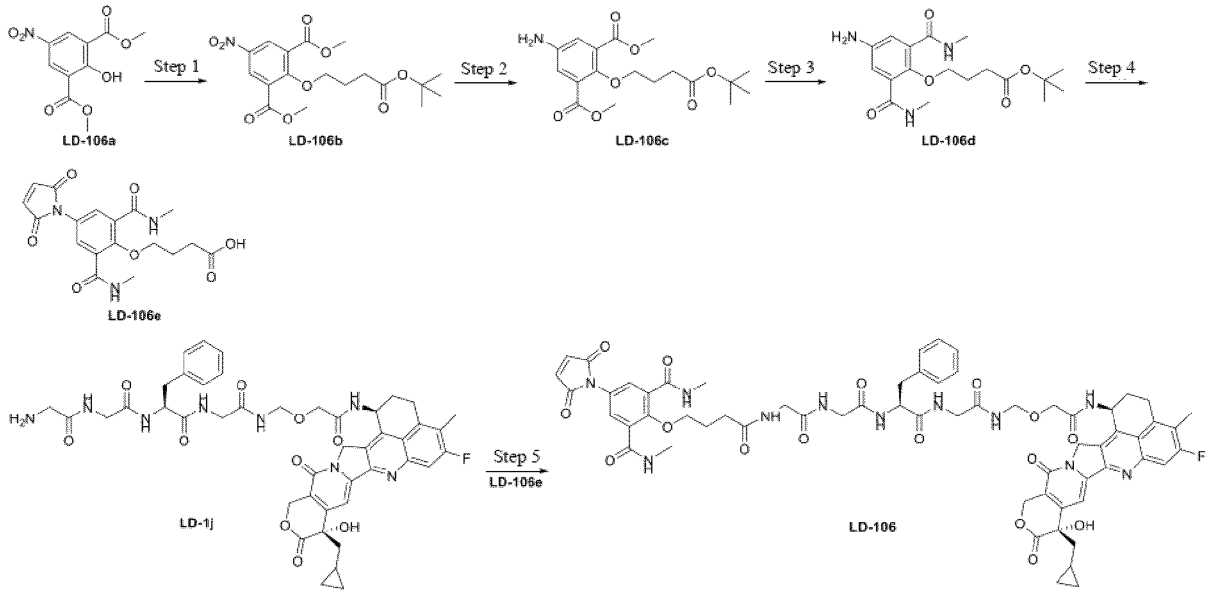


LD-106a LD-106b LD-106c LD-106d LD-106e LD-1j LD-106

Step 1

**LD-106a** (5 g, 19.55 mmol, prepared by applying the method disclosed in Reference Example 121 on page 287 of patent application "CN1572882A"), *tert*-butyl 4-hydroxybutyrate (3.76 g, 23.47 mmol) and triphenylphosphine (7.69 g, 29.33 mmol) were dissolved in 40 mL of *N,N*-dimethylformamide and 120 mL tetrahydrofuran mixed solvent, the reaction solution was cooled to 0 °C, then diisopropyl azodicarboxylate (5.93 g, 29.33 mmol) was slowly added dropwise, replacement with nitrogen was performed three times, and the reaction mixture was stirred at 25 °C for 20 hours under nitrogen protection. 150 mL of water was added, ethyl acetate (200 mLx3) was used for extraction, the organic phase was dried over anhydrous sodium sulfate and filtered, and the filtrate was concentrated by vacuum distillation. The residue was purified by reverse phase high performance liquid chromatography (chromatographic column: I.D.100mmxH300mm Welch Ultimate XB_C18 20-40μm; mobile phase: A-water, B-acetonitrile; isocratic elution: B%: 78%, instrument: Agela Astra) to obtain the title product **LD-106b** (4.8 g, yield: 61%) as a yellow solid.

MS m/z (ESI): 420.2 [M+23].

Step 2

**LD-106b** (3.50 g, 8.65 mmol) was dissolved in 30 mL of methanol and 10 mL of water, added with iron powder (2.42 g, 43.3 mmol) and ammonium chloride (2.31 g, 43.3 mmol) in sequence at 25 °C, and stirred at 80 °C for 2 hours. The reaction solution was filtered, and the filtrate was extracted with dichloromethane (50 mLx2). The organic phase was washed with saturated sodium chloride solution (50 mLx2), then dried over anhydrous sodium sulfate and filtered, and the filtrate was concentrated by vacuum distillation to obtain the crude title product **LD-106c** (2.80 g) as a yellow oil. The product was used directly in the next step without purification.

MS m/z (ESI): 390.0 [M+23].

Step 3

**LD-106c** (2.80 g, 7.46 mmol) was added to a methanol solution of methylamine (10.5 g, purity 30%), and stirred at 50 °C for 12 hours. The reaction solution was concentrated by vacuum distillation to obtain the crude title product **LD-106d** (2.67 g) as a yellow solid. The product was used directly in the next reaction without purification.

MS m/z (ESI): 310.0 [M+1-56].

Step 4

**LD-106d** (200 mg, 518 μmol) and 2,5-furandione (76.0 mg, 775 μmol) were dissolved in 5 mL of dioxane, and stirred at 25 °C for 1 hour. Dimethyl sulfoxide (80.8 mg, 1.03 mmol) and ammonium persulfate (236 mg, 1.03 mmol) were added at 25 °C, and stirred at 100 °C for 12 hours. The reaction solution was concentrated under reduced pressure, and the residue was purified by pre-HPLC (chromatographic column: Welch Xtimate C18 150x40mmx10μm; mobile phase: A-water (0.1% trifluoroacetic acid), B-acetonitrile; gradient elution: B%: 0%-28%) to obtain the title product **LD-106e** (50.0 mg, yield: 23%) as a yellow solid.

MS m/z (ESI): 390.0 [M+1].

Step 5

**LD-106e** (10.8 mg, 27.7 μmol) and **LD-1j** (20.0 mg, 23.1 μmol) were dissolved in 1 mL of *N,N*-dimethylformamide, then added with 1-hydroxybenzotriazole (4.68 mg, 34.6 μmol), *N,N*-diisopropylethylamine (8.95 mg, 69.2 μmol) and 1-(3-dimethylaminopropyl)-3-ethylcarbodiimide hydrochloride (6.63 mg, 34.6 μmol) in sequence, and stirred at 25 °C for 1 hour. The reaction solution was concentrated by vacuum distillation, and the resulting residue was purified by pre-HPLC (chromatographic column: Welch Xtimate C18 150x25mmx5μm; mobile phase: A-water (0.1% trifluoroacetic acid), B-acetonitrile; gradient elution: B%: 28%-48%) to obtain the title product **LD-106** (15.8 mg, yield: 57%) as a white solid.

MS m/z (ESI): 1238.3 [M+1].

$^1$H NMR (400 MHz, DMSO-d6) δ 8.66 - 8.60 (m, 1H), 8.55 - 8.49 (m, 1H), 8.33 - 8.23 (m, 3H), 8.16 - 8.09 (m, 2H), 8.06 - 8.02 (m, 1H), 7.81 - 7.76 (m, 1H), 7.53 - 7.48 (m, 2H), 7.39 - 7.36 (m, 1H), 7.27 - 7.14 (m, 7H), 6.63 - 6.52 (m, 1H), 5.63 - 5.55 (m, 1H), 5.44 - 5.38 (m, 2H), 5.24 - 5.18 (m, 2H), 4.68 - 4.61 (m, 2H), 4.50 - 4.43 (m, 1H), 4.05 - 3.99 (m, 2H), 3.94 - 3.89 (m, 2H), 3.78 - 3.74 (m, 1H), 3.72 - 3.68 (m, 4H), 3.62 - 3.59 (m, 1H), 3.20 - 3.11 (m, 2H), 3.05 - 2.98 (m, 1H), 2.83 - 2.79 (m, 1H), 2.79 - 2.74 (m, 6H), 2.41 - 2.36 (m, 3H), 2.30 - 2.24 (m, 2H), 2.21 - 2.13 (m, 2H), 1.92 - 1.85 (m, 2H), 1.84 - 1.79 (m, 1H), 1.78 - 1.71 (m, 1H), 0.85 - 0.77 (m, 1H), 0.37 - 0.27 (m, 2H), 0.08 - 0.03 (m, 1H), -0.06 - -0.11 (m, 1H).

Example 3-107: LD-107

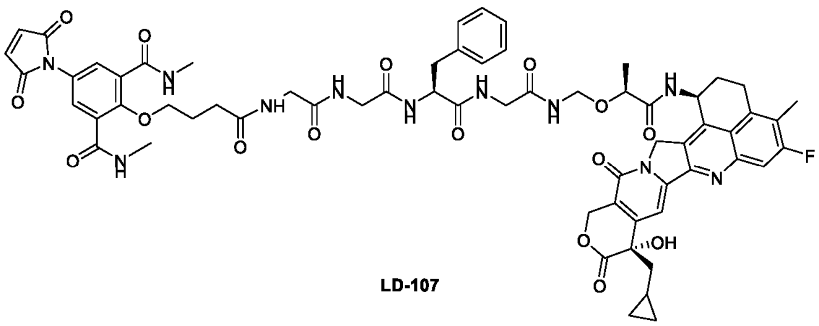

LD-107

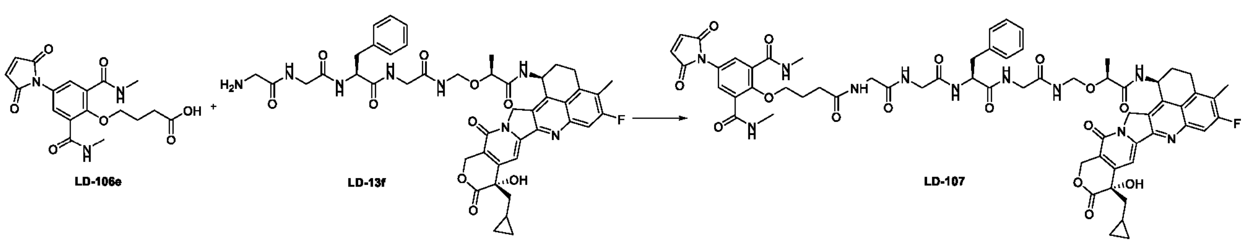

**LD-106e** (10.6 mg, 25.6 μmol) and **LD-13f** (20.0 mg, 21.3 μmol) were dissolved in 1 mL of *N,N*-dimethylformamide, then added with 1-hydroxybenzotriazole (4.3 mg, 32.1 μmol), *N,N*-diisopropylethylamine (8.3 mg, 64.1 μmol) and 1-(3-dimethylaminopropyl)- 3-ethylcarbodiimide hydrochloride (6.2 mg, 32.1 μmol) in sequence, and stirred at 25 °C for 1 hour. The reaction solution was concentrated by vacuum distillation, and the residue was purified by pre-HPLC (chromatographic column: Welch Xtimate C18 150x25mmx5μm; mobile phase: A-water (0.1% trifluoroacetic acid), B-acetonitrile; gradient elution: B%: 32%-52%) to obtain the title product **LD-107** (4.23 mg, yield: 15%) as a yellow solid.

MS m/z (ESI): 1252.6 [M+1].

$^1$H NMR (400 MHz, DMSO-d6) δ 8.67 - 8.62 (m, 1H), 8.57 - 8.52 (m, 1H), 8.30 - 8.21 (m, 3H), 8.15 - 8.08 (m, 2H), 8.04 - 8.00 (m, 1H), 7.80 - 7.75 (m, 1H), 7.54 - 7.48 (m, 2H), 7.41 - 7.36 (m, 1H), 7.28 - 7.12 (m, 7H), 6.60 - 6.56 (m, 1H), 5.57 - 5.52 (m, 1H), 5.44 - 5.37 (m, 2H), 5.26 - 5.19 (m, 2H), 4.74 - 4.69 (m, 1H), 4.61 - 4.56 (m, 1H), 4.48 - 4.43 (m, 1H), 4.15 - 4.09 (m, 1H), 3.95 - 3.88 (m, 2H), 3.73 - 3.64 (m, 4H), 3.61 - 3.55 (m, 1H), 3.17 - 3.12 (m, 2H), 3.02 - 2.98 (m, 1H), 2.82 - 2.72 (m, 6H), 2.40 - 2.36 (m, 3H), 2.29 - 2.25 (m, 2H), 2.19 - 2.14 (m, 2H), 1.91 - 1.80 (m, 4H), 1.76 - 1.70 (m, 2H), 1.34 - 1.29 (m, 3H), 0.83 - 0.79 (m, 1H), 0.36 - 0.28 (m, 2H), 0.07 - 0.03 (m, 1H), -0.06 - -0.10 (m, 1H).

Example 3-108: LD-108

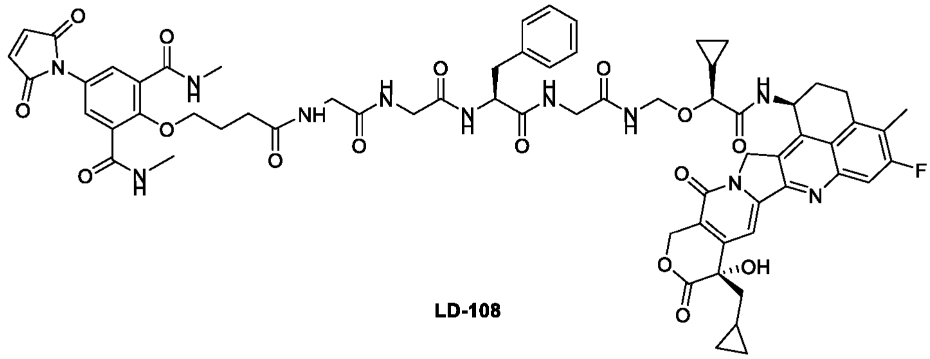

LD-108

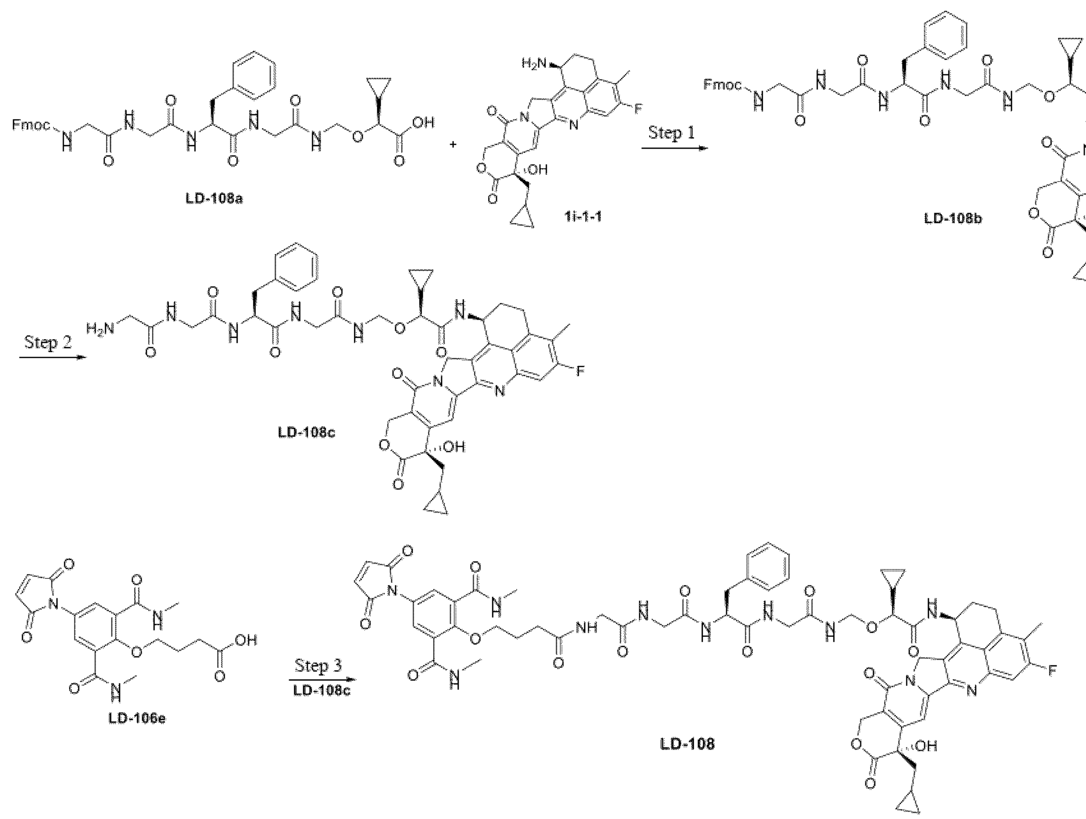


LD-108a 1i-1-1 LD-108b LD-108c LD-106e LD-108

Step 1

**LD-108a** (100 mg, 126 μmol, prepared by applying the method disclosed in Example 1-19 on page 65 of patent application "WO2021190602 A1") and **1i-1-1** (70.5 mg, 126 μmol, HCl) were dissolved in 5 mL of *N,N*-dimethylformamide, then added with 1-hydroxybenzotriazole (25.7 mg, 190 μmol), *N,N*-diisopropylethylamine (49.2 mg, 380 μmol) and 1-(3-dimethylaminopropyl)- 3-ethylcarbodiimide hydrochloride (36.5 mg, 190 μmol) in sequence, and stirred at 25 °C for 1 hour. 10 mL of water was added to the reaction solution, dichloromethane (15 mLx3) was used for extraction, the organic phase was dried over anhydrous sodium sulfate and filtered, and the filtrate was concentrated by vacuum distillation. The obtained residue was purified by silica gel column chromatography using the eluent system A to obtain the title product **LD-108b** (103 mg, yield: 54%) was obtained as a white solid.

MS m/z (ESI): 1129.2 [M+1].

Step 2

**LD-108b** (102 mg, 67.7 μmol) was dissolved in 4 mL of acetonitrile, added with diethylamine (1.0 mL), and stirred at 25 °C for 1 hour. The reaction solution was concentrated by vacuum distillation, and the obtained residue was purified by pre-HPLC (chromatographic column: Welch Xtimate C18 150x25mmx5μm; mobile phase: A-water (0.1% trifluoroacetic acid), B-acetonitrile; gradient elution: B%: 22%-42%) to obtain the title product **LD-108c** (32.0 mg, yield: 49%) as a yellow solid.

MS m/z (ESI): 907.2 [M+1].

Step 3

**LD-106e** (6.88 mg, 16.2 μmol) and **LD-108c** (15.9 mg, 14.7 μmol, trifluoroacetate) were dissolved in 1 mL of *N,N*-dimethylformamide, then added with 1-hydroxybenzotriazole (2.98 mg, 22.0 μmol), *N,N*-diisopropylethylamine (5.70 mg, 44.1 μmol) and 1-(3-dimethylaminopropyl)- 3-ethylcarbodiimide hydrochloride (4.22 mg, 22.0 μmol) in sequence, and stirred at 25 °C for 1 hour. 50 mL of water was added to the reaction solution, and dichloromethane (45 mLx3) was used for extraction. The organic phase was dried over anhydrous sodium sulfate and filtered, and the filtrate was concentrated by vacuum distillation. The residue was purified by pre-HPLC (chromatographic column: Phenomenex luna C18 150x25mmx10μm; mobile phase: A-water (0.225% formic acid), B-acetonitrile; gradient elution: B%: 28%-58%) to obtain the title product **LD-108** (4.22 mg, yield: 22%) as a white solid.

MS m/z (ESI): 1278.3 [M+1].

$^1$H NMR (400 MHz, CD3CN) δ 8.03 - 7.96 (m, 1H), 7.67 - 7.63 (m, 2H), 7.60 - 7.44 (m, 4H), 7.40 - 7.31 (m, 2H), 7.30 - 7.05 (m, 7H), 6.93 - 6.85 (m, 2H), 5.61 - 5.46 (m, 2H), 5.30 - 5.18 (m, 2H), 4.91 - 4.80 (m, 2H), 4.78 - 4.71 (m, 1H), 4.66 - 4.60 (m, 1H), 4.24 - 4.15 (m, 1H), 3.96 - 3.86 (m, 2H), 3.80 - 3.67 (m, 3H), 3.64 - 3.51 (m, 3H), 3.49 - 3.39 (m, 1H), 3.25 - 3.15 (m, 1H), 3.14 - 3.03 (m, 1H), 3.00 - 2.91 (m, 1H), 2.91 - 2.81 (m, 6H), 2.81 - 2.75 (m, 1H), 2.44 - 2.31 (m, 5H), 2.30 - 2.22 (m, 2H), 2.02 - 1.97 (m, 2H), 1.86 - 1.75 (m, 2H), 1.72 - 1.65 (m, 1H), 1.16 - 1.08 (m, 1H), 0.84 - 0.75 (m, 1H), 0.62 - 0.45 (m, 4H), 0.40 - 0.31 (m, 2H), 0.07 - -0.06 (m, 2H).

Example 3-109: LD-109

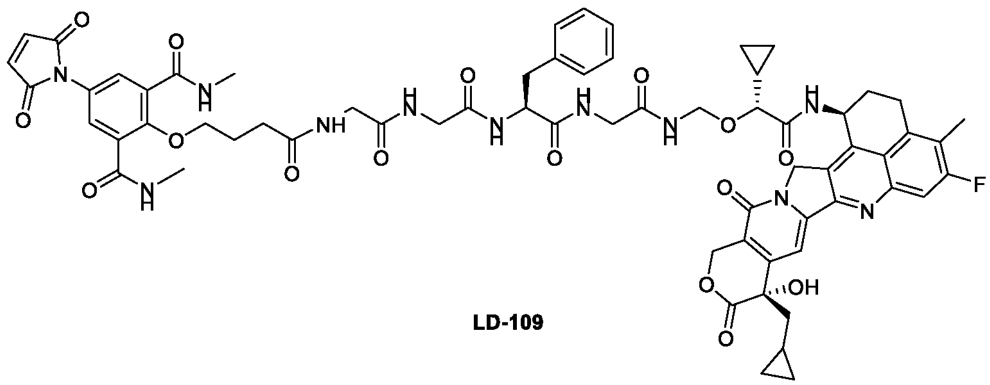

LD-109

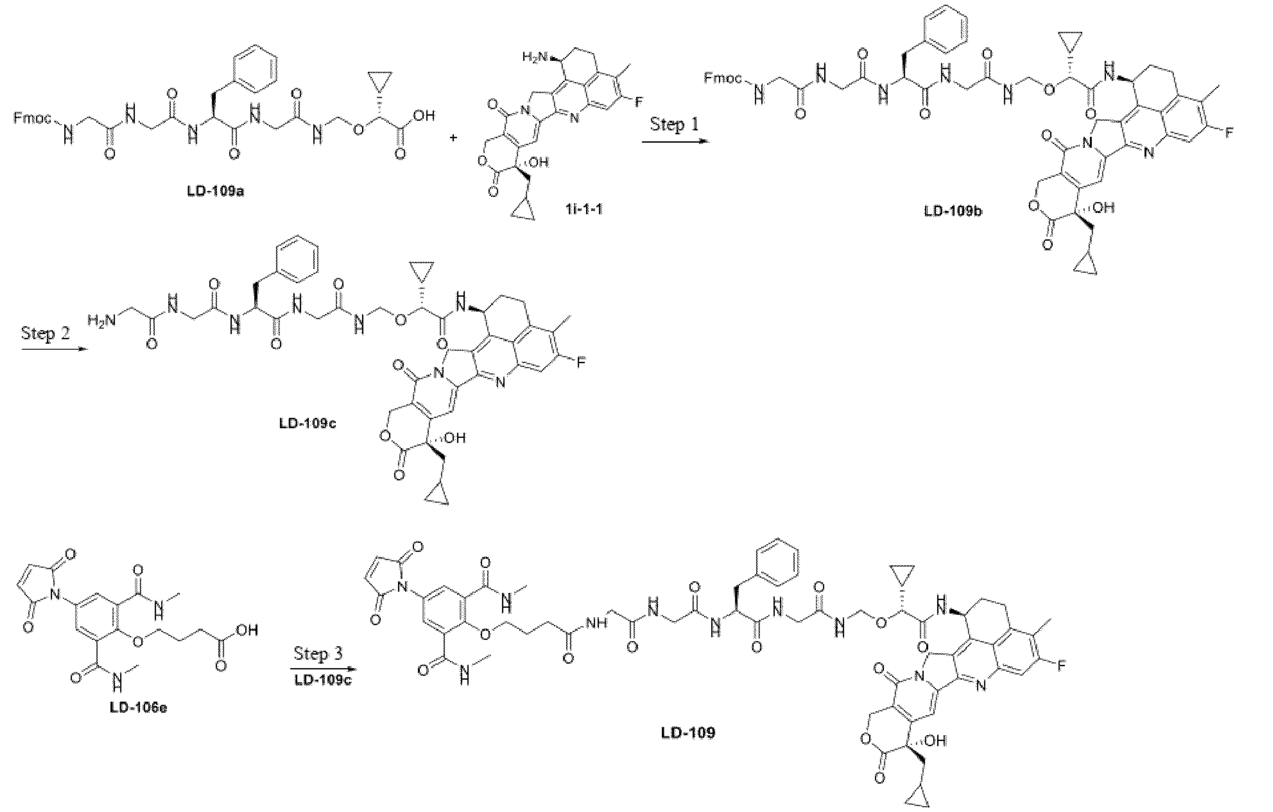


Step 1

**LD-109a** (135 mg, 197 μmol, prepared by applying the method disclosed in Example 1-18 on page 61 of patent application "WO2021190602 A1") and **1i-1-1** (100 mg, 179 μmol, HCl) were dissolved in 3 mL of *N,N*-dimethylformamide, then added with 1-hydroxybenzotriazole (36.2 mg, 268 μmol), *N,N*-diisopropylethylamine (69.3 mg, 536 μmol) and 1-(3-dimethylaminopropyl)- 3-ethylcarbodiimide hydrochloride (51.4 mg, 268 μmol) in sequence, and stirred at 25 °C for 2 hours. 5 mL of water was added to the reaction solution, dichloromethane (5 mLx3) was used for extraction, the organic phase was dried over anhydrous sodium sulfate and filtered, and the filtrate was concentrated by vacuum distillation. The obtained residue was purified by silica gel column chromatography with the eluent system A to obtain the title product **LD-109b** (180 mg, yield: 77%) as a yellow solid.

MS m/z (ESI): 1129.6 [M+1].

Step 2

**LD-109b** (100 mg, 88.5 μmol) was dissolved in 2 mL of acetonitrile, added with diethylamine (284 mg, 3.88 mmol), and stirred at 25 °C for 1 hour. The reaction solution was concentrated by vacuum distillation, and the residue was purified by pre-HPLC (chromatographic column: Welch Xtimate C18 150x25mmx5μm; mobile phase: A-water (0.1% trifluoroacetic acid), B-acetonitrile; gradient elution: B%: 40%-60%) to obtain the title product **LD-109c** (45.0 mg, yield: 54%) as a yellow solid.

MS m/z (ESI): 907.5 [M+1].

Step 3

**LD-106e** (10.7 mg, 25.3 μmol) and **LD-109c** (20.0 mg, 21.1 μmol) were dissolved in 1 mL of *N,N*-dimethylformamide, then added with 1-hydroxybenzotriazole (4.3 mg, 31.6 μmol), *N,N*-diisopropylethylamine (8.2 mg, 63.3 μmol) and 1-(3-dimethylaminopropyl)- 3-ethylcarbodiimide hydrochloride (6.1 mg, 31.6 μmol) in sequence, and stirred at 25 °C for 2 hours. 3 mL of water was added to the reaction solution, dichloromethane (5 mLx2) was used for extraction, the organic phase was dried over anhydrous sodium sulfate and filtered, and the filtrate was concentrated by vacuum distillation. The residue was purified by pre-HPLC (chromatographic column: Phenomenex luna C18 150x25mmx10μm; mobile phase: A-water (0.225% formic acid), B-acetonitrile; gradient elution: B%: 24%-54%) to obtain the title product **LD-109** (11 mg, yield: 40%) as a white solid.

MS m/z (ESI): 1278.6 [M+1].

$^1H$ NMR (400 MHz, CD3CN) δ 7.99 - 7.94 (m, 1H), 7.86 - 7.81 (m, 1H), 7.68 - 7.64 (m, 2H), 7.62 - 7.54 (m, 2H), 7.51 - 7.45 (m, 2H), 7.37 - 7.34 (m, 1H), 7.32 - 7.27 (m, 1H), 7.24 - 7.14 (m, 5H), 7.11 - 7.06 (m, 2H), 6.93 - 6.88 (m, 2H), 5.61 - 5.56 (m, 1H), 5.53 - 5.48 (m, 1H), 5.31 - 5.24 (m, 2H), 4.99 - 4.93 (m, 1H), 4.84 - 4.79 (m, 1H), 4.58 - 4.56 (m, 1H), 4.54 - 4.49 (m, 1H), 4.24 - 4.19 (m, 1H), 3.95 - 3.89 (m, 2H), 3.73 - 3.69 (m, 2H), 3.65 - 3.61 (m, 1H), 3.55 - 3.51 (m, 3H), 3.28 - 3.23 (m, 1H), 3.14 - 3.09 (m, 1H), 3.03 - 2.98 (m, 1H), 2.91 - 2.82 (m, 6H), 2.82 - 2.77 (m, 1H), 2.40 - 2.34 (m, 5H), 2.28 - 2.24 (m, 2H), 2.01 - 1.99 (m, 2H), 1.85 - 1.82 (m, 1H), 1.78 - 1.73 (m, 2H), 1.22 - 1.18 (m, 1H), 0.87 - 0.82 (m, 1H), 0.59 - 0.51 (m, 3H), 0.44 - 0.37 (m, 3H), 0.08 - 0.01 (m, 2H).

Example 3-110: LD-110

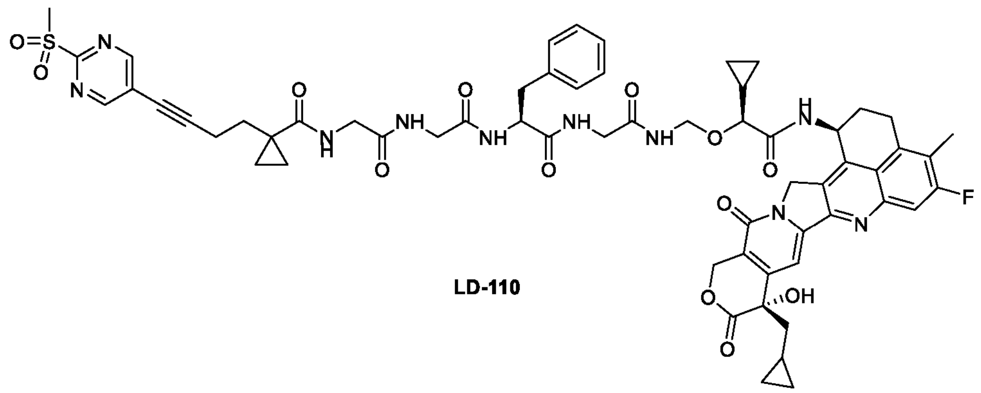

LD-110

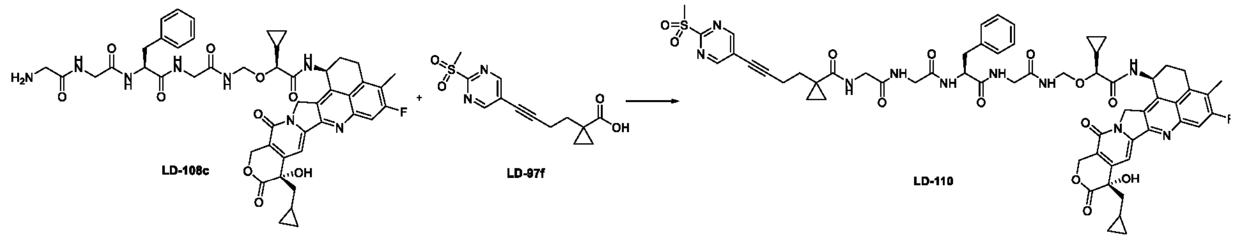

LD-108c

LD-97f

LD-110

**LD-97f** (4.55 mg, 16.2 μmol) and **LD-108c** (15.0 mg, 14.7 μmol, trifluoroacetate) were dissolved in 1 mL of *N,N*-dimethylformamide, then added with 1-hydroxybenzotriazole (2.98 mg, 22.0 μmol), *N,N*-diisopropylethylamine (5.70 mg, 44.1 μmol) and 1-(3-dimethylaminopropyl)- 3-ethylcarbodiimide hydrochloride (4.22 mg, 22.0 μmol) in sequence, and stirred at 25 °C for 1 hour. 50 mL of water was added to the reaction solution, and dichloromethane (45 mLx3) was used for extraction. The organic phase was dried over anhydrous sodium sulfate and filtered, and the filtrate was concentrated by vacuum distillation. The residue was purified by pre-HPLC (chromatographic column: Phenomenex Kinetex EVO C18 150x30mmx5μm; mobile phase: A-water (0.225% formic acid), B-acetonitrile; gradient elution: B%: 37%-57%) to obtain the title product **LD-110** (5.3 mg, yield: 31%) as a white solid.

MS m/z (ESI): 1183.2 [M+1].

$^1H$ NMR (400 MHz, $CD_3OD$) δ 8.91 - 8.82 (m, 2H), 7.68 - 7.58 (m, 2H), 7.27 - 7.10 (m, 5H), 5.71 - 5.64 (m, 1H), 5.61 - 5.54 (m, 1H), 5.53 - 5.45 (m, 1H), 5.34 - 5.28 (m, 1H), 5.21 - 5.13 (m, 2H), 4.78 - 4.74 (m, 1H), 4.36 - 4.30 (m, 1H), 3.91 - 3.71 (m, 6H), 3.65 - 3.58 (m, 2H), 3.35 - 3.34 (m, 3H), 3.22 - 3.16 (m, 1H), 2.96 - 2.87 (m, 2H), 2.72 - 2.65 (m, 2H), 2.46 - 2.39 (m, 3H), 2.37 - 2.31 (m, 1H), 2.27 - 2.19 (m, 1H), 2.02 - 1.92 (m, 2H), 1.90 - 1.83 (m, 1H), 1.79 - 1.71 (m, 1H), 1.26 - 1.10 (m, 4H), 0.86 - 0.80 (m, 2H), 0.66 - 0.51 (m, 4H), 0.45 - 0.33 (m, 2H), 0.09 - 0.03 (m, 1H), 0.01 - -0.07 (m, 1H).

Example 3-111: LD-111

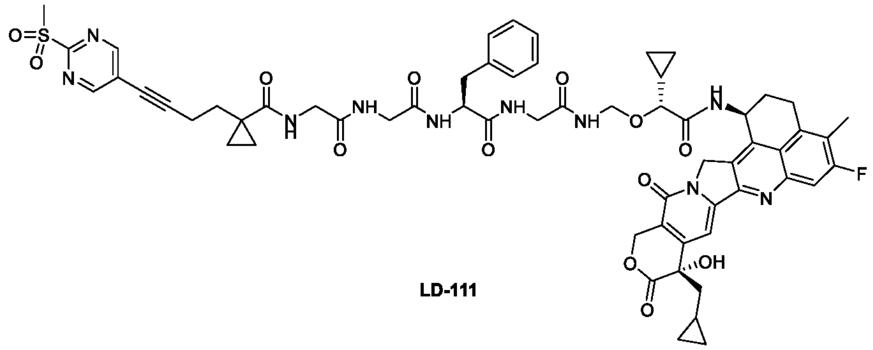

LD-111

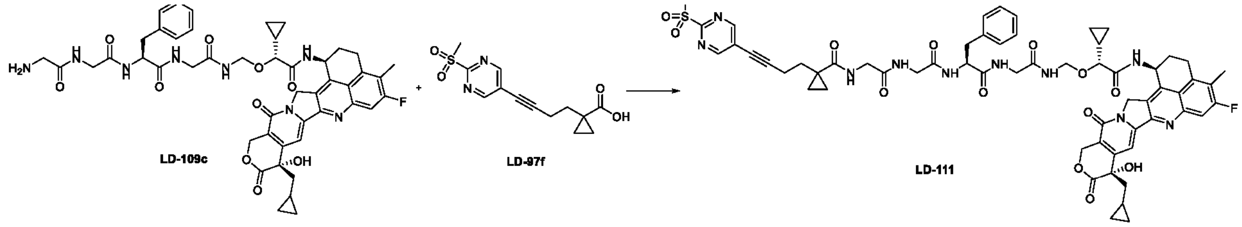

LD-109c LD-97f LD-111

**LD-97f** (7.1 mg, 24.2 μmol) and **LD-109c** (20.9 mg, 22.1 μmol) were dissolved in 0.2 mL of *N,N*-dimethylformamide, then added with 1-hydroxybenzotriazole (4.5 mg, 33.0 μmol), *N,N*-diisopropylethylamine (8.6 mg, 66.2 μmol) and 1-(3-dimethylaminopropyl)-3-ethylcarbodiimide hydrochloride (6.3 mg, 33.1 μmol) in sequence, and stirred at 25 °C for 2 hours. 5 mL of water was added to the reaction solution, dichloromethane (5 mLx3) was used for extraction, the organic phase was dried over anhydrous sodium sulfate and filtered, and the filtrate was concentrated by vacuum distillation. The residue was purified by pre-HPLC (chromatographic column: Phenomenex Kinetex EVO C18 150x30mmx5μm; mobile phase: A-water (0.225% formic acid), B-acetonitrile; gradient elution: B%: 37%-57%) to obtain the title product **LD-111** (5.58 mg, yield: 20%) as a white solid.

MS m/z (ESI): 1183.6 [M+1].

$^1$H NMR (400 MHz, $CD_3OD$) δ 8.90 - 8.82 (m, 2H), 7.68 - 7.62 (m, 2H), 7.25 - 7.11 (m, 5H), 5.72 - 5.66 (m, 1H), 5.59 - 5.54 (m, 1H), 5.50 - 5.45 (m, 1H), 5.37 - 5.32 (m, 1H), 5.23 - 5.16 (m, 2H), 4.70 - 4.65 (m, 1H),4.40 - 4.36 (m, 1H), 3.83 - 3.76 (m, 4H), 3.70 - 3.60 (m, 3H), 3.35 - 3.34 (m, 3H), 3.16 - 3.11 (m, 1H), 3.08 - 3.02 (m, 1H), 2.93 - 2.87 (m, 1H), 2.72 - 2.67 (m, 2H), 2.47 - 2.39 (m, 3H), 2.37 - 2.28 (m, 2H), 2.04 - 1.88 (m, 4H), 1.84 - 1.77 (m, 1H), 1.17 - 1.10 (m, 2H), 0.93 - 0.83 (m, 2H), 0.82 - 0.76 (m, 2H), 0.69 - 0.58 (m, 3H), 0.56 - 0.50 (m, 1H), 0.47 - 0.37 (m, 2H), 0.13 - 0.07 (m, 1H), 0.04 - -0.01 (m, 1H).

Example 3-112: LD-112

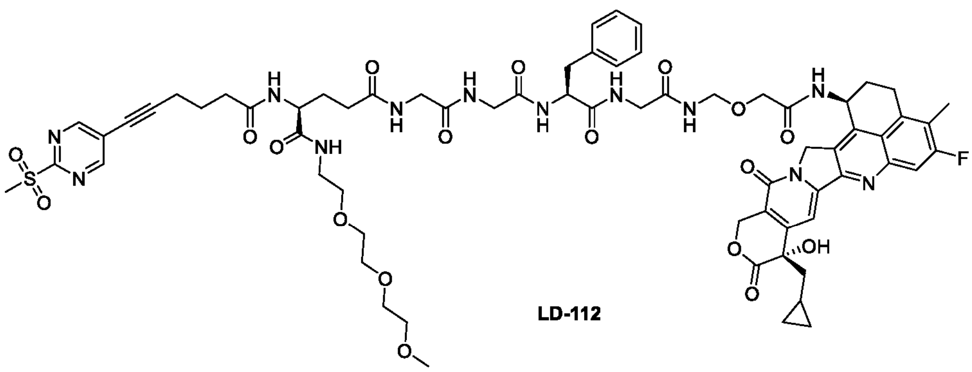

LD-112

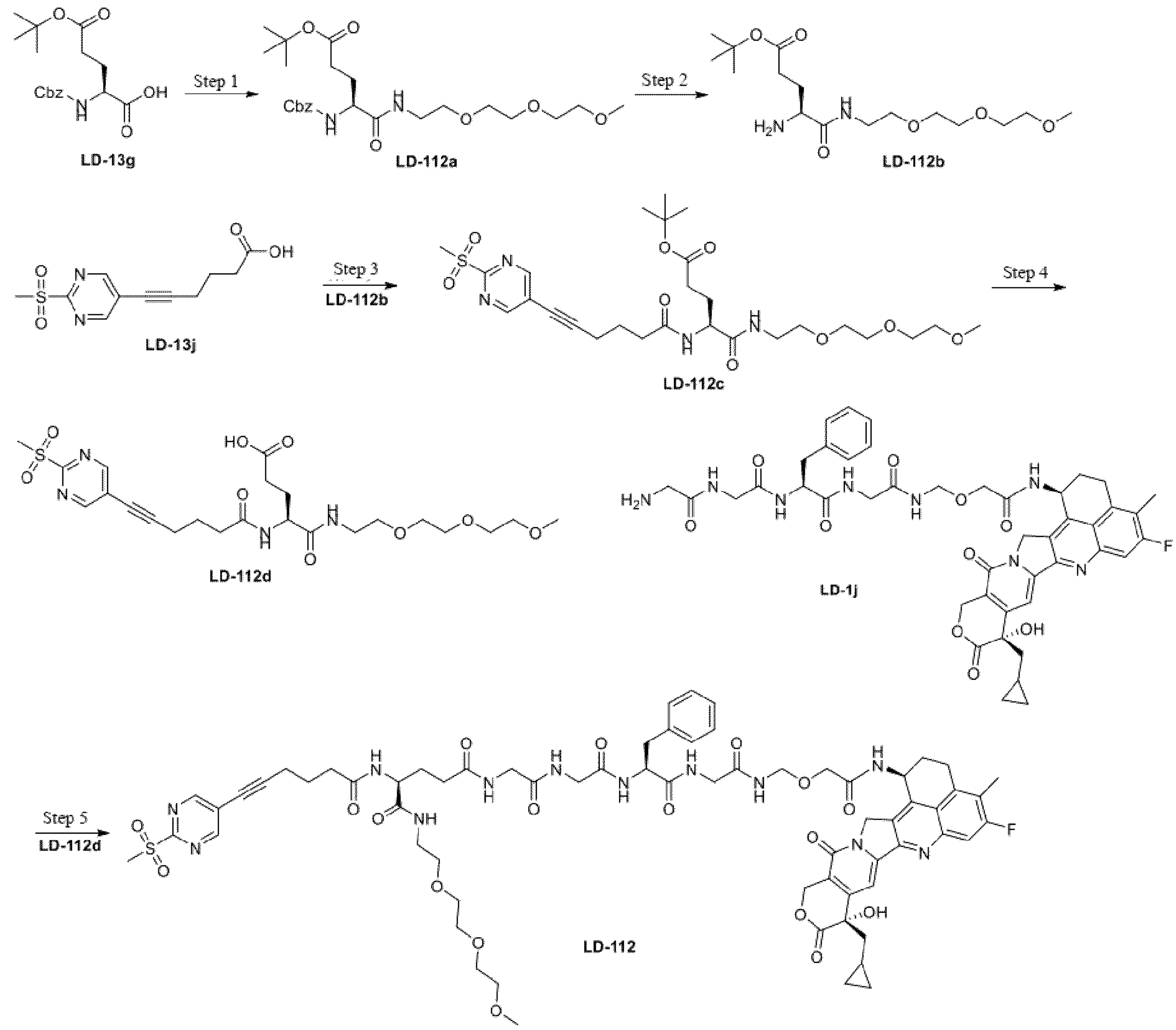


Step 1

**LD-13g** (500 mg, 1.48 mmol) was dissolved in 5 mL of *N,N*-dimethylformamide, then added with 2-(7-azobenzotriazole)-*N,N,N',N'*-tetramethyluronium hexafluorophosphate (564 mg, 1.48 mmol), *N,N*-diisopropylethylamine (574 mg, 4.44 mmol) and 3,6,9-trioxa-1-aminodecane (242 mg, 1.48 mmol) in sequence, and stirred at 25 °C for 2 hours. 15 mL of water was added, and ethyl acetate (30 mLx3) was used for extraction. The organic phase was dried over anhydrous sodium sulfate and filtered, and the filtrate was concentrated by distillation under reduced pressure. The resulting residue was purified by silica gel column chromatography with the eluent system A to obtain the title product **LD-112a** (600 mg, yield: 84%).

MS m/z (ESI): 483.3 [M+1].

Step 2

**LD-112a** (600 mg, 1.24 mmol) was dissolved in 6 mL of methanol, added with palladium-on-carbon (60 mg, 10%, wetted with ca. 55% water) under nitrogen protection, subjected to replacement with hydrogen gas, and stirred at 25 °C for 1 hour. The reaction solution was subjected to replacement with hydrogen gas three times, and stirred at 25 °C for 1 hour under hydrogen gas (15 Psi). The reaction solution was filtered through Celite, and the filtrate was concentrated by vacuum distillation to obtain the title product **LD-112b** (350 mg, yield: 81%) as a colorless oil.

MS m/z (ESI): 349.3 [M+ 1].

Step 3

**LD-112b** (350 mg, 1.01 mmol) was dissolved in 5 mL of N,N-dimethylformamide, then added with 2-(7-azobenzotriazole)-*N,N,N',N'*-tetramethyluronium hexafluorophosphate (384 mg, 1.01 mmol), *N,N*-diisopropylethylamine (390 mg, 3.02 mmol) in sequence, then added with **LD-13j** (271 mg, 1.01 mmol), and stirred at 25 °C for 2 hours. 1 mL of water was added, and dichloromethane (3 mLx2) was used for extraction. The organic phase was dried over anhydrous sodium sulfate and filtered, and the filtrate was concentrated by vacuum distillation. The residue was purified by reverse phase high performance liquid chromatography (mobile phase: A-water, B-acetonitrile; isocratic elution: B%: 36%) to obtain the title product **LD-112c** (301.78 mg, yield: 50%) as a white solid.

MS m/z (ESI): 599.2 [M+1].

Step 4

**LD-112c** (50.0 mg, 0.08 mmol) was dissolved in 1 mL of dichloromethane, then slowly added with 0.5 mL of trifluoroacetic acid, and stir at 25 °C for 0.5 hours. The reaction solution was concentrated by distillation to obtain the crude title product **LD-112d** (45 mg) as a colorless oil. The product was used directly in the next reaction without purification.

MS m/z (ESI): 543.24 [M+1].

Step 5

**LD-112d** (8.9 mg, 16 μmol) and formate of **LD-1j** (15.0 mg, 16.4 μmol) were in 1.5 mL of *N,N*-dimethylformamide, then added with 1-hydroxybenzotriazole (3.3 mg, 25 μmol), *N,N*-diisopropylethylamine (6.4 mg, 49 μmol) and 1-(3-dimethylaminopropyl)- 3-ethylcarbodiimide hydrochloride (4.7 mg, 25 μmol) in sequence, and stirred at 25 °C for 1 hour. 50 mL of water was added to the reaction solution, and dichloromethane (45 mLx3) was used for extraction. The organic phase was dried over anhydrous sodium sulfate and filtered, and the filtrate was concentrated by vacuum distillation. The residue was purified by pre-HPLC (chromatographic column: Phenomenex luna C18 150x25mmx10μm; mobile phase: A-water (0.225% formic acid), B-acetonitrile; gradient elution: B%: 26%-56%) to obtain the title product **LD-112** (7.80 mg, yield: 34%) as a white solid.

MS m/z (ESI): 1391.4 [M+1].

$^1$H NMR (400 MHz, $CD_3OD$) δ 8.97 - 8.87 (m, 2H), 7.68 - 7.58 (m, 2H), 7.29 - 7.13 (m, 5H), 5.76 - 5.69 (m, 1H), 5.61 - 5.53 (m, 1H), 5.38 - 5.27 (m, 2H), 5.16 - 5.06 (m, 1H), 4.84 - 4.79 (m, 2H), 4.68 - 4.63 (m, 1H), 4.40 - 4.32 (m, 2H), 4.27 - 4.21 (m, 1H), 4.18 - 4.13 (m, 1H), 3.94 - 3.81 (m, 4H), 3.69 - 3.64 (m, 2H), 3.62 - 3.59 (m, 1H), 3.59 - 3.58 (m, 3H), 3.54 - 3.47 (m, 4H), 3.43 - 3.36 (m, 2H), 3.36 - 3.34 (m, 3H), 3.33 - 3.32 (m, 2H), 3.28 - 3.25 (m, 2H), 3.21 - 3.17 (m, 1H), 3.00 - 2.96 (m, 1H), 2.95 - 2.90 (m, 1H), 2.58 - 2.53 (m, 2H), 2.50 - 2.31 (m, 9H), 2.28 - 2.23 (m, 1H), 2.11 - 2.04 (m, 1H), 1.96 - 1.85 (m, 4H), 1.81 - 1.74 (m, 1H), 0.89 - 0.82 (m, 1H), 0.44 - 0.35 (m, 2H), 0.10 - 0.04 (m, 1H), 0.01 - -0.05 (m, 1H).

Example 3-113: LD-113

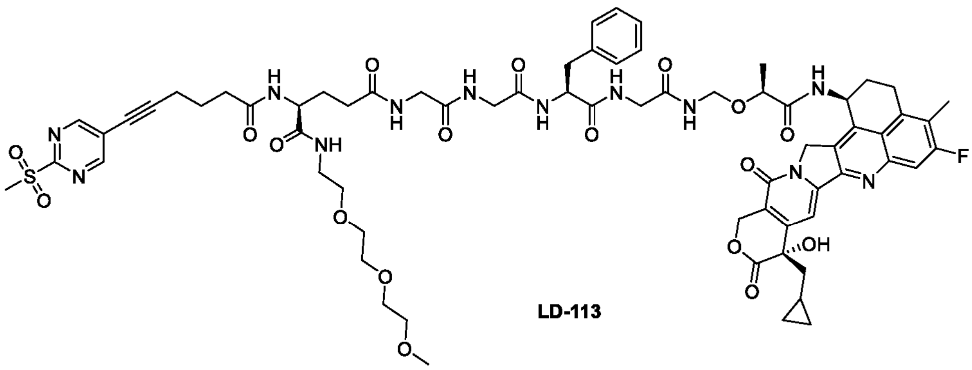

LD-113

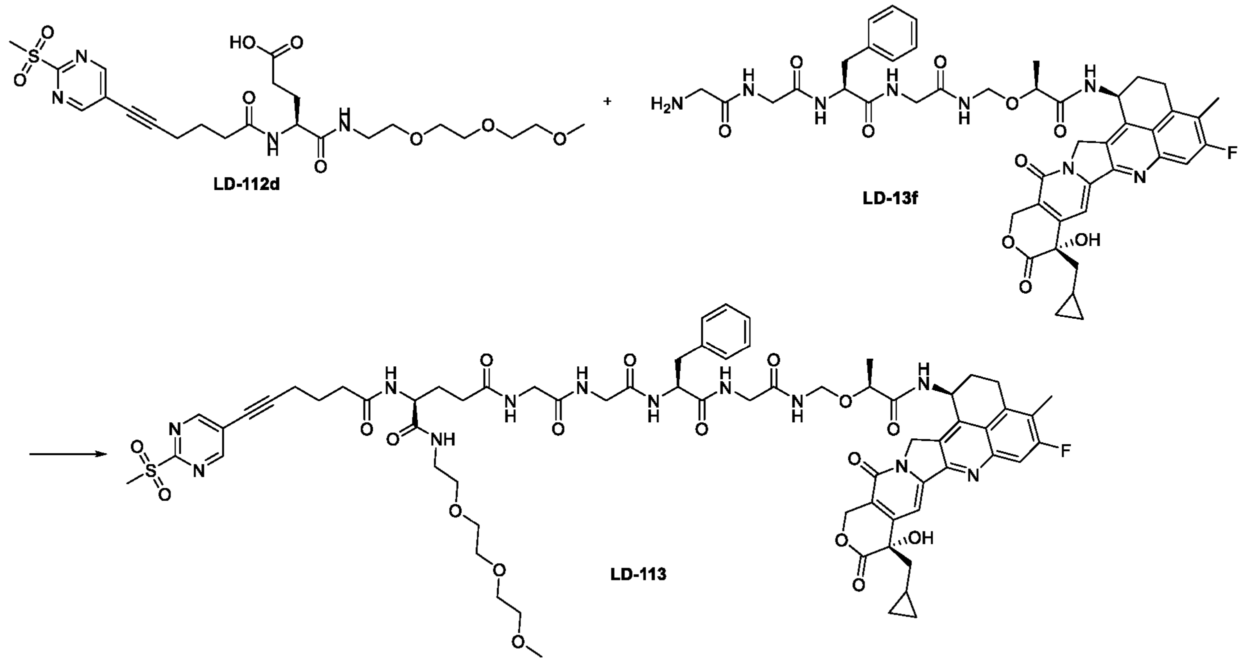

LD-112d

LD-13f

LD-113

**LD-112d** (14.8 mg, 27.2 μmol), **LD-13f** (22.0 mg, 25.0 μmol) and 2-(7-azobenzotriazole)-*N,N,N',N'*-tetramethyluronium hexafluorophosphate (11.3 mg, 29.7 μmol) were dissolved in 1 mL of *N,N*-dimethylformamide, then added with *N,N*-diisopropylethylamine (9.6 mg, 74.2 μmol), and stirred at 25 °C for 1 hour. The reaction solution was concentrated by vacuum distillation and the residue was purified by Pre-HPLC purification (chromatographic column: InfinityLab Poroshell 120 SB-C18 21.2x150mm, 4μm; mobile phase: A-water (0.1% formic acid), B-acetonitrile; gradient elution: B%: 10%-90%) to obtain the title product **LD-113** (19.45 mg, yield: 55%) as a white solid.

MS m/z (ESI): 1405.9 [M+1].

$^1$H NMR (400 MHz, DMSO-d6) δ 9.14 - 9.08 (m, 2H), 8.70 - 8.64 (m, 1H), 8.61 - 8.54 (m, 1H), 8.37 - 8.29 (m, 1H), 8.14 - 7.95 (m, 5H), 7.80 - 7.75 (m, 1H), 7.40 - 7.36 (m, 1H), 7.27 - 7.13 (m, 5H), 6.63 - 6.57 (m, 1H), 5.58 - 5.50 (m, 1H), 5.47 - 5.35 (m, 2H), 5.31 - 5.15 (m, 2H), 4.77 - 4.68 (m, 1H), 4.62 - 4.54 (m, 1H), 4.51 - 4.41 (m, 1H), 4.26 - 4.18 (m, 1H), 4.16 - 4.08 (m, 1H), 3.77 - 3.70 (m, 1H)), 3.69 - 3.64 (m, 3H), 3.62 - 3.54 (m, 1H), 3.51 - 3.45 (m, 6H), 3.42 - 3.36 (m, 7H), 3.34 - 3.34 (m, 2H), 3.26 - 3.09 (m, 7H)), 3.04 - 2.95 (m, 1H), 2.79 - 2.69 (m, 1H), 2.57 - 2.51 (m, 2H), 2.41 - 2.34 (m, 3H), 2.34 - 2.26 (m, 2H), 2.18 - 2.11 (m, 3H), 1.90 - 1.66 (m, 6H), 1.35 - 1.27 (m, 3H), 0.86 - 0.75 (m, 1H), 0.40 - 0.25 (m, 2H), 0.09 - 0.02 (m, 1H), -0.04 - -0.13 (m, 1H).

Example 3-114: LD-114

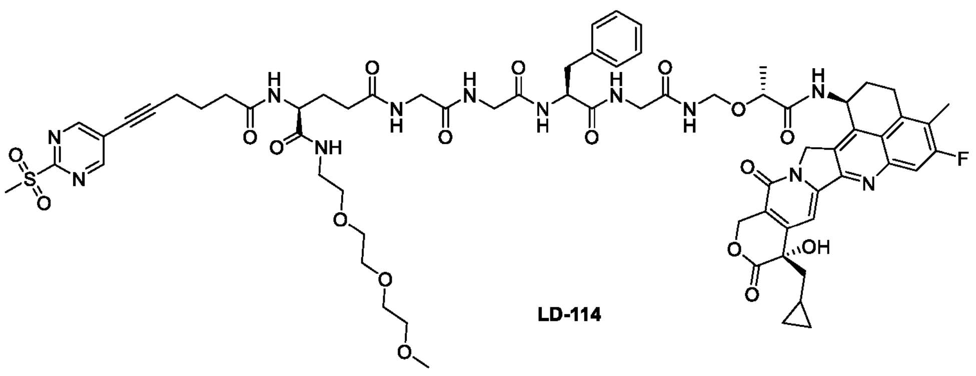

LD-114

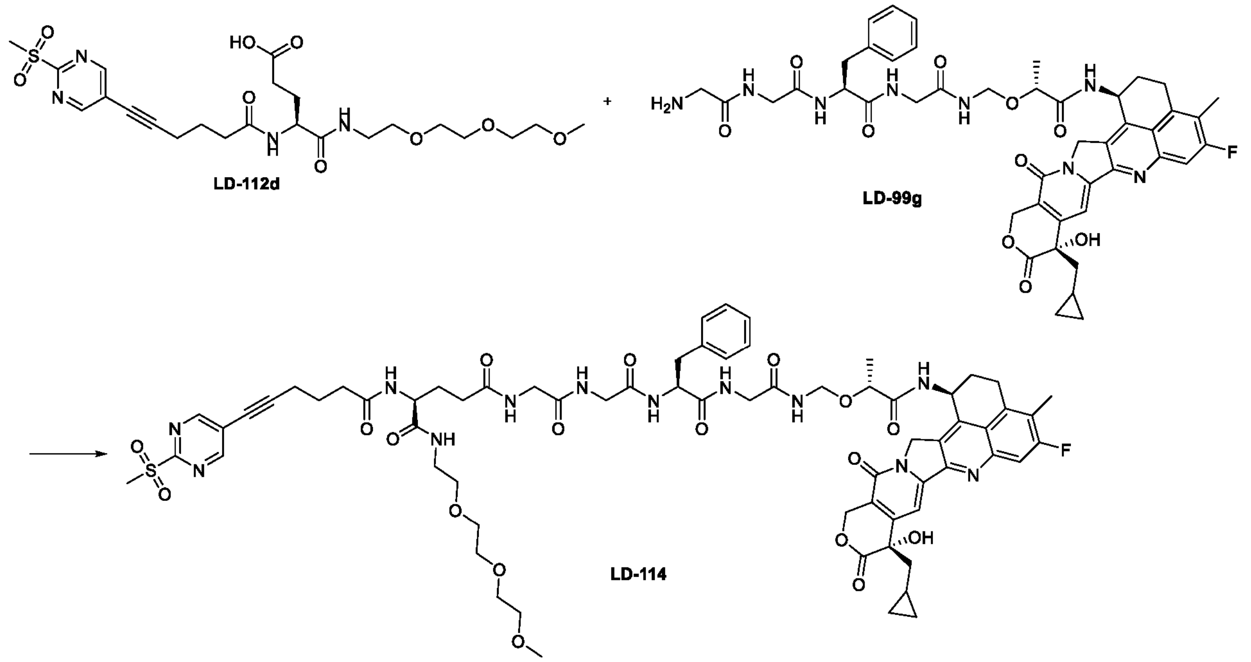

LD-112d

LD-99g

LD-114

**LD-112d** (10.2 mg, 18.7 μmol) and **LD-99g** (15.0 mg, 17.0 μmol) were dissolved in 1.0 mL of *N,N*-dimethylformamide, then added with 1-hydroxybenzotriazole (3.5 mg, 25.5 μmol), *N,N*-diisopropylethylamine (6.6 mg, 51 μmol) and 1-(3-dimethylaminopropyl)-3-ethylcarbodiimide hydrochloride (4.9 mg, 25.5 μmol), and stirred at 25 °C for 2 hours. 3 mL of water was added to the reaction solution, dichloromethane (5 mLx2) was used for extraction, the organic phase was dried over anhydrous sodium sulfate and filtered, and the filtrate was concentrated by vacuum distillation. The obtained residue was purified by pre-HPLC (chromatographic column: Phenomenex luna C18 150x25mmx10μm; mobile phase: A-water (0.225% formic acid), B-acetonitrile; gradient elution: B%: 28%-58%) to obtain the title product **LD-114** (10.1 mg, yield: 42%) as a white solid.

MS m/z (ESI): 1405.7 [M+1].

$^1$H NMR (400 MHz, $CD_3OD$) δ 8.95 - 8.88 (m, 2H), 7.68 - 7.61 (m, 2H), 7.29 - 7.21 (m, 2H), 7.20 - 7.12 (m, 3H), 5.68 - 5.64 (m, 1H), 5.60 - 5.54 (m, 1H), 5.42 - 5.33 (m, 2H), 5.16 - 5.10 (m, 1H), 4.82 - 4.79 (m, 1H), 4.70 - 4.66 (m, 1H), 4.43 - 4.35 (m, 2H), 4.26 - 4.20 (m, 1H), 3.90 - 3.80 (m, 4H), 3.78 - 3.73 (m, 1H), 3.71 - 3.66 (m, 1H), 3.63 - 3.54 (m, 6H), 3.54 - 3.46 (m, 5H), 3.43 - 3.35 (m, 4H), 3.35 - 3.33 (m, 3H), 3.27 - 3.22 (m, 1H), 3.21 - 3.16 (m, 1H), 3.15 - 3.12 (m, 1H), 3.07 - 3.02 (m, 1H), 2.92 - 2.86 (m, 1H), 2.59 - 2.52 (m, 2H), 2.47 - 2.38 (m, 5H), 2.36 - 2.29 (m, 3H), 2.11 - 2.04 (m, 1H), 1.99 - 1.87 (m, 4H), 1.85 - 1.79 (m, 1H), 1.60 - 1.51 (m, 3H), 0.92 - 0.85 (m, 1H), 0.49 - 0.39 (m, 2H), 0.15 - 0.08 (m, 1H), 0.06 - 0.00 (m, 1H).

Example 3-115: LD-115

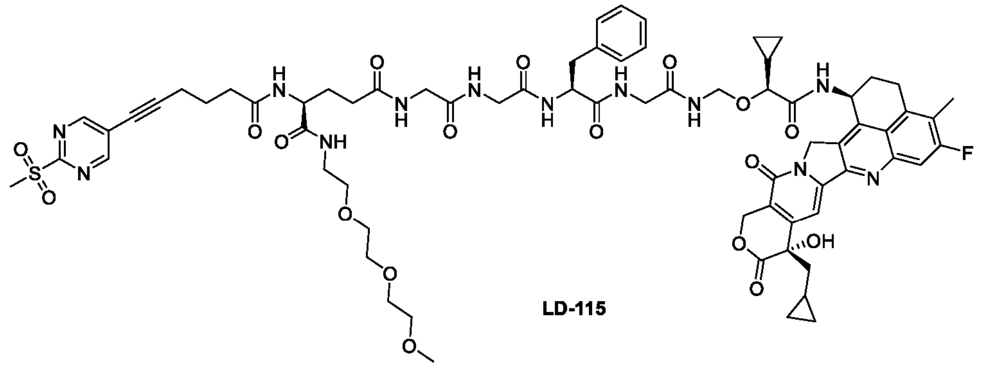

LD-115

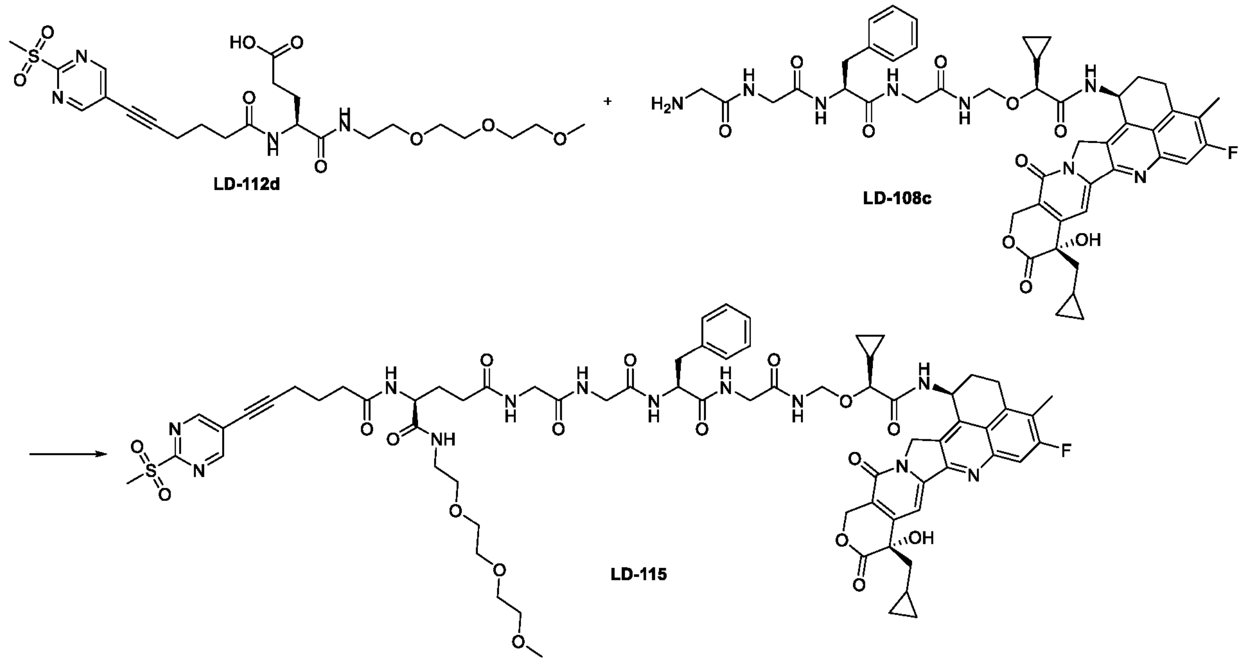

LD-112d

LD-108c

LD-115

**LD-112d** (8.4 mg, 16 μmol) and formate of **LD-108c** (15.0 mg, 15.5 μmol) were dissolved in 1.5 mL ofN,N-dimethylformamide, then added with 1-hydroxybenzotriazole (3.2 mg, 23 μmol), *N,N*-diisopropylethylamine (6.0 mg, 47 μmol) and 1-(3-dimethylaminopropyl)- 3-ethylcarbodiimide hydrochloride (4.5 mg, 23 μmol) in sequence, and stirred at 25 °C for 3 hours. 50 mL of water was added to the reaction solution, dichloromethane (45 mLx3) was used for extraction. The organic phase was dried over anhydrous sodium sulfate and filtered, and the filtrate was concentrated by vacuum distillation. The residue was purified by pre-HPLC (chromatographic column: Phenomenex luna C18 150x25mmx10μm; mobile phase: A-water (0.225% formic acid), B-acetonitrile; gradient elution: B%: 30%-60%) to obtain the title product **LD-115** (8.37 mg, yield: 37%) as a white solid.

MS m/z (ESI): 1431.2 [M+1].

$^1$H NMR (400 MHz, $CD_3OD$) δ 8.96 - 8.89 (m, 2H), 7.65 - 7.57 (m, 2H), 7.26 - 7.13 (m, 5H), 5.70 - 5.64 (m, 1H), 5.60 - 5.54 (m, 1H), 5.49 - 5.41 (m, 1H), 5.34 - 5.28 (m, 1H), 5.14 - 5.07 (m, 1H), 4.77 - 4.72 (m, 1H), 4.41 - 4.33 (m, 2H), 3.92 - 3.86 (m, 3H), 3.80 - 3.75 (m, 2H), 3.69 - 3.65 (m, 2H), 3.62 - 3.57 (m, 6H), 3.56 - 3.47 (m, 5H), 3.44 - 3.37 (m, 2H), 3.36 - 3.34 (m, 3H), 3.33 - 3.33 (m, 2H), 3.29 - 3.23 (m, 2H), 3.21 - 3.11 (m, 2H), 3.02 - 2.96 (m, 1H), 2.92 - 2.84 (m, 1H), 2.58 - 2.54 (m, 2H), 2.45 - 2.40 (m, 2H), 2.39 - 2.33 (m, 5H), 2.23 - 2.17 (m, 1H), 2.11 - 2.02 (m, 1H), 1.97 - 1.84 (m, 4H), 1.79 - 1.73 (m, 1H), 1.24 - 1.16 (m, 1H), 0.89 - 0.81 (m, 1H), 0.66 - 0.52 (m, 4H), 0.45 - 0.34 (m, 2H), 0.11 - 0.04 (m, 1H), 0.01 - -0.06 (m, 1H).

Example 3-116: LD-116

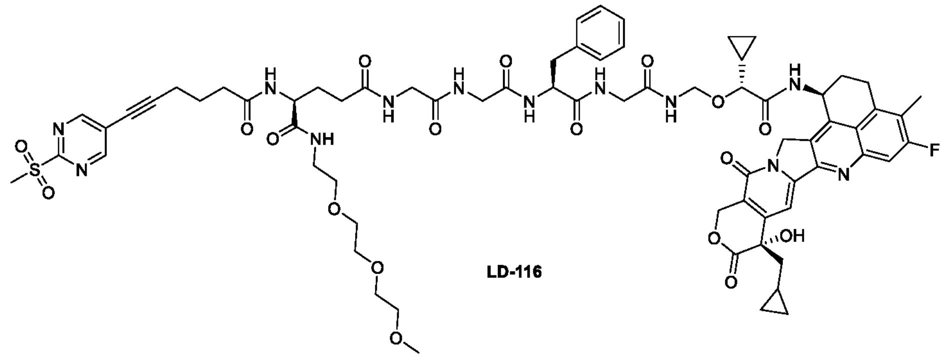

LD-116

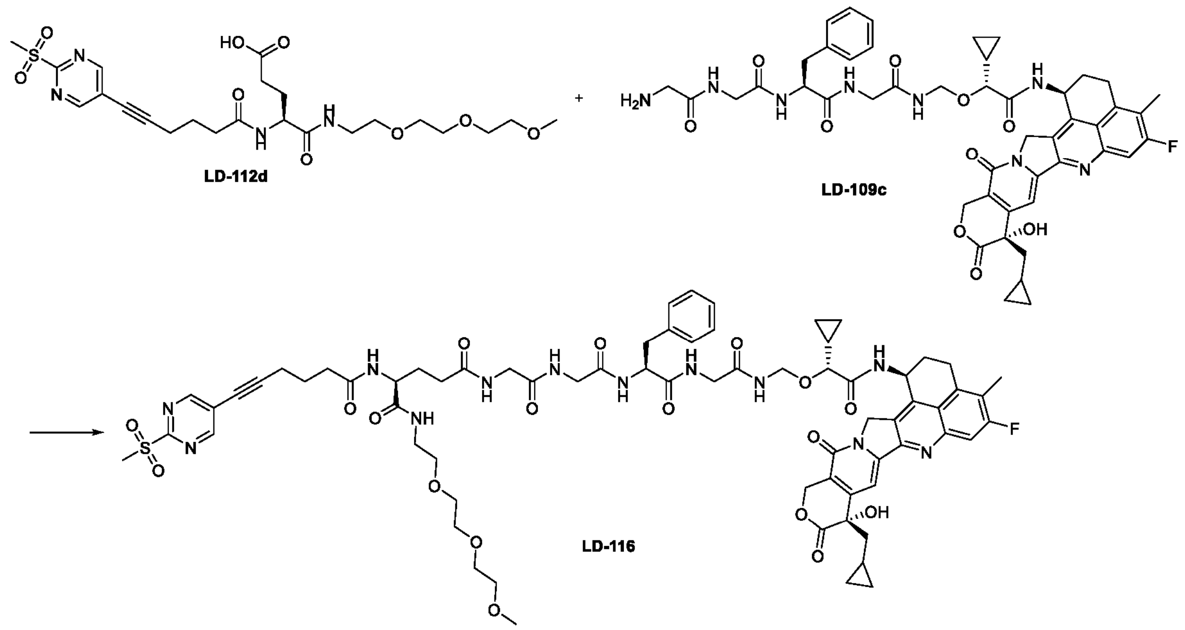

LD-112d

LD-109c

LD-116

**LD-112d** (9.17 mg, 16.9 μmol) and formate of **LD-109c** (15.0 mg, 15.3 μmol) were dissolved in 1.5 mL of *N,N*-dimethylformamide, then added with 1-hydroxybenzotriazole (3.11 mg, 23.0 μmol), *N,N*-diisopropylethylamine (5.96 mg, 46.0 μmol) and 1-(3-dimethylaminopropyl)-3-ethylcarbodiimide hydrochloride (4.42 mg, 23.0 μmol) in sequence, and stirred at 25 °C for 2 hours. 3 mL of water was added to the reaction solution, dichloromethane (5 mLx2) was used for extraction, the organic phase was dried over anhydrous sodium sulfate and filtered, and the filtrate was concentrated by vacuum distillation. The obtained residue was purified by pre-HPLC (chromatographic column: Phenomenex luna C18 150x25mmx10μm; mobile phase: A-water (0.225% formic acid), B-acetonitrile; gradient elution: B%: 27%-57%) to obtain the title product **LD-116** (8.75 mg, yield: 39%) as a white solid.

MS m/z (ESI): 1431.7 [M+1].

$^{1}$H NMR (400 MHz, $CD_3OD$) δ 8.94 - 8.89 (m, 2H), 7.65 - 7.55 (m, 2H), 7.26 - 7.20 (m, 2H), 7.19 - 7.10 (m, 3H), 5.73 - 5.65 (m, 1H), 5.60 - 5.52 (m, 1H), 5.45 - 5.31 (m, 2H), 5.13 - 5.05 (m, 1H), 4.68 - 4.62 (m, 1H), 4.42 - 4.34 (m, 2H), 3.90 - 3.83 (m, 3H), 3.81 - 3.75 (m, 2H), 3.74 - 3.45 (m, 14H), 3.43 - 3.34 (m, 5H), 3.32 - 3.32 (m, 2H), 3.29 - 3.25 (m, 2H), 3.22 - 3.16 (m, 1H), 3.07 - 3.00 (m, 1H), 2.92 - 2.85 (m, 1H), 2.60 - 2.52 (m, 2H), 2.44 - 2.42 (m, 1H), 2.41 - 2.39 (m, 3H), 2.38 - 2.32 (m, 3H), 2.31 - 2.26 (m, 1H), 2.12 - 2.03 (m, 1H), 1.97 - 1.87 (m, 4H), 1.84 - 1.76 (m, 1H), 1.33 - 1.27 (m, 1H), 0.92 - 0.84 (m, 1H), 0.70 - 0.58 (m, 3H), 0.56 - 0.49 (m, 1H), 0.47 - 0.37 (m, 2H), 0.14 - 0.06 (m, 1H), 0.05 - -0.03 (m, 1H).

IV. ADC Conjugate Examples

Steps for Preparation of Antibody Conjugates:

A: An antibody was exchanged into 50 mM PBS/1.0 mM EDTA (ethylenediaminetetraacetic acid) buffer (adjusted with sodium hydroxide solution to pH 6.0) using an ultrafiltration tube with a molecular weight cutoff of 50 kD, 5 to 10

equivalents of 10 mM TCEP (tris(2-carboxyethyl)phosphine) aqueous solution was added, and shaken at 37 °C for 3 hours. A linker-drug conjugate was dissolved in DMSO, 10 to 20 equivalents of the linker-drug conjugate was taken therefrom and added dropwise to the reduced antibody solution, mixed well by vortexing and shaking, and the shaking was continued at 25 °C for 2 hours. After the reaction was completed, 200 equivalents of 100 mM NAC (N-acetylcysteine) aqueous solution was added, shaken at 25 °C for 20 minutes to terminate the linking reaction, an ultrafiltration tube with a molecular weight cutoff of 50 kD or a Sephadex G-25 desalting column was used to remove excess small molecules, and the antibody-drug conjugate was exchanged into 10 mM PBS buffer (pH 6.0). After ultrafiltration purification, the sample was filtered with a 0.22 μm filter membrane to obtain the unhydrolyzed antibody-drug conjugate, which was stored in a refrigerator at 4 °C. A part of the unhydrolyzed antibody-drug conjugate was taken, and exchanged into 50 mM PBS/1.0 mM EDTA buffer (pH 7.8) using an ultrafiltration tube with a molecular weight cutoff of 50 kD, and shaken at a constant temperature of 35 °C for 2 hours, the antibody-drug conjugate was exchanged into 10 mM PBS buffer (pH 6.0) using an ultrafiltration tube with a molecular weight cutoff of 50 kD. After ultrafiltration purification, the sample was filtered with a 0.22 μm filter membrane to obtain the hydrolyzed antibody-drug conjugate, which was stored in a refrigerator at 4 °C. The DAR value of the conjugate was determined by reversed-phase high-performance liquid chromatography or mass spectrometry. A-1: An antibody was exchanged into 50 mM PBS/1.0 mM EDTA buffer (adjusted with sodium hydroxide solution to pH 6.5) using an ultrafiltration tube with a molecular weight cutoff of 50 kD, 5 to 10 equivalents of 10 mM TCEP aqueous solution was added and shaken at 25 °C for 3 hours. A linker-drug conjugate was dissolved in DMSO, 10 to 20 equivalents of the linker-drug conjugate was taken therefrom, added dropwise to the reduced antibody solution, mixed well by vortexing and shaking, and the shaking was continued at 25 °C for 2 hours; after the reaction was completed, 40 equivalents of 100 mM NAC aqueous solution was added and shaken at 25 °C for 20 minutes to terminate the linking reaction, and an ultrafiltration tube with a molecular weight cutoff of 50 kD or a Sephadex G-25 desalting column was used to remove excess small molecules, the antibody-drug conjugate was exchanged into 50 mM PBS buffer (pH 6.0). After ultrafiltration purification, the sample was filtered with a 0.22 μm filter membrane to obtain the antibody-drug conjugate, which was stored in a refrigerator at 4 °C. A part of the unhydrolyzed antibody-drug conjugate was taken and exchanged into 50 mM PBS/1.0 mM EDTA buffer (pH 7.6) using an ultrafiltration tube with a molecular weight cutoff of 50 kD, and shaken at a constant temperature of 35 °C for 2 hours. The antibody-drug conjugate was exchanged into 50 mM PBS buffer (pH 6.0) using an ultrafiltration tube with a molecular weight cutoff of 50 kD. After ultrafiltration purification, the sample was filtered with a 0.22 μm filter membrane to obtain the hydrolyzed antibody-drug conjugate, which was stored in a refrigerator at 4 °C. The DAR value of the conjugate was determined by reversed-phase high-performance liquid chromatography or mass spectrometry.

B: An antibody was exchanged into 50 mM PBS/1.0 mM EDTA buffer (adjusted with sodium hydroxide solution to pH 6.5) using an ultrafiltration tube with a molecular weight cutoff of 50 kD, 5 to 10 equivalents of 10 mM TCEP aqueous solution was added, and shaken at 25 °C for 3 hours. A linker-drug conjugate was dissolved in DMSO, 10 to 20 equivalents of the linker-drug conjugate was taken therefrom, added dropwise to the reduced antibody solution, and mixed well by vortexing and shaking, and the shaking was continued at 25 °C for 2 hours; after the reaction was completed, 40 equivalents of 100 mM NAC aqueous solution was added and shaken at 25 °C for 20 minutes to terminate the linking reaction, an ultrafiltration tube with a molecular weight cutoff of 50 kD or a Sephadex G-25 desalting column was used to remove excess small molecules, and the antibody-drug conjugate was exchanged into 50 mM PBS buffer (pH 6.0). After ultrafiltration purification, the sample was filtered with a 0.22 μm filter membrane to obtain the antibody-drug conjugate, which was stored in a refrigerator at 4 °C. The DAR value of the conjugate was determined by reversed-phase high-performance liquid chromatography or mass spectrometry.

C: An antibody was exchanged into 50 mM PBS/1.0 mM EDTA buffer (adjusted with sodium hydroxide solution to pH 7.4) using an ultrafiltration tube with a molecular weight cutoff of 50 kD, 5 to 10 equivalents of 10 mM TCEP aqueous solution was added and shaken at 25 °C for 3 hours. A linker-drug conjugate was dissolved in DMSO, 10 to 20 equivalents of the linker-drug conjugate was taken therefrom, added to the reduced antibody solution, and mixed well by vortexing and shaking, and the shaking was continued at 25 °C for 2 hours; after the reaction was completed, 40 equivalents of 100 mM NAC aqueous solution was added and shaken at 25 °C for 20 minutes to terminate the linking reaction, an ultrafiltration centrifuge tube with a molecular weight cutoff of 50 kD or a Sephadex G-25 desalting column was used to remove excess small molecules, and the antibody-drug conjugate was exchanged into 50 mM PBS buffer (pH 6.0), and the sample was filtered with a 0.22 μm filter membrane to obtain the antibody-drug conjugate, which was stored in a refrigerator at 4 °C. The DAR value of the conjugate was determined by reverse phase high performance liquid chromatography or mass spectrometry.

Example 4-1: ADC-1

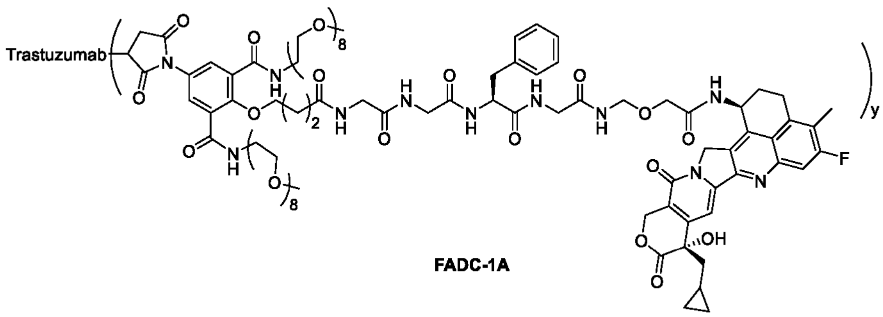

FADC-1A

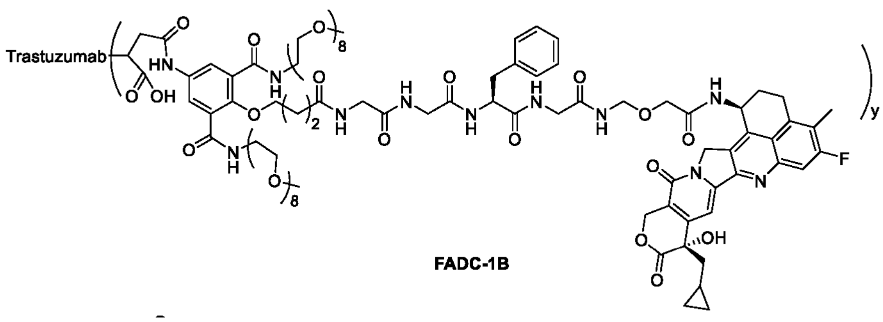

FADC-1B

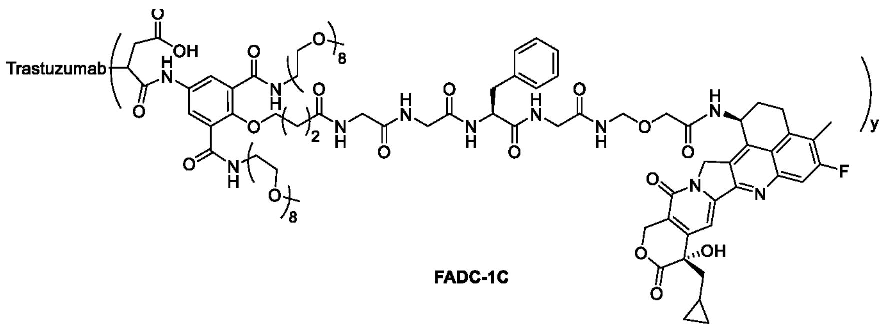

FADC-1C

Using antibody Trastuzumab and LD-1 as starting materials, the preparation was carried out by applying step A. The PBS buffer with the exemplary product ADC-1 of the conjugate mixture (mixture of FADC-1A and/or FADC-1B and/or FADC-1C) was obtained and stored at 4 °C.

Example 4-2: ADC-2 to Example 4-10: ADC-10

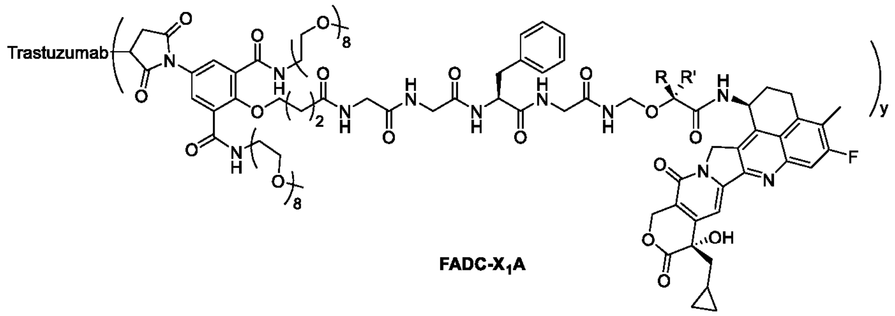

FADC-$X_1$A

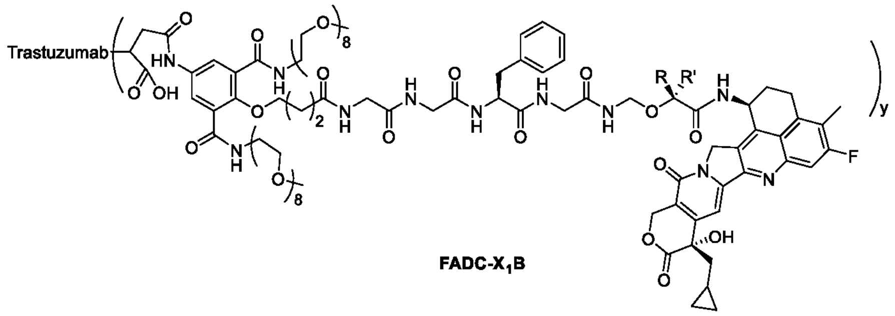

FADC-$X_1$B

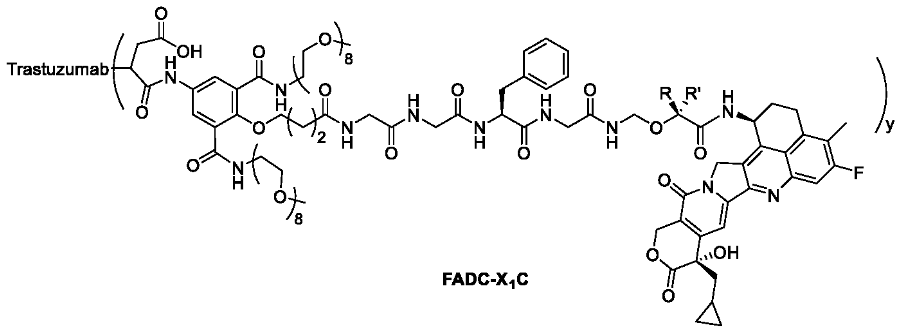

FADC-$X_1$C

| Exemplary ADC product | Mixture of conjugates FADC-$X_1$A and/or FADC-$X_1$B and/or FADC-$X_1$C | R | R' |
|---|---|---|---|
| ADC-2 | FADC-2A and/or FADC-2B and/or FADC-2C | $-CH_3$ | -H |
| ADC-3 | FADC-3A and/or FADC-3B and/or FADC-3C | -H | $-CH_3$ |
| ADC-4 | FADC-4A and/or FADC-4B and/or FADC-4C | 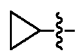 | -H |
| ADC-5 | FADC-5A and/or FADC-5B and/or FADC-5C | -H | 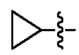 |
| Exemplary ADC product | Mixture of conjugates FADC-$X_2$A and/or FADC-$X_2$B and/or FADC-$X_2$C | R | R' |
| ADC-6 | FADC-6A and/or FADC-6B and/or FADC-6C | -H | -H |
| ADC-7 | FADC-7A and/or FADC-7B and/or FADC-7C | $-CH_3$ | -H |
| ADC-8 | FADC-8A and/or FADC-8B and/or FADC-8C | -H | $-CH_3$ |
| ADC-9 | FADC-9A and/or FADC-9B and/or FADC-9C | 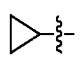 | -H |
| ADC-10 | FADC-10A and/or FADC-10B and/or FADC-10C | -H | 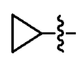 |

**LD-2, LD-3, LD-4, LD-5, LD-6, LD-7, LD-8, LD-9 and LD-10** were used to replace LD-1 respectively, and a similar preparation method was applied to obtain the corresponding **ADC-2, ADC-3, ADC-4, ADC-5, ADC-6, ADC-7, ADC-8, ADC-9** and **ADC-10,** which were Examples 4-2 to 4-10.

Example 4-11: ADC-11

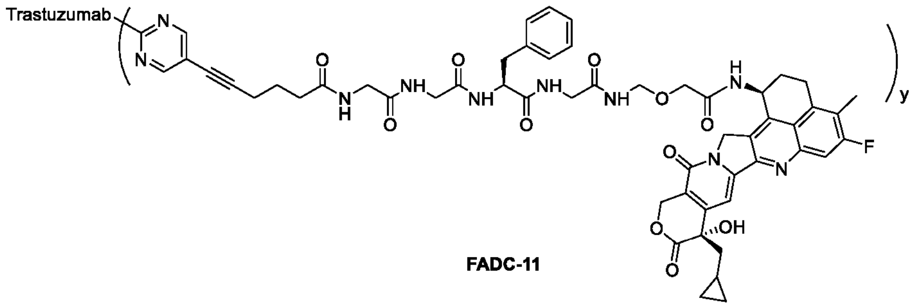

**FADC-11**

Using the antibody Trastuzumab and **LD-11** as starting materials, the preparation was carried out by applying the method disclosed in the patent application "WO2019114666A1". The PBS buffer with the exemplary product **ADC-11** of the general Formula **FADC-11** was obtained and stored at 4 °C.

Example 4-12: ADC-12 to Example 4-16: ADC-16

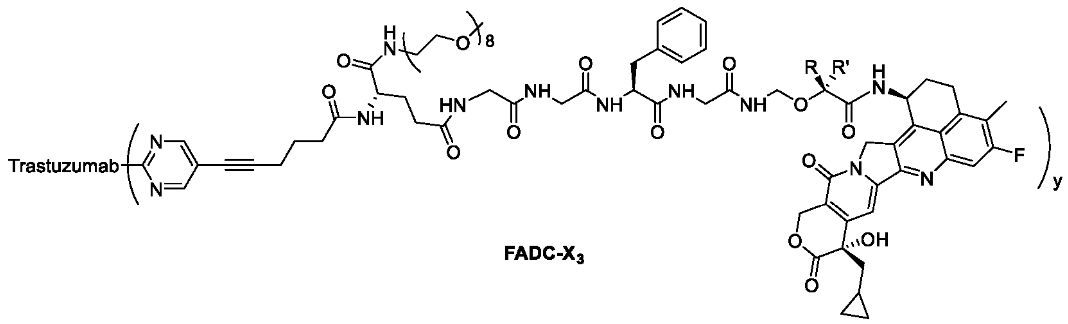

**FADC-$X_3$**

| Exemplary ADC product | General formula FADC-$X_3$ | |
|---|---|---|
| | R | R' |
| ADC-12 | -H | -H |
| ADC-13 | $-CH_3$ | -H |
| ADC-14 | -H | $-CH_3$ |
| ADC-15 | 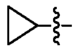 | -H |
| ADC-16 | -H | 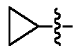 |

Using the antibody Trastuzumab and **LD-13** as starting materials, the preparation was carried out by applying the method of step C. The PBS buffer with the exemplary product **ADC-13** of the conjugate mixture **FADC-13** was obtained and stored at 4 °C.

The average drug loading was calculated by mass spectrometry: y=7.36.

**LD-12, LD-14, LD-15** and **LD-16** were used to replace **LD-13,** respectively, and similar preparation methods were used to obtain the corresponding **ADC-12, ADC-14, LD-15** and **ADC-16,** which were Example 4-12, and Examples 4-14 to 4-16.

Example 4-17: ADC-17

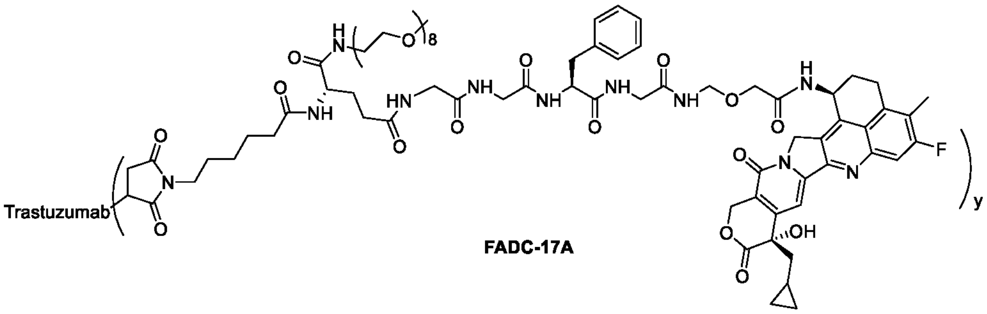

FADC-17A

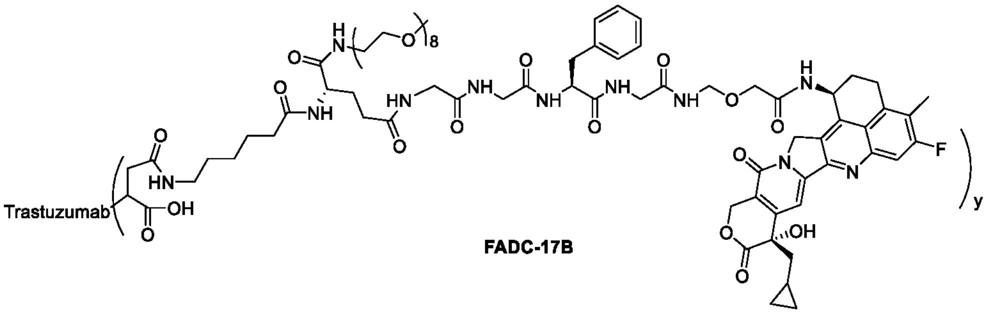

FADC-17B

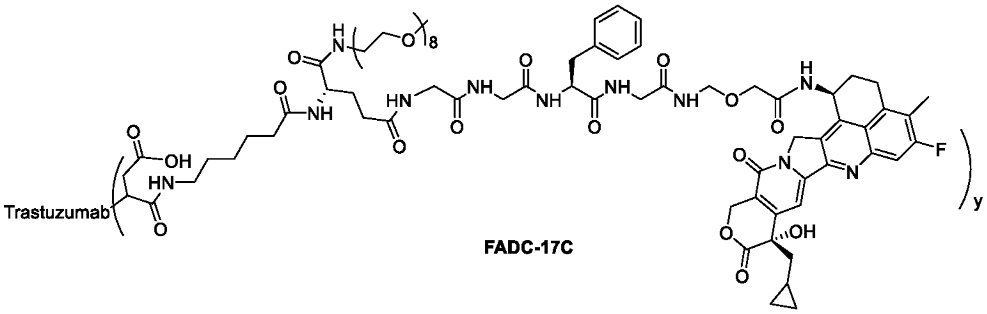

FADC-17C

Using antibody Trastuzumab and **LD-17** as starting materials, the preparation was carried out by applying the method disclosed in patent application "WO2022068898A1". The PBS buffer with the exemplary product **ADC-17** of the conjugate mixture (mixture of **FADC-17A** and/or **FADC-17B** and/or **FADC-17C**) was obtained and stored at 4 °C.

Example 4-18: ADC-18 to Example 4-21: ADC-21

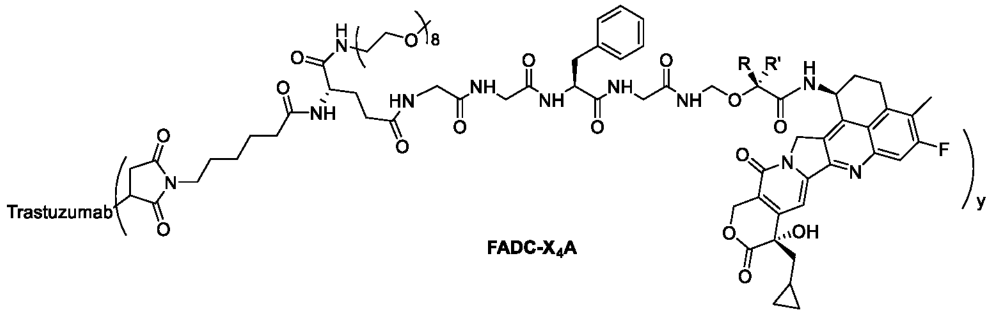

FADC-X$_4$A

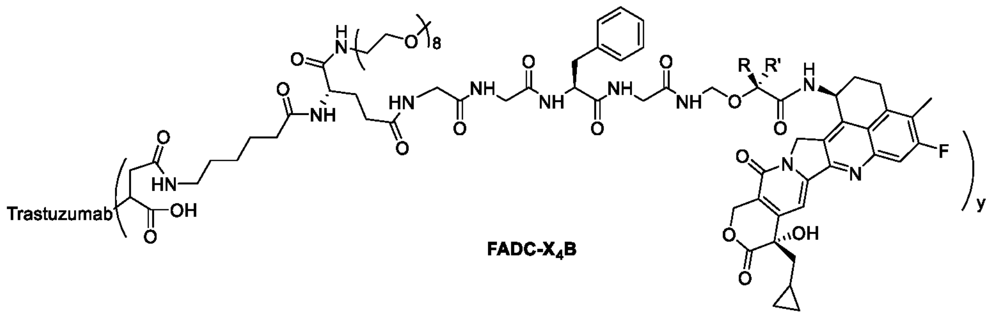

FADC-X$_4$B

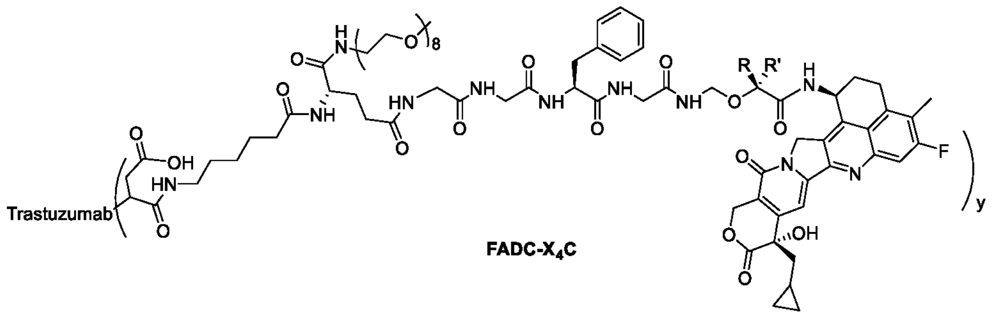

FADC-X$_4$C

| Exemplary ADC product | Conjugate mixture of FADC-X$_4$A and/or FADC-X$_4$B and/or FADC-X$_4$C | R | R' |
|---|---|---|---|
| ADC-18 | FADC-18A and/or FADC-18B and/or FADC-18C | $-CH_3$ | -H |
| ADC-19 | FADC-19A and/or FADC-19B and/or FADC-19C | -H | $-CH_3$ |
| ADC-20 | FADC-20A and/or FADC-20B and/or FADC-20C | 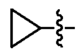 | -H |
| ADC-21 | FADC-21A and/or FADC-21B and/or FADC-21C | -H | 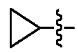 |

**LD-18, LD-19, LD-20** and **LD-21** were used to replace **LD-17,** respectively, and the corresponding **ADC-18, ADC-19, ADC-20** and **ADC-21** were obtained by applying similar preparation methods, which were Examples 4-18 to 4-21.

Example 4-22: ADC-22

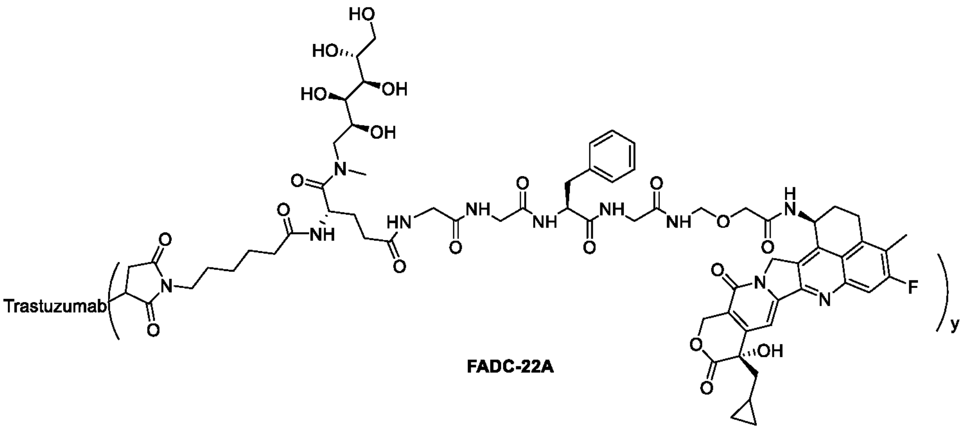

FADC-22A

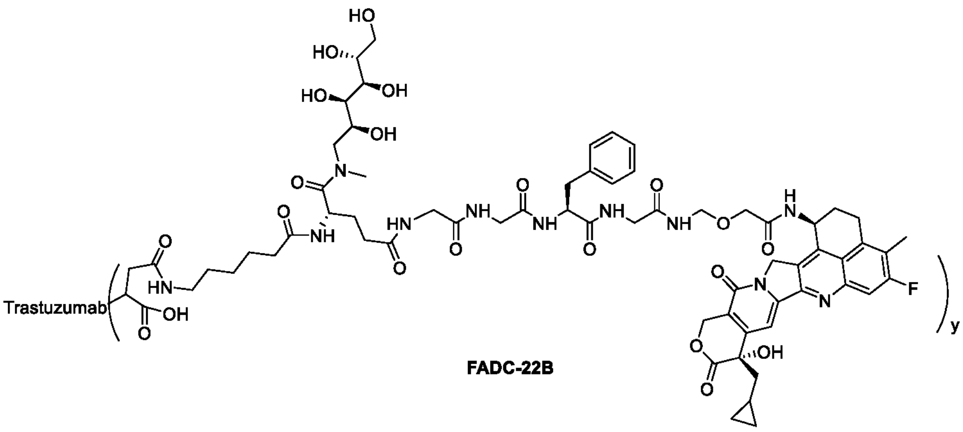

FADC-22B

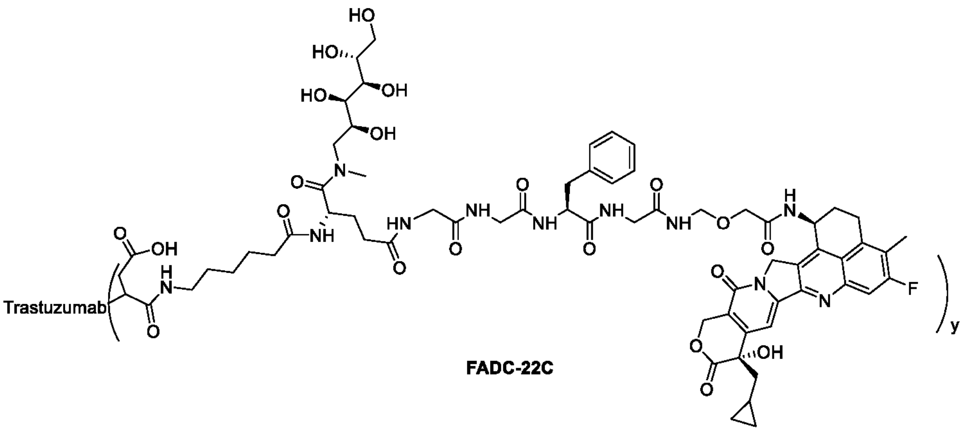

FADC-22C

Using antibody Trastuzumab and **LD-22** as starting materials, the preparation was carried out by applying the method disclosed in the patent application "WO2022068898A1". The PBS buffer with the exemplary product **ADC-22** of the conjugate mixture (mixture of **FADC-22A** and/or **FADC-22B** and/or **FADC-22C**) was obtained and stored at 4 °C.

Example 4-23: ADC-23 to Example 4-26: ADC-26

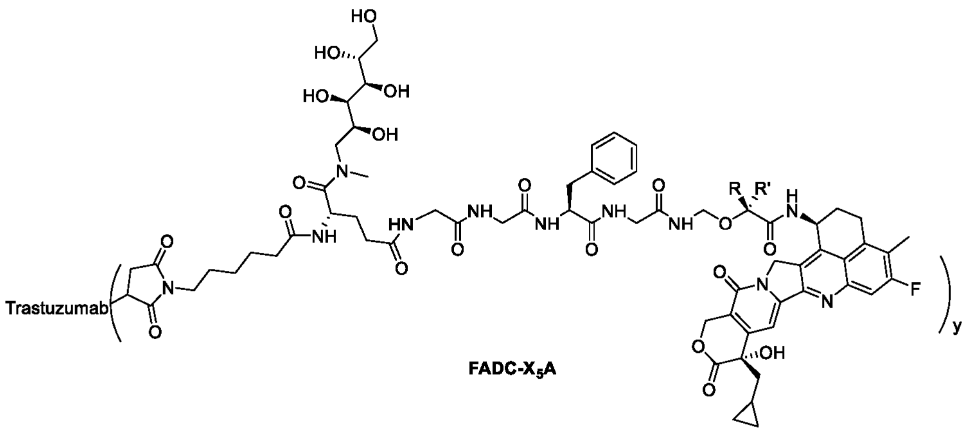

FADC-X$_5$A

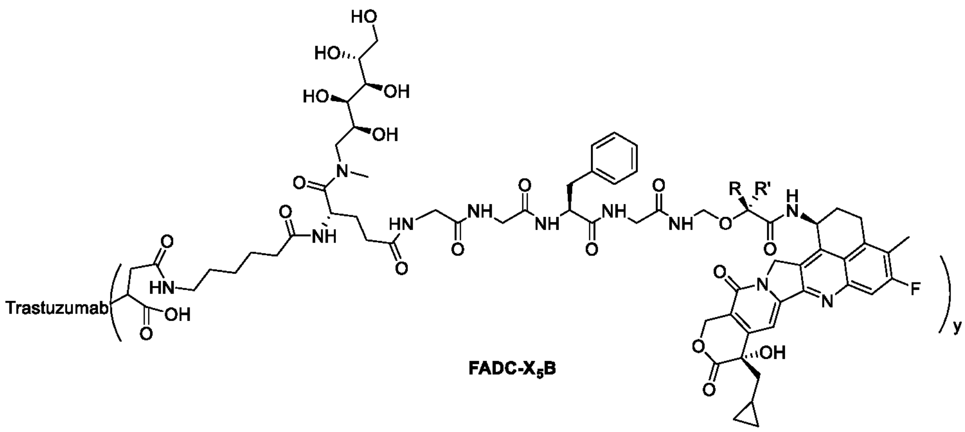

FADC-X$_5$B

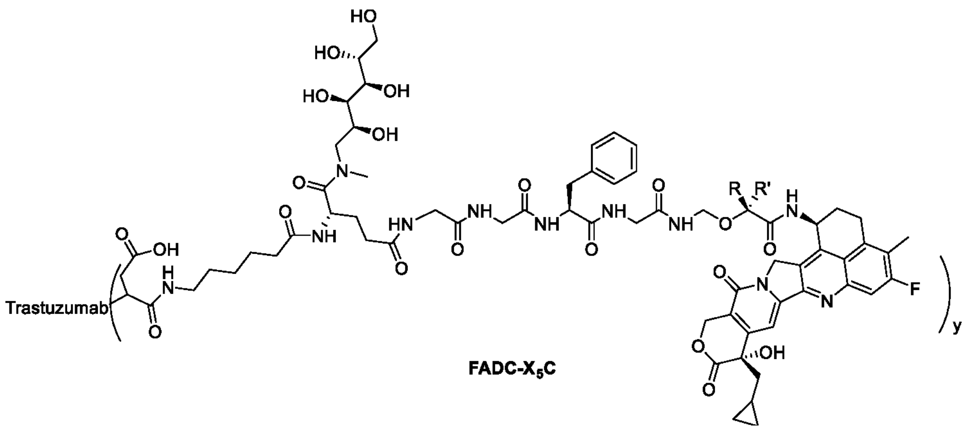

FADC-X$_5$C

| Exemplary ADC product | Conjugate mixture of FADC-X$_5$A and/or FADC-X$_5$B and/or FADC-X$_5$C | R | R' |
|---|---|---|---|
| ADC-23 | FADC-23A and/or FADC-23B and/or FADC-23C | $-CH_3$ | -H |
| ADC-24 | FADC-24A and/or FADC-24B and/or FADC-24C | -H | $-CH_3$ |
| ADC-25 | FADC-25A and/or FADC-25B and/or FADC-25C | 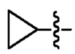 | -H |
| ADC-26 | FADC-26A and/or FADC-26B and/or FADC-26C | -H | 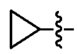 |

**LD-23, LD-24, LD-25** and **LD-26** were used to replace **LD-22,** respectively, and the corresponding **ADC-23, ADC-24, ADC-25** and **ADC-26** were obtained by applying similar preparation methods, which were Examples 4-23 to

4-26.

Example 4-27 ADC-27

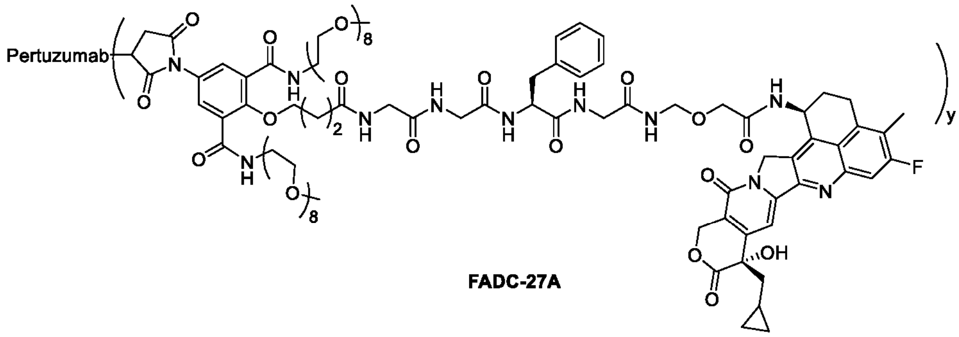

**FADC-27A**

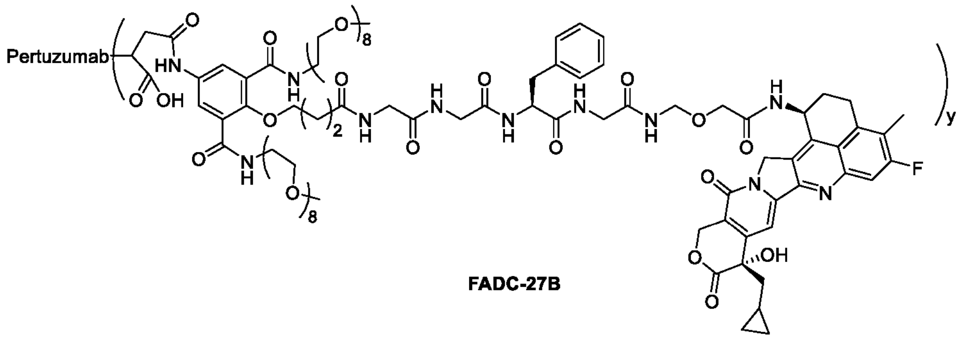

**FADC-27B**

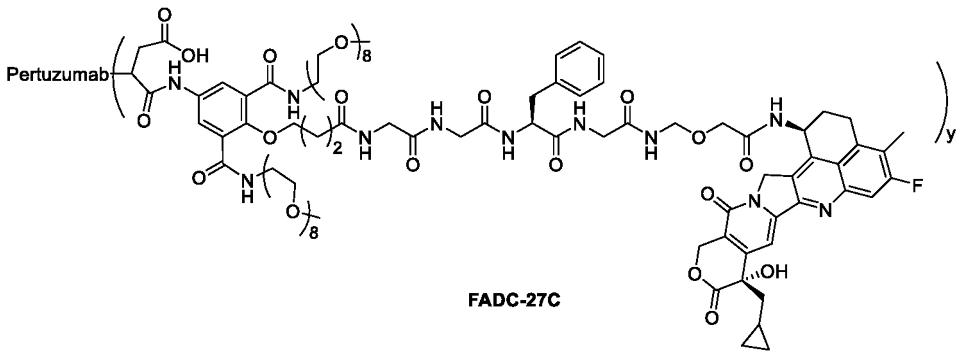

**FADC-27C**

Using the antibody Pertuzumab and **LD-1** as starting materials, the preparation was carried out by applying the method of step A. The PBS buffer with the exemplary product **ADC-27** of the conjugate mixture (mixture of **FADC-27A** and/or **FADC-27B** and/or **FADC-27C**) was obtained and stored at 4 °C.

Example 4-28: ADC-28

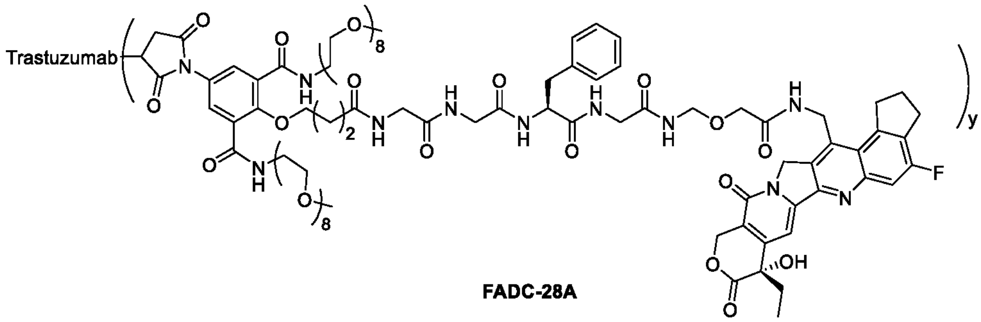

FADC-28A

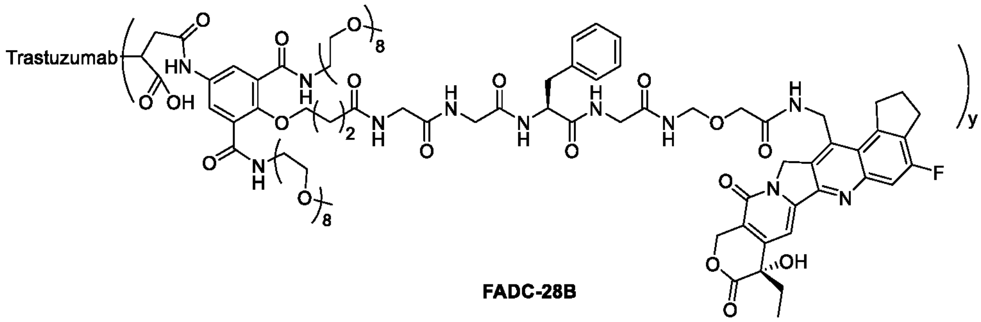

FADC-28B

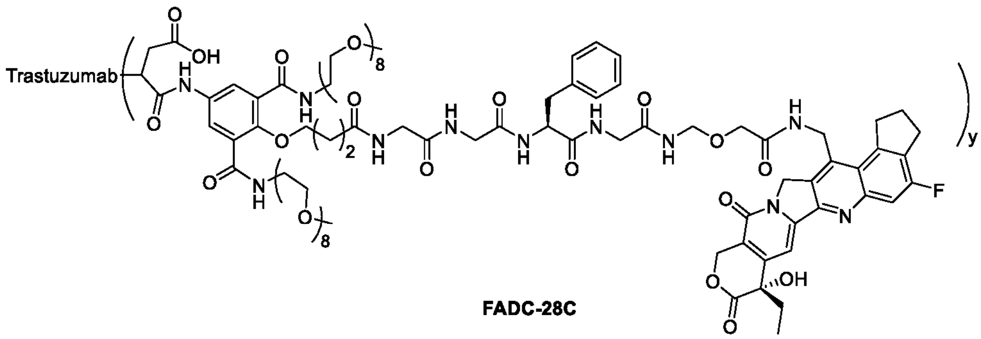

FADC-28C

Using the antibody Trastuzumab and **LD-27** as starting materials, the preparation was carried out by applying the method of step A. The PBS buffer with the exemplary product **ADC-28** of the conjugate mixture (mixture of **FADC-28A** and/or **FADC-28B** and/or **FADC-28C**) was obtained and stored at 4 °C.

Example 4-29: ADC-29

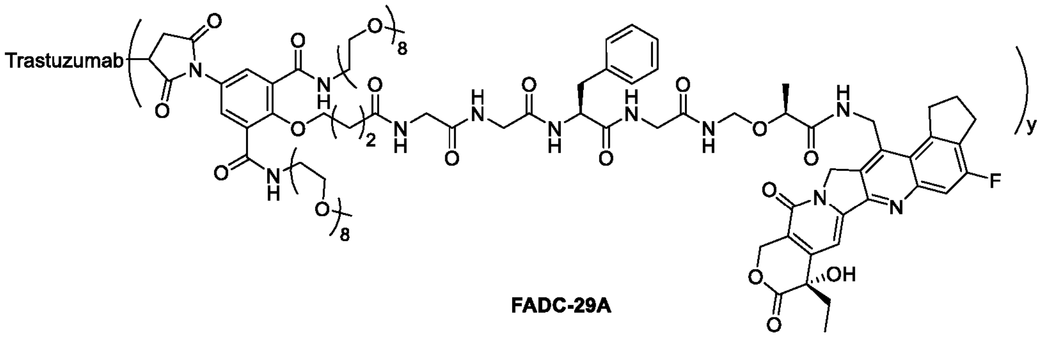

FADC-29A

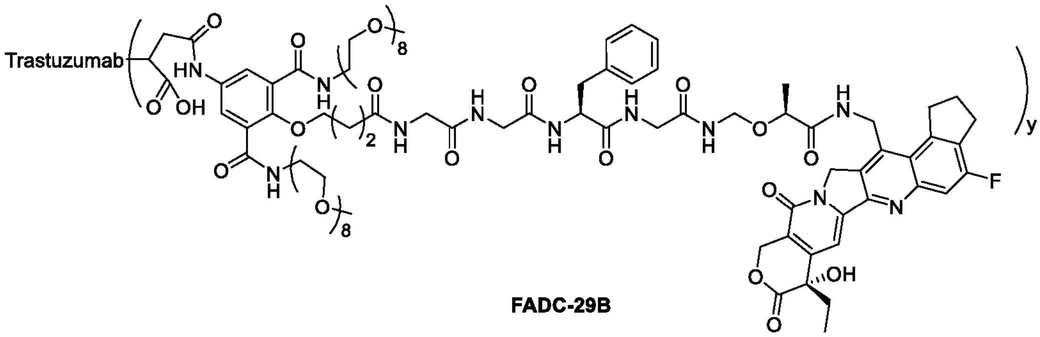

**FADC-29B**

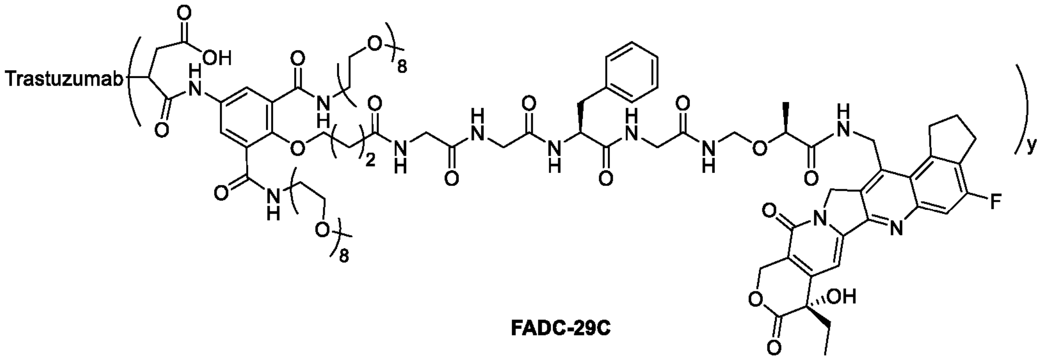

**FADC-29C**

Using the antibody Trastuzumab and **LD-28** as starting materials, the preparation was carried out by applying the method of step A. The PBS buffer with the exemplary product **ADC-29** of the conjugate mixture (mixture of **FADC-29A** and/or **FADC-29B** and/or **FADC-29C**) was obtained and stored at 4 °C.

Example 4-30: ADC-30

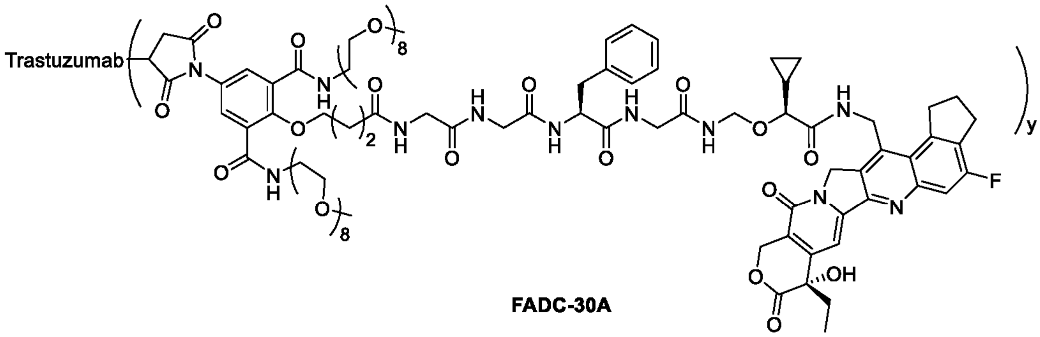

**FADC-30A**

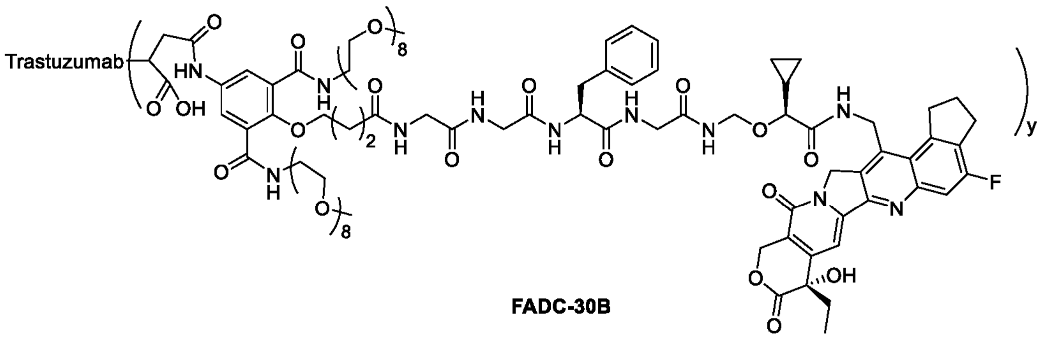

**FADC-30B**

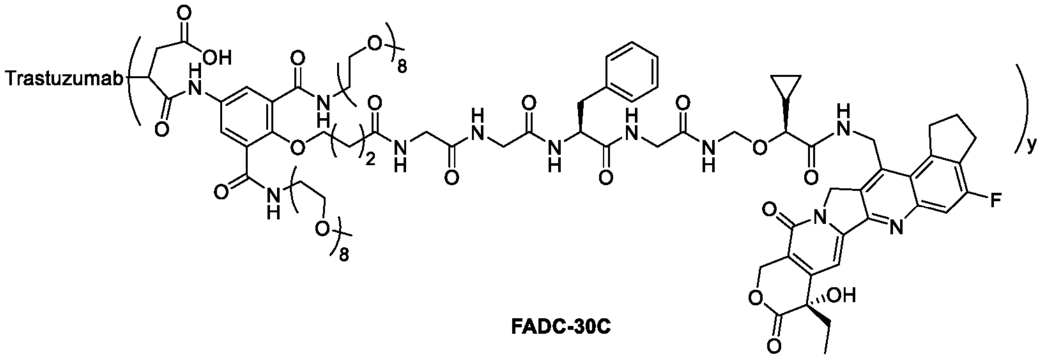

FADC-30C

Using the antibody Trastuzumab and **LD-29** as starting materials, the preparation was carried out by applying the method of step A. The PBS buffer with the exemplary product **ADC-30** of the conjugate mixture (mixture of **FADC-30A** and/or **FADC-30B** and/or **FADC-30C**) was obtained and stored at 4 °C.

Example 4-31: ADC-31

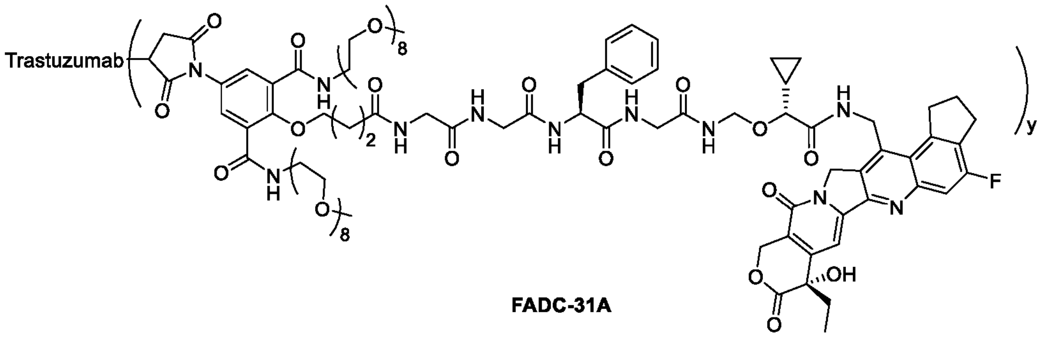

FADC-31A

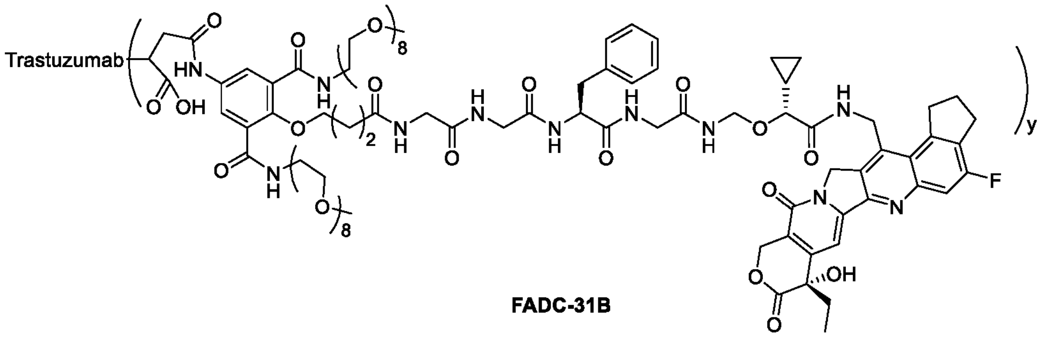

FADC-31B

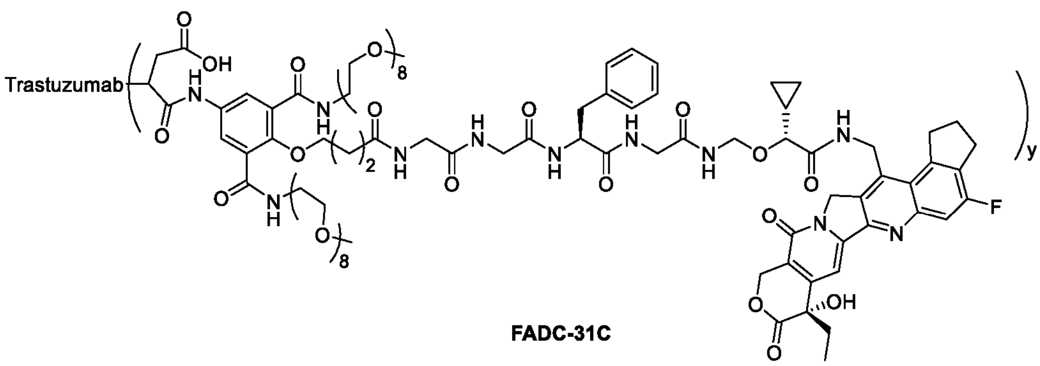

FADC-31C

Using the antibody Trastuzumab and **LD-30** as starting materials, the preparation was carried out by applying the

method of step A. The PBS buffer with the exemplary product **ADC-31** of conjugate mixture (mixture of **FADC-31A** and/or **FADC-31B** and/or **FADC-31C**) was obtained and stored at 4 °C.

Example 4-32: ADC-32 to Example 4-35: ADC-35

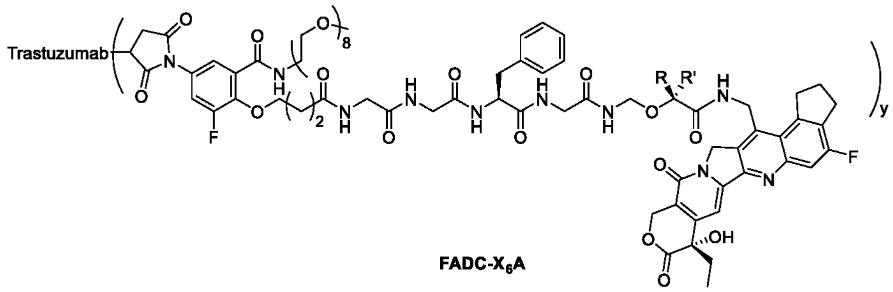

FADC-$X_6$A

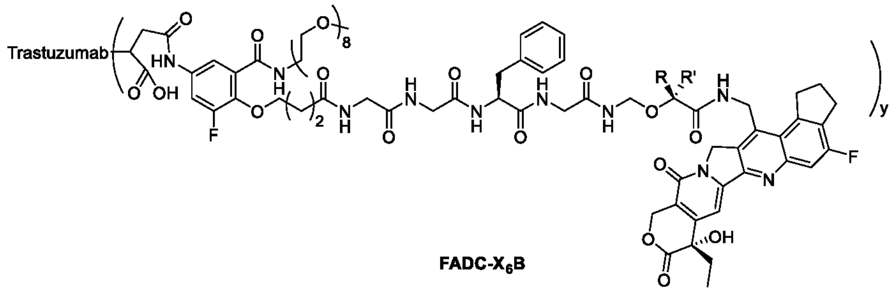

FADC-$X_6$B

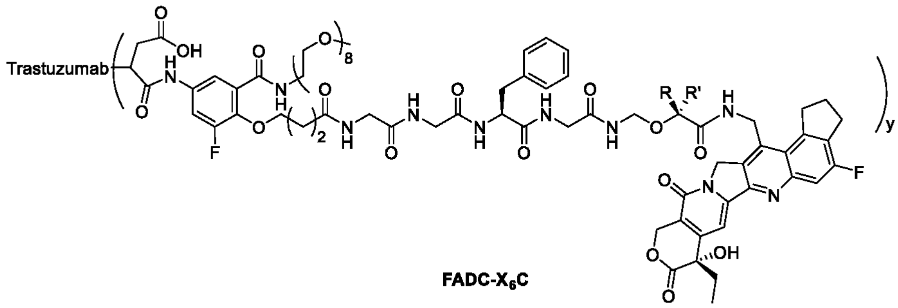

FADC-$X_6$C

| Exemplary ADC product | Conjugate mixture of FADC-$X_6$A and/or ADC-$X_6$B and/or FADC-$X_6$C | R | R' |
|---|---|---|---|
| ADC-32 | FADC-32A and/or FADC-32B and/or FADC-32C | -H | -H |
| ADC-33 | FADC-33A and/or FADC-33B and/or FADC-33C | $-CH_3$ | -H |
| ADC-34 | FADC-34A and/or FADC-34B and/or FADC-34C | 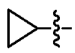 | -H |
| ADC-35 | FADC-35A and/or FADC-35B and/or FADC-35C | -H | 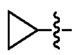 |

The preparation was carried out by applying the method of step A using the antibody Trastuzumab with **LD-31** to **LD-34,** respectively, and the PBS buffers with the exemplary products **ADC-32** to **ADC-35** of the corresponding conjugate mixture (mixture of **FADC-A** and/or **FADC-B** and/or **FADC-C**) were obtained and stored at 4 °C, respectively, which were Examples 4-32 to 4-35.

Example 4-36: ADC-36 to Example 4-38: ADC-38

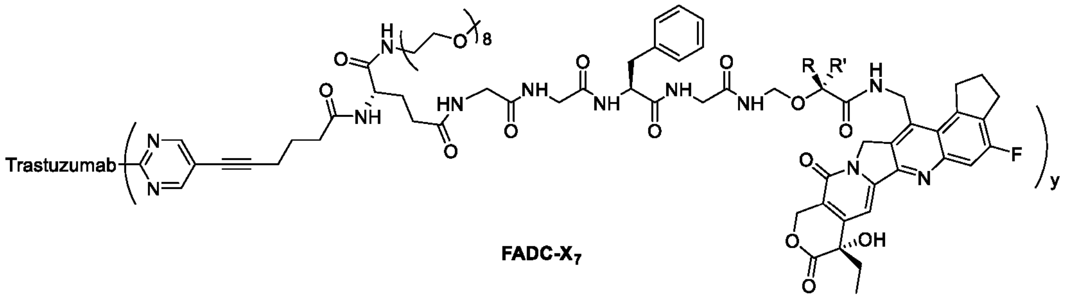

FADC-$X_7$

| Exemplary ADC product | General formula FADC-$X_7$ | |
|---|---|---|
| | R | R' |
| ADC-36 | -H | -H |
| ADC-37 | $-CH_3$ | -H |
| ADC-38 | 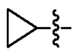 | -H |

The preparation was carried out by applying the method disclosed in the patent application "WO2019114666A1" using the antibody Trastuzumab with **LD-35** to **LD-37,** respectively, and the PBS buffers with the exemplary products **ADC-36** to **ADC-38** of the general formulas **FADC-36** to **FADC-38** were obtained and stored at 4 °C, respectively, which were Examples 4-36 to 4-38.

Example 4-39: ADC-39 to Example 4-42: ADC-42

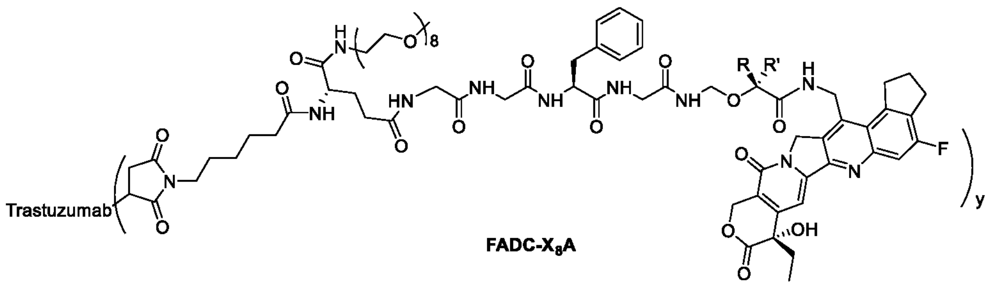

FADC-$X_8$A

(continued)

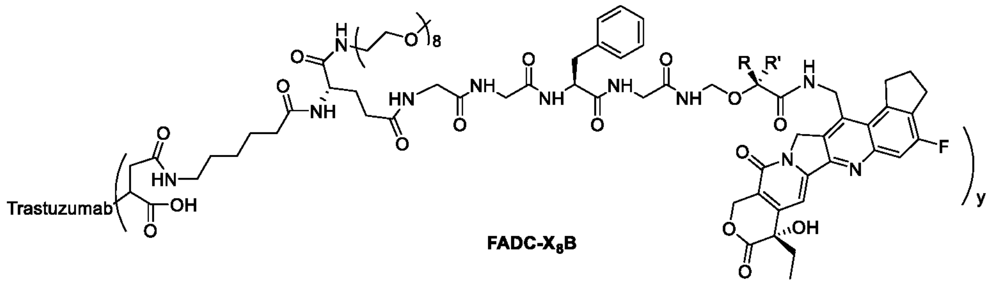

FADC-X$_8$B

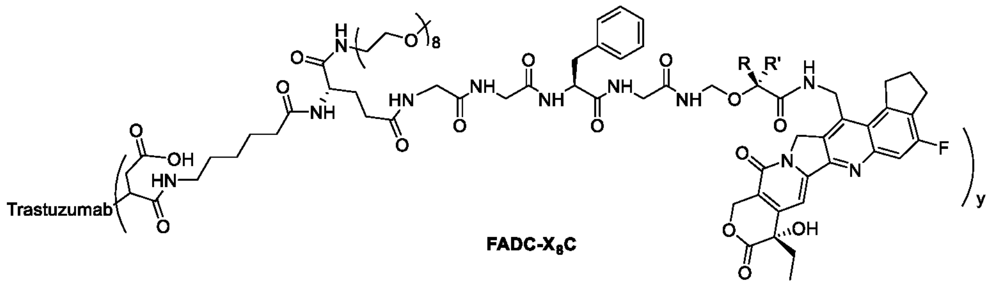

FADC-X$_8$C

| Exemplary ADC product | Conjugate mixture of FADC-X$_8$A and/or ADC-X$_8$B and/or FADC-X$_8$C | R | R' |
|---|---|---|---|
| ADC-39 | FADC-39A and/or FADC-39B and/or FADC-39C | -H | -H |
| ADC-40 | FADC-40A and/or FADC-40B and/or FADC-40C | $-CH_3$ | -H |
| ADC-41 | FADC-41A and/or FADC-41B and/or FADC-41C | 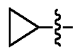 | -H |
| ADC-42 | FADC-42A and/or FADC-42B and/or FADC-42C | -H | 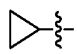 |

The preparation was carried out by applying the method disclosed in the patent application "WO2022068898A1" using the antibody Trastuzumab with **LD-38** to **LD-41,** respectively, and the PBS buffers with the exemplary products **ADC-39** to **ADC-42** of the corresponding conjugate mixture (mixture of **FADC-A** and/or **FADC-B** and/or **FADC-C**) were obtained and stored at 4 °C, respectively, which were Examples 4-39 to 4-42.

Example 4-43: ADC-43 to Example 4-45: ADC-45

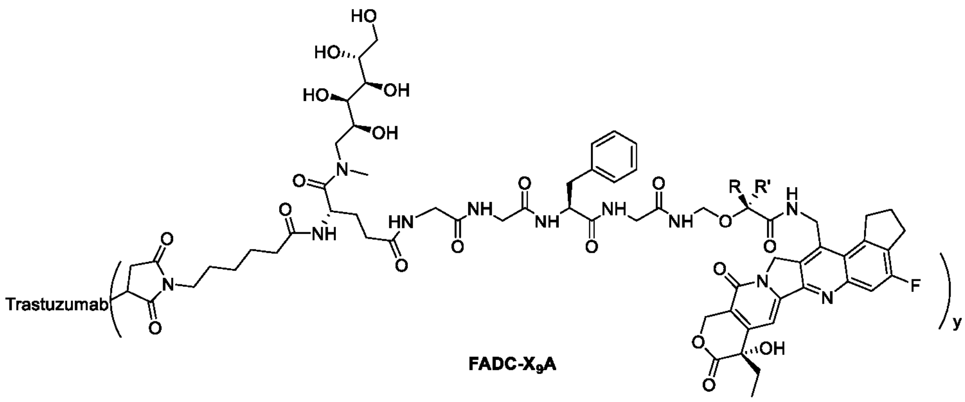

FADC-$X_9$A

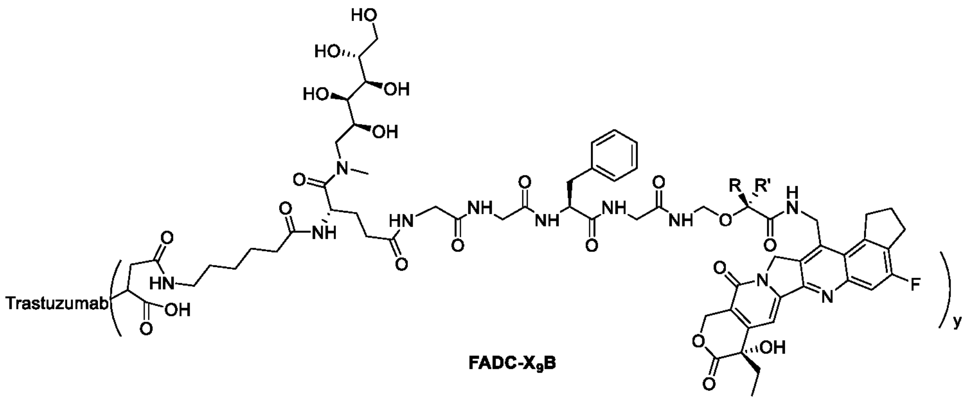

FADC-$X_9$B

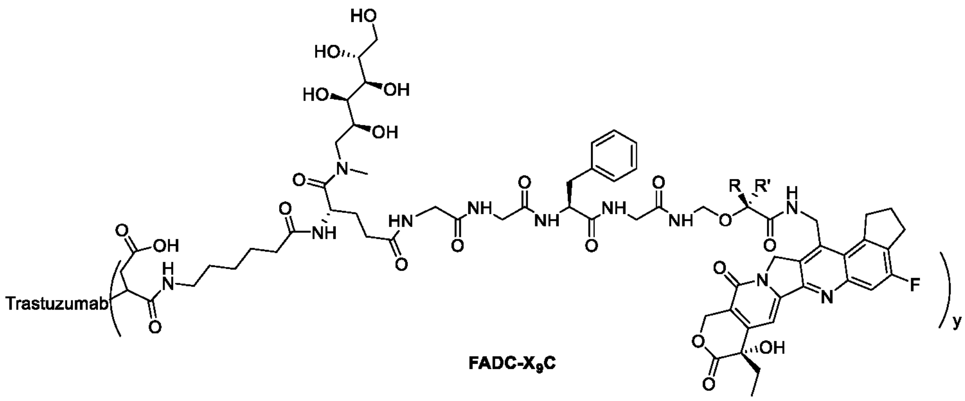

FADC-$X_9$C

| Exemplary ADC product | Conjugate mixture of FADC-$X_9$A and/or FADC-$X_9$B and/or FADC-$X_9$C | R | R' |
|---|---|---|---|
| ADC-43 | FADC-43A and/or FADC-43B and/or FADC-43C | -H | -H |
| ADC-44 | FADC-44A and/or FADC-44B and/or FADC-44C | $-CH_3$ | -H |
| ADC-45 | FADC-45A and/or FADC-45B and/or FADC-45C | 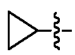 | -H |

The preparation was carried out by applying the method disclosed in the patent application "WO2022068898A1" using the antibody Trastuzumab with **LD-42** to **LD-44,** respectively, and the PBS buffers with the exemplary products **ADC-43** to **ADC-45** of the corresponding conjugate mixture (mixture of **FADC-A** and/or **FADC-B** and/or **FADC-C**) were obtained and stored at 4 °C, respectively, which were Examples 4-43 to 4-45.

Example 4-46: ADC-46 to Example 4-48: ADC-48

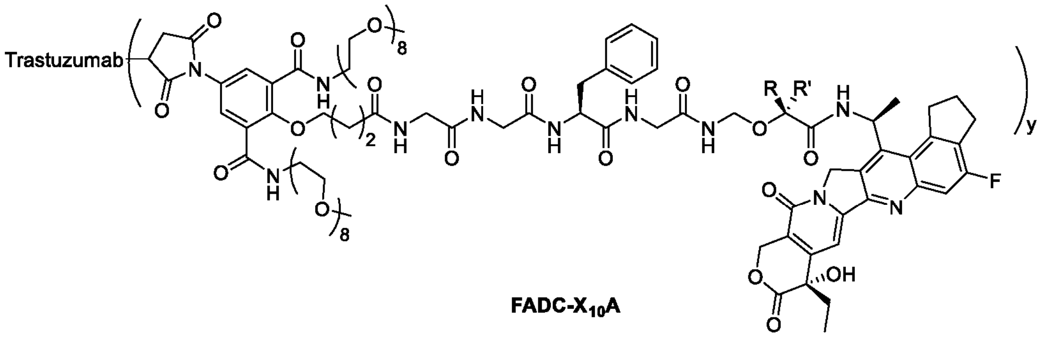

**FADC-X$_{10}$A**

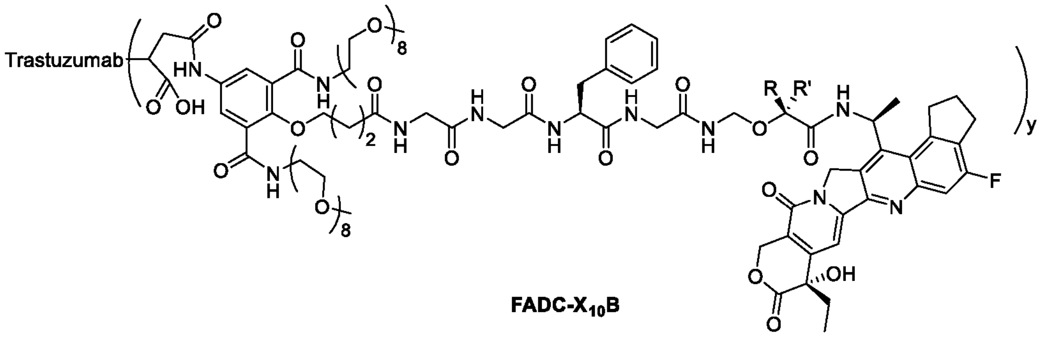

**FADC-X$_{10}$B**

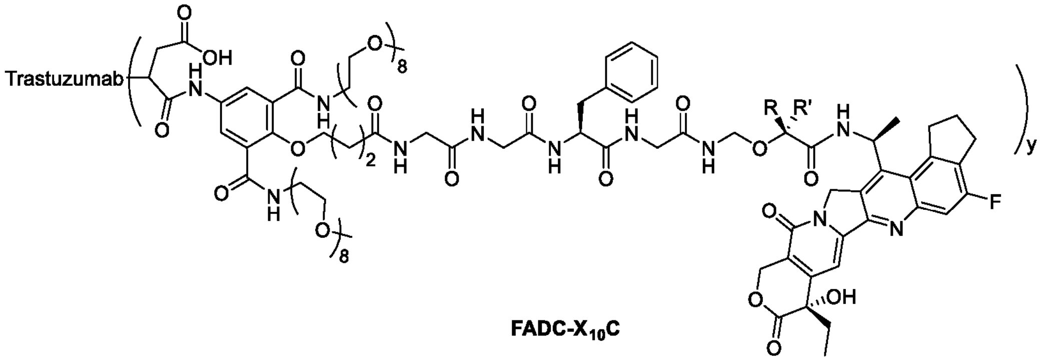

**FADC-X$_{10}$C**

| Exemplary ADC product | Conjugate mixture of FADC-x10A and/or FADC-x10B and/or FADC-x10C | R | R' |
|---|---|---|---|
| ADC-46 | FADC-46A and/or FADC-46B and/or FADC-46C | -H | -H |
| ADC-47 | FADC-47A and/or FADC-47B and/or FADC-47C | 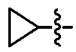 | -H |
| ADC-48 | FADC-48A and/or FADC-48B and/or FADC-48C | -H | 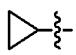 |

The preparation was carried out by applying the method of step A using the antibody Trastuzumab with **LD-45** to **LD-47,** respectively, and the PBS buffers with the exemplary products **ADC-46** to **ADC-48** of the corresponding conjugate mixture (mixture of **FADC-A** and/or **FADC-B** and/or **FADC-C**) were obtained and stored at 4 °C, respectively, which were Examples 4-46 to 4-48.

Example 4-49: ADC-49 to Example 4-51: ADC-51

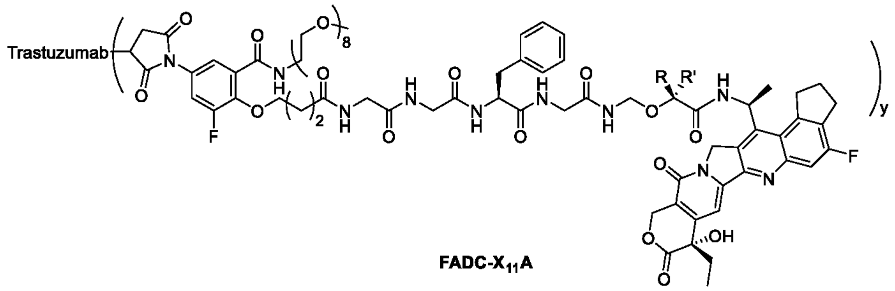

FADC-$X_{11}$A

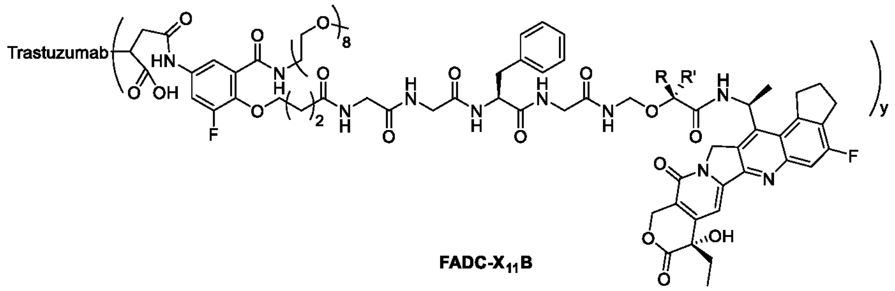

FADC-$X_{11}$B

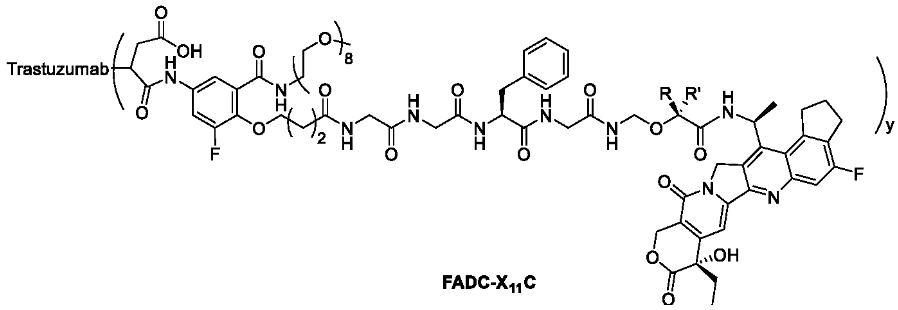

FADC-$X_{11}$C

| Exemplary ADC product | Conjugate mixture of FADC-$X_{11}$A and/or FADC-$X_{11}$B and/or FADC-$X_{11}$C | R | R' |
|---|---|---|---|
| ADC-49 | FADC-49A and/or FADC-49B and/or FADC-49C | -H | -H |
| ADC-50 | FADC-50A and/or FADC-50B and/or FADC-50C | 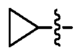 | -H |
| ADC-51 | FADC-51A and/or FADC-51B and/or FADC-51C | -H | 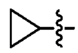 |

The preparation was carried out by applying the method of step A using the antibody Trastuzumab with **LD-48** to **LD-50,** respectively, and the PBS buffers with the exemplary products **ADC-49** to **ADC-51** of the corresponding conjugate mixture (mixture of **FADC-A** and/or **FADC-B** and/or **FADC-C**) were obtained and stored at 4 °C, respectively, which were Examples 4-49 to 4-51.

Example 4-52: ADC-52 to Example 4-54: ADC-54

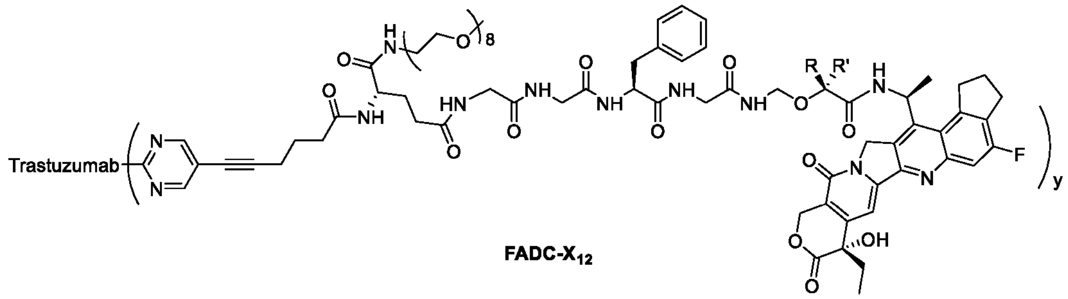

FADC-$X_{12}$

| Exemplary ADC product | General formula FADC-$X_{12}$ | |
|---|---|---|
| | R | R' |
| ADC-52 | -H | -H |
| ADC-53 | 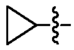 | -H |
| ADC-54 | -H | 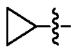 |

The preparation was carried out by applying the method disclosed in the patent application "WO2019114666A1" using the antibody Trastuzumab with **LD-51** to **LD-53,** respectively, and the PBS buffers with the exemplary products **ADC-52** to **ADC-54** of the general formulas **FADC-52** to **FADC-54** were obtained and stored at 4 °C, respectively, which were Examples 4-52 to 4-54.

Example 4-55: ADC-55 to Example 4-57: ADC-57

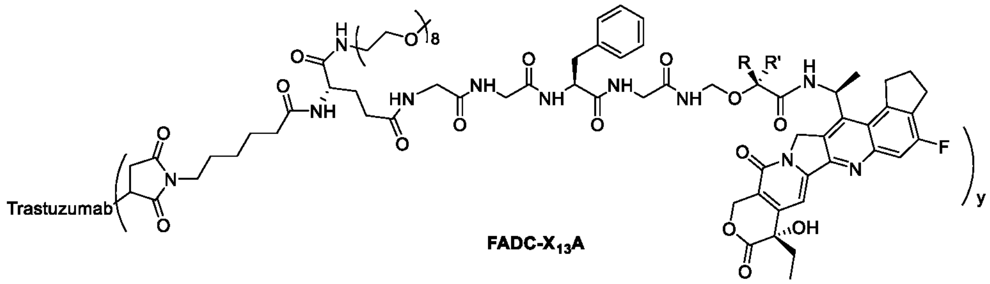

FADC-X13A

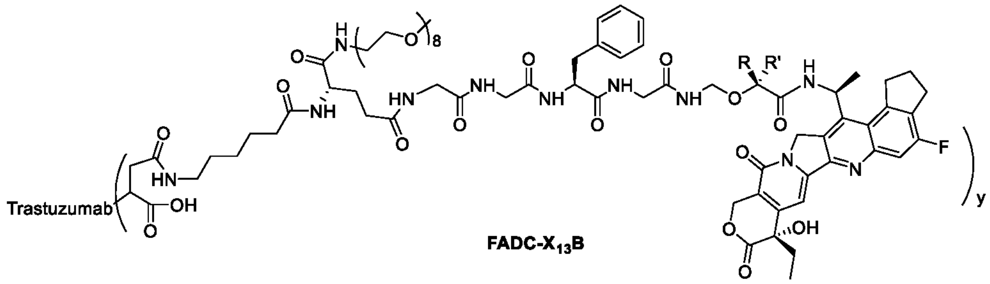

FADC-X13B

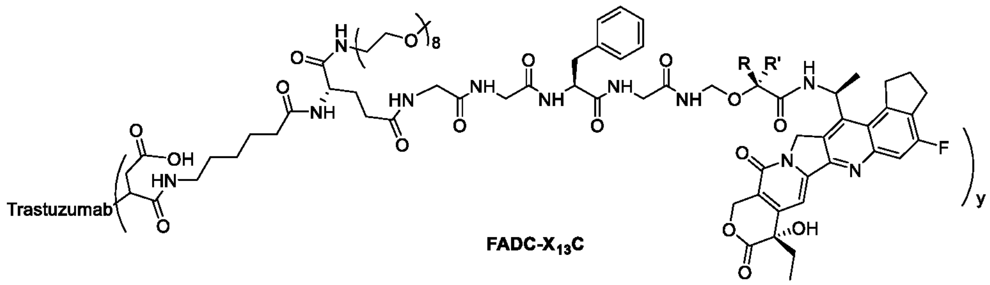

FADC-X13C

| Exemplary ADC product | Conjugate mixture of FADC-$X_{13}$A and/or FADC-$X_{13}$B and/or FADC-$X_{13}$C | R | R' |
|---|---|---|---|
| ADC-55 | FADC-55A and/or FADC-55B and/or FADC-55C | -H | -H |
| ADC-56 | FADC-56A and/or FADC-56B and/or FADC-56C | 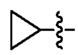 | -H |
| ADC-57 | FADC-57A and/or FADC-57B and/or FADC-57C | -H | 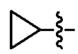 |

The preparation was carried out by applying the method disclosed in patent application "WO2022068898A1" using the antibody Trastuzumab with **LD-54** to **LD-56,** respectively, and the PBS buffers with the exemplary products **ADC-55** to **ADC-57** of the corresponding conjugate mixture (mixture of **FADC-A** and/or **FADC-B** and/or **FADC-C**) were obtained and stored at 4 °C, respectively, which were Examples 4-55 to 4-57.

Example 4-58: ADC-58 to Example 4-60: ADC-60

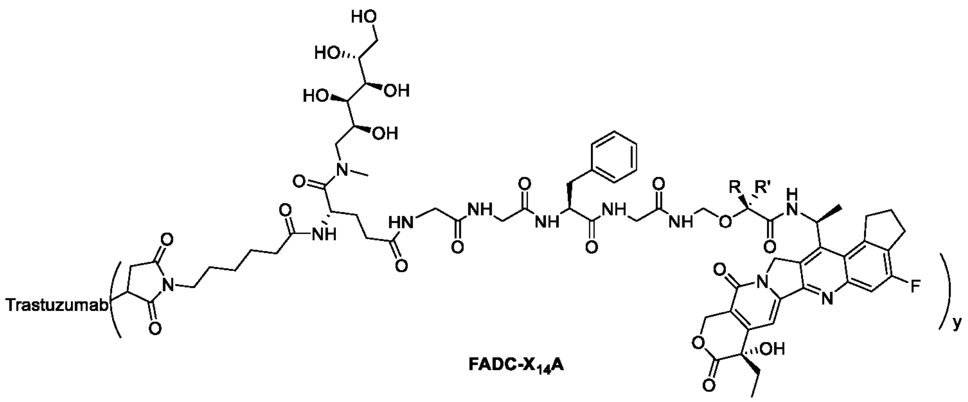

FADC-X14A

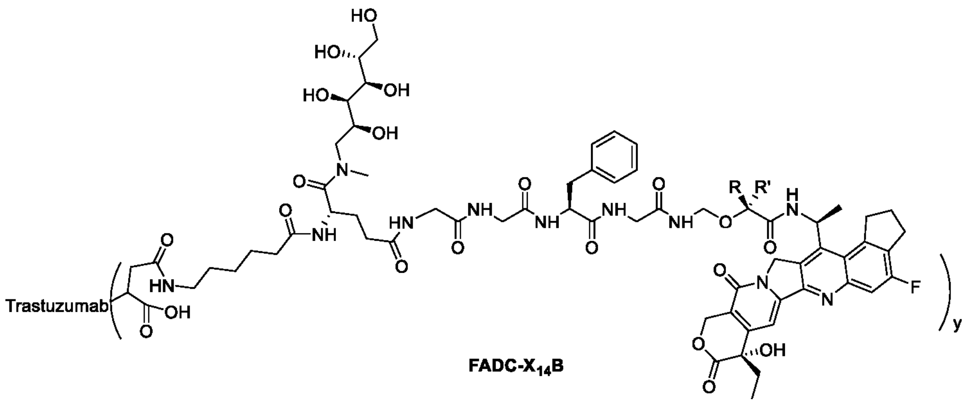

FADC-X14B

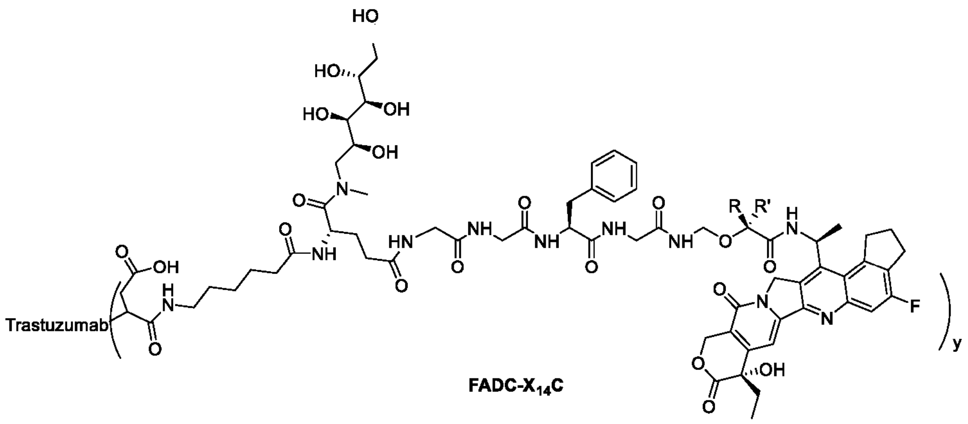

FADC-X14C

| Exemplary ADC product | Conjugate mixture of FADC-$X_{14}$A and/or FADC-$X_{14}$B and/or FADC-$X_{14}$C | R | R' |
|---|---|---|---|
| ADC-58 | FADC-58A and/or FADC-58B and/or FADC-58C | -H | -H |
| ADC-59 | FADC-59A and/or FADC-59B and/or FADC-59C | 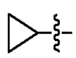 | -H |
| ADC-60 | FADC-60A and/or FADC-60B and/or FADC-60C | -H | 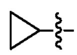 |

The preparation was carried out by applying the method disclosed in patent application "WO2022068898A1" using the antibody Trastuzumab with **LD-57** to **LD-59,** respectively, and the PBS buffers with the exemplary products **ADC-58** to **ADC-60** of the corresponding conjugate mixture (mixture of **FADC-A** and/or **FADC-B** and/or **FADC-C**) were obtained and stored at 4 °C, respectively, which were Examples 4-58 to 4-60.

Example 4-61: ADC-61

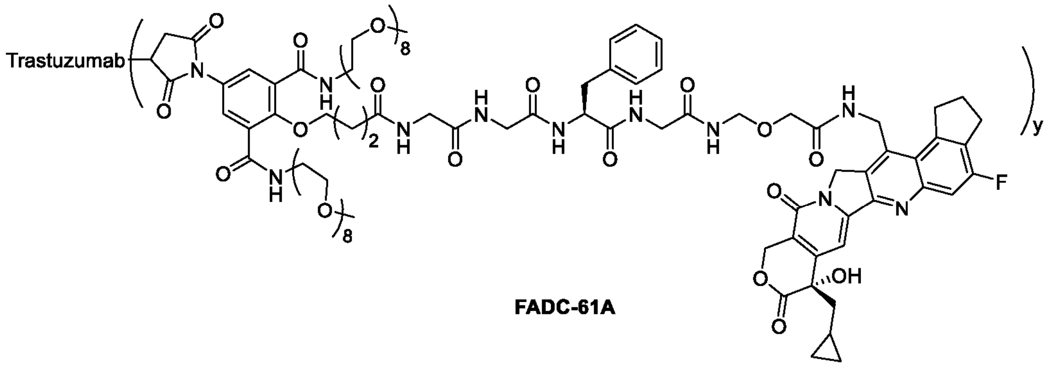

**FADC-61A**

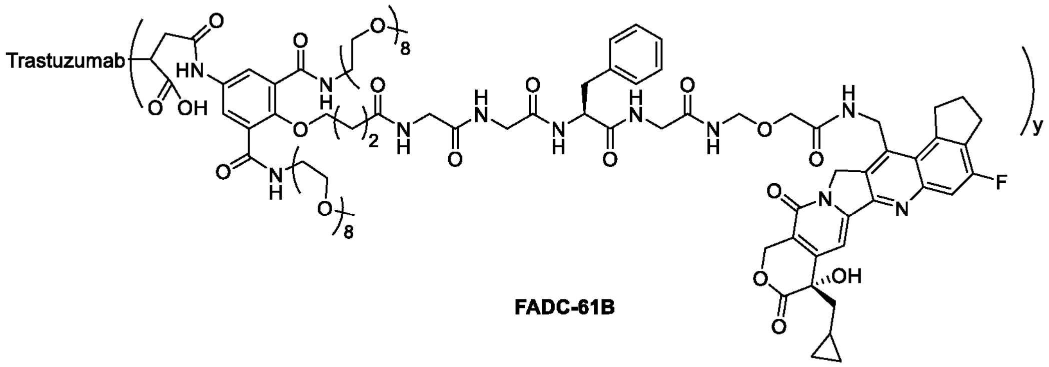

**FADC-61B**

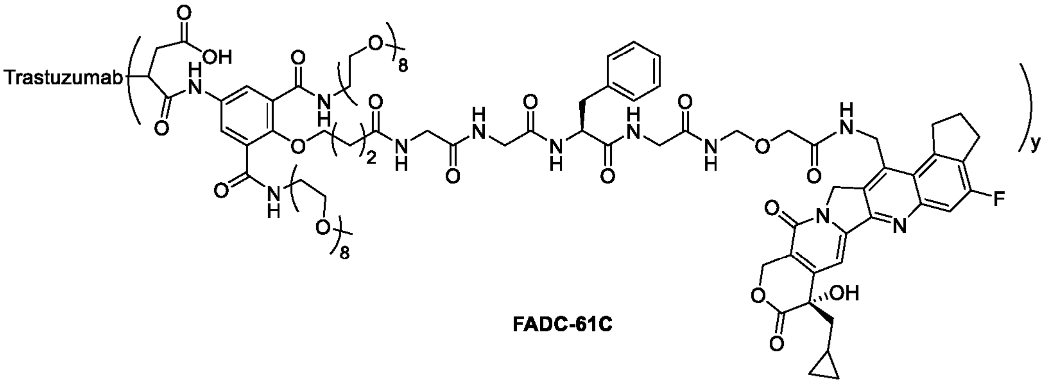

**FADC-61C**

Using the antibody Trastuzumab and LD-60 as starting materials, the preparation was carried out by applying the method of step A. The PBS buffer with the exemplary product **ADC-61** of the conjugate mixture (mixture of **FADC-61A** and/or **FADC-61B** and/or **FADC-61C**) was obtained and stored at 4 °C.

Example 4-62: ADC-62

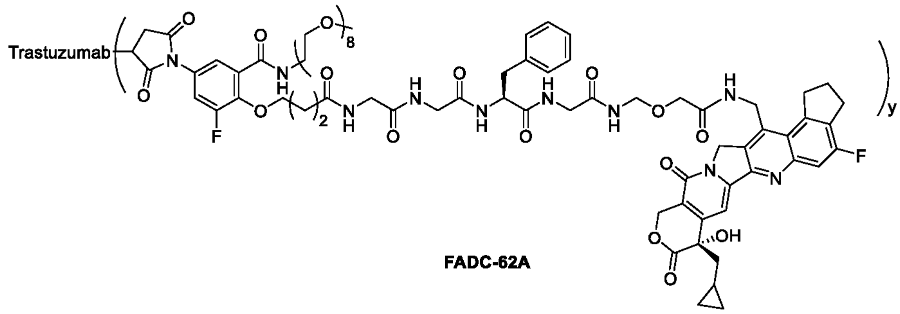

FADC-62A

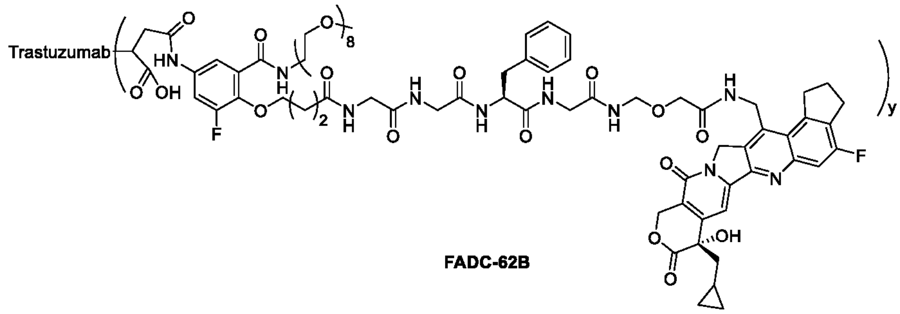

FADC-62B

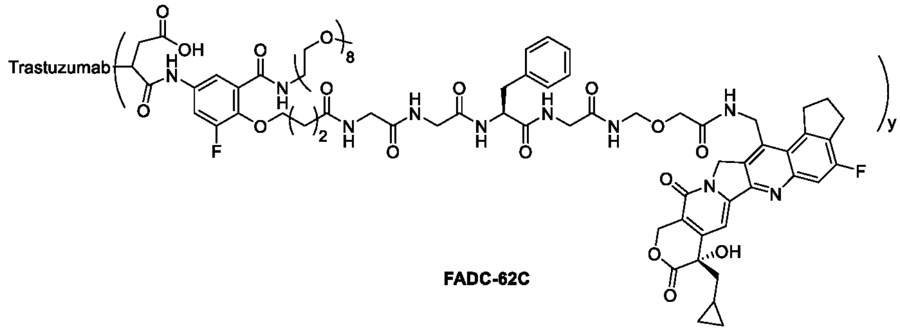

FADC-62C

Using the antibody Trastuzumab and **LD-61** as starting materials, the preparation was carried out by applying the method of step A. The PBS buffer with the exemplary product **ADC-62** of the conjugate mixture (mixture of **FADC-62A** and/or **FADC-62B** and/or **FADC-62C**) was obtained and stored at 4 °C.

Example 4-63: ADC-63

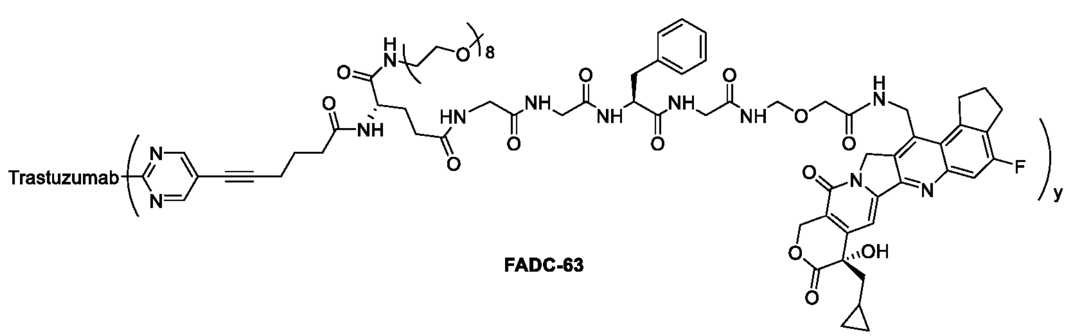

FADC-63

Using the antibody Trastuzumab and LD-62 as starting materials, the preparation was carried out by applying the method disclosed in the patent application "WO2019114666A1". The PBS buffer with the exemplary product **ADC-63** of the general formula of **FADC-63** was obtained and stored at 4 °C.

Example 4-64: ADC-64

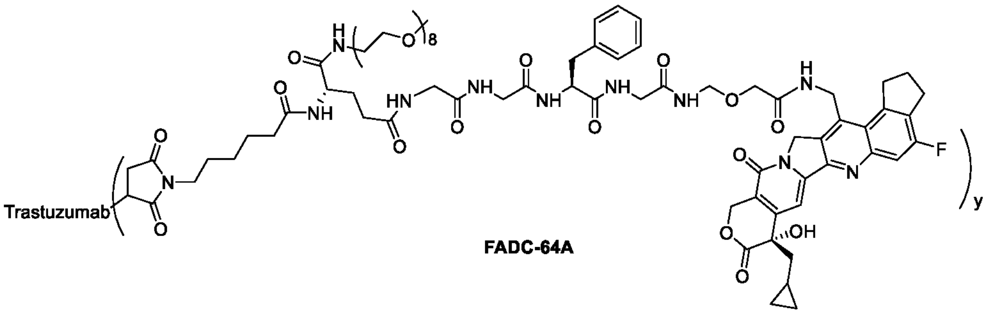

FADC-64A

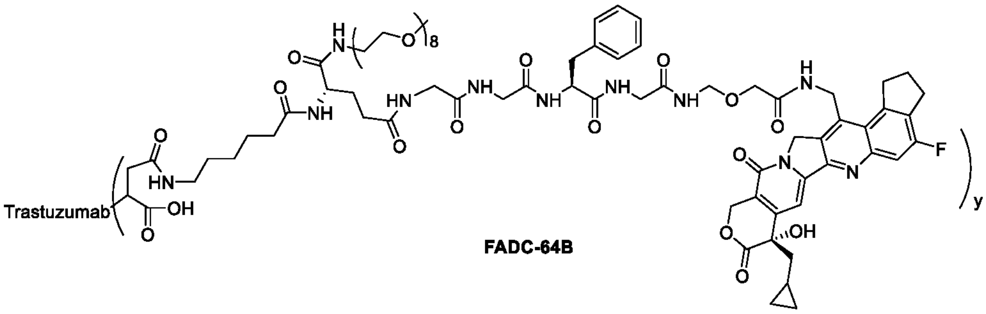

FADC-64B

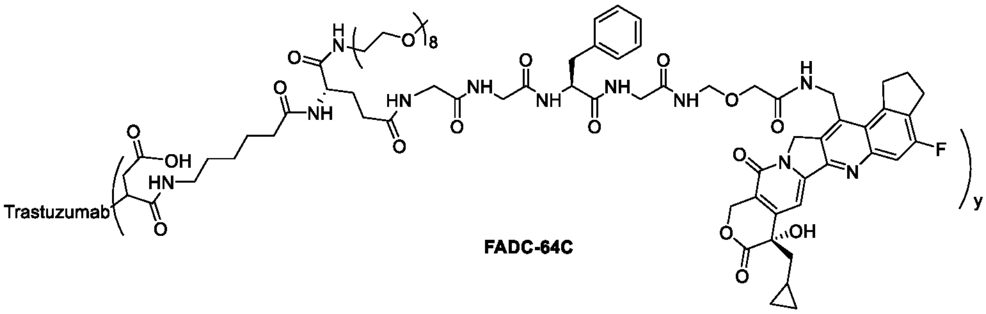

FADC-64C

Using the antibody Trastuzumab and LD-63 as starting materials, the preparation was carried out by applying the method disclosed in the patent application "WO2022068898A1". The PBS buffer with the exemplary product **ADC-64** of the conjugate mixture (mixture of **FADC-64A** and/or **FADC-64B** and/or **FADC-64C**) was obtained and stored at 4 °C.

Example 4-65: ADC-65

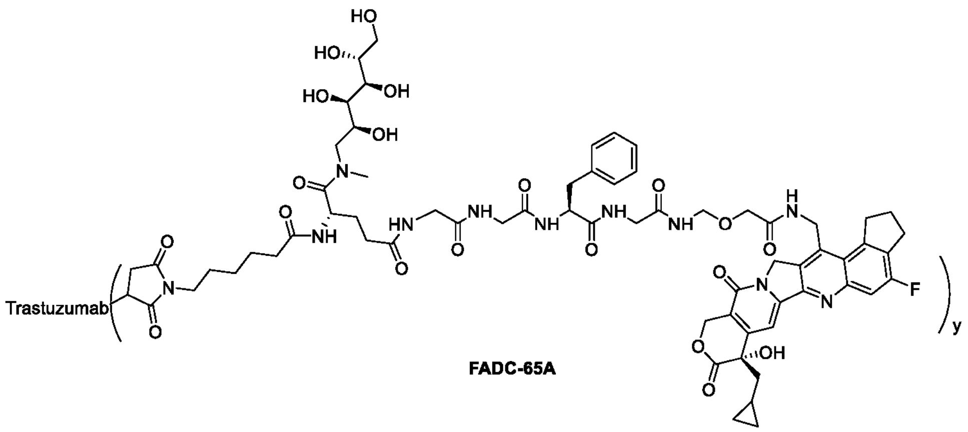

FADC-65A

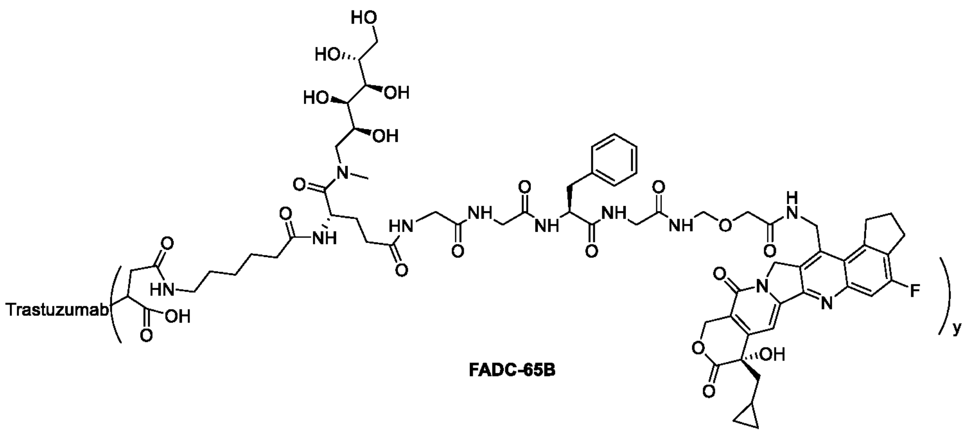

FADC-65B

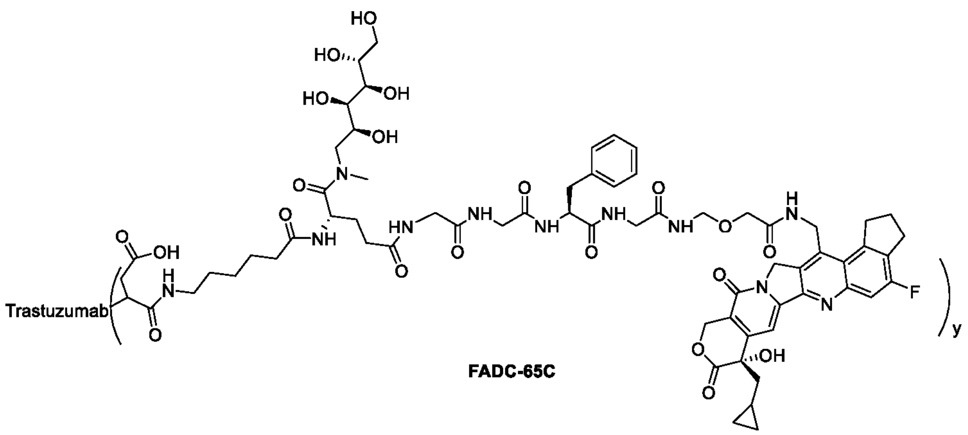

FADC-65C

Using the antibody Trastuzumab and **LD-64** as starting materials, the preparation was carried out by applying the method disclosed in the patent application "WO2022068898A1". The PBS buffer with the exemplary product **ADC-65** of the conjugate mixture (mixture of **FADC-65A** and/or **FADC-65B** and/or **FADC-65C**) was obtained and stored at 4 °C.

Example 4-66: ADC-66

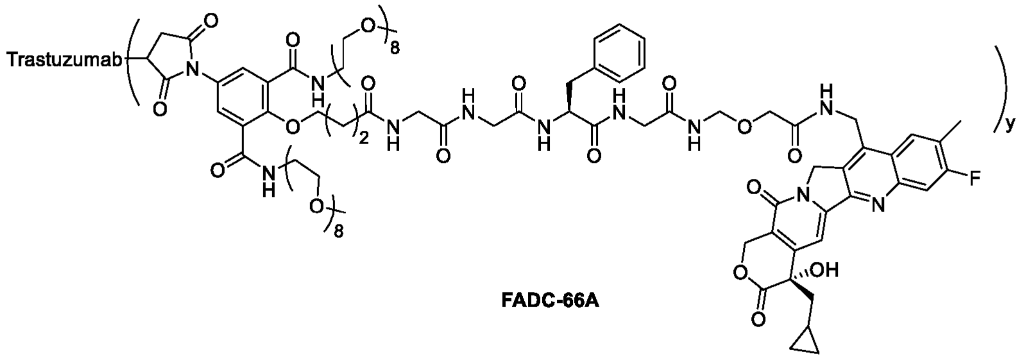

FADC-66A

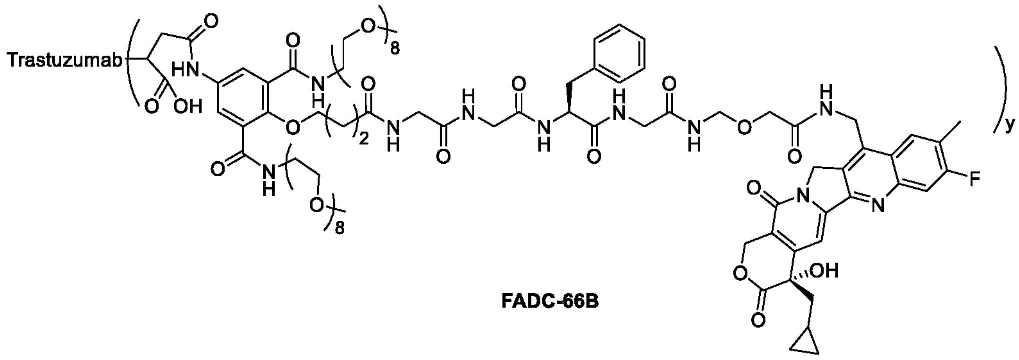

FADC-66B

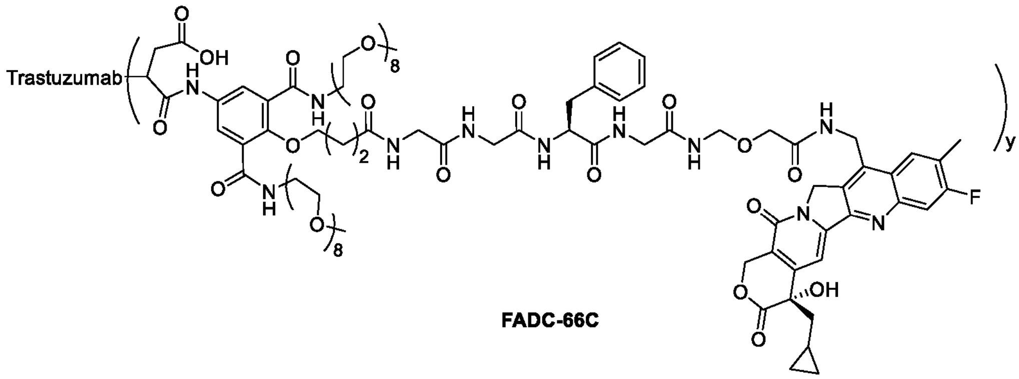

FADC-66C

Using the antibody Trastuzumab and **LD-65** as starting materials, the preparation was carried out by applying the method of step A. The PBS buffer with the exemplary product **ADC-66** of the conjugate mixture (mixture of **FADC-66A** and/or **FADC-66B** and/or **FADC-66C**) was obtained and stored at 4 °C.

Example 4-67: ADC-67 to Example 4-75: ADC-75

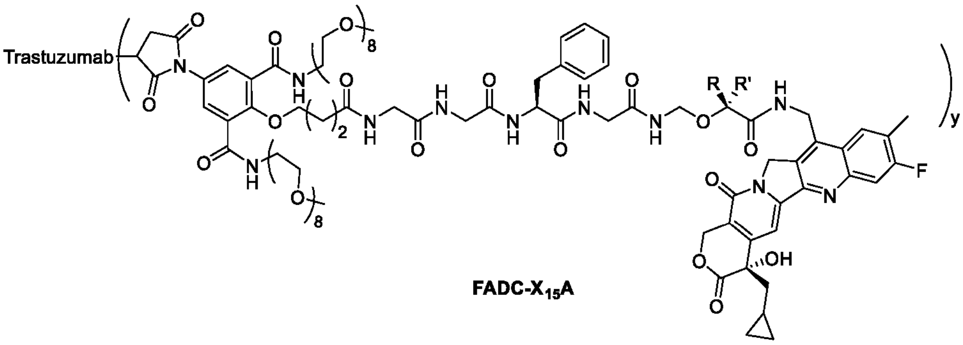

FADC-$X_{15}$A

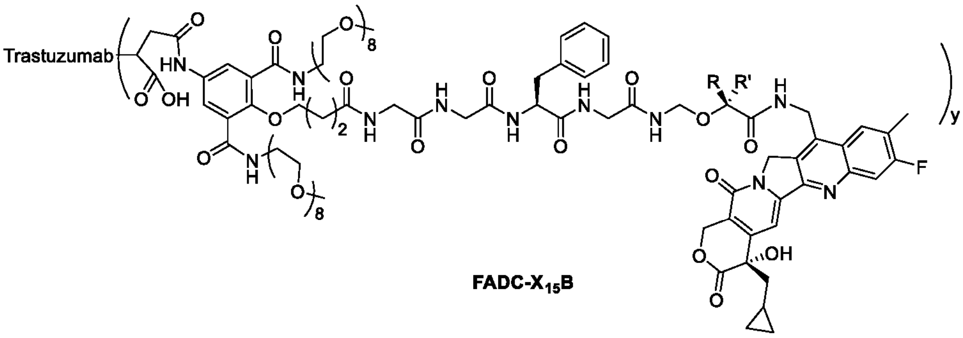

FADC-$X_{15}$B

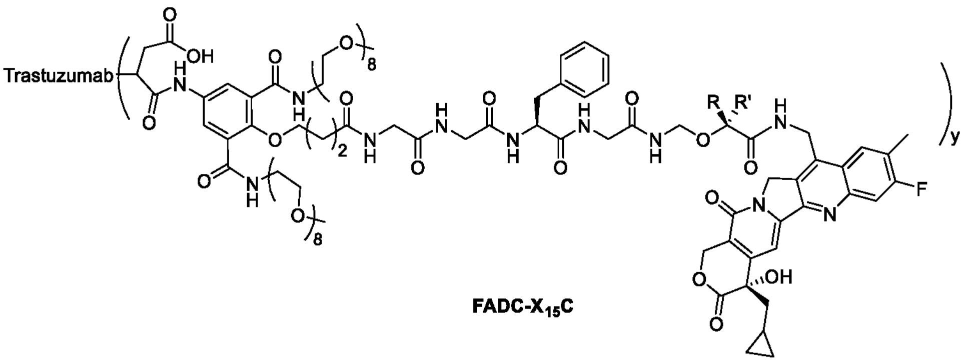

FADC-$X_{15}$C

| Exemplary ADC product | Conjugate mixture of FADC-$X_{15}$A and/or FADC-$X_{15}$R and/or FADC-$X_{15}$C | R | R' |
|---|---|---|---|
| ADC-67 | FADC-67A and/or FADC-67B and/or FADC-67C | $-CH_3$ | -H |
| ADC-68 | FADC-68A and/or FADC-68B and/or FADC-68C | -H | $-CH_3$ |
| ADC-69 | FADC-69A and/or FADC-69B and/or FADC-69C | 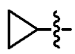 | -H |
| ADC-70 | FADC-70A and/or FADC-70B and/or FADC-70C | -H | 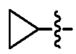 |

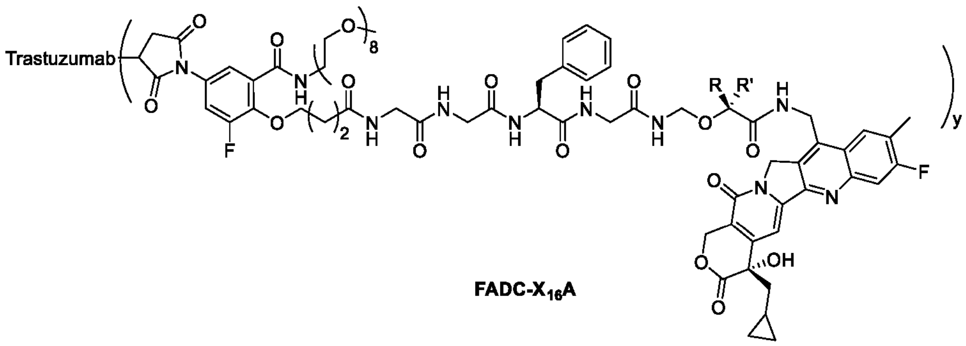

FADC-$X_{16}$A

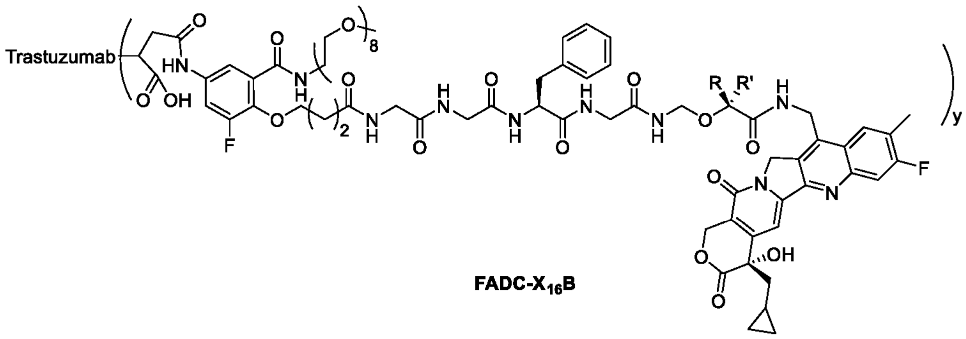

FADC-$X_{16}$B

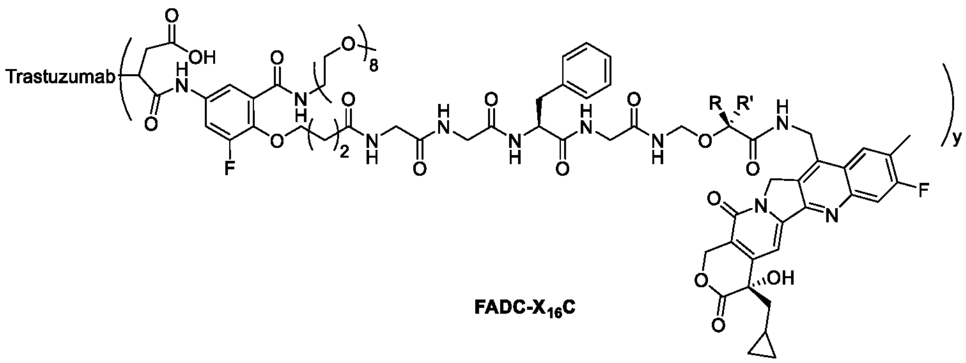

FADC-$X_{16}$C

| Exemplary ADC product | Conjugate mixture of FADC-$X_{16}$A and/or FAD-$X_{16}$B and/or FADC-$X_{16}$C | R | R' |
|---|---|---|---|
| ADC-71 | FADC-71A and/or FADC-71B and/or FADC-71C | -H | -H |
| ADC-72 | FADC-72A and/or FADC-72B and/or FADC-72C | $-CH_3$ | -H |
| ADC-73 | FADC-73A and/or FADC-73B and/or FADC-73C | -H | $-CH_3$ |
| ADC-74 | FADC-74A and/or FADC-74B and/or FADC-74C | 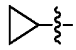 | -H |
| ADC-75 | FADC-75A and/or FADC-75B and/or FADC-75C | -H | 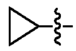 |

**LD-66** to **LD-74** were used instead of **LD-65,** respectively, and similar preparation methods were used to obtain the corresponding **ADC-67** to **ADC-75,** which were Examples 4-67 to 4-75.

Example 4-76: ADC-76 to Example 4-80: ADC-80

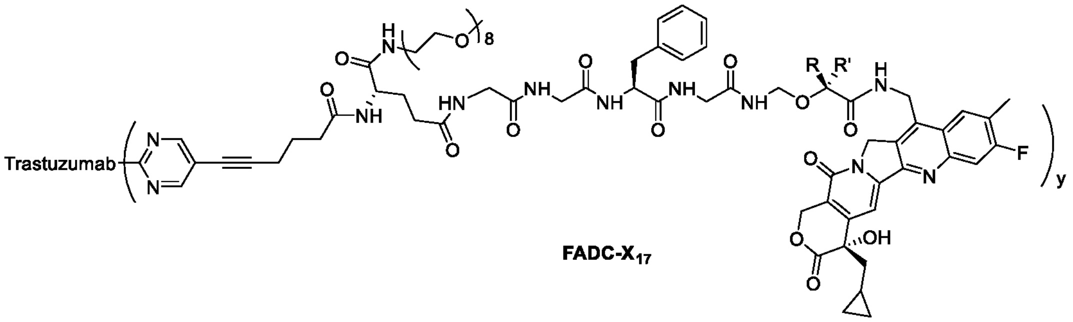

FADC-X$_{17}$

| Exemplary ADC product | General formula FADC-X$_{17}$ | |
|---|---|---|
| | R | R' |
| ADC-76 | -H | -H |
| ADC-77 | $-CH_3$ | -H |
| ADC-78 | -H | $-CH_3$ |
| ADC-79 | 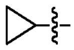 | -H |
| ADC-80 | -H | 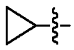 |

Using the antibody Trastuzumab and **LD-75** to **LD-79** as starting materials, respectively, the preparation was carried out by applying the method disclosed in the patent application "WO2019114666A1". PBS buffers with exemplary products **ADC-76** to **ADC-80** of the general formulas **FADC-76** to **FADC-80** were obtained, respectively, and stored at 4 °C.

Example 4-81: ADC-81

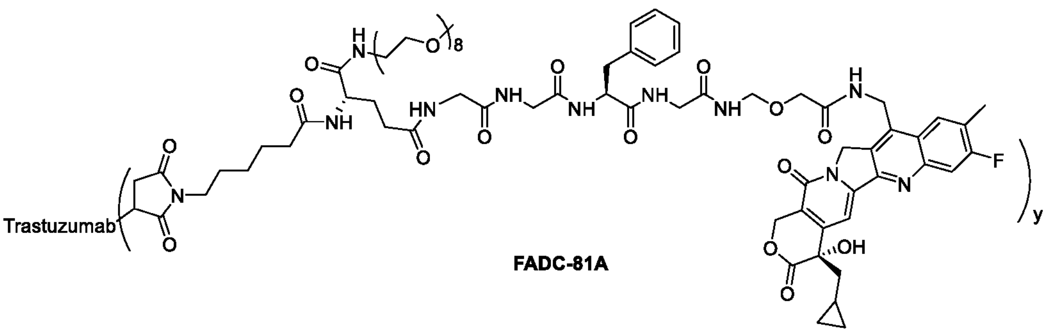

**FADC-81A**

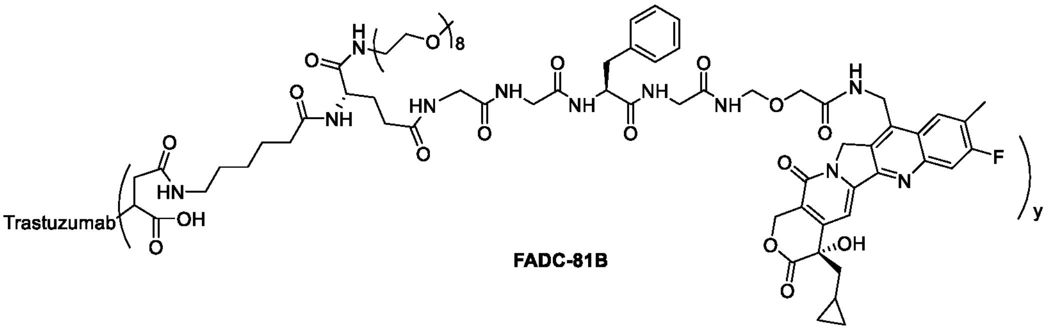

**FADC-81B**

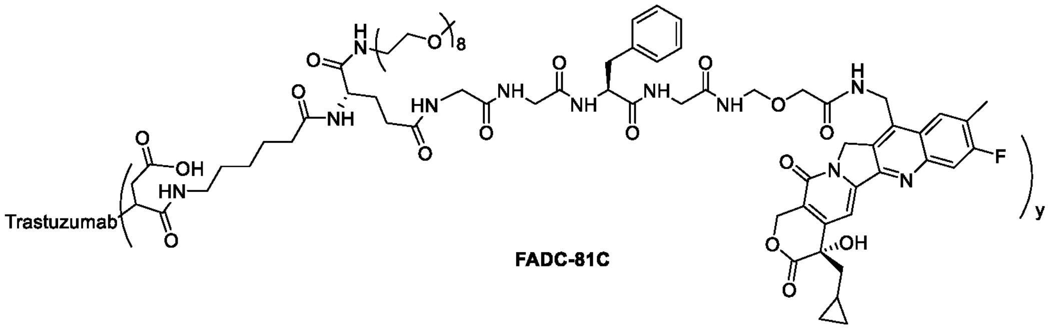

FADC-81C

Using the antibody Trastuzumab and **LD-80** as starting materials, the preparation was carried out by applying the method disclosed in the patent application "WO2022068898A1". The PBS buffer with the exemplary product **ADC-81** of the conjugate mixture (mixture of **FADC-81A** and/or **FADC-81B** and/or **FADC-81C**) was obtained and stored at 4 °C.

Example 4-82: ADC-82 to Example 4-85: ADC-85

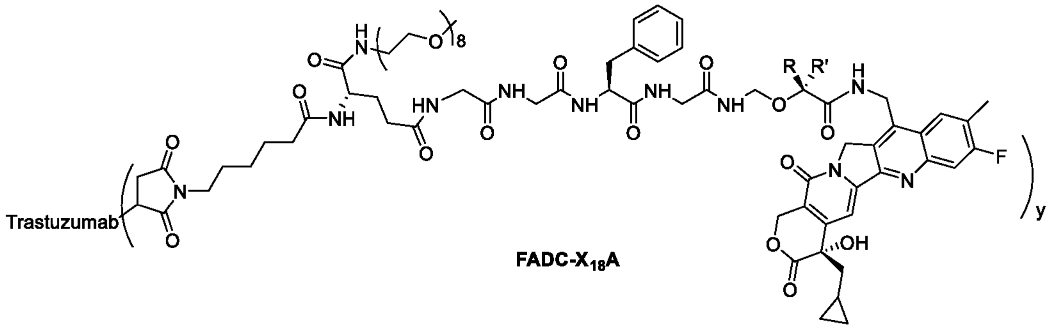

FADC-$X_{18}$A

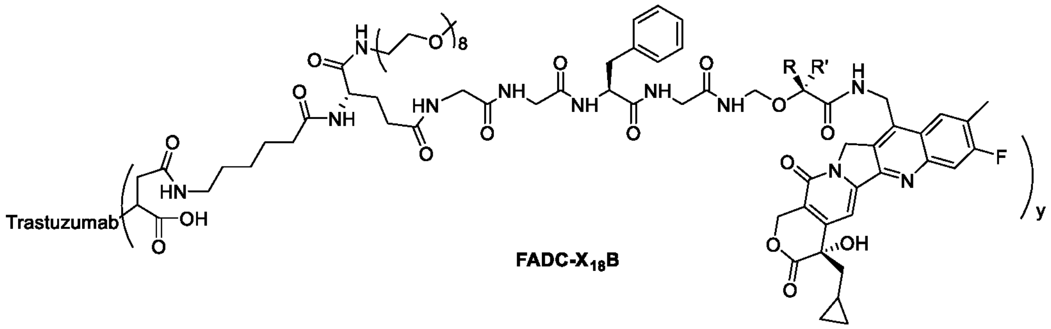

FADC-$X_{18}$B

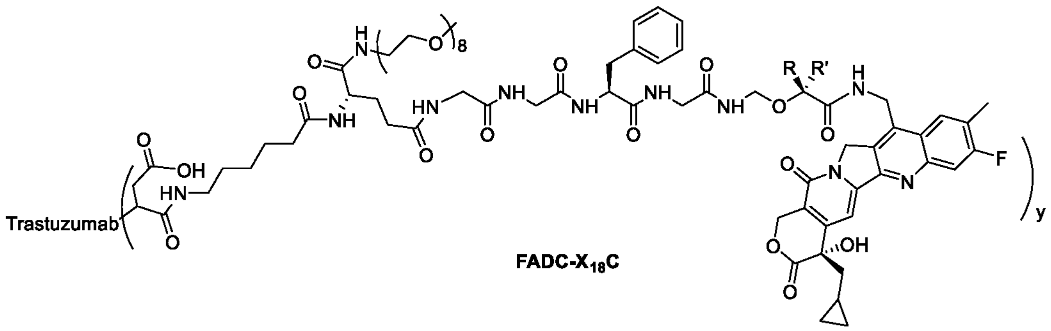

FADC-$X_{18}$C

| Exemplary ADC product | Conjugate mixture of FADC-$X_{18}$A and/or FADC-$X_{18}$B and/or FADC-$X_{18}$C | R | R' |
|---|---|---|---|
| ADC-82 | FADC-82A and/or FADC-82B and/or FADC-82C | $-CH_3$ | -H |
| ADC-83 | FADC-83A and/or FADC-83B and/or FADC-83C | -H | $-CH_3$ |
| ADC-84 | FADC-84A and/or FADC-84B and/or FADC-84C | 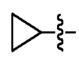 | -H |
| ADC-85 | FADC-85A and/or FADC-85B and/or FADC-85C | -H | 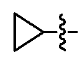 |

Using **LD-81** to **LD-84** instead of LD-80, respectively, similar preparation methods were applied to obtain the corresponding **ADC-82** to **ADC-85,** which are Examples 4-82 to 4-85.

Example 4-86: ADC-86

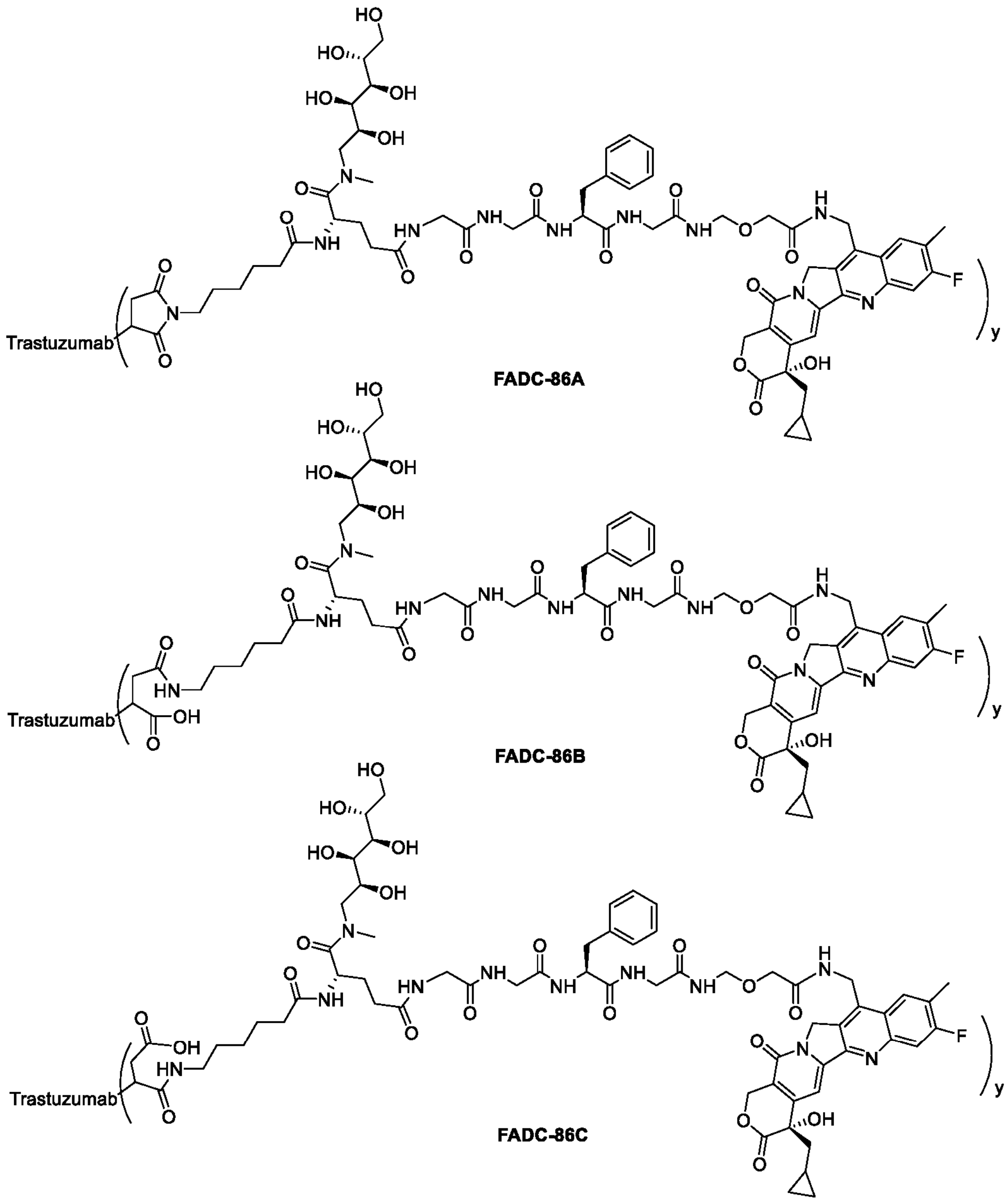

FADC-86A

FADC-86B

FADC-86C

Using the antibody Trastuzumab and **LD-85** as starting materials, the preparation was carried out by applying the method disclosed in the patent application "WO2022068898A1". The PBS buffer with the exemplary product **ADC-86** of the conjugate mixture (mixture of **FADC-86A** and/or **FADC-86B** and/or **FADC-86C**) was obtained and stored at 4 °C.

Example 4-87: ADC-87 to Example 4-90: ADC-90

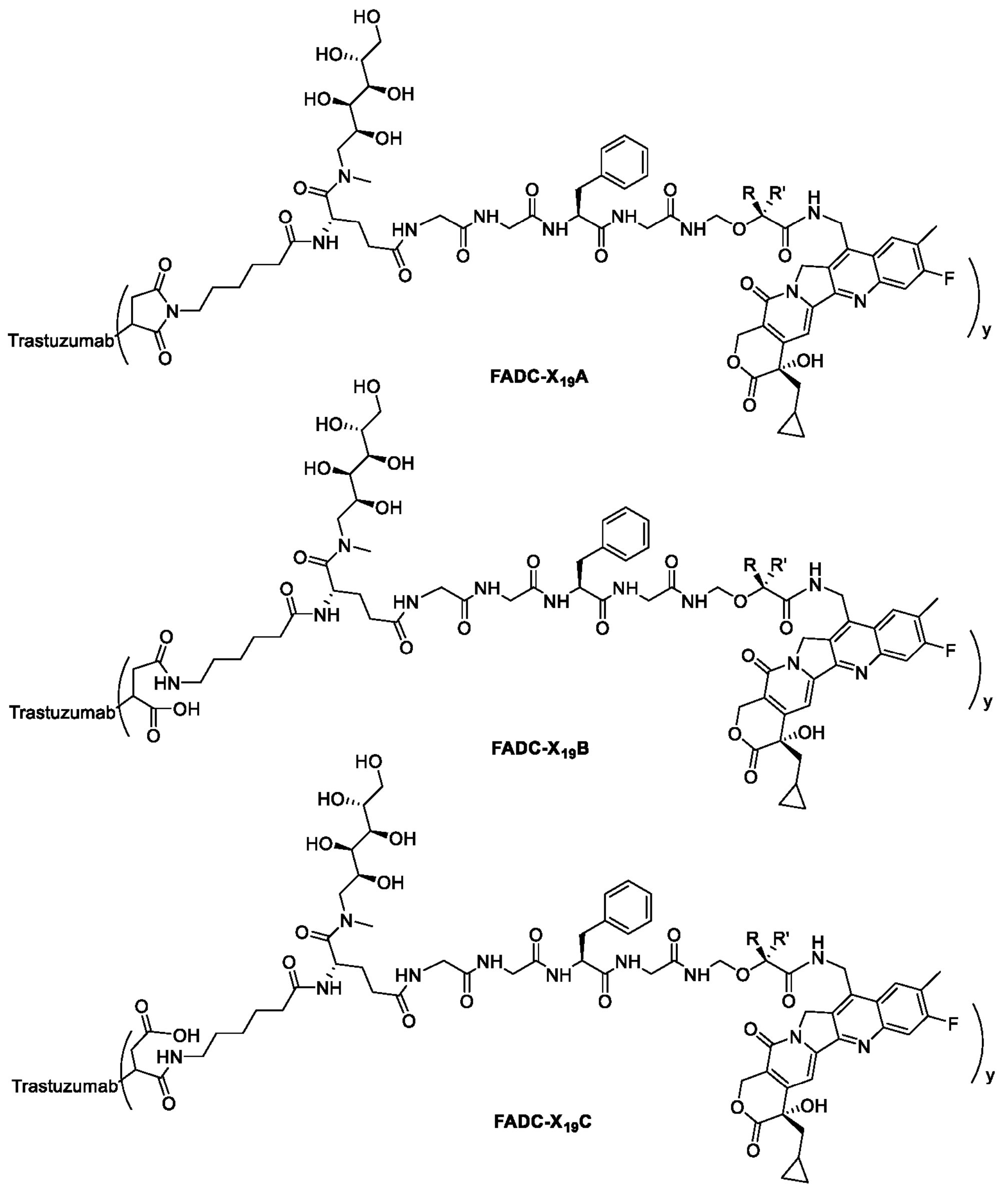

FADC-$X_{19}$A

FADC-$X_{19}$B

FADC-$X_{19}$C

| Exemplary ADC product | Conjugate mixture of FADC-$X_{19}$A and/or FADC-$X_{19}$B and/or FADC-$X_{19}$C | R | R' |
|---|---|---|---|
| ADC-87 | FADC-87A and/or FADC-87B and/or FADC-87C | $-CH_3$ | -H |
| ADC-88 | FADC-88A and/or FADC-88B and/or FADC-88C | -H | $-CH_3$ |
| ADC-89 | FADC-89A and/or FADC-89B and/or FADC-89C | 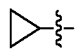 | -H |
| ADC-90 | FADC-90A and/or FADC-90B and/or FADC-90C | -H | 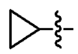 |

**LD-86** to **LD-89** were used instead of LD-85, respectively, and similar preparation methods were used to obtain the corresponding **ADC-87** to **ADC-90,** which were Examples 4-87 to 4-90.

Example 4-91: ADC-91 to Example 4-97: ADC-97

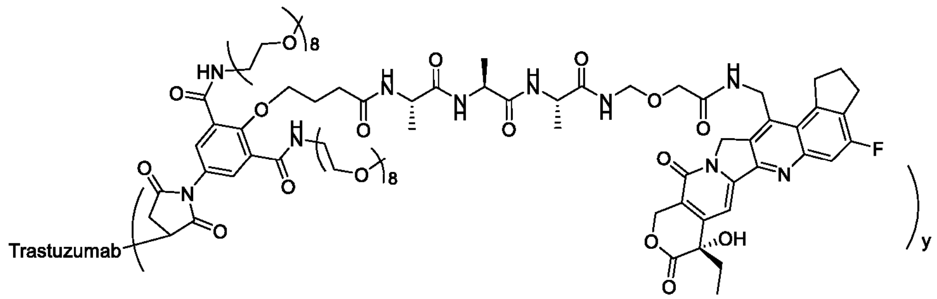

**FADC-91A**

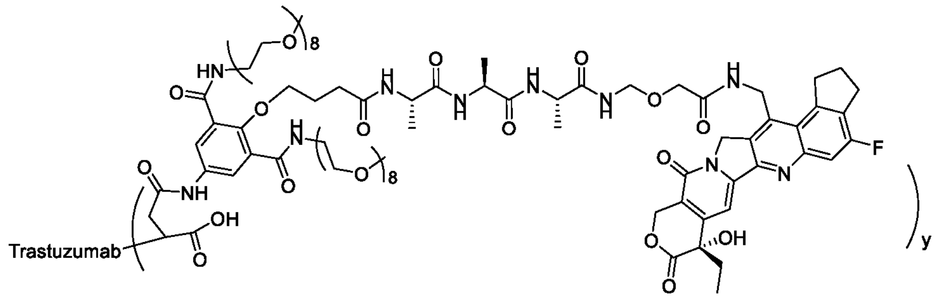

**FADC-91B**

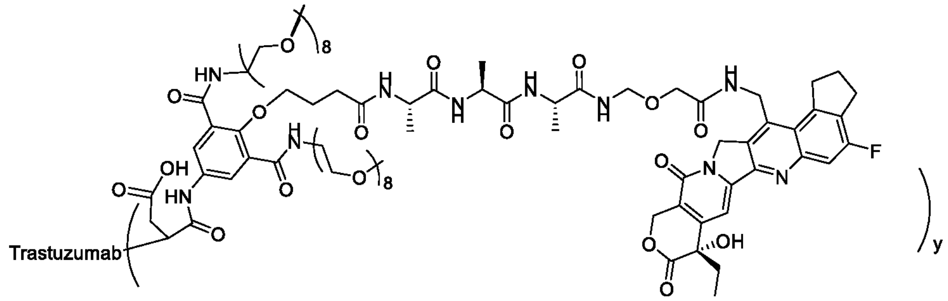

**FADC-91C**

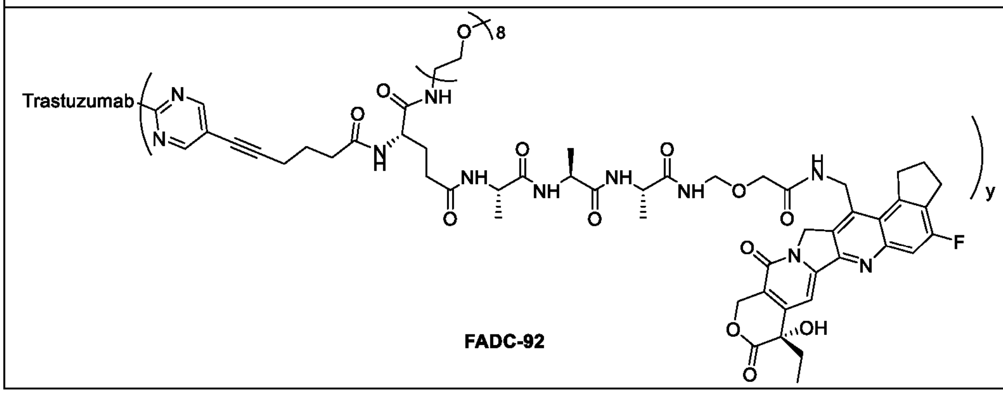

**FADC-92**

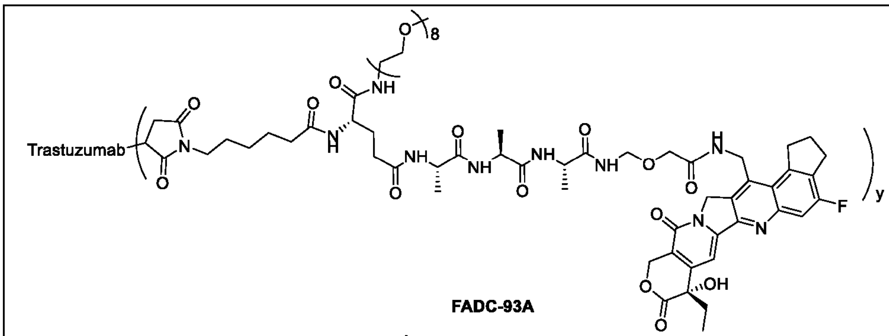

FADC-93A

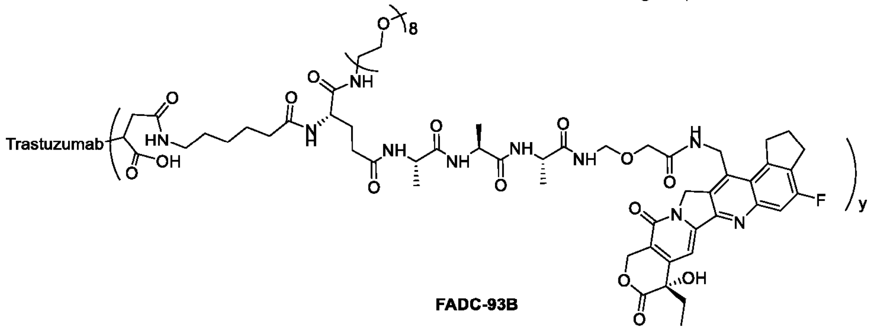

FADC-93B

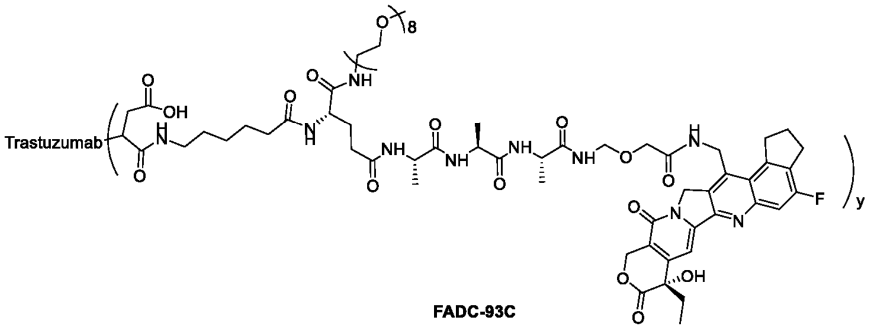

FADC-93C

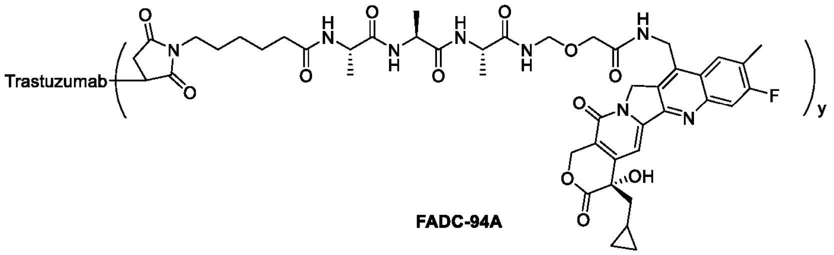

FADC-94A

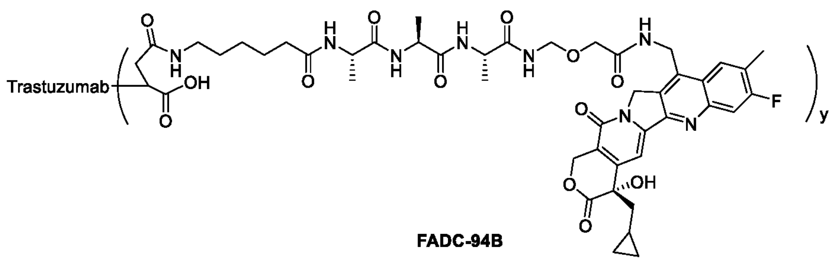

FADC-94B

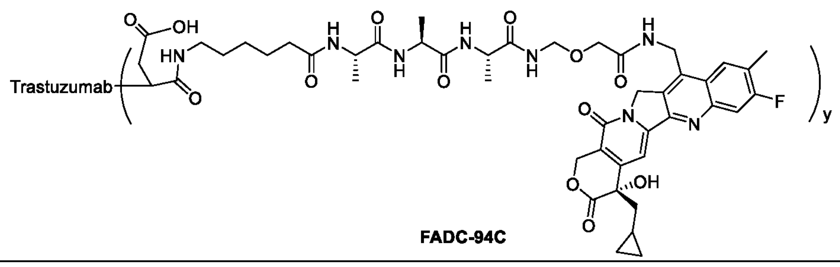

FADC-94C

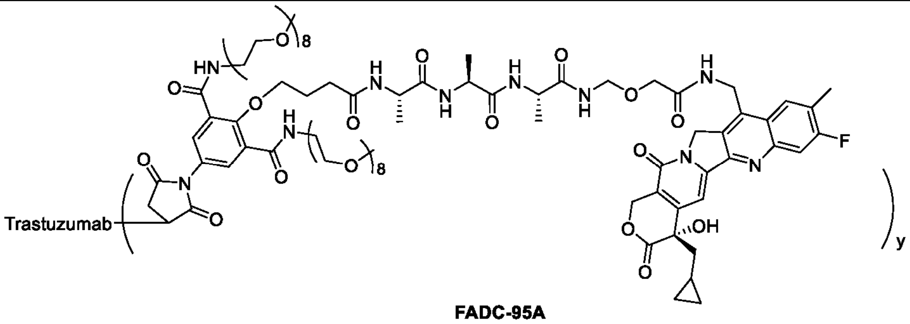

FADC-95A

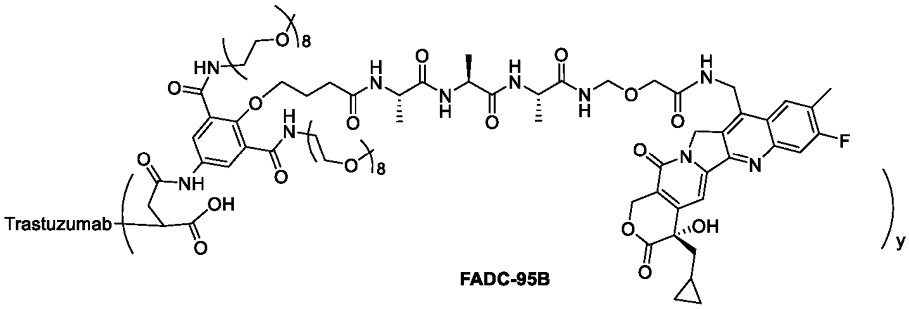

FADC-95B

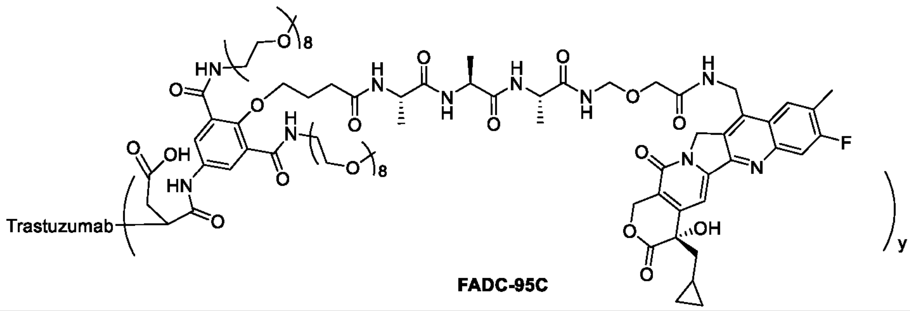

FADC-95C

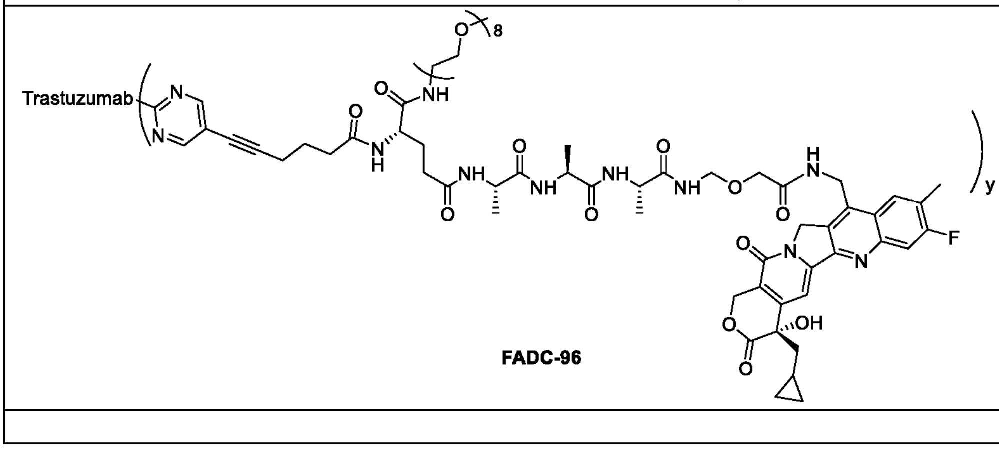

FADC-96

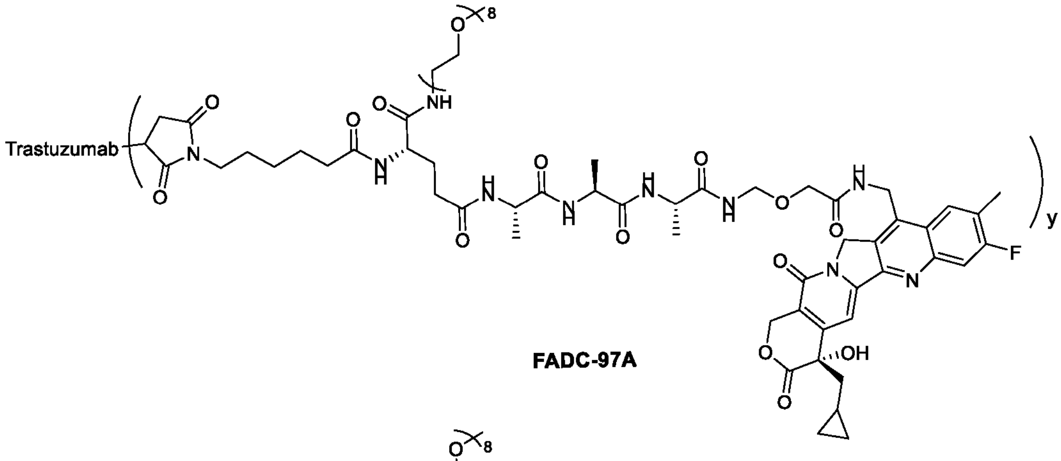

**FADC-97A**

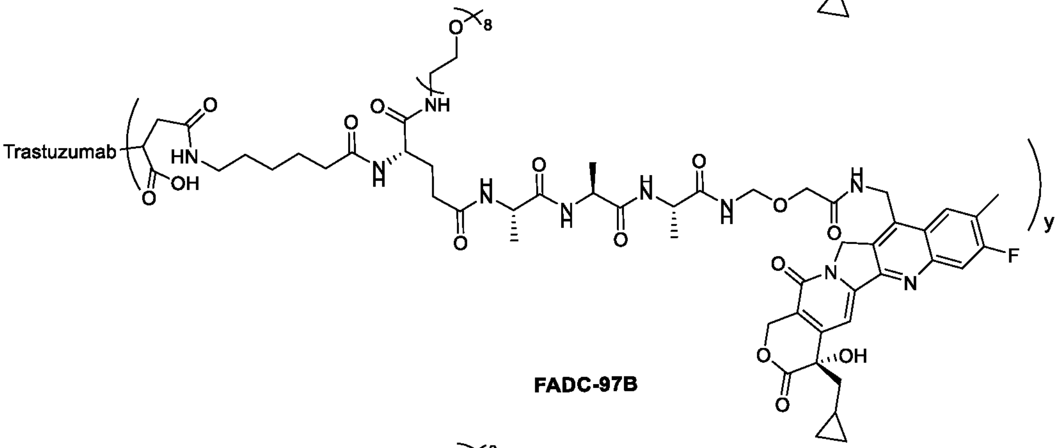

**FADC-97B**

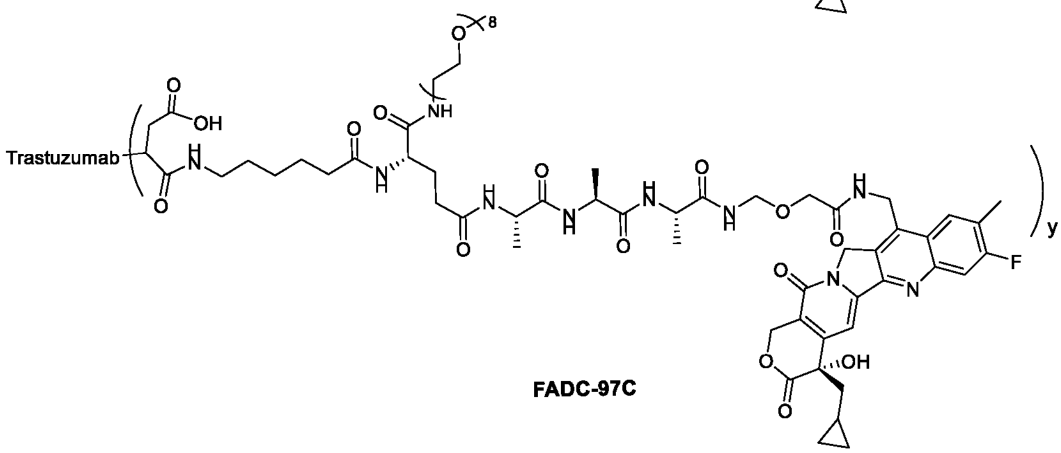

**FADC-97C**

**LD-90** to **LD-96** and antibody Trastuzumab were used as starting materials, respectively, and similar preparation methods were used to obtain the corresponding **ADC-91** to **ADC-97,** which were Examples 4-91 to 4-97.

Example 4-98: ADC-98 to Example 4-100: ADC-100

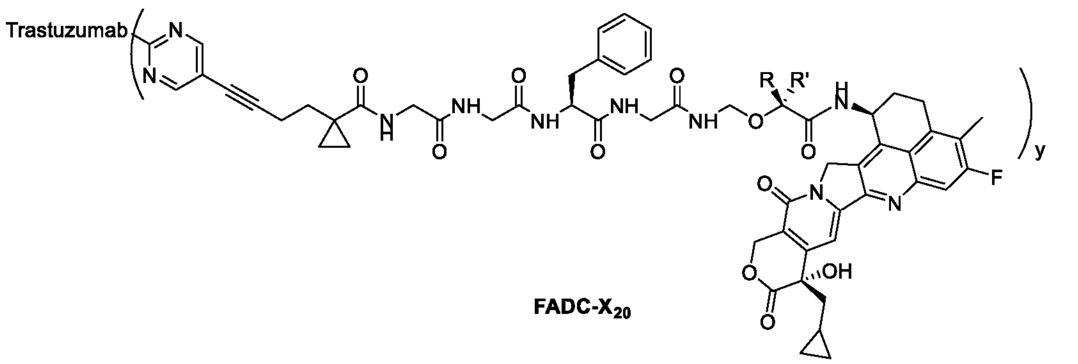

FADC-$X_{20}$

| Exemplary ADC product | General formula FADC-$X_{20}$ | |
|---|---|---|
| | R | R' |
| ADC-98 | -H | -H |
| ADC-99 | $-CH_3$ | -H |
| ADC-100 | -H | $-CH_3$ |

**LD-97** to **LD-99** and antibody Trastuzumab were used as starting materials, respectively, and the preparation was carried out by applying the method of step C. The PBS buffers with the exemplary products **ADC-98** to **ADC-100** of the corresponding conjugate mixtures **FADC-98** to **FADC-100** were obtained and stored at 4 °C, which were Examples 4-98 to 4-100.

The average drug loading values were calculated by mass spectrometry:

ADC-98, y=7.97;

ADC-99, y=8.00;

ADC-100, y=8.02.

Example 4-101: ADC-101 to Example 4-103: ADC-103

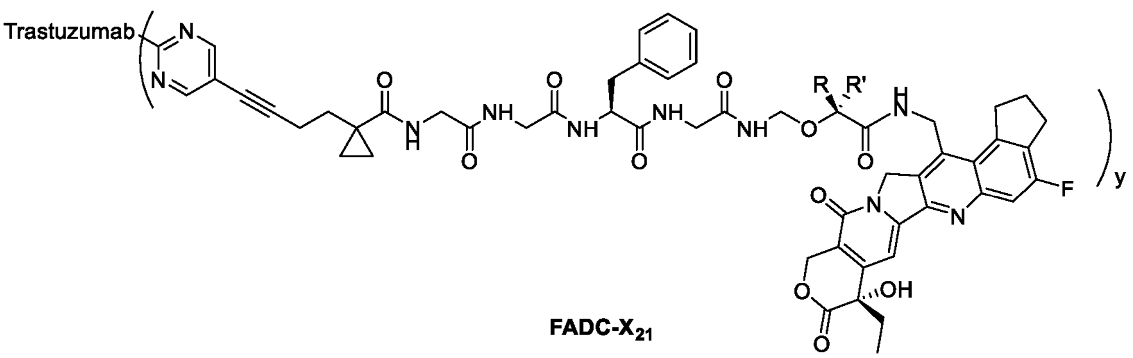

FADC-$X_{21}$

| Exemplary ADC product | General formula FADC-$X_{21}$ | |
|---|---|---|
| | R | R' |
| ADC-101 | -H | -H |
| ADC-102 | $-CH_3$ | -H |
| ADC-103 | -H | $-CH_3$ |

**LD-100** to **LD-102** and antibody Trastuzumab were used as starting materials, respectively, and the preparation was carried out by applying the method disclosed in patent application "WO2019114666A1". The corresponding **ADC-101** to **ADC-103** were obtained, which were Examples 4-101 to 4-103.

Example 4-104: ADC-104

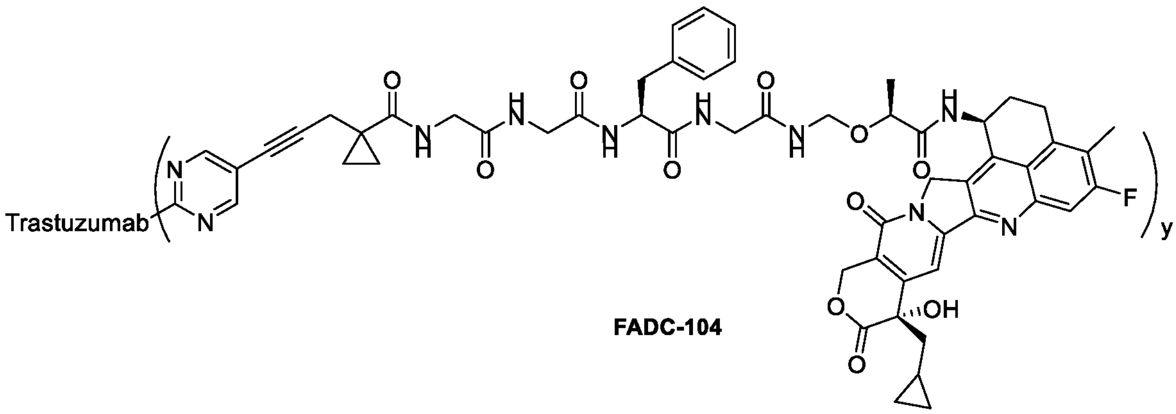

**FADC-104**

**LD-103** and antibody Trastuzumab were used as starting materials, and the preparation was carried out by applying the method of step C. The PBS buffer with the exemplary product **ADC-104** of the corresponding conjugate mixture **FADC-104** was obtained and stored at 4 °C, which was Example 4-104.

The average drug loading value was calculated by mass spectrometry: y=5.85.

Example 4-105: ADC-105 to Example 4-106: ADC-106

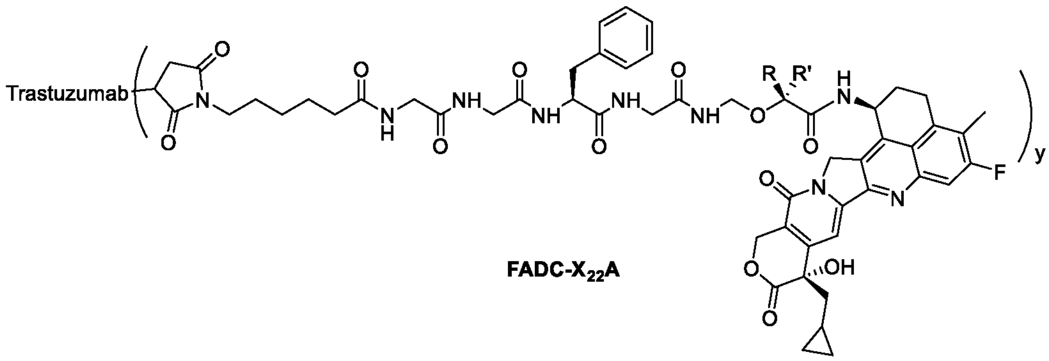

**FADC-$X_{22}$A**

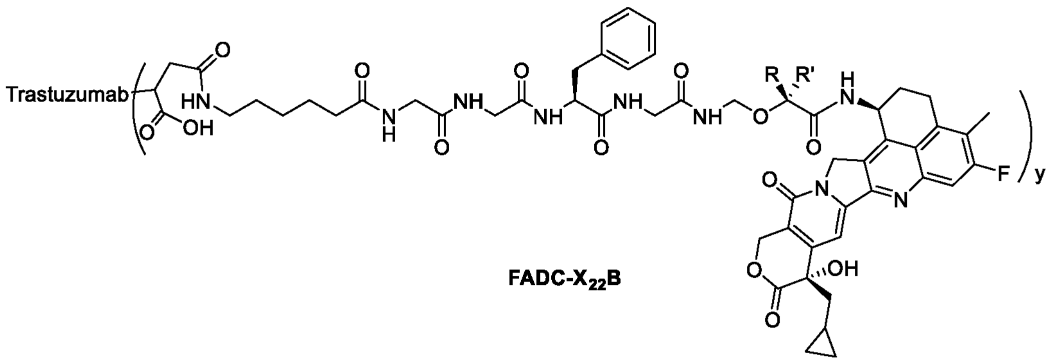

**FADC-$X_{22}$B**

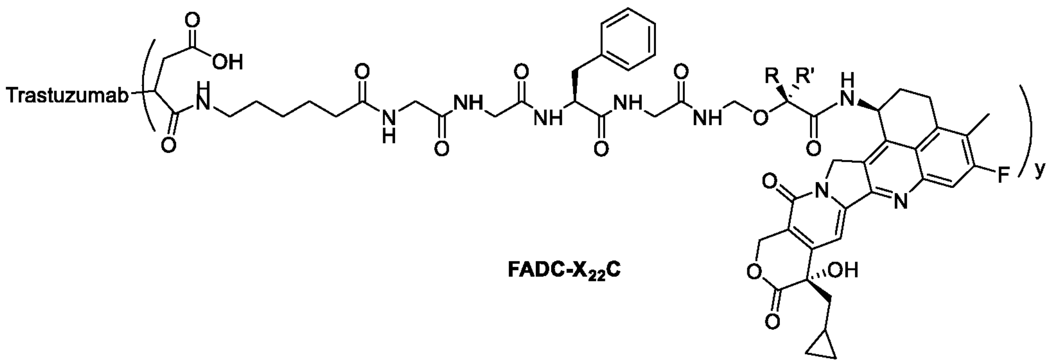

**FADC-$X_{22}$C**

| Exemplary ADC product | Conjugate mixture of FADC-$X_{22}$A and/or FADC-$X_{22}$B and/or FADC-$X_{22}$C | R | R' |
|---|---|---|---|
| ADC-105 | FADC-105A and/or FADC-105B and/or FADC-105C | $-CH_3$ | -H |
| ADC-106 | FADC-106A and/or FADC-106B and/or FADC-106C | -H | $-CH_3$ |

Using the antibody Trastuzumab and **LD-104** as starting materials, the preparation was carried out by applying the method of step B. The PBS buffer with the exemplary product **ADC-105** of the conjugate mixture (mixture of **FADC-105A** and/or **FADC-105B** and/or **FADC-105C**) was obtained and stored at 4 °C.

The average drug loading value calculated by mass spectrometry: ADC-105, y=8.04.

Using the antibody Trastuzumab and **LD-105** as starting materials, the preparation was carried out by applying the method of step B. The PBS buffer with the exemplary product **ADC-106** of the conjugate mixture (mixture of **FADC-106A** and/or **FADC-106B** and/or **FADC-106C**) was obtained and stored at 4 °C.

The average drug loading value was calculated by mass spectrometry: ADC-106, y=7.95.

Example 4-107: ADC-107 to Example 4-110: ADC-110

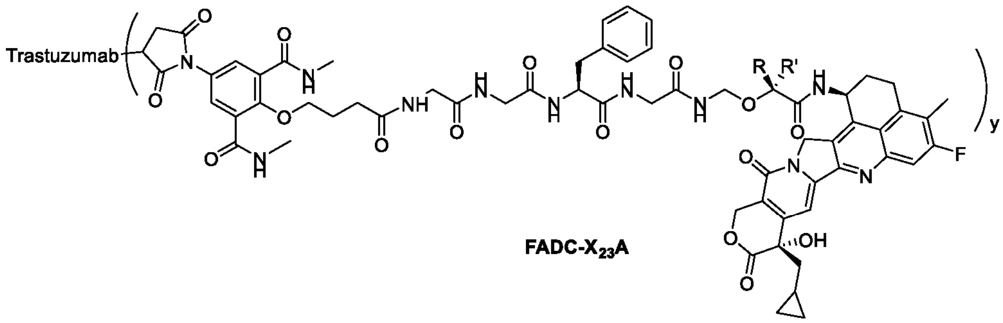

FADC-$X_{23}$A

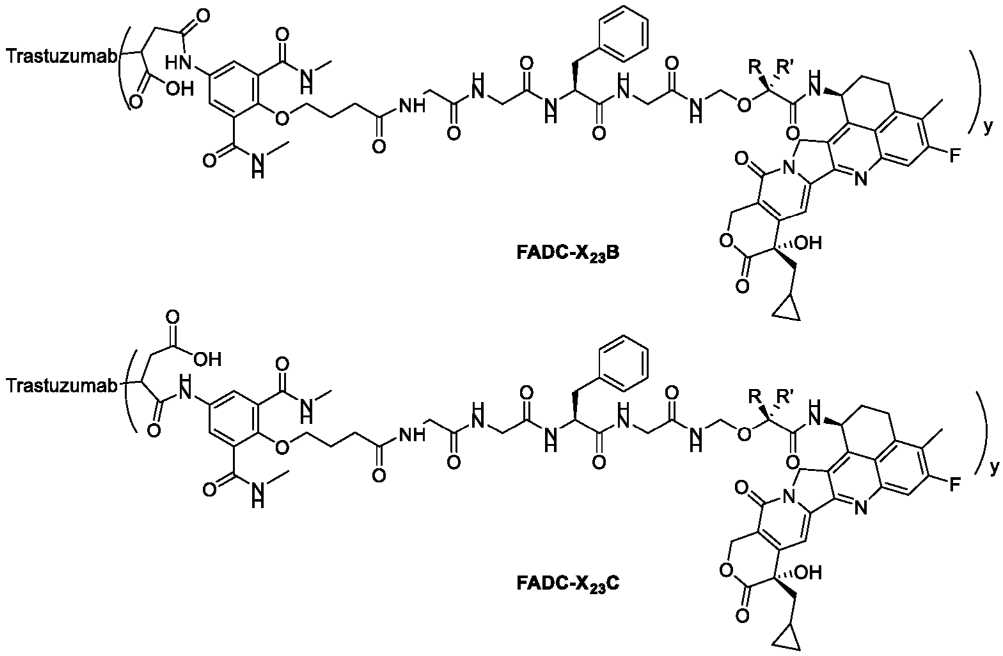

FADC-$X_{23}$B

FADC-$X_{23}$C

| Exemplary ADC product | Conjugate mixture of FADC-$X_{23}$A and/or FADC-$X_{23}$B and/or FADC-$X_{23}$C | R | R' |
|---|---|---|---|
| ADC-107 | FADC-107A and/or FADC-107B and/or FADC-107C | -H | -H |
| ADC-108 | FADC-108A and/or FADC-108B and/or FADC-108C | $-CH_3$ | -H |
| ADC-109 | FADC-109A and/or FADC-109B and/or FADC-109C | 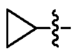 | -H |
| ADC-110 | FADC-110A and/or FADC-110B and/or FADC-110C | -H | 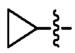 |

Using the antibody Trastuzumab and **LD-106** as starting materials, the preparation was carried out by applying the method of step A-1. The PBS buffer with the exemplary product **ADC-107** of the conjugate mixture (mixture of **FADC-107A** and/or **FADC-107B** and/or **FADC-107C)** was obtained and stored at 4 °C, which was Example 4-107.

Using **LD-107** to **LD-109** and antibody Trastuzumab as starting materials, respectively, the preparation was carried out by applying the method of step A-1, and the corresponding PBS buffers of **ADC-108** to **ADC110** were obtained and stored at 4 °C, which were Examples 4-108 to 4-110.

The average drug loading values were calculated by mass spectrometry:

ADC-107, y=8.09;

ADC-108, y=7.43;

ADC-109, y=7.80;

ADC-110, y=7.66.

Example 4-111: ADC-111

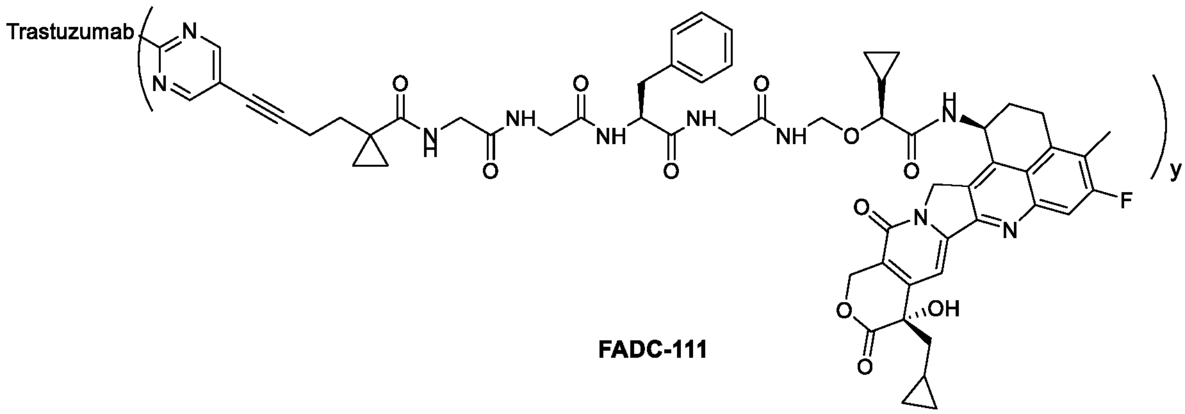

**FADC-111**

Using the antibody Trastuzumab and **LD-110** as starting materials, the preparation was carried out by applying the method of step C. The PBS buffer with the exemplary product **ADC-111** of the conjugate mixture **FADC-111** was obtained and stored at 4 °C, which was Example 4-111.
The average drug loading value was calculated by mass spectrometry:
ADC-111, y=7.74;

Example 4-112: ADC-112

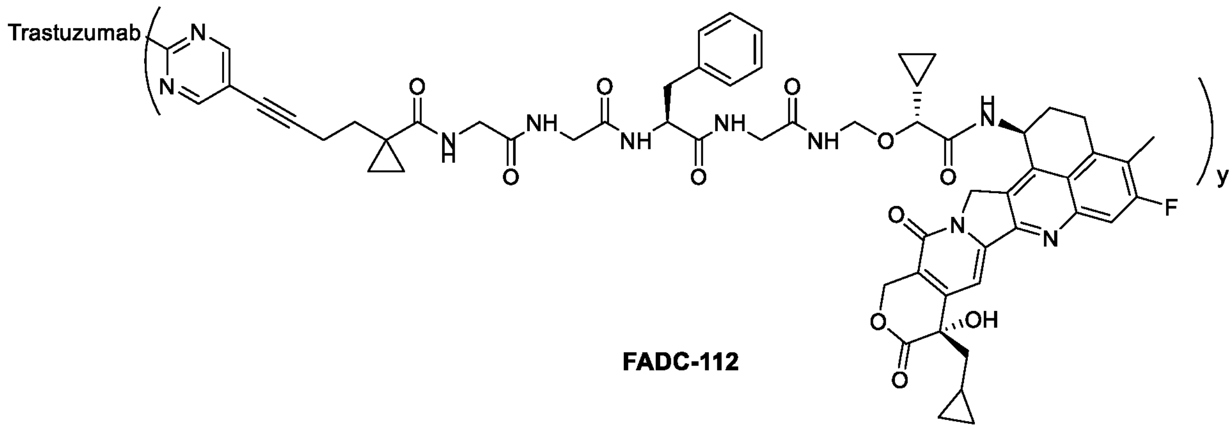

**FADC-112**

Using the antibody Trastuzumab and **LD-111** as starting materials, the preparation was carried out by applying the method of step C, and the PBS buffer with the exemplary product **ADC-112** of the conjugate mixture **FADC-112** was obtained and stored at 4 °C, which was Example 4-112.
The average drug loading value was calculated by mass spectrometry:
ADC-112, y=7.86.

Example 4-113: ADC-113 to Example 4-117: ADC-117

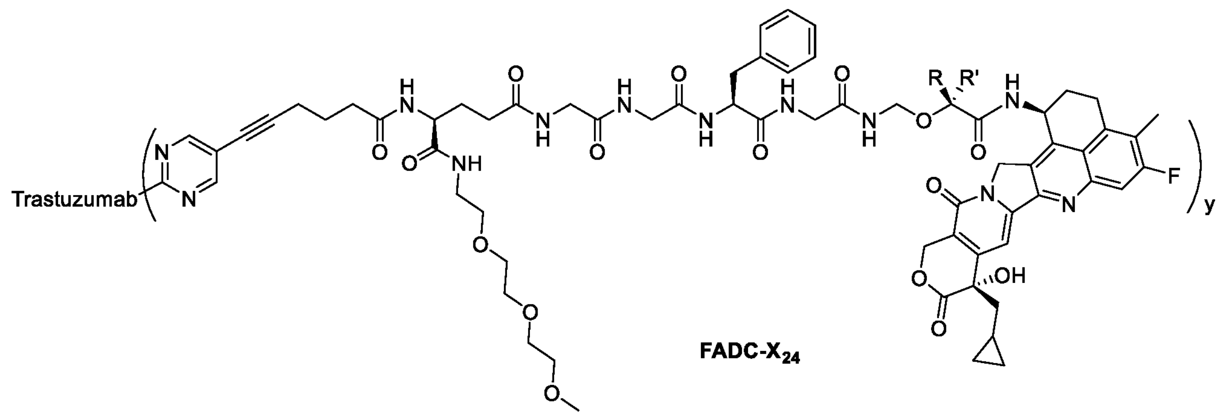

**FADC-X$_{24}$**

| Exemplary ADC product | General formula FADC-X$_{24}$ | |
|---|---|---|
| | R | R' |
| AD-C-113 | -H | -H |
| AD-C-114 | $-CH_3$ | -H |
| AD-C-115 | -H | $-CH_3$ |
| AD-C-116 | 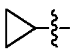 | -H |
| AD-C-117 | -H | 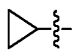 |

Using the antibody Trastuzumab and **LD-112** as starting materials, the preparation was carried out by applying the method of step C. The PBS buffer with the exemplary product **ADC-113** of the conjugate mixture **FADC-113** was obtained and stored at 4 °C, which was Example 4-113.

Using **LD-113** to **LD-116** and the antibody Trastuzumab as starting materials, respectively, the preparation was carried out by similar preparation methods, and the corresponding PBS buffers of **ADC-114** to **ADC117** were obtained and stored at 4 °C, respectively, which were Examples 4-114 to 4-117.

The average drug loading values were calculated by mass spectrometry:

ADC-113, y=8.02;

ADC-114, y=7.91;

ADC-115, y=6.93;

ADC-116, y=7.88;

ADC-117, y=7.99.

Example 4-118: Reference 1:

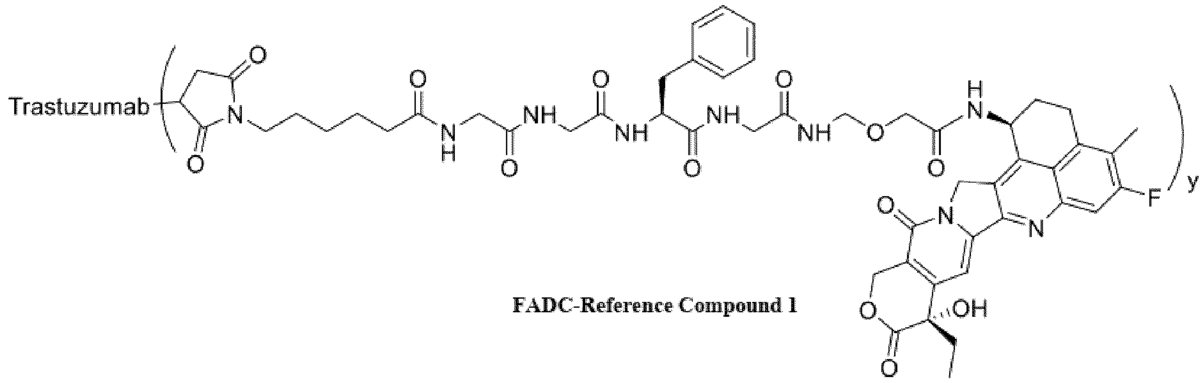

FADC-Reference Compound 1

Using the antibody Trastuzumab and N-((*S*)-10-benzyl-1-(((1*S*,9*S*)-9-ethyl-5-fluoro- 9-hydroxy-4-methyl-10,13-dioxo-2,3,9,10,13,15-hexahydro-1*H*,12*H*-benzo[de]pyrano[3',4':6,7] indolizino[1,2-*b*]quinolin-1-yl)amino)-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide as starting materials, the preparation was carried out by applying the method of step B. The PBS buffer with the exemplary product **Reference 1** of the conjugate mixture **FADC-Reference 1** was obtained and stored at 4 °C, which was Example 4-118.

The average drug loading value was calculated by mass spectrometry: y=8.00.

Example 4-119: Reference 2

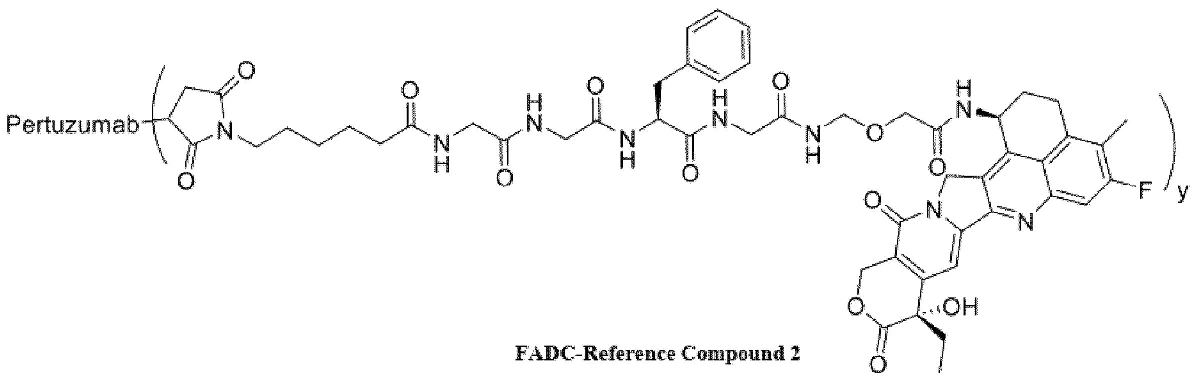

FADC-Reference Compound 2

Using the antibody Pertuzumab and *N*-((*S*)-10-benzyl-1-(((1*S*,9*S*)-9-ethyl-5-fluoro- 9-hydroxy-4-methyl-10,13-dioxo-2,3,9,10,13,15-hexahydro-1*H*,12*H*-benzo[*de*]pyrano[3',4':6,7] indolizino[1,2-*b*]quinolin-1-yl)amino)-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide as starting materials, the preparation was carried out by applying the method of step B. The PBS buffer of the exemplary product **Reference 2** of the conjugate mixture **FADC-Reference 2** was obtained and stored at 4 °C, which was Example 4-119.

The average drug loading value was calculated by mass spectrometry: y=7.67.

Example 4-120: ADC-118

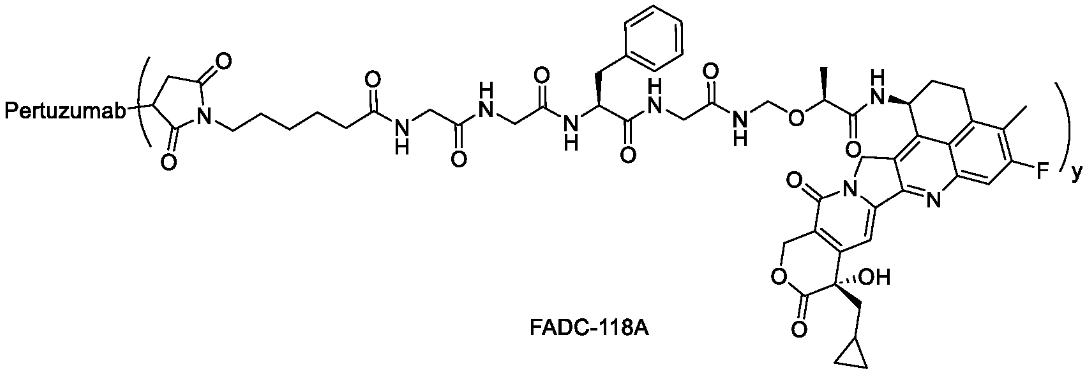

FADC-118A

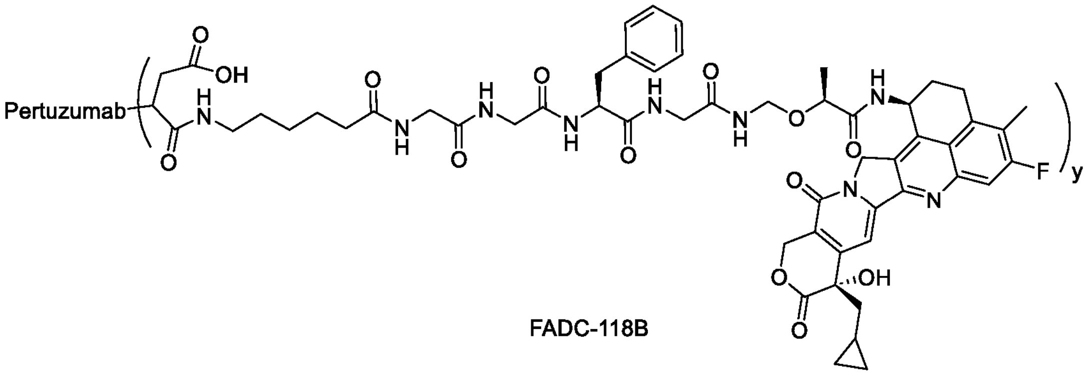

FADC-118B

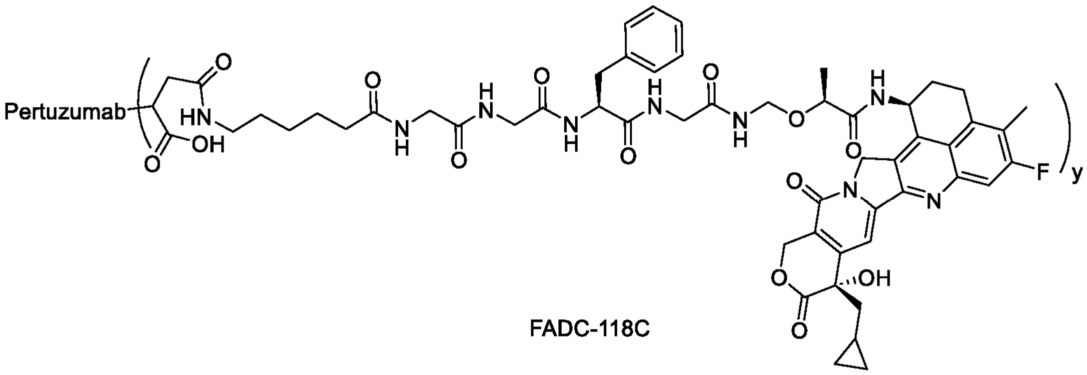

FADC-118C

Using the antibody Pertuzumab and **LD-104** as starting materials, the preparation was carried out by applying the method of step B. The PBS buffer with the exemplary product **ADC-118** of the conjugate mixture (mixture of **FADC-118A** and/or **FADC-118B** and/or **FADC-118C**) was obtained and stored at 4 °C, which was Example 4-120.

The average drug loading value was calculated by mass spectrometry: y=4.95.

Example 4-121: ADC-119

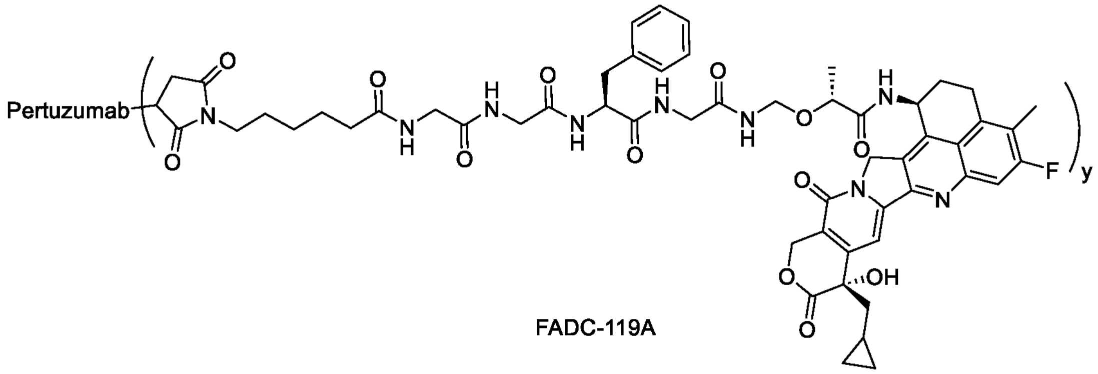

FADC-119A

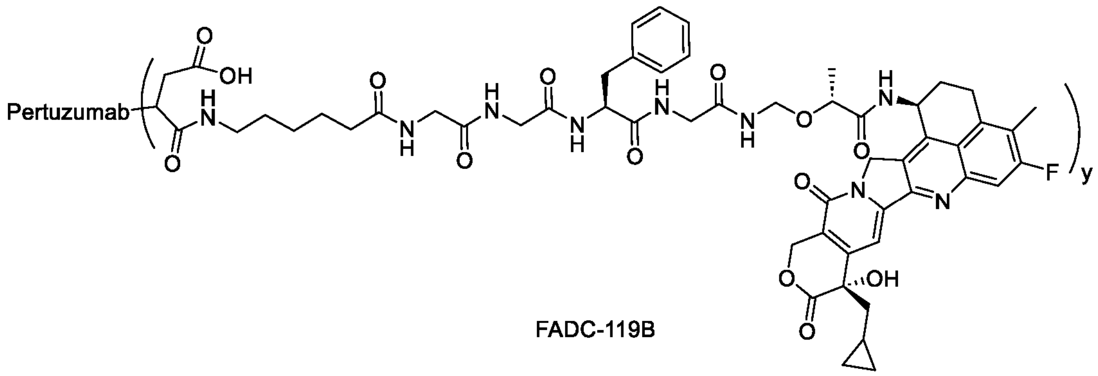

FADC-119B

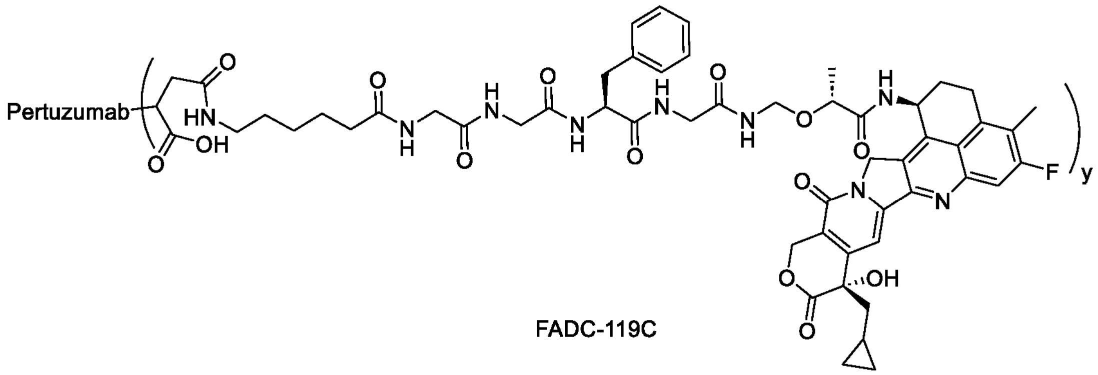

FADC-119C

Using the antibody Pertuzumab and **LD-105** as starting materials, the preparation was carried out by applying the method of step B. The PBS buffer with the exemplary product **ADC-119** of the conjugate mixture (mixture of **FADC-119A** and/or **FADC-119B** and/or **FADC-119C**) was obtained and stored at 4 °C, which was Example 4-121.

The average drug loading value was calculated by mass spectrometry: y=3.06.

Example 4-122: ADC-120

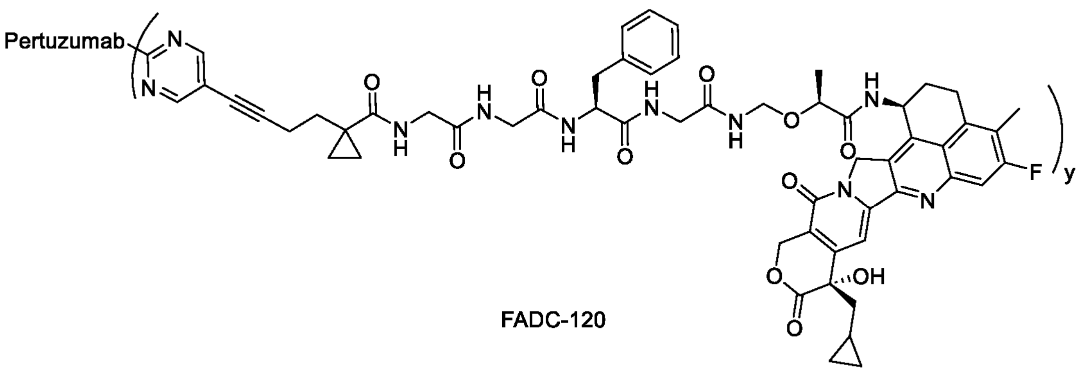

FADC-120

Using the antibody Pertuzumab and **LD-98** as starting materials, the preparation was carried out by applying the method of step C. The PBS buffer with the exemplary product **ADC-120** of the conjugate mixture **FADC-120** was obtained and stored at 4 °C, which was Example 4-122.

The average drug loading value was calculated by mass spectrometry: y=7.48.

Example 4-123: ADC-121

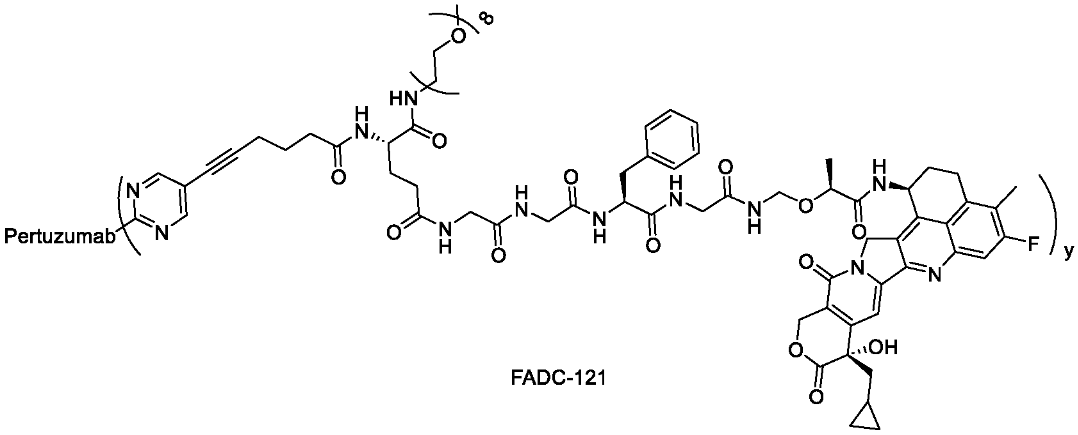

FADC-121

Using the antibody Pertuzumab and **LD-13** as starting materials, the preparation was carried out by applying the method of step C. The PBS buffer with the exemplary product **ADC-121** of the conjugate mixture **FADC-121** was obtained and stored at 4 °C, which was Example 4-123.

The average drug loading value was calculated by mass spectrometry: y=7.59.

Example 4-124: ADC-122

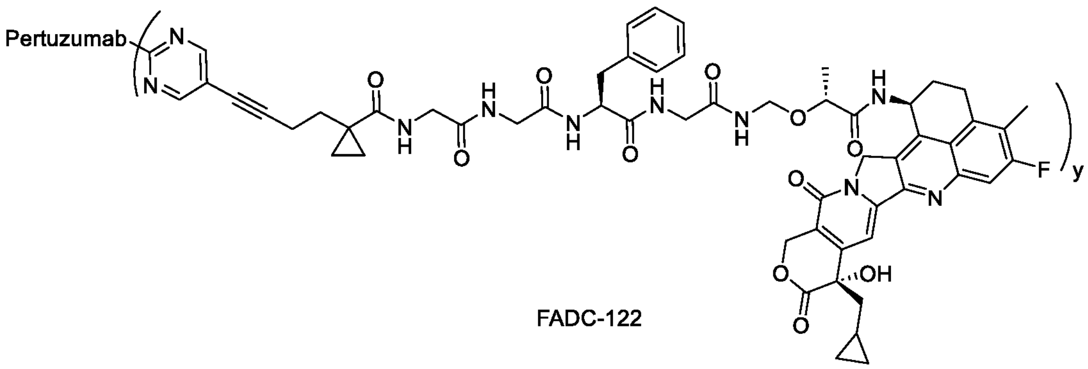

FADC-122

Using the antibody Pertuzumab and **LD-99** as starting materials, the preparation was carried out by applying the method of step C. The PBS buffer with the exemplary product **ADC-122** of the conjugate mixture **FADC-122** was obtained and stored at 4 °C, which was Example 4-124.

The average drug loading value was calculated by mass spectrometry: y=7.89.

Example 4-125: ADC-123

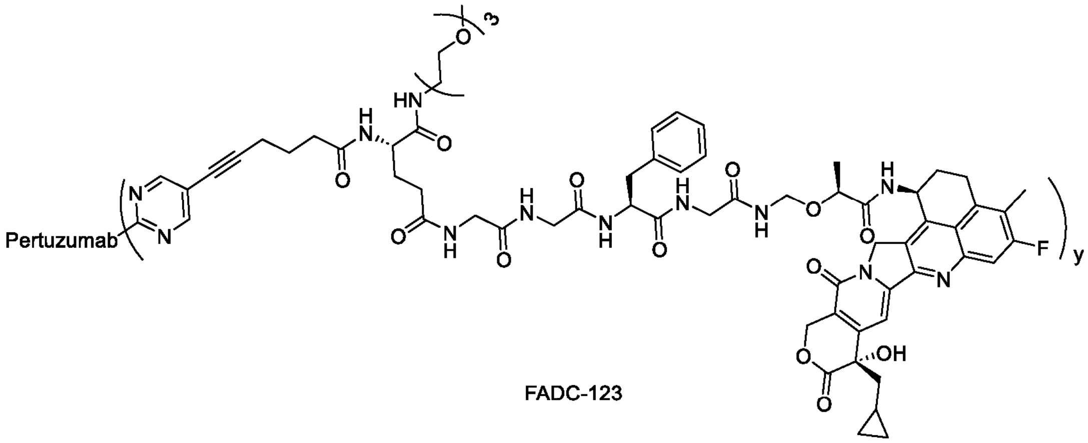

FADC-123

Using the antibody Pertuzumab and **LD-113** as starting materials, the preparation was carried out by applying the method of step C. The PBS buffer with the exemplary product **ADC-123** of the conjugate mixture **FADC-123** was obtained and stored at 4 °C, which was Example 4-125.

The average drug loading value was calculated by mass spectrometry: y=7.99.

Example 4-126: ADC-124

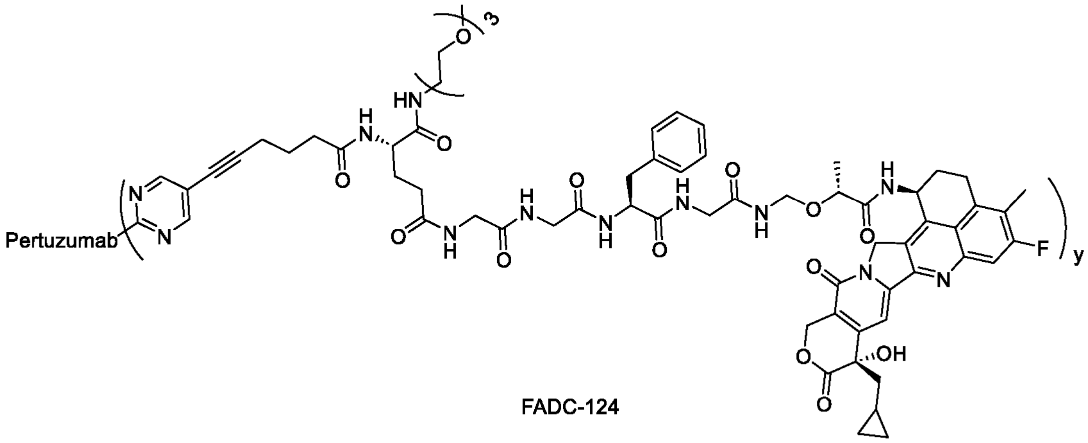

FADC-124

Using the antibody Pertuzumab and **LD-114** as starting materials, the preparation was carried out by applying the method of step C. The PBS buffer with the exemplary product **ADC-124** of the conjugate mixture **FADC-124** was obtained and stored at 4 °C, which was Example 4-126.

The average drug loading value was calculated by mass spectrometry: y=7.82.

Example 4-127: ADC-125

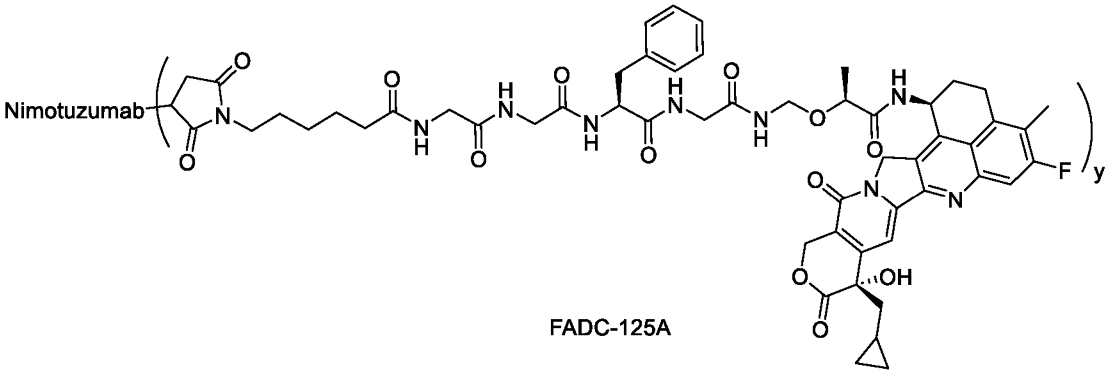

FADC-125A

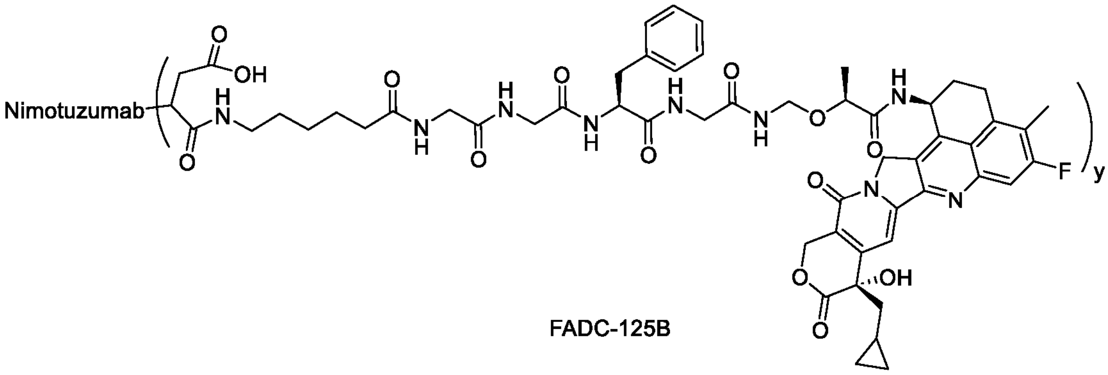

FADC-125B

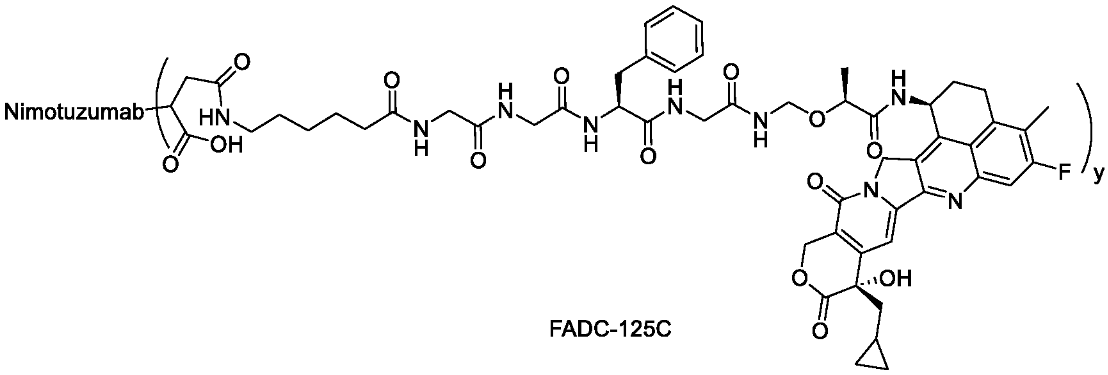

FADC-125C

Using the antibody Nimotuzumab and **LD-104** as starting materials, the preparation was carried out by applying the method of step B. The PBS buffer with the exemplary product **ADC-125** of the conjugate mixture (mixture of **FADC-125A** and/or **FADC-125B** and/or **FADC-125C**) was obtained and stored at 4 °C, which was Example 4-127.

The average drug loading value was calculated by mass spectrometry: y=5.66.

Example 4-128: ADC-126

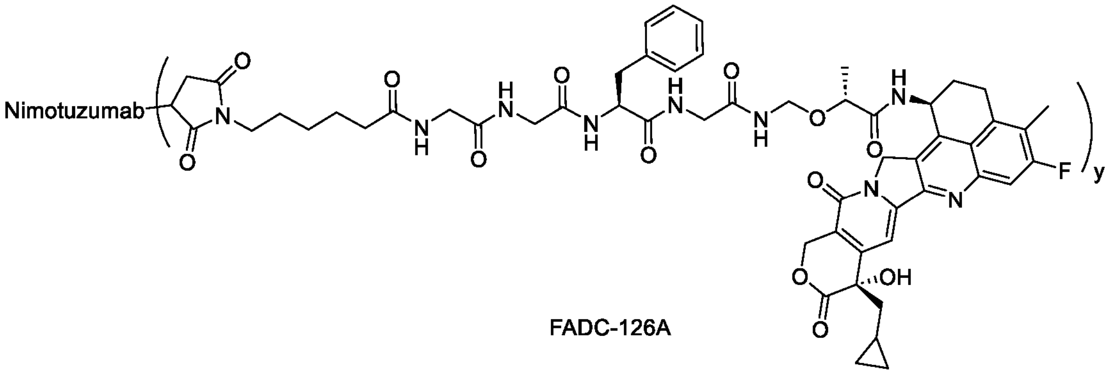

FADC-126A

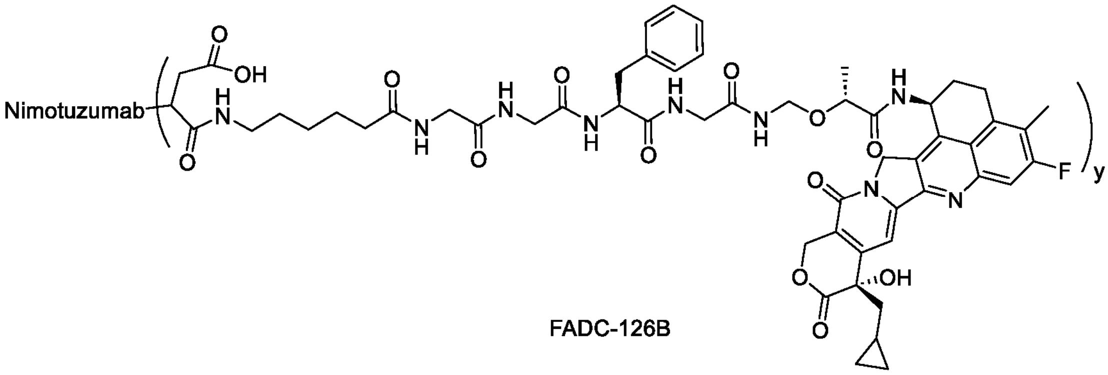

FADC-126B

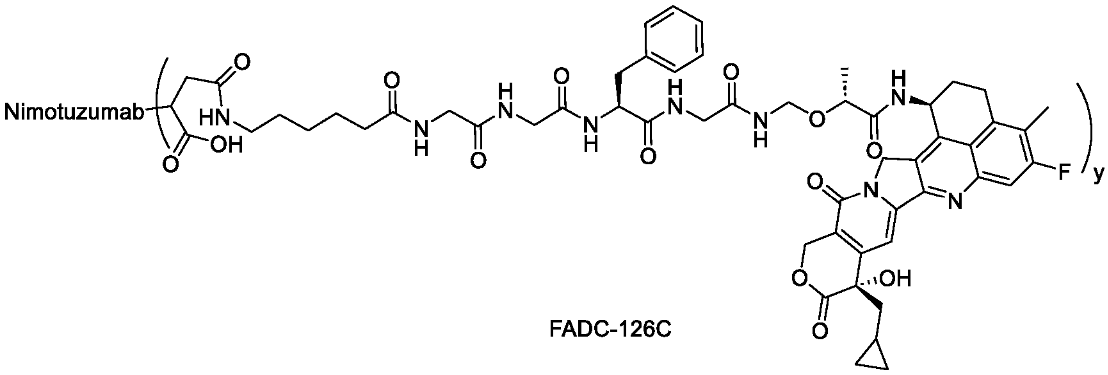

FADC-126C

Using the antibody Nimotuzumab and **LD-105** as starting materials, the preparation was carried out by applying the method of step B. The PBS buffer with the exemplary product **ADC-126** of the conjugate mixture (mixture of **FADC-126A** and/or **FADC-126B** and/or **FADC-126C**) was obtained and stored at 4 °C, which was Example 4-128.
The average drug loading value was calculated by mass spectrometry: y=4.96.

Example 4-129: ADC-127

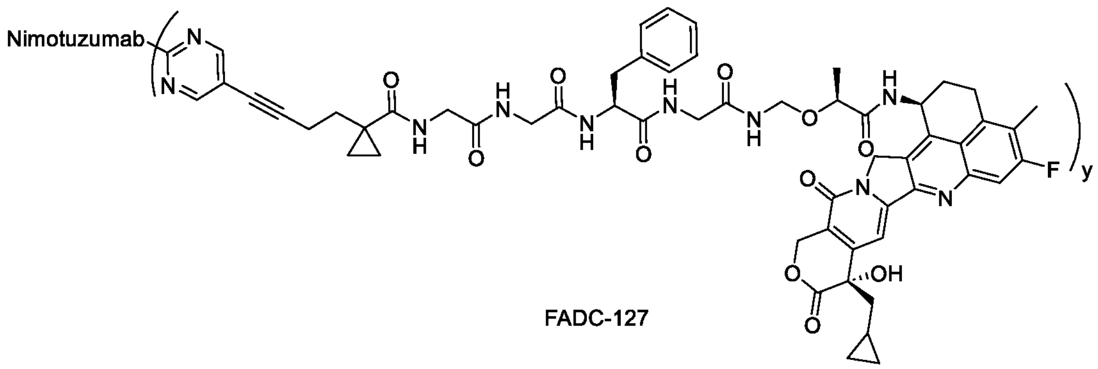

FADC-127

Using the antibody Nimotuzumab and **LD-98** as starting materials, the preparation was carried out by applying the method of step C. The PBS buffer with the exemplary product **ADC-127** of the conjugate mixture **FADC-127** was obtained and stored at 4 °C, which was Example 4-129.

The average drug loading value was calculated by mass spectrometry: y=6.31.

Example 4-130: ADC-128

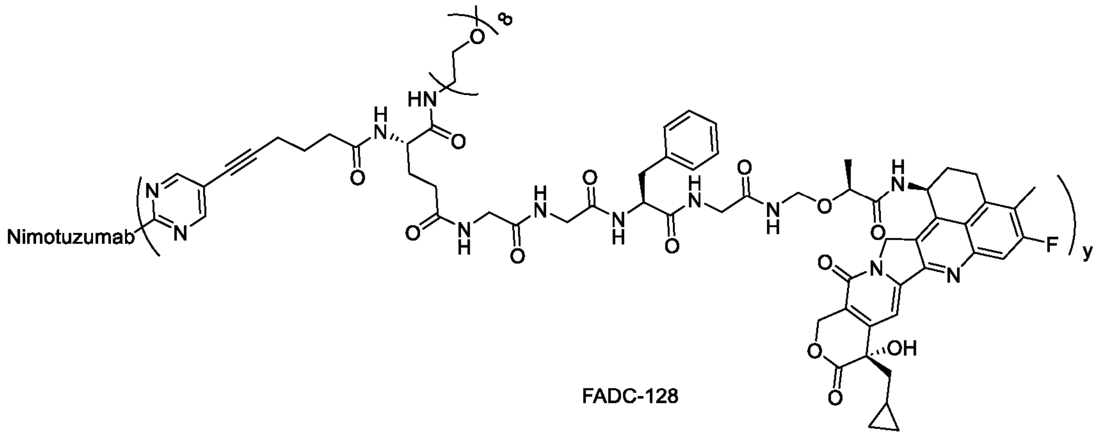

FADC-128

Using the antibody Nimotuzumab and **LD-13** as starting materials, the preparation was carried out by applying the method of step C. The PBS buffer with the exemplary product ADC-128 of the conjugate mixture **FADC-128** was obtained and stored at 4 °C, which was Example 4-130.

The average drug loading value was calculated by mass spectrometry: y=7.62.

Example 4-131: ADC-129

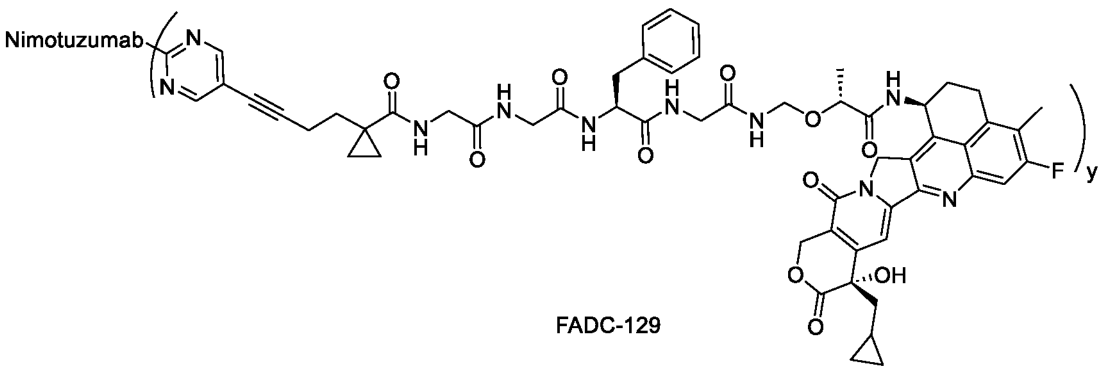

FADC-129

Using the antibody Nimotuzumab and **LD-99** as starting materials, the preparation was carried out by applying the method of step C. The PBS buffer with the exemplary product **ADC-129** of the conjugate mixture **FADC-129** was obtained and stored at 4 °C, which was Example 4-131.

The average drug loading value was calculated by mass spectrometry: y=7.11.

Example 4-132: ADC-130

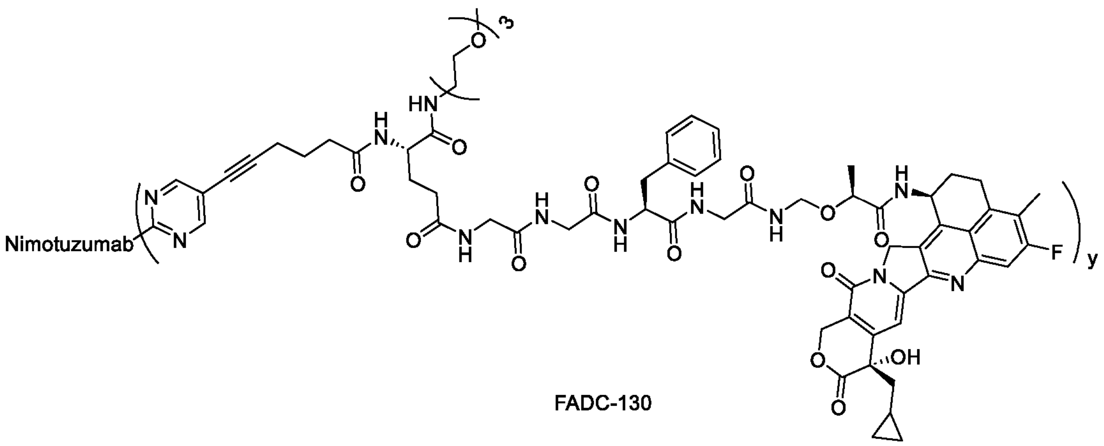

FADC-130

Using the antibody Nimotuzumab and **LD-113** as starting materials, the preparation was carried out by applying the method of step C. The PBS buffer with the exemplary product **ADC-130** of the conjugate mixture **FADC-130** was obtained and stored at 4 °C, which was Example 4-132.

The average drug loading value was calculated by mass spectrometry: y=7.18.

Example 4-133: ADC-131

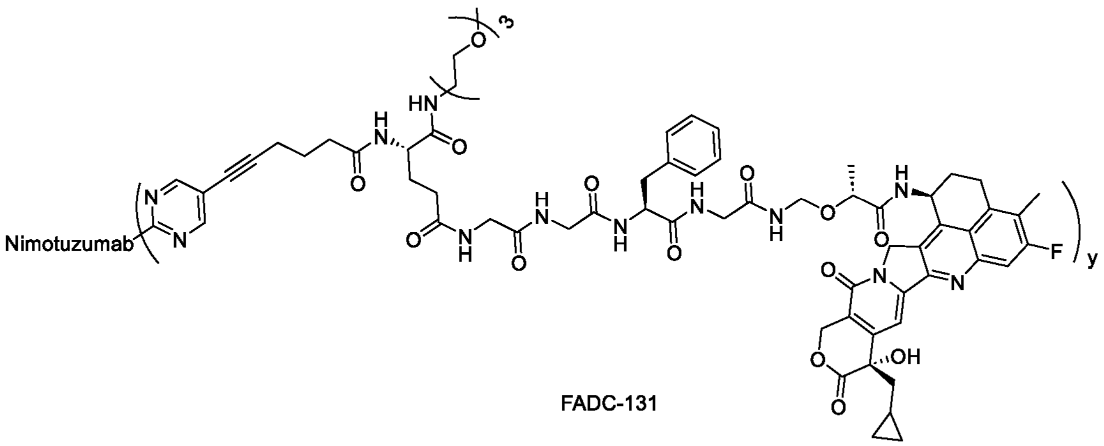

FADC-131

Using the antibody Nimotuzumab and **LD-114** as starting materials, the preparation was carried out by applying the method of step C. The PBS buffer with the exemplary product **ADC-131** of the conjugate mixture **FADC-131** was obtained and stored at 4 °C, which was Example 4-133.

The average drug loading value was calculated by mass spectrometry: y=7.30.

Example 4-134: Reference 3

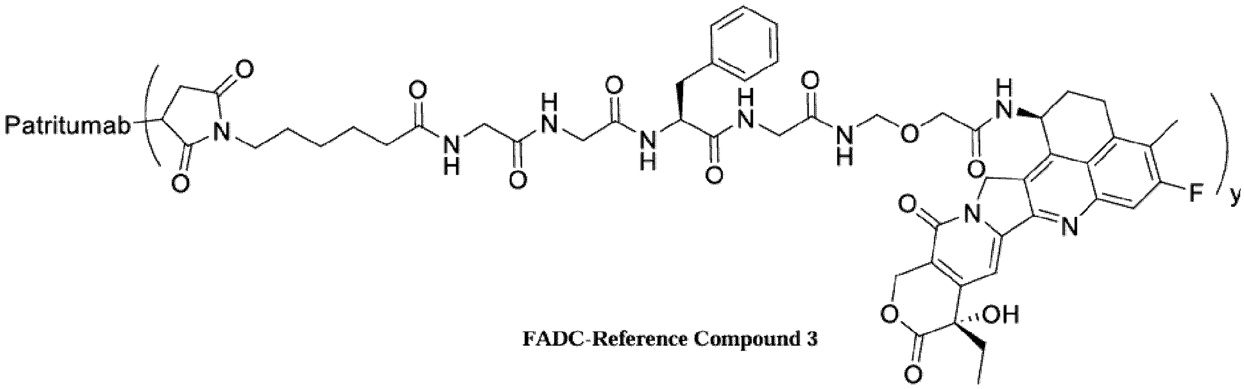

FADC-Reference Compound 3

Using the antibody Patritumab and *N*-((*S*)-10-benzyl-1-(((1*S*,9*S*)-9-ethyl-5-fluoro- 9-hydroxy-4-methyl-10,13-dioxo-2,3,9,10,13,15-hexahydro-1*H*,12*H*-benzo[de]pyrano[3',4':6,7] indolizino[1,2-*b*]quinolin-1-yl)amino)-1,6,9,12,15-pentaoxo-3-oxa-5,8,11,14-tetraazahexadecan-16-yl)-6-(2,5-dioxo-2,5-dihydro-1*H*-pyrrol-1-yl)hexanamide as starting materials, the preparation was carried out by applying the method of step B. The PBS buffer with the exemplary product **Reference 3** of the conjugate mixture **FADC-Reference 3** was obtained and stored at 4 °C, which was Example 4-134.

The average drug loading value was calculated by mass spectrometry: y=7.59.

Example 4-135: ADC-132

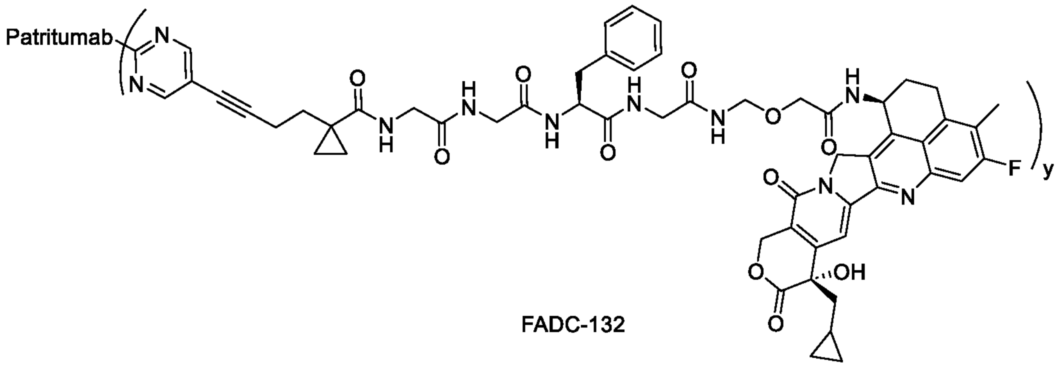

FADC-132

Using the antibody Patritumab and **LD-97** as starting materials, the preparation was carried out by applying the method of step C. The PBS buffer with the exemplary product **ADC-132** of the conjugate mixture **FADC-132** was obtained and stored at 4 °C, which was Example 4-135.

The average drug loading value was calculated by mass spectrometry: y=7.73.

Example 4-136: ADC-133

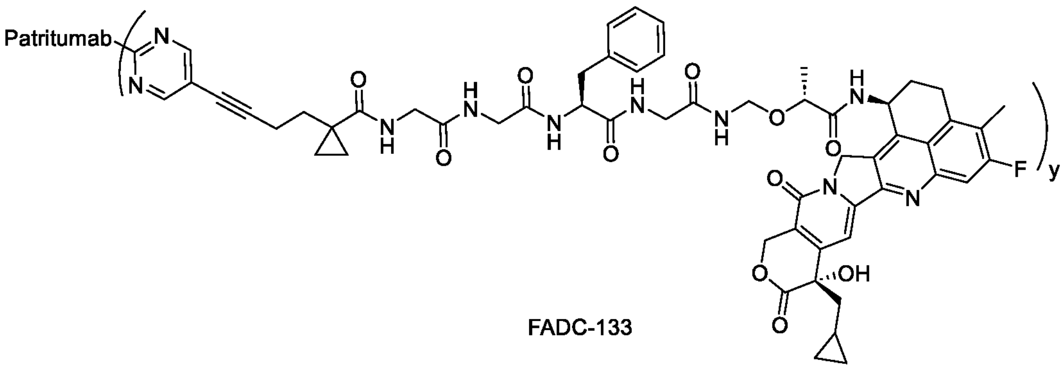

FADC-133

Using the antibody Patritumab and **LD-99** were used as starting materials, the preparation was carried out by applying the method of step C. The PBS buffer with the exemplary product **ADC-133** of the conjugate mixture **FADC-133** was obtained and stored at 4 °C, which was Example 4-136.

The average drug loading value was calculated by mass spectrometry: y=7.72.

Example 4-137: ADC-134

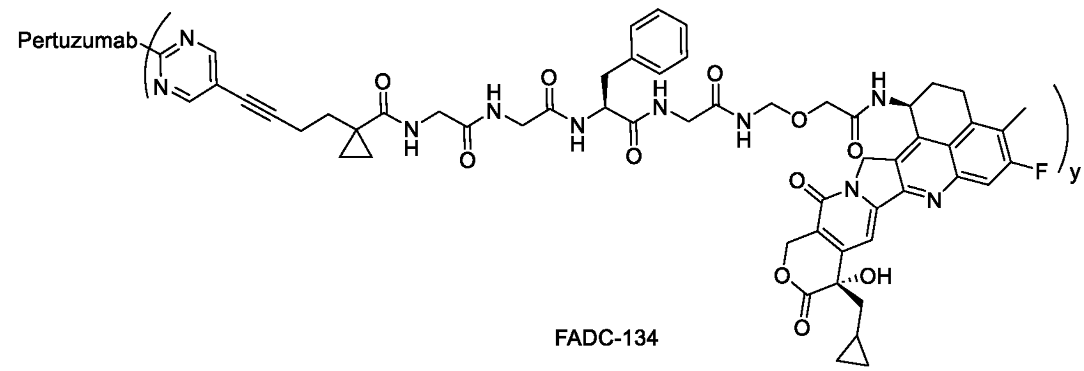

FADC-134

Using the antibody Pertuzumab and LD-97 as starting materials, the preparation was carried out by applying the method of step C. The PBS buffer with the exemplary product **ADC-134** of the conjugate mixture **FADC-134** was obtained and stored at 4 °C, which was Example 4-137.

The average drug loading value was calculated by mass spectrometry: y=7.80.

Example 4-138: ADC-135

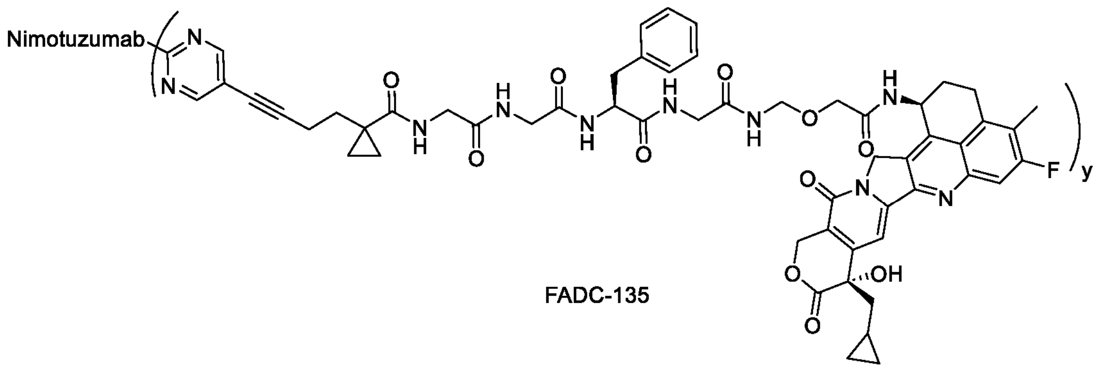

FADC-135

Using the antibody Nimotuzumab and **LD-97** as starting materials, the preparation was carried out by applying the method of step C. The PBS buffer with the exemplary product **ADC-135** of the conjugate mixture **FADC-135** was obtained and stored at 4 °C, which was Example 4-138.

The average drug loading value was calculated by mass spectrometry: y=7.02.

Example 4-139: ADC-136

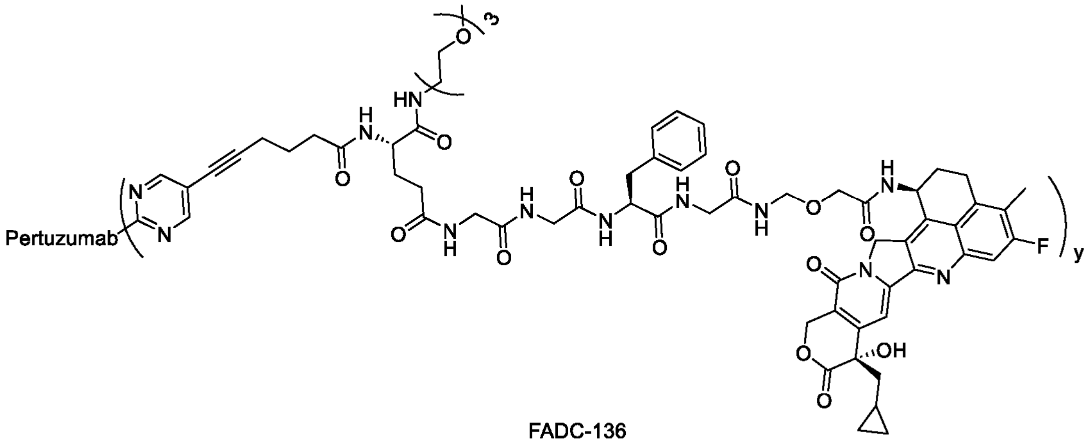

FADC-136

Using the antibody Pertuzumab and **LD-112** as starting materials, the preparation was carried out by applying the method of step C. The PBS buffer with the exemplary product **ADC-136** of the conjugate mixture **FADC-136** was obtained and stored at 4 °C, which was Example 4-139.

The average drug loading value was calculated by mass spectrometry: y=7.90.

Example 4-140: ADC-137

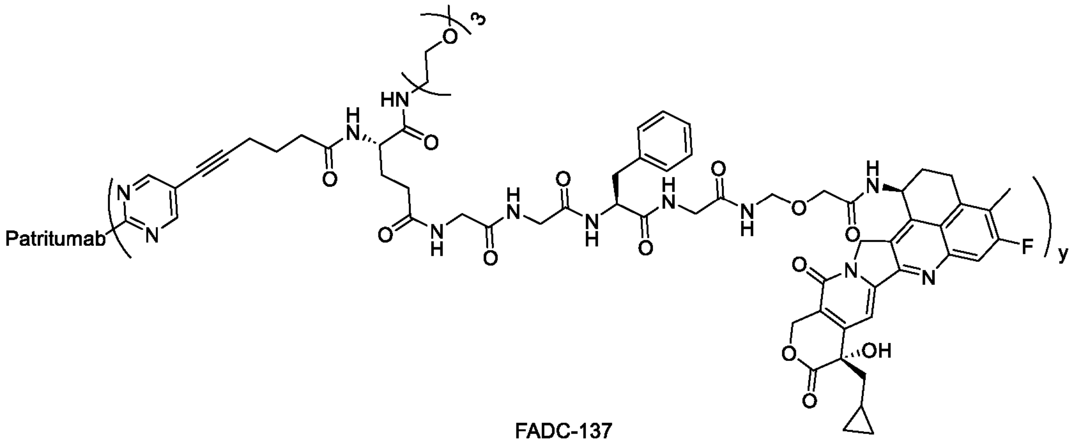

FADC-137

Using the antibody Patritumab and **LD-112** as starting materials, the preparation was carried out by applying the method of step C. The PBS buffer with the exemplary product **ADC-137** of the conjugate mixture **FADC-137** was obtained and stored at 4 °C, which was Example 4-140.

The average drug loading value was calculated by mass spectrometry: y=7.67.

Example 4-141: ADC-138

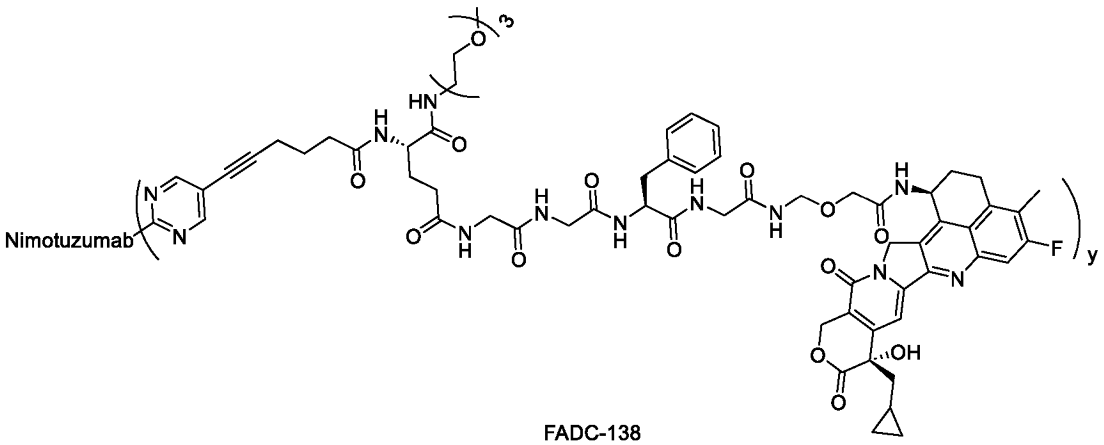

FADC-138

Using the antibody Nimotuzumab and **LD-112** as starting materials, the preparation was carried out by applying the method of step C. The PBS buffer with the exemplary product **ADC-138** of the conjugate mixture **FADC-138** was obtained and stored at 4 °C, which was Example 4-141.

The average drug loading value was calculated by mass spectrometry: y=6.84.

Example 4-142: ADC-139

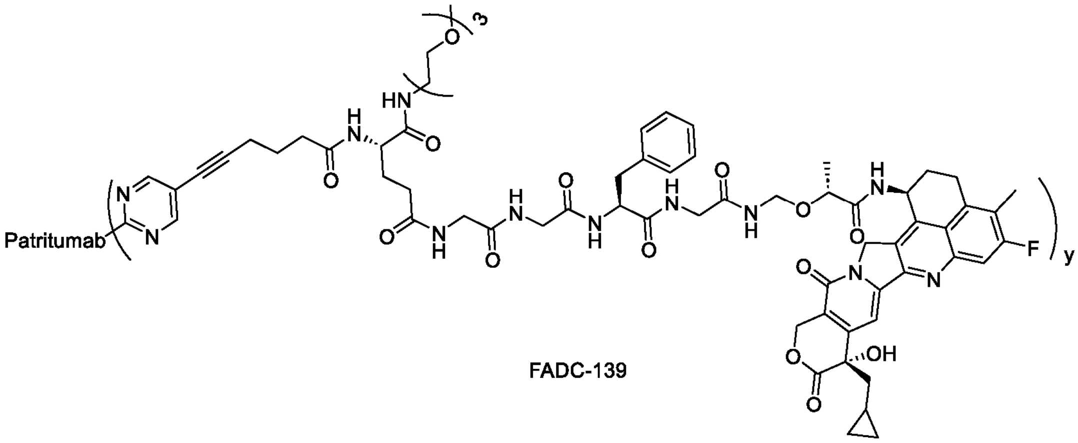

FADC-139

Using the antibody Nimotuzumab and **LD-114** as starting materials, the preparation was carried out by applying the method of step C. The PBS buffer with the exemplary product **ADC-139** of the conjugate mixture **FADC-139** was obtained and stored at 4 °C, which was Example 4-142.

The average drug loading value was calculated by mass spectrometry: y=7.73.

Analysis of drug loading of ADC stock solution

Experimental objective and principle

ADC stock solution is an antibody conjugate drug, and its mechanism in treating diseases relies on the targeting property of the antibody to deliver the toxin molecule into a cell, thereby killing the cell. Drug loading plays a decisive role in drug efficacy. The drug loading of ADC stock solution was determined by mass spectrometry and reversed-phase high-performance liquid chromatography.

Method 1: Reversed-phase high-performance liquid chromatography:

(1) Experimental method: The test sample was diluted with ultrapure water to 0.5 mg/mL, reduced with dithiothreitol having a final concentration of 25 mM at 37 °C for 30 min, and then directly subjected to injection, with an injection volume of 5 μL. The chromatographic column was a reversed-phase column (Agilent PLRP-S 1000Å (5 μm) 50x2.1 mm), and gradient elution was performed with mobile phases A and B (A: 0.1% formic acid-0.025% trifluoroacetic

acid-water, B: 0.1% formic acid-0.025% trifluoroacetic acid-acetonitrile). The flow rate was 0.25 mL/min, the detection wavelength was 280 nm, and the column temperature was 70 °C. (2) Data analysis: The positions of the light and heavy chains were distinguished by comparing the spectra of the sample with those of the naked antibody, and then the spectra of the test sample were integrated to calculate the DAR value. The calculation Formula was as follows: LC: 0 (number of conjugated drugs), LC+1: 2 (number of conjugated drugs), HC: 0 (number of conjugated drugs), HC+1: 2 (number of conjugated drugs), HC+2: 4 (number of conjugated drugs), HC+3: 6 (number of conjugated drugs). LC peak area sum = LC peak area + LC+1 peak area; HC peak area sum = HC peak area + HC+1 peak area + HC+2 peak area + HC+3 peak area; LC DAR = Σ (number of conjugated drugs x peak area percentage) / LC peak area sum; HC DAR = Σ (number of conjugated drugs x peak area percentage) / HC peak area sum; DAR = LC DAR + HC DAR.

Method 2: Mass spectrometry: (1) Experimental method: 10 μL of the test sample with a concentration of 1 mg/mL was taken and transferred into a 1.5 mL centrifuge tube, added with 0.5 μL of Rapid PNGase F (Adamas), vortexed and mixed well, and incubated at 37 °C for 60 min. After the N-glycan was cleaved, 6 μL of ultrapure water and 4 μL of 0.5 M dithiothreitol aqueous solution were added to the centrifuge tube, vortexed and mixed well, and incubated at 37 °C for 30 min. After the reaction was completed, centrifugation was performed and the supernatant was taken and transferred into an injection bottle, and injection volume was 2 μL. The column was a reverse phase chromatography column (Agilent PLRP-S 1000Å (5 μm) 50x2.1 mm), the gradient elution was performed using mobile phases A and B (A: 0.1% formic acid-water, B: 0.1% formic acid-acetonitrile), the flow rate was 0.5 mL/min, the column temperature was 60 °C, the detection wavelength was 280 nm, ESI-Tof (LC-MS) was used to acquire m/z data, the mass spectrometry acquisition mode was positive ion mode, the range for acquisition of m/z was 200-3200, and then the original mass spectrum was deconvoluted by software. (2) Data analysis: The DAR value was calculated using the peak height value by a method similar to the above-mentioned reverse phase high performance liquid chromatography method.

III. Test Examples

Biological Evaluation

Test Example 1: Determination of *in vitro* proliferation inhibitory activity of the compound of Formula (GH) on SK-BR-3 tumor cells

1. Test objective:

The objective of this experiment is to detect the *in vitro* killing activity of the compound of Formula (GH) of the present disclosure on SK-BR-3 tumor cells (human breast cancer cells, Wuhan Procell, Catalog No. CL-0211). The cells were treated with different concentrations of the compound *in vitro.* After 3 days of culture, the number of viable cells was detected using CCK8 (Cell Counting Kit-8, Catalog No. TS547) reagents, and the *in vitro* activity of the test compound was evaluated according to the $IC_{50}$ value.

2. Experimental Method:

Taking the test method of *in vitro* killing activity on SK-BR-3 cells as an example, the following test was performed. It should be understood that this method was also applicable to, but not limited to, *in vitro* proliferation inhibitory activity tests on other tumor cells (HCC1569, JIMT-1, DIFI, etc.).

(1) Cell culture: SK-BR-3 cells were cultured in RPMI 1640 medium containing 10% FBS (Shanghai BasalMedia Technologies Co., Ltd., Catalog No. G211018).

(2) Cell preparation: SK-BR-3 cells in the logarithmic growth phase were taken, washed once with PBS (phosphate buffered saline, Shanghai BasalMedia Technologies Co., Ltd., Catalog No. E211004), added with 2 to 3 mL of trypsin (0.25% Trypsin-EDTA (1×), Shanghai BasalMedia Technologies Co., Ltd., Catalog No. A121002) to allow digestion for 2 to 3 minutes. After the cells were completely digested, 10 mL of cell culture medium containing 2% FBS was added to wash out the digested cells, centrifugation was performed at 300 g for 5 min, the supernatant was discarded, and then 10 to 20 mL of cell culture medium containing 2% FBS was added to resuspend the cells to prepare a single cell suspension.

(3) Cell plating: The SK-BR-3 single cell suspension was mixed well, the viable cell density was adjusted to $6x10^4$ cells/mL with the cell culture medium containing 2% FBS, the cell suspension after density adjustment was mixed well, and added to a 96-well cell culture plate at 100 μL/well. Only 200 μL of PBS was added to the peripheral wells of the 96-well plate. The culture plate was incubated in an incubator for 24 hours (37 °C, 5% $CO_2$).

(4) Compound preparation: The compound was dissolved in DMSO (dimethyl sulfoxide, SIGMA) to prepare a 2 mM stock solution.

The starting concentrations of small molecule compounds and Dxd were both 600 nM, and the preparation method was as follows.

Dxd (MedChemExpress, Catalog No. HY-13631D, CAS: 1599440-33-1) and 2 mM small molecule compound sample stock solution were diluted to 1200 nM with the cell culture medium containing 2% FBS, and then sterilized by filtration with a 0.22 μM filter. Then, 315 μL of different samples to be tested were added to the first column of a 96-well U-bottomed drug distribution plate, and the sample concentration was 1200 nM; 210 μL of the cell culture medium containing 2% FBS was added to each well of the second to tenth columns. 105 μL of the sample was taken from the first column and added to the 210 μL cell culture medium in the second column and mixed well, and 105 μL was taken and added to the third column, and so on to the tenth column.

(5) Sample loading operation: 100 μL of the sample to be tested of different concentrations was added to the culture plate, and two replicate wells were set for each sample. The culture plate was incubated in an incubator for 3 days (37 °C, 5% $CO_2$).

(6) Color development operation: The 96-well cell culture plate was taken out, the cell culture supernatant was removed by tapping, 100 μL of the RPMI 1640 basal medium (without FBS) containing 10% CCK8 was added, and then it was incubated in an incubator for about 1.5 hours (37 °C, 5% $CO_2$).

(7) Plate reading operation: The 96-well cell culture plate was taken out, placed in an ELISA reader (MD SpectraMax i3X), and $A_{450}$ was determined by the ELISA reader.

(8) Data analysis: Microsoft Excel and Graphpad Prism 5 were used to process and analyze the data.

The results of the above tests were summarized in Table 1:

Table 1: $IC_{50}$ values of the compounds to be tested for inhibiting proliferation of SK-BR-3 cells *in vitro.*

| Compound No. | $IC_{50}$ (nM) |
|---|---|
| Single-configuration Compound **1-B** of Compound **1** in Example 2-1 | 1.94 |
| Single-configuration Compound **1-A** of Compound **1** in Example 2-1 | 0.93 |
| Single-configuration Compound **2-A** of Compound **2** in Example 2-2 | 5.88 |
| Single-configuration Compound **2-B** of Compound **2** in Example 2-2 | 12.00 |
| Single-configuration Compound **3-A** of Compound **3** in Example 2-3 | 5.91 |
| Single-configuration Compound **3-B** of Compound **3** in Example 2-3 | 2.36 |
| Single-configuration Compound **4-A** of Compound **4** in Example 2-4 | 6.42 |
| Single-configuration Compound **4-B** of Compound **4** in Example 2-4 | 5.01 |
| Single-configuration Compound **5-A** of Compound **5** in Example 2-5 | 9.27 |
| Single-configuration Compound **5-B** of Compound **5** in Example 2-5 | 4.92 |
| Compound **24** in Example 2-24 | 5.79 |
| Compound **25** in Example 2-25 | 5.41 |
| Compound **26** in Example 2-26 | 8.39 |
| Compound **27** in Example 2-27 | 13.90 |
| Compound **28** in Example 2-28 | 15.07 |

Conclusion: The small molecule compounds disclosed in the present invention had significant proliferation inhibitory activity on SK-BR-3 cells.

Test Example 2: Determination of *in vitro* proliferation inhibitory activity of the compound of Formula (GH) on NCI-N87 tumor cells

1. Test objective:

The objective of this experiment is to detect the *in vitro* killing activity of the compound of Formula (GH) disclosed in the present invention on NCI-N87 tumor cells (human gastric cancer cells, Cell Resource Center, Institute of Basic Medicine, Chinese Academy of Medical Sciences, Catalog No. 3111C0001CCC000481). The cells were treated with different concentrations of the compound *in vitro,* and after 6 days of culture, the number of viable cells was detected using CCK8 (Cell Counting Kit-8, Catalog No. TS547) reagents, and the *in vitro* activity of the test compound was evaluated according to the $IC_{50}$ value.

2. Experimental methods:

(1) Cell culture: NCI-N87 cells were cultured in the RPMI 1640 medium containing 2% FBS (Shanghai BasalMedia Technologies Co., Ltd., Catalog No. L210KJ).

(2) Cell preparation: NCI-N87 cells in the logarithmic growth phase were taken, washed once with PBS (phosphate buffered saline, Shanghai BasalMedia Technologies Co., Ltd., Catalog No. E211004), added with 1 mL of trypsin (0.25% Trypsin-EDTA (1×), Shanghai BasalMedia Technologies Co., Ltd., Catalog No. A121002) to allow digestion for 2 to 3 minutes. After the cells were completely digested, 10 mL of the cell culture medium was added to wash off and obtain the digested cells, centrifugation was performed at 300 times gravitational acceleration (g) for 5 min, the supernatant was discarded, then 10 mL of PBS was added to wash the cells once, centrifugation was performed at 300 times gravitational acceleration (g) for 5 minutes, the supernatant was discarded, and 10 mL of the cell culture medium containing 2% FBS was added to resuspend the cells to prepare a single cell suspension.

(3) Cell plating: The NCI-N87 single cell suspension was mixed, the density of live cells was adjusted to $3x10^4$ cells/mL with 2% FBS cell culture medium, the cell suspension after density adjustment was mixed well, and added to a 96-well cell culture plate at 100 μL/well. Only 200 μL of PBS was added to each of the peripheral wells of the 96-well plate. The plate was incubated in an incubator for 24 hours (37 °C, 5% $CO_2$).

(4) Preparation of sample to be tested:

The sample to be tested was diluted to 3200 nM with cell culture medium containing 2% FBS, then 300 μL of the sample was added to the first well of a 96-well U-bottomed plate with a sample concentration of 3200 nM; and 210 μL of cell culture medium containing 2% FBS was added to each well of the second to tenth columns. 70 μL of the sample was taken from the first column and added to the 210 μL cell culture medium in the second column and mixed well, 70 μL was taken and added to the third column, and so on to the ninth column.

(5) Sample loading operation: 100 μL of the sample to be tested with different concentrations was added to the culture plate, two replicate wells were set for each sample. The culture plate was incubated in an incubator for 6 days (37 °C, 5% $CO_2$).

(6) Color development operation: The 96-well cell culture plate was taken out, the supernatant of the culture medium was removed by tapping, 100 μL of 10% CCK8 (prepared with RPMI 1640 basal culture medium, without FBS) solution was added to each well, and incubation was performed in an incubator for 2 hours.

(7) Plate reading operation: The 96-well cell culture plate was taken out, placed in an ELISA reader (SpectraMax i3X), and $A_{450}$ was determined by the ELISA reader.

(8) Data analysis: Microsoft Excel and Graphpad Prism 5 were used to process and analyze the data.

The results of the above tests were summarized in Table 2:

Table 2: $IC_{50}$ values of the compounds to be tested for inhibiting proliferation of NCI-N87 cells *in vitro.*

| Compound No. | $IC_{50}$ (nM) |
|---|---|
| Single-configuration Compound **1-B** of Compound **1** in Example 2-1 | 5.36 |
| Single-configuration Compound **2-A** of Compound **1** in Example 2-2 | 1.92 |
| Single-configuration Compound **3-A** of Compound **3** in Example 2-3 | 5.28 |
| Single-configuration Compound **4-A** of Compound **4** in Example 2-4 | 2.40 |
| Single-configuration Compound **5-B** of Compound **5** in Example 2-5 | 2.96 |
| Compound **25** in Example 2-25 | 5.46 |
| Dxd | 10.16 |

Conclusion: The small molecule compounds disclosed in the present disclosure had significant proliferation inhibitory activity on NCI-N87 cells, and the activity was stronger than that of Dxd.

Test Example 3: *In vitro* proliferation inhibition test of the antibody-drug conjugates of the present disclosure targeting HER2 tumor cells

1. Test objective:

The objective of this experiment is to detect the inhibitory activity of the HER2-targeting antibody-drug conjugates of the present disclosure on the *in vitro* proliferation of SK-BR-3 cells (human breast cancer cells, Wuhan Procell, Catalog No. CL-0211), NCI-N87 cells (human gastric cancer cells, Cell Resource Center, Institute of Basic Medicine, Chinese Academy of Medical Sciences, Catalog No. 3111C0001CCC000481) and MDA-MB-468 cells (human breast cancer cells, Cell Center, Shanghai Institutes for Biological Sciences, Chinese Academy of Sciences, Catalog No. 3131C0001000700120). The cells were treated with the compounds having different concentrations *in vitro,* and after 6 days of culture, the proliferation of the cells was detected using the CCK8 (Cell Counting Kit-8, Catalog No. TS547) reagents, and the *in vitro* activity was evaluated according to the $IC_{50}$ values.

2. Experimental methods:

Taking the test method of *in vitro* proliferation activity of SK-BR-3 cells, NCI-N87 cells and MDA-MB-468 cells as an example, the following test was performed. It should be understood that this method is also applicable to, but not limited to, tests of *in vitro* proliferation inhibition activity of other tumor cells (HCC1569, JIMT-1, DIFI, etc.).

(1) Cell culture: SK-BR-3 cells, NCI-N87 cells and MDA-MB-468 cells were cultured with RPMI 1640 medium containing 2% FBS (Shanghai BasalMedia Technologies Co., Ltd., Catalog No. L210KJ).

(2) Cell preparation: The cells in the logarithmic growth phase were taken, washed once with PBS (phosphate buffered saline, Shanghai BasalMedia Technologies Co., Ltd., Catalog No. E211004), then added with 1 mL of trypsin (0.25% Trypsin-EDTA (1×), Shanghai BasalMedia Technologies Co., Ltd., Catalog No. A121002) to allow digestion for 2 to 3 minutes. After the cells were completely digested, 10 mL of cell culture medium was added to wash off the digested cells, centrifugation was performed at 300 times gravitational acceleration (g) for 5 min, the supernatant was discarded, and then 10 mL of cell culture medium was added to resuspend the cells to prepare a single cell suspension.

(3) Cell plating: The single cell suspension was mixed well, the viable cell density was adjusted to $3x10^4$ cells/mL with cell culture medium, the cell suspension was mixed well after density adjustment, and added to a 96-well cell culture plate at 100 μL/well. Only 200 μL of PBS was added to each of the peripheral wells of the 96-well plate. The culture plate was incubated in an incubator for 24 hours (37°C, 5% $CO_2$).

(4) Preparation of sample to be tested:

The sample to be tested was diluted to 400 nM with cell culture medium containing 2% FBS, then 300 μL of the sample was added to the first well of a 96-well U-bottomed plate with a sample concentration of 400 nM; 210 μL of cell culture medium containing 2% FBS was added to each of the wells of the second to tenth columns. 70 μL of the sample was

taken from the first column and added to the 210 μL cell culture medium in the second column and mixed well, 70 μL was taken and added to the third column, and so on to the ninth column.

(5) Sample loading operation: 100 μL of the test sample of different concentrations was added to the culture plate, two replicate wells were set for each sample. The culture plate was incubation in an incubator for 6 days (37°C, 5% $CO_2$).

(6) Color development operation: The 96-well cell culture plate was taken out, the supernatant of the culture medium was removed by tapping, 100 μL of 10% CCK8 (prepared with RPMI 1640 basal culture medium, without FBS) solution was added to each well, and incubated at room temperature for 2 hours.

(7) Plate reading operation: The 96-well cell culture plate was taken out, placed in an ELISA reader (SpectraMax i3X), and $A_{450}$ was determined by the ELISA reader.

(8) Data analysis: Microsoft Excel and Graphpad Prism 5 were used to process and analyze the data.

The results of the above tests were summarized in Table 3:

Table 3: $IC_{50}$ values of *in vitro* proliferation inhibition of HER2-targeted tumor cells by the antibody-drug conjugates of the present disclosure.

| Compound No. | $IC_{50}$ (nM) | | |
|---|---|---|---|
| | SK-BR-3 | NCI-N87 | MDA-MB-468 |
| Example 4-13 ADC-13 | 0.06 | 0.13 | >50 |
| Example 4-98 ADC-98 | 0.09 | 0.10 | >50 |
| Example 4-99 ADC-99 | 0.10 | 0.19 | >50 |
| Example 4-100 ADC-100 | 0.12 | 0.23 | >50 |
| Example 4-104 ADC-104 | 0.11 | 0.15 | >50 |
| Example 4-105 ADC-105 | 0.11 | 0.14 | >50 |
| Example 4-106 ADC-106 | 0.13 | 0.16 | >50 |
| Example 4-107 ADC-107 | 0.09 | 0.16 | >50 |
| Example 4-108 ADC-108 | 0.11 | 0.16 | >50 |
| Example 4-109 ADC-109 | 0.08 | 0.15 | >50 |
| Example 4-110 ADC-110 | 0.09 | 0.15 | >50 |
| Example 4-111 ADC-111 | 0.08 | 0.17 | >50 |
| Example 4-112 ADC-112 | 0.10 | 0.23 | >50 |
| Example 4-113 ADC-113 | 0.08 | 0.20 | >50 |
| Example 4-114 ADC-114 | 0.08 | 0.15 | >50 |
| Example 4-115 ADC-115 | 0.11 | 0.19 | >50 |
| Example 4-116 ADC-116 | 0.08 | 0.16 | >50 |
| Example 4-117 ADC-117 | 0.08 | 0.18 | >50 |
| Example 4-120 ADC-118 | 0.11 | N.D. | >50 |
| Example 4-121 ADC-119 | 0.34 | 0.34 | >50 |
| Example 4-122 ADC-120 | 0.07 | N.D. | >50 |
| Example 4-123 ADC-121 | 0.08 | 0.31 | >50 |
| Example 4-124 ADC-122 | 0.12 | 0.16 | >50 |
| Example 4-125 ADC-123 | 0.08 | N.D. | >50 |

(continued)

| Compound No. | $IC_{50}$ (nM) | | |
|---|---|---|---|
| | SK-BR-3 | NCI-N87 | MDA-MB-468 |
| Example 4-126 ADC-124 | 0.12 | 0.19 | >50 |
| N.D.: represents not detected. | | | |

Conclusion: The HER2-targeting antibody-drug conjugates of the present disclosure had significant proliferation inhibitory activity on HER2-positive SK-BR-3 and NCI-N87 cells, meanwhile, they had weak proliferation inhibitory activity on HER2-negative MDA-MB-468 cells, indicating good selectivity.

Test Example 4: Test of bystander killing effect of the antibody-drug conjugates of the present disclosure

HER2-positive SK-BR-3 cells (human breast cancer cells, Wuhan Procell, Catalog No. CL-0211) and HER2-negative MDA-MB-468 cells (human breast cancer cells, Cell Center of Shanghai Institutes for Biological Sciences, Chinese Academy of Sciences, Catalog No. 3131C0001000700120) were adjusted to $1.2x10^5$ cells/ml and $4x10^4$ cells/ml with RPMI-1640+10% FBS, respectively, 1 mL of SK-BR-3 and MDA-MB-468 cells each was taken and added to a 6-well plate, mixed and cultured overnight at 37°C and 5% $CO_2$. The ADC sample was prepared to a concentration of 4.5 μg/mL (30 nM), and 1 mL was added to the cells to make the total volume 3mL and the final concentration 10 nM. A solvent control group was set up, and culturing was performed at 37°C and 5% $CO_2$ for 5 days. Trypsin (0.25% Trypsin-EDTA (1×), Shanghai BasalMedia Technologies Co., Ltd., Catalog No.: A121002) was added to the cells in the 6-well plate for digestion, 1 mL of FACS buffer (PBS + 4% FBS) was added for resuspension, the cells were taken and stained with trypan blue, and counted. The remaining cells were centrifuged. Trastuzumab was diluted to 10 μg/mL with FACS buffer. The cells were resuspended with 200 μL of antibody solution, incubated on ice for 30 minutes, Centrifugation was performed and the supernatant was discarded, washing was performed once with 1 mL of FACS buffer, 200 μL of AF647-labeled sheep anti-human Fc antibody (Jackson, Catalog No.: 109-606-170) solution was added and incubated on ice for 15 minutes, 5 μg/mL PI (Sigma, Catalog No.: 31845) solution was added and incubated on ice for 5 minutes, centrifugation was performed and the supernatant was discarded, washing was performed once with 1 mL FACS buffer, the cells were resuspended with 400 μL of PBS, and analyzed by BD Celesta flow cytometer. The ratio and number of cells of the two types were determined according to the flow cytometry and counting results.

The results of the above tests were summarized in Figure 1, which showed that the antibody-drug conjugates of the present disclosure had excellent bystander killing effects.

Test Example 5: Evaluation of efficacy of antibody-drug conjugates in NCI-N87 transplanted tumor mice

Test 5A:

1. Test objective:
The objective of this experiment is to detect the efficacy of HER2 antibody ADC drugs on nude mice with human gastric cancer cell NCI-N87 transplanted tumors.
2. Test drugs and materials:

    (1) Test drug: antibody-drug conjugate: 2 mg/kg.

    (2) Experimental animals: Balb/c-nude female mice, 6 to 8 weeks old, weighing 20 to 22g (Gempharmatech), raised in a sterile animal room at 25 °C with a 12-hour day and night cycle, and were allowed to access food and water freely.

3. Experimental methods:

(1) NCI-N87 subcutaneous transplanted tumor model in mice

Inoculation method: NCI-N87 cells in the logarithmic growth period (Cell Resource Center, Institute of Basic Medicine, Chinese Academy of Medical Sciences, 3111C0001CCC000481) were taken, centrifuged at 300 times gravitational acceleration (g) for 7 minutes, washed twice with pre-cooled PBS and counted. The cell density was adjusted to $6e^7$/mL, a total of 10 mL, mixed thoroughly in PBS, dispense into sterile 1.5 mL EP tubes, and placed on ice for

later use. 1 mL sterile syringe was used to subcutaneously inoculate the tumor at a volume of 100 μL/mouse, liquid leakage should be avoided.

(2) Drug administration and tumor volume detection

When the volume of the transplanted tumor reached 100 to 300 $mm^3$ (on the 8th day after tumor inoculation), the tumor-bearing mice with similar tumor volumes were selected for grouping and administration, 5 mice in each group. Drug administration was performed via tail vein, once on day 0. The tumor volume was measured by a vernier caliper every 2 to 3 days; the length (a) and width (b) of tumor were taken, and volume = $a \times b \times b/2$. A tumor growth curve was plotted (in which the tumor volume did not exceed 3000 $mm^3$, and when it reached 3000 $mm^3$, the mice were sacrificed), and the body weight changes in mice were detected at the same time.

(3) Data analysis:

All experimental results were expressed as mean ± standard error. Graphpad Prism 7.0 software was used to perform statistical analysis on the tumor volume of each group. For tumor volume, one-way ANOVA and Dunnett's test were used for statistical analysis to evaluate the differences between groups for the vehicle control group and all treatment groups. $P < 0.05$ was considered to be significant different.
*P < 0.05, **P < 0.01, ***P < 0.001, ****P < 0.0001.

T/C (%) = $(T-T_0) / (C-C_0) \times 100$, wherein T and C were the tumor volumes of the experimental group and the control group at the end of the experiment, respectively; $T_0$ and $C_0$ were the tumor volumes at the beginning of the experiment.

$$\text{Tumor growth inhibition rate (TGI) (\%)} = 100\text{- T/C (\%)}.$$

When the tumor regressed, the tumor growth inhibition rate (TGI) (%) = $100-(T-T_0)/T_0 \times 100$. If the tumor was smaller than the starting volume, that was, $T<T_0$ or $C<C_0$, it was defined as partial tumor regression (PR); if the tumor disappeared completely, it was defined as complete tumor regression (CR).

The results of the average tumor volume change, relative tumor growth rate and tumor growth inhibition rate of the above tests were summarized in Figure 2 and Table 4, which indicated that the antibody-drug conjugates to be tested could effectively inhibit the growth of NCI-N87 transplanted tumors in nude mice, among which **ADC-13, ADC-98, ADC-99,** and **ADC-100** had better tumor inhibition effects on the same batch of tumor cells at the same dose than **Reference 1.**

Table 4:

| Group | Average tumor volume ($mm^3$) | | Relative tumor proliferation rate, T/C% | Tumor growth inhibition rate, TGI% |
|---|---|---|---|---|
| | Day 0 | Day 20 | | |
| Vehicle | 240.50±20.91 | 984.70±215.50 | - | - |
| Reference 1 | 209.40±19.42 | 407.20±77.75 | 26.58 | 73.42 |
| ADC-13 | 222.50±15.01 | 255.80±83.05 | 4.49 | 95.51 |
| ADC-98 | 225.60±18.27 | 253.80±48.09 | 3.79 | 96.21 |
| ADC-99 | 220.90±14.03 | 229.10±39.84 | 1.09 | 98.91 |
| ADC-100 | 223.40±15.83 | 245.30±31.25 | 2.94 | 97.06 |

The body weight changes of experimental mice were summarized in Figure 3. The body weight of animals of each group was normal during the drug administration process, indicating that the mice had good tolerance to the antibody-drug conjugates to be tested.

Test 5B:

The same experimental method as the above Test 5A was used, except that the specific batches of NCI-N87 cells in the logarithmic growth phase (Cell Resource Center, Institute of Basic Medicine, Chinese Academy of Medical Sciences, 3111C0001CCC000481) used were different. When the volume of the transplanted tumor reached 100 to 300 $mm^3$ (on the 4th day after tumor inoculation), the tumor-bearing mice with similar tumor volumes were selected for

grouping and administration, and the efficacy evaluation of the antibody-drug conjugates in NCI-N87 transplanted tumor mice was performed.

The results of the average tumor volume change, relative tumor growth rate and tumor growth inhibition rate were summarized in Figure 4 and Table 5, indicating that the tested antibody-drug conjugate **ADC-114** could effectively inhibit the growth of NCI-N87 transplanted tumors in nude mice, and its tumor inhibition effect on the same batch of tumor cells at the same dose was better than that of **Reference 1.**

Table 5:

| Group | Average tumor volume ($mm^3$) | | Relative tumor proliferation rate, T/C% | Tumor growth inhibition rate, TGI% |
|---|---|---|---|---|
| | Day 0 | Day 21 | | |
| Vehicle | 228.00±14.78 | 748.60±66.28 | - | - |
| Reference 1 | 222.90±15.10 | 169.50±72.40 | -8.09 | 108.09 |
| ADC-114 | 222.60±12.40 | 39.54±8.85 | -37.13 | 137.13 |

The body weight changes of the experimental mice in the above tests were summarized in Figure 5. The results showed that the weight of animals in each group was normal during the administration process, indicating that the mice had good tolerance to the antibody-drug conjugates to be tested.

Test 5C:

The same experimental method as the above Test 5A was used; the difference lied in that the dosage of the antibody-drug conjugates in this test was 6 mg/kg, and the specific batches of the used NCI-N87 cells (Cell Resource Center, Institute of Basic Medicine, Chinese Academy of Medical Sciences, 3111C0001CCC000481) in the logarithmic growth phase were different. When the volume of the transplanted tumor reached 100 to 300 $mm^3$ (on the $4^{th}$ day after tumor inoculation), the tumor-bearing mice with similar tumor volumes were selected for grouping and administration, and the efficacy evaluation of the antibody-drug conjugates in NCI-N87 transplanted tumor mice was performed.

The results of average tumor volume change, relative tumor growth rate and tumor growth inhibition rate of the test were summarized in Figure 7 and Table 6, indicating that the tested antibody-drug conjugates could effectively inhibit the growth of NCI-N87 transplanted tumors in nude mice, and at the same dose, **ADC-120** and **ADC-123** had better tumor inhibition effects on the same batch of tumor cells than **Reference 2.**

Table 6:

| Group | Average tumor volume ($mm^3$) | | Relative tumor proliferation rate, T/C% | Tumor growth inhibition rate, TGI% |
|---|---|---|---|---|
| | Day 0 | Day 20 | | |
| Vehicle | 175.03±13.53 | 676.06±53.36 | - | - |
| Pertuzumab | 174.15±20.53 | 687.90±123.83 | 102.53 | -2.53 |
| Reference 2 | 174.80±19.36 | 137.51±46.60 | -7.44 | 121.33 |
| ADC-120 | 172.19±21.28 | 96.63±26.51 | -15.07 | 143.87 |
| ADC-123 | 174.26±16.56 | 49.33±15.50 | -24.93 | 171.68 |

The weight changes of the experimental mice in the above test are summarized in Figure 8. The results show that the weight of each group of animals was normal during the administration process, indicating that the mice had good tolerance to the tested antibody-drug conjugates.

Test Example 6: Determination of plasma stability of antibody-drug conjugates

I. Test objective:

After incubation for a certain period of time, the stability of ADC in the following plasma of four different species was determined: human plasma (collected from volunteers), monkey plasma (Beijing Iphasebio, 032B11.21), mouse plasma (Beijing Iphasebio, 032E13.22), and rat plasma (Beijing Iphasebio, 032D11.11). The ADC samples were diluted to the same concentration with plasma from different species, placed in an incubator at 37 °C for a certain period of time, and

then taken out. The free toxin was detected by mass spectrometry (Thermo, TSQ Altis), and the plasma stability of the ADC was evaluated based on the dissociation degree value.

II. Experimental method:

1. Sample dilution: The ADC sample was diluted to 200 μg/mL with plasma from different species, with a volume of 2 mL.

2. Filtration sterilization: 2 mL of the ADC sample solution was filtered using 0.22 μm Syringe Driven Filters (Biofil, 220509-051-A) (ensuring that the volume after filtration was >1.8 mL).

3. Sample aliquoting: The filtered ADC sample solution was aliquoted into 1.5 mL EP tubes (Axygen, MCT-150-C-S), 300 μL/tube, labeled and sealed with sealing film.

4. Sample incubation: The aliquoted samples were placed in an incubator at 37 °C for 0, 1, 3, 7, 14, and 21 days, and the sample on Day 0 was directly stored at -80 °C.

5. Sample delivery and testing: After incubation, the samples were sent for mass spectrometry, and free toxins were detected by a mass spectrometer.

Data analysis: The data were processed and analyzed using Microsoft Excel and Graphpad Prism 5. The results of the above tests were summarized in Figure 6.

The experimental results showed that the free toxins of the antibody-drug conjugates decreased to a lower level in the plasma of humans, monkeys, mice and rats. On the Day 21, the release rates of free toxins of **ADC-98, ADC-99** and **ADC-100** did not exceed 2%.

Test Example 7: Test on bystander killing effect of pertuzumab antibody-drug conjugate of the present disclosure

HER2-positive SK-BR-3 cells (human breast cancer cells, Wuhan Procell, Catalog No. CL-0211) and HER2-negative MDA-MB-468 cells (human breast cancer cells, Cell Center of Shanghai Institutes for Biological Sciences, Chinese Academy of Sciences, Catalog No. 3131C0001000700120) were adjusted to $1.2\times10^5$ cells/ml and $4\times10^4$ cells/ml with RPMI-1640+10% FBS, respectively, 1 mL of SK-BR-3 and MDA-MB-468 cells each was added to a 6-well plate, mixed and cultured at 37 °C and 5% $CO_2$ overnight. The ADC sample was prepared to a concentration of 4.5 μg/mL (30 nM), and 1 mL was added to the cells to make the total volume 3mL and the final concentration 10 nM. A solvent control group was set up and cultured at 37 °C and 5% $CO_2$ for 5 days. The cells were digested by adding trypsin (0.25% Trypsin-EDTA (1×), Shanghai BasalMedia Technologies Co., Ltd., Catalog No. A121002) to the 6-well plate, and resuspended in 1 mL of FACS buffer (PBS+4% FBS), and the cells were stained with trypan blue and counted. The remaining cells were centrifuged, Pertuzumab was diluted to 10 μg/mL with FACS buffer, the cells were resuspended with 200 μL of antibody solution and incubated on ice for 30 minutes, centrifugation was performed and the supernatant was discarded, washing was performed once with 1 mL of FACS buffer, 200 μL of AF647-labeled sheep anti-human Fc antibody (Jackson, Catalog No.: 109-606-170) solution was added and incubated on ice for 15 minutes, 5 μg/mL PI (Sigma, Catalog No.: 31845) solution was added and incubated on ice for 5 min, centrifugation was performed and the supernatant was discarded, washing was performed once with 1 mL FACS buffer, and the cells were resuspended with 400 μL PBS, and analyzed by BD Celesta flow cytometer. The ratio and number of cells of the kinds were determined according to the flow cytometry and counting results.

The results of the above tests were summarized in Figure 9, which showed that the Pertuzumab antibody-drug conjugate of the present disclosure had excellent bystander killing effect.

Test Example 8: Test on *in vitro* proliferation inhibition of EGFR-targeted tumor cells by the antibody-drug conjugates of the present disclosure

1. Test objective:

The objective of this experiment is to detect the inhibitory activity of the EGFR-targeting antibody-drug conjugates of the present disclosure on the *in vitro* proliferation of MDA-MB-468 cells (human breast cancer cells, Cell Center, Shanghai Institutes for Biological Sciences, Chinese Academy of Sciences, Catalog No. 3131C0001000700120),

SK-BR-3 cells (human breast cancer cells, Wuhan Procell, Catalog No. CL-0211), and SW620 cells (human colon cancer cells, Biofeng, bc067). The cells were treated with compounds of different concentrations *in vitro,* and after 6 days of culture, the proliferation of cells was detected using CCK8 (Cell Counting Kit-8, Catalog No.: TS547) reagents, and the *in vitro* activity was evaluated according to the $IC_{50}$ values.

2. Experimental method:

Taking the test method of *in vitro* proliferation activity of MDA-MB-468 cells, SK-BR-3 cells and SW620 cells as an example, the following test was performed. It should be understood that this method was also applicable to, but not limited to, *in vitro* proliferation inhibition activity tests on other tumor cells (HCC1569, JIMT-1, DIFI, etc.).

(1) Cell culture: MDA-MB-468 cells and SW620 cells were cultured in L-15 medium containing 10% FBS (Gibco, Catalog No. 11415-064); SK-BR-3 cells were cultured in RPMI 1640 medium containing 10% FBS (Shanghai BasalMedia Technologies Co., Ltd., Catalog No. L210KJ).

(2) Cell preparation: The cells in the logarithmic growth phase were taken, washed once with PBS (phosphate buffered saline, Shanghai BasalMedia Technologies Co., Ltd., Catalog No. E211004), then added with 1 mL of trypsin (0.25% Trypsin-EDTA (1×), Shanghai BasalMedia Technologies Co., Ltd., Catalog No. A121002) for digestion for 2 to 3 minutes. After the cells were completely digested, 10 mL of the corresponding cell culture medium containing 10% FBS was added to wash off the digested cells, centrifugation was performed at 300 times gravitational acceleration (g) for 5 min and the supernatant was discarded, and then 10 mL of cell culture medium was added to resuspend the cells to prepare a single cell suspension.

(3) Cell plating: The single cell suspension was mixed well, the viable cell density was adjusted to $3x10^4$ cells/mL with the corresponding cell culture medium containing 2% FBS, the cell suspension after density adjustment was mixed well, and add to a 96-well cell culture plate at 100 μL/well. Only 200 μL of PBS was added to the peripheral wells of the 96-well plate. The culture plates of MDA-MB-468 cells and SW620 cells were cultured in a $CO_2$-free incubator for 24 hours (37°C), and the culture plate of SK-BR-3 cells was cultured in a 5% $CO_2$ incubator for 24 hours (37°C).

(4) Preparation of samples to be tested:

The samples to be tested were diluted to 400 nM with the cell culture medium containing 2% FBS corresponding to the cells to be added, and then 300 μL of the sample was added to the first well of a 96-well U-bottomed plate with a sample concentration of 400 nM; 210 μL of the corresponding FBS-containing cell culture medium was added to each well of the second to tenth columns. 70 μL of the sample in the first column was taken and added to the 210 μL cell culture medium in the second column and mix welled, 70 μL was taken and added to the third column, and so on to the ninth column.

(5) Sample loading operation: 100 μL of the sample to be tested with different concentrations was added to the culture plate, and two replicate wells were set for each sample. The culture plates of MDA-MB-468 cells and SW620 cells were placed in a $CO_2$-free incubator for 6 days (37°C), and the culture plate of SK-BR-3 cells were placed in a 5% $CO_2$ incubator for 6 days (37°C).

(6) Color development operation: The 96-well cell culture plate was taken out, the supernatant of the culture medium was removed by tapping, 100 μL of 10% CCK8 (prepared with RPMI 1640 basal culture medium, without FBS) solution was added to each well, and incubated at room temperature for 2 hours.

(7) Plate reading operation: The 96-well cell culture plate was taken out, placed in an ELISA reader (SpectraMax i3X), and $A_{450}$ was determined by the ELISA reader.

(8) Data analysis: Microsoft Excel and Graphpad Prism 5 were used to process and analyze the data.

The results of the above tests were summarized in Table 7:

Table 7: $IC_{50}$ values of *in vitro* proliferation inhibition of EGFR-targeted tumor cells by disclosed antibody-drug conjugates.

| Compound No. | $IC_{50}$ (nM) | | |
|---|---|---|---|
| | MDA-MB-468 | SK-BR-3 | SW620 |
| Example 4-127 ADC-125 | 0.19 | 9.53 | 22.67 |
| Example 4-128 ADC-126 | 0.21 | N.D. | >50 |
| Example 4-129 ADC-127 | 0.14 | 34.39 | >50 |
| Example 4-130 ADC-128 | 0.21 | 8.63 | 29.92 |
| Example 4-131 ADC-129 | 0.20 | N.D. | >50 |
| Example 4-132 ADC-130 | 0.19 | 44.05 | >50 |
| Example 4-133 ADC-131 | 0.23 | N.D. | >50 |
| N.D. represents not detected. | | | |

Conclusion: The EGFR-targeting antibody-drug conjugates of the present disclosure had significant proliferation inhibition activity on EGFR-positive MDA-MB-468 cells, meanwhile, they had weak proliferation inhibition activity on EGFR-negative SW620 cells, indicating good selectivity.

Test Example 9: Test of bystander killing effect of the antibody-drug conjugates of the present disclosure on EGFR-targeted tumor cells

EGFR-positive MDA-MB-468 cells (human breast cancer cells, Cell Center, Shanghai Institutes for Biological Sciences, Chinese Academy of Sciences, Catalog No. 3131C0001000700120) and EGFR-negative SW620 cells (human colon cancer cells, Biofeng, bc067) were adjusted to cell density of $1.2 \times 10^5$ cells/ml and $4 \times 10^4$ cells/ml with RPMI-1640+10% FBS, respectively, and 1 mL of MDA-MB-468 and SW620 cells each was added to 6-well plates, mixed and cultured overnight at 37°C and 5% $CO_2$. The ADC sample was prepared to a concentration of 4.5 μg/mL (30 nM), and 1 mL was taken and added to the cells to make the total volume 3 mL and the final concentration 10 nM. A solvent control group was set up and cultured at 37 °C and 5% $CO_2$ for 5 days. The cells were digested by adding trypsin (0.25% Trypsin-EDTA (1×), Shanghai BasalMedia Technologies Co., Ltd., Catalog No.: A121002) to the 6-well plate, and 1 mL of FACS buffer (PBS + 4% FBS) was added to resuspend, and the cells were stained with trypan blue and counted. The remaining cells were centrifuged, Nimotuzumab was diluted to 10 μg/mL with FACS buffer, the cells were resuspended with 200 μL of antibody solution and incubated on ice for 30 min, centrifugation was performed and the supernatant was discarded, washing was performed once with 1 mL FACS buffer, 200 μL of AF647 labeled sheep anti-human Fc antibody (Jackson, Catalog No.: 109-606-170) solution was added and incubated on ice for 15 minutes, 5 μg/mL PI (Sigma, Catalog No.: 31845) solution was added and incubated on ice for 5 minutes, centrifugation was performed and the supernatant was discarded, washing was performed once with 1 mL FACS buffer, and the cells were resuspended with 400 μL PBS and analyzed by BD Celesta flow cytometer. The ratio and number of cells of the two types were determined according to the flow cytometry and counting results.

The results of the above tests were summarized in Figure 10, which showed that the Nimotuzumab antibody-drug conjugate of the present disclosure had excellent bystander killing effect.

Test Example 10: Evaluation of efficacy of antibody-drug conjugates in JIMT-1 transplanted tumor mice

1. Test objective:
The objective of this experiment is to detect the efficacy of HER2 antibody ADC drugs on human breast cancer JIMT-1 transplanted tumors in nude mice.
2. Test drugs and materials:

(1) Test drug: antibody-drug conjugate: 3 mg/kg, 10 mg/kg.

(2) Experimental animals: Balb/c-nude female mice, 6 to 8 weeks old, weighing 20 to 22 g (Gempharmatech), raised in a sterile animal room at 25 °C with a 12-hour day and night cycle, and were allowed to access food and

water freely.

3. Experimental methods:

(1) JIMT-1 mouse subcutaneous transplanted tumor model

Inoculation method: JIMT-1 cells (DSMZ, ACC 589v) in the logarithmic growth period were taken, and centrifuged at 300 times gravitational acceleration (g) for 7 minutes. After washing twice with pre-cooled PBS, the cells were counted, adjusted to reach a cell density to 1e8/mL, mixed well with Matrigel (1:1), and aliquoted into sterile 2 mL EP tubes, and placed on ice for later use. 1 mL sterile syringe was used to subcutaneously inoculate the tumor at a volume of 100 μL/mouse, liquid leakage should be avoided.

(2) Drug administration and tumor volume detection

When the volume of the transplanted tumor reached 100 to 300 $mm^3$ (around the 4th day after tumor inoculation), tumor-bearing mice with similar tumor volumes were selected for grouping and administration, with 6 mice in each group. Drug administration was performed via tail vein, twice on day 0 and day 7. The tumor volume was measured by a vernier caliper every 2 to 3 days; the length (a) and width (b) of tumor were taken, and volume = $a \times b \times b/2$. A tumor growth curve was drown (in which the tumor volume did not exceed 3000 $mm^3$, and when it reached 3000 $mm^3$, the mice were sacrificed), and the changes in mouse body weight was detected at the same time.

(3) Data analysis:

All experimental results were expressed as mean ± standard error, and the tumor volume of each group was statistically analyzed using Graphpad Prism 7.0 software. For tumor volume, the solvent control group and all treatment groups were statistically analyzed using one-way ANOVA and Dunnett's test to evaluate the differences between groups. $P<0.05$ was considered to be significantly different.
*P<0.05, **P<0.01, ***P<0.001, ****P<0.0001.

$$T/C\ (\%) = (T-T_0) / (C-C_0) \times 100,$$

wherein T and C were the tumor volumes of the experimental group and the control group at the end of the experiment; $T_0$ and $C_0$ were the tumor volumes at the beginning of the experiment.

$$\text{Tumor growth inhibition rate (TGI) (\%)} = 100-T/C\ (\%).$$

When the tumor regressed, the tumor growth inhibition rate (TGI) (%) = $100-(T-T_0)/T_0 \times 100$. If the tumor was smaller than the starting volume, that was, $T<T_0$ or $C<C_0$, it was defined as partial tumor regression (PR); if the tumor disappeared completely, it was defined as complete tumor regression (CR).

The results of the average tumor volume change, relative tumor growth rate and tumor groth inhibition rate of the above tests were summarized in Figure 11 and Table 8, which indicated that the antibody-drug conjugates to be tested could effectively inhibit the growth of JIMT-1 transplanted tumors in nude mice, among which **ADC-13, ADC-98, ADC-99, ADC-100, ADC-105,** and **ADC-108** had better tumor inhibition effects on the same batch of tumor cells at the same dose than **Reference 1.**

Table 8:

| Group | Average tumor volume ($mm^3$) | | Relative tumor proliferation rate, T/C% | Tumor growth inhibition rate, TGI% |
|---|---|---|---|---|
| | Day 0 | Day 35 | | |
| Vehicle | 232.33 ± 15.2 | 1088.05 ± 129.9 | - | - |
| Reference 1 (3 mg/kg) | 215.14 ± 11.4 | 375.78 ± 94.07 | 18.77 | 81.23 |
| ADC-13 (3 mg/kg) | 218.45 ± 13.64 | 109.55 ± 13.38 | -12.73 | 149.85 |
| ADC-98 (3 mg/kg) | 217.64 ± 14.06 | 248.53 ± 36.91 | 3.61 | 96.39 |
| ADC-99 (3 mg/kg) | 217.52 ± 16.07 | 251.04 ± 65.63 | 3.92 | 96.08 |
| ADC-100 (3 mg/kg) | 217.17 ± 13.18 | 134.12 ± 16.65 | -9.71 | 138.24 |

(continued)

| Group | Average tumor volume ($mm^3$) | | Relative tumor proliferation rate, T/C% | Tumor growth inhibition rate, TGI% |
|---|---|---|---|---|
| | Day 0 | Day 35 | | |
| ADC-105 (3 mg/kg) | 216.64 ± 16.43 | 359.07 ± 50.97 | 16.64 | 83.36 |
| ADC-108 (3 mg/kg) | 217.57 ± 13.27 | 300.96 ± 40.77 | 9.75 | 90.25 |
| Reference 1 (10 mg/kg) | 229.07 ± 13.6 | 177.41 ± 36.03 | -6.59 | 122.55 |
| ADC-13 (10 mg/kg) | 213.62 ± 11.89 | 81.97± 5.1 | -16.79 | 161.63 |
| ADC-98 (10 mg/kg) | 214.51 ± 9.37 | 95.76±8.8 | -15.14 | 155.36 |
| ADC-99 (10 mg/kg) | 219.68 ± 11.8 | 120.74 ± 16.79 | -12.62 | 145.04 |
| ADC-100 (10 mg/kg) | 216.06±7.6 | 81.01±5.05 | -17.22 | 162.51 |
| ADC-105 (10 mg/kg) | 219.71 ± 12.04 | 81.19±13.82 | -17.66 | 163.04 |
| ADC-108 (10 mg/kg) | 220.67 ± 12.14 | 77.6±9.67 | -18.24 | 164.84 |

The body weight changes of the experimental mice in the above test were summarized in Figure 12. The results showed that the body weight of animals of each group was normal during the administration process, indicating that the mice had good tolerance to the antibody-drug conjugates to be tested.

Test Example 11: Evaluation of efficacy of antibody-drug conjugates in NCI-H322 transplanted tumor mice

1. Test purpose:
The purpose of this experiment is to detect the efficacy of HER3 antibody ADC drugs on nude mice with human lung cancer transplanted tumors.

2. Test drugs and materials:

(1) Test drug: antibody-drug conjugate: 6 mg/kg.

(2) Experimental animals: B-NDG female mice, 6 to 8 weeks old, weighing 20 to 22 g (Biosai Figure), raised in a sterile animal room at 25°C with a 12-hour day and night cycle, and were allowed to access food and water freely.

3. Experimental methods:

(1) NCI-H322 mouse subcutaneous transplant tumor model:

Inoculation method: NCI-H322 cells (human lung cancer cells, China Food and Drug Inspection Institute, 16 generations) in the logarithmic growth period were taken, centrifuged at 300 times gravitational acceleration (g) for 7 minutes, washed twice with pre-cooled PBS, and counted. The cells were adjusted to reach a cell density of $6e^7$/mL, mixed thoroughly with Matrigel (1:1), aliquoted into sterile 2 mL EP tubes, and placed on ice for later use. 1 mL sterile syringe was used to subcutaneously inoculate the tumor at a volume of 100 μL/cell, wherein liquid leakage should be avoided.

(2) Administration and tumor volume detection

When the volume of the transplanted tumor reached 100 to 300 $mm^3$ (about the 20th day after tumor inoculation), tumor-bearing mice with similar tumor volumes were selected for grouping and administration, with 6 mice in each group. The drug was administered via tail vein, once on day 0. The tumor volume was measured every 2 to 3 days using a vernier caliper. The length (a) and width (b) of the tumor were taken, and volume = a×b×b/2. A tumor growth curve was drawn (the tumor volume did not exceed 3000 $mm^3$, and when it reached 3000 $mm^3$, the mice were sacrificed). The body weight changes of the mice were also detected.

(3) Data analysis:

All experimental results were expressed as mean ± standard error, and the tumor volume of each group was statistically analyzed using Graphpad Prism 7.0 software. The control group and all treatment groups were statistically analyzed using one-way ANOVA and Dunnett's test to evaluate the differences between groups. P<0.05 was considered to be significant different.
*P<0.05, **P<0.01, ***P<0.001, ****P<0.0001.

$$T/C\ (\%) = (T\text{-}T_0) / (C\text{-}C_0) \times 100,$$

wherein T and C were the tumor volumes of the experimental group and the control group at the end of the experiment; $T_0$ and $C_0$ were the tumor volumes at the beginning of the experiment.

$$\text{Tumor growth inhibition rate (TGI) (\%)} = 100\text{-}T/C\ (\%).$$

When the tumor regressed, the tumor growth inhibition rate (TGI) (%) = 100-(T-$T_0$)/$T_0$×100. When the tumor volume decreased, that was, T<$T_0$ or C<$C_0$, it was defined as partial tumor regression (PR); if the tumor disappeared completely, it was defined as complete tumor regression (CR).

The average tumor volume change, relative tumor growth rate, and tumor growth inhibition rate in the above test were summarized in Figure 13 and Table 9, which indicated that the antibody-drug conjugates tested could effectively inhibit the growth of NCI-H322 transplanted tumors in nude mice, and at the same dose, the tumor growth inhibition effect of **ADC-132** and **ADC-133** on the same batch of tumor cells was better than that of **Reference 3.**

Table 9:

| Group | Average tumor volume ($mm^3$) | | Relative tumor proliferation rate, T/C% | Tumor growth inhibition rate, TGI% |
|---|---|---|---|---|
| | Day 0 | Day 13 | | |
| Vehicle | 205.60 ± 16.32 | 562.96±45.13 | - | - |
| Patritumab | 204.21 ± 17.02 | 498.73±57.08 | 82.41 | 17.58 |
| Reference 3 | 192.93 ± 13.66 | 318.18±43.47 | 35.04 | 64.95 |
| ADC-132 | 193.16 ± 17.56 | 284.38±29.41 | 25.52 | 74.47 |
| ADC-133 | 192.71 ± 16.56 | 248.97±35.71 | 15.74 | 84.25 |

The body weight changes of the experimental mice in the above test were summarized in Figure 14. The results showed that the body weight of animals of each group was normal during the administration process, indicating that the mice had good tolerance to the antibody-drug conjugates tested.

Test Example 12: Pharmacokinetics of antibody-drug conjugates of the present disclosure in rats

1. Test purpose:
The purpose of this experiment is to study the pharmacokinetic characteristics of HER2 antibody ADC drugs and provide a reference for subsequent research.

2. Test drugs and materials:

   (1) Test drug: HER2 antibody-drug conjugate: 3 mg/kg.

   (2) Experimental animals: SD rats, 6 to 8 weeks old, weighing 180 to 200 g (Sichuan Weitong Lihua Experimental Animal Technology Co., Ltd.), raised in a sterile animal room at 25 °C with a 12-hour day and night cycle, and were allowed to access food and water freely.

3. Experimental methods:

4. Rats, half female and half male, were divided into 3 groups, namely, ADC-105, ADC-107, and ADC-98, wherein the animals were administered with single dose, at a dosing volume of 10 mL/kg.

Sampling time: Blood samples were collected once before administration, and at 5 min, 7 h, 24 h, 72 h, 168 h, 336 h, and 504 h after administration. Sampling method: Blood samples were collected from jugular vein of rats.
Blood sample processing: After the blood was collected, it was placed in an EDTA anticoagulant tube, and the sample tube was gently inverted several times to ensure well mixing. After the blood was collected, it was placed in a 37 °C constant temperature oven for 30 minutes, centrifuged at 4 °C and 4500rpm for 12 minutes, then the plasma was pipetted, placed in a -20 °C refrigerator, and transferred to a ultra-low temperature refrigerator (-60 °C and below) after 24 hours. Sample analysis: The established ELISA method was used to analyze the blood concentrations of antibody and ADC drug in serum. The existing LC-MS /MS method was used to analyze the blood concentration of cytotoxic drug in plasma.
The experimental results were shown in Table 10

Table 10

| Analytes | | $T_{1/2}$(h) | $T_{max}$(h) | $C_{max}$(ug/mL) | $AUC_{0-t}$(h.ug/mL) |
|---|---|---|---|---|---|
| Experimental group I | ADC-105 | 160.20 | 0.083 | 93.9 | 6604.91 |
| | ADC-105 total antibody | 183.67 | 0.083 | 69.41 | 6703.19 |
| | 2-A | 91.56 | 0.083 | 0.0017 | 0.039 |
| Experimental group II | ADC-107 | 132.97 | 0.083 | 124.16 | 9309.29 |
| | ADC-107 total antibody | 170.48 | 0.083 | 102.15 | 9093.86 |
| | 1-B | 5.13 | 0.083 | 0.0034 | 0.014 |
| Experimental group III | ADC-98 | 141.00 | 0.083 | 110.81 | 8699.15 |
| | ADC-98 total antibody | 163.15 | 0.083 | 91.74 | 9172.86 |
| | 1-B | N.A. | 0.083 | 0.0003 | 0.0013 |
| N.A. represents that the data was too small to fit. | | | | | |

Experimental Conclusion: According to the results in Table 10, no pharmacokinetic differences were observed between the the antibody-drug conjugates (ADCs) of the present disclosure and the total antibody, indicating that the antibody-drug conjugates of the present disclosure had good stability in plasma *in vivo.* At the same time, low blood concentrations of cytotoxic drugs in plasma were detected, which indicated that due to the stability of the antibody-drug conjugates of the present disclosure and the short systemic half-life of the corresponding cytotoxins, the systemic exposure of each cytotoxic drug was low, and thus the ADCs had a higher safety.

Test Example 13: Test of toxicity of the antibody-drug conjugates of the present disclosure in rats

1. Test purpose:
The purpose of this experiment is to study the toxicity characteristics of HER2 antibody ADC drugs and provide a reference for subsequent research.

2. Test drugs and materials:

(1) Test drug: HER2 antibody-drug conjugate: 20 mg/kg, 60 mg/kg, 197 mg/kg.

(2) Experimental animals: SD rats, 6 to 8 weeks old, weighing 180 to 200 g (Sichuan Weitonglihua Experimental Animal Technology Co., Ltd.), raised in a sterile animal room at 25 °C with a 12-hour day and night cycle, and were allowed to access food and water freely.

3. Experimental method:

Each group had 4 rats, half female and half male. The rats were divided into 3 groups and given **ADC-98, ADC-100,** and **ADC-113** respectively, with a single dose of 10 mL/kg via tail vein injection.

The survival and clinical symptoms of the rats were then observed every other day.

4. Experimental results: The rats survived for more than 21 days at each dose.

5. Experimental conclusion: According to the results of this test example, the maximum tolerable dose of the antibody-drug conjugates of the present disclosure in rats was greater than 197 mg/kg, and thus they had high safety.

Test Example 14: Test of bystander killing effect of the antibody-drug conjugates of the present disclosure on HER2-targeted tumor cells

HER2-positive SK-BR-3 cells (human breast cancer cells, Wuhan Procell, Catalog No. CL-0211) and HER2-negative MDA-MB-468 cells (human Breast cancer cells, Cell Center, Shanghai Institutes for Biological Sciences, Chinese Academy of Sciences, Catalog No. 3131C0001000700120) were adjusted with RPMI-1640+10% FBS to reach cell density of $1.2 \times 10^5$ cells/ml and $4 \times 10^4$ cells/ml, respectively. 1 mL of each of SK-BR-3 and MDA-MB-468 cells was added to a 6-well plate and cultured overnight at 37°C with 5% $CO_2$. The sample **ADC-113** was prepared to a concentration of 4.5 μg/mL (30 nM), and 1 mL was added to the cells to make the total volume 3 mL and the final concentration 10 nM. A solvent control group was set up and cultured at 37 °C and 5% $CO_2$ for 5 days. The cells were digested by adding trypsin (0.25% Trypsin-EDTA (1×), Shanghai BasalMedia Technologies Co., Ltd., Catalog No.: A121002) to the cells in the 6-well plate, and added with 1 mL of FACS buffer (PBS + 4% FBS) to resuspend. The cells were stained with trypan blue and counted. The remaining cells were centrifuged, and Trastuzumab was diluted to 10 μg/mL in FACS buffer. The cells were resuspended with 200 μL of antibody solution and incubated on ice for 30 min. The cells were centrifuged and the supernatant was discarded. The cells were washed once with 1 mL of FACS buffer, and 200 μL of AF647-labeled goat anti-human Fc antibody (Jackson, Catalog No.: 109-606-170) solution was added and incubated on ice for 15 min, 5 μg/mL PI (Sigma, Catalog No.: 31845) solution was added and incubated on ice for 5 min, centrifugation was performed and the supernatant was discarded, washing was performed once with 1 mL FACS buffer and the cells were resuspended in 400 μL PBS. BD Celesta flow cytometer was used for detection and analysis. The ratio and number of cells were determined based on the flow cytometry and counting results.

The results of the above tests were summarized in Figure 15, which indicated that the antibody-drug conjugate of the present disclosure had excellent bystander killing effect.

Test Example 15: Test of bystander killing effect of the antibody-drug conjugates of the present disclosure on HER2-targeted tumor cells

HER2-positive SK-BR-3 cells (human breast cancer cells, Wuhan Procell, Catalog No. CL-0211) and HER2-negative MDA-MB-468 cells (human breast cancer cells, Cell Center, Shanghai Institutes for Biological Sciences, Chinese Academy of Sciences, Catalog No. 3131C0001000700120) were adjusted with RPMI-1640 + 10% FBS to reach cell density of $1.2 \times 10^5$ cells/ml and $4 \times 10^4$ cells/ml, respectively. 1 mL of each of SK-BR-3 and MDA-MB-468 cells was added to a 6-well plate, mixed and cultured overnight at 37 °C and 5% $CO_2$. Sample **ADC-115** was prepared to a concentration of 4.5 μg/mL (30 nM), and 1 mL was taken and added to the cells to make the total volume 3 mL and the final concentration 10 nM. A solvent control group was set up and cultured at 37°C and 5% $CO_2$ for 5 days. Trypsin (0.25% Trypsin-EDTA (1×), Shanghai BasalMedia Technologies Co., Ltd., Catalog No.: A121002) was added to the 6-well plate for digestion. The cells were resuspend in 1 mL FACS buffer (PBS + 4% FBS), stained with trypan blue, and counted. The remaining cells were centrifuged, and Trastuzumab was diluted to 10 μg/mL in FACS buffer. The cells were resuspended with 200 μL of antibody solution and incubated on ice for 30 min. The cells were centrifuged and the supernatant was discarded. The cells were washed once with 1 mL of FACS buffer, and 200 μL of AF647-labeled goat anti-human Fc antibody (Jackson, Catalog No.: 109-606-170) solution was added and incubated on ice for 15 min, 5 μg/mL PI (Sigma, Catalog No.: 31845) solution was added and incubated on ice for 5 min, centrifugation was performed and the supernatant was discarded, the cells were washed once with 1 mL FACS buffer, resuspended by adding 400 μL of PBS, and analyzed by BD Celesta flow cytometer. The ratio and number of cells were determined based on the flow cytometry and counting results.

The results of the above tests were summarized in Figure 16, which indicated that the antibody-drug conjugate of the present disclosure had excellent bystander killing effect.

Test Example 16: Evaluation of efficacy of antibody-drug conjugates in NCI-N87 transplanted tumor mice

1. Test purpose: The purpose of this experiment is to detect the efficacy of HER2 antibody ADC drugs on nude mice with human gastric cancer cell NCI-N87 transplanted tumor.

2. Test drugs and materials:

(1) Test drug: antibody-drug conjugate: 2 mg/kg, 6 mg/kg.

(2) Experimental animals: Balb/c-nude female mice, 6 to 8 weeks old, weighing 20 to 22g (Gempharmatech), raised in a sterile animal room at 25 °C with 12-hour day and night cycle, and were allowed to access food and water freely.

3. Experimental methods:

(1) NCI-N87 subcutaneously transplanted tumor mouse model

Inoculation method: NCI-N87 cells in the logarithmic growth phase (Cell Resource Center, Institute of Basic Medical Sciences, Chinese Academy of Medical Sciences, 3111C0001CCC000481) were taken, and centrifuged at 300 times gravitational acceleration (g) for 7 min. The cells were washed twice with pre-cooled PBS and counted, adjusted to reach a cell density to $6e^7$/mL, a total of 10 mL, mixed thoroughly in PBS, aliquoted into sterile 1.5 mL EP tubes, and placed on ice for later use. 1 mL sterile syringe was used to subcutaneously inoculate the tumor at a volume of 100 μL/mouse, in which the liquid leakage should be avoided.

(2) Drug administration and tumor volume detection

When the volume of transplanted tumor reached 100 to 300 $mm^3$ (on the 8th day after tumor inoculation), tumor-bearing mice with similar tumor volumes were selected for grouping and administration, with 5 mice in each group. Drugs were administered via tail vein. For the 2 mg/kg group, the drug was administered once on day 0 and once on day 7. For the 6 mg/kg group, the drug was administered once on day 0. Tumor volume was measured every 2 to 3 days using a vernier caliper. The length (a) and width (b) of tumor were taken, and volume = a×b×b/2. Tumor growth curves were drawn (the tumor volume did not exceed 3000 $mm^3$, and when it reached 3000 $mm^3$, the mice were sacrificed). The body weight changes of the mice were also detected.

(3) Data analysis:

All experimental results were expressed as mean ± standard error. Graphpad Prism 7.0 software was used to perform statistical analysis on the tumor volume of each group. For tumor volume, one-way ANOVA and Dunnett's test were used for statistical analysis of the vehicle control group and all treatment groups to evaluate the differences between groups. P<0.05 was considered to be significantly different.
*P<0.05, **P<0.01, ***P<0.001, ****P<0.0001.

$$T/C\ (\%) = (T-T_0)/(C-C_0) \times 100,$$

wherein T and C were the tumor volumes of the experimental group and the control group at the end of the experiment; $T_0$ and $C_0$ were the tumor volumes at the beginning of the experiment.

$$\text{Tumor growth inhibition rate (TGI) (\%)} = 100\text{-}T/C\ (\%).$$

When the tumor regressed, the tumor growth inhibition rate (TGI) (%) = 100-(T-$T_0$)/$T_0$×100. If the tumor was smaller than the starting volume, that was, T<$T_0$ or C<$C_0$, it was defined as partial tumor regression (PR); if the tumor disappeared completely, it was defined as complete tumor regression (CR).

The results of the average tumor volume change, relative tumor growth rate and tumor growth inhibition rate of the above test were summarized in Figure 17 and Table 11, which indicated that the antibody-drug conjugates tested could effectively inhibit the growth of NCI-N87 transplanted tumors in nude mice, and at the same dose, **ADC-113** had a better tumor inhibition effect on the same batch of tumor cells than **Reference 1.**

Table 11

| Group | Average tumor volume ($mm^3$) | | Relative tumor proliferation rate, T/C% | Tumor growth inhibition rate, TGI% |
|---|---|---|---|---|
| | Day 0 | Day 19 | | |
| Vehicle | 175.03±13.53 | 676.06±53.36 | - | - |

(continued)

| Group | Average tumor volume ($mm^3$) | | Relative tumor proliferation rate, T/C% | Tumor growth inhibition rate, TGI% |
|---|---|---|---|---|
| | Day 0 | Day 19 | | |
| Reference 1 (2 mg/kg) | 162.79±15.37 | 382.35±64.42 | 43.82 | 56.17 |
| ADC-113 (2 mg/kg) | 175.11±13.71 | 305.31±49.51 | 25.98 | 74.013 |
| Reference 1 (6 mg/kg) | 175.81±14.42 | 125.09±35.92 | -10.12 | 128.84 |
| ADC-113 (6 mg/kg) | 175.58±13.64 | 82.22±20.91 | -18.63 | 153.16 |

The body weight changes of the experimental mice in the above test were summarized in Figure 18. The results showed that the body weight of the animals in each group was normal during the administration process, indicating that the mice had good tolerance to the antibody-drug conjugates tested.

Test Example 17: Evaluation of efficacy of antibody-drug conjugates in NCI-N87 transplanted tumor mice

1. Test objective:
The objective of this experiment is to detect the efficacy of HER2 antibody ADC drugs on nude mice with human gastric cancer cell NCI-N87 transplanted tumor.

2. Test drugs and materials:

(1) Test drug: antibody-drug conjugate: 2 mg/kg.
(2) Experimental animals: Balb/c-nude female mice, 6 to 8 weeks old, weighing 20 to 22g (Gempharmatech), raised in a sterile animal room at 25 °C with 12 h day and night cycle, and were allowed to access food and water freely.

3. Experimental methods:

(1) NCI-N87 subcutaneously transplanted tumor mouse model

Inoculation method: NCI-N87 cells in the logarithmic growth phase (Cell Resource Center, Institute of Basic Medical Sciences, Chinese Academy of Medical Sciences, 3111C0001CCC000481) were taken, and centrifuged at 300 times gravitational acceleration (g) for 7 min. The cells were washed twice with pre-cooled PBS and counted, adjusted to reach a cell density of 6e7/mL, a total of 10 mL, mixed thoroughly in PBS, aliquoted into sterile 1.5 mL EP tubes, and placed on ice for later use. 1 mL sterile syringe was used to subcutaneously inoculate the tumor at a volume of 100 μL/mouse, liquid leakage should be avoided.

(2) Drug administration and tumor volume detection

When the volume of the transplanted tumor reached 100 to 300 $mm^3$ (on the 8th day after tumor inoculation), tumor-bearing mice with similar tumor volumes were selected for grouping and administration, with 5 mice in each group. Drugs were administered via tail vein, once on day 0. Tumor volume was measured every 2 to 3 days using a vernier caliper. The length (a) and width (b) of the tumor were taken, and volume = a×b×b/2. Tumor growth curves were drawn (the tumor volume did not exceed 3000 $mm^3$, and when it reached 3000 $mm^3$, the mice were sacrificed), and the body weight changes of mice were detected at the same time.

(3) Data analysis:

All experimental results were expressed as mean ± standard error. Graphpad Prism 7.0 software was used to perform statistical analysis on the tumor volume of each group. For tumor volume, one-way ANOVA and Dunnett's test were used for statistical analysis of the vehicle control group and all treatment groups to evaluate the differences between groups. P<0.05 was considered to be significantly different.
*P<0.05, **P<0.01, ***P<0.001, ****P<0.0001.

$$T/C (\%) = (T-T0) / (C-C0) \times 100,$$

where Tand C are the tumor volumes of the experimental group and the control group at the end of the experiment; T0 and C0 are the tumor volumes at the beginning of the experiment.

$$\text{Tumor inhibition rate (TGI) (\%)} = 100-T/C (\%).$$

When the tumor regressed, the tumor growth inhibition rate (TGI) (%) = 100-(T-$T_0$)/$T_0$×100. If the tumor was smaller than the starting volume, that was, T<$T_0$ or C<$C_0$, it was defined as partial tumor regression (PR); if the tumor disappeared completely, it was defined as complete tumor regression (CR).

The results of the average tumor volume change, relative tumor growth rate and tumor growth inhibition rate of the above test were summarized in Figure 19 and Table 12, which indicated that the antibody-drug conjugates tested could effectively inhibit the growth of NCI-N87 transplanted tumors in nude mice.

Table 12

| Group | Average tumor volume ($mm^3$) | | Relative tumor proliferation rate, T/C% | Tumor growth inhibition rate, TGI% |
|---|---|---|---|---|
| | Day 0 | Day 21 | | |
| Vehicle | 227.97±14.78 | 748.59±66.28 | - | - |
| ADC-115 | 222.68±12.18 | 147.67±64.89 | -14.41 | 133.69 |

The body weight changes of the experimental mice in the above test were summarized in Figure 20. The results showed that the body weight of animals of each group was normal during the administration process, indicating that the mice had good tolerance to the antibody-drug conjugate tested.

Test Example 18: Determination of plasma stability of antibody-drug conjugate

The plasma stability of **ADC-115** was evaluated by the same method as in Test Example 6.

The experiment showed that the free toxin of antibody-drug conjugate **ADC-115** is released less in the plasma of humans, monkeys, mice and rats, and the release rate of its free toxin did not exceed 2% on the 21st day.

The above is the exemplary descriptions of the embodiments of the technical solution of the present disclosure. It should be understood that the protection scope of the present disclosure is not limited to the above embodiments. Any modifications, equivalent substitutions, improvements, etc. made by a person skilled in the art within the spirit and principle of the present disclosure shall be included in the protection scope of the claims of the present application.

## Claims

1. A conjugate represented by the following Formula (C), or a stereoisomer, racemate, tautomer, isotopologue, isotope-labeled compound, nitroxide or pharmaceutically acceptable salt thereof:

Tp-L-G (C)

wherein, Tp represents a targeting moiety;
L is selected from a chemical bond or a linker;
G represents a group represented by the following Formula (G):

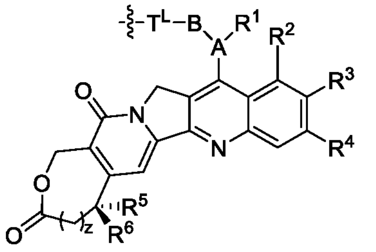

(G)

wherein, A is selected from N or C-$R^A$;

when A is N, $R^1$ is selected from the group consisting of hydrogen, hydroxyl, and the following groups which are unsubstituted or substituted with one, two or more $R^B$: alkyl, cycloalkyl, cycloalkylalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

when A is C-$R^A$, $R^A$ and $R^1$ are the same or different and are independently selected from the group consisting of hydrogen, halogen, hydroxyl, sulfhydryl, cyano, nitro, and the following groups which are unsubstituted or substituted with one, two or more $R^C$: alkyl, cycloalkyl, cycloalkylalkyl, alkyloxy, cycloalkyloxy, alkyloxyalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

or, $R^A$ and $R^1$, together with the atom to which they are attached, form a 3- to 10-membered ring structure which is unsubstituted or substituted with one, two or more $R^D$;

z is selected from 0 or 1;

$R^2$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

$R^3$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

or, $R^1$ and $R^2$, together with the atoms to which they are attached, form a ring structure which is unsubstituted or substituted with one, two or more $R^E$, for example, the ring structure is a 5- to 10-membered ring structure, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a ring structure which is unsubstituted or substituted with one, two or more $R^F$, for example, the ring structure is a 4- to 10-membered ring structure, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of alkyl, alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: cycloalkyl, cycloalkylalkyl, cycloalkyloxy, heterocyclyl, heterocyclylalkyl, heterocyclyloxy, alkyl, alkyloxy, aryl, arylalkyl, aryloxy, heteroaryl, heteroarylalkyl, heteroaryloxy, amino;

B is absent or selected from the group consisting of triazolyl, -$NR^7$- or -$N(NR^7R^8)$-;

$R^7$ and $R^8$ are the same or different and are independently selected from the group consisting of hydrogen and the following groups which are unsubstituted or substituted with one, two or more $R^H$: alkyl, cycloalkyl, cycloalkylalkyl, aryl, arylalkyl, heteroaryl, heteroarylalkyl, heterocyclyl, heterocyclylalkyl, HC(=O)-, alkyl-C(=O)-, cycloalkyl-C(=O)NH-, heterocyclyl-C(=O)NH-, aryl-C(=O)NH-, heteroaryl-C(=O)NH-;

$T^L$ is selected from the group consisting of chemical bond, #O-$(CR^9R^{10})_m$-$(CR^{11}R^{12})_n$-C(=O)*, #O-$(CR^9R^{10})_m$-$(CR^{11}R^{12})_n$*, #N($R^{13}$)-$(CR^9R^{10})_m$-$(CR^{11}R^{12})_n$-C(O)*, #S-$(CR^9R^{10})_m$-$(CR^{11}R^{12})_n$-C(O)*, #O-$(CR^9R^{10})_m$-$(CR^{11}R^{12})_n$-C($NR^{13}$)*, #O-$(CR^9R^{10})_m$-$(CR^{11}R^{12})_n$-P(=O)($OR^{14}$)*, *O-$(CR^9R^{10})_m$-$(CR^{11}R^{12})_n$-C(=O)#, *O-$(CR^9R^{10})_m$-$(CR^{11}R^{12})_n$#, *N($R^{13}$)-$(CR^9R^{10})_m$-$(CR^{11}R^{12})_n$-C(O)#, *S-$(CR^9R^{10})_m$-$(CR^{11}R^{12})_n$-C(O)#, *O-$(CR^9R^{10})_m$-$(CR^{11}R^{12})_n$-C($NR^{13}$)#, *O-$(CR^9R^{10})_m$-$(CR^{11}R^{12})_n$-P(=O)($OR^{14}$)#, wherein # represents the site attached to L, and * represents the site attached to B;

each of $R^9$, $R^{10}$, $R^{11}$ and $R^{12}$ is the same or different, and is independently selected from the group consisting of hydrogen, halogen, cyano, and the following groups which are unsubstituted or substituted with one, two or more $R^I$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy;

or, $R^9$ and $R^{10}$, together with the atoms to which they are attached, form a ring structure which is unsubstituted or

substituted with one, two or more $R^I$, for example, the ring structure is a 3- to 10-membered ring structure; or, $R^{11}$ and $R^{12}$, together with the atoms to which they are attached, form a ring structure which is unsubstituted or substituted with one, two or more $R^I$, for example, the ring structure is a 3- to 10-membered ring structure; or, $R^9$ and $R^{11}$, together with the atoms to which they are attached, form a ring structure which is unsubstituted or substituted with one, two or more $R^I$, for example, the ring structure is a 3- to 10-membered ring structure; wherein any of the ring structures can be a monocyclic hydrocarbyl, bicyclic hydrocarbyl, heteromonocyclic hydrocarbyl, or heterobicyclic hydrocarbyl, each of which is unsubstituted or substituted with one, two or more $R^I$; for example, any of the ring structures can be the following group which is unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: alkyl, cycloalkyl, cycloalkylalkyl, aryl, arylalkyl, heteroaryl, heteroarylalkyl, heterocyclyl, heterocyclylalkyl;

each m is the same or different, and is independently selected from an integer of 0 to 10; each n is the same or different, and is independently selected from an integer of 0 to 10; each of $R^B$, $R^C$, $R^D$, $R^E$, $R^F$, $R^G$, $R^H$, $R^I$ and $R^J$ is the same or different, and is independently selected from the group consisting of deuterium, halogen, hydroxyl, cyano, nitro, and the following groups which are unsubstituted or substituted with one, two or more $R^K$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^L$ is the same or different and is independently selected from the group consisting of deuterium, halogen, hydroxyl, cyano, nitro, alkyl, aryloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

the wavy line represents the site attached to L;

provided that, when $R^6$ is selected from the group consisting of alkyl, alkyloxy, aryl, arylalkyl, aryloxy, heteroaryl, heteroarylalkyl, heteroaryloxy or amino, each of which is unsubstituted or substituted with one, two or more $R^G$:

A is N; $R^1$ and $R^2$, together with the atoms to which they are attached, do not form a 5- to 10-membered ring structure which is unsubstituted or substituted with one, two or more $R^E$; and $T^L$ is not a chemical bond when $R^7$ is hydrogen;

or, $R^1$ and $R^2$, together with the atoms to which they are attached, form a 5- to 10-membered ring structure which is unsubstituted or substituted with one, two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl; and $R^A$ is selected from the following groups which are unsubstituted or substituted with one, two or more $R^C$: cycloalkyl, cycloalkylalkyl, cycloalkyloxy;

or, B is $-N(NR^7R^8)-$;

or, $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl.

**2.** The conjugate, or a stereoisomer, racemate, tautomer, isotopologue, isotope-labeled compound, nitroxide or pharmaceutically acceptable salt thereof according to claim 1, wherein definitions of the groups in Formula (G) are independently selected from:

A is selected from N or C-$R^A$;

when A is N, $R^1$ is selected from the group consisting of hydrogen, hydroxyl, and the following groups which are unsubstituted or substituted with one, two or more $R^B$: alkyl, cycloalkyl, cycloalkylalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

when A is C-$R^A$, $R^A$ and $R^1$ are the same or different and are independently selected from the group consisting of

hydrogen, halogen, hydroxyl, sulfhydryl, cyano, nitro, and the following groups which are unsubstituted or substituted with one, two or more $R^C$: alkyl, cycloalkyl, cycloalkylalkyl, alkyloxy, cycloalkyloxy, alkyloxyalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

or, $R^A$ and $R^1$, together with the atom to which they are attached, form a 3- to 10-membered ring structure which is unsubstituted or substituted with one, two or more $R^D$;

z is selected from 0 or 1;

$R^2$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

$R^3$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

or, $R^1$ and $R^2$, together with the atoms to which they are attached, form a 5- to 10-membered ring structure which is unsubstituted or substituted with one, two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, or heterobicyclic hydrocarbyl;

or $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, or heterobicyclic hydrocarbyl;

$R^4$ is selected from the group consisting of alkyl, alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: cycloalkyl, cycloalkylalkyl, cycloalkyloxy, heterocyclyl, heterocyclylalkyl, heterocyclyloxy;

B is absent or selected from the group consisting of triazolyl, $-NR^7-$ or $-N(NR^7R^8)-$;

$R^7$ and $R^8$ are the same or different and are independently selected from the group consisting of hydrogen, and the following groups which are unsubstituted or substituted with one, two or more $R^H$: alkyl, cycloalkyl, cycloalkylalkyl, aryl, arylalkyl, heteroaryl, heteroarylalkyl, heterocyclyl, heterocyclylalkyl, HC(=O)-, alkyl-C(=O)-, cycloalkyl-C(=O)NH-, heterocyclyl-C(=O)NH-, aryl-C(=O)NH-, heteroaryl-C(=O)NH-;

$T^L$ is selected from the group consisting of chemical bond, $\#O-(CR^9R^{10})_m-(CR^{11}R^{12})_n-C(=O)*$, $\#O-(CR^9R^{10})_m-(CR^{11}R^{12})_n*$, $\#N(R^{13})-(CR^9R^{10})_m-(CR^{11}R^{12})_n-C(O)*$, $\#S-(CR^9R^{10})_m-(CR^{11}R^{12})_n-C(O)*$, $\#O-(CR^9R^{10})_m-(CR^{11}R^{12})_n-C(NR^{13})*$, $\#O-(CR^9R^{10})_m-(CR^{11}R^{12})_n-P(=O)(OR^{14})*$, $*O-(CR^9R^{10})_m-(CR^{11}R^{12})_n-C(=O)\#$, $*O-(CR^9R^{10})_m-(CR^{11}R^{12})_n\#$, $*N(R^{13})-(CR^9R^{10})_m-(CR^{11}R^{12})_n-C(O)\#$, $*S-(CR^9R^{10})_m-(CR^{11}R^{12})_n-C(O)\#$, $*O-(CR^9R^{10})_m-(CR^{11}R^{12})_n-C(NR^{13})\#$, $*O-(CR^9R^{10})_m-(CR^{11}R^{12})_n-P(=O)(OR^{14})\#$, wherein # represents the site attached with L, and * represents the site attached with B;

each of $R^9$, $R^{10}$, $R^{11}$ and $R^{12}$ is the same or different and is independently selected from the group consisting of hydrogen, halogen, cyano, and the following groups which are unsubstituted or substituted with one, two or more $R^I$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; wherein any of the 3- to 10-membered ring structures can be selected from the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: alkyl, cycloalkyl, cycloalkylalkyl, aryl, arylalkyl, heteroaryl, heteroarylalkyl, heterocyclyl, heterocycloalkyl;

m and n are the same or different and are independently selected from an integer of 0 to 10;

each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: alkyl, alkyloxy, cycloalkyl, cycloalkylalkyl, cycloalkyloxy, aryl, arylalkyl, aryloxy, heteroaryl, heteroarylalkyl,

heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^L$ is the same or different and is independently selected from the group consisting of deuterium, halogen, hydroxyl, cyano, nitro, alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-.

**3.** The conjugate, or a stereoisomer, racemate, tautomer, isotopologue, isotope-labeled compound, nitroxide or pharmaceutically acceptable salt thereof according to claim 1, wherein definitions of the groups in Formula (G) are independently selected from:

A is selected from N or C-$R^A$;

when A is N, $R^1$ is selected from the group consisting of hydrogen, hydroxyl, and the following groups which are unsubstituted or substituted with one, two or more $R^B$: alkyl, cycloalkyl, cycloalkylalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

when A is C-$R^A$, $R^A$ and $R^1$ are the same or different and are independently selected from the group consisting of hydrogen, halogen, hydroxyl, sulfhydryl, cyano, nitro, and the following groups which are unsubstituted or substituted with one, two or more $R^C$: alkyl, cycloalkyl, cycloalkylalkyl, alkyloxy, cycloalkyloxy, alkyloxyalkyl, heterocyclyl, heterocyclylalkyl, aryl, arylalkyl, heteroaryl or heteroarylalkyl, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

or, $R^A$ and $R^1$, together with the atom to which they are attached, form a 3- to 10-membered ring structure which is unsubstituted or substituted with one, two or more $R^D$;

z is selected from 0 or 1;

$R^2$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

$R^3$ is selected from the group consisting of hydrogen, halogen, and the following groups which are unsubstituted or substituted with one, two or more $R^E$: alkyl, alkyloxy, cycloalkyl, cycloalkylalkyl, cycloalkyloxy;

or, $R^1$ and $R^2$, together with the atoms to which they are attached, form a 5- to 10-membered ring structure which is unsubstituted or substituted with one, two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of alkyl, alkyloxy, halogen, hydroxyl, aminoand cyano;

$R^5$ is selected from hydroxyl;

$R^6$ is selected from the following groups which are unsubstituted or substituted with one, two or more $R^G$: cycloalkyl, cycloalkylalkyl, cycloalkyloxy, heterocyclyl, heterocyclylalkyl, heterocyclyloxy;

B is absent or selected from -$NR^7$- or -N($NR^7R^8$)-;

$R^7$ and $R^8$ are the same or different and are independently selected from the group consisting of hydrogen, and the following groups which are unsubstituted or substituted with one, two or more $R^H$: alkyl, cycloalkyl, cycloalkylalkyl, aryl, arylalkyl, heteroaryl, heteroarylalkyl, heterocyclyl, heterocyclylalkyl, HC(=O)-, alkyl-C(=O)-, cycloalkyl-C(=O)NH-, heterocyclyl-C(=O)NH-, aryl-C(=O)NH-, heteroaryl-C(=O)NH-;

$T^L$ is selected from the group consisting of chemical bond, #O-$(CR^9R^{10})_m$-$(CR^{11}R^{12})_n$-C(=O)*, #O-$(CR^9R^{10})_m$-$(CR^{11}R^{12})_n$*, #N($R^{13}$)-$(CR^9R^{10})_m$-$(CR^{11}R^{12})_n$-C(O)*, #S-$(CR^9R^{10})_m$-$(CR^{11}R^{12})_n$-C(O)*, #O-$(CR^9R^{10})_m$-$(CR^{11}R^{12})_n$-C($NR^{13}$)*, #O-$(CR^9R^{10})_m$-$(CR^{11}R^{12})_n$-P(=O)($OR^{14}$)*, *O-$(CR^9R^{10})_m$-$(CR^{11}R^{12})_n$-C(=O)#, *O-$(CR^9R^{10})_m$-$(CR^{11}R^{12})_n$#, *N($R^{13}$)-$(CR^9R^{10})_m$-$(CR^{11}R^{12})_n$-C(O)#, *S-$(CR^9R^{10})_m$-$(CR^{11}R^{12})_n$-C(O)#, *O-$(CR^9R^{10})_m$-$(CR^{11}R^{12})_n$-C($NR^{13}$)#, *O-$(CR^9R^{10})_m$-$(CR^{11}R^{12})_n$-P(=O)($OR^{14}$)#, wherein # represents the site attached to L, and * represents the site attached to B;

each of $R^9$, $R^{10}$, $R^{11}$ and $R^{12}$ is the same or different and is independently selected from the group consisting of hydrogen, halogen, cyano, and the following groups which are unsubstituted or substituted with one, two or more $R^I$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$ together with the atoms to which they are attached form a 3- to 10-membered ring structure which is unsubstituted or substituted with one, two or more $R^I$;

or, $R^9$ and $R^{11}$, together with the atoms to which they are attached, form a 3- to 10-membered ring structure which is unsubstituted or substituted with one, two or more $R^I$; wherein any of the 3- to 10-membered ring structures can be selected from, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: alkyl, cycloalkyl, cycloalkylalkyl, aryl, arylalkyl, heteroaryl, heteroarylalkyl, heterocyclyl, heterocyclylalkyl;

m and n are the same or different, and are independently selected from an integer of 0 to 10;

each of $R^B$, $R^C$, $R^D$, $R^E$, $R^F$, $R^G$, $R^H$, $R^I$ and $R^J$ is the same or different, and is independently selected from the group consisting of deuterium, halogen, hydroxyl, cyano, nitro, and the following groups which are unsubstituted or substituted with one, two or more $R^K$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^K$ is the same or different, and is independently selected from the group consisting of deuterium, halogen, hydroxyl, cyano, nitro, and the following groups which are unsubstituted or substituted with one, two or more $R^L$: alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-;

each $R^L$ is the same or different and is independently selected from the group consisting of deuterium, halogen, hydroxyl, cyano, nitro, alkyl, alkyloxy, cycloalkyl, cycloalkylalkyl, cycloalkyloxy, aryl, arylalkyl, aryloxy, heteroaryl, heteroarylalkyl, heteroaryloxy, heterocyclyl, heterocyclylalkyl, heterocyclyloxy, $NH_2$, HC(=O)NH-, alkyl-C(=O)NH-, cycloalkyl-C(=O)NH-, heterocyclyl-C(=O)NH-, aryl-C(=O)NH-, heteroaryl-C(=O)NH-.

**4.** The conjugate, or a stereoisomer, racemate, tautomer, isotopologue, isotope-labeled compound, nitroxide or pharmaceutically acceptable salt thereof according to claim 1, wherein definitions of the groups in Formula (G) are independently selected from:

A is selected from N or C-$R^A$;

when A is N, $R^1$ is selected from the group consisting of $^1H$, $^2H$, hydroxyl, deuterated hydroxyl, and the following groups which are unsubstituted or substituted with one, two or more $R^B$: $C_{1-10}$ alkyl, $C_{3-10}$ cycloalkyl, $C_{3-10}$ cycloalkyl-$C_{1-10}$ alkyl, 3- to 10-membered heterocyclyl, 3- to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is N, $R^1$ is selected from the group consisting of $^1H$, $^2H$, hydroxyl, deuterated hydroxyl, and the following groups which are unsubstituted or substituted with one, two or more $R^B$: $C_{1-6}$ alkyl, $C_{3-6}$ cycloalkyl, $C_{3-6}$ cycloalkyl-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

when A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-10}$ alkyl, $C_{3-10}$ cycloalkyl, $C_{3-10}$ cycloalkyl-$C_{1-10}$ alkyl, $C_{1-10}$ alkyloxy, $C_{3-10}$ cycloalkyloxy, $C_{1-10}$ alkyloxy-$C_{1-10}$ alkyl, 3- to 10-membered heterocyclyl, 3- to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-6}$ alkyl, $C_{3-6}$ cycloalkyl, $C_{3-6}$ cycloalkyl-$C_{1-6}$ alkyl, $C_{1-6}$ alkyloxy, $C_{3-6}$ cycloalkyloxy, $C_{1-6}$ alkyloxy-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

or, $R^A$ and $R^1$, together with the atom to which they are attached, form a 3- to 8-membered ring structure which is unsubstituted or substituted with one, two or more $R^D$, for example, the 3-to 8-membered ring structure is 3-, 4-, 5-, 6-, 7- or 8-membered ring structure;

z is selected from 0 or 1;

$R^2$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^2$ is selected from the group consisting of $^1H$, $^2H$, hydrogen, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy;

$R^3$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^3$ is selected from the group consisting of $^1H$, $^2H$, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-10}$ alkyl, $C_{3-6}$ cycloalkyloxy;

or, $R^1$ and $R^2$, together with the atoms to which they are attached, form a 5- to 10-membered ring structure which is unsubstituted or substituted with two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a 4- to 10-membered ring structure which is unsubstituted or substituted with one, two or more $R^F$, the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of $C_{1-10}$ alkyl, $C_{1-10}$ alkyloxy, halogen, hydroxyl, sulfhydryl, amino, cyano; preferably, $R^4$ is selected from the group consisting of $C_{1-6}$ alkyl, $C_{1-6}$ alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: $C_{3-10}$ cycloalkyl, $C_{3-10}$ cycloalkyl-$C_{1-10}$ alkyl, $C_{3-10}$ cycloalkyloxy, 3- to 10-membered heterocyclyl, 3- to 10-membered heterocyclyl-$C_{1-10}$ alkyl, 3- to 10-membered heterocyclyloxy; preferably, $R^6$ is selected from the following groups which are unsubstituted or substituted with one, two or more $R^G$: $C_{3-6}$ cycloalkyl, $C_{3-6}$ cycloalkyl-$C_{1-6}$ alkyl, $C_{3-6}$ cycloalkyloxy, 3- to 6-membered heterocyclyl, 3- to 6-membered heterocyclyl-$C_{1-6}$ alkyl, 3- to 6-membered heterocyclyloxy;

B is absent or selected from the group consisting of triazolyl, - $NR^7$- or -$N(NR^7R^8)$-;

$R^7$ and $R^8$ are the same or different and are independently selected from the group consisting of $^1H$, $^2H$, and the following groups which are unsubstituted or substituted with one, two or more $R^H$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl, HC(=O)-, $C_{1-10}$ alkyl-C(=O)-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, $R^7$ and $R^8$ are the same or different and are independently selected from the group consisting of hydrogen, and the following groups which are unsubstituted or substituted with one, two or more $R^H$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl, HC(=O)-, $C_{1-6}$ alkyl-C(=O)-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

$T^L$ is selected from the group consisting of chemical bond, #O-$(CR^9R^{10})_m$-$(CR^{11}R^{12})_n$-C(=O)*, #O-$(CR^9R^{10})_m$-$(CR^{11}R^{12})_n$*, #N($R^{13}$)-$(CR^9R^{10})_m$-$(CR^{11}R^{12})_n$-C(O)*, #S-$(CR^9R^{10})_m$-$(CR^{11}R^{12})_n$-C(O)*, #O-$(CR^9R^{10})_m$-$(CR^{11}R^{12})_n$-C($NR^{13}$)*, #O-$(CR^9R^{10})_m$-$(CR^{11}R^{12})_n$-P(=O)($OR^{14}$)*, *O-$(CR^9R^{10})_m$-$(CR^{11}R^{12})_n$-C(=O)#, *O-$(CR^9R^{10})_m$-$(CR^{11}R^{12})_n$#, *N($R^{13}$)-$(CR^9R^{10})_m$-$(CR^{11}R^{12})_n$-C(O)#, *S-$(CR^9R^{10})_m$-$(CR^{11}R^{12})_n$-C(O)#, *O-$(CR^9R^{10})_m$-$(CR^{11}R^{12})_n$-C($NR^{13}$)#, *O-$(CR^9R^{10})_m$-$(CR^{11}R^{12})_n$-P(=O)($OR^{14}$)#, wherein # represents the site attached to L, and * represents the site attached to B;

each of $R^9$, $R^{10}$, $R^{11}$ and $R^{12}$ is the same or different, and is independently selected from the group consisting of $^1H$, $^2H$, halogen, cyano, and the following groups which are unsubstituted or substituted with one, two or more $R^I$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy; preferably, each of $R^9$, $R^{10}$, $R^{11}$ and $R^{12}$ is the same or different and is independently selected from the group consisting of hydrogen, and the following groups which are unsubstituted or substituted with one, two or more $R^I$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3-to 6-membered heterocyclyl, 3- to 6-membered heterocyclyl-$C_{1-10}$ alkyl, 3- to 6-membered heterocyclyloxy;

or, $R^9$ and $R^{10}$, together with the atoms to which they are attached, form a 3- to 10-membered ring structure which

is unsubstituted or substituted with one, two or more $R^I$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; wherein any of the 3- to 10-membered ring structures can be selected from, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the groups which are unsubstituted or substituted with one, two or more $R^J$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl; preferably, each $R^{13}$ is the same or different and is independently selected from the following groups which are unsubstituted or substituted with one, two or more $R^J$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl;

m and n are the same or different and are independently selected from an integer of 0 to 10, such as 0, 1, 2, 3, 4, 5, 6, 7, 8, 9 or 10;

each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each $R^K$ is the same or different and is independently selected from the group consisting of deuterium, halogen, hydroxyl, cyano, nitro, and the following groups which are unsubstituted or substituted with one, two or more $R^L$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^L$ is the same or different and is independently selected from the group consisting of deuterium, halogen, hydroxyl, cyano, nitro, $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-.

**5.** The conjugate, or a stereoisomer, racemate, tautomer, isotopologue, isotope-labeled compound, nitroxide or pharmaceutically acceptable salt thereof according to claim 1, wherein definitions of the groups in Formula (G) are independently selected from:

A is selected from N or C-$R^A$;

when A is N, $R^1$ is selected from the group consisting of $^1H$, $^2H$, hydroxyl, deuterated hydroxyl, and the following groups which are unsubstituted or substituted with one, two or more $R^B$: $C_{1-10}$ alkyl, $C_{3-10}$ cycloalkyl, $C_{3-10}$ cycloalkyl-$C_{1-10}$ alkyl, 3- to 10-membered heterocyclyl, 3- to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is N, $R^1$ is selected from the group consisting of $^1H$, $^2H$, hydroxyl, deuterated hydroxyl, and the following groups which are unsubstituted or substituted with one, two or more $R^B$: $C_{1-6}$ alkyl, $C_{3-6}$ cycloalkyl, $C_{3-6}$ cycloalkyl-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

when A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-10}$ alkyl, $C_{3-10}$ cycloalkyl, $C_{3-10}$ cycloalkyl-$C_{1-10}$ alkyl, $C_{1-10}$ alkyloxy, $C_{3-10}$ cycloalkyloxy, $C_{1-10}$ alkyloxy-$C_{1-10}$ alkyl, 3- to 10-membered heterocyclyl, 3- to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is C-$R^A$, $R^A$ and $R^1$ are the same or different and are independently selected from the group consisting of $^1H$, $^2H$, halogen, hydroxyl, sulfhydryl, deuterated hydroxyl, deuterated sulfhydryl, cyano, nitro, and the following groups which are unsubstituted or substituted with one, two or more $R^C$: $C_{1-6}$ alkyl, $C_{3-6}$ cycloalkyl, $C_{3-6}$ cycloalkyl-$C_{1-6}$ alkyl, $C_{1-6}$ alkyloxy, $C_{3-6}$ cycloalkyloxy, $C_{1-6}$ alkyloxy-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

or, $R^A$ and $R^1$, together with the atom to which they are attached, form a 3- to 8-membered ring structure which is unsubstituted or substituted with one, two or more $R^D$, for example, the 3-to 8-membered ring structure is a 3-, 4-, 5-, 6-, 7- or 8-membered ring structure;

z is selected from 0 or 1;

$R^2$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^2$ is selected from the group consisting of $^1H$, $^2H$, hydrogen, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy;

$R^3$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^3$ is selected from the group consisting of $^1H$, $^2H$, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-10}$ alkyl, $C_{3-6}$ cycloalkyloxy;

provided that, $R^1$, $R^2$ and the atoms to which they are attached, or $R^2$, $R^3$ and the atoms to which they are attached, at least one of these two sets of groups together form a 4- to 10-membered ring structure which is unsubstituted or substituted with one, two or more $R^E$, and the 4- to 10-membered ring structure can be selected from, for example, the group consisting of 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of $C_{1-10}$ alkyl, $C_{1-10}$ alkyloxy, halogen, hydroxyl, sulfhydryl, amino, cyano; preferably, $R^4$ is selected from the group consisting of $C_{1-6}$ alkyl, $C_{1-6}$ alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: $C_{1-10}$ alkyl, $C_{1-10}$ alkyloxy, $C_{6-10}$ aryl, $C_{6-10}$ arylalkyl, $C_{6-10}$ aryloxy, 5- to 6-membered heteroaryl, 5- to 6-membered heteroarylalkyl, 5- to 6-membered heteroaryloxy;

B is absent or selected from the group consisting of triazolyl, -$NR^7$- or -N($NR^7R^8$)-;

$R^7$ and $R^8$ are the same or different and are independently selected from the group consisting of $^1H$, $^2H$, and the following groups which are unsubstituted or substituted with one, two or more $R^H$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl, HC(=O)-, $C_{1-10}$ alkyl-C(=O)-, $C_{3-10}$ cycloalkyl-C(=O)NH-, 3- to 10-membered heterocyclyl-C(=O)NH-, $C_{6-10}$ aryl-C(=O)

NH-, 5- to 10-membered heteroaryl-C(=O)NH-; preferably, $R^7$ and $R^8$ are the same or different and are independently selected from the group consisting of hydrogen, and the following groups which are unsubstituted or substituted with one, two or more $R^H$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl, HC(=O)-, $C_{1-6}$ alkyl-C(=O)-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

$T^L$ is selected from the group consisting of chemical bond, #O-$(CR^9R^{10})_m$-$(CR^{11}R^{12})_n$-C(=O)*, #O-$(CR^9R^{10})_m$-$(CR^{11}R^{12})_n$*, #N($R^{13}$)-$(CR^9R^{10})_m$-$(CR^{11}R^{12})_n$-C(O)*, #S-$(CR^9R^{10})_m$-$(CR^{11}R^{12})_n$-C(O)*, #O-$(CR^9R^{10})_m$-$(CR^{11}R^{12})_n$-C($NR^{13}$)*, #O-$(CR^9R^{10})_m$-$(CR^{11}R^{12})_n$-P(=O)($OR^{14}$)*, *O-$(CR^9R^{10})_m$-$(CR^{11}R^{12})_n$-C(=O)#, *O-$(CR^9R^{10})_m$-$(CR^{11}R^{12})_n$#, *N($R^{13}$)-$(CR^9R^{10})_m$-$(CR^{11}R^{12})_n$-C(O)#, *S-$(CR^9R^{10})_m$-$(CR^{11}R^{12})_n$-C(O)#, *O-$(CR^9R^{10})_m$-$(CR^{11}R^{12})_n$-C($NR^{13}$)#, *O-$(CR^9R^{10})_m$-$(CR^{11}R^{12})_n$-P(=O)($OR^{14}$)#, wherein # represents the site attached to L, and * represents the site attached to B;

each of $R^9$, $R^{10}$, $R^{11}$ and $R^{12}$ is the same or different, and is independently selected from the group consisting of $^1H$, $^2H$, halogen, cyano, and the following groups which are unsubstituted or substituted with one, two or more $R^I$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy; preferably, each of $R^9$, $R^{10}$, $R^{11}$ and $R^{12}$ is the same or different and is independently selected from the group consisting of hydrogen, halogen, cyano, and the following groups which are unsubstituted or substituted with one, two or more $R^I$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-10}$ alkyl, 3- to 6-membered heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; wherein, any of the 3- to 10-membered ring structures can be selected from, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl; preferably, each $R^{13}$ is the same or different and is independently selected from the following groups which are unsubstituted or substituted with one, two or more $R^J$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl;

m and n are the same or different and are independently selected from an integer of 0 to 10, such as 0, 1, 2, 3, 4, 5, 6, 7, 8, 9 or 10;

each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, amino, nitro and the following groups which are unsubstituted or substituted with one, two or more $R^K$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each of $R^B$, $R^C$, $R^D$, $R^E$, $R^F$, $R^G$, $R^H$, $R^I$ and $R^J$ is the same or different, and is independently selected from the group consisting of deuterium, halogen, hydroxyl, cyano, nitro, and the following groups which are unsubstituted or substituted with one, two or more $R^K$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$:

$C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each $R^K$ is the same or different, and is independently selected from the group consisting of deuterium, halogen, hydroxyl, cyano, nitro, and the following groups which are unsubstituted or substituted with one, two or more $R^L$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^L$ is the same or different and is independently selected from the group consisting of deuterium, halogen, hydroxyl, cyano, nitro, $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-.

**6.** The conjugate, or a stereoisomer, racemate, tautomer, isotopologue, isotope-labeled compound, nitroxide or pharmaceutically acceptable salt thereof according to claim 1, wherein definitions of the groups in Formula (G) are independently selected from:

A is selected from N;

$R^1$ is selected from the group consisting of $^1H$, $^2H$, hydroxyl, deuterated hydroxyl, and the following groups which are unsubstituted or substituted with one, two or more $R^B$: $C_{1-10}$ alkyl, $C_{3-10}$ cycloalkyl, $C_{3-10}$ cycloalkyl-$C_{1-10}$ alkyl, 3- to 10-membered heterocyclyl, 3- to 10-membered heterocyclyl-$C_{1-10}$ alkyl, $C_{6-10}$ aryl, $C_{6-10}$ aryl-$C_{1-10}$ alkyl, 5- to 10-membered heteroaryl or 5- to 10-membered heteroaryl-$C_{1-10}$ alkyl, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, when A is N, $R^1$ is selected from the group consisting of $^1H$, $^2H$, hydroxyl, deuterated hydroxyl, and the following groups which are unsubstituted or substituted with one, two or more $R^B$: $C_{1-6}$ alkyl, $C_{3-6}$ cycloalkyl, $C_{3-6}$ cycloalkyl-$C_{1-6}$ alkyl, 3- to 6-membered heterocyclyl, 3- to 6-membered heterocyclyl-$C_{1-6}$ alkyl, phenyl, phenyl-$C_{1-6}$ alkyl, 5- to 6-membered heteroaryl or 5- to 6-membered heteroaryl-$C_{1-6}$ alkyl, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, phenyl-C(=O)NH-, 5- to 6-membered heteroaryl-C(=O)NH-;

z is selected from 0 or 1;

$R^2$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^2$ is selected from the group consisting of $^1H$, $^2H$, hydrogen, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy;

$R^3$ is selected from the group consisting of $^1H$, $^2H$, halogen, and the following groups which are unsubstituted or substituted with one, two or more $R^E$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy; preferably, $R^3$ is selected from the group consisting of $^1H$, $^2H$, halogen, and the following groups which are unsubstituted or substituted with one, two or more $R^E$: $C_{1-6}$ alkyl, $C_{1-6}$ alkyloxy, $C_{3-6}$ cycloalkyl, $C_{3-6}$ cycloalkyl-$C_{1-10}$ alkyl, $C_{3-6}$ cycloalkyloxy;

or, $R^1$ and $R^2$, together with the atoms to which they are attached, form a 5- to 10-membered ring structure which is unsubstituted or substituted with one, two or more $R^E$, and the 5- to 10-membered ring structure can be selected from, for example, the group consisting of 5-, 6-, 7-, 8-, 9- or 10-membered monocyclic hydrocarbyl, bicyclic hydrocarbyl, heteromonocyclic hydrocarbyl, heterobicyclic hydrocarbyl;

or, $R^2$ and $R^3$, together with the atoms to which they are attached, form a 4- to 10-membered ring structure which is unsubstituted or substituted with one, two or more $R^F$, and the 4- to 10-membered ring structure can be selected from, for example, the group consisting of 4, 5-, 6-, 7-, 8-, 9- or 10-membered monocyclic hydrocarbyl, bicyclic hydrocarbyl, heteromonocyclic hydrocarbyl, heterobicyclic hydrocarbyl;

$R^4$ is selected from the group consisting of $C_{1-10}$ alkyl, $C_{1-10}$ alkyloxy, halogen, hydroxyl, sulfhydryl, amino, cyano; preferably, $R^4$ is selected from the group consisting of $C_{1-6}$ alkyl, $C_{1-6}$ alkyloxy, halogen, hydroxyl, sulfhydryl, amino, cyano;

$R^5$ is selected from hydroxyl;

$R^6$ is selected from the following groups which are unsubstituted or substituted with one, two or more $R^G$: $C_{3-10}$ cycloalkyl, $C_{3-10}$ cycloalkyl-$C_{1-10}$ alkyl, $C_{3-10}$ cycloalkyloxy, 3- to 10-membered heterocyclyl, 3- to 10-membered heterocyclyl-$C_{1-10}$ alkyl, 3- to 10-membered heterocyclyloxy; preferably, $R^6$ is selected from the following groups which are unsubstituted or substituted with one, two or more $R^G$: $C_{3-6}$ cycloalkyl, $C_{3-6}$ cycloalkyl-$C_{1-6}$ alkyl, $C_{3-6}$ cycloalkyloxy, 3- to 6-membered heterocyclyl, 3- to 6-membered heterocyclyl-$C_{1-6}$ alkyl, 3- to 6-membered heterocyclyloxy;

B is selected from -$NR^7$- or -$N(NR^7R^8)$-;

$R^7$ and $R^8$ are the same or different and are independently selected from the group consisting of $^1H$, $^2H$, and the following groups which are unsubstituted or substituted with one, two or more $R^H$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl, HC(=O)-, $C_{1-10}$ alkyl-C(=O)-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, $R^7$ and $R^8$ are the same or different and are independently selected from the group consisting of hydrogen, and the following groups which are unsubstituted or substituted with one, two or more $R^H$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl, HC(=O)-, $C_{1-6}$ alkyl-C(=O)-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

$T^L$ is selected from the group consisting of chemical bond, #O-$(CR^9R^{10})_m$-$(CR^{11}R^{12})_n$-C(=O)*, #O-$(CR^9R^{10})_m$-$(CR^{11}R^{12})_n$*, #N($R^{13}$)-$(CR^9R^{10})_m$-$(CR^{11}R^{12})_n$-C(O)*, #S-$(CR^9R^{10})_m$-$(CR^{11}R^{12})_n$-C(O)*, #O-$(CR^9R^{10})_m$-$(CR^{11}R^{12})_n$-C($NR^{13}$)*, #O-$(CR^9R^{10})_m$-$(CR^{11}R^{12})_n$-P(=O)($OR^{14}$)*, *O-$(CR^9R^{10})_m$-$(CR^{11}R^{12})_n$-C(=O)#, *O-$(CR^9R^{10})_m$-$(CR^{11}R^{12})_n$#, *N($R^{13}$)-$(CR^9R^{10})_m$-$(CR^{11}R^{12})_n$-C(O)#, *S-$(CR^9R^{10})_m$-$(CR^{11}R^{12})_n$-C(O)#, *O-$(CR^9R^{10})_m$-$(CR^{11}R^{12})_n$-C($NR^{13}$)#, *O-$(CR^9R^{10})_m$-$(CR^{11}R^{12})_n$-P(=O)($OR^{14}$)#, wherein # represents the site attached to L, and * represents the site attached to B;

each of $R^9$, $R^{10}$, $R^{11}$ and $R^{12}$ is the same or different and is independently selected from the group consisting of $^1H$, $^2H$, halogen, cyano, and the following groups which are unsubstituted or substituted with one, two or more $R^I$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy; preferably, each of $R^9$, $R^{10}$, $R^{11}$ and $R^{12}$ is the same or different and is independently selected from the group consisting of hydrogen, halogen, cyano, and the following groups which are unsubstituted or substituted with one, two or more $R^I$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-10}$ alkyl, 3- to 6-membered heterocyclyloxy;

or, $R^9$ and $R^{10}$, together with the atoms to which they are attached, form a 3- to 10-membered ring structure which is unsubstituted or substituted with one, two or more $R^I$; or, $R^{11}$ and $R^{12}$, together with the atoms to which they are attached, form a 3- to 10-membered ring structure which is unsubstituted or substituted with one, two or more $R^I$; or, $R^9$ and $R^{11}$, together with the atoms to which they are attached, form a 3- to 10-membered ring structure which is unsubstituted or substituted with one, two or more $R^I$; any of the 3- to 10-membered ring structures can be selected from, for example, the following groups which are unsubstituted or substituted with one, two or more $R^I$: 3-, 4-, 5-, 6-, 7-, 8-, 9- or 10-membered monocyclic hydrocarbyl, bicyclic hydrocarbyl, heteromonocyclic hydrocarbyl, heterobicyclic hydrocarbyl;

each $R^{13}$ is the same or different and is independently selected from the following groups which are unsubstituted or substituted with one, two or more $R^J$: $C_{1-10}$ alkyl, $C_{3-10}$ cycloalkyl, $C_{3-10}$ cycloalkyl-$C_{1-10}$ alkyl, $C_{6-10}$ aryl, $C_{6-10}$ aryl-$C_{1-10}$ alkyl, 5- to 10-membered heteroaryl, 5- to 10-membered heteroaryl-$C_{1-10}$ alkyl, 3- to 10-membered heterocyclyl, 3- to 10-membered heterocyclyl-$C_{1-10}$ alkyl; preferably, each $R^{13}$ is the same or different and is independently selected from the following groups which are unsubstituted or substituted with one, two or more $R^J$: $C_{1-6}$ alkyl, $C_{3-6}$ cycloalkyl, $C_{3-6}$ cycloalkyl-$C_{1-6}$ alkyl, $C_{6-10}$ aryl, $C_{6-10}$ aryl-$C_{1-6}$ alkyl, 5- to 6-membered heteroaryl, 5- to 6-membered heteroaryl-$C_{1-6}$ alkyl, 3- to 6-membered heterocyclyl, 3- to 6-membered heterocyclyl-$C_{1-6}$ alkyl;

m and n are the same or different and are independently selected from an integer of 0 to 10, such as 0, 1, 2, 3, 4, 5, 6, 7, 8, 9 or 10;

each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-

membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each of $R^B$, $R^C$, $R^D$, $R^E$, $R^F$, $R^G$, $R^H$, $R^I$ and $R^J$ is the same or different and is independently selected from the group consisting of deuterium, halogen, hydroxyl, cyano, nitro, and the following groups which are unsubstituted or substituted with one, two or more $R^K$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^K$ is the same or different and is independently selected from the group consisting of deuterium, halogen, hydroxyl, cyano, nitro, and the following groups which are unsubstituted or substituted with one, two or more $R^L$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-; preferably, each $R^K$ is the same or different and is independently selected from the group consisting of deuterium, halogen, hydroxyl, cyano, nitro, and the following groups which are unsubstituted or substituted with one, two or more $R^L$: $C_{1-6}$ alkyl, $C_{1-6}$ alkyloxy, $C_{3-6}$ cycloalkyl, $C_{3-6}$ cycloalkyl-$C_{1-6}$ alkyl, $C_{3-6}$ cycloalkyloxy, $C_{6-10}$ aryl, $C_{6-10}$ aryl-$C_{1-6}$ alkyl, $C_{6-10}$ aryloxy, 5- to 6-membered heteroaryl, 5- to 6-membered heteroaryl-$C_{1-6}$ alkyl, 5- to 6-membered heteroaryloxy, 3- to 6-membered heterocyclyl, 3- to 6-membered heterocyclyl-$C_{1-6}$ alkyl, 3- to 6-membered heterocyclyloxy, $NH_2$, HC(=O)NH-, $C_{1-6}$ alkyl-C(=O)NH-, $C_{3-6}$ cycloalkyl-C(=O)NH-, 3- to 6-membered heterocyclyl-C(=O)NH-, $C_{6-10}$ aryl-C(=O)NH-, 5- to 6-membered heteroaryl-C(=O)NH-;

each $R^L$ is the same or different and is independently selected from the group consisting of deuterium, halogen, hydroxyl, cyano, nitro, $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy, $C_{6-10}$ aryl, $C_{6-10}$ aryl-$C_{1-10}$ alkyl, $C_{6-10}$ aryloxy, 5- to 10-membered heteroaryl, 5- to 10-membered heteroaryl-$C_{1-10}$ alkyl, 5- to 10-membered heteroaryloxy, 3- to 10-membered heterocyclyl, 3- to 10-membered heterocyclyl-$C_{1-10}$ alkyl, 3- to 10-membered heterocyclyloxy, $NH_2$, HC(=O)NH-, $C_{1-10}$ alkyl-C(=O)NH-, $C_{3-10}$ cycloalkyl-C(=O)NH-, 3- to 10-membered heterocyclyl-C(=O)NH-, $C_{6-10}$ aryl-C(=O)NH-, 5- to 10-membered heteroaryl-C(=O)NH-.

**7.** The conjugate, or a stereoisomer, racemate, tautomer, isotopologue, isotope-labeled compound, nitroxide or pharmaceutically acceptable salt thereof according to any one of claims 1 to 6, wherein:

$R^1$ and $R^2$, together with the quinolyl to which they are connected, form one of the following substructures, wherein the substructures may be unsubstituted or substituted with one, two or more $R^E$:

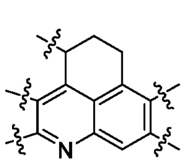, 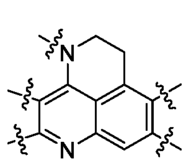, 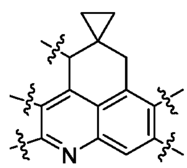, 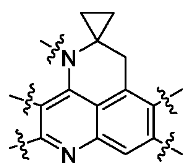, 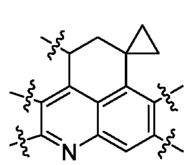,

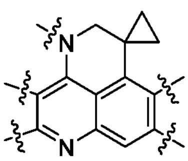, 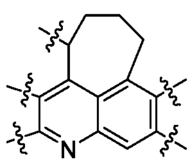, 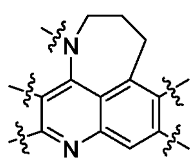, 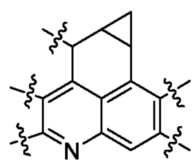, 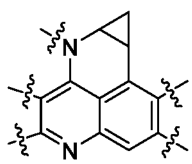;

or, $R^2$ and $R^3$, together with the quinolyl to which they are connected, form one of the following substructures, wherein the substructures may be unsubstituted or substituted with one, two or more $R^F$:

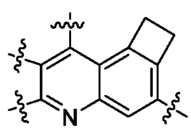, 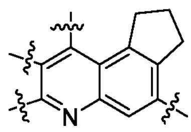, 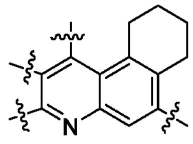;

or preferably, B is absent or selected from the group consisting of

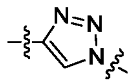, 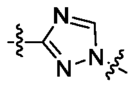, -NH- or -N($NR^7R^8$)-, wherein $R^7$ and $R^8$ independently have the definitions as described in any one of claims 1 to 6.

**8.** The conjugate, or a stereoisomer, racemate, tautomer, isotopologue, isotope-labeled compound, nitroxide or pharmaceutically acceptable salt thereof according to any one of claims 1 to 7, wherein G is selected from the group represented by the following Formula (G-1):

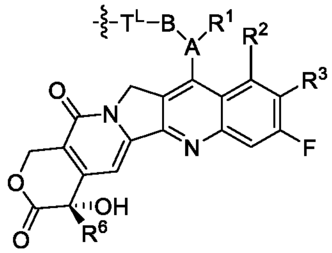

(G-1)

wherein, A, B, $T^L$, $R^1$, $R^2$, $R^3$, and $R^6$ independently have the definitions as described in any one of claims 1 to 7.

**9.** The conjugate, or a stereoisomer, racemate, tautomer, isotopologue, isotope-labeled compound, nitroxide or pharmaceutically acceptable salt thereof according to any one of claims 1 to 7, wherein G is selected from the group represented by Formula (G-2) or (G-3):

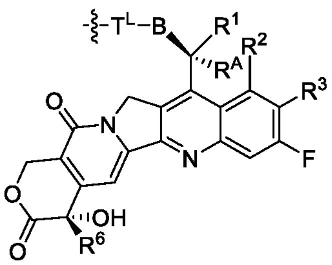

(G-2)

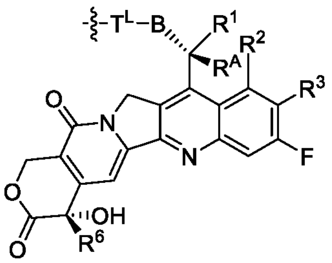

(G-3)

wherein, $R^A$, B, $T^L$, $R^1$, $R^2$, $R^3$, and $R^6$ have the definitions as described in any one of claims 1 to 7.

**10.** The conjugate, or a stereoisomer, racemate, tautomer, isotopologue, isotope-labeled compound, nitroxide or

pharmaceutically acceptable salt thereof according to any one of claims 1 to 7, wherein G is selected from the following group or the group formed by connecting -$T^L$- with the following group, at the wavy line of the following group:

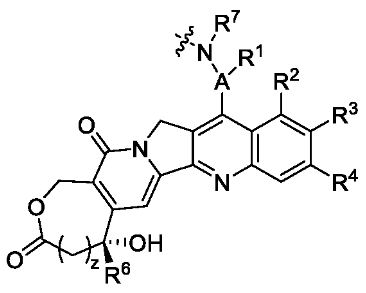

wherein, A, $R^1$, $R^2$, $R^3$, $R^4$, $R^6$, $R^7$, and z have the definitions as described in any one of claims 1 to 7.

**11.** The conjugate, or a stereoisomer, racemate, tautomer, isotopologue, isotope-labeled compound, nitroxide or pharmaceutically acceptable salt thereof according to any one of claims 1 to 7, wherein G is selected from the following group or the group formed by connecting -$T^L$- with the following group, at the wavy line of the following group:

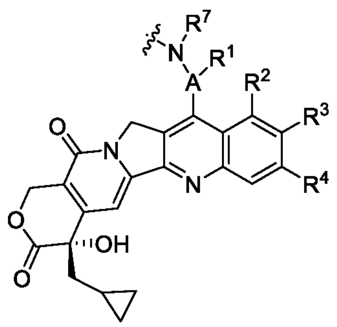

wherein, A, $R^1$, $R^2$, $R^3$, $R^4$, and $R^7$ have the definitions as described in any one of claims 1 to 7.

**12.** The conjugate, or a stereoisomer, racemate, tautomer, isotopologue, isotope-labeled compound, nitroxide or pharmaceutically acceptable salt thereof according to any one of claims 1 to 7, wherein G is selected from the following group or the group formed by connecting -$T^L$- with the following group, at the wavy line of the following group:

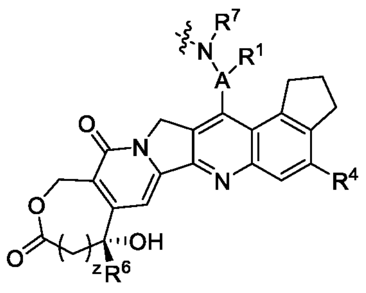

wherein, A, $R^1$, $R^4$, $R^6$, $R^7$, and z have the definitions as described in any one of claims 1 to 7.

**13.** The conjugate, or a stereoisomer, racemate, tautomer, isotopologue, isotope-labeled compound, nitroxide or pharmaceutically acceptable salt thereof according to any one of claims 1 to 7, wherein G is selected from the following groups or the groups formed by connecting -$T^L$- with the following group, at the wavy line of the following groups:

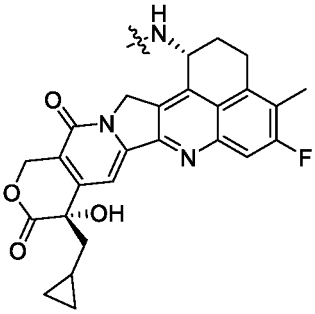

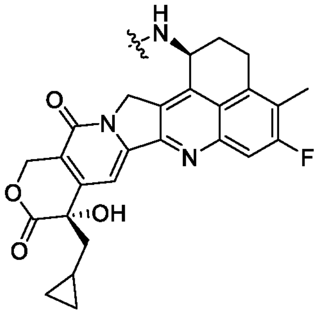

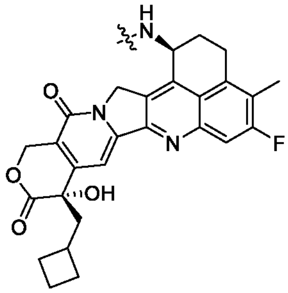

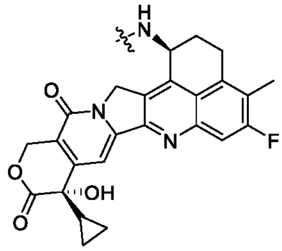

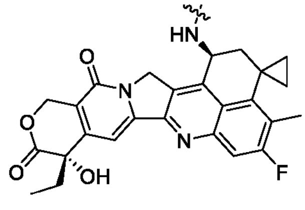

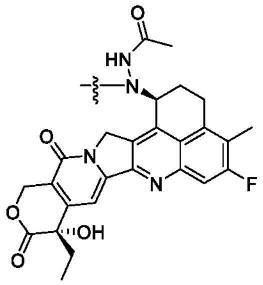

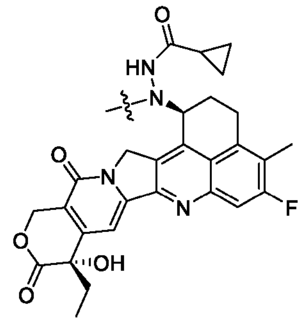

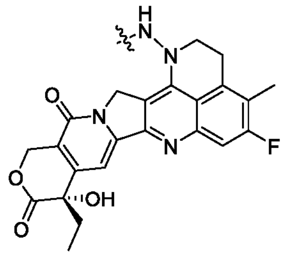

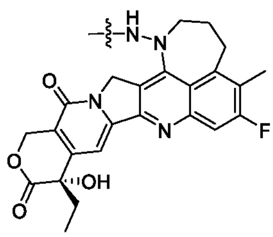

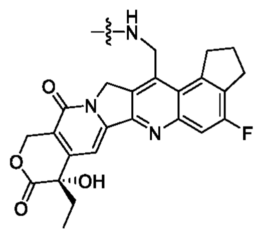

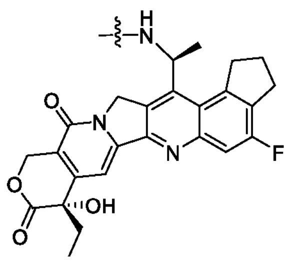

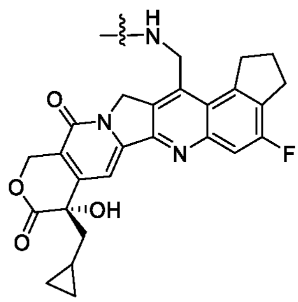

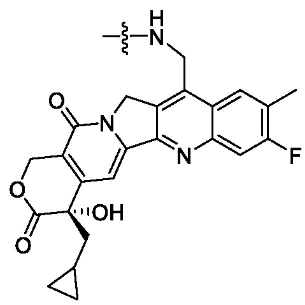

**14.** The conjugate, or a stereoisomer, racemate, tautomer, isotopologue, isotope-labeled compound, nitroxide or pharmaceutically acceptable salt thereof according to any one of claims 1 to 7, wherein L is selected from a chemical bond or a linker represented by the Formula (L) as defined below:

#$L^1$-$L^2$-$L^3$-$L^4$-$L^5$* (L)

wherein, $L^1$ is a linking moiety to the targeting moiety Tp, formed from a reactive group $L^{1'}$ through its reaction with the targeting moiety Tp, and # represents the site attached to the targeting moiety Tp;
for example, $L^{1'}$ is a maleimide group, then $L^1$ is the following structure:

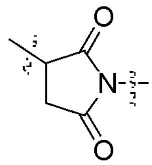

or its ring-opening form

: 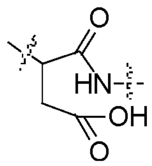 or 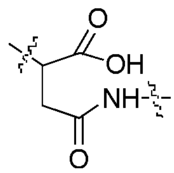 ;

$L^2$ is absent or a spacer between $L^1$ and $L^3$;
$L^3$ is a peptide moiety;
$L^4$ is absent or a spacer between the peptide moiety and $L^5$
$L^5$ is a linking moiety between $L^4$ and a bioactive molecule G, formed from a reactive group $L^{5'}$ through its reaction with the bioactive molecule G or intermediates thereof, and * represents the site attached to the bioactive molecule G;
optionally:
the following hydrophilic moiety or steric hindrance moiety is inserted between the above moieties of L, preferably between $L^1$ and $L^2$, or between $L^2$ and $L^3$, or is inserted by replacing $L^2$:

hydrophilic moiety:

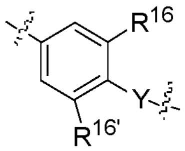 or 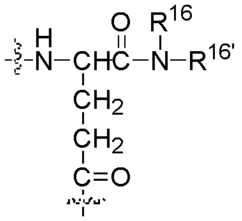 or 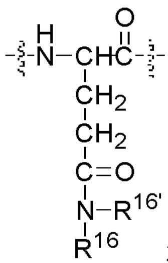 ;

steric hindrance moiety:

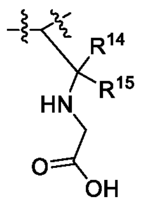 ;

$R^{16}$ and $R^{16'}$ are the same or different, and at least one of them is selected from a hydrophilic group, and the other is selected from the following substituents: hydrogen, halogen, cyano, amino, nitro, and the following groups which are unsubstituted or substituted with one, two or more $R^{zg}$: $C_{1-10}$ alkyl, $C_{1-10}$ alkyloxy, $C_{3-10}$

cycloalkyl, $C_{3-10}$ cycloalkyl-$C_{1-10}$ alkyl, $C_{3-10}$ cycloalkyloxy, aminocarbonyl;
the hydrophilic group is selected from a polyethylene glycol group, a $C_{1-10}$ alkyl substituted with 1 to 10 hydroxyl groups, or a sugar ring-containing group, or $C_{1-6}$ alkyl-NHCO- (e.g., $C_{1-4}$ alkyl-NHCO-), preferably a polyethylene glycol group, more preferably -C(=O)-NH-$(CH_2CH_2O)_p$-$C_{1-10}$ alkyl or -NH-$(CH_2CH_2O)_p$-$C_{1-10}$ alkyl, or preferably a $C_{1-10}$ alkyl substituted with 1 to 10 hydroxyl groups, more preferably

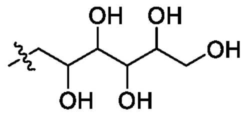

;

each p is the same or different, and is independently selected from an integer of 0 to 10, preferably 1, 2, 3, 4, 5, 6, 7 or 8;
Y is selected from the group consisting of O, S, $C_{1-10}$ alkylene, wherein 1, 2 or 3 of the methylene groups of the alkylene can be optionally replaced by O or S;
$R^{14}$ and $R^{15}$ are the same or different and are independently selected from the group consisting of hydrogen, halogen, cyano, amino, nitro, and the following groups which are unsubstituted or substituted with one, two or more $R^{zh}$: $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy;
or, $R^{14}$ and $R^{15}$, together with the atom to which they are attached, form a $C_{3-10}$ cycloalkyl which is unsubstituted or substituted with one, two or more $R^{zh}$;
each of $R^{zg}$ and $R^{zh}$ is the same or different and is independently selected from the group consisting of halogen, hydroxyl, amino, cyano, nitro, $C_{1-10}$ alkyl, $C_{1-10}$ alkyloxy, $C_{3-10}$ cycloalkyl, $C_{3-10}$ cycloalkyl-$C_{1-10}$ alkyl, $C_{3-10}$ cycloalkyloxy;
the hydrophilic moiety or steric hindrance moiety as defined above is present or absent.

**15.** The conjugate, or a stereoisomer, racemate, tautomer, isotopologue, isotope-labeled compound, nitroxide or pharmaceutically acceptable salt thereof according to claim 14, wherein

$L^1$ is formed from any of $L^{1'}$ through its reaction with the targeting moiety Tp, $L^{1'}$ is preferably a sulfhydryl-reactive group, an amine-reactive group, a carboxyl-reactive group, a proline residue-reactive group, a tyrosine residue-reactive group, a disulfide bridge-containing group, etc.; for an antibody introduced with a non-natural amino acid, it can also be selected from reactive groups in click chemistry such as ketone, azide, alkyne, cyclopropene or diene;
$L^{1'}$ is preferably a sulfhydryl-reactive group;
$L^{1'}$ is further preferably a maleimide group or a substituted maleimide group, and $L^1$-$L^2$ preferably has the following structure:

a fragment that is prepared from (N-maleimidomethyl)-carboxylic acid-N-hydroxysuccinimide ester, and has the structure as follows:

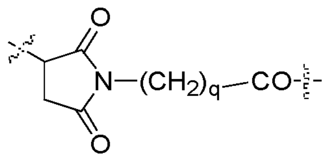

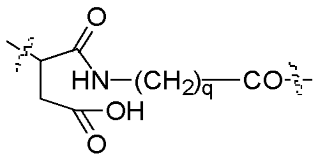

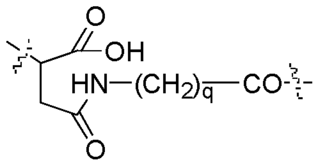

(q is an integer of 0 to 10, preferably 1, 2, 3, 4, 5, 6, 7 or 8);

or a fragment that is prepared from (meta)-maleimidobenzoic acid N-hydroxysuccinimide ester (MBS), and has the structure as follows:

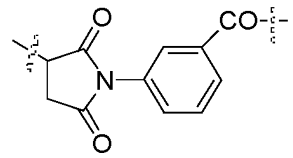

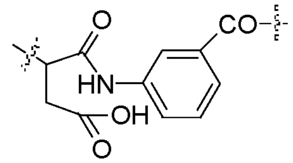

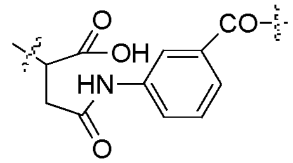

or a fragment that is prepared from 4-(N-maleimidomethyl)-cyclohexane-1-carboxylic acid N-hydroxysuccinimide ester (SMCC), and has the structure as follows:

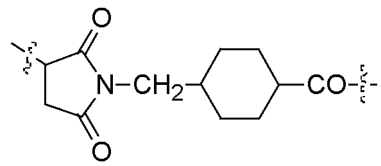

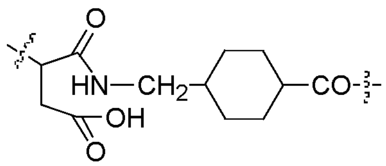

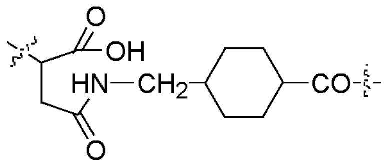

or $L^{1'}$ is preferably a sulfhydryl-reactive group having the following structure:

Hal-Het-

Hal is selected from the group consisting of halogen, OMs, OTs, OTf, nitro, and the following groups which are optionally substituted with one or more $R^{z6}$: alkyl thioether group, aryl thioether group, heteroaryl thioether group, alkyl sulfoxide group, aryl sulfoxide group, heteroaryl sulfoxide group, alkyl sulfonyl group, aryl sulfonyl group, heteroaryl sulfonyl group; wherein $R^{z6}$ is independently selected from the group consisting of H (hydrogen), D (deuterium), halogen, CN, nitro, $C_{1-6}$ alkyl, halogenated $C_{1-6}$ alkyl, $C_{1-6}$ alkoxy, 6- to 10-membered aryl and 5- to 12-membered heteroaryl;

Het is selected from 5- to 10-membered heteroaryl which is optionally substituted with one or more $R^{z7}$; wherein $R^{z7}$ is independently selected from the group consisting of H (hydrogen), D (deuterium), halogen, CN, nitro, $C_{1-4}$ alkyl and halogenated $C_{1-4}$ alkyl;

in a preferred embodiment, Hal is preferably mesyl, and Het is preferably pyrimidinyl;

in a preferred embodiment, Hal-Het- is:

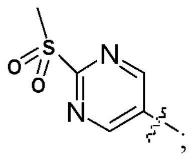

the corresponding $L^1$ has the structure as follows:

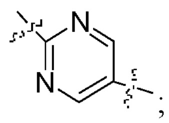

and $L^{1'}$-$L^2$ preferably has the structure as follows:

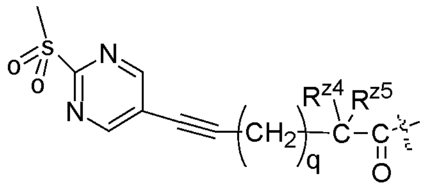

q is an integer selected from 0 to 10, preferably 1, 2, 3, 4, 5, 6, 7 or 8;
$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl;
further preferably has the structure as follows:

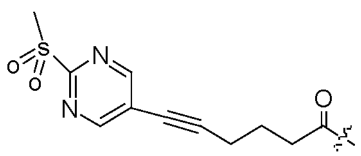 or 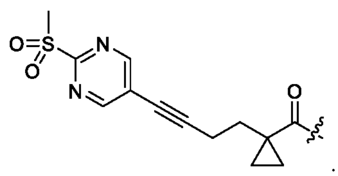.

**16.** The conjugate, or a stereoisomer, racemate, tautomer, isotopologue, isotope-labeled compound, nitroxide or pharmaceutically acceptable salt thereof according to claim 14, wherein

$L^2$ is absent or selected from the group consisting of $C_{1-10}$ alkylene, $C_{2-10}$ alkenylene, $C_{2-10}$ alkynylene, $C_{3-10}$ cycloalkyl, $C_{6-12}$ aryl or 6- to 12-membered heteroaryl or a combination of the above fragments, and is optionally interrupted by a carbonyl group, O, S, or N, and optionally substituted with $C_{1-6}$ alkyl, $C_{3-6}$ cycloalkyl, halogen, halogenated $C_{1-6}$ alkyl, and optionally, the alkyl or halogenated alkyl, together with the C atom to which it is attached, forms a $C_{3-6}$ cycloalkyl, $L^2$ is connected to $L^1$ or $L^3$ through any functional group or covalent bond;
preferably, $L^2$ is connected to the N-terminal of the peptide moiety through -C($R^{z4}R^{z5}$)-CO-;
preferably, $R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl;
further preferably, $L^2$ is $-(CH_2)_q-$, $-(CH_2)_q-C(=O)-$, or $-(C\equiv C)-(CH_2)_q-C(R^{z4}R^{z5})-C(=O)-$, wherein q is an integer of 0 to 10, preferably 1, 2, 3, 4, 5, 6, 7 or 8;
preferably, $R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl.

**17.** The conjugate, or a stereoisomer, racemate, tautomer, isotopologue, isotope-labeled compound, nitroxide or pharmaceutically acceptable salt thereof according to claim 14, wherein

$L^3$ is selected from a divalent peptide group comprising 2 to 8 optionally substituted natural amino acid residues or optionally substituted non-natural amino acid residues, each of the amino acid residues is the same or different and is independently selected from the following amino acid residues: alanine (Ala), cysteine (Cys), aspartic acid

(Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), selenocysteine (Sec), pyrrolysine (Pyl), homoserine, homocysteine, demethylpyrrolysine.
further preferably, $L^3$ is selected from a divalent peptide group composed of 2, 3, 4, 5 or 6 optionally substituted natural amino acid residues or optionally substituted non-natural amino acid residues, each of the amino acid residues is the same or different and is independently selected from the following amino acid residues: alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), selenocysteine (Sec), pyrrolysine (Pyl), homoserine, homocysteine, demethylpyrrolysine; for example, -ValCit-; -CitVal-; -AlaAla-; -AlaCit-; -CitAla-; -AsnCit-; - CitAsn-; -CitCit-; -ValGlu-; -GluVal-; -SerCit-; -CitSer-; -LysCit-; -CitLys-; -AspCit-; -CitAsp-; - AlaVal-; -ValAla-; -PheAla-; -AlaPhe-; -PheLys-; -LysPhe-; -ValLys-; -LysVal-; -AlaLys-; - LysAla-; -PheCit-; -CitPhe-; -LeuCit-; -CitLeu-; -IleCit-; -CitIle-; -PheArg-; -ArgPhe-; -CitTrp-; - TrpCit-; -PhePheLys-; -LysPhePhe-; -DPhePheLys-; -DLysPhePhe-; -GlyPheLys-; -LysPheGly-; -GlyPheLeuGly-; -GlyLeuPheGly-; -AlaLeuAlaLeu-; -GlyGlyGly-; -GlyGlyGlyGly-; - GlyPheValGly-; -GlyValPheGly-; -GlyGlyPheGly-; -AlaAlaAla-; most preferably, $L^3$ is - GlyGlyPheGly-.

**18.** The conjugate, or a stereoisomer, racemate, tautomer, isotopologue, isotope-labeled compound, nitroxide or pharmaceutically acceptable salt thereof according to claim 14, wherein

$L^4$ is preferably a group with self-cleaving properties, and a self-cleaving group or self-immolative group is a group that initiates drug release through an intramolecular reaction such as 1,4-elimination, 1,6-elimination or cyclization elimination independent of the action of an enzyme;
$L^5$ is formed from any reactive group $L^{5'}$ through its reaction with a bioactive molecule or intermediate thereof, wherein $L^{5'}$ is preferably a carboxylic acid group or an active ester group, which reacts with OH, SH or NH or $NH_2$ in the bioactive molecule to form $L^5$ with the structure of: -C(O)O-, -C(O)S-, -C(O)N- or -C(O)NH- (including the atom of O, S or N in the bioactive molecule);
$L^4$-$L^5$ is preferably:

a PABC spacer arm with the structure as follows:

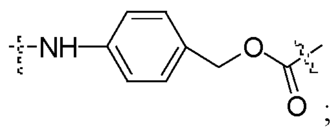
;

a GABA spacer arm with the structure as follows:

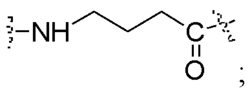
;

an α,α-dimethyl GABA spacer arm with the structure as follows:

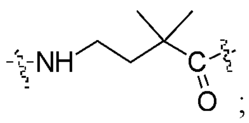
;

or a β,β-dimethyl GABA spacer arm with the structure as follows:

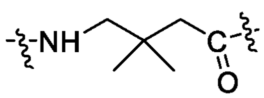
;

most preferably, $L^4$-$L^5$ is: $-NR^{z1}-C(R^{z2}R^{z3})-T^L-$;
wherein:

$R^{z1}$ is H or $C_{1-4}$ alkyl;
$R^{z2}$ and $R^{z3}$ are the same or different and are independently selected from H or $C_{1-4}$ alkyl;
$T^L$ has the definition as described in any one of claims 1 to 7, and one of $T^L$ in $L^4$-$L^5$ or G is a chemical bond.

**19.** The conjugate, or a stereoisomer, racemate, tautomer, isotopologue, isotope-labeled compound, nitroxide or pharmaceutically acceptable salt thereof according to any one of claims 14 to 18, wherein

$L^1$ is generated by coupling Tp with a sulfhydryl-reactive group contained in maleimide group, a substituted maleimide group or Hal-Het-;
the following hydrophilic moiety is inserted between $L^1$ and $L^2$, or between $L^2$ and $L^3$, or is inserted by replacing $L^2$:
hydrophilic moiety:

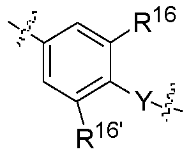 or 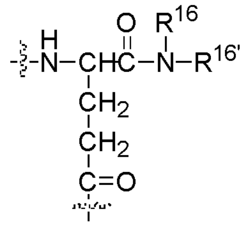 or 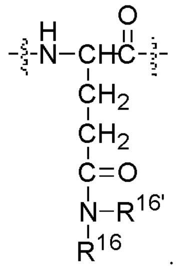 .

**20.** The conjugate, or a stereoisomer, racemate, tautomer, isotopologue, isotope-labeled compound, nitroxide or pharmaceutically acceptable salt thereof according to any one of claims 14 to 18, wherein

$L^1$ is generated by coupling Tp with a sulfhydryl-reactive group contained in maleimide group or a substituted maleimide group;
the following steric hindrance moiety is inserted between $L^1$ and $L^2$, or between $L^2$ and $L^3$, or is inserted by replacing $L^2$:
steric hindrance moiety:

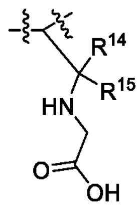 .

**21.** The conjugate, or a stereoisomer, racemate, tautomer, isotopologue, isotope-labeled compound, nitroxide or pharmaceutically acceptable salt thereof according to any one of claims 14 to 18, wherein

$L^1$ is generated by coupling Tp with a sulfhydryl-reactive group contained in Hal-Het-;
$L^4$-$L^5$ is: $-NR^{z1}-C(R^{z2}R^{z3})-T^L-$;
wherein:

$R^{z1}$ is H or $C_{1-4}$ alkyl;
$R^{z2}$ and $R^{z3}$ are the same or different and are independently selected from H or $C_{1-4}$ alkyl;
$T^L$ has the definition as described in any one of claims 1 to 13, and one of $T^L$ in $L^4$-$L^5$ or G is a chemical bond;
further preferably:

when $T^L$ in G is a chemical bond, $L^4$-$L^5$ is: $-NR^{z1}-C(R^{z2}R^{z3})-O-(CH_2)_m-C(R^{z4}R^{z5})-CO-$;
wherein:

$R^{z1}$ is H or $C_{1-4}$ alkyl;
m is an integer of 0 to 4;
$R^{z2}$ and $R^{z3}$ are the same or different and are independently selected from H or $C_{1-4}$ alkyl;
$R^{z4}$ and $R^{z5}$ are the same or different and are independently selected from the group consisting of H,

$C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl.

**22.** The conjugate, or a stereoisomer, racemate, tautomer, isotopologue, isotope-labeled compound, nitroxide or pharmaceutically acceptable salt thereof according to any one of claims 14 to 18, wherein

$L^1$-$L^2$ is generated by coupling Tp with $L^{1'}$-$L^2$ of the following structure:

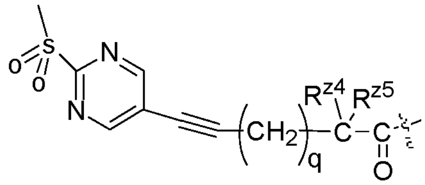

q is an integer selected from 0 to 10, preferably 1, 2, 3, 4, 5, 6, 7 or 8;
$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl, wherein at least one of $R^{z4}$ and $R^{z5}$ is not H;
further preferably, $R^{z4}$ and $R^{z5}$ together forms a $C_{3-6}$ cycloalkyl;
$L^1$-$L^2$ is most preferably generated by coupling Tp with

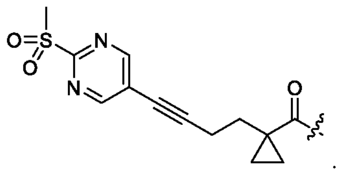

**23.** The conjugate, or a stereoisomer, racemate, tautomer, isotopologue, isotope-labeled compound, nitroxide or pharmaceutically acceptable salt thereof according to any one of claims 1 to 22,

Tp is a targeting moiety selected from the group consisting of small molecule ligands, proteins, polypeptides, non-protein agents (e.g., sugar, RNA or DNA);
preferably, the target of Tp is selected from the group consisting of epidermal growth factor, Trop-2, CD37, HER2, CD70, EGFRvIII, Mesothelin, Folate receptor1, Mucin 1, CD138, CD20, CD19, CD30, SLTRK6, Nectin 4, Tissue factor, Mucin16, Endothelin receptor, STEAP1, SLC39A6, Guanylylcyclase C, PSMA, CCD79b, CD22, Sodium phosphate cotransporter 2B, GPNMB, Trophoblast glycoprotein, AGS-16, EGFR, CD33, CD66e, CD74, CD56, PD-L1, TACSTD2, DR5, E16, 0772P, MPF, Napi3b, Sema 5b, PSCA hlg, ETBR, MSG783, STEAP2, TrpM4, CRIPTO, CD21, CD79b, FcRH2, NCA, MDP, IL20R$\alpha$, Brevican, E phB2R, ASLG659, PSCA, GEDA, BAFF-R, CD79a, CXCR5, HLA-DOB, P2X5, CD72, LY64, FcRH1, IRTA2, TENB2, integrin $\alpha5\beta6$, $\alpha4\beta7$, FGF2, FGFR2, HER3, CA6, DLL3, DLL4, P-cadherin, EpCAM, pCAD, CD223, LYPD3, LY6E, EFNA4, ROR1, SLITRK6, 5T4, ENPP3, Claudin18.2, BMPR1B, Tyro7, c-Met, ApoE, CD1 lc, CD40, CD45(PTPRC), CD49D(ITGA4), CD80, CSF1R, CTSD, GZMB, Ly86, MS4A7, PIK3AP1, PIK3CD, CCR5, IFNG, IL10RA1, IL-6, ACTA2, COL7A1, LOX, LRRC15, MCPT8, MMP10, NOG, SERPINEl, STAT1, TGFBR1, CTSS, PGF, VEGFA, C1QA, C1QB, ANGPTL4, EGLN, EGLN3, BNIP3, AIF1, CCL5, CXCL10, CXCL11, IFI6, PLOD2, KISS1R, STC2, DDIT4, PFKFB3, PGK1, PDK1, AKR1C1, AKR1C2, CADM1, CDH11, COL6A3, CTGF, HMOX1, KRT33A, LUM, WNT5A, IGFBP3, MMP14, CDCP1, PDGFRA, TCF4, TGF, TGFB1, TGFB2, CDllb, ADGRE1, EMR2, TNFRSF21, UPK1B, TNFSF9, MMP16, MFI2, IGF-1R, RNF43, NaPi2b and BCMA;
preferably, Tp is a small molecule ligand, such as a folic acid derivative, a glutamate urea derivative, a somatostatin derivative, an arylsulfonamide derivative (e.g., a carbonic anhydrase IX inhibitor), an ICG dye, a cyanine dye or a derivative thereof;
preferably, Tp is selected from an antibody or antigen-binding fragment thereof, and the antibody is selected from the group consisting of chimeric antibody, humanized antibody or fully human antibody; preferably monoclonal antibody;
preferably, the antibody or antigen-binding fragment thereof is at least one selected from the following antibodies or antigen-binding fragments: anti-CD20 antibody, anti-CD22 antibody, anti-CD30 antibody, anti-CD33 antibody, anti-CD44 antibody, anti-CD56 antibody, anti-CD70 antibody, anti-CD73 antibody, anti-CD105 antibody, anti-CEA antibody, anti-A33 antibody, anti-Cripto antibody, anti-EphA2 antibody, anti-G250 antibody, anti-HER2

(ErbB2) antibody, anti-EGFR antibody, anti-B7-H3 antibody, anti-c-Met antibody, anti-HER3 (ErbB3) antibody, anti-HER4 (ErbB4) antibody, anti-MUCl antibody, anti-Lewis Y antibody, anti-VEGFR antibody, anti-GPNMB antibody, anti-Integrin antibody, anti-PSMA antibody, anti-Tenascin-C antibody, anti-SLC44A4 antibody or anti-Mesothelin antibody, the antibody can be a bispecific antibody or a multispecific antibody;
further preferably, the antibody or antigen-binding fragment thereof is at least one selected from the following antibodies or antigen-binding fragments: Trastuzumab, Pertuzumab, Nimotuzumab, Enoblituzumab, Emibetuzumab, Inotuzumab, Pinatuzumab, Brentuximab, Gemtuzumab, Bivatuzumab, Lorvotuzumab, cBR 96, and Glematumamab or Glembatumumab;
for example, Trastuzumab has sequences selected from the following:

light chain

DIQMTQSPSSLSASVGDRVTITCRASQDVNTAVAWYQQKPGKAPKLLIYSASFLYSG
VPSRFSGSRSGTDFTLTISSLQPEDFATYYCQQHYTTPPTFGQGTKVEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC

SEQ ID NO:1

heavy chain

EVQLVESGGGLVQPGGSLRLSCAASGFNIKDTYIHWVRQAPGKGLEWVARIYPTNG
YTRYADSVKGRFTISADTSKNTAYLQMNSLRAEDTAVYYCSRWGGDGFYAMDYWGQ
GTLVTVSSASTKGPSVFPLAPSSKSTSGGTAALGCLVKDYFPEPVTVSWNSGALTSGVHT
FPAVLQSSGLYSLSSVVTVPSSSLGTQTYICNVNHKPSNTKVDKKVEPKSCDKTHTCPPC
PAPELLGGPSVFLFPPKPKDTLMISRTPEVTCVVVDVSHEDPEVKFNWYVDGVEVHNAK
TKPREEQYNSTYRVVSVLTVLHQDWLNGKEYKCKVSNKALPAPIEKTISKAKGQPREPQ
VYTLPPSREEMTKNQVSLTCLVKGFYPSDIAVEWESNGQPENNYKTTPPVLDSDGSFFLY
SKLTVDKSRWQQGNVFSCSVMHEALHNHYTQKSLSLSPGK

SEQ ID NO:2

Pertuzumab has sequences selected from the following:

light chain

DIQMTQSPSSLSASVGDRVTITCKASQDVSIGVAWYQQKPGKAPKLLIYSASYRYTG
VPSRFSGSGSGTDFTLTISSLQPEDFATYYCQQYYIYPYTFGQGTKVEIKRTVAAPSVFIFP
PSDEQLKSGTASVVCLLNNFYPREAKVQWKVDNALQSGNSQESVTEQDSKDSTYSLSST
LTLSKADYEKHKVYACEVTHQGLSSPVTKSFNRGEC

SEQ ID NO.3

heavy chain

EVQLVESGGGLVQPGGSLRLSCAASGFTFTDYTMDWVRQAPGKGLEWVADVNPNSGGSIYNQRFKGRFTLSVDRSKNTLYLQMNSLRAEDTAVYYCARNLGPSFYFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO.4

Nimotuzumab has sequences selected from the following:

light chain

DIQMTQSPSSLSASVGDRVTITCRSSQNIVHSNGNTYLDWYQQTPGKAPKLLIYKVSNRFSGVPSRFSGSGSGTDFTFTISSLQPEDIATYYCFQYSHVPWTFGQGTKLQITREVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

SEQ ID NO:5

heavy chain

QVQLQQSGAEVKKPGSSVKVSCKASGYTFTNYYIYWVRQAPGQGLEWIGGINPTSGGSNFNEKFKTRVTITVDESTNTAYMELSSLRSEDTAFYFCARQGLWFDSDGRGFDFWGQGSTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK

SEQ ID NO:6

Patritumab has following sequences:

light chain

DIEMTQSPDSLAVSLGERATINCRSSQSVLYSSSNRNYLAWYQQNPGQPPKLLIYWASTRESGVPDRFSGSGSGTDFTLTISSLQAEDVAVYYCQQYYSTPRTFGQGTKVEIKRTVA

APSVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC

SEQ ID NO:7

heavy chain

QVQLQQWGAGLLKPSETLSLTCAVYGGSFSGYYWSWIRQPPGKGLEWIGEINHSGS TNYNPSLKSRVTISVETSKNQFSLKLSSVTAADTAVYYCARDKWTWYFDLWGRGTLVT VSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKRVEPKSCDKTHTCPPCPAPELL GGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPP SREEMTKNQVSLTCLVKGFYPSDIAVEWESNGQPENNYKTTPPVLDSDGSFFLYSKLTV DKSRWQQGNVFSCSVMHEALHNHYTQKSLSLSPGK

SEQ ID NO:8.

**24.** The conjugate, or a stereoisomer, racemate, tautomer, isotopologue, isotope-labeled compound, nitroxide or pharmaceutically acceptable salt thereof according to any one of claims 1 to 23, wherein the conjugate, or linker or linker-drug thereof is selected from one of the following:

wherein, u, v, w are independently selected from an integer of 0 to 10 (e.g., 0, 1, 2, 3, 4, 5, 6, 7, 8, 9 or 10), G has the definition as described in any one of claims 1 to 23, LG has the definition of Tp of any one of claims 1 to 23; $R^{20}$, $R^{20'}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{20}$, $R^{20'}$ and the carbon atom to which they are attached together form a $C_{3-6}$ cycloalkyl;

for example, the $C_{1-4}$ alkyl can be independently selected from the group consisting of methyl, ethyl, propyl, isopropyl, butyl, isobutyl or *tert*-butyl; the $C_{3-6}$ cycloalkyl can be independently selected from the group consisting of cyclopropyl, cyclobutyl, cyclopentyl or cyclohexyl;

for example, both of $R^{20}$ and $R^{20'}$ are H, or one of them is not H, or both of them are not H:

| No. | Conjugate, or linker or linker-drug thereof |
|---|---|
| 1 | 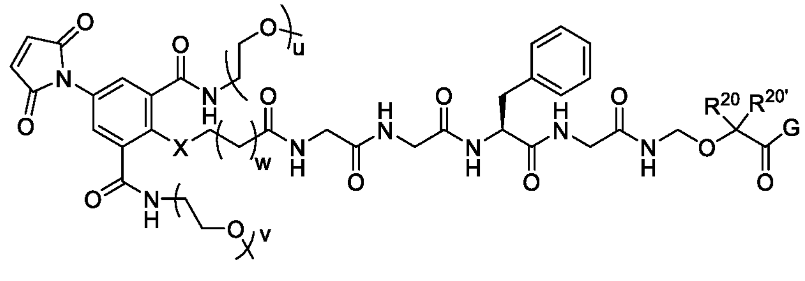 |
| 2 | 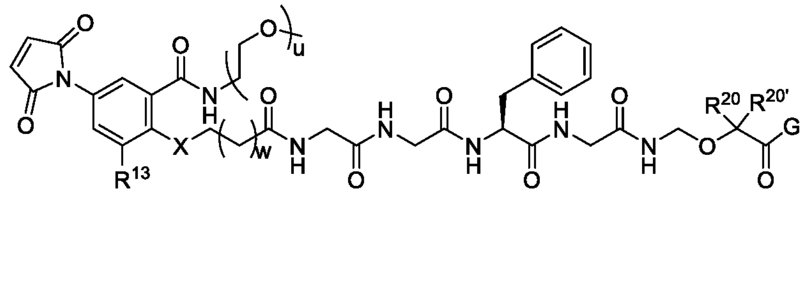 |

(continued)

| No. | Conjugate, or linker or linker-drug thereof |
| --- | --- |
| 3 | 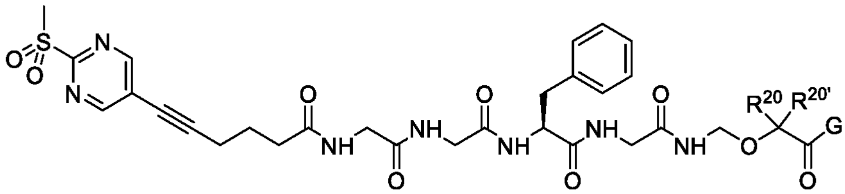 |
| 4 | 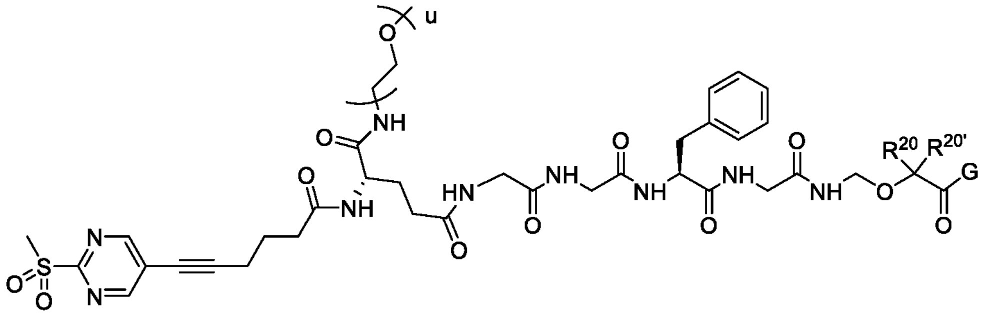 |
| 5 | 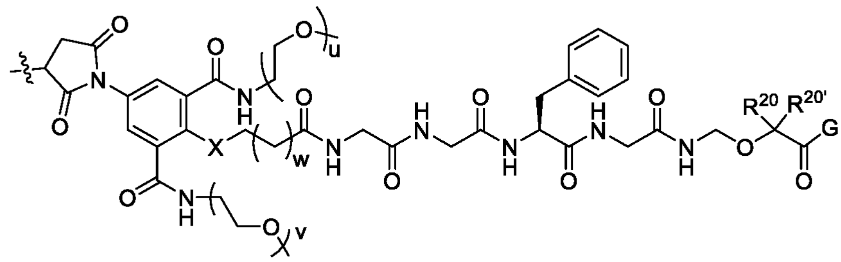 |
| 6 | 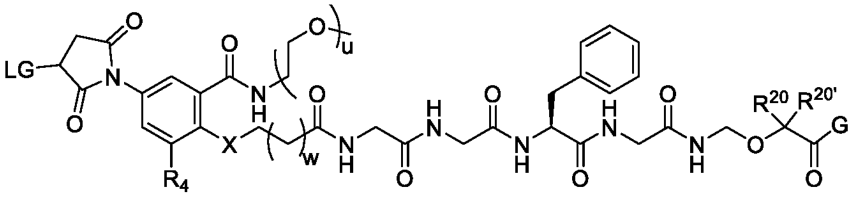 |
| 7 | 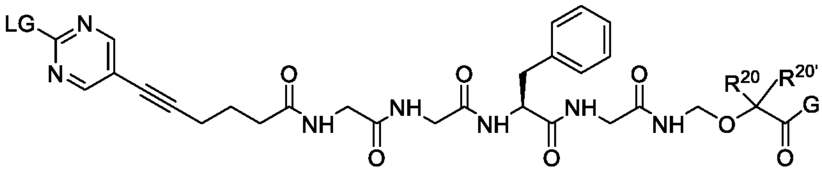 |
| 8 | 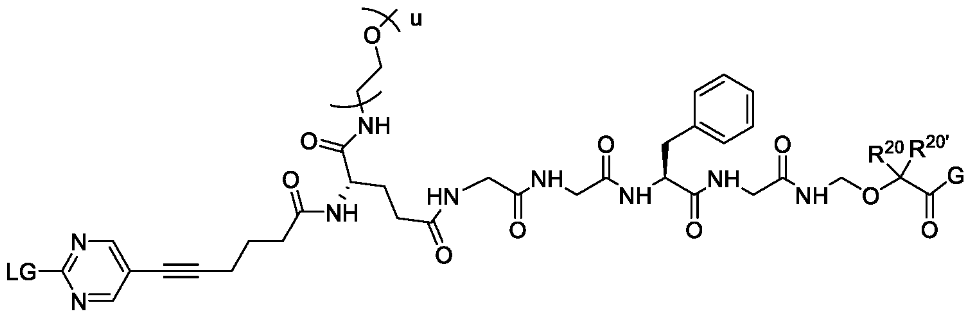 |
| 9 | 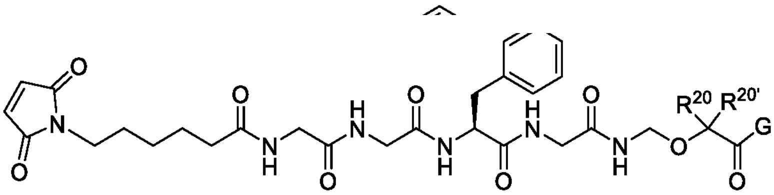 |

(continued)

| No. | Conjugate, or linker or linker-drug thereof |
|---|---|
| 10 | 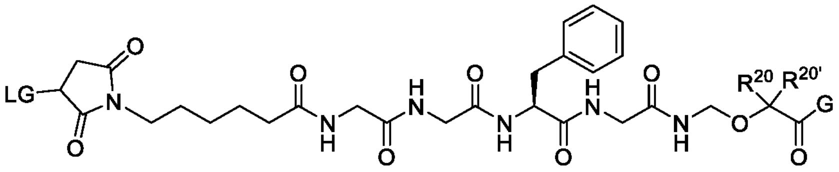 |
| 11 | 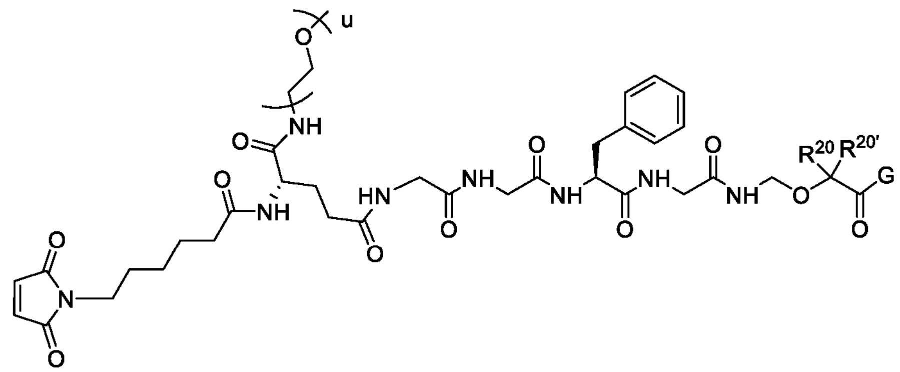 |
| 12 | 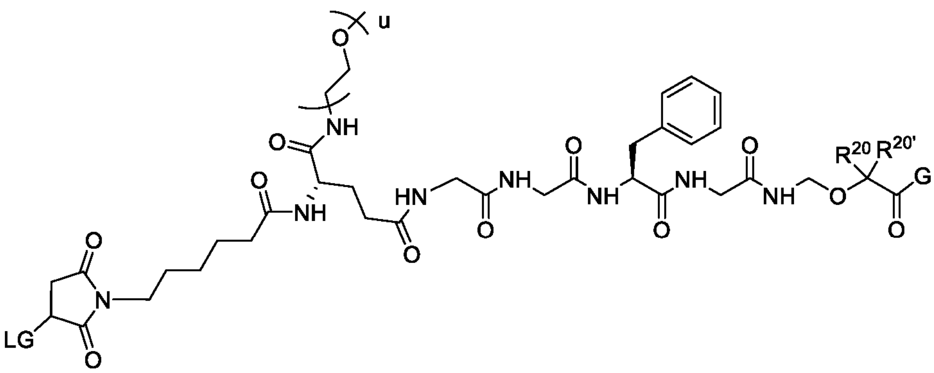 |
| 13 | 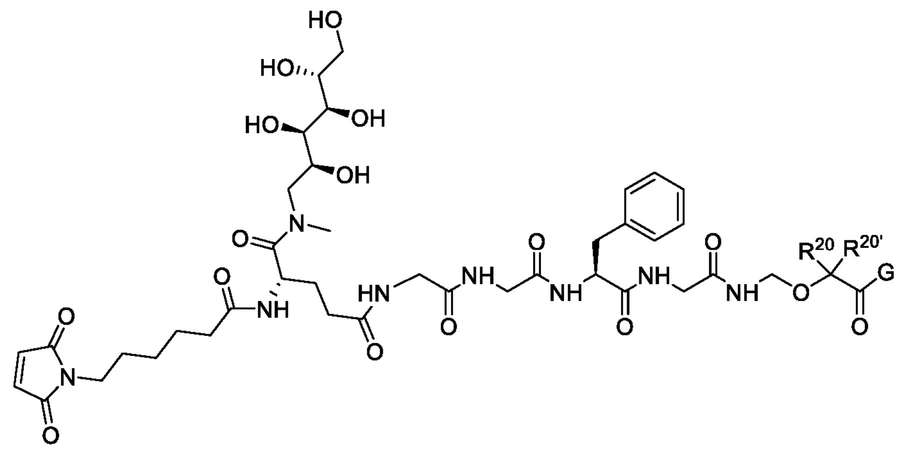 |

(continued)

| No. | Conjugate, or linker or linker-drug thereof |
|---|---|
| 14 | 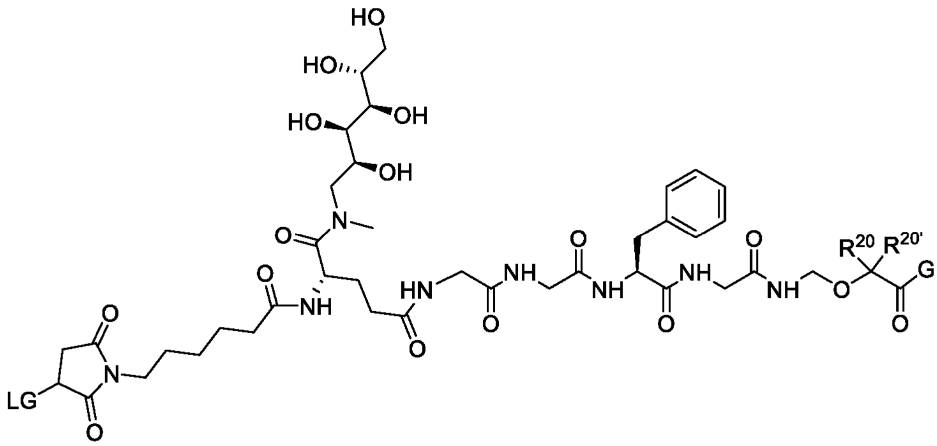 |
| 15 | 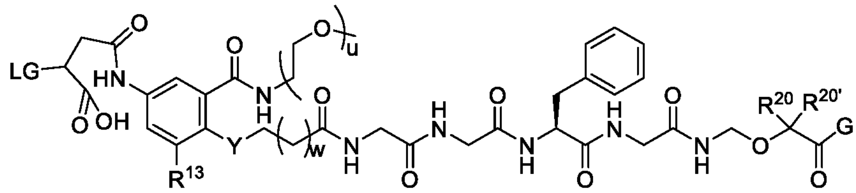 |
| 16 | 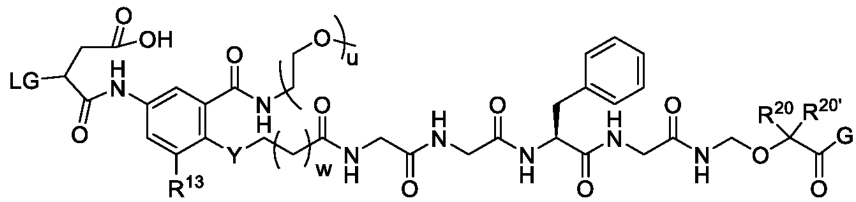 |
| 17 | 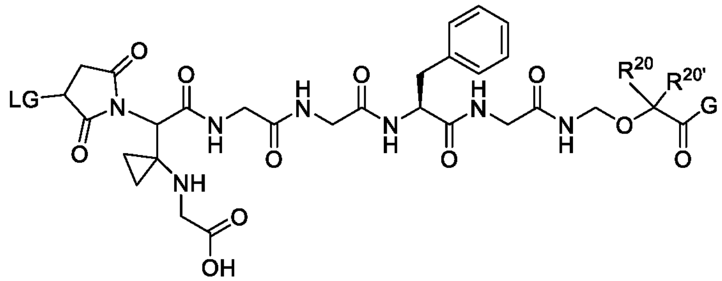 |
| 18 | 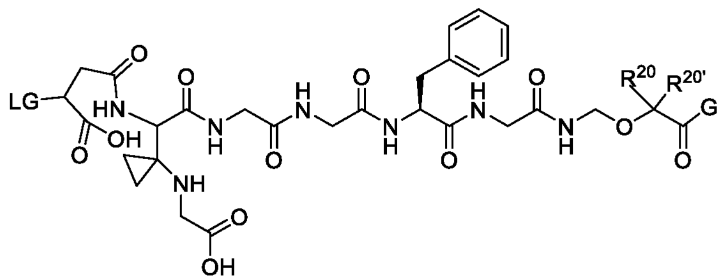 |
| 19 | 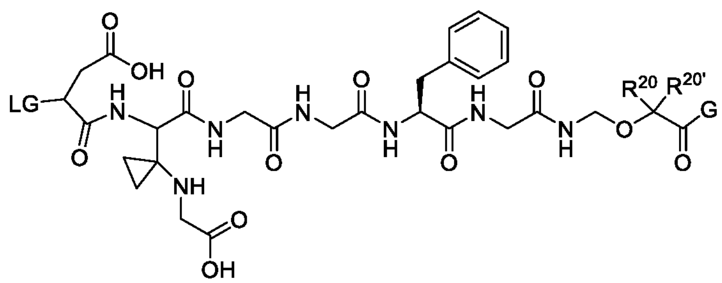 |

(continued)

| No. | Conjugate, or linker or linker-drug thereof |
| --- | --- |
| 20 | 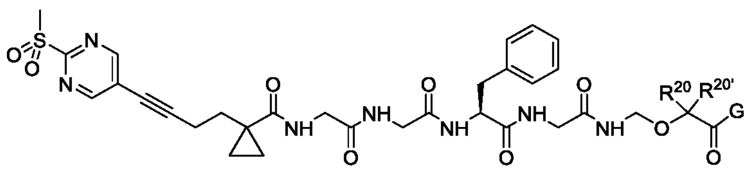 |
| 21 | 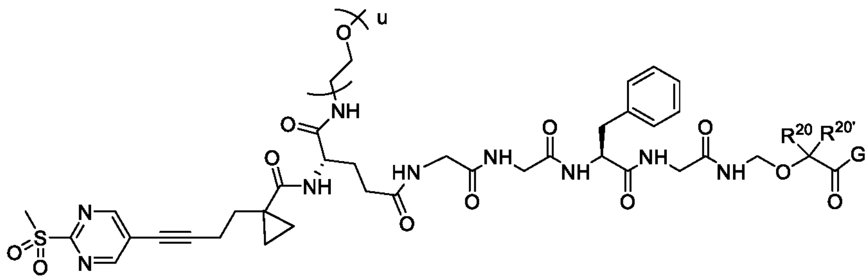 |
| 22 | 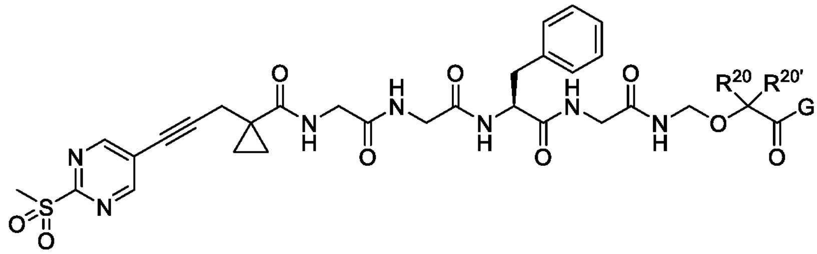 |
| 23 | 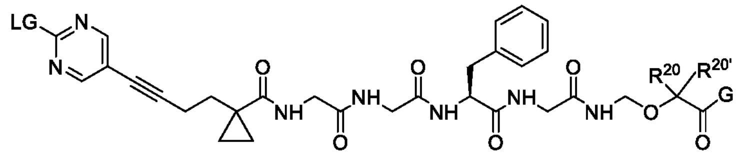 |
| 24 | 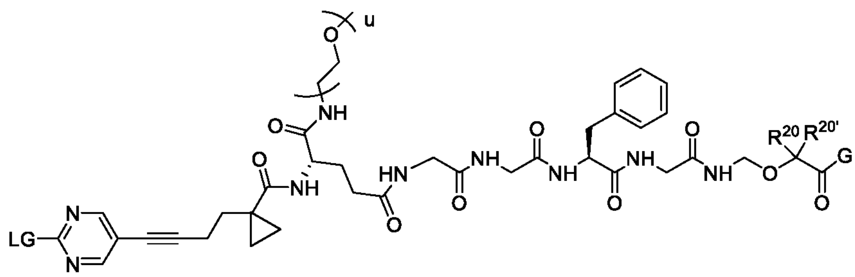 |
| 25 | 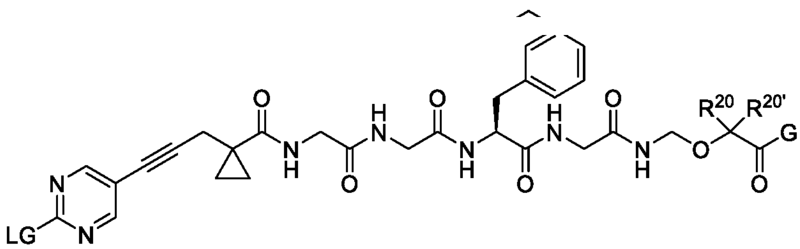 |
| 26 | 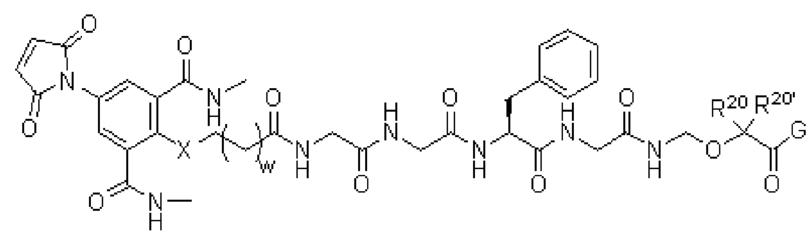 |

(continued)

| No. | Conjugate, or linker or linker-drug thereof |
|---|---|
| 27 | 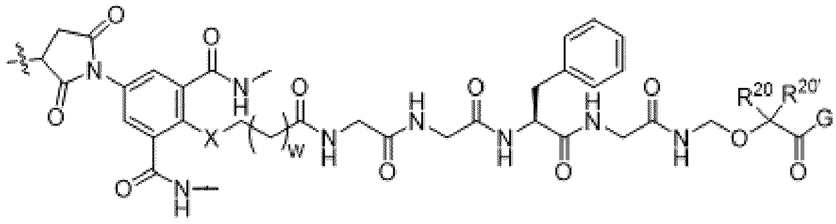 |

**25.** The conjugate, or a stereoisomer, racemate, tautomer, isotopologue, isotope-labeled compound, nitroxide or pharmaceutically acceptable salt thereof according to any one of claims 1 to 23, wherein the conjugate has a structure as shown in the following formula:

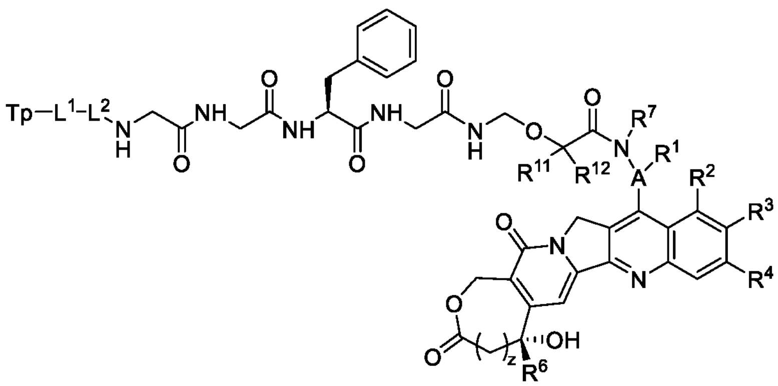

wherein, A, $R^1$, $R^2$, $R^3$, $R^4$, $R^6$, $R^7$, $R^{11}$, $R^{12}$ , $L^1$, $L^2$, and Tp have the definitions as described in any one of claims 1 to 23.

**26.** The conjugate, or a stereoisomer, racemate, tautomer, isotopologue, isotope-labeled compound, nitroxide or pharmaceutically acceptable salt thereof according to any one of claims 1 to 23, wherein the conjugate has a structure as shown in the following formula:

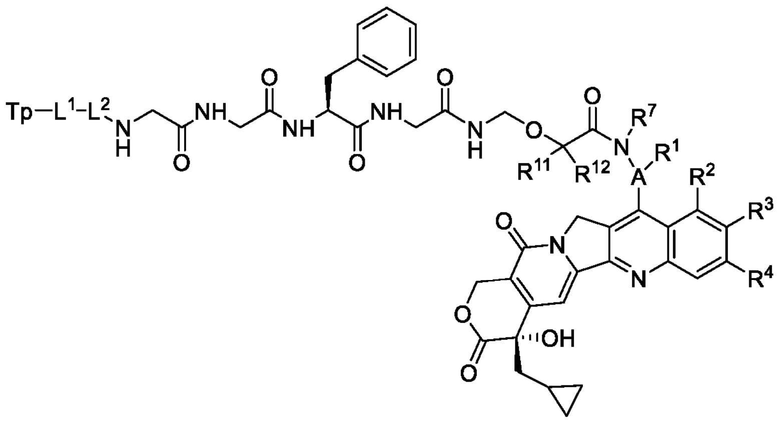

wherein, A, $R^1$, $R^2$, $R^3$, $R^4$, $R^7$, $R^{11}$, $R^{12}$, $L^1$, $L^2$, and Tp have the definitions as described in any one of claims 1 to 13.

**27.** The conjugate, or a stereoisomer, racemate, tautomer, isotopologue, isotope-labeled compound, nitroxide or pharmaceutically acceptable salt thereof according to any one of claims 1 to 23, wherein the conjugate has a structure as shown in the following formula:

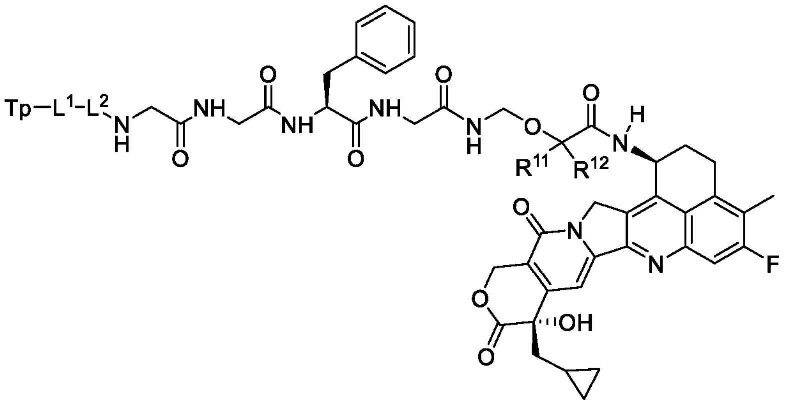

wherein, $R^{11}$, $R^{12}$, $L^1$, $L^2$, and Tp have the definitions as described in any one of claims 1 to 13.

**28.** The conjugate, or a stereoisomer, racemate, tautomer, isotopologue, isotope-labeled compound, nitroxide or pharmaceutically acceptable salt thereof according to any one of claims 1 to 23, wherein the conjugate is one selected from the followings:

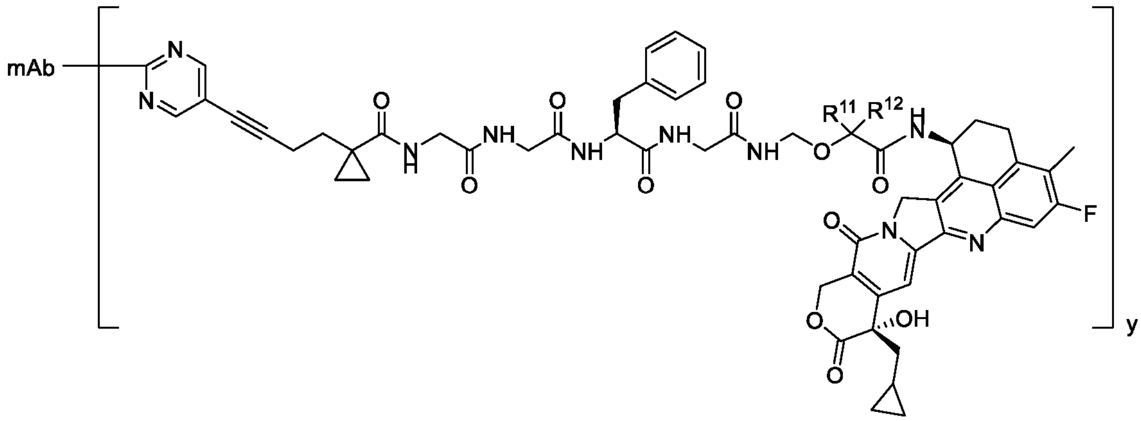

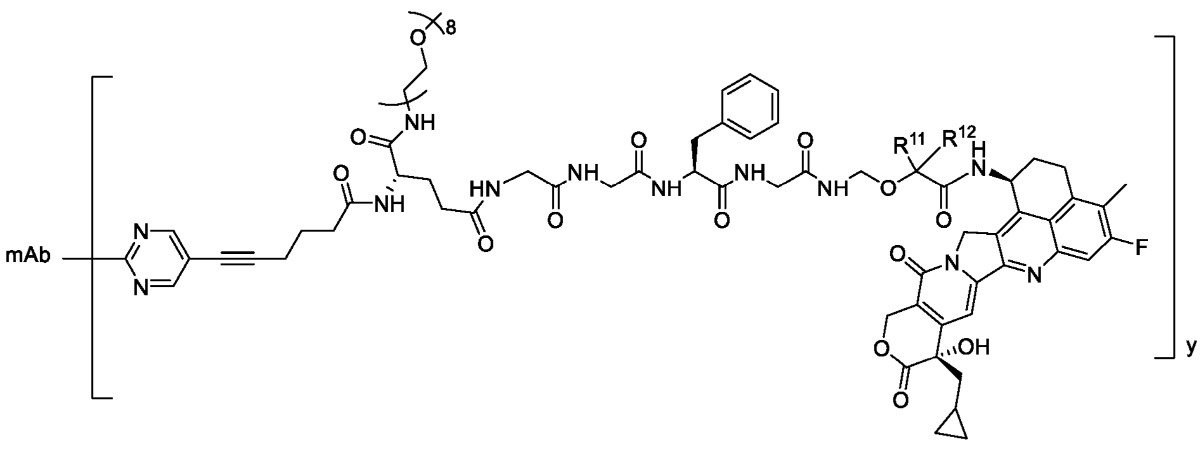

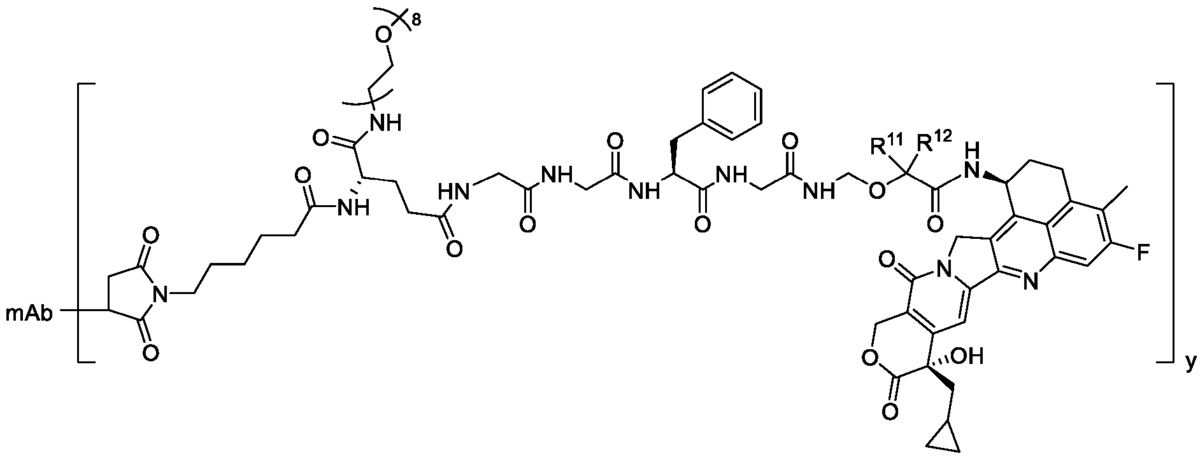

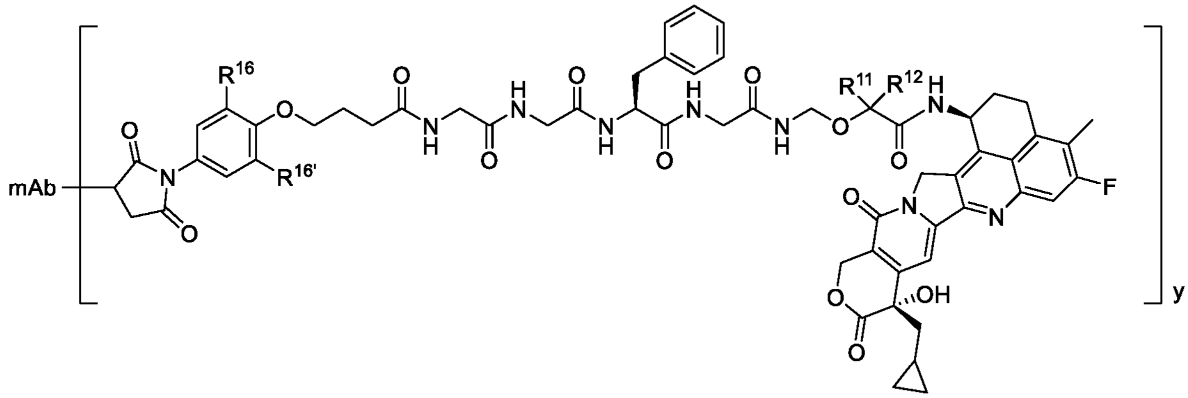

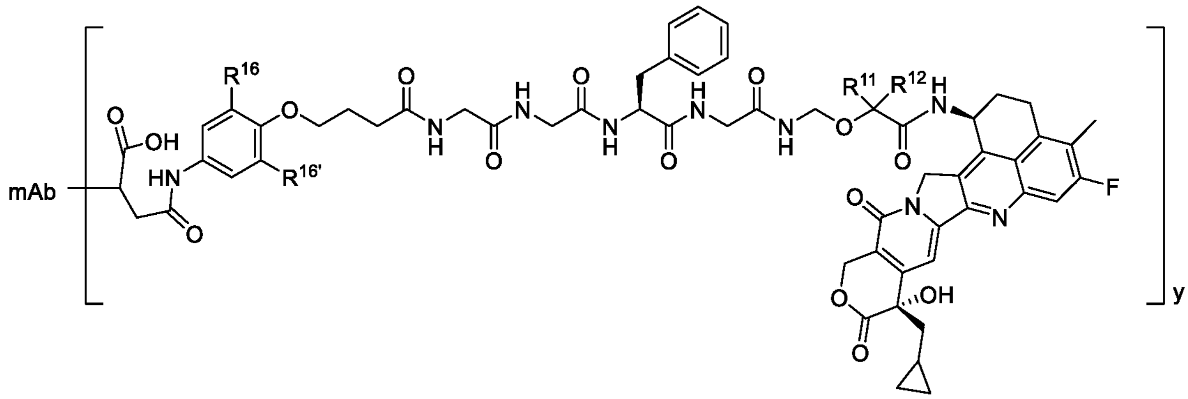

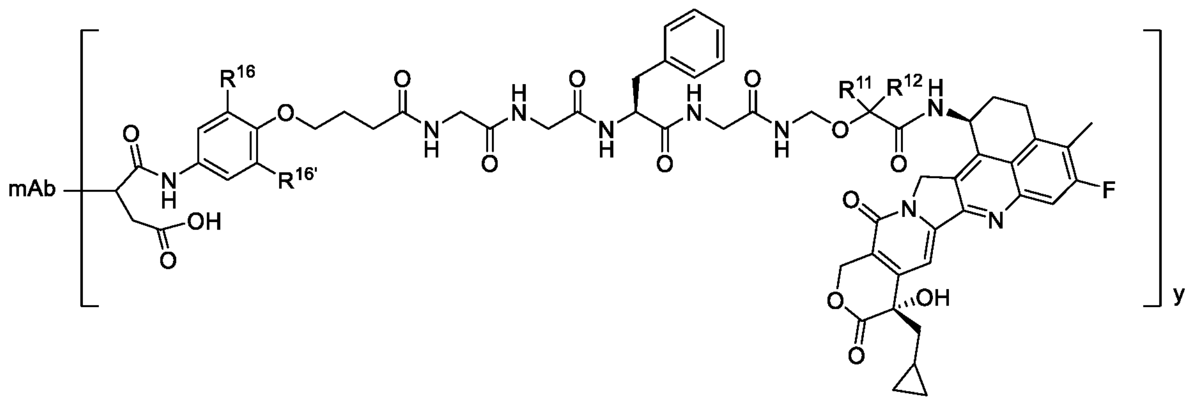

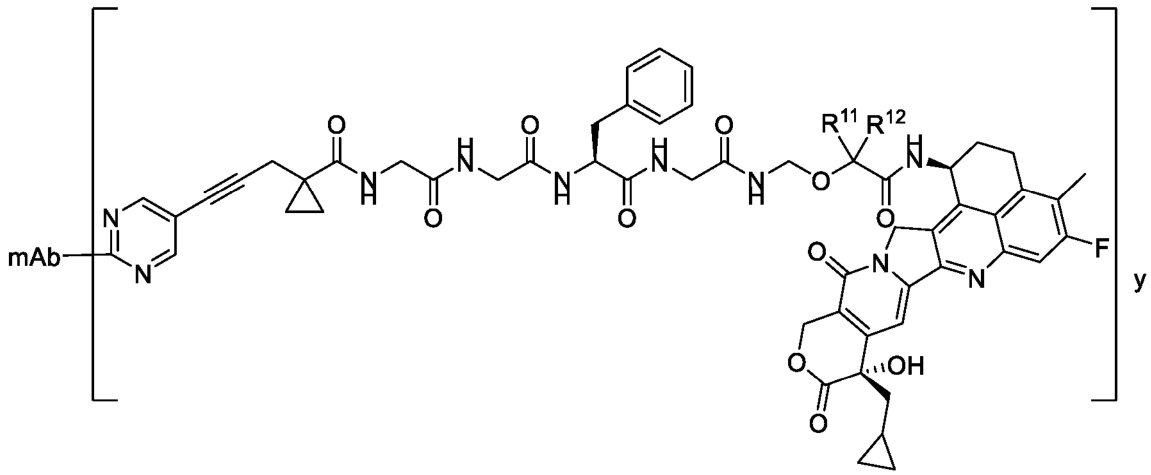

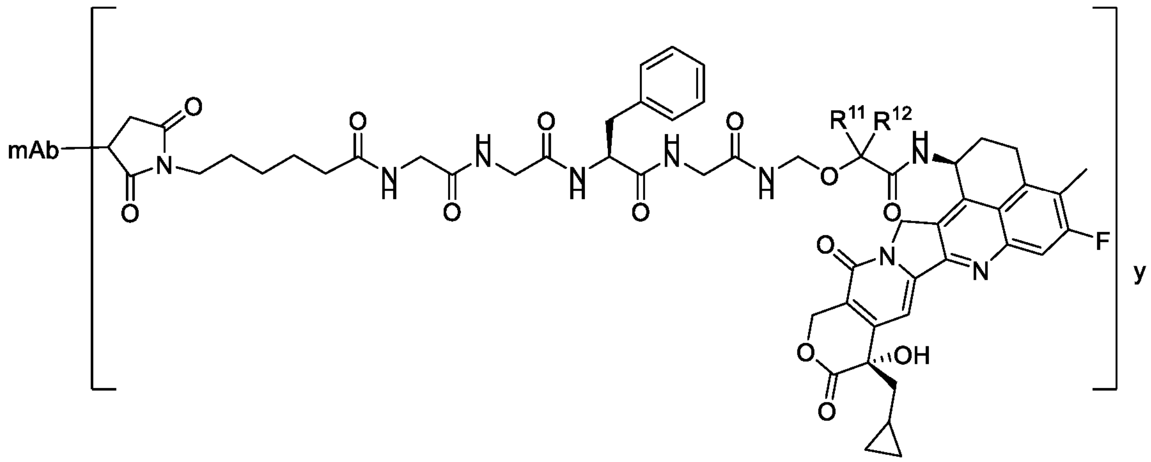

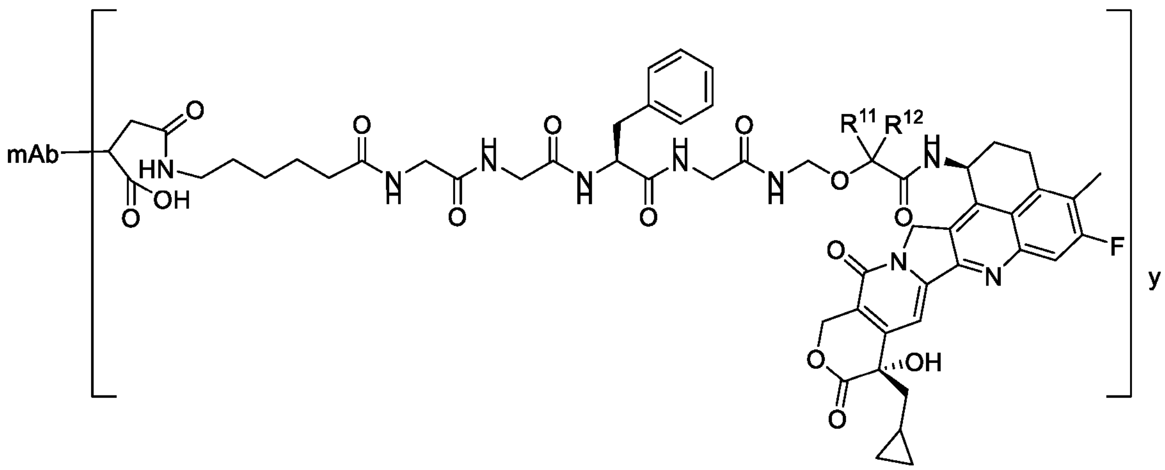

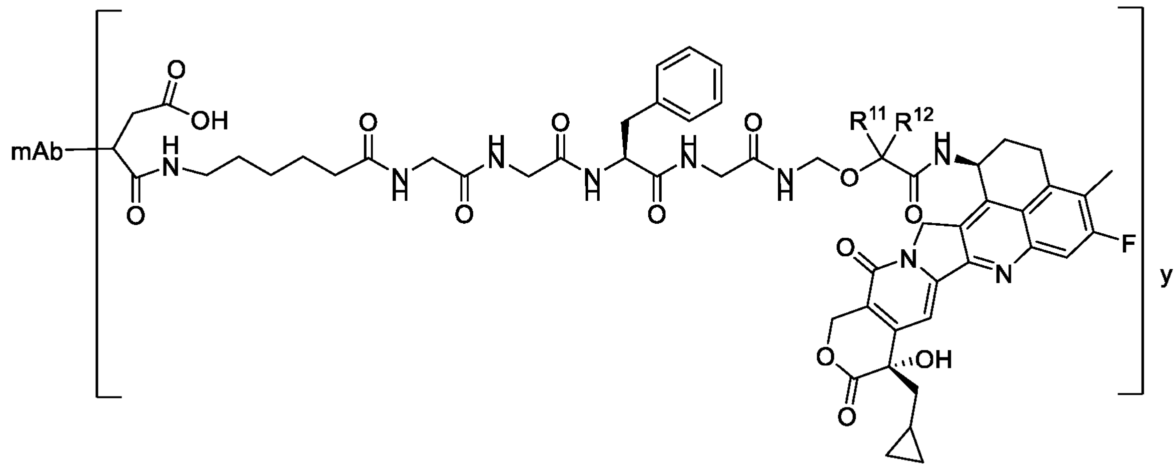

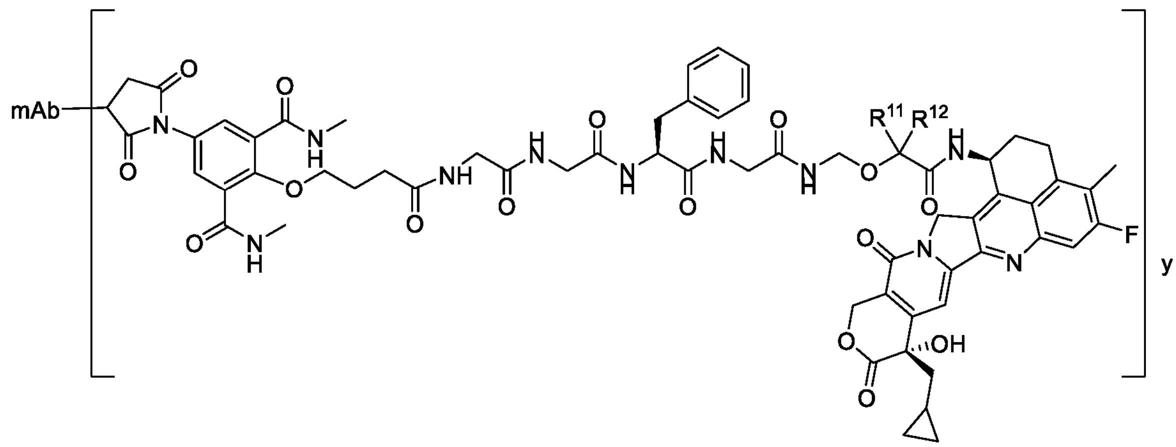

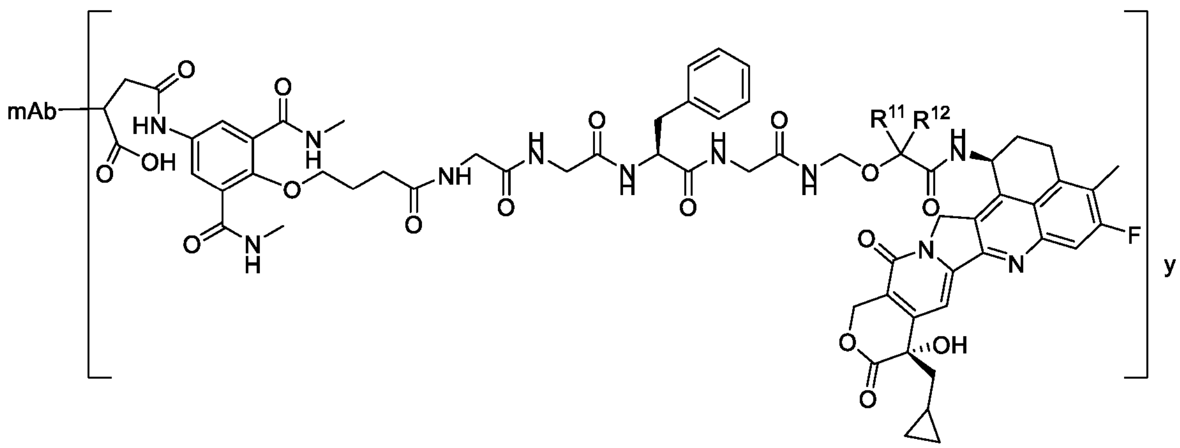

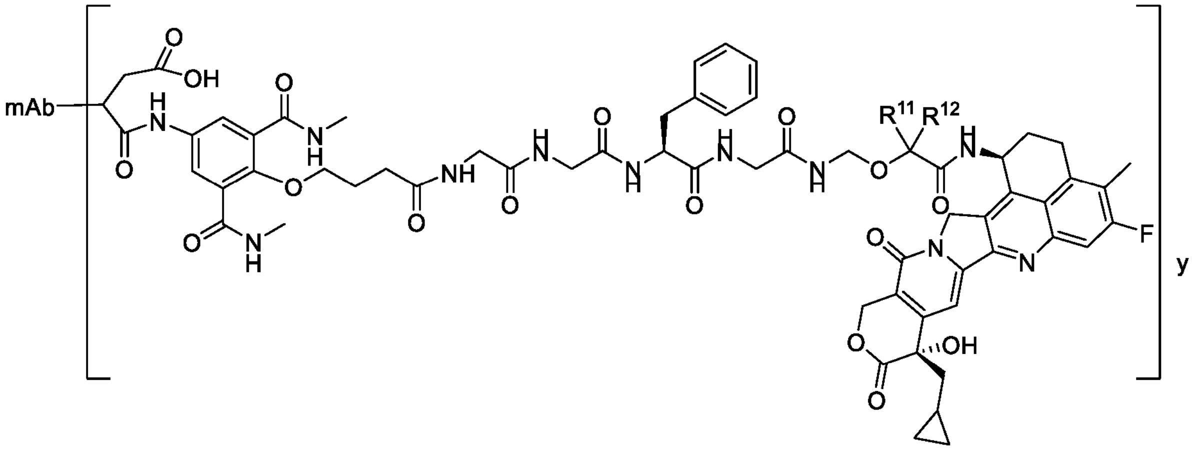

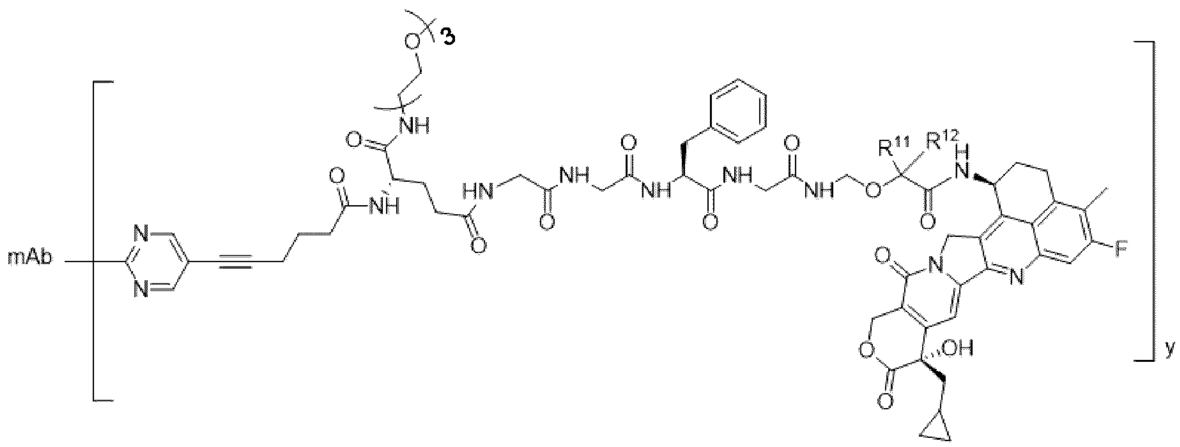

wherein, $R^{11}$ and $R^{12}$ are the same or different and independently have the definitions as described in any one of claims 1 to 23; for example, $R^{11}$ and $R^{12}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{11}$ and $R^{12}$, together with the carbon atom to which they are attached, form a $C_{3-6}$ cycloalkyl;

for example, the $C_{1-4}$ alkyl group can be independently selected from the group consisting of methyl, ethyl, propyl, isopropyl, butyl, isobutyl or *tert*-butyl; the $C_{3-6}$ cycloalkyl group can be independently selected from the group consisting of cyclopropyl, cyclobutyl, cyclopentyl or cyclohexyl;

for example, both of $R^{11}$ and $R^{12}$ are H, or one of them is not H, or both of them are not H;

$R^{16}$ and $R^{16'}$ independently have definitions as described in claim 14;

mAb represents a monoclonal antibody;

y represents the average number of small molecule drugs attached to each monoclonal antibody (DAR), which can be selected from an integer or decimal, for example, an integer or decimal selected from 1 to 50, an integer or decimal selected from 1 to 20, or an integer or decimal selected from 1 to 10.

**29.** The conjugate, or a stereoisomer, racemate, tautomer, isotopologue, isotope-labeled compound, nitroxide or pharmaceutically acceptable salt thereof according to any one of claims 1 to 23, wherein the conjugate has a structure as shown in the following formula:

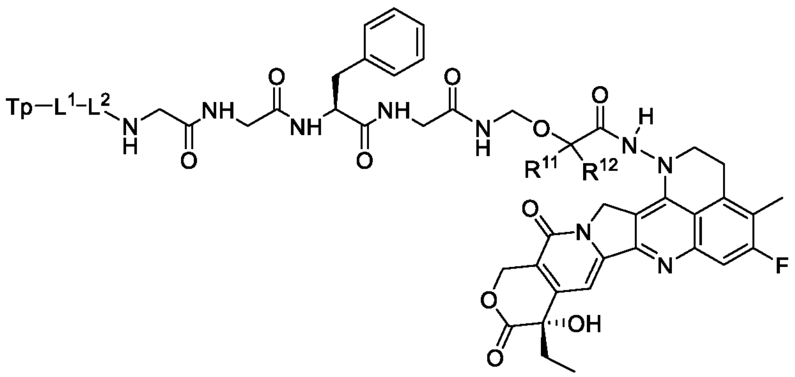

wherein, $R^{11}$, $R^{12}$, $L^1$, $L^2$, and Tp have the definitions as described in any one of claims 1 to 23.

**30.** The conjugate, or a stereoisomer, racemate, tautomer, isotopologue, isotope-labeled compound, nitroxide or pharmaceutically acceptable salt thereof according to any one of claims 1 to 23, wherein the conjugate is one selected from the followings:

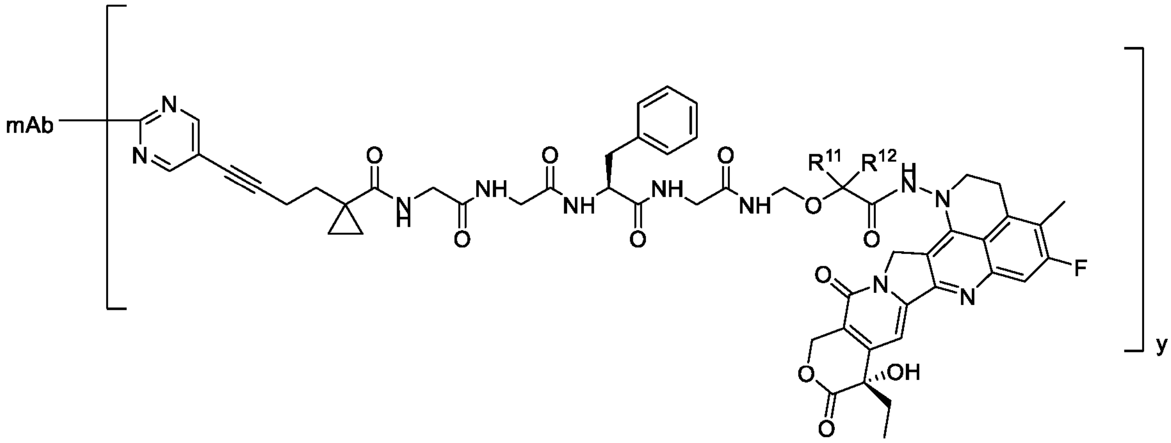

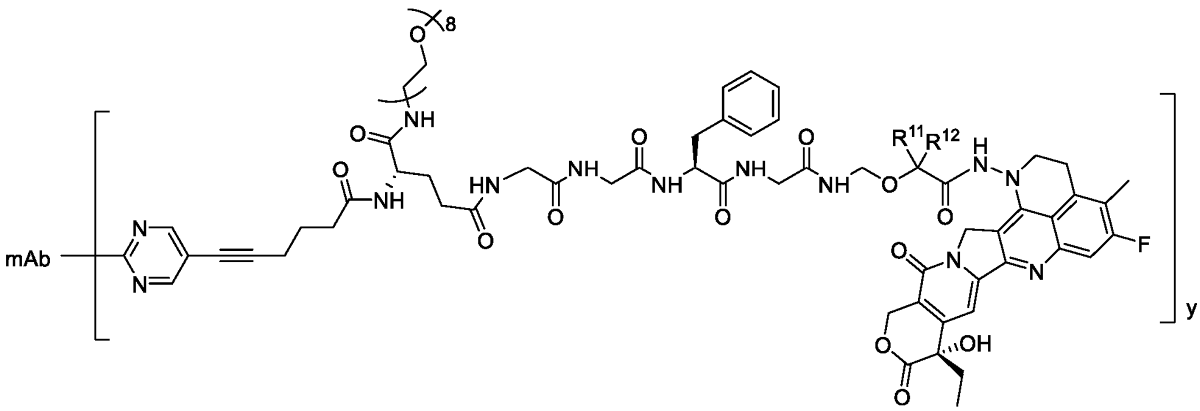

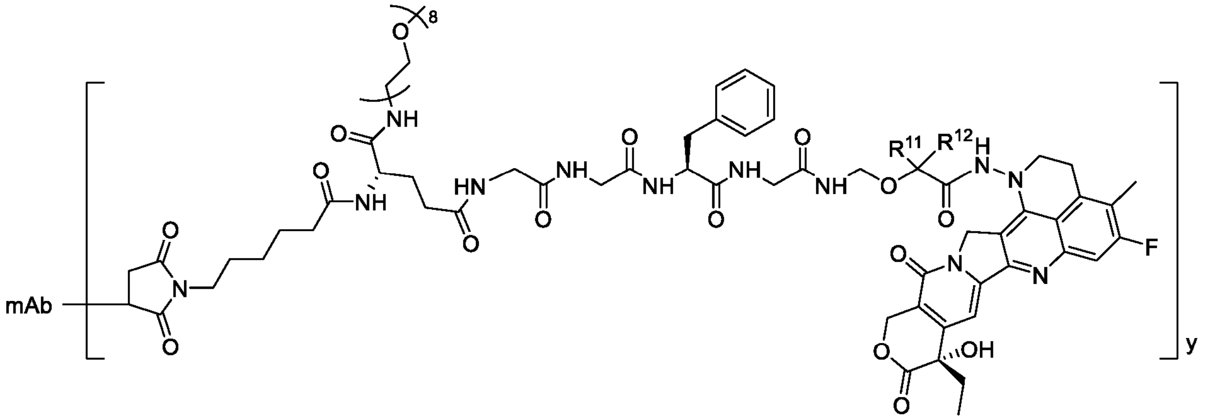

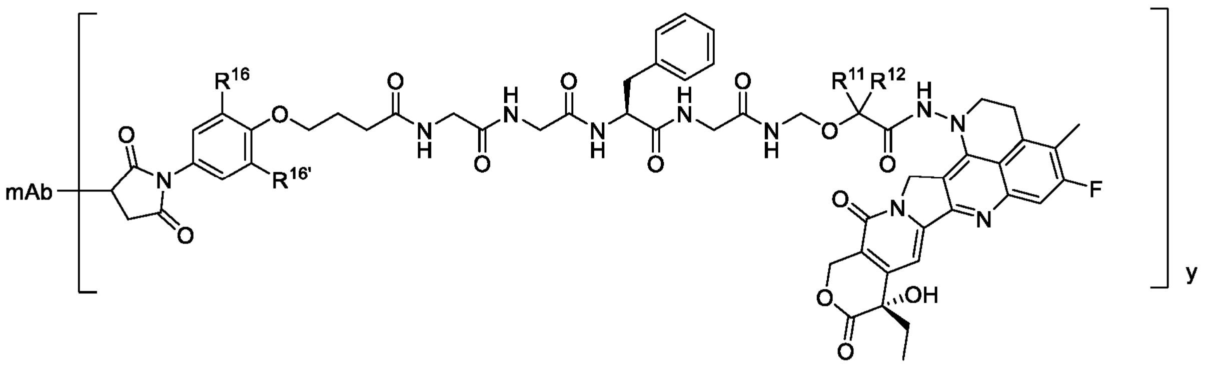

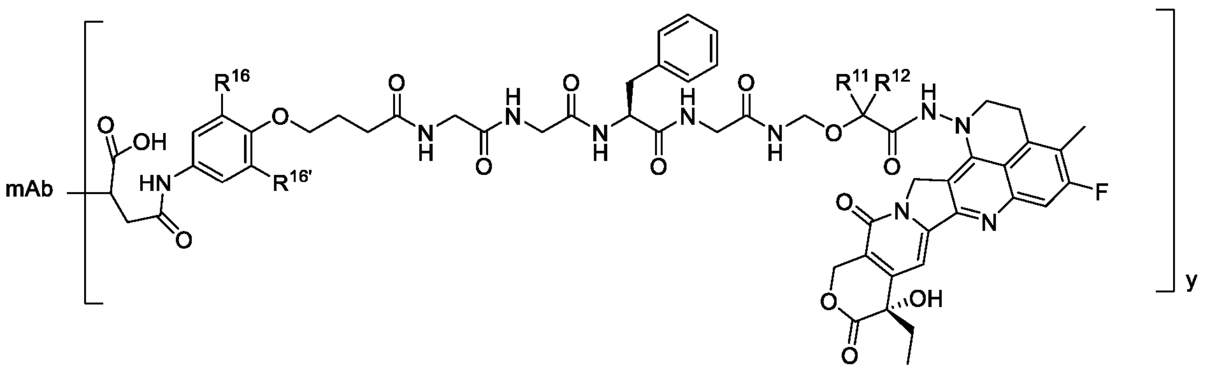

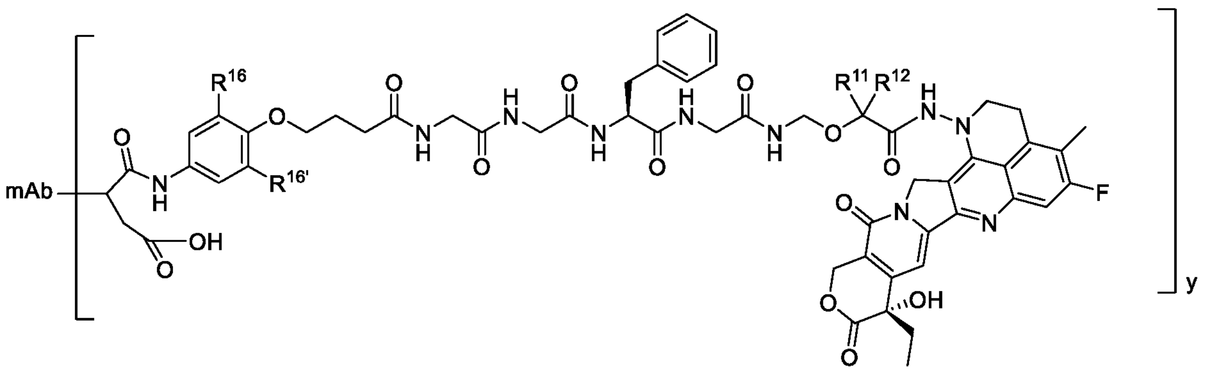

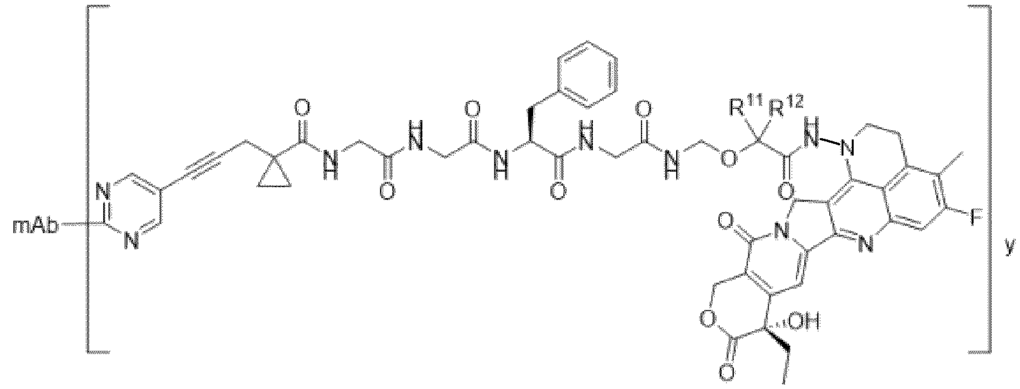

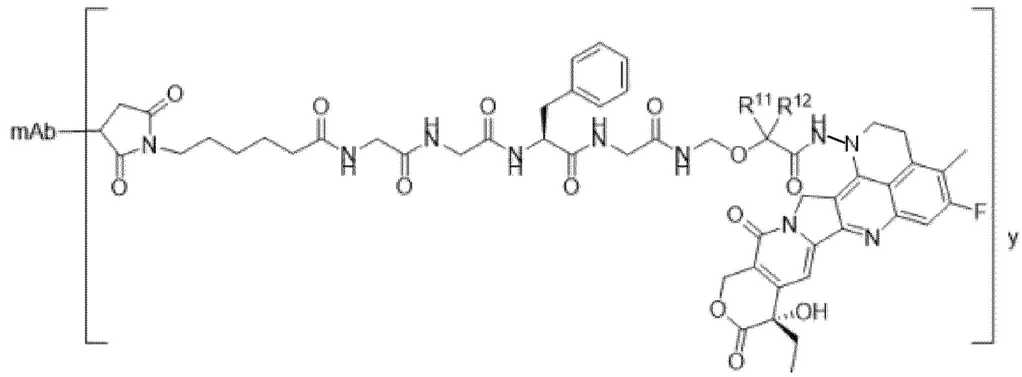

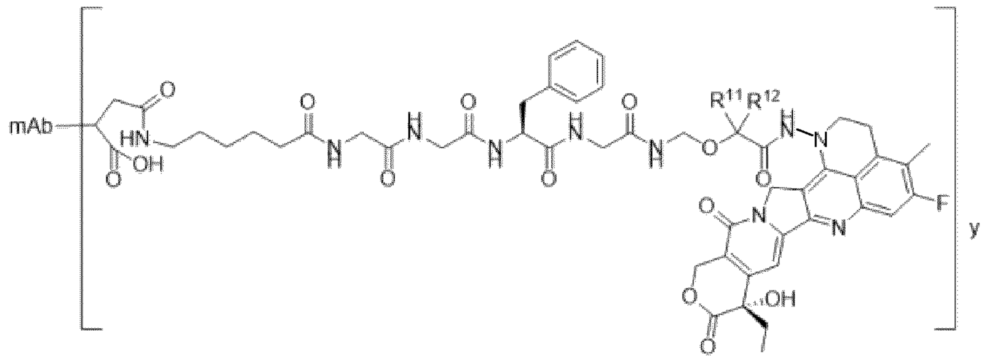

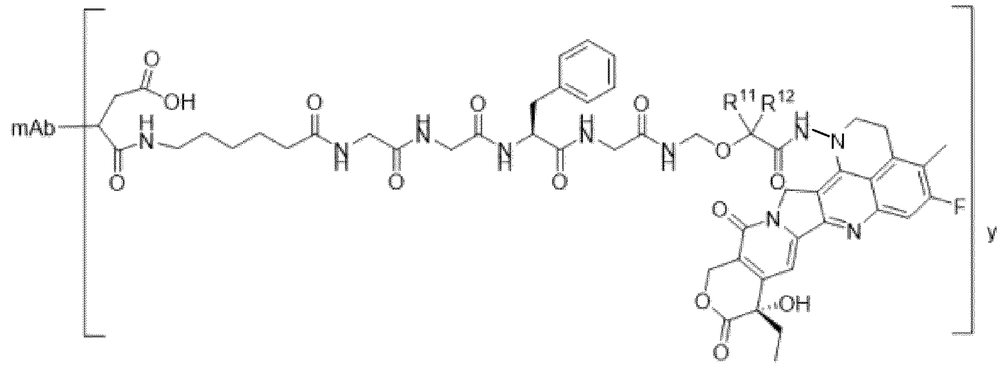

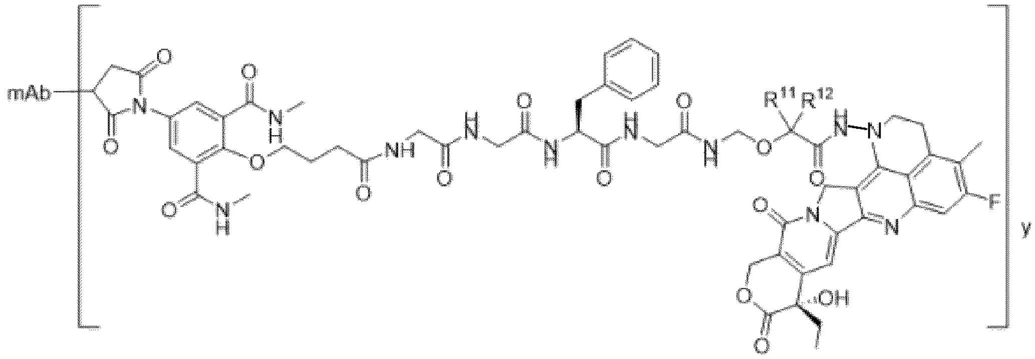

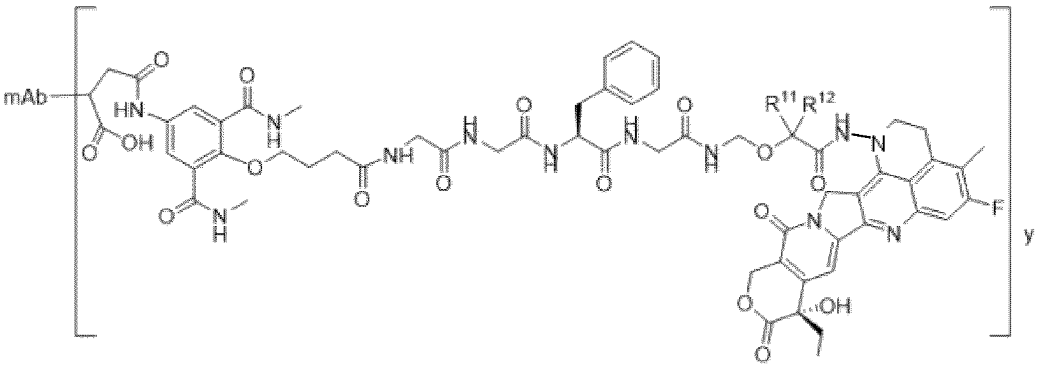

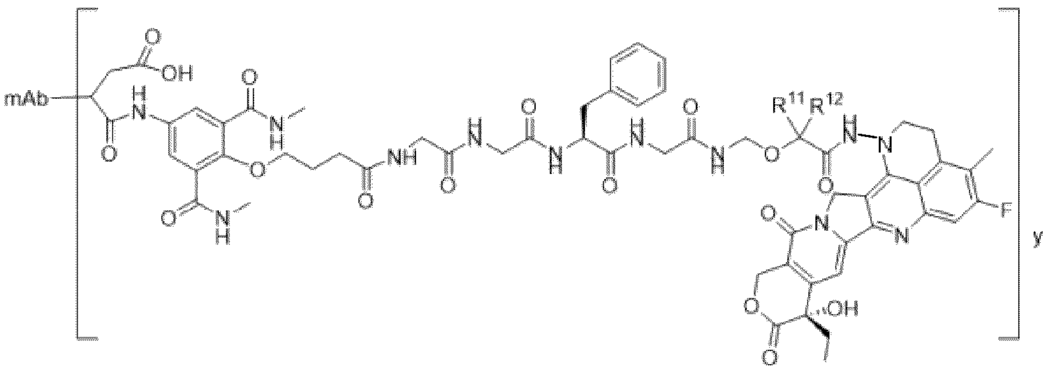

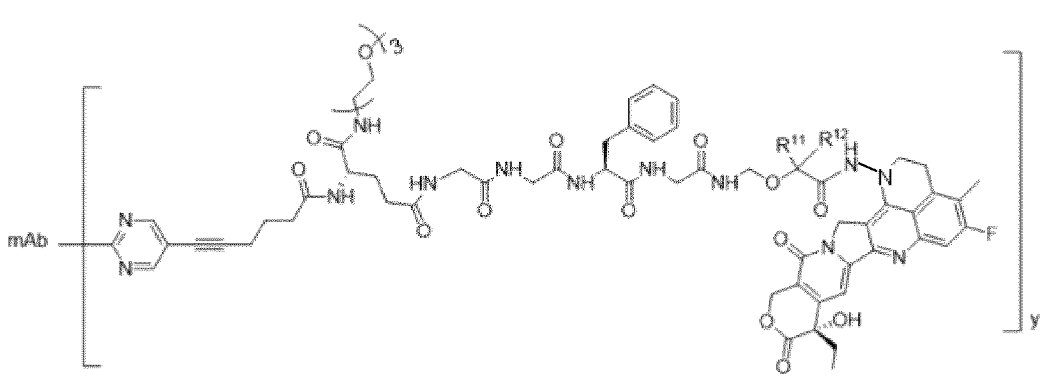

wherein, $R^{11}$ and $R^{12}$ independently have the definitions as described in any one of claims 1 to 23; for example, $R^{11}$, $R^{12}$ are the same or different, and are independently selected from the group consisting of H, $C_{1-4}$ alkyl, $C_{3-6}$ cycloalkyl, $C_{3-6}$ cycloalkyl-$C_{1-4}$ alkyl, or $R^{11}$ and $R^{12}$, together with the carbon atom to which they are attached, form a $C_{3-6}$ cycloalkyl;

for example, the $C_{1-4}$ alkyl can be independently selected from the group consisting of methyl, ethyl, propyl, isopropyl, butyl, isobutyl or *tert*-butyl; the $C_{3-6}$ cycloalkyl can be independently selected from the group consisting

of cyclopropyl, cyclobutyl, cyclopentyl or cyclohexyl;

for example, both of $R^{11}$ and $R^{12}$ are H, or one of them is not H, or both of them are not H;

$R^{16}$ and $R^{16'}$ independently have the definitions as described in claim 14;

mAb represents a monoclonal antibody;

y represents the average number of small molecule drugs attached to each monoclonal antibody (DAR), which can be selected from an integer or decimal, for example, an integer or a decimal selected from 1 to 50, an integer or decimal selected from 1 to 20, or an integer or decimal selected from 1 to 10.

**31.** The conjugate, or a stereoisomer, racemate, tautomer, isotopologue, isotope-labeled compound, nitroxide or pharmaceutically acceptable salt thereof according to any one of claims 1 to 23, wherein the conjugate has a structure as shown in the following formula:

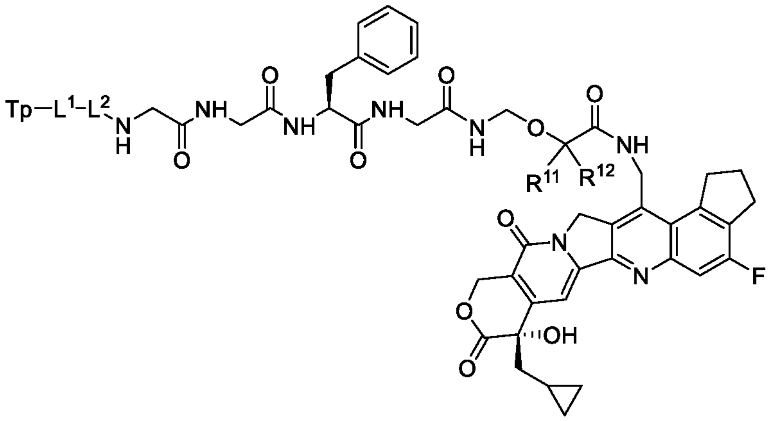

wherein, $R^{11}$ and $R^{12}$ independently have the definitions as described in any one of claims 1 to 23; for example, $R^{11}$ and $R^{12}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{11}$, $R^{12}$ and the carbon atom to which they are connected together form a $C_{3-6}$ cycloalkyl;

for example, the $C_{1-4}$ alkyl can be independently selected from the group consisting of methyl, ethyl, propyl, isopropyl, butyl, isobutyl or *tert*-butyl; the $C_{3-6}$ cycloalkyl can be independently selected from the group consisting of cyclopropyl, cyclobutyl, cyclopentyl or cyclohexyl;

for example, both of $R^{11}$ and $R^{12}$ are H, or one is not H, or both are not H;

$L^1$, $L^2$, and Tp independently have the definitions as described in any one of claims 1 to 23.

**32.** The conjugate, or a stereoisomer, racemate, tautomer, isotopologue, isotope-labeled compound, nitroxide or pharmaceutically acceptable salt thereof according to any one of claims 1 to 23, wherein the conjugate is one selected from the followings:

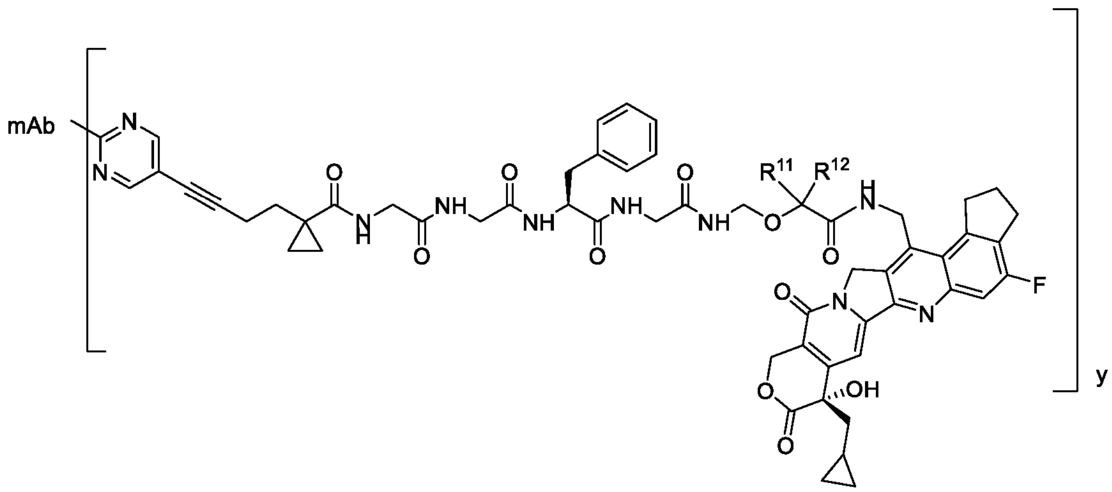

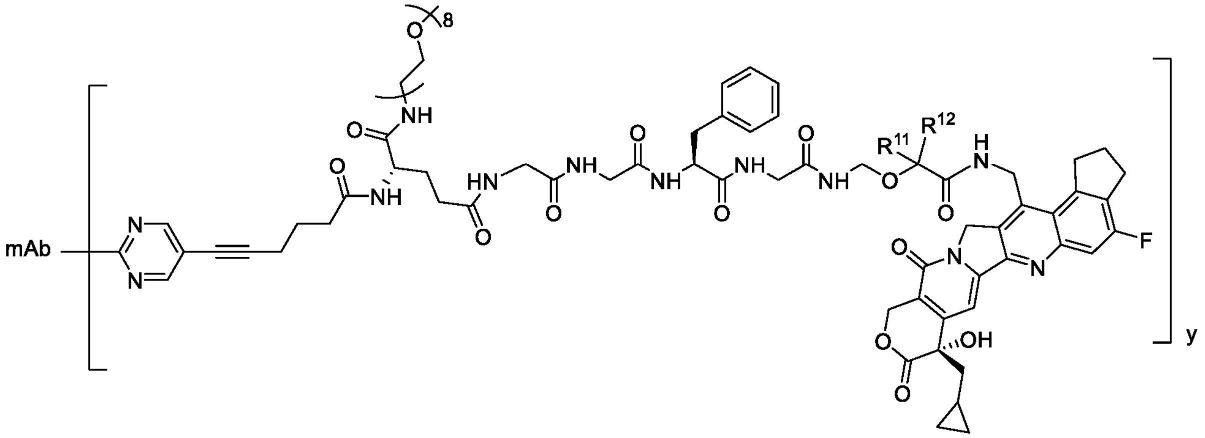

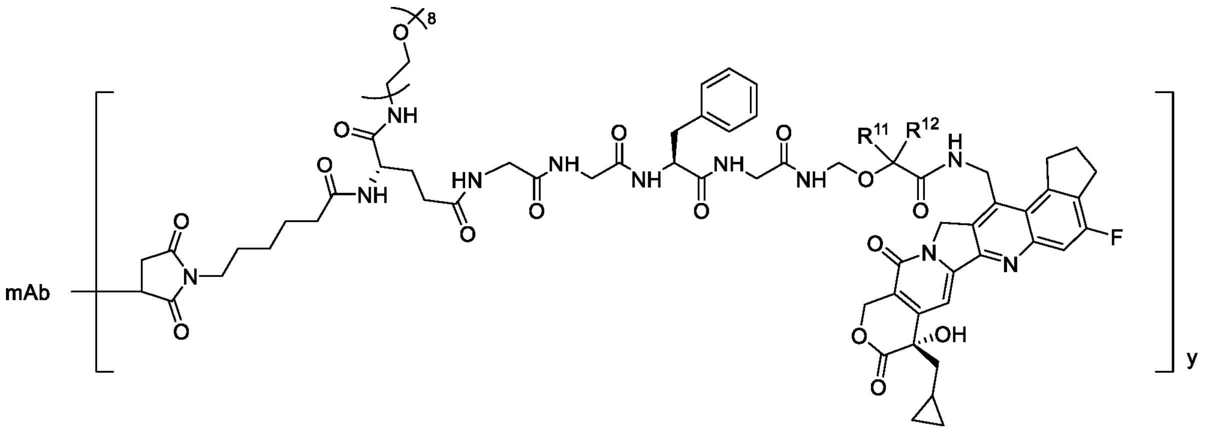

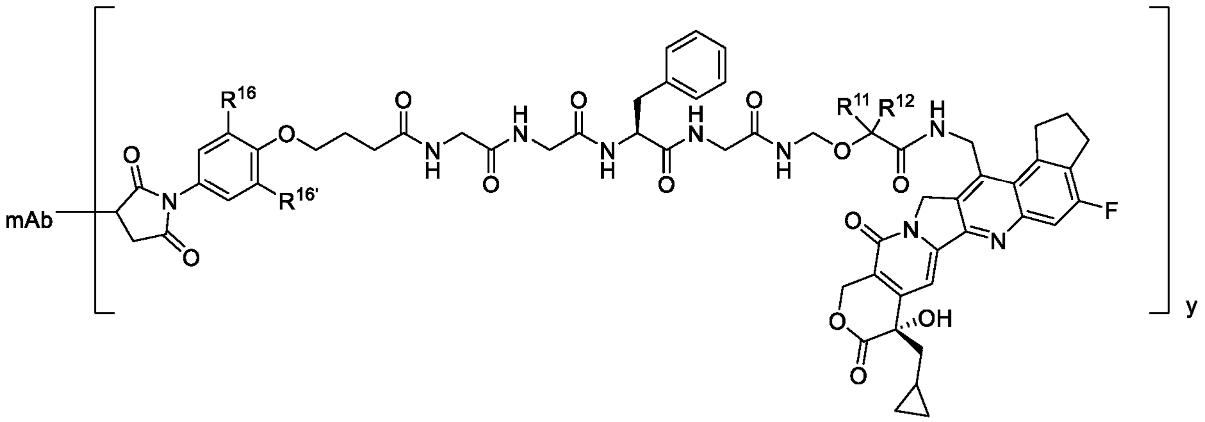

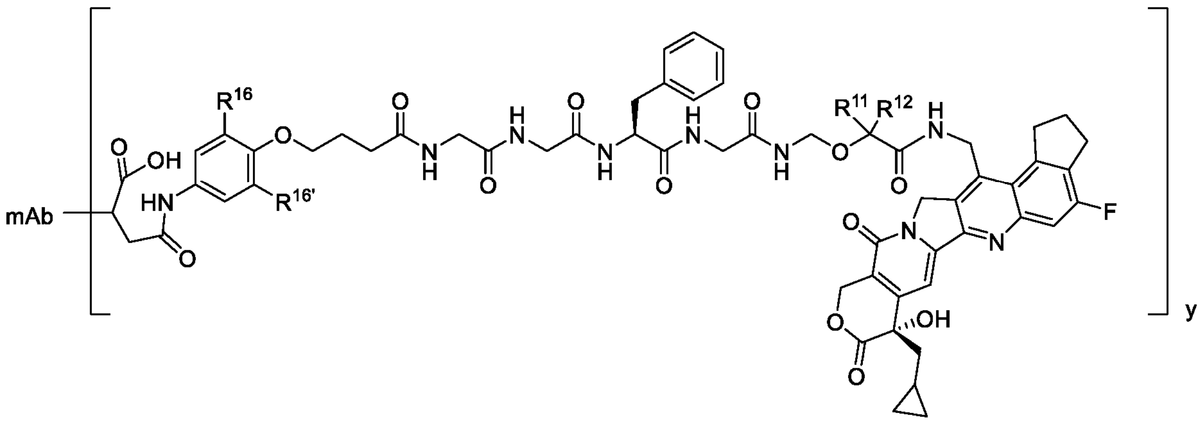

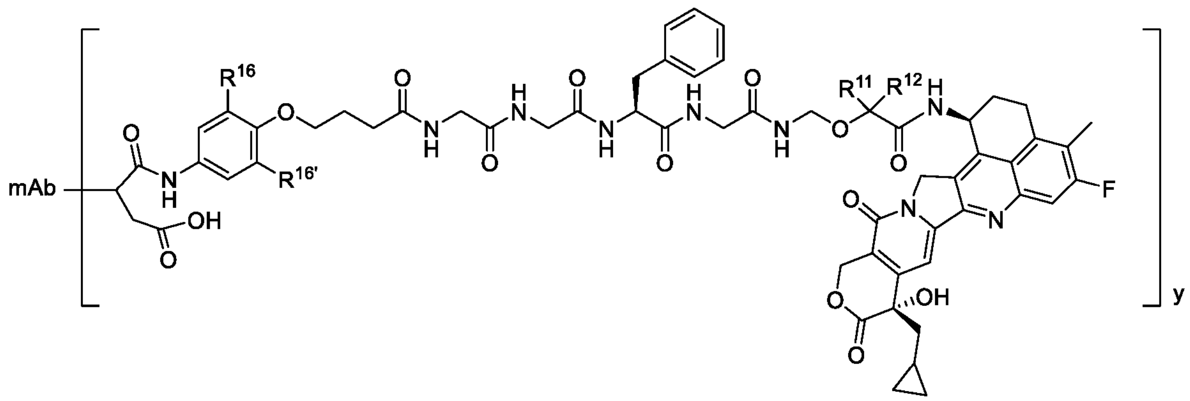

wherein, $R^{11}$ and $R^{12}$ independently have the definitions as described in any one of claims 1 to 23; for example, $R^{11}$ and $R^{12}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{11}$ and $R^{12}$, together with the carbon atom to which they are attached, form a $C_{3-6}$ cycloalkyl;

for example, the $C_{1-4}$ alkyl can be independently selected from the group consisting of methyl, ethyl, propyl, isopropyl, butyl, isobutyl or *tert*-butyl; and the $C_{3-6}$ cycloalkyl can be independently selected from the group consisting of cyclopropyl, cyclobutyl, cyclopentyl or cyclohexyl;

for example, both of $R^{11}$ and $R^{12}$ are H, or one of them is not H, or both of them are not H;

$R^{16}$ and $R^{16'}$ independently have the definitions as described in claim 14;

mAb represents a monoclonal antibody;

y represents the average number of small molecule drugs attached to each monoclonal antibody (DAR), which can be selected from an integer or decimal, for example, an integer or decimal selected from 1 to 50, an integer or decimal selected from 1 to 20, or an integer or decimal selected from 1 to 10.

**33.** The conjugate, or a stereoisomer, racemate, tautomer, isotopologue, isotope-labeled compound, nitroxide or pharmaceutically acceptable salt thereof according to any one of claims 1 to 32, wherein the conjugate is one selected from the followings:

y represents the average number of small molecule drugs attached to each monoclonal antibody (DAR), which can be selected from an integer or decimal, such as an integer or decimal selected from 1 to 50, an integer or decimal selected from 1 to 20, or an integer or decimal selected from 1 to 10;

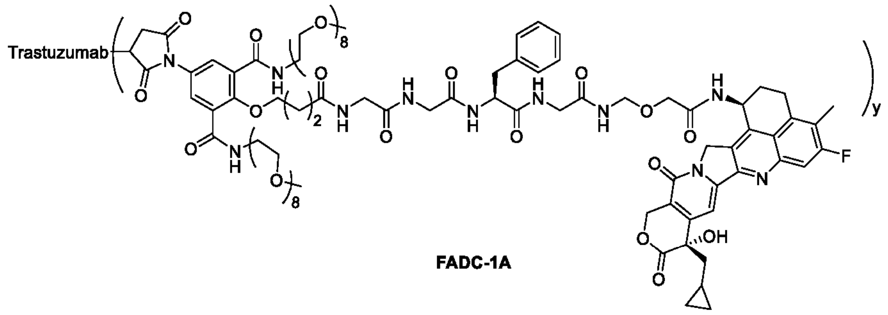

FADC-1A

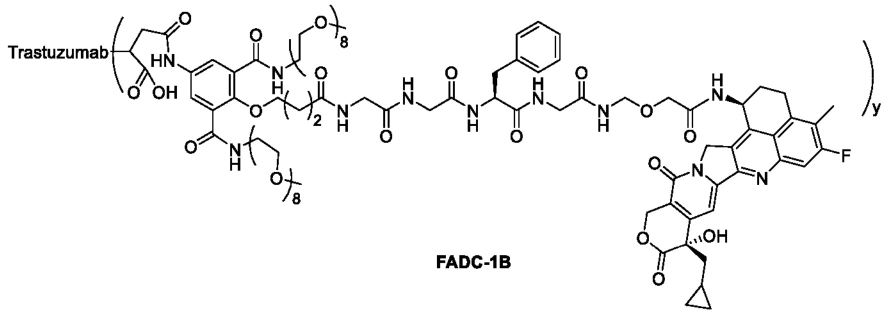

FADC-1B

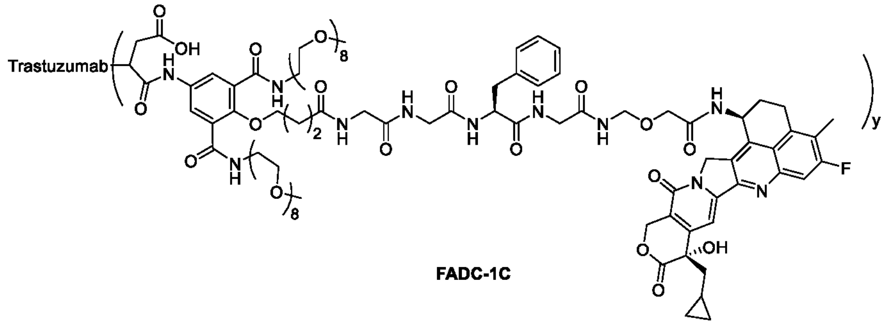

FADC-1C

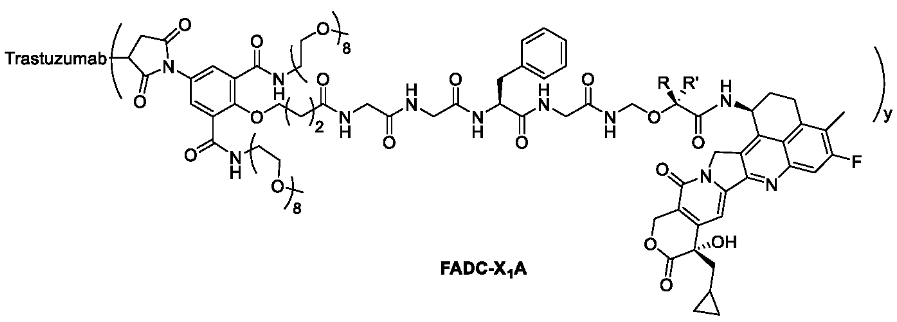

FADC-$X_1$A

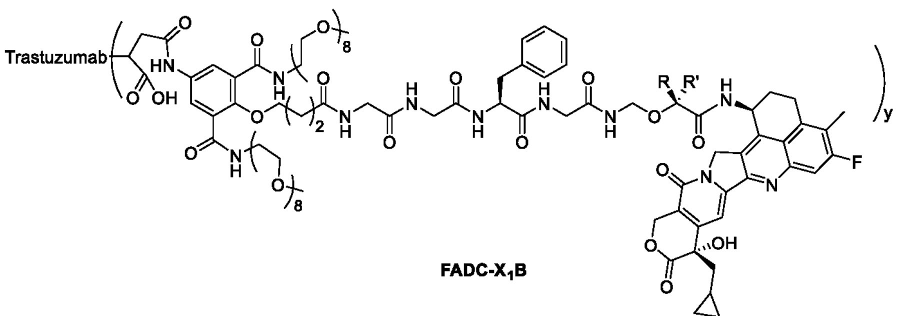

FADC-$X_1$B

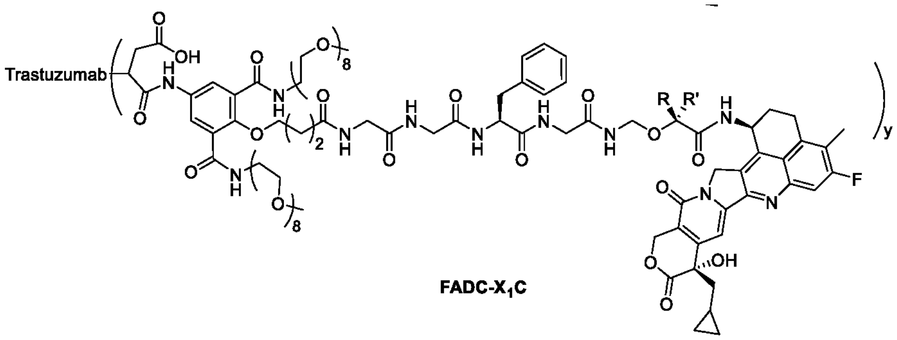

FADC-$X_1$C

wherein, R is selected from $-CH_3$, R' is selected from -H;
R is selected from -H, R' is selected from $-CH_3$;
R is selected from

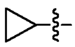

,

R' is selected from from -H; or
R is selected from -H, R' is selected from

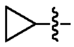

;

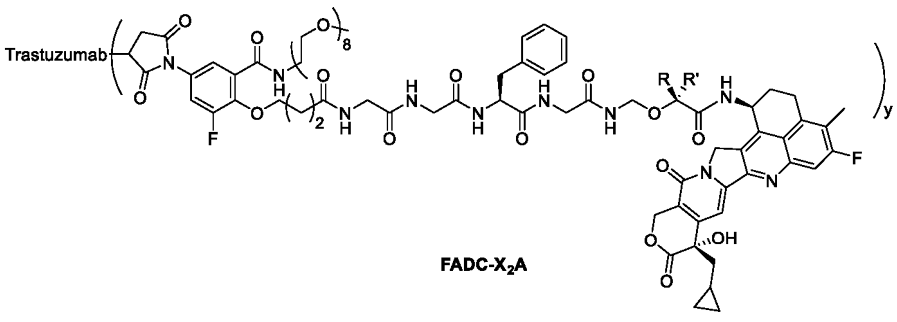

FADC-$X_2$A

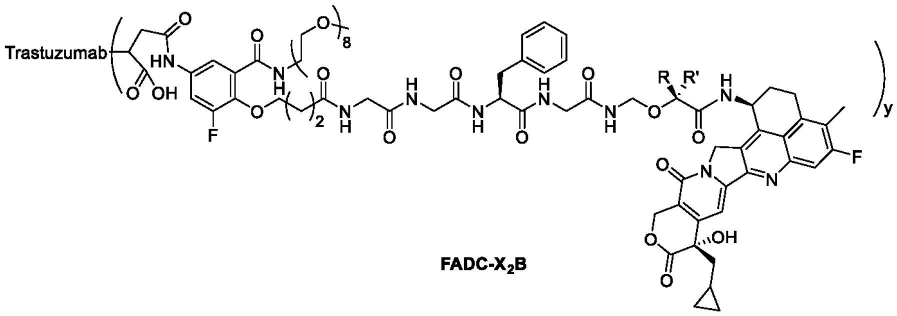

FADC-$X_2$B

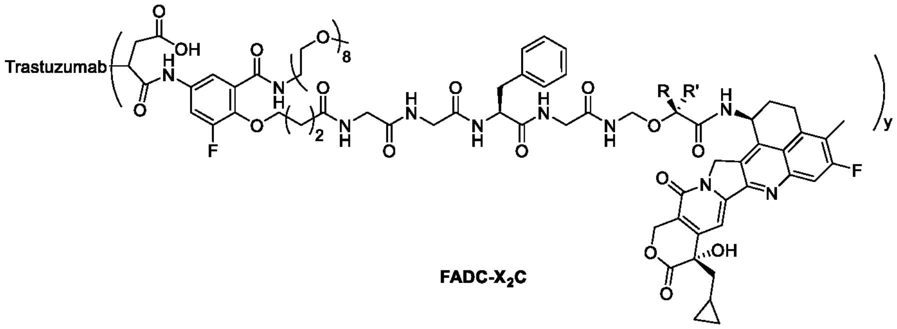

FADC-$X_2$C

wherein, R is selected from -H, R' is selected from -H;
R is selected from $-CH_3$, R' is selected from -H;
R is selected from -H, R' is selected from $-CH_3$;
R is selected from

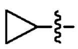,

R' is selected from -H; or
R is selected from -H, R' is selected from

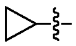;

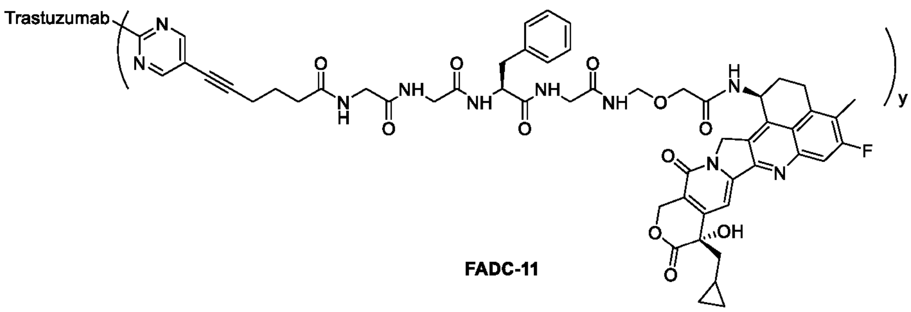

FADC-11

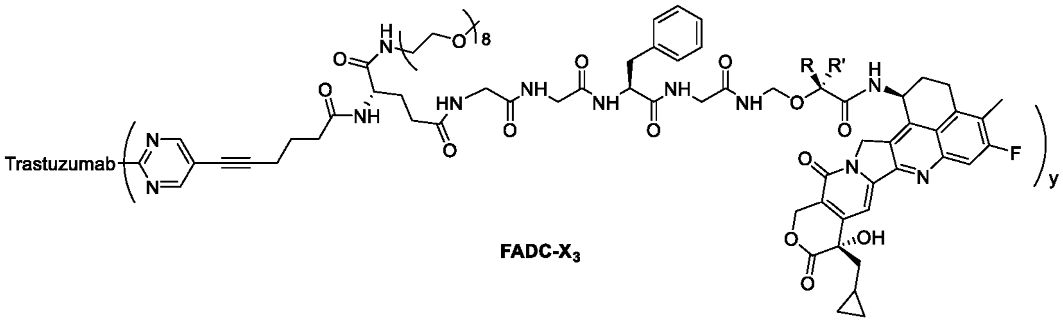

FADC-X$_3$

wherein, R is selected from -H, R' is selected from -H;
R is selected from $-CH_3$, R' is selected from -H;
R is selected from -H, R' is selected from $-CH_3$;
R is selected from

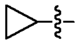,

R' is selected from -H; or
R is selected from -H, R' is selected from

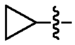;

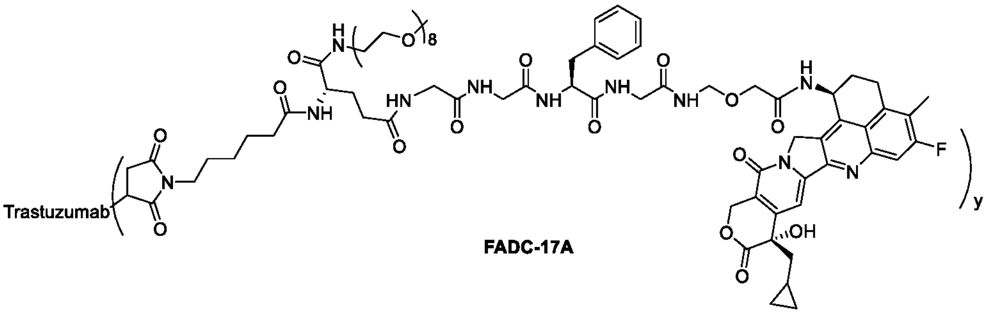

FADC-17A

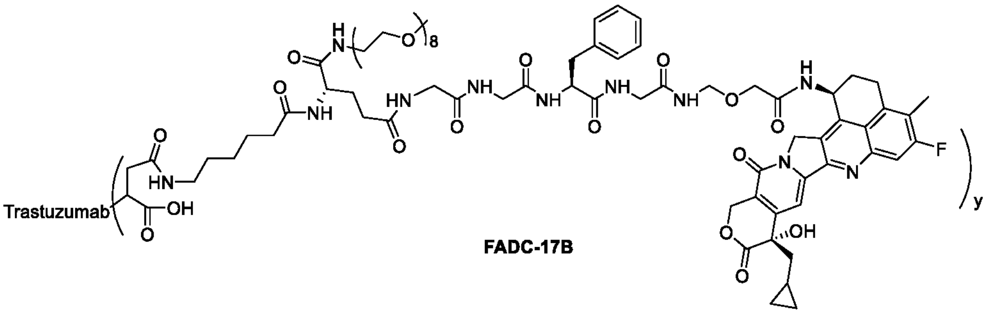

FADC-17B

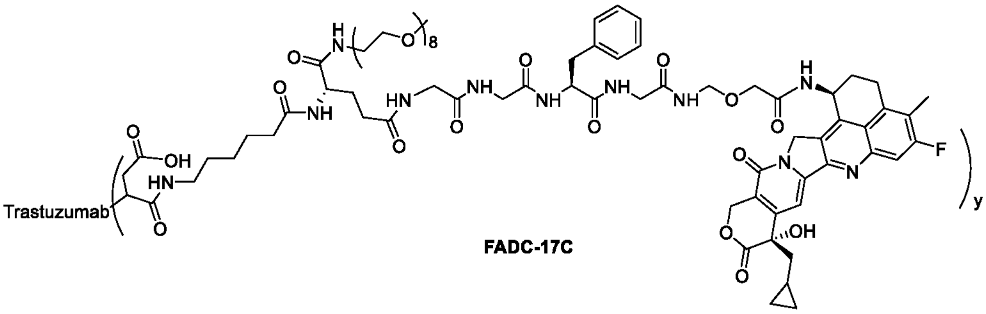

FADC-17C

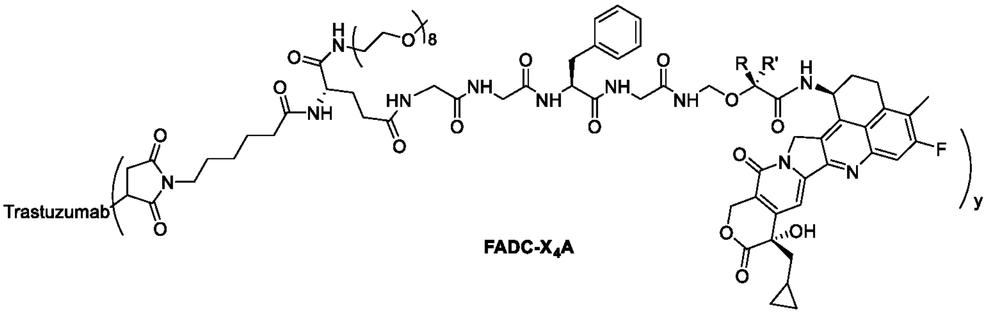

FADC-$X_4$A

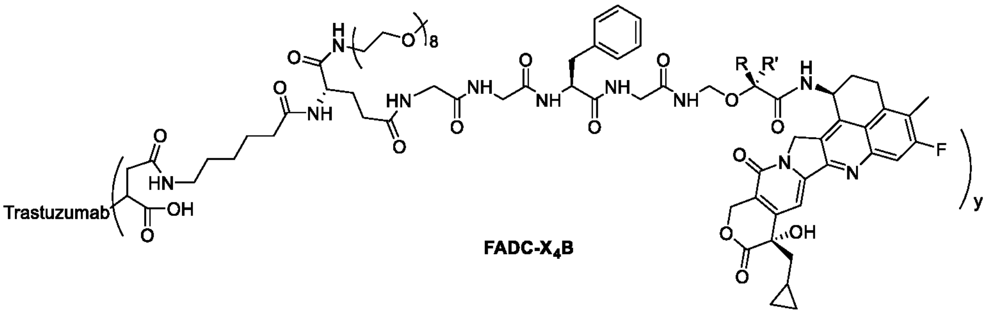

FADC-$X_4$B

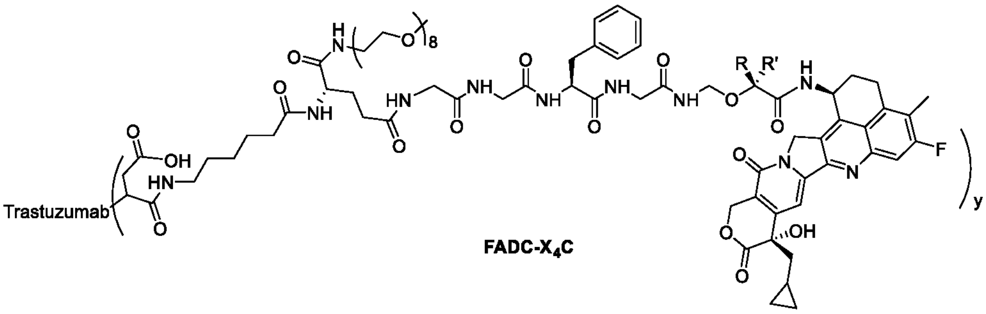

FADC-$X_4$C

wherein, R is selected from $-CH_3$, R' is selected from -H;
R is selected from -H, R' is selected from $-CH_3$;
R is selected from

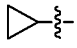,

R' is selected from -H; or
R is selected from -H, R' is selected from

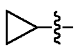;

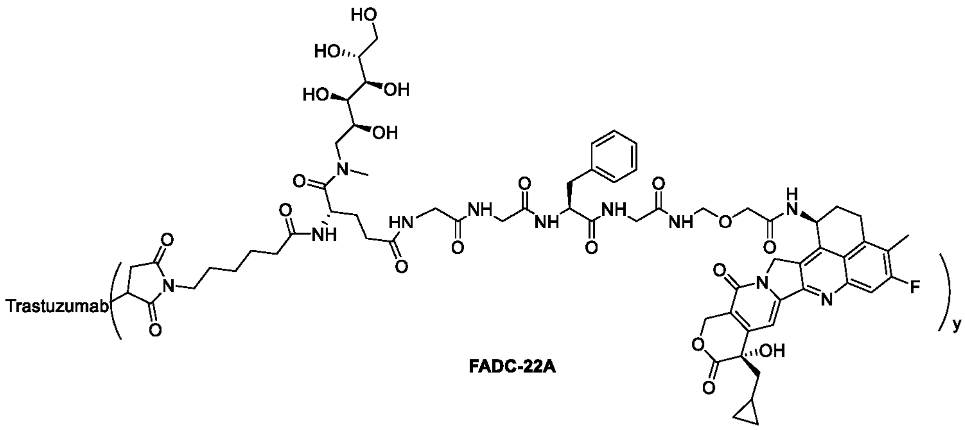

FADC-22A

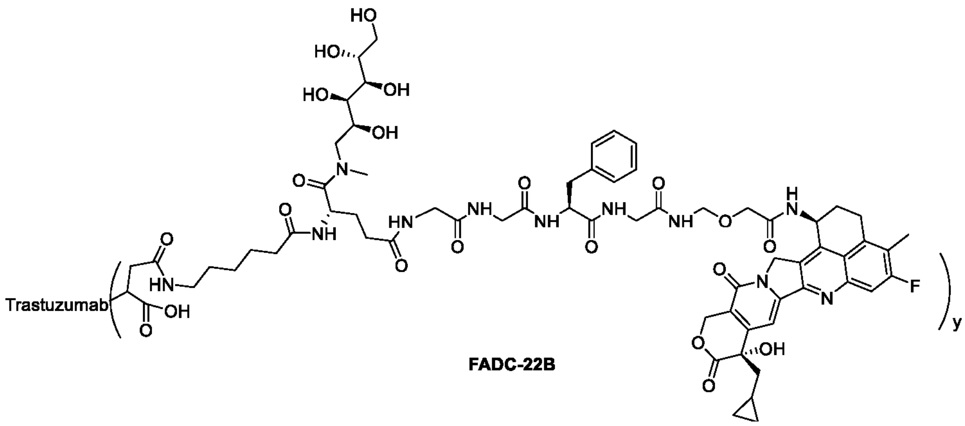

FADC-22B

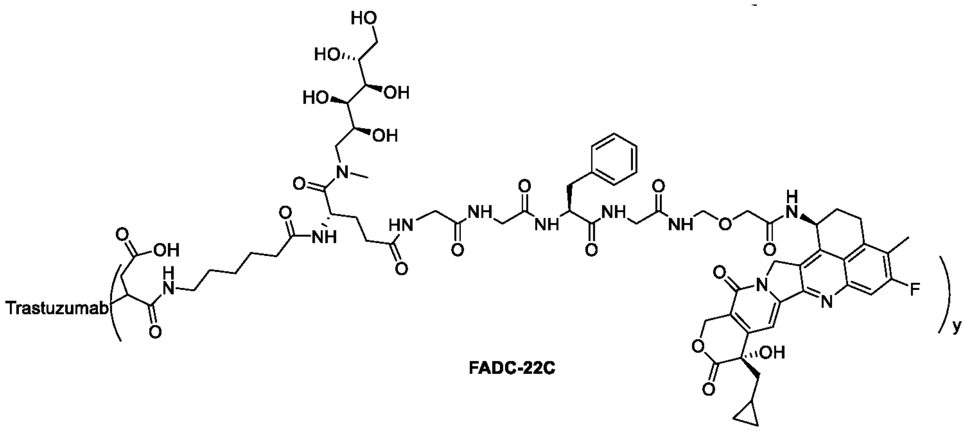

FADC-22C

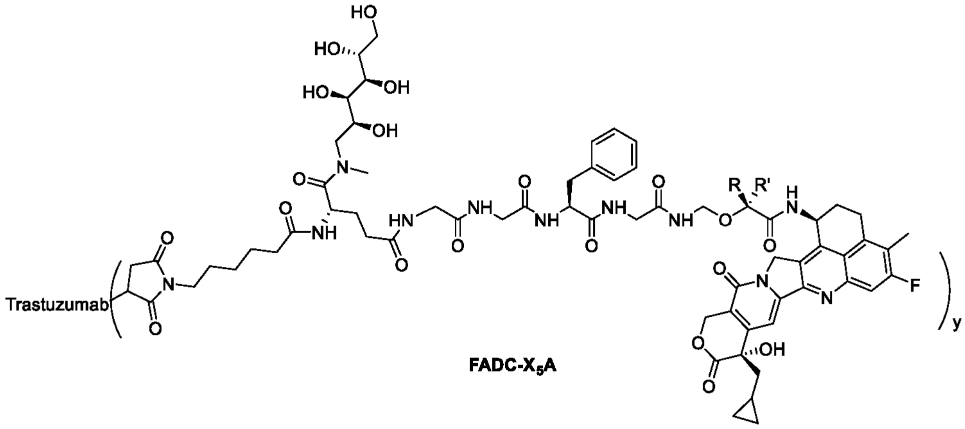

FADC-$X_5$A

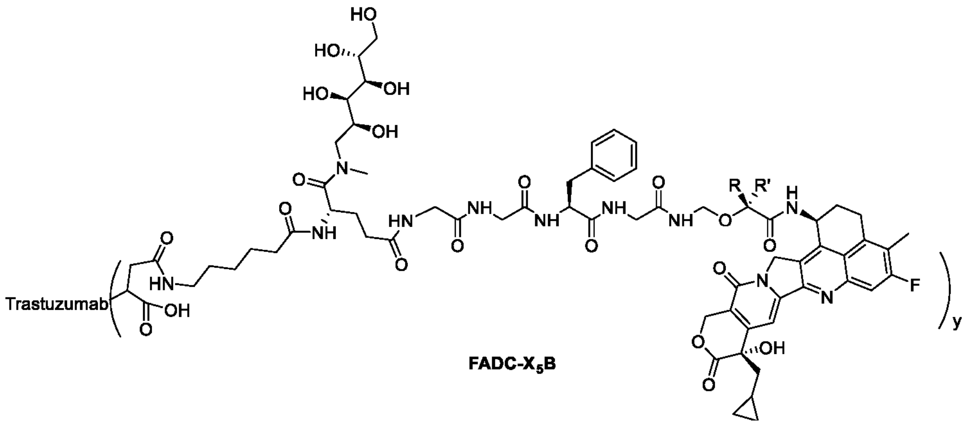

FADC-$X_5$B

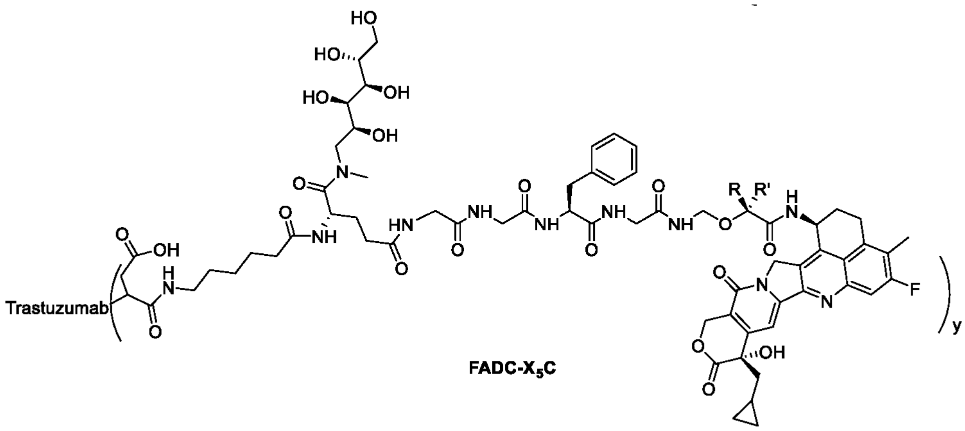

FADC-$X_5$C

wherein, R is selected from $-CH_3$, R' is selected from -H;
R is selected from -H, R' is selected from $-CH_3$;
R is selected from

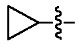,

R' is selected from -H; or
R is selected from -H, R' is selected from

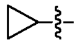;

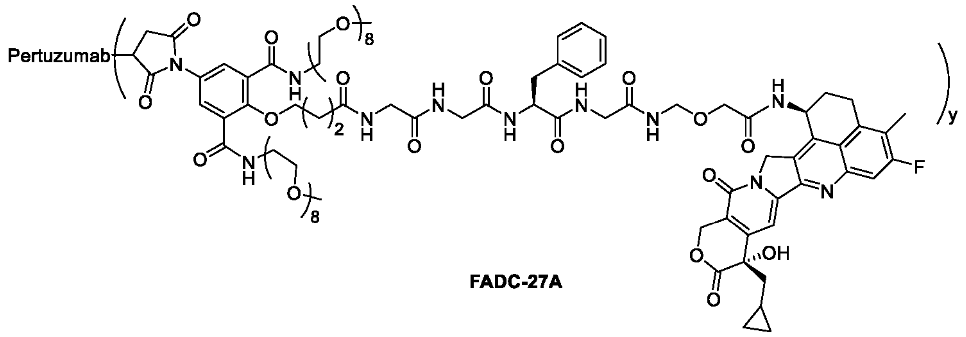

FADC-27A

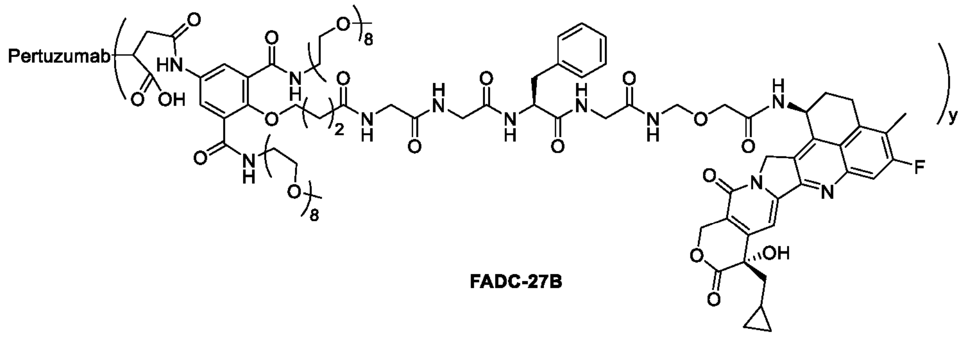

FADC-27B

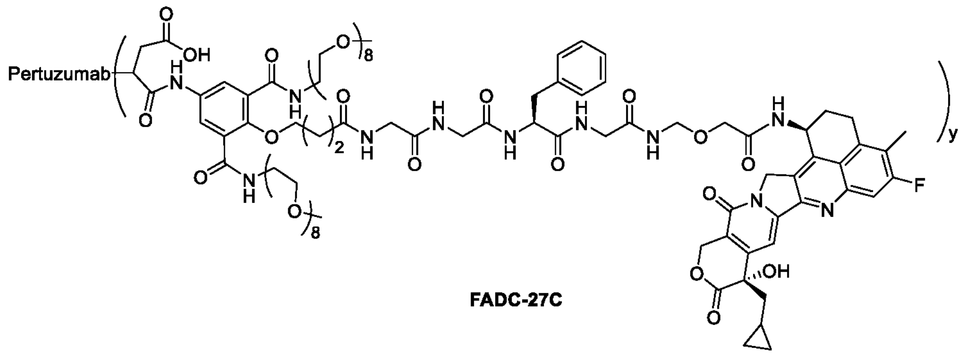

FADC-27C

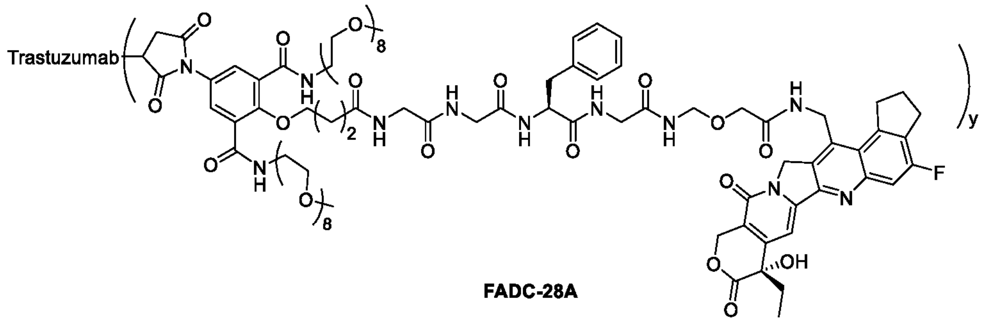

FADC-28A

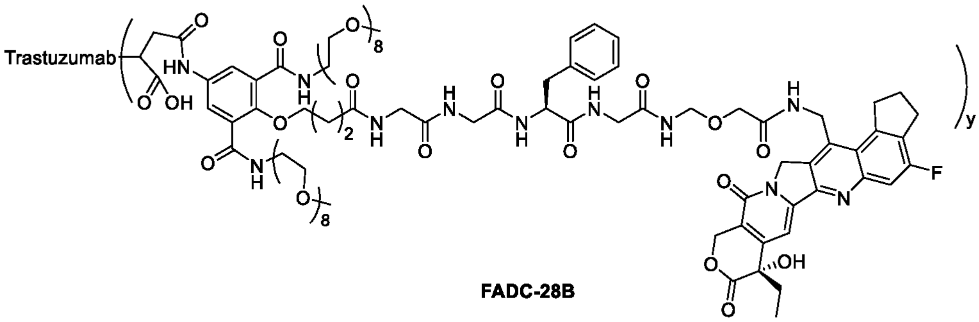

FADC-28B

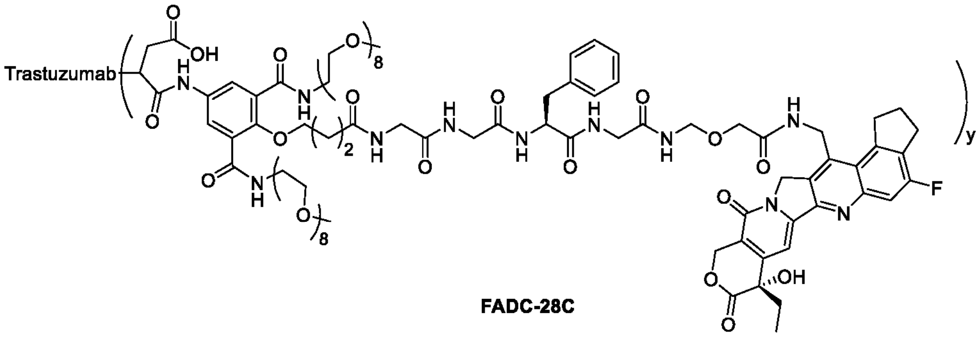

FADC-28C

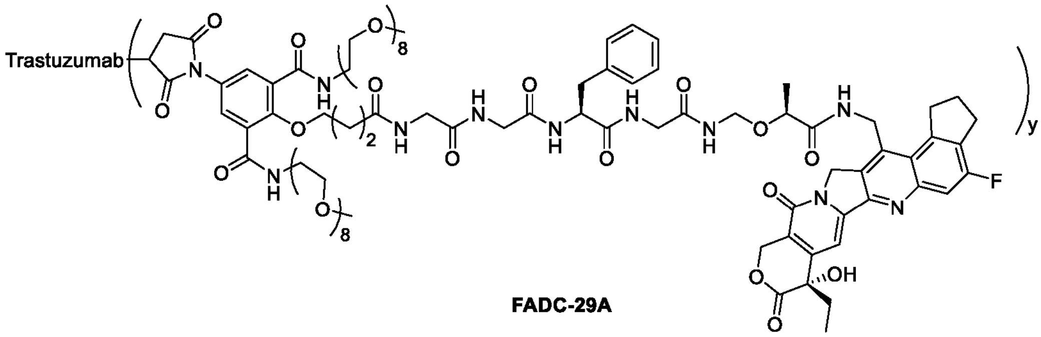

FADC-29A

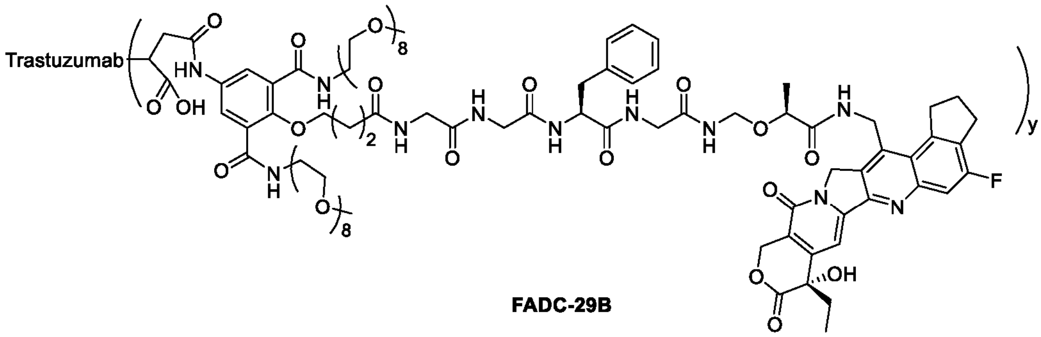

FADC-29B

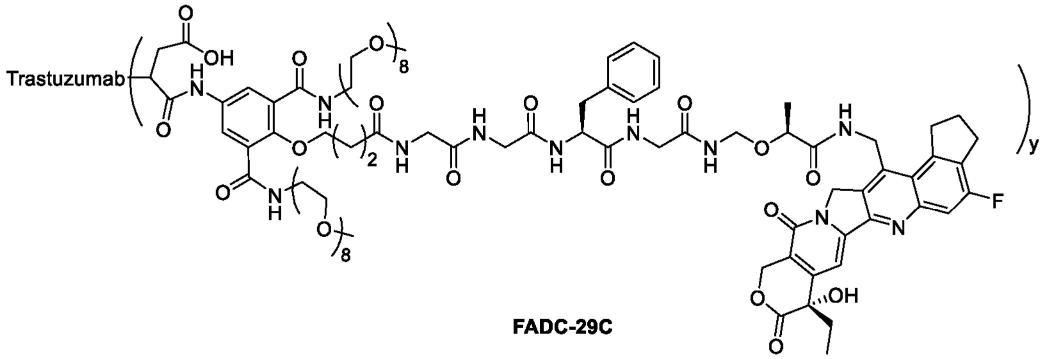

FADC-29C

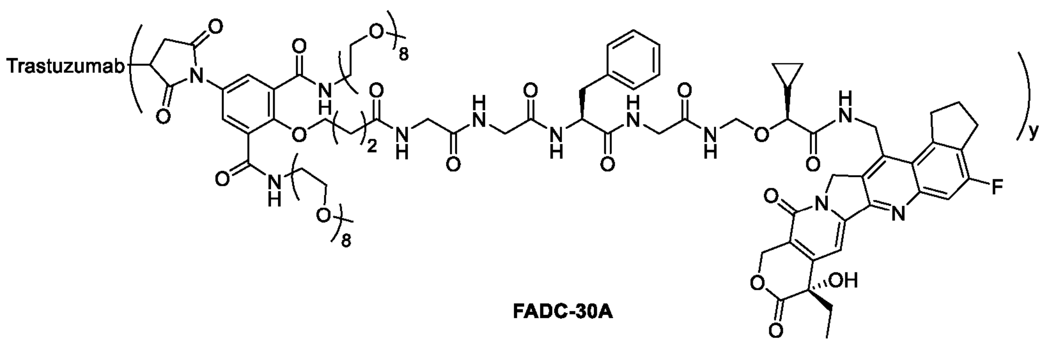

FADC-30A

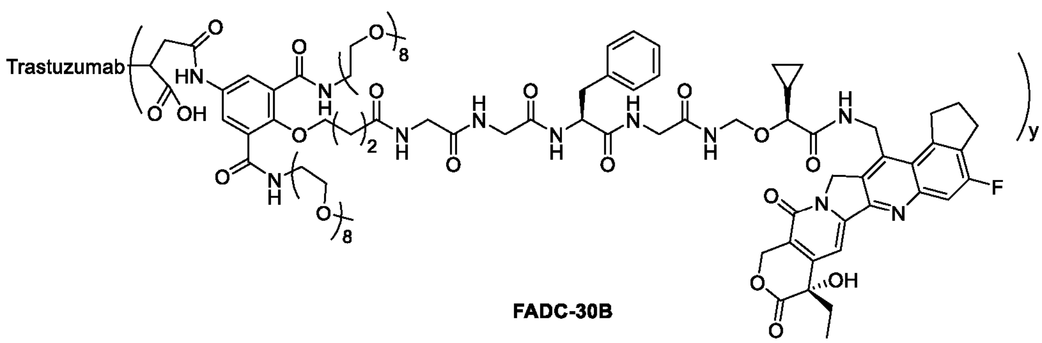

FADC-30B

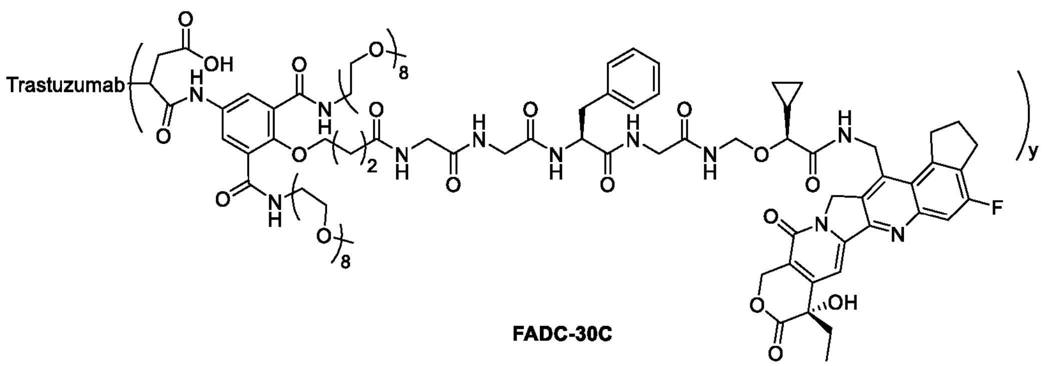

FADC-30C

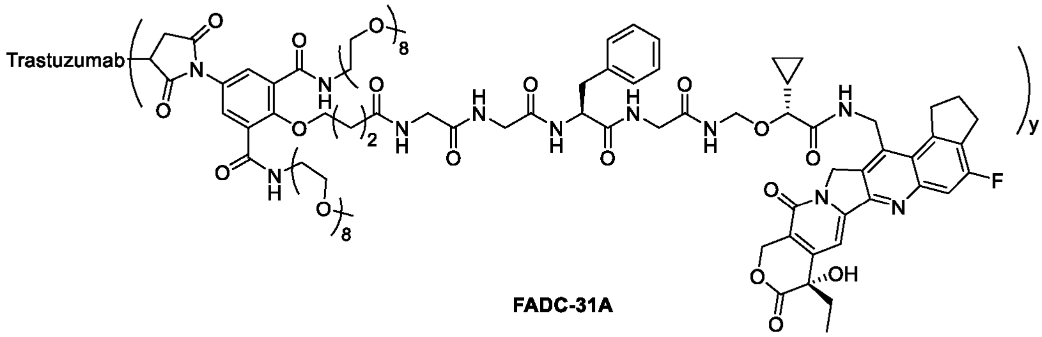

FADC-31A

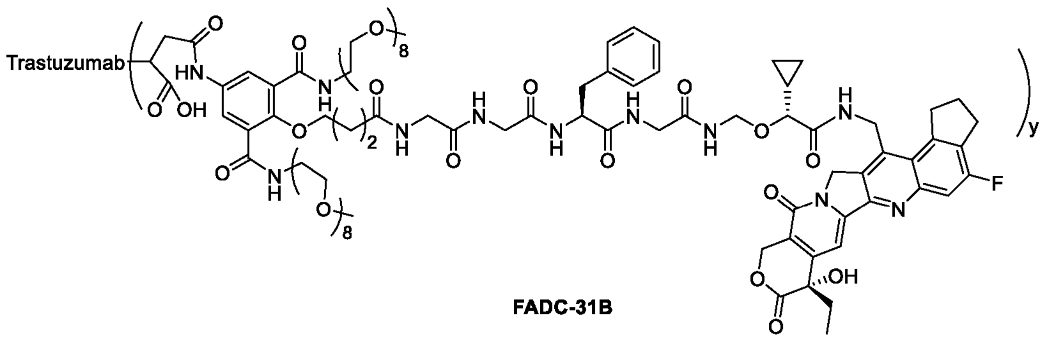

FADC-31B

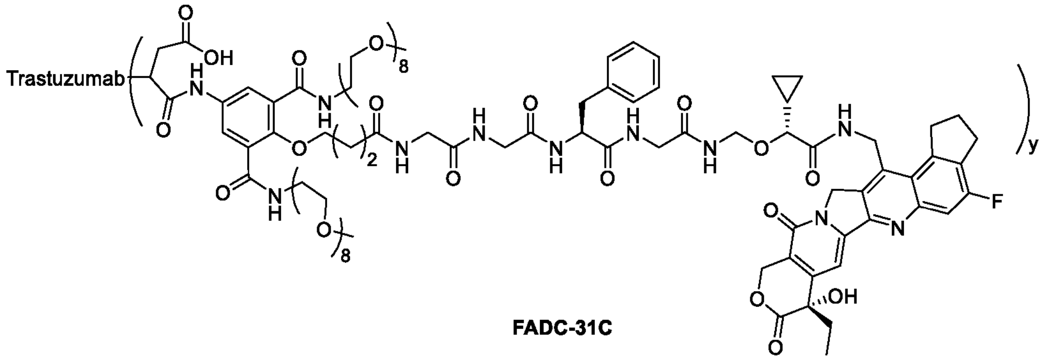

FADC-31C

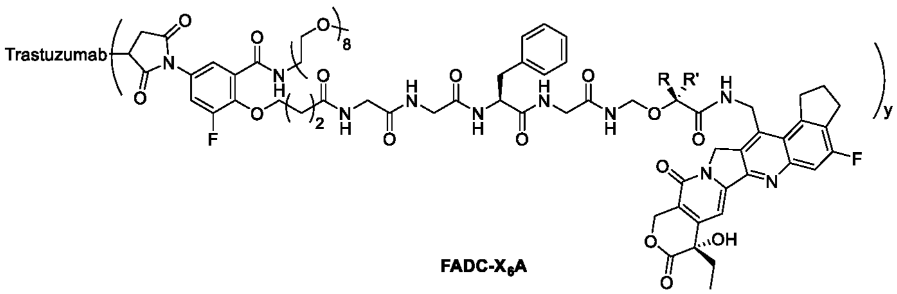

FADC-$X_6$A

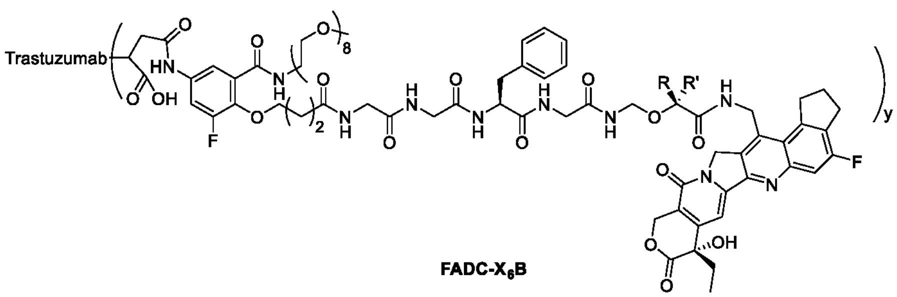

FADC-$X_6$B

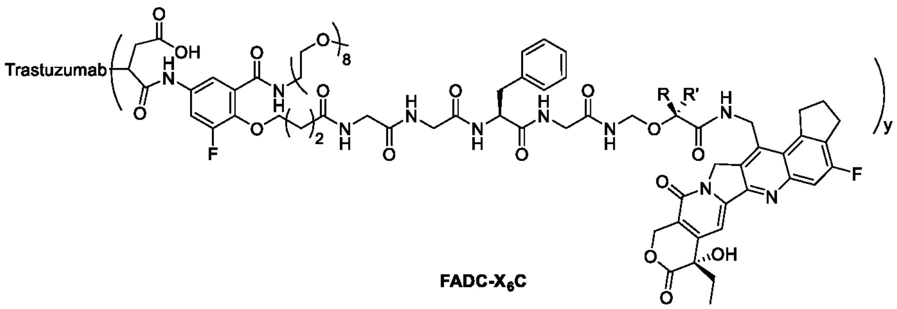

FADC-$X_6$C

wherein, R is selected from -H, R' is selected from -H;
R is selected from -$CH_3$, R' is selected from -H;
R is selected from

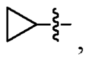,

R' is selected from -H; or
R is selected from -H, R' is selected from

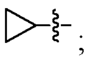;

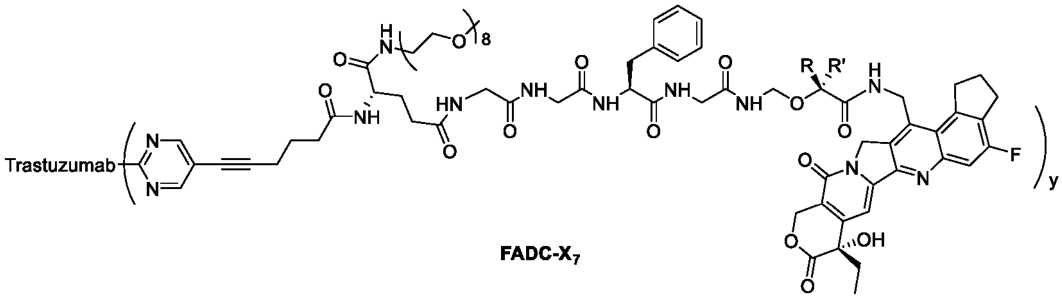

FADC-$X_7$

wherein, R is selected from -H, R' is selected from -H;
R is selected from -$CH_3$, R' is selected from -H; or
R is selected from

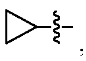;

R' is selected from -H;

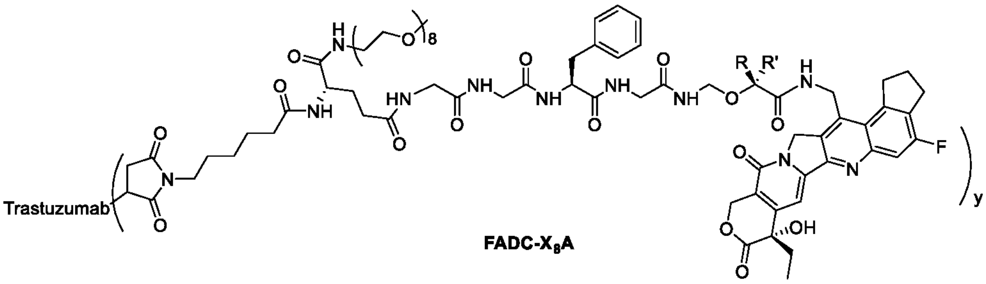

FADC-$X_8$A

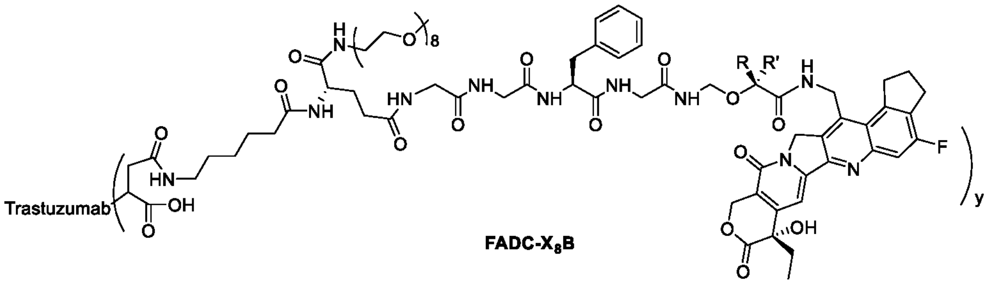

FADC-$X_8$B

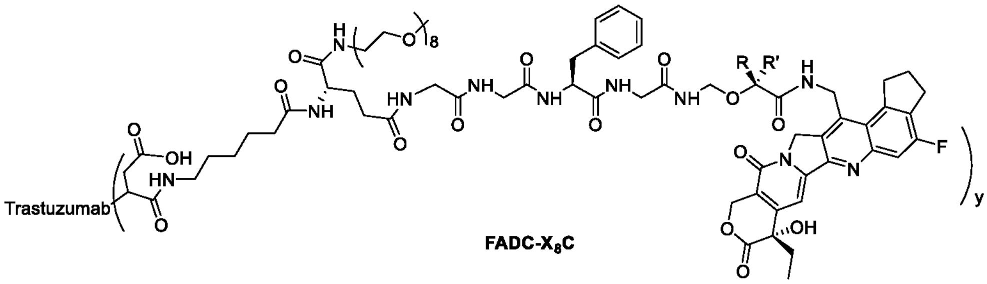

FADC-$X_8$C

wherein, R is selected from -H, R' is selected from -H;
R is selected from -$CH_3$, R' is selected from -H;
R is selected from

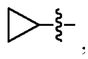

R' is selected from -H; or
R is selected from -H, R' is selected from

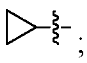

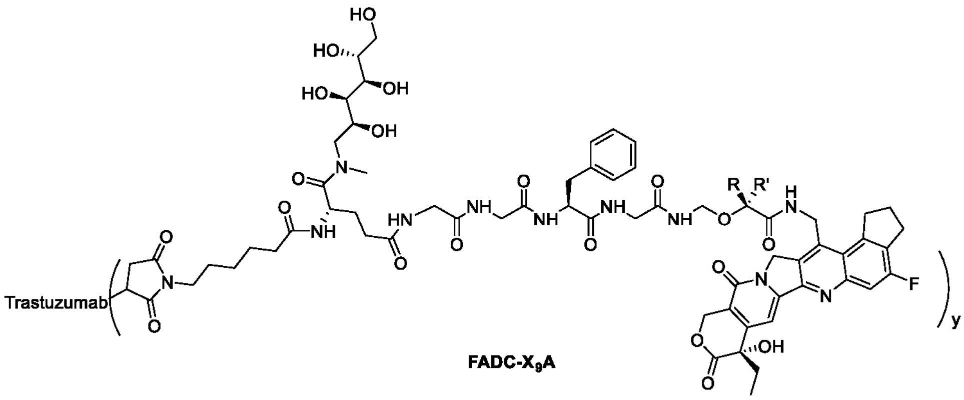

FADC-$X_9$A

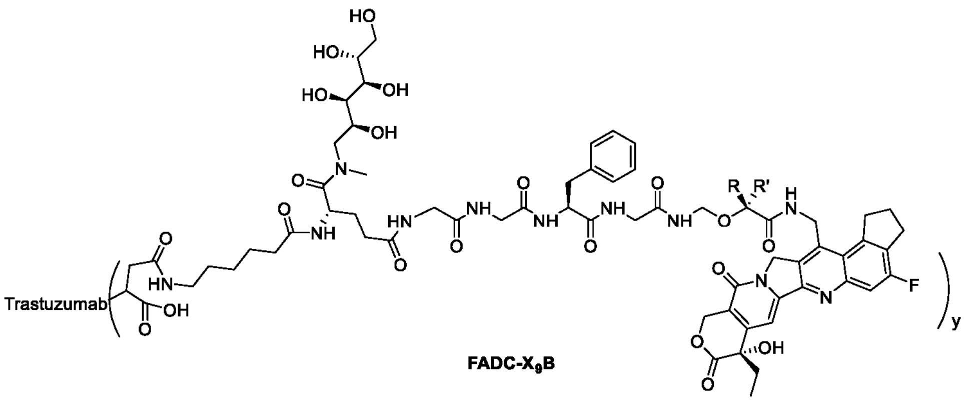

FADC-$X_9$B

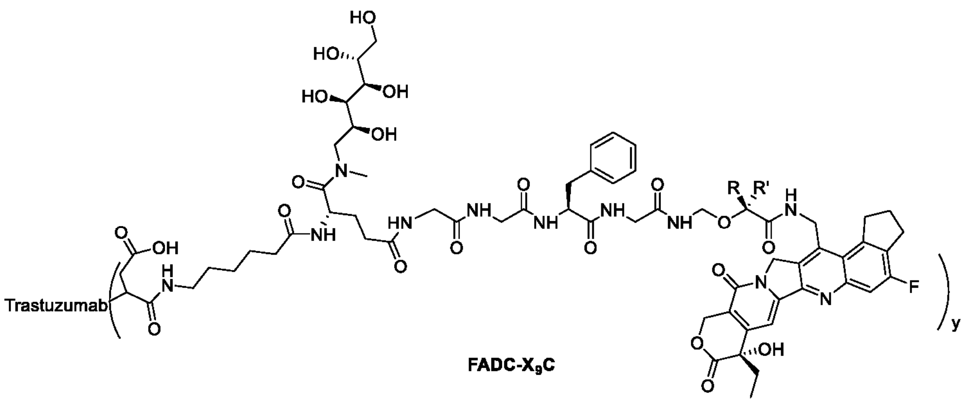

FADC-$X_9$C

wherein, R is selected from -H, R' is selected from -H;
R is selected from $-CH_3$, R' is selected from -H; or
R is selected from

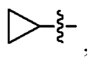

R' is selected from -H;

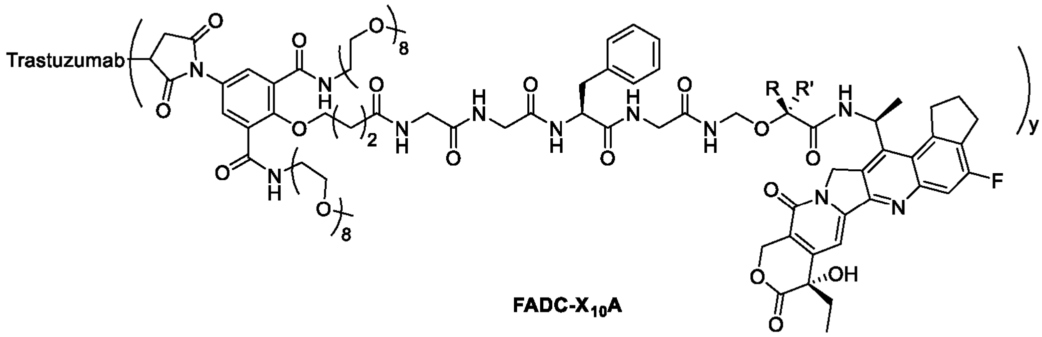

**FADC-X$_{10}$A**

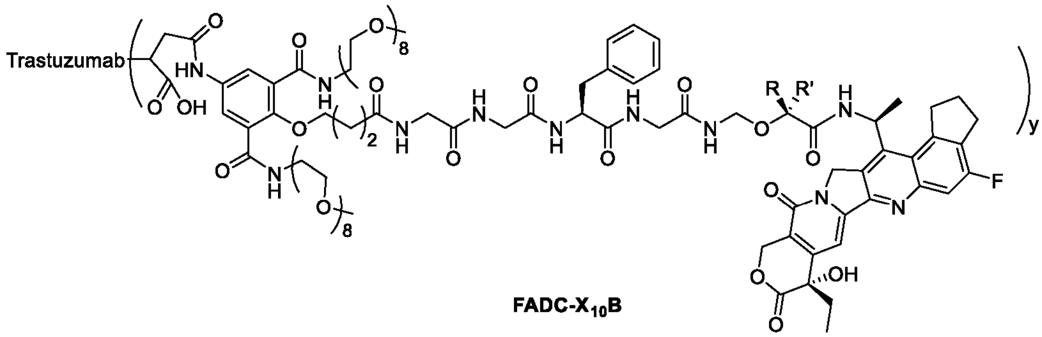

**FADC-X$_{10}$B**

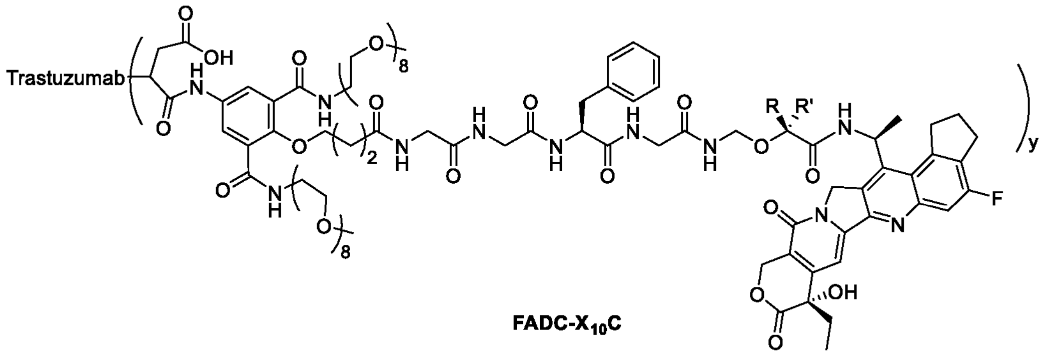

**FADC-X$_{10}$C**

wherein, R is selected from -H, R' is selected from -H;
R is selected from

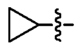

,

R' is selected from -H; or
R is selected from -H, R' is selected from

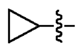

;

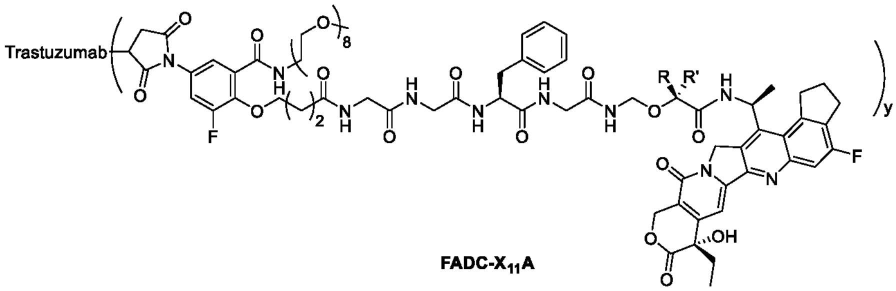

**FADC-X$_{11}$A**

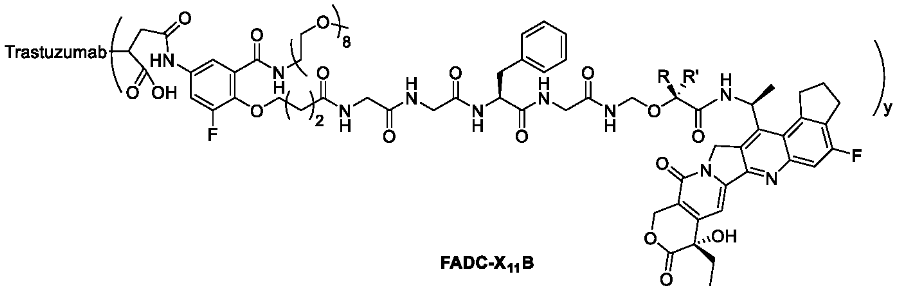

FADC-$X_{11}$B

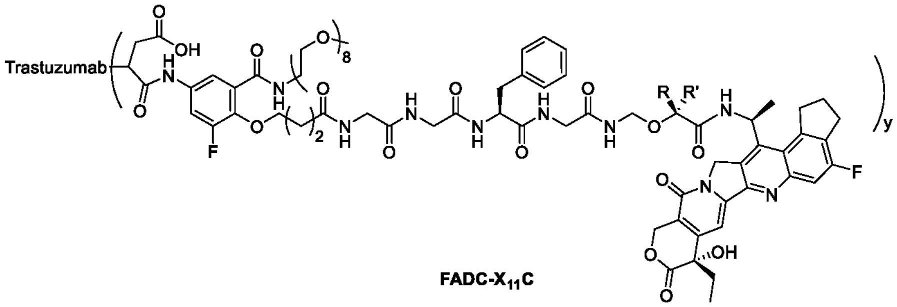

FADC-$X_{11}$C

wherein, R is selected from -H, R' is selected from -H;
R is selected from

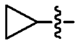

,

R' is selected from -H; or
R is selected from -H, R' is selected from

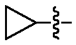

;

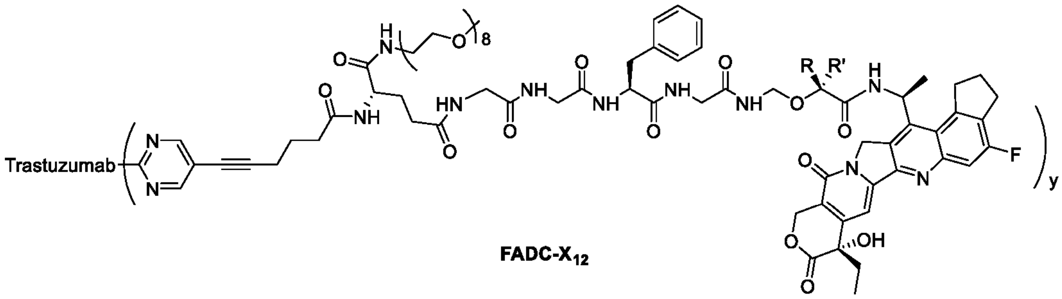

FADC-$X_{12}$

wherein, R is selected from -H, R' is selected from -H;
R is selected from

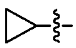

,

R' is selected from -H; or
R is selected from -H, , R' is selected from

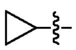

;

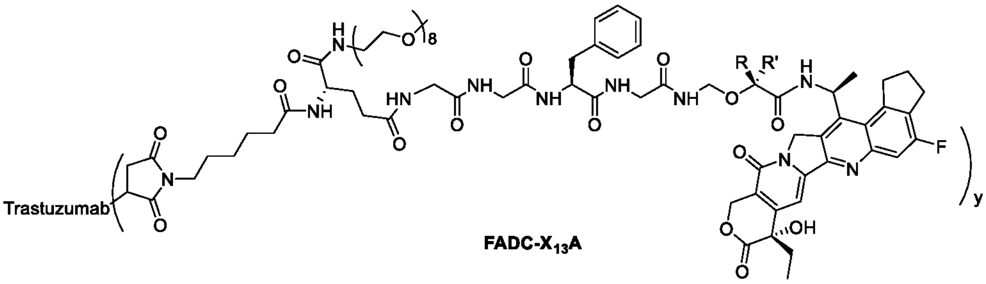

FADC-$X_{13}$A

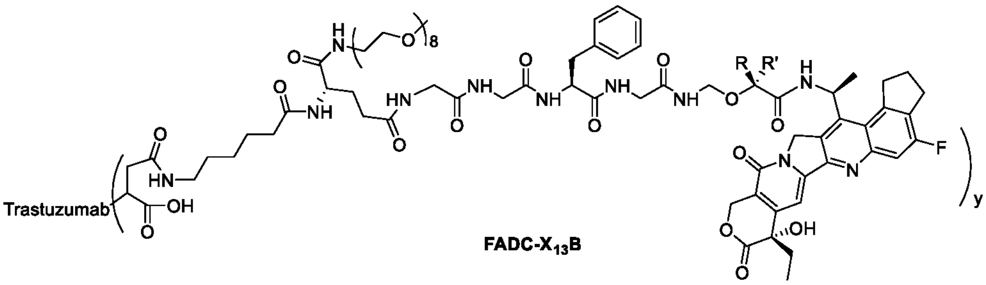

FADC-$X_{13}$B

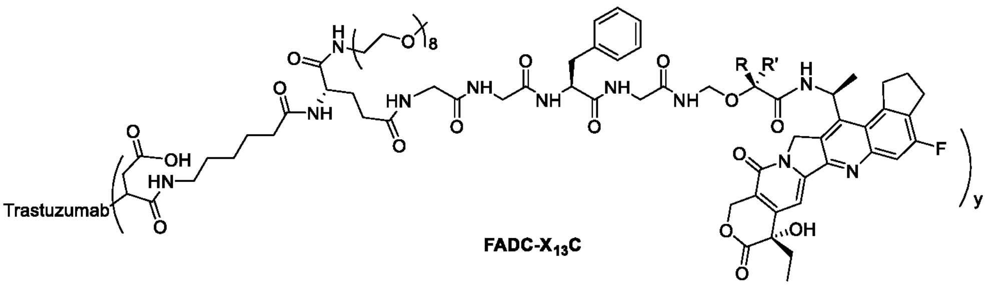

FADC-$X_{13}$C

wherein, R is selected from -H, R' is selected from -H;
R is selected from

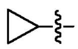

,

R' is selected from -H; or
R is selected from -H, R' is selected from

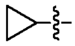;

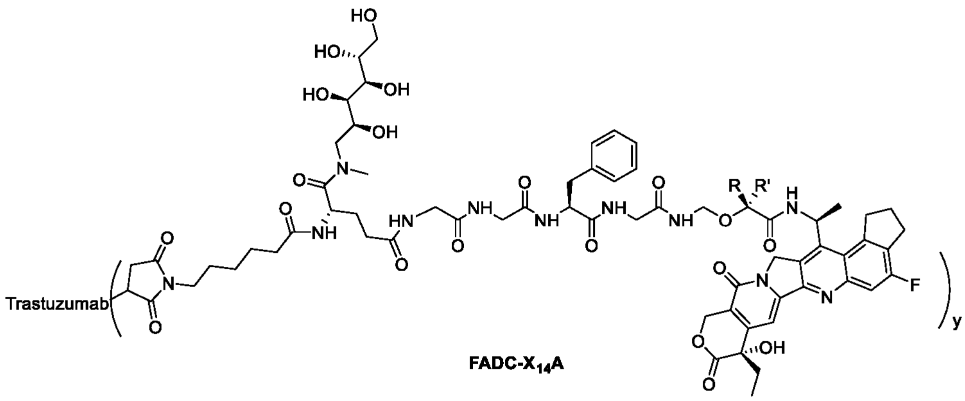

FADC-$X_{14}$A

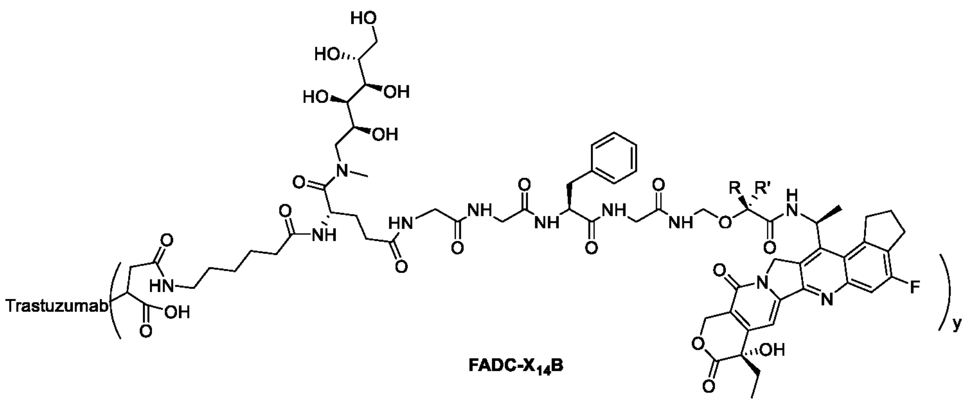

FADC-$X_{14}$B

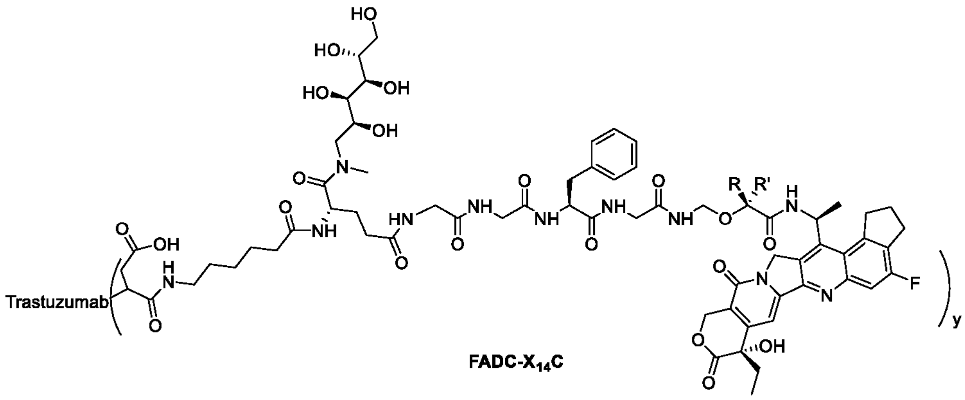

FADC-$X_{14}$C

wherein, R is selected from -H, R' is selected from -H;
R is selected from

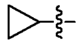,

R' is selected from -H; or
R is selected from -H, R' is selected from

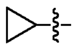;

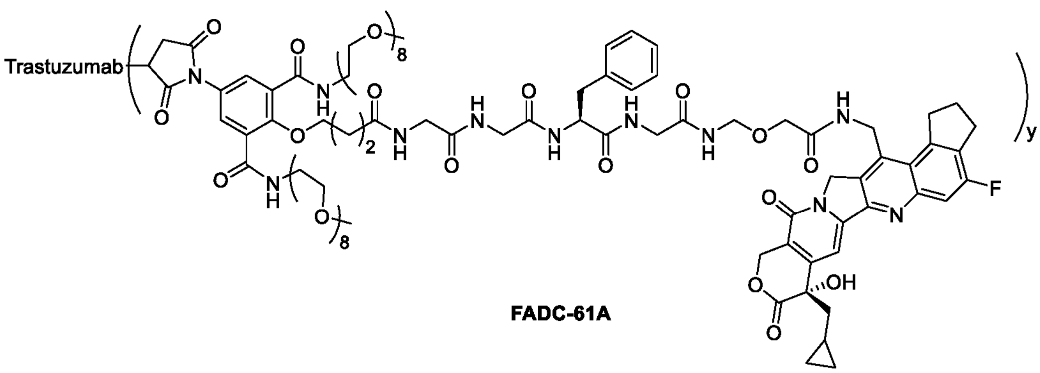

FADC-61A

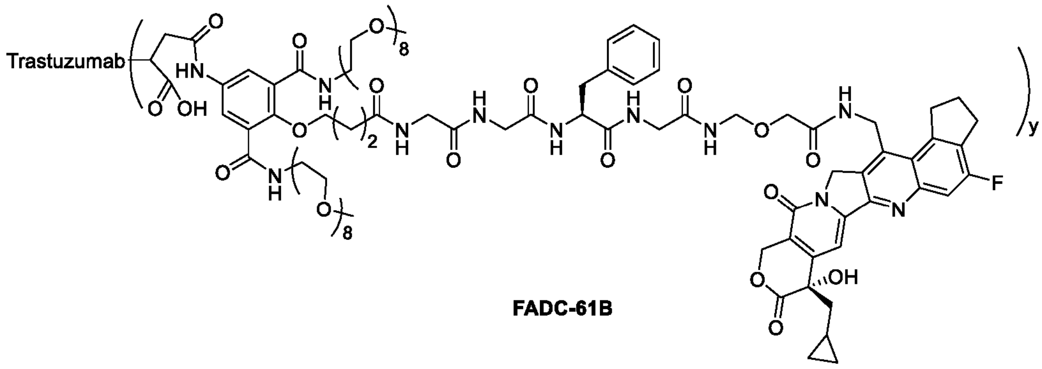

**FADC-61B**

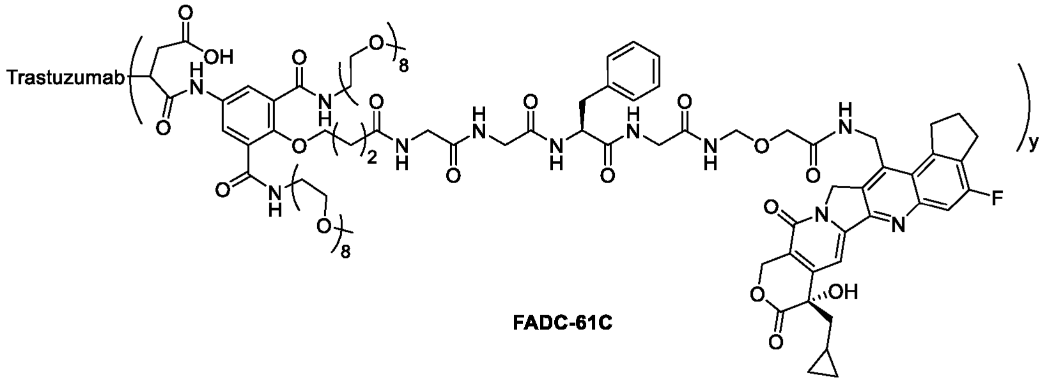

**FADC-61C**

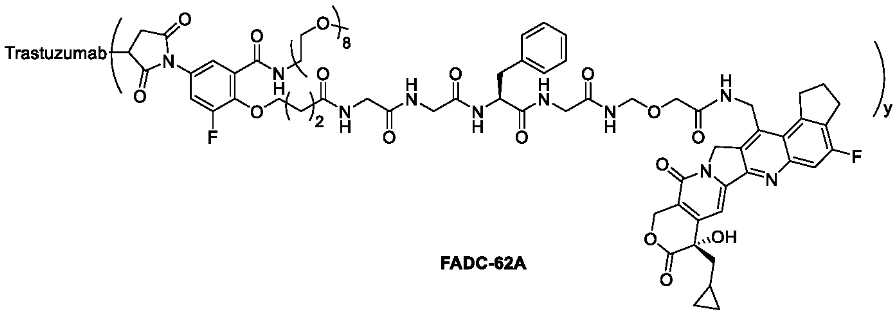

**FADC-62A**

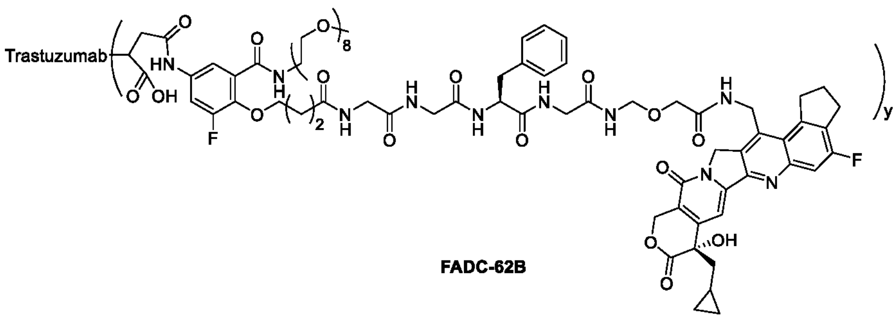

**FADC-62B**

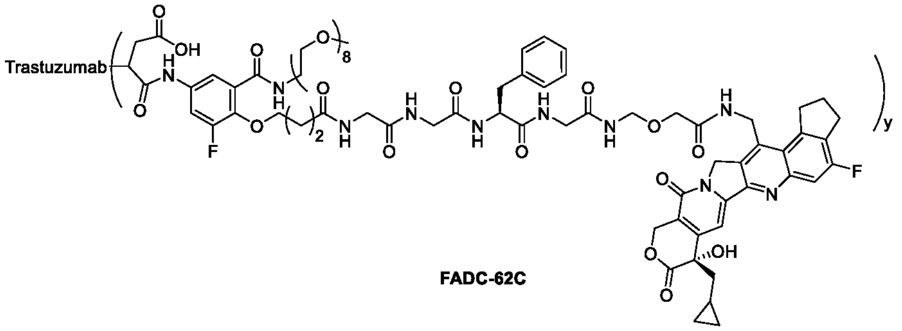

FADC-62C

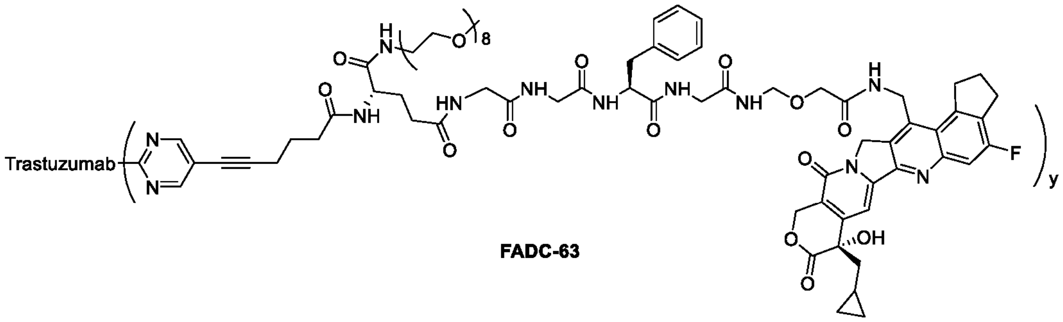

FADC-63

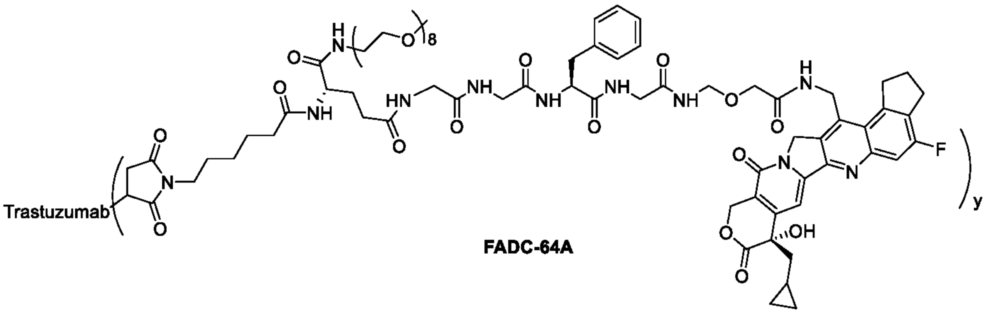

FADC-64A

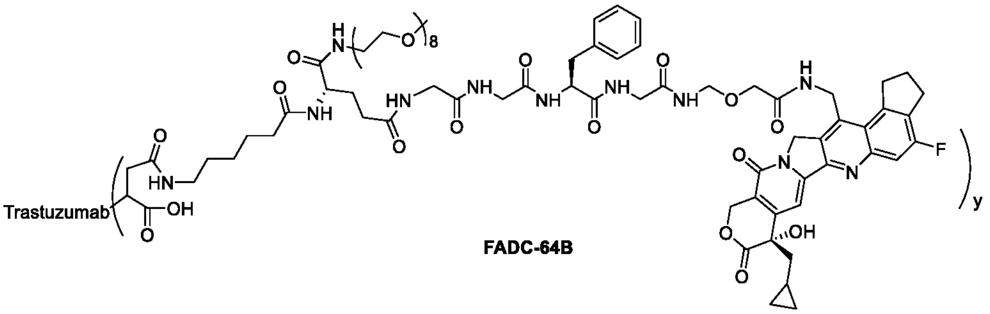

FADC-64B

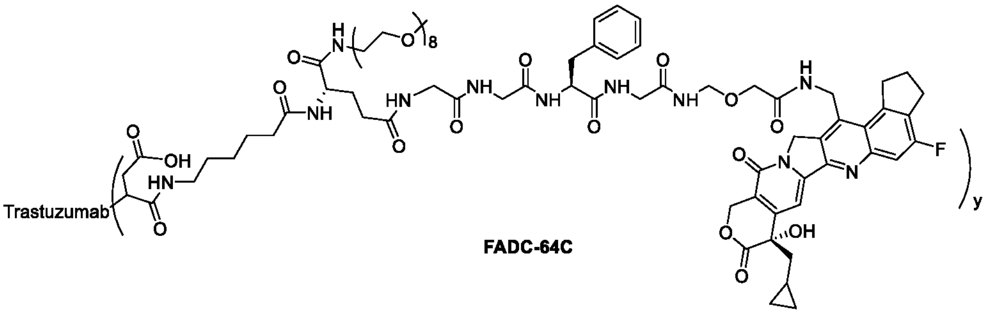

FADC-64C

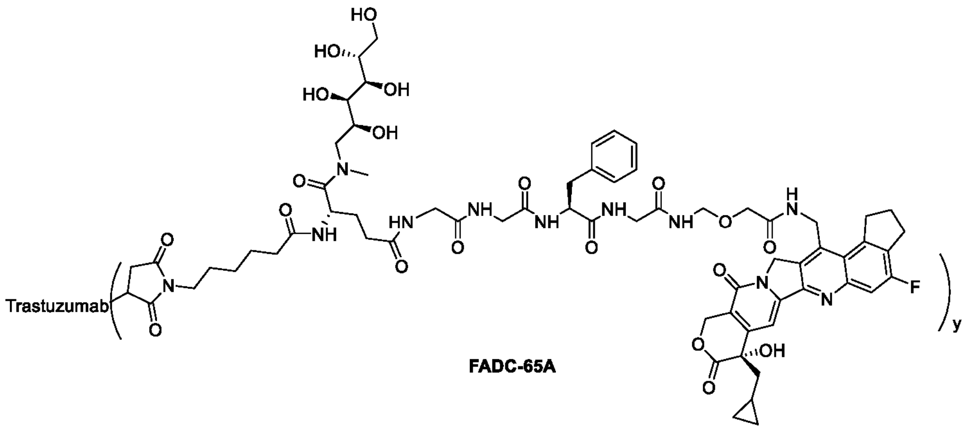

**FADC-65A**

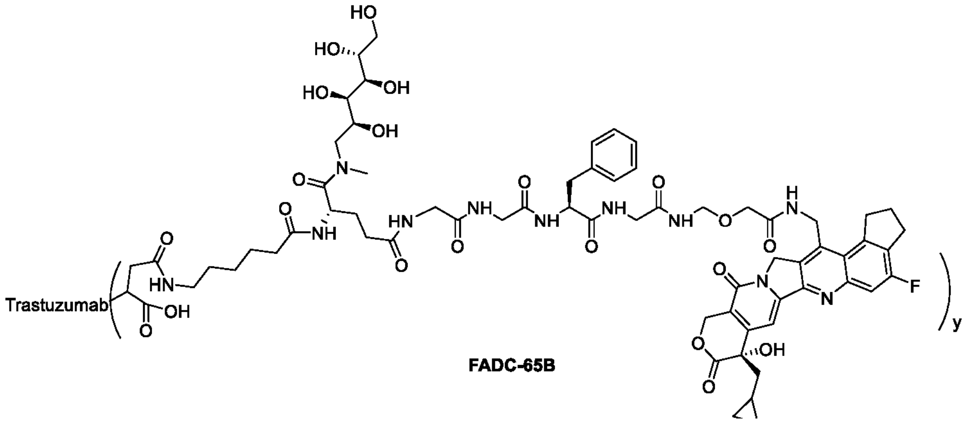

**FADC-65B**

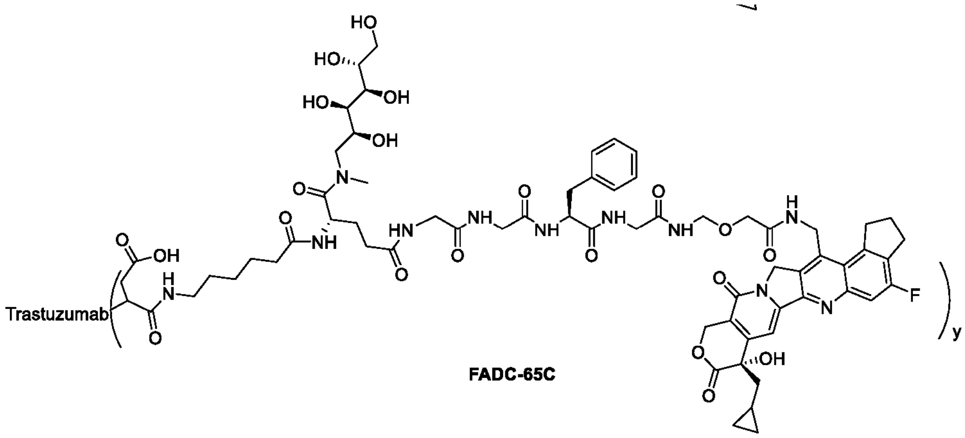

**FADC-65C**

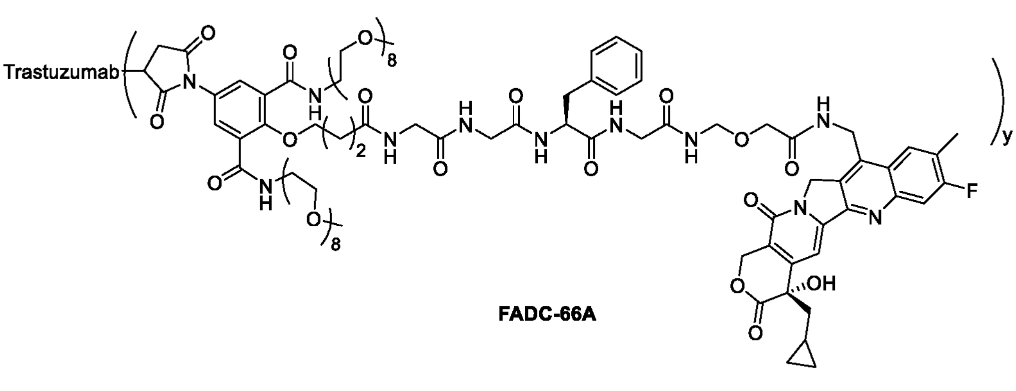

**FADC-66A**

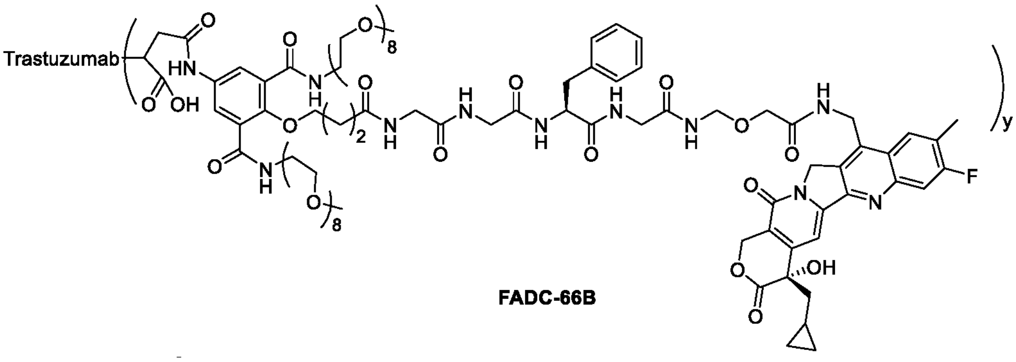

**FADC-66B**

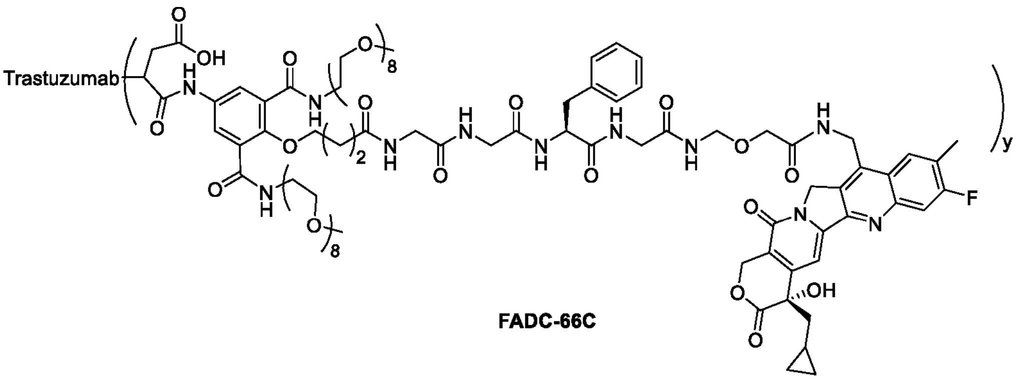

**FADC-66C**

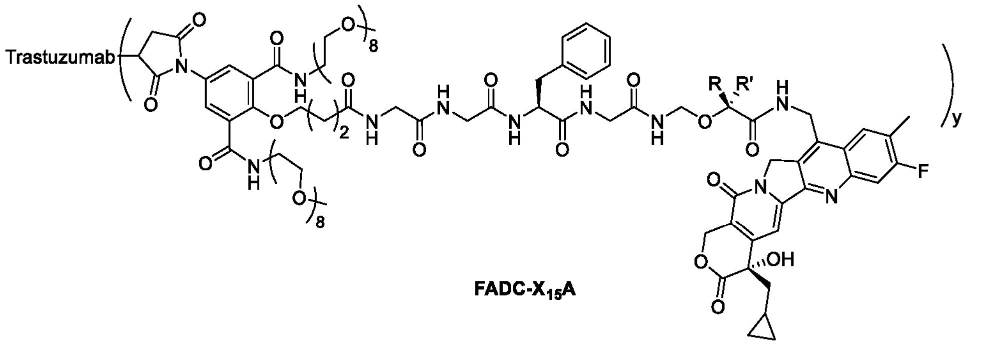

**FADC-$X_{15}$A**

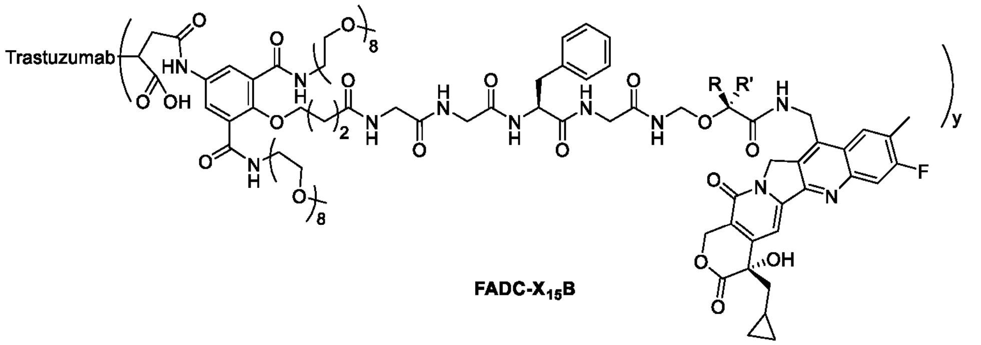

**FADC-$X_{15}$B**

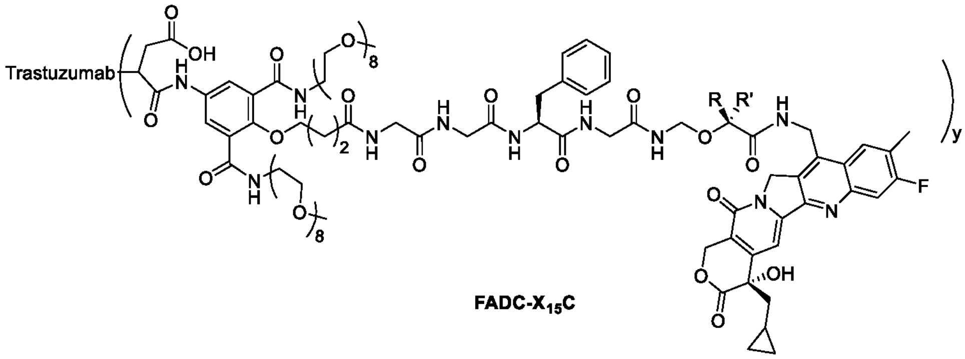

FADC-X$_{15}$C

wherein, R is selected from -$CH_3$, R' is selected from -H;
R is selected from -H, R' is selected from -$CH_3$;
R is selected from

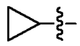,

R' is selected from -H; or
R is selected from -H, R' is selected from

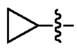;

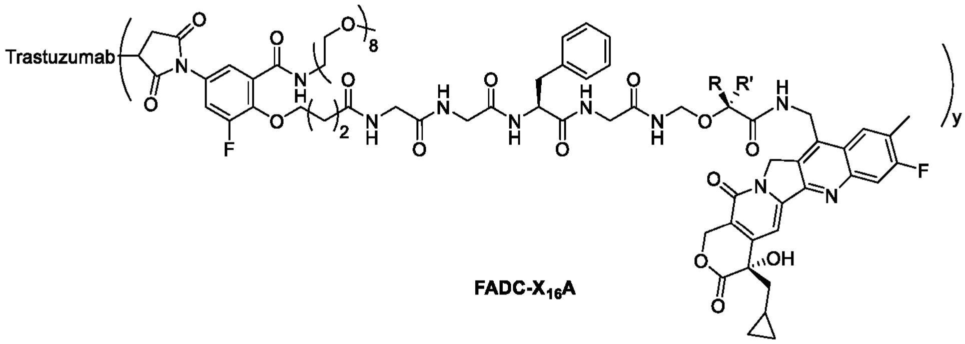

FADC-X$_{16}$A

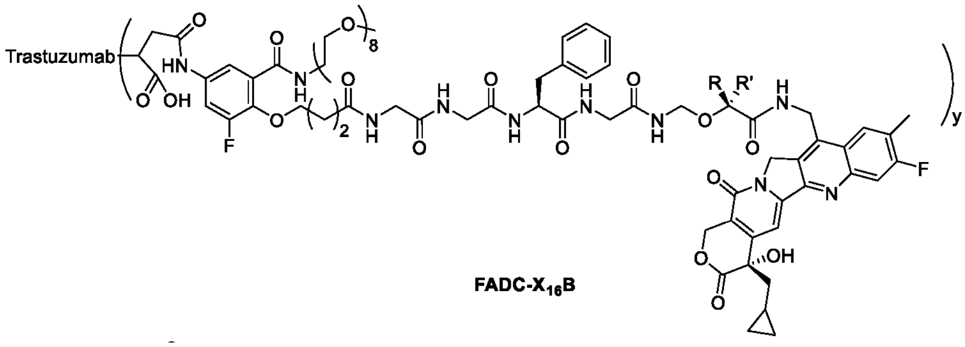

FADC-X$_{16}$B

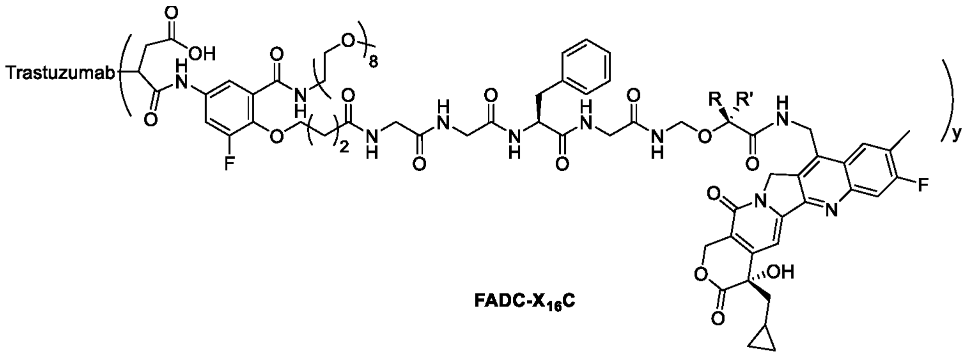

FADC-$X_{16}$C

wherein, R is selected from -H, R' is selected from -H;
R is selected from $-CH_3$, R' is selected from -H;
R is selected from -H, R' is selected from $-CH_3$;
R is selected from

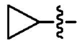,

R' is selected from -H; or
R is selected from -H, R' is selected from

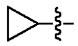;

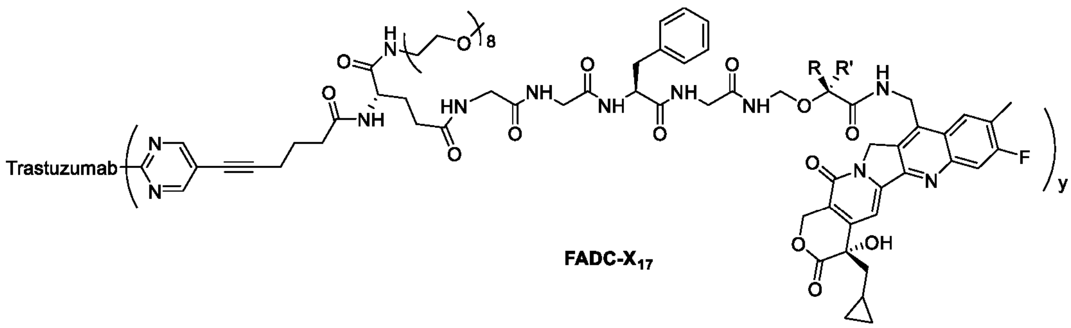

FADC-$X_{17}$

wherein, R is selected from -H, R' is selected from -H;
R is selected from $-CH_3$, R' is selected from -H;
R is selected from -H, R' is selected from $-CH_3$;
R is selected from

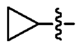,

R' is selected from -H; or
R is selected from -H, R' is selected from

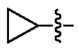;

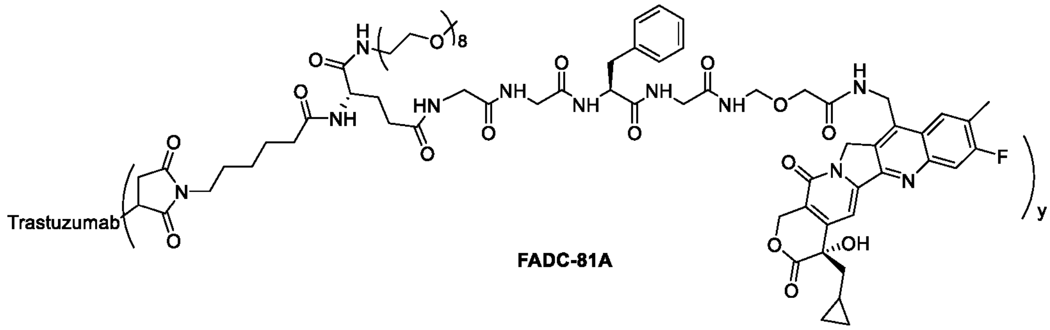

FADC-81A

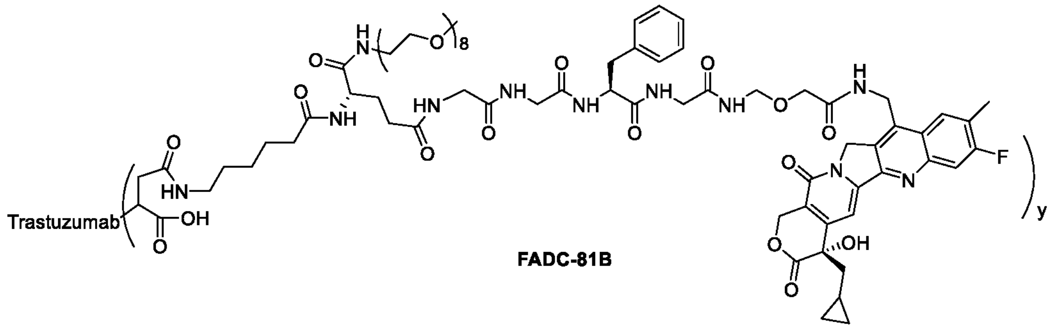

FADC-81B

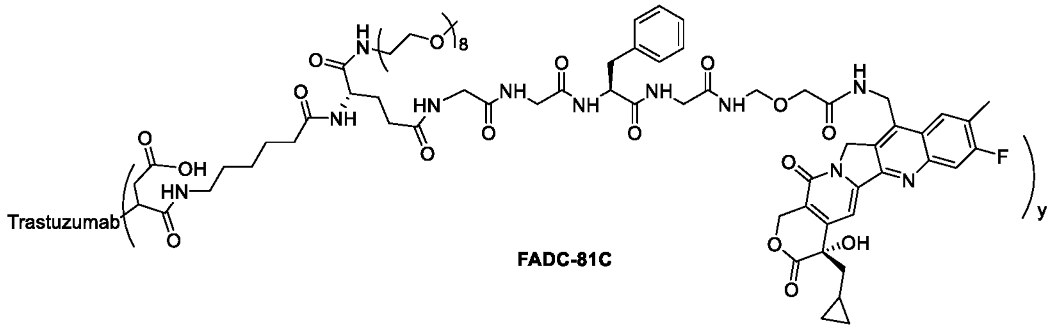

FADC-81C

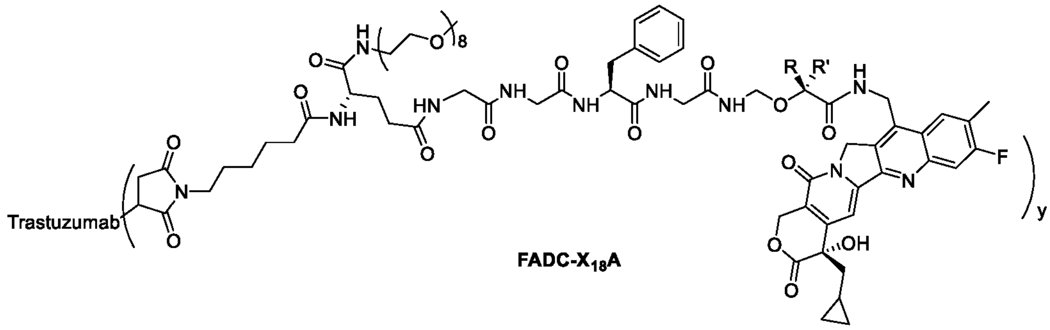

FADC-$X_{18}$A

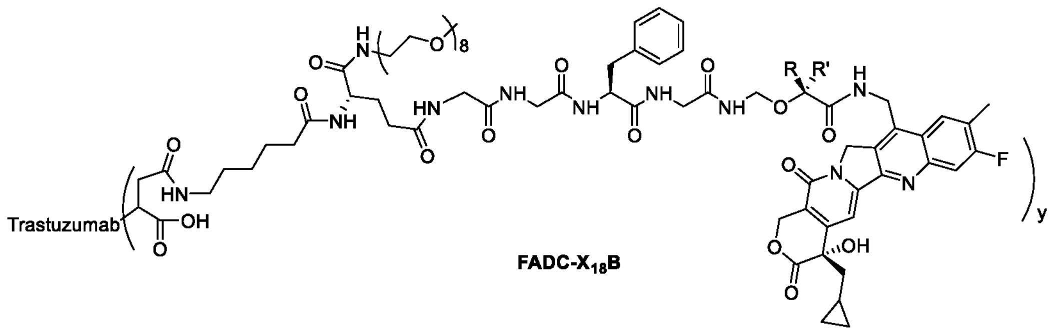

FADC-$X_{18}$B

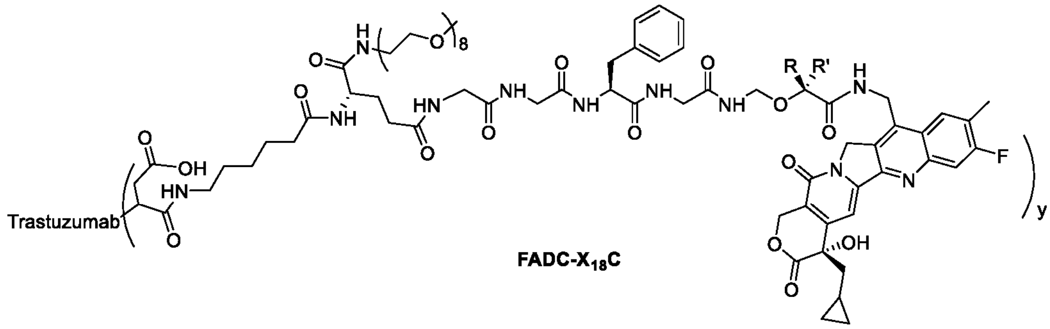

FADC-$X_{18}$C

wherein, R is selected from $-CH_3$, R' is selected from -H;
R is selected from -H, R' is selected from $-CH_3$;
R is selected from

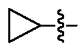,

R' is selected from -H; or
R is selected from -H, R' is selected from

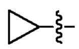;

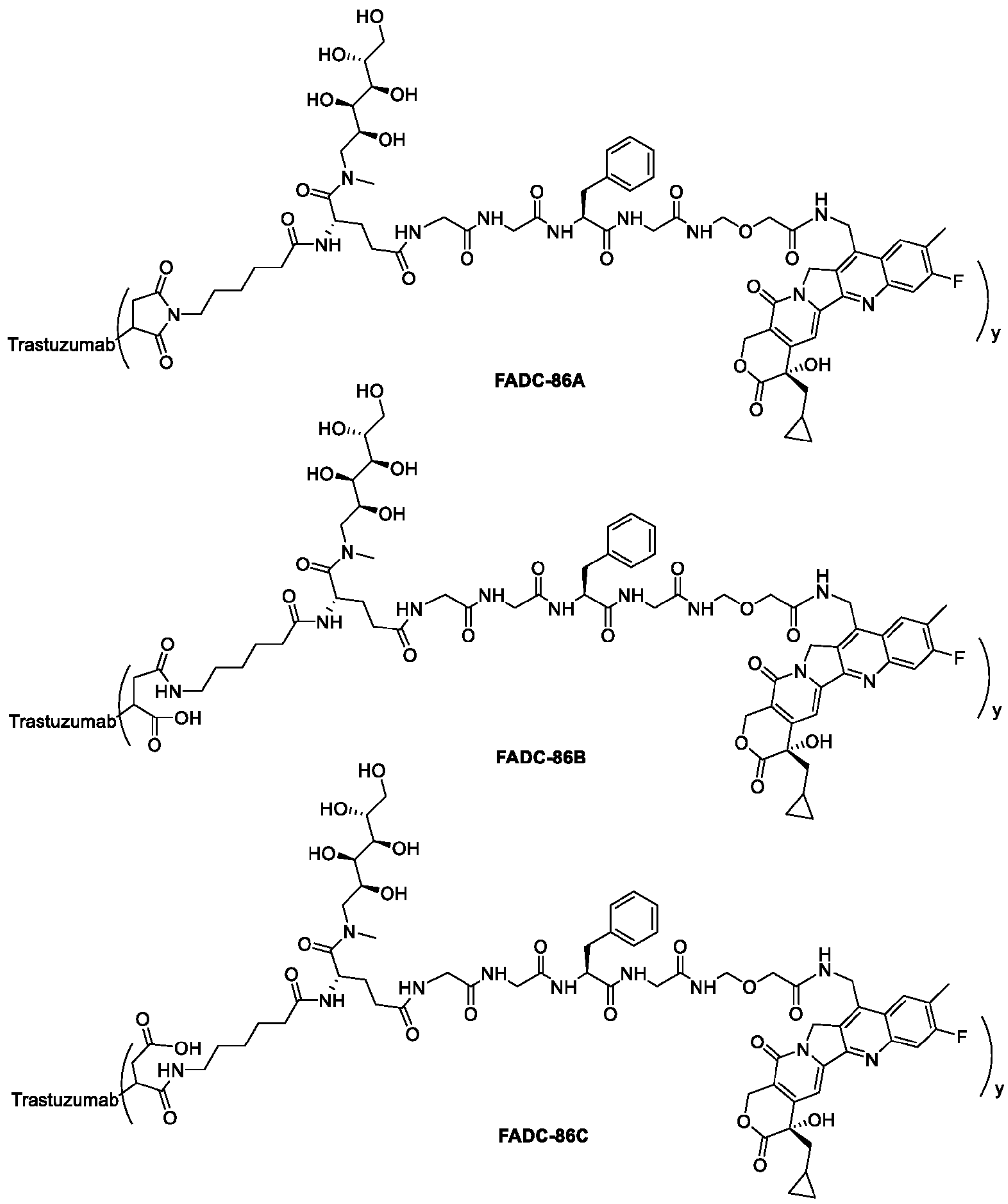

FADC-86A

FADC-86B

FADC-86C

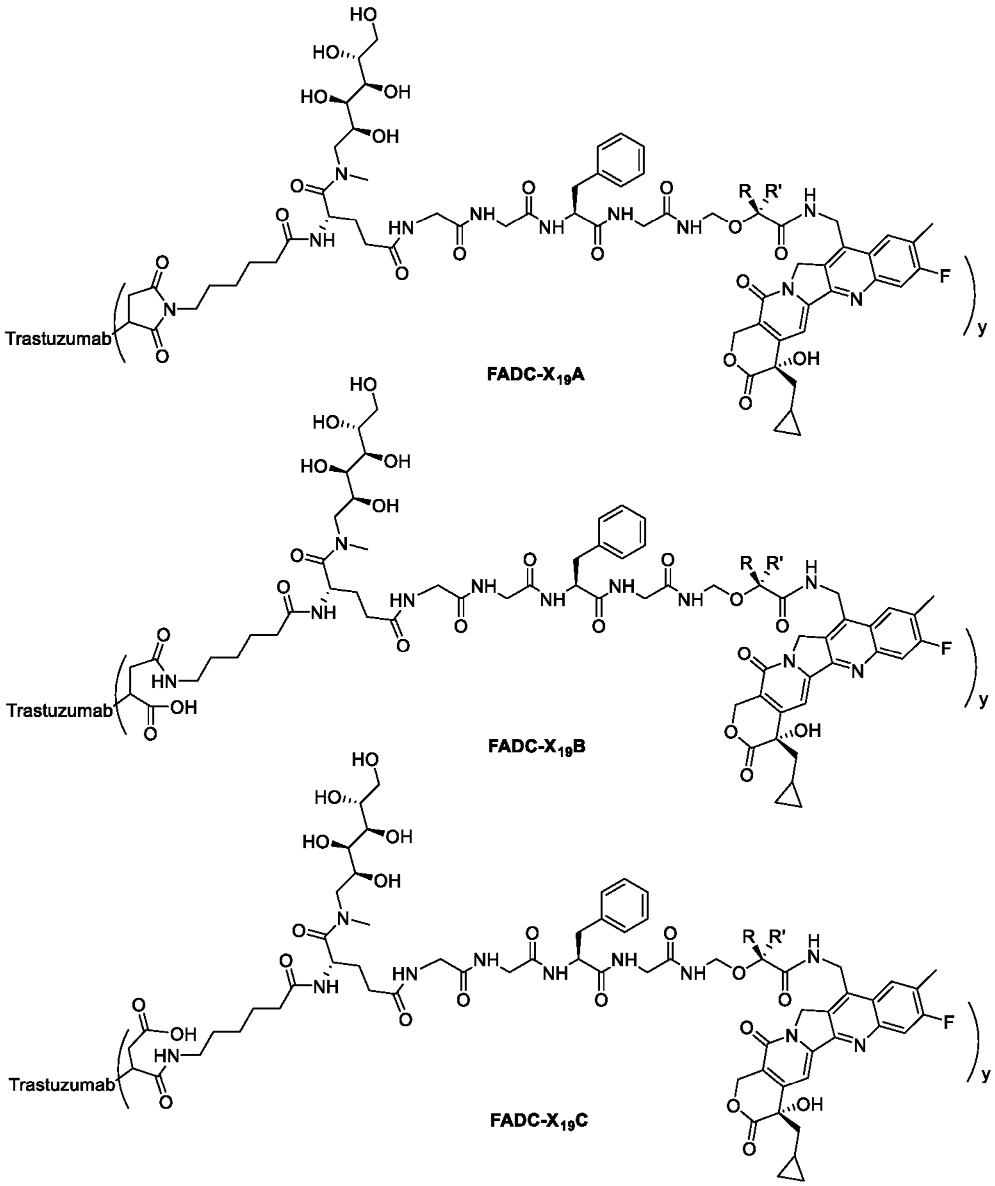

FADC-$X_{19}$A

FADC-$X_{19}$B

FADC-$X_{19}$C

wherein, R is selected from $-CH_3$, R' is selected from -H;
R is selected from -H, R' is selected from $-CH_3$;
R is selected from

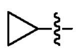,

R' is selected from -H; or
R is selected from -H, R' is selected from

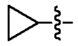;

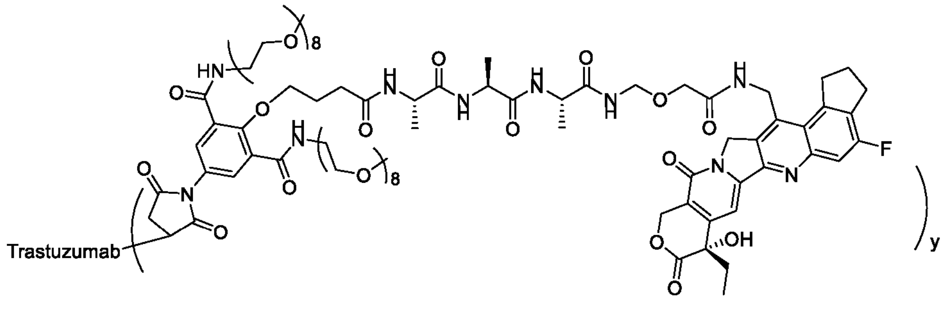

**FADC-91A**

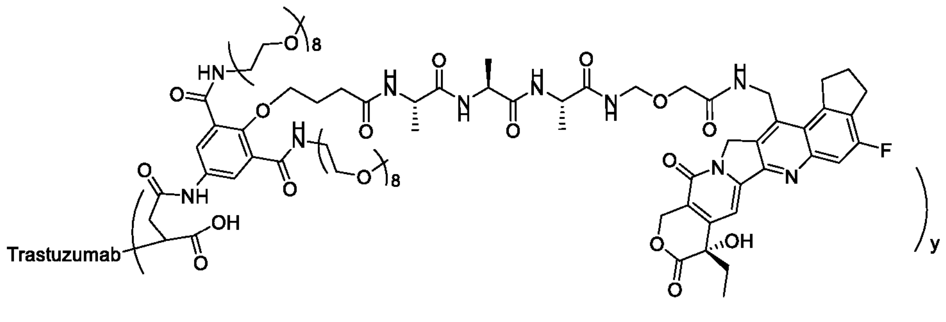

**FADC-91B**

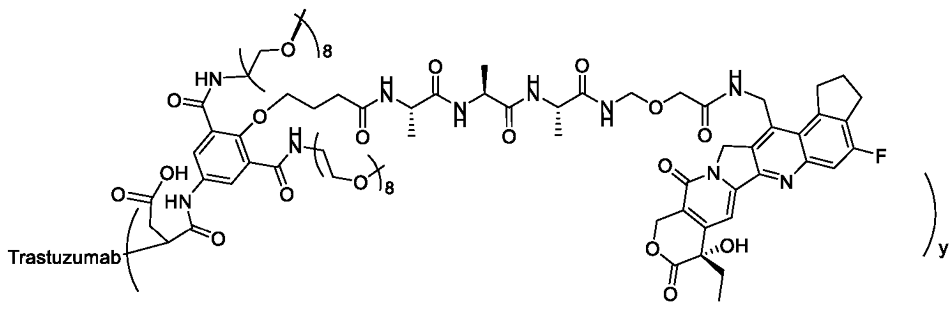

**FADC-91C**

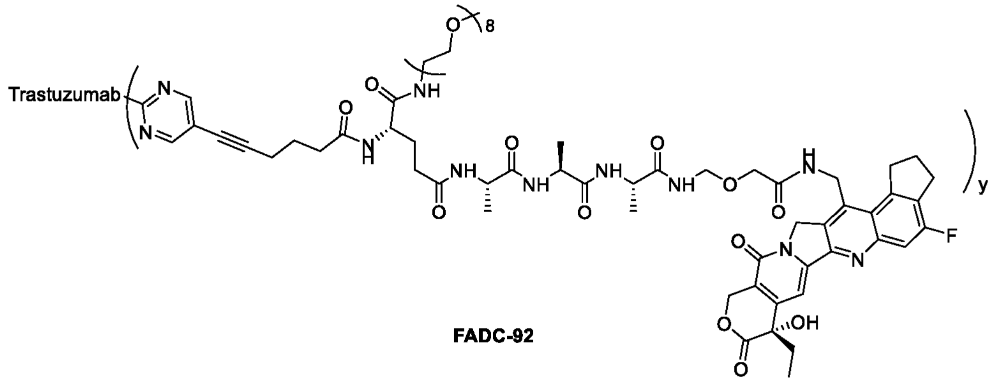

FADC-92

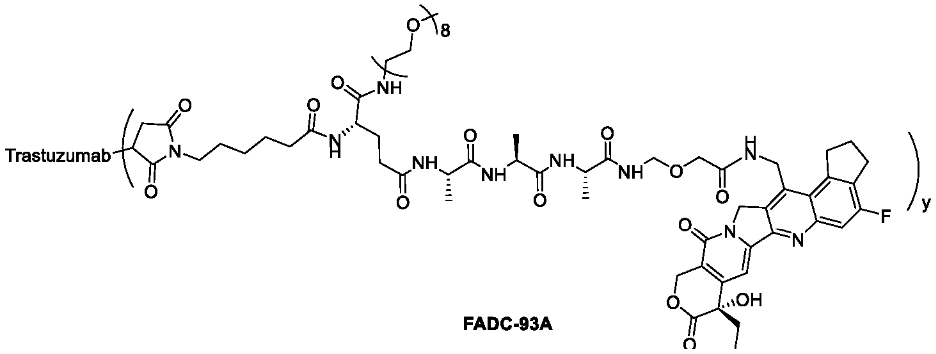

FADC-93A

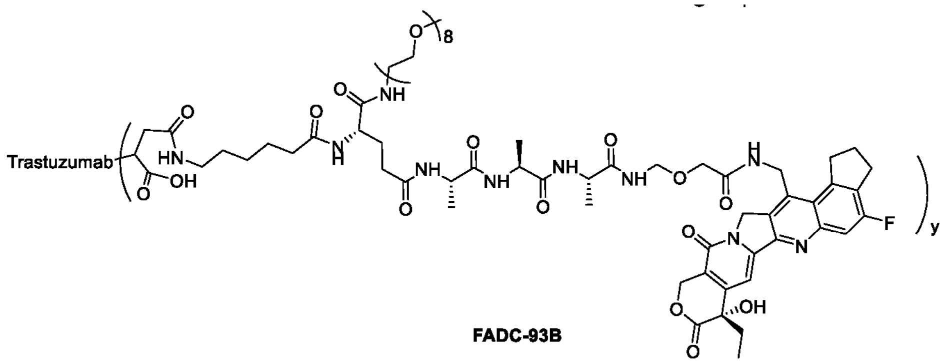

FADC-93B

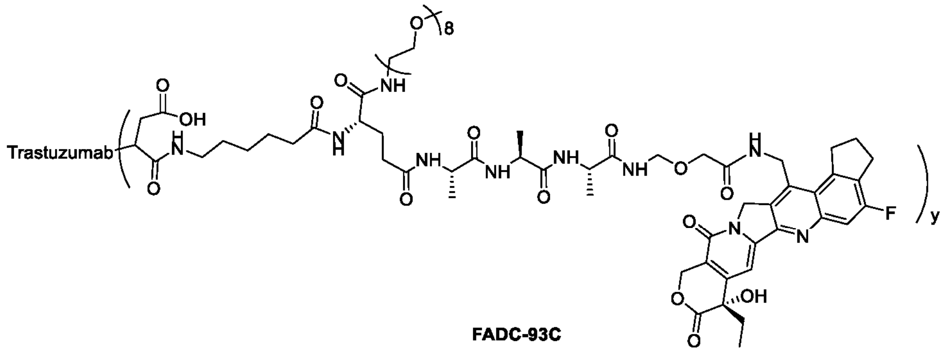

FADC-93C

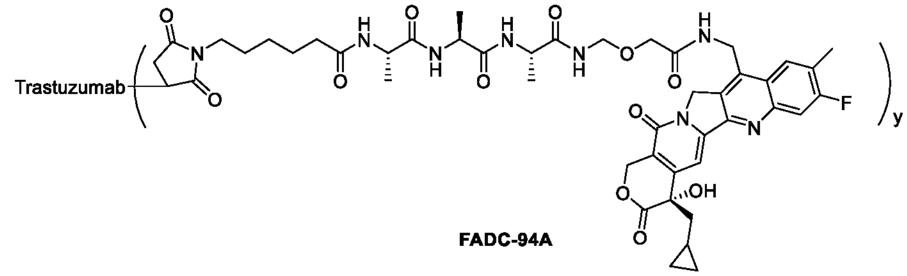

FADC-94A

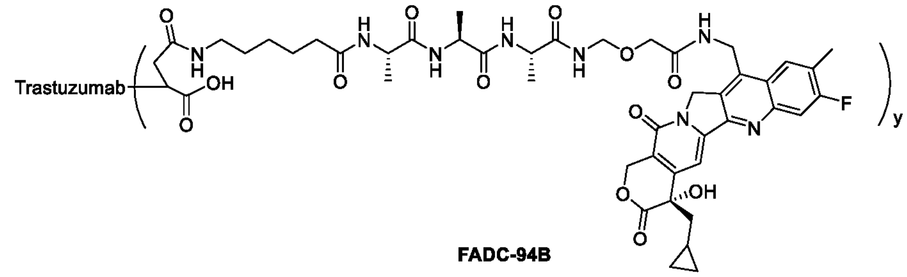

FADC-94B

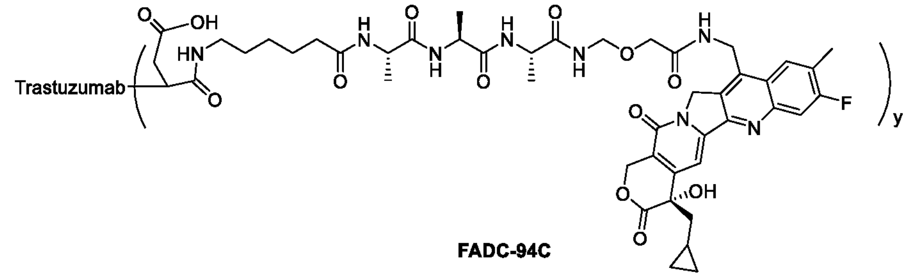

FADC-94C

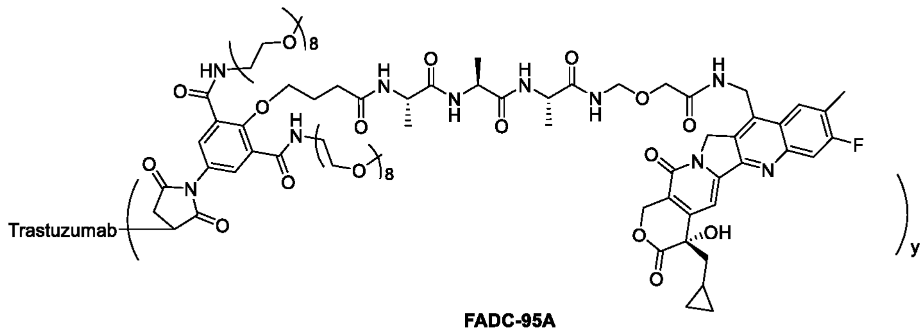

FADC-95A

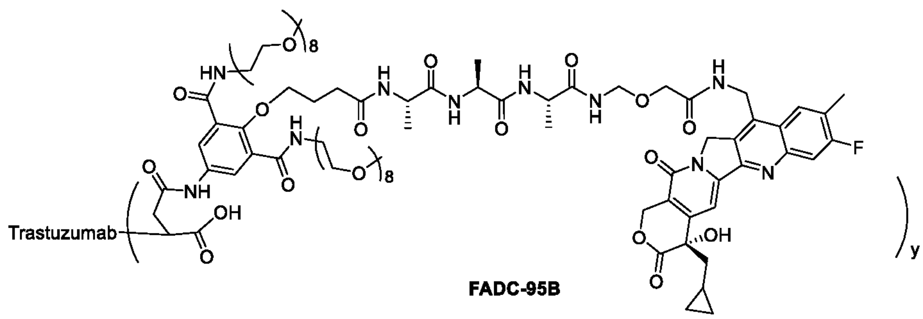

FADC-95B

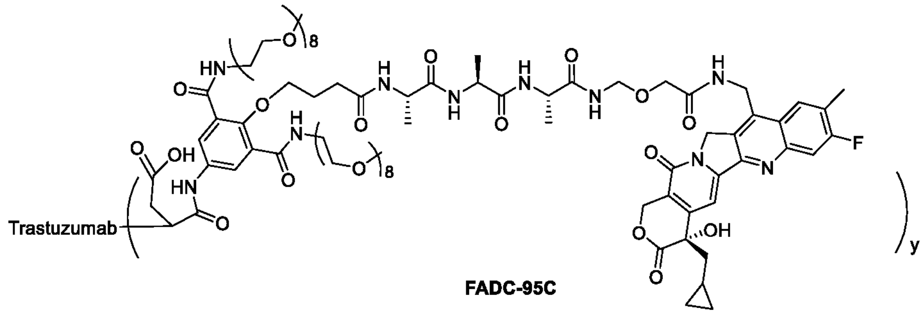

FADC-95C

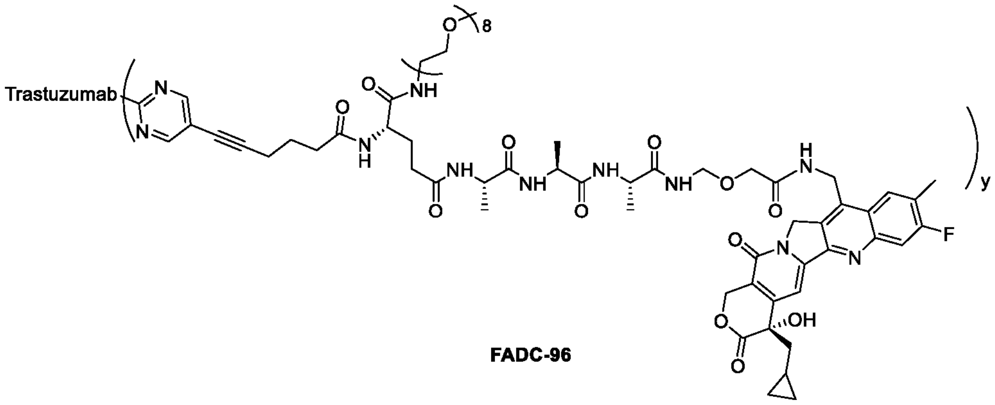

FADC-96

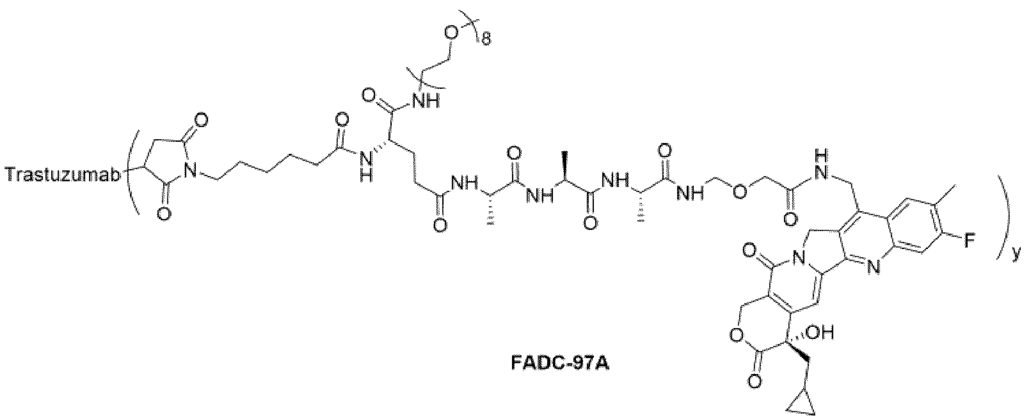

FADC-97A

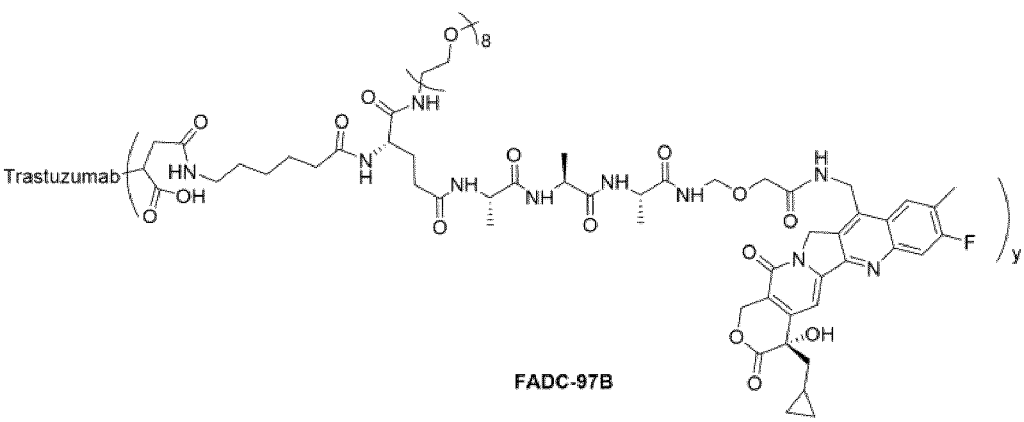

FADC-97B

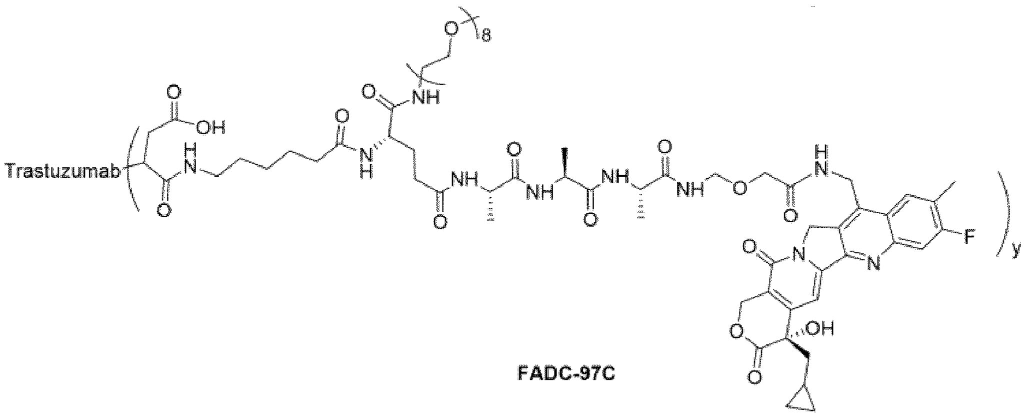

FADC-97C

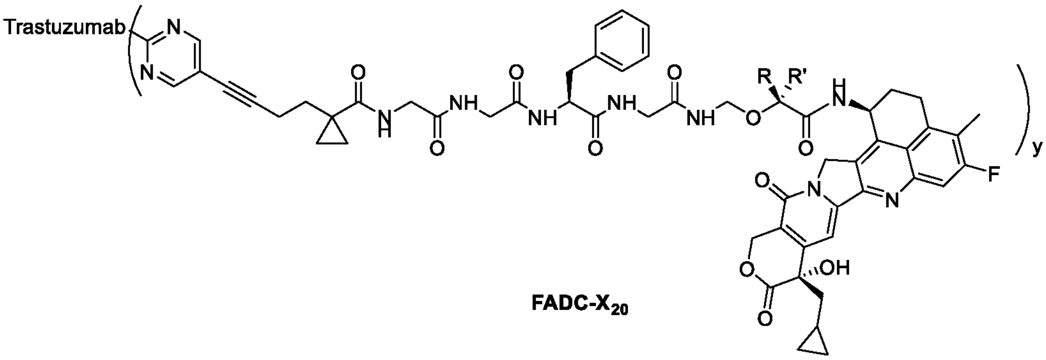

FADC-$X_{20}$

wherein, R = -H, R' = -H;
R = $-CH_3$, R' = -H;
R = -H, R' = $-CH_3$;
R=

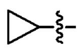,

R' = -H; or
R = -H, R' =

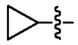;

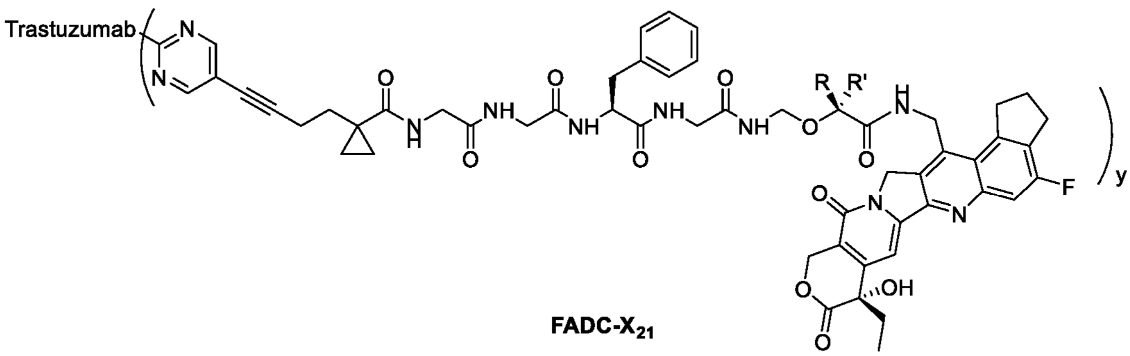

FADC-$X_{21}$

wherein, R = -H, R' = -H;
R = $-CH_3$, R' = -H; or
R = -H, R' = $-CH_3$;

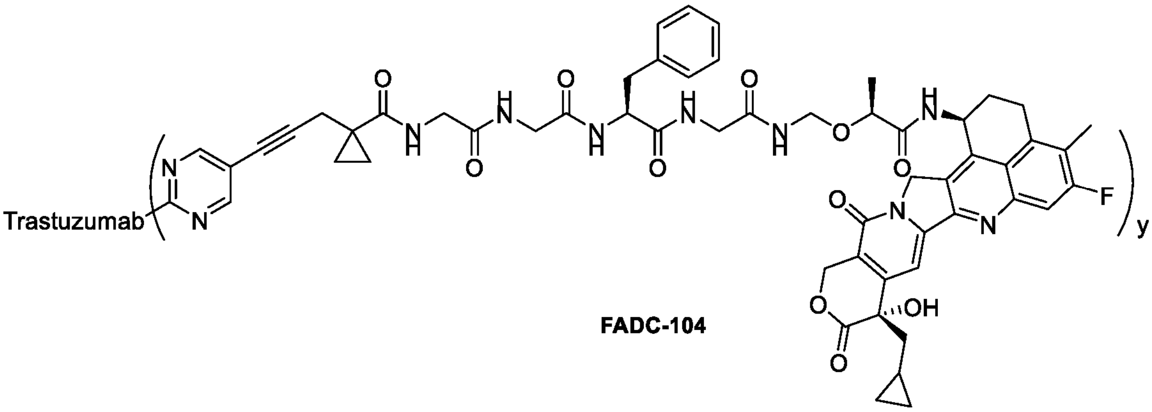

**FADC-104**

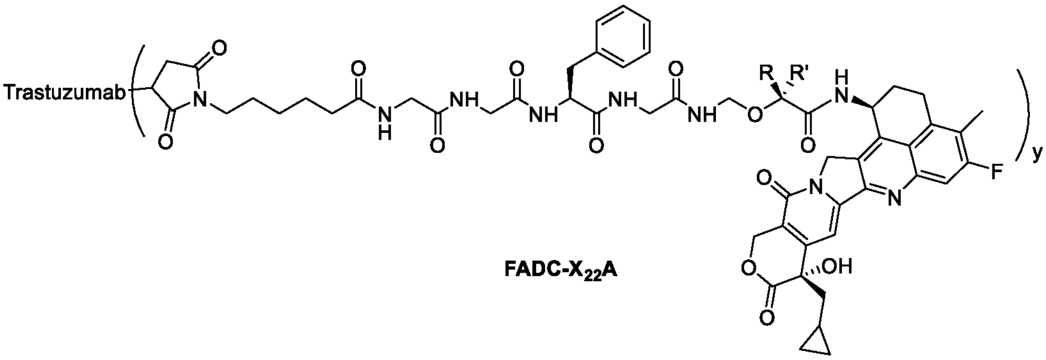

**FADC-X$_{22}$A**

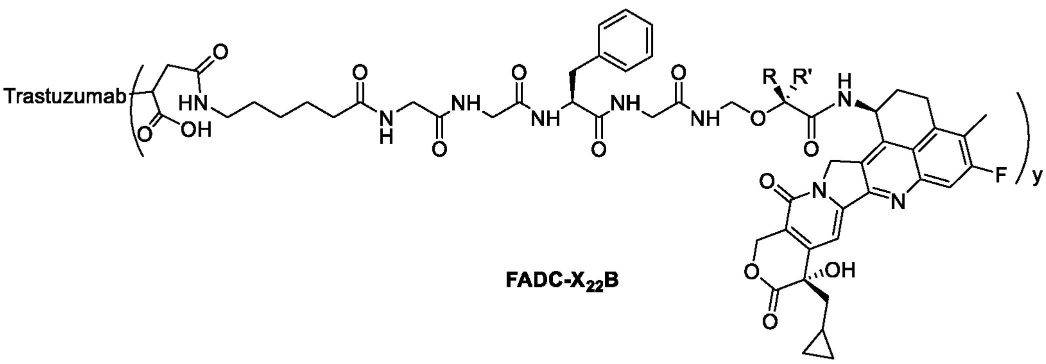

**FADC-X$_{22}$B**

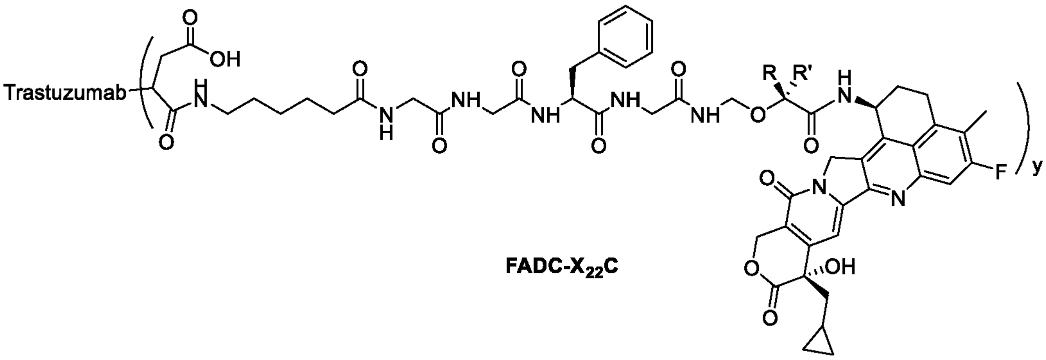

**FADC-X$_{22}$C**

wherein, R = -H, R' = $-CH_3$; or
R = $-CH_3$, R' = -H;

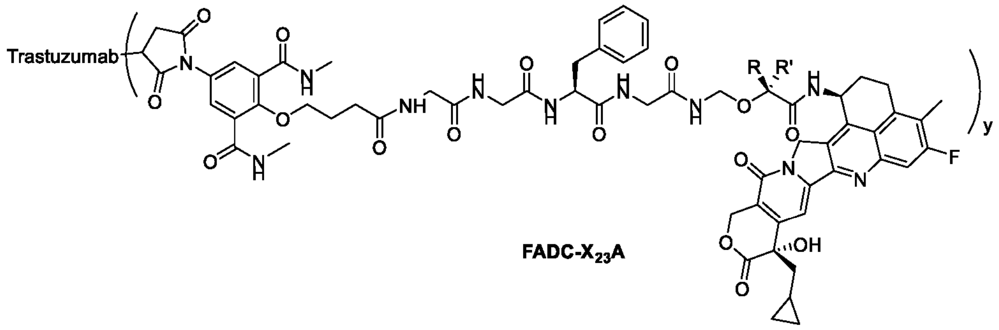

FADC-$X_{23}$A

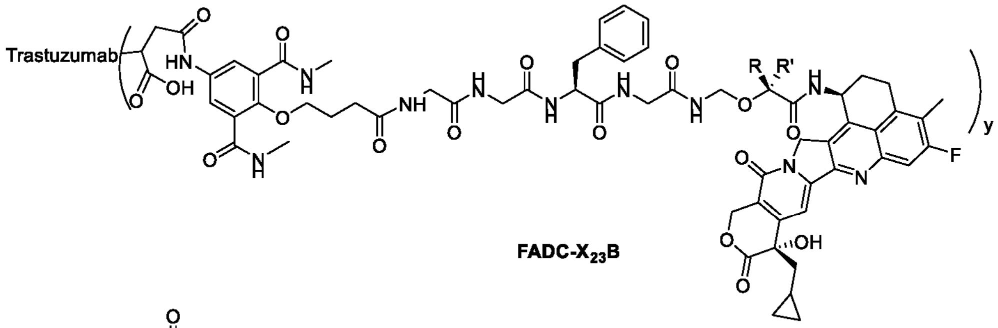

FADC-$X_{23}$B

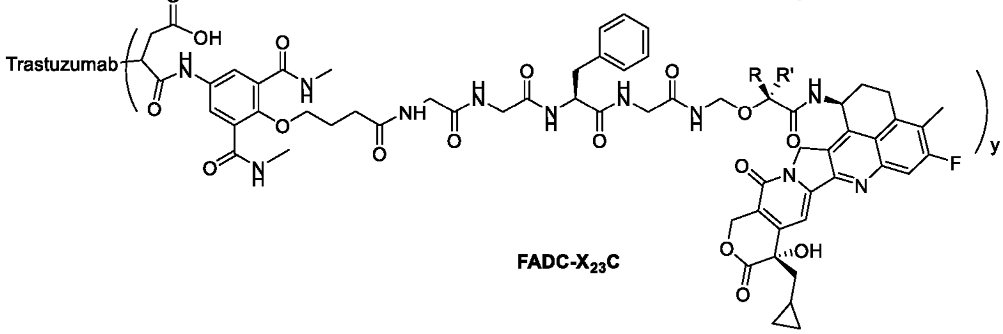

FADC-$X_{23}$C

wherein, R = -H, R' = -H;
R = $-CH_3$, R' = -H;
R=

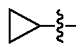,

R' = -H; or
R = -H, R' =

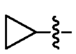;

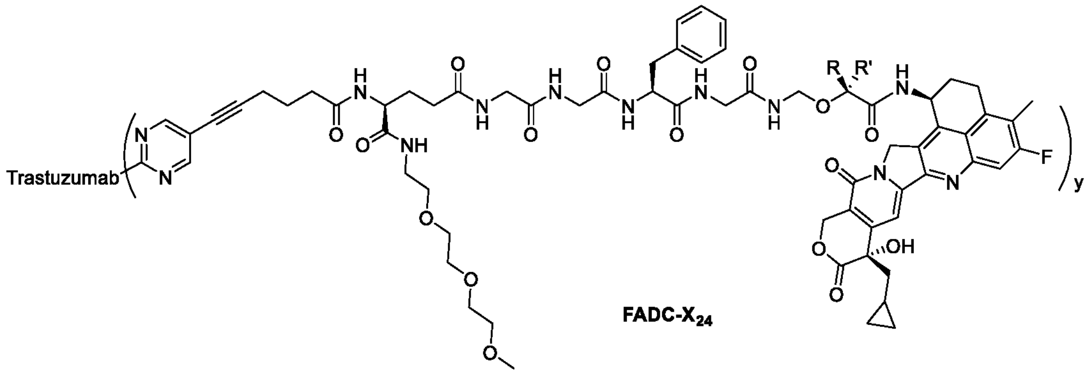

**FADC-X$_{24}$**

wherein, R = -H, R' = -H;
R = $-CH_3$, R' = -H;
R = -H, R' = $-CH_3$;
R=

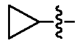
,

R' = -H; or
R = -H, R' =

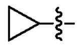
;

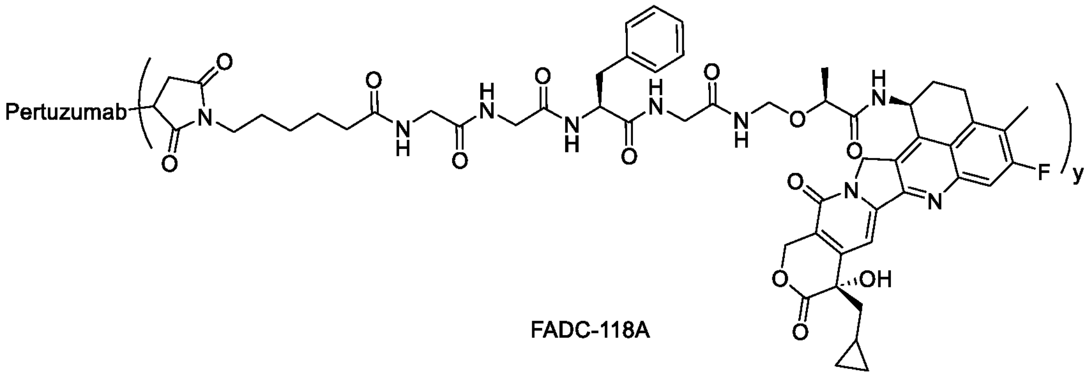

FADC-118A

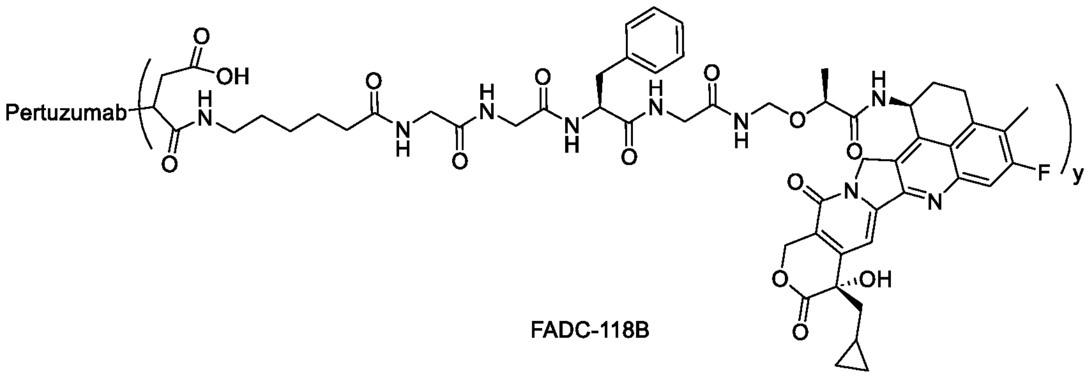

FADC-118B

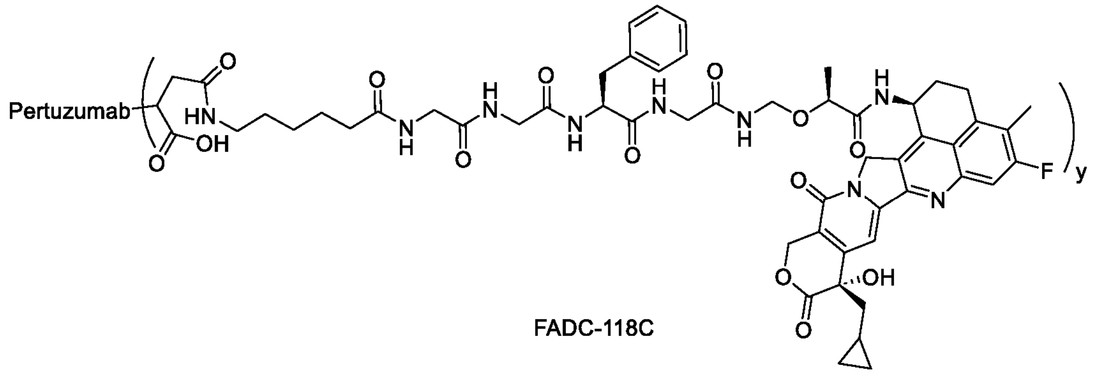

FADC-118C

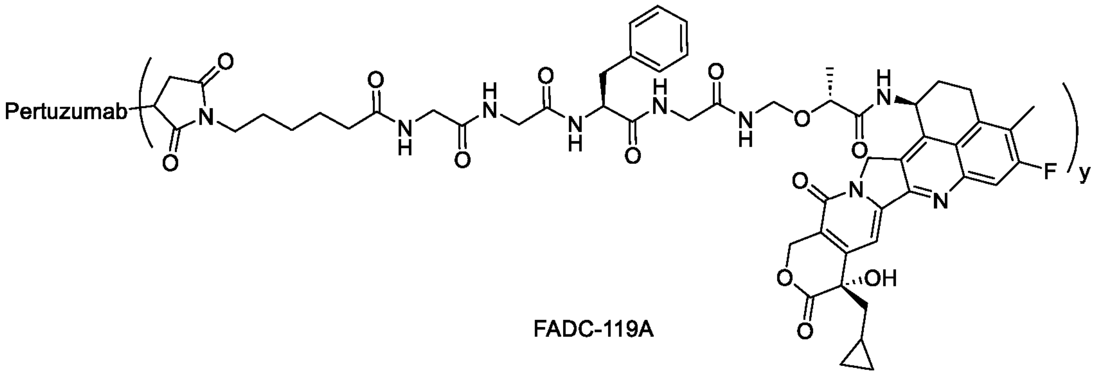

FADC-119A

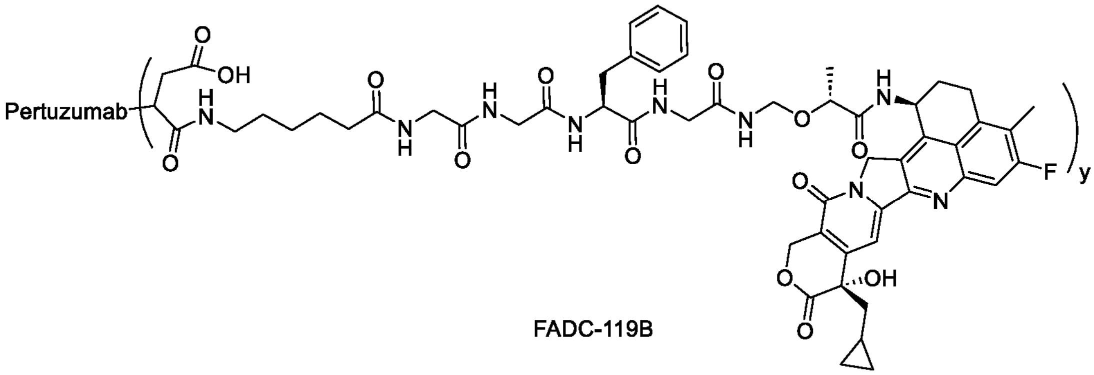

FADC-119B

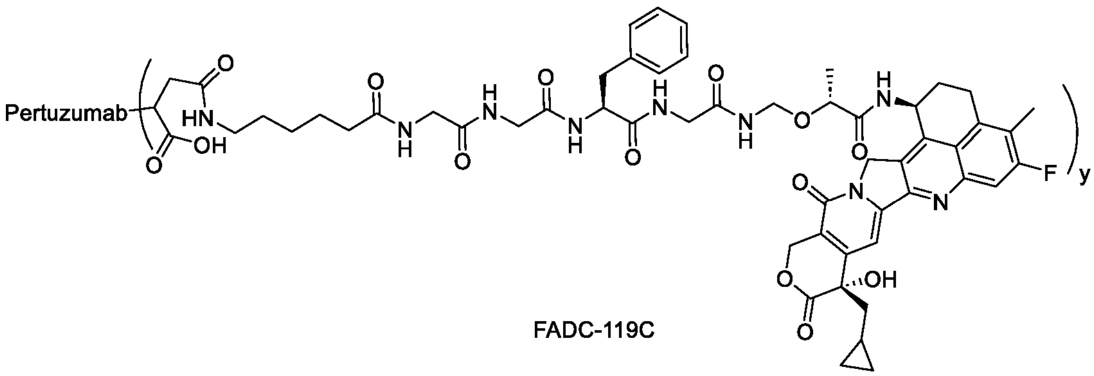

FADC-119C

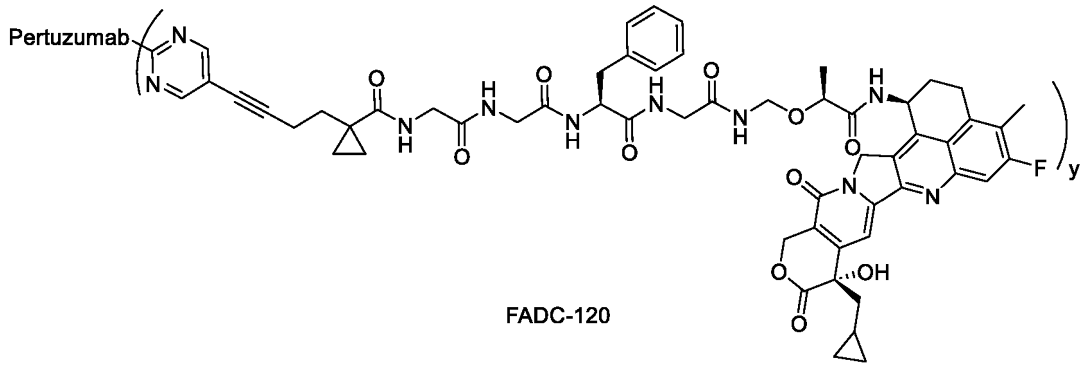

FADC-120

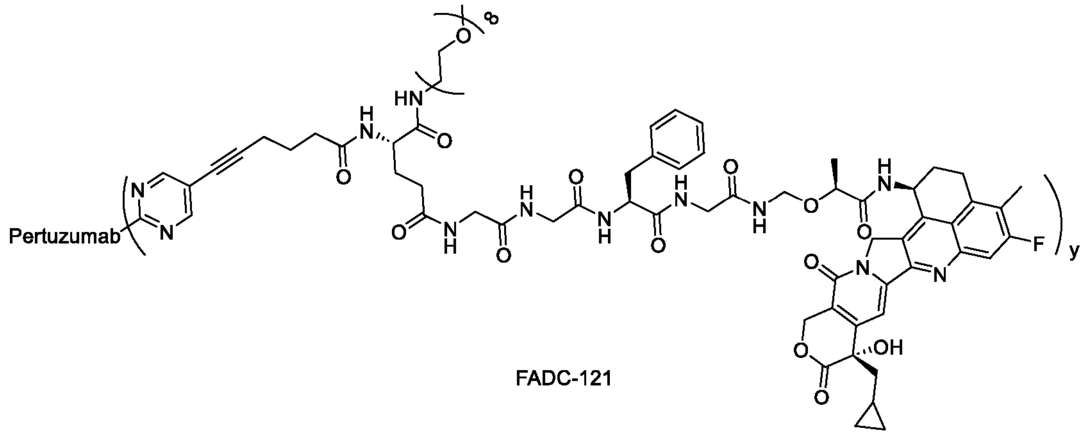

FADC-121

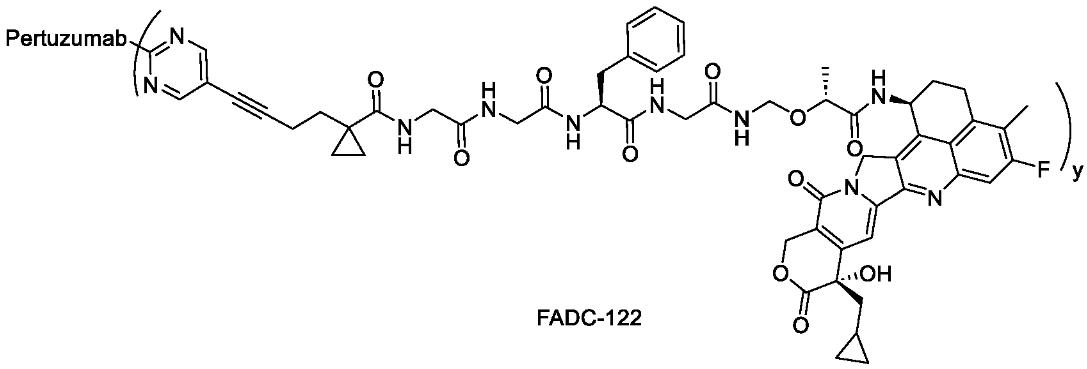

FADC-122

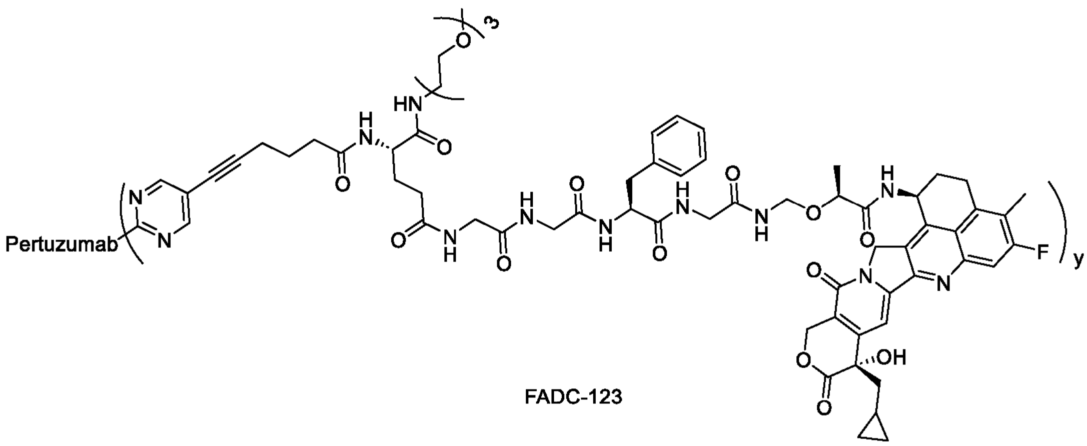

FADC-123

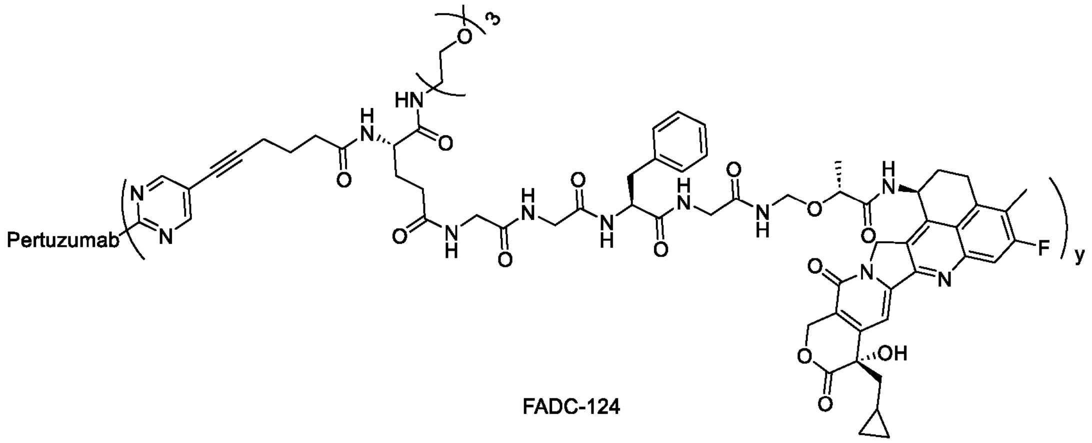

FADC-124

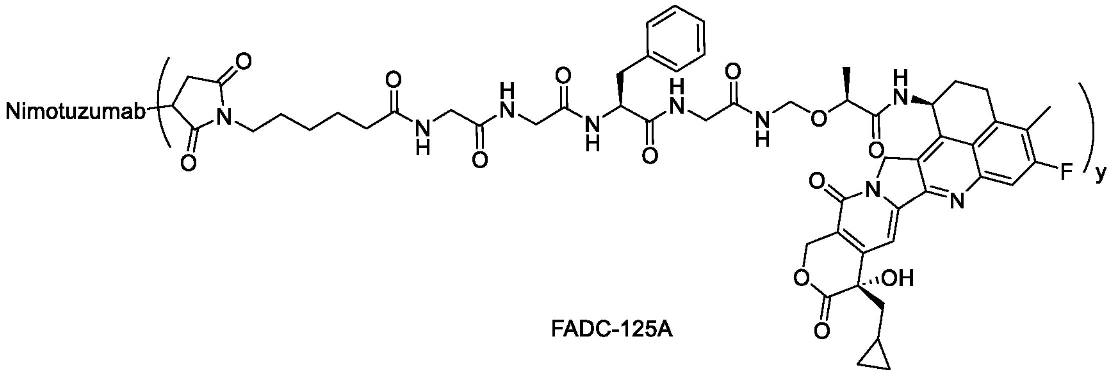

FADC-125A

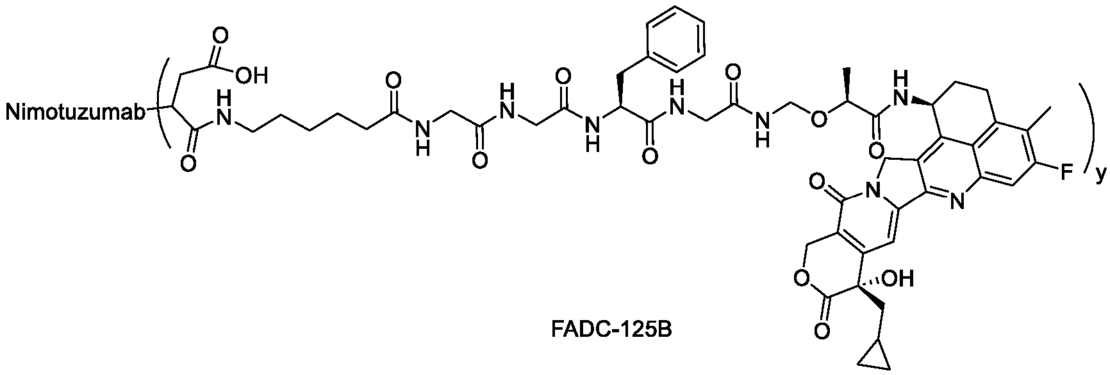

FADC-125B

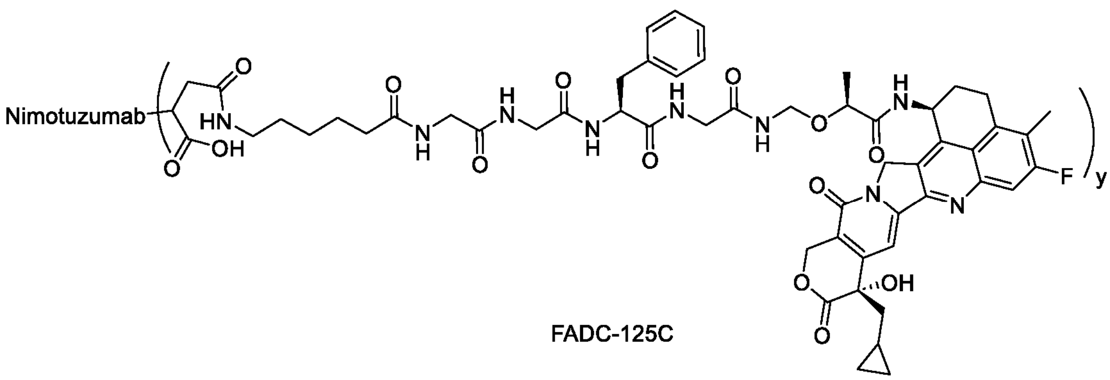

FADC-125C

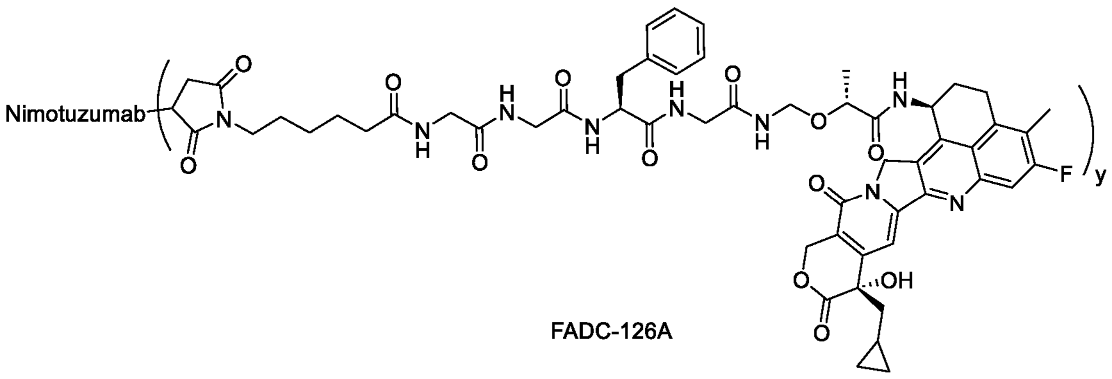

FADC-126A

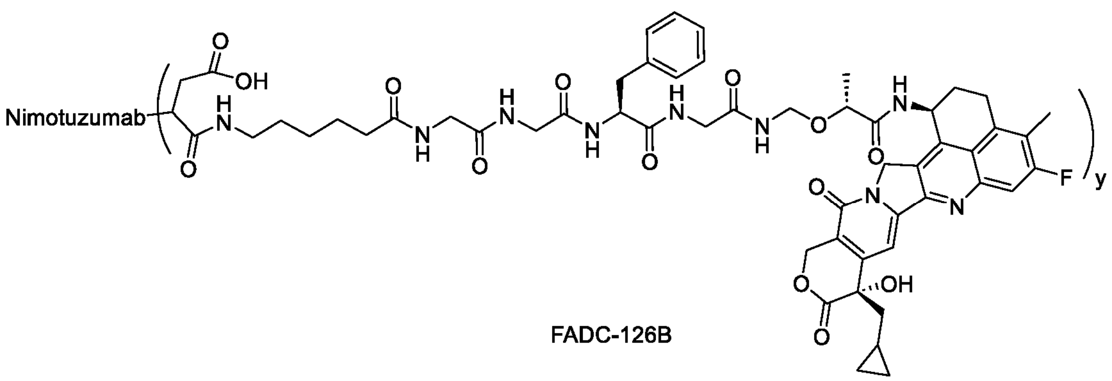

FADC-126B

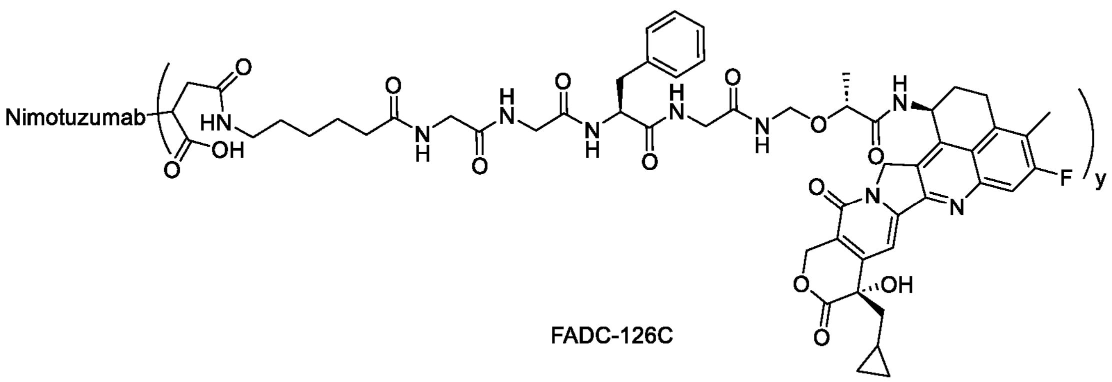

FADC-126C

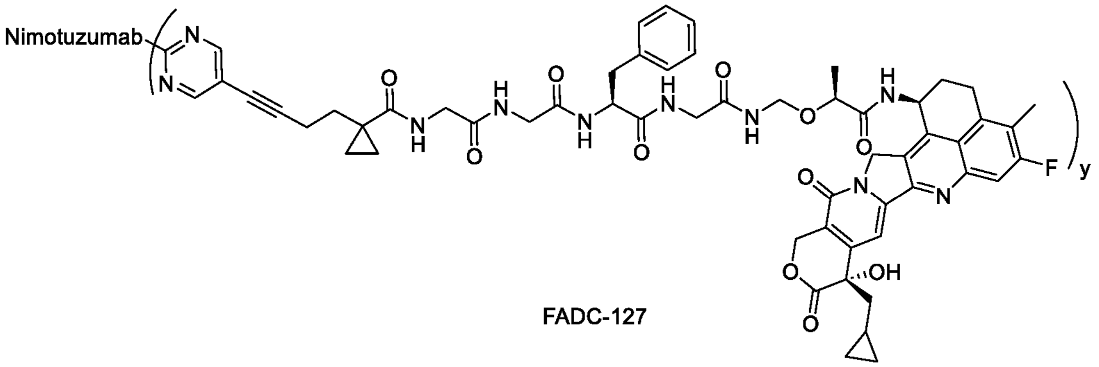

FADC-127

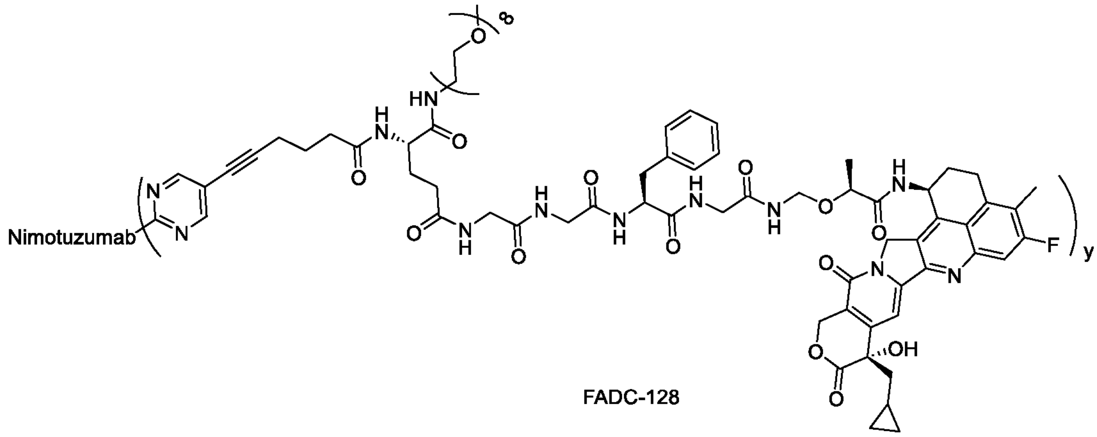

FADC-128

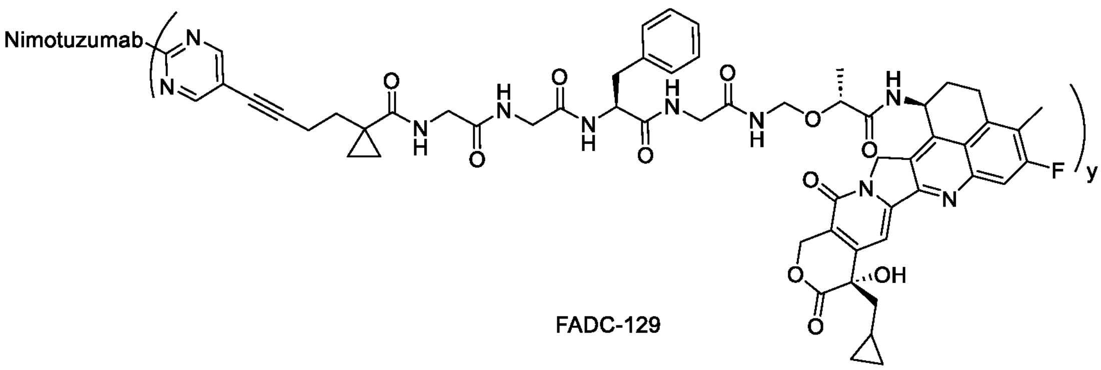

FADC-129

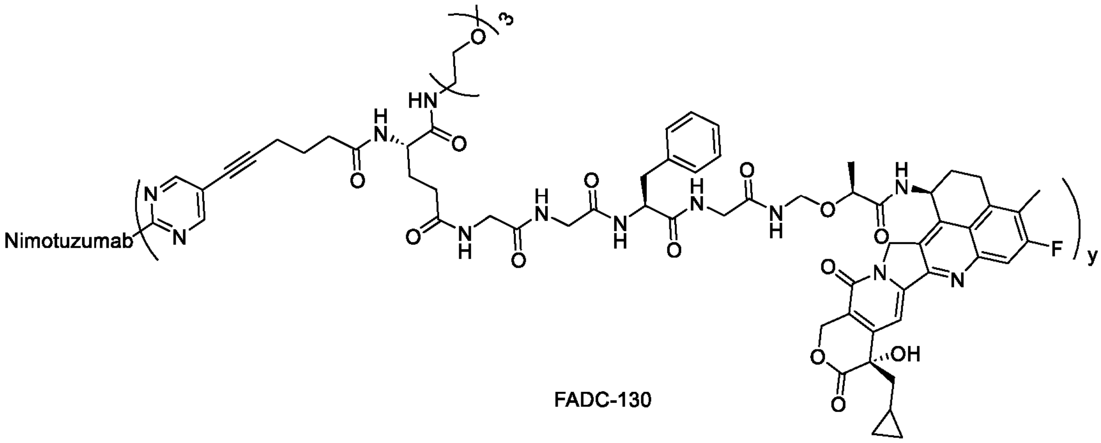

FADC-130

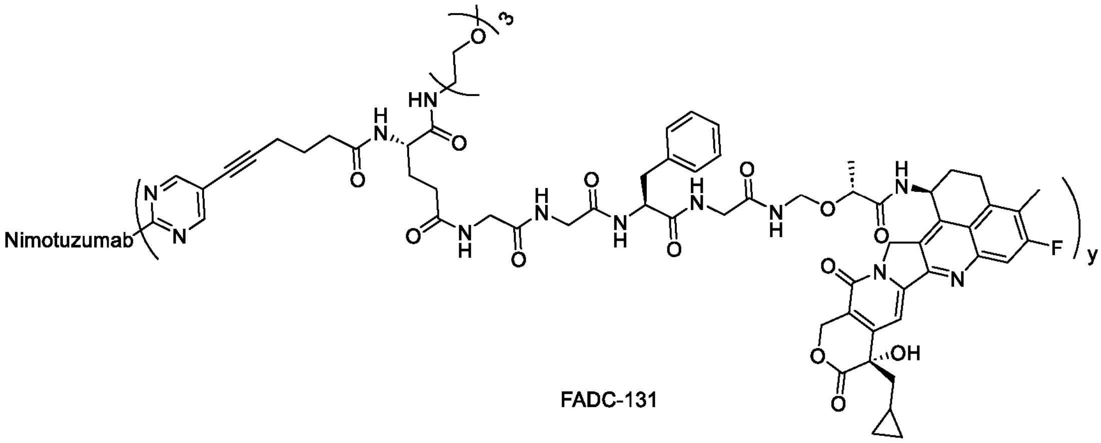

FADC-131

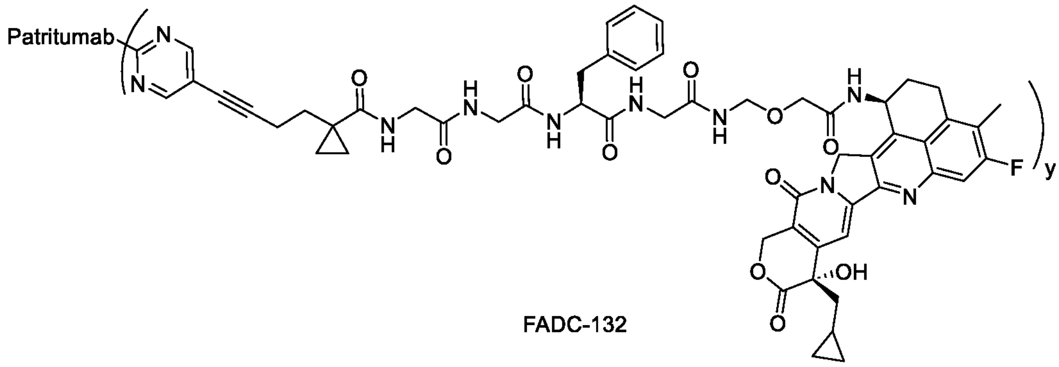

FADC-132

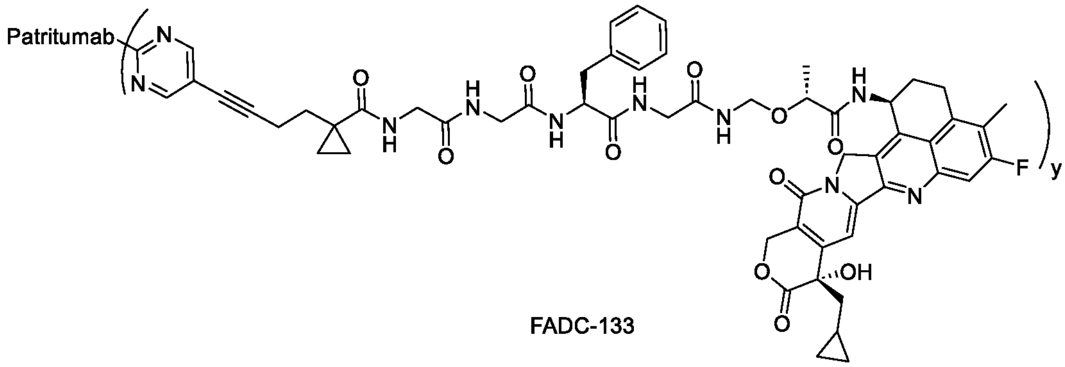

FADC-133

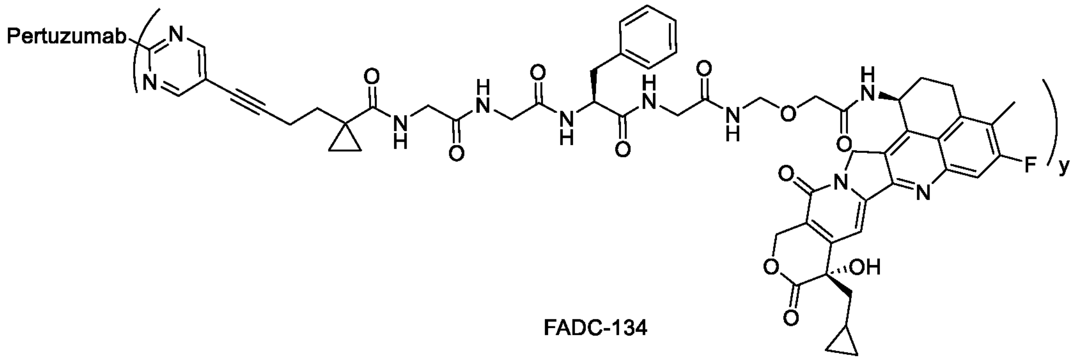

FADC-134

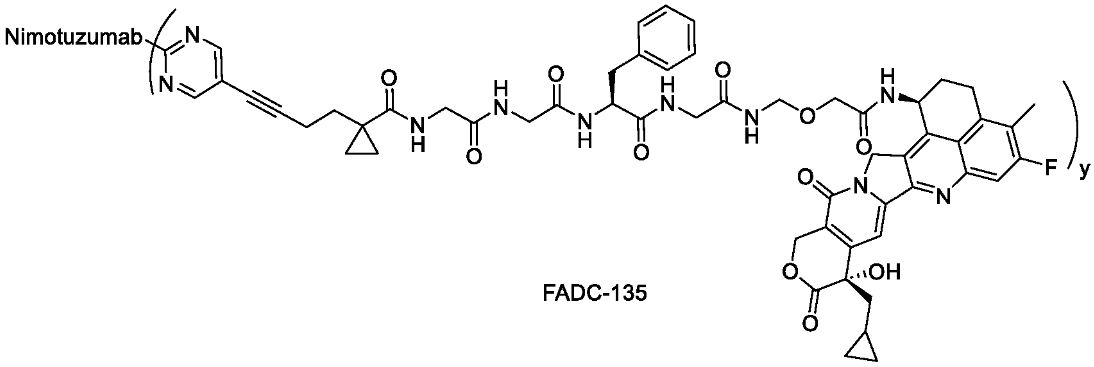

FADC-135

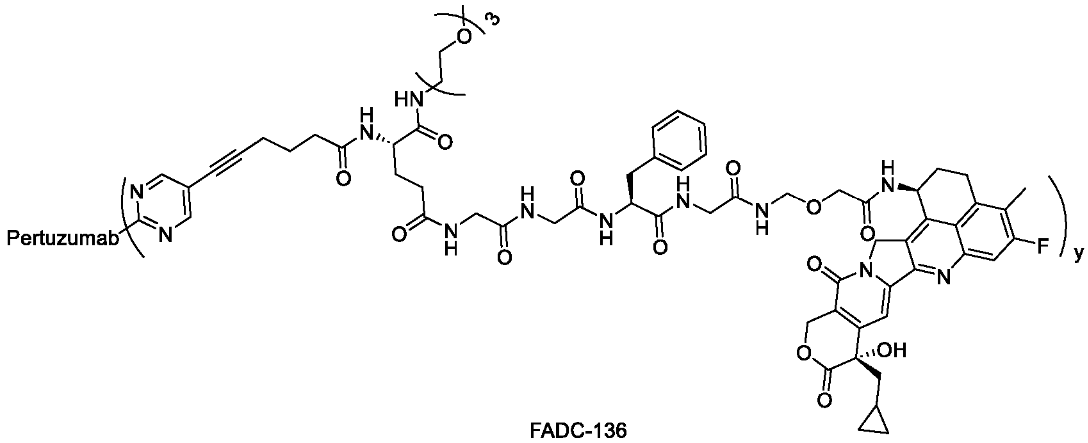

FADC-136

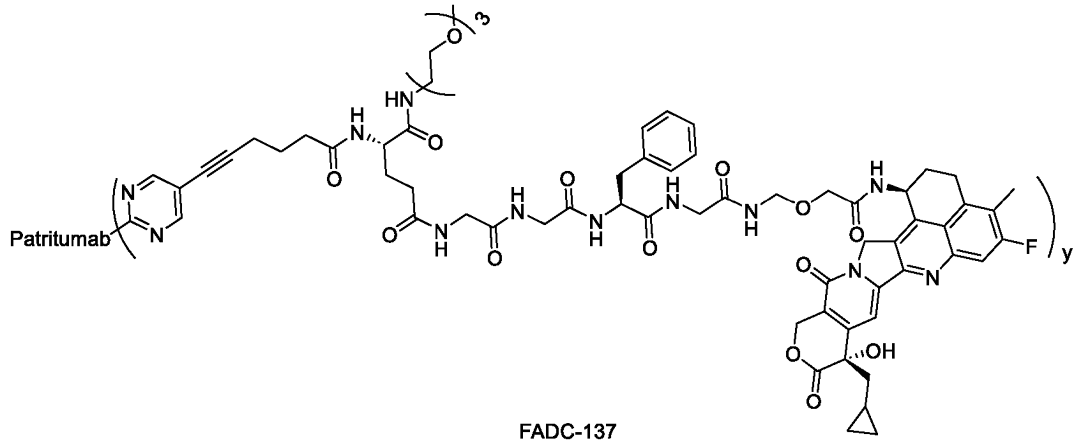

FADC-137

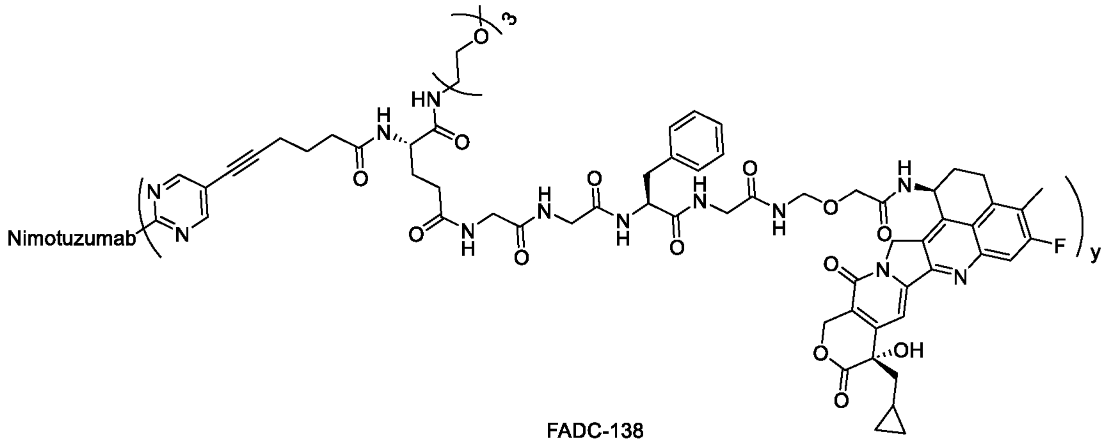

FADC-138

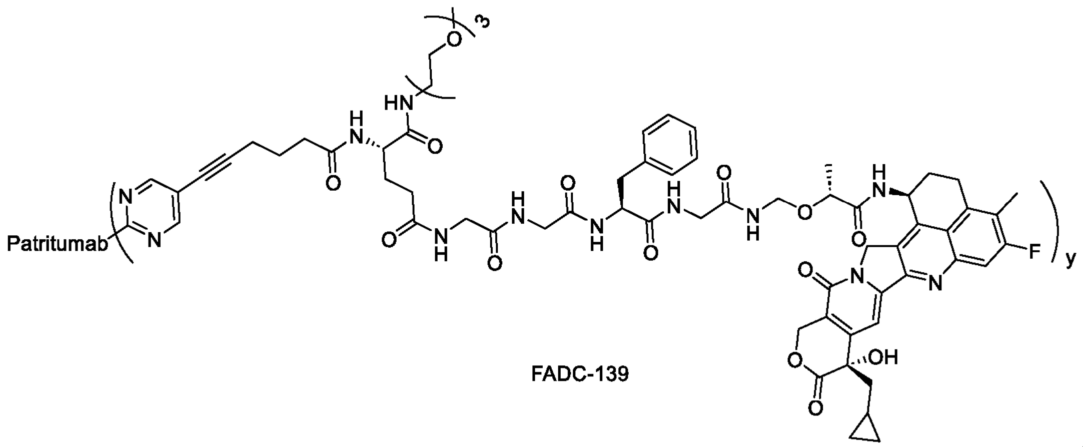

FADC-139

.

**34.** A method for preparing the conjugate, or a stereoisomer, racemate, tautomer, isotopologue, isotope-labeled compound, nitroxide or pharmaceutically acceptable salt thereof according to any one of claims 1 to 33, comprising:

Step 1: providing a linker as shown in $L^{1'}$-$L^2$-$L^3$-$L^4$-$L^{5'}$ (L');

preferably, in the linker,
$L^4$-$L^{5'}$ is: $-NR^{z1}-C(R^{z2}R^{z3})-T^L-$ in its carboxylic acid form or active ester form;
wherein:

$R^{z1}$ is H or $C_{1-4}$ alkyl;
$R^{z2}$ and $R^{z3}$ are the same or different and are independently selected from H or $C_{1-4}$ alkyl;
further preferably:

$L^4$-$L^{5'}$ is: $-NR^{z1}-C(R^{z2}R^{z3})-O-(CH_2)_m-C(R^{z4}R^{z5})-CO-$ in its carboxylic acid form or active ester form;
wherein:

$R^{z1}$ is H or $C_{1-4}$ alkyl;
m is an integer selected from 0 to 4;
$R^{z2}$ and $R^{z3}$ are the same or different and are independently selected from H or $C_{1-4}$ alkyl;
$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl;

Step 2: coupling the linker with an intermediate compound as represented by Formula (G') to obtain a coupled intermediate as shown in $L^{1'}$-$L^2$-$L^3$-$L^4$-$L^5$-G (C');

the structure of the intermediate compound as represented by Formula (G') is:

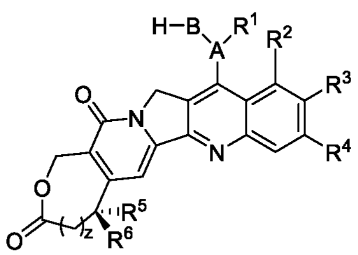

(G')

wherein, A, B, $T^L$, $R^1$, $R^2$, $R^3$, $R^4$, $R^5$, $R^6$, $L^{1'}$, $L^1$, $L^2$, $L^3$, $L^4$, $L^5$, and $L^{5'}$ independently have the definitions as described in any one of claims 1 to 33;
preferably, the preparation method further comprises Step 3: coupling the coupled intermediate as represented by Formula (C') with the targeting moiety Tp;
optionally, if necessary, functional groups of reaction substrates can also be protected with a protecting group

known in the art to allow the reaction to proceed smoothly, and the protecting group is removed after the reaction is completed.

**35.** A compound represented by the following Formula (GH), or a stereoisomer, racemate, tautomer, isotopologue, isotope-labeled compound, nitroxide or pharmaceutically acceptable salt thereof:

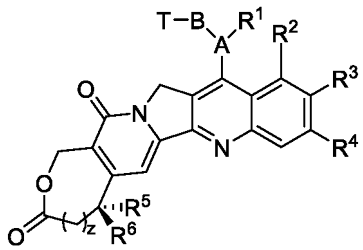

(GH)

wherein, T is selected from H-$T^L$;
$T^L$, A, B, $R^1$, $R^2$, $R^3$, $R^4$, $R^5$, $R^6$, and z independently have the definitions as described in any one of claims 1 to 32.

**36.** The compound, or a stereoisomer, racemate, tautomer, isotopologue, isotope-labeled compound, nitroxide or pharmaceutically acceptable salt thereof according to claim 35, wherein the compound represented by Formula (GH) can be selected from the compound represented by the following Formula (GH-1):

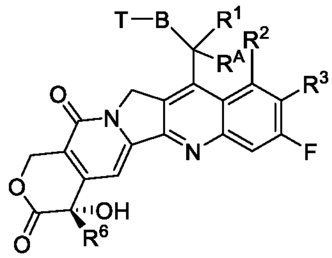

(GH-1)

wherein, $R^A$, B, T, $R^1$, $R^2$, $R^3$, and $R^6$ independently have the definitions as described in claim 35.

**37.** The compound, or a stereoisomer, racemate, tautomer, isotopologue, isotope-labeled compound, nitroxide or pharmaceutically acceptable salt thereof according to claim 35, wherein the compound represented by Formula (GH) can be selected from the compound represented by the following Formula (GH-2) or (GH-3):

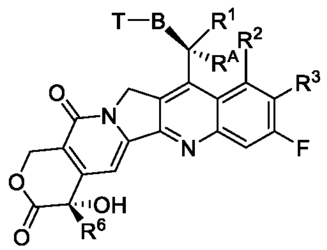

(GH-2)

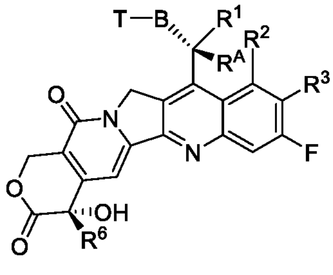

(GH-3)

wherein, $R^A$, B, T, $R^1$, $R^2$, $R^3$, and $R^6$ independently have the definitions as described in any one of claims 1 to 32.

**38.** The compound, or a stereoisomer, racemate, tautomer, isotopologue, isotope-labeled compound, nitroxide or pharmaceutically acceptable salt thereof according to claim 35, wherein the compound is selected from the following compounds:

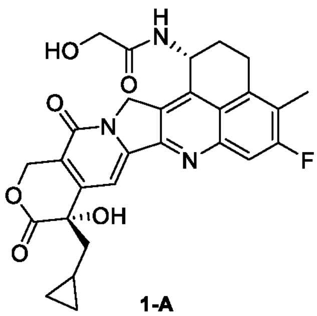
1-A

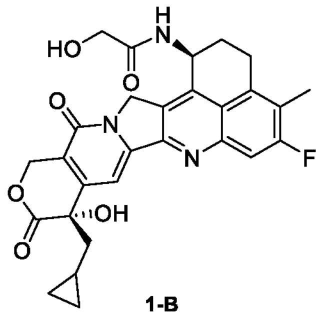
1-B

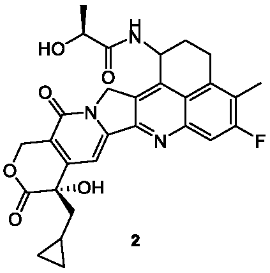
2

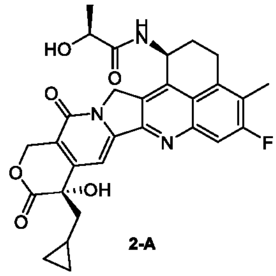
2-A

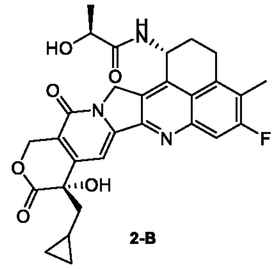
2-B

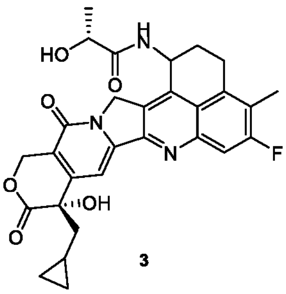
3

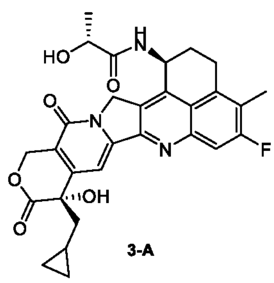
3-A

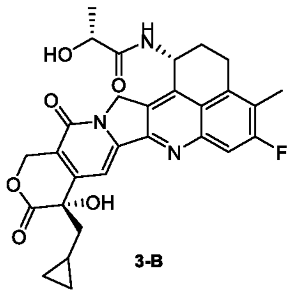
3-B

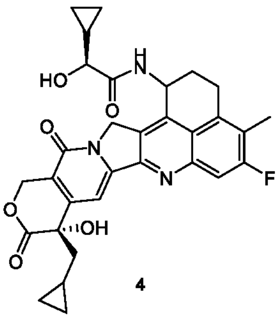
4

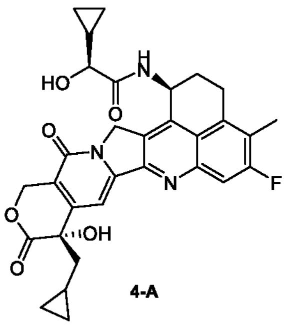
4-A

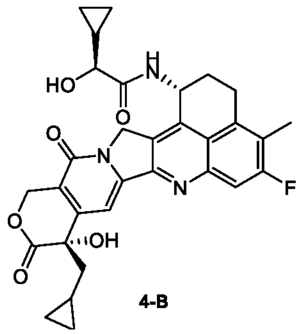
4-B

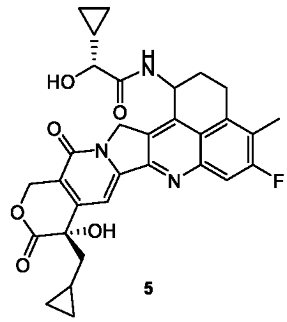
5

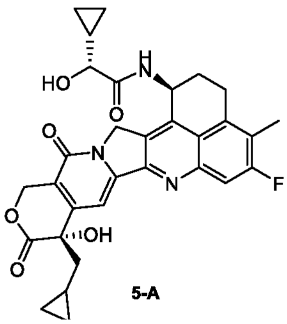
5-A

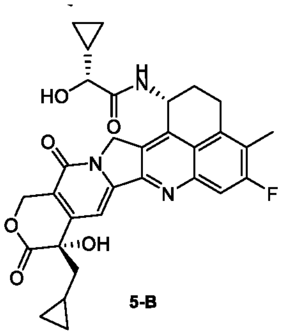
5-B

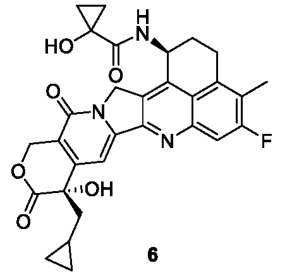
6

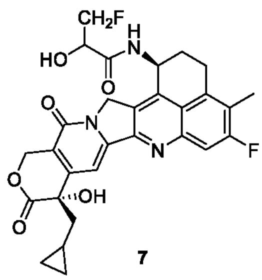
7

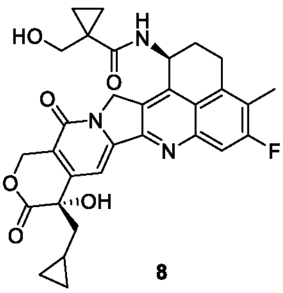
8

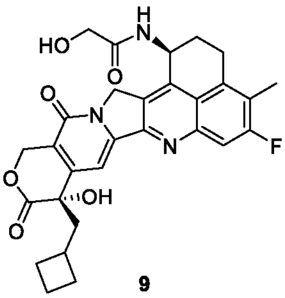
9

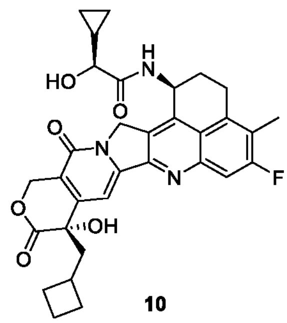
10

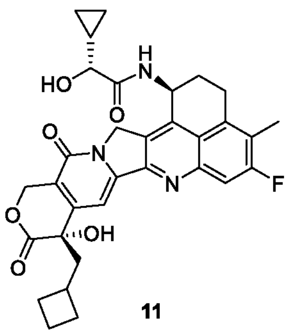
11

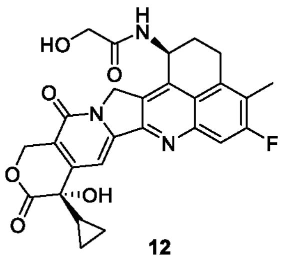
12

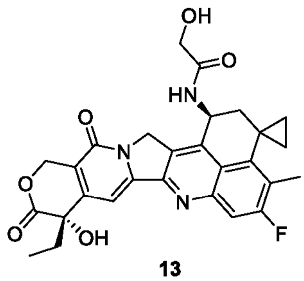
13

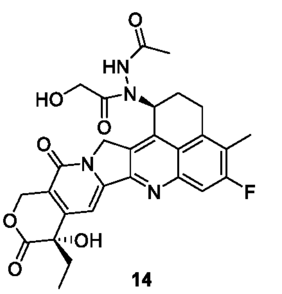
14

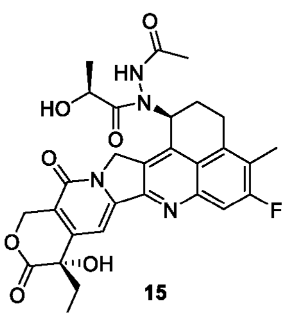
15

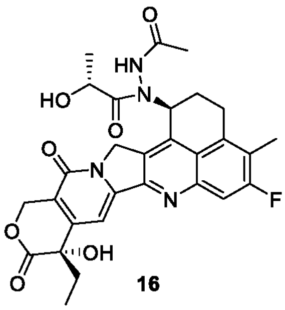

16

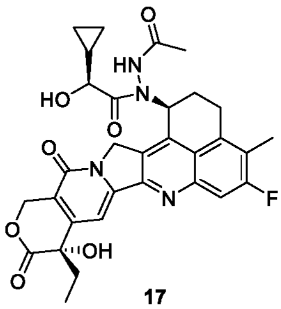

17

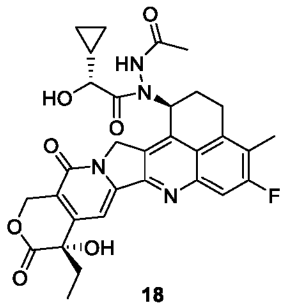

18

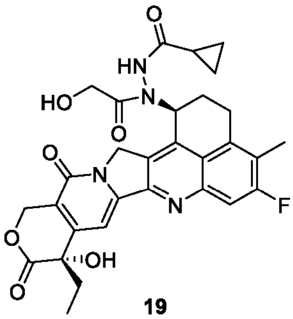

19

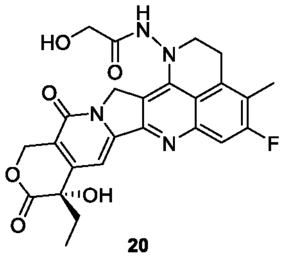

20

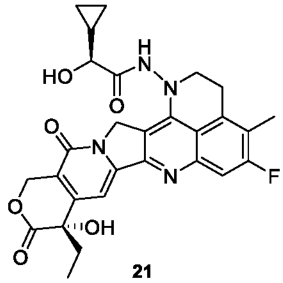

21

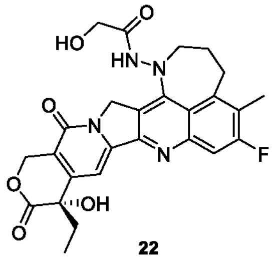

22

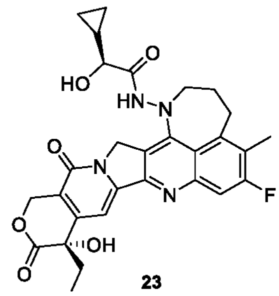

23

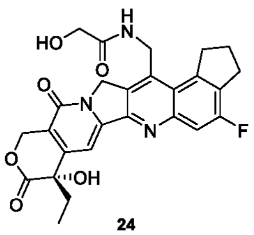

24

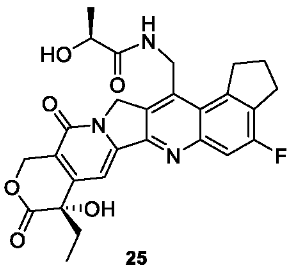

25

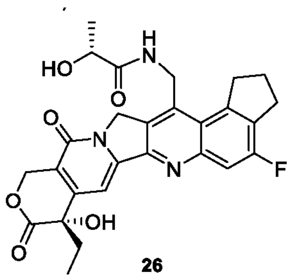

26

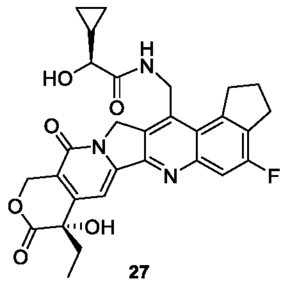

27

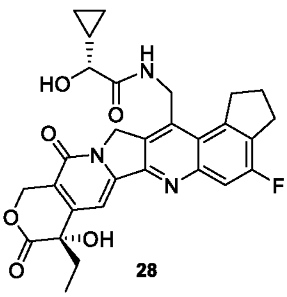

28

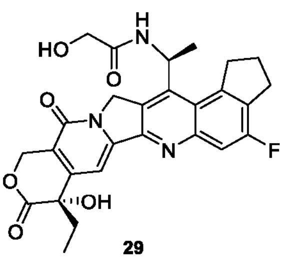

29

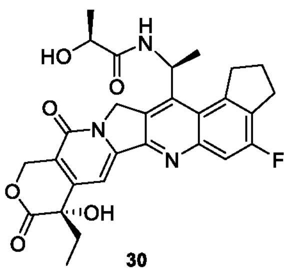

30

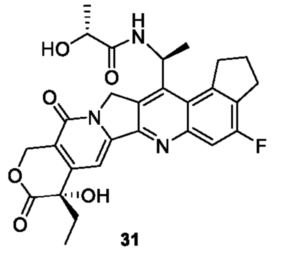
31

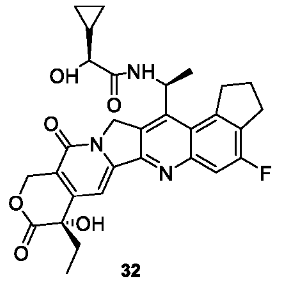
32

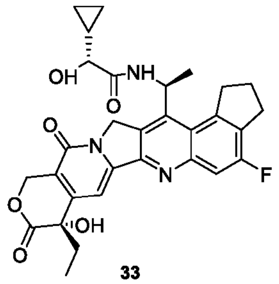
33

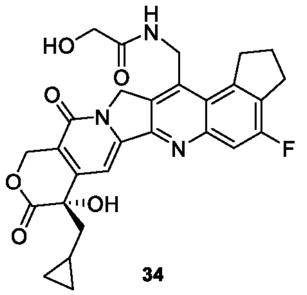
34

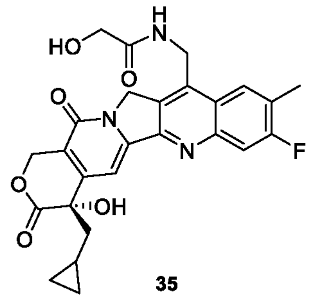
35

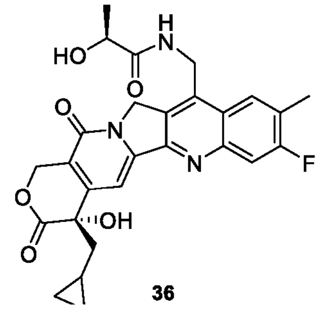
36

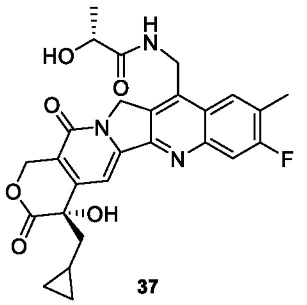
37

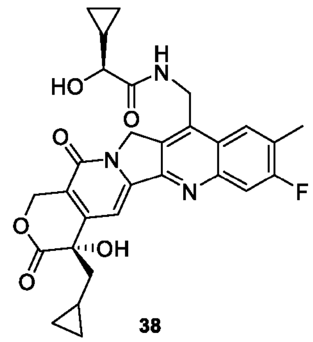
38

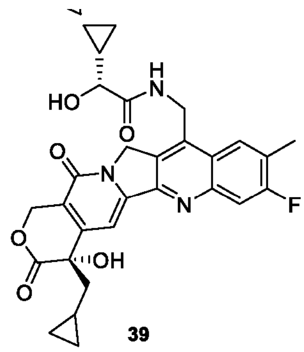
39

**39.** A method for preparing the compound, or a stereoisomer, racemate, tautomer, isotopologue, isotope-labeled compound, nitroxide or pharmaceutically acceptable salt thereof according to any one of claims 35 to 38, the method comprises a step of reacting a compound represented by Formula (i) with a compound represented by Formula (ii):

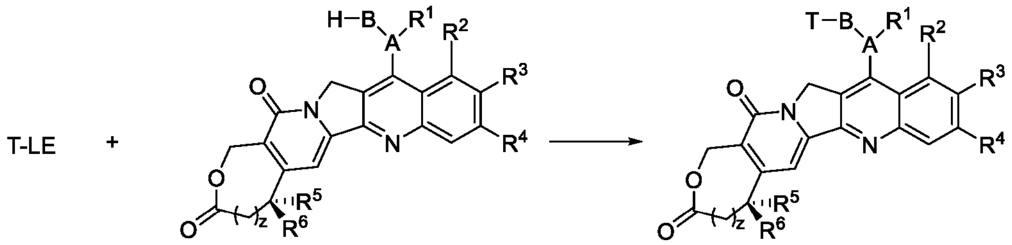

(i) (ii) (GH)

wherein, A, B, T, $R^1$, $R^2$, $R^3$, $R^4$, $R^5$, $R^6$, and z independently have the definitions as described in any one of claims 35 to 38;

LE represents a group that is removed or leaves after the reaction.

**40.** A method for preparing the compound represented by Formula (ii) in claim 35, the method comprises a step of reacting a compound represented by Formula (iii) with a compound represented by Formula (iv) to obtain a compound represented by Formula (v):

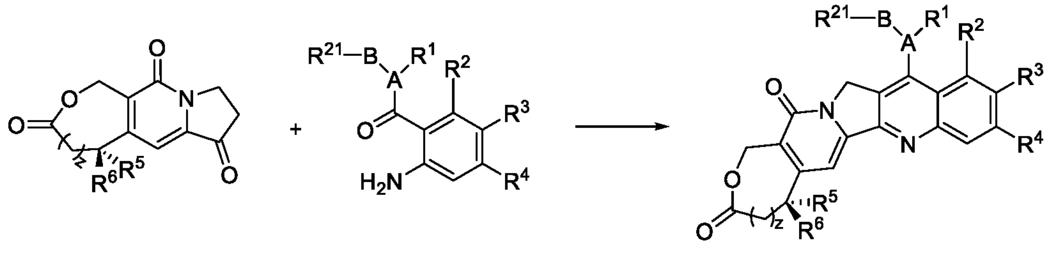

(iii) (iv) (v)

wherein, A, B, $R^1$, $R^2$, $R^3$, $R^4$, $R^5$, $R^6$, and z independently have the definitions as described in any one of claims 35 to 38;
$R^{21}$ is selected from H or a protecting group, preferably an amino protecting group.

**41.** A compound represented by Formula (iii):

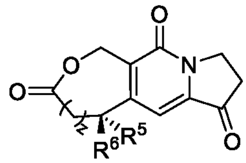

(iii)

wherein, $R^5$, $R^6$, and z independently have the definitions as described in any one of claims 35-38.

**42.** A compound represented by Formula (v):

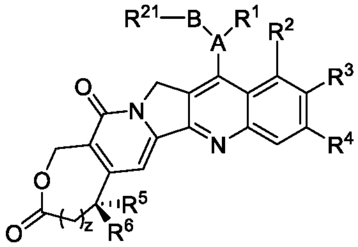

(v)

wherein, A, B, $R^1$, $R^2$, $R^3$, $R^4$, $R^5$, $R^6$, $R^{21}$, and z independently have the definitions as described in any one of claims 35 to 38.

**43.** A conjugate of the following structure:

Tp-L-D (D)

wherein, Tp represents a targeting moiety;
D represents a bioactive molecular fragment, preferably a molecular fragment with anti-tumor biological activity;
wherein, L is selected from the linker represented by Formula (L):

$\#L^1\text{-}L^2\text{-}L^3\text{-}L^4\text{-}L^5*$ (L)

wherein, $L^1$ is a linking moiety to the targeting moiety Tp, and formed from a reactive group $L^{1'}$ through its reaction with the targeting moiety Tp, and # represents the site connected to the Tp moiety;
$L^2$ is absent or a spacer between $L^1$ and $L^3$;
$L^3$ is a peptide moiety;
$L^4$ is absent or a spacer between the peptide moiety and $L^5$;

$L^5$ is a linking moiety connecting $L^4$ with the bioactive molecule D, and formed from a reactive group $L^{5'}$ through its reaction with the bioactive molecule, and * represents the site connected to the bioactive molecule D; preferably, $L^{1'}$, $L^1$, $L^2$, $L^3$, $L^4$, $L^5$, and $L^{5'}$ have the definitions as described in any one of claims 14 to 22; provided that:

Condition I:
the following hydrophilic moiety is inserted between the fragments of L as defined above:
hydrophilic moiety:

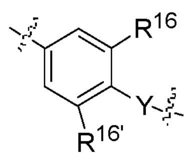 or 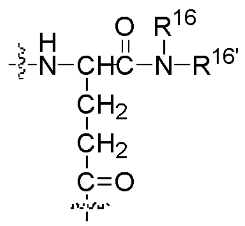 or 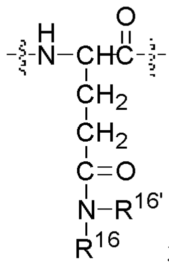 ;

or Condition II:

$L^1$ is generated by coupling Tp with a sulfhydryl-reactive group contained in maleimide group or a substituted maleimide group;
the following steric hindrance moiety is inserted between $L^1$ and $L^2$, or between $L^2$ and $L^3$, or is inserted by replacing $L^2$:
steric hindrance moiety:

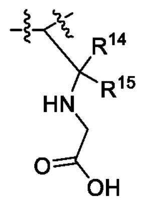 ;

or Condition III:

$L^1$ is generated by coupling Tp with a sulfhydryl-reactive group contained in Hal-Het-;
$L^4$-$L^5$ is: $-NR^{z1}-C(R^{z2}R^{z3})-T^L-$;
wherein:

$R^{z1}$ is H or $C_{1-4}$ alkyl;
$R^{z2}$ and $R^{z3}$ are the same or different, and are independently selected from H or $C_{1-4}$ alkyl;
$T^L$ has the definition as described in any one of claims 1 to 13;
in a further embodiment,
$L^1$ is generated by coupling Tp with a sulfhydryl-reactive group contained in maleimide group or Hal-Het-;
the following hydrophilic moiety is inserted between $L^1$ and $L^2$, or between $L^2$ and $L^3$, or is inserted by replacing $L^2$:

hydrophilic moiety:

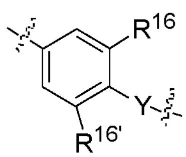 or 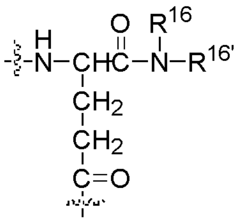 or 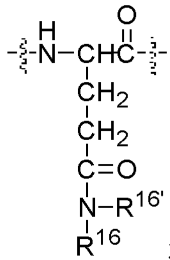 ;

in a further embodiment:

$L^1$ is generated by coupling Tp with a sulfhydryl-reactive group contained in Hal-Het-;
$L^4$-$L^5$ is: $-NR^{z1}-C(R^{z2}R^{z3})-O-(CH_2)_m-C(R^{z4}R^{z5})-CO-$;
wherein:

$R^{z1}$ is H or $C_{1-4}$ alkyl;
m is an integer of 0 to 4;
$R^{z2}$ and $R^{z3}$ are the same or different and are independently selected from H or $C_{1-4}$ alkyl;
$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl;

or Condition IV:

$L^1$-$L^2$ is generated by coupling Tp with $L^{1'}$-$L^2$ of the following structure:

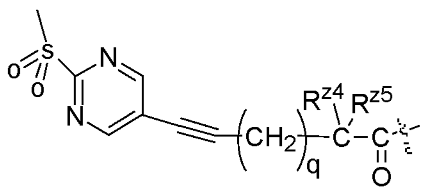

q is an integer selected from 0 to 10, preferably 1, 2, 3, 4, 5, 6, 7 or 8;
$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl, wherein at least one of $R^{z4}$ and $R^{z5}$ is not H;
it is further preferred that $R^{z4}$ and $R^{z5}$ together form a $C_{3-6}$ cycloalkyl;
$L^1$-$L^2$ is most preferably generated by coupling Tp with

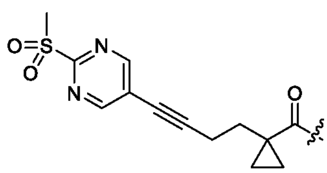.

**44.** The conjugate according to claim 43, wherein the bioactive molecule D is a compound with biological activity or potential biological activity disclosed in the Chinese Pharmacopoeia, The United States Pharmacopoeia or European Pharmacopoeia or disclosed in other publications;

the drug can be selected from the group consisting of cytotoxic drugs, cytostatic drugs or immunosuppressive drugs, preferably anti-tubulin agents, tubulin inhibitors, DNA minor groove binders, DNA replication inhibitors, alkylating agents, antibiotics, antifolates, antimetabolites, chemosensitizers, topoisomerase inhibitors, vinca alkaloids, etc.;
the drug is further preferably selected from the group consisting of auristatin, camptothecin, duocarmycin, etoposide, maytansine and maytansine alkaloids, taxanes, benzodiazepines or benzodiazepines-containing

drugs, and vinca alkaloids.

**45.** A pharmaceutical composition, wherein the pharmaceutical composition comprises at least one selected from the following:

the conjugate represented by Formula (C), or a stereoisomer, racemate, tautomer, isotopologue, isotope-labeled compound, nitroxide or pharmaceutically acceptable salt thereof according to any one of claims 1 to 33; and/or the compound represented by Formula (GH), or a stereoisomer, racemate, tautomer, isotopologue, isotope-labeled compound, nitroxide or pharmaceutically acceptable salt thereof according to any one of claims 35 to 38; and/or
the conjugate represented by Formula (D), or a stereoisomer, racemate, tautomer, isotopologue, isotope-labeled compound, nitroxide or pharmaceutically acceptable salt thereof according to any one of claims 43 to 44;
preferably, the compound represented by the Formula (GH), or a stereoisomer, racemate, tautomer, isotopologue, isotope-labeled compound, nitroxide or pharmaceutically acceptable salt thereof, or the conjugate represented by the Formula (C) or (D), or a stereoisomer, racemate, tautomer, isotopologue, isotope-labeled compound, nitroxide or pharmaceutically acceptable salt thereof in the pharmaceutical composition is present in a therapeutically effective amount.

**46.** Use of the compound represented by Formula (GH), or a stereoisomer, racemate, tautomer, isotopologue, isotope-labeled compound, nitroxide or pharmaceutically acceptable salt thereof according to any one of claims 35 to 38, or the conjugate represented by of Formula (C) according to any one of claims 1 to 33 or the conjugate represented by Formula (D) according to any one of claims 43 to 44, or a stereoisomer, racemate, tautomer, isotopologue, isotope-labeled compound, nitroxide or pharmaceutically acceptable salt thereof, in preventing and/or treating a disease or disorder, and/or in the manufacture of a medicament;
preferably, the disease or disorder can be selected from a tumor, such as a solid tumor or a hematological cancer.

**47.** The use according to claim 46, wherein the medicament is used for preventing and/or treating a disease or disorder;

preferably, the disease or disorder may be selected from a tumor, such as a solid tumor or a hematological cancer;
the solid tumor is selected from malignancies of various organ systems, such as sarcomas, adenocarcinomas, blastomas, and carcinomas, such as those affecting liver, lung, breast, lymph, biliary-intestinal (e.g., colon), genitourinary tract (e.g., kidney, urothelial cell), prostate and pharynx. adenocarcinomas comprise, for example, malignancies of most colon cancers, rectal cancers, renal cell carcinomas, liver cancers, small cell lung cancers, non-small cell lung cancers, small intestine cancers and esophageal cancers;
the hematological cancer is selected from the group consisting of leukemia, lymphoma, and malignant lymphoproliferative disorders affecting blood, bone marrow and lymphatic system.

**48.** A conjugate of a linker and a drug, which has the following structure:

$$L^{1'}\text{-}L^{2}\text{-}L^{3}\text{-}L^{4}\text{-}L^{5}\text{-}G \qquad (C')$$

wherein: G had the definition as described in any one of claims 1 to 13;
$L^{1'}$, $L^2$, $L^3$, $L^4$, and $L^5$ have the definitions as described in any one of claims 14 to 22.

**49.** The conjugate of linker and drug according to claim 4, wherein the conjugate is one selected from the following:

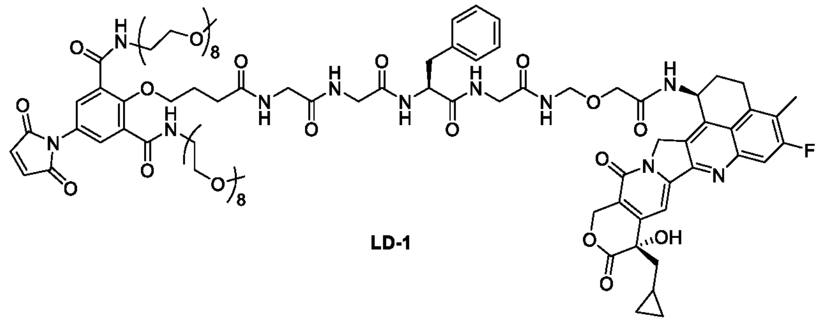

LD-1

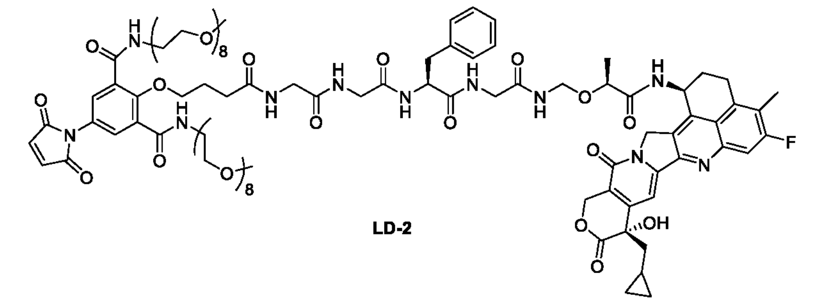

LD-2

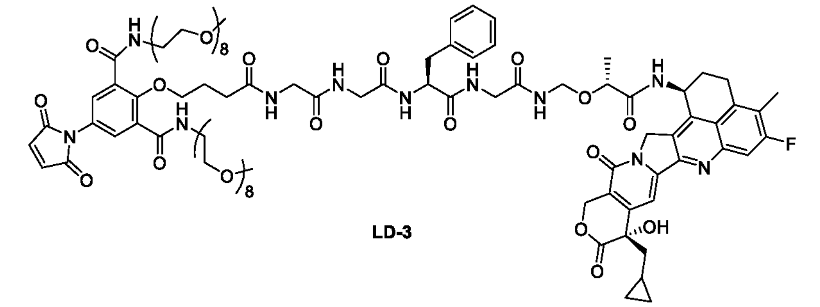

LD-3

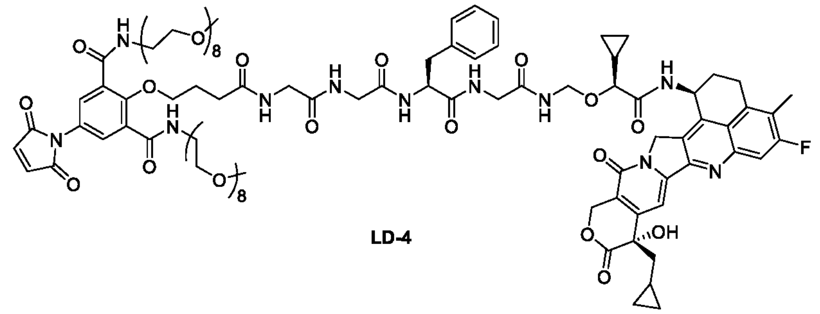

LD-4

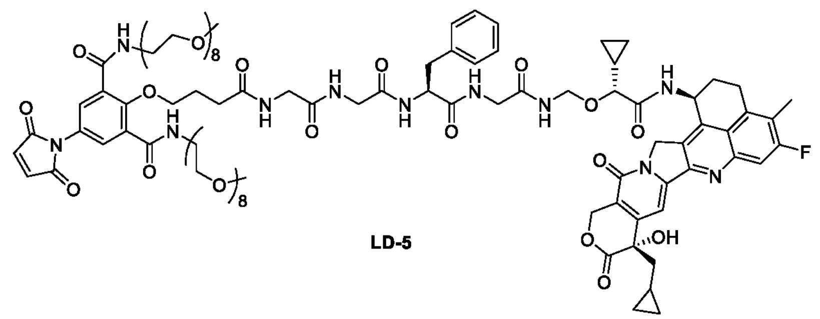

LD-5

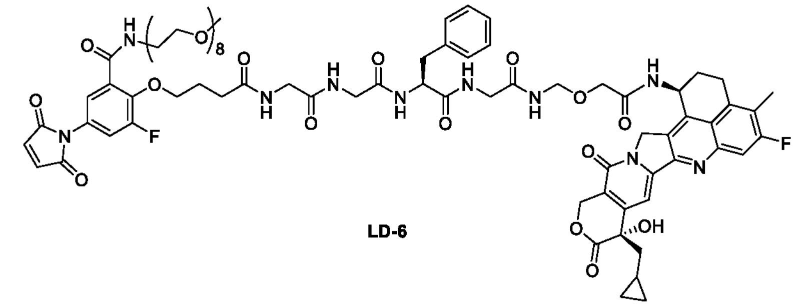

LD-6

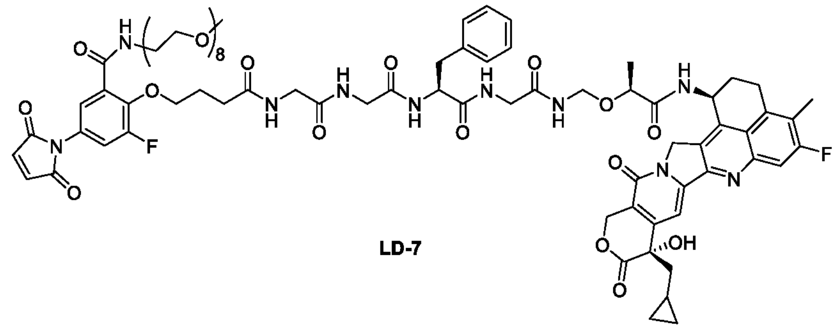

LD-7

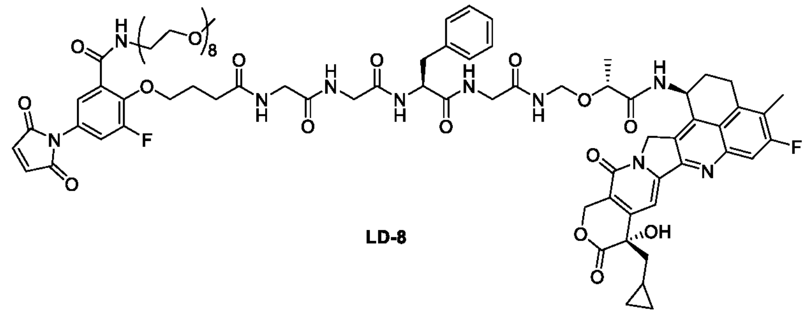

LD-8

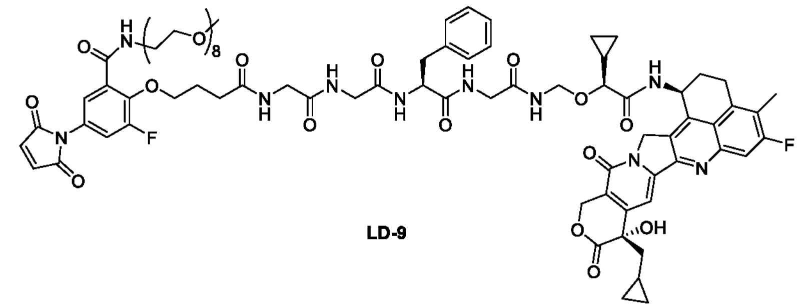

LD-9

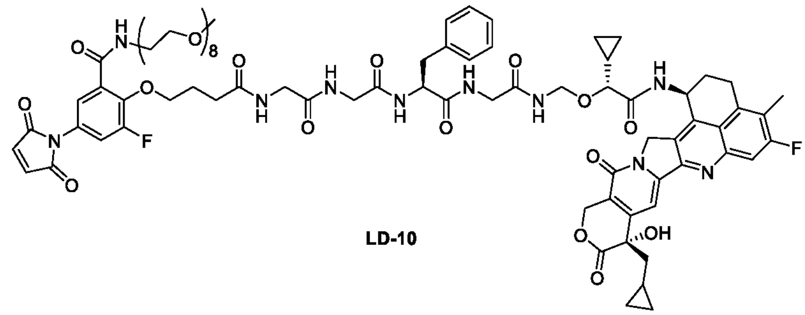

LD-10

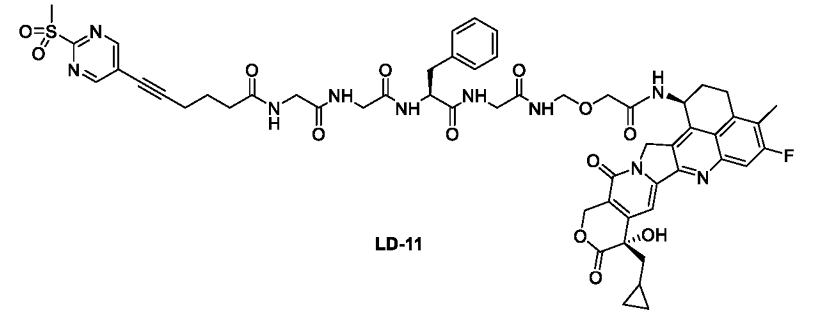

LD-11

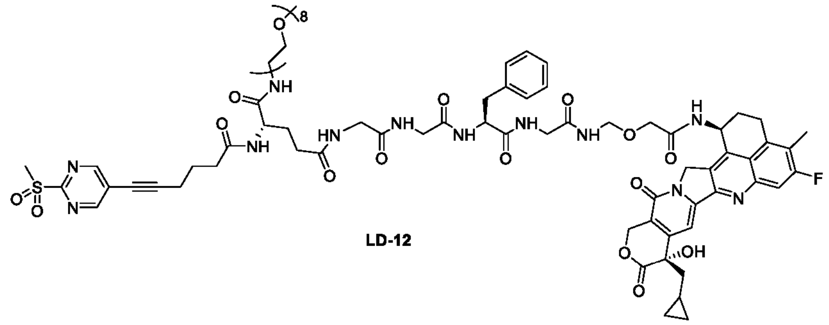

LD-12

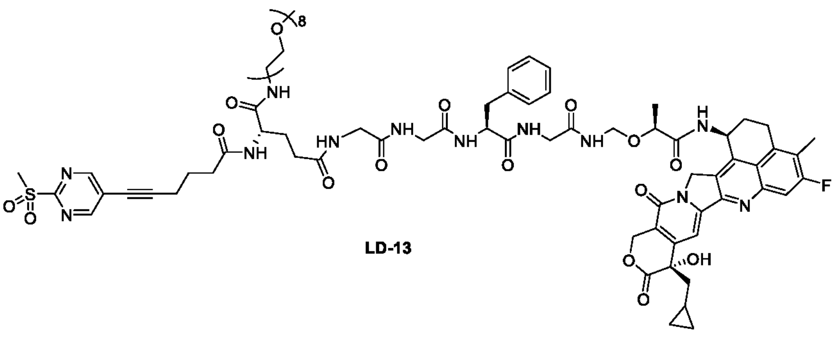

LD-13

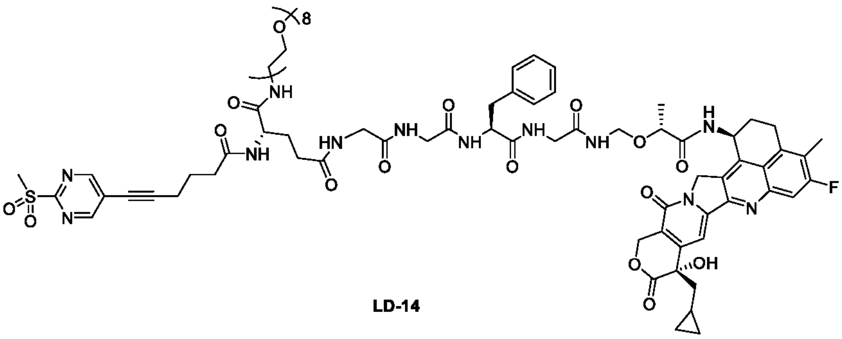

LD-14

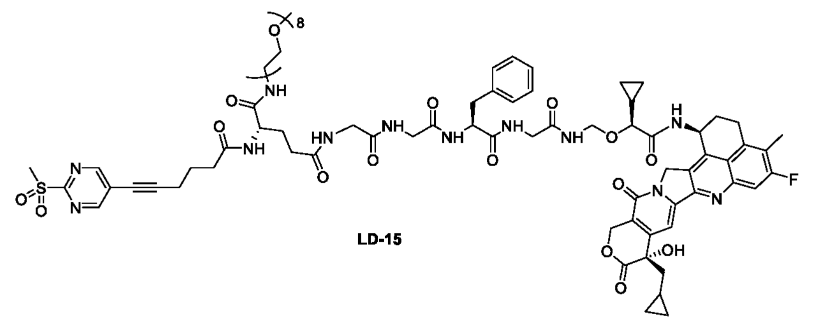

LD-15

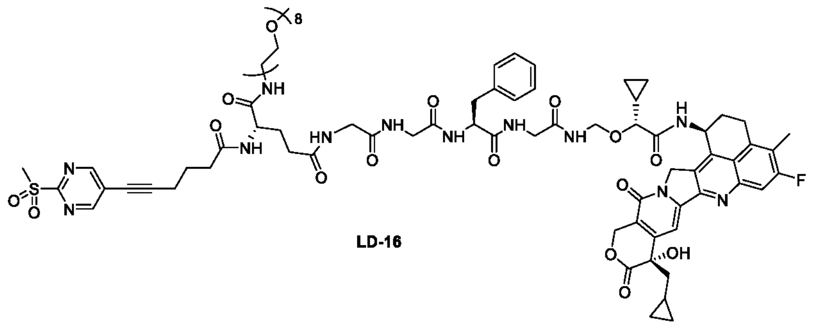

LD-16

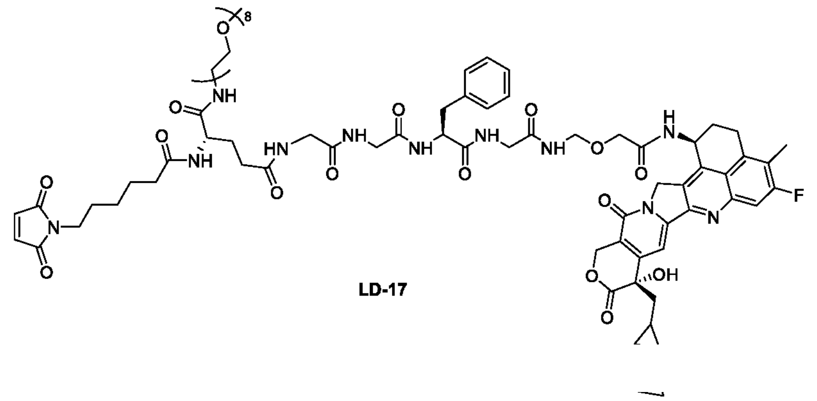

LD-17

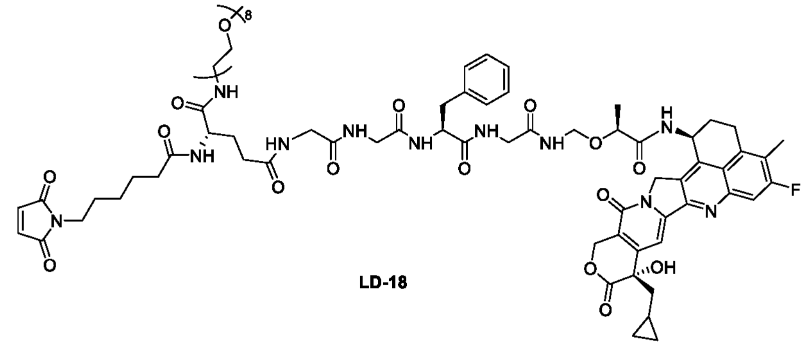

LD-18

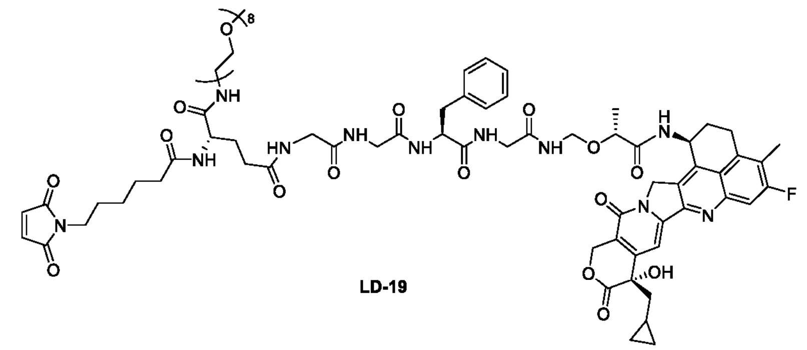

LD-19

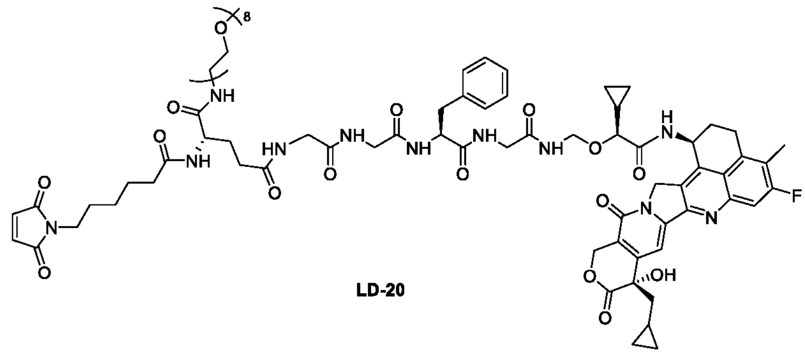

LD-20

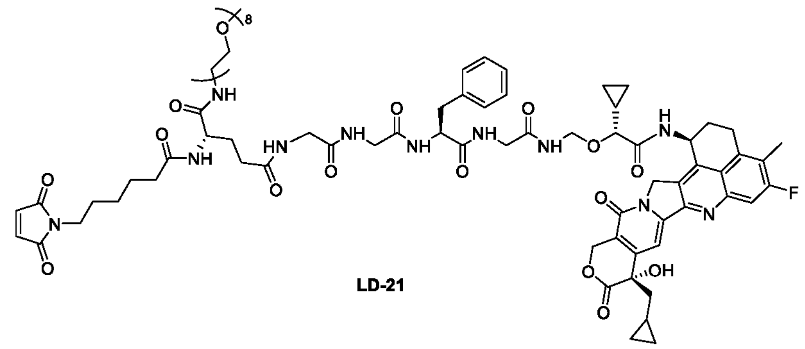

LD-21

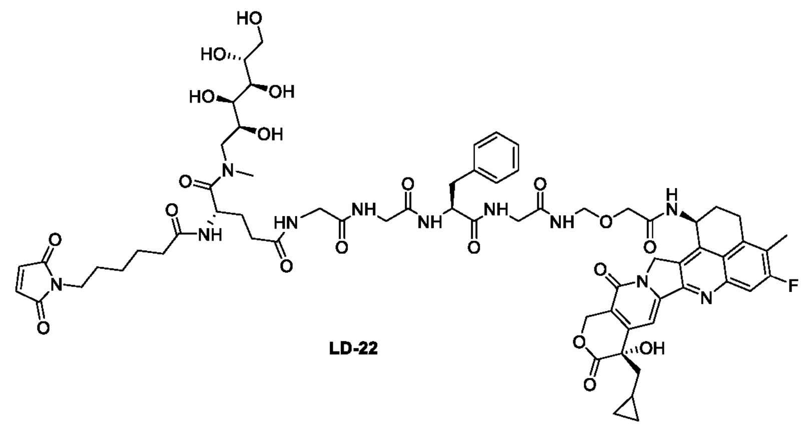

LD-22

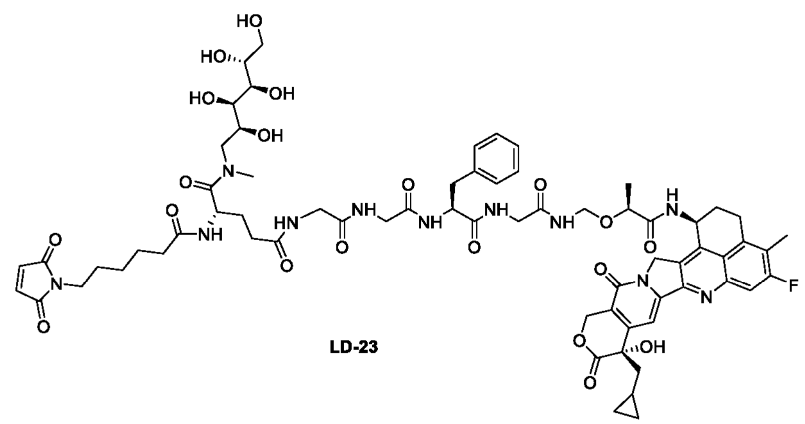

LD-23

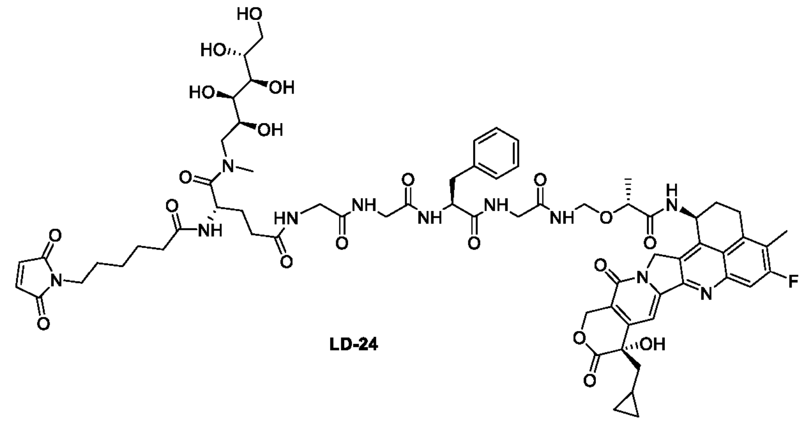

LD-24

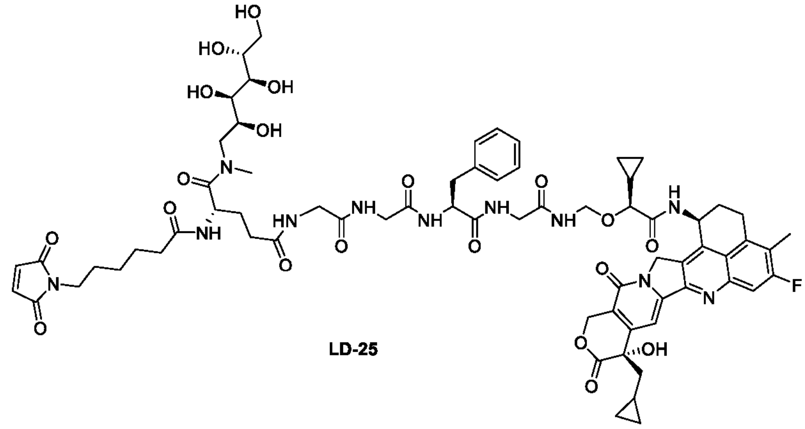

LD-25

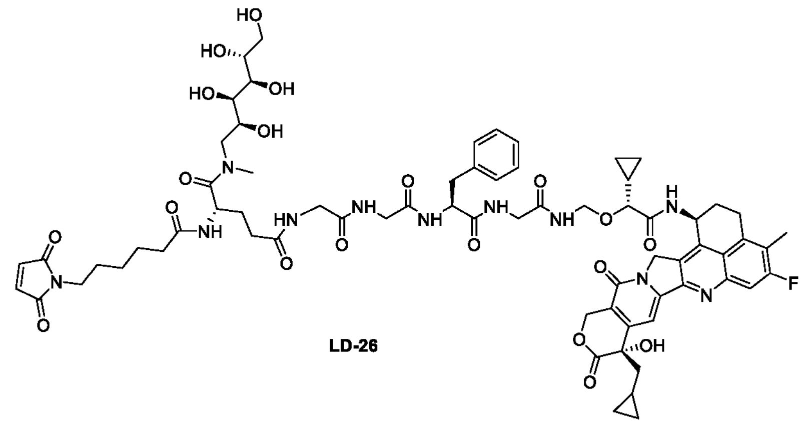

LD-26

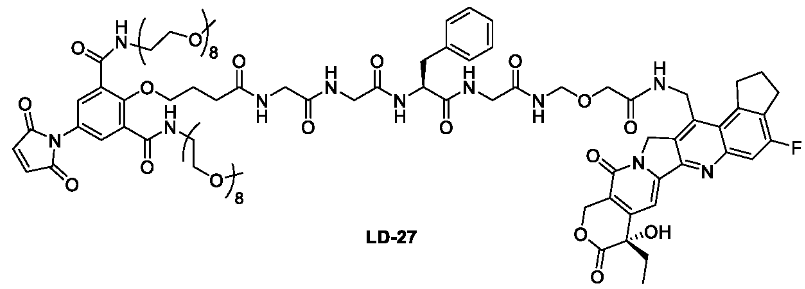

LD-27

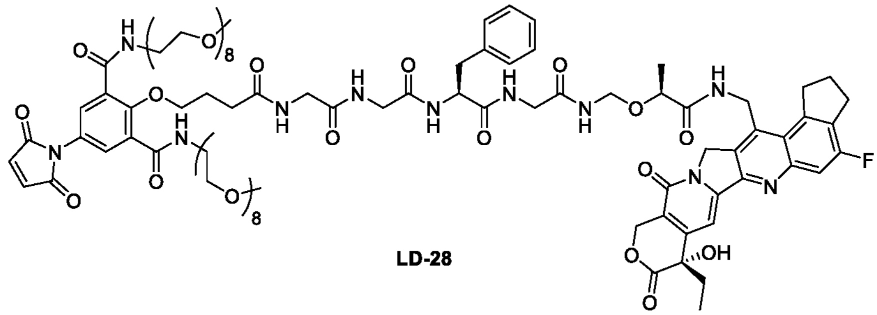

LD-28

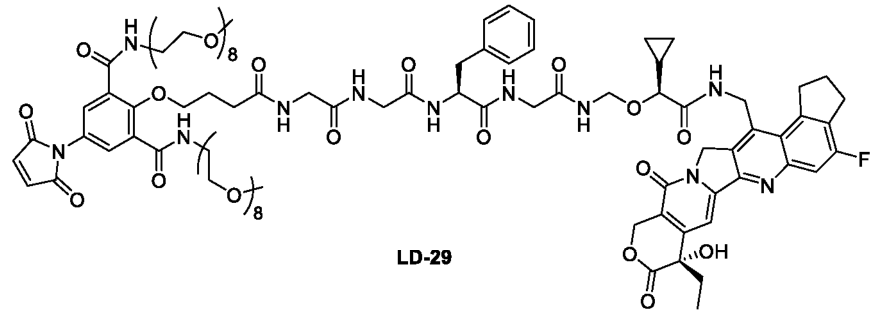

LD-29

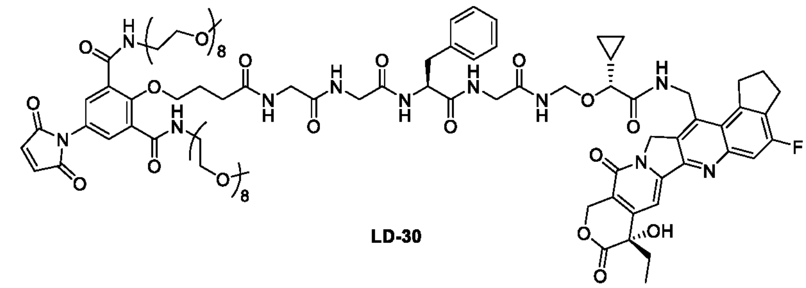

LD-30

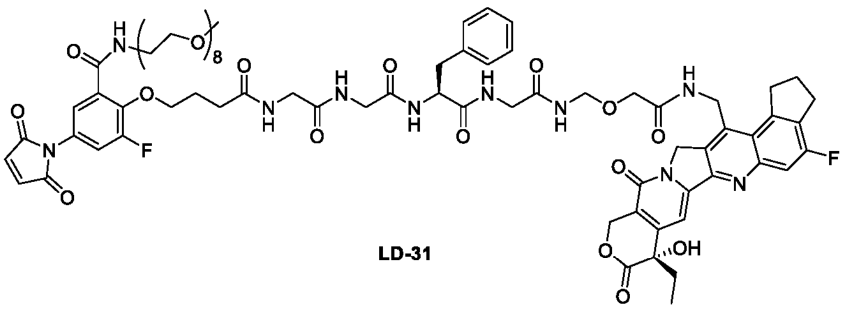

LD-31

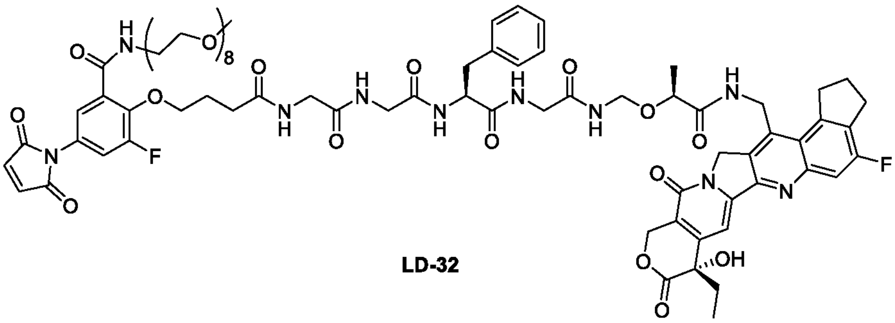

LD-32

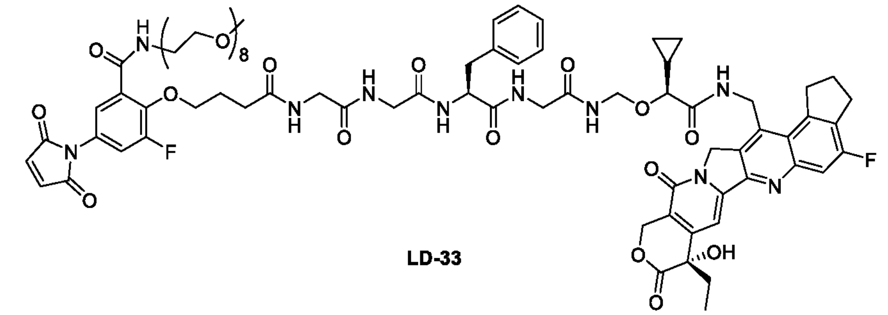

**LD-33**

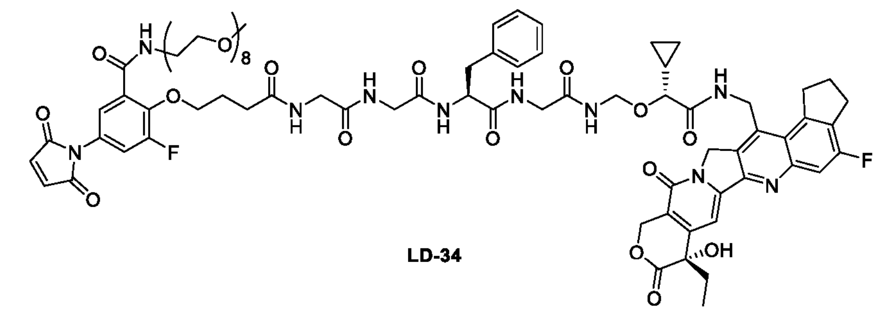

**LD-34**

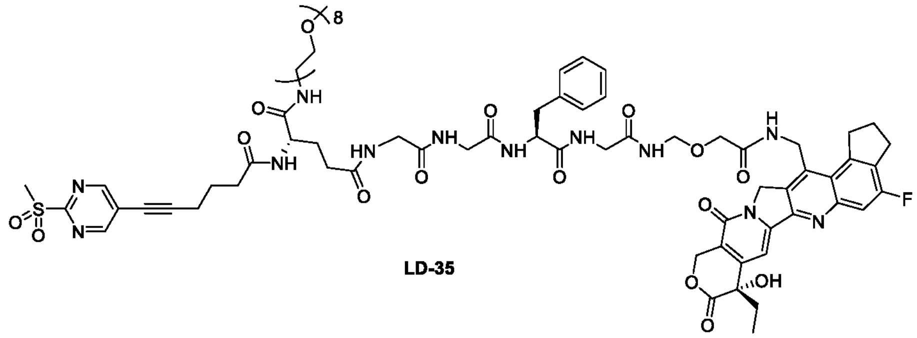

**LD-35**

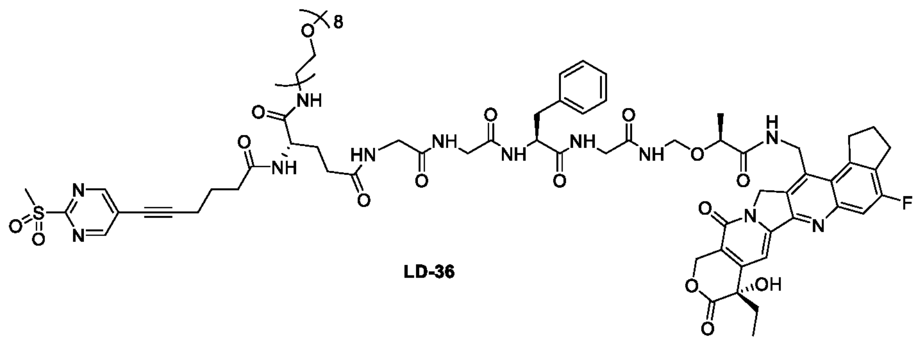

**LD-36**

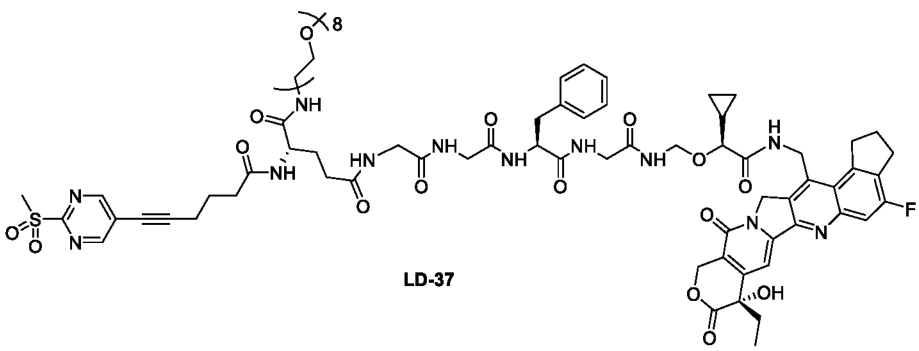

**LD-37**

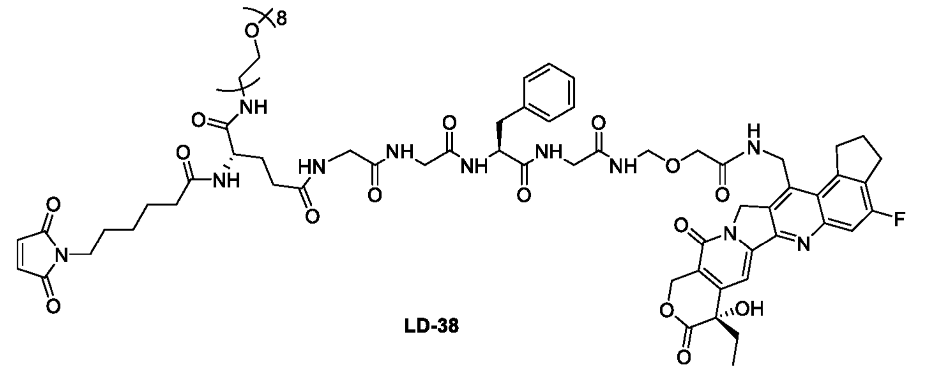

LD-38

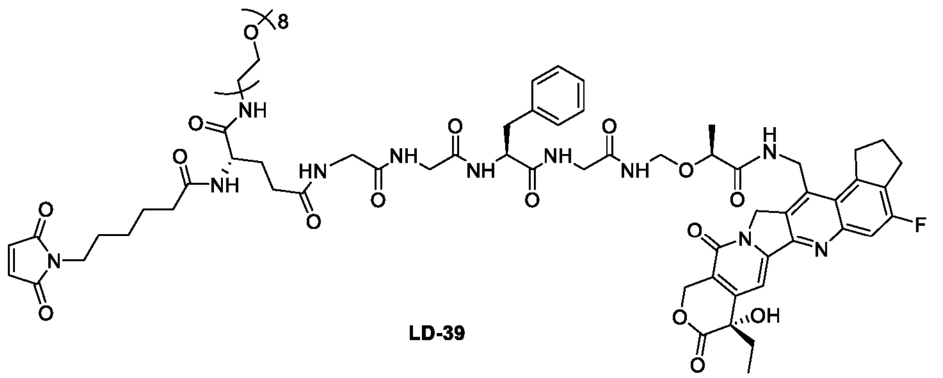

LD-39

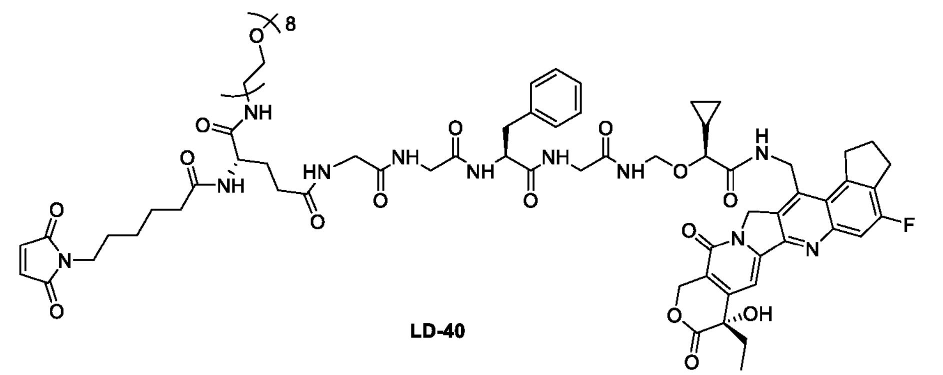

LD-40

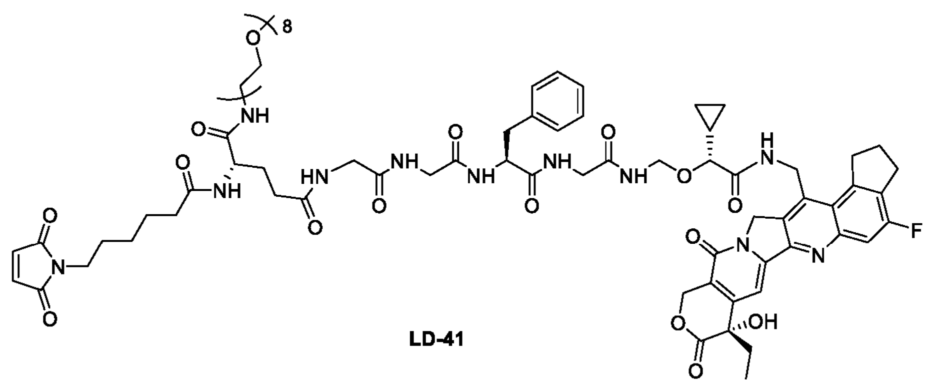

LD-41

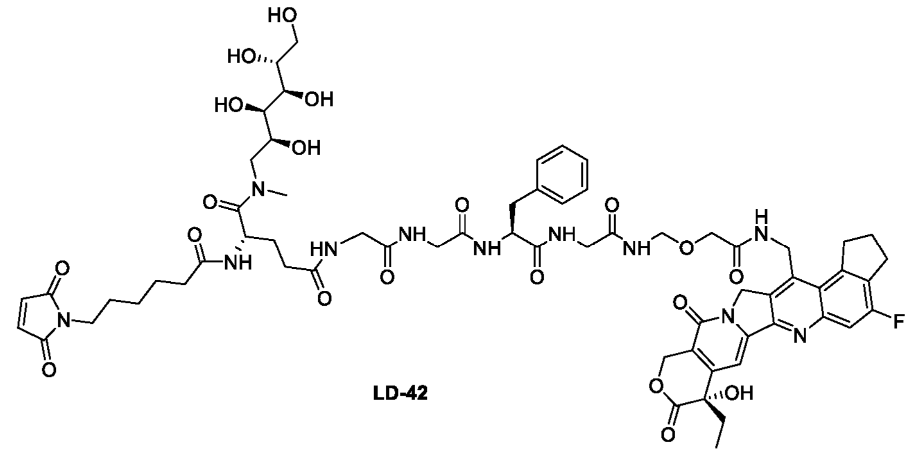

LD-42

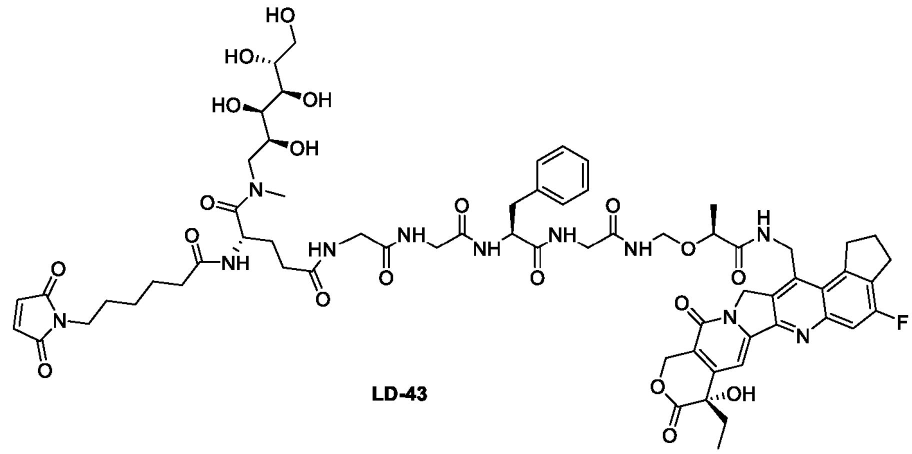

LD-43

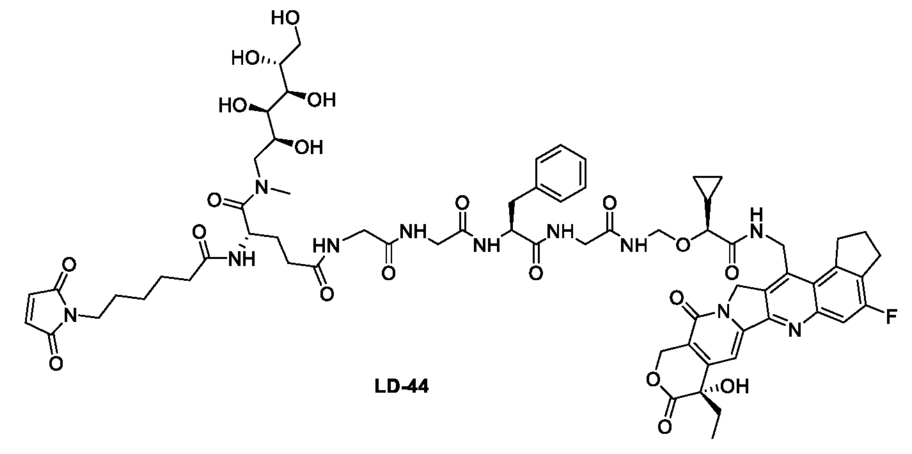

LD-44

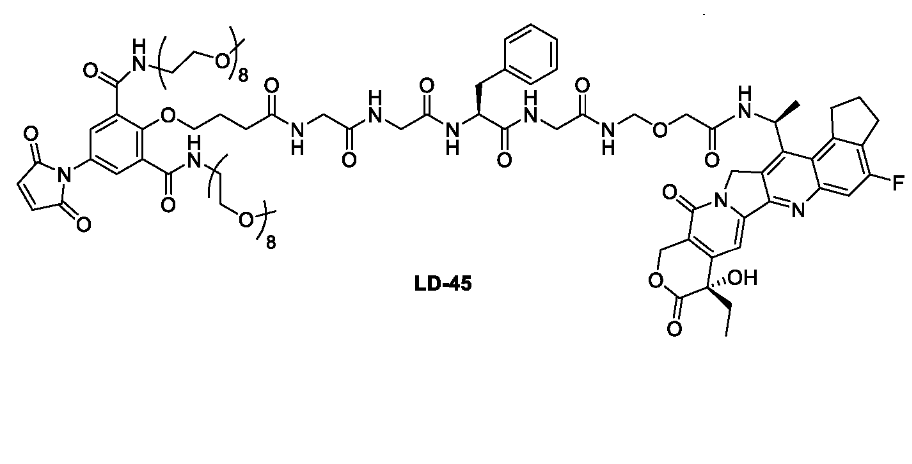

LD-45

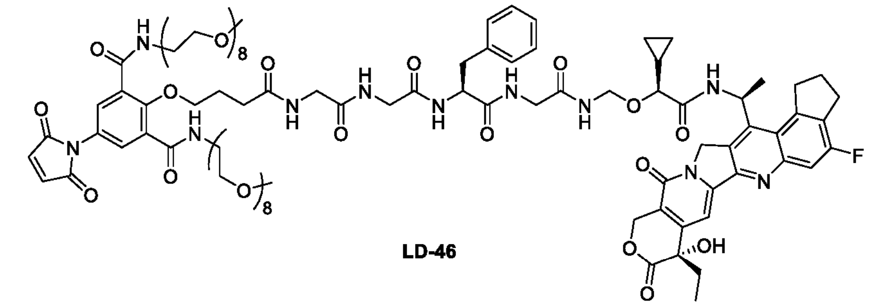

LD-46

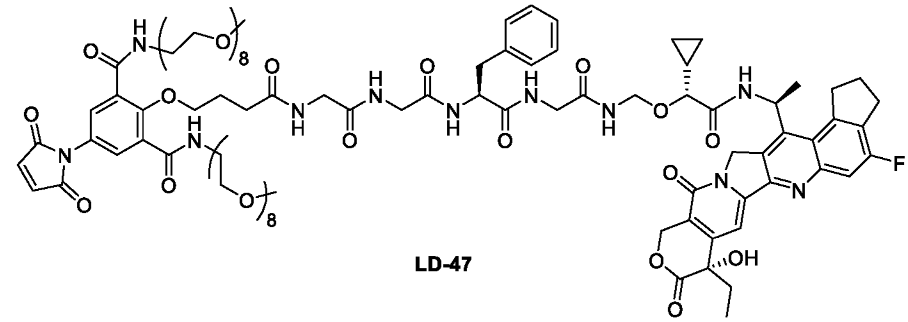

LD-47

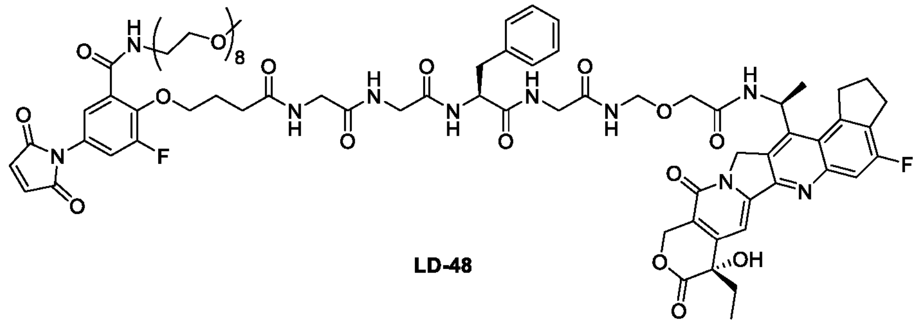

LD-48

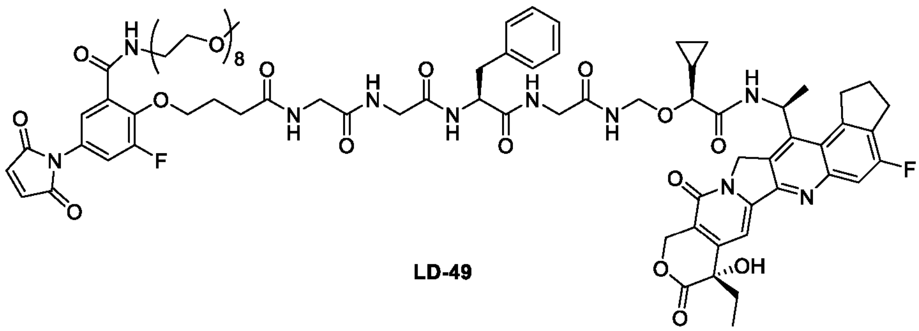

LD-49

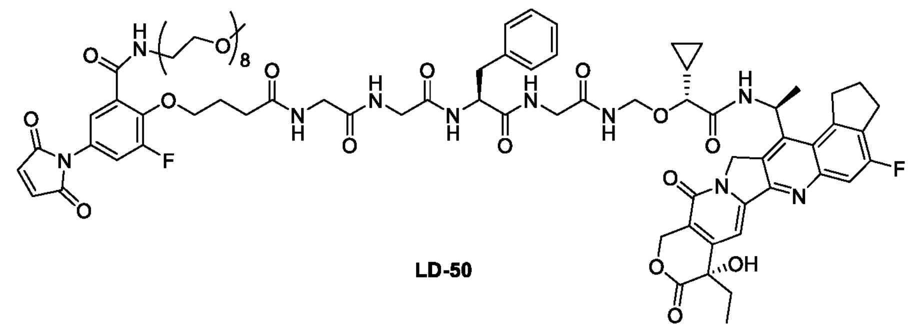

LD-50

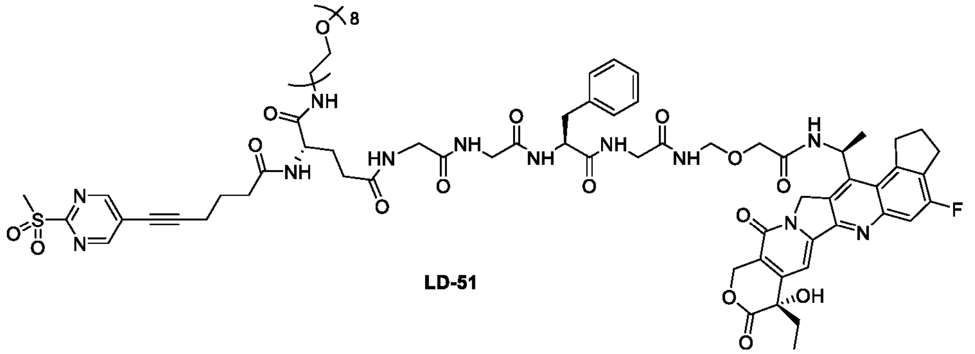

LD-51

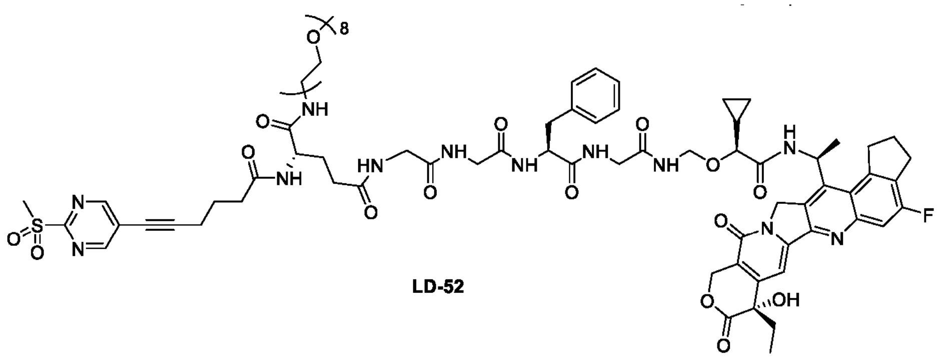

LD-52

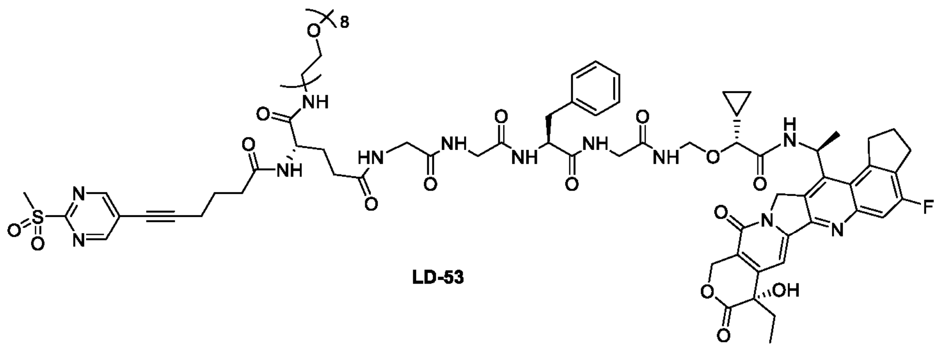

LD-53

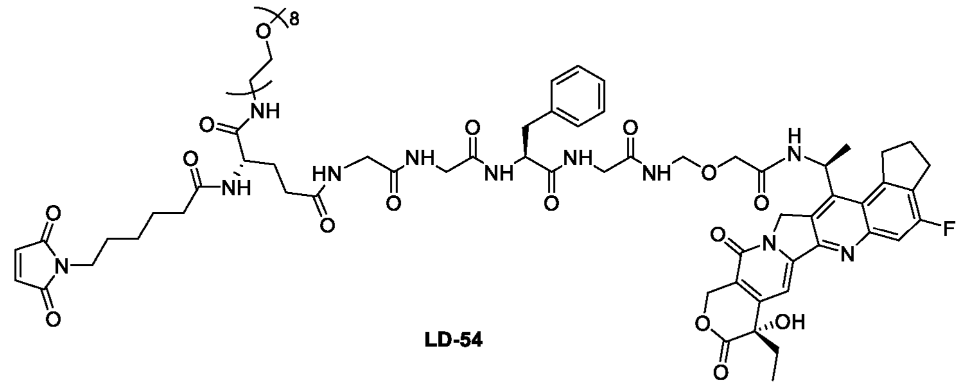

LD-54

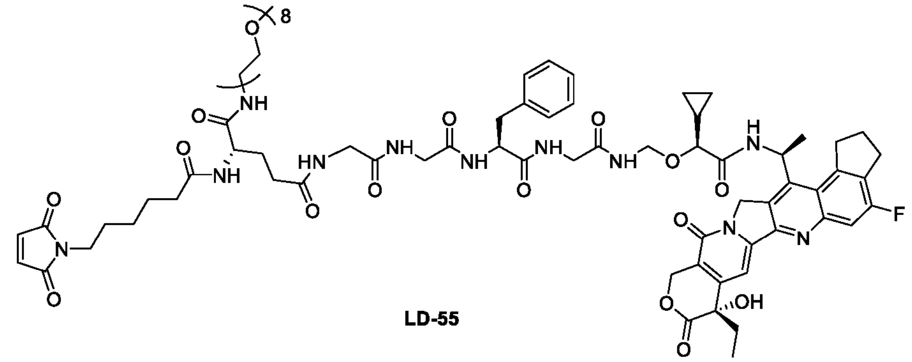

**LD-55**

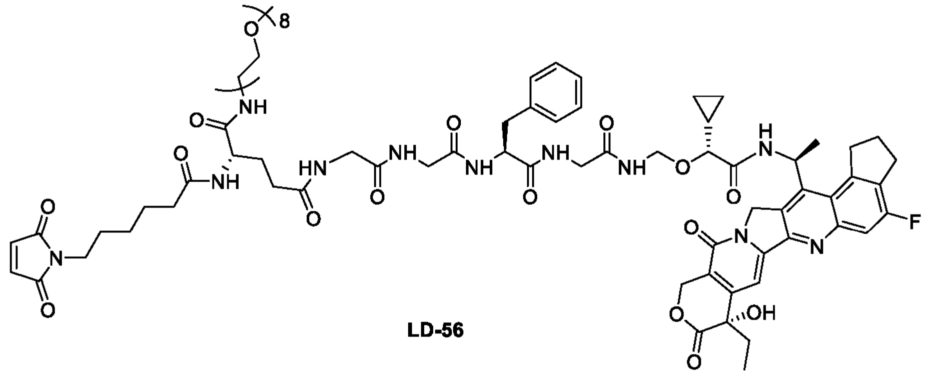

**LD-56**

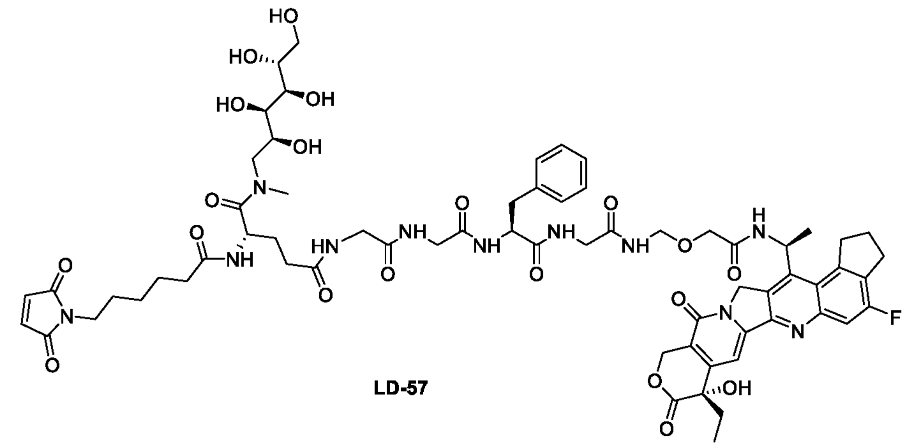

**LD-57**

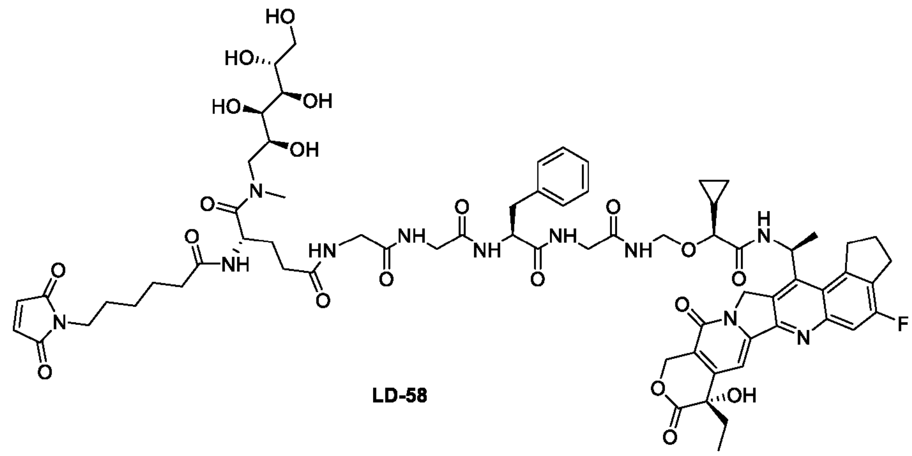

**LD-58**

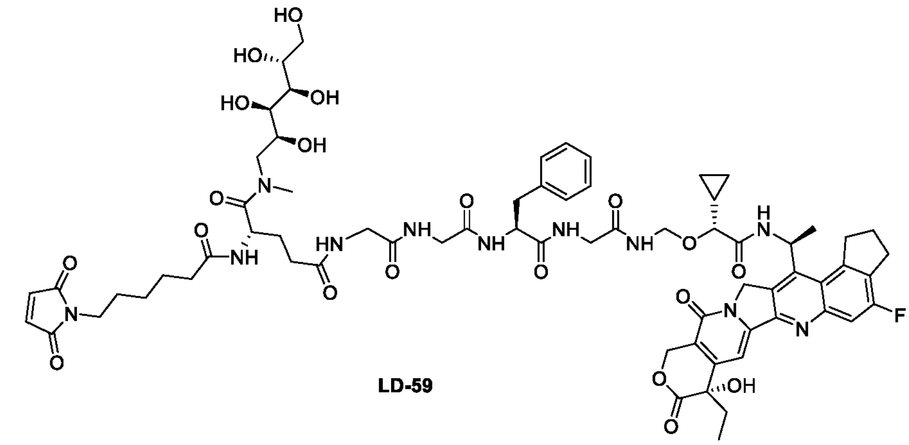

LD-59

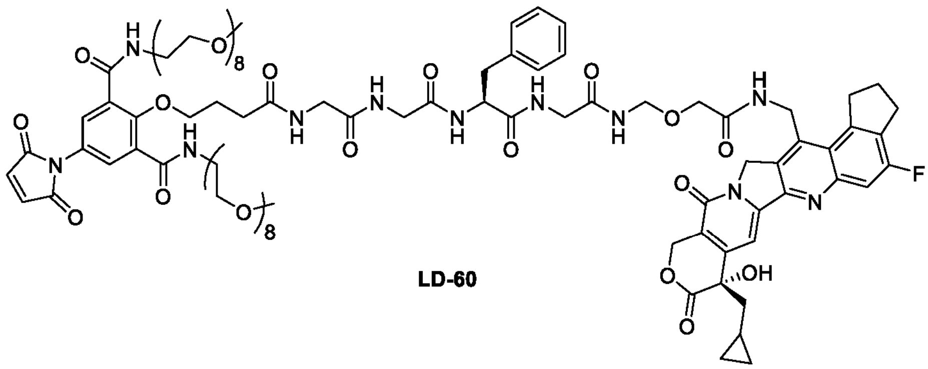

LD-60

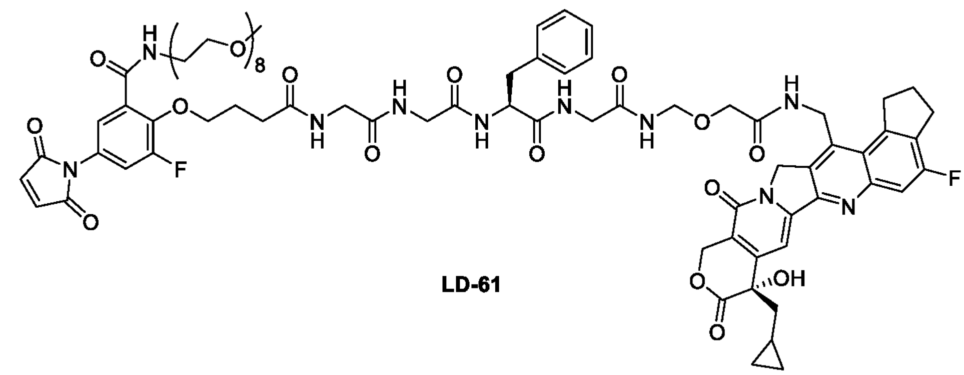

LD-61

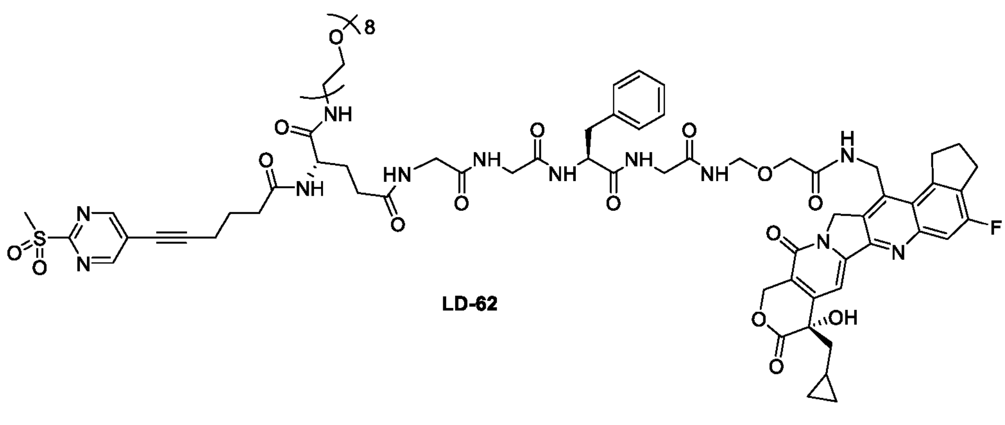

LD-62

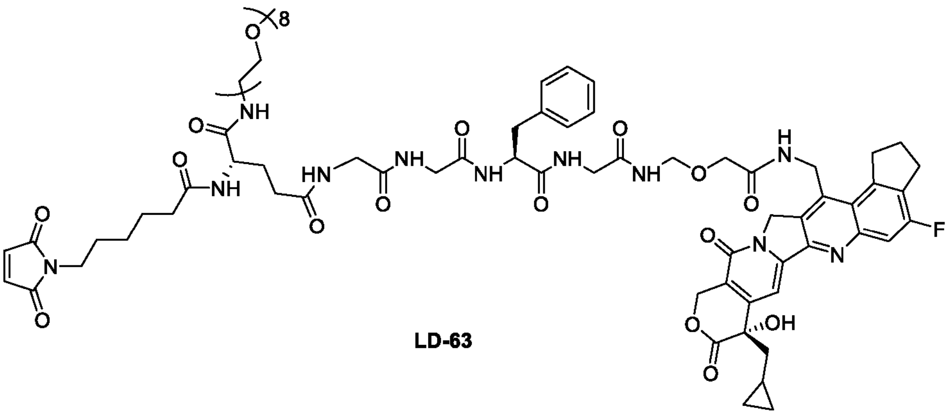

LD-63

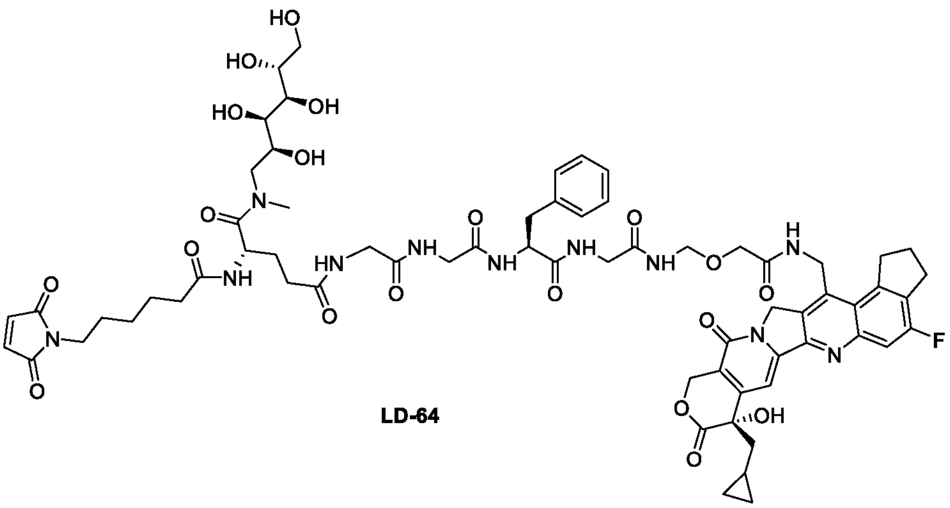

LD-64

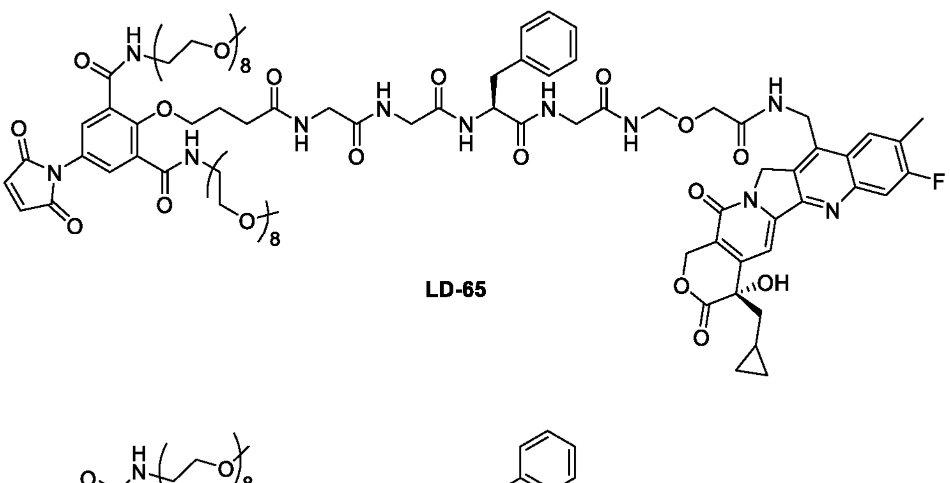

LD-65

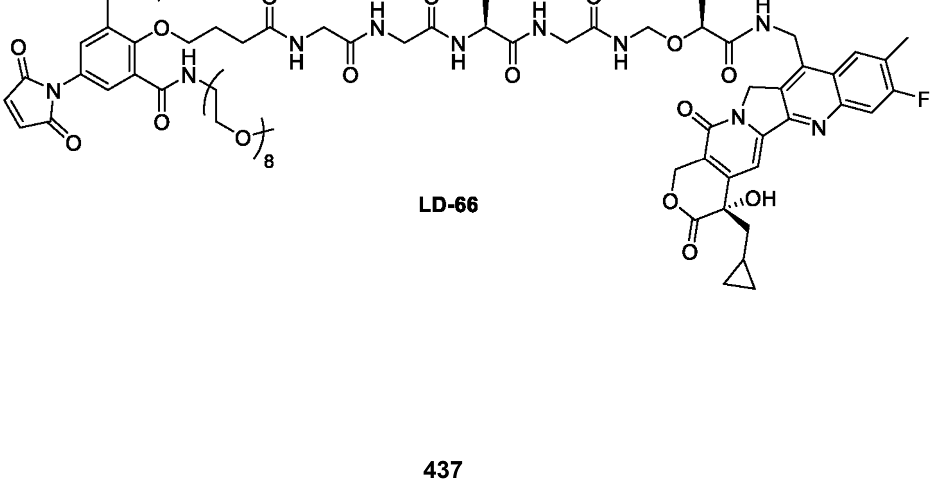

LD-66

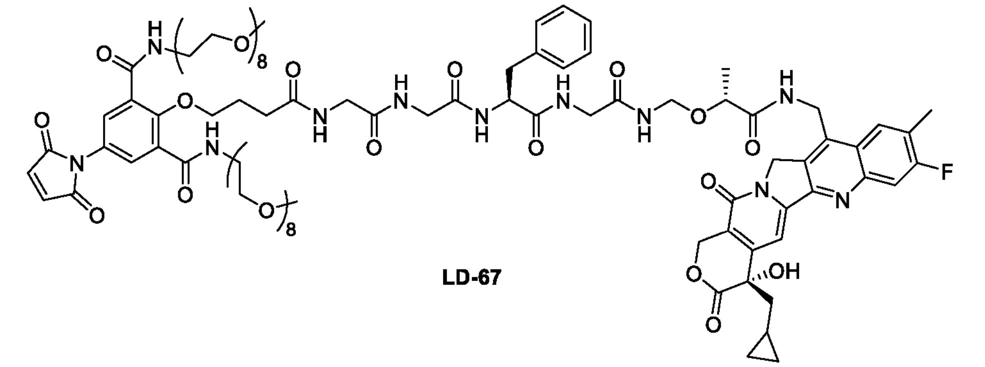

**LD-67**

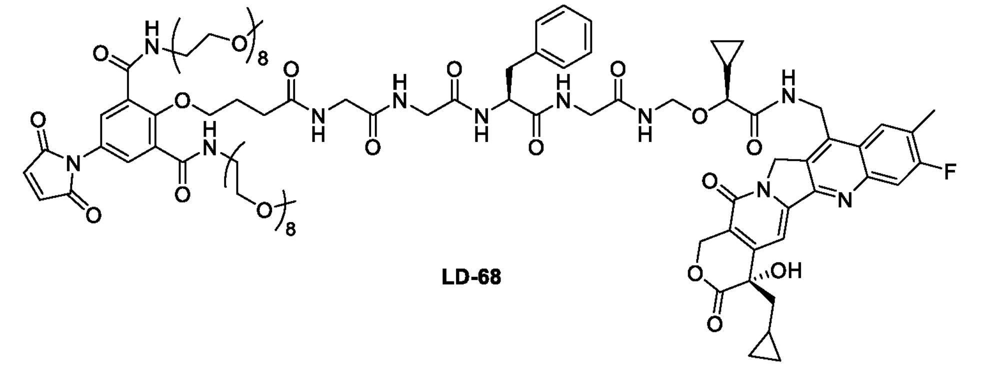

**LD-68**

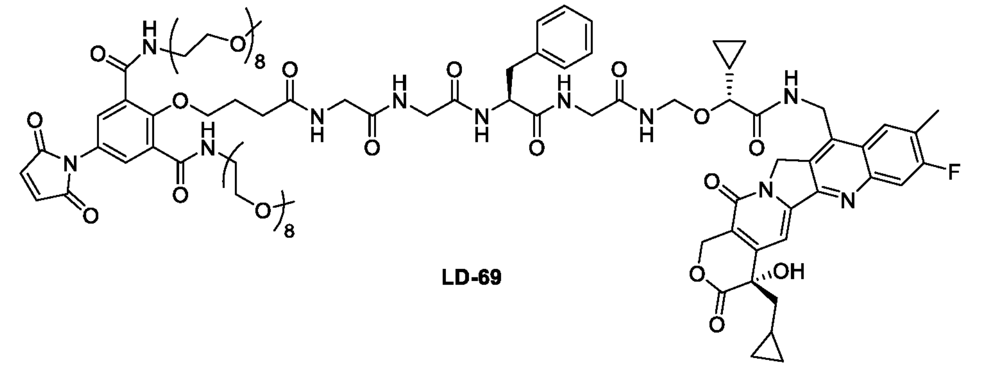

**LD-69**

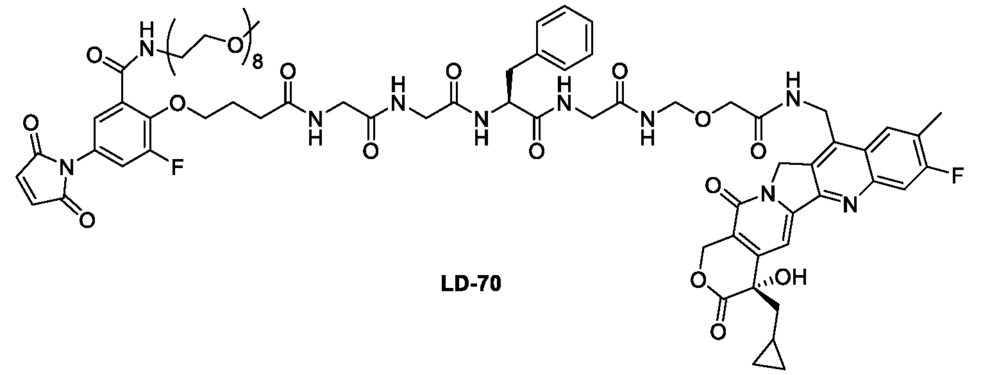

**LD-70**

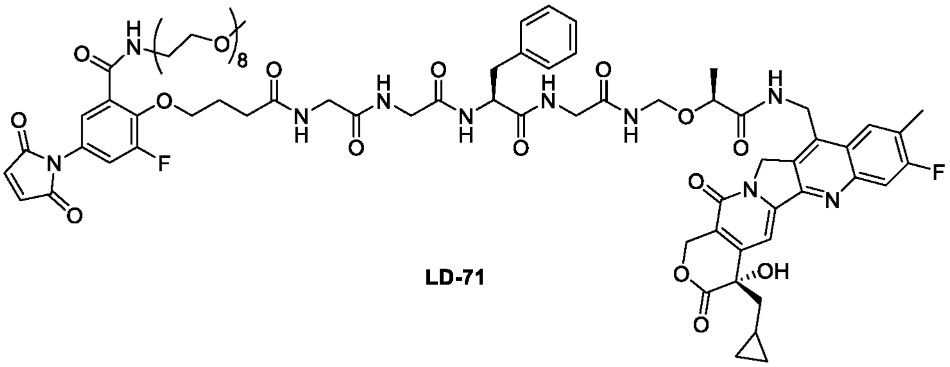

LD-71

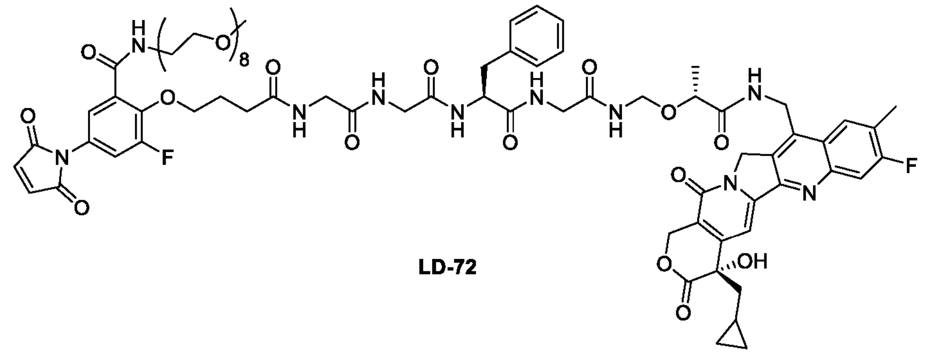

LD-72

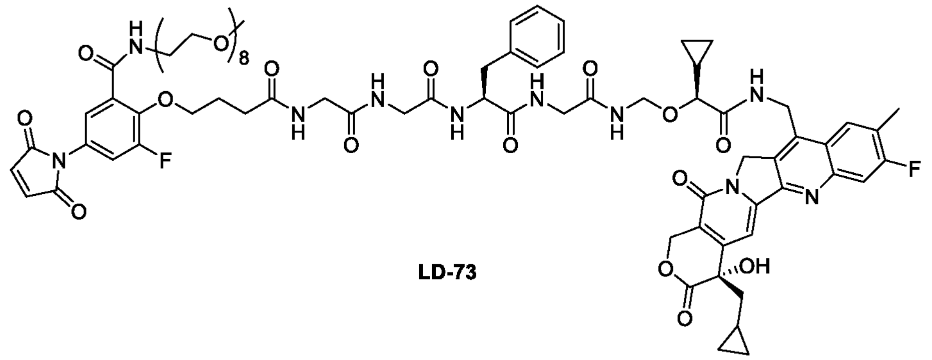

LD-73

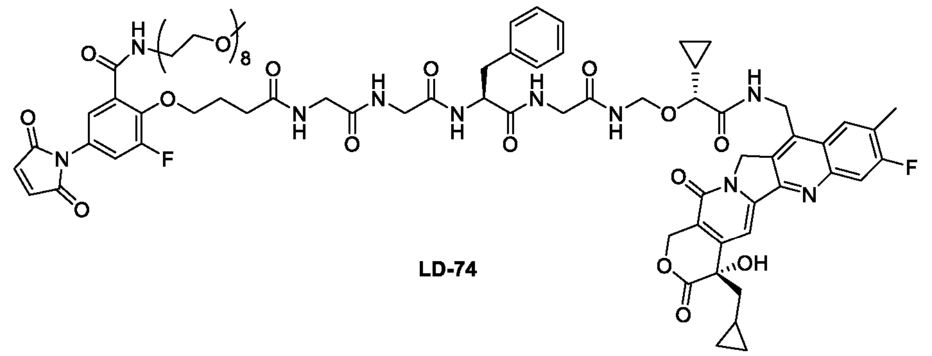

LD-74

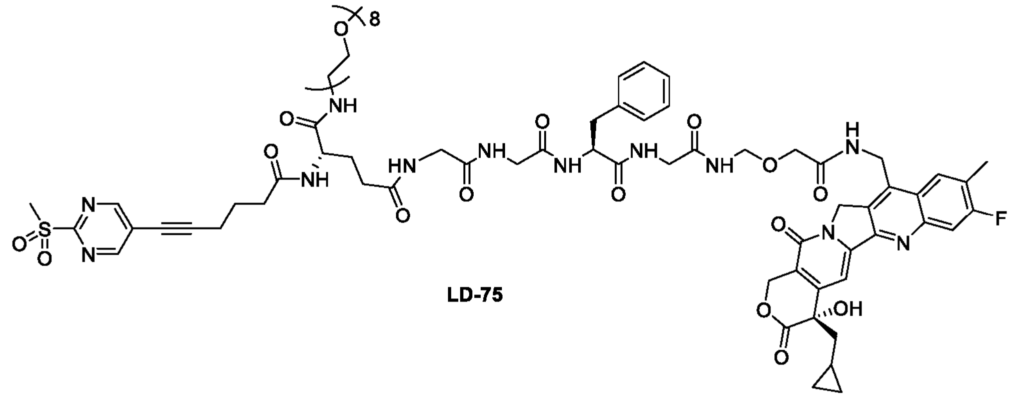

LD-75

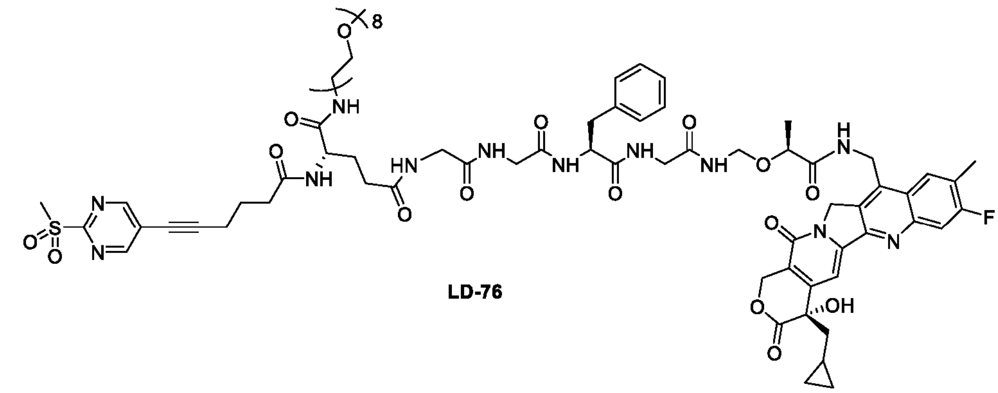

LD-76

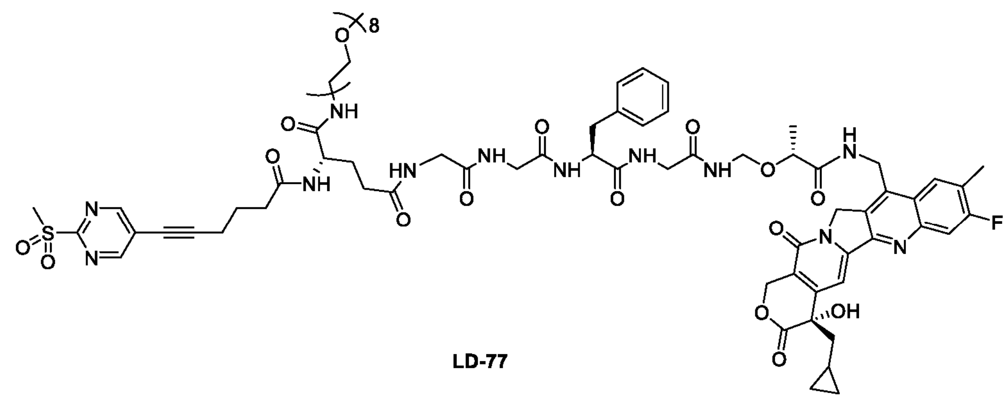

LD-77

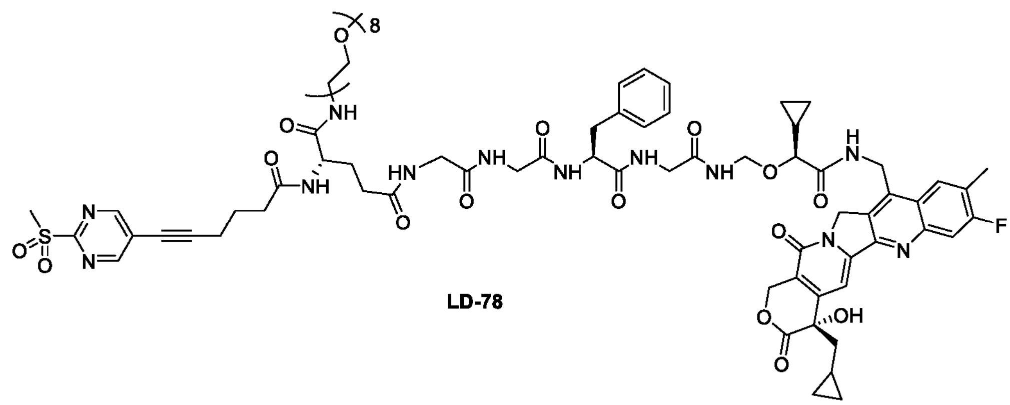

LD-78

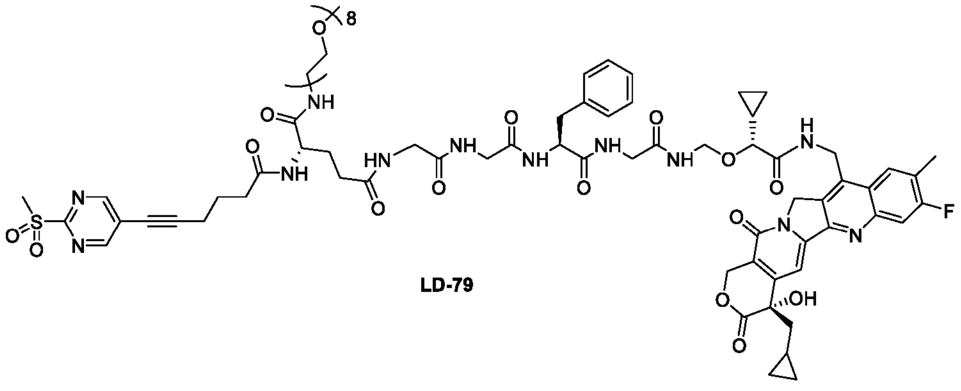

LD-79

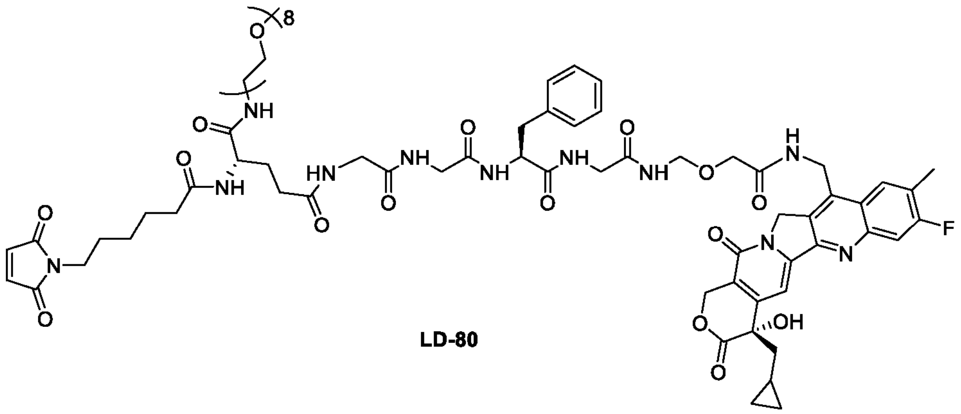

LD-80

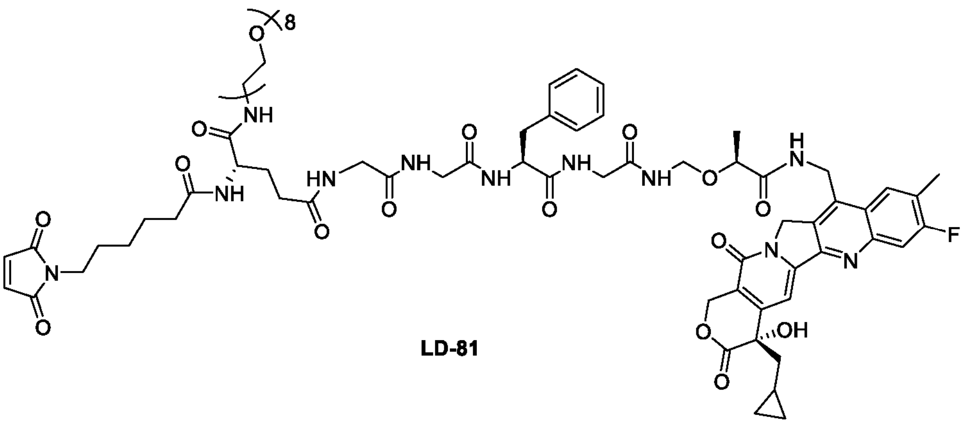

LD-81

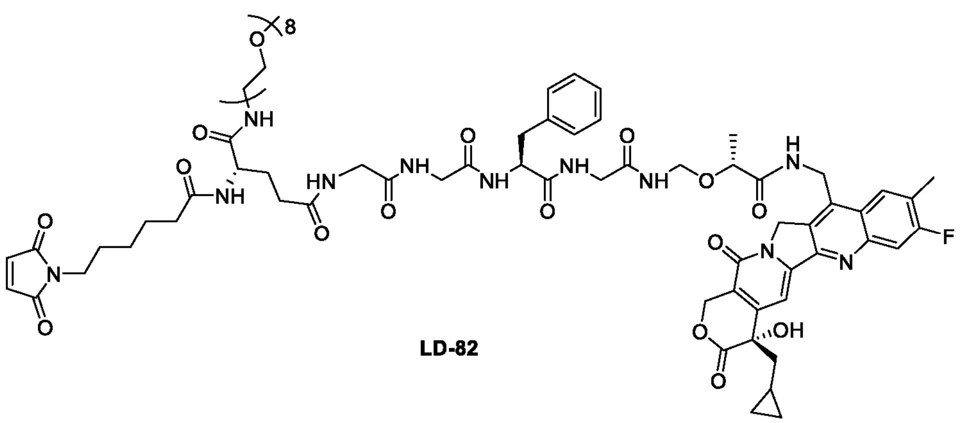

LD-82

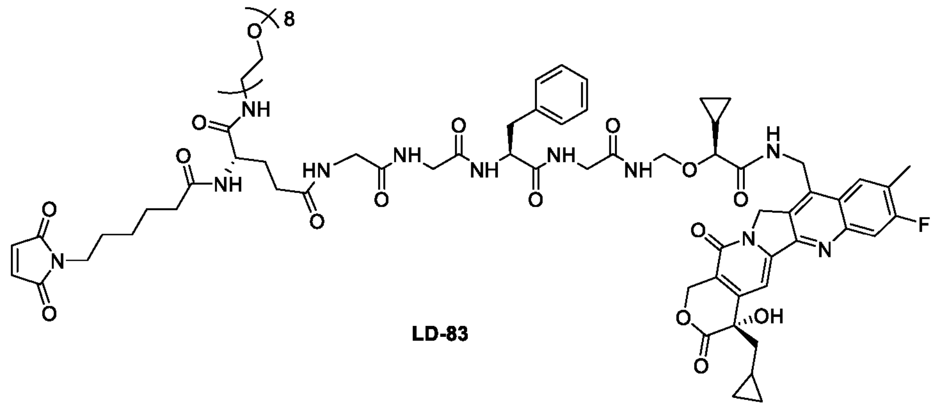

LD-83

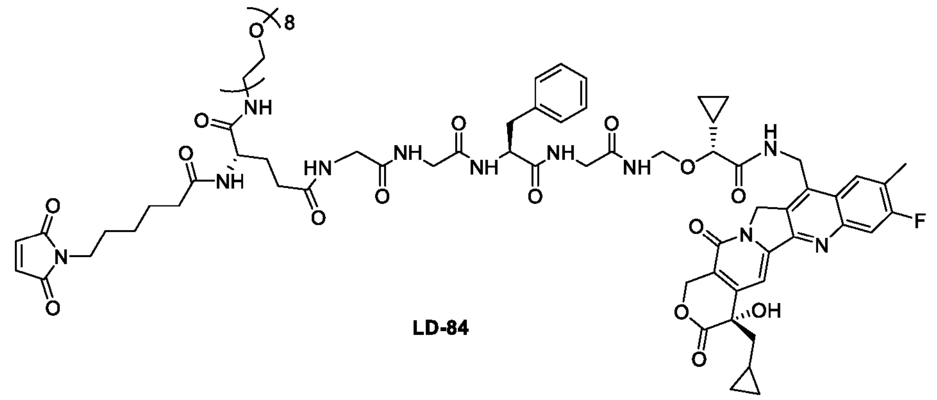

LD-84

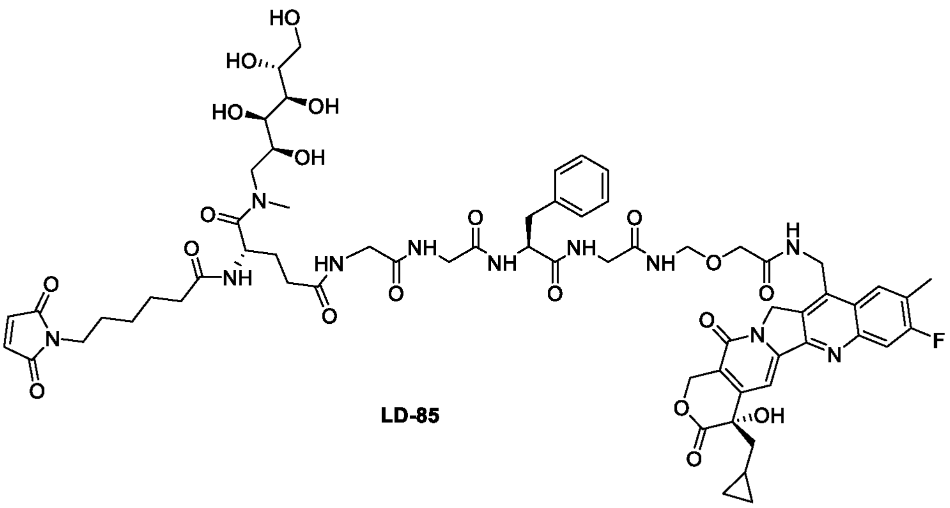

LD-85

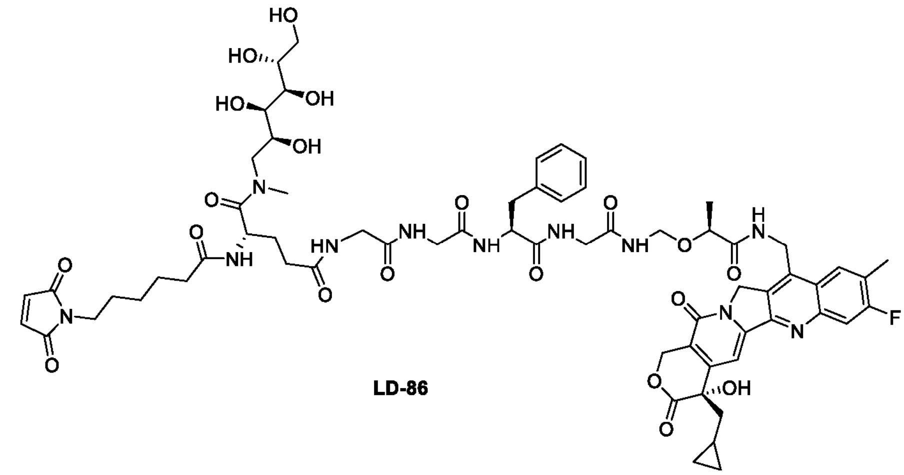

**LD-86**

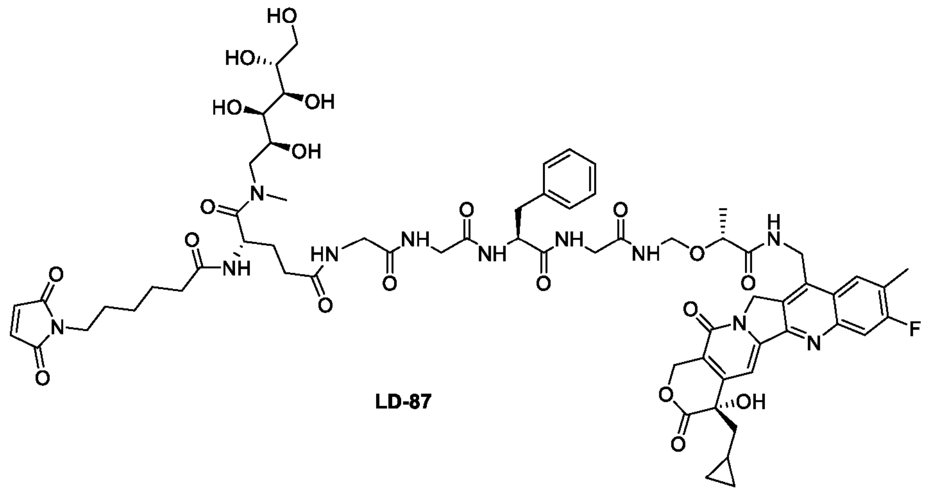

**LD-87**

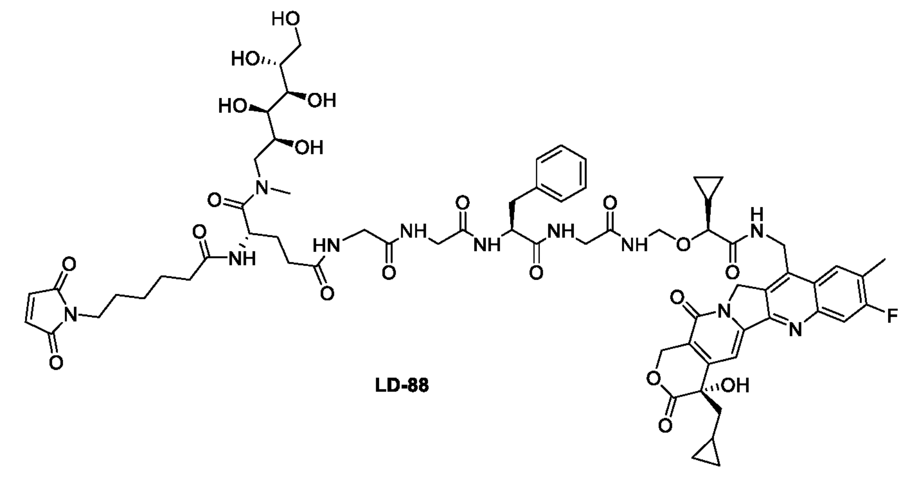

**LD-88**

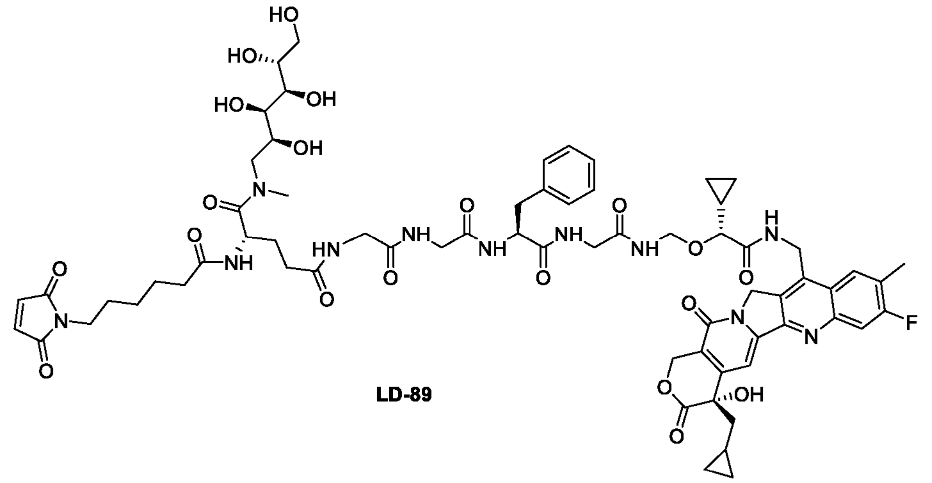

**LD-89**

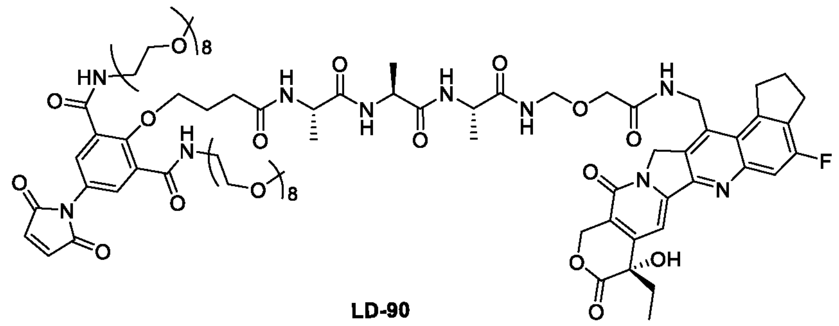

**LD-90**

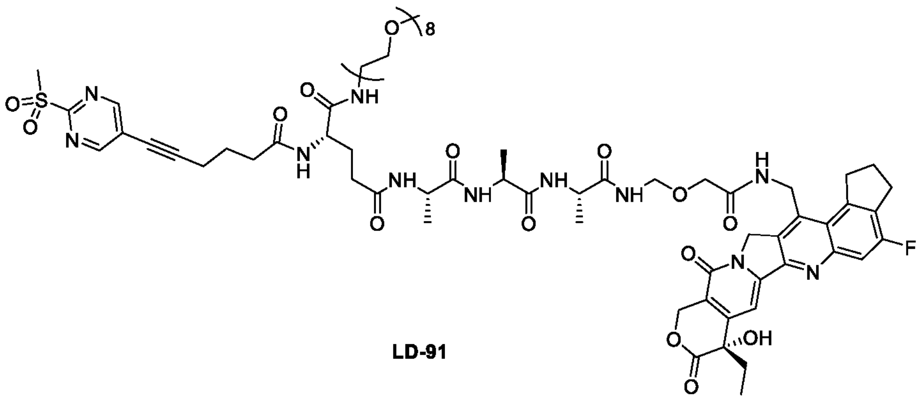

**LD-91**

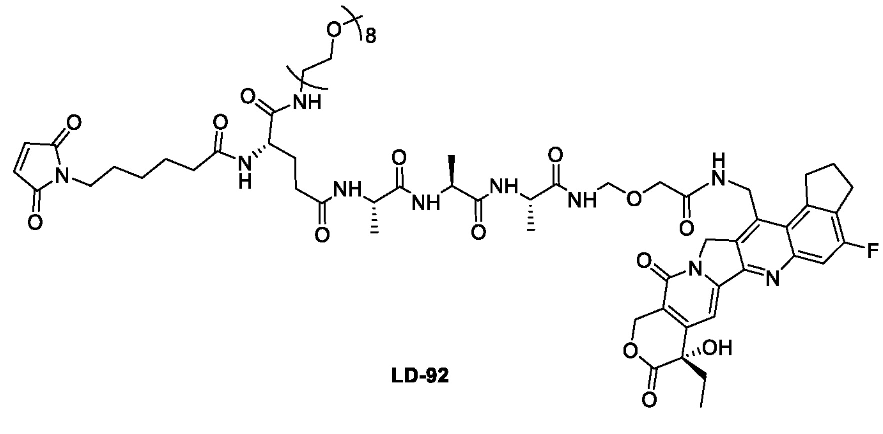

**LD-92**

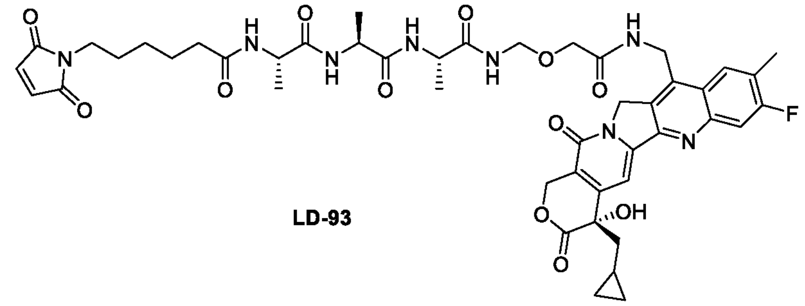

LD-93

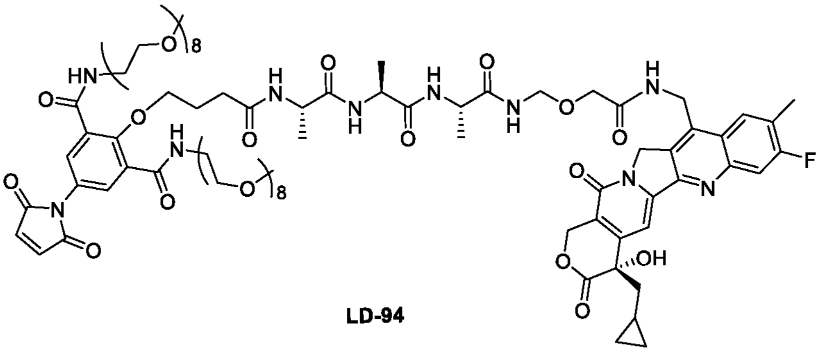

LD-94

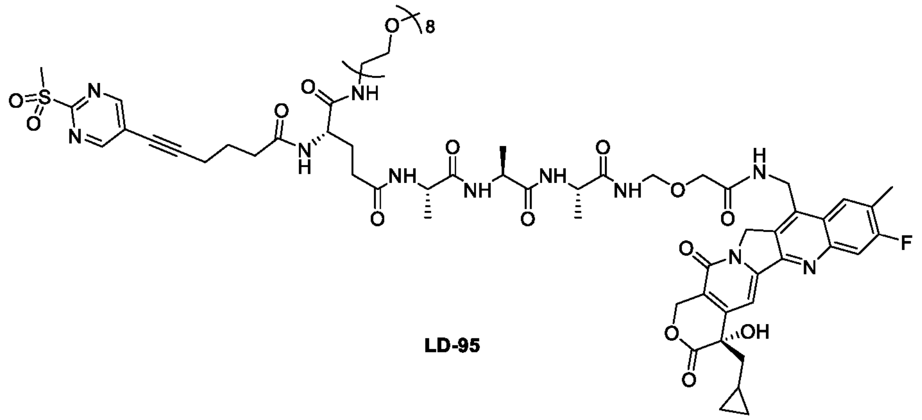

LD-95

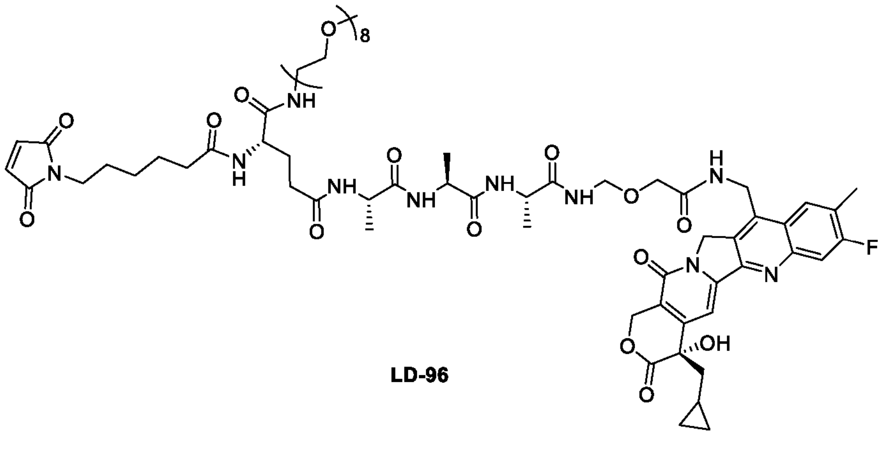

LD-96

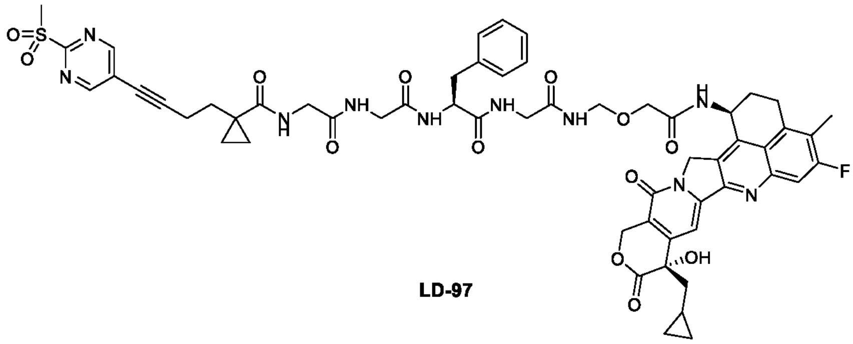

LD-97

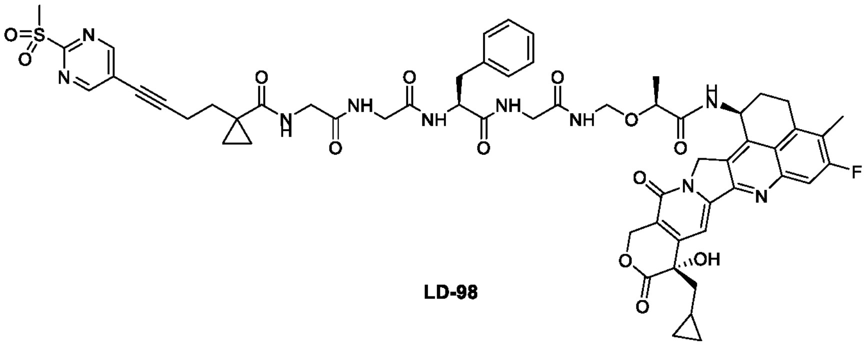

LD-98

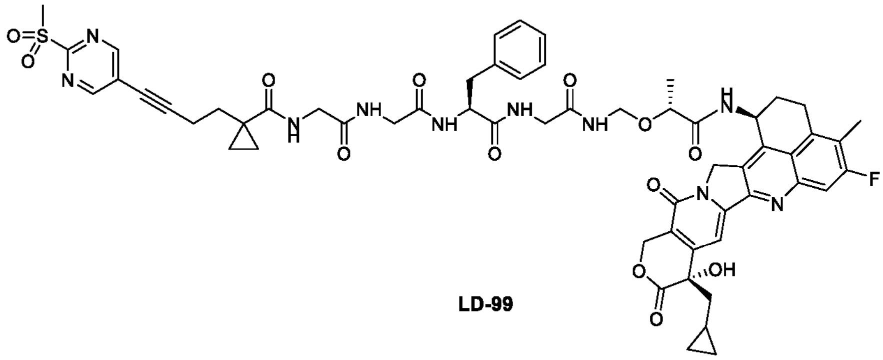

LD-99

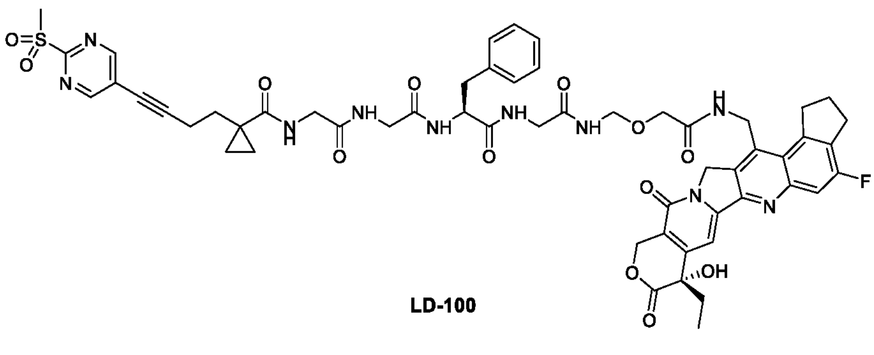

LD-100

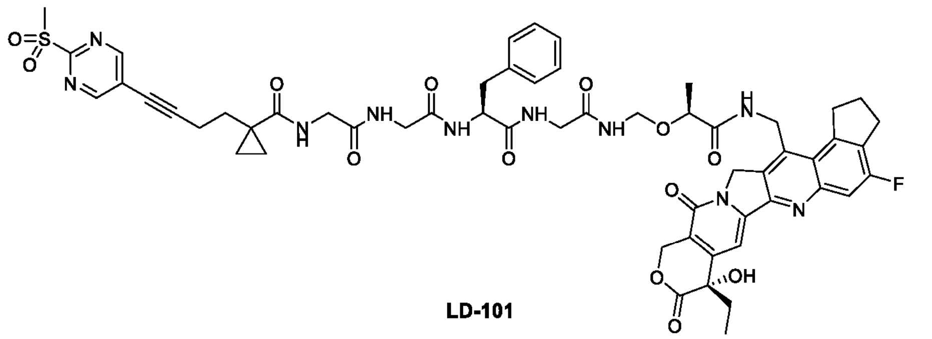

LD-101

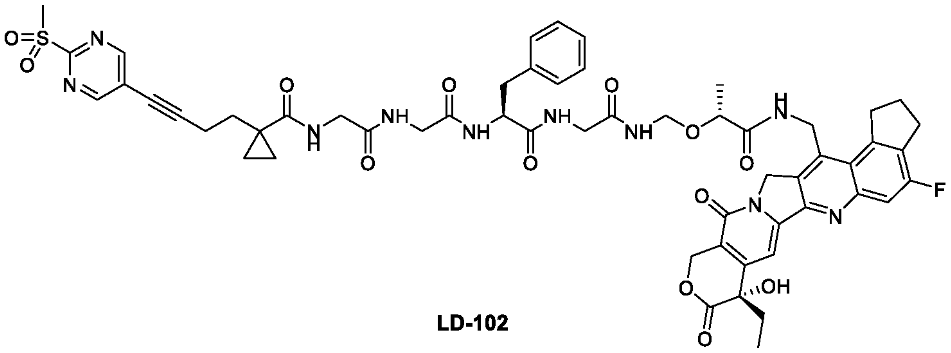

LD-102

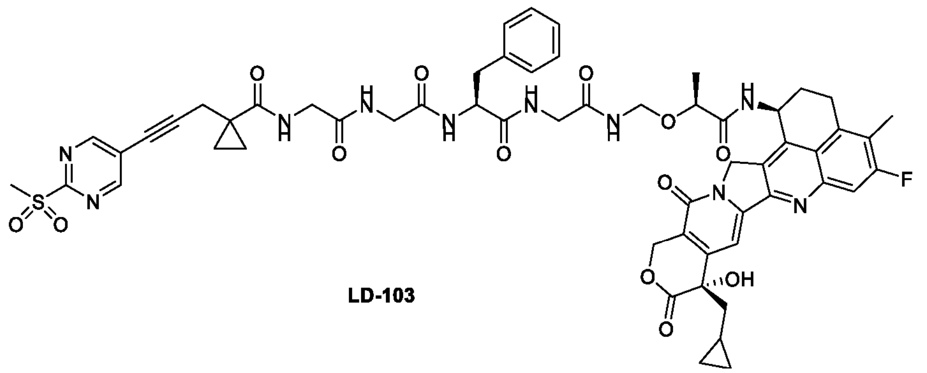

LD-103

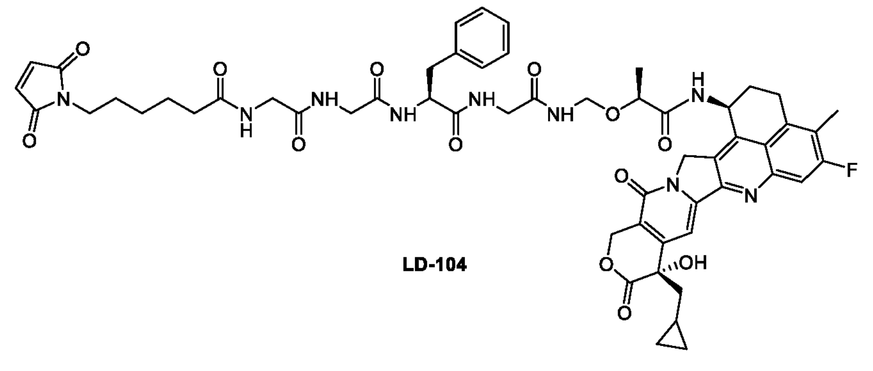

LD-104

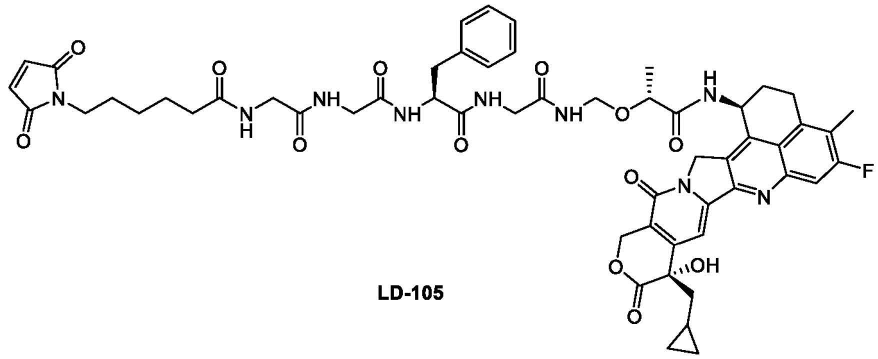

LD-105

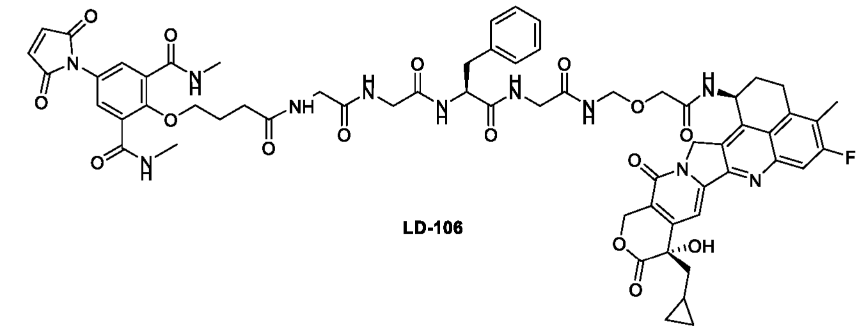

LD-106

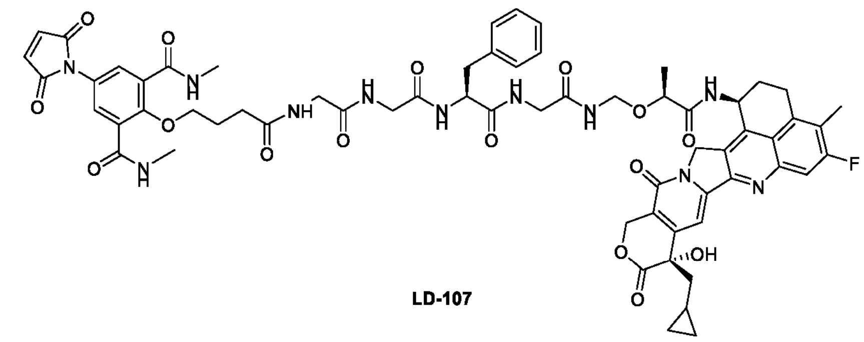

LD-107

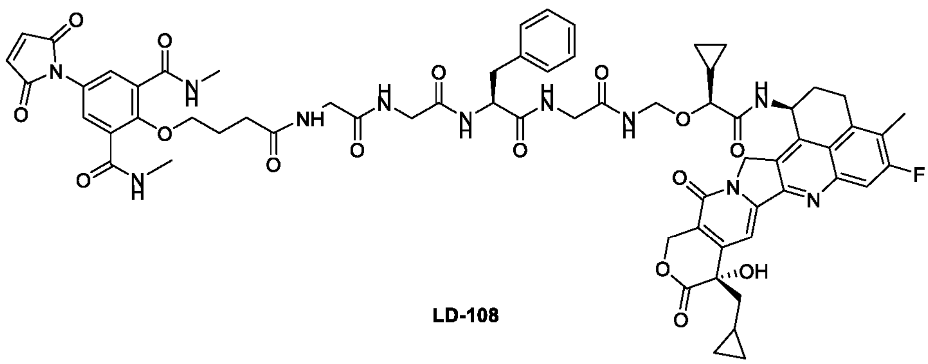

LD-108

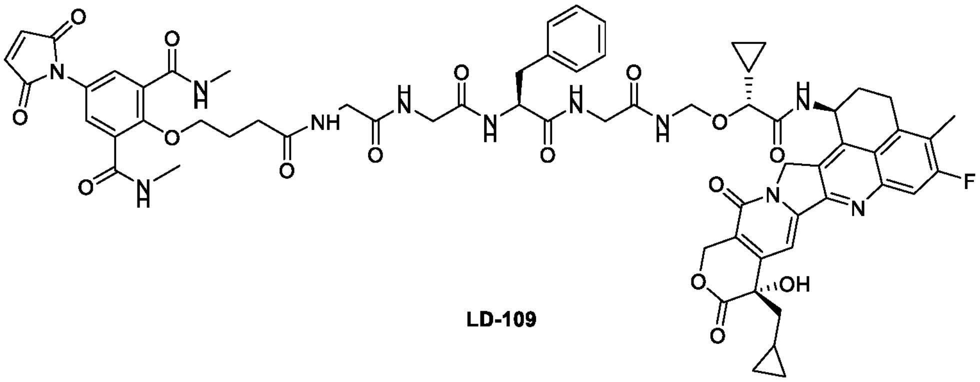

LD-109

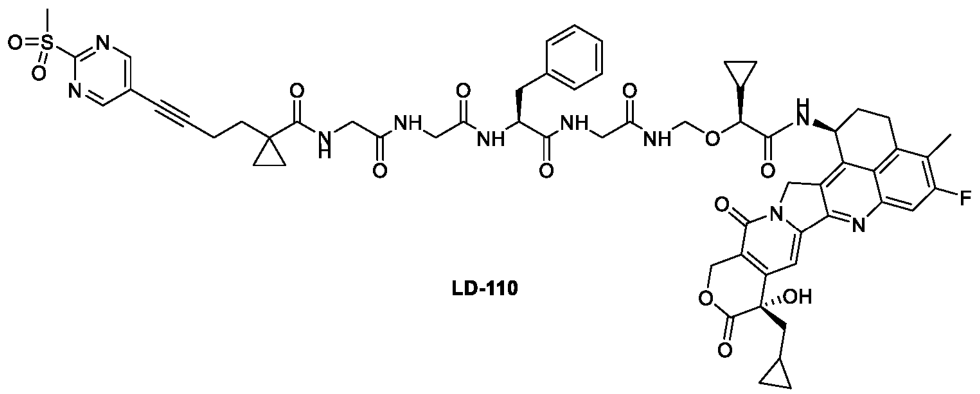

LD-110

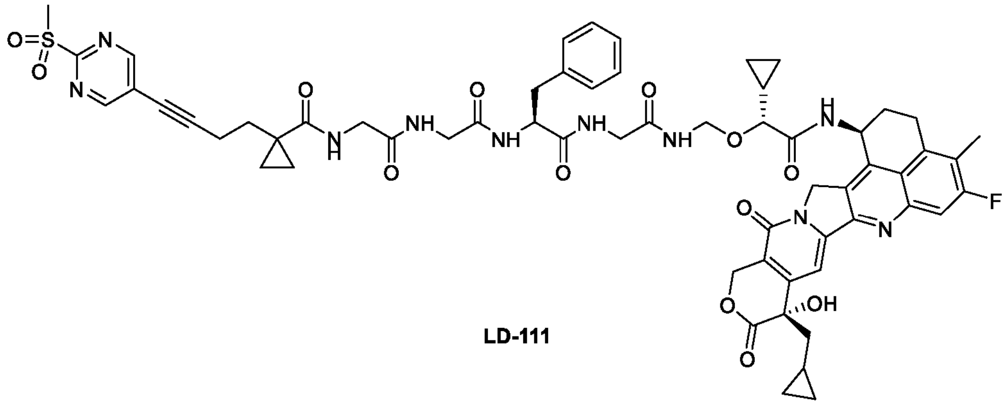

LD-111

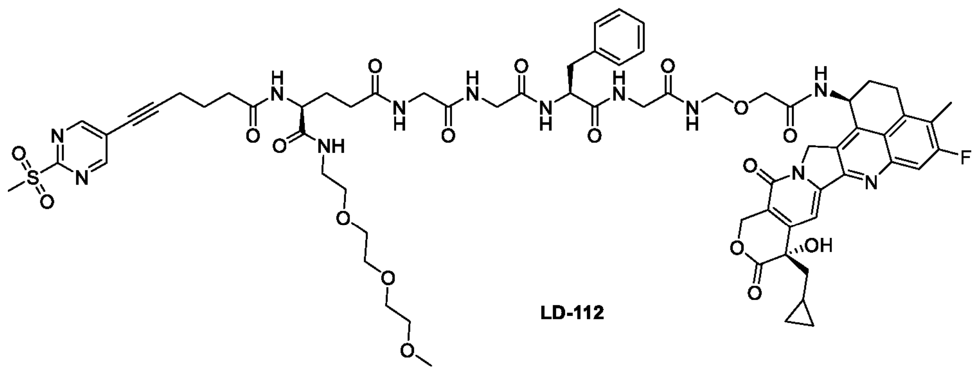

LD-112

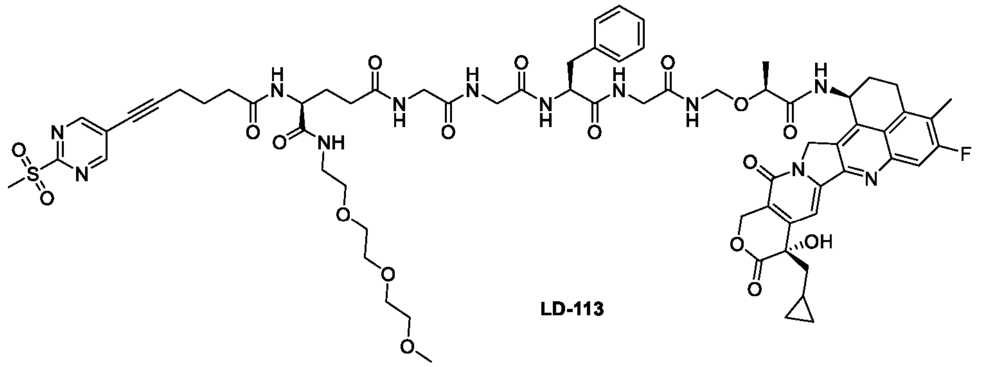

**LD-113**

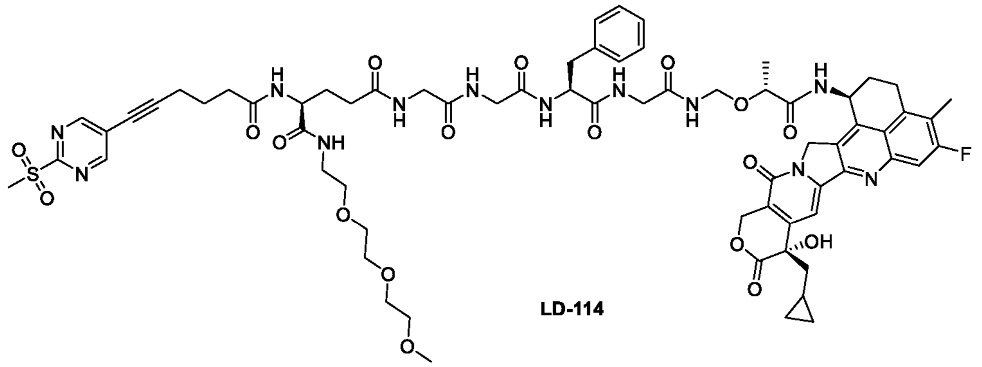

**LD-114**

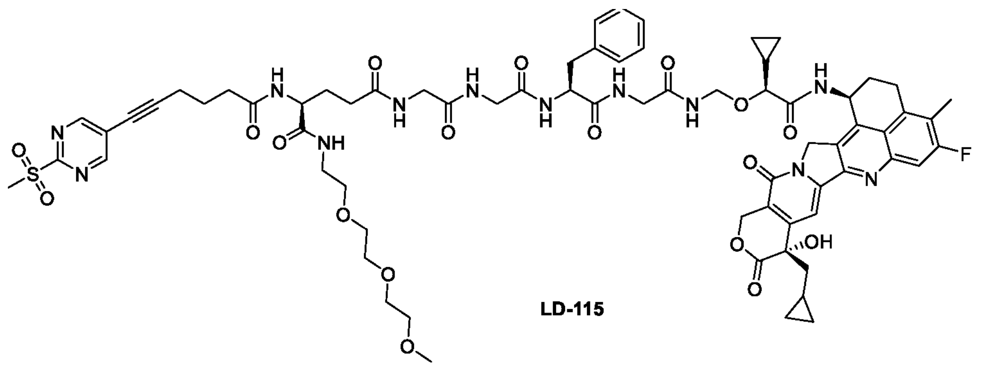

**LD-115**

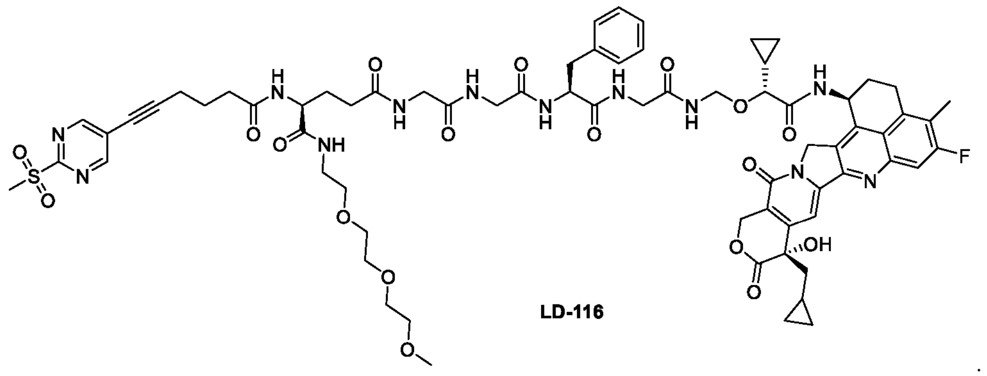

**LD-116**

**50.** A conjugate of a linker and a drug, which has the following structure:

$L^{1'}$-$L^2$-$L^3$-$L^4$-$L^5$-D

wherein, $L^{1'}$, $L^2$, $L^3$, $L^4$, and $L^5$ have the definitions as described in any one of claims 14 to 22, and D has the definition as described in any one of claims 43 to 44,
provided that:

Condition I:
the following hydrophilic moiety is inserted between the fragments as defined above, preferably between $L^{1'}$ and $L^2$, or between $L^2$ and $L^3$, or is inserted by replacing $L^2$:
hydrophilic moiety:

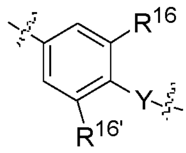 or 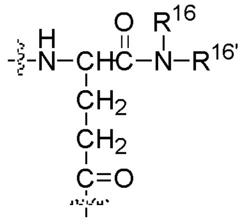 or 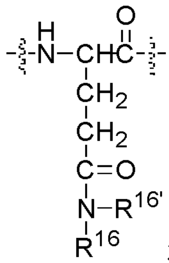 ;

or Condition II:

$L^{1'}$ is a sulfhydryl-reactive group contained in maleimide group or a substituted maleimide group;
the following steric hindrance moiety is inserted between $L^{1'}$ and $L^2$, or between $L^2$ and $L^3$, or is inserted by replacing $L^2$:
Steric hindrance moiety:

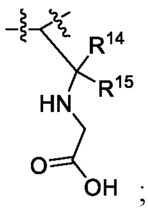 ;

or Condition III:

$L^{1'}$ is a sulfhydryl-reactive group contained in Hal-Het-;
$L^4$-$L^5$ is: $-NR^{z1}-C(R^{z2}R^{z3})-T^L-$;
wherein:

$R^{z1}$ is H or $C_{1-4}$ alkyl;
$R^{z2}$ and $R^{z3}$ are the same or different, and are independently selected from H or $C_{1-4}$ alkyl;
$T^L$ has the definition as described above;

or Condition IV:

$L^{1'}$-$L^2$ has the following structure:

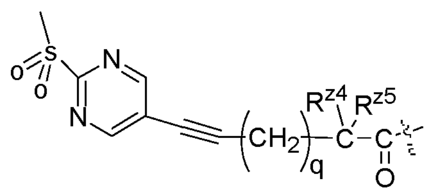

q is an integer of 0 to 10, preferably 1, 2, 3, 4, 5, 6, 7 or 8;
$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl, wherein at

least one of $R^{z4}$ and $R^{z5}$ is not H;
further preferably, $R^{z4}$ and $R^{z5}$ together form a $C_{3-6}$ cycloalkyl;
$L^{1'}$-$L^2$ is most preferably selected from:

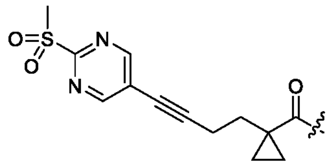

.

**51.** The conjugate of linker and drug according to claim 50, wherein:

$L^{1'}$ is a sulfhydryl-reactive group contained in maleimide group, a substituted maleimide group or Hal-Het-;
the following hydrophilic moiety is inserted between $L^1$ and $L^2$, or between $L^2$ and $L^3$, or is inserted by replacing $L^2$:
hydrophilic moiety:

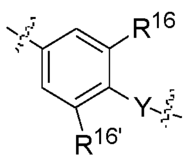
or
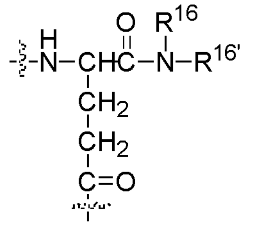
or
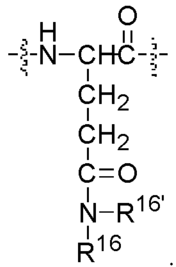
.

**52.** The conjugate of a linker and a drug according to claim 50, wherein:

$L^{1'}$ is a sulfhydryl-reactive group contained in maleimide group or a substituted maleimide group;
the following steric hindrance moiety is inserted between $L^1$ and $L^2$, or between $L^2$ and $L^3$, or is inserted by replacing $L^2$:
steric hindrance moiety:

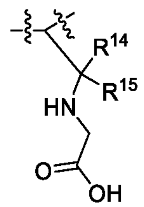
.

**53.** The conjugate of a linker and a drug according to claim 50, wherein:

$L^{1'}$ is a sulfhydryl-reactive group contained in Hal-Het-;
$L^4$-$L^5$ is: $-NR^{z1}-C(R^{z2}R^{z3})-O-(CH_2)_m-C(R^{z4}R^{z5})-CO-$;
wherein:

$R^{z1}$ is H or $C_{1-4}$ alkyl;
m is an integer of 0 to 4;
$R^{z2}$ and $R^{z3}$ are the same or different and are independently selected from H or $C_{1-4}$ alkyl;
$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl.

**54.** The conjugate of a linker and a drug according to claim 50, wherein:

$L^{1'}$-$L^2$ has the following structure:

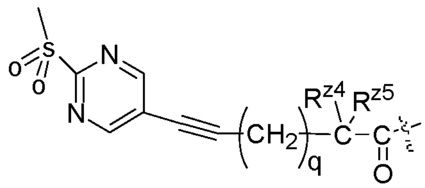

q is an integer of 0 to 10, preferably 1, 2, 3, 4, 5, 6, 7 or 8;

$R^{z4}$ and $R^{z5}$ are the same or different and are independently selected from the group consisting of H, $C_{1-4}$ alkyl, $C_{3-6}$ cycloalkyl, $C_{3-6}$ cycloalkyl-$C_{1-4}$ alkyl, or $R^{z4}$ and $R^{z5}$ together form a $C_{3-6}$ cycloalkyl, wherein at least one of $R^{z4}$ and $R^{z5}$ is not H;

further preferably, $R^{z4}$ and $R^{z5}$ together form a $C_{3-6}$ cycloalkyl;

$L^{1'}$-$L^2$ is most preferably selected from:

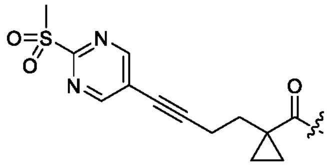

.

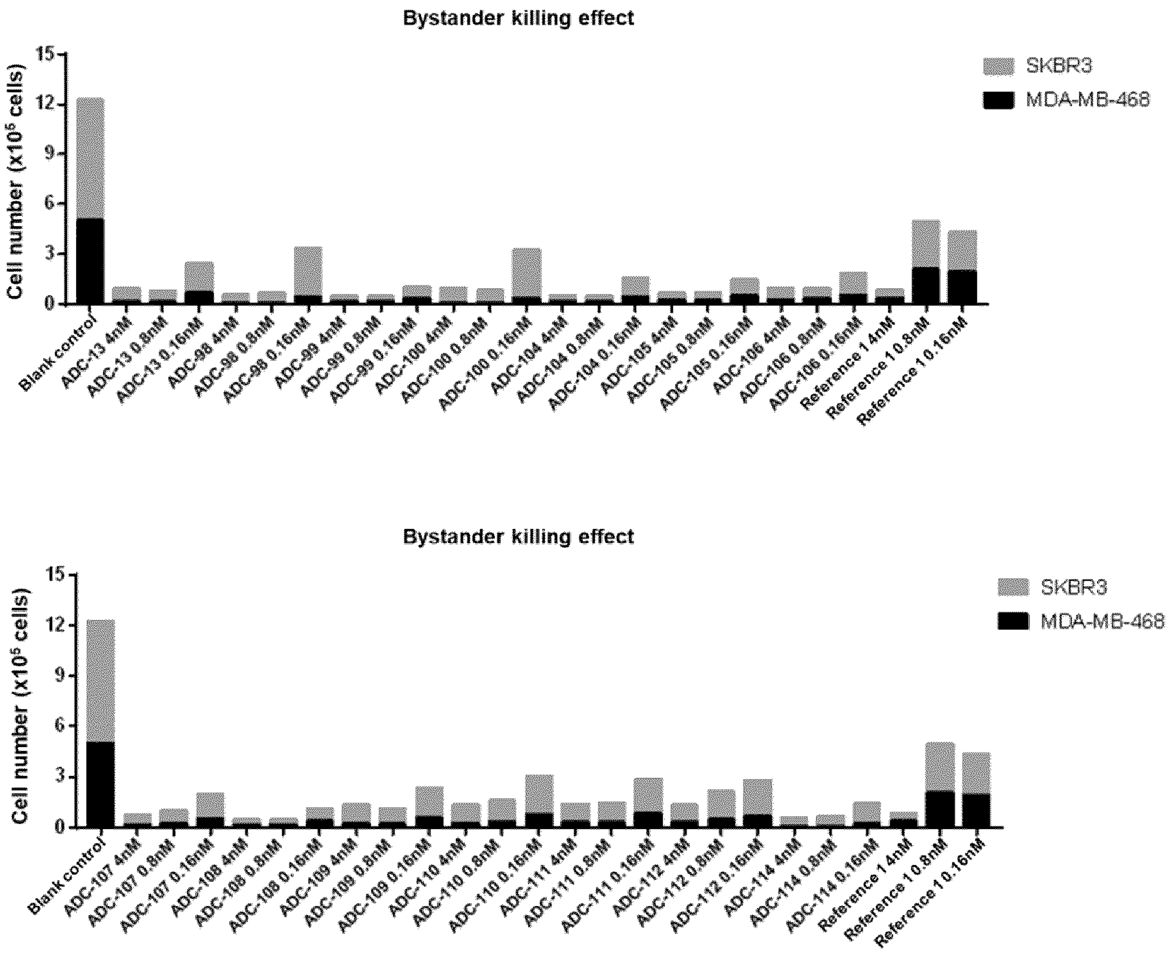
Bystander killing effect
Cell number (x$10^5$ cells)
15
12
9
6
3
0
SKBR3
MDA-MB-468
Blank control
ADC-13 4nM
ADC-13 0.8nM
ADC-13 0.16nM
ADC-98 4nM
ADC-98 0.8nM
ADC-98 0.16nM
ADC-99 4nM
ADC-99 0.8nM
ADC-99 0.16nM
ADC-100 4nM
ADC-100 0.8nM
ADC-100 0.16nM
ADC-104 4nM
ADC-104 0.8nM
ADC-104 0.16nM
ADC-105 4nM
ADC-105 0.8nM
ADC-105 0.16nM
ADC-106 4nM
ADC-106 0.8nM
ADC-106 0.16nM
Reference 1 4nM
Reference 1 0.8nM
Reference 1 0.16nM
Bystander killing effect
Cell number (x$10^5$ cells)
15
12
9
6
3
0
SKBR3
MDA-MB-468
Blank control
ADC-107 4nM
ADC-107 0.8nM
ADC-107 0.16nM
ADC-108 4nM
ADC-108 0.8nM
ADC-108 0.16nM
ADC-109 4nM
ADC-109 0.8nM
ADC-109 0.16nM
ADC-110 4nM
ADC-110 0.8nM
ADC-110 0.16nM
ADC-111 4nM
ADC-111 0.8nM
ADC-111 0.16nM
ADC-112 4nM
ADC-112 0.8nM
ADC-112 0.16nM
ADC-114 4nM
ADC-114 0.8nM
ADC-114 0.16nM
Reference 1 4nM
Reference 1 0.8nM
Reference 1 0.16nM

Figure 1

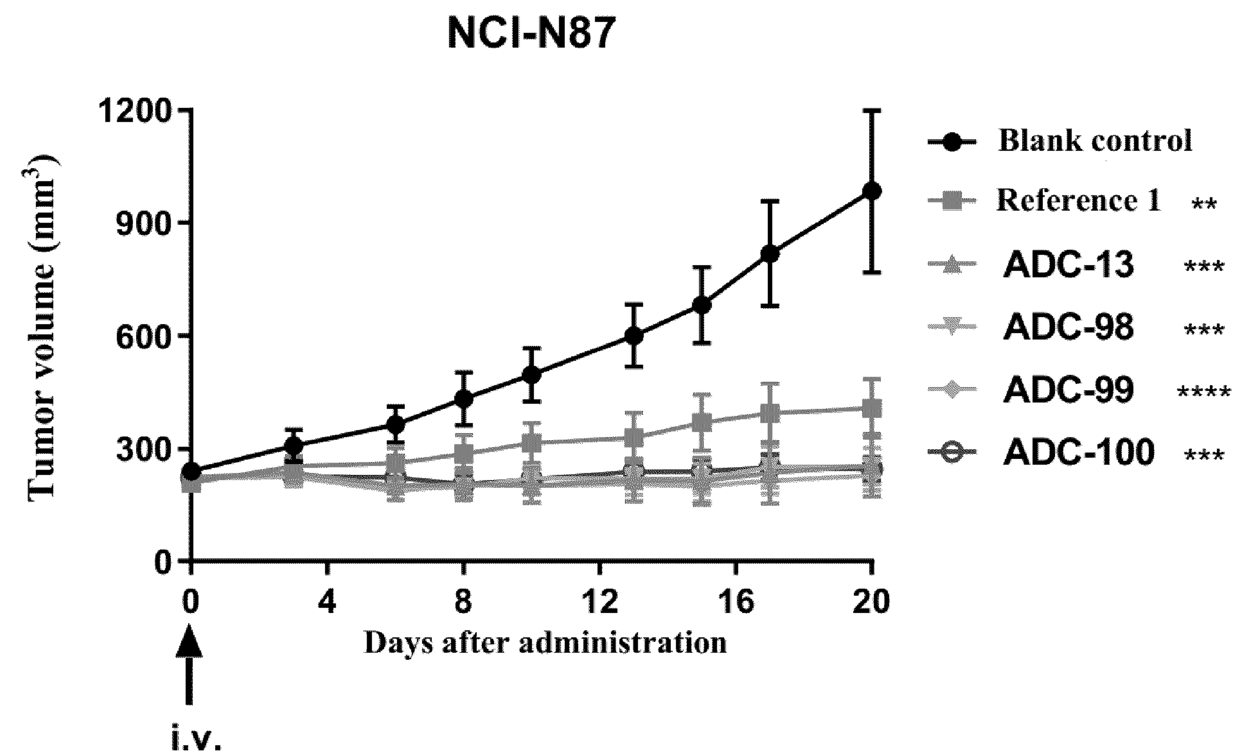


Figure 2

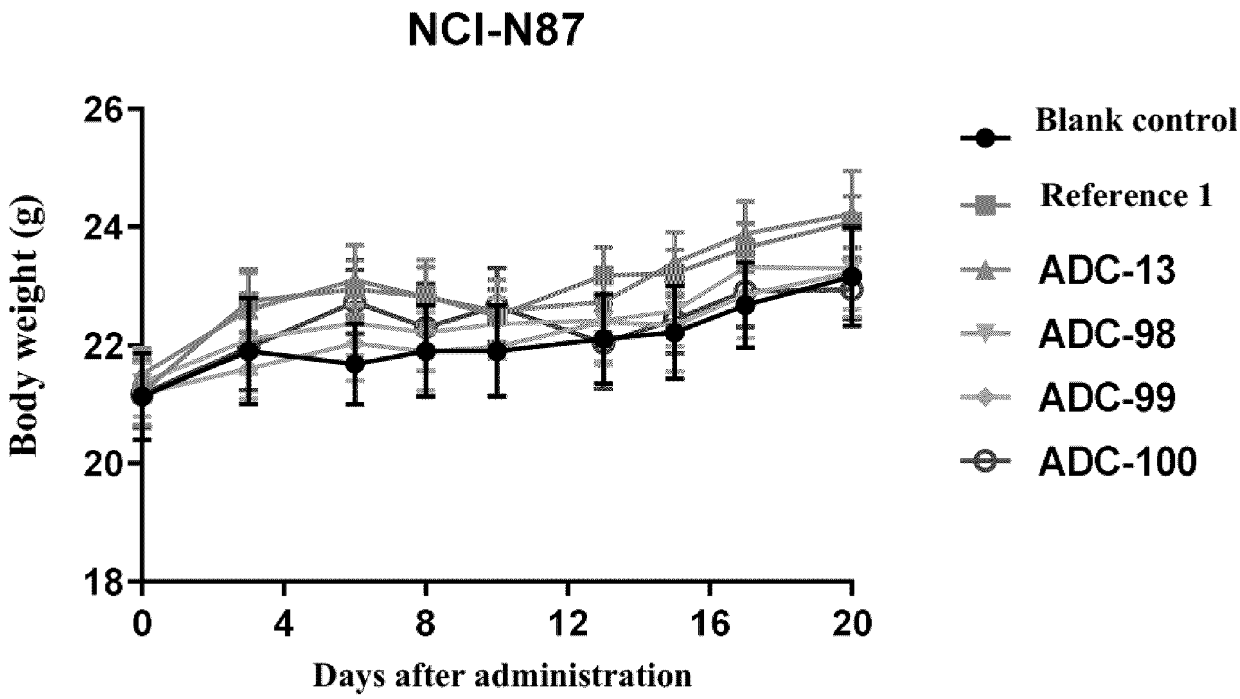


Figure 3

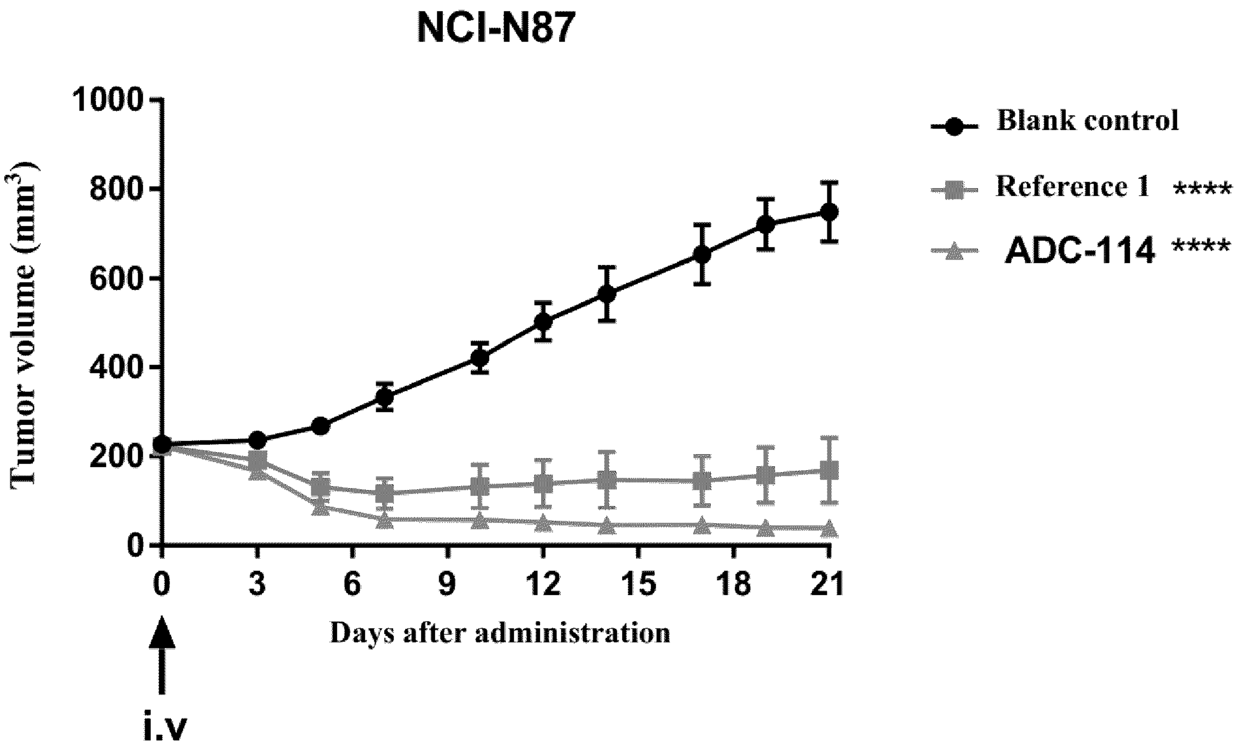


Figure 4

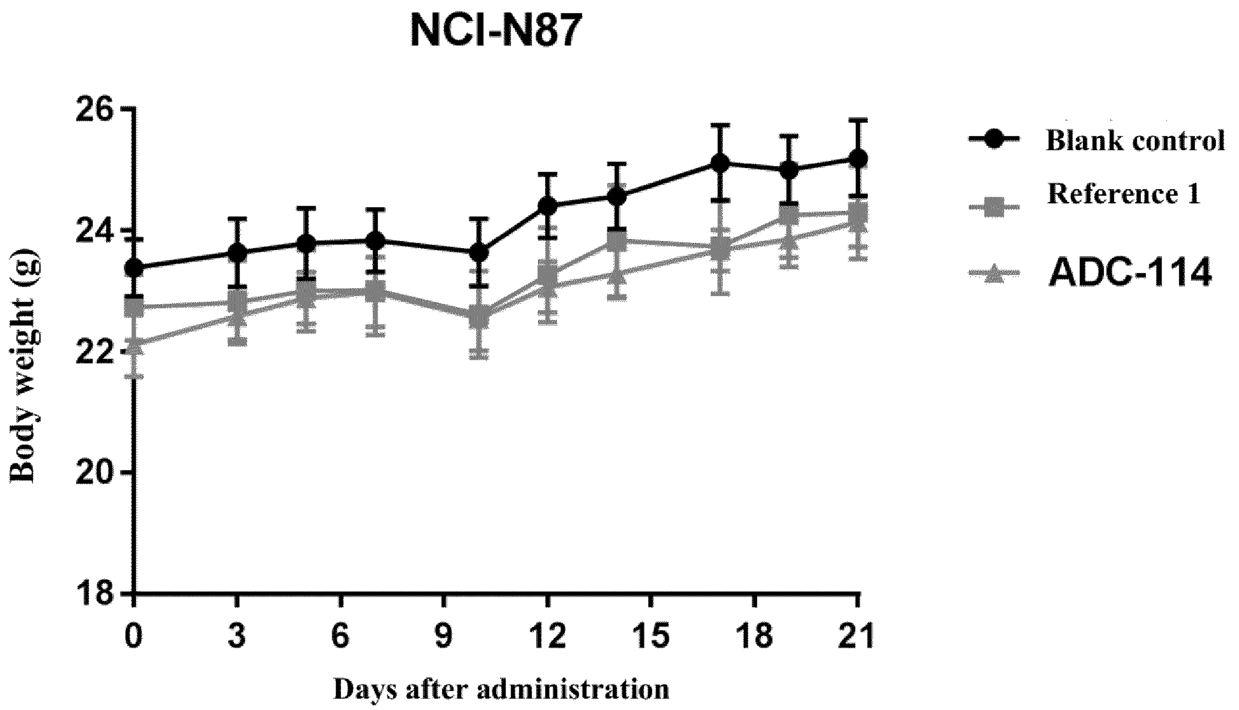


Figure 5

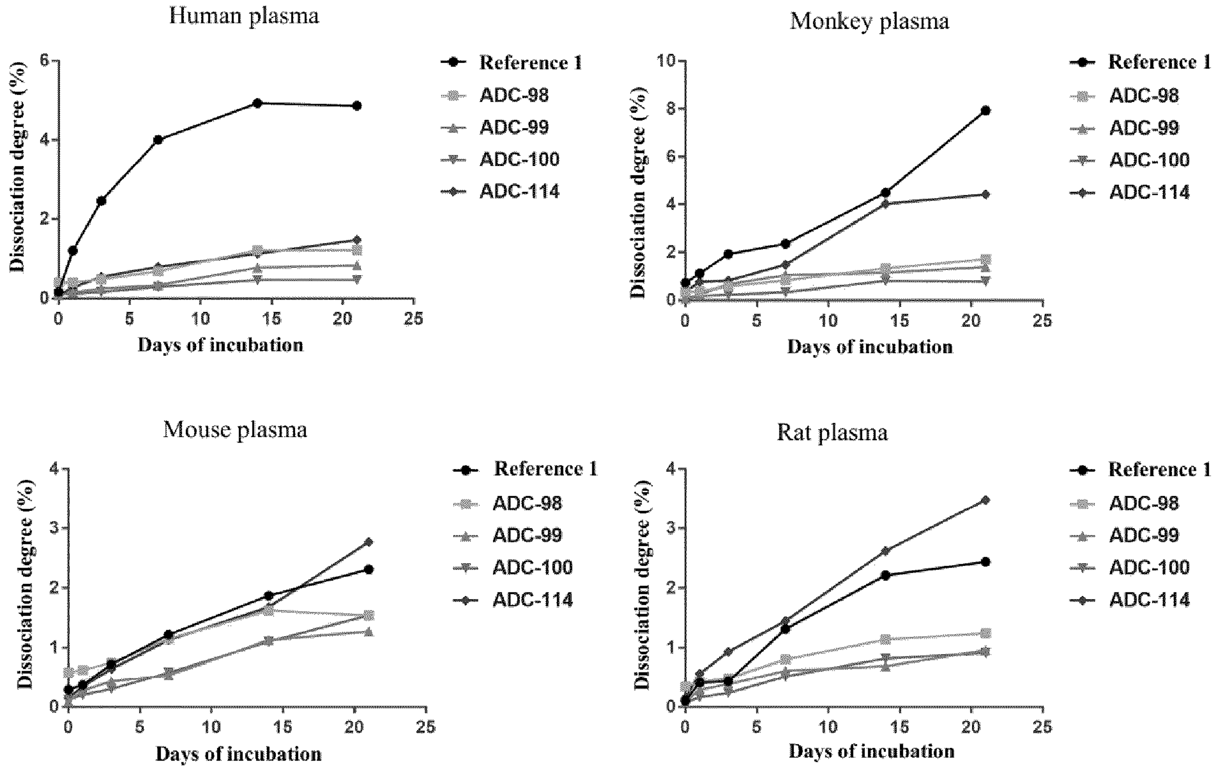


Figure 6

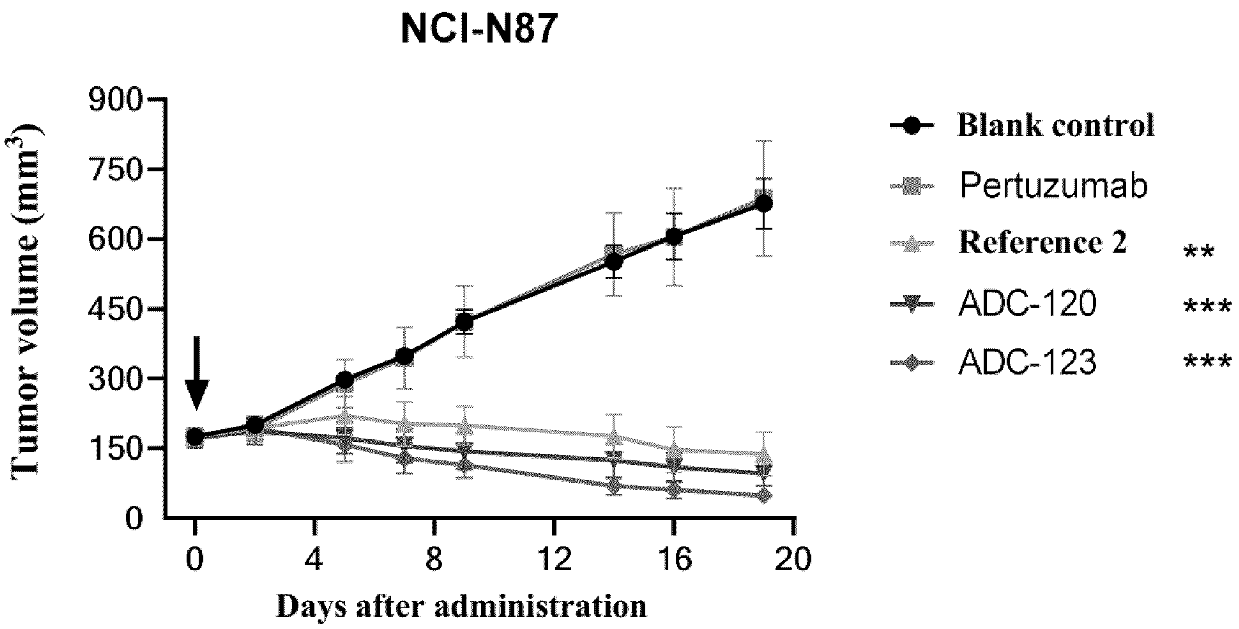


Figure 7

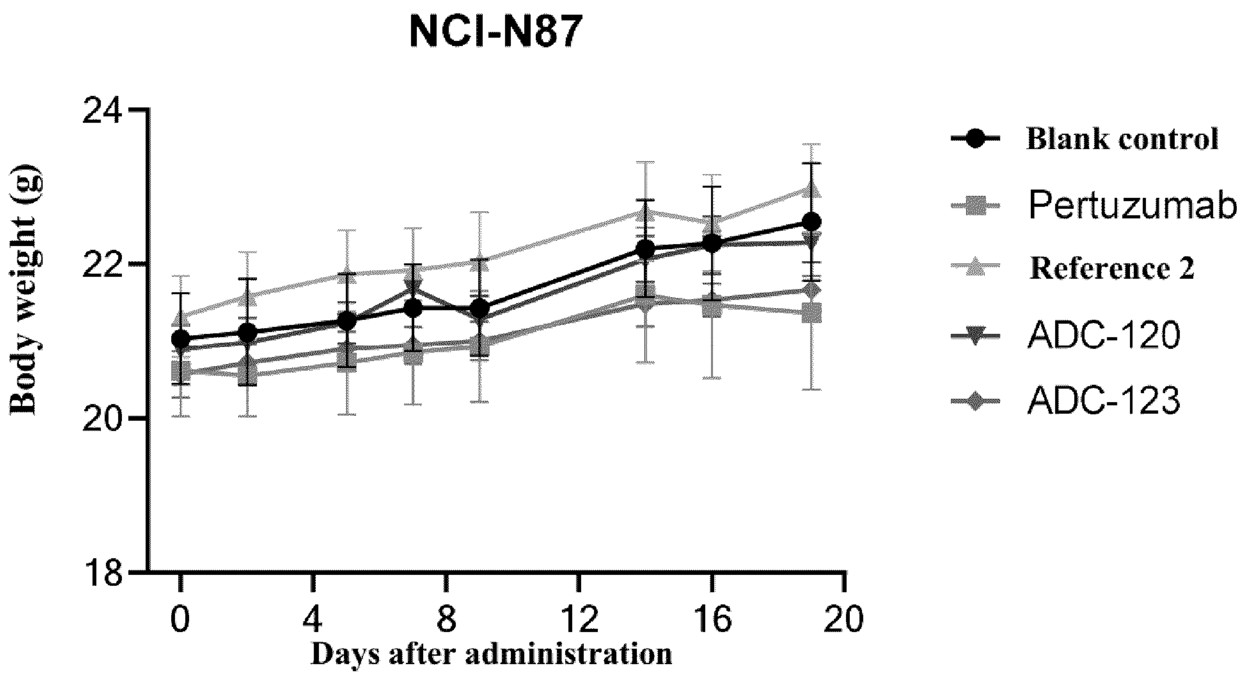


Figure 8

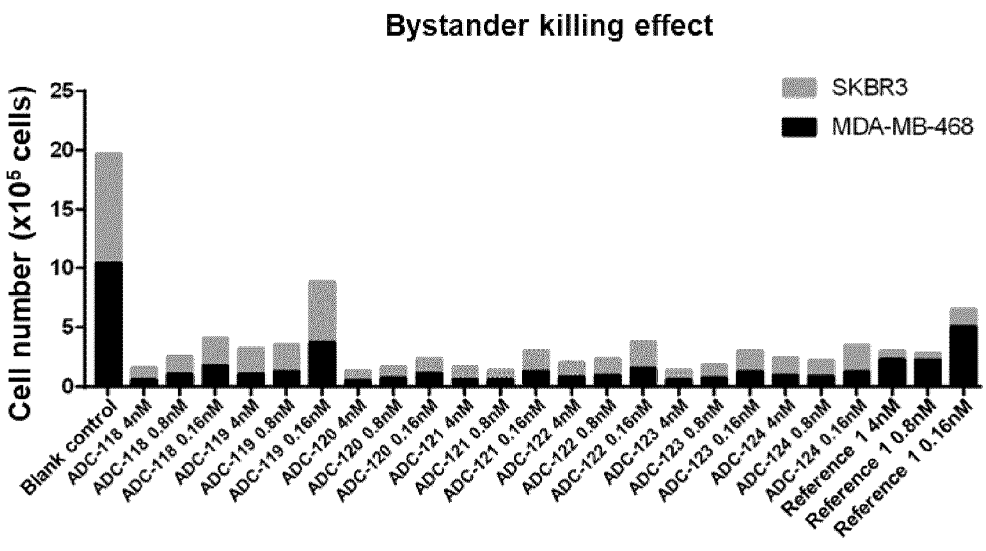


Figure 9

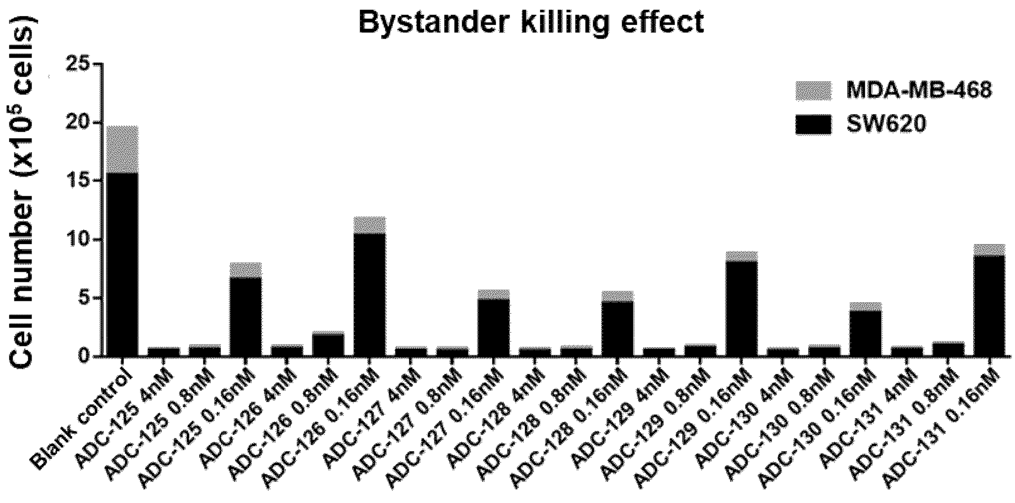


Figure 10

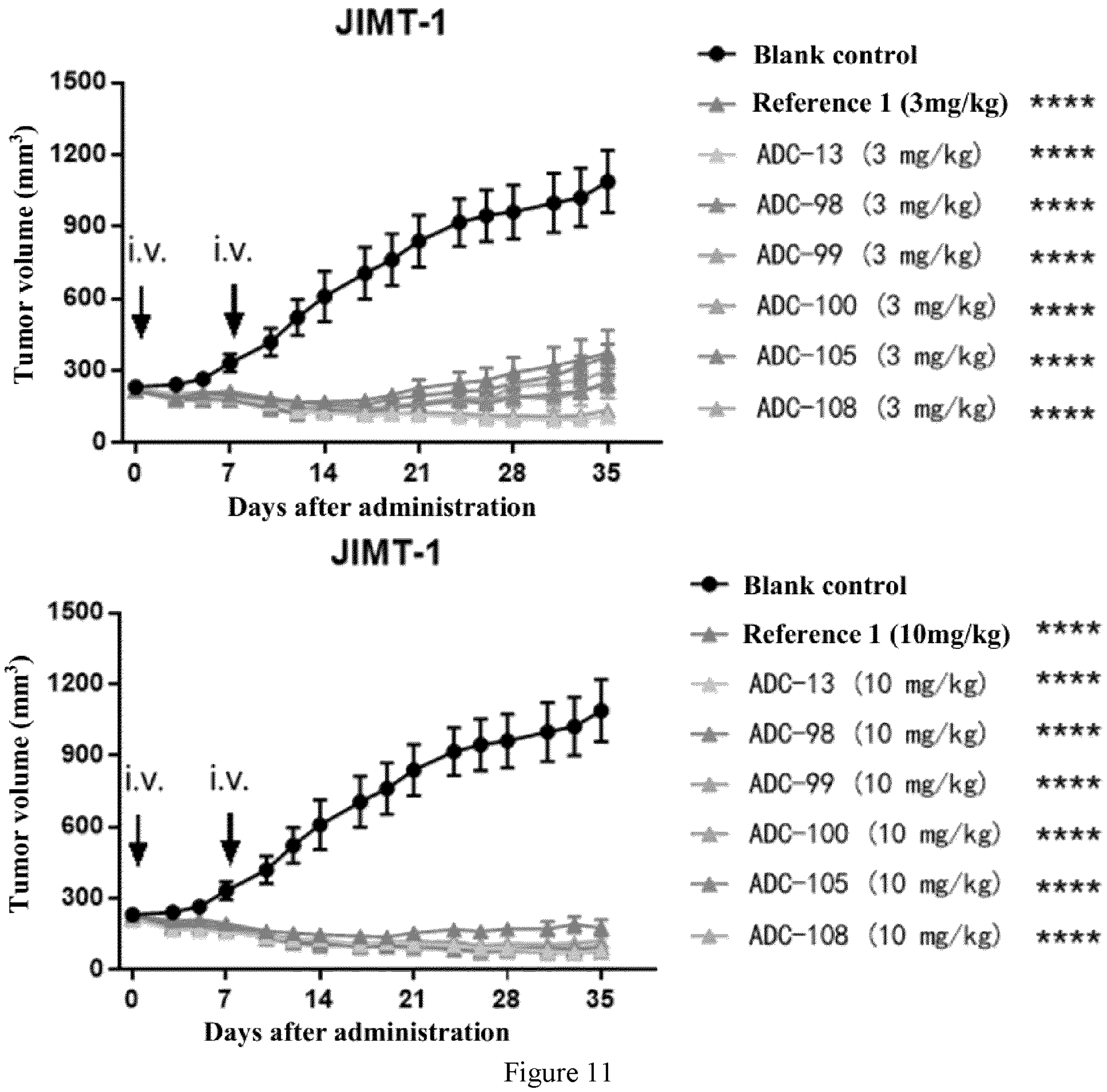


Figure 11

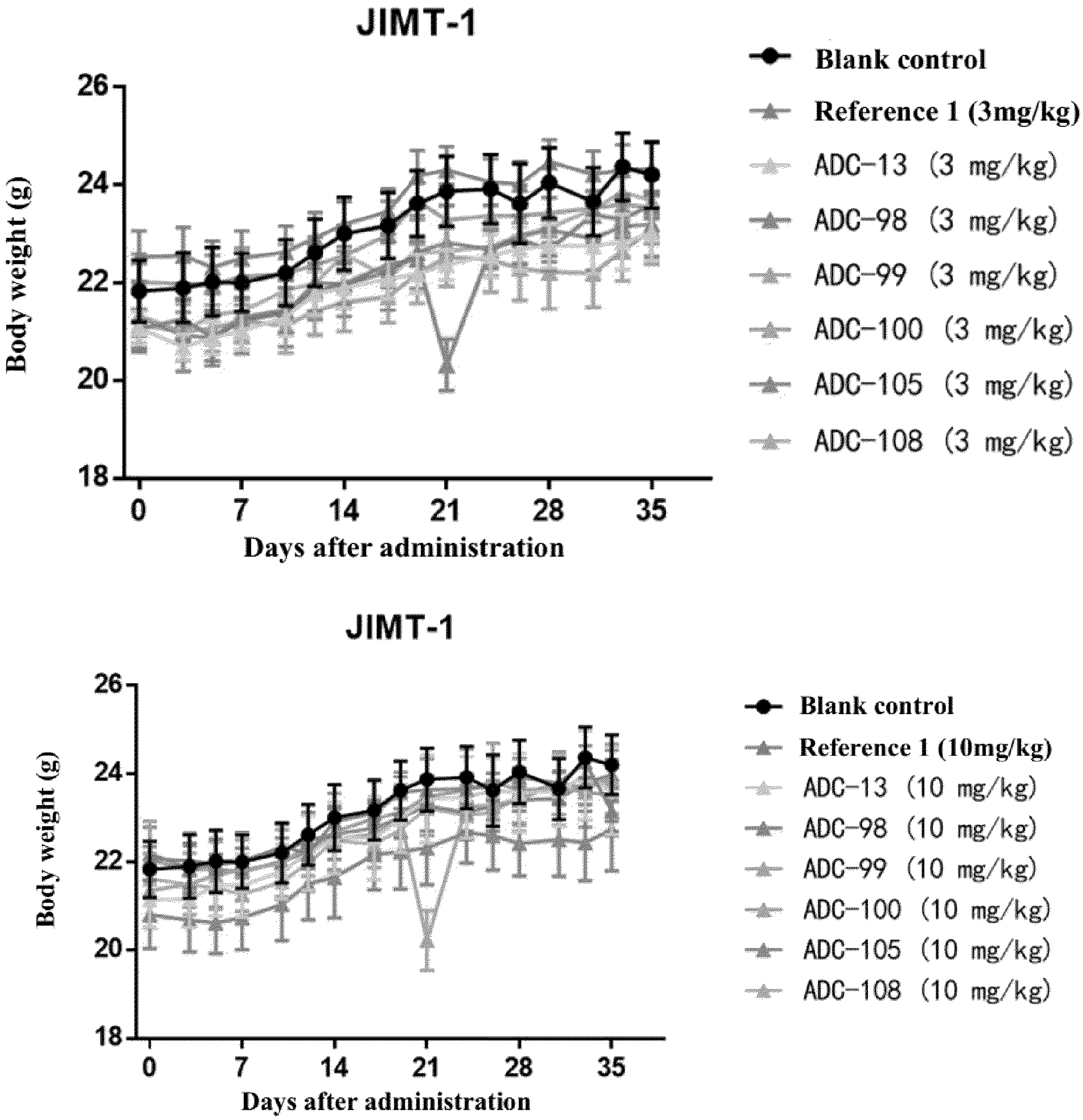
JIMT-1
Body weight (g)
26
24
22
20
18
0
7
14
21
28
35
Days after administration
Blank control
Reference 1 (3mg/kg)
ADC-13 (3 mg/kg)
ADC-98 (3 mg/kg)
ADC-99 (3 mg/kg)
ADC-100 (3 mg/kg)
ADC-105 (3 mg/kg)
ADC-108 (3 mg/kg)
JIMT-1
Body weight (g)
26
24
22
20
18
0
7
14
21
28
35
Days after administration
Blank control
Reference 1 (10mg/kg)
ADC-13 (10 mg/kg)
ADC-98 (10 mg/kg)
ADC-99 (10 mg/kg)
ADC-100 (10 mg/kg)
ADC-105 (10 mg/kg)
ADC-108 (10 mg/kg)

Figure 12

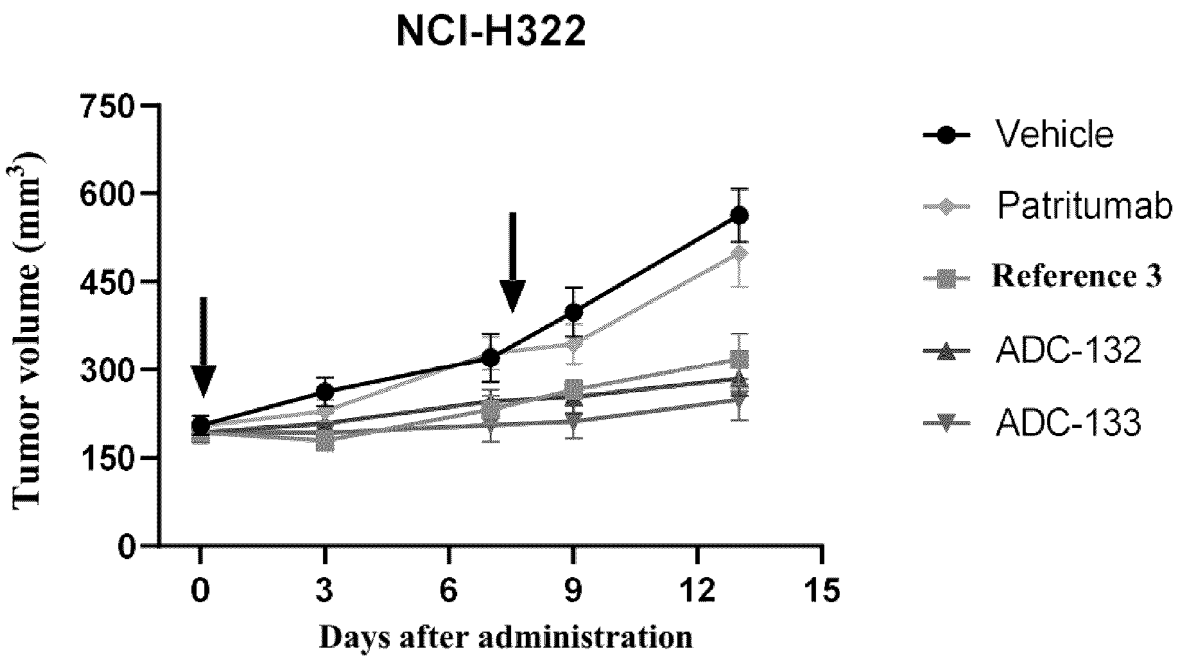


Figure 13

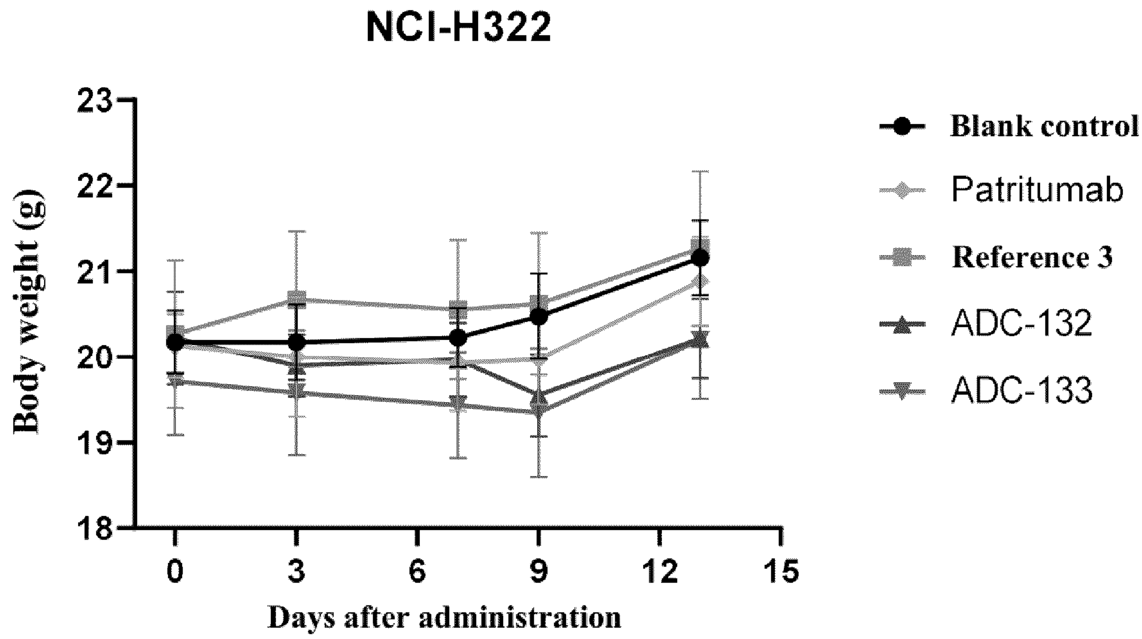


Figure 14

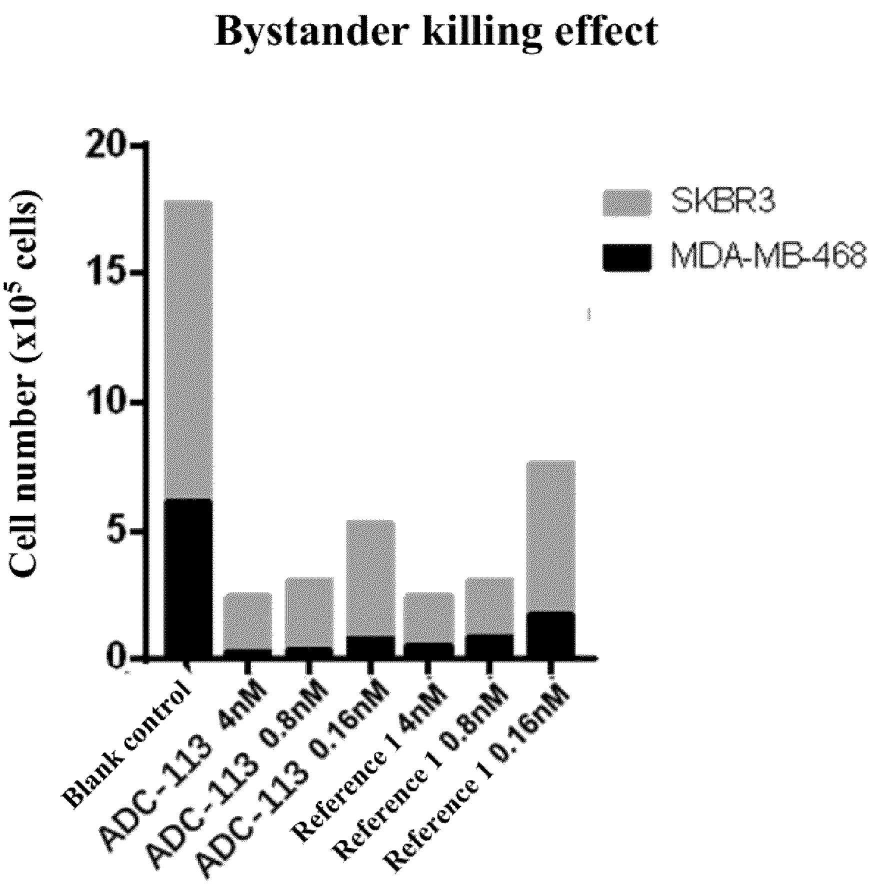


Figure 15

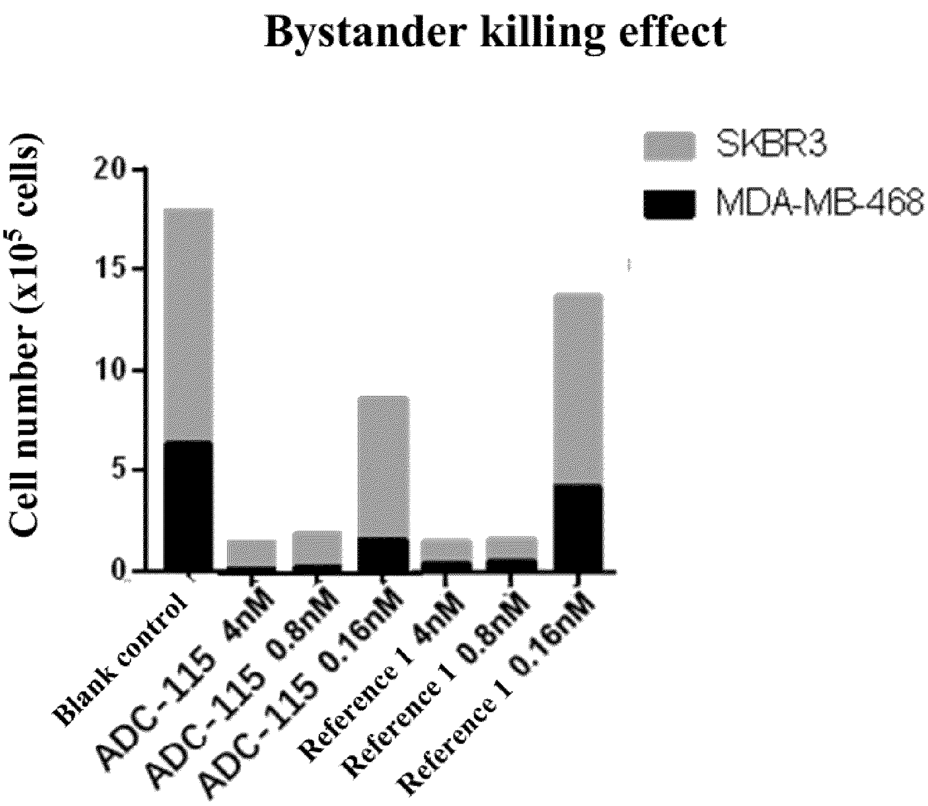


Figure 16

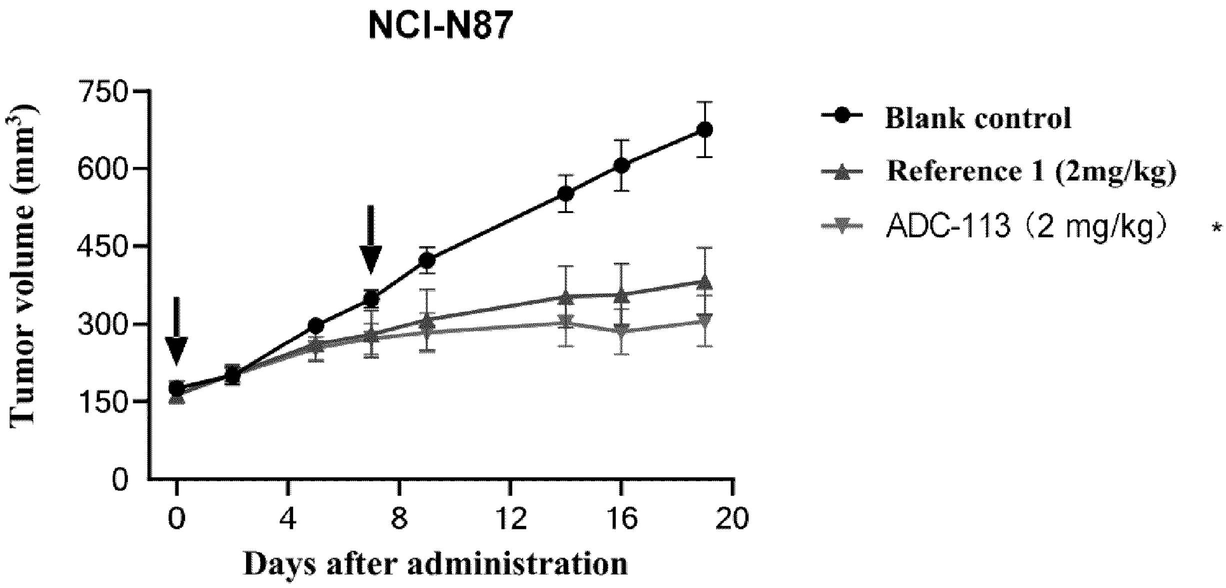
NCI-N87
Tumor volume (mm³)
750
600
450
300
150
0
0
4
8
12
16
20
Days after administration
Blank control
Reference 1 (2mg/kg)
ADC-113 (2 mg/kg) *

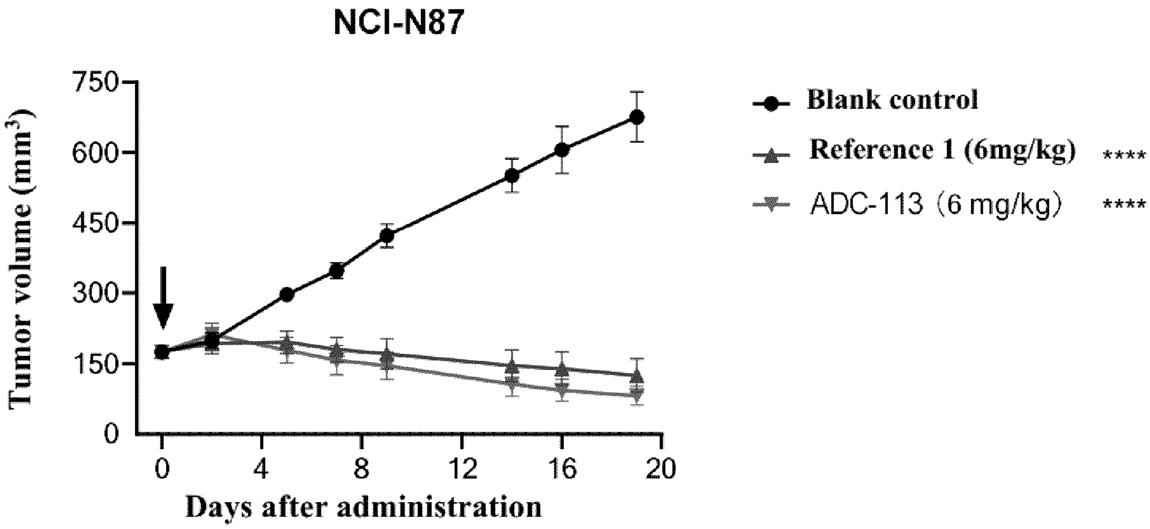
NCI-N87
Tumor volume (mm³)
750
600
450
300
150
0
0
4
8
12
16
20
Days after administration
Blank control
Reference 1 (6mg/kg) ****
ADC-113 (6 mg/kg) ****

Figure 17

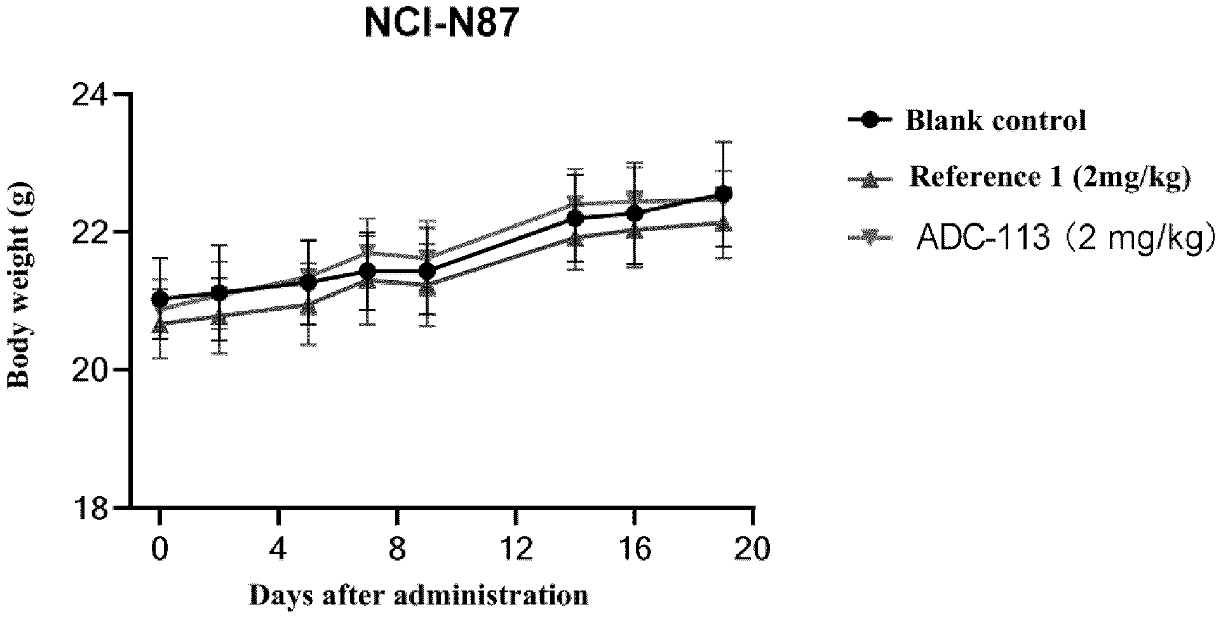
NCI-N87
Body weight (g)
24
22
20
18
0
4
8
12
16
20
Days after administration
Blank control
Reference 1 (2mg/kg)
ADC-113 (2 mg/kg)

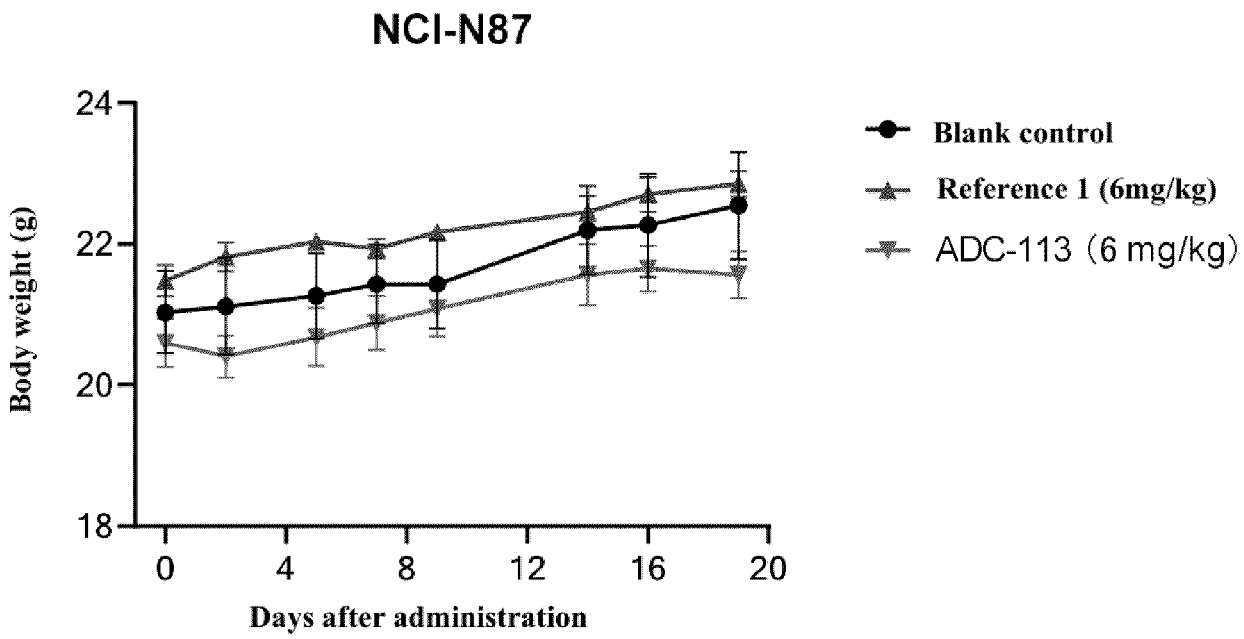


Figure 18

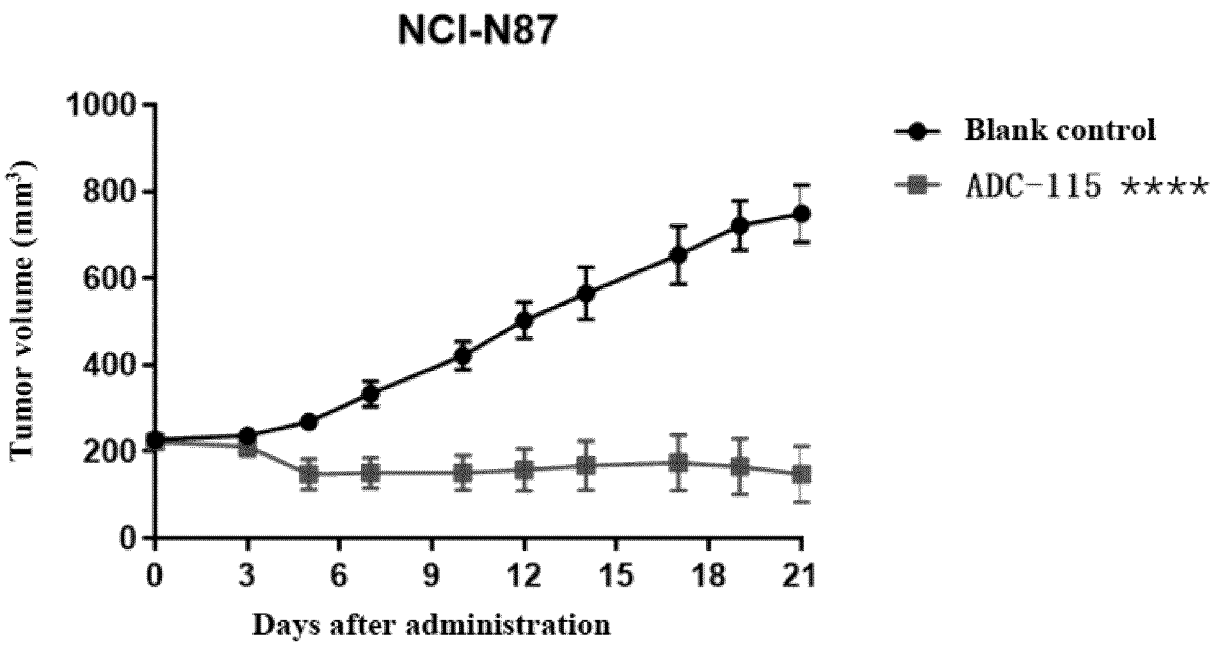


Figure 19

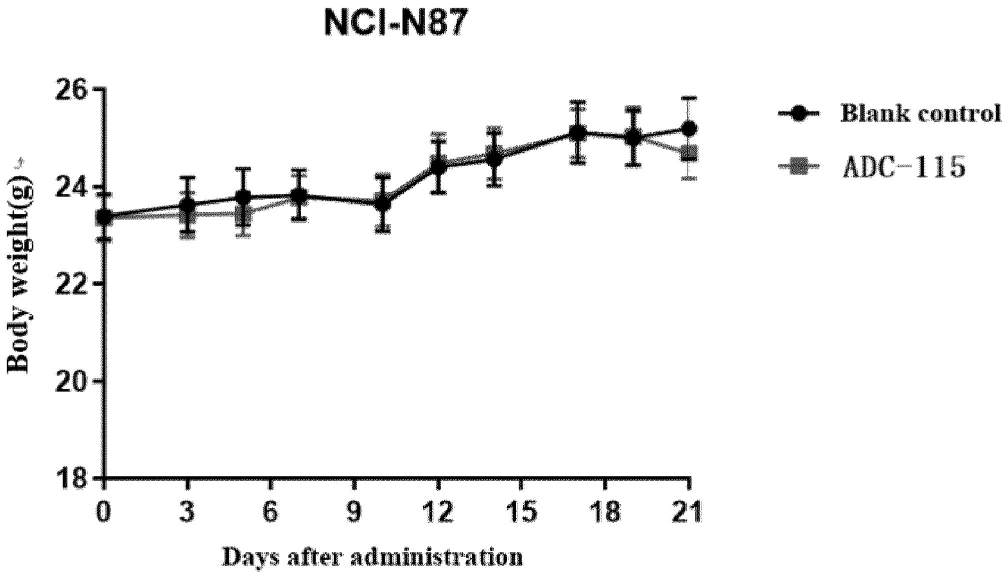

NCI-N87
Body weight(g)
26
24
22
20
18
0
3
6
9
12
15
18
21
Days after administration
Blank control
ADC-115

Figure 20

## INTERNATIONAL SEARCH REPORT

International application No.
**PCT/CN2023/133434**

### A. CLASSIFICATION OF SUBJECT MATTER

A61K47/68(2017.01)i； A61K47/65(2017.01)i； A61K39/395(2006.01)i； C07K16/28(2006.01)i； C07K16/30(2006.01)i； C07D491/22(2006.01)i； C07D491/147(2006.01)i； A61K31/47(2006.01)i； A61K31/4745(2006.01)i； A61K31/43 7(2006.01)i； A61K31/357(2006.01)i； A61K31/40(2006.01)i； A61K31/165(2006.01)i； A61K31/4375(2006.01)i； A61P35/ 00(2006.01)i； A61P35/02(2006.01)i

According to International Patent Classification (IPC) or to both national classification and IPC

### B. FIELDS SEARCHED

Minimum documentation searched (classification system followed by classification symbols)

A61K47/-； A61K39/-； C07K16/-； C07D491/-； A61K31/-； A61P35/-

Documentation searched other than minimum documentation to the extent that such documents are included in the fields searched

Electronic data base consulted during the international search (name of data base and, where practicable, search terms used)

CNTXT, VEN, DWPI, WPABS, WPABSC, ENTXT, ENTXTC, WOTXT, USTXT, EPTXT, JPTXT, CNKI, 百度, Baidu, 百度学术, Baidu Scholar, 万方, WANFANG, ISI-WEB OF SCIENCE, 必应, Bing, STN-REG, STN-CAPLUS: 康诺亚生物医药, 陈博, 喜树碱, 抗体药物偶联物, 结合物, camptothecin, antibody, ADC, conjugate, 式(G), formula (G), 权利要求7-13, 24-33, 35-38, 41-42, 49中结构式, structural formulae in claims 7-13, 24-33, 35-38, 41-42 and 49, 马来酰亚胺片段, 五元醇糖基单元, 权利要求15最后两个结构片段, the last two structural fragments of claim 15

### C. DOCUMENTS CONSIDERED TO BE RELEVANT

| Category* | Citation of document, with indication, where appropriate, of the relevant passages | Relevant to claim No. |
|---|---|---|
| X | WO 2022166762 A1 (SICHUAN KELUN-BIOTECH BIOPHARMACEUTICAL CO., LTD.) 11 August 2022 (2022-08-11)<br>claims 7-14, and description, page 30, lines 29-35, page 34, reaction formulae, page 55, line 1, and page 124, line 5 to page 127, line 5 | 1-45, 46-47 (in part), 48-54 |
| X | WO 2022078260 A1 (SICHUAN BAILI PHARM CO., LTD. et al.) 21 April 2022 (2022-04-21)<br>description, page 21, reaction formula 2, and page 29, paragraph 1, and embodiments 1-7 and 10-18, and claims 7-28 | 1-45, 46-47 (in part), 48-54 |
| X | CN 113943310 A (SICHUAN BAILI PHARMACEUTICAL CO., LTD. et al.) 18 January 2022 (2022-01-18)<br>claims 6-27, and description, paragraph 183, and embodiments 1-13, 21-27 and 30-35 | 1-45, 46-47 (in part), 48-54 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **27 February 2024** | **13 March 2024** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No. **PCT/CN2023/133434**

**C. DOCUMENTS CONSIDERED TO BE RELEVANT**

| Category* | Citation of document, with indication, where appropriate, of the relevant passages | Relevant to claim No. |
|---|---|---|
| X | WO 2021249228 A1 (SICHUAN BAILI PHARM CO., LTD. et al.) 16 December 2021 (2021-12-16)<br>claims 1-20, and embodiments 4-31, 47-83 and 108 | 1-45, 46-47 (in part), 48-54 |
| X | WO 2020063676 A1 (JIANGSU HENGRUI MEDICINE CO., LTD. et al.) 02 April 2020 (2020-04-02)<br>description, pages 14 to 18, and tables, claims 1-46, and embodiments 1-16, 20-33 and 37-44 | 1-45, 46-47 (in part), 48-54 |
| X | WO 2022011075 A1 (VELOSBIO INC.) 13 January 2022 (2022-01-13)<br>description, page 10, second compound, page 11, first compound from the bottom, and paragraphs 71, 78-79, 103, 135 and 140, and claims 1-31 | 1-45, 46-47 (in part), 48-54 |
| X | LI, W. et al. "Synthesis and Evaluation of Camptothecin Antibody-Drug Conjugates" *ACS Medicinal Chemistry Letters,* Vol. vol. 10, 06 September 2019 (2019-09-06), pages 1386-1392<br>ISSN: 1948-5875,<br>abstract, figures 1-3, and schematic diagrams 1-2 | 1-45, 46-47 (in part), 48-54 |
| X | WO 2021173773 A1 (MEDIBOSTON INC.) 02 September 2021 (2021-09-02)<br>claims 1-132, and description, paragraphs 32, 79 and 129-130 | 35-45, 46-47 (in part), 50-51 |
| X | WO 2021190602 A1 (JIANGSU HENGRUI MEDICINE CO., LTD. et al.) 30 September 2021 (2021-09-30)<br>embodiments 1-1~1-16, 1-20~1-33 and 1-37~1-44, and claims 1-15 | 1-18, 23-42, 45, 46-47 (in part), 48-49 |
| X | WO 2021190581 A1 (JIANGSU HENGRUI MEDICINE CO., LTD.) 30 September 2021 (2021-09-30)<br>embodiments 1-1~1-16, 1-20~1-33, 1-37~1-44 and 1-52~1-53, table 3, and claims 1-19 | 1-18, 23-42, 45, 46-47 (in part), 48-49 |
| X | WO 2022228406 A1 (JIANGSU HENGRUI PHARMACEUTICALS CO., LTD. et al.) 03 November 2022 (2022-11-03)<br>embodiments 4-5, and claims 1-25 | 1-18, 23-34, 45, 46-47 (in part), 48-49 |
| X | WO 2020259258 A1 (SHANGHAI FUDAN ZHANGJIANG BIO-PHARMACEUTICAL CO., LTD.) 30 December 2020 (2020-12-30)<br>claims 1-17, and embodiments 1-2 | 1-18, 23-38, 42, 45, 46-47 (in part), 48-49 |
| X | WO 2022033578 A1 (CHIA TAI TIANQING PHARMACEUTICAL GROUP CO., LTD. et al.) 17 February 2022 (2022-02-17)<br>claims 1-25 | 1-18, 23-38, 45, 46-47 (in part), 48-49 |
| X | WO 2022056696 A1 (SICHUAN BAILI PHARMACEUTICAL CO., LTD.) 24 March 2022 (2022-03-24)<br>embodiments 9 and 18, claims 1-10, and description, page 3 | 1-18, 23-39, 45, 46-47 (in part), 48-49 |
| X | WO 2021190586 A1 (JIANGSU HENGRUI MEDICINE CO., LTD. et al.) 30 September 2021 (2021-09-30)<br>embodiments 2-1~2-9 and 3-1~3-6, and claims 1-30 | 1-18, 23-39, 42, 45, 46-47 (in part), 48-49 |
| X | WO 2022054009 A2 (ASTRAZENECA UK LTD.) 17 March 2022 (2022-03-17)<br>claims 1-123, and description, paragraphs 85, 88, 90, 95 and 129 | 1-18, 23-38, 42, 45, 46-47 (in part), 48-49 |
| X | WO 2022068914 A1 (JIANGSU HENGRUI MEDICINE CO., LTD.) 07 April 2022 (2022-04-07)<br>claims 1-18, and description, pages 7-8 | 1-18, 23-33, 45, 46-47 (in part), 48-49 |
| X | WO 2022022508 A1 (TUOJIE BIOTECH(SHANGHAI) CO., LTD.) 03 February 2022 (2022-02-03)<br>claims 1-51, and description, pages 6-7, and embodiments 3-5~3-7 | 1-18, 23-33, 45, 46-47 (in part), 48-49 |
| X | CN 113939318 A (DAIICHI SANKYO COMPANY, LIMITED) 14 January 2022 (2022-01-14)<br>claims 1-66, embodiment 1, and description, paragraphs 222, 264, 346 and 387 | 1-18, 23-33, 35-38, 42, 45, 46-47 (in part), 48-49 |

Form PCT/ISA/210 (second sheet) (July 2022)

INTERNATIONAL SEARCH REPORT

International application No. PCT/CN2023/133434

C. DOCUMENTS CONSIDERED TO BE RELEVANT

| Category* | Citation of document, with indication, where appropriate, of the relevant passages | Relevant to claim No. |
|---|---|---|
| X | CN 110382535 A (DAIICHI SANKYO COMPANY, LIMITED) 25 October 2019 (2019-10-25)<br>claims 1-63, and description, page 23 to page 25, paragraphs 158 and 210-212, and embodiment 7 | 1-18, 23-33, 35-38, 42, 45, 46-47 (in part), 48-49 |
| X | CN 107922477 A (DAIICHI SANKYO COMPANY, LIMITED) 17 April 2018 (2018-04-17)<br>claims 1-25, and description, paragraphs 179, 195 and 241-243 | 1-18, 23-33, 35-38, 42, 45, 46-47 (in part), 48-49 |
| X | CN 112566942 A (DAIICHI SANKYO COMPANY, LIMITED) 26 March 2021 (2021-03-26)<br>claims 1-82, and description, paragraphs 438-439 | 1-18, 23-33, 35-38, 42, 45, 46-47 (in part), 48-49 |
| X | WO 2022078425 A1 (JIANGSU HENGRUI MEDICINE CO., LTD. et al.) 21 April 2022 (2022-04-21)<br>claims 1-19, and embodiments 3-1 and 3-4~3-5 | 1-18, 23-33, 45, 46-47 (in part), 48-49 |
| X | TASHIMA T. et al. "Brain Cancer Chemotherapy through a Delivery System across the Blood-Brain Barrier into the Brain Based on Receptor-Mediated Transcytosis Using Monoclonal Antibody Conjugates"<br>*Biomedicines,* Vol. vol. 10, 05 July 2022 (2022-07-05), page 1597<br>ISSN: 2227-9059,<br>figure 4, and abstract | 1-18, 23-33, 48-49 |
| X | CN 115215921 A (SHANGHAI FUDAN-ZHANGJIANG BIO-PHARMACEUTICAL CO., LTD.) 21 October 2022 (2022-10-21)<br>claims 1-14, and description, paragraph 275, and embodiment 3 | 1-18, 23-33, 35-39, 42, 48-49 |
| X | WO 2022198232 A1 (SEAGEN INC.) 22 September 2022 (2022-09-22)<br>claims 1-92, and description, paragraph 79, and tables 14-15 | 1-18, 23-33, 45, 46-47 (in part), 48-49 |
| X | WO 2022180581 A2 (MEDIBOSTON LTD.) 01 September 2022 (2022-09-01)<br>claims 1-51, and description, paragraphs 37-38 and 324-338, and schematic diagram 1 | 35-45, 46-47 (in part), 50-51 |
| PX | WO 2022253035 A1 (SICHUAN KELUN-BIOTECH BIOPHARMACEUTICAL CO., LTD. et al.) 08 December 2022 (2022-12-08)<br>claims 1-22, and description, pages 47-48 and 52-55 | 1-18, 21-39, 42-45, 46-47 (in part), 48-50, 53-54 |
| PX | WO 2023100829 A1 (DAIICHI SANKYO CO., LTD.) 08 June 2023 (2023-06-08)<br>description, paragraphs 140-157, and claims 1-35 | 1-18, 23-33, 45, 46-47 (in part), 48-49 |
| PX | WO 2023001300 A1 (SHANGHAI SENHUI MEDICINE CO., LTD. et al.) 26 January 2023 (2023-01-26)<br>claims 1-24, and embodiments 2-3 and 6 | 1-18, 23-38, 42, 45, 46-47 (in part), 48-49 |
| PX | WO 2023216956 A1 (SICHUAN KELUN-BIOTECH BIOPHARMACEUTICAL CO., LTD.) 16 November 2023 (2023-11-16)<br>claims 1-17, and description, page 41 | 1-18, 21-45, 46-47 (in part), 48-50, 53-54 |
| PX | WO 2023217227 A1 (SIMCERE ZAIMING PHARMACEUTICAL CO., LTD.) 16 November 2023 (2023-11-16)<br>embodiments 1-13, and claims 1-33 | 1-18, 23-42, 45, 46-47 (in part), 48-49 |
| PX | WO 2023208216 A1 (SICHUAN KELUN-BIOTECH BIOPHARMACEUTICAL CO., LTD.) 02 November 2023 (2023-11-02)<br>claims 1-71 | 1-18, 21-38, 42-45, 46-47 (in part), 48-50, 53-54 |
| PX | WO 2023109953 A1 (INNOVENT BIOLOGICS(SUZHOU) CO., LTD.) 22 June 2023 (2023-06-22)<br>claims 1-28, and embodiment 2.1.2 | 1-18, 23-33, 45, 46-47 (in part), 48-49 |
| PX | WO 2023207773 A1 (SHANGHAI MICURX PHARMACEUTICAL CO., LTD.) 02 November 2023 (2023-11-02)<br>claims 1-18 | 1-18, 23-33, 35-38, 42, 45, 46-47 (in part), 48-49 |

INTERNATIONAL SEARCH REPORT

International application No.
PCT/CN2023/133434

C. DOCUMENTS CONSIDERED TO BE RELEVANT

| Category* | Citation of document, with indication, where appropriate, of the relevant passages | Relevant to claim No. |
| --- | --- | --- |
| PX | WO 2023186015 A1 (DUALITY BIOLOGICS(SUZHOU) CO., LTD.) 05 October 2023 (2023-10-05)<br>claims 1-30, and preparation examples 1-3 | 1-18, 23-39, 42, 45, 46-47 (in part), 48-49 |
| PX | WO 2023178452 A1 (ZYMEWORKS BC INC.) 28 September 2023 (2023-09-28)<br>claims 1-46 | 1-18, 23-33, 35-39, 42, 45, 46-47 (in part), 48-49 |
| PX | WO 2023143263 A1 (MEDILINK THERAPEUTICS(SUZHOU) CO., LTD.) 03 August 2023 (2023-08-03)<br>claims 1-28 | 1-18, 21-33, 43-45, 46-47 (in part), 48-50, 53-54 |
| X | WO 2022078279 A1 (KUNSHAN XINYUNDA BIOTECH CO., LTD.) 21 April 2022 (2022-04-21)<br>claims 8-43 | 35-38, 42, 45, 46-47 (in part) |
| X | CN 103509030 A (LI, Yuliang) 15 January 2014 (2014-01-15)<br>embodiments 11-12, claims 11-13, and description, paragraphs 134-136 | 35-36, 40-42, 45, 46-47 (in part) |
| X | CN 102718770 A (SECOND MILITARY MEDICAL UNIVERSITY OF PLA) 10 October 2012 (2012-10-10)<br>claim 1, and description, paragraphs 5-8 | 41 |
| X | STN Registry. "CAS RN 110351-94-5"<br>*STN Registry*, 19 September 1987 (1987-09-19), p. 1<br>page 1 | 41 |
| X | STN Registry. "CAS RN 1260616-76-9"<br>*STN Registry*, 27 January 2011 (2011-01-27), p. 1<br>page 1 | 41 |
| X | STN Registry. "CAS RN 1332605-57-8"<br>*STN Registry*, 16 September 2011 (2011-09-16), p. 1<br>page 1 | 41 |
| X | STN Registry. "CAS RN 1346617-23-9"<br>*STN Registry*, 30 November 2011 (2011-11-30), p. 1<br>page 1 | 41 |
| X | STN Registry. "CAS RN 1586781-79-4"<br>*STN Registry*, 18 April 2014 (2014-04-18), p. 1<br>page 1 | 41 |
| X | STN Registry. "CAS RN 2778374-64-2"<br>*STN Registry*, 26 June 2022 (2022-06-26), p. 1<br>page 1 | 41 |
| X | STN Registry. "CAS RN 2781026-74-0"<br>*STN Registry*, 29 June 2022 (2022-06-29), p. 1<br>page 1 | 41 |
| X | STN Registry. "CAS RN 1401998-65-9"<br>*STN Registry*, 24 October 2012 (2012-10-24), p. 1<br>page 1 | 41 |
| X | STN Registry. "CAS RN 1519060-37-7"<br>*STN Registry*, 13 January 2014 (2014-01-13), p. 1<br>page 1 | 35, 42 |
| X | STN Registry. "CAS RN 1332575-17-3"<br>*STN Registry*, 16 September 2011 (2011-09-16), p. 1<br>page 1 | 35, 42 |

Form PCT/ISA/210 (second sheet) (July 2022)

**INTERNATIONAL SEARCH REPORT**

International application No.
**PCT/CN2023/133434**

**C. DOCUMENTS CONSIDERED TO BE RELEVANT**

| Category* | Citation of document, with indication, where appropriate, of the relevant passages | Relevant to claim No. |
|---|---|---|
| X | STN Registry. "CAS RN 1207112-67-1" *STN Registry*, 22 February 2010 (2010-02-22), p. 1 page 1 | 35, 42 |
| X | STN Registry. "CAS RN 289654-73-5" *STN Registry*, 19 September 2000 (2000-09-19), p. 1 page 1 | 35, 42 |
| X | STN Registry. "CAS RN 289654-71-3" *STN Registry*, 19 September 2000 (2000-09-19), p. 1 page 1 | 35, 42 |
| X | STN Registry. "CAS RN 289654-58-6" *STN Registry*, 19 September 2000 (2000-09-19), p. 1 page 1 | 35, 42 |
| X | STN Registry. "CAS RN 289654-52-0" *STN Registry*, 19 September 2000 (2000-09-19), p. 1 page 1 | 35, 42 |
| X | STN Registry. "CAS RN 289654-51-9" *STN Registry*, 19 September 2000 (2000-09-19), p. 1 page 1 | 35, 42 |
| X | STN Registry. "CAS RN 289654-48-4" *STN Registry*, 19 September 2000 (2000-09-19), p. 1 page 1 | 35, 42 |
| X | STN Registry. "CAS RN 289654-47-3" *STN Registry*, 19 September 2000 (2000-09-19), p. 1 page 1 | 35, 42 |
| X | STN Registry. "CAS RN 289654-46-2" *STN Registry*, 19 September 2000 (2000-09-19), p. 1 page 1 | 35, 42 |
| X | STN Registry. "CAS RN 289654-45-1" *STN Registry*, 19 September 2000 (2000-09-19), p. 1 page 1 | 35, 42 |
| X | STN Registry. "CAS RN 289654-44-0" *STN Registry*, 19 September 2000 (2000-09-19), p. 1 page 1 | 35, 42 |
| X | STN Registry. "CAS RN 289654-43-9" *STN Registry*, 19 September 2000 (2000-09-19), p. 1 page 1 | 35, 42 |
| X | STN Registry. "CAS RN 289654-42-8" *STN Registry*, 19 September 2000 (2000-09-19), p. 1 page 1 | 35, 42 |
| X | STN Registry. "CAS RN 289654-41-7" *STN Registry*, 19 September 2000 (2000-09-19), p. 1 page 1 | 35, 42 |
| X | STN Registry. "CAS RN 289654-40-6" *STN Registry*, 19 September 2000 (2000-09-19), p. 1 page 1 | 35, 42 |
| X | STN Registry. "CAS RN 289654-39-3" *STN Registry*, 19 September 2000 (2000-09-19), p. 1 page 1 | 35, 42 |

Form PCT/ISA/210 (second sheet) (July 2022)

INTERNATIONAL SEARCH REPORT

International application No. PCT/CN2023/133434

C. DOCUMENTS CONSIDERED TO BE RELEVANT

| Category* | Citation of document, with indication, where appropriate, of the relevant passages | Relevant to claim No. |
|---|---|---|
| X | STN Registry. "CAS RN 289654-38-2" *STN Registry*, 19 September 2000 (2000-09-19), p. 1 page 1 | 35, 42 |
| X | STN Registry. "CAS RN 289654-37-1" *STN Registry*, 19 September 2000 (2000-09-19), p. 1 page 1 | 35, 42 |
| X | STN Registry. "CAS RN 289654-36-0" *STN Registry*, 19 September 2000 (2000-09-19), p. 1 page 1 | 35, 42 |
| X | STN Registry. "CAS RN 289654-35-9" *STN Registry*, 19 September 2000 (2000-09-19), p. 1 page 1 | 35, 42 |
| X | STN Registry. "CAS RN 289654-34-8" *STN Registry*, 19 September 2000 (2000-09-19), p. 1 page 1 | 35, 42 |
| X | STN Registry. "CAS RN 289654-33-7" *STN Registry*, 19 September 2000 (2000-09-19), p. 1 page 1 | 35, 42 |
| X | STN Registry. "CAS RN 289654-32-6" *STN Registry*, 19 September 2000 (2000-09-19), p. 1 page 1 | 35, 42 |
| X | STN Registry. "CAS RN 289654-31-5" *STN Registry*, 19 September 2000 (2000-09-19), p. 1 page 1 | 35, 42 |
| X | STN Registry. "CAS RN 289654-30-4" *STN Registry*, 19 September 2000 (2000-09-19), p. 1 page 1 | 35, 42 |
| X | STN Registry. "CAS RN 289654-29-1" *STN Registry*, 19 September 2000 (2000-09-19), p. 1 page 1 | 35, 42 |
| X | STN Registry. "CAS RN 289654-28-0" *STN Registry*, 19 September 2000 (2000-09-19), p. 1 page 1 | 35, 42 |
| X | STN Registry. "CAS RN 289654-27-9" *STN Registry*, 19 September 2000 (2000-09-19), p. 1 page 1 | 35, 42 |
| X | STN Registry. "CAS RN 289654-26-8" *STN Registry*, 19 September 2000 (2000-09-19), p. 1 page 1 | 35, 42 |
| X | STN Registry. "CAS RN 289653-91-4" *STN Registry*, 19 September 2000 (2000-09-19), p. 1 page 1 | 35, 42 |
| X | STN Registry. "CAS RN 289653-90-3" *STN Registry*, 19 September 2000 (2000-09-19), p. 1 page 1 | 35, 42 |
| X | STN Registry. "CAS RN 289653-86-7" *STN Registry*, 19 September 2000 (2000-09-19), p. 1 page 1 | 35, 42 |

Form PCT/ISA/210 (second sheet) (July 2022)

**INTERNATIONAL SEARCH REPORT**

International application No. **PCT/CN2023/133434**

**C. DOCUMENTS CONSIDERED TO BE RELEVANT**

| Category* | Citation of document, with indication, where appropriate, of the relevant passages | Relevant to claim No. |
|---|---|---|
| X | STN Registry. "CAS RN 289653-84-5" *STN Registry,* 19 September 2000 (2000-09-19), p. 1 page 1 | 35, 42 |
| X | STN Registry. "CAS RN 289653-83-4" *STN Registry,* 19 September 2000 (2000-09-19), p. 1 page 1 | 35, 42 |
| X | STN Registry. "CAS RN 289653-81-2" *STN Registry,* 19 September 2000 (2000-09-19), p. 1 page 1 | 35, 42 |
| X | STN Registry. "CAS RN 220998-10-7" *STN Registry,* 07 April 1999 (1999-04-07), p. 1 page 1 | 35, 42 |
| X | STN Registry. "CAS RN 220997-99-9" *STN Registry,* 07 April 1999 (1999-04-07), p. 1 page 1 | 35, 42 |
| X | STN Registry. "CAS RN 1348557-54-9" *STN Registry,* 04 December 2011 (2011-12-04), p. 1 page 1 | 35, 42 |
| X | STN Registry. "CAS RN 2839561-81-6" *STN Registry,* 13 October 2022 (2022-10-13), p. 1 page 1 | 35-36, 42 |
| X | STN Registry. "CAS RN 2839561-74-7" *STN Registry,* 13 October 2022 (2022-10-13), p. 1 page 1 | 35-36, 42 |
| X | STN Registry. "CAS RN 2839561-38-3" *STN Registry,* 13 October 2022 (2022-10-13), p. 1 page 1 | 35-36, 42 |
| X | STN Registry. "CAS RN 2839561-29-2" *STN Registry,* 13 October 2022 (2022-10-13), p. 1 page 1 | 35-36, 42 |
| X | STN Registry. "CAS RN 2839561-24-7" *STN Registry,* 13 October 2022 (2022-10-13), p. 1 page 1 | 35-36, 42 |
| X | STN Registry. "CAS RN 2839561-23-6" *STN Registry,* 13 October 2022 (2022-10-13), p. 1 page 1 | 35-36, 42 |
| X | STN Registry. "CAS RN 2839561-18-9" *STN Registry,* 13 October 2022 (2022-10-13), p. 1 page 1 | 35-36, 42 |
| X | STN Registry. "CAS RN 2839561-16-7" *STN Registry,* 13 October 2022 (2022-10-13), p. 1 page 1 | 35-36, 42 |
| X | STN Registry. "CAS RN 2839561-10-1" *STN Registry,* 13 October 2022 (2022-10-13), p. 1 page 1 | 35-36, 42 |
| X | STN Registry. "CAS RN 2839561-41-8" *STN Registry,* 13 October 2022 (2022-10-13), p. 1 page 1 | 35-36, 42 |

Form PCT/ISA/210 (second sheet) (July 2022)

**INTERNATIONAL SEARCH REPORT**

International application No.
**PCT/CN2023/133434**

**C. DOCUMENTS CONSIDERED TO BE RELEVANT**

| Category* | Citation of document, with indication, where appropriate, of the relevant passages | Relevant to claim No. |
|---|---|---|
| X | STN Registry. "CAS RN 2813269-35-9" *STN Registry*, 26 August 2022 (2022-08-26), p. 1 page 1 | 35-36, 42 |
| X | STN Registry. "CAS RN 2813269-32-6" *STN Registry*, 26 August 2022 (2022-08-26), p. 1 page 1 | 35-36, 42 |
| X | STN Registry. "CAS RN 2813268-95-8" *STN Registry*, 26 August 2022 (2022-08-26), p. 1 page 1 | 35-36, 42 |
| X | STN Registry. "CAS RN 2813268-94-7" *STN Registry*, 26 August 2022 (2022-08-26), p. 1 page 1 | 35-36, 42 |
| X | STN Registry. "CAS RN 2813268-81-2" *STN Registry*, 26 August 2022 (2022-08-26), p. 1 page 1 | 35-36, 42 |
| X | STN Registry. "CAS RN 2813268-76-5" *STN Registry*, 26 August 2022 (2022-08-26), p. 1 page 1 | 35-36, 42 |
| X | STN Registry. "CAS RN 2813268-73-2" *STN Registry*, 26 August 2022 (2022-08-26), p. 1 page 1 | 35-36, 42 |
| X | STN Registry. "CAS RN 2761690-62-2" *STN Registry*, 08 May 2022 (2022-05-08), p. 1 page 1 | 35-36, 42 |
| X | STN Registry. "CAS RN 2761690-61-1" *STN Registry*, 08 May 2022 (2022-05-08), p. 1 page 1 | 35-36, 42 |
| X | STN Registry. "CAS RN 2761690-60-0" *STN Registry*, 08 May 2022 (2022-05-08), p. 1 page 1 | 35-36, 42 |
| X | STN Registry. "CAS RN 2761690-58-6" *STN Registry*, 08 May 2022 (2022-05-08), p. 1 page 1 | 35-36, 42 |
| X | STN Registry. "CAS RN 2761690-56-4" *STN Registry*, 08 May 2022 (2022-05-08), p. 1 page 1 | 35-36, 42 |
| X | STN Registry. "CAS RN 2761690-55-3" *STN Registry*, 08 May 2022 (2022-05-08), p. 1 page 1 | 35-36, 42 |
| X | STN Registry. "CAS RN 2761690-54-2" *STN Registry*, 08 May 2022 (2022-05-08), p. 1 page 1 | 35-36, 42 |
| X | STN Registry. "CAS RN 2761690-53-1" *STN Registry*, 08 May 2022 (2022-05-08), p. 1 page 1 | 35-36, 42 |
| X | STN Registry. "CAS RN 2761690-52-0" *STN Registry*, 08 May 2022 (2022-05-08), p. 1 page 1 | 35-36, 42 |

Form PCT/ISA/210 (second sheet) (July 2022)

INTERNATIONAL SEARCH REPORT

International application No.
PCT/CN2023/133434

C. DOCUMENTS CONSIDERED TO BE RELEVANT

| Category* | Citation of document, with indication, where appropriate, of the relevant passages | Relevant to claim No. |
|---|---|---|
| X | STN Registry. "CAS RN 2813270-44-7" *STN Registry,* 26 August 2022 (2022-08-26), p. 1 page 1 | 35-37, 42 |
| X | STN Registry. "CAS RN 2813270-17-4" *STN Registry,* 26 August 2022 (2022-08-26), p. 1 page 1 | 35-37, 42 |
| X | STN Registry. "CAS RN 2813269-37-1" *STN Registry,* 26 August 2022 (2022-08-26), p. 1 page 1 | 35-37, 42 |
| X | STN Registry. "CAS RN 2813269-36-0" *STN Registry,* 26 August 2022 (2022-08-26), p. 1 page 1 | 35-37, 42 |
| X | STN Registry. "CAS RN 2813269-34-8" *STN Registry,* 26 August 2022 (2022-08-26), p. 1 page 1 | 35-37, 42 |
| X | STN Registry. "CAS RN 2813269-33-7" *STN Registry,* 26 August 2022 (2022-08-26), p. 1 page 1 | 35-37, 42 |
| X | STN Registry. "CAS RN 2813268-85-6" *STN Registry,* 26 August 2022 (2022-08-26), p. 1 page 1 | 35-37, 42 |
| X | STN Registry. "CAS RN 2813268-84-5" *STN Registry,* 26 August 2022 (2022-08-26), p. 1 page 1 | 35-37, 42 |
| X | STN Registry. "CAS RN 2813268-83-4" *STN Registry,* 26 August 2022 (2022-08-26), p. 1 page 1 | 35-37, 42 |
| X | STN Registry. "CAS RN 2813268-82-3" *STN Registry,* 26 August 2022 (2022-08-26), p. 1 page 1 | 35-37, 42 |
| X | STN Registry. "CAS RN 2813268-80-1" *STN Registry,* 26 August 2022 (2022-08-26), p. 1 page 1 | 35-37, 42 |
| X | STN Registry. "CAS RN 2813268-79-8" *STN Registry,* 26 August 2022 (2022-08-26), p. 1 page 1 | 35-37, 42 |
| X | STN Registry. "CAS RN 2813268-78-7" *STN Registry,* 26 August 2022 (2022-08-26), p. 1 page 1 | 35-37, 42 |
| X | STN Registry. "CAS RN 2813268-77-6" *STN Registry,* 26 August 2022 (2022-08-26), p. 1 page 1 | 35-37, 42 |
| X | STN Registry. "CAS RN 2813268-75-4" *STN Registry,* 26 August 2022 (2022-08-26), p. 1 page 1 | 35-37, 42 |
| X | STN Registry. "CAS RN 2813268-74-3" *STN Registry,* 26 August 2022 (2022-08-26), p. 1 page 1 | 35-37, 42 |

Form PCT/ISA/210 (second sheet) (July 2022)

INTERNATIONAL SEARCH REPORT

International application No.
PCT/CN2023/133434

C. DOCUMENTS CONSIDERED TO BE RELEVANT

| Category* | Citation of document, with indication, where appropriate, of the relevant passages | Relevant to claim No. |
|---|---|---|
| X | STN Registry. "CAS RN 2813268-42-5" *STN Registry*, 26 August 2022 (2022-08-26), p. 1 page 1 | 35-37, 42 |
| X | STN Registry. "CAS RN 2813268-41-4" *STN Registry*, 26 August 2022 (2022-08-26), p. 1 page 1 | 35-37, 42 |
| X | STN Registry. "CAS RN 2813268-40-3" *STN Registry*, 26 August 2022 (2022-08-26), p. 1 page 1 | 35-37, 42 |
| X | STN Registry. "CAS RN 2813268-39-0" *STN Registry*, 26 August 2022 (2022-08-26), p. 1 page 1 | 35-37, 42 |
| X | STN Registry. "CAS RN 2813268-38-9" *STN Registry*, 26 August 2022 (2022-08-26), p. 1 page 1 | 35-37, 42 |
| X | STN Registry. "CAS RN 2813268-37-8" *STN Registry*, 26 August 2022 (2022-08-26), p. 1 page 1 | 35-37, 42 |
| X | STN Registry. "CAS RN 2769923-10-4" *STN Registry*, 29 May 2022 (2022-05-29), p. 1 page 1 | 35-37, 42 |
| X | STN Registry. "CAS RN 2766786-88-1" *STN Registry*, 02 May 2022 (2022-05-02), p. 1 page 1 | 35-37, 42 |
| X | STN Registry. "CAS RN 2766786-87-0" *STN Registry*, 02 May 2022 (2022-05-02), p. 1 page 1 | 35-37, 42 |
| X | STN Registry. "CAS RN 2766786-86-9" *STN Registry*, 02 May 2022 (2022-05-02), p. 1 page 1 | 35-37, 42 |
| X | STN Registry. "CAS RN 2766786-85-8" *STN Registry*, 02 May 2022 (2022-05-02), p. 1 page 1 | 35-37, 42 |
| X | STN Registry. "CAS RN 2766786-84-7" *STN Registry*, 02 May 2022 (2022-05-02), p. 1 page 1 | 35-37, 42 |
| X | STN Registry. "CAS RN 2766786-64-3" *STN Registry*, 02 May 2022 (2022-05-02), p. 1 page 1 | 35-37, 42 |
| X | STN Registry. "CAS RN 2766786-63-2" *STN Registry*, 02 May 2022 (2022-05-02), p. 1 page 1 | 35-37, 42 |
| X | STN Registry. "CAS RN 2766786-62-1" *STN Registry*, 02 May 2022 (2022-05-02), p. 1 page 1 | 35-37, 42 |
| X | STN Registry. "CAS RN 2766786-61-0" *STN Registry*, 02 May 2022 (2022-05-02), p. 1 | 35-37, 42 |

Form PCT/ISA/210 (second sheet) (July 2022)

**INTERNATIONAL SEARCH REPORT**

International application No.
**PCT/CN2023/133434**

**C. DOCUMENTS CONSIDERED TO BE RELEVANT**

| Category* | Citation of document, with indication, where appropriate, of the relevant passages | Relevant to claim No. |
|---|---|---|
| X | STN Registry. "CAS RN 2766786-60-9" *STN Registry*, 02 May 2022 (2022-05-02), p. 1 page 1 | 35-37, 42 |
| X | STN Registry. "CAS RN 2766786-59-6" *STN Registry*, 02 May 2022 (2022-05-02), p. 1 | 35-37, 42 |
| X | STN Registry. "CAS RN 2766786-58-5" *STN Registry*, 02 May 2022 (2022-05-02), p. 1 | 35-37, 42 |
| X | STN Registry. "CAS RN 2761690-29-1" *STN Registry*, 08 May 2022 (2022-05-08), p. 1 | 35-37, 42 |
| X | STN Registry. "CAS RN 2761690-28-0" *STN Registry*, 08 May 2022 (2022-05-08), p. 1 | 35-37, 42 |
| X | STN Registry. "CAS RN 2761690-25-7" *STN Registry*, 08 May 2022 (2022-05-08), p. 1 | 35-37, 42 |
| X | STN Registry. "CAS RN 2761690-24-6" *STN Registry*, 08 May 2022 (2022-05-08), p. 1 | 35-37, 42 |
| X | STN Registry. "CAS RN 2761690-22-4" *STN Registry*, 08 May 2022 (2022-05-08), p. 1 | 35-37, 42 |
| X | STN Registry. "CAS RN 2761690-20-2" *STN Registry*, 08 May 2022 (2022-05-08), p. 1 | 35-37, 42 |
| X | STN Registry. "CAS RN 2761690-17-7" *STN Registry*, 08 May 2022 (2022-05-08), p. 1 | 35-37, 42 |
| X | STN Registry. "CAS RN 2761690-15-5" *STN Registry*, 08 May 2022 (2022-05-08), p. 1 | 35-37, 42 |
| X | STN Registry. "CAS RN 2761690-12-2" *STN Registry*, 08 May 2022 (2022-05-08), p. 1 | 35-37, 42 |
| X | STN Registry. "CAS RN 2761690-10-0" *STN Registry*, 08 May 2022 (2022-05-08), p. 1 | 35-37, 42 |
| X | STN Registry. "CAS RN 2761690-08-6" *STN Registry*, 08 May 2022 (2022-05-08), p. 1 | 35-37, 42 |
| X | STN Registry. "CAS RN 2761690-06-4" *STN Registry*, 08 May 2022 (2022-05-08), p. 1 | 35-37, 42 |
| X | STN Registry. "CAS RN 2760715-90-8" *STN Registry*, 27 February 2022 (2022-02-27), p. 1 | 35-37, 42 |

Form PCT/ISA/210 (second sheet) (July 2022)

**INTERNATIONAL SEARCH REPORT**

International application No.
**PCT/CN2023/133434**

| C. DOCUMENTS CONSIDERED TO BE RELEVANT | | |
|---|---|---|
| Category* | Citation of document, with indication, where appropriate, of the relevant passages | Relevant to claim No. |
| X | STN Registry. "CAS RN 2750624-39-4" *STN Registry*, 27 December 2021 (2021-12-27), p. 1 | 35-37, 42 |
| X | STN Registry. "CAS RN 2750624-38-3" *STN Registry*, 27 December 2021 (2021-12-27), p. 1 | 35-37, 42 |
| X | STN Registry. "CAS RN 2577204-75-0" *STN Registry*, 27 January 2021 (2021-01-27), p. 1 | 35-37, 42 |
| X | STN Registry. "CAS RN 2577204-73-8" *STN Registry*, 27 January 2021 (2021-01-27), p. 1 | 35-37, 42 |
| X | STN Registry. "CAS RN 2577204-71-6" *STN Registry*, 27 January 2021 (2021-01-27), p. 1 | 35-37, 42 |
| X | STN Registry. "CAS RN 2577204-69-2" *STN Registry*, 27 January 2021 (2021-01-27), p. 1 | 35-37, 42 |
| X | STN Registry. "CAS RN 2577204-54-5" *STN Registry*, 27 January 2021 (2021-01-27), p. 1 | 35-37, 42 |
| X | STN Registry. "CAS RN 2577204-50-1" *STN Registry*, 27 January 2021 (2021-01-27), p. 1 | 35-37, 42 |
| X | STN Registry. "CAS RN 2577204-48-7" *STN Registry*, 27 January 2021 (2021-01-27), p. 1 | 35-37, 42 |
| X | STN Registry. "CAS RN 2577204-46-5" *STN Registry*, 27 January 2021 (2021-01-27), p. 1 | 35-37, 42 |
| X | STN Registry. "CAS RN 2577204-44-3" *STN Registry*, 27 January 2021 (2021-01-27), p. 1 | 35-37, 42 |
| X | STN Registry. "CAS RN 2577204-16-9" *STN Registry*, 27 January 2021 (2021-01-27), p. 1 | 35-37, 42 |
| X | STN Registry. "CAS RN 2451488-64-3" *STN Registry*, 30 July 2020 (2020-07-30), p. 1 | 35-37, 42 |
| X | STN Registry. "CAS RN 2414393-21-6" *STN Registry*, 20 April 2020 (2020-04-20), p. 1 | 35-37, 42 |
| X | STN Registry. "CAS RN 2414393-20-5" *STN Registry*, 20 April 2020 (2020-04-20), p. 1 | 35-37, 42 |
| X | STN Registry. "CAS RN 2414254-40-1" *STN Registry*, 17 April 2020 (2020-04-17), p. 1 | 35-37, 42 |

Form PCT/ISA/210 (second sheet) (July 2022)

**INTERNATIONAL SEARCH REPORT**

International application No.
**PCT/CN2023/133434**

**C. DOCUMENTS CONSIDERED TO BE RELEVANT**

| Category* | Citation of document, with indication, where appropriate, of the relevant passages | Relevant to claim No. |
|---|---|---|
| X | STN Registry. "CAS RN 2414254-39-8" *STN Registry*, 17 April 2020 (2020-04-17), p. 1 | 35-37, 42 |
| X | STN Registry. "CAS RN 2414254-38-7" *STN Registry*, 17 April 2020 (2020-04-17), p. 1 | 35-37, 42 |
| X | STN Registry. "CAS RN 2414254-37-6" *STN Registry*, 17 April 2020 (2020-04-17), p. 1 | 35-37, 42 |
| X | STN Registry. "CAS RN 2414254-36-5" *STN Registry*, 17 April 2020 (2020-04-17), p. 1 | 35-37, 42 |
| X | STN Registry. "CAS RN 2414254-35-4" *STN Registry*, 17 April 2020 (2020-04-17), p. 1 | 35-37, 42 |
| X | STN Registry. "CAS RN 1599440-33-1" *STN Registry*, 07 May 2014 (2014-05-07), p. 1 | 35-37, 42 |

Form PCT/ISA/210 (second sheet) (July 2022)

**INTERNATIONAL SEARCH REPORT**

International application No.
**PCT/CN2023/133434**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2023/133434**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **46-47 (in part)**
because they relate to subject matter not required to be searched by this Authority, namely:

Claim 46 sets forth the use of a compound as shown in formula (GH) of any one of claims 35-38, and a stereoisomer, racemate, tautomer, isotope body, isotope marker, nitrogen oxide or pharmaceutically acceptable salt thereof, or a conjugate as shown in formula (C) of any one of claims 1-33 or a conjugate as shown in formula (D) of any one of claims 43-44, and a stereoisomer, racemate, tautomer, isotope body, isotope marker, nitrogen oxide or pharmaceutically acceptable salt thereof for preventing and/or treating diseases or conditions and/or preparing a drug; and claim 47 directly refers to claim 46. Therefore, part of the subject matter of claims 46-47 relates to a method for treatment of the human or animal body, which falls within the cases set out in PCT Rule 39.1(iv) for which a search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2023/133434**

| Patent document cited in search report | Publication date (day/month/year) | Patent family member(s) | Publication date (day/month/year) |
|---|---|---|---|
| WO 2022166762 A1 | 11 August 2022 | KR 20230142710 A | 11 October 2023 |
| | | CA 3209426 A1 | 11 August 2022 |
| | | AU 2022216696 A1 | 17 August 2023 |
| | | EP 4289851 A1 | 13 December 2023 |
| WO 2022078260 A1 | 21 April 2022 | JP 2023545581 A | 30 October 2023 |
| | | AU 2021359457 A1 | 11 May 2023 |
| | | IL 302054 A | 01 June 2023 |
| | | EP 4227310 A1 | 16 August 2023 |
| CN 113943310 A | 18 January 2022 | JP 2023545580 A | 30 October 2023 |
| | | AU 2021361938 A1 | 25 May 2023 |
| | | IL 302053 A | 01 June 2023 |
| | | EP 4227309 A1 | 16 August 2023 |
| | | WO 2022078259 A1 | 21 April 2022 |
| | | US 2023381332 A1 | 30 November 2023 |
| WO 2021249228 A1 | 16 December 2021 | US 2023226207 A1 | 20 July 2023 |
| | | IL 298787 A | 01 February 2023 |
| | | AU 2021289927 A1 | 19 January 2023 |
| | | CA 3186295 A1 | 16 December 2021 |
| | | BR 112022024930 A2 | 27 December 2022 |
| | | JP 2023529415 A | 10 July 2023 |
| | | KR 20230022211 A | 14 February 2023 |
| | | EP 4162954 A1 | 12 April 2023 |
| WO 2020063676 A1 | 02 April 2020 | ZA 202102544 B | 25 October 2023 |
| | | CA 3114137 A1 | 02 April 2020 |
| | | BR 112021004656 A2 | 01 June 2021 |
| | | JP 2022511351 A | 31 January 2022 |
| | | EP 3858386 A1 | 04 August 2021 |
| | | EP 3858386 A4 | 12 October 2022 |
| | | KR 20210066833 A | 07 June 2021 |
| | | MX 2021003382 A | 27 May 2021 |
| | | US 2021353764 A1 | 18 November 2021 |
| | | AU 2019349207 A1 | 08 April 2021 |
| | | TW 202027795 A | 01 August 2020 |
| WO 2022011075 A1 | 13 January 2022 | WO 2022011075 A9 | 10 March 2022 |
| | | EP 4178559 A1 | 17 May 2023 |
| | | US 2023293712 A1 | 21 September 2023 |
| WO 2021173773 A1 | 02 September 2021 | JP 2023520605 A | 17 May 2023 |
| | | TW 202146055 A | 16 December 2021 |
| | | US 2021283125 A1 | 16 September 2021 |
| | | CA 3168882 A1 | 02 September 2021 |
| | | AU 2021226341 A1 | 29 September 2022 |
| | | MX 2022010457 A | 16 November 2022 |
| | | EP 4110402 A1 | 04 January 2023 |
| | | KR 20230004453 A | 06 January 2023 |
| | | BR 112022017064 A2 | 16 November 2022 |
| WO 2021190602 A1 | 30 September 2021 | CA 3175048 A1 | 30 September 2021 |
| | | TW 202144014 A | 01 December 2021 |
| | | AU 2021243080 A1 | 22 September 2022 |
| | | JP 2023521956 A | 26 May 2023 |

Form PCT/ISA/210 (patent family annex) (July 2022)

**INTERNATIONAL SEARCH REPORT**
**Information on patent family members**

International application No.
**PCT/CN2023/133434**

| Patent document cited in search report | Publication date (day/month/year) | Patent family member(s) | Publication date (day/month/year) |
|---|---|---|---|
| | | US 2023241242 A1 | 03 August 2023 |
| | | MX 2022011769 A | 18 October 2022 |
| | | BR 112022019042 A2 | 01 November 2022 |
| | | EP 4130045 A1 | 08 February 2023 |
| | | EP 4130045 A4 | 27 December 2023 |
| | | KR 20220157425 A | 29 November 2022 |
| WO 2021190581 A1 | 30 September 2021 | MX 2022011770 A | 18 October 2022 |
| | | KR 20220157998 A | 29 November 2022 |
| | | BR 112022019073 A2 | 08 November 2022 |
| | | JP 2023518583 A | 02 May 2023 |
| | | AU 2021243073 A1 | 13 October 2022 |
| | | TW 202144013 A | 01 December 2021 |
| | | US 2023165969 A1 | 01 June 2023 |
| | | EP 4129345 A1 | 08 February 2023 |
| | | EP 4129345 A4 | 29 November 2023 |
| | | CA 3175733 A1 | 30 September 2021 |
| WO 2022228406 A1 | 03 November 2022 | TW 202302649 A | 16 January 2023 |
| | | KR 20240000532 A | 02 January 2024 |
| | | AU 2022266934 A1 | 26 October 2023 |
| | | CA 3218512 A1 | 03 November 2022 |
| WO 2020259258 A1 | 30 December 2020 | EP 3991754 A1 | 04 May 2022 |
| | | EP 3991754 A4 | 17 May 2023 |
| | | KR 20220025861 A | 03 March 2022 |
| | | AU 2020301289 A1 | 24 February 2022 |
| | | BR 112021026580 A2 | 03 May 2022 |
| | | US 2022233708 A1 | 28 July 2022 |
| | | JP 2022542222 A | 30 September 2022 |
| | | JP 7407845 B2 | 04 January 2024 |
| | | CA 3144790 A1 | 30 December 2020 |
| WO 2022033578 A1 | 17 February 2022 | IL 300446 A | 01 April 2023 |
| | | AU 2021323863 A1 | 23 March 2023 |
| | | JP 2023537051 A | 30 August 2023 |
| | | BR 112023002417 A2 | 21 March 2023 |
| | | EP 4183421 A1 | 24 May 2023 |
| | | US 2023405141 A1 | 21 December 2023 |
| | | CA 3188508 A1 | 17 February 2022 |
| | | KR 20230051214 A | 17 April 2023 |
| WO 2022056696 A1 | 24 March 2022 | MX 2022003397 A | 26 May 2022 |
| | | AU 2023285718 A1 | 18 January 2024 |
| | | AU 2020442003 A1 | 31 March 2022 |
| | | AU 2020442003 B2 | 28 September 2023 |
| | | BR 112022002353 A2 | 26 April 2022 |
| | | TW 202216724 A | 01 May 2022 |
| | | KR 20220038601 A | 29 March 2022 |
| | | US 2022378928 A1 | 01 December 2022 |
| | | JP 2022552757 A | 20 December 2022 |
| | | CA 3170019 A1 | 24 March 2022 |
| | | IL 288215 A | 01 January 2022 |
| | | EP 3995496 A1 | 11 May 2022 |

Form PCT/ISA/210 (patent family annex) (July 2022)

**INTERNATIONAL SEARCH REPORT**
**Information on patent family members**

International application No.
**PCT/CN2023/133434**

| Patent document cited in search report | Publication date (day/month/year) | Patent family member(s) | Publication date (day/month/year) |
|---|---|---|---|
| | | EP 3995496 A4 | 30 November 2022 |
| WO 2021190586 A1 | 30 September 2021 | TW 202144015 A | 01 December 2021 |
| | | CN 115605510 A | 13 January 2023 |
| WO 2022054009 A2 | 17 March 2022 | IL 301265 A | 01 May 2023 |
| | | WO 2022054009 A3 | 21 April 2022 |
| | | CA 3195062 A1 | 17 March 2022 |
| | | KR 20230066094 A | 12 May 2023 |
| | | JP 2023545359 A | 30 October 2023 |
| | | EP 4211598 A2 | 19 July 2023 |
| | | AU 2021340002 A1 | 25 May 2023 |
| WO 2022068914 A1 | 07 April 2022 | KR 20230079096 A | 05 June 2023 |
| | | EP 4223785 A1 | 09 August 2023 |
| | | IL 301658 A | 01 May 2023 |
| | | AU 2021354827 A1 | 01 June 2023 |
| | | JP 2023545382 A | 30 October 2023 |
| | | US 2024024498 A1 | 25 January 2024 |
| | | BR 112023005789 A2 | 25 April 2023 |
| | | CA 3193104 A1 | 07 April 2022 |
| | | TW 202220702 A | 01 June 2022 |
| WO 2022022508 A1 | 03 February 2022 | KR 20230044275 A | 03 April 2023 |
| | | EP 4190812 A1 | 07 June 2023 |
| | | AU 2021317378 A1 | 09 March 2023 |
| | | BR 112023001359 A2 | 14 February 2023 |
| | | CA 3184724 A1 | 03 February 2022 |
| | | TW 202214306 A | 16 April 2022 |
| | | US 2023405138 A1 | 21 December 2023 |
| | | JP 2023535598 A | 18 August 2023 |
| CN 113939318 A | 14 January 2022 | KR 20220015445 A | 08 February 2022 |
| | | BR 112021023901 A2 | 18 January 2022 |
| | | TW 202108180 A | 01 March 2021 |
| | | SG 11202112429 PA | 30 December 2021 |
| | | EP 3976113 A1 | 06 April 2022 |
| | | CA 3142119 A1 | 03 December 2020 |
| | | WO 2020240467 A1 | 03 December 2020 |
| | | IL 288485 A | 01 January 2022 |
| | | JP 2022534725 A | 03 August 2022 |
| | | US 2023270870 A1 | 31 August 2023 |
| | | AU 2020285681 A1 | 27 January 2022 |
| CN 110382535 A | 25 October 2019 | EP 3572428 A1 | 27 November 2019 |
| | | EP 3572428 A4 | 30 December 2020 |
| | | AU 2018210081 A1 | 08 August 2019 |
| | | KR 20190104160 A | 06 September 2019 |
| | | KR 102537651 B1 | 26 May 2023 |
| | | JP 2021105063 A | 26 July 2021 |
| | | JP 7064635 B2 | 10 May 2022 |
| | | WO 2018135501 A1 | 26 July 2018 |
| | | IL 268102 A | 26 September 2019 |
| | | US 2020362032 A1 | 19 November 2020 |
| | | US 10906974 B2 | 02 February 2021 |

Form PCT/ISA/210 (patent family annex) (July 2022)

**INTERNATIONAL SEARCH REPORT**
**Information on patent family members**

International application No.
**PCT/CN2023/133434**

| Patent document cited in search report | | | Publication date (day/month/year) | Patent family member(s) | | | Publication date (day/month/year) |
|---|---|---|---|---|---|---|---|
| | | | | TW | 201831214 | A | 01 September 2018 |
| | | | | TWI | 780104 | B | 11 October 2022 |
| | | | | RU | 2019123616 | A | 19 February 2021 |
| | | | | RU | 2019123616 | A3 | 02 June 2021 |
| | | | | BR | 112019012847 | A2 | 10 December 2019 |
| | | | | CA | 3050668 | A1 | 26 July 2018 |
| | | | | CA | 3050668 | C | 15 August 2023 |
| | | | | MX | 2019008059 | A | 11 December 2019 |
| | | | | JPWO | 2018135501 | A1 | 07 November 2019 |
| | | | | JP | 6679762 | B2 | 15 April 2020 |
| | | | | JP | 2020099351 | A | 02 July 2020 |
| | | | | JP | 6875575 | B2 | 26 May 2021 |
| | | | | US | 2019225686 | A1 | 25 July 2019 |
| | | | | US | 11434289 | B2 | 06 September 2022 |
| | | | | CO | 2019004791 | A2 | 21 May 2019 |
| | | | | PH | 12019501663 | A1 | 24 February 2020 |
| | | | | SG | 11201906554 | RA | 27 August 2019 |
| CN | 107922477 | A | 17 April 2018 | BR | 112017027690 | A2 | 09 October 2018 |
| | | | | IL | 256558 | A | 28 February 2018 |
| | | | | IL | 256558 | B | 01 April 2022 |
| | | | | US | 2018147292 | A1 | 31 May 2018 |
| | | | | US | 11173213 | B2 | 16 November 2021 |
| | | | | CA | 2990572 | A1 | 05 January 2017 |
| | | | | CA | 2990572 | C | 26 July 2022 |
| | | | | HK | 1247211 | A1 | 21 September 2018 |
| | | | | TW | 201705982 | A | 16 February 2017 |
| | | | | TWI | 725037 | B | 21 April 2021 |
| | | | | IL | 290959 | A | 01 April 2022 |
| | | | | IL | 290959 | B | 01 December 2022 |
| | | | | IL | 290959 | B2 | 01 April 2023 |
| | | | | EP | 4180455 | A1 | 17 May 2023 |
| | | | | JP | 2021020956 | A | 18 February 2021 |
| | | | | JP | 7118117 | B2 | 15 August 2022 |
| | | | | US | 2021386865 | A1 | 16 December 2021 |
| | | | | HUE | 061408 | T2 | 28 June 2023 |
| | | | | TW | 202130368 | A | 16 August 2021 |
| | | | | KR | 20180021723 | A | 05 March 2018 |
| | | | | ES | 2938186 | T3 | 05 April 2023 |
| | | | | EP | 3315512 | A1 | 02 May 2018 |
| | | | | EP | 3315512 | A4 | 13 February 2019 |
| | | | | EP | 3315512 | B1 | 23 November 2022 |
| | | | | AU | 2016286898 | A1 | 04 January 2018 |
| | | | | AU | 2016286898 | B2 | 08 December 2022 |
| | | | | JPWO | 2017002776 | A1 | 31 May 2018 |
| | | | | JP | 6787890 | B2 | 18 November 2020 |
| | | | | WO | 2017002776 | A1 | 05 January 2017 |
| | | | | CN | 107922477 | B | 01 November 2022 |
| CN | 112566942 | A | 26 March 2021 | AU | 2019311557 | A1 | 04 February 2021 |
| | | | | CA | 3107417 | A1 | 30 January 2020 |
| | | | | CA | 3107417 | C | 17 October 2023 |

Form PCT/ISA/210 (patent family annex) (July 2022)

**INTERNATIONAL SEARCH REPORT**
**Information on patent family members**

International application No.
**PCT/CN2023/133434**

| Patent document cited in search report | Publication date (day/month/year) | Patent family member(s) | Publication date (day/month/year) |
|---|---|---|---|
| | | KR 20210038904 A | 08 April 2021 |
| | | IL 280295 A | 25 March 2021 |
| | | SG 11202100653 YA | 25 February 2021 |
| | | TW 202019972 A | 01 June 2020 |
| | | US 2021283269 A1 | 16 September 2021 |
| | | EP 3828206 A1 | 02 June 2021 |
| | | EP 3828206 A4 | 20 April 2022 |
| | | JPWO 2020022363 A1 | 02 August 2021 |
| | | BR 112021001194 A2 | 27 April 2021 |
| | | WO 2020022363 A1 | 30 January 2020 |
| WO 2022078425 A1 | 21 April 2022 | CA 3196940 A1 | 21 April 2022 |
| | | JP 2023545925 A | 01 November 2023 |
| | | KR 20230086702 A | 15 June 2023 |
| | | AU 2021360625 A1 | 01 June 2023 |
| | | TW 202304983 A | 01 February 2023 |
| | | US 2024026028 A1 | 25 January 2024 |
| | | EP 4230653 A1 | 23 August 2023 |
| CN 115215921 A | 21 October 2022 | None | |
| WO 2022198232 A1 | 22 September 2022 | CA 3213625 A1 | 22 September 2022 |
| | | KR 20230158006 A | 17 November 2023 |
| | | TW 202300179 A | 01 January 2023 |
| | | AU 2022237791 A1 | 14 September 2023 |
| | | IL 305886 A | 01 November 2023 |
| | | EP 4308171 A1 | 24 January 2024 |
| WO 2022180581 A2 | 01 September 2022 | US 2022378929 A1 | 01 December 2022 |
| | | CA 3208591 A1 | 01 September 2022 |
| | | WO 2022180581 A3 | 13 October 2022 |
| | | KR 20230154892 A | 09 November 2023 |
| | | TW 202302643 A | 16 January 2023 |
| | | EP 4297797 A2 | 03 January 2024 |
| | | AU 2022228004 A1 | 28 September 2023 |
| | | AU 2022228004 A9 | 25 January 2024 |
| WO 2022253035 A1 | 08 December 2022 | AU 2022287001 A1 | 26 October 2023 |
| | | CA 3218527 A1 | 08 December 2022 |
| WO 2023100829 A1 | 08 June 2023 | TW 202334238 A | 01 September 2023 |
| | | WO 2023100829 A8 | 19 October 2023 |
| WO 2023001300 A1 | 26 January 2023 | AU 2022316425 A1 | 01 February 2024 |
| | | TW 202310878 A | 16 March 2023 |
| WO 2023216956 A1 | 16 November 2023 | None | |
| WO 2023217227 A1 | 16 November 2023 | None | |
| WO 2023208216 A1 | 02 November 2023 | None | |
| WO 2023109953 A1 | 22 June 2023 | TW 202333798 A | 01 September 2023 |
| | | WO 2023109953 A9 | 27 July 2023 |
| WO 2023207773 A1 | 02 November 2023 | None | |
| WO 2023186015 A1 | 05 October 2023 | None | |
| WO 2023178452 A1 | 28 September 2023 | None | |
| WO 2023143263 A1 | 03 August 2023 | TW 202334206 A | 01 September 2023 |
| WO 2022078279 A1 | 21 April 2022 | None | |

**INTERNATIONAL SEARCH REPORT**
**Information on patent family members**

International application No.
**PCT/CN2023/133434**

| Patent document cited in search report | | | Publication date (day/month/year) | Patent family member(s) | | | Publication date (day/month/year) |
|---|---|---|---|---|---|---|---|
| CN | 103509030 | A | 15 January 2014 | WO | 2013189266 | A1 | 27 December 2013 |
| | | | | US | 2015166559 | A1 | 18 June 2015 |
| | | | | US | 9458170 | B2 | 04 October 2016 |
| | | | | EP | 2862571 | A1 | 22 April 2015 |
| | | | | EP | 2862571 | A4 | 16 March 2016 |
| CN | 102718770 | A | 10 October 2012 | CN | 102718770 | B | 04 June 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211545125
- CN 202310683602
- WO 2019238046 A
- CN 111065621 A
- WO 2019238046 A1
- WO 2021093820 A1
- WO 2022140504 A1
- CN 113402584 A
- WO 2022056696 A1
- WO 2021228141 A1
- WO 2022262516 A1
- WO 2020077038 A1
- WO 2022135332 A1
- CN 1572882 A
- WO 2021190602 A1
- WO 2019114666 A1
- WO 2022068898 A1


### Non-patent literature cited in the description


- **KABAT, E.A. et al.** Sequences of Proteins of Immunological Interest. 1991
- **QUEEN et al.** *Proc., Natl. Acad. Sci. USA*, 1991, vol. 88, 2869
- **JONES et al.** *Nature*, 1986, vol. 321, 522
- **RIECHMANN et al.** *Nature*, 1988, vol. 332, 323-327
- **VERHOEYEN et al.** *Science*, 1988, vol. 239, 1534
- **WARD et al.** *Nature*, 1989, vol. 341, 544-546
- **BIRD et al.** *Science*, 1988, vol. 242, 423-426
- **HUSTON et al.** *Proc. Natl. Acad. Sci USA*, 1988, vol. 85, 5879-5883
- **HOLLIGER et al.** *Proc. Natl. Acad. Sci. USA*, 1993, vol. 90, 6444-6448
- **ALFTHAN et al.** *Proc. Natl. Acad. Sci. USA*, 1995, vol. 90, 6444-6448
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol*, 2001
- **KABAT E.A. et al.** Sequences of proteins of immunological interest. 1991
- Epitope Mapping Protocols in Methods in Molecular Biology. 1996, vol. 66
- *Journal of Immunoglobulins*, 2001, ISBN 012441351
- *Organic Letters*, 2005, vol. 7 (4), 713-716
- *Organic Letters*, 2017, vol. 19 (11), 2869-2872
- *Journal of Physical Organic Chemistry*, 2010, vol. 16 (10), 682-690
- *CHEMICAL ABSTRACTS*, 1599440-33-1